(19) Europäisches Patentamt
European Patent Office
Office européen des brevets

(11) EP 4 671 249 A1

(12) # EUROPEAN PATENT APPLICATION
published in accordance with Art. 153(4) EPC

(43) Date of publication:
**31.12.2025 Bulletin 2026/01**

(21) Application number: **24759743.8**

(22) Date of filing: **22.02.2024**

(51) International Patent Classification (IPC):
***C07D 491/22*** (2006.01) ***C07D 491/147*** (2006.01)
***A61K 47/68*** (2017.01) ***A61K 47/54*** (2017.01)
***A61K 47/65*** (2017.01) ***A61K 31/4745*** (2006.01)
***A61K 31/437*** (2006.01) ***A61K 39/395*** (2006.01)
***A61P 35/00*** (2006.01) ***C07K 5/062*** (2006.01)
***C07K 5/103*** (2006.01) ***C07K 16/28*** (2006.01)

(52) Cooperative Patent Classification (CPC):
**A61K 31/437; A61K 31/4745; A61K 39/395; A61K 47/54; A61K 47/65; A61K 47/68; A61P 35/00; C07D 491/147; C07D 491/22; C07K 5/06017; C07K 5/1005; C07K 16/28**

(86) International application number:
**PCT/CN2024/078189**

(87) International publication number:
**WO 2024/175069 (29.08.2024 Gazette 2024/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **23.02.2023 CN 202310158951**
**14.07.2023 PCT/CN2023/107489**

(71) Applicant: **Phrontline Biopharma (Hangzhou)Co., Ltd**
**Hangzhou, Zhejiang 311103 (CN)**

(72) Inventors:
- **CHEN, Zhaoyuan**
**Hangzhou, Zhejiang 311103 (CN)**
- **MAO, Yanli**
**Hangzhou, Zhejiang 311103 (CN)**

(74) Representative: **Dantz, Jan Henning et al**
**Loesenbeck - Specht - Dantz**
**Patent- und Rechtsanwälte**
**Am Zwinger 2**
**33602 Bielefeld (DE)**

(54) **CAMPTOTHECIN DERIVATIVE, CONJUGATE THEREOF, PREPARATION METHOD THEREFOR AND MEDICAL USE THEREOF**

(57) The present invention relates to a camptothecin derivative, a conjugate thereof, a preparation method therefor and the medical use thereof, and particularly relates to a derivative of a camptothecin compound, a ligand-drug conjugate containing same, a pharmaceutical composition containing said conjugate, and the use of said conjugate in treatment of cancers.

EP 4 671 249 A1

## Description

### TECHNICAL FIELD

The present invention relates to a novel camptothecin derivative, a conjugate thereof, a preparation method thereof, a pharmaceutical composition comprising the same, and use thereof for preparing antineoplastic drugs.

### BACKGROUND

Antibody-Drug Conjugates (ADCs) are a relatively new class of anti-cancer drugs designed to combine the selectivity of monoclonal antibodies with the cell-killing properties of cytotoxic agents. ADCs have garnered great interest as a new therapeutic approach and continue to evolve. The design of ADCs involves linking monoclonal antibodies or antibody fragments to cytotoxic agents with biological activity through linkers. This harnesses the specificity of antibodies for tumor cell surface antigens and the efficiency of cytotoxic substances while avoiding the drawbacks of low antibody efficacy and high toxicity of cytotoxic agents. This means that, compared to traditional chemotherapy drugs, ADCs can more precisely target and kill tumor cells while reducing side effects on normal cells. The linker in ADC drugs is not only the molecular part that forms a covalent bond between the antibody and the small molecule drug but also a crucial element with design properties in targeted drug therapy. This involves multiple considerations: the addition of linkers should not induce aggregation; ensuring acceptable PK (pharmacokinetic) properties; improving stability in blood circulation; and the effective release of active molecules at the targeted site.

Camptothecin (CPT) is a pentacyclic quinoline alkaloid that was originally isolated from the wood and bark of the native Chinese tree species, Camptotheca acuminata. Camptothecin exhibits significant antitumor activity by inhibiting topoisomerase I. Topoisomerase I is an enzyme that is overexpressed in various tumor cell lines and is crucial for DNA synthesis. Camptothecin binds to the Topo I-DNA complex, stabilizing this complex, thereby preventing the re-ligation of the broken DNA strands, which in turn stops DNA replication and RNA synthesis. Due to its broad-spectrum antitumor activity and unique mechanism of action, efforts have been made to develop clinical analogs of camptothecin. Currently, only three camptothecin analogs are on the market: Irinotecan (approved by the FDA in 1994, Pfizer Inc.), Topotecan (approved by the FDA in 2007, Novartis), and Belotecan (approved for marketing in South Korea in 2003, ChongKunDang Pharmaceuticals).

However, camptothecin and most of its derivatives have poor solubility and low activity under physiological conditions, limiting the clinical development of camptothecin analogs. Therefore, making camptothecin into ADCs can overcome these limitations. Irinotecan is a prodrug, and its active metabolite, SN-38, has poor solubility and a short half-life. Immunomedics has linked SN-38 as a cytotoxic agent to sacituzumab, which targets cancer cells expressing Trop-2, for treating adult patients with metastatic triple-negative breast cancer (TNBC). The humanized sacituzumab govitecan-hziy lyophilized injection was approved by the FDA in April 2022 for treatment (US7999083 B2).

The amino group of DX-8951f (exatecan) contributes to its solubility, while the rigidity conferred by the cyclohexane ring is thought to favor the balance between the active lactone form and the inactive hydroxy acid form, thus enhancing its activity. However, clinical trials did not meet the expected endpoints. Daiichi Sankyo used amino-hydroxyacetylation to generate DXd, which is 2-4 times less active than exatecan (US 20210169852 A). DXd, linked to an anti-HER2 antibody via an enzyme-cleavable Gly-Gly-Phe-Gly tetrapeptide linker, produced an ADC (Enhertu) that showed significant potential in targeting HER2-expressing cancers in clinical settings. Consequently, Enhertu received accelerated FDA approval on December 20, 2019, for the treatment of HER2-positive, unresectable, or metastatic breast cancer in adults who have received two or more prior anti-HER2 regimens for metastatic disease. While the cyclohexane ring of DXd is thought to stabilize the biologically active lactone form, it introduces a chiral center, complicating synthesis and SAR studies. To overcome this challenge, researchers at ImmunoGen designed a set of new camptothecin analogs with the ring opened and the additional chiral center eliminated, introducing a group at position 7 for antibody conjugation. When conjugated with an anti-EGFR antibody (HuEGFR), the resulting ADC was effective against EGFR-positive HSC-2 tumor xenograft models (US20210077482A1). Researchers at MediBoston applied a similar approach by deriving a functional group for antibody conjugation at position 9, incorporating a hydrophilic polypeptide linker, and achieved favorable preclinical results with the resulting ADC (WO 2021173773).

Although camptothecin analogs exhibit good antitumor activity, especially when conjugated with humanized antibodies, offering excellent targeting and tumor-killing activity with lower effective doses, thereby reducing toxicity and increasing the therapeutic window, different types of camptothecin derivatives have significantly different half-lives, membrane permeability, and *in vitro* activity. Additionally, their cytotoxicity against different cancer cells varies greatly. Therefore, we aim to design new structures to improve the activity and membrane permeability of camptothecin derivatives (i.e., increase the bystander effect of camptothecin derivatives), thereby enhancing the killing of tumor cells with low or no antigen expression, in the hope of achieving certain clinical effects.

## SUMMARY

One aspect of the present invention provides a compound represented by general formula (A) or a pharmaceutically acceptable salt thereof;

$$L\text{-}L_2\text{-}L_1\text{-}Dr \qquad (A)$$

wherein:

Dr is selected from the following structures:

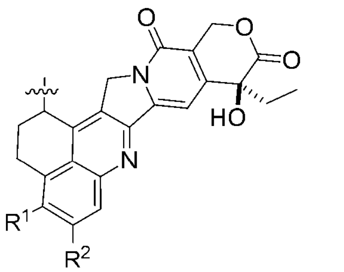
,
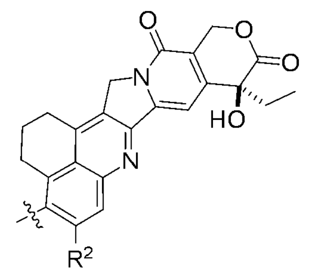
,
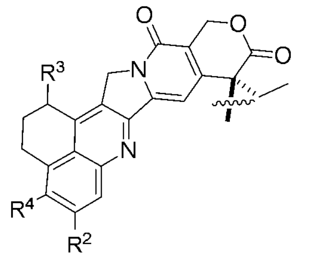
,
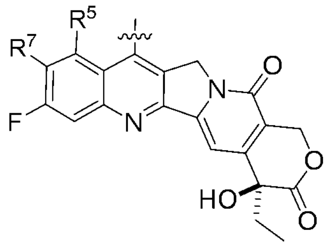
, and
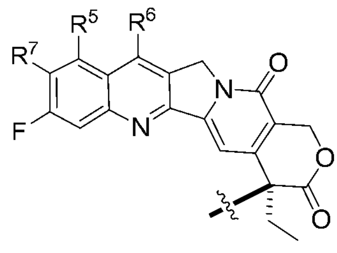
;

$R^1$ is selected from hydrogen, halogen, hydroxyl, amino, cyano, alkyl, alkoxy, alkenyl, alkynyl, cycloalkyl, $-NR^dR^e$, $-(CH_2)_m-OH$, $-(CH_2)_m-NR^dR^e$, $-OC(=O)NR^f-(CH_2)_m-OH$, $-OC(=O)NR^f-(CH_2)_m-NR^dR^e$, $-(CH_2)_m-C(=O)OH$, $-(CH_2)_m-C(=O)-NR^dR^e$, $-(CH_2)_m-C(=O)NR^f-(CH_2)_n-OH$, $-(CH_2)_m-C(=O)NR^f-(CH_2)_n-NR^dR^e$, $-NR^f-(CH_2)_m-OH$, $-NR^f-(CH_2)_m-NR^dR^e$, $-O-(CH_2)_m-OH$, $-O-(CH_2)_m-NR^dR^e$, $-NR^fC(=O)O-(CH_2)_m-OH$, $-NR^bC(=O)O-(CH_2)_m-NR^dR^e$, $-(CH_2)_m-NR^fC(=O)O-(CH_2)_n-OH-$, $-(CH_2)_m-NR^fC(=O)O-(CH_2)_n-NR^dR^e$, $-(CH_2)_m-OC(=O)NR^f-(CH_2)_n-NR^dR^e$, $-(CH_2)_m-OC(=O)NR^f-(CH_2)_n-OH$, $-(CH_2)_m-NR^fC(=O)-(CH_2)_n-NR^dR^e$, $-(CH_2)_m-NR^fC(=O)-(CH_2)_n-OH$, and $-(CH_2)_m-NR^fC(=O)-G-(CH_2)_n-OH$, wherein the alkyl, alkoxy, alkenyl, alkynyl, and cycloalkyl are optionally further substituted by one or more groups selected from halogen, deuterium, amino, alkyl, and hydroxyl;

$R^2$ is selected from hydrogen, halogen, hydroxyl, amino, cyano, alkyl, alkoxy, alkenyl, and alkynyl, wherein the alkyl, alkoxy, alkenyl, and alkynyl are optionally further substituted by one or more groups selected from halogen;

$R^3$ is selected from hydrogen, halogen, hydroxyl, amino, cyano, alkyl, alkoxy, alkenyl, alkynyl, $CH_2=$, $-NR^dR^e$, $-(CH_2)_m-OH$, $-(CH_2)_m-NR^dR^e$, $-OC(=O)NR^f-(CH_2)_m-OH$, $-OC(=O)NR^f-(CH_2)_m-NR^dR^e$, - $(CH_2)_m-C(=O)OH$, $-(CH_2)_m-C(=O)-NR^dR^e$, $-(CH_2)_m-C(=O)NR^f-(CH_2)_n-OH$, $-(CH_2)_m-C(=O)NR^f-(CH_2)_n-NR^dR^e$, $-NR^f-(CH_2)_m-OH$, $-NR^f-(CH_2)_m-NR^dR^e$, $-NR^f-C(=O)R^d$, $-NR^fC(=O)-(CH_2)_m-R^d$, - $NR^fC(=O)O-(CH_2)_m-R^d$, $-O-(CH_2)_m-OH$, $-O-(CH_2)_m-NR^dR^e$, $-NR^fC(=O)O-(CH_2)_m-OH$, - $NR^fC(=O)NR^d-(CH_2)_m-OH$, $-NR^fC(=O)NR^d-(CH_2)_m-O-(CH_2)_n-OH$, $-NR^fC(=O)O-(CH_2)_m-O-(CH_2)_n-OH$, $-NR^fC(=O)NR^d-(CH_2)_m-O-(CH_2)_n-NR^dR^e$, $-NR^fC(=O)O-(CH_2)_m-O-(CH_2)_n-NR^dR^e$, - $NR^fC(=O)NR^d-(CH_2)_m-NR^dR^e$, $-NR^fC(=O)O-(CH_2)_m-NR^dR^e$, $-(CH_2)_m-NR^fC(=O)O-(CH_2)_n-OH$, - $(CH_2)_m-NR^fC(=O)O-(CH_2)_n-NR^dR^e$, $-(CH_2)_m-OC(=O)NR^f-(CH_2)_n-NR^dR^e$, $-(CH_2)_m-OC(=O)NR^f-(CH_2)_n-OH$, $-(CH_2)_m-NR^fC(=O)-(CH_2)_n-NR^dR^e$, and $-(CH_2)_m-NR^fC(=O)-(CH_2)_n-OH$, wherein the alkyl, alkoxy, alkenyl, and alkynyl are optionally further substituted by one or more groups selected from halogen, deuterium, amino, alkyl, and hydroxyl;

$R^4$ is selected from hydrogen, halogen, hydroxyl, carboxyl, amino, cyano, alkyl, alkoxy, alkenyl, alkynyl, cycloalkyl, $-NR^dR^e$, $-(CH_2)_m-OH$, $-(CH_2)_m-NR^dR^e$, $-OC(=O)NR^f-(CH_2)_m-OH$, $-OC(=O)NR^f-(CH_2)_m-NR^dR^e$, $-(CH_2)_m-C(=O)OH$, $-(CH_2)_m-C(=O)-NR^dR^e$, $-(CH_2)_m-C(=O)NR^f-(CH_2)_n-OH$, - $(CH_2)_m-C(=O)NR^f-(CH_2)_n-NR^dR^e$, $-NR^f-(CH_2)_m-OH$,

$-NR^f-(CH_2)_m-NR^dR^e$, $-O-(CH_2)_m-OH$, $-O-(CH_2)_m-NR^dR^e$, $-NR^fC(=O)O-(CH_2)_m-OH$, $-NR^bC(=O)O-(CH_2)_m-NR^dR^e$, $-(CH_2)_m-NR^fC(=O)O-(CH_2)_n-OH-$, $-(CH_2)_m-NR^fC(=O)O-(CH_2)_n-NR^dR^e$, $-(CH_2)_m-OC(=O)NR^f-(CH_2)_n-NR^dR^e$, $-(CH_2)_m-OC(=O)NR^f-(CH_2)_n-OH$, $-(CH_2)_m-NR^fC(=O)-(CH_2)_n-NR^dR^e$, $-(CH_2)_m-NR^fC(=O)-(CH_2)_n-OH$, and $-(CH_2)_m-NR^fC(=O)-G-(CH_2)_n-OH$, wherein the alkyl, alkoxy, alkenyl, alkynyl, and cycloalkyl are optionally further substituted by one or more groups selected from halogen, deuterium, amino, alkyl, and hydroxyl; the $-(CH_2)m-$ is optionally further substituted by one or more deuterium or halogen groups; and G is selected from cycloalkylene, heterocyclylene, heteroarylene, and arylene;

$R^5$ is selected from hydrogen, halogen, hydroxyl, amino, cyano, alkyl, alkoxy, alkenyl, and alkynyl, wherein the alkyl, alkoxy, alkenyl, and alkynyl are optionally further substituted by one or more groups selected from halogen;

$R^6$ is selected from hydrogen, halogen, hydroxyl, amino, cyano, alkyl, alkoxy, alkenyl, and alkynyl, wherein the alkyl, alkoxy, alkenyl, and alkynyl are optionally further substituted by one or more groups selected from halogen;

$R^7$ is selected from hydrogen, halogen, hydroxyl, amino, cyano, alkyl, alkoxy, alkenyl, and alkynyl, wherein the alkyl, alkoxy, alkenyl, and alkynyl are optionally further substituted by one or more groups selected from halogen;

$R^d$ and $R^e$ are each independently selected from hydrogen and alkyl;

$R^f$ is selected from hydrogen, alkyl, $-C(O)R^c$, $-S(O)R^c$, and $-S(O)_2R^c$, wherein the alkyl is optionally further substituted by cycloalkyl; and $R^c$ is selected from hydrogen, hydroxyl, and alkyl;

$L_1$ is selected from a bond, $-(CH_2)_m-*$, $-O-*$, $-NR^a-*$, $-(CH_2)_m-O-*$, $-(CH_2)_m-NR^a-*$, $-OC(=O)NR^b-(CH_2)_m-O-*$, $-OC(=O)NR^b-(CH_2)_m-NR^a-*$, $-(CH_2)_m-C(=O)O-*$, $-(CH_2)_m-C(=O)NR^a-*$, $-(CH_2)_m-C(=O)NR^b-(CH_2)_n-O-*$, $-(CH_2)_m-C(=O)NR^b-(CH_2)_n-NR^a-*$, $-NR^b-(CH_2)_m-O-*$, $-NR^b-(CH_2)_m-NR^a-*$, $-O-(CH_2)_m-O-*$, $-O-(CH_2)_m-NR^a-*$, $-NR^bC(=O)O-(CH_2)_m-O-*$, $-NR^bC(=O)O-(CH_2)_m-NR^a-*$, $-(CH_2)_m-NR^bC(=O)O-(CH_2)_n-O-*$, $-(CH_2)_m-NR^bC(=O)O-(CH_2)_n-NR^a-*$, $-(CH_2)_m-OC(=O)NR^b-(CH_2)_n-NR^a-*$, $-(CH_2)_m-OC(=O)NR^b-(CH_2)_n-O-*$, $-(CH_2)_m-NR^bC(=O)-(CH_2)_n-NR^a-*$, and $-(CH_2)_m-NR^bC(=O)-(CH_2)_n-O-*$, wherein * is a connection site with $L_2$;

$R^a$ and $R^b$ are each independently selected from hydrogen, alkyl, $-C(O)R^c$, $-S(O)R^c$, and $-S(O)_2R^c$, wherein the alkyl is optionally further substituted by cycloalkyl; and $R^c$ is selected from hydrogen, hydroxyl, and alkyl;

$L_2$ is selected from a bond,

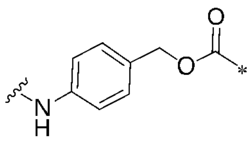,
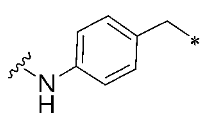,
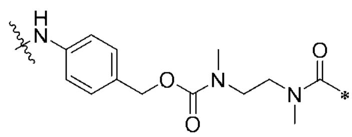,

and

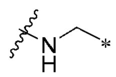,

wherein * is a connection site with $L_1$;

L is

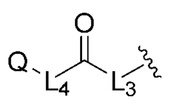 or 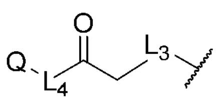;

$L_3$ is an amino acid residue formed by two or more amino acids, $L_3$ optionally comprises one or more of the following structures, and $L_6$ is selected from one or more of the following structures:

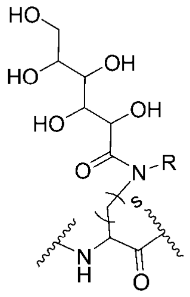, 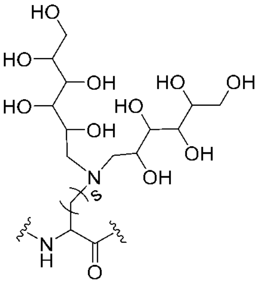, 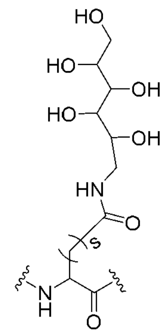, 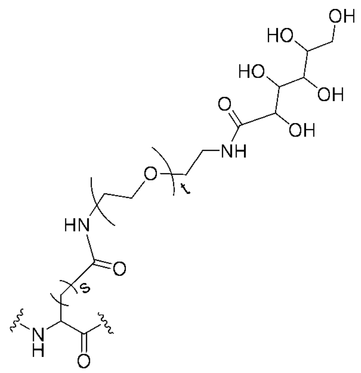,

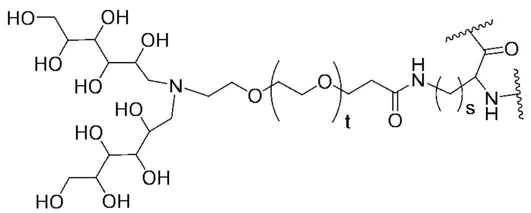, 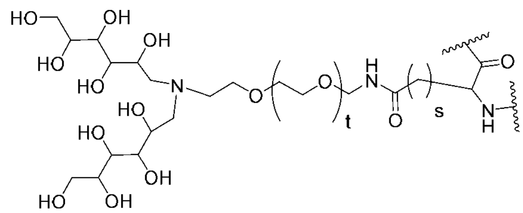,

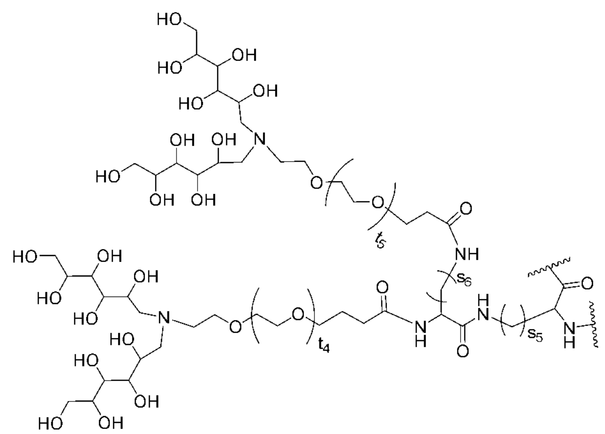, 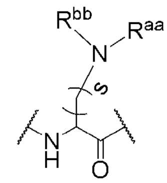,

or

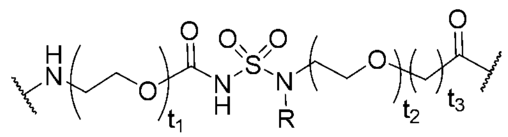,

wherein R, $R^{aa}$, and $R^{bb}$ are each independently selected from hydrogen and alkyl;

$L_4$ is

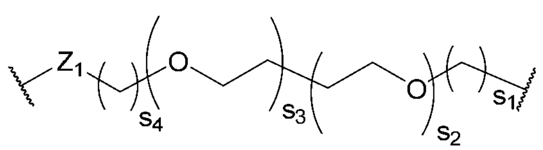;

$Z_1$ is selected from a bond, $-(CH_2)_p-$, $-(C_2H_4O)_q-$, $-(CH_2)_p-C(O)NH-$, $-(CH_2)_p-O-(CH_2)_p-C(O)NH-$, $-(CH_2)_p-C(O)-L_6-NH-$, $-(CH_2)_p-O-(CH_2)_p-C(O)-L_6-NH-$, -C(O)NH-, -C(O)O-, -C(O)-, -OC(O)-, -NH-, -O-, and -OC(O)NH-;

m is an integer from 1 to 6;

n is an integer from 1 to 6;

s is an integer from 1 to 6;

t is an integer from 0 to 10;

$s_1$, $s_2$, $s_3$, and $s_4$ are each independently an integer from 0 to 10; preferably, an integer from 0 to 8, or an integer from 0 to 6, more preferably, an integer from 0 to 4, more preferably, an integer from 0 to 2, or an integer from 1 to 2;

$s_5$ and $s_6$ are each independently an integer from 1 to 6;

$t_1$ is an integer from 1 to 6;

$t_2$ is an integer from 0 to 6;

$t_3$ is an integer from 1 to 6;

$t_4$ is an integer from 0 to 10;

$t_5$ is an integer from 0 to 10;

p is an integer from 1 to 10;

q is an integer from 1 to 10; and

Q is a linker unit;

wherein $Z_1$ is connected to Q.

In a specific embodiment, in the compound represented by the general formula (A) or the pharmaceutically acceptable salt thereof according to the present invention,

Dr is selected from the following structures:

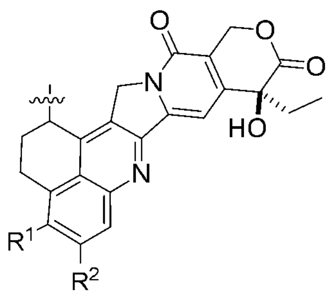
;

$L_1$ is selected from -O-*, $-NR^a-*$, $-(CH_2)_m-O-*$, $-(CH_2)_m-NR^a-*$, $-OC(=O)NR^d-(CH_2)_m-O-*$, $-OC(=O)NR^b-(CH_2)_m-NR^a-*$, $-(CH_2)_m-C(=O)O-*$, $-(CH_2)_m-C(=O)NR^a-*$, $-NR^b-(CH_2)_m-O-*$, $-NR^b-(CH_2)_m-NR^a-*$, $-O-(CH_2)_m-O-*$, $-O-(CH_2)_m-NR^a-*$, $-NR^bC(=O)O-(CH_2)_m-O-*$, $-NR^bC(=O)O-(CH_2)_m-NR^a-*$, $-(CH_2)_m-NR^bC(=O)-(CH_2)_n-NR^a-*$, and $-(CH_2)_m-NR^bC(=O)-(CH_2)_n-O-*$, wherein * is a connection site with $L_2$;

$R^a$ is selected from hydrogen and $C_1$-$C_6$ alkyl;

$R^b$ is selected from hydrogen, alkyl, $-C(O)R^c$, $-S(O)R^c$, and $-S(O)_2R^c$, wherein $R^c$ is selected from hydrogen, hydroxyl, and $C_1$-$C_6$ alkyl;

m is an integer from 1 to 6; preferably, an integer from 1 to 4; more preferably, an integer from 1 to 2;

n is an integer from 1 to 6; preferably, an integer from 1 to 4; more preferably, an integer from 1 to 2;

$R^1$ is selected from hydrogen, halogen, hydroxyl, amino, cyano, $C_1$-$C_6$ alkyl, $C_1$-$C_6$ alkoxy, $C_2$-$C_6$ alkenyl, and $C_2$-$C_6$ alkynyl, wherein the $C_1$-$C_6$ alkyl, $C_1$-$C_6$ alkoxy, $C_2$-$C_6$ alkenyl, and $C_2$-$C_6$ alkynyl are optionally further substituted by one or more groups selected from halogen; and the $C_1$-$C_6$ alkyl and $C_1$-$C_6$ alkoxy are preferred; and

$R^2$ is selected from hydrogen, halogen, hydroxyl, amino, cyano, $C_1$-$C_6$ alkyl, $C_1$-$C_6$ alkoxy, $C_2$-$C_6$ alkenyl, and $C_2$-$C_6$ alkynyl, wherein the $C_1$-$C_6$ alkyl, $C_1$-$C_6$ alkoxy, $C_2$-$C_6$ alkenyl, and $C_2$-$C_6$ alkynyl are optionally further substituted by one or more groups selected from halogen; wherein halogen is preferred.

In another specific embodiment, in the compound represented by the general formula (A) or the pharmaceutically acceptable salt thereof according to the present invention,

Dr is selected from the following structures:

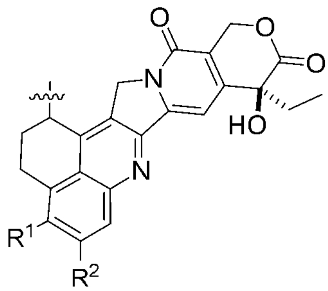
;

$L_1$ is selected from -O-*, -$NR^a$-*, -$(CH_2)_m$-O-*, -$(CH_2)_m$-$NR^a$-*, -OC(=O)$NR^d$-$(CH_2)_m$-O-*, - OC(=O)$NR^b$-$(CH_2)_m$-$NR^a$-*, -$(CH_2)_m$-C(=O)O-*, -$(CH_2)_m$-C(=O)$NR^a$-*, -$NR^b$-$(CH_2)_m$-O-*, -$NR^b$-$(CH_2)_m$-$NR^a$-*, -O-$(CH_2)_m$-O-*, -O-$(CH_2)_m$-$NR^a$-*, -$NR^b$C(=O)O-$(CH_2)_m$-O-*, -$NR^b$C(=O)O-$(CH_2)_m$-$NR^a$-*, -$(CH_2)_m$-$NR^b$C(=O)-$(CH_2)_n$-$NR^a$-*, and -$(CH_2)_m$-$NR^b$C(=O)-$(CH_2)_n$-O-*, wherein * is a connection site with $L_2$;

$R^a$ is selected from hydrogen and $C_1$-$C_6$ alkyl;

$R^b$ is selected from hydrogen, alkyl, -C(O)$R^c$, -S(O)$R^c$, and -S(O)$_2R^c$, wherein the alkyl is optionally further substituted by cycloalkyl; and $R^c$ is selected from hydrogen, hydroxyl, and $C_1$-$C_6$ alkyl;

m is an integer from 1 to 6; preferably, an integer from 1 to 4; more preferably, an integer from 1 to 2;

n is an integer from 1 to 6; preferably, an integer from 1 to 4; more preferably, an integer from 1 to 2;

$R^1$ is selected from hydrogen, halogen, hydroxyl, amino, cyano, $C_1$-$C_6$ alkyl, $C_1$-$C_6$ alkoxy, $C_2$-$C_6$ alkenyl, $C_2$-$C_6$ alkynyl, $C_3$-$C_6$ cycloalkyl, -$NR^dR^e$, -$(CH_2)_m$-OH, -$(CH_2)_m$-$NR^dR^e$, -OC(=O)$NR^f$-$(CH_2)_m$-OH, -OC(=O)$NR^f$-$(CH_2)_m$-$NR^dR^e$, -$(CH_2)_m$-C(=O)OH, -$(CH_2)_m$-C(=O)-$NR^dR^e$, -$(CH_2)_m$-C(=O)$NR^f$-$(CH_2)_n$-OH, -$(CH_2)_m$-C(=O)$NR^f$-$(CH_2)_n$-$NR^dR^e$, -$NR^f$-$(CH_2)_m$-OH, -$NR^f$-$(CH_2)_m$-$NR^dR^e$, -O-$(CH_2)_m$-OH, -O-$(CH_2)_m$-$NR^dR^e$, -$NR^f$C(=O)O-$(CH_2)_m$-OH, -$NR^b$C(=O)O-$(CH_2)_m$-$NR^dR^e$, - $(CH_2)_m$-$NR^f$C(=O)O-$(CH_2)_n$-OH-, -$(CH_2)_m$-$NR^f$C(=O)O-$(CH_2)_n$-$NR^dR^e$, -$(CH_2)_m$-OC(=O)$NR^f$-$(CH_2)_n$-$NR^dR^e$, -$(CH_2)_m$-OC(=O)$NR^f$-$(CH_2)_n$-OH, -$(CH_2)_m$-$NR^f$C(=O)-$(CH_2)_n$-$NR^dR^e$, -$(CH_2)_m$-$NR^f$C(=O)-$(CH_2)_n$-OH, and -$(CH_2)_m$-$NR^f$C(=O)-G-$(CH_2)_n$-OH, wherein the $C_1$-$C_6$ alkyl, $C_1$-$C_6$ alkoxy, $C_2$-$C_6$ alkenyl, $C_2$-$C_6$ alkynyl, and $C_3$-$C_6$ cycloalkyl are optionally further substituted by one or more groups selected from halogen, deuterium, amino, $C_1$-$C_6$ alkyl, and hydroxyl; the $C_1$-$C_6$ alkyl and $C_1$-$C_6$ alkoxy are preferred; the -$(CH_2)_m$- is optionally substituted by one or more deuterium or halogen groups; and G is selected from cycloalkylene, heterocyclylene, heteroarylene, and arylene;

$R^2$ is selected from hydrogen, halogen, hydroxyl, amino, cyano, $C_1$-$C_6$ alkyl, $C_1$-$C_6$ alkoxy, $C_2$-$C_6$ alkenyl, and $C_2$-$C_6$ alkynyl, wherein the $C_1$-$C_6$ alkyl, $C_1$-$C_6$ alkoxy, $C_2$-$C_6$ alkenyl, and $C_2$-$C_6$ alkynyl are optionally further substituted by one or more groups selected from halogen; wherein halogen is preferred; and

$R^d$, $R^e$, $R^f$, m, and n are defined as those in the general formula (A).

In another specific embodiment, in the compound represented by the general formula (A) or the pharmaceutically acceptable salt thereof according to the present invention,

Dr is selected from the following structures:

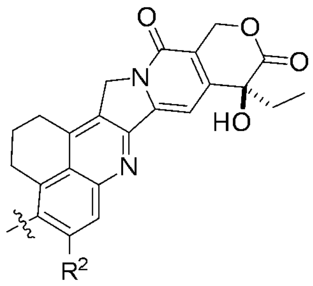

;

$L_1$ is selected from a bond, $-(CH_2)_m-*$, -O-*, $-(CH_2)_m-O-*$, $-NR^a-*$, $-(CH_2)_m-NR^a-*$, $-NR^b-(CH_2)_m-O-*$, $-NR^b-(CH_2)_m-NR^a-*$, $-O-(CH_2)_m-O-*$, $-O-(CH_2)_m-NR^a-*$, $-(CH_2)_m-OC(=O)NR^b-(CH_2)_n-NR^a-*$, $-(CH_2)_m-OC(=O)NR^b-(CH_2)_n-O-*$, $-(CH_2)_m-NR^bC(=O)-(CH_2)_n-NR^a-*$, and $-(CH_2)_m-NR^bC(=O)-(CH_2)_n-O-*$, wherein * is a connection site with $L_2$;

$R^a$ is selected from hydrogen and $C_1$-$C_6$ alkyl;

$R^b$ is selected from hydrogen, alkyl, $-C(O)R^c$, $-S(O)R^c$, and $-S(O)_2R^c$, wherein the alkyl is optionally further substituted by cycloalkyl; and $R^c$ is selected from hydrogen, hydroxyl, and $C_1$-$C_6$ alkyl;

m is an integer from 1 to 6; preferably, an integer from 1 to 4;

n is an integer from 1 to 6; preferably, an integer from 1 to 4; and

$R^2$ is selected from hydrogen, halogen, hydroxyl, amino, cyano, $C_1$-$C_6$ alkyl, $C_1$-$C_6$ alkoxy, $C_2$-$C_6$ alkenyl, and $C_2$-$C_6$ alkynyl, wherein the $C_1$-$C_6$ alkyl, $C_1$-$C_6$ alkoxy, $C_2$-$C_6$ alkenyl, and $C_2$-$C_6$ alkynyl are optionally further substituted by one or more groups selected from halogen; wherein halogen is preferred.

In another specific embodiment, in the compound represented by the general formula (A) or the pharmaceutically acceptable salt thereof according to the present invention,

Dr is selected from the following structures:

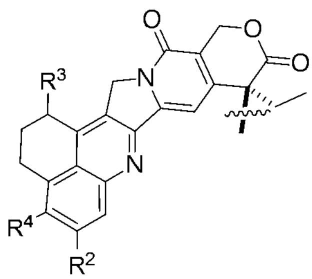

;

$L_1$ is selected from -O-*, wherein * is a connection site with $L_2$;

$R^2$ is selected from hydrogen, halogen, hydroxyl, amino, cyano, $C_1$-$C_6$ alkyl, $C_1$-$C_6$ alkoxy, $C_2$-$C_6$ alkenyl, and $C_2$-$C_6$ alkynyl, wherein the $C_1$-$C_6$ alkyl, $C_1$-$C_6$ alkoxy, $C_2$-$C_6$ alkenyl, and $C_2$-$C_6$ alkynyl are optionally further substituted by one or more groups selected from halogen; wherein halogen is preferred;

$R^3$ is selected from hydrogen, halogen, hydroxyl, amino, cyano, $C_1$-$C_6$ alkyl, $C_1$-$C_6$ alkoxy, $C_2$-$C_6$ alkenyl, $C_2$-$C_6$ alkynyl, $CH_2$=, $-NR^dR^e$, $-(CH_2)_m-OH$, $-(CH_2)_m-NR^dR^e$, $-OC(=O)NR^f-(CH_2)_m-OH$, $-OC(=O)NR^f-(CH_2)_m-NR^dR^e$, $-(CH_2)_m-C(=O)OH$, $-(CH_2)_m-C(=O)-NR^dR^e$, $-(CH_2)_m-C(=O)NR^f-(CH_2)_n-OH$, $-(CH_2)_m-C(=O)NR^f-(CH_2)_n-NR^dR^e$,

$-NR^f-(CH_2)_m-OH$, $-NR^f-(CH_2)_m-NR^dR^e$, $-NR^f-C(=O)R^d$, $-NR^f-C(=O)-(CH_2)_m-R^d$, $-NR^fC(=O)O-(CH_2)_m-R^d$, $-O-(CH_2)_m-OH$, $-O-(CH_2)_m-NR^dR^e$, - $NR^fC(=O)O-(CH_2)_m-OH$, $-NR^fC(=O)NR^d-(CH_2)_m-OH$, $-NR^fC(=O)NR^d-(CH_2)_m-O-(CH_2)_n-OH$, - $NR^fC(=O)O-(CH_2)_m-O-(CH_2)_n-OH$, $-NR^fC(=O)NR^d-(CH_2)_m-O-(CH_2)_n-NR^dR^e$, $-NR^fC(=O)O-(CH_2)_m-O-(CH_2)_n-NR^dR^e$, $-NR^fC(=O)NR^d-(CH_2)_m-NR^dR^e$, $-NR^fC(=O)O-(CH_2)_m-NR^dR^e$, $-(CH_2)_m-NR^fC(=O)O-(CH_2)_n-OH$, $-(CH_2)_m-NR^fC(=O)O-(CH_2)_n-NR^dR^e$, $-(CH_2)_m-OC(=O)NR^f-(CH_2)_n-NR^dR^e$, $-(CH_2)_m-OC(=O)NR^f-(CH_2)_n-OH$, $-(CH_2)_m-NR^fC(=O)-(CH_2)_n-NR^dR^e$, and $-(CH_2)_m-NR^fC(=O)-(CH_2)_n-OH$, wherein the $C_1$-$C_6$ alkyl, $C_1$-$C_6$ alkoxy, $C_2$-$C_6$ alkenyl, and $C_2$-$C_6$ alkynyl are optionally further substituted by one or more groups selected from halogen, deuterium, amino, $C_1$-$C_6$ alkyl, and hydroxyl; and hydrogen or hydroxyl is preferred;

$R^4$ is selected from hydrogen, halogen, hydroxyl, carboxyl, amino, cyano, $C_1$-$C_6$ alkyl, $C_1$-$C_6$ alkoxy, $C_2$-$C_6$ alkenyl, $C_2$-$C_6$ alkynyl, $C_3$-$C_6$ cycloalkyl, $-NR^dR^e$, $-(CH_2)_m-OH$, $-(CH_2)_m-NR^dR^e$, - $OC(=O)NR^f-(CH_2)_m-OH$, $-OC(=O)NR^f-(CH_2)_m-NR^dR^e$, $-(CH_2)_m-C(=O)OH$, $-(CH_2)_m-C(=O)-NR^dR^e$, $-(CH_2)_m-C(=O)NR^f-(CH_2)_n-OH$, $-(CH_2)_m-C(=O)NR^f-(CH_2)_n-NR^dR^e$, $-NR^f-(CH_2)_m-OH$, $-NR^f-(CH_2)_m-NR^dR^e$, $-O-(CH_2)_m-OH$, $-O-(CH_2)_m-NR^dR^e$, $-NR^fC(=O)O-(CH_2)_m-OH$, $-NR^bC(=O)O-(CH_2)_m-NR^dR^e$, $-(CH_2)_m-NR^fC(=O)O-(CH_2)_n-OH$-, $-(CH_2)_m-NR^fC(=O)O-(CH_2)_n-NR^dR^e$, $-(CH_2)_m-OC(=O)NR^f-(CH_2)_n-NR^dR^e$, $-(CH_2)_m-OC(=O)NR^f-(CH_2)_n-OH$, $-(CH_2)_m-NR^fC(=O)-(CH_2)_n-NR^dR^e$, - $(CH_2)_m-NR^fC(=O)-(CH_2)_n-OH$, and $-(CH_2)_m-NR^fC(=O)-G-(CH_2)_n-OH$, wherein the $C_1$-$C_6$ alkyl, $C_1$-$C_6$ alkoxy, $C_2$-$C_6$ alkenyl, $C_2$-$C_6$ alkynyl, and $C_3$-$C_6$ cycloalkyl are optionally further substituted by one or more groups selected from halogen, deuterium, amino, $C_1$-$C_6$ alkyl, and hydroxyl; hydroxyl, $C_1$-$C_6$ alkyl, and $C_1$-$C_6$ alkoxy are preferred; the $-(CH_2)_m-$ is optionally substituted by one or more deuterium or halogen groups; and G is selected from cycloalkylene, heterocyclylene, heteroarylene, and arylene; and

$R^d$, $R^e$, $R^f$, m, and n are defined as those in the general formula (A).

In another specific embodiment, in the compound represented by the general formula (A) or the pharmaceutically acceptable salt thereof according to the present invention,

Dr is selected from the following structures:

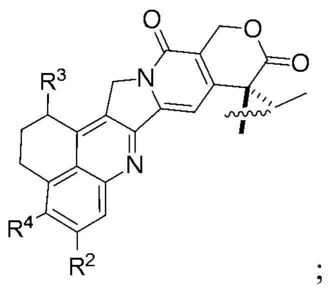
;

$L_1$ is selected from -O-*, wherein * is a connection site with $L_2$;

$R^2$ is selected from hydrogen, halogen, hydroxyl, amino, cyano, $C_1$-$C_6$ alkyl, $C_1$-$C_6$ alkoxy, $C_2$-$C_6$ alkenyl, and $C_2$-$C_6$ alkynyl, wherein the $C_1$-$C_6$ alkyl, $C_1$-$C_6$ alkoxy, $C_2$-$C_6$ alkenyl, and $C_2$-$C_6$ alkynyl are optionally further substituted by one or more groups selected from halogen; wherein halogen is preferred;

$R^3$ is selected from hydrogen, halogen, hydroxyl, amino, cyano, $C_1$-$C_6$ alkyl, $C_1$-$C_6$ alkoxy, $C_2$-$C_6$ alkenyl, $C_2$-$C_6$ alkynyl, and $CH_2$=, wherein the $C_1$-$C_6$ alkyl, $C_1$-$C_6$ alkoxy, $C_2$-$C_6$ alkenyl, and $C_2$-$C_6$ alkynyl are optionally further substituted by one or more groups selected from halogen; and hydrogen and hydroxyl are preferred; and

$R^4$ is selected from hydrogen, halogen, hydroxyl, amino, cyano, $C_1$-$C_6$ alkyl, $C_1$-$C_6$ alkoxy, $C_2$-$C_6$ alkenyl, and $C_2$-$C_6$ alkynyl, wherein the $C_1$-$C_6$ alkyl, $C_1$-$C_6$ alkoxy, $C_2$-$C_6$ alkenyl, and $C_2$-$C_6$ alkynyl are optionally further substituted by one or more groups selected from halogen; and hydroxyl, $C_1$-$C_6$ alkyl, and $C_1$-$C_6$ alkoxy are preferred.

In another specific embodiment, in the compound represented by the general formula (A) or the pharmaceutically acceptable salt thereof according to the present invention,

Dr is selected from the following structures:

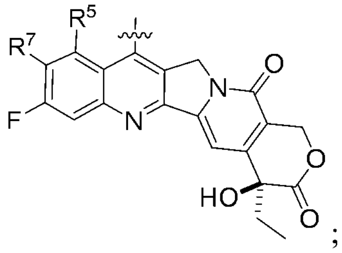

$L_1$ is selected from $-(CH_2)_m-O-*$ and $-(CH_2)_m-NR^a-*$, wherein * is a connection site with $L_2$;

m is an integer from 1 to 6; preferably, an integer from 1 to 4;

$R^a$ is selected from hydrogen and $C_1-C_6$ alkyl;

$R^5$ is selected from hydrogen, halogen, hydroxyl, amino, cyano, $C_1-C_6$ alkyl, $C_1-C_6$ alkoxy, $C_2-C_6$ alkenyl, and $C_2-C_6$ alkynyl, wherein the $C_1-C_6$ alkyl, $C_1-C_6$ alkoxy, $C_2-C_6$ alkenyl, and $C_2-C_6$ alkynyl are optionally further substituted by one or more groups selected from halogen; wherein halogen is preferred; and

$R^7$ is selected from hydrogen, halogen, hydroxyl, amino, cyano, $C_1-C_6$ alkyl, $C_1-C_6$ alkoxy, $C_2-C_6$ alkenyl, and $C_2-C_6$ alkynyl, wherein the $C_1-C_6$ alkyl, $C_1-C_6$ alkoxy, $C_2-C_6$ alkenyl, and $C_2-C_6$ alkynyl are optionally further substituted by one or more groups selected from halogen; wherein hydroxyl and amino are preferred.

In another specific embodiment, in the compound represented by the general formula (A) or the pharmaceutically acceptable salt thereof according to the present invention,

Dr is selected from the following structures:

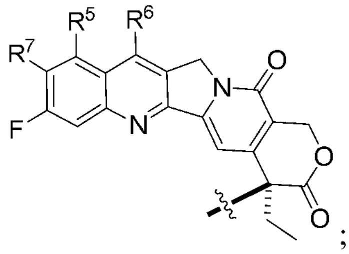

$L_1$ is selected from -O-*, wherein * is a connection site with $L_2$;

$R^5$ is selected from hydrogen, halogen, hydroxyl, amino, cyano, $C_1-C_6$ alkyl, $C_1-C_6$ alkoxy, $C_2-C_6$ alkenyl, and $C_2-C_6$ alkynyl, wherein the $C_1-C_6$ alkyl, $C_1-C_6$ alkoxy, $C_2-C_6$ alkenyl, and $C_2-C_6$ alkynyl are optionally further substituted by one or more groups selected from halogen; wherein halogen is preferred;

$R^6$ is selected from hydrogen, halogen, hydroxyl, amino, cyano, $C_1-C_6$ alkyl, $C_1-C_6$ alkoxy, $C_2-C_6$ alkenyl, and $C_2-C_6$ alkynyl, wherein the $C_1-C_6$ alkyl, $C_1-C_6$ alkoxy, $C_2-C_6$ alkenyl, and $C_2-C_6$ alkynyl are optionally further substituted by one or more groups selected from halogen; wherein hydroxyl and amino are preferred; and

$R^7$ is selected from hydrogen, halogen, hydroxyl, amino, cyano, $C_1-C_6$ alkyl, $C_1-C_6$ alkoxy, $C_2-C_6$ alkenyl, and $C_2-C_6$ alkynyl, wherein the $C_1-C_6$ alkyl, $C_1-C_6$ alkoxy, $C_2-C_6$ alkenyl, and $C_2-C_6$ alkynyl are optionally further substituted by one or more groups selected from halogen; wherein hydroxyl and amino are preferred.

In a preferred embodiment, in the compound represented by the general formula (A) or the pharmaceutically acceptable salt thereof according to the present invention, Dr is selected from

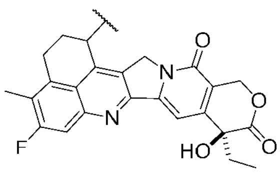, 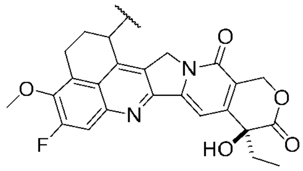, 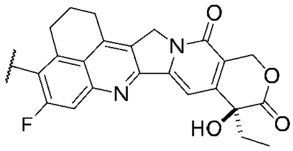, 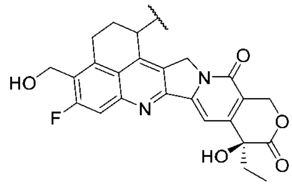,

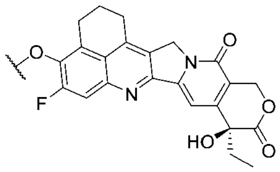, 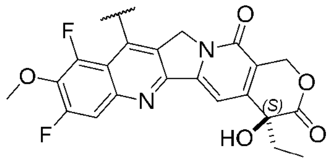, 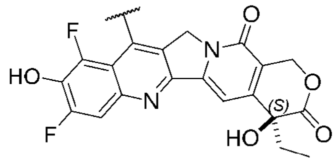,

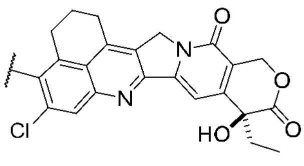, and 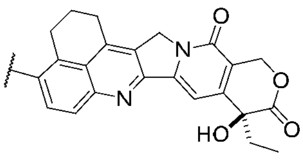.

In another preferred embodiment, in the compound represented by the general formula (A) or the pharmaceutically acceptable salt thereof according to the present invention, when $L_1$ is selected from $-NR^a-*$, $-(CH_2)_m-NR^a-*$, $-OC(=O)NR^b-(CH_2)_m-NR^a-*$, $-(CH_2)_m-C(=O)NR^a-*$, $-(CH_2)_m-C(=O)NR^b-(CH_2)_n-NR^a-*$, $-NR^b-(CH_2)_m-NR^a-*$, $-O-(CH_2)_m-NR^a-*$, $-NR^bC(=O)O-(CH_2)_m-NR^a-*$, $-(CH_2)_m-NR^bC(=O)O-(CH_2)_n-NR^a-*$, $-(CH_2)_m-OC(=O)NR^b-(CH_2)_n-NR^a-*$, and $-(CH_2)_m-NR^bC(=O)-(CH_2)_n-NR^a-*$, wherein * is a connection site with $L_2$,

$L_2$ is selected from a bond or

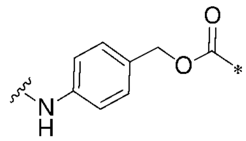,

wherein * is a connection site with $L_1$; and

$R^a$, $R^b$, m, and n are defined as those in the general formula (A).

In another preferred embodiment, in the compound represented by the general formula (A) or the pharmaceutically acceptable salt thereof according to the present invention,

$L_1$ is selected from a bond, $-O-*$, $-(CH_2)_m-O-*$, $-NR^a-*$, $-OC(=O)NR^b-(CH_2)_m-O-*$, $-(CH_2)_m-C(=O)O-*$, $-(CH_2)_m-C(=O)NR^d-(CH_2)_n-O-*$, $-NR^b-(CH_2)_m-O-*$, $-O-(CH_2)_m-O-*$, $-NR^bC(=O)O-(CH_2)_m-O-*$, $-(CH_2)_m-NR^bC(=O)O-(CH_2)_n-O-*$, $-(CH_2)_m-OC(=O)NR^b-(CH_2)_n-O-*$, and $-(CH_2)_m-NR^bC(=O)-(CH_2)_n-O-*$, wherein * is a connection site with $L_2$;

$L_2$ is selected from a bond,

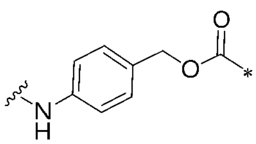, 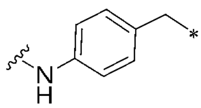, 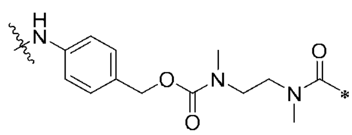,

and

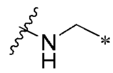,

wherein * is a connection site with $L_1$; and

$R^a$, $R^b$, m, and n are defined as those in the general formula (A).

In another preferred embodiment, in the compound represented by the general formula (A) or the pharmaceutically acceptable salt thereof according to the present invention,

$L_3$ is an amino acid residue formed by two or more amino acids selected from phenylalanine, alanine, glycine, valine, leucine, isoleucine, tryptophan, tyrosine, histidine, lysine, citrulline, serine, threonine, cysteine, glutamic acid, glutamine, aspartic acid, asparagine, methionine, and arginine, and $L_3$ optionally comprises one or more of the following structures:

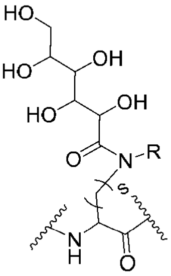, 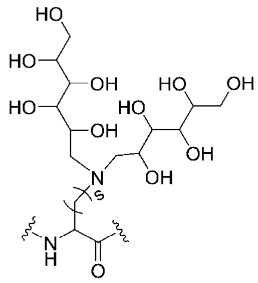, 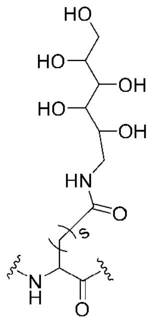, 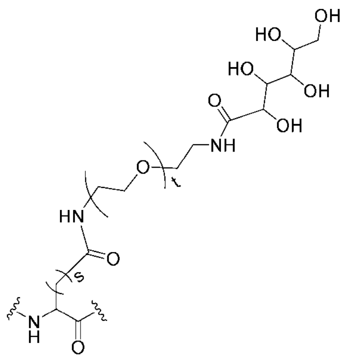,

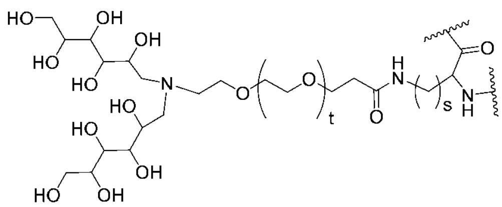, 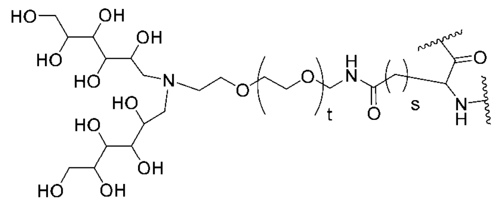,

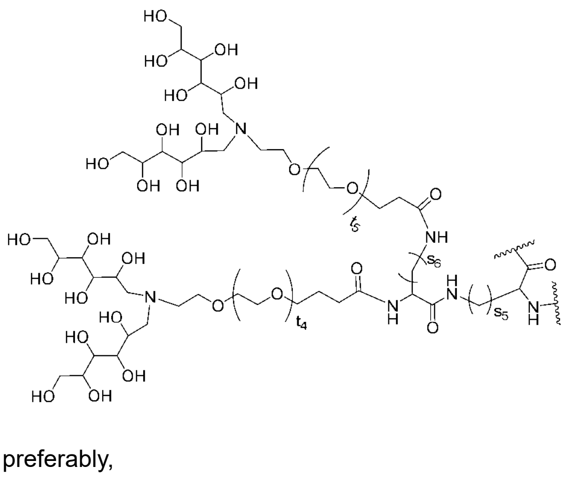, 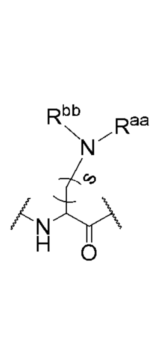, or 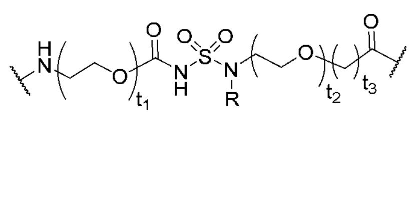;

preferably,

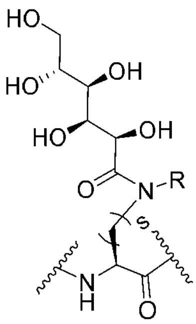, 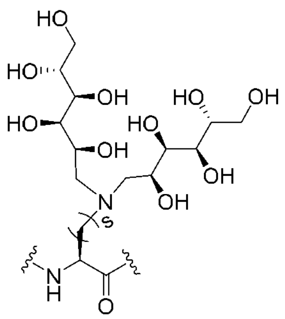, 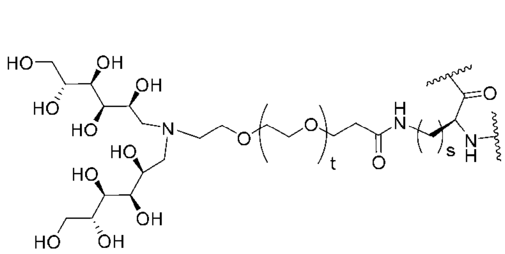,

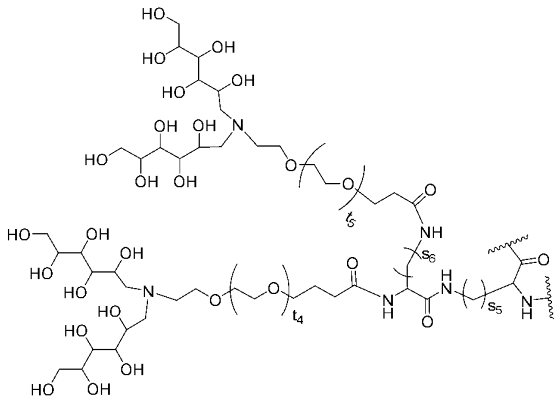, 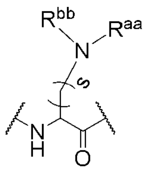, or 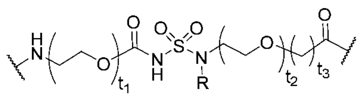,

wherein R, $R^{aa}$, and $R^{bb}$ are each independently selected from hydrogen and $C_1$-$C_6$ alkyl;

s is an integer from 1 to 6; preferably, an integer from 2 to 6; more preferably, an integer from 2 to 4;

$s_5$ and $s_6$ are each independently an integer from 1 to 6; preferably, an integer from 2 to 6; more preferably, an integer from 2 to 4;

t is an integer from 0 to 10; preferably, an integer from 2 to 8; more preferably, an integer from 3 to 7;

$t_1$ is an integer from 1 to 6; preferably, an integer from 1 to 4; more preferably, an integer from 2 to 4; or even more preferably 1 or 2;

$t_2$ is an integer from 0 to 6; preferably, an integer from 1 to 4; more preferably 1 or 2;

$t_3$ is an integer from 1 to 6; preferably, an integer from 1 to 4; more preferably 1 or 2;

$t_4$ is an integer from 0 to 10; and

$t_5$ is an integer from 0 to 10.

In another embodiment, $L_3$ is an amino acid residue formed by two or more amino acids selected from phenylalanine, alanine, glycine, valine, leucine, isoleucine, tryptophan, tyrosine, histidine, lysine, citrulline, serine, threonine, cysteine, glutamic acid, glutamine, aspartic acid, asparagine, methionine, and arginine, and $L_3$ optionally comprises one or more of the following structures:

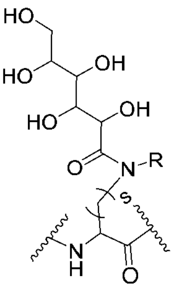,
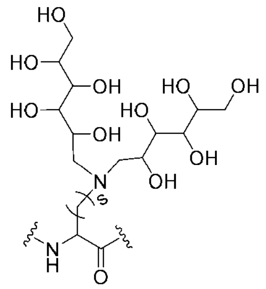,
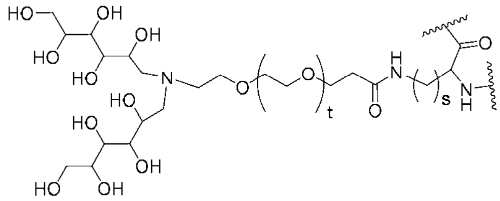,

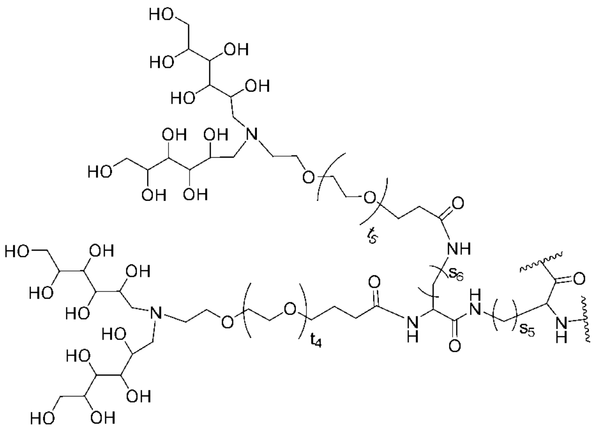,
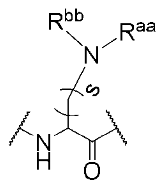,

or

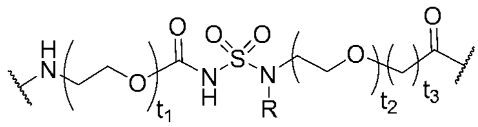;

wherein R, $R^{aa}$, $R^{bb}$, s, t, $t_1$ to $t_5$, $s_5$, and $s_6$ are the same as those described above.

In another preferred embodiment, in the compound represented by the general formula (A) or the pharmaceutically acceptable salt thereof according to the present invention, $L_3$ is

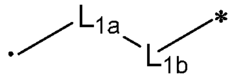,

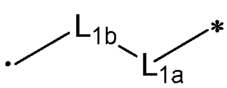, or 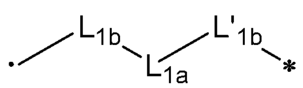,

* is a connection site with $L_2$, and ·is a connection site with carbonyl or methylene;

$L_{1b}$ and $L'_{1b}$ are each independently an amino acid residue formed by one or more amino acids selected from phenylalanine, alanine, glycine, valine, leucine, isoleucine, tryptophan, tyrosine, histidine, lysine, citrulline, serine, threonine, cysteine, glutamic acid, glutamine, aspartic acid, asparagine, methionine, and arginine;

$L_{1a}$ is a bond or selected from one or more of the following structures:

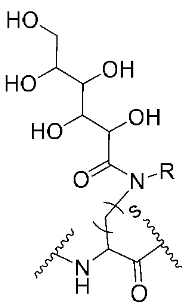, 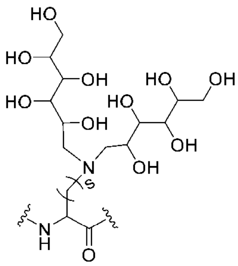, 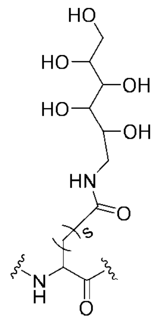, 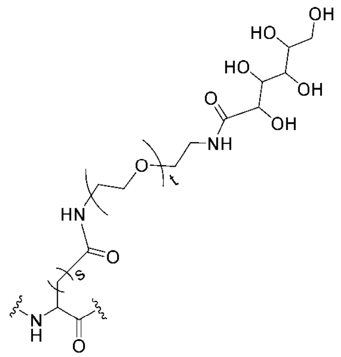,

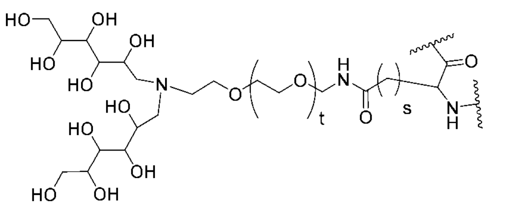, 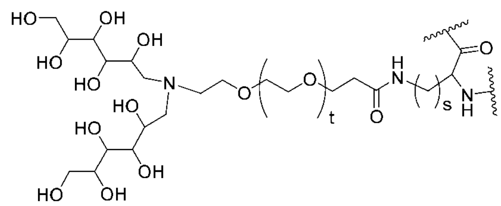,

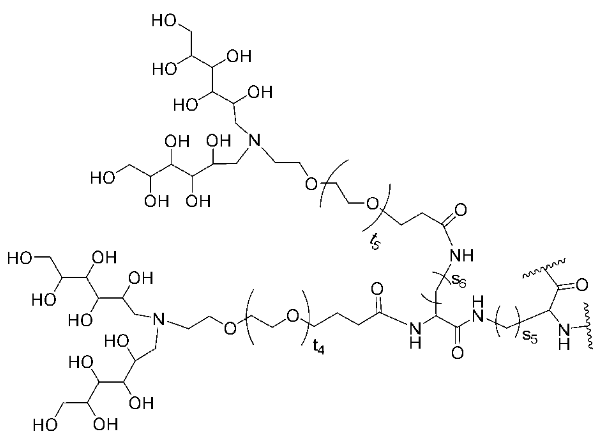, 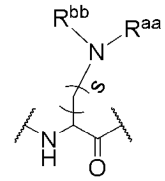,

or

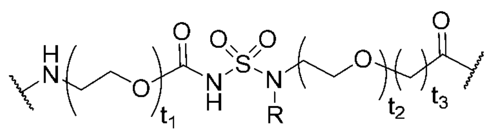;

preferably,

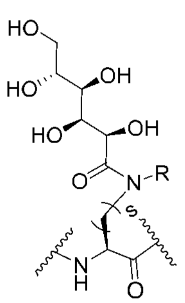, 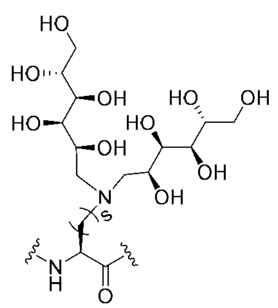, 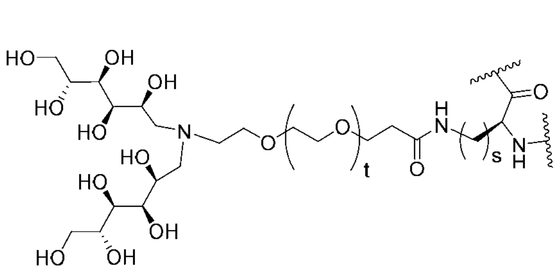,

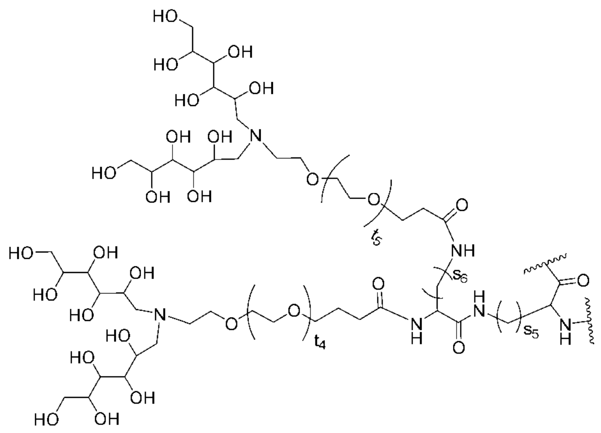,

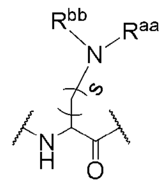,

or

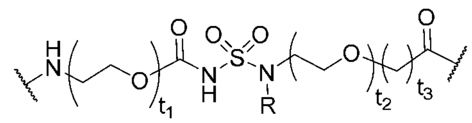,

wherein R is selected from hydrogen and $C_1$-$C_6$ alkyl, preferably hydrogen;

$R^{aa}$ and $R^{bb}$ are each independently selected from $C_1$-$C_6$ alkyl;

s is an integer from 1 to 6; preferably, an integer from 2 to 6; more preferably, an integer from 2 to 4;

$s_5$ and $s_6$ are each independently an integer from 1 to 6; preferably, an integer from 2 to 6; more preferably, an integer from 2 to 4;

t is an integer from 0 to 10; preferably, an integer from 2 to 8; more preferably, an integer from 3 to 7;

$t_1$ is an integer from 1 to 6; preferably, an integer from 1 to 4; more preferably, an integer from 2 to 4; more preferably 1 or 2;

$t_2$ is an integer from 0 to 6; preferably, an integer from 1 to 4; more preferably 1 or 2;

$t_3$ is an integer from 1 to 6; preferably, an integer from 1 to 4; more preferably 1 or 2;

$t_4$ is an integer from 0 to 10; and

$t_5$ is an integer from 0 to 10.

In another embodiment, $L_3$ is

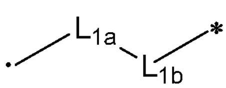, 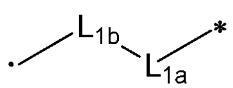, or 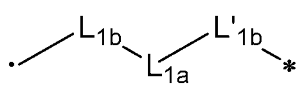,

* is a connection site with $L_2$, and ·is a connection site with carbonyl or methylene;

wherein $L_{1b}$ and $L'_{1b}$ are each independently an amino acid residue formed by one or more amino acids selected from phenylalanine, alanine, glycine, valine, leucine, isoleucine, tryptophan, tyrosine, histidine, lysine, citrulline, serine, threonine, cysteine, glutamic acid, glutamine, aspartic acid, asparagine, methionine, and arginine; and

$L_{1a}$ is a bond or selected from one or more of the following structures:

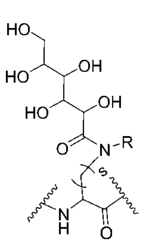,
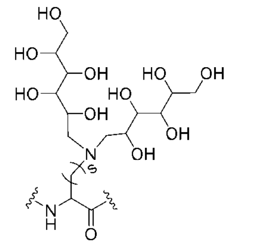,
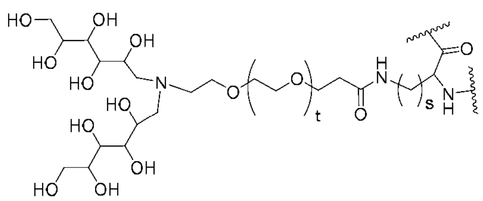,

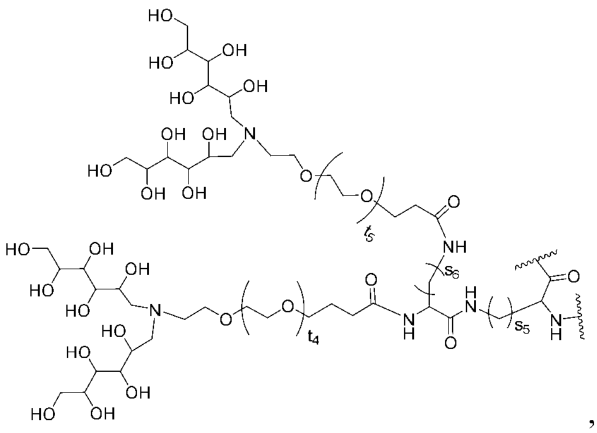,
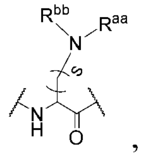, or
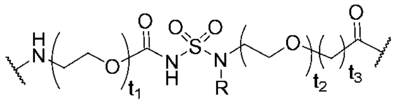;

wherein R, $R^{aa}$, $R^{bb}$, s, t, $t_1$ to $t_5$, $s_5$, and $s_6$ are the same as those described above.

In another preferred embodiment, in the compound represented by the general formula (A) or the pharmaceutically acceptable salt thereof according to the present invention, $L_3$ is selected from:

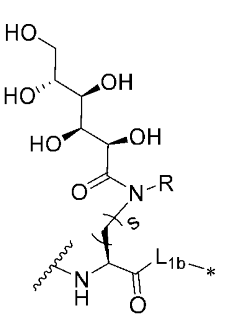,
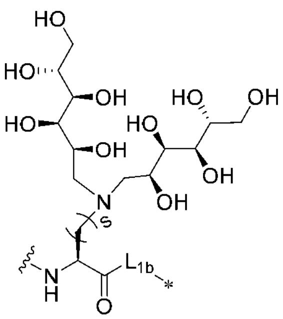,
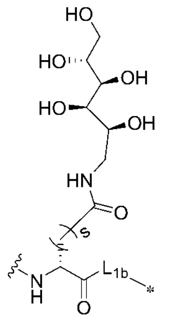,
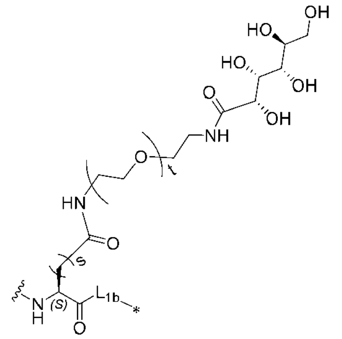,

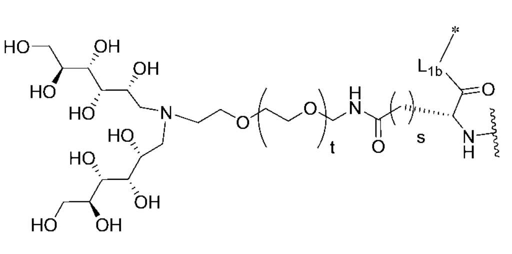,
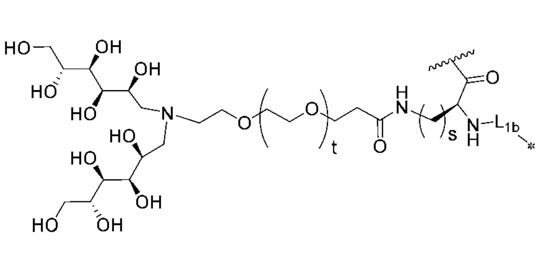,

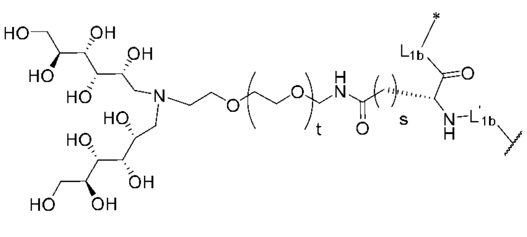,
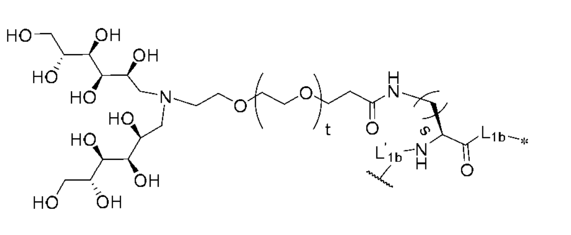,

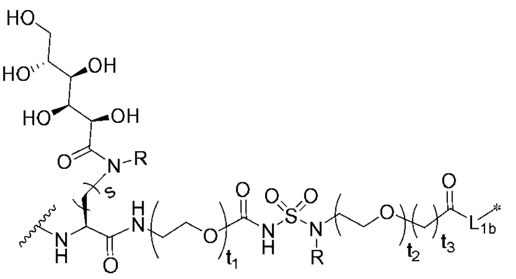,
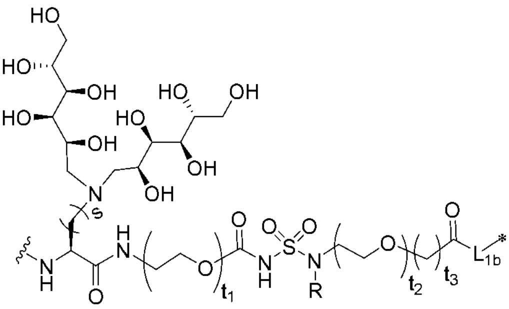,

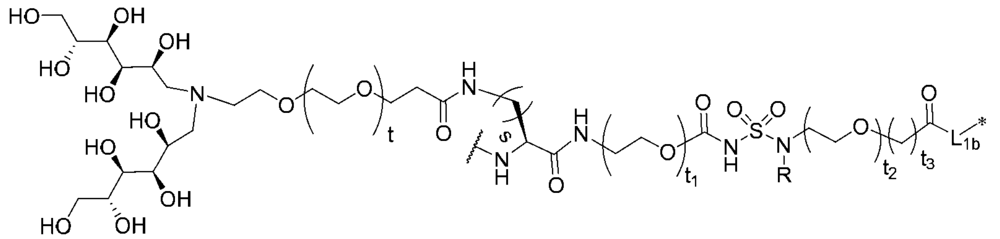,
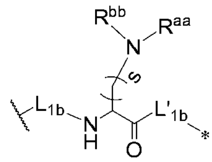,

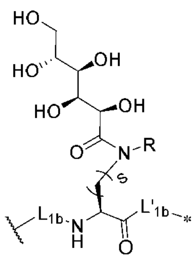, and
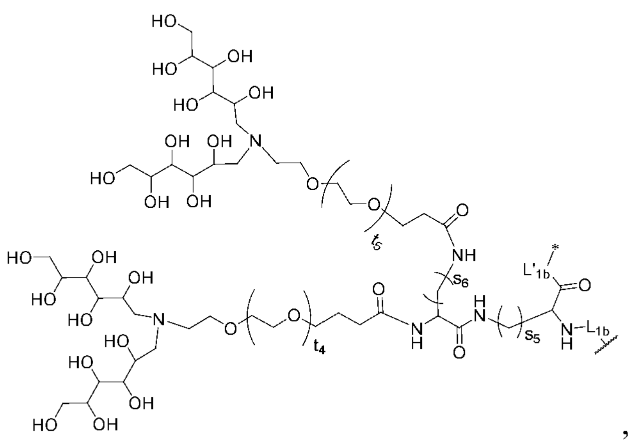,

wherein $L_{1b}$ and $L'_{1b}$ are each independently an amino acid residue formed by one or more amino acids selected from phenylalanine, alanine, glycine, valine, leucine, isoleucine, tryptophan, tyrosine, histidine, lysine, citrulline, serine, threonine, cysteine, glutamic acid, glutamine, aspartic acid, asparagine, methionine, and arginine;

R is selected from hydrogen and $C_1$-$C_6$ alkyl, and is preferably hydrogen;

$R^{aa}$ and $R^{bb}$ are each independently selected from $C_1$-$C_6$ alkyl;

s is an integer from 1 to 6; preferably, an integer from 2 to 6; more preferably, an integer from 2 to 4;

$s_5$ and $s_6$ are each independently an integer from 1 to 6; preferably, an integer from 2 to 6; more preferably, an integer from 2 to 4;

t is an integer from 0 to 10; preferably, an integer from 2 to 8; more preferably, an integer from 3 to 7;

$t_1$ is an integer from 1 to 6; preferably, an integer from 1 to 4; more preferably, an integer from 2 to 4; more preferably 1 or 2;

$t_2$ is an integer from 0 to 6; preferably, an integer from 1 to 4; more preferably 1 or 2;

$t_3$ is an integer from 1 to 6; preferably, an integer from 1 to 4; more preferably 1 or 2;

$t_4$ is an integer from 0 to 10;

$t_5$ is an integer from 0 to 10;

* is a connection site with $L_2$; and

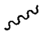 is a connection site with carbonyl or methylene.

In another preferred embodiment, in the compound represented by the general formula (A) or the pharmaceutically acceptable salt thereof according to the present invention, $L_3$ is selected from:

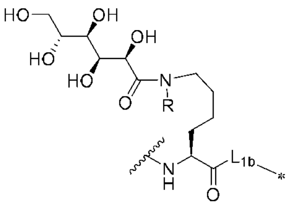 , 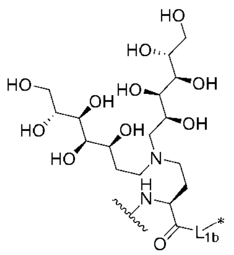 , 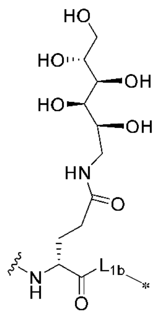 , 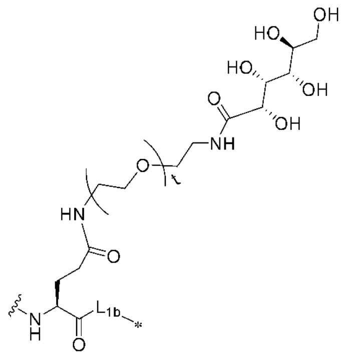 ,

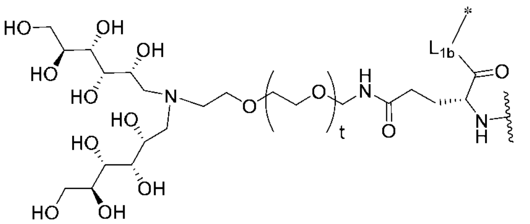 , 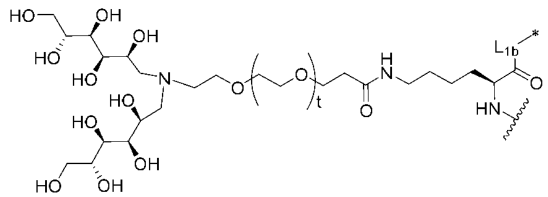 ,

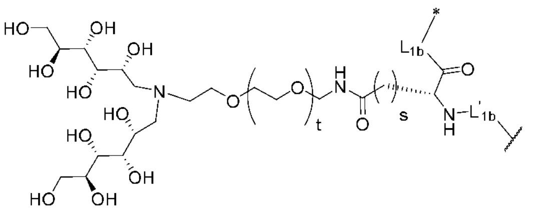 , 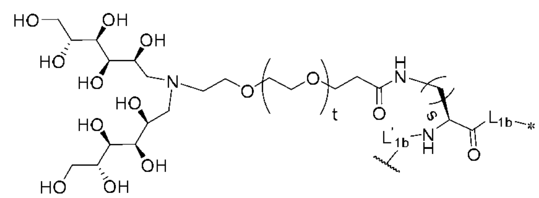 ,

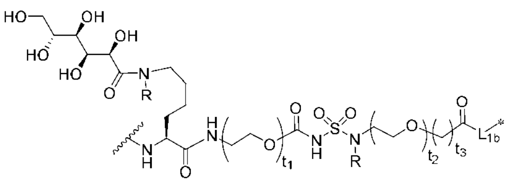 , 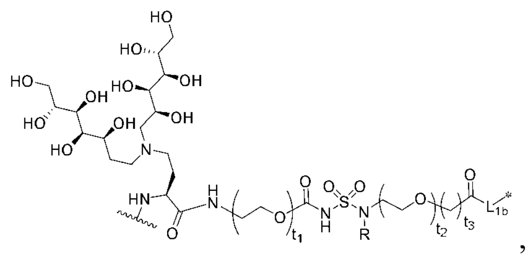 ,

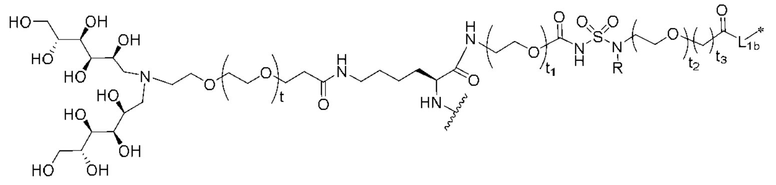

,

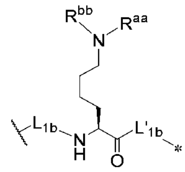

,

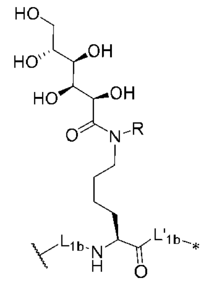

,

and

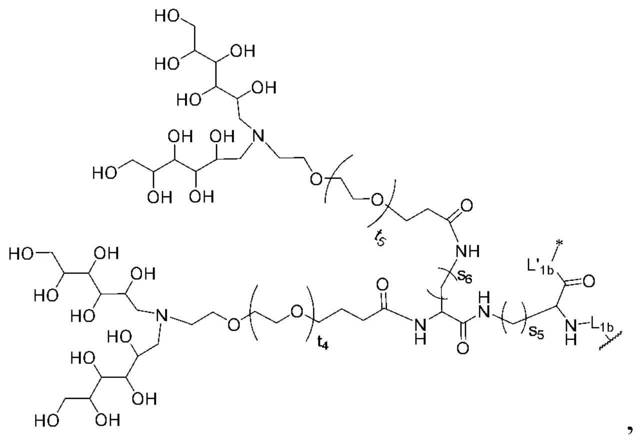

,

wherein $L_{1b}$ and $L'_{1b}$ are each independently an amino acid residue formed by one or more amino acids selected from phenylalanine, alanine, glycine, valine, leucine, isoleucine, tryptophan, tyrosine, histidine, lysine, citrulline, serine, threonine, cysteine, glutamic acid, glutamine, aspartic acid, asparagine, methionine, and arginine;

R is selected from hydrogen and $C_1$-$C_6$ alkyl, and is preferably hydrogen;

$R^{aa}$ and $R^{bb}$ are each independently selected from $C_1$-$C_6$ alkyl;

$s_5$ and $s_6$ are each independently an integer from 1 to 6; preferably, an integer from 2 to 6; more preferably, an integer from 2 to 4;

t is an integer from 0 to 10; preferably, an integer from 2 to 8; more preferably, an integer from 3 to 7;

$t_1$ is an integer from 1 to 6; preferably, an integer from 1 to 4; more preferably, an integer from 2 to 4; more preferably 1 or 2;

$t_2$ is an integer from 0 to 6; preferably, an integer from 1 to 4; more preferably 1 or 2;

$t_3$ is an integer from 1 to 6; preferably, an integer from 1 to 4; more preferably 1 or 2;

$t_4$ is an integer from 0 to 10;

$t_5$ is an integer from 0 to 10;

* is a connection site with $L_2$; and

∿∿ is a connection site with carbonyl or methylene.

In another preferred embodiment, in the compound represented by the general formula (A) or the pharmaceutically acceptable salt thereof according to the present invention, $L_{1b}$ and $L'_{1b}$ are each independently an amino acid

residue formed by one or more amino acids selected from glycine, phenylalanine, citrulline, leucine, isoleucine, alanine, valine, asparagine, glutamine, arginine, glutamic acid, and lysine; preferably an amino acid residue formed by two or more amino acids selected from glycine, phenylalanine, citrulline, valine, lysine, glutamine, glutamic acid, leucine, and alanine.

In another preferred embodiment, in the compound represented by the general formula (A) or the pharmaceutically acceptable salt thereof according to the present invention, $L_{1b}$ and $L'_{1b}$ are each independently selected from -Gly-*, -Val-*, -Gly-Phe-Gly-*, -Phe-Gly-*, -Gly-Val-Cit-*, -Val-Cit-*, -Gly-Val-Arg-*, -Val-Arg-*, -Gly-Val-Ala-*, -Val-Ala-*, -Gly-Phe-*, -Phe-Gly-*, -Gly-Gly-Gly-*, Gly-Gly-*, -Gly-Val-Gly-*, -Gly-Ala-Gly-*, -Gly-Phe-Cit-*, -Gly-Phe-Val-*, -Gly-Phe-Ala-*, -Gly-Phe-Lys-*, -Phe-Lys-*, -Gly-Val-*, -Gly-Cit-*, -Gly-Ala-*, -Gly-Gly-Lys-*, Gly-Lys'-*, -Ala-Ala-Ala-*, -Gln-Val-Ala-*, -Gln-Val-Cit-*, -Asp-Val-Ala-*, -Asp-Val-Cit-*, -Lys-Gly-Val-Ala-*, -Lys-Gly-Val-Cit-*, -Lys-Gly-Gly-Val-Ala-*, -Lys-Gly-Gly-Val-Cit-*, Gly-Gly-Phe-Gly-*, -Lys-Gln-Val-Cit-*, -Lys-Gln-Val-Ala-*, -Lys-Glu-Val-Cit-*, -Lys-Glu-Val-ALa-*, -Lys-Asp-Val-Cit-*, -Lys-Asp-Val-Ala-*, Glu-Val-Cit-*, Glu-Val-Ala-*, -Lys-Val-Ala-*, -Lys-Val-Cit-*, -Val-Lys-Gly-*, and -Val-Lys*; preferably, -Gly-Phe-Gly-*, -Gly-Val-Cit-*, -Val-Cit-*, -Gly-Val-Ala-*, -Val-Ala-*, -Gly-Phe-Gly-*, -Phe-Gly-*, -Gly-Phe-Lys-, -Phe-Lys- *, -Gln-Val-Ala-*, -Gln-Val-Cit-*, -Asp-Val-Ala-*, - Lys-Gly-Val-Ala-*, -Lys-Gly-Val-Cit-*, -Lys-Gly-Gly-Val-Ala-*, -Lys-Gly-Gly-Val-Cit-*, Gly-Gly-Phe-Gly-*, -Lys-Gln-Val-Cit-*, -Lys-Gln-Val-Ala-*, -Lys-Glu-Val-Cit-*, -Lys-Glu-Val-ALa-*, -Lys-Asp-Val-Cit-*, -Lys-Asp-Val-Ala-*, Glu-Val-Cit-*, Glu-Val-Ala-*, -Lys-Val-Ala-*, -Lys-Val-Cit-*, - Val-Lys-Gly-*, -Val-Lys-*, and -Asp-Val-Cit-*, wherein * is a connection site with $L_2$.

In another preferred embodiment, in the compound represented by the general formula (A) or the pharmaceutically acceptable salt thereof according to the present invention, $L_3$ is selected from:

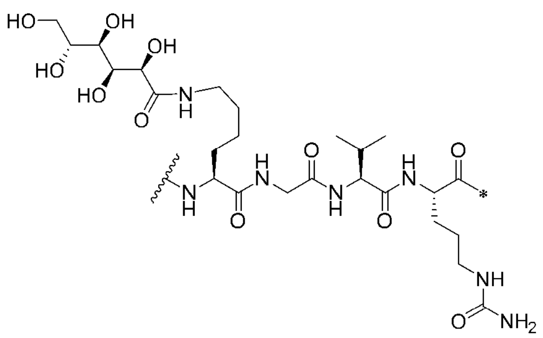
,
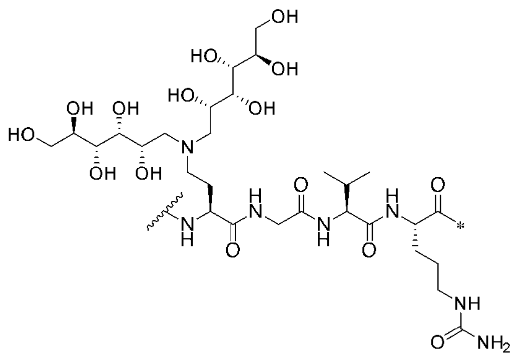
,
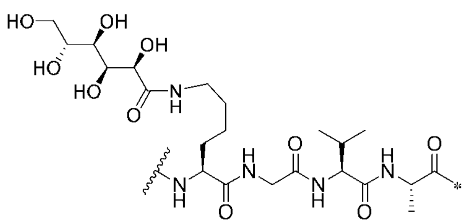
,
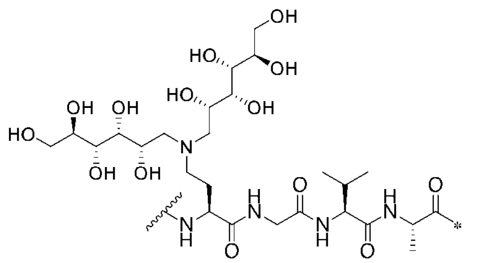
,
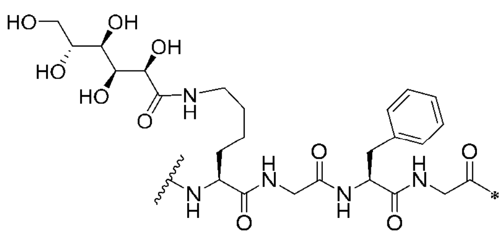
,
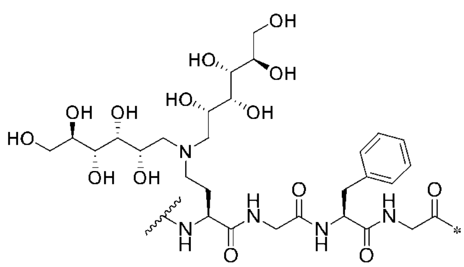
,

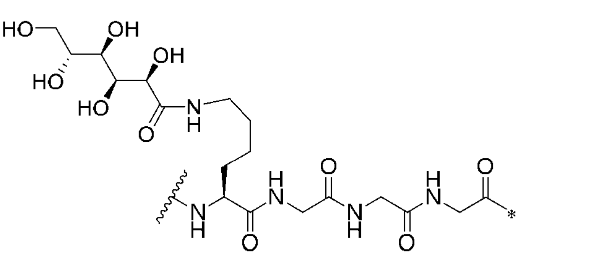
,

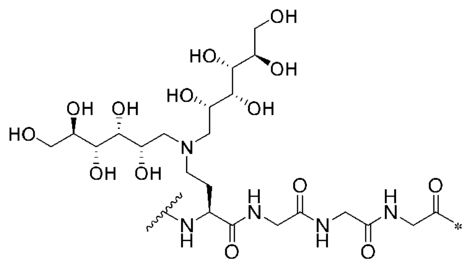
,

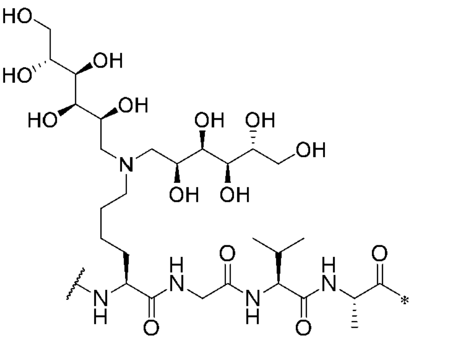
,

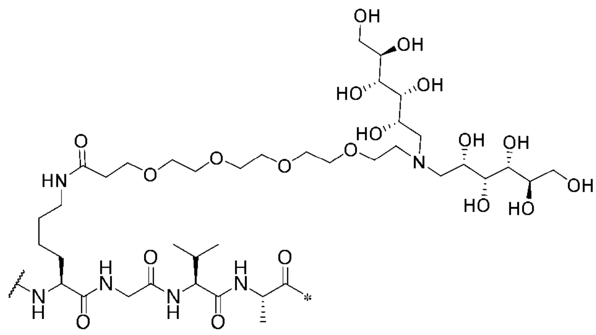
,

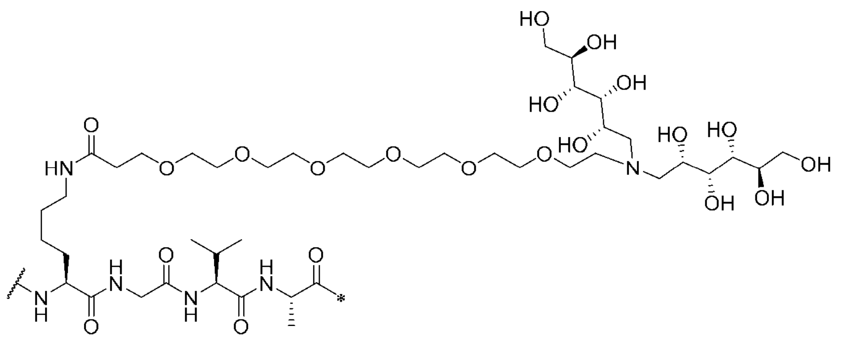
,

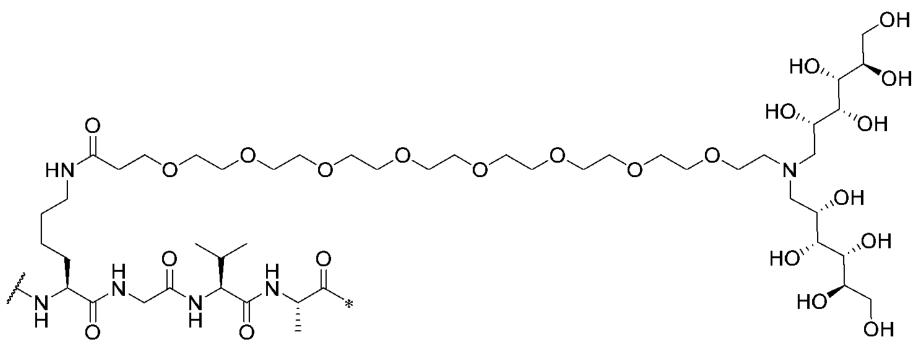
,

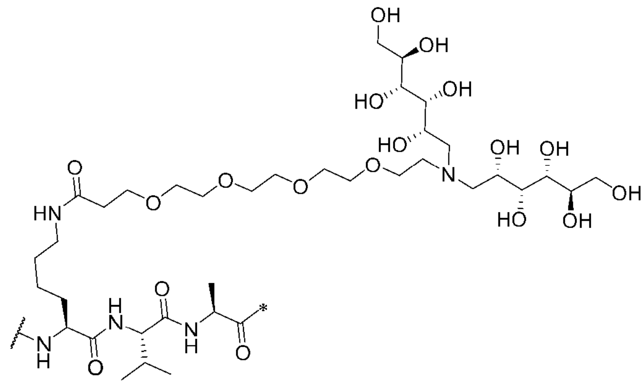

,

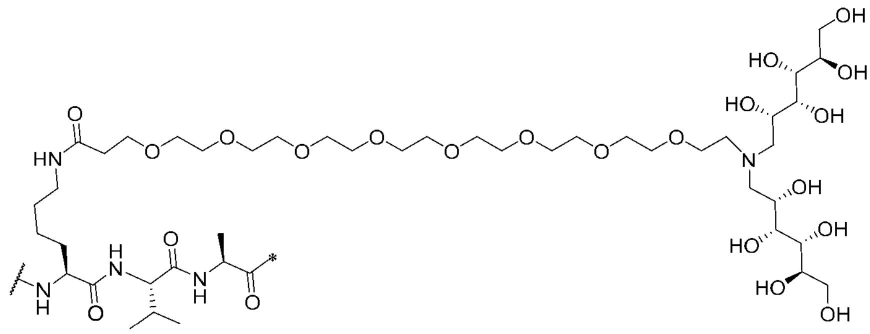

,

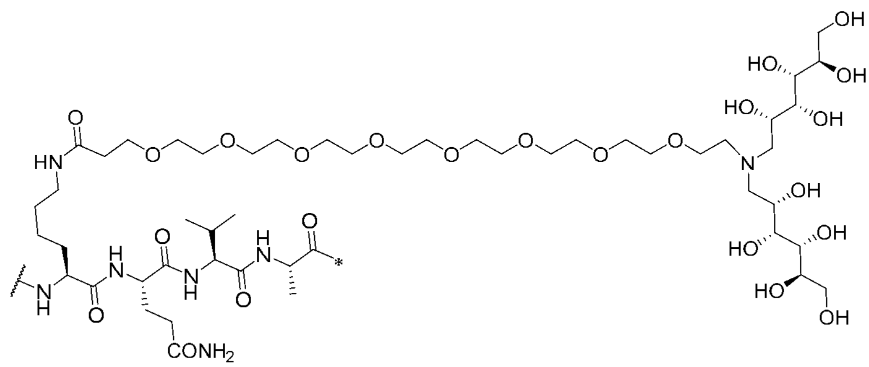

,

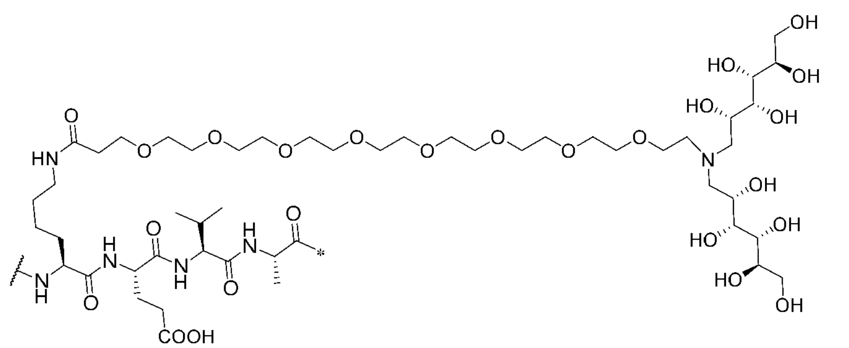

,

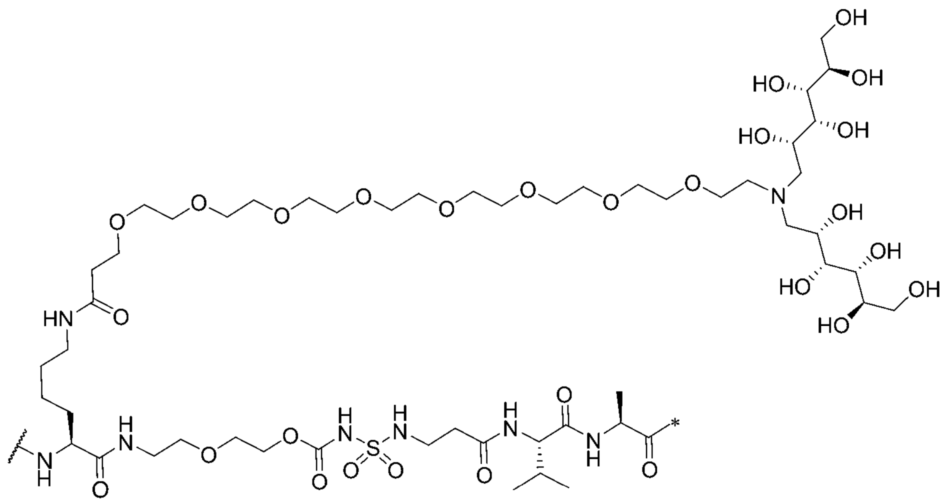

,

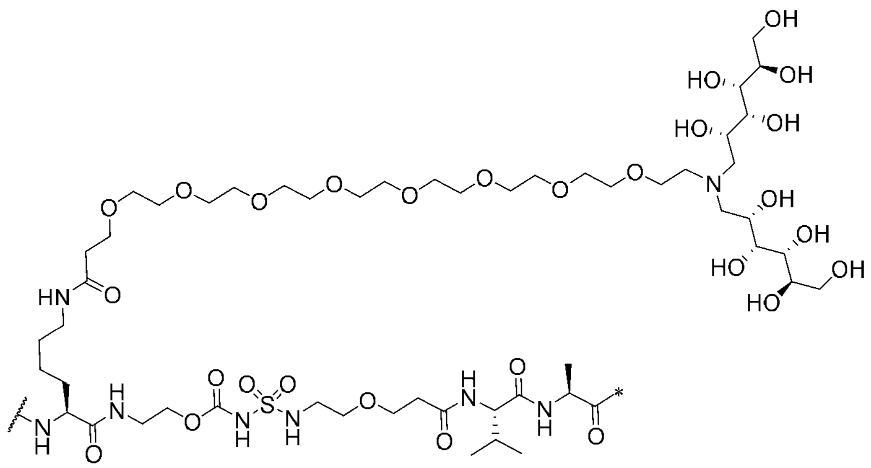

,

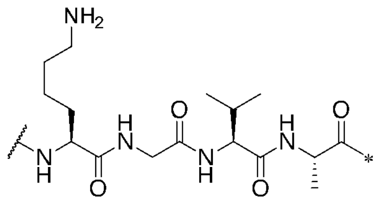 , 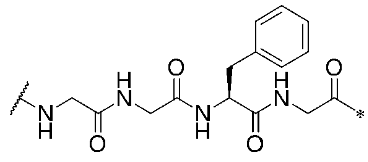 , 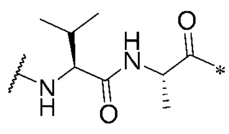 ,

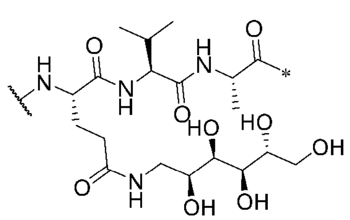 , 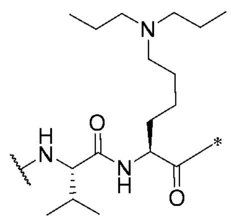 , 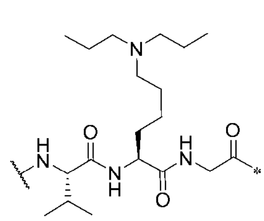 , 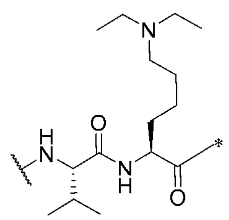 ,

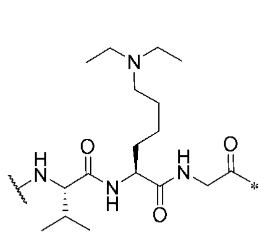 , 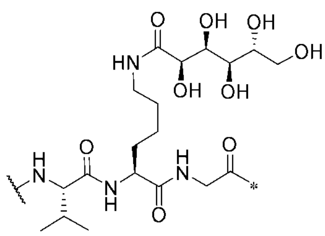 , 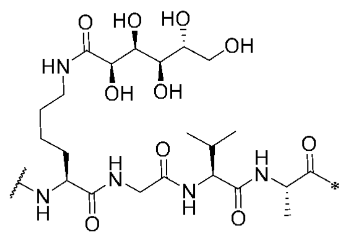 ,

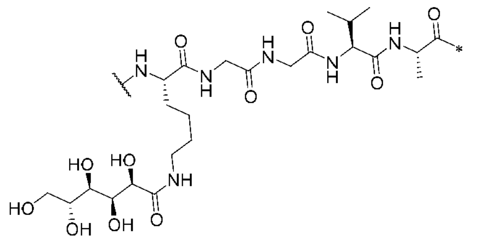

,

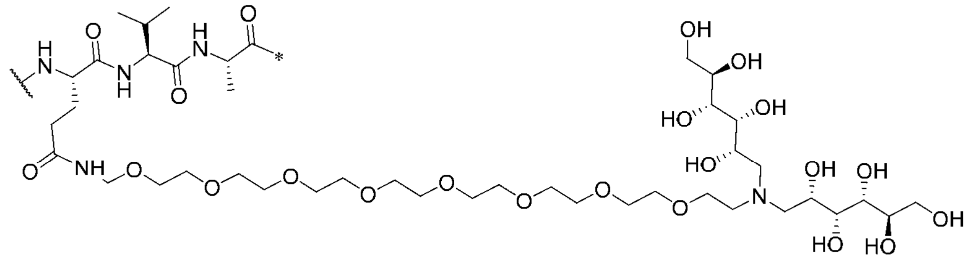

,

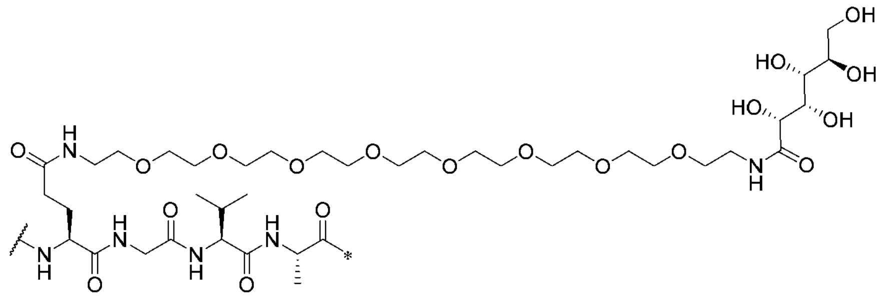

,

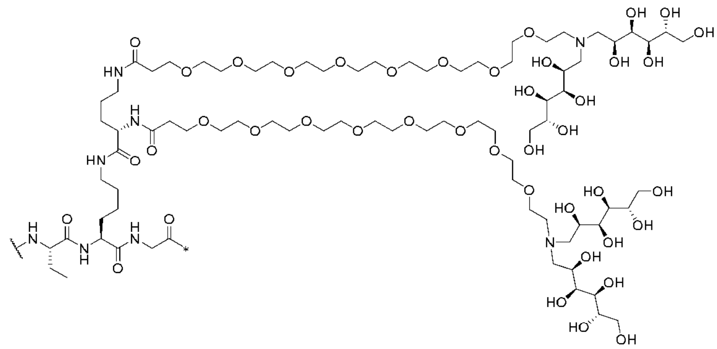

,

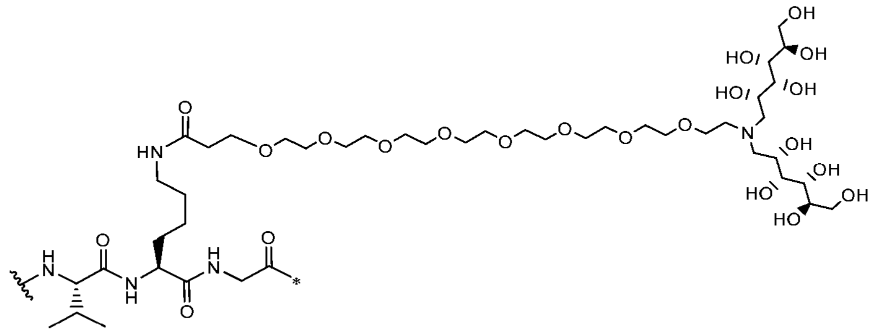

,

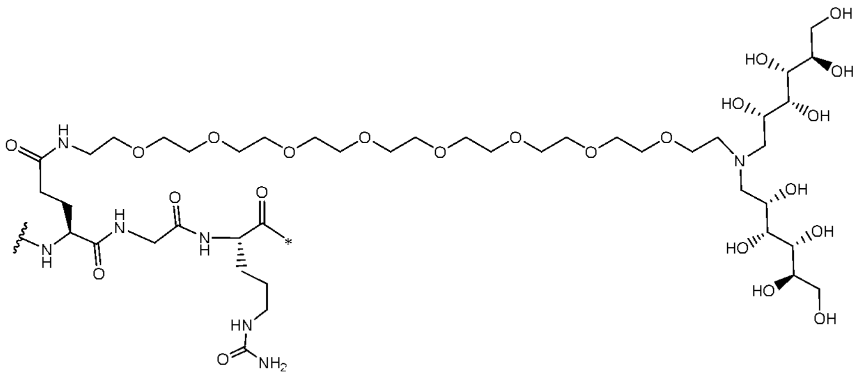

,

and

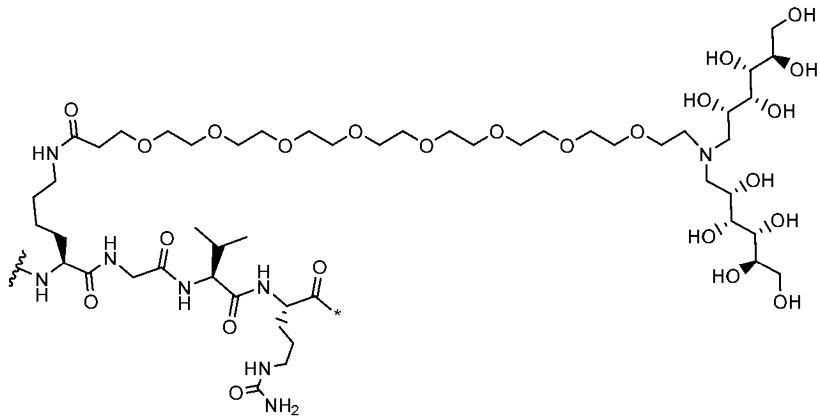

;

* is a connection site with $L_2$; and

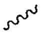 is a connection site with carbonyl or methylene.

In another preferred embodiment, in the compound represented by the general formula (A) or the pharmaceutically acceptable salt thereof according to the present invention, Q is selected from:

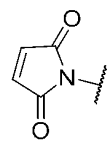, 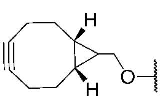, 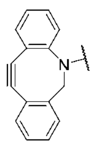, 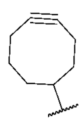, 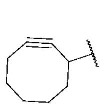, 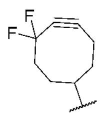, 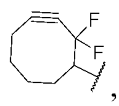, 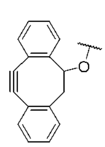, 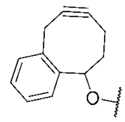, 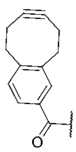,

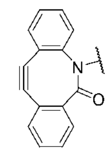, 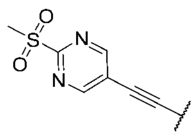, 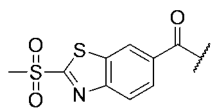, 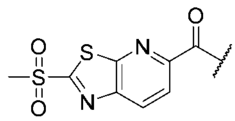, 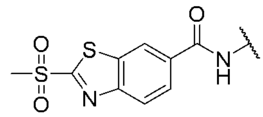,

and

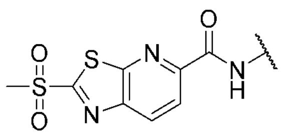

;

preferably,

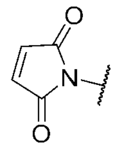, 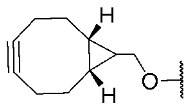, 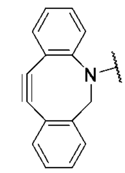, 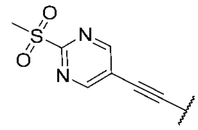, 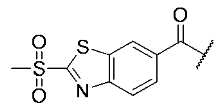,

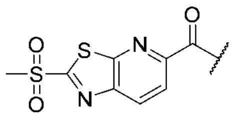, 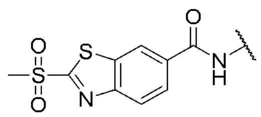, and 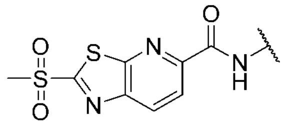.

In another preferred embodiment, in the compound represented by the general formula (A) or the pharmaceutically acceptable salt thereof according to the present invention, $Z_1$ is selected from a bond, $-(CH_2)_p-$, $-(CH_2)_p-C(O)NH-$, $-(CH_2)_p-O-(CH_2)_p-C(O)NH-$, $-(CH_2)_p-C(O)-L_6-NH-$, $-(CH_2)_p-O-(CH_2)_p-C(O)-L_6-NH-$, -C(O)NH-, -C(O)O-, -C(O)-, -OC(O)-, and -OC(O)NH-, wherein p is an integer from 1 to 10; preferably, an integer from 1 to 6, more preferably, an integer from 2 to 4, most preferably, an integer from 2 to 3;

$s_1$, $s_2$, $s_3$, and $s_4$ are each independently an integer from 0 to 10; preferably, an integer from 0 to 8, or an integer from 0 to 6; preferably, an integer from 0 to 4; more preferably, an integer from 0 to 2, or an integer from 1 to 2;
$L_6$ is selected from

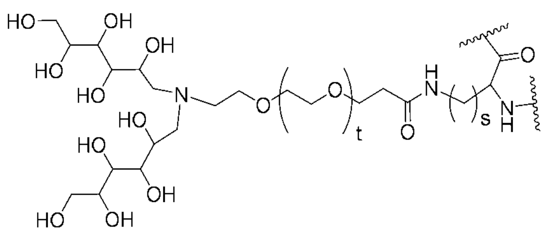

and

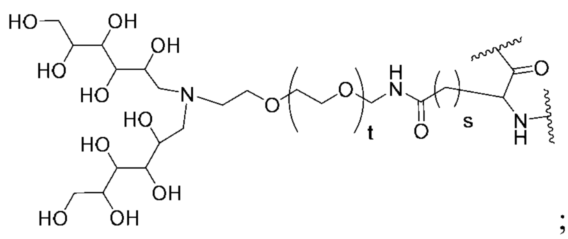;

s is an integer from 1 to 6; preferably, an integer from 2 to 6; more preferably, an integer from 2 to 4;
t is an integer from 0 to 10; preferably, an integer from 2 to 8; more preferably, an integer from 3 to 7; and
$L_6$ is preferably

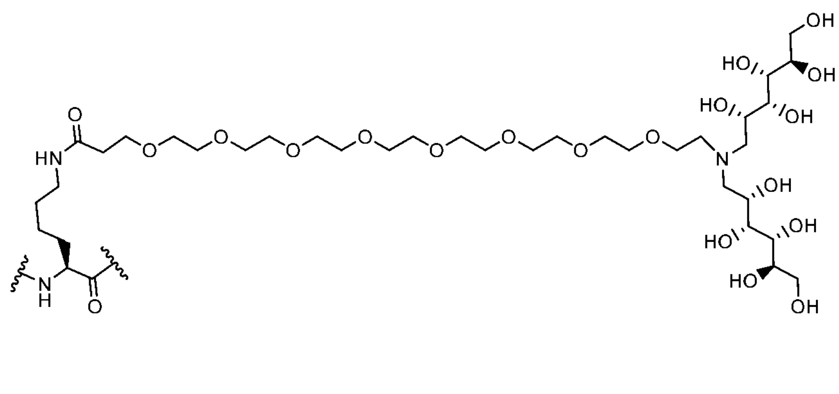.

In another preferred embodiment, in the compound represented by the general formula (A) or the pharmaceutically acceptable salt thereof according to the present invention,

$Z_1$ is selected from a bond, $-(CH_2)_p-$, $-(CH_2)_p-C(O)NH-$, $-(CH_2)_p-O-(CH_2)_p-C(O)NH-$, -C(O)NH-, - C(O)O-, -C(O)-, -OC(O)-, and -OC(O)NH-;
$s_1$ is an integer from 0 to 6; preferably, an integer from 0 to 2;
$s_2$ is an integer from 0 to 6; preferably, an integer from 0 to 2;
$s_3$ is 0;
$s_4$ is 0; and
p is an integer from 1 to 10; preferably, an integer from 1 to 6; more preferably, an integer from 2 to 4, most preferably, an integer from 2 to 3.

In another preferred embodiment, in the compound represented by the general formula (A) or the pharmaceutically acceptable salt thereof according to the present invention,

$Z_1$ is selected from $-(CH_2)_p-C(O)NH-$, $-(CH_2)_p-O-(CH_2)_p-C(O)NH-$, and -C(O)NH-;
$s_1$ is an integer from 1 to 6; preferably, an integer from 2 to 6;
$s_2$ is an integer from 1 to 10; preferably, an integer from 2 to 10;
$s_3$ is 0;
$s_4$ is 0; and
p is an integer from 1 to 10; preferably, an integer from 1 to 6; more preferably, an integer from 2 to 4, most preferably, an integer from 2 to 3.

In another preferred embodiment, in the compound represented by the general formula (A) or the pharmaceutically acceptable salt thereof according to the present invention, Q-$L_4$- is selected from:

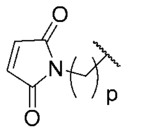, 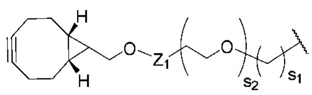, 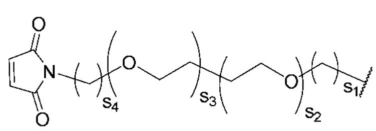, 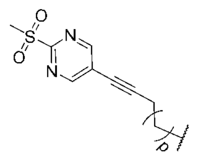,

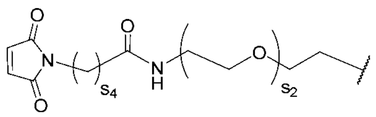, 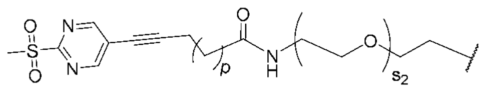,

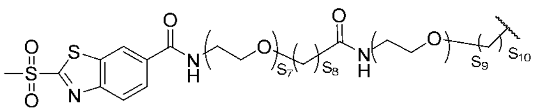, and 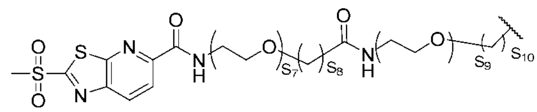;

wherein:

$Z_1$ is selected from -C(O)NH-, -C(O)O-, -C(O)-, -OC(O)-, and -OC(O)NH-, preferably -C(O)NH-;

p is an integer from 1 to 10; preferably, an integer from 1 to 6; more preferably, an integer from 2 to 4, most preferably, an integer from 2 to 3;

$s_1$ is an integer from 0 to 6; preferably, an integer from 0 to 2;

$s_2$ is an integer from 1 to 10; preferably, an integer from 1 to 8;

$s_3$ is an integer from 0 to 6; preferably, an integer from 0 to 2;

$s_4$ is an integer from 1 to 6; preferably, an integer from 1 to 2;

$s_7$ is an integer from 0 to 6; preferably, an integer from 1 to 2;

$s_8$ is an integer from 1 to 4; preferably, an integer from 1 to 2;

$s_9$ is an integer from 1 to 10; preferably, an integer from 1 to 8; and

$s_{10}$ is an integer from 1 to 4; preferably, an integer from 1 to 2.

In another preferred embodiment, in the compound represented by the general formula (A) or the pharmaceutically acceptable salt thereof according to the present invention, L is selected from:

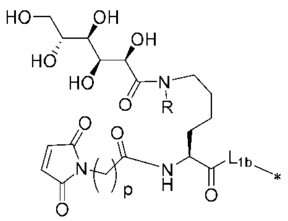
,
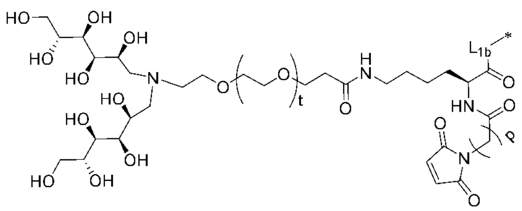
,
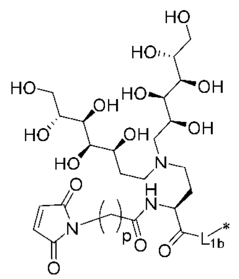
,

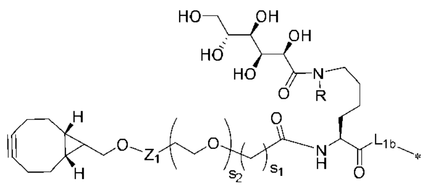
,
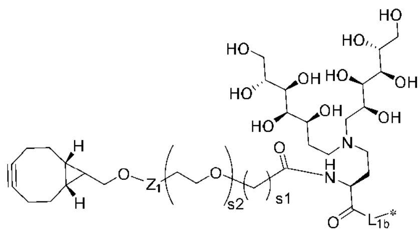
,

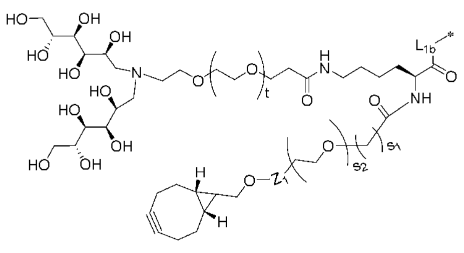
,
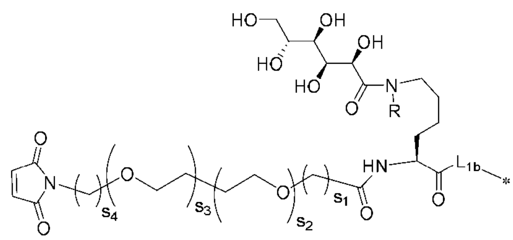
,

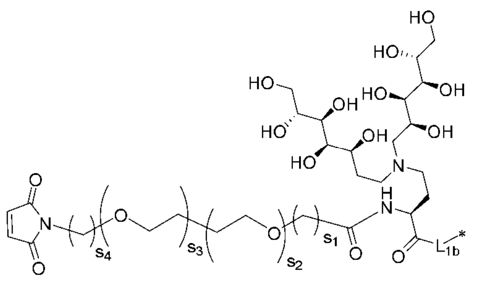
,
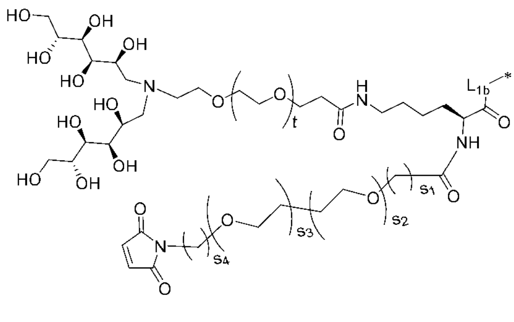
,

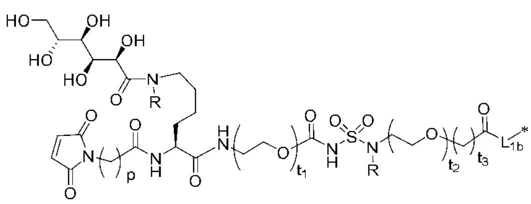
,

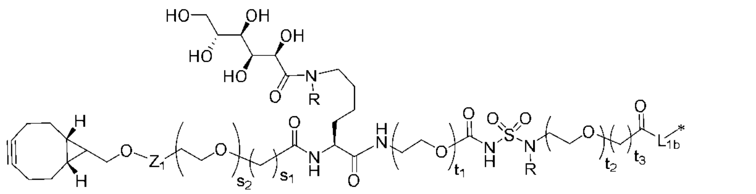

,

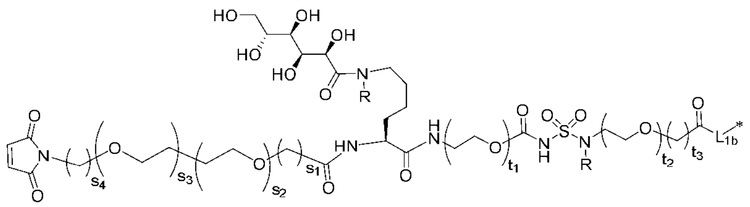

,

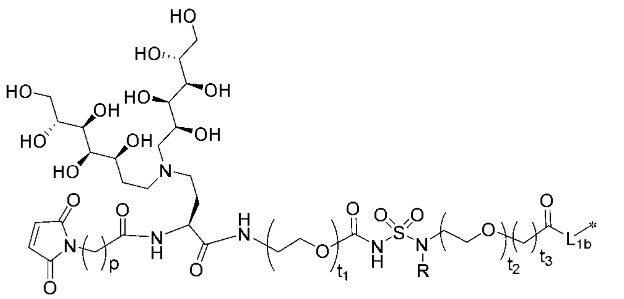

,

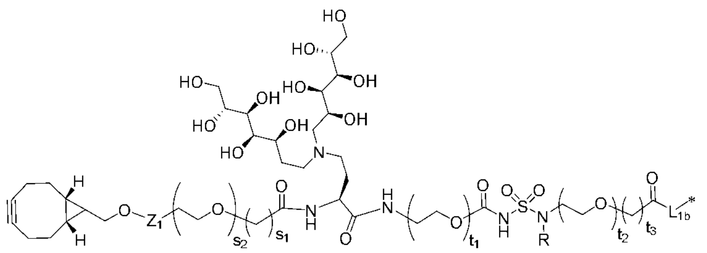

,

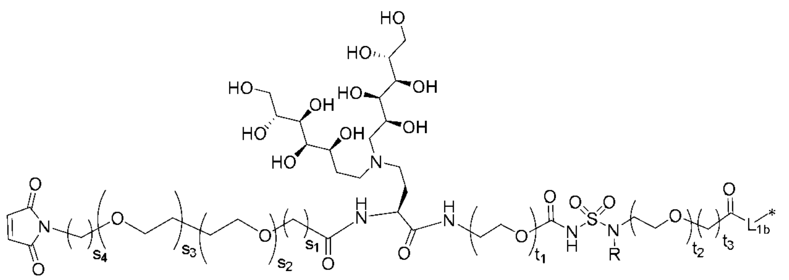

,

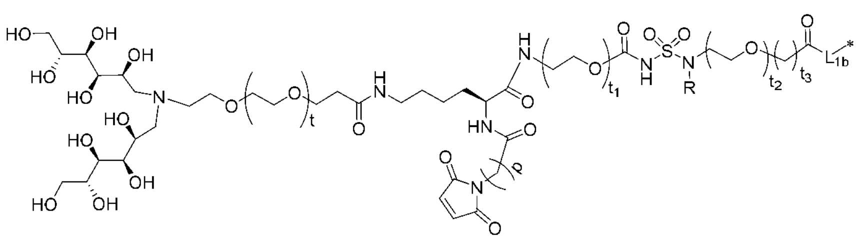

,

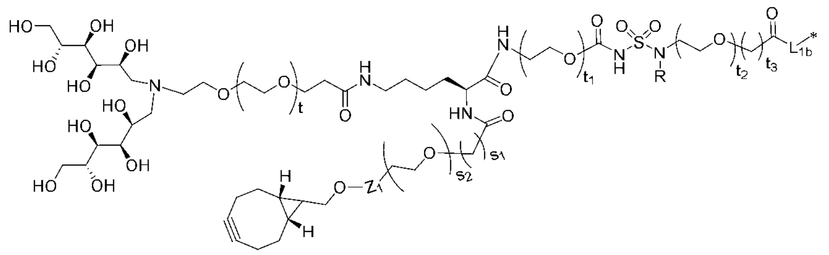

,

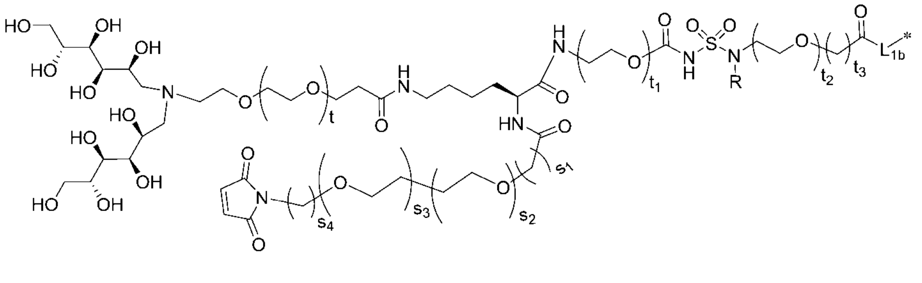

,

and

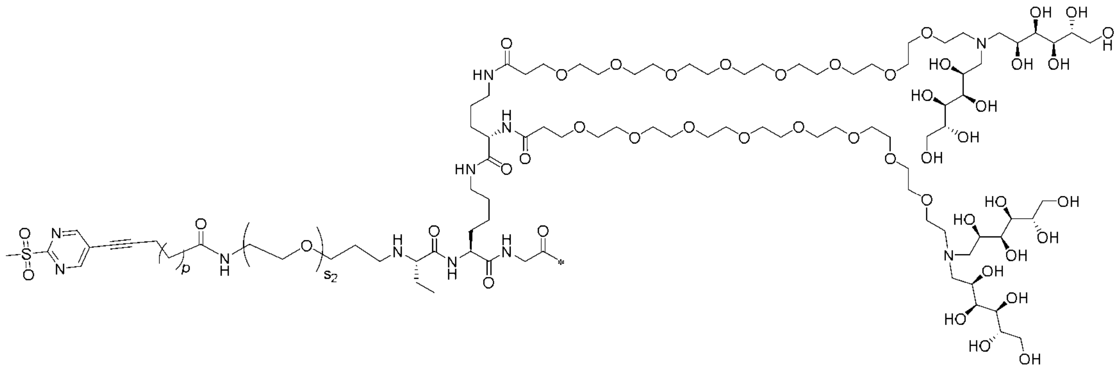

wherein,

$L_{1b}$ is an amino acid residue formed by two or more amino acids selected from glycine, phenylalanine, citrulline, leucine, isoleucine, alanine, valine, asparagine, glutamine, arginine, glutamic acid, and lysine, or is preferably an amino acid residue formed by two or more amino acids selected from glycine, phenylalanine, citrulline, leucine, alanine, valine, glutamine, glutamic acid, and lysine; more preferably selected from -Gly-*, -Val-*, -Gly-Phe-Gly-*, -Phe-Gly-*, -Gly-Val-Cit-*, -Val-Cit-*, - Gly-Val-Arg-*, -Val-Arg-*, -Gly-Val-Ala-*, -Val-Ala-*, -Gly-Phe-*, -Phe-Gly-*, -Gly-Gly-Gly-*, Gly-Gly-*, -Gly-Val-Gly-*, -Gly-Ala-Gly-*, -Gly-Phe-Cit-*, -Gly-Phe-Val-*, -Gly-Phe-Ala-*, -Gly-Phe-Lys-*, -Phe-Lys-*, -Gly-Val-*, -Gly-Cit-*, -Gly-Ala-*, -Gly-Gly-Lys-*, Gly-Lys'-*, -Ala-Ala-Ala-*, -Gln-Val-Ala-*, -Gln-Val-Cit-*, -Asp-Val-Ala-*, -Asp-Val-Cit-*, -Lys-Gly-Val-Ala-*, -Lys-Gly-Val-Cit-*, -Lys-Gly-Gly-Val-Ala-*, -Lys-Gly-Gly-Val-Cit-*, Gly-Gly-Phe-Gly-*, -Lys-Gln-Val-Cit-*, -Lys-Gln-Val-Ala-*, -Lys-Glu-Val-Cit-*, -Lys-Glu-Val-ALa-*, -Lys-Asp-Val-Cit-*, -Lys-Asp-Val-Ala-*, Glu-Val-Cit-*, Glu-Val-Ala-*, -Lys-Val-Ala-*, -Lys-Val-Cit-*, -Val-Lys-Gly-*, and -Val-Lys-*; preferably, -Gly-Phe-Gly-*, -Gly-Val-Cit-*, -Val-Cit-*, -Gly-Val-Ala-*, -Val-Ala-*, -Gly-Phe-Gly-*, -Phe-Gly-*, -Gly-Phe-Lys-, -Phe-Lys-*, -Gln-Val-Ala-*, -Gln-Val-Cit-*, -Asp-Val-Ala-*, - Lys-Gly-Val-Ala-*, -Lys-Gly-Val-Cit-*, -Lys-Gly-Gly-Val-Ala-*, -Lys-Gly-Gly-Val-Cit-*, Gly-Gly-Phe-Gly-*, -Lys-Gln-Val-Cit-*, -Lys-Gln-Val-Ala-*, -Lys-Glu-Val-Cit-*, -Lys-Glu-Val-ALa-*, -Lys-Asp-Val-Cit-*, -Lys-Asp-Val-Ala-*, Glu-Val-Cit-*, Glu-Val-Ala-*, -Lys-Val-Ala-*, -Lys-Val-Cit-*, - Val-Lys-Gly-*, -Val-Lys-*, and -Asp-Val-Cit-*,

* is a connection site with $L_2$;

$Z_1$ is selected from -C(O)NH-, -C(O)O-, -C(O)-, -OC(O)-, and -OC(O)NH-, preferably -C(O)NH-;

p is an integer from 1 to 10; preferably, an integer from 1 to 6;

$s_1$ is an integer from 0 to 6; preferably, an integer from 0 to 2;

$s_2$ is an integer from 1 to 6; preferably, an integer from 1 to 2;

$s_3$ is an integer from 0 to 6; preferably, an integer from 0 to 2;

$s_4$ is an integer from 1 to 6; preferably, an integer from 1 to 2;

t is an integer from 0 to 10;

$t_1$ is an integer from 1 to 6; preferably, an integer from 1 to 4; more preferably, an integer from 2 to 4; more preferably 1 or 2;

$t_2$ is an integer from 0 to 6; preferably, an integer from 1 to 4; more preferably 1 or 2; and

$t_3$ is an integer from 1 to 6; preferably, an integer from 1 to 4; more preferably 1 or 2.

In a preferred embodiment, in the compound represented by the general formula (A) or the pharmaceutically acceptable salt thereof according to the present invention, $L_{1b}$ is an amino acid residue formed by one or more amino acids selected from glycine, phenylalanine, citrulline, leucine, isoleucine, alanine, valine, asparagine, glutamine, arginine, glutamic acid, and lysine, or is preferably an amino acid residue formed by two or more amino acids selected from glycine, phenylalanine, citrulline, leucine, alanine, valine, glutamine, glutamic acid, and lysine; more preferably selected from -Gly-*, -Val-*, -Gly-Phe-Gly-*, -Phe-Gly-*, -Gly-Val-Cit-*, -Val-Cit-*, -Gly-Val-Arg-*, -Val-Arg-*, -Gly-Val-Ala-*, -Val-Ala-*, -Gly-Phe-*, -Phe-Gly-*, -Gly-Gly-Gly-*, Gly-Gly-*, -Gly-Val-Gly-*, -Gly-Ala-Gly-*, -Gly-Phe-Cit-*, -Gly-Phe-Val-*, -Gly-Phe-Ala-*, -Gly-Phe-Lys-*, -Phe-Lys- *, -Gly-Val-*, -Gly-Cit-*, -Gly-Ala-*, -Gly-Gly-Lys-*, Gly-Lys'-*, -Ala-Ala-Ala-*, -Gln-Val-Ala-*, -Gln-Val-Cit-*, -Asp-Val-Ala-*, -Asp-Val-Cit-*, -Lys-Gly-Val-Ala-*, -Lys-Gly-Val-Cit-*, -Lys-Gly-Gly-Val-Ala-*, -Lys-Gly-Gly-Val-Cit-*, Gly-Gly-Phe-Gly-*, -Lys-Gln-Val-Cit-*, -Lys-Gln-Val-Ala- *, -Lys-Glu-Val-Cit-*, -Lys-Glu-Val-ALa-*, -Lys-Asp-Val-Cit-*, -Lys-Asp-Val-Ala-*, Glu-Val-Cit-*, Glu-Val-Ala-*, -Lys-Val-Ala-*, -Lys-Val-Cit-*, -Val-Lys-Gly-*, and -Val-Lys-*; preferably, -Gly-Phe-Gly-*, -Gly-Val-Cit-*, -Val-Cit-*, -Gly-Val-Ala-*, -Val-Ala-*, -Gly-Phe-Gly-*, -Phe-Gly-*, - Gly-Phe-Lys-, -Phe-Lys-*, -Gln-Val-Ala-*, -Gln-Val-Cit-*, -Asp-Val-Ala-*, -Lys-Gly-Val-Ala-*, - Lys-Gly-Val-Cit-*, -Lys-Gly-Gly-Val-Ala-*, -Lys-Gly-Gly-Val-Cit-*, Gly-Gly-Phe-Gly-*, -Lys-Gln-Val-Cit-*, -Lys-Gln-Val-Ala-*, -Lys-Glu-Val-Cit-*, -Lys-Glu-Val-ALa-*, -Lys-Asp-Val-Cit-*, -Lys-Asp-Val-Ala-*, Glu-Val-Cit-*, Glu-Val-Ala-*, -Lys-Val-Ala-*, -Lys-Val-Cit-*, -Val-Lys-Gly-*, -Val-Lys-*, and -Asp-Val-Cit-*.

In another preferred embodiment, in the compound represented by the general formula (A) or the pharmaceutically acceptable salt thereof according to the present invention, $L'_{1b}$ is an amino acid residue formed by one or more amino acids selected from glycine, phenylalanine, citrulline, leucine, isoleucine, alanine, valine, asparagine, glutamine, arginine, glutamic acid, and lysine, or is preferably an amino acid residue formed by two or more amino acids selected from glycine, phenylalanine, citrulline, leucine, aspartic acid, alanine, valine, glutamine, glutamic acid, and lysine; more preferably selected from -Gly-*, -Val-*, -Gly-Phe-Gly-*, -Phe-Gly-*, -Gly-Val-Cit-*, -Val-Cit-*, -Gly-Val-Arg- *, -Val-Arg-*, -Gly-Val-Ala-*, -Val-Ala-*, -Gly-Phe-*, -Phe-Gly-*, -Gly-Gly-Gly-*, Gly-Gly-*, - Gly-Val-Gly-*, -Gly-Ala-Gly-*, -Gly-Phe-Cit-*, -Gly-Phe-Val-*, -Gly-Phe-Ala-*, -Gly-Phe-Lys-*, - Phe-Lys-*, -Gly-Val-*, -Gly-Cit-*, -Gly-Ala-*, -Gly-Gly-Lys-*, Gly-Lys'-*, -Ala-Ala-Ala-*, -Gln-Val-Ala-*, -Gln-Val-Cit-*, -Asp-Val-Ala-*, -Asp-Val-Cit-*, -Lys-Gly-Val-Ala-*, -Lys-Gly-Val-Cit-*, -Lys-Gly-Gly-Val-Ala-*, -Lys-Gly-Gly-Val-Cit-*, Gly-Gly-Phe-Gly-*, -Lys-Gln-Val-Cit-*, -Lys-Gln-Val-Ala-*, -Lys-Glu-Val-Cit-*, -Lys-Glu-Val-ALa-*, -Lys-Asp-Val-Cit-*, -Lys-Asp-Val-Ala-*, Glu-Val-Cit-*, Glu-Val-Ala-*, -Lys-Val-Ala-*, -Lys-Val-Cit-*, -Val-Lys-Gly-*, and -Val-Lys-*; preferably, -Gly-Phe-Gly-*, -Gly-Val-Cit-*, -Val-Cit-*, -Gly-Val-Ala-*, -Val-Ala-*, -Gly-Phe-Gly-*, -Phe-Gly-*, -Gly-Phe-Lys-, -Phe-Lys-*, -Gln-Val-Ala-*, -Gln-Val-Cit-*, -Asp-Val-Ala-*, -Lys-Gly-Val-Ala-*, -Lys-Gly-Val-Cit-*, -Lys-Gly-Gly-Val-Ala-*, -Lys-Gly-Gly-Val-Cit-*, Gly-Gly-Phe-Gly- *, -Lys-Gln-Val-Cit-*, -Lys-Gln-Val-Ala-*, -Lys-Glu-Val-Cit-*, -Lys-Glu-Val-ALa-*, -Lys-Asp-Val-Cit-*, -Lys-Asp-Val-Ala-*, Glu-Val-Cit-*, Glu-Val-Ala-*, -Lys-Val-Ala-*, -Lys-Val-Cit-*, -Val-Lys-Gly-*, -Val-Lys-*, and -Asp-Val-Cit-*.

In another preferred embodiment, in the compound represented by the general formula (A) or the pharmaceutically acceptable salt thereof according to the present invention, $L_{1b}$ and $L'_{1b}$ are -Gly-* or -Val-*.

In another preferred embodiment, in the compound represented by the general formula (A) or the pharmaceutically acceptable salt thereof according to the present invention, the compound is selected from:

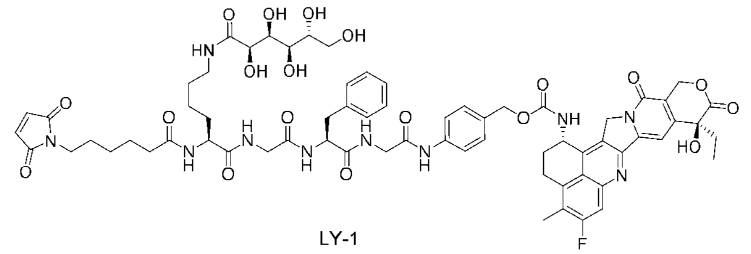

LY-1

,

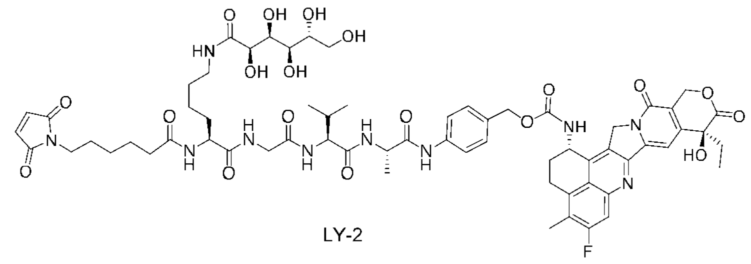

LY-2

,

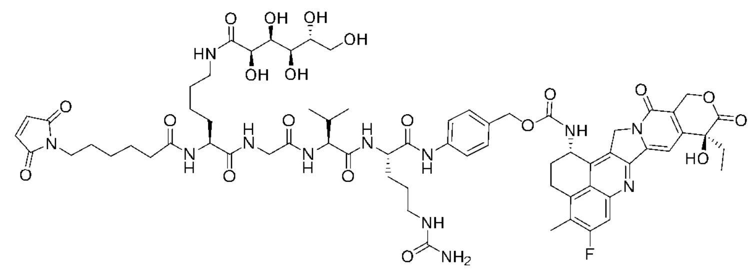

LY-3

,

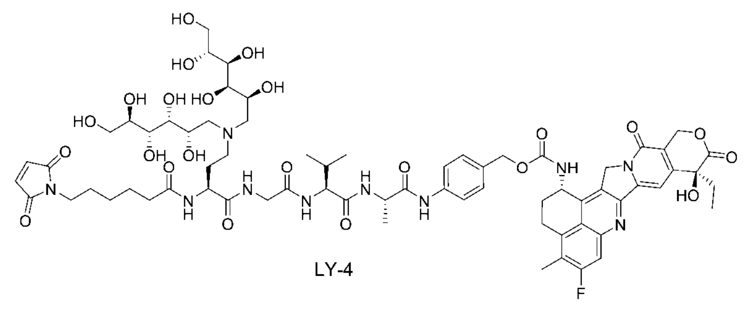

LY-4

,

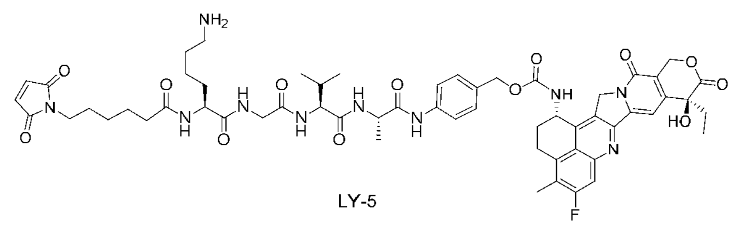

LY-5

,

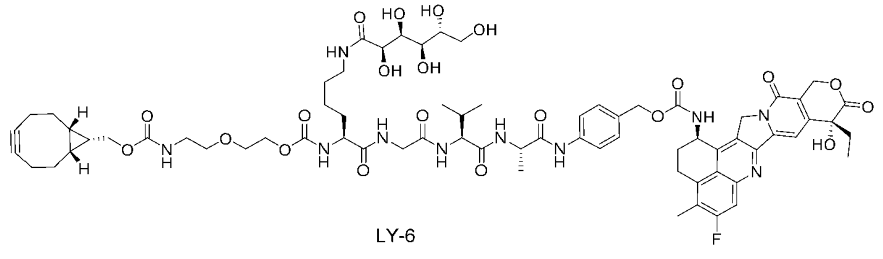

LY-6

,

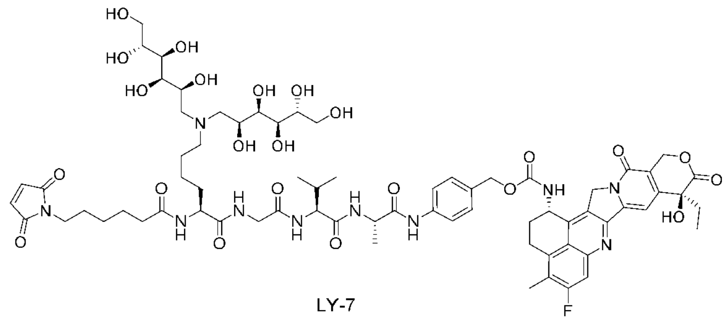

LY-7

,

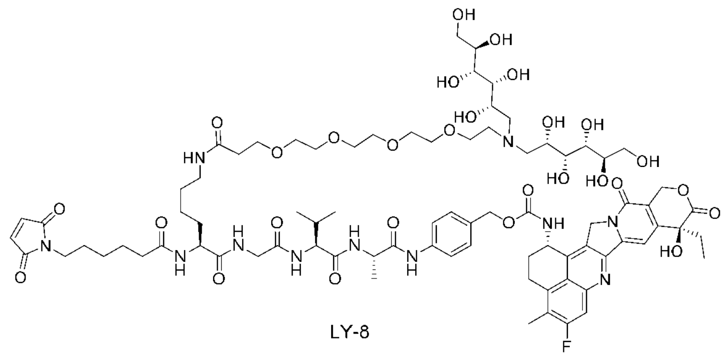

LY-8

,

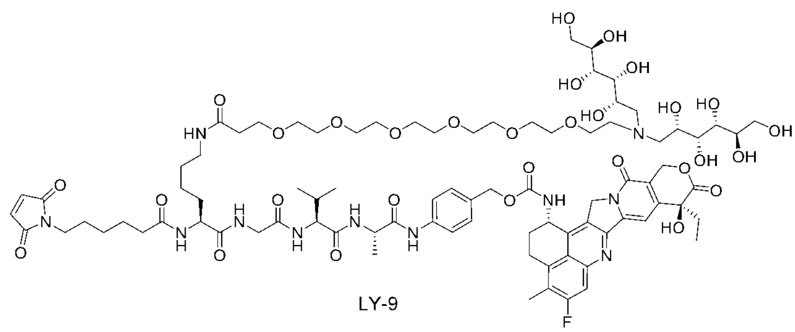

LY-9

,

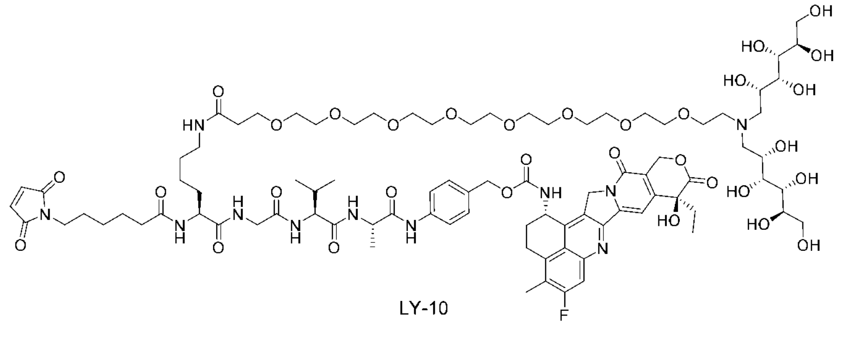

LY-10

,

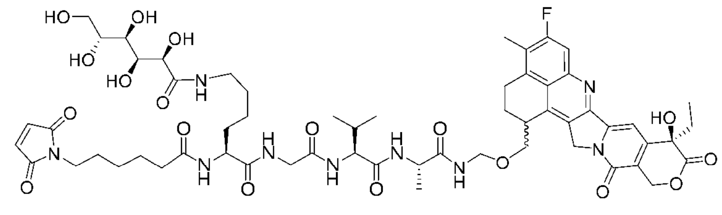

LY-11

,

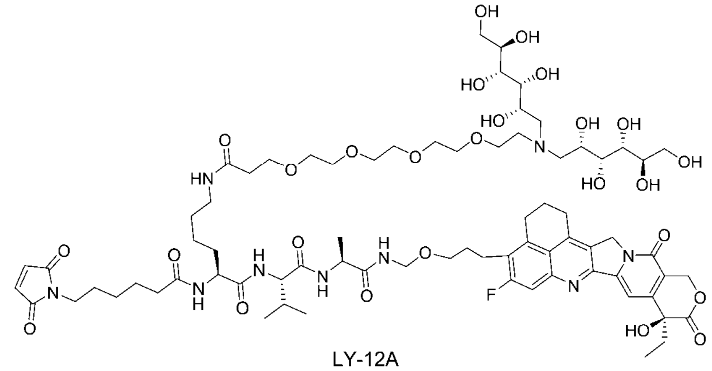

LY-12A

,

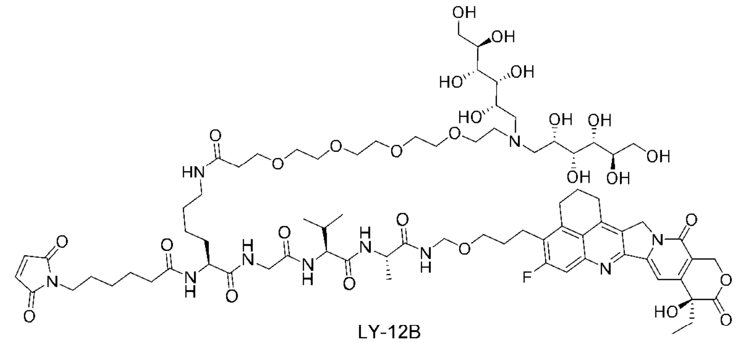

LY-12B

,

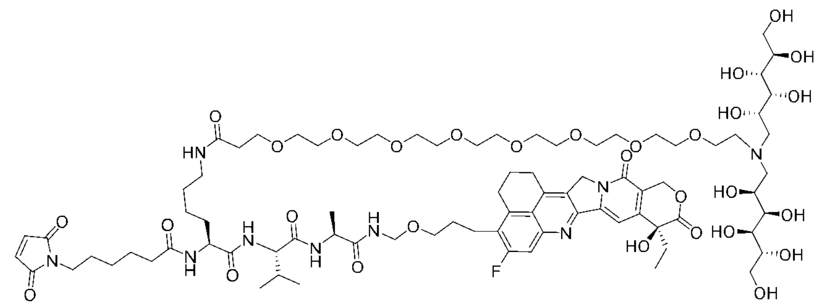

LY-18A

,

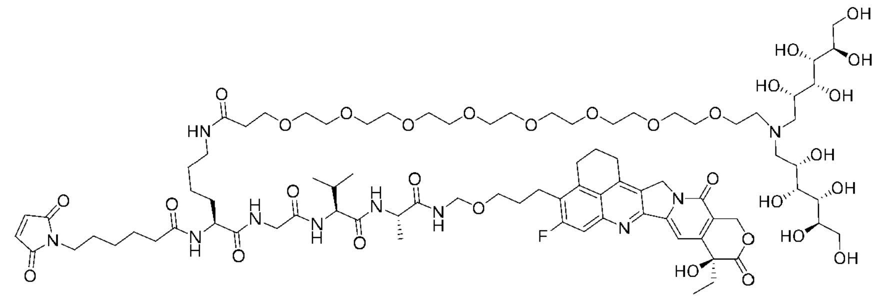

LY-18B

,

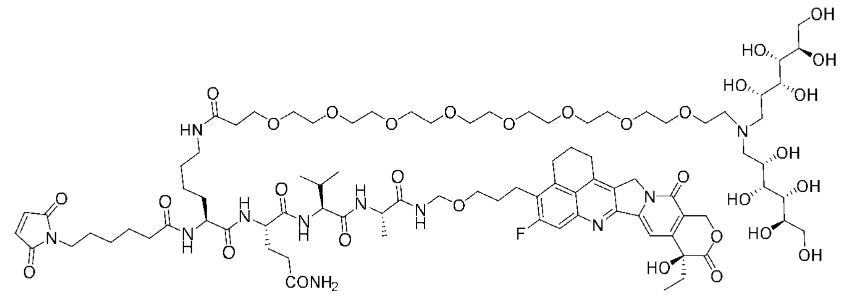

LY-18C

,

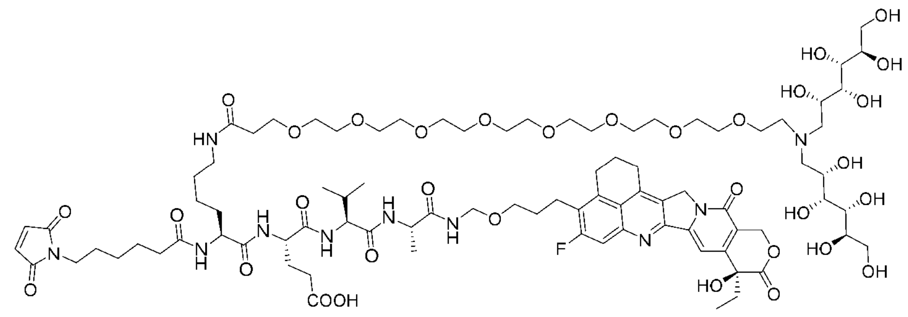

LY-18D

,

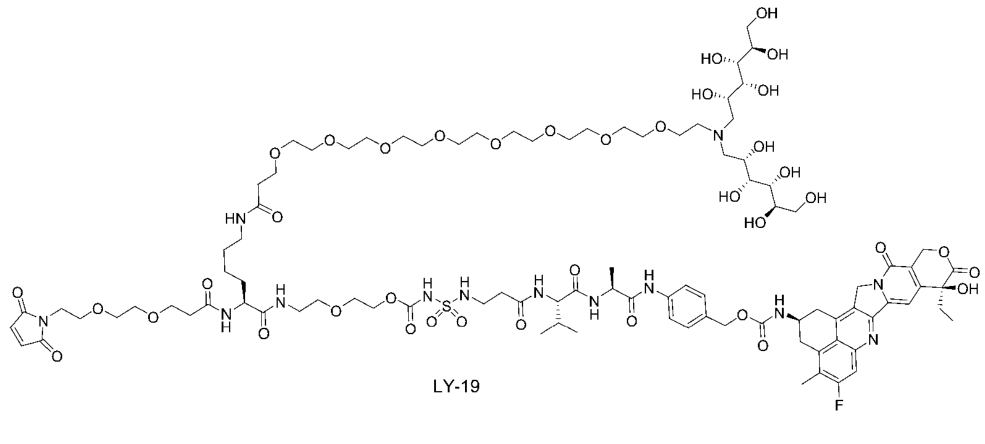

LY-19

,

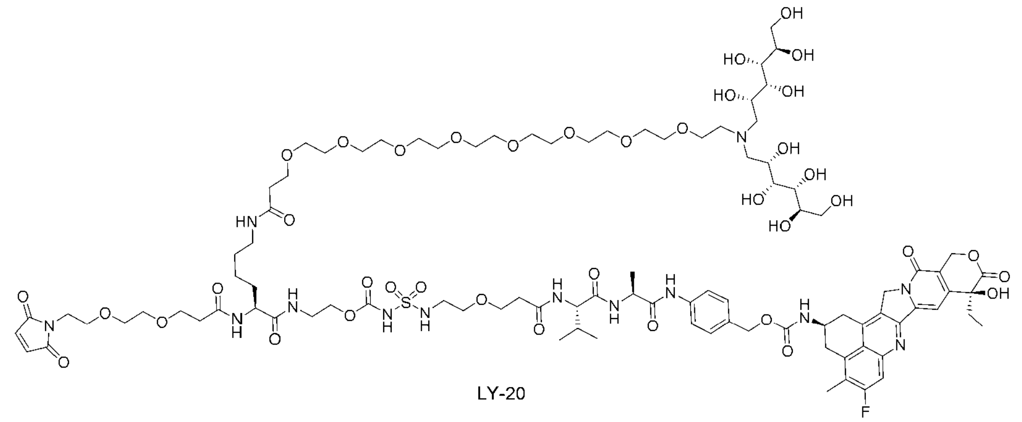

LY-20

,

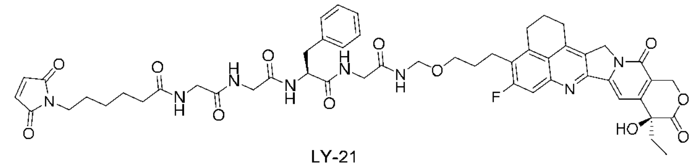

LY-21

,

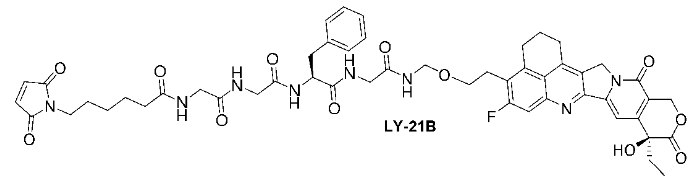

LY-21B

,

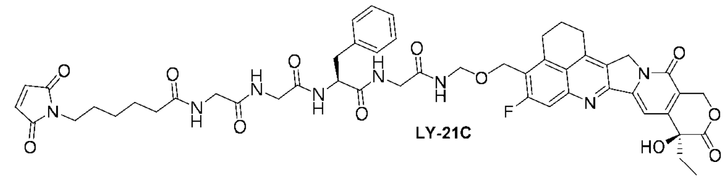

LY-21C

,

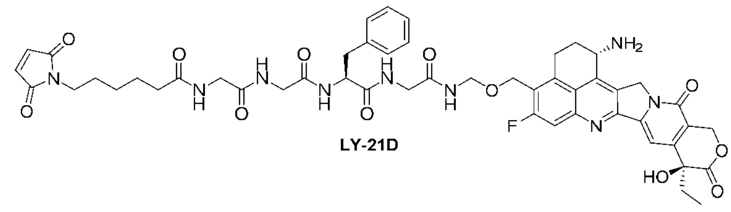

LY-21D

,

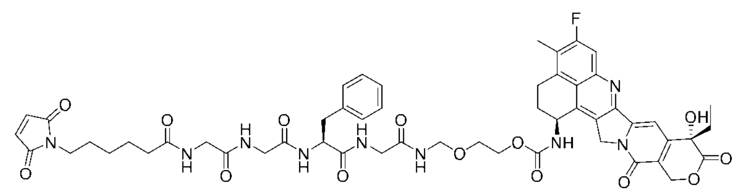

LY-22

,

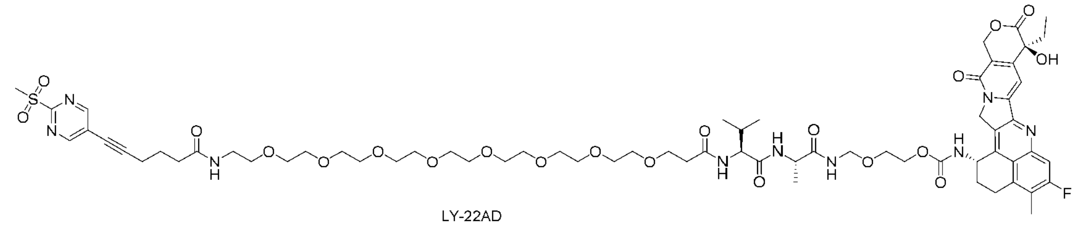

LY-22AD

,

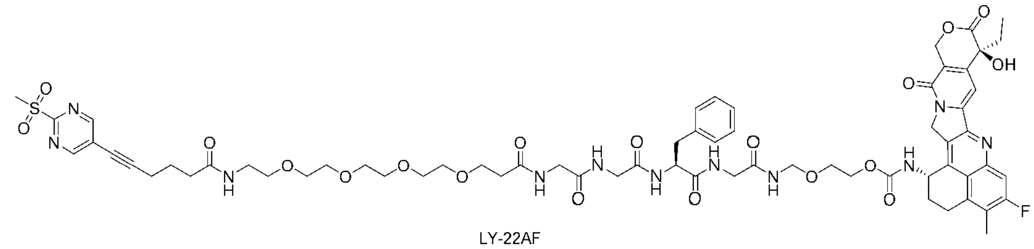

LY-22AF

,

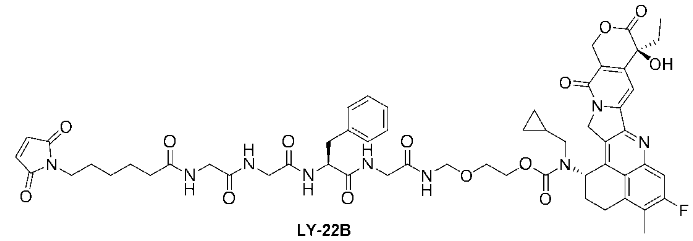

**LY-22B**

,

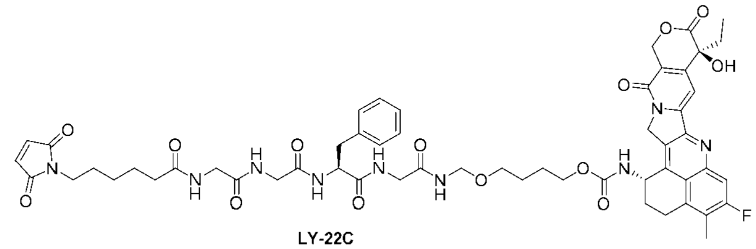

**LY-22C**

,

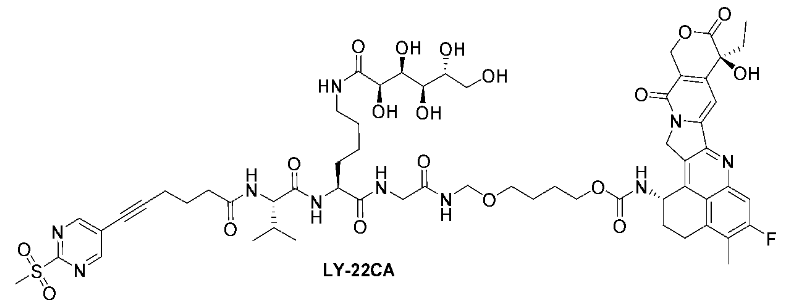

**LY-22CA**

,

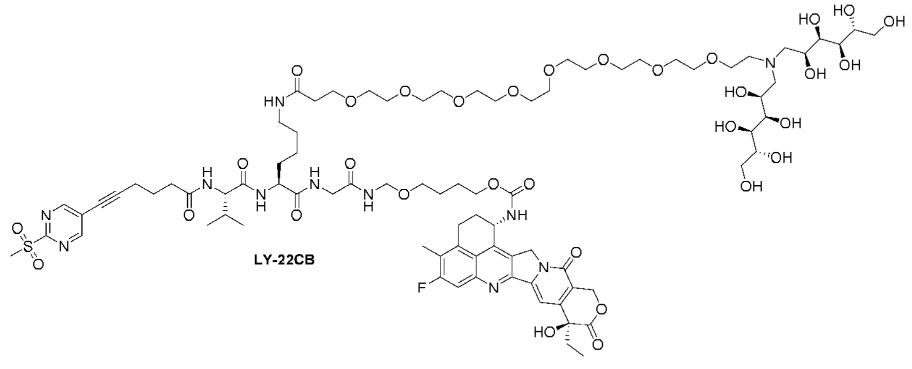

LY-22CB

,

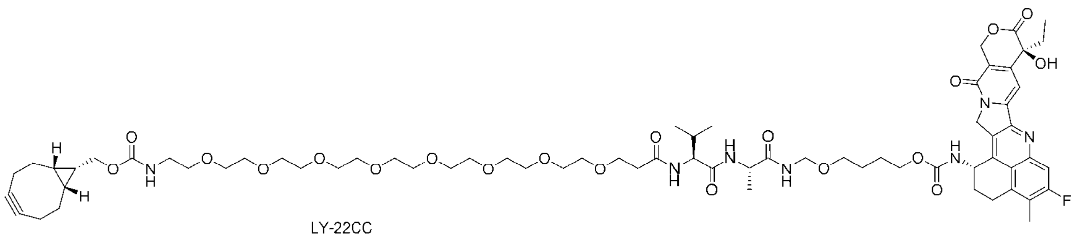

LY-22CC

,

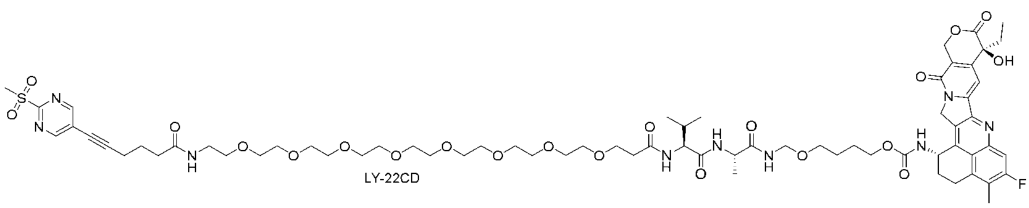

LY-22CD

,

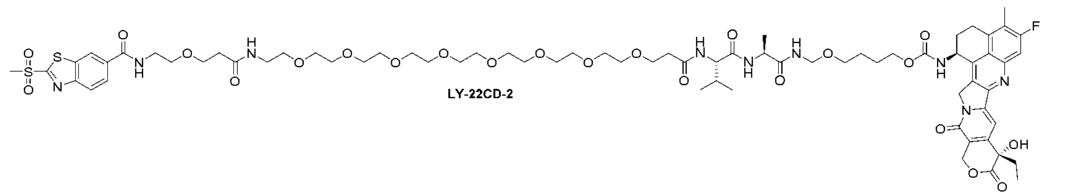

LY-22CD-2

,

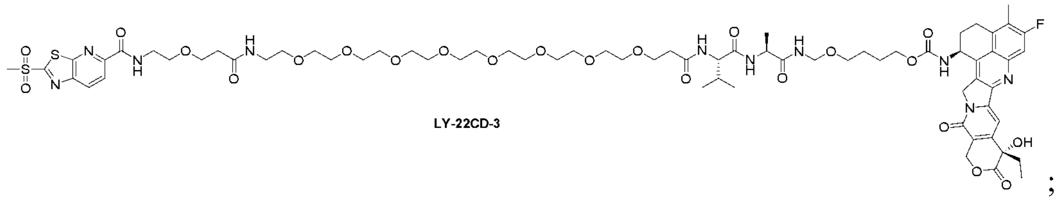

LY-22CD-3

;

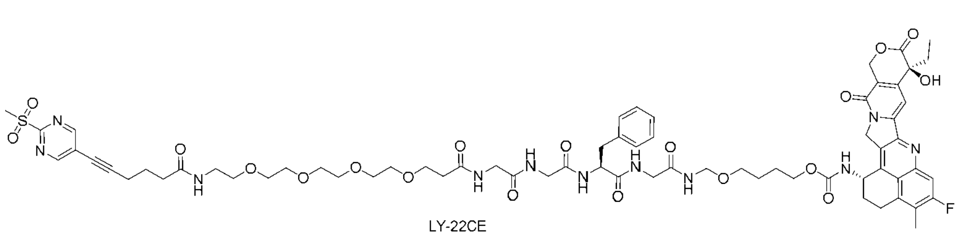

LY-22CE

,

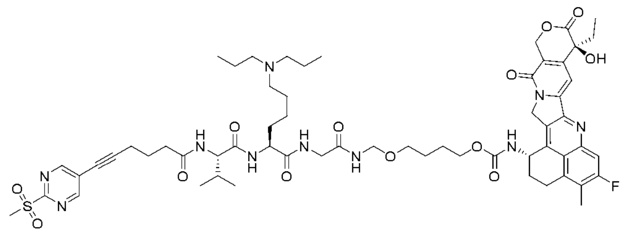

LY-22CF

,

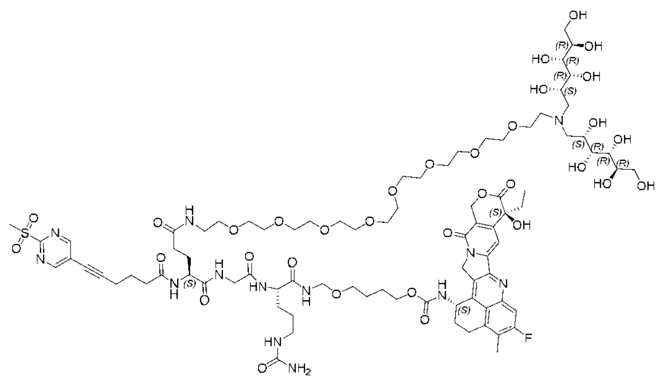

LY-22CG

,

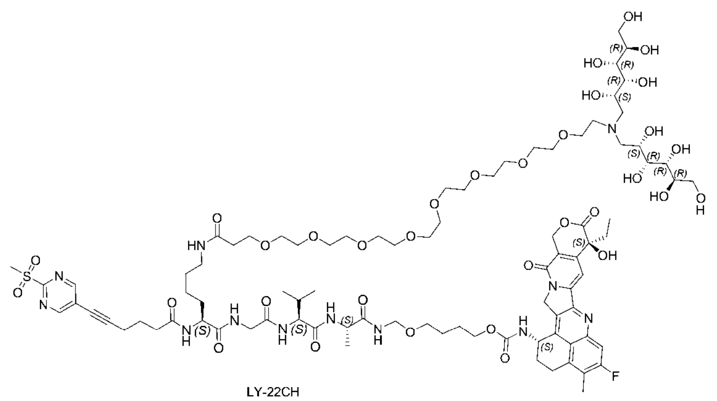

LY-22CH

,

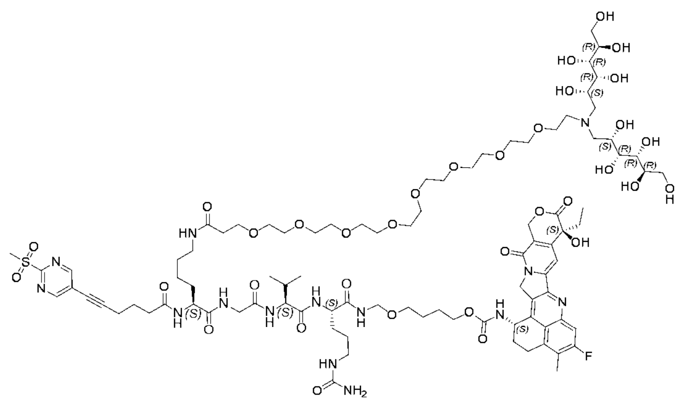

LY-22CI

,

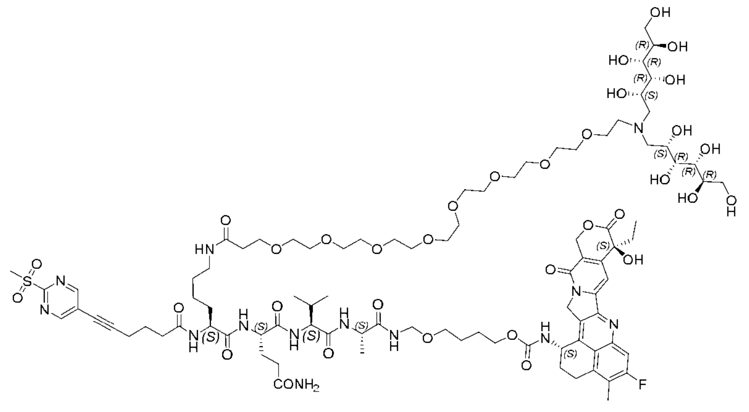

LY-22CJ

,

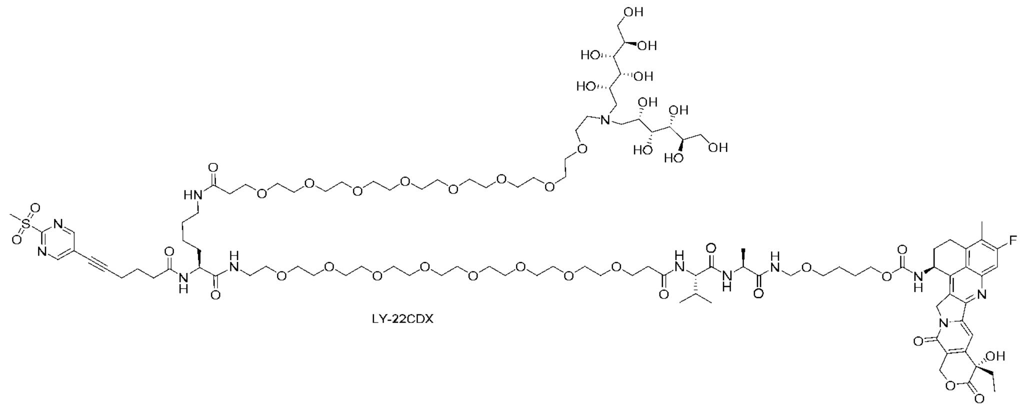

LY-22CDX

,

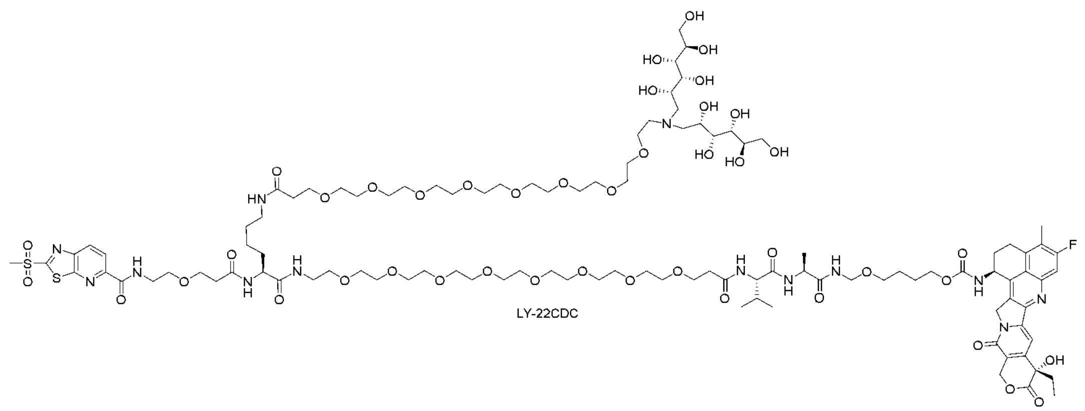

LY-22CDC

,

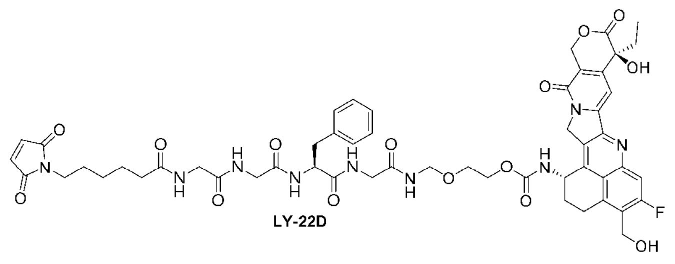

**LY-22D**

,

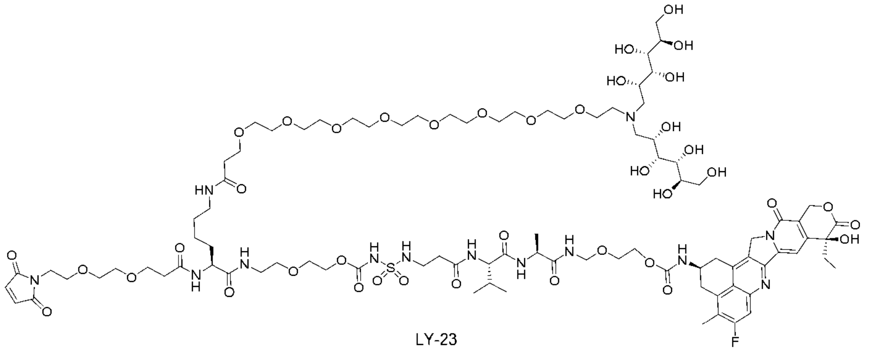

LY-23

,

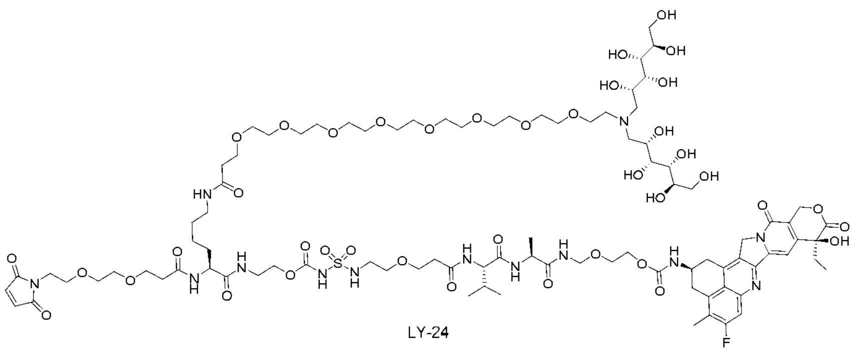

LY-24

;

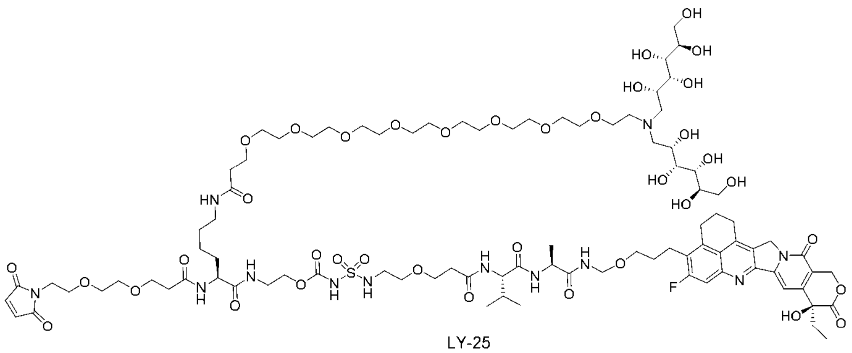

LY-25

,

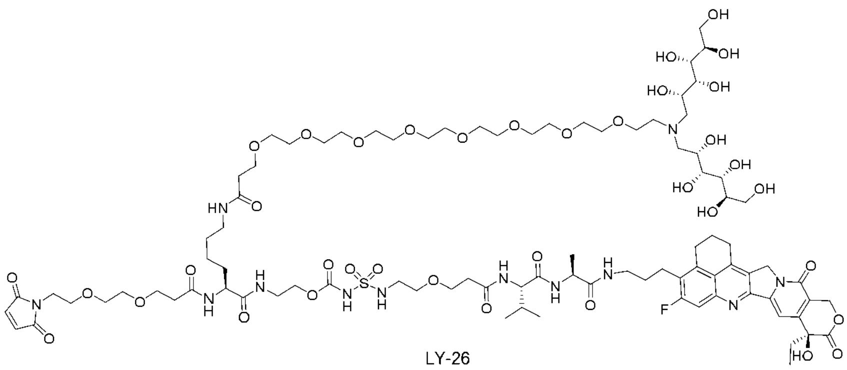

LY-26

,

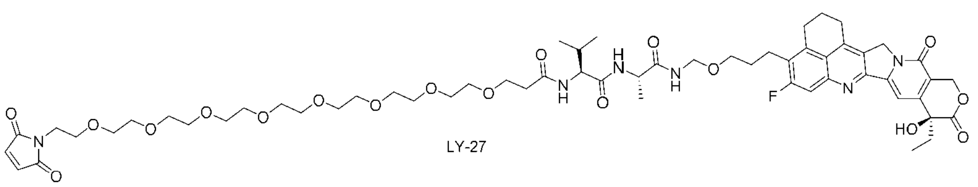

LY-27

,

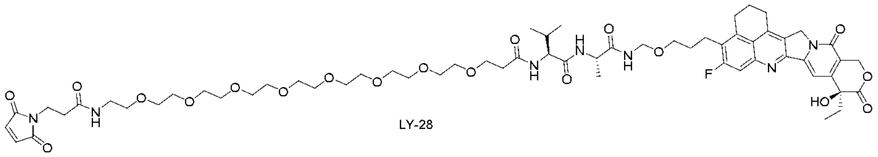
LY-28
,

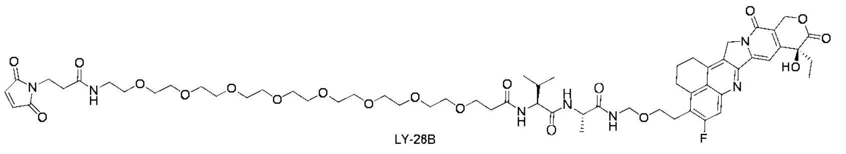
LY-28B
,

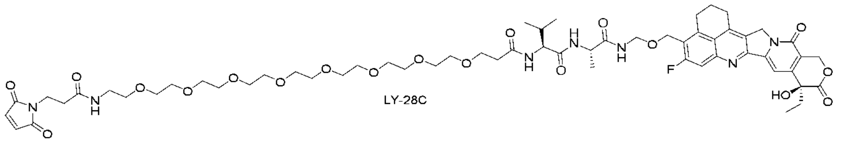
LY-28C
,

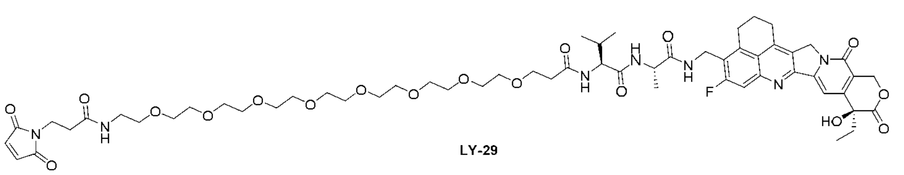
**LY-29**
,

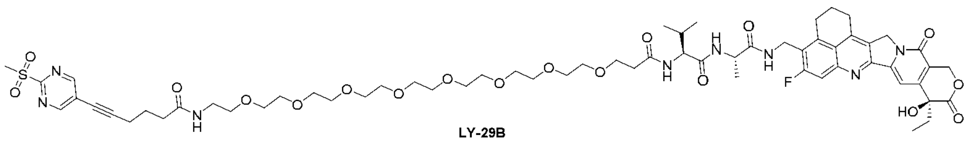
**LY-29B**
,

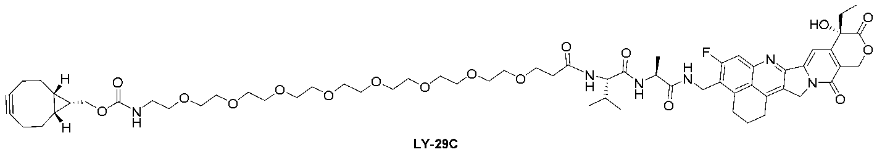
**LY-29C**
,

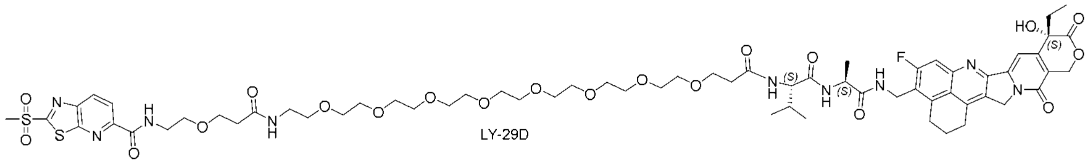
LY-29D
,

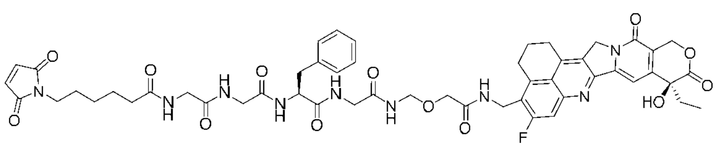
LY-33
,

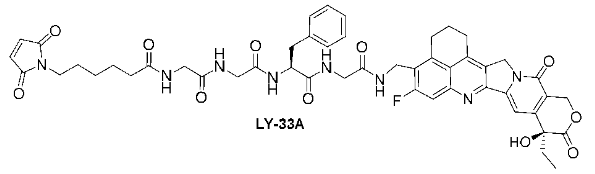
LY-33A
,

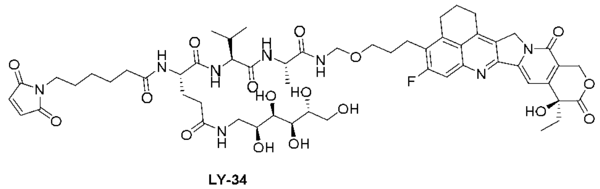
LY-34
,

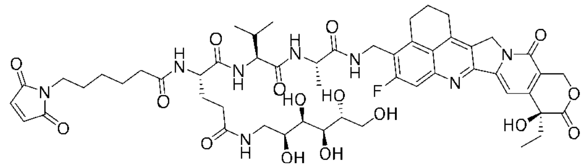
LY-34B
,

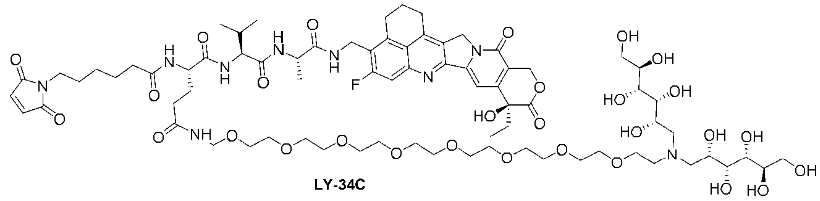
LY-34C
,

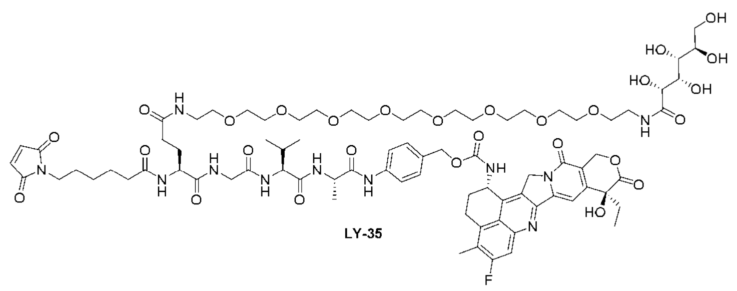
LY-35
,

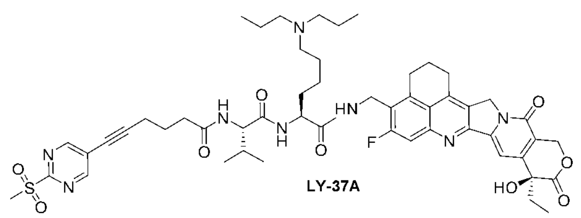
LY-37A
,

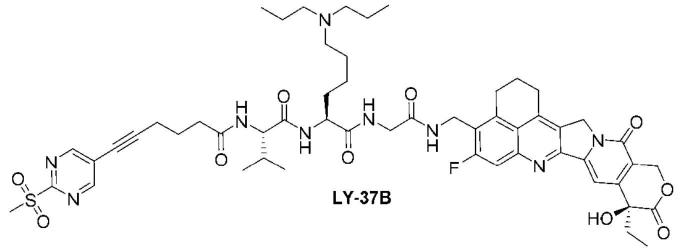
LY-37B
,

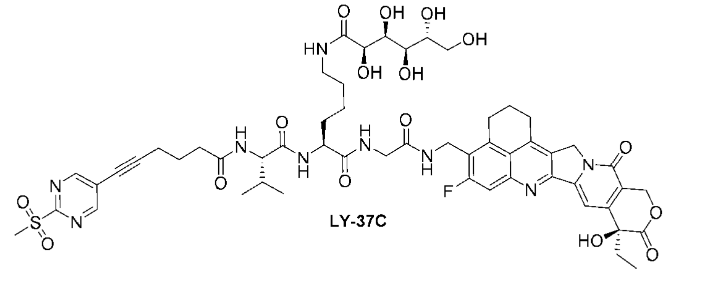

LY-37C

,

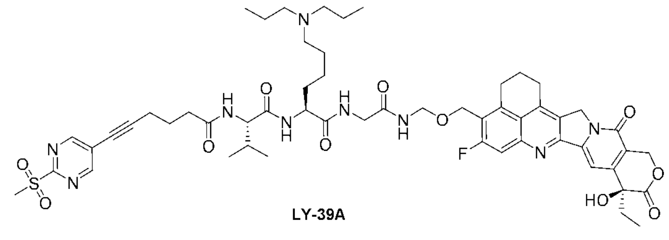

LY-39A

,

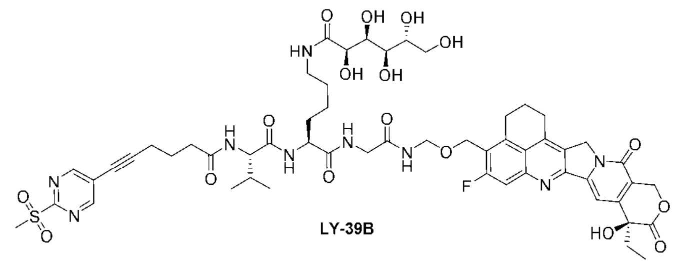

LY-39B

,

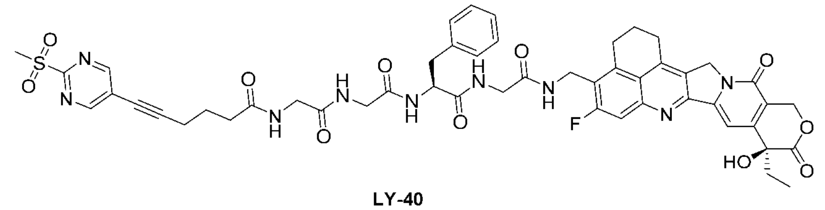

LY-40

,

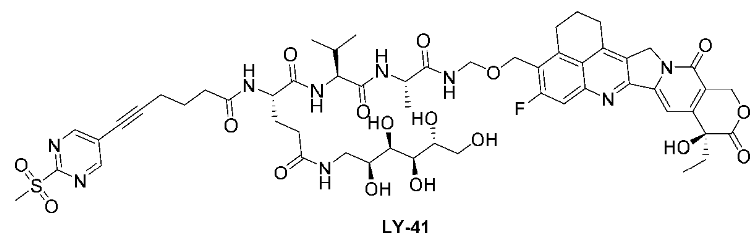

LY-41

,

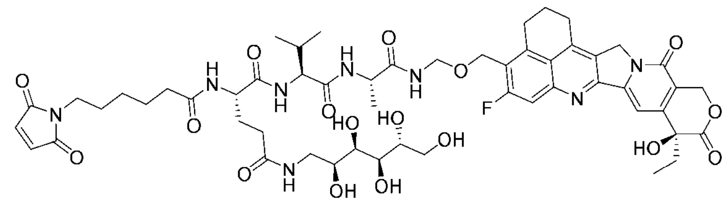

LY-41B

,

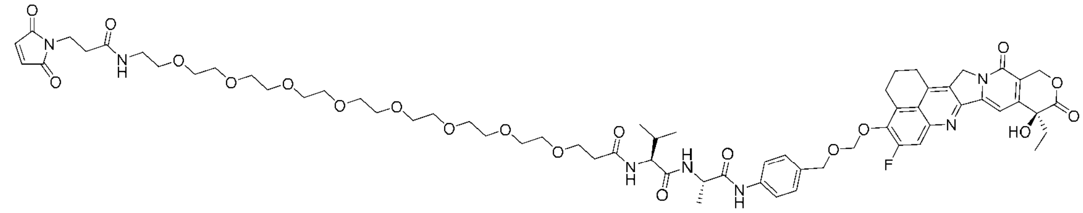

LY-42A

,

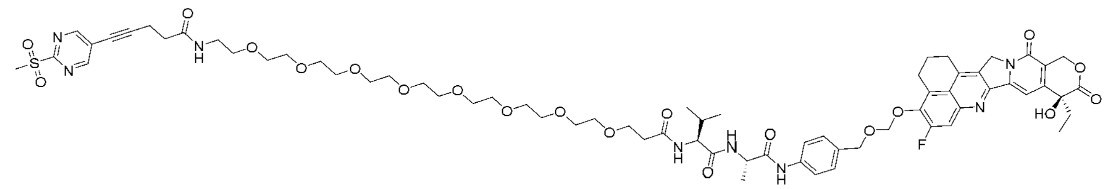

LY-42B

,

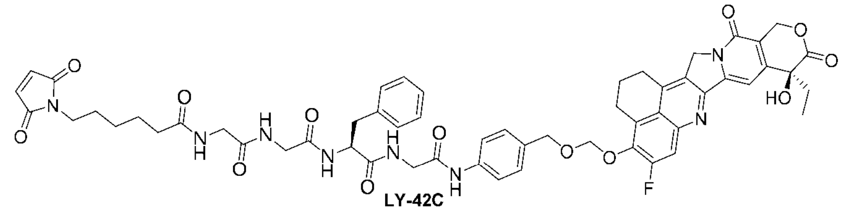

LY-42C

,

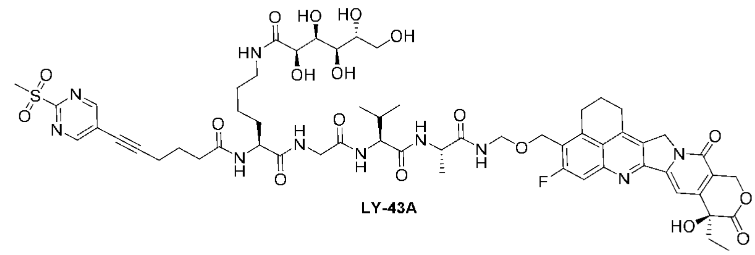

LY-43A

,

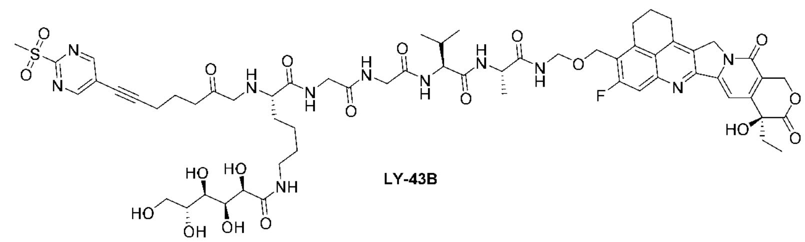

LY-43B

,

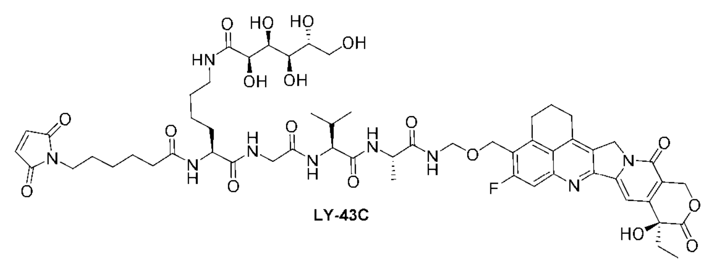

LY-43C

,

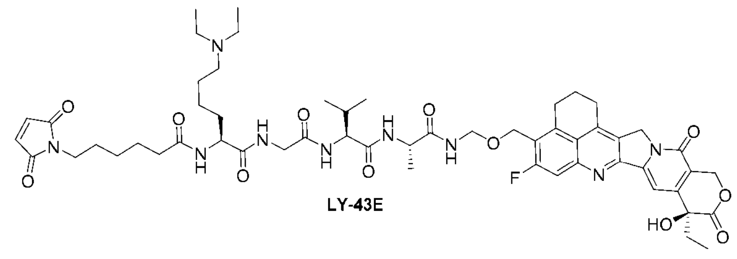

LY-43E

,

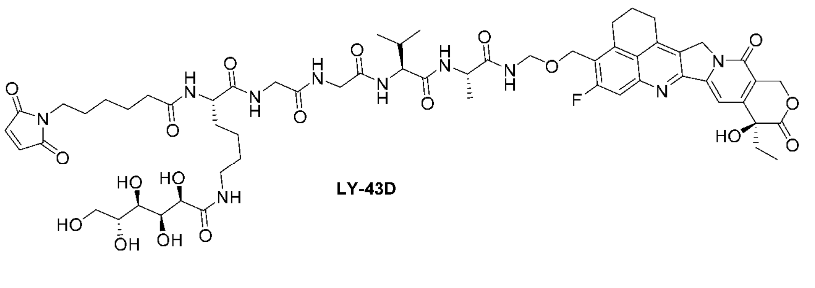

LY-43D

,

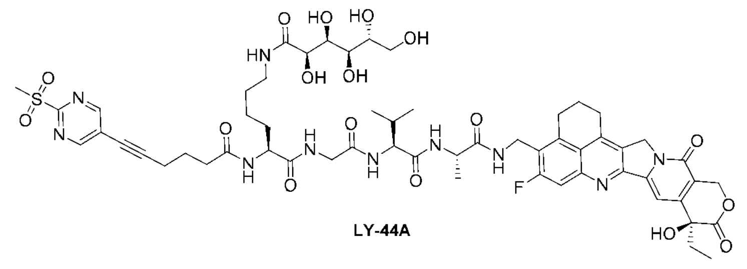

LY-44A

,

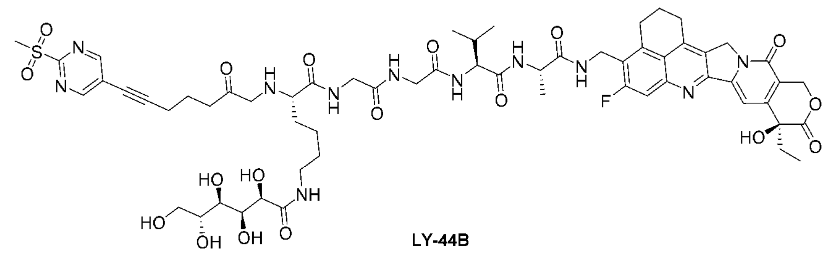

LY-44B

,

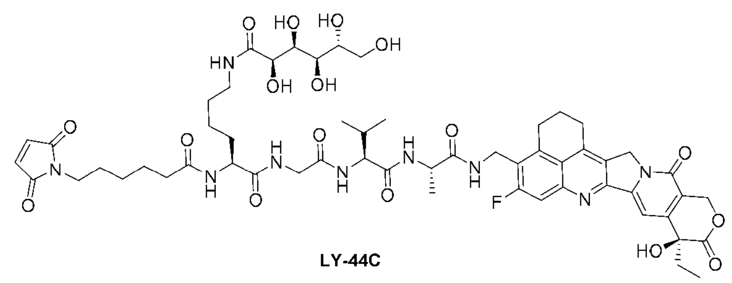

LY-44C

,

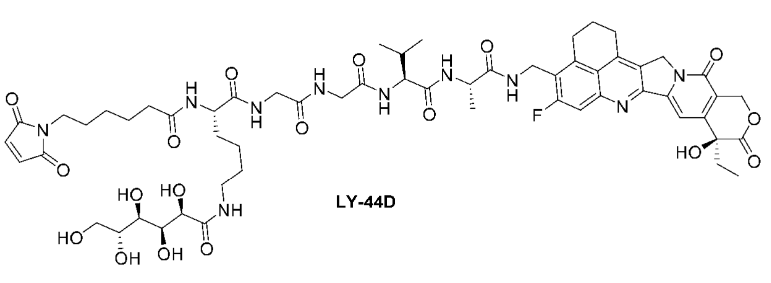

LY-44D

,

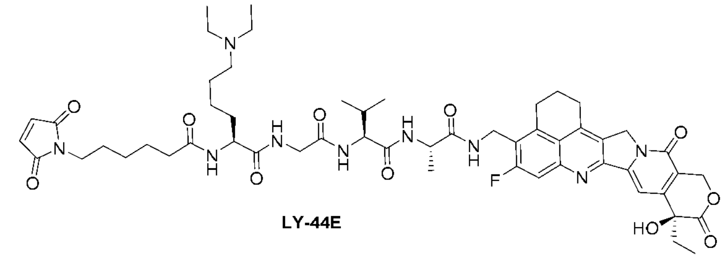

LY-44E

,

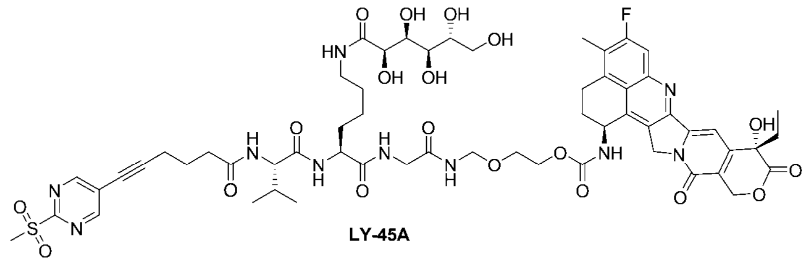

LY-45A

,

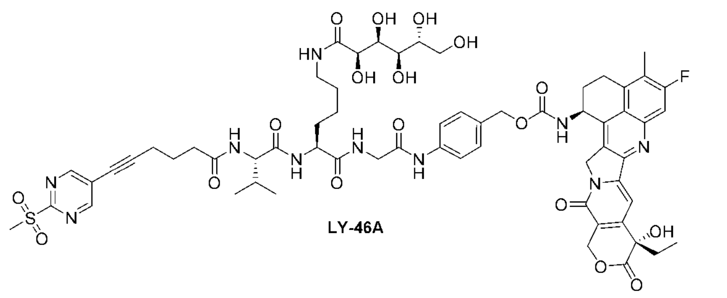

LY-46A

,

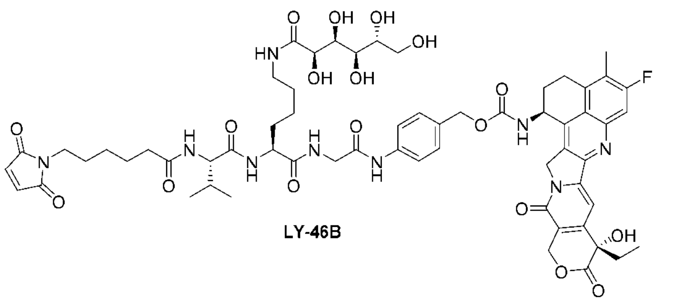

LY-46B

,

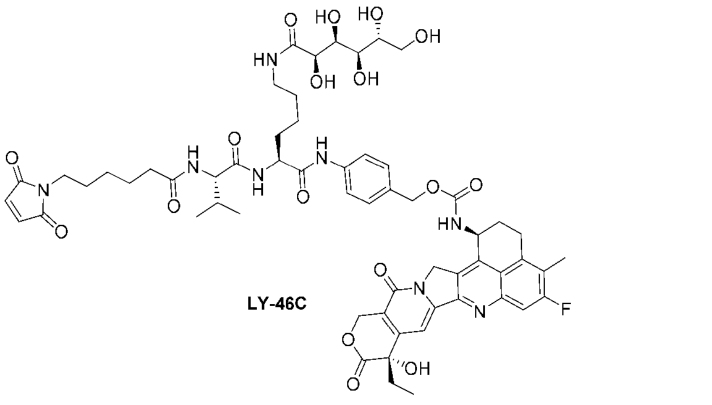

LY-46C

,

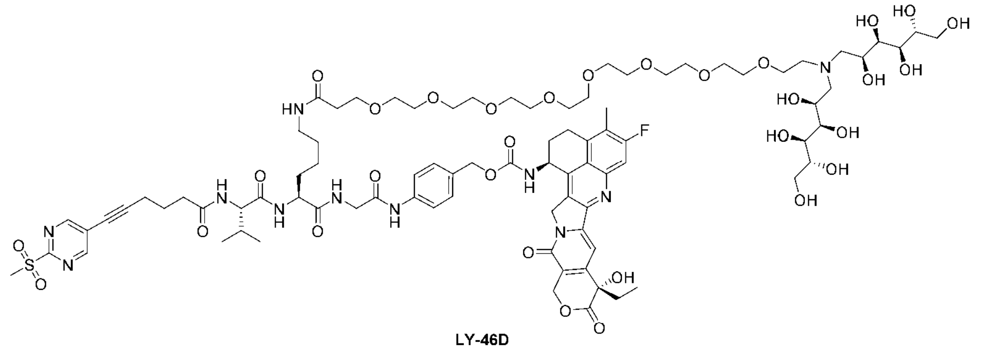

LY-46D

,

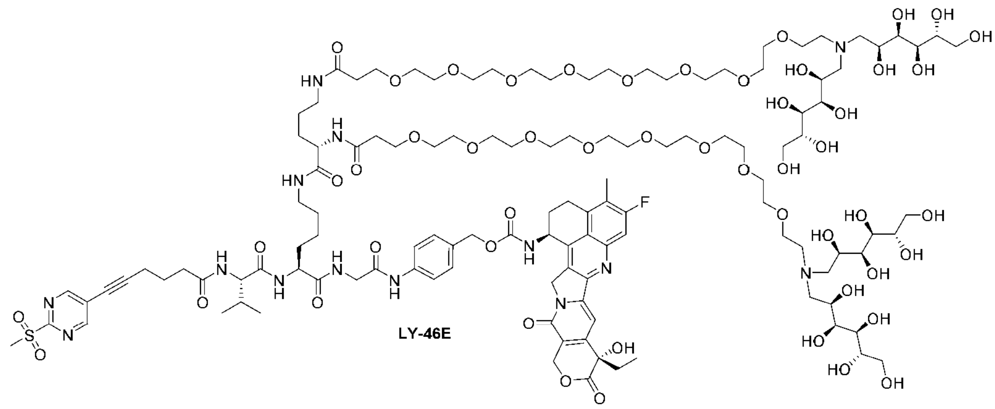

LY-46E

,

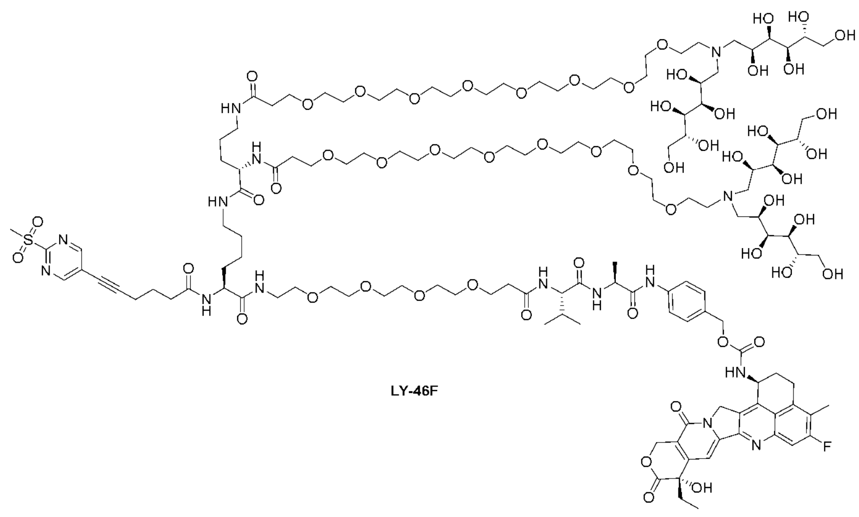

LY-46F

,

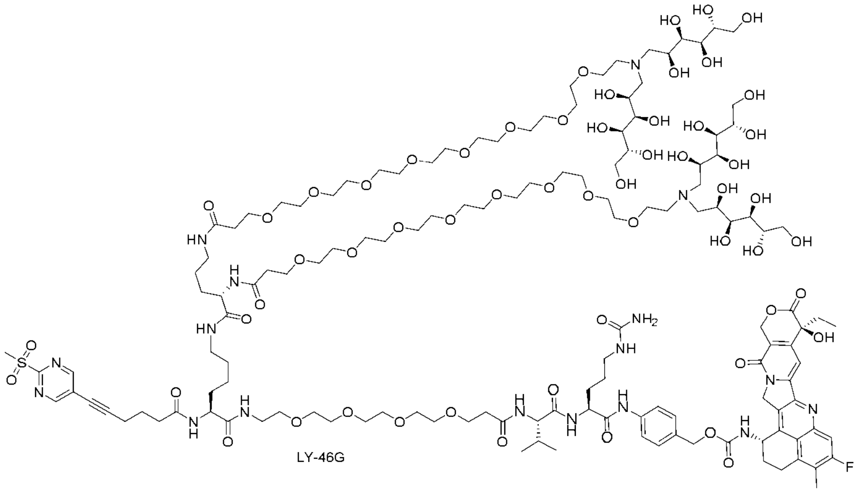

LY-46G

,

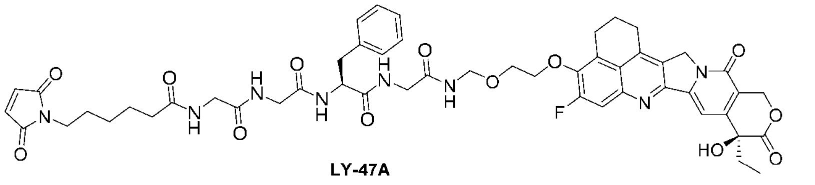

**LY-47A**

,

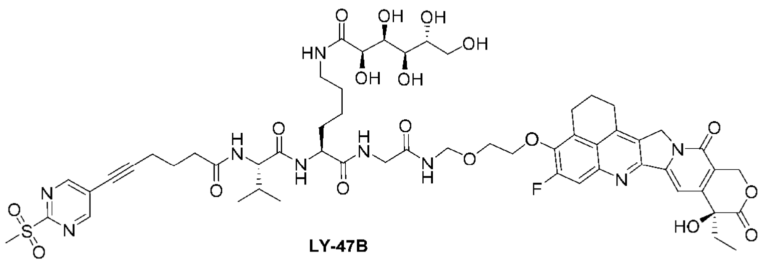

**LY-47B**

,

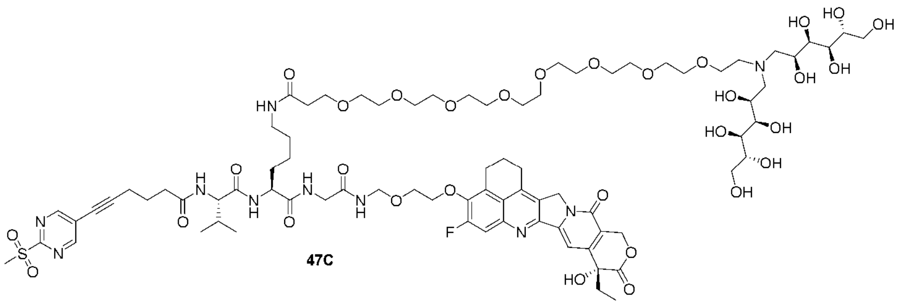

**47C**

,

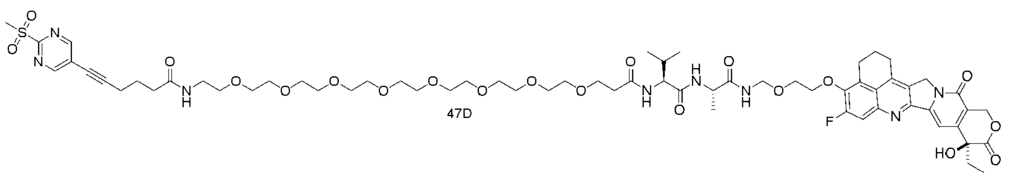

47D

,

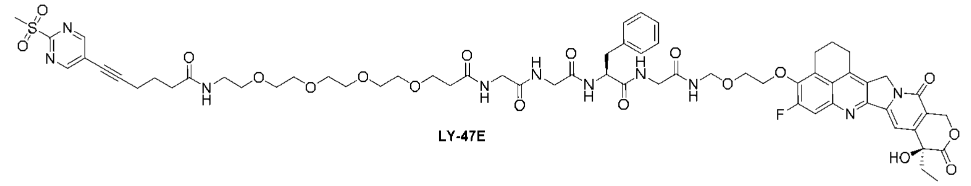

LY-47E

,

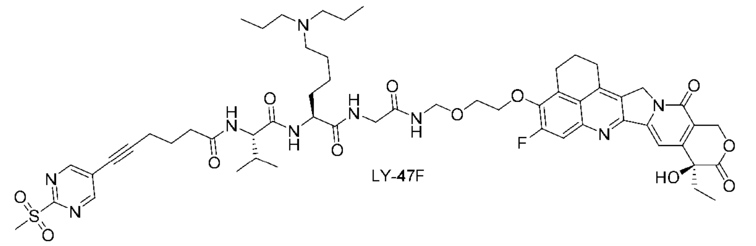

LY-47F

,

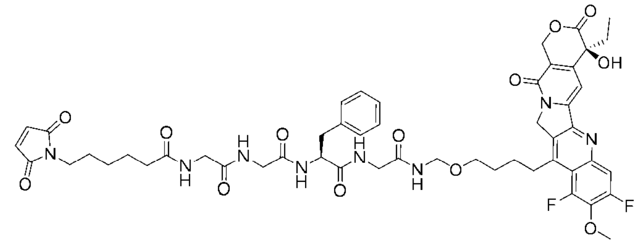

LY-48A

,

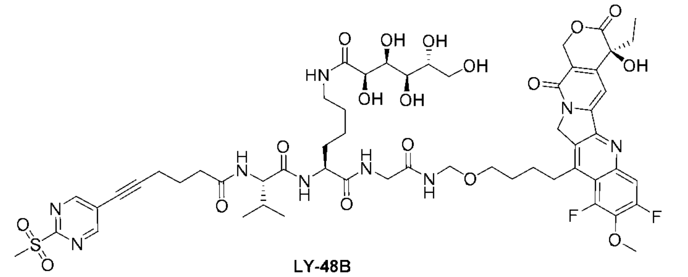

LY-48B

,

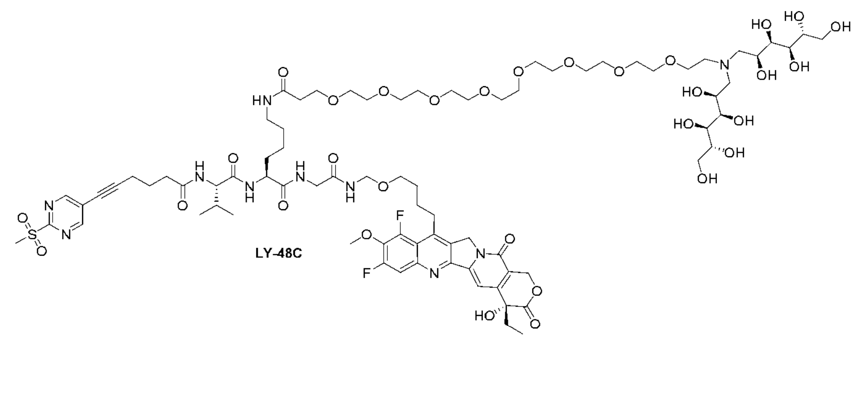

LY-48C

,

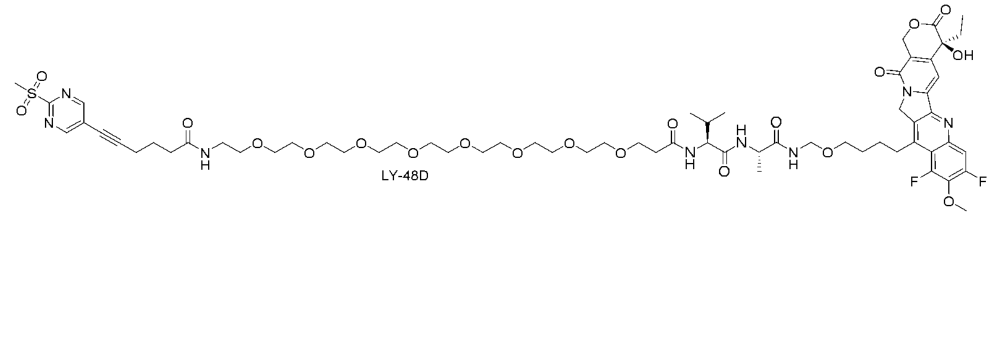

LY-48D

,

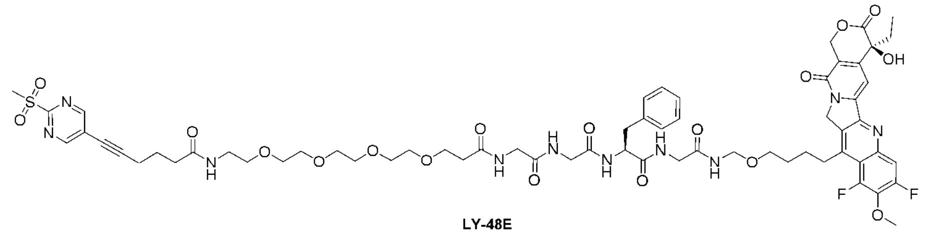

**LY-48E**

,

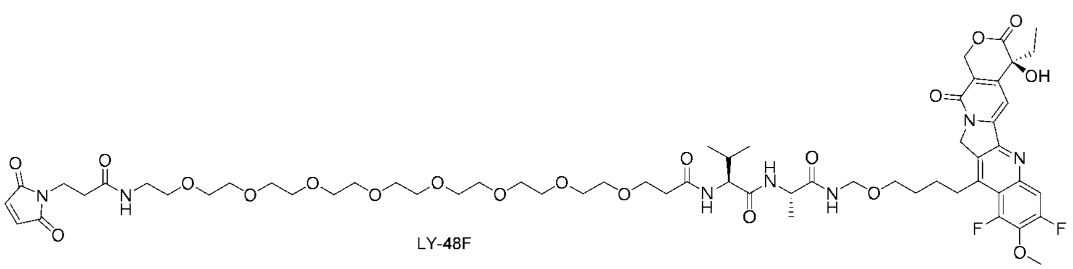

LY-48F

,

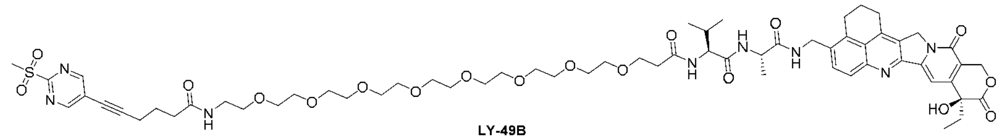

**LY-49B**

,

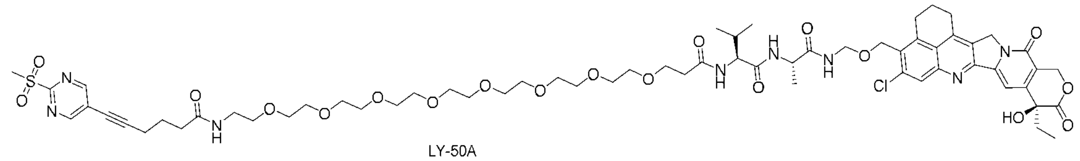

LY-50A

,

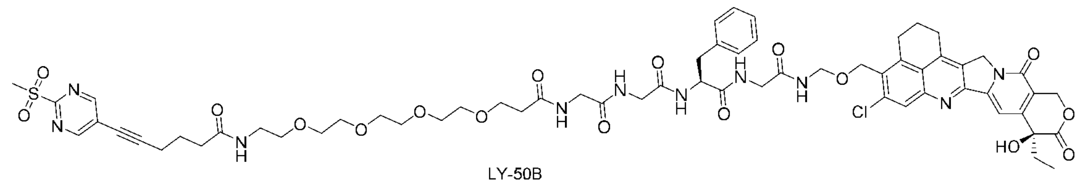

LY-50B

,

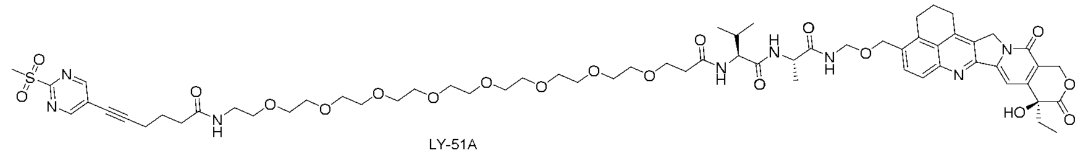

LY-51A

,

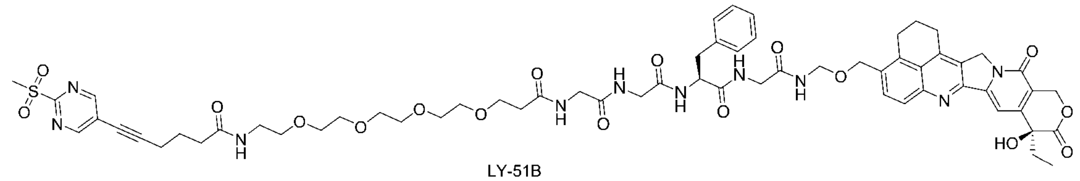

LY-51B

,

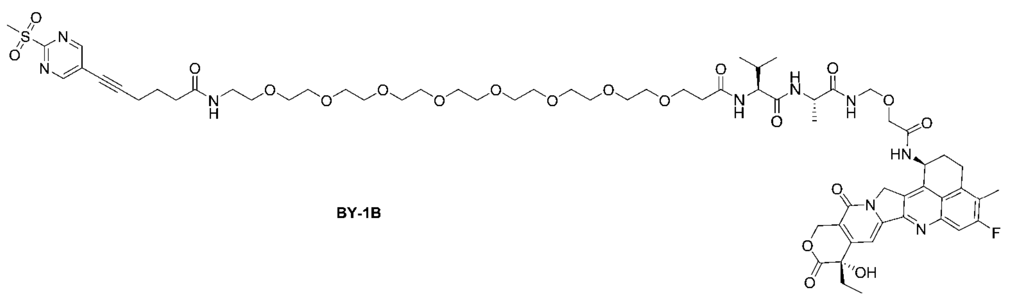

BY-1B

,

and

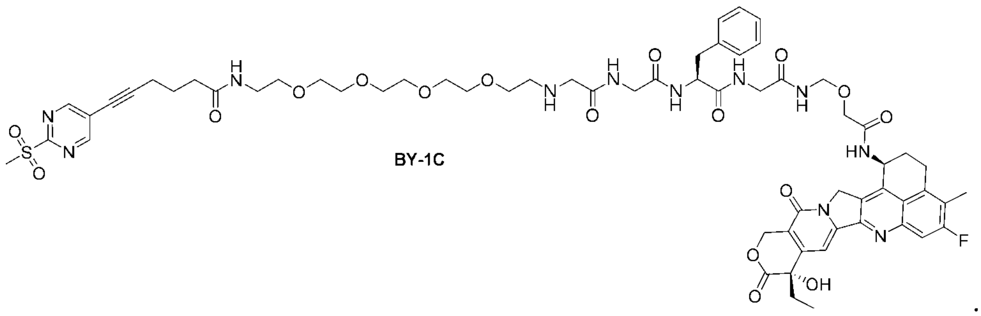

BY-1C

.

Another aspect of the present invention provides a compound represented by general formula (I) or a stereoisomer, tautomer, mesomer, racemate, enantiomer, or diastereomer thereof, or a mixture thereof, or a pharmaceutically acceptable salt thereof;

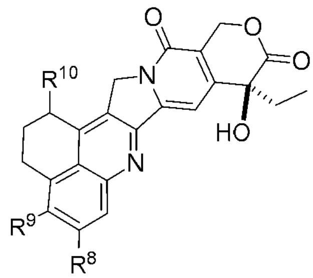

(I)

wherein,

$R^8$ is selected from hydrogen, halogen, hydroxyl, amino, cyano, alkyl, alkoxy, alkenyl, and alkynyl, wherein the alkyl, alkoxy, alkenyl, and alkynyl are optionally further substituted by one or more groups selected from halogen;

$R^9$ is selected from hydrogen, halogen, hydroxyl, carboxyl, cyano, alkyl, alkoxy, alkenyl, alkynyl, cycloalkyl, $-NR^dR^e$, $-(CH_2)_m-OH$, $-(CH_2)_m-NR^dR^e$, $-OC(=O)NR^f-(CH_2)_m-OH$, $-OC(=O)NR^f-(CH_2)_m-NR^dR^e$, $-(CH_2)_m-C(=O)OH$, $-(CH_2)_m-C(=O)-NR^dR^e$, $-(CH_2)_m-C(=O)NR^f-(CH_2)_n-OH$, $-(CH_2)_m-C(=O)NR^f-(CH_2)_n-NR^dR^e$, $-NR^f-(CH_2)_m-OH$, $-NR^f-(CH_2)_m-NR^dR^e$, $-O-(CH_2)_m-OH$, $-O-(CH_2)_m-NR^dR^e$, $-NR^fC(=O)O-(CH_2)_m-OH$, $-NR^bC(=O)O-(CH_2)_m-NR^dR^e$, $-(CH_2)_m-NR^fC(=O)O-(CH_2)_n-OH$, $-(CH_2)_m-NR^fC(=O)O-(CH_2)_n-NR^dR^e$, $-(CH_2)_m-OC(=O)NR^f-(CH_2)_n-NR^dR^e$, $-(CH_2)_m-OC(=O)NR^f-(CH_2)_n-OH$, $-(CH_2)_m-NR^fC(=O)-(CH_2)_n-NR^dR^e$, $-(CH_2)_m-NR^fC(=O)-(CH_2)_n-OH$, and $-(CH_2)_m-NR^fC(=O)-G-(CH_2)_n-OH$, wherein the alkyl, alkoxy, alkenyl, alkynyl, and cycloalkyl are optionally further substituted by one or more groups selected from deuterium, halogen, amino, alkyl, and hydroxyl; and the $-(CH_2)_m-$ is optionally substituted by one or more deuterium or halogen groups;

G is selected from cycloalkylene, heterocyclylene, heteroarylene, and arylene;

$R^{10}$ is selected from hydrogen, halogen, hydroxyl, cyano, alkyl, alkoxy, alkenyl, alkynyl, $CH_2=$, $-NR^dR^e$, $-(CH_2)_m-OH$,

$-(CH_2)_m-NR^dR^e$, $-OC(=O)NR^f-(CH_2)_m-OH$, $-OC(=O)NR^f-(CH_2)_m-NR^dR^e$, - $(CH_2)_m-C(=O)OH$, $-(CH_2)_m-C(=O)-NR^dR^e$, $-(CH_2)_m-C(=O)NR^f(CH_2)_n-OH$, $-(CH_2)_m-C(=O)NR^f-(CH_2)_n-NR^dR^e$, $-NR^f-(CH_2)_m-OH$, $-NR^f-(CH_2)_m-NR^dR^e$, $-NR^f-C(=O)R^d$, $-NR^f-C(=O)-(CH_2)_m-R^d$ - $NR^fC(=O)O-(CH_2)_m-R^d$, $-O-(CH_2)_m-OH$, $-O-(CH_2)_m-NR^dR^e$, $-NR^fC(=O)O-(CH_2)_m-OH$, - $NR^fC(=O)NR^d-(CH_2)_m-OH$, $-NR^fC(=O)NR^d-(CH_2)_m-O-(CH_2)_n-OH$, $-NR^fC(=O)O-(CH_2)_m-O-(CH_2)_n-OH$, $-NR^fC(=O)NR^d-(CH_2)_m-O-(CH_2)_n-NR^dR^e$, $-NR^fC(=O)O-(CH_2)_m-O-(CH_2)_n-NR^dR^e$, - $NR^fC(=O)NR^d-(CH_2)_m-NR^dR^e$, $-NR^fC(=O)O-(CH_2)_m-NR^dR^e$, $-(CH_2)_m-NR^fC(=O)O-(CH_2)_n-OH$, - $(CH_2)_m-NR^fC(=O)O-(CH_2)_n-NR^dR^e$, $-(CH_2)_m-OC(=O)NR^f-(CH_2)_n-NR^dR^e$, $-(CH_2)_m-OC(=O)NR^f-(CH_2)_n-OH$, $-(CH_2)_m-NR^fC(=O)-(CH_2)_n-NR^dR^e$, and $-(CH_2)_m-NR^fC(=O)-(CH_2)_n-OH$, wherein the alkyl, alkoxy, alkenyl, and alkynyl are optionally further substituted by one or more groups selected from halogen;

$R^d$ and $R^e$ are each independently selected from hydrogen and $C_1$-$C_6$ alkyl;

$R^f$ is selected from hydrogen, $C_1$-$C_6$ alkyl, $-C(O)R^c$, $-S(O)R^c$, and $-S(O)_2R^c$, wherein the $C_1$-$C_6$ alkyl is optionally further substituted by $C_3$-$C_6$ cycloalkyl, and $R^c$ is selected from hydrogen, hydroxyl, and $C_1$-$C_6$ alkyl;

m is an integer from 1 to 6; and

n is an integer from 1 to 6.

In a preferred embodiment, in the compound represented by the general formula (I) or the stereoisomer, tautomer, mesomer, racemate, enantiomer, or diastereomer thereof, or the mixture thereof, or the pharmaceutically acceptable salt thereof according to the present invention, $R^8$ is halogen, preferably fluorine or chlorine.

In another preferred embodiment, in the compound represented by the general formula (I) or the stereoisomer, tautomer, mesomer, racemate, enantiomer, or diastereomer thereof, or the mixture thereof, or the pharmaceutically acceptable salt thereof according to the present invention, $R^9$ is selected from hydroxyl, $C_1$-$C_6$ alkyl, $C_1$-$C_6$ alkoxy, $C_3$-$C_6$ cycloalkyl, $-NR^dR^e$, $-(CH_2)_m-OH$, $-(CH_2)_m-NR^dR^e$, $-(CH_2)_m-NR^bC(=O)O-(CH_2)_n-OH$, $-(CH_2)_m-OC(=O)NR^f-(CH_2)_n-OH$, $-(CH_2)_m-NR^fC(=O)-(CH_2)_n-OH$, $-O-(CH_2)_m-NR^dR^e$, $-O-(CH_2)_m-OH$, and $-(CH_2)_m-NR^fC(=O)-G-(CH_2)_n-OH$; and - $(CH_2)_m$- is optionally substituted by one or more deuterium or halogen groups;

G is selected from cycloalkylene, heterocyclylene, heteroarylene, and arylene;

$R^d$ and $R^e$ are each independently selected from hydrogen and $C_1$-$C_6$ alkyl, or is preferably hydrogen;

$R^f$ is selected from hydrogen and $C_1$-$C_6$ alkyl, or is preferably hydrogen;

m is an integer from 1 to 6; preferably, an integer from 1 to 4; and

n is an integer from 1 to 6, preferably 1 or 2.

In another preferred embodiment, in the compound represented by the general formula (I) or the stereoisomer, tautomer, mesomer, racemate, enantiomer, or diastereomer thereof, or the mixture thereof, or the pharmaceutically acceptable salt thereof according to the present invention, $R^{10}$ is selected from hydrogen, hydroxyl, amino, $-(CH_2)_m-OH$, $-OC(=O)NR^f-(CH_2)_m-OH$, $-(CH_2)_m-C(=O)OH$, $-(CH_2)_m-C(=O)NR^f-(CH_2)_n-OH$, $-NR^f-(CH_2)_m-OH$, $-NR^fC(=O)O-(CH_2)_m-OH$, - $NR^fC(=O)NR^d-(CH_2)_m-OH$, $-NR^fC(=O)NR^d-(CH_2)_m-O-(CH_2)_n-OH$, $-NR^fC(=O)O-(CH_2)_m-O-(CH_2)_n-OH$, $-NR^fC(=O)NR^d-(CH_2)_m-O-(CH_2)_n-NR^dR^e$, $-NR^fC(=O)O-(CH_2)_m-O-(CH_2)_n-NR^dR^e$, - $NR^fC(=O)NR^d-(CH_2)_m-NR^dR^e$, and $-NR^fC(=O)O-(CH_2)_m-NR^dR^e$;

$R^d$ and $R^e$ are each independently selected from hydrogen and $C_1$-$C_6$ alkyl, or is preferably hydrogen;

$R^f$ is selected from hydrogen, $C_1$-$C_6$ alkyl, $-C(O)R^c$, $-S(O)R^c$, and $-S(O)_2R^c$, wherein $R^c$ is selected from hydrogen, hydroxyl, and $C_1$-$C_6$ alkyl;

m is an integer from 1 to 6; preferably, an integer from 1 to 4; more preferably 1 or 2; and

n is an integer from 1 to 6, preferably 1 or 2.

In another preferred embodiment, in the compound represented by the general formula (I) or the stereoisomer, tautomer, mesomer, racemate, enantiomer, or diastereomer thereof, or the mixture thereof, or the pharmaceutically

acceptable salt thereof according to the present invention, $R^9$ is selected from hydrogen, a $C_1$-$C_6$ alkyl, $C_1$-$C_6$ alkoxy, hydroxyl, carboxyl, $-NR^dR^e$, $-(CH_2)_m-OH$, $-(CH_2)_m-NR^dR^e$, $-(CH_2)_m-NR^fC(=O)O-(CH_2)_n-OH$, $-(CH_2)_m-OC(=O)NR^f-(CH_2)_n-OH$, $-(CH_2)_m-NR^fC(=O)-(CH_2)_n-OH$, $-O-(CH_2)_m-NR^dR^e$, and $-O-(CH_2)_m-OH$;

$R^d$ and $R^e$ are each independently selected from hydrogen and $C_1$-$C_6$ alkyl;

$R^f$ is selected from hydrogen and $C_1$-$C_6$ alkyl;

m is an integer from 1 to 6; and

n is an integer from 1 to 4, preferably 1 or 2.

In another preferred embodiment, in the compound represented by the general formula (I) or the stereoisomer, tautomer, mesomer, racemate, enantiomer, or diastereomer thereof, or the mixture thereof, or the pharmaceutically acceptable salt thereof according to the present invention, $R^{10}$ is selected from hydrogen, hydroxyl, amino, $CH_2=$, $-(CH_2)_m-OH$, $-OC(=O)NR^f-(CH_2)_m-OH$, $-(CH_2)_m-C(=O)OH$, $-(CH_2)_m-C(=O)NR^f-(CH_2)_n-OH$, $-NR^f-(CH_2)_m-OH$, $-NR^f-C(=O)R^d$, $-NR^f-C(=O)-(CH_2)_m-R^d$, $-NR^fC(=O)O-(CH_2)_m-R^d$, $-NR^f-C(=O)-(CH_2)_m-OH$, and $-NR^fC(=O)O-(CH_2)_m-OH$;

$R^d$ and $R^e$ are each independently selected from hydrogen and $C_1$-$C_6$ alkyl;
$R^f$ is selected from hydrogen, $C_1$-$C_6$ alkyl, $-C(O)R^c$, $-S(O)R^c$, and $-S(O)_2R^c$, wherein the $C_1$-$C_6$ alkyl is optionally further substituted by $C_3$-$C_6$ cycloalkyl, and $R^c$ is selected from hydrogen, hydroxyl, and $C_1$-$C_6$ alkyl;
m is an integer from 1 to 6; preferably, an integer from 1 to 4 or an integer from 4 to 6; and
n is an integer from 1 to 4, preferably 1 or 2.

In another preferred embodiment, the compound represented by the general formula (I) or the stereoisomer, tautomer, mesomer, racemate, enantiomer, or diastereomer thereof, or the mixture thereof, or the pharmaceutically acceptable salt thereof according to the present invention is selected from

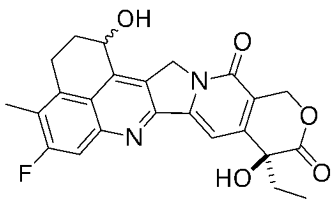
,
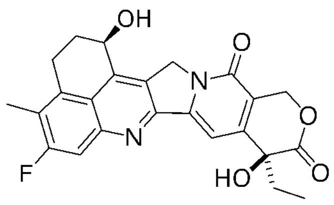
,
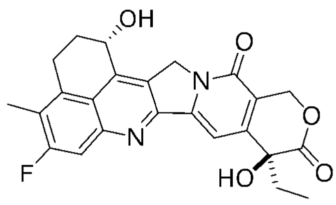
,

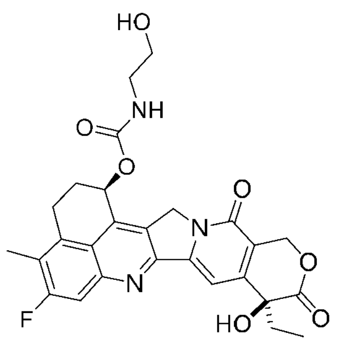
,
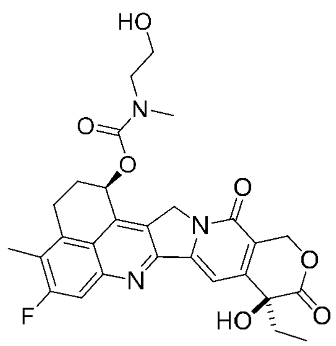
,
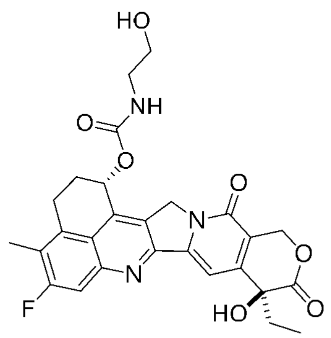
,

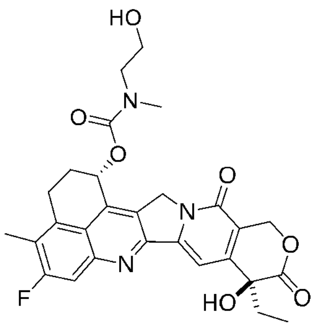
,
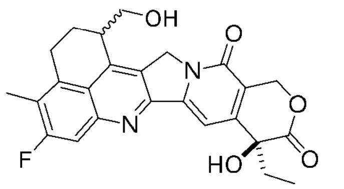
,
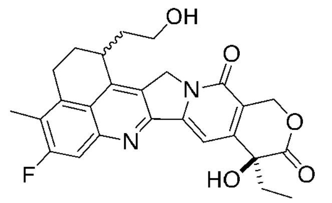
,

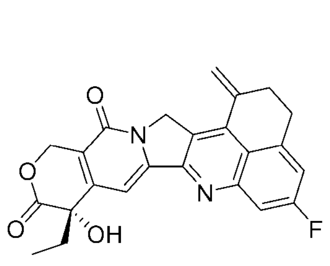
,
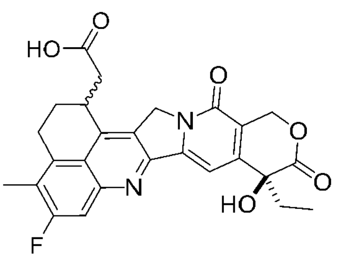
,
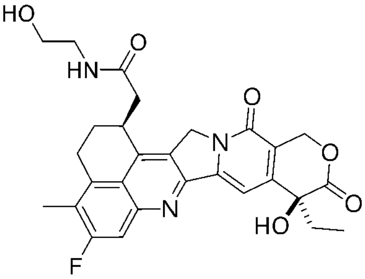
,

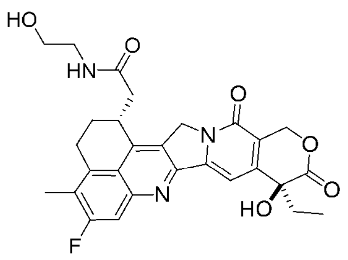
,
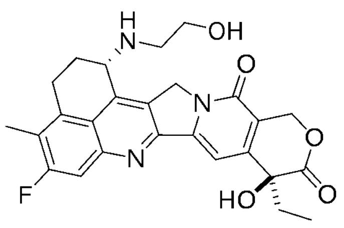
,
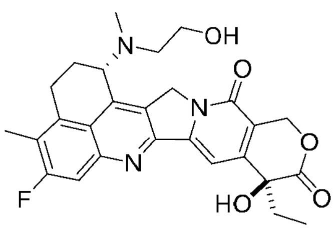
,

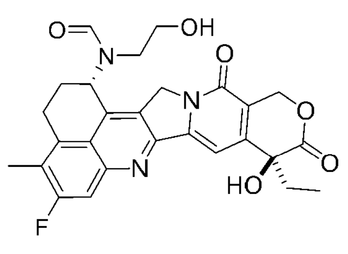
,
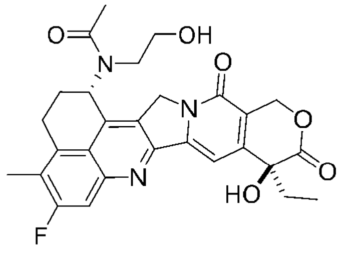
,
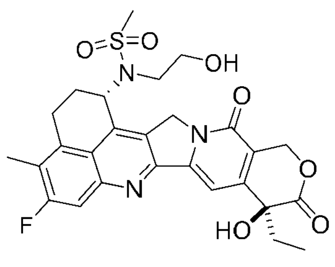
,

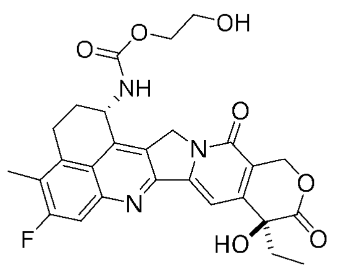
,
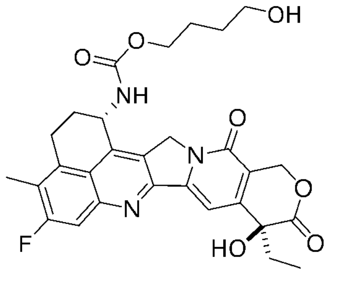
,
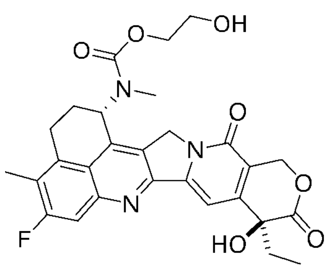
,

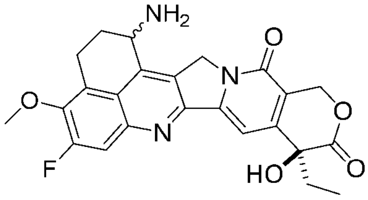 , 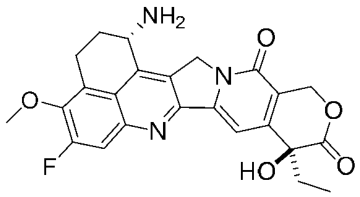 , 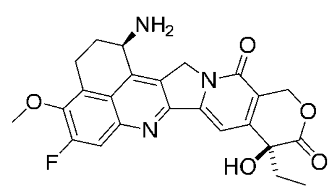 ,

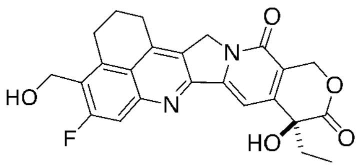 , 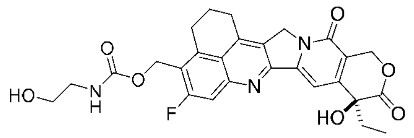 , 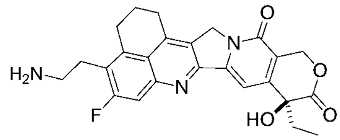 ,

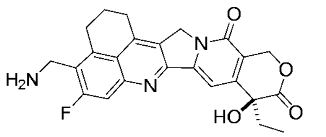 , 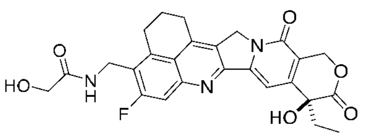 , 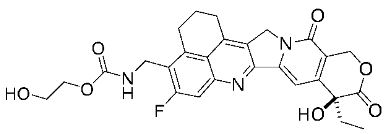 ,

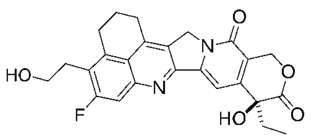 , 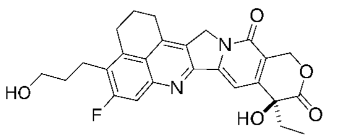 , 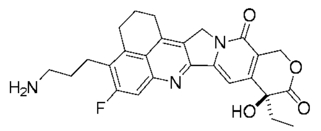 ,

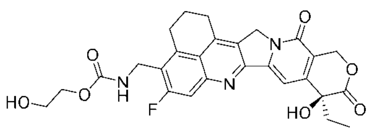 , 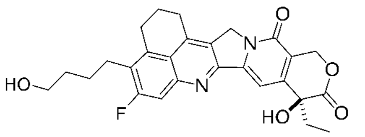 , 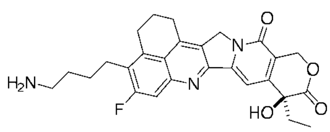 ,

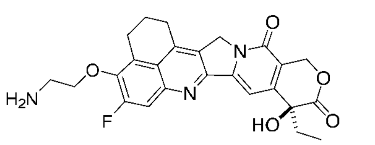 , 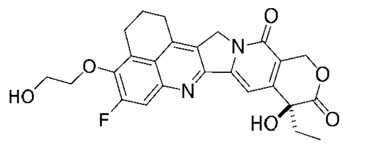 , 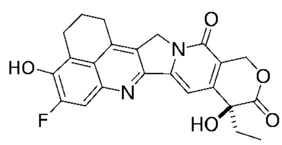 ,

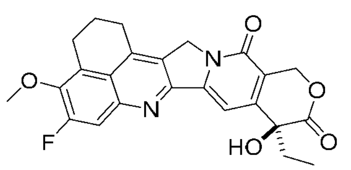 , 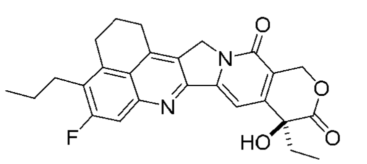 , 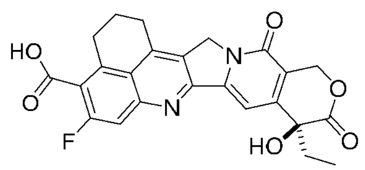 ,

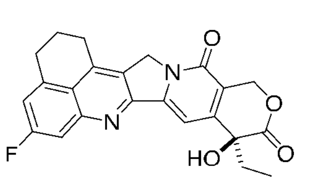 , 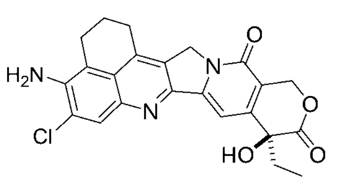 , 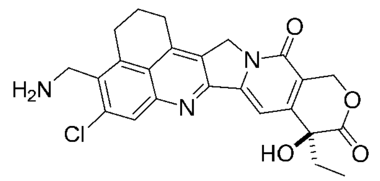 ,

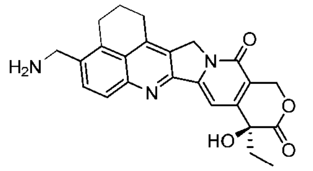 ,
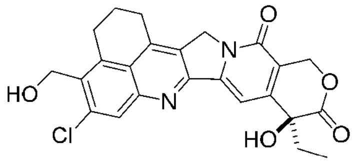 ,
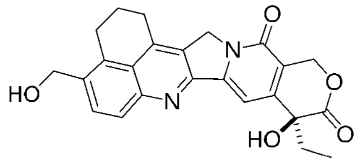 ,

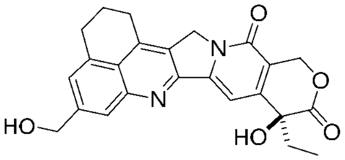 ,
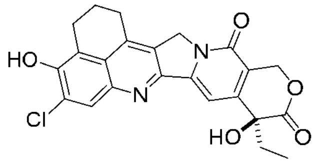 ,
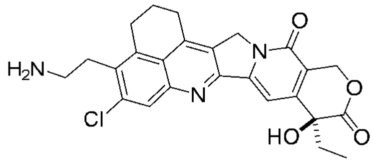 ,

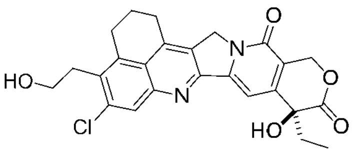 ,
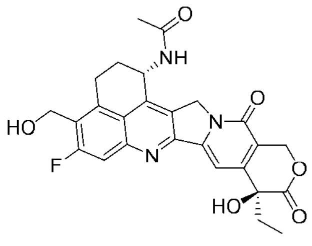 ,
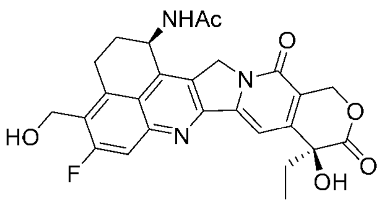 ,

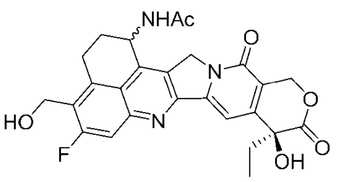 ,
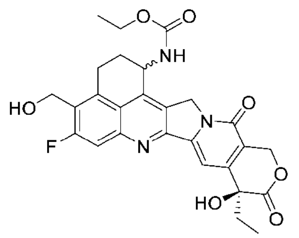 ,
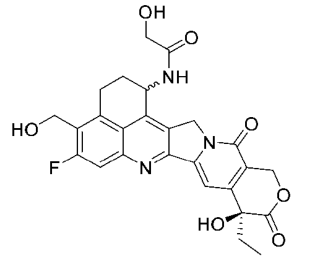 ,

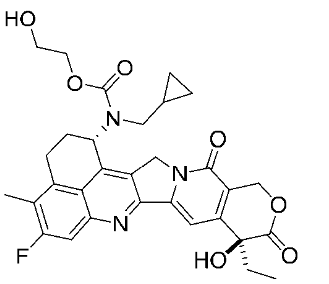 ,
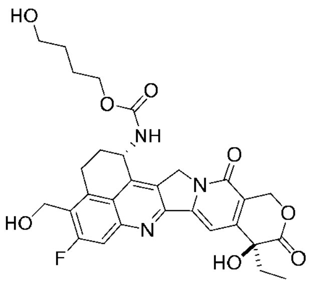 ,
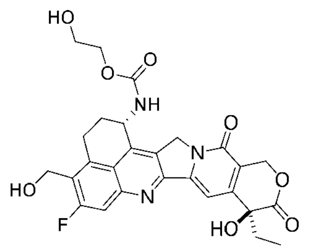 ,

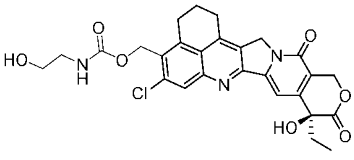 ,
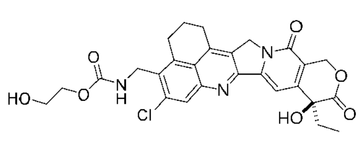 ,
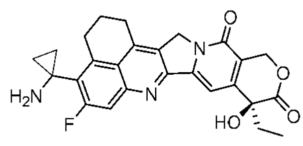 ,

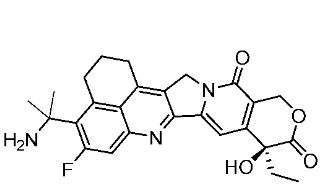 ,
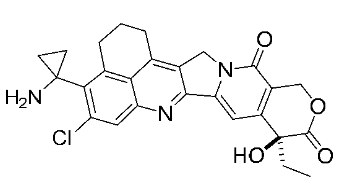 ,
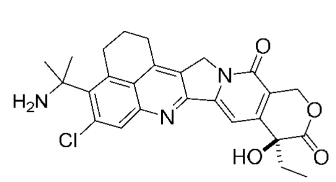 ,

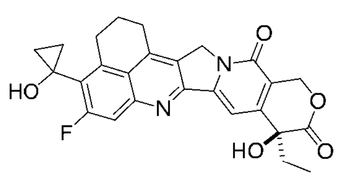,
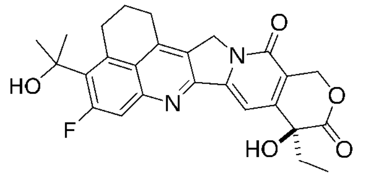,
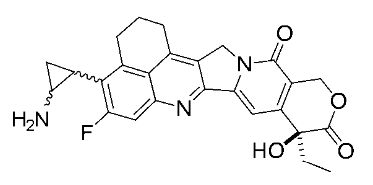,

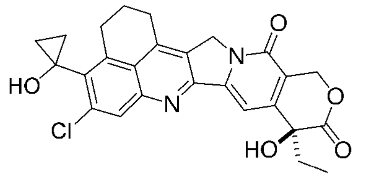,
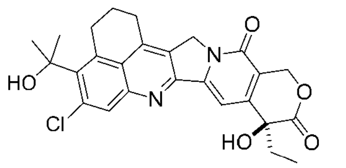,
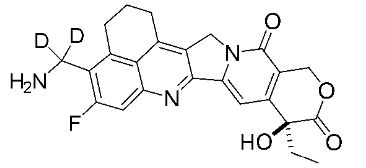,

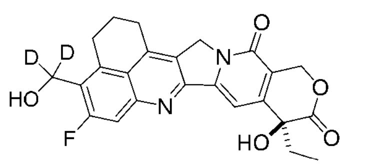,
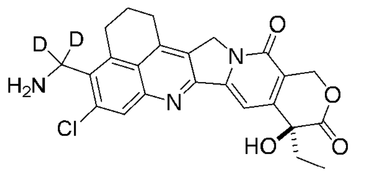,
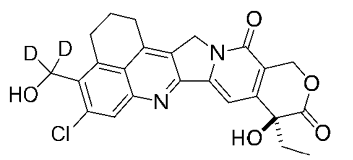,

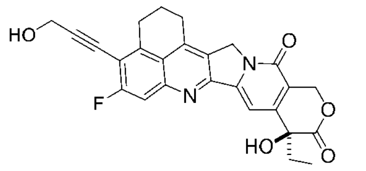,
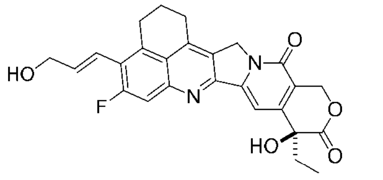,
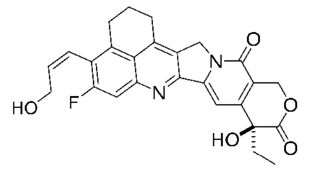,

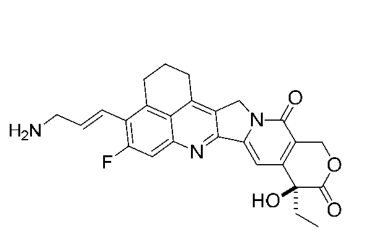,
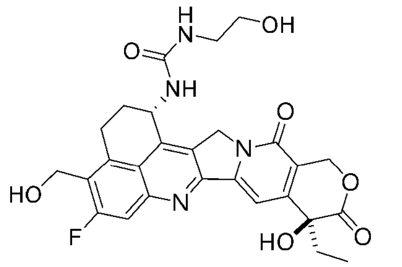,
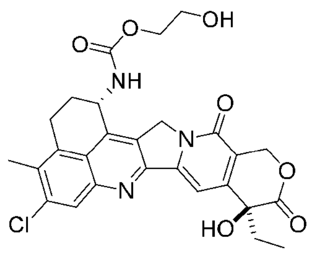,

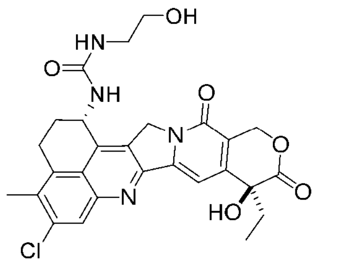,
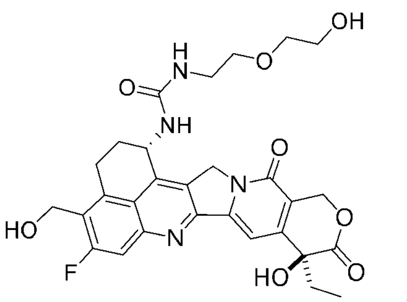,
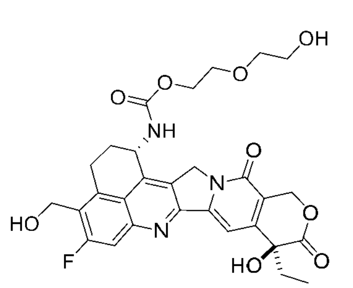,

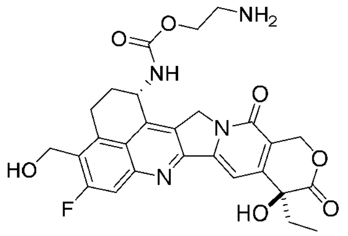 , 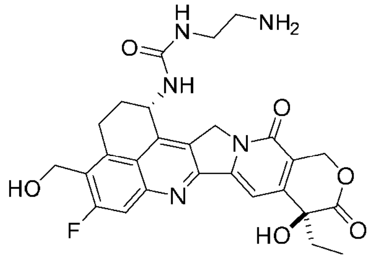 , 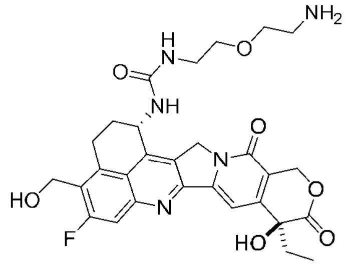 ,

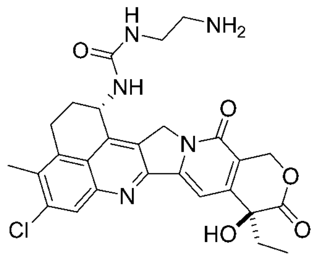 , 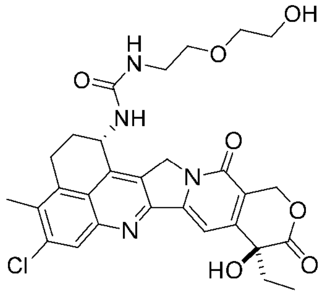 , 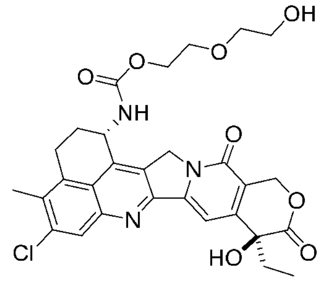 ,

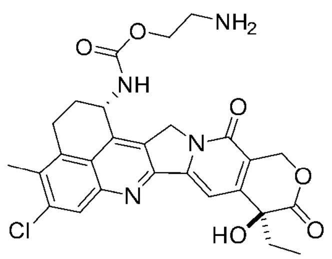 , 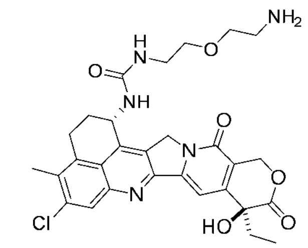 , 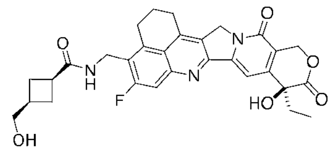 ,

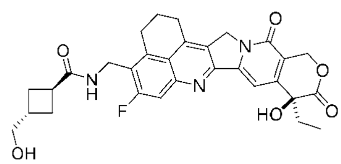 , 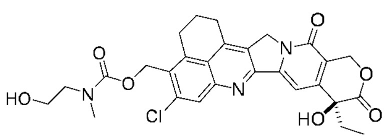 , 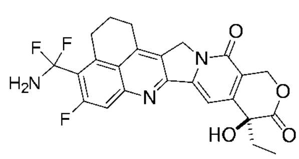 ,

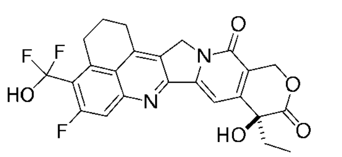 , 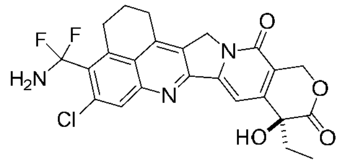 , 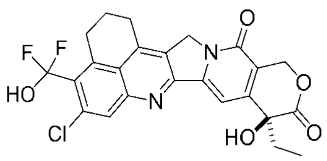 ,

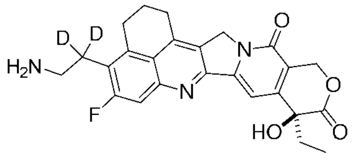 , 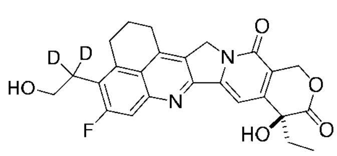 , 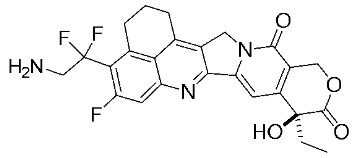 ,

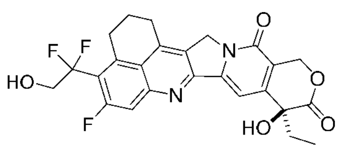 , 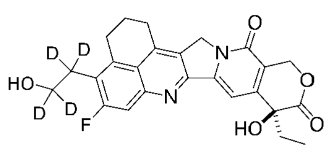 , 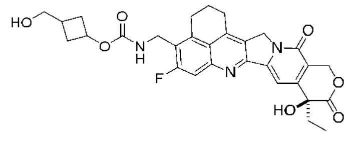 ,

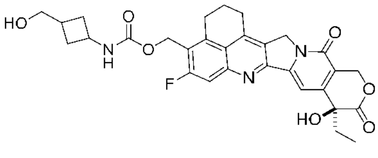, 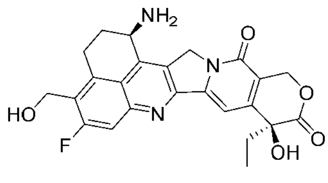, and 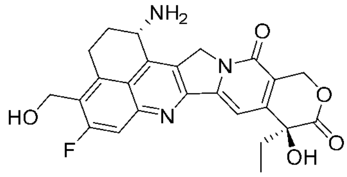.

Still another aspect of the present invention provides a compound represented by general formula (II) or a stereoisomer, tautomer, mesomer, racemate, enantiomer, or diastereomer thereof, or a mixture thereof, or a pharmaceutically acceptable salt thereof;

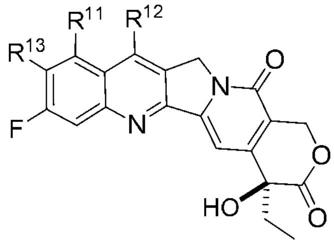

(II)

wherein,

$R^{11}$ is selected from hydrogen, halogen, hydroxyl, amino, cyano, alkyl, alkoxy, alkenyl, and alkynyl, wherein the alkyl, alkoxy, alkenyl, and alkynyl are optionally further substituted by one or more groups selected from halogen;

$R^{12}$ is selected from hydrogen, halogen, hydroxyl, amino, cyano, alkyl, alkoxy, alkenyl, alkynyl, - $NR^dR^e$, $-(CH_2)_m-OH$, $-(CH_2)_m-NR^dR^e$, $-OC(=O)NR^f-(CH_2)_m-OH$, $-OC(=O)NR^f-(CH_2)_m-NR^dR^e$, - $(CH_2)_m-C(=O)OH$, $-(CH_2)_m-C(=O)-NR^dR^e$, $-(CH_2)_m-C(=O)NR^f-(CH_2)_n-OH$, $-(CH_2)_m-C(=O)NR^f-(CH_2)_n-NR^dR^e$, $-NR^f-(CH_2)_m-OH$, $-NR^f-(CH_2)_m-NR^dR^e$, $-O-(CH_2)_m-OH$, $-O-(CH_2)_m-NR^dR^e$, - $NR^fC(=O)O-(CH_2)_m-OH$, $-NR^bC(=O)O-(CH_2)_m-NR^a-*$, $-(CH_2)_m-NR^bC(=O)O-(CH_2)_n-O-*$, $-(CH_2)_m-NR^fC(=O)O-(CH_2)_n-NR^dR^e$, $-(CH_2)_m-OC(=O)NR^f-(CH_2)_n-NR^dR^e$, $-(CH_2)_m-OC(=O)NR^f-(CH_2)_n-OH$, $-(CH_2)_m-NR^fC(=O)-(CH_2)_n-NR^dR^e$, and $-(CH_2)_m-NR^fC(=O)-(CH_2)_n-OH$, wherein the alkyl, alkoxy, alkenyl, and alkynyl are optionally further substituted by one or more groups selected from halogen;

$R^{13}$ is selected from hydrogen, halogen, hydroxyl, amino, cyano, alkyl, alkoxy, alkenyl, alkynyl, - $NR^dR^e$, $-(CH_2)_m-OH$, $-(CH_2)_m-NR^dR^e$, $-OC(=O)NR^f-(CH_2)_m-OH$, $-OC(=O)NR^f-(CH_2)_m-NR^dR^e$, - $(CH_2)_m-C(=O)OH$, $-(CH_2)_m-C(=O)-NR^dR^e$, $-(CH_2)_m-C(=O)NR^f-(CH_2)_n-OH$, $-(CH_2)_m-C(=O)NR^f-(CH_2)_n-NR^dR^e$, $-NR^f-(CH_2)_m-OH$, $-NR^f-(CH_2)_m-NR^dR^e$, $-O-(CH_2)_m-OH$, $-O-(CH_2)_m-NR^dR^e$, - $NR^fC(=O)O-(CH_2)_m-OH$, $-NR^bC(=O)O-(CH_2)_m-NR^a-*$, $-(CH_2)_m-NR^bC(=O)O-(CH_2)_n-O-*$, $-(CH_2)_m-NR^fC(=O)O-(CH_2)_n-NR^dR^e$, $-(CH_2)_m-OC(=O)NR^f-(CH_2)_n-NR^dR^e$, $-(CH_2)_m-OC(=O)NR^f-(CH_2)_n-OH$, $-(CH_2)_m-NR^fC(=O)-(CH_2)_n-NR^dR^e$, and $-(CH_2)_m-NR^fC(=O)-(CH_2)_n-OH$, wherein the alkyl, alkoxy, alkenyl, and alkynyl are optionally further substituted by one or more groups selected from halogen;

$R^d$ and $R^e$ are each independently selected from hydrogen and $C_1$-$C_6$ alkyl;

$R^f$ is selected from hydrogen, $C_1$-$C_6$ alkyl, $-C(O)R^c$, $-S(O)R^c$, and $-S(O)_2R^c$, wherein $R^c$ is selected from hydrogen, hydroxyl, and $C_1$-$C_6$ alkyl;

m is an integer from 1 to 6; and

n is an integer from 1 to 6.

In a preferred embodiment, in the compound represented by the general formula (II) or the stereoisomer, tautomer, mesomer, racemate, enantiomer, or diastereomer thereof, or the mixture thereof, or the pharmaceutically acceptable salt thereof according to the present invention, $R^{11}$ is selected from halogen, or is preferably fluorine or cyano.

In another preferred embodiment, in the compound represented by the general formula (II) or the stereoisomer, tautomer, mesomer, racemate, enantiomer, or diastereomer thereof, or the mixture thereof, or the pharmaceutically acceptable salt thereof according to the present invention, $R^{12}$ is selected from hydroxyl, amino, a $C_1$-$C_6$ alkyl,

$-(CH_2)_m-OH$, and $-(CH_2)_m-NR^dR^e$;

$R^d$ and $R^e$ are each independently selected from hydrogen and $C_1$-$C_6$ alkyl; and m is an integer from 1 to 6; preferably, an integer from 1 to 4.

In another preferred embodiment, in the compound represented by the general formula (II) or the stereoisomer, tautomer, mesomer, racemate, enantiomer, or diastereomer thereof, or the mixture thereof, or the pharmaceutically acceptable salt thereof according to the present invention, $R^{13}$ is selected from hydroxyl and amino.

In another preferred embodiment, in the compound represented by the general formula (II) or the stereoisomer, tautomer, mesomer, racemate, enantiomer, or diastereomer thereof, or the mixture thereof, or the pharmaceutically acceptable salt thereof according to the present invention, $R^{13}$ is selected from hydroxyl and $C_1$-$C_6$ alkoxy.

In another preferred embodiment, the compound represented by the general formula (II) or the stereoisomer, tautomer, mesomer, racemate, enantiomer, or diastereomer thereof, or the mixture thereof, or the pharmaceutically acceptable salt thereof according to the present invention is selected from:

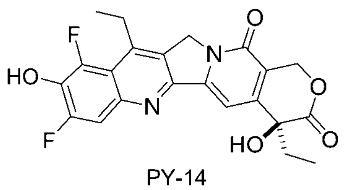
PY-14 ,

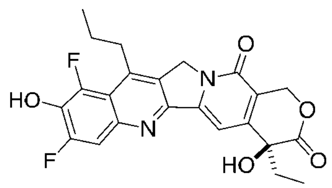
PY-21 ,

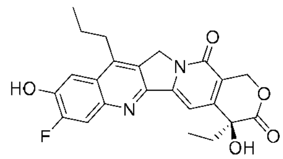
**PY-21-A** ,

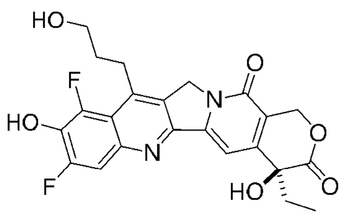
PY-22 ,

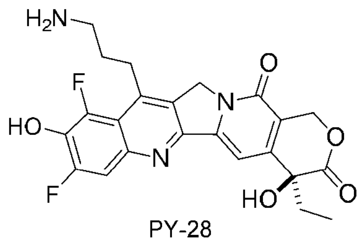
PY-28 ,

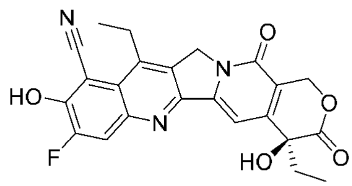
PY-15 ,

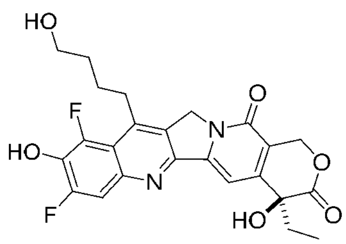
PY-29 ,

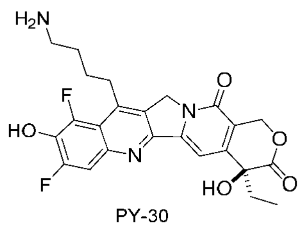
PY-30 ,

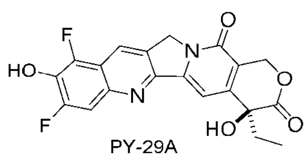
PY-29A ,

and

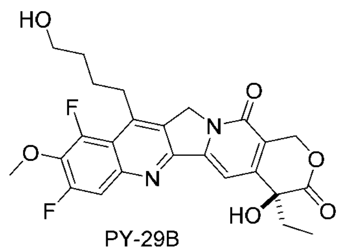
PY-29B .

Yet another aspect of the present invention provides a ligand-drug conjugate represented by general formula (B) or a pharmaceutically acceptable salt thereof;

$$Pc-\left[L'-L_2-L_1-Dr\right]_v$$

(B)

wherein:

Dr is selected from the following structures:

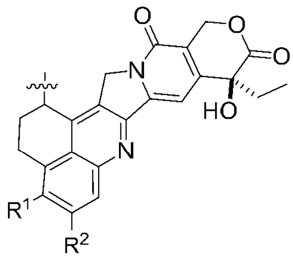
,
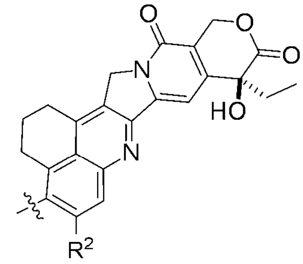
,
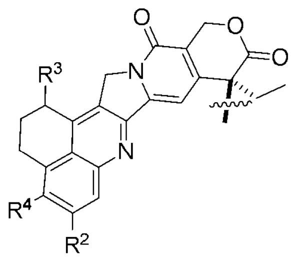
,
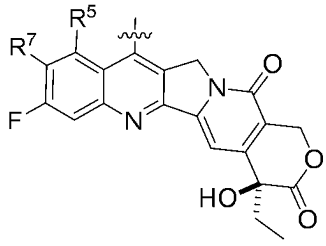
, and
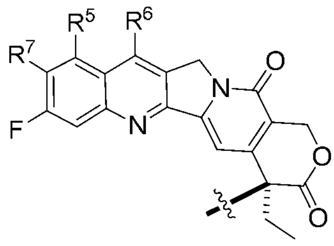
;

$R^1$ is selected from hydrogen, halogen, hydroxyl, amino, cyano, alkyl, alkoxy, alkenyl, alkynyl, cycloalkyl, $-NR^dR^e$, $-(CH_2)_m-OH$, $-(CH_2)_m-NR^dR^e$, $-OC(=O)NR^f-(CH_2)_m-OH$, $-OC(=O)NR^f-(CH_2)_m-NR^dR^e$, $-(CH_2)_m-C(=O)OH$, $-(CH_2)_m-C(=O)-NR^dR^e$, $-(CH_2)_m-C(=O)NR^f-(CH_2)_n-OH$, $-(CH_2)_m-C(=O)NR^f-(CH_2)_n-NR^dR^e$, $-NR^f-(CH_2)_m-OH$, $-NR^f-(CH_2)_m-NR^dR^e$, $-O-(CH_2)_m-OH$, $-O-(CH_2)_m-NR^dR^e$, $-NR^fC(=O)O-(CH_2)_m-OH$, $-NR^bC(=O)O-(CH_2)_m-NR^dR^e$, $-(CH_2)_m-NR^fC(=O)O-(CH_2)_n-OH-$, $-(CH_2)_m-NR^fC(=O)O-(CH_2)_n-NR^dR^e$, $-(CH_2)_m-OC(=O)NR^f-(CH_2)_n-NR^dR^e$, $-(CH_2)_m-OC(=O)NR^f-(CH_2)_n-OH$, $-(CH_2)_m-NR^fC(=O)-(CH_2)_n-NR^dR^e$, $-(CH_2)_m-NR^fC(=O)-(CH_2)_n-OH$, and $-(CH_2)_m-NR^fC(=O)-G-(CH_2)_n-OH$, wherein the alkyl, alkoxy, alkenyl, alkynyl, and cycloalkyl are optionally further substituted by one or more groups selected from halogen, deuterium, amino, alkyl, and hydroxyl; the $-(CH_2)_m-$ is optionally substituted by one or more deuterium or halogen groups; and G is selected from cycloalkylene, heterocyclylene, heteroarylene, and arylene;

$R^2$ is selected from hydrogen, halogen, hydroxyl, amino, cyano, alkyl, alkoxy, alkenyl, and alkynyl, wherein the alkyl, alkoxy, alkenyl, and alkynyl are optionally further substituted by one or more groups selected from halogen;

$R^3$ is selected from hydrogen, halogen, hydroxyl, amino, cyano, alkyl, alkoxy, alkenyl, alkynyl, $CH_2=$, $-NR^dR^e$, $-(CH_2)_m-OH$, $-(CH_2)_m-NR^dR^e$, $-OC(=O)NR^f-(CH_2)_m-OH$, $-OC(=O)NR^f-(CH_2)_m-NR^dR^e$, $-\ (CH_2)_m-C(=O)OH$, $-(CH_2)_m-C(=O)-NR^dR^e$, $-(CH_2)_m-C(=O)NR^f\ (CH_2)_n-OH$, $-(CH_2)_m-C(=O)NR^f-(CH_2)_n-NR^dR^e$, $-NR^f-(CH_2)_m-OH$, $-NR^f-(CH_2)_m-NR^dR^e$, $-NR^f-C(=O)R^d$, $-NR^f-C(=O)-(CH_2)_m-R^d$, $-\ NR^fC(=O)O-(CH_2)_m-R^d$, $-O-(CH_2)_m-OH$, $-O-(CH_2)_m-NR^dR^e$, $-NR^fC(=O)O-(CH_2)_m-OH$, $-\ NR^fC(=O)NR^d-(CH_2)_m-OH$, $-NR^fC(=O)NR^d-(CH_2)_m-O-(CH_2)_n-OH$, $-NR^fC(=O)O-(CH_2)_m-O-(CH_2)_n-OH$, $-NR^fC(=O)NR^d-(CH_2)_m-O-(CH_2)_n-NR^dR^e$, $-NR^fC(=O)O-(CH_2)_m-O-(CH_2)_n-NR^dR^e$, $-\ NR^fC(=O)NR^d-(CH_2)_m-NR^dR^e$, $-NR^fC(=O)O-(CH_2)_m-NR^dR^e$, $-(CH_2)_m-NR^fC(=O)O-(CH_2)_n-OH$, $-\ (CH_2)_m-NR^fC(=O)O-(CH_2)_n-NR^dR^e$, $-(CH_2)_m-OC(=O)NR^f-(CH_2)_n-NR^dR^e$, $-(CH_2)_m-OC(=O)NR^f-(CH_2)_n-OH$, $-(CH_2)_m-NR^fC(=O)-(CH_2)_n\ NR^dR^e$, and $-(CH_2)_m-NR^fC(=O)-(CH_2)_n-OH$, wherein the alkyl, alkoxy, alkenyl, and alkynyl are optionally further substituted by one or more groups selected from halogen, deuterium, amino, alkyl, and hydroxyl;

$R^4$ is selected from hydrogen, halogen, hydroxyl, carboxyl, amino, cyano, alkyl, alkoxy, alkenyl, alkynyl, cycloalkyl, $-NR^dR^e$, $-(CH_2)_m-OH$, $-(CH_2)_m-NR^dR^e$, $-OC(=O)NR^f-(CH_2)_m-OH$, $-OC(=O)NR^f-(CH_2)_m-NR^dR^e$, $-(CH_2)_m-C(=O)OH$,

$-(CH_2)_m-C(=O)-NR^dR^e$, $-(CH_2)_m-C(=O)NR^f-(CH_2)_n-OH$, $-(CH_2)_m-C(=O)NR^f-(CH_2)_n-NR^dR^e$, $-NR^f-(CH_2)_m-OH$, $-NR^f-(CH_2)_m-NR^dR^e$, $-O-(CH_2)_m-OH$, $-O-(CH_2)_m-NR^dR^e$, $-NR^fC(=O)O-(CH_2)_m-OH$, $-NR^bC(=O)O-(CH_2)_m-NR^dR^e$, $-(CH_2)_m-NR^fC(=O)O-(CH_2)_n-OH-$, $-(CH_2)_m-NR^fC(=O)O-(CH_2)_n-NR^dR^e$, $-(CH_2)_m-OC(=O)NR^f-(CH_2)_n-NR^dR^e$, $-(CH_2)_m-OC(=O)NR^f-(CH_2)_n-OH$, $-(CH_2)_m-NR^fC(=O)-(CH_2)_n-NR^dR^e$, $-(CH_2)_m-NR^fC(=O)-(CH_2)_n-OH$, and $-(CH_2)_m-NR^fC(=O)-G-(CH_2)_n-OH$, wherein the alkyl, alkoxy, alkenyl, alkynyl, and cycloalkyl are optionally further substituted by one or more groups selected from halogen, deuterium, amino, alkyl, and hydroxyl; the $-(CH_2)_m-$ is optionally substituted by one or more deuterium or halogen groups; and G is selected from cycloalkylene, heterocyclylene, heteroarylene, and arylene;

$R^5$ is selected from hydrogen, halogen, hydroxyl, amino, cyano, alkyl, alkoxy, alkenyl, and alkynyl, wherein the alkyl, alkoxy, alkenyl, and alkynyl are optionally further substituted by one or more groups selected from halogen;

$R^6$ is selected from hydrogen, halogen, hydroxyl, amino, cyano, alkyl, alkoxy, alkenyl, and alkynyl, wherein the alkyl, alkoxy, alkenyl, and alkynyl are optionally further substituted by one or more groups selected from halogen;

$R^7$ is selected from hydrogen, halogen, hydroxyl, amino, cyano, alkyl, alkoxy, alkenyl, and alkynyl, wherein the alkyl, alkoxy, alkenyl, and alkynyl are optionally further substituted by one or more groups selected from halogen;

$R^d$ and $R^e$ are each independently selected from hydrogen and alkyl;

$R^f$ is selected from hydrogen, alkyl, $-C(O)R^c$, $-S(O)R^c$, and $-S(O)_2R^c$, wherein the $C_1$-$C_6$ alkyl is optionally further substituted by $C_3$-$C_6$ cycloalkyl; and $R^c$ is selected from hydrogen, hydroxyl, and alkyl;

$L_1$ is selected from a bond, $-(CH_2)_m-*$, $-O-*$, $-NR^a-*$, $-(CH_2)_m-O-*$, $-(CH_2)_m-NR^a-*$, $-OC(=O)NR^b-(CH_2)_m-O-*$, $-OC(=O)NR^b-(CH_2)_m-NR^a-*$, $-(CH_2)_m-C(=O)O-*$, $-(CH_2)_m-C(=O)NR^a-*$, $-(CH_2)_m-C(=O)NR^b-(CH_2)_n-O-*$, $-(CH_2)_m-C(=O)NR^b-(CH_2)_n-NR^a-*$, $-NR^b-(CH_2)_m-O-*$, $-NR^d-(CH_2)_m-NR^a-*$, $-O-(CH_2)_m-O-*$, $-O-(CH_2)_m-NR^a-*$, $-NR^bC(=O)O-(CH_2)_m-O-*$, $-NR^bC(=O)O-(CH_2)_m-NR^a-*$, $-(CH_2)_m-NR^bC(=O)O-(CH_2)_n-O-*$, $-(CH_2)_m-NR^bC(=O)O-(CH_2)_n-NR^a-*$, $-(CH_2)_m-OC(=O)NR^b-(CH_2)_n-NR^a-*$, $-(CH_2)_m-OC(=O)NR^b-(CH_2)_n-O-*$, $-(CH_2)_m-NR^bC(=O)-(CH_2)_n-NR^a-*$, and $-(CH_2)_m-NR^bC(=O)-(CH_2)_n-O-*$, wherein * is a connection site with $L_2$;

$R^a$ and $R^d$ are each independently selected from hydrogen, alkyl, $-C(O)R^c$, $-S(O)R^c$, and $-S(O)_2R^c$, wherein the alkyl is optionally further substituted by cycloalkyl; and $R^c$ is selected from hydrogen, hydroxyl, and alkyl;

$L_2$ is selected from a bond,

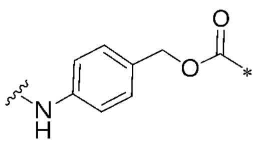,
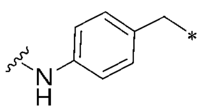,
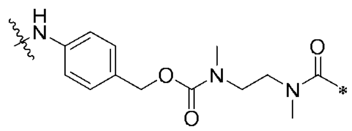,

and

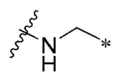,

wherein * is a connection site with $L_1$;

L' is 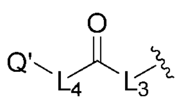 or 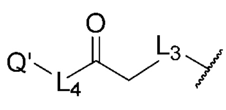;

Q' is selected from

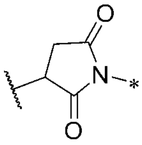, 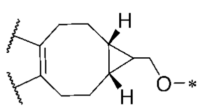, 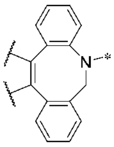, 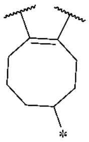, 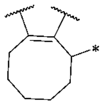, 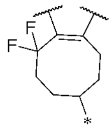, 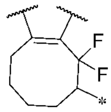,

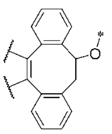, 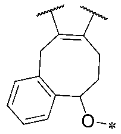, 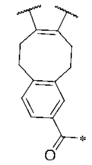, 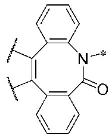, 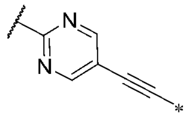, 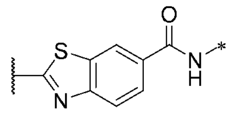,

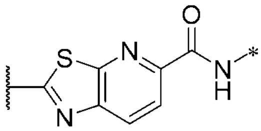, 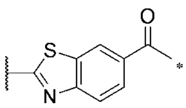, and 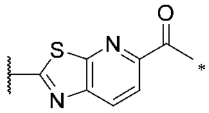;

wherein, * is a connection site with $L_4$, and ∿ is a connection site with Pc;

$L_3$ is an amino acid residue formed by two or more amino acids, and $L_3$ optionally comprises one or more of the following structures, and $L_6$ is selected from one or more of the following structures:

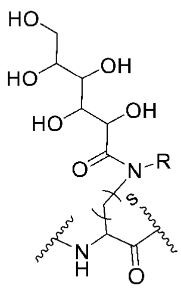, 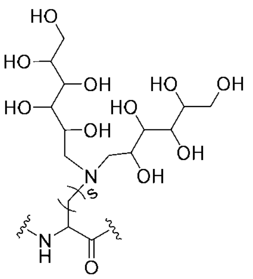, 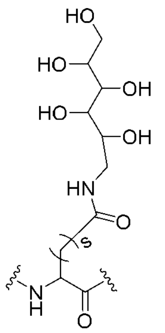, 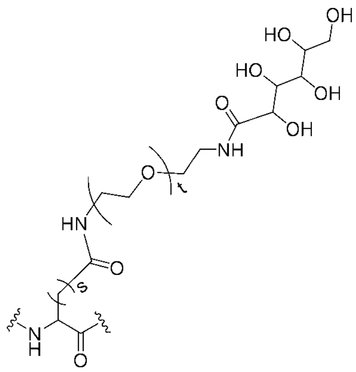,

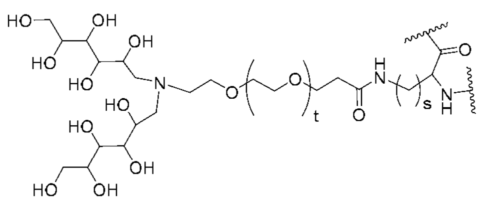, 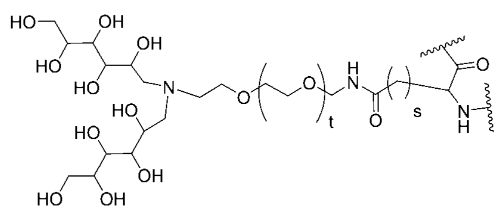,

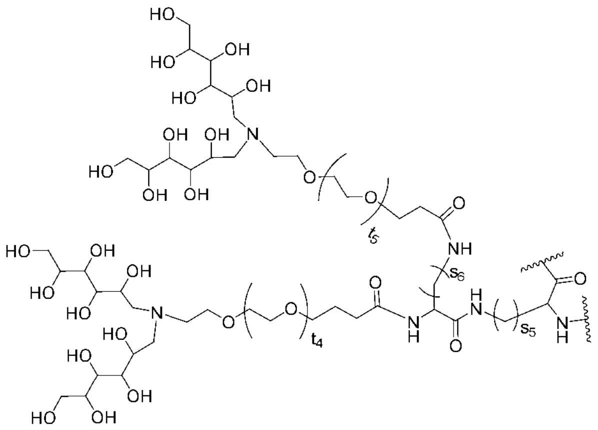 , 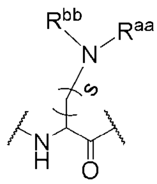 ,

or

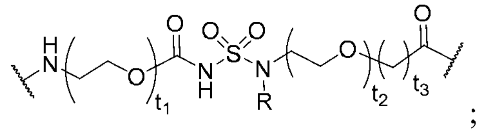 ;

wherein R, $R^{aa}$, and $R^{bb}$ are each independently selected from hydrogen and alkyl;

$L_4$ is

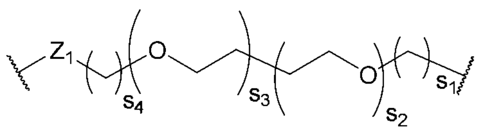 ;

$Z_1$ is selected from a bond, $-(CH_2)_p-$, $-(C_2H_4O)_q-$, $-(CH_2)_p-C(O)NH-$, $-(CH_2)_p-O-(CH_2)_p-C(O)NH-$, $-(CH_2)_p-C(O)-L_6-NH-$, $-(CH_2)_p-O-(CH_2)_p-C(O)-L_6-NH-$, -C(O)NH-, -C(O)O-, -C(O)-, -OC(O)-, -NH-, -O-, and -OC(O)NH-;

m is an integer from 1 to 6;

n is an integer from 1 to 6;

s is an integer from 1 to 6;

t is an integer from 0 to 10;

$s_1$, $s_2$, $s_3$, and $s_4$ are each independently an integer from 0 to 10; preferably, an integer from 0 to 8, or an integer from 0 to 6; preferably, an integer from 0 to 4; more preferably, an integer from 0 to 2, or an integer from 1 to 2;

$s_5$ and $s_6$ are each independently an integer from 1 to 6;

$t_1$ is an integer from 1 to 6;

$t_2$ is an integer from 0 to 6;

$t_3$ is an integer from 1 to 6;

$t_4$ is an integer from 0 to 10;

$t_5$ is an integer from 0 to 10;

p is an integer from 1 to 10;

q is an integer from 1 to 10;

v is from 1 to 10, and v is a decimal or an integer;

Pc is an antibody or an antigen-binding fragment thereof, or a modified antibody;

the modified antibody has a Pc'-$((L_5)_w\text{-}F)_x$ structure, wherein:

Pc' is an antibody;

$L_5$ is a linker;

w is 0 or 1;

F is a clickable probe or a sulfhydryl group or a precursor thereof that can be connected to Q' after a reaction such as a metal-free click reaction, and preferably, F is an azido group; and

x is an integer from 1 to 8;

wherein $Z_1$ is connected to Q'.

In a preferred embodiment, in the ligand-drug conjugate represented by the general formula (B) or the pharmaceutically acceptable salt thereof according to the present invention,

$L_3$ is selected from:

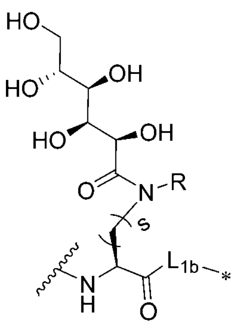
,
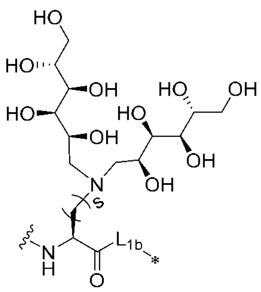
,
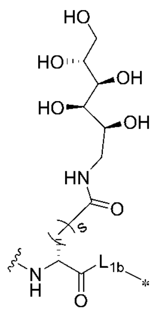
,
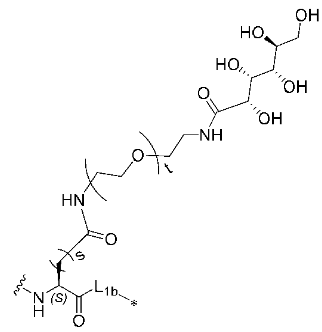
,

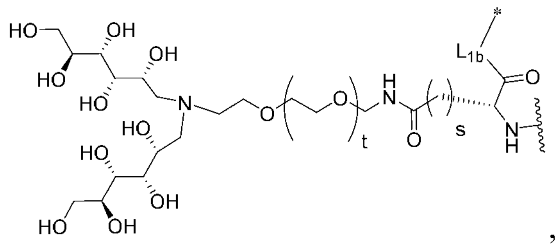
,
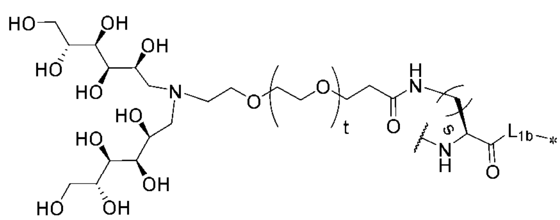
;

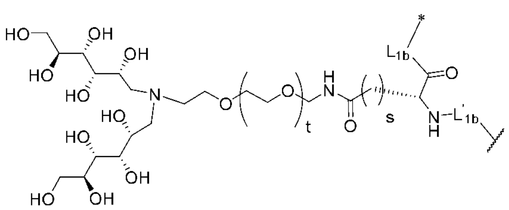
,
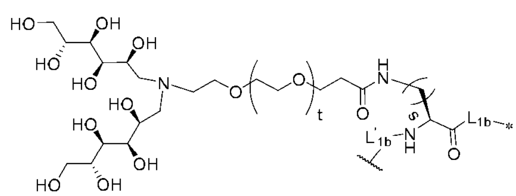
,

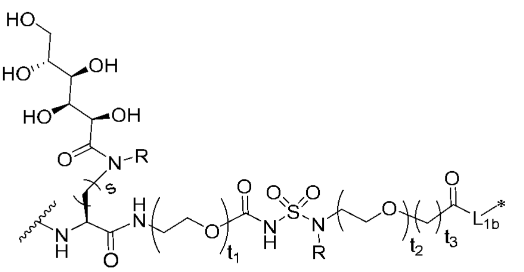
,
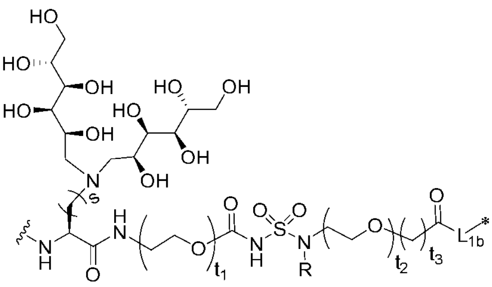
,

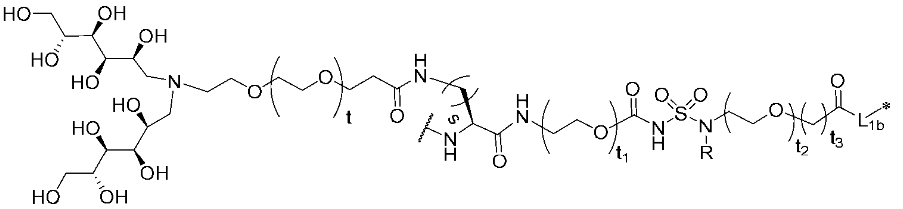
,
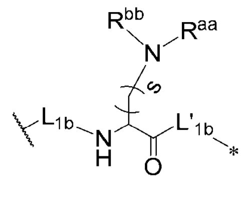
,

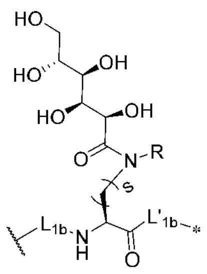
, and
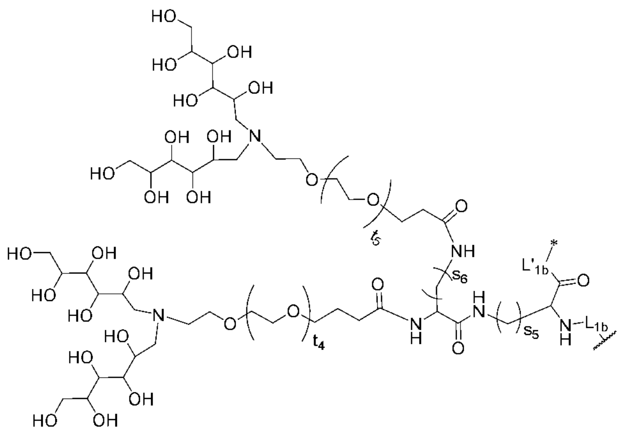
,

wherein R is selected from hydrogen and $C_1$-$C_6$ alkyl, and is preferably hydrogen;

$R^{aa}$ and $R^{bb}$ are each independently selected from $C_1$-$C_6$ alkyl;

s is an integer from 1 to 6; preferably, an integer from 2 to 6; more preferably, an integer from 2 to 4;

$s_5$ and $s_6$ are each independently an integer from 1 to 6; preferably, an integer from 2 to 6; more preferably, an integer from 2 to 4;

t is an integer from 0 to 10; preferably, an integer from 2 to 8; more preferably, an integer from 3 to 7;

$t_1$ is an integer from 1 to 6; preferably, an integer from 1 to 4; more preferably, an integer from 2 to 4; more preferably 1 or 2;

$t_2$ is an integer from 0 to 6; preferably, an integer from 1 to 4; more preferably 1 or 2;

$t_3$ is an integer from 1 to 6; preferably, an integer from 1 to 4; more preferably 1 or 2;

$t_4$ is an integer from 0 to 10;

$t_5$ is an integer from 0 to 10;

* is a connection site with $L_2$;

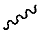 is a connection site with carbonyl or methylene;

$L_{1b}$ and $L'_{1b}$ are each independently an amino acid residue formed by one or more amino acids selected from glycine, phenylalanine, citrulline, leucine, isoleucine, alanine, valine, asparagine, glutamine, arginine, glutamic acid, and lysine; preferably an amino acid residue formed by two or more amino acids selected from glycine, phenylalanine, citrulline, valine, lysine, glutamine, glutamic acid, leucine, and alanine; and

preferably, $L_{1b}$ and $L'_{1b}$ are each independently selected from -Gly-*, -Val-*, -Gly-Phe-Gly-*, -Phe-Gly-*, -Gly-Val-Cit-*, -Val-Cit-*, -Gly-Val-Arg-*, -Val-Arg-*, -Gly-Val-Ala-*, -Val-Ala-*, -Gly-Phe-*, -Phe-Gly-*, -Gly-Gly-Gly-*, Gly-Gly-*, -Gly-Val-Gly-*, -Gly-Ala-Gly-*, -Gly-Phe-Cit-*, - Gly-Phe-Val-*, -Gly-Phe-Ala-*, -Gly-Phe-Lys-*, -Phe-Lys-*, -Gly-Val-*, -Gly-Cit-*, -Gly-Ala-*, - Gly-Gly-Lys-*, Gly-Lys'-*, -Ala-Ala-Ala-*, -Gln-Val-Ala-*, -Gln-Val-Cit-*, -Asp-Val-Ala-*, and -Asp-Val-Cit-*; preferably, -Gly-Phe-Gly-*, -Gly-Val-Cit-*, -Val-Cit-*, -Gly-Val-Ala-*, -Val-Ala-*, - Gly-Phe-Gly-*, -Phe-Gly-*, -Gly-Phe-Lys-, -Phe-Lys- *, -Gln-Val-Ala-*, -Gln-Val-Cit-*, -Asp-Val-Ala-*, and -Asp-Val-Cit-, wherein * is a

connection site with $L_2$.

In a preferred embodiment, in the ligand-drug conjugate represented by the general formula (B) or the pharmaceutically acceptable salt thereof according to the present invention,

L' is

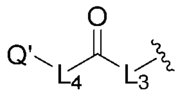 or 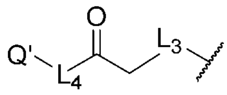;

Q' is selected from

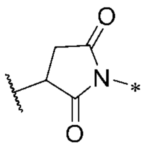, 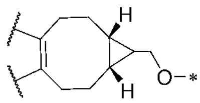, 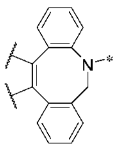, 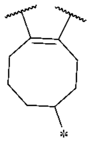, 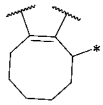, 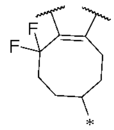, 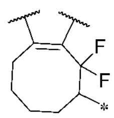,

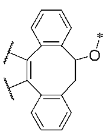, 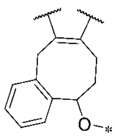, 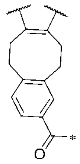, 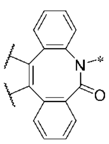, 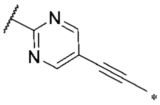, 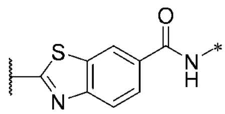, 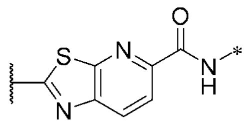,

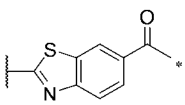, and 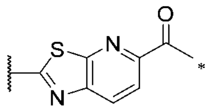,

Wherein * is a connection site with $L_4$, and ∿ is a connection site with Pc;

$L_3$ is an amino acid residue formed by two or more amino acids selected from glycine, phenylalanine, citrulline, leucine, isoleucine, alanine, valine, asparagine, glutamine, arginine, glutamic acid, and lysine, or is preferably an amino acid residue formed by two or more amino acids selected from glycine, phenylalanine, citrulline, valine, lysine, glutamine, glutamic acid, leucine, and alanine;

$L_4$ is

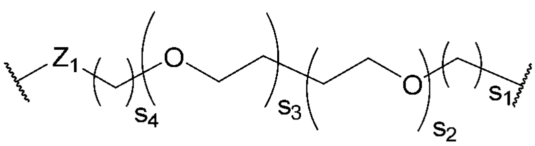;

$Z_1$ is selected from a bond, $-(CH_2)_p-$, $-(CH_2)_p-C(O)NH-$, $-(CH_2)_p-O-(CH_2)_p-C(O)NH-$, $-(CH_2)_p-C(O)-L_6-NH-$, $-(CH_2)_p-O-(CH_2)_p-C(O)-L_6-NH-$, -C(O)NH-, -C(O)O-, -C(O)-, -OC(O)-, -NH-, -O-, and - OC(O)NH-;

$s_1$ is an integer from 1 to 6; preferably, an integer from 2 to 6;

$s_2$ is an integer from 1 to 10; preferably, an integer from 2 to 10;

$s_3$ is 0;

$s_4$ is 0;

p is an integer from 1 to 10; preferably, an integer from 1 to 6; more preferably, an integer from 2 to 4, most preferably, an integer from 2 to 3;

$L_6$ is selected from

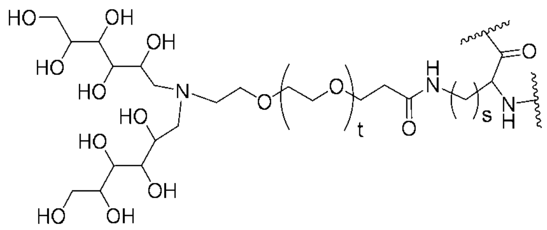

and

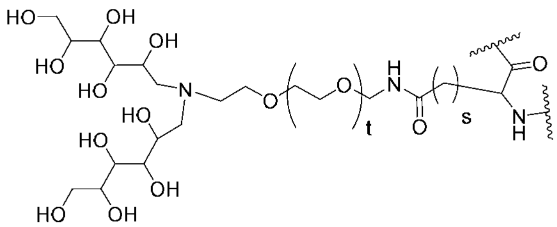

;

s is an integer from 1 to 6; preferably, an integer from 2 to 6; more preferably, an integer from 2 to 4;

t is an integer from 0 to 10; preferably, an integer from 2 to 8; more preferably, an integer from 3 to 7; and

$L_6$ is preferably

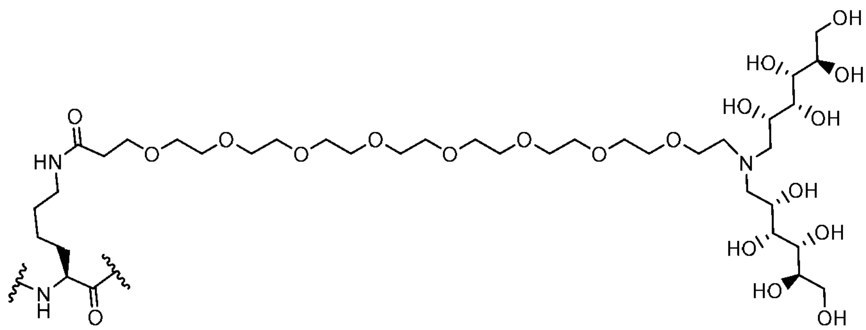

.

In a specific embodiment, in the ligand-drug conjugate represented by the general formula (B) or the pharmaceutically acceptable salt thereof according to the present invention, $L_5$ is

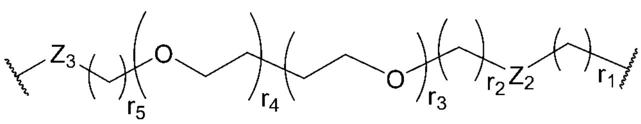

;

$Z_2$ and $Z_3$ are each independently selected from -C(O)NH-, -C(O)O-, -C(O)-, -OC(O)-, -NH-, -O-, and -OC(O)NH-, preferably -C(O)NH-;

$r_1$ is an integer from 1 to 8; preferably, an integer from 1 to 6; more preferably, an integer from 1 to 3;

$r_2$ is an integer from 1 to 6; preferably, an integer from 1 to 4; more preferably, an integer from 1 to 2;

$r_3$ is an integer from 0 to 6; preferably, an integer from 0 to 4; more preferably, an integer from 0 to 2;

$r_4$ is an integer from 0 to 6; preferably, an integer from 0 to 4; more preferably, an integer from 0 to 2; and

$r_5$ is an integer from 1 to 6; preferably, an integer from 1 to 4; more preferably, an integer from 1 to 2.

In another specific embodiment, in the ligand-drug conjugate represented by the general formula (B) or the pharmaceutically acceptable salt thereof according to the present invention, when w is 0, F is sulfhydryl; or when w is 1, F is a clickable probe that can be connected to Q' after a reaction such as a metal-free click reaction; and F is preferably an azido group.

In another specific embodiment, in the ligand-drug conjugate represented by the general formula (B) or the pharmaceutically acceptable salt thereof according to the present invention,
Pc is a modified antibody and the modified antibody has a structure:

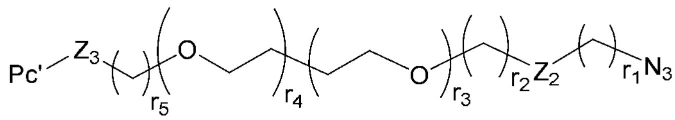

wherein:

Pc' is an antibody;

$Z_2$ and $Z_3$ are each independently selected from -C(O)NH-, -C(O)O-, -C(O)-, -OC(O)-, -NH-, -O-, and -OC(O)NH-, preferably -C(O)NH-;

$r_1$ is an integer from 1 to 8; preferably, an integer from 1 to 6; more preferably, an integer from 1 to 3;

$r_2$ is an integer from 1 to 6; preferably, an integer from 1 to 4; more preferably, an integer from 1 to 2;

$r_3$ is an integer from 0 to 6; preferably, an integer from 0 to 4; more preferably, an integer from 0 to 2;

$r_4$ is an integer from 0 to 6; preferably, an integer from 0 to 4; more preferably, an integer from 0 to 2;

$r_5$ is an integer from 1 to 6; preferably, an integer from 1 to 4; more preferably, an integer from 1 to 2.

In a preferred embodiment, in the ligand-drug conjugate represented by the general formula (B) or the pharmaceutically acceptable salt thereof according to the present invention, Pc is a modified antibody, and Pc-Q' is selected from:

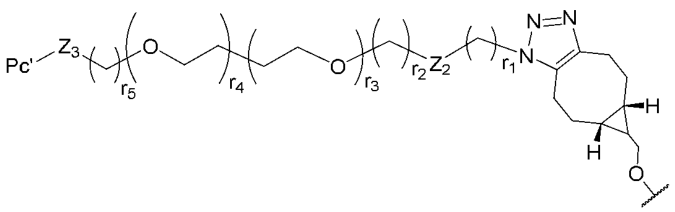

,

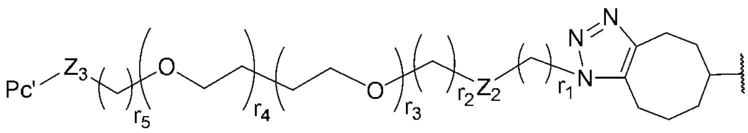

,

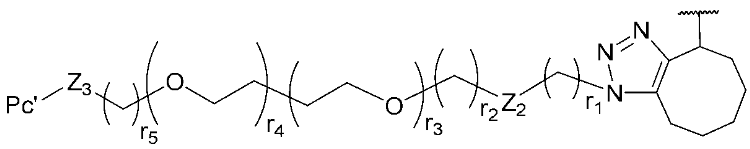

,

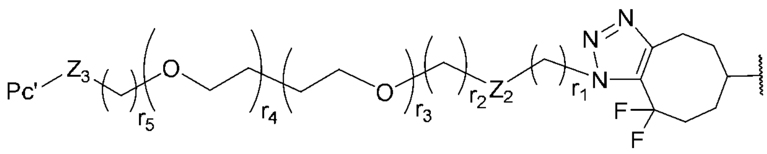

,

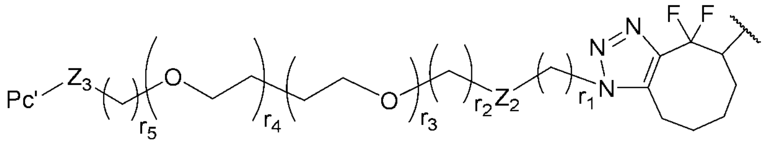

,

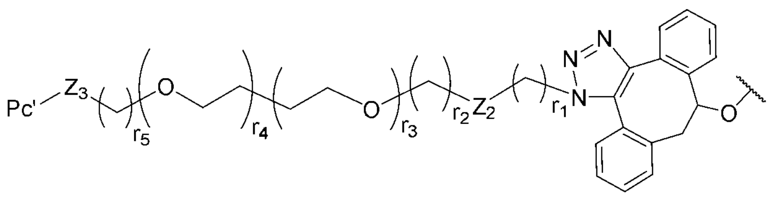

,

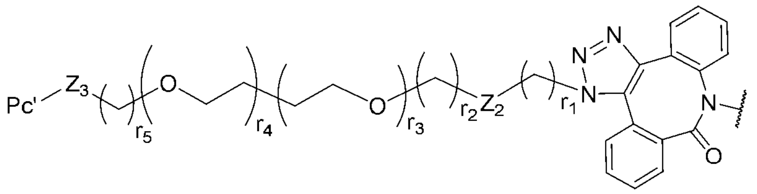

,

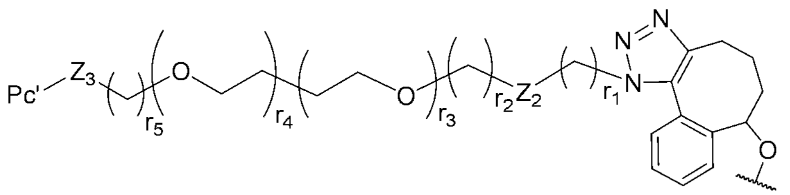

,

and

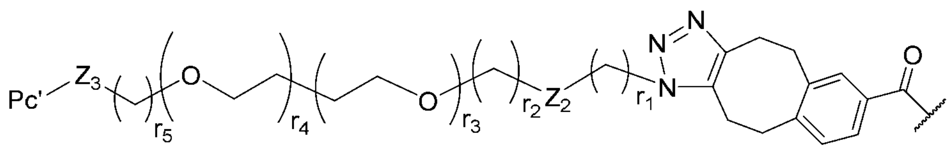

,

wherein Pc' is an antibody;

$Z_2$ and $Z_3$ are each independently selected from -C(O)NH-, -C(O)O-, -C(O)-, -OC(O)-, -NH-, -O-, and -OC(O)NH-, preferably -C(O)NH-;

$r_1$ is an integer from 1 to 8; preferably, an integer from 1 to 6; more preferably, an integer from 1 to 3;

$r_2$ is an integer from 1 to 6; preferably, an integer from 1 to 4; more preferably, an integer from 1 to 2;

$r_3$ is an integer from 0 to 6; preferably, an integer from 0 to 4; more preferably, an integer from 0 to 2;

$r_4$ is an integer from 0 to 6; preferably, an integer from 0 to 4; more preferably, an integer from 0 to 2; and

$r_5$ is an integer from 1 to 6; preferably, an integer from 1 to 4; more preferably, an integer from 1 to 2.

In a preferred embodiment, the ligand-drug conjugate represented by the general formula (B) or the pharmaceutically acceptable salt thereof according to the present invention is selected from:

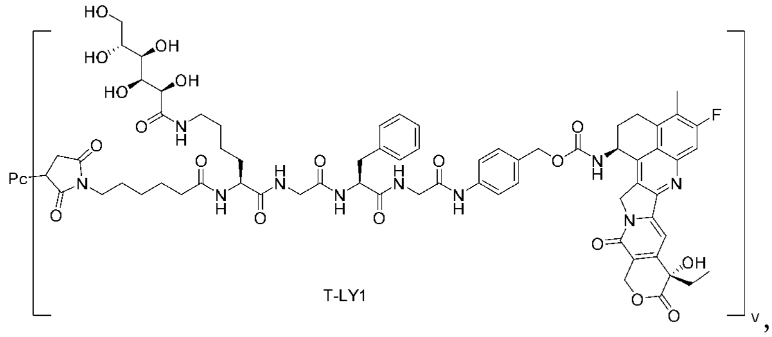

T-LY1

,

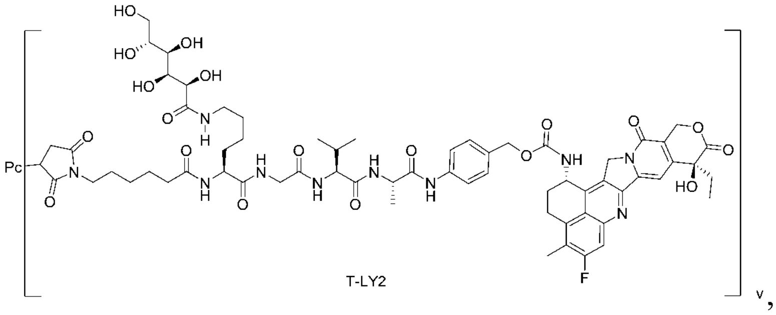

T-LY2

,

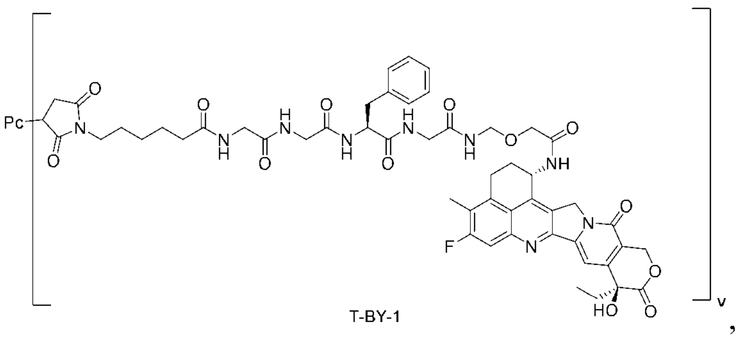

T-BY-1

,

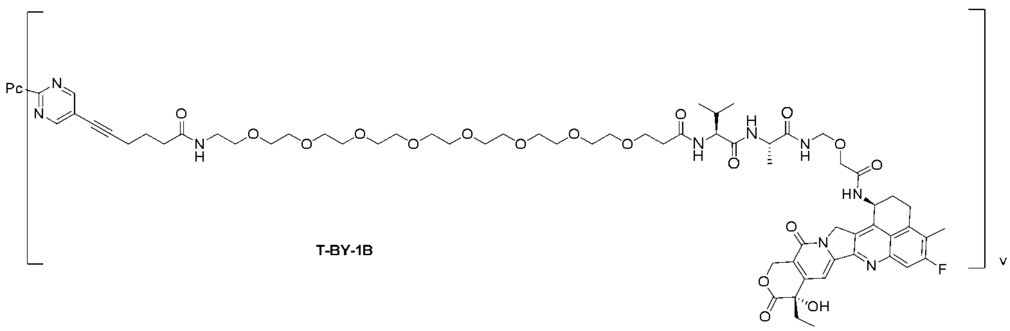

**T-BY-1B**

,

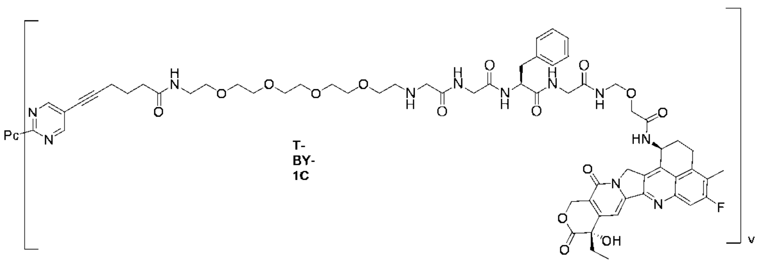

T-BY-1C

,

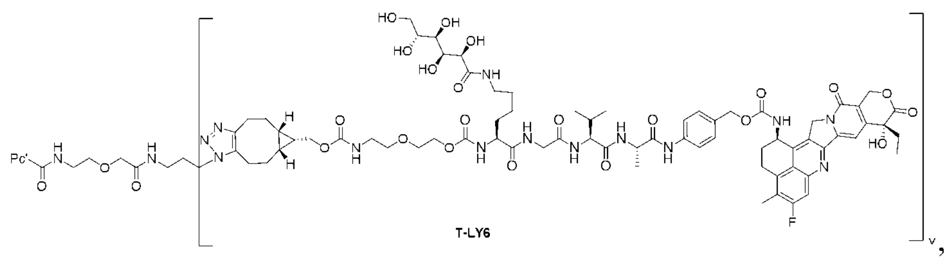

T-LY6

,

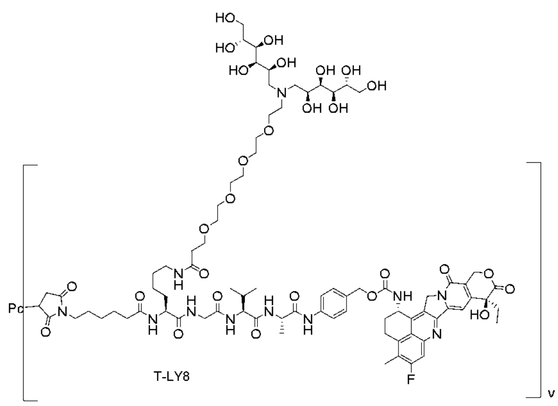

T-LY8

,

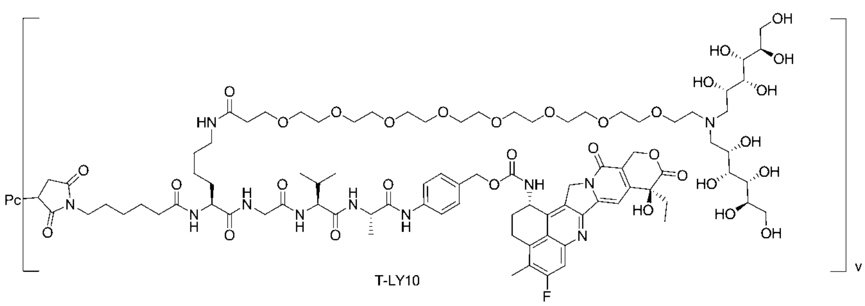

T-LY10

,

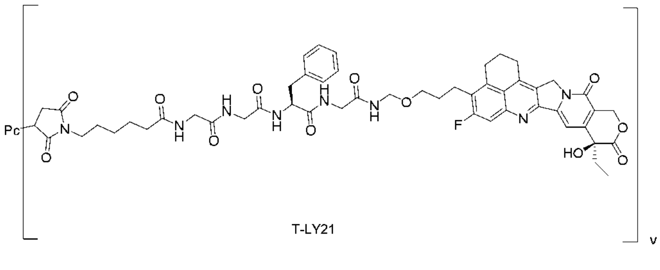

T-LY21

,

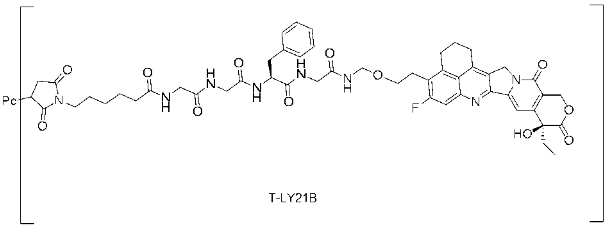

T-LY21B

v,

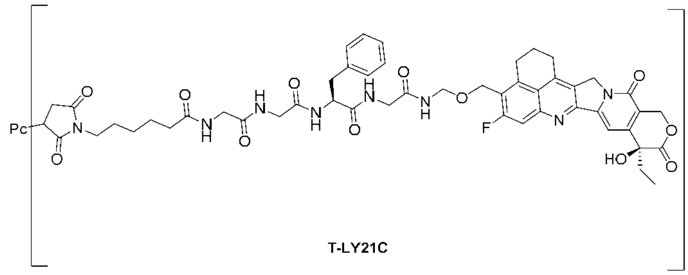

**T-LY21C**

v,

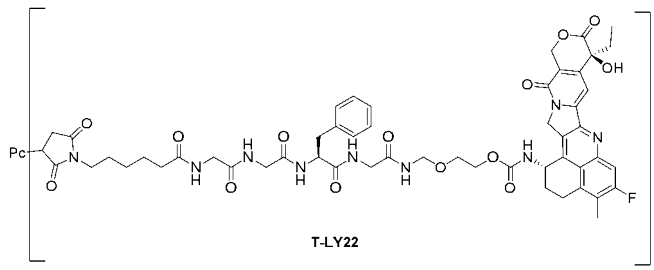

**T-LY22**

v,

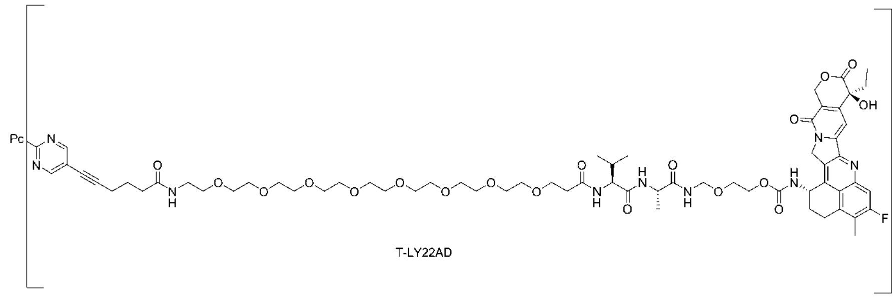

T-LY22AD

v,

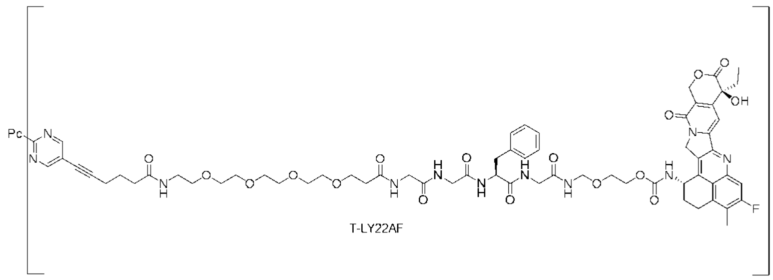

T-LY22AF

v,

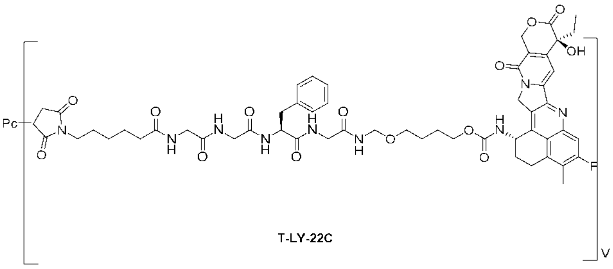

T-LY-22C

,

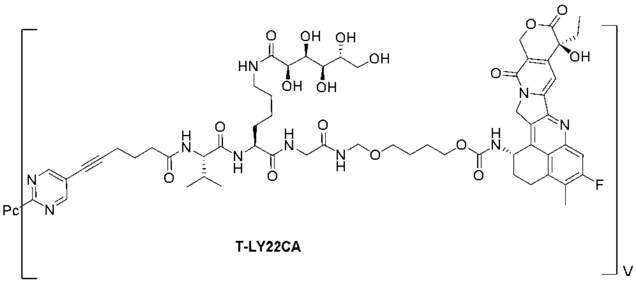

T-LY22CA

,

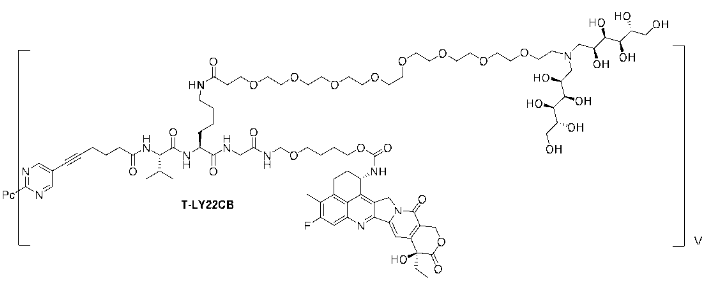

T-LY22CB

,

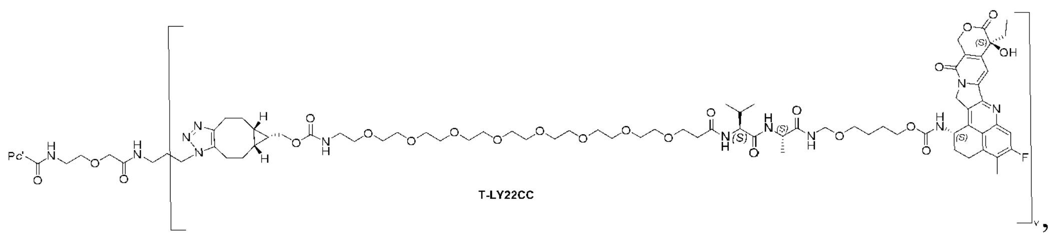

T-LY22CC

,

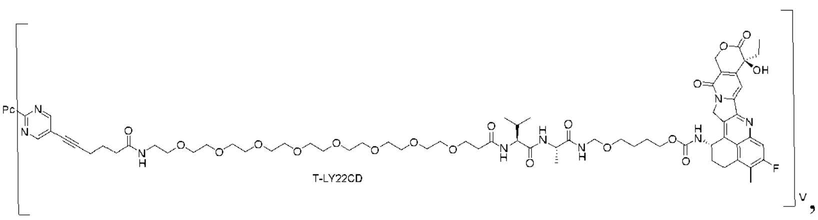

T-LY22CD

,

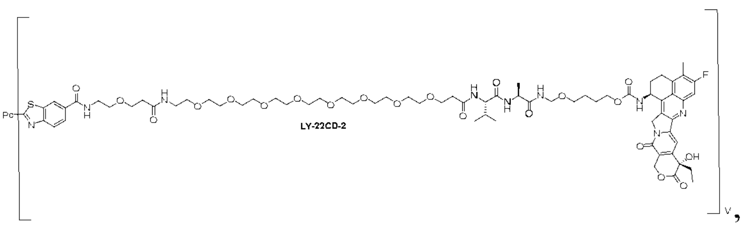

LY-22CD-2

,

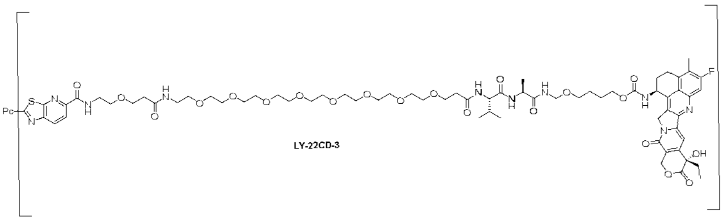

LY-22CD-3

,

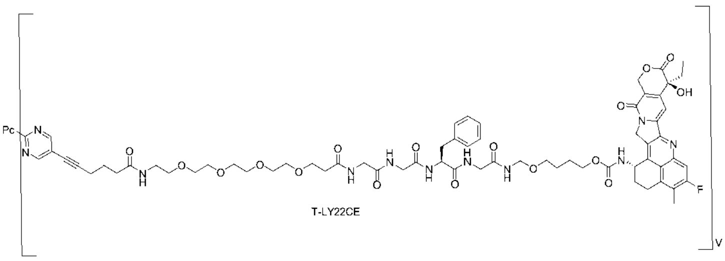

T-LY22CE

,

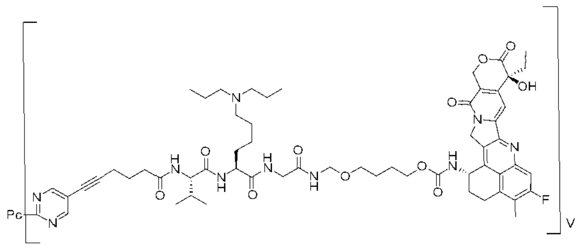

T-LY22CF

,

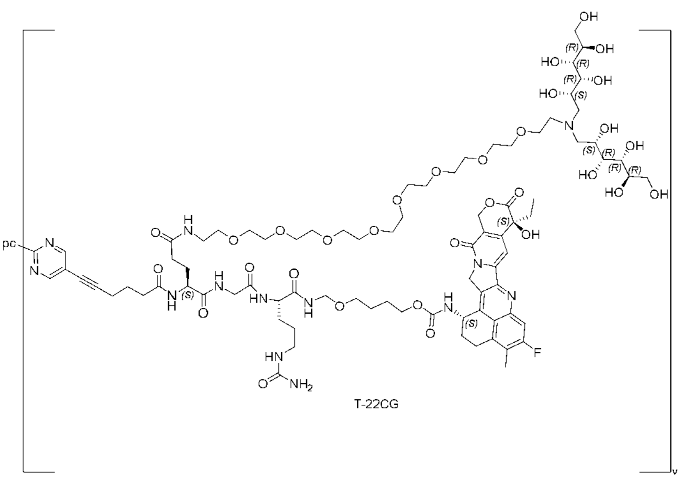

T-22CG

,

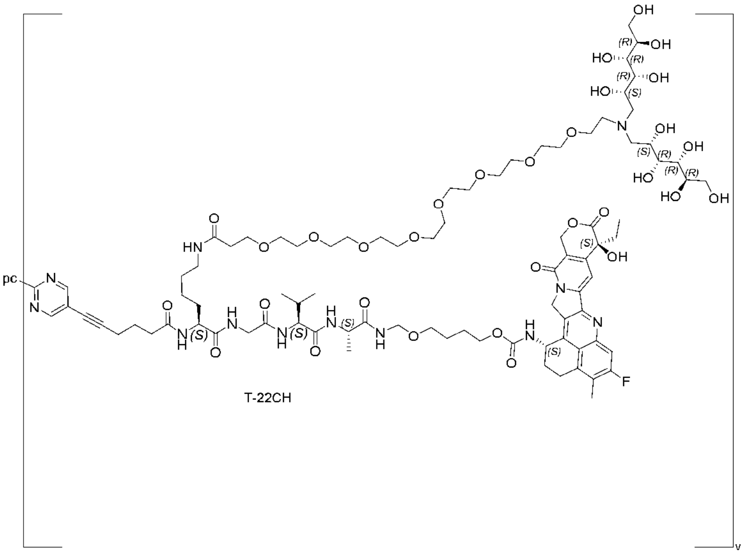

T-22CH

,

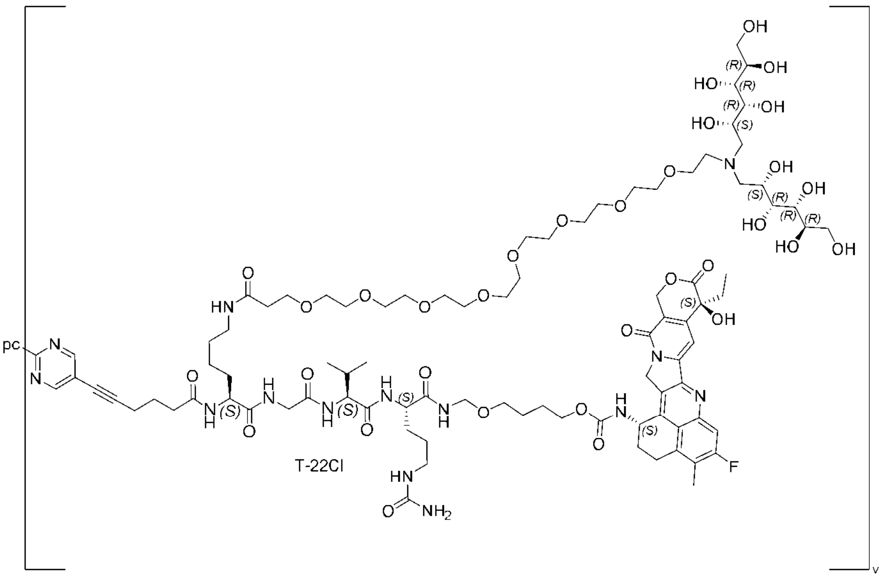

T-22CI

,

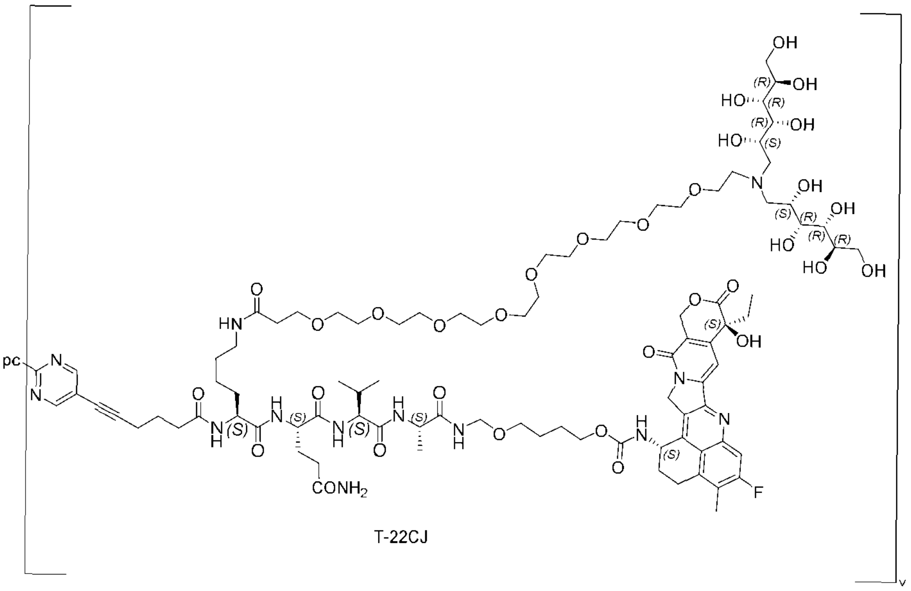

T-22CJ

,

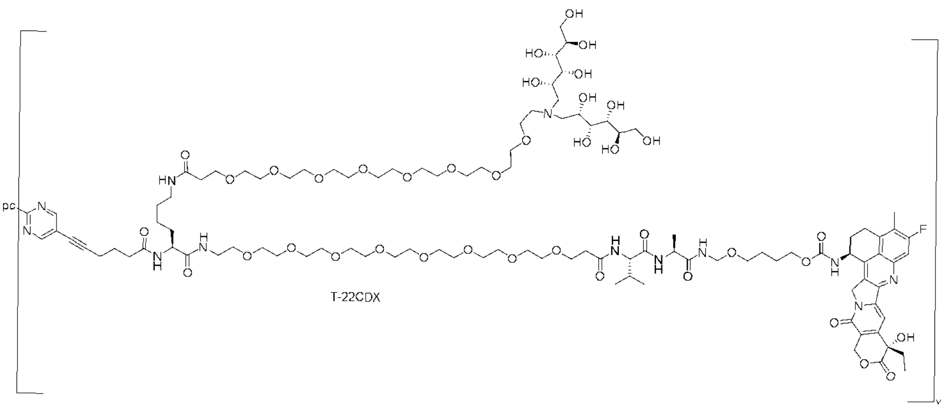

T-22CDX

,

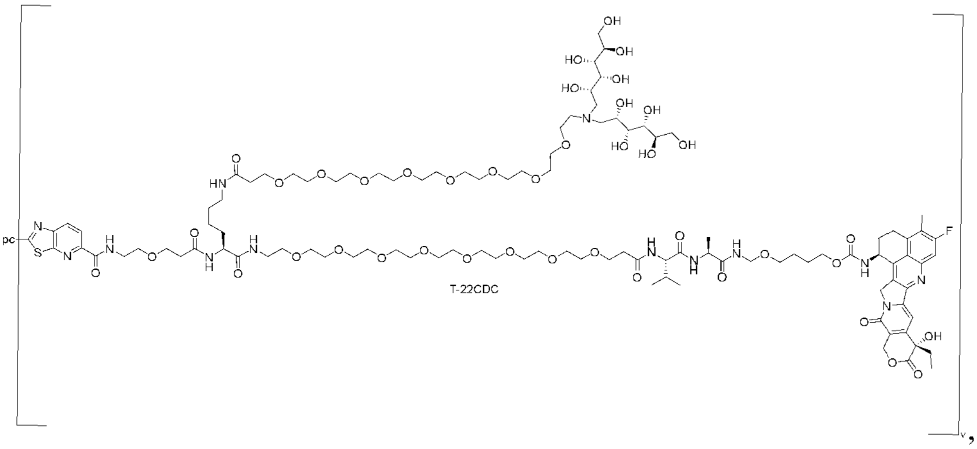

T-22CDC

,

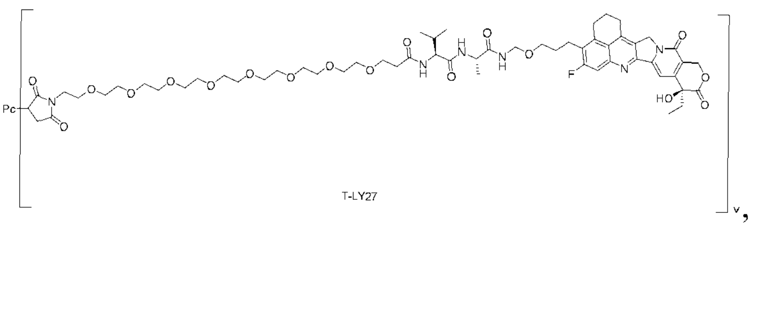

T-LY27

,

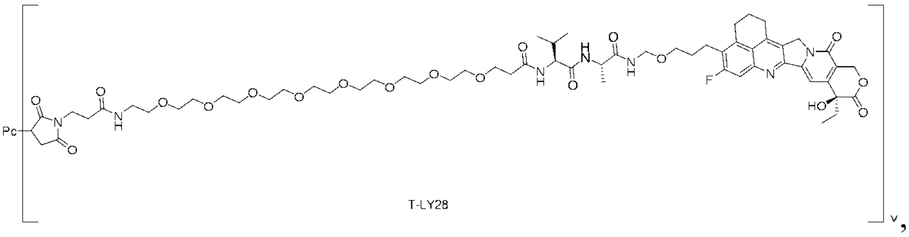

T-LY28

,

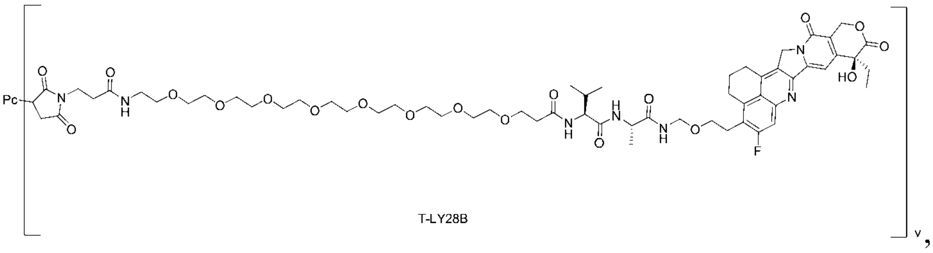

T-LY28B

,

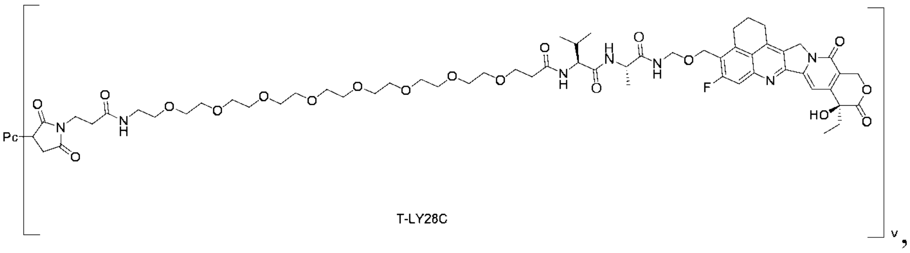

T-LY28C

,

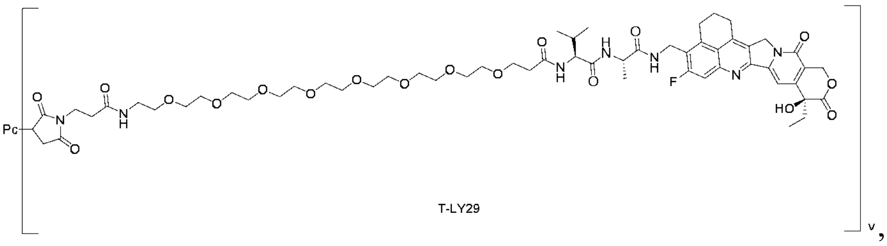

T-LY29

,

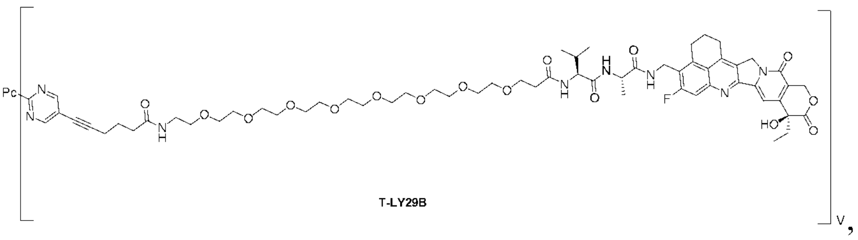

T-LY29B

,

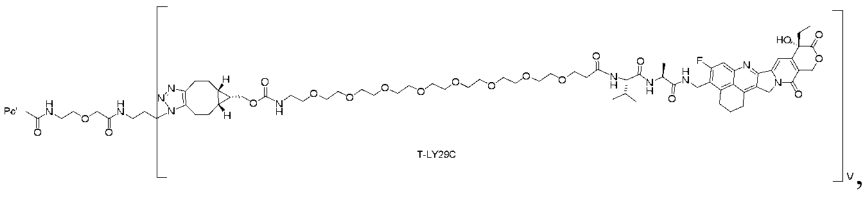

T-LY29C

,

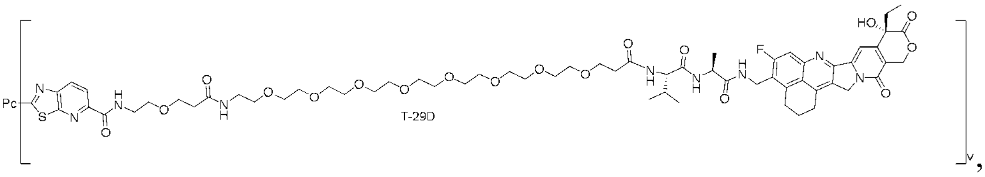

T-29D

,

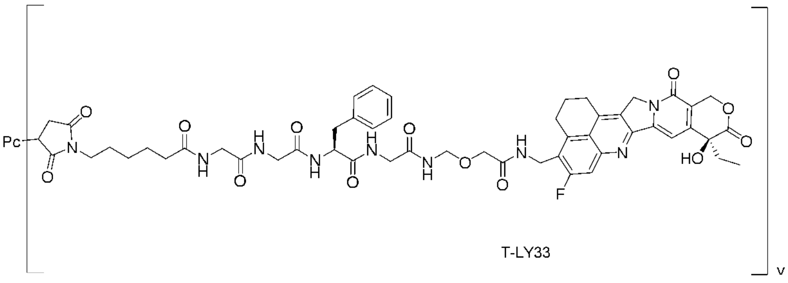

T-LY33

,

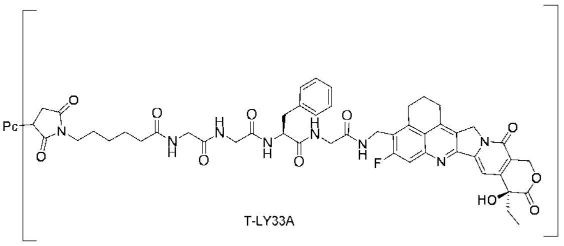

T-LY33A

,

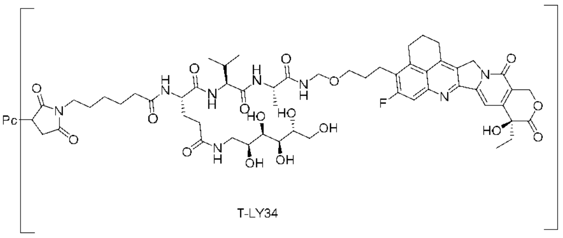

T-LY34

,

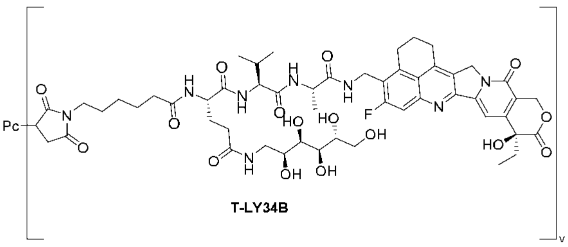

T-LY34B

,

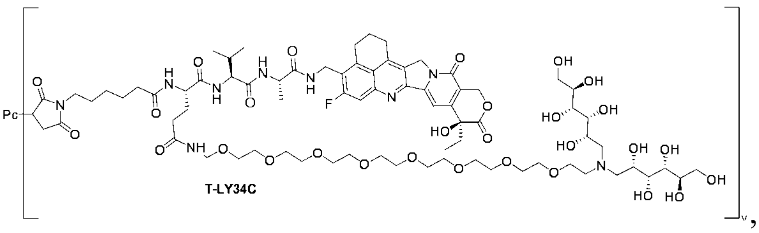

T-LY34C

,

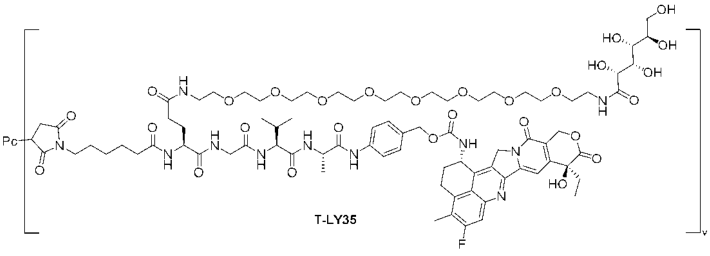

T-LY35

,

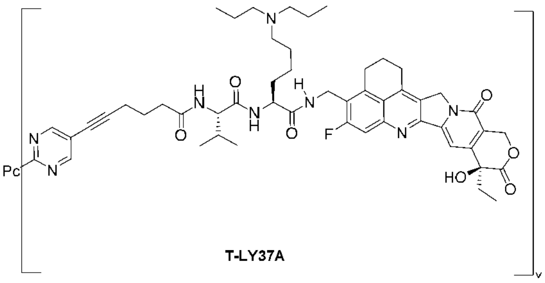

T-LY37A

,

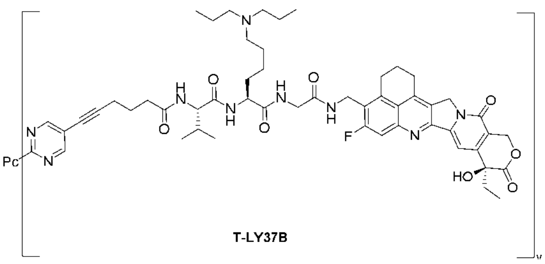

T-LY37B

,

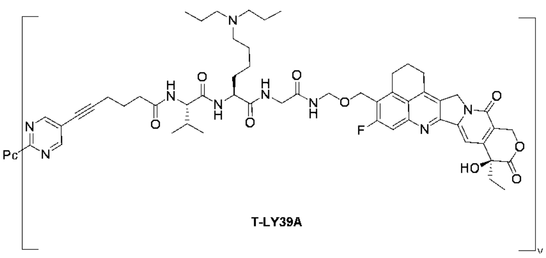

T-LY39A

,

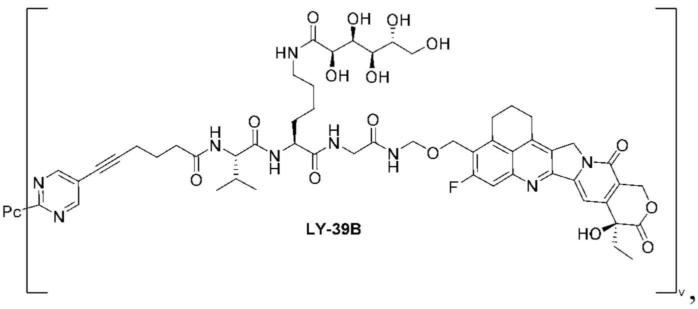

LY-39B

,

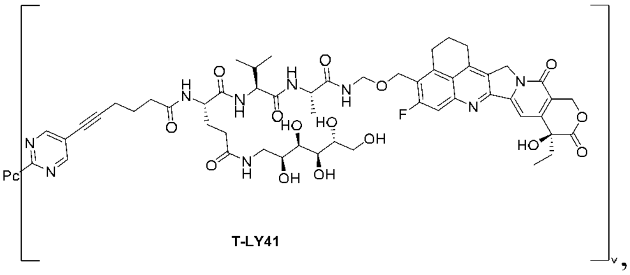

T-LY41

,

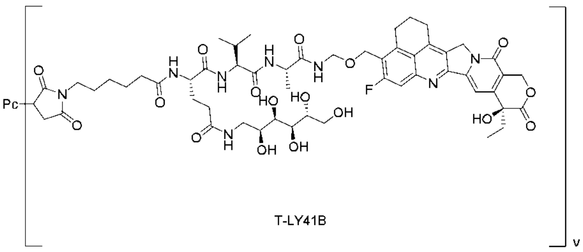

T-LY41B

,

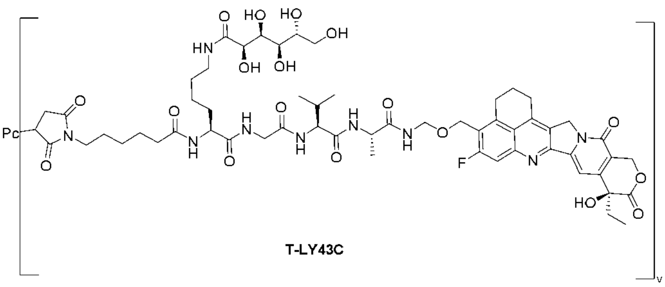

T-LY43C

,

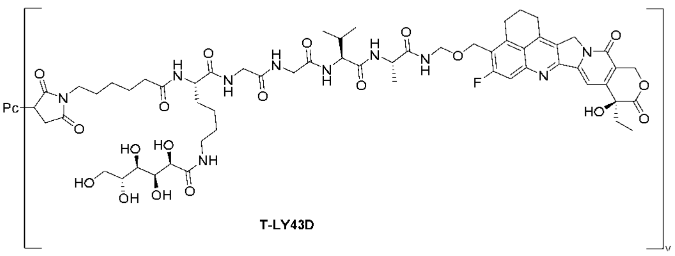

T-LY43D

,

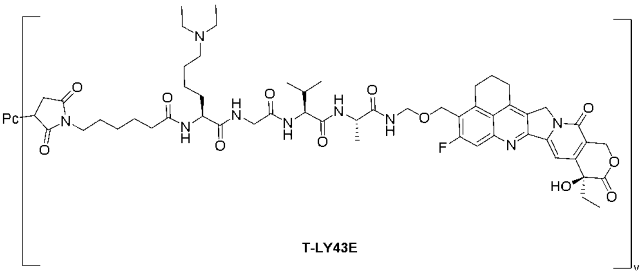

T-LY43E

,

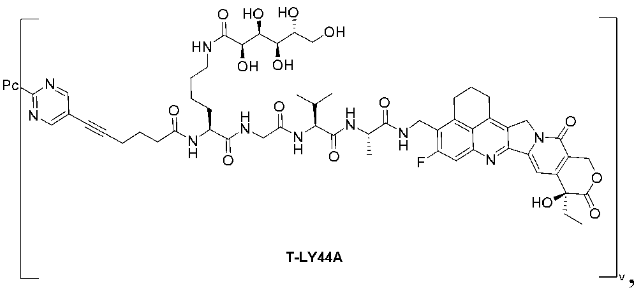

T-LY44A ,

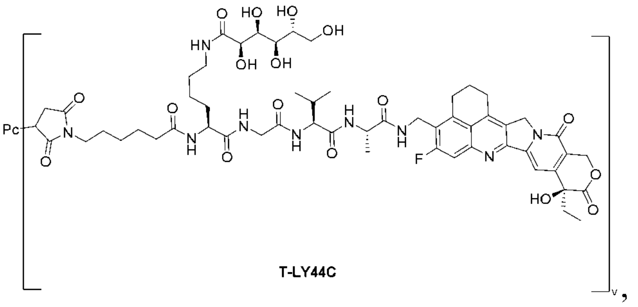

T-LY44C ,

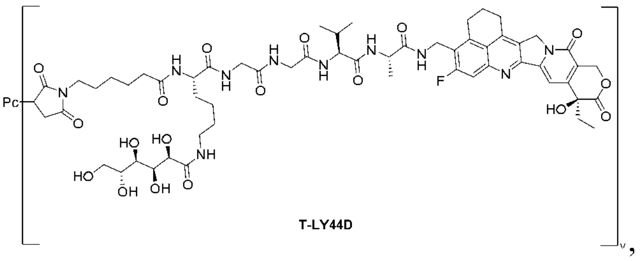

T-LY44D ,

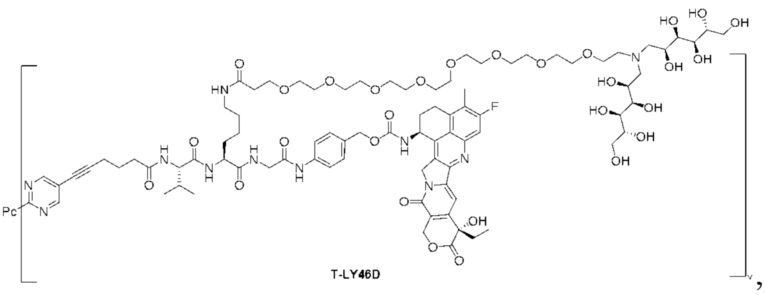

T-LY46D ,

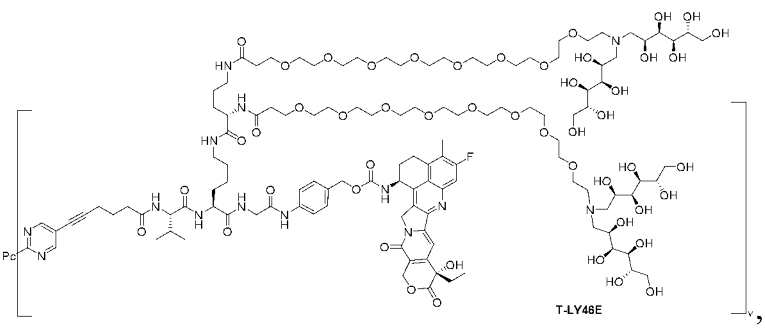

T-LY46E ,

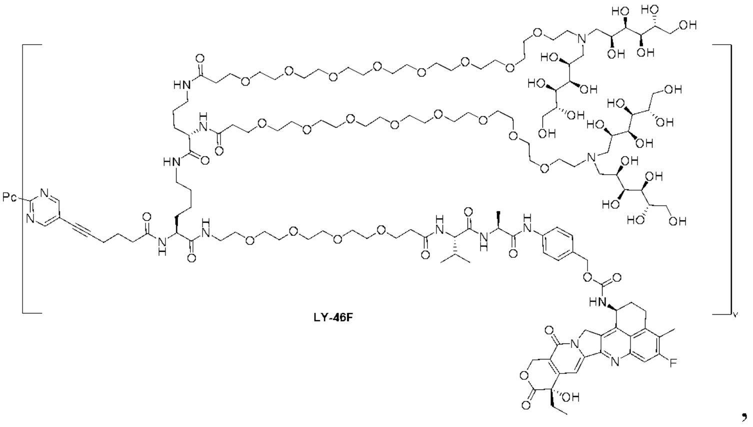

LY-46F

,

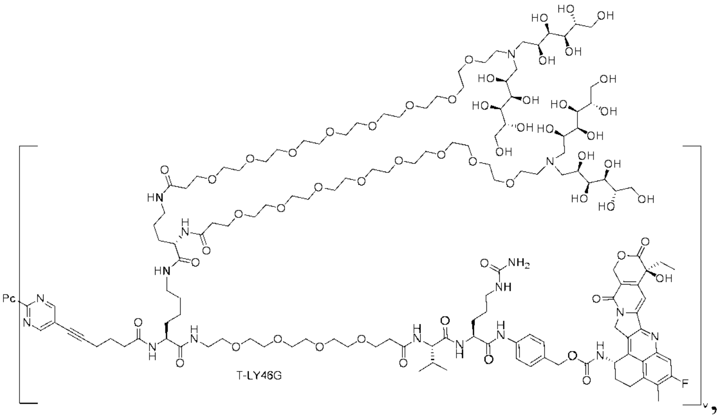

T-LY46G

,

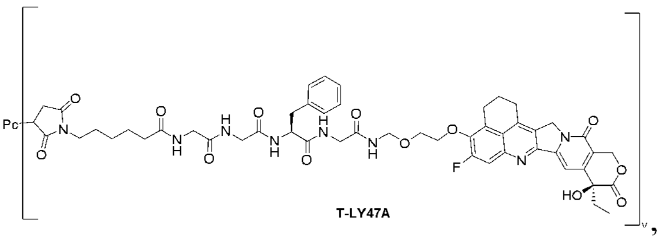

T-LY47A

,

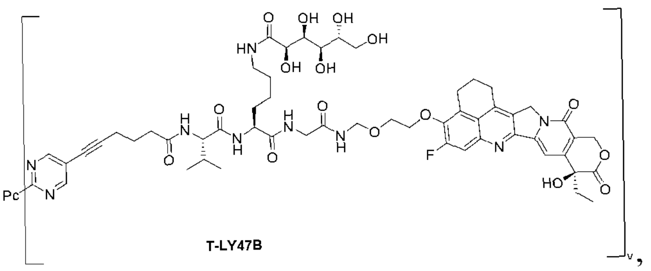

T-LY47B

,

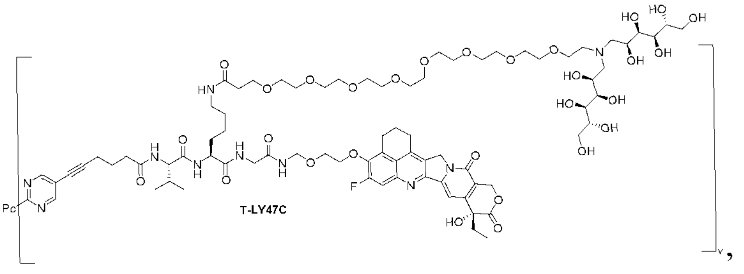

T-LY47C

,

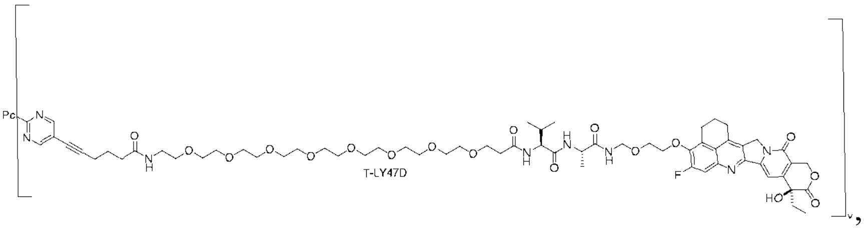

T-LY47D ,

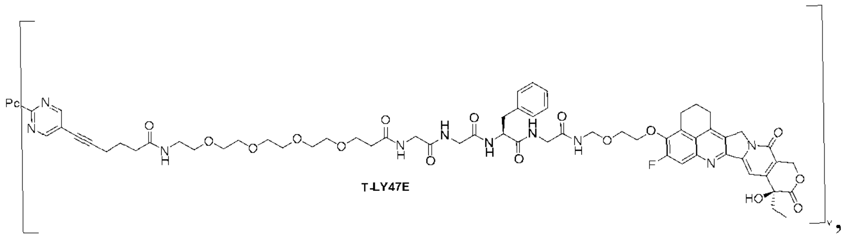

T-LY47E ,

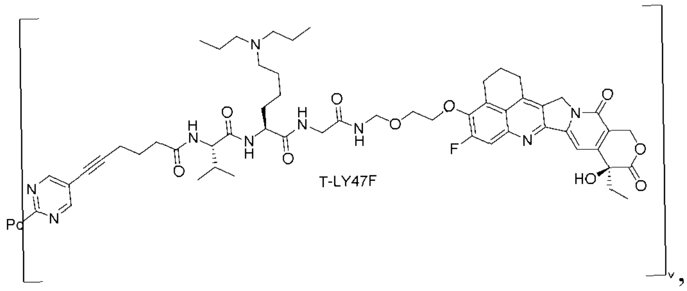

T-LY47F ,

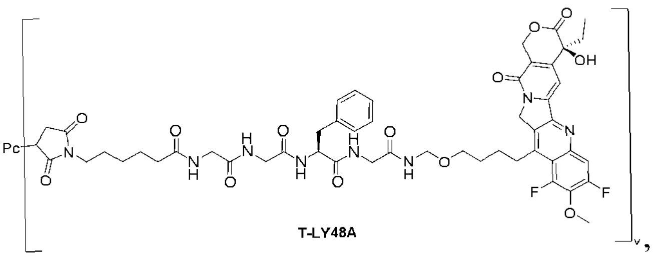

T-LY48A ,

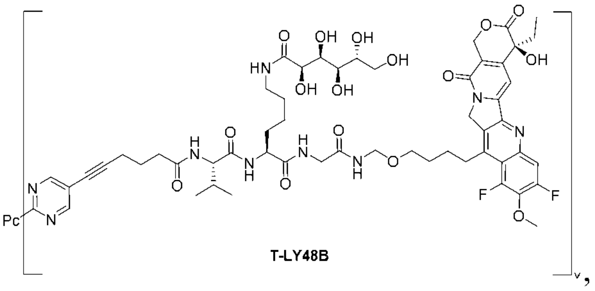

T-LY48B ,

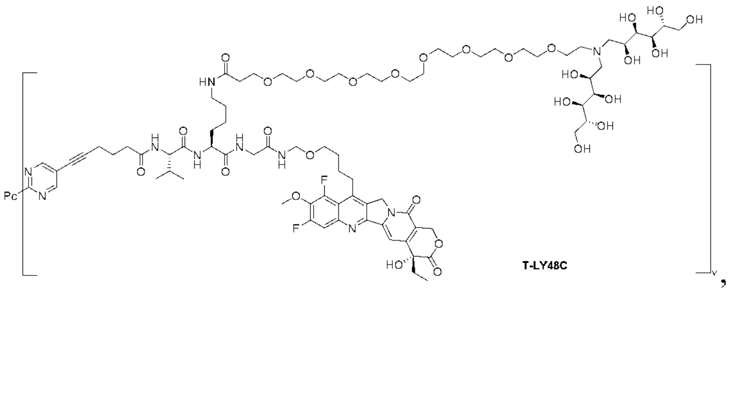

T-LY48C ,

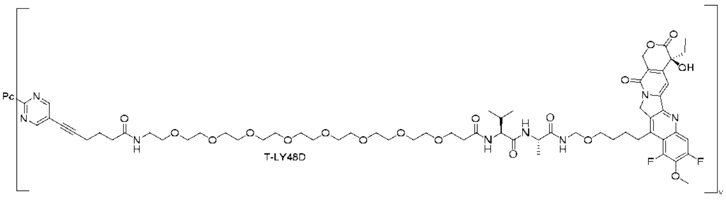

T-LY48D

,

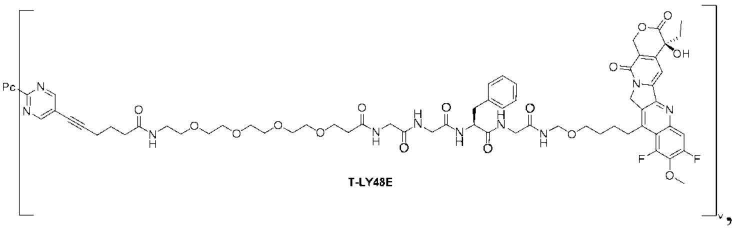

T-LY48E

,

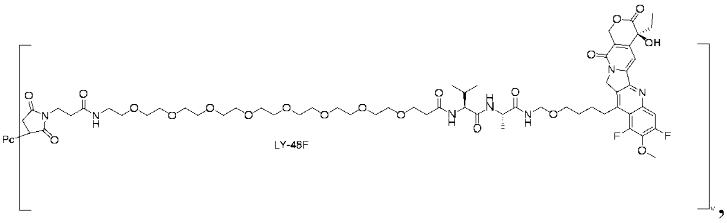

LY-48F

,

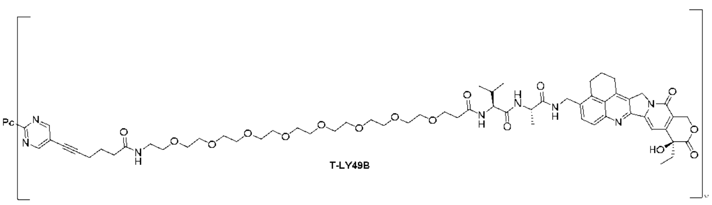

T-LY49B

,

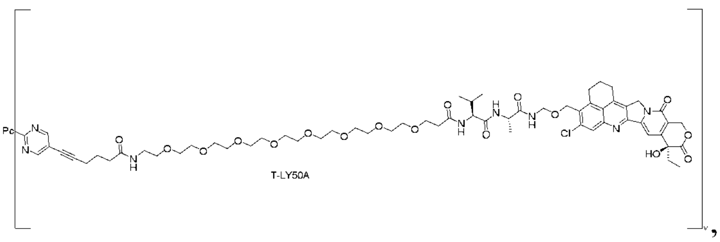

T-LY50A

,

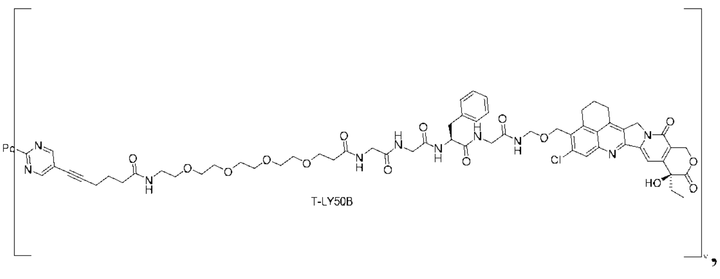

T-LY50B

,

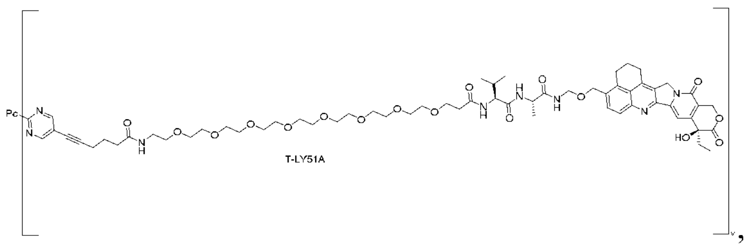

T-LY51A

,

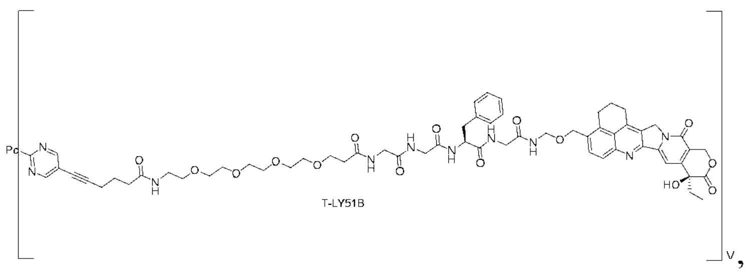

T-LY51B

,

wherein

v is from 1 to 10, and v is a decimal or an integer,

Pc is an antibody or an antigen-binding fragment thereof; and

Pc' is an antibody.

In some embodiments, in the ligand-drug conjugate represented by the general formula (B) or the pharmaceutically acceptable salt thereof according to the present invention, the antibody is selected from a murine antibody, a chimeric antibody, a humanized antibody, and a fully human antibody.

In other embodiments, in the ligand-drug conjugate represented by the general formula (B) or the pharmaceutically acceptable salt thereof according to the present invention, the antibody or an antigen-binding fragment thereof is selected from anti-HER2 (ErbB2) antibody, anti-EGFR antibody, anti-B7-H3 antibody, anti-c-Met antibody, anti-HER3 (ErbB3) antibody, anti-HER4 (ErbB4) antibody, anti-ROR1 antibody, anti-CLDN6 antibody, anti-CLDN9 antibody, anti-CLDN18.2 antibody, anti-NaPi-2b antibody, anti-TNF-$\alpha$ antibody, anti-ENPP3 antibody, anti-DLL3 antibody, anti-CD20 antibody, anti-CD22 antibody, anti-CD28 antibody, anti-CD30 antibody, anti-CD33 antibody, anti-CD37 antibody, anti-CD38 antibody, anti-CD44 antibody, anti-CD45 antibody, anti-CD47 antibody, anti-CD48 antibody, anti-CD56 antibody, anti-CD70 antibody, anti-CD73 antibody, anti-CD98 antibody, anti-CD105 antibody, anti-CEA antibody, anti-EphA2 antibody, anti-MUCI antibody, anti-Lewis Y antibody, anti-VEGFR antibody, anti-GPNMB antibody, anti-Integrin antibody, anti-PSMA antibody, anti-Tenascin-C antibody, anti-SLC44A4 antibody, anti-CD79 antibody, anti-TROP-2 antibody, anti-CD79B antibody, anti-Mesothelin antibody, anti-Nectin-4 antibody, anti-TPBG antibody, or an antigen-binding fragment thereof.

In other embodiments, in the ligand-drug conjugate represented by the general formula (B) or the pharmaceutically acceptable salt thereof according to the present invention, the antibody or the antigen-binding fragment thereof is selected from Trastuzumab, Cetuximab, Pertuzumab, Nimotuzumab, Enoblituzumab, Emibetuzumab, Inotuzumab, Pinatuzumab, Brentuximab, Gemtuzumab, Bivatuzumab, Lorvotuzumab, or an antigen-binding fragment thereof.

In other embodiments, the ligand-drug conjugate represented by the general formula (B) or the pharmaceutically acceptable salt thereof according to the present invention is selected from:

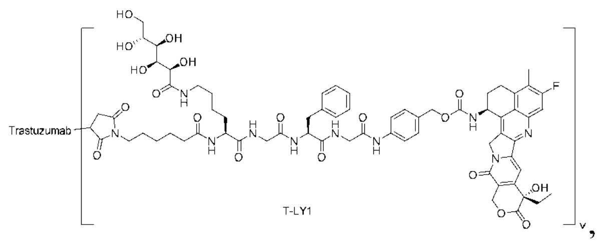

T-LY1

,

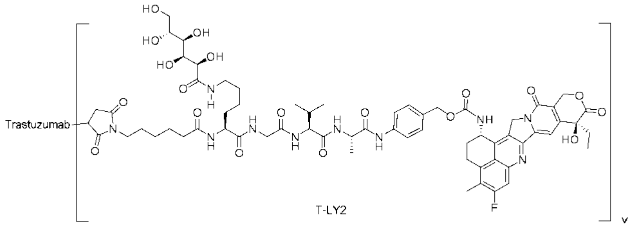

T-LY2 ,

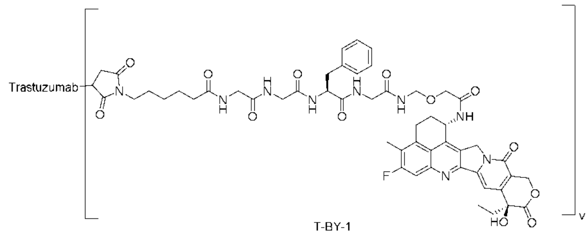

T-BY-1 ,

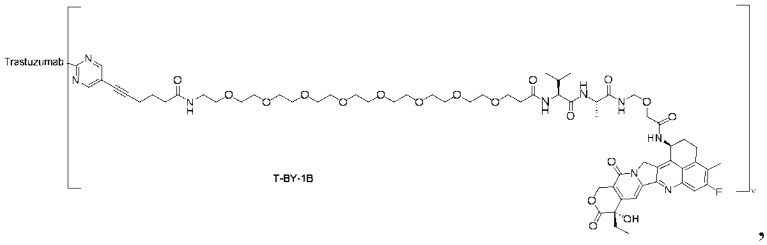

T-BY-1B ,

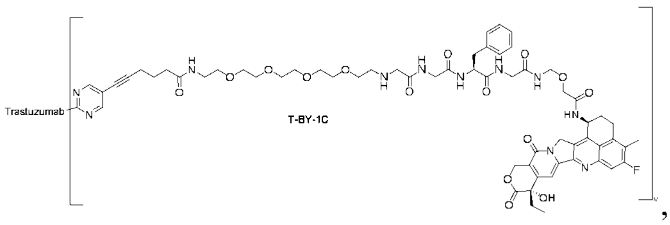

T-BY-1C ,

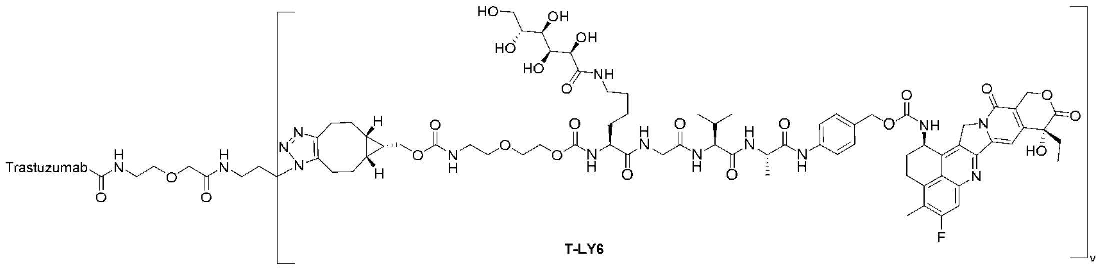

T-LY6 ,

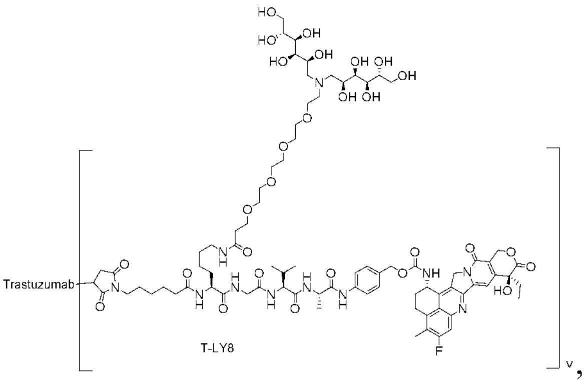
T-LY8 ,

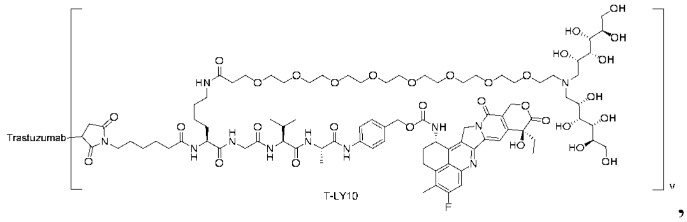
T-LY10 ,

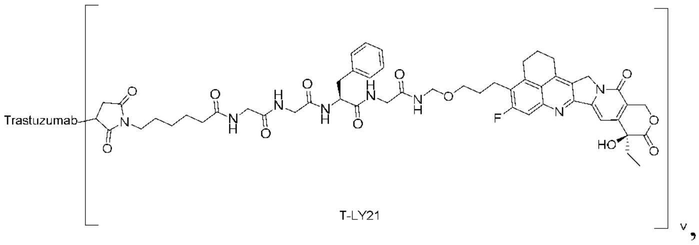
T-LY21 ,

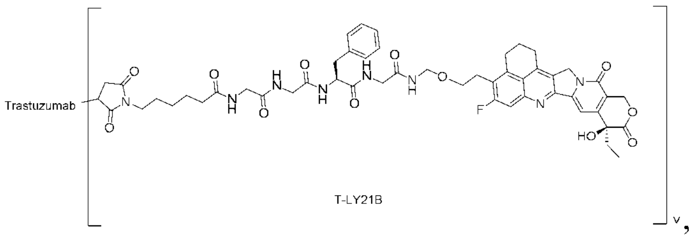
T-LY21B ,

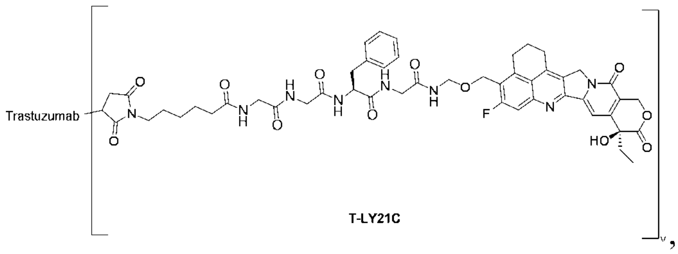
**T-LY21C** ,

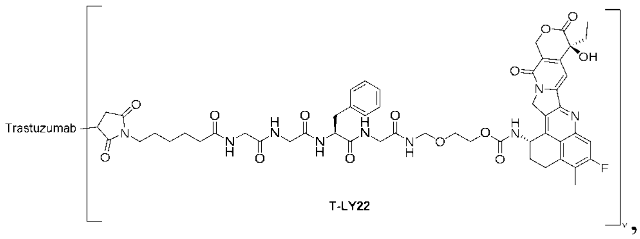
**T-LY22** ,

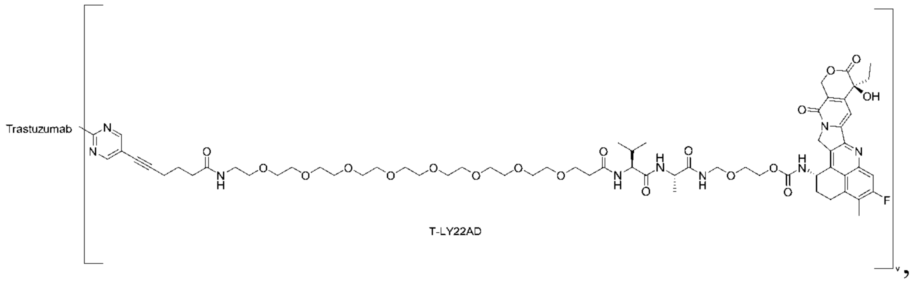

T-LY22AD

,

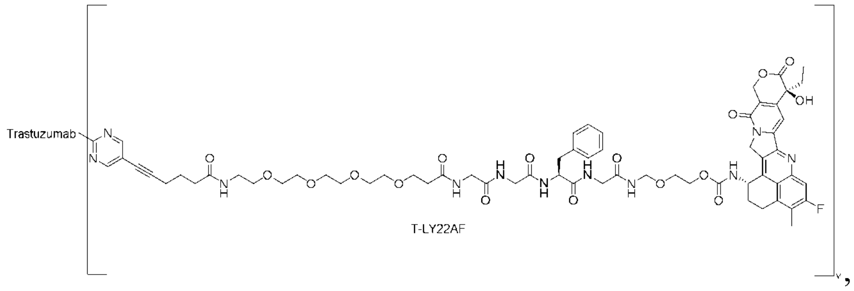

T-LY22AF

,

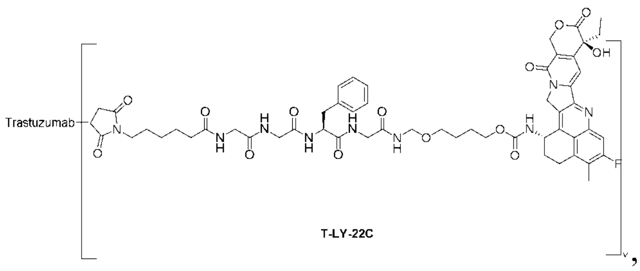

T-LY-22C

,

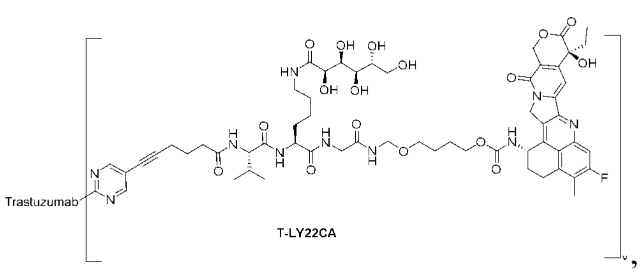

T-LY22CA

,

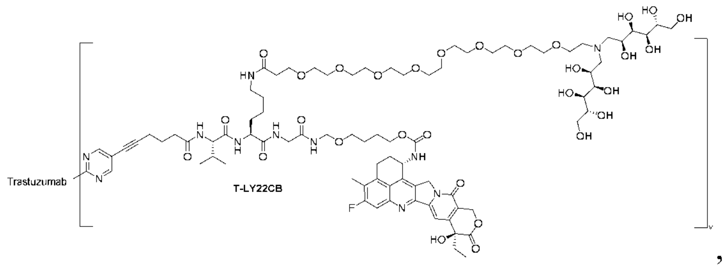

T-LY22CB

,

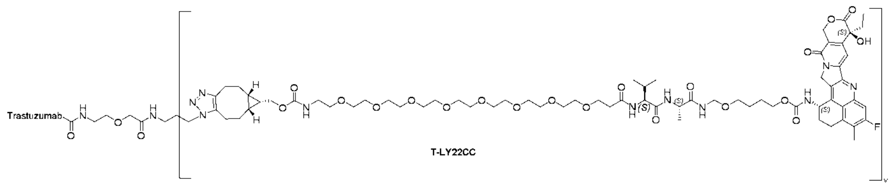

T-LY22CC

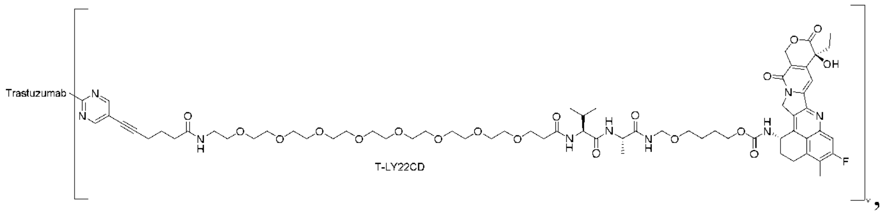

T-LY22CD

,

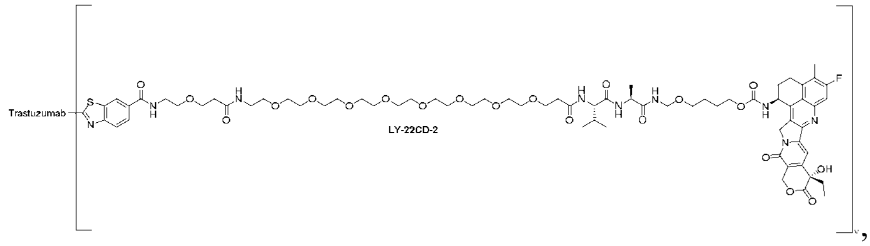

LY-22CD-2

,

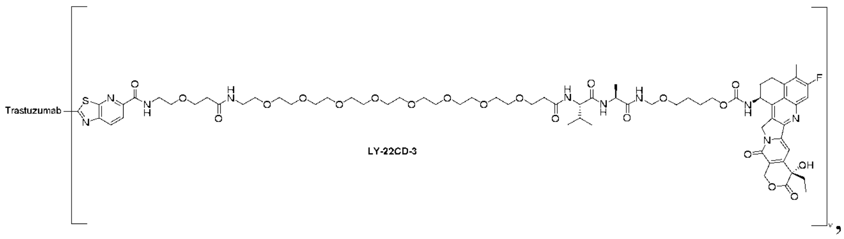

LY-22CD-3

,

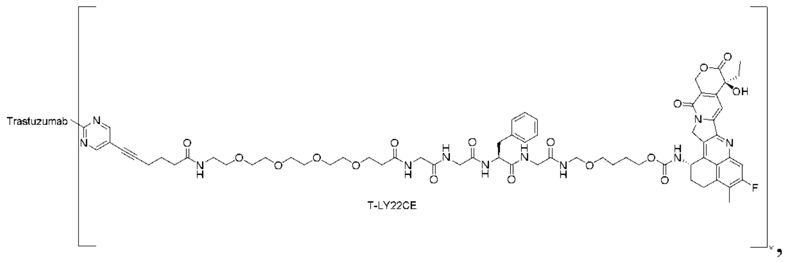

T-LY22CE

,

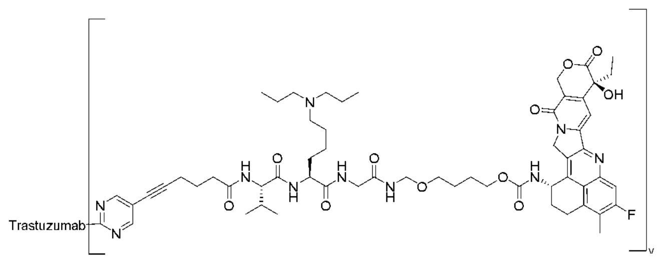

T-LY22CF

,

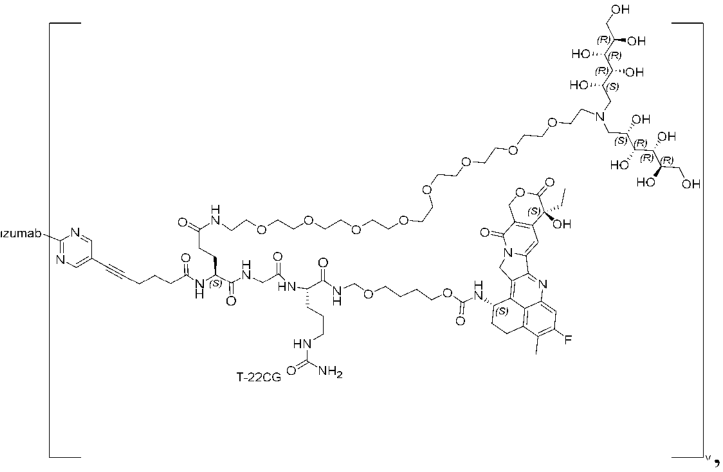

T-22CG

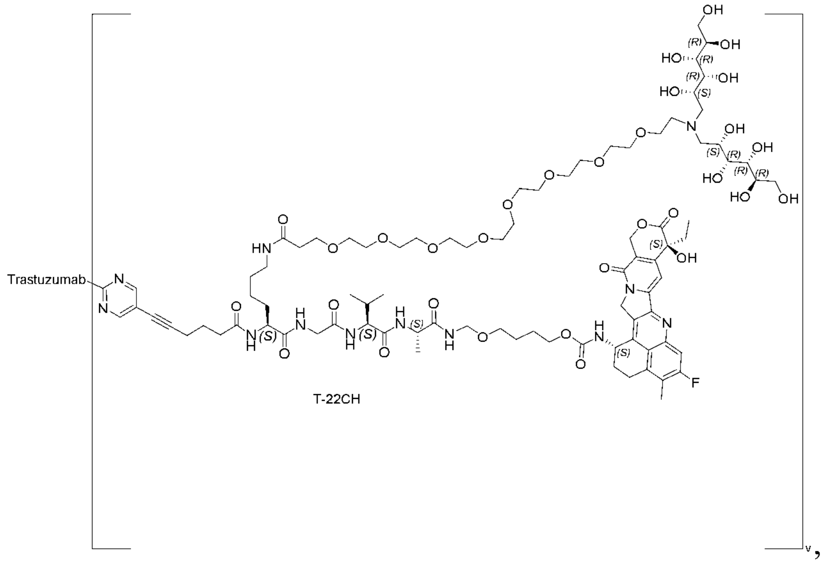

T-22CH

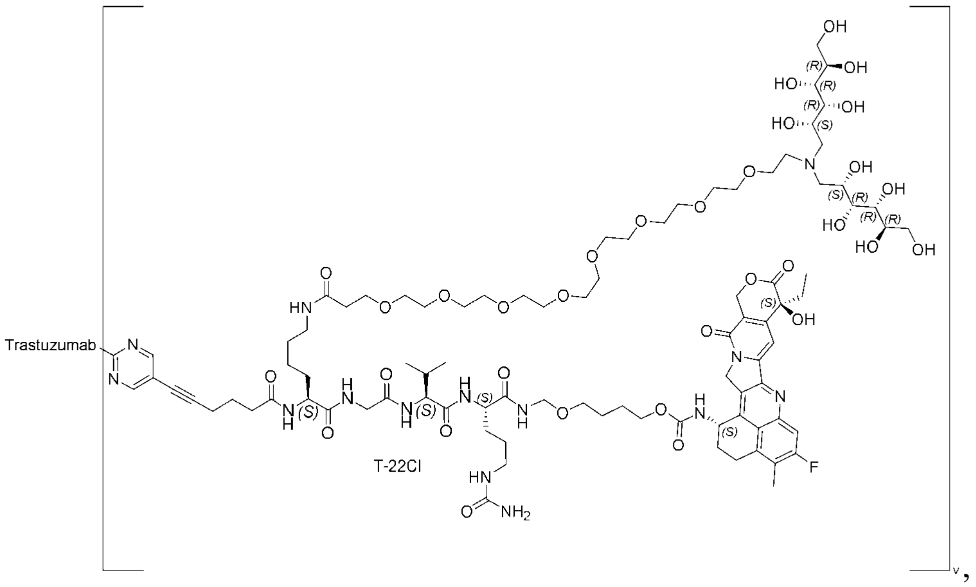

T-22CI

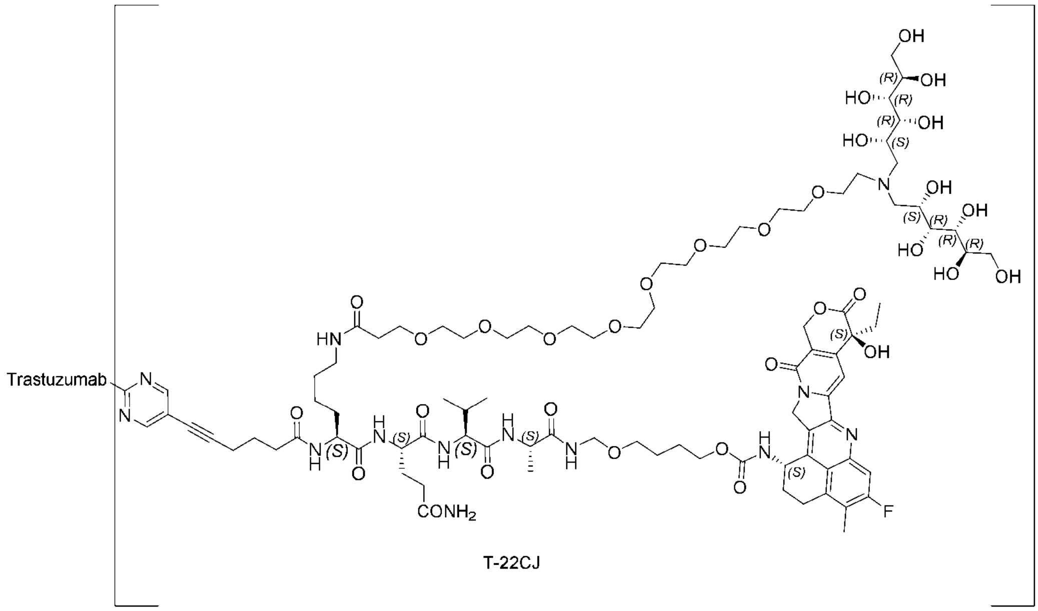

T-22CJ

,

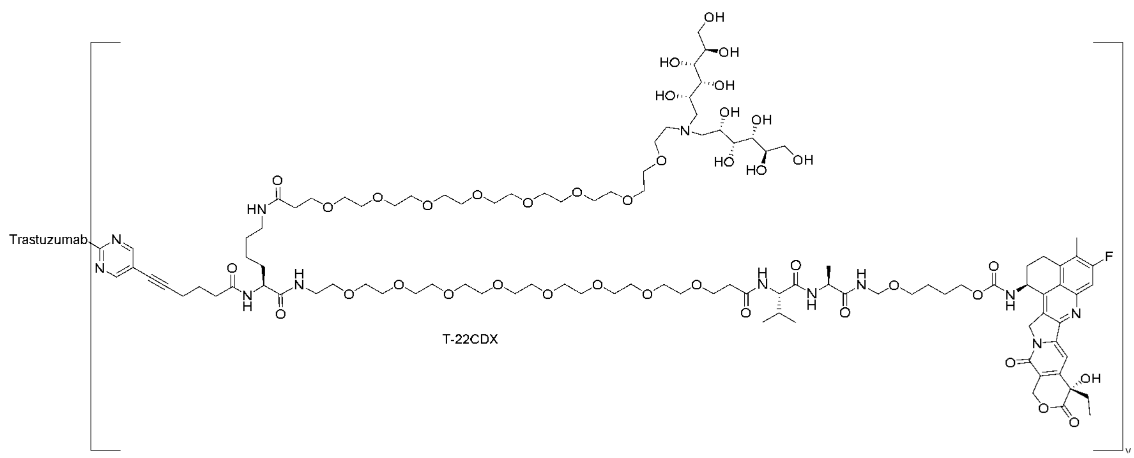

T-22CDX

,

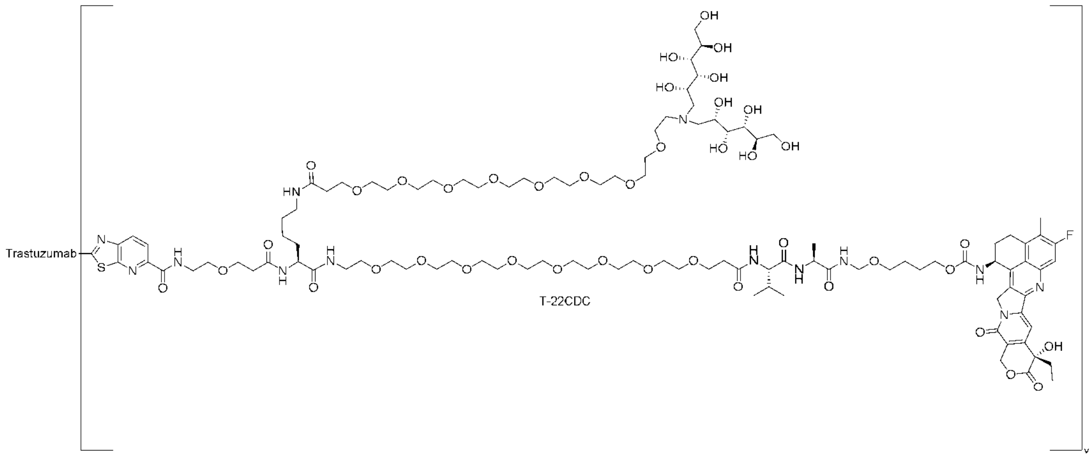

T-22CDC

,

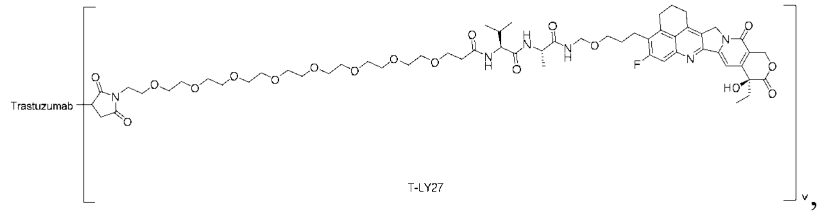

T-LY27

,

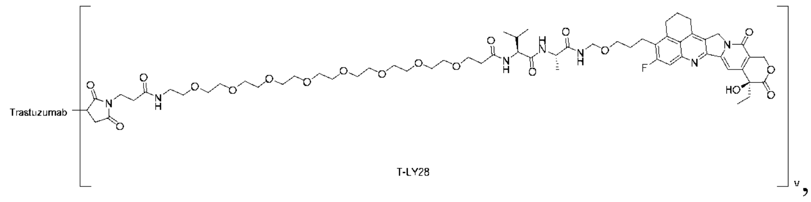

T-LY28

,

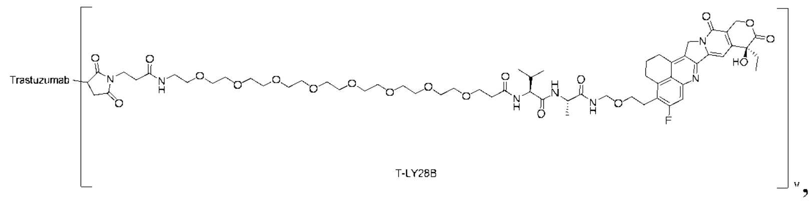

T-LY28B

,

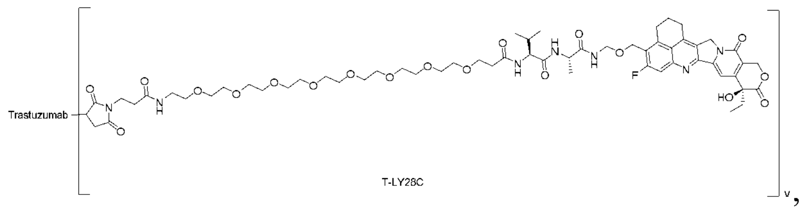

T-LY28C

,

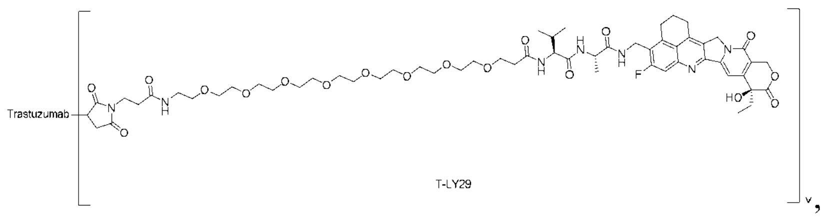

T-LY29

,

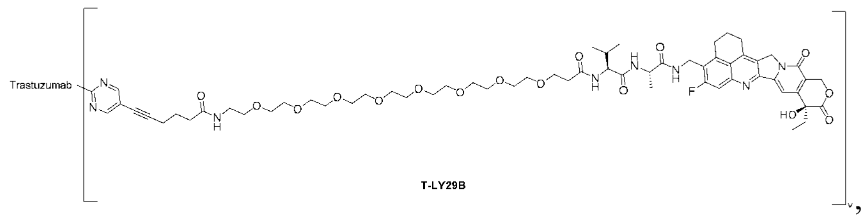

**T-LY29B**

,

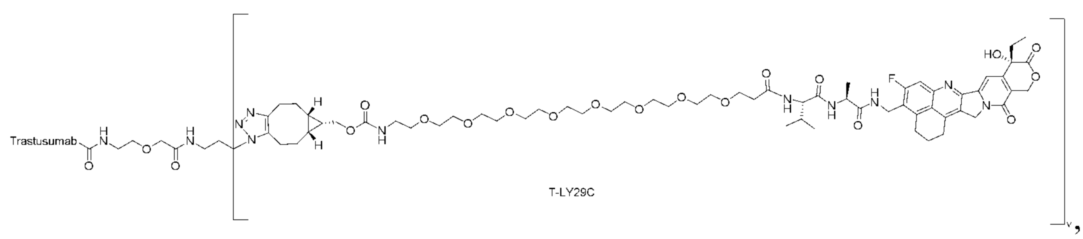

T-LY29C

,

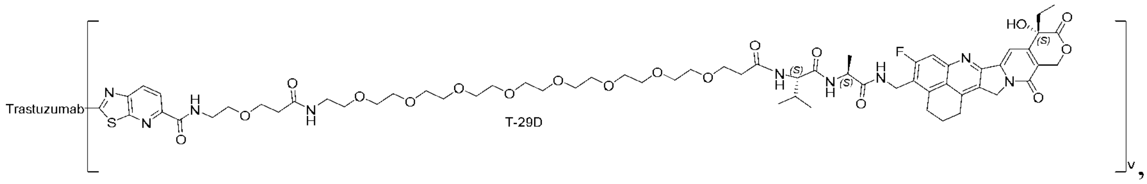

T-29D

,

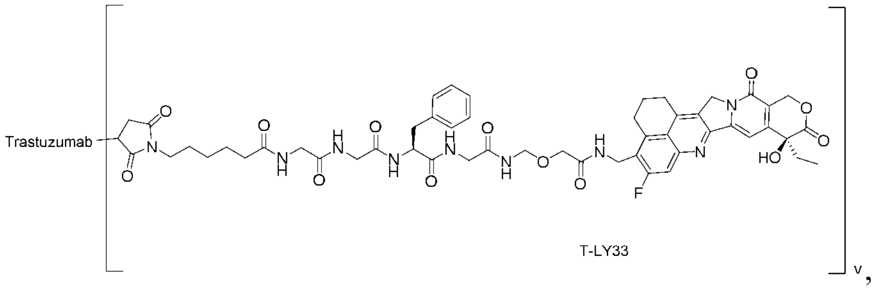

T-LY33

,

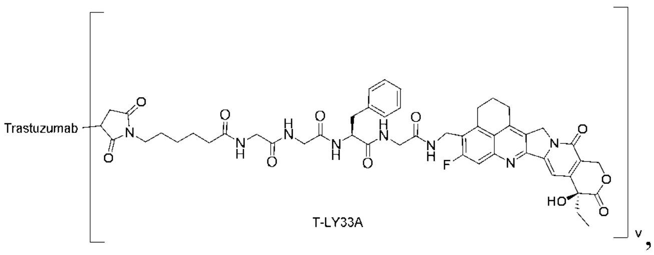

T-LY33A

,

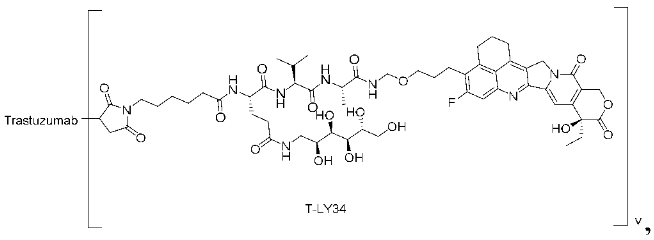

T-LY34

,

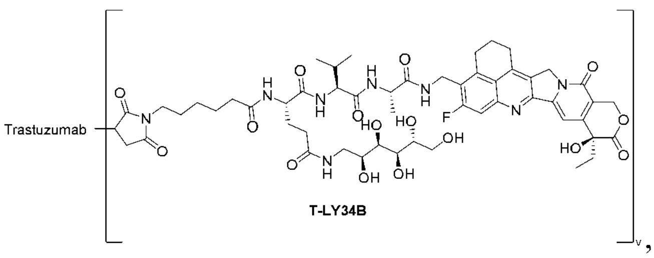

**T-LY34B**

,

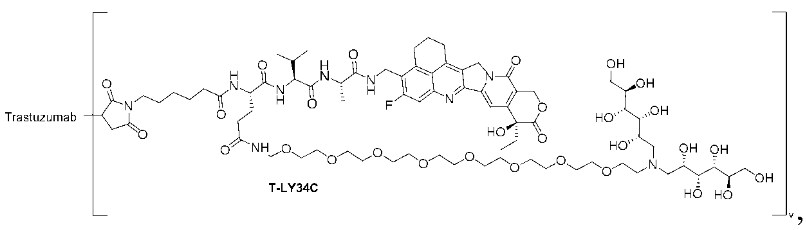

**T-LY34C**

,

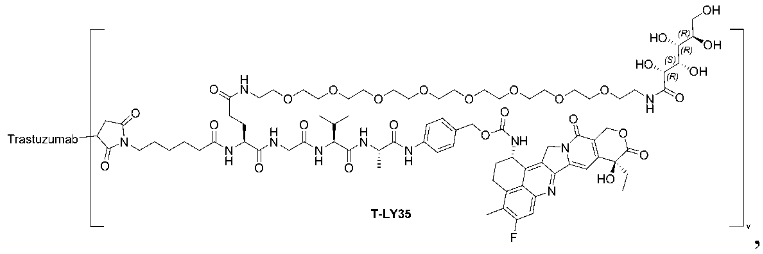

T-LY35

,

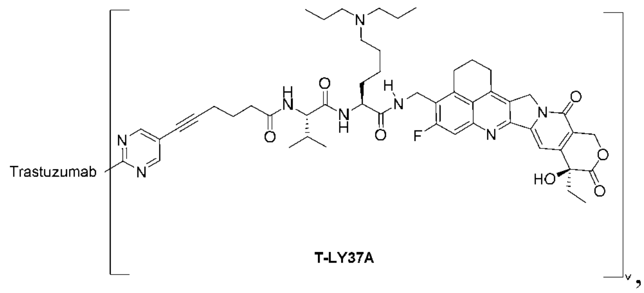

T-LY37A

,

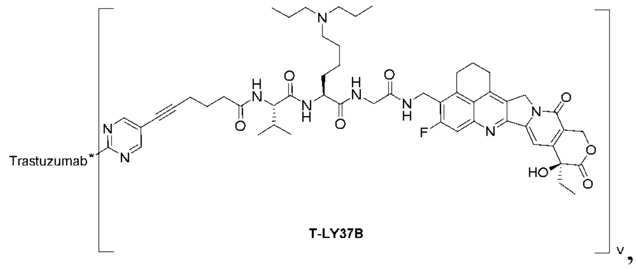

T-LY37B

,

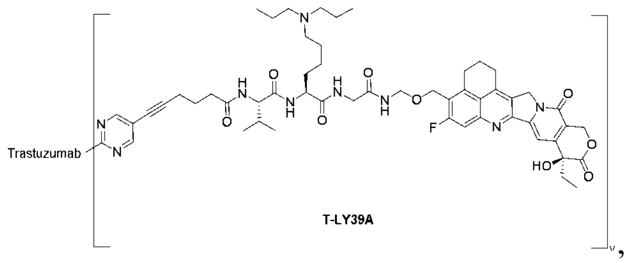

T-LY39A

,

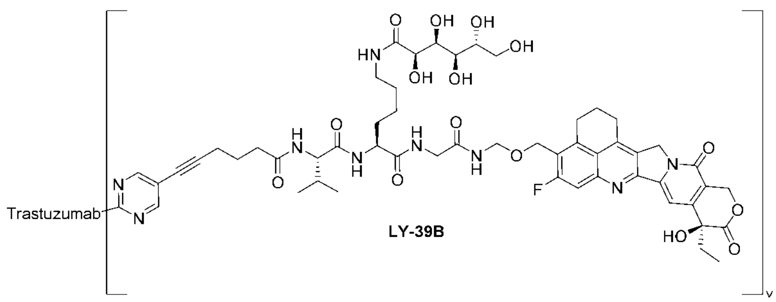

LY-39B

,

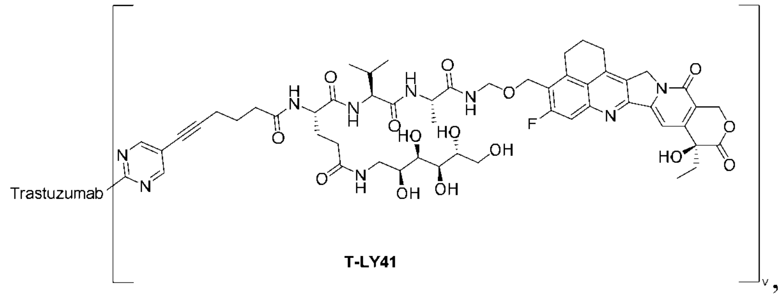

**T-LY41**

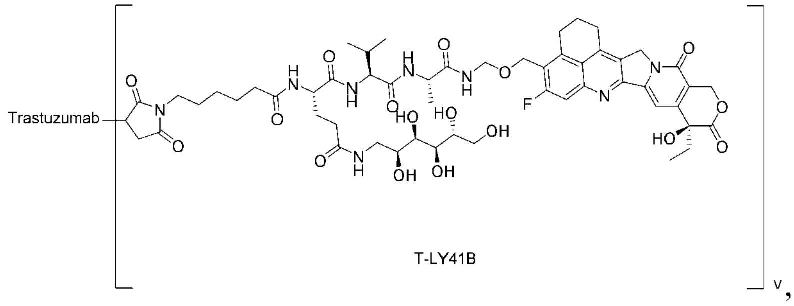

T-LY41B

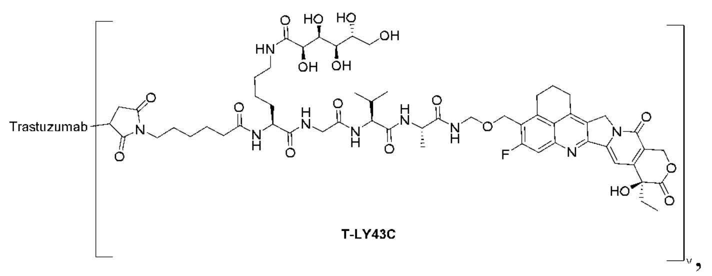

**T-LY43C**

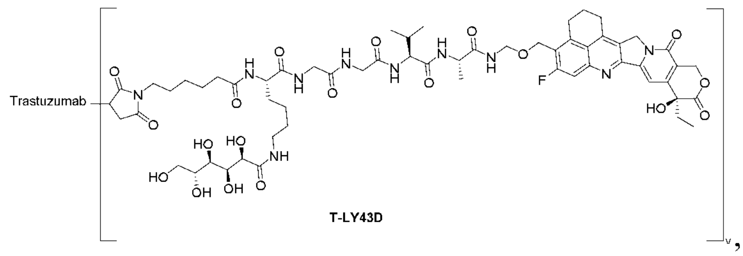

**T-LY43D**

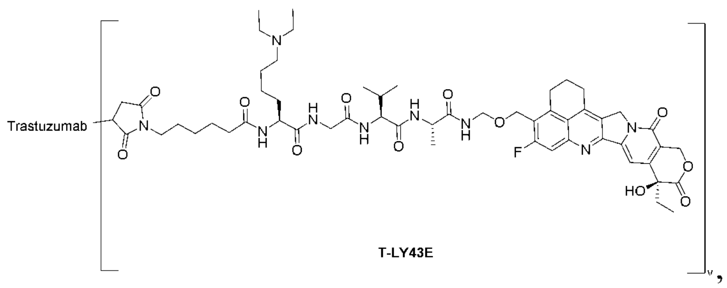

**T-LY43E**

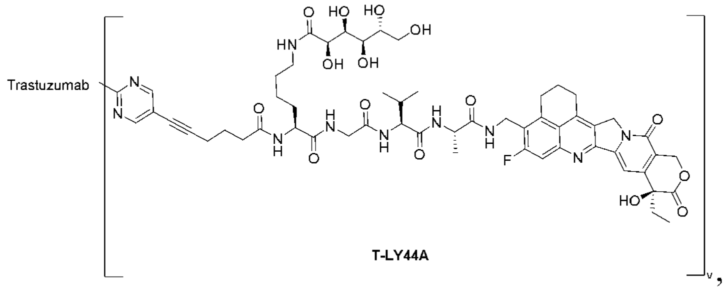

T-LY44A ,

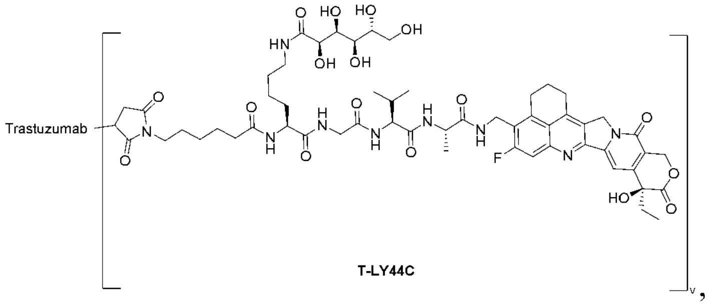

T-LY44C ,

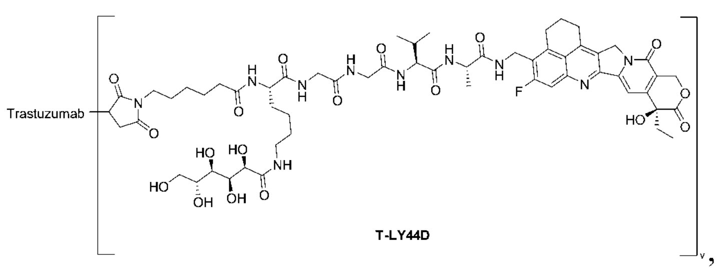

T-LY44D ,

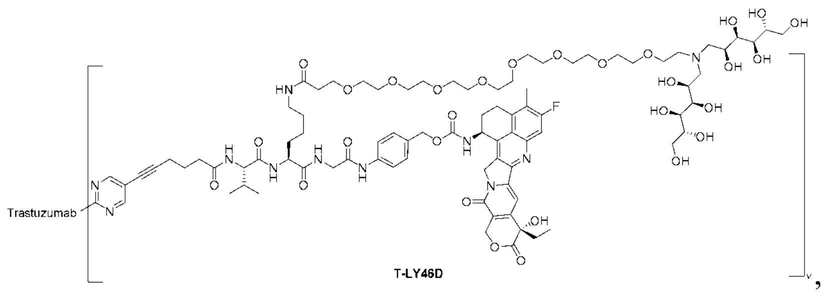

T-LY46D ,

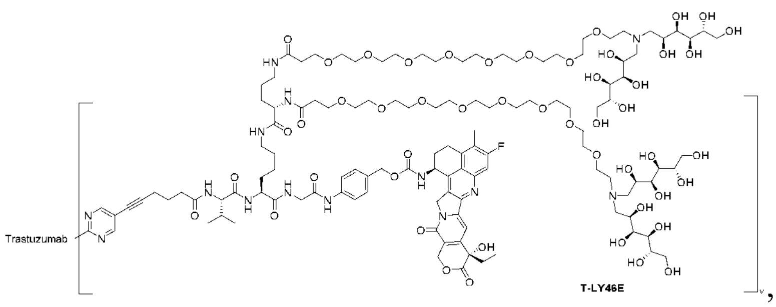

T-LY46E ,

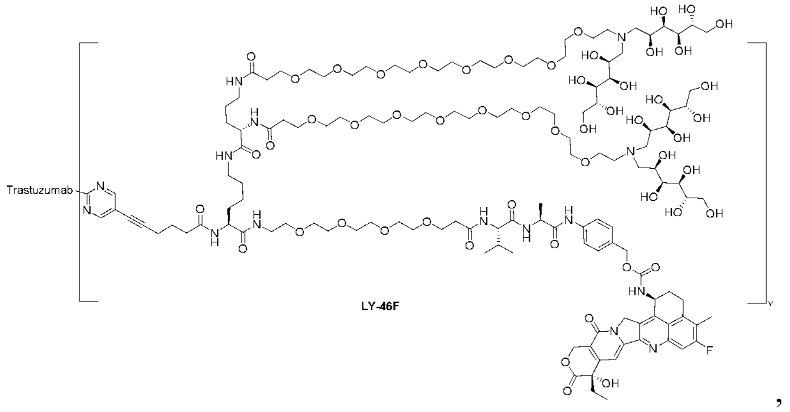

LY-46F

,

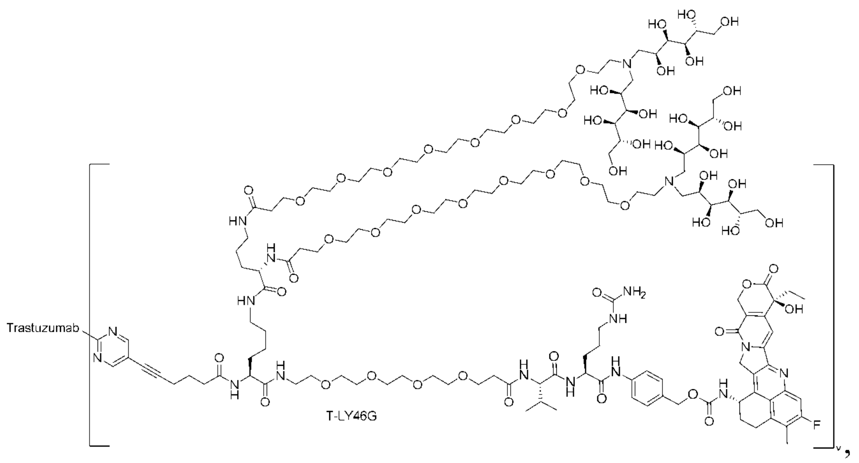

T-LY46G

,

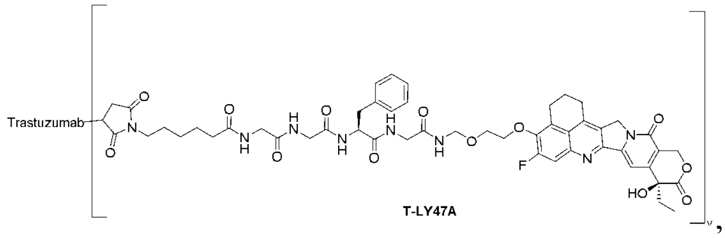

T-LY47A

,

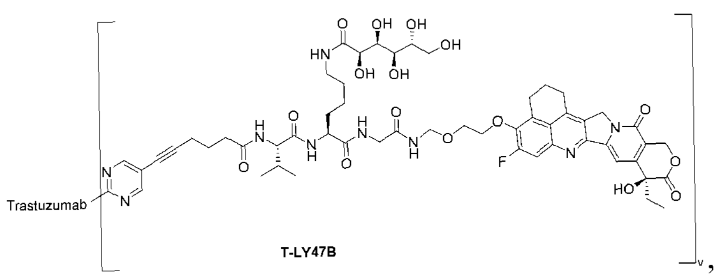

T-LY47B

,

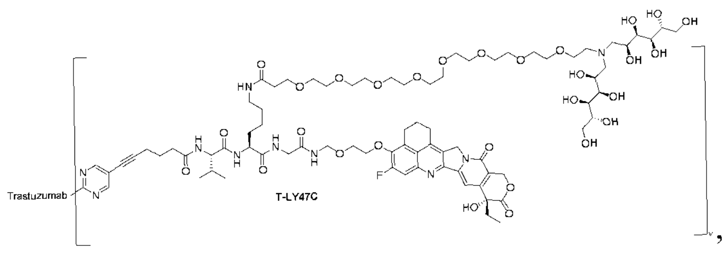

T-LY47C

,

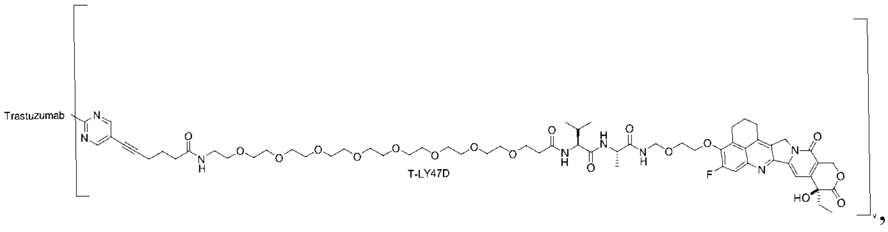

T-LY47D ,

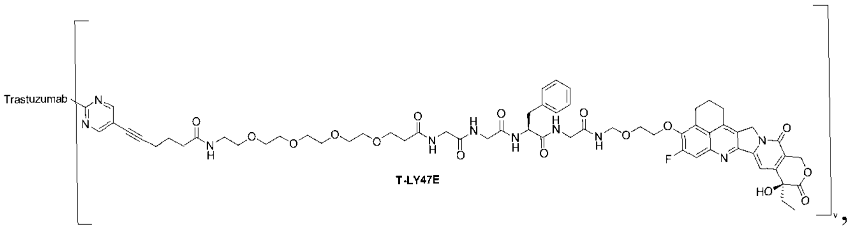

T-LY47E ,

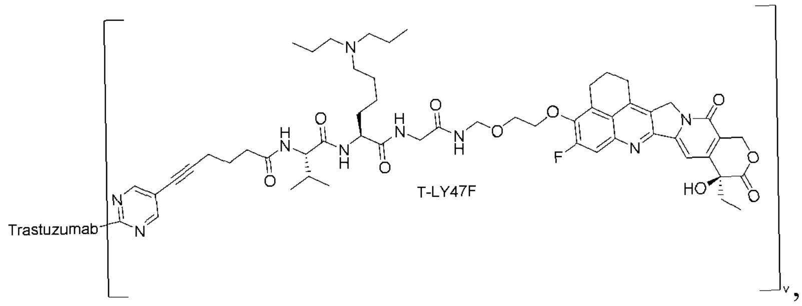

T-LY47F ,

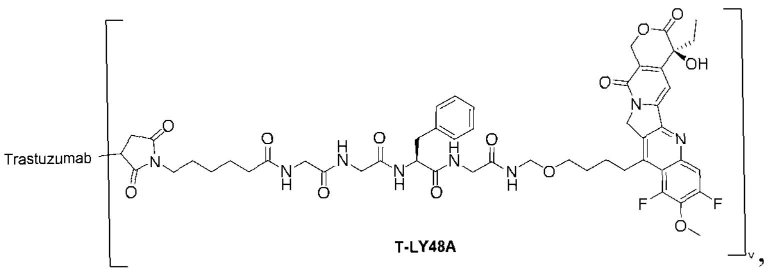

T-LY48A ,

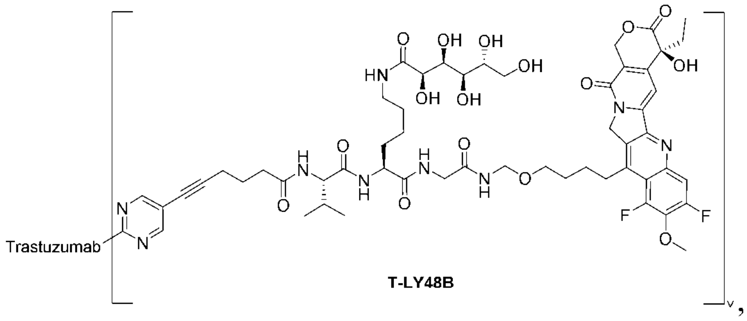

T-LY48B ,

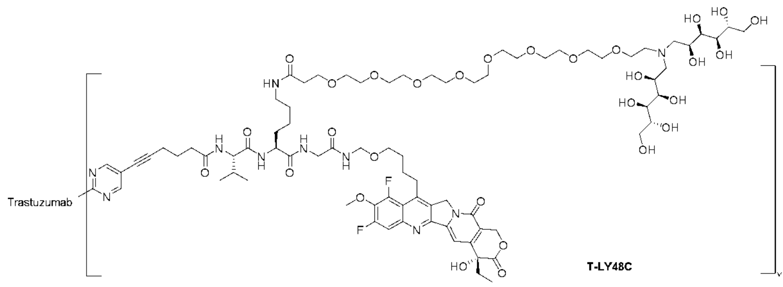

T-LY48C,

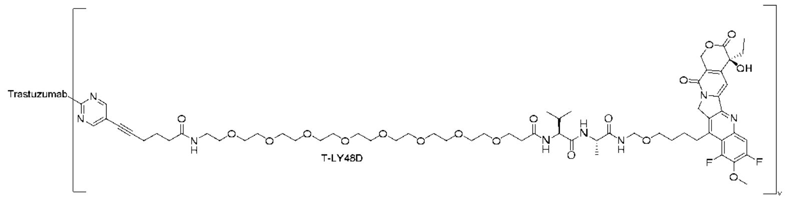

T-LY48D,

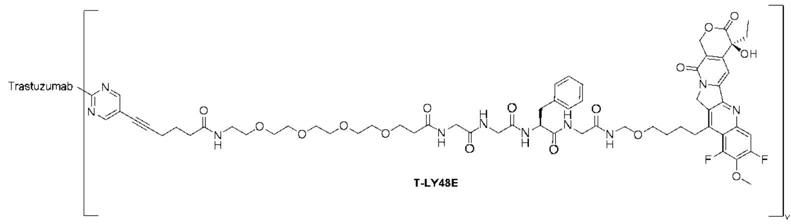

T-LY48E,

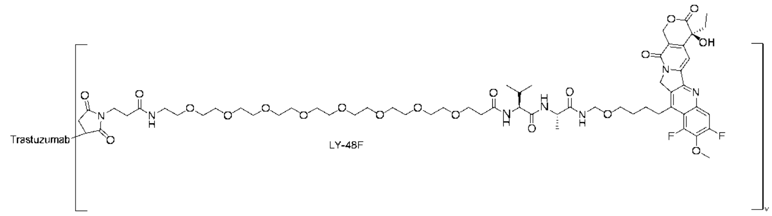

LY-48F,

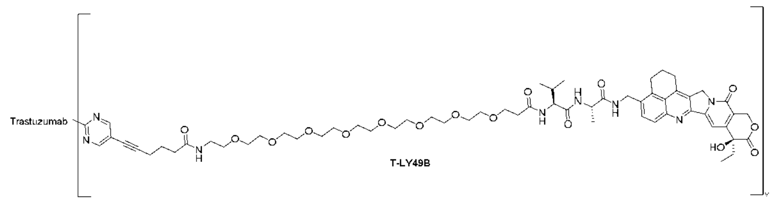

T-LY49B,

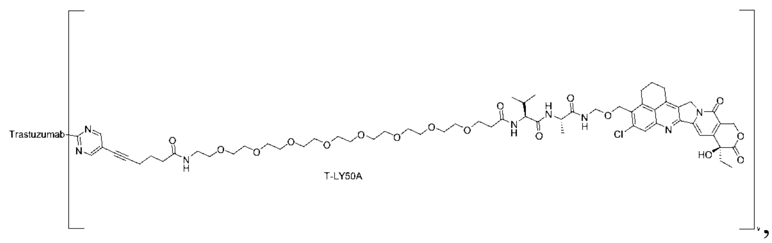

T-LY50A,

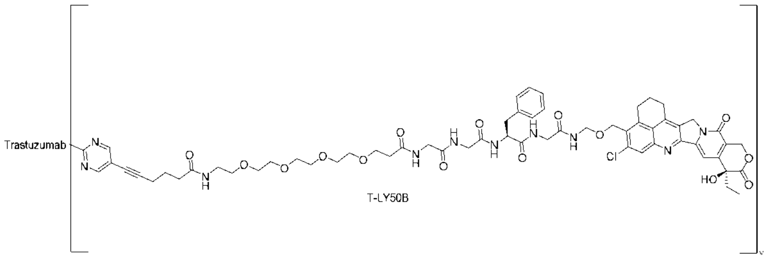

T-LY50B ,

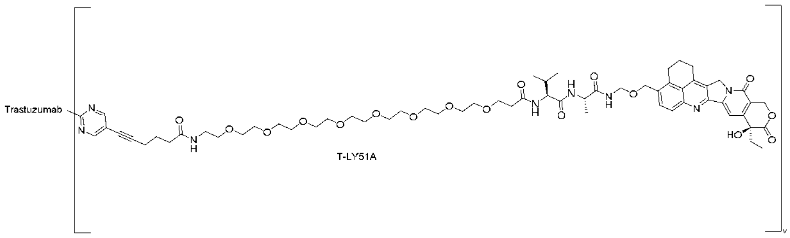

T-LY51A ,

and

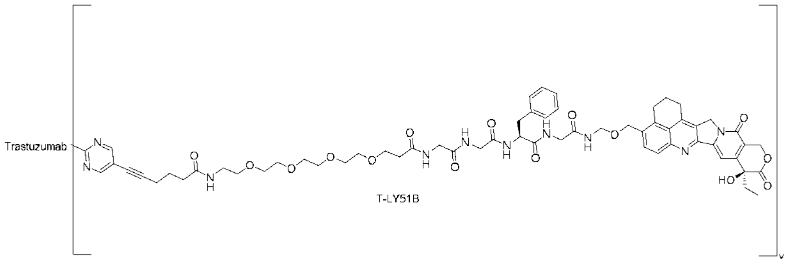

T-LY51B ;

wherein v is an integer or a decimal from 1 to 10; preferably, an integer from 2 to 8.

The present invention further relates to a pharmaceutical composition, which comprises the ligand-drug conjugate represented by the general formula (B) or the pharmaceutically acceptable salt thereof according to the present invention, and one or more pharmaceutically acceptable carriers or excipients.

The present invention also relates to use of the compound represented by the general formula (A) or the pharmaceutically acceptable salt thereof according to the present invention in preparation of a ligand-drug conjugate.

The present invention also relates to use of the compound represented by the general formula (I) or the stereoisomer, tautomer, mesomer, racemate, enantiomer, or diastereomer thereof, or the mixture thereof, or the pharmaceutically acceptable salt thereof according to the present invention, or the compound represented by the general formula (II) or the stereoisomer, tautomer, mesomer, racemate, enantiomer, or diastereomer thereof, or the mixture thereof, or the pharmaceutically acceptable salt thereof according to the present invention, in preparation of a ligand-drug conjugate.

The present invention further relates to use of the ligand-drug conjugate or the pharmaceutically acceptable salt thereof, or the pharmaceutical composition comprising the same according to the present invention, in preparation of a medication for treating a tumor or cancer, wherein the cancer is preferably breast cancer, ovarian cancer, soft tissue sarcoma, liposarcoma, lung cancer, non-small cell lung cancer, gastric cancer, melanoma, head and neck cancer, cervical cancer, or prostate cancer.

The present invention also relates to the ligand-drug conjugate or the pharmaceutically acceptable salt thereof, or the pharmaceutical composition comprising the same according to the present invention, for use in treating a tumor or cancer, wherein the cancer is preferably breast cancer, ovarian cancer, soft tissue sarcoma, liposarcoma, lung cancer, non-small cell lung cancer, gastric cancer, melanoma, head and neck cancer, cervical cancer, or prostate cancer.

The present invention further relates to a method for treating a tumor or cancer, comprising administering to a subject in need an effective amount of the ligand-drug conjugate or the pharmaceutically acceptable salt thereof, or the pharmaceutical composition comprising the same according to the present invention, wherein the cancer is preferably breast cancer, ovarian cancer, soft tissue sarcoma, liposarcoma, lung cancer, non-small cell lung cancer, gastric cancer, melanoma, head and neck cancer, cervical cancer, or prostate cancer.

The present invention further relates to use of the compound represented by the general formula (I) or the

stereoisomer, tautomer, mesomer, racemate, enantiomer, or diastereomer thereof, or the mixture thereof, or the pharmaceutically acceptable salt thereof, or the compound represented by the general formula (II) or the stereoisomer, tautomer, mesomer, racemate, enantiomer, or diastereomer thereof, or the mixture thereof, or the pharmaceutically acceptable salt thereof, or the pharmaceutical composition comprising the same according to the present invention, in preparation of a medication for treating a tumor or cancer, wherein the cancer is preferably breast cancer, ovarian cancer, soft tissue sarcoma, liposarcoma, lung cancer, non-small cell lung cancer, gastric cancer, melanoma, head and neck cancer, cervical cancer, or prostate cancer.

The present invention also relates to the compound represented by the general formula (I) or the stereoisomer, tautomer, mesomer, racemate, enantiomer, or diastereomer thereof, or the mixture thereof, or the pharmaceutically acceptable salt thereof, or the compound represented by the general formula (II) or the stereoisomer, tautomer, mesomer, racemate, enantiomer, or diastereomer thereof, or the mixture thereof, or the pharmaceutically acceptable salt thereof, or the pharmaceutical composition comprising the same according to the present invention, for use in treating a tumor or cancer, wherein the cancer is preferably breast cancer, ovarian cancer, soft tissue sarcoma, liposarcoma, lung cancer, non-small cell lung cancer, gastric cancer, melanoma, head and neck cancer, cervical cancer, or prostate cancer.

The present invention further relates to a method for treating a tumor or cancer, comprising administering to a subject in need an effective amount of the compound represented by the general formula (I) or the stereoisomer, tautomer, mesomer, racemate, enantiomer, or diastereomer thereof, or the mixture thereof, or the pharmaceutically acceptable salt thereof, or the compound represented by the general formula (II) or the stereoisomer, tautomer, mesomer, racemate, enantiomer, or diastereomer thereof, or the mixture thereof, or the pharmaceutically acceptable salt thereof, or the pharmaceutical composition comprising the same according to the present invention, wherein the cancer is preferably breast cancer, ovarian cancer, soft tissue sarcoma, liposarcoma, lung cancer, non-small cell lung cancer, gastric cancer, melanoma, head and neck cancer, cervical cancer, or prostate cancer.

## DETAILED DESCRIPTION

Unless otherwise defined, all technical and scientific terms used herein have the same meanings as those commonly understood by persons of ordinary skills in the art to which the present disclosure belongs. Although the present disclosure may also be implemented or tested by using any method and material similar or equivalent to that described herein, preferred methods and materials are described herein.

Unless otherwise stated, terms used in the description and claims have the following meanings.

The term "linker unit (or bonding fragment)" refers to a chemical structure fragment or bond that is connected to a ligand at one end and to a drug at the other end, and can also be connected to other linkers before connecting to the drug.

The term "ligand-drug conjugate" means that the ligand is connected to a biologically active drug through a stable linker unit. In the present disclosure, the "ligand-drug conjugate" is preferably an antibody drug conjugate (ADC), meaning that a monoclonal antibody or an antibody fragment is connected to a biologically active toxic drug, namely a camptothecin derivative, through the stable linker unit.

Examples of three-letter and single-letter codes for amino acids used in the present disclosure and structures thereof are shown in the following table:

| Acronym | Abbreviation | Name | Structure |
|---|---|---|---|
| G | Gly | Glycine | 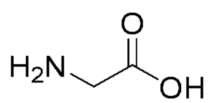 |
| A | Ala | Alanine | 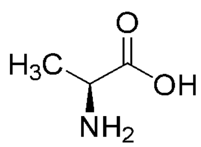 |
| V | Val | Valine | 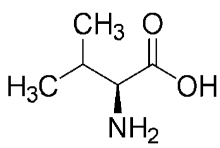 |

(continued)

| Acronym | Abbreviation | Name | Structure |
|---|---|---|---|
| L | Leu | Leucine | 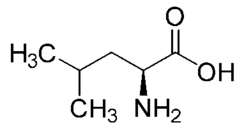 |
| I | Ile | Isoleucine | 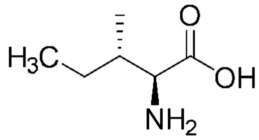 |
| F | Phe | Phenylalanine | 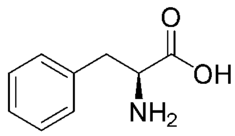 |
| W | Trp | Tryptophan | 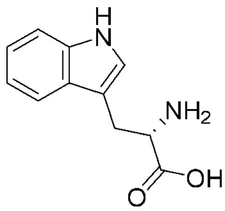 |
| Y | Tyr | Tyrosine | 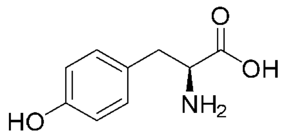 |
| D | Asp | Aspartic acid | 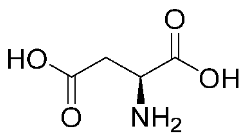 |
| H | His | Histidine | 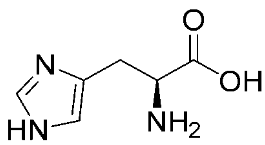 |
| N | Asn | Asparagine | 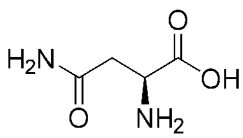 |
| E | Glu | Glutamic acid | 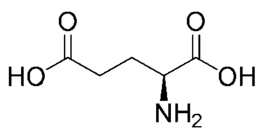 |
| K | Lys | Lysine | 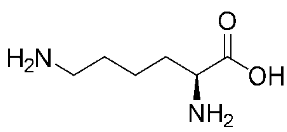 |

(continued)

| Acronym | Abbreviation | Name | Structure |
|---|---|---|---|
| Q | Gln | Glutamine | 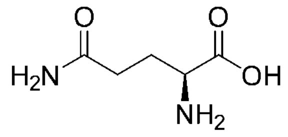 |
| M | Met | Methionine | 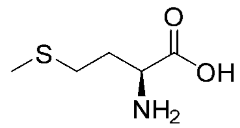 |
| R | Arg | Arginine | 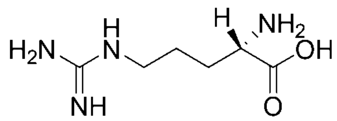 |
| S | Ser | Serine | 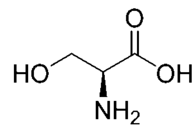 |
| T | Thr | Threonine | 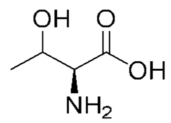 |
| C | Cys | Cysteine | 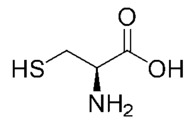 |
| P | Pro | Proline | 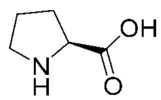 |
| C | Cit | Citrulline | 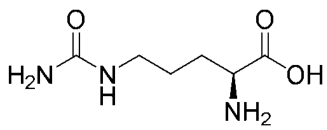 |

The term "antibody" refers to immunoglobulins, which are tetrapeptide chain structures composed of two identical heavy chains and two identical light chains connected by interchain disulfide bonds. The amino acid composition and sequence of the constant region of immunoglobulin heavy chains differ, thus their antigenicity also differs. Accordingly, immunoglobulins can be classified into five types or isotypes, namely IgM, IgD, IgG, IgA, and IgE, with their corresponding heavy chains being $\mu$ chain, $\delta$ chain, $\gamma$ chain, $\alpha$ chain, and $\varepsilon$ chain, respectively. The same class of Ig can be further divided into different subclasses based on differences in the amino acid composition of the hinge region and the number and position of heavy chain disulfide bonds, such as IgG, which can be divided into IgG1, IgG2, IgG3, and IgG4. The light chains are classified into $\kappa$ chain or $\lambda$ chain based on their constant region. Each class of Ig can have either $\kappa$ chains or $\lambda$ chains.

The heavy and light chains of antibodies have sequences of approximately 110 amino acids near the N-terminus that vary greatly, known as the variable region (Fv region). The remaining amino acid sequences near the C-terminus are relatively stable and are called the constant region. The variable region includes three hypervariable regions (HVRs) and four relatively conserved framework regions (FRs). The three hypervariable regions determine the specificity of the antibody and are also known as complementarity-determining regions (CDRs). Each light chain variable region (LCVR) and heavy chain variable region (HCVR) consists of three CDRs and four FRs arranged in the following sequence from the N-terminus to the C-terminus: FR1, CDR1, FR2, CDR2, FR3, CDR3, FR4. The three CDRs in the light chain are referred to as LCDR1, LCDR2, and LCDR3, while the three CDRs in the heavy chain are referred to as HCDR1, HCDR2, and HCDR3.

The CDR amino acid residues in the LCVR and HCVR regions of the antibodies or antigen-binding fragments described in this disclosure correspond in number and position to the known Kabat numbering scheme (LCDR1-3, HCDR2-3) or the Kabat and Chothia numbering schemes (HCDR1).

The term "antigen-binding fragment" refers to one or more fragments of an antibody that retain the ability to specifically bind to an antigen. It has been shown that fragments of a full-length antibody can be used for the antigen-binding function of the antibody. Examples of binding fragments included in the "antigen-binding fragment" include: (i) Fab fragments, which are monovalent fragments that consist of the VL, VH, CL, and CH1 domains; (ii) F(ab')2 fragments, which are bivalent fragments that include two Fab fragments connected by disulfide bridges in the hinge region; (iii) Fd fragments that consist of the VH and CH1 domains; (iv) Fv fragments that consist of the VH and VL domains of one arm of an antibody; (v) single-domain or dAb fragments, which consist of the VH domain; and (vi) isolated complementarity-determining regions (CDR) or (vii) combinations of two or more isolated CDRs that can be optionally connected by synthetic linkers. Additionally, although the two domains of an Fv fragment, VL and VH, are encoded by separate genes, recombinant methods can be used to connect them with a synthetic linker, thereby producing a single protein chain in which the VL and VH regions pair to form a monovalent molecule (known as a single-chain Fv (scFv)). Such single-chain antibodies are also intended to be included within the term "antigen-binding fragment." Such antibody fragments are obtained using conventional techniques known to those skilled in the art and are functionally screened in the same manner as full-length antibodies. Antigen-binding portions may be produced by recombinant DNA technology or by enzymatic or chemical cleavage of full-length immunoglobulins. Antibodies can be of various isotypes, such as IgG (e.g., IgG1, IgG2, IgG3, or IgG4 subtypes), IgA1, IgA2, IgD, IgE, or IgM antibodies.

The term "alkyl" refers to a saturated aliphatic hydrocarbon group, which is a linear or branched group including 1 to 20 carbon atoms; preferably, alkyl including 1 to 12 carbon atoms, more preferably, alkyl including 1 to 10 carbon atoms, most preferably, alkyl including 1 to 6 carbon atoms (including 1, 2, 3, 4, 5, or 6 carbon atoms). Non-limiting examples include methyl, ethyl, n-propyl, isopropyl, n-butyl, isobutyl, tert-butyl, sec-butyl, n-amyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, various branched isomers thereof, and the like. More preferably, the alkyl group is a lower alkyl group including 1 to 6 carbon atoms, and non-limiting examples include methyl, ethyl, n-propyl, isopropyl, n-butyl, isobutyl, tert-butyl, sec-butyl, n-amyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. The alkyl group may be substituted or unsubstituted, and when substituted, the substituent can be attached at any available connection point. The substituent is preferably one or more of the following groups: alkyl, alkenyl, alkynyl, alkoxy, alkylthio, alkylamino, halogen, sulfhydryl, hydroxyl, nitro, cyano, cycloalkyl, heterocycloalkyl, aryl, heteroaryl, cycloalkoxy, heterocycloalkyloxy, cycloalkylthio, heterocycloalkylthio, and oxo.

The term "alkoxy" refers to -O-(alkyl) and -O-(unsubstituted cycloalkyl), wherein the alkyl or cycloalkyl is as defined above. Non-limiting examples of alkoxy include: methoxy, ethoxy, propoxy, butoxy, cyclopropoxy, cyclobutoxy, cyclopentyloxy, and cyclohexyloxy. The alkoxy group may be optionally substituted or unsubstituted, and when substituted, the substituent is preferably one or more of the following groups: alkyl, alkenyl, alkynyl, alkoxy, alkylthio, alkylamino, halogen, sulfhydryl, hydroxyl, nitro, cyano, cycloalkyl, heterocycloalkyl, aryl, heteroaryl, cycloalkoxy, heterocycloalkyloxy, cycloalkylthio, and heterocycloalkylthio.

The term "cycloalkyl" refers to a saturated or partially unsaturated monocyclic or polycyclic hydrocarbon substituent, and a cycloalkyl ring includes 3 to 20 carbon atoms; preferably, 3 to 12 carbon atoms, more preferably, 3 to 10 carbon atoms, most preferably, 3 to 8 carbon atoms (including 3, 4, 5, 6, 7, or 8 carbon atoms). Non-limiting examples of monocyclic cycloalkyl groups include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like; and polycyclic cycloalkyl groups include spiro, fused, and bridged cycloalkyl groups.

The term "heterocyclyl" refers to a saturated or partially unsaturated monocyclic or polycyclic hydrocarbon substituent including 3 to 20 ring atoms, where one or more ring atoms are heteroatoms selected from nitrogen, oxygen, or $S(O)_m$ (m is an integer of 0, 1, or 2), but a ring portion of -O-O-, - O-S-, or -S-S- is excluded, and the other ring atoms are carbon. Preferably, the heterocyclic group includes 3 to 12 ring atoms, including 1 to 4 heteroatoms (1, 2, 3, or 4 heteroatoms). More preferably, the cycloalkyl ring includes 3 to 10 ring atoms (including 3, 4, 5, 6, 7, 8, 9, or 10 ring atoms). Non-limiting examples of monocyclic heterocyclic groups include pyrrolidinyl, piperidinyl, piperazinyl, morpholinyl, thiomorpholinyl, homopiperazinyl, and the like. The polycyclic heterocyclic groups include spiro, fused, and bridged

cycloalkyl groups.

The term "spiroheterocyclyl" refers to a polycyclic heterocyclic group in which 5 to 20-membered monocyclic rings share one atom (called a spiro atom), wherein one or more ring atoms are heteroatoms selected from nitrogen, oxygen, or $S(O)_m$ (m is an integer from 0 to 2), and the other ring atoms are carbon. The spiroheterocyclic group can include one or more double bonds, but includes no ring having a fully conjugated π electronic system. The spiroheterocyclic group is preferably 6- to 14-membered, more preferably, 7- to 10-membered. The spiroheterocyclic groups are divided into monospiroheterocyclic groups, dispiroheterocyclic groups and polyspiroheterocyclic groups according to the number of spiro atoms shared between rings, and the monospiroheterocyclic groups and the dispiroheterocyclic groups are preferred. The spiroheterocyclic group is more preferably a 4/4-membered, 4/5-membered, 4/6-membered, 5/5-membered, or 5/6-membered monospiroheterocyclic group. Non-limiting examples of the spiroheterocyclic groups include:

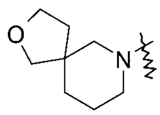 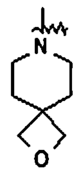 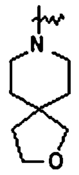 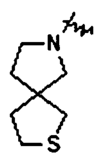 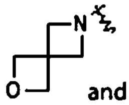 and 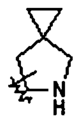 .

The term "fused heterocyclic group" refers to a 5- to 20-membered polycyclic heterocyclic group in which each ring in the system shares a pair of adjacent atoms with another ring in the system, one or more rings can include one or more double bonds, but no ring has a fully conjugated π electronic system, one or more ring atoms are heteroatoms selected from nitrogen, oxygen, or $S(O)_m$ (m is an integer of 0, 1, or 2), and the other ring atoms are carbon. The fused heterocyclic group is preferably 6- to 14-membered, more preferably, 7- to 10-membered (7-membered, 8-membered, 9-membered or 10-membered rings). The fused heterocyclic groups can be divided into bicyclic, tricyclic, tetracyclic, and polycyclic fused heterocyclic groups according to the number of constituent rings. Bicyclic or tricyclic fused heterocyclic groups are preferred, and 5-membered/5-membered or 5-membered/6-membered bicyclic fused heterocyclic groups are more preferred. Non-limiting examples of the fused heterocyclic groups include:

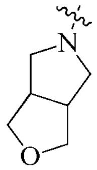 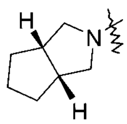 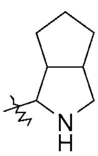 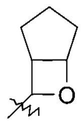 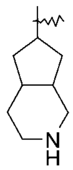, 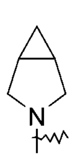, 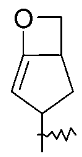, 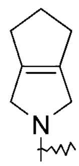, 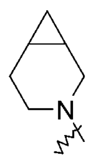, 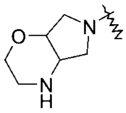,

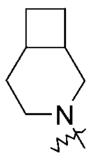, 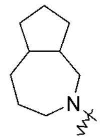, 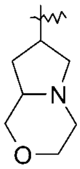, 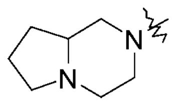, and 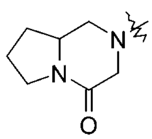 .

The term "bridged heterocyclic group" refers to a 5- to 14-membered polycyclic heterocyclic group in which any two rings share two atoms that are not directly bound, and can include one or more double bonds, but no ring has a fully conjugated π electronic system, one or more ring atoms are heteroatoms selected from nitrogen, oxygen, or $S(O)_m$ (m is an integer of 0, 1, or 2), and the other ring atoms are carbon. The bridged heterocyclic group is preferably 6- to 14-membered, more preferably, 7- to 10-membered (7-membered, 8-membered, 9-membered or 10-membered rings). The bridged heterocyclic groups can be divided into bicyclic, tricyclic, tetracyclic and polycyclic bridged heterocyclic groups according to the number of constituent rings. Bicyclic, tricyclic, or tetracyclic bridged heterocyclic groups are preferred, and bicyclic or tricyclic bridged heterocyclic groups are more preferred. Non-limiting examples of the bridged heterocyclic groups include:

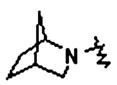 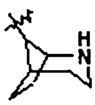 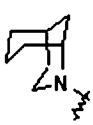 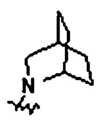

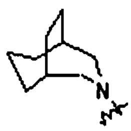 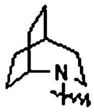 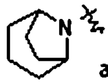 and 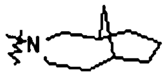 .

The heterocyclic group ring may be fused to an aryl, heteroaryl or cycloalkyl ring, wherein the ring attached to the parent structure is a heterocyclic group, and non-limiting examples include:

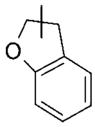 , 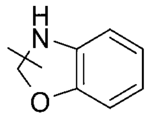 , 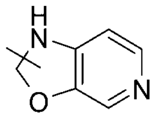 , 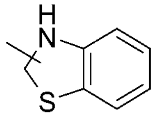 ,

and the like.

The heterocyclic group may be optionally substituted or unsubstituted, and when substituted, the substituent is preferably one or more of the following groups: alkyl, alkenyl, alkynyl, alkoxy, alkylthio, alkylamino, halogen, sulfhydryl, hydroxyl, nitro, cyano, cycloalkyl, heterocycloalkyl, aryl, heteroaryl, cycloalkoxy, heterocycloalkyloxy, cycloalkylthio, heterocycloalkyl thio, and oxo.

The term "aryl" refers to a 6- to 14-membered all-carbon monocyclic or fused polycyclic group (that is, rings that share adjacent pairs of carbon atoms) having a conjugated $\pi$ electronic system, or is preferably 6- to 10-membered (6-membered, 7-membered, 8-membered, 9-membered, or 10-membered) group, for example, phenyl and naphthyl, preferably phenyl. The aryl ring may be fused to heteroaryl, heterocyclyl, or cycloalkyl ring, wherein the ring attached to the parent structure is an aryl ring, and non-limiting examples include:

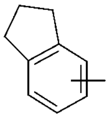 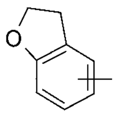 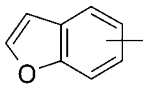 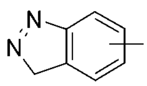 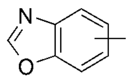 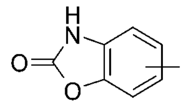 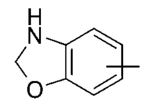 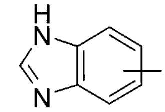 ,

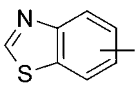 , 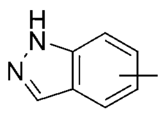 , 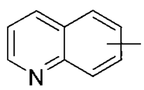 , 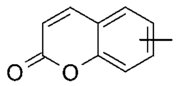 , and 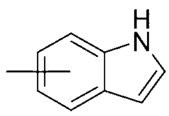 .

The aryl group may be substituted or unsubstituted, and when substituted, the substituent is preferably one or more of the following groups: alkyl, alkenyl, alkynyl, alkoxy, alkylthio, alkylamino, halogen, sulfhydryl, hydroxyl, nitro, cyano, cycloalkyl, heterocycloalkyl, aryl, heteroaryl, cycloalkoxy, heterocycloalkyloxy, cycloalkylthio, and heterocycloalkylthio.

The term "heteroaryl" refers to a heteroaromatic system including 1 to 4 heteroatoms (1, 2, 3 or 4 heteroatoms) and 5 to 14 ring atoms, wherein the heteroatoms are selected from oxygen, sulfur and nitrogen. The heteroaryl group is preferably 5-membered to 10-membered (5-membered, 6-membered, 7-membered, 8-membered, 9-membered, or 10-membered heteroaryl), more preferably, 5-membered or 6-membered, for example, furanyl, thienyl, pyridinyl, pyrrolyl, N-alkyl pyrrolyl, pyrimidinyl, pyrazinyl, imidazolyl, tetrazolyl, and the like. The heteroaryl ring may be fused to an aryl, heterocyclyl, or cycloalkyl ring, wherein the ring attached to the parent structure is a heteroaryl ring, and non-limiting examples include:

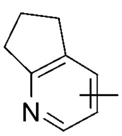 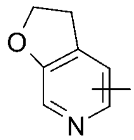 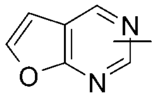 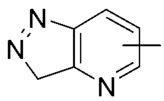 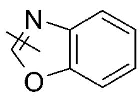 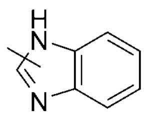 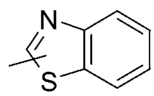

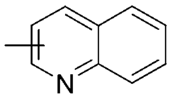 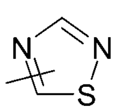 and 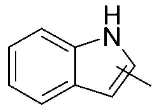 .

The heteroaryl group may be optionally substituted or unsubstituted, and when substituted, the substituent is preferably one or more of the following groups: alkyl, alkenyl, alkynyl, alkoxy, alkylthio, alkylamino, halogen, sulfhydryl, hydroxyl, nitro, cyano, cycloalkyl, heterocycloalkyl, aryl, heteroaryl, cycloalkoxy, heterocycloalkyloxy, cycloalkylthio, and heterocycloalkylthio.

The term "amino-protective group" refers to a group that is used to protect the amino group during reactions at other parts of the molecule, ensuring that the amino group remains unchanged. Non-limiting examples include 9-fluorenyl methoxycarbonyl, tert-butoxycarbonyl, acetyl, benzyl, allyl, p-methoxybenzyl, and the like. These groups can optionally be substituted by 1 to 3 substituents (1, 2, or 3 substituents) selected from halogen, alkoxy, and nitro. The amino-protective group is preferably 9-fluorenyl methoxycarbonyl.

The term "haloalkyl" refers to an alkyl group substituted with one or more halogens, wherein the alkyl is as defined above.

The term "deuterated alkyl" refers to an alkyl group substituted with one or more deuterium atoms, wherein the alkyl is as defined above.

The term "hydroxyalkyl" refers to an alkyl group substituted with one or more hydroxyl groups, wherein the alkyl is as defined above.

The term "hydroxyl" refers to an -OH group.

The term "halogen" refers to fluorine, chlorine, bromine, or iodine.

The term "amino" refers to $-NH_2$.

The term "nitro" refers to $-NO_2$.

The term "cyano" refers to -CN.

The term "amido" refers to -C(O)N(alkyl) or (cycloalkyl), wherein the alkyl and cycloalkyl are as defined above.

The term "carboxylic ester group" refers to-C(O)O(alkyl) or (cycloalkyl), wherein the alkyl and cycloalkyl are as defined above.

The present disclosure also includes various deuterated forms of compounds of formula (I). Each available hydrogen atom connected to a carbon atom can be independently replaced by a deuterium atom. Those skilled in the art can refer to relevant literature to synthesize deuterated forms of compounds of formula (I). In preparing deuterated forms of compounds of formula (I), commercially available deuterated starting materials may be used, or they may be synthesized using conventional techniques employing deuterated reagents, including but not limited to deuterated borane, trideuterated borane in tetrahydrofuran, deuterated lithium aluminum hydride, deuterated iodoethane, deuterated iodomethane, and the like.

"Optional" or "optionally" means that the event or circumstance described can, but need not, occur. This description includes the case where the event or circumstance occurs and the case where it does not. For example, "optionally alkyl-substituted heterocyclic group" means that the alkyl group may or may not be present, including both the scenario where the heterocyclic group is substituted with an alkyl group and where it is not.

"Substituted" refers to one or more hydrogen atoms in the group, preferably up to 5, more preferably 1, 2, or 3 hydrogen atoms being independently replaced by a corresponding number of substituents. It goes without saying that substituents are only in their possible chemical positions. Those skilled in the art can determine, with minimal effort (experimentally or theoretically), which substitutions are possible or not. For example, an amino or hydroxyl group with free hydrogen combined with a carbon atom having unsaturated bonds (e.g., ethylenic bonds) may be unstable.

The term "pharmaceutical composition" refers to a mixture containing one or more of the compounds described herein or their physiologically/pharmaceutically acceptable salts or prodrugs and other chemical components, as well as other components such as physiologically/pharmaceutically acceptable carriers and excipients. The purpose of the pharmaceutical composition is to facilitate the administration to a biological organism, promoting the absorption of the active ingredient and thereby exhibiting biological activity.

The term "pharmaceutically acceptable salt" refers to salts of the ligand-drug conjugates disclosed herein, or the salts of the compounds described herein, which are safe and effective when used in a mammalian body and possess the desired biological activity. The disclosed ligand-drug conjugate contains at least one amino group and can form salts with acids. Non-limiting examples of pharmaceutically acceptable salts include: hydrochloride, hydrobromide, hydroiodide, sulfate, bisulfate, citrate, acetate, succinate, ascorbate, oxalate, nitrate, sorbate, hydrophosphate, dihydrogen phosphate, salicylate, hydrogen citrate, tartrate, maleate, fumarate, formate, benzoate, methanesulfonate, ethanesulfonate, benzenesulfonate, and p-toluenesulfonate.

The term "carrier" as used in the present disclosure refers to a system that can change the way a drug enters the human body and its distribution in the body, control the release rate of the drug, and deliver the drug to the target organ.

Drug carrier release and targeting systems can reduce drug degradation and loss, lower side effects, and improve bioavailability. For example, polymer surfactants that can be used as carriers can self-assemble due to their unique amphiphilic structure, forming various types of aggregates. Preferred examples are micelles, microemulsions, gels, liquid crystals, vesicles, and the like. These aggregates have the ability to encapsulate drug molecules and have good membrane permeability, making them excellent drug carriers.

The term "excipient" refers to additional substances in drug formulations other than the main drug, also known as auxiliary materials. For example, binders, fillers, disintegrants, and lubricants in tablets; the matrix part in semi-solid formulations like ointments and creams; and preservatives, antioxidants, flavoring agents, aromatics, co-solvents, emulsifiers, solubilizers, osmotic agents, colorants, and the like in liquid formulations, can all be referred to as excipients.

The pharmaceutical compositions including the active components may be in forms suitable for oral administration, for example, tablets, lozenges, troches, aqueous or oil suspensions, dispersible powders or granules, emulsions, hard or soft capsules, or syrups or elixirs. Oral compositions may be prepared by any known method for preparing pharmaceutical compositions in the art, and such compositions may include one or more ingredients selected from: sweeteners, flavoring agents, colorants and preservatives to provide appealing and palatable pharmaceutical formulations. Tablets contain active ingredients and suitable non-toxic, pharmaceutically acceptable excipients for tablet preparation. These excipients can be inert excipients such as calcium carbonate, sodium carbonate, lactose, calcium phosphate or sodium phosphate; granulating agents and disintegrants such as microcrystalline cellulose, cross-linked carboxymethyl cellulose sodium, corn starch or alginic acid; binders such as starch, gelatin, polyvinylpyrrolidone or gum arabic; and lubricants such as magnesium stearate, stearic acid or talc. These tablets may be uncoated or coated using known techniques to mask the drug's taste or delay disintegration and absorption in the gastrointestinal tract, providing extended-release effects. For example, water-soluble odor-masking substances such as hydroxypropyl methylcellulose or hydroxypropyl cellulose may be used, or time-extension substances such as ethylcellulose or cellulose acetate butyrate may be used.

Hard gelatin capsules containing active ingredients mixed with inert solid diluents such as calcium carbonate, calcium phosphate, or kaolin, or soft gelatin capsules containing active ingredients mixed with water-soluble carriers such as polyethylene glycol or oil solvents like peanut oil, liquid paraffin, or olive oil can be used as oral formulations.

The aqueous suspensions contain active substances in admixture with suitable excipients for preparing aqueous suspensions. Such excipients are suspending agents such as sodium carboxymethylcellulose, methylcellulose, hydroxypropyl methylcellulose, sodium alginate, polyvinylpyrrolidone, and gum arabic; dispersants or wetting agents which may be naturally occurring phospholipids such as lecithin, or condensation products of alkylene oxide with fatty acids, for example, polyoxyethylene stearate; or condensation products of ethylene oxide with long-chain fatty alcohols, for example, heptadecaethyleneoxy cetanol, or condensation products of ethylene oxide with partial esters derived from fatty acids and hexitol, for example, poly(ethylene oxide) sorbitol monooleate, or condensation products of ethylene oxide and partial esters derived from fatty acids and hexitol anhydrides, for example, polyoxyethylene dehydrated sorbitan monooleate. The aqueous suspensions may contain one or more preservatives such as ethylparaben or n-propylparaben, one or more colorants, one or more flavoring agents, and one or more sweeteners such as sucrose, saccharin, or aspartame.

The oil suspensions may be prepared by suspending the active ingredients in vegetable oils such as peanut oil, olive oil, sesame oil or coconut oil, or mineral oils such as liquid paraffin. The oil suspensions may contain thickeners such as beeswax, hard paraffin, or cetyl alcohol. The sweeteners and flavoring agents may be added to prepare a platable formulation. These compositions can be preserved by adding antioxidants such as butyl hydroxyanisole or $\alpha$-tocopherol.

Dispersible powders and granules for preparing aqueous suspensions by adding water provide the active ingredient in admixture with dispersants or wetting agents, suspending agents, or one or more preservatives. Suitable dispersants or wetting agents and suspensions are described above. Other excipients such as sweeteners, flavoring agents and colorants can also be added. These compositions can be preserved by adding antioxidants such as ascorbic acid.

The pharmaceutical composition of the present invention can also be in a form of an oil-in-water emulsion. The oil phase can be a vegetable oil such as olive oil or peanut oil, or a mineral oil such as liquid paraffin or a mixture thereof. Suitable emulsifiers may be natural phospholipids such as soy lecithin; esters or partial esters derived from fatty acids and hexitol anhydride such as sorbitol monooleate; and condensation products of the partial ester and ethylene oxide, for example polyoxyethylene sorbitol monooleate. Emulsions may also contain sweeteners, flavoring agents, preservatives, and antioxidants. Syrups and elixirs can be prepared with sweeteners such as glycerin, propylene glycol, sorbitol, or sucrose. Such formulations may also include demulcents, preservatives, colorants, and antioxidants.

The pharmaceutical composition of the present invention may be in a form of a sterile injection aqueous solution. Acceptable solvents and vehicles are water, Ringer's injection, and isotonic sodium chloride solution. Sterile injectable preparations can be sterile injectable oil-in-water microemulsions, where the active ingredient is dissolved in the oil phase. For example, the active ingredient can be dissolved in a mixture of soybean oil and lecithin, and then the oil solution can be added to a mixture of water and glycerol to form a microemulsion. Injectable solutions or microemulsions can be

administered to the patient by local bulk injection into the bloodstream, preferably by maintaining a constant circulating concentration of the compound through continuous intravenous administration. For this purpose, continuous intravenous drug delivery devices can be used.

The pharmaceutical compositions of the present invention can be in the form of sterile injectable water or oil suspensions for intramuscular and subcutaneous administration. These suspensions can be formulated using suitable dispersants or wetting agents and suspending agents described above. Sterile injectable preparations can also be sterile injectable solutions or suspensions in non-toxic, parenterally acceptable diluents or solvents, such as solutions prepared in 1,3-butanediol. Additionally, sterile fixed oils can conveniently be used as solvents or suspending media. For this purpose, any bland fixed oil including synthetic mono- or diglycerides can be used. Furthermore, fatty acids such as oleic acid can be used to prepare injectable formulations.

It is well known to those skilled in the art that the dosage of the drug depends on various factors, including but not limited to the following: the activity of the specific compound used, the patient's age, weight, health condition, habits, diet, administration time, administration method, excretion rate, and drug combination. Additionally, the optimal therapeutic regimen, such as the mode of treatment, daily dosage of the general formula compound or pharmaceutically acceptable salt, can be validated according to conventional therapeutic protocols..

## BRIEF DESCRIPTION OF THE DRAWINGS

FIG. 1 shows a tumor growth curve of a tumor-bearing mouse model of NCI-N87 gastric cancer in Test Example 3.

FIG. 2 shows a tumor growth curve of a JIMT-1 mouse model of human breast cancer in Test Example 4.

FIG. 3A shows a growth inhibition curve of Her2-negative 468-luc cells in Test Example 6.

FIG. 3B shows a growth inhibition curve of Her2-positive N87 cells in Test Example 6.

FIG. 3C shows a growth inhibition curve of N87 cells and 468-luc cells under co-incubation in Test Example 6.

FIG. 4 and FIG. 5 show a weight change curve of rats after a single dose of 200 mg/kg in Test Example 7.

FIG. 6A and FIG. 6B show a tumor growth curve of a tumor-bearing mouse model of NCI-N87 gastric cancer in Test Example 5.

## DETAILED DESCRIPTION OF EMBODIMENTS

The compounds of the present invention and their preparation can be further understood through the following examples, which illustrate methods for preparing or using the compounds. However, it is to be understood that these examples do not limit the scope of the present invention. Variations of the present invention, whether currently known or developed in the future, are considered to fall within the scope of the present disclosure and the claims.

The compounds of the present invention are prepared using convenient starting materials and general preparation steps. The present invention provides typical or preferred reaction conditions, such as reaction temperature, time, solvent, pressure, and molar ratios of reactants. However, unless specifically stated otherwise, other reaction conditions may also be adopted. Optimization conditions may vary depending on the specific reactants or solvents used, but in general, the steps and conditions for reaction optimization can be determined.

Additionally, some protective groups may be used in the invention to protect certain functional groups from unnecessary reactions. Protective groups suitable for various functional groups and their protection or deprotection conditions are widely known to those skilled in the art. For example, "Protective groups in Organic Synthesis" by T. W. Greene and G. M. Wuts (3rd edition, Wiley, New York, 1999, and references therein) describes in detail the protection or deprotection of many protective groups.

The isolation and purification of compounds and intermediates are carried out using appropriate methods and steps, such as filtration, extraction, distillation, crystallization, column chromatography, preparative thin-layer chromatography, preparative high-performance liquid chromatography, or a combination of these methods based on specific needs. For specific usage methods, refer to examples described in the present invention. Certainly, other similar isolation and purification methods can also be adopted. Conventional methods (including physical constants and spectral data) can be used for characterization.

The structure of the compound is determined through nuclear magnetic resonance (NMR) and/or mass

spectrometry (MS). NMR shifts are given in units of $10^{-6}$ (ppm). NMR measurements are carried out using a Bruker DPS 400 nuclear magnetic instrument. The assay solvents are deuterated dimethyl sulfoxide (DMSO-$d_6$), deuterated chloroform ($CDCl_3$), deuterated methanol ($CD_3OD$), and deuterated water ($D_2O$), with tetramethylsilane (TMS) as the internal standard.

MS measurements of small molecules are performed using an LC (Waters 2695)/MS (Quattro Premier XE) mass spectrometer (manufacturer: Waters) (Photodiode Array Detector).

MS measurements of ADCs are performed using UPLC-MS (Thermo Fisher Scientific, Dionex UltiMate 3000 UPLC-Q Exactive MS; High resolution mass spectrometer, Thermo Q EXACTIVE HF-X).

For ADC HIC analysis, an Agilent 1200 high-performance liquid chromatograph is used for HIC assays. The chromatographic column is a Thermo MAbPac HIC-Butyl (5 μm, 4.6×100 mm). A mixed solution (pH 7.0) of 1.5 M ammonium sulfate and 25 mM phosphate and isopropanol (95:5) is used as mobile phase A, and 25 mM phosphate solution (pH 7.0) and isopropanol (80:20) is used as mobile phase B to perform gradient elution at a flow rate of 1.0 mL/min.

For ADC SEC analysis, an Agilent 1200 high-performance liquid chromatograph is used for SEC assays. The chromatographic column is a Waters BioResolve SEC mAb (2.5 μm, 7.8×300 mm), and PBS is used as the mobile phase for isocratic elution at a flow rate of 0.5 mL/min.

For ADC RP analysis, an Agilent 1200 high-performance liquid chromatograph is used for HIC assays. The chromatographic column is a Thermo MAbPac RP (4 μm, 3×100 mm). A 0.1% trifluoroacetic acid solution is used as mobile phase A, and acetonitrile, isopropanol, and trifluoroacetic acid (80:20:0.1) is used as mobile phase B for gradient elution at a flow rate of 1.0 mL/min.

For preparative high-performance liquid chromatography, an LC6000 high-performance liquid chromatograph (manufacturer: Beijing Chuangxintongheng Science & Technology Co., Ltd) is used. The chromatographic column is a Daisogel C18 10 μm 100A (30 mm × 250 mm). The mobile phase is acetonitrile/water. Orienda BRIX-2860 is used. Phenomenex Luna C18 (250 × 50 mm × 10 μm) is used as a chromatographic column. The mobile phase is water (0.225% trifluoroacetic acid)-acetonitrile.

The TLC silica gel plate uses Qingdao Marine Chemical GF254 silica gel plate. The specifications for the silica gel plates used in thin-layer chromatography (TLC) are 0.20 mm to 0.25 mm, while the specifications for the preparative thin-layer chromatography separation and purification products are 0.5 mm.

100- to 200-mesh, 200- to 300-mesh, and 300- to 400-mesh silica gel (Qingdao Marine Chemical) are generally used as carriers in column chromatography.

The known starting materials in the present invention can be synthesized by methods known in the art or can be purchased from online malls and Beijing Ouhe, Sigma, J&K Scientific, Yishiming, BePharm Ltd., InnoChem, Energy Chemical, Bidepharm, ChemExpress, Yilai Biotechnology, BirdoTech, and other companies.

Unless otherwise specified in the examples, all reactions can be carried out in a nitrogen atmosphere.

An argon atmosphere or nitrogen atmosphere means that a reaction flask is connected to an argon balloon or a nitrogen balloon with a volume of about 1L.

Reaction solvents, organic solvents, or inert solvents are each described as solvents that do not participate in reactions under the described reaction conditions, including benzene, toluene, acetonitrile, tetrahydrofuran (THF), dimethylformamide (DMF), chloroform, dichloromethane, ether, methanol, N-methylpyrrolidone (NMP), pyridine, and the like. Unless otherwise specified in the embodiments, the solution refers to an aqueous solution.

Chemical reactions described in the present invention are generally carried out under atmospheric pressure. Reaction temperatures are between -78°C and 200°C. The reaction time and conditions are, for example, standard atmospheric pressure, -78°C to 200°C, and approximately 1 to 24 hours. If the reaction lasts overnight, the reaction time is generally 16 hours. In the examples, unless otherwise specified, the reaction temperature is room temperature, namely, 20°C to 30°C.

The reaction process in the examples is monitored using thin-layer chromatography (TLC). The developing solvent systems used in the reactions are: A: Dichloromethane and methanol system, B: Petroleum ether and ethyl acetate system, C: Acetone. The volume ratio of the solvents is adjusted according to the polarity of the compounds.

The eluent systems used in column chromatography for purifying compounds and the developing solvent systems used in thin-layer chromatography include: A: Dichloromethane and methanol system, B: Petroleum ether and ethyl acetate system. The volume ratio of the solvents is adjusted according to the polarity of the compounds, and small amounts of basic or acidic reagents such as triethylamine and trifluoroacetic acid may be added for adjustment.

Unless otherwise defined, all professional and scientific terms used herein have the same meanings as those familiar to those skilled in the field. Moreover, any similar or equivalent methods and materials to those described can be applied to the methods of the present invention.

The compounds of the present invention are prepared according to the exemplary procedures provided herein and the known modifications to those skilled in the art.

Preparation Examples

Preparation Example 1: Preparation of (9R)-9-ethyl-5-fluoro-1,9-dihydroxy-4-methyl-1,2,3,9,12,15-hexahydro-10H,13H-benzo[de]pyrano[3',4:6,7]indolizino[1,2-b]quinolin-10,13-dione (**PY-1**)

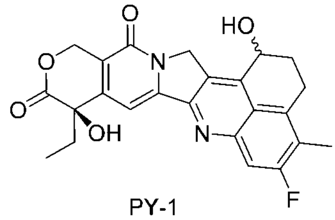

PY-1

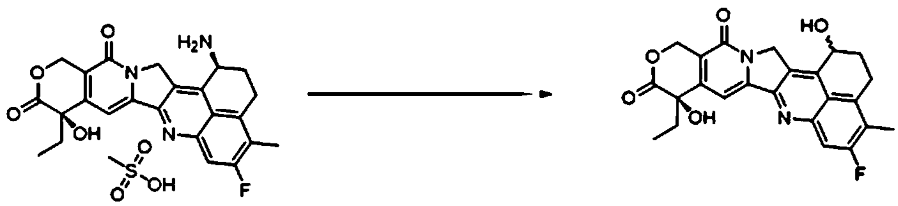

Exatecan

PY-1

$NaNO_2$ (270 mg, 3.91 mmol) was added into a 25 mL three-necked flask, 6 mL of water was added for dissolution, then 1 mL of glacial acetic acid was added, and then an acetic acid solution (3 mL of glacial acetic acid, 3 mL of water) of exatecan mesylate (ChemExpress, 100 mg, 0.188 mmol) was slowly added to the foregoing $NaNO_2$ solution, and the reaction was carried out for 1.5 hours at room temperature. The reaction solution was filtered to collect a solid, the solid was dried to obtain a reddish-brown solid, the reddish-brown solid was isolated and purified by high performance liquid chromatograph (Oriendo, BRIX-2860; column: Phenomenex Luna C18 250 × 50 mm × 10 μm; mobile phase: water (0.5% formic acid)-acetonitrile, eluted from 40% to 80%), and lyophilized, to obtain 12 mg of reddish powder with a yield of 14%.

LCMS (ESI): m/z, 437.1$[M+1]^+$.

$^1H$ NMR (400 MHz, DMSO-d6) δ 7.75 (d, *J* = 11.0 Hz, 1H), 7.31 (s, 1H), 6.50 (s, 1H), 6.04- 5.83 (m, 1H), 5.48-5.29 (m, 4H), 5.16 (d, *J* = 5.6 Hz, 1H), 3.23 (dd, *J* = 13.4, 8.6 Hz, 2H), 3.09-2.98 (m, 1H), 2.37 (s, 3H), 2.02 (dd, *J* = 16.4, 6.8 Hz, 1H), 1.86 (dq, *J* = 14.0, 7.2 Hz, 2H), 0.88 (t, *J* = 7.3 Hz, 3H).

Preparation Example 2: Preparation of (9S)-9-ethyl-5-fluoro-9-hydroxy-4-methyl-10,13-dioxy-2,3,9,10,13,15-hexahydro-1H,12H-benzo[de]pyrano[3',4':6,7]indolizino[1,2-b]quinolin-1-yl(2-hydroxyethyl)carbamate (**PY-1AB**)

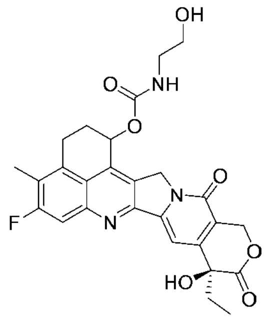

PY-1AB

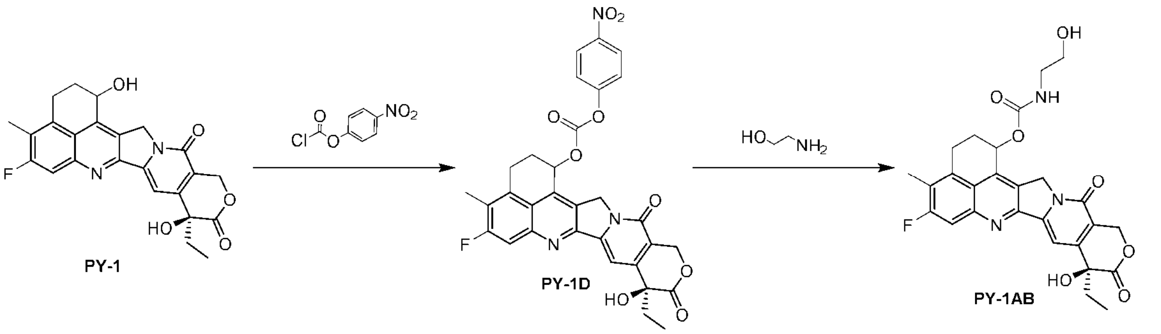


PY-1 PY-1D PY-1AB

Step 1: Preparation of (9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl(4-nitrophenyl)carbonate (**PY-1D**)

The compound **PY-1** (40.0 mg, 0.09 mmol, 1.0 eq) and triethylamine (27.3 mg, 0.27 mmol, 3.0 eq) were dissolved in methylene chloride (5 mL), 4-nitrophenyl chloroformate (36.2 mg, 0.18 mmol, 2.0 eq) was added at 0°C, and then the mixture was heated to 35°C and stirred for 3 hours. The reaction solution was cooled to room temperature and then concentrated to obtain a crude yellow solid (60 mg of crude product; purity: 49.3%; 0.05 mmol; yield: 55.6%).

LCMS: RT = 0.909 min, MS (ESI) m/z =602.1 $[M+H]^+$.

Step 2: Preparation of (9S)-9-ethyl-5-fluoro-9-hydroxy-4-methyl-10,13-dioxy-2,3,9,10,13,15-hexahydro-1H,12H-benzo[de]pyrano[3',4':6,7]indolizino[1,2-b]quinolin-1-yl(2-hydroxyethyl)carbamate (**PY-1AB**)

The compound **PY-1D** (0.05 mmol, 1.0 *eq*) and triethylamine (10.1 mg, 0.1 mmol, 2.0 *eq*) were dissolved in methylene chloride (5 mL), ethanolamine (31.4 mg, 0.04 mmol, 1.1 *eq*) was added at 0°C, and then the mixture was stirred for 3 hours at 25°C. The reaction solution was cooled and concentrated to obtain a crude product, and the crude product was purified by reversed-phase chromatography (column: Welch Xtimate C18 150 × 30 mm× 5 μm; mobile phase: [water(FA)-ACN]; gradient: 6%-46% B over 25 min) to obtain the target product **PY-1AB** (2.23 mg, 0.004 mmol, yield: 8.00%) as a white solid.

LCMS: RT = 1.197 min, MS (ESI) m/z =524.2 $[M+H]^+$.

$^1$HNMR (400MHz, DMSO-$d^6$) δ ppm 7.81 (d, *J* = 10.8 Hz, 1 H), 7.45 (t, *J* = 5.6 Hz, 1 H), 7.33 (s, 1 H), 6.52 (s, 1 H), 6.27 (s, 1 H), 5.44 - 5.24 (m, 4 H), 4.70 (t, *J* = 5.6 Hz, 1 H), 3.48-3.44 (m, 2 H), 3.22-3.12 (m, 4 H), 2.40 (s , 3 H), 2.20-2.17 (m, 1 H), 1.91-1.86 (m, 2 H), 1.27-1.22 (m, 1 H), 0.88 (t, *J* = 7.2 Hz , 3 H).

Preparation Example 3: Preparation of (9S)-9-ethyl-5-fluoro-9-hydroxy-1-(hydroxymethyl)-4-methyl-1,2,3,9,12,15-hexahydro-10H,13H-benzo[de]pyrano[3',4':6,7]indolizino[1,2-b]quinolin-10,13-dione (**PY-2**) and (S)-9-ethyl-5-fluoro-9-hydroxy-4-methyl-1-methylene-1,2,3,9,12,15-hexahydro-10H,13H-benzo[de]pyrano[3',4':6,7]indolizino[1,2-b]quinolin-10,13-dione (**PY-2b**)

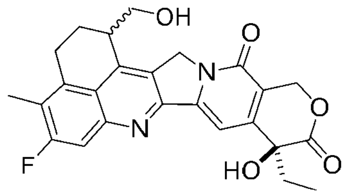

PY-2

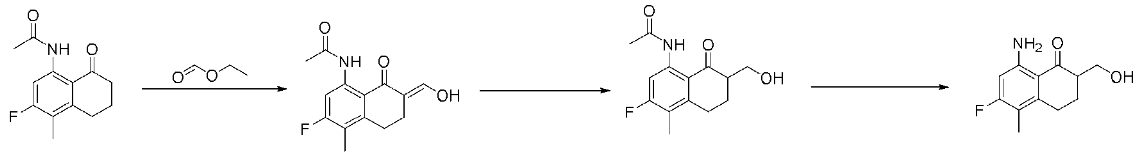

PY-21 PY-22 PY-23 PY-24

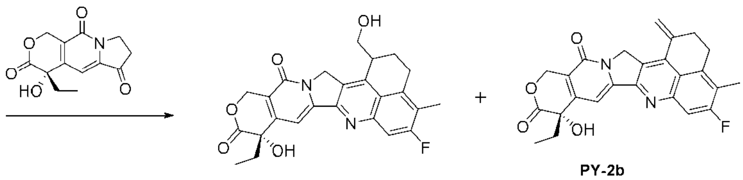

PY-2 PY-2b

Step 1: Preparation of (E)-N-(3-fluoro-7-(hydroxymethylene)-4-methyl-8-oxo-5,6,7,8-tetrahydronaphthalene-1-yl)acetamide (**PY-22**)

N-(3-fluoro-4-methyl-8-oxo-5,6,7,8-tetrahydronaphthalene-1-yl)acetamide (PY-21) (1.0g, 4.25 mmol, 1.0 eq) was dissolved in THF (20 mL) in a 100 mL three-necked flask and cooled to 0°C in ice water, t-BuOK (12.75 mmol, 1 M, 12.75 mL, 3 eq) was slowly added, the system was maintained at a temperature of 0°C and stirred for 30 minutes, then ethyl formate (787 mg, 10.63 mmol, 2.5 eq) was added to the reaction system and the reaction system was stirred for 2 hours. Ammonium chloride (20 mL) was added for quenching, and the reaction system was extracted with ethyl acetate, dried with anhydrous sodium sulfate, filtered, and concentrated under reduced pressure to obtain the yellow solid compound (E)-N-(3-fluoro-7-(hydroxymethylene)-4-methyl-8-oxo-5,6,7,8-tetrahydronaphthalene-1-yl)acetamide (1.22g, yield: 95%).

LCMS(ESI): m/z, 264.1$[M+1]^+$.

Step 2: Preparation of N-(3-fluoro-7-(hydroxymethyl)-4-methyl-8-oxy-5,6,7,8-tetrahydronaphthalene-1-yl)acetamide (**PY-23**)

$PtO_2$ (69.00 mg, 303.90 μmol, 0.2 eq) was added to the solution of compound (E)-N-(3-fluoro-7-(hydroxymethylene)-4-methyl-8-oxo-5,6,7,8-tetrahydronaphthalene-1-yl)acetamide (PY-22) (400 mg, 1.52 mmol, 1.0 eq) in methanol (50 mL) under nitrogen atmosphere, nitrogen gas was displaced by hydrogen gas three times, the reaction system reacted under hydrogen atmosphere for 24 hours (40 °C), remaining raw materials were detected, $PtO_2$ (69.00 mg, 303.90 μmol, 0.2 eq) was added again and the reaction system further reacted under hydrogen atmosphere for 24 hours. The reaction system was filtered, a filtrate was concentrated under reduced pressure, a residue was purified by silica gel column chromatography (eluent: ethyl acetate/n-hexane = 1:20-1:5) to obtain the yellow solid compound N-(3-fluoro-7-(hydroxymethyl)-4-methyl-8-oxy-5,6,7,8-tetrahydronaphthalene-1-yl)acetamide (324 mg, yield: 63%).

LCMS(ESI):m/z, 266.1$[M+1]^+$.

Step 3: Preparation of 8-amino-6-fluoro-2-(hydroxymethyl)-5-methyl-3,4-dihydronaphthalene-1(2H)-one (**PY-24**)

$SOCl_2$ (2.71 mmol, 1 M/L, 2.71 mL, 4.0 eq) was added to a solution of compound N-(3-fluoro-7-(hydroxymethyl)-4-methyl-8-oxy-5,6,7,8-tetrahydronaphthalene-1-yl)acetamide (PY-23) (180 mg, 0.68 μmol, 1.0 eq) an anhydrous MeOH (15 mL) under an ice water bath, after addition, nitrogen gas displacement was performed three times and the reaction system was stirred at 50°C for 60 minutes. The reaction system was concentrated under reduced pressure, and the residue was purified by preparative high performance liquid chromatograph (Oriendo, BRIX-2860; column: Phenomenex Luna C18 250 × 50 mm × 10 μm; mobile phase: water (0.225% trifluoroacetic acid)-acetonitrile, eluted from 40% to 80%) to obtain a light yellow solid compound 8-amino-6-fluoro-2-(hydroxymethyl)-5-methyl-3,4-dihydronaphthalene-1(2H)-one (28.5 mg, yield: 25%).

LCMS(ESI): m/z, 264.1[M+1]$^+$.

$^1$H NMR (400 MHz, DMSO-*d*$^6$) δ 7.43 (brs, 2H), 6.35 (d, *J* = 12.0 Hz, 1H), 3.74 (dd, *J* = 10.8, 4.4 Hz, 1H), 3.60 (dd, *J* = 10.8, 7.2 Hz, 1H), 2.90 (dt, *J* = 17.2, 4.8 Hz, 1H), 2.75-2.54 (m, 2H), 2.18-2.08 (m, 1H), 1.98 (s, 3H), 1.84-1.79 (m, 1H).

Step 4: Preparation of (9S)-9-ethyl-5-fluoro-9-hydroxy-1-(hydroxymethyl)-4-methyl-1,2,3,9,12,15-hexahydro-10H,13H-benzo[de]pyrano[3',4':6,7]indolizino[1,2-b]quinolin-10,13-dione (**PY-2**) and (S)-9-ethyl-5-fluoro-9-hydroxy-4-methyl-1-methylene-1,2,3,9,12,15-hexahydro-10H,13H-benzo[de]pyrano[3',4':6,7]indolizino[1,2-b]quinolin-10,13-dione (**PY-2b**)

(S)-4-ethyl-4-hydroxy-7,8-dihydro-1H-pyrano[3,4-f]indolizino-3,6,10(4H)-trione (26 mg, 98.5 μmol, 1.0 eq) and PPTS (5 mg, 19.70 μmol, 0.2 eq) were added to a solution of compound 8-amino-6-fluoro-2-(hydroxymethyl)-5-methyl-3,4-dihydronaphthalene-1(2H)-one (22 mg, 98.5 μmol, 1.0 eq) in xylene (5 mL). After addition, nitrogen gas displacement was performed three times and the reaction system was stirred at 140°C for 3 hours. The reaction system was concentrated under reduced pressure, and the residue was purified by preparative high performance liquid chromatograph (Oriendo, BRIX-2860; column: Phenomenex Luna C18 250 × 50 mm × 10 μm; mobile phase: water (0.225% trifluoroacetic acid)-acetonitrile, 40% to 80%) to obtain a yellow solid PY-2 (3.42 mg, yield: 7%) and dark brown sticky solid PY-2b (5.31 mg, yield: 10%).

**PY-2:**

LCMS(ESI): m/z, 451.2[M+1]$^+$.

$^1$H NMR (400 MHz, DMSO-*d*$^6$) δ 11.80 (s, 1H), 8.14 (s, 1H), 7.17 (d, *J* = 12.4 Hz, 1H), 6.73 (s, 1H), 6.46 (s, 1H), 6.11 (d, *J* = 2.0 Hz, 1H), 5.61 (d, *J* = 2.0 Hz, 1H), 5.36 (s, 1H), 4.73 (d, *J* = 2.4 Hz, 1H), 2.95 (t, *J* = 6.8 Hz, 2H), 2.79 (t, *J* = 6.8 Hz, 2H), 2.25 - 2.03 (m, 5H), 2.00 (d, *J* = 2.0 Hz, 1H), 1.86-1.76 (m, 2H), 0.85 (t, *J* = 7.2 Hz, 3H).

**PY-2b:**

LCMS(ESI): m/z, 433.2[M+1]$^+$.

$^1$H NMR (400 MHz, $CDCl_3$) δ 7.67 (d, *J* = 10.8 Hz, 1H), 7.62 (s, 1H), 5.77-5.70 (m, 3H), 5.38-5.30 (m, 3H), 3.77 (s, 1H), 3.22-3.19 (m, 2H), 2.85-2.82 (m, 2H), 2.41 (s, 3H), 1.96 - 1.82 (m, 2H), 1.04 (t, *J*= 8.0 Hz, 3H).

Preparation Example 4: Preparation of (1S,9S)-9-ethyl-5-fluoro-9-hydroxy-1-((2-hydroxymethyl)amino)-4-methyl-1,2,3,9,12,15-hexahydro-10H,13H-benzo[de]pyrano[3',4':6,7]indolizino[1,2-b]quinolin-10,13-dione (**PY-4**)

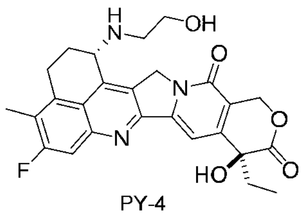

PY-4

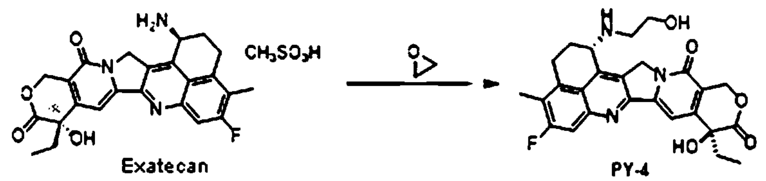


Exatecan

PY-4

Exatecan mesylate (ChemExpress) (50 mg, 114.82 μmol) was dissolved in DMF (2 mL), ethylene oxide (1 M, 1.15 mL) and glacial acetic acid (3.45 mg, 57.41 μmol) were added to the solution, and the reaction system was stirred at 90°C for 16 hours. The reaction solution was cooled to room temperature, 20 mL of water was added to the system, and ethyl acetate was used for extraction (20 mL × 3). After being combined, the organic phases were rinsed with saturated brine, dried with anhydrous $Na_2SO_4$, and filtered, and the filtrate was concentrated under reduced pressure. The residue was purified by preparative high performance liquid chromatograph (Oriendo, BRIX-2860; column: Phenomenex Luna C18 250 × 50 mm × 10 μm; mobile phase: water (0.225% trifluoroacetic acid)-acetonitrile, eluted from 40% to 80%), 1

drop of hydrochloric acid was added to the resulting solution and the mixture was lyophilized to obtain a yellow solid compound PY-4 (5.82 mg, yield: 21%).

LCMS: $[M+H]^+$ = 480.9.

$^1H$ NMR(DMSO-$d^6$, 400 MHz): $\delta$ (ppm) 0.72 - 0.83 (t, $J$=14.7 Hz, 3H), 1.72 - 1.85 (m, 2H), 2.13-2.22 (s, 3H), 2.43-2.56 (m, 1H), 2.80- 3.06 (m, 2H), 3.18 - 3.31 (s, 2H), 3.31-3.40 (m, 1H), 3.74-3.82 (m, 2H), 5.08-5.13 (s, 1H), 5.18 - 5.41 (m, 4H), 7.10 -7.14 (s, 1H), 7.15-7.21 (s, 1H).

Preparation Example 5: Preparation of 2-hydroxyethyl((1S,9S)-9-ethyl-5-fluoro-9-hydroxy-4-methyl-10,13-dioxy-2,3,9,10,13,15-hexahydro-1H,12H-benzo[de]pyran[3',4:6,7]indolizino[1,2-b]quinolin-1-yl)carbamate (**PY-4Car**)

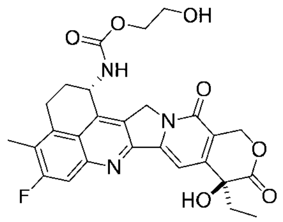

PY-4Car

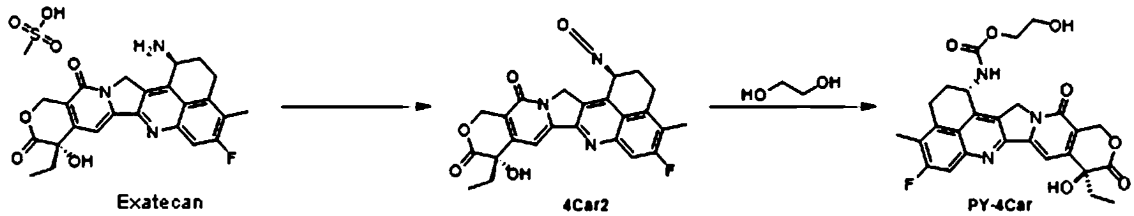

Exatecan 4Car2 PY-4Car

Step 1: Preparation of (1S,9S)-9-ethyl-5-fluoro-9-hydroxy-1-isocyanate-4-methyl-1,2,3,9,12,15-hexahydro-10H,13H-benzo[de]pyrano[3',4:6,7]indolizino[1,2-b]quinolin-10,13-dione (**4Car2**)

At 0°C, three equivalents of triethylamine (14 mg, 0.14 mmol) and 1 equivalent of triphosgene (14 mg, 0.046 mmol) were added to a solution of exatecan mesylate (20 mg, 0.046 mmol) in dichloromethane (2 mL) and the reaction system was stirred at 0°C for three hours. The reaction system was concentrated under reduced pressure to obtain 20 mg of crude product, and the crude product was directly used for the next reaction.

Step 2: Preparation of 2-hydroxyethyl((1S,9S)-9-ethyl-5-fluoro-9-hydroxy-4-methyl-10,13-dioxy-2,3,9,10,13,15-hexahydro-1H,12H-benzo[de]pyran[3',4:6,7]indolizino[1,2-b]quinolin-1-yl)carbamate (PY-4Car)

The compound (1S,9S)-9-ethyl-5-fluoro-9-hydroxy-1-isocyanate-4-methyl-1,2,3,9,12,15-hexahydro-10H,13H-benzo[de]pyrano[3',4]:6,7]indolizino[1,2-b]quinolin-10,13-dione (4Car2) (20 mg, 0.043 mmol) was dissolved in 1 mL of dichloromethane, 10 equivalents of ethylene glycol (27 mg, 0.43 mmol) were added dropwise, and the reaction system was stirred for 3 hours at room temperature. The reaction system was concentrated under reduced pressure. The residue was purified by reversed-phase preparative chromatograph (Oriendo, BRIX-2860; column: Phenomenex Luna C18 250 × 50 mm × 10 μm; mobile phase: water (0.225% trifluoroacetic acid)-acetonitrile, eluted from 40% to 80%), to obtain 6.5 mg of a yellow solid compound **PY-4Car.**

LCMS: m/z = 524.2 $[M+1]^+$.

$^1H$ NMR (400 MHz, DMSO-$d^6$) $\delta$ 8.43 (s, 1 H), 8.00 (d, $J$ = 8.4 Hz, 1 H), 7.78 (d, $J$ = 10.8 Hz, 1 H), 7.31 (s, 1 H), 6.55 (s, 1 H), 5.43 (s, 2 H), 5.24 (d, $J$ = 7.20 Hz, 2 H), 4.81 (t, $J$ = 5.2 Hz, 1 H), 3.97-4.17 (m, 2 H), 3.61 (d, $J$=3.6 Hz, 2 H), 3.02-3.18 (m, 2 H), 2.30-2.41 (m, 3 H), 2.05 - 2.28 (m, 2 H), 1.75-1.94 (m, 2 H), 0.87 (t, $J$ = 7.2 Hz, 3 H).

Preparation Example 6: Preparation of 4-hydroxybutyl((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamate (**PY-4Car2**)

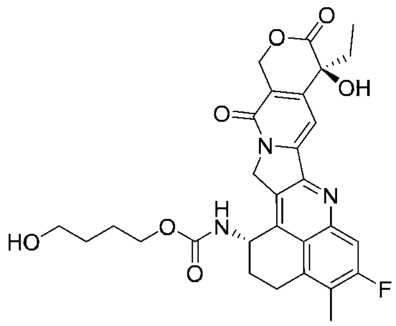

PY-4Car2

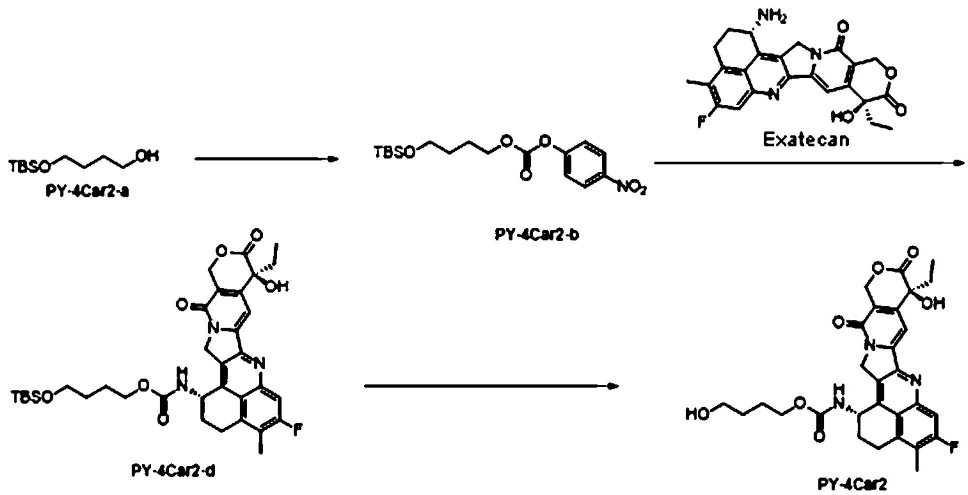


PY-4Car2-a

PY-4Car2-b

PY-4Car2-d

PY-4Car2

Step 1: Preparation of 4-((tert-butyldimethylsilyl)oxy)butyl(4-nitrophenyl)carbonate (**PY-4car2-b**)

N,N-diisopropylethylamine (2.43 mL, 14.68 mmol, 3 eq) was added to a solution of 4-((tertbutyldimethylsilyl)oxy) butyl-1-ol (1g, 4.89 mmol, 1 eq) and bis(p-nitrobenzene) carbonate (2.98g, 9.79 mmol, 2 eq) in N,N-dimethylformamide (15 mL), and the mixture was stirred overnight at room temperature. The reaction solution was concentrated, diluted with dichloromethane (10 mL), the organic phase was washed with water (10 mL) 3 times and washed with saturated brine (10 mL) once, and the organic phase was dried with anhydrous sodium sulfate, filtered, and concentrated. The residue was purified by silica gel column chromatography (eluent: ethyl acetate/petroleum ether = 1:10, dichloromethane/petroleum ether = 40%) to obtain the product 4-((tertbutyldimethylsilyl)oxy)butyl(4-nitrophenyl)carbonate (**PY-4car2-b**) (1.63g, yield = 90.2%).

LCMS(ESI): m/z, 370[M+H]$^+$.

Step 2: Preparation of 4-((tert-butyldimethylsilyl)oxy)butyl((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamate (**PY-4car2-d**)

Triethylamine (20.92 μL, 150.48 μmol, 4 eq), compound PY-4car2-b (18.1 mg, 48.92 μmol, 1.3 eq) and 1-hydroxybenzotriazole (2.4 mg, 18.81 μmol, 0.5 eq) were added to a solution of exatecan (20 mg, 37.62 μmol, 1 eq, mesylate, ChemExpress) and N,N-diisopropylethylamine (6.22 μL, 37.62 μmol, 1 eq) in dichloromethane (6 mL), the resulting solution was stirred for 48 hours at room temperature and concentrated, and the residue was purified by high performance liquid chromatograph (preparative chromatograph manufacturer: Oriendo, model: BRIX-2860, column: Welch Xtimate C18 250 × 30 mm × 10 μm, mobile phase: water (0.225% HCOOH)-acetonitrile, an elution ratio of acetonitrile: 50% to 60%), to obtain a target compound **PY-4car2-d** (15 mg, yield = 59.8%).

LCMS(ESI): m/z, 666[M+H]$^+$.

Step 3: Preparation of 4-hydroxybutyl((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamate (**PY-4Car2**)

Triethylamine trihydrofluoride (14.69 μL, 90.12 μmol, 4 eq) was added to a solution of compound **PY-4car2-d** (15

mg, 22.53 μmol, 1 eq) in tetrahydrofuran (3 mL) under an ice water bath, the resulting solution was stirred for 5 minutes and then stirred at 25°C overnight, and the residue was purified by reversed-phase preparative chromatograph (preparative chromatograph manufacturer: SHIMADZU, model: LC-20AP, column: YMC-Triart Prep C18 250 × 30 mm × 10 μm, mobile phase: water (0.225% HCOOH)-acetonitrile, an elution ratio of acetonitrile: 20% to 55%), to obtain a target compound **PY-4Car2** (3.77 mg, yield = 30.34%).

LCMS(ESI): m/z, 552[M+H]$^+$.

$^1$H NMR (400 MHz, DMSO-$d_6$) δ 7.91 (d, *J* = 8.8 Hz, 1H), 7.78 (d, *J* = 10.8 Hz, 1H), 7.31 (s, 1H), 6.51 (s, 1H), 5.42 (s, 2H), 5.24 (d, *J* = 4.2 Hz, 2H), 4.41 (t, *J* = 5.2 Hz, 1H), 4.15-4.03 (m, 2H), 3.43 (q, *J* = 6.2 Hz, 2H), 3.29 - 3.06 (m, 3H), 2.38 (d, *J* = 1.8 Hz, 3H), 2.25-2.08 (m, 2H), 1.93-1.81 (m, 2H), 1.65 (p, *J* = 6.8 Hz, 2H), 1.50 (p, *J* = 6.6 Hz, 2H), 0.87 (t, *J* = 7.2 Hz, 3H). $^{19}$F NMR (377 MHz, DMSO-$d_6$) δ -111.37.

Preparation Example 7: Preparation of 3-hydroxypropyl((1S,9S)-9-ethyl-5-fluoro-9-hydroxy-4-methyl-10,13-dioxo-2,3,9,10,13,15-hexahydro-1H,12H-benzo[d]pyrano[3',4':6,7]indolizino[1,2-b]quinolin-1-yl)carbamate (**PY-4Car3C**)

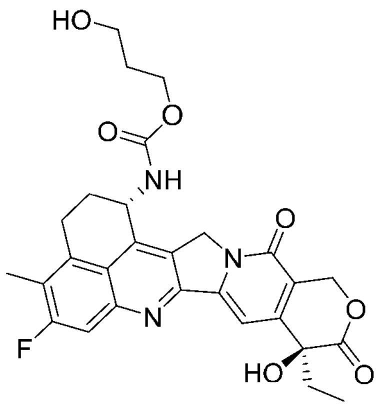

**PY-4Car 3C**

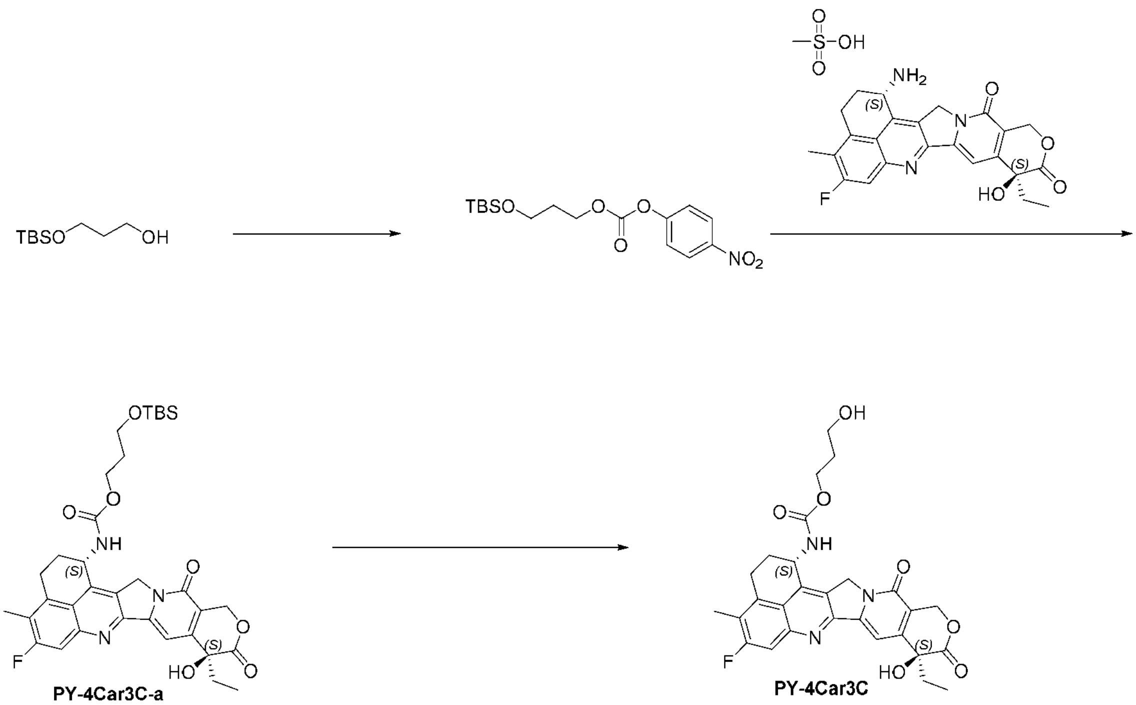

**PY-4Car3C-a**

**PY-4Car3C**

Step 1: Preparation of compound 3-((tert-butyldimethylsilyl)oxy)propyl(4-nitrophenyl)carbonate

Bis(p-nitrobenzene)carbonate (1.28g, 852.31 μL, 4.20 mmol, 2 eq) and DIPEA (543.14 mg, 694.55 μL, 4.20

mmol, 2 eq) were added to a solution of compound 3-((tert-butyldimethylsilyl)oxy)-propanol (400 mg, 2.10 mmol, 1 eq) in DMF (5 mL) at 0°C and the resulting solution was stirred at room temperature (25°C) for 16 hours. The reaction solution was added into 60 mL of water and extracted with ethyl acetate 3 times, 20 mL of ethyl acetate was added each time, and the organic phases were combined, washed with brine (60 mL), dried with anhydrous sodium sulfate, and filtered. The filtrate was spun dry to obtain a crude product, and the crude product was purified by silica gel column chromatography (eluent: ethyl acetate/petroleum ether = 1:30-1:20) to obtain a light yellow oily compound 3-((tert-butyldimethylsilyl)oxy) propyl(4-nitrophenyl)carbonate (740 mg, yield: 99.07%).

$^1$H NMR (400 MHz, Chloroform-d) δ 8.30-8.26 (m, 2H), 7.40-7.35 (m, 2H), 4.41 (t, *J* = 6.4 Hz, 2H), 3.76 (t, *J* = 5.9 Hz, 2H), 1.99-1.93 (m, 2H), 0.90 (s, 9H), 0.07 (s, 6H).

Step 2: Preparation of compound PY-4Car 3C-a

HOBt (10.17 mg, 99.96%, 6.78 μL, 75.24 μmol, 1 eq) and DIPEA (29.17 mg, 99%, 37.31 μL, 225.73 μmol, 3 eq) were added to a solution of compound 3-((tert-butyldimethylsilyl)oxy)propyl(4-nitrophenyl)carbonate (32.10 mg, 90.29 μmol, 1.2 eq) and exatecan mesylate (40 mg, 75.24 μmol, 1 eq) in DMF (2 mL), and after addition, the resulting solution was stirred at room temperature (25°C) for 1 hour, and LCMS showed that the reaction ended. The reaction solution was purified by high performance liquid chromatograph (preparative chromatograph manufacturer: Oriendo, model: BRIX-2860, column: Welch Xtimate C18 150 × 21.2 mm × 5 μm, mobile phase: water (0.225% HCOOH)-acetonitrile, an elution ratio of water: 50% to 80%) and the reaction solution was lyophilized, to obtain a light yellow solid compound PY-4Car 3C-a (45 mg, yield: 91.75%).

LCMS: m/z = 652.3 [M+1]$^+$; Rt = 3.098 min.

Step 3: Preparation of compound PY-4Car 3C

Triethylamine trihydrofluoride (499.70 mg, 98%, 505.26 μL, 3.04 mmol, 50 eq) was added to a solution of compound PY-4Car3C-a (45 mg, 60.75 μmol, 1 eq) in THF (2 mL) at room temperature, and after addition, the reaction solution was stirred at 40°C for 10 hours. LCMS showed that the reaction ended. The reaction solution was purified by high performance liquid chromatograph (preparative chromatograph manufacturer: SHIMADZU, model: LC-20AP, column: Welch Xtimate C18 250 × 30 mm × 10 μm, mobile phase: water (0.225% HCOOH)-acetonitrile, an elution ratio of water: 30% to 60%) and the reaction solution was lyophilized, to obtain a yellow solid compound PY-4Car3C(27.40 mg, yield: 83.90%, purity: 98.19%).

LCMS: m/z = 538.2 [M+1]$^+$; Rt = 1.508 min.

$^1$H NMR (400 MHz, DMSO-$d_6$) δ 7.92 (d, *J* = 8.8 Hz, 1H), 7.77 (d, *J* = 10.9 Hz, 1H), 7.30 (s, 1H), 6.52 (s, 1H), 5.42 (s, 2H), 5.23 (d, *J* = 6.4 Hz, 3H), 4.51 (s, 1H), 4.20-4.15 (m, 1H), 4.13-4.05 (m, 1H), 3.50 (t, *J* = 6.4 Hz, 2H), 3.23 (s, 1H), 3.14-3.07 (m, 1H), 2.37 (d, *J* = 1.8 Hz, 3H), 2.24-2.09 (m, 2H), 1.92-1.82 (m, 2H), 1.76 (p, *J* = 6.5 Hz, 2H), 0.87 (t, *J* = 7.3 Hz, 3H).

Preparation Example 8: Preparation of (1S,9S)-9-ethyl-5-fluoro-9-hydroxy-1-(2-hydroxyethyl)(methyl)amino)-4-methyl-1,2,3,9,12,15-hexahydro-10H,13H-benzo[de]pyrano[3',4:6,7]indolizino[1,2-b]quinolin-10,13-dione (**PY-5**)

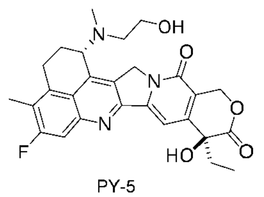

PY-5

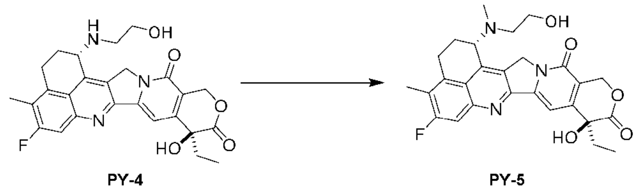

PY-4 PY-5

The compound (1S,9S)-9-ethyl-5-fluoro-9-hydroxy-1-((2-hydroxymethyl)amino)-4-methyl-1,2,3,9,12,15-hexahydro-10H,13H-benzo[de]pyrano[3',4':6,7]indolizino[1,2-b]quinolin-10,13-dione (**PY-4**) (25 mg, 52.14 μmol) was dis-

solved in methanol (2 mL), paraformaldehyde (7.83 mg, 260.69 μmol) and glacial acetic acid (626.19 μg, 10.43 μmol) were added to the solution, and the reaction system was stirred at 60°C for two hours. Sodium cyanoborohydride (16.38 mg, 260.69 μmol) was added and the reaction system was stirred at 30°C for 16 hours. The reaction solution was cooled to room temperature, 20 mL of saturated ammonium chloride solution was added to the system, and ethyl acetate was used for extraction (20 mL × 3). After being combined, the organic phases were rinsed with saturated brine, dried with anhydrous $Na_2SO_4$, and filtered, and the filtrate was concentrated under reduced pressure. The residue was purified by preparative high performance liquid chromatograph (Oriendo, BRIX-2860; column: Phenomenex Luna C18 250 × 50 mm × 10 μm; mobile phase: water (0.225% trifluoroacetic acid)-acetonitrile, eluted from 40% to 80%), to obtain a cleansed product solution, 1 drop of hydrochloric acid was added to the solution and the mixture was lyophilized to obtain a yellow solid PY-5 (7.63 mg, yield: 29.65%).

LCMS: $[M+H]^+$ = 494.8.

$^1H$ NMR(deuterated dimethyl sulfoxide, 400 MHz): δ (ppm) 0.65-0.81 (t, J=7.4 Hz, 3H), 1.73-1.84 (m, 2H), 2.05-2.22 (s, 3H), 2.48-2.57 (m, 1H), 2.57-2.69 (s, 3H), 2.93-3.15 (m, 2H), 3.19-3.31 (m, 1H), 3.34-3.42 (m, 1H), 3.78-3.92 (m, 2H), 5.08-5.44 (m, 4H), 7.12-7.14 (s, 1H), 7.14-7.18 (d, J=10.4 Hz, 1H).

Preparation Example 9: Preparation of N-(1S,9S)-9-ethyl-5-fluoro-9-hydroxy-4-methyl-10,13-dioxy-2,3,9,10,13,15-hexahydro-1H,12H-benzo[de]pyrano[3',4:6,7]indolizino[1,2-b]quinolin-1-yl)-N-(2-hydroxyethyl)formamide (**PY-6A1**)

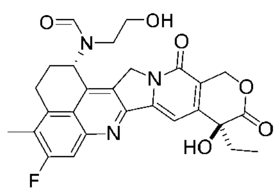

PY-6A1

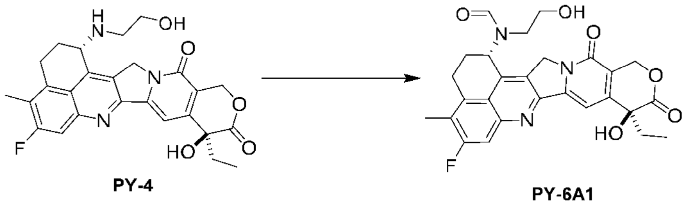

PY-4 PY-6A1

Acetic anhydride (63.87 mg, 625.65 μmol) was added dropwise to a solution of acetic acid (31.31 mg, 521.38 μmol) in anhydrous tetrahydrofuran (1 mL) and the reaction system reacted at 50°C for 1 hour. A solution of the compound (1S,9S)-9-ethyl-5-fluoro-9-hydroxy-1-((2-hydroxymethyl)amino)-4-methyl-1,2,3,9,12,15-hexahydro-10H,13H-benzo [de]pyrano[3',4':6,7]indolizino[1,2-b]quinolin-10,13-dione (**PY-4**) (100 mg, 208.55 μmol) in anhydrous tetrahydrofuran (5 mL) was added to the reaction system, and the reaction system reacted at 20°C for 4 hours. 20 mL of water was added to the reaction system, and ethyl acetate was used for extraction (20 mL × 3). After being combined, the organic phases were rinsed with saturated brine, dried with anhydrous $Na_2SO_4$, and filtered, and the filtrate was concentrated under reduced pressure. The residue was purified by preparative high performance liquid chromatograph (Oriendo, BRIX-2860; column: Phenomenex Luna C18 250 × 50 mm × 10 μm; mobile phase: water (0.225% trifluoroacetic acid)-acetonitrile, eluted from 40% to 80%), the resulting solution was lyophilized to obtain a white solid compound **PY-6A1** (4.02 mg, 7.92 μmol, yield: 3.80%).

LCMS: $[M+H]^+$ = 508.2.

$^1H$ NMR(DMSO-$d_6$, 400 MHz): δ (ppm) 0.77-0.95 (t, *J*=0.9 Hz, 3H), 1.75-1.91 (m, 2H), 2.15-2.33 (m, 1H), 2.322.39 (s, 3H), 2.94-3.14 (m, 2H), 3.40-3.66 (m, 2H), 4.67-5.30 (m, 3H), 5.31-5.57 (m, 3H), 6.46-6.51 (s, 1H), 7.26-7.30 (s, 1H), 7.71-7.82 (m, 1H), 8.11-8.29 (d, *J*=44.6 Hz, 1H).

Preparation Example 10: Preparation of N-(1S,9S)-9-ethyl-5-fluoro-9-hydroxy-4-methyl-10,13-dioxy-2,3,9,10,13,15-hexahydro-1H,12H-benzo[de]pyrano[3',4:6,7]indolizino[1,2-b]quinolin-1-yl)-N-(2-hydroxyethyl)acetamide (**PY-6A2**)

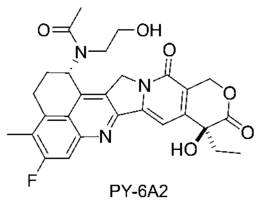

PY-6A2

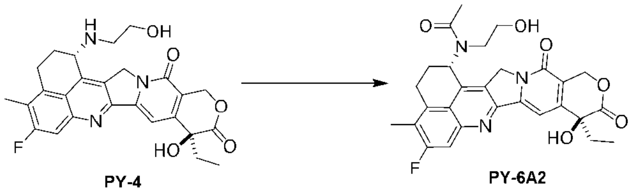

PY-4 PY-6A2

Three equivalents of triethylamine (12 mg, 0.12 mmol) and 1 equivalent of acetyl chloride (3.27 mg, 41.70 μmol) were added to a solution of the compound (1S,9S)-9-ethyl-5-fluoro-9-hydroxy-1-((2-hydroxymethyl)amino)-4-methyl-1,2,3,9,12,15-hexahydro-10H,13H-benzo[de]pyrano[3',4':6,7]indolizino[1,2-b]quinolin-10,13-dione (**PY-4**) (20 mg, 0.04 mmol) in dichloromethane (1 mL) at 0°C, and the reaction system was stirred at 0°C for two hours. The reaction solution was concentrated under reduced pressure. The residue was purified by high performance liquid chromatograph (Oriendo, BRIX-2860; column: Phenomenex Luna C18 250 × 50 mm × 10 μm; mobile phase: water (0.225% trifluoroacetic acid)-acetonitrile, eluted from 40% to 80%), to obtain a white solid compound **PY-6A2** (3.25 mg, 15%).

LCMS(ESI): m/z, 522.2 $[M+1]^+$.

$^1H$ NMR (400 MHz, DMSO-$d^6$) δ 8.13 (s, 1 H) 7.75 (br d, *J* = 9.6 Hz, 1 H) 7.25-7.38 (m, 1 H) 6.52 (s, 1 H) 5.30-5.53 (m, 4 H) 4.04 - 4.33 (m, 2 H) 2.85-3.19 (m, 5 H) 2.29-2.41 (m, 4 H) 2.03-2.23 (m, 5 H) 1.83-1.95 (m, 2 H) 0.87 (t, *J* = 7.2 Hz, 3 H).

Preparation Example 11: Preparation of N-(1S,9S)-9-ethyl-5-fluoro-9-hydroxy-4-methyl-10,13-dioxy-2,3,9,10,13,15-hexahydro-1H,12H-benzo[de]pyrano[3',4:6,7]indolizino[1,2-b]quinolin-1-yl)-N-(2-hydroxyethyl)methanesulfonamide (**PY-6B1**)

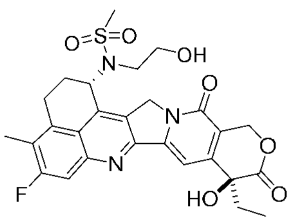

PY-6B1

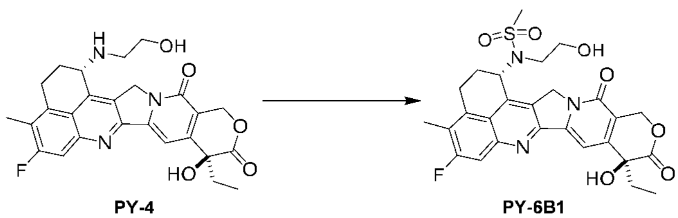

PY-4 PY-6B1

Three equivalents of triethylamine (12 mg, 0.12 mmol) and 1 equivalent of methanesulfonyl chloride (5 mg, 0.04 mmol) were added to a solution of the compound (1S,9S)-9-ethyl-5-fluoro-9-hydroxy-1-((2-hydroxymethyl)amino)-4-methyl-1,2,3,9,12,15-hexahydro-10H,13H-benzo[de]pyrano[3',4':6,7]indolizino[1,2-b]quinolin-10,13-dione (**PY-4**) (20 mg, 0.04 mmol) in dichloromethane (1 mL) at 0°C, and the reaction system was stirred at 0°C for two hours. The reaction solution was concentrated under reduced pressure. The residue was purified by high performance liquid chromatograph (Oriendo, BRIX-2860; column: Phenomenex Luna C18 250 × 50 mm × 10 μm; mobile phase: water (0.225% trifluoroacetic acid)-acetonitrile, eluted from 48% to 80%), to obtain a white solid compound PY-6B1 (1.3 mg, yield: 15%).

LCMS(ESI): m/z, 558.2 $[M+1]^+$.

$^1H$ NMR (400 MHz, DMSO-$d^6$) δ 8.22 (d, *J* = 13.6 Hz, 1 H), 7.70-7.81 (m, 1H), 7.28-7.35 (m, 1 H), 6.55 (d, *J* = 1.6

Hz, 1 H), 5.43 (s, 3 H), 4.15-4.37 (m, 1 H), 2.80-3.11 (m, 4 H), 2.63-2.70 (m, 1 H), 2.27-2.40 (m, 6 H), 2.01-2.24 (m, 2 H), 1.53-1.95 (m, 4 H), 0.80-0.94 (m, 3 H).

Preparation Example 12: Preparation of (S)-9-ethyl-5-fluoro-9-hydroxy-4-(3-hydroxypropyl)-1,2,3,9,12,15-hexahydro-10H,13H-benzo[de]pyrano[3',4:6,7]indolizino[1,2-b]quinolin-10,13-dione (**PY-8**)

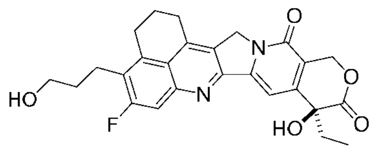

PY-8

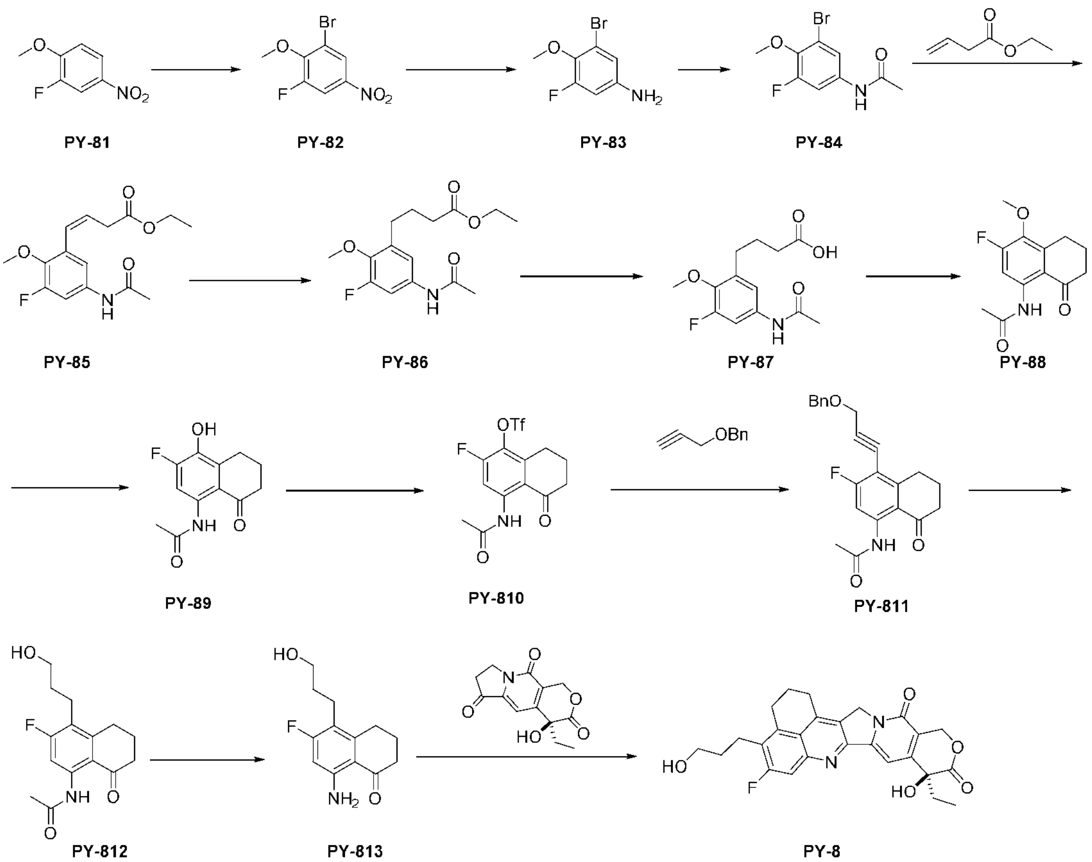

PY-81 PY-82 PY-83 PY-84

PY-85 PY-86 PY-87 PY-88

PY-89 PY-810 PY-811

PY-812 PY-813 PY-8

Step 1: Preparation of 1-bromo-3-fluoro-2-methoxy-5-nitrobenzene (**PY-82**)

Concentrated sulfuric acid (30 mL) was added to a solution of 2-fluoro-1-methoxy-4-nitrobenzene (PY-81) (50 g, 292 mmol) and NBS (57.2 g, 321 mmol) in acetic acid (500 mL) and the resulting solution was stirred overnight at 120°C. The reaction solution was concentrated, left standing to precipitate a milky white solid, and filtered, and a filter cake was washed with water and ethanol, and dried to obtain compound PY-82 (58g, yield: 79%).

LCMS(ESI): m/z, 249.9 $[M+H]^+$.

$^1H$ NMR (400 MHz, DMSO-$d^6$) δ 8.34-8.23 (m, 2H), 4.07 (d, *J* = 3.2 Hz, 3H).

Step 2: Preparation of 3-bromo-5-fluoro-4-methoxyaniline (**PY-83**)

Concentrated hydrochloric acid (3.34 mL, 0.55 equiv.) and 83 mL of water were sequentially added to a solution of compound 1-bromo-3-fluoro-2-methoxy-5-nitrobenzene (**PY-82**) (50g, 200 mmol) and ferrous powder (55.85g, 1 mol) in ethanol (500 mL) and the resulting solution was stirred overnight at 80°C. The reaction solution was filtered by diatomite,

and concentrated under reduced pressure, and the residue was isolated and purified by silica gel column chromatography (PE: EA = 3:1) to obtain compound **PY-83** (40g, yield: 90%).

LCMS(ESI): m/z, 219.9 $[M+H]^+$.

$^1H$ NMR (400 MHz, DMSO-d$^6$) δ 6.59 (t, *J* = 2.0 Hz, 1H), 6.41 (dd, *J* = 13.2, 2.6 Hz, 1H), 5.40 (s, 2H), 3.67 (s, 3H).

Step 3: Preparation of N-(3-bromo-5-fluoro-4-methoxyphenyl)acetamide (**PY-84**)

Acetyl chloride (4 mL, 1.2 equiv.) was added to a solution of compound 3-bromo-5-fluoro-4-methoxyaniline (**PY-83**) (10.0g, 45.4 mmol) and triethylamine (13.9 mL, 2.2 equiv.) in dichloromethane (500 mL) and the resulting solution was stirred overnight at 25°C. The reaction was quenched by using 150 mL of saturated ammonium chloride, then 3 × 80 mL of dichloromethane was added for extraction, the organic phase was washed with 80 mL of saturated sodium chloride solution and concentrated under reduced pressure, and the residue was isolated and purified by silica gel column chromatography (PE: EA = 3:1) to obtain compound **PY-84** (10g, yield: 84%).

LCMS(ESI): m/z, 264.0 $[M+H]^+$.

Step 4: Preparation of ethyl (Z)-4-(5-acetamido-3-fluoro-2-methoxyphenyl)butyl-3-enoate (**PY-85**)

DIPEA (60 mL, 6.0 equiv.) and ethyl crotonate (1.5 equiv., 10.89 mL) were sequentially added to a solution of compound N-(3-bromo-5-fluoro-4-methoxyphenyl)acetamide (PY-84) (15g, 57.23 mmol) and $Pd(t\text{-}Bu_3P)_2$ (1.46g, 0.05 equiv) in toluene (200.0 mL) and the resulting solution was stirred overnight at 13°C. The reaction mixture was directly concentrated under reduced pressure, and the residue was isolated and purified by silica gel column chromatography (PE: EA = 1:2) to obtain compound **PY-85** (7.9g, yield: 46%).

LCMS(ESI): m/z, 296.1 $[M+H]^+$.

Step 5: Preparation of ethyl 4-(5-acetamido-3-fluoro-2-methoxyphenyl)butyrate (**PY-86**)

Palladium on carbon (0.1 equiv., 2.88g) was added to a solution of compound ethyl (Z)-4-(5-acetamido-3-fluoro-2-methoxyphenyl)butyl-3-enoate (**PY-85**) (8.0g, 27.08 mmol) in methanol (100.0 mL) and the resulting solution reacted at 50°C overnight. The reaction mixture was filtered by diatomite, and the filtrate was concentrated under reduced pressure, and dried to obtain a crude product compound **PY-86,** which was directly used for the next reaction.

LCMS(ESI): m/z, 298.1 $[M+H]^+$.

Step 6: Preparation of 4-(5-acetamido-3-fluoro-2-methoxyphenyl)butyric acid (**PY-87**)

Lithium hydroxide (3 equiv., 1.93g) was added to a mixed solvent of compound ethyl 4-(5-acetamido-3-fluoro-2-methoxyphenyl)butyrate (**PY-86**) (8g, 26.91 mmol) in methanol, tetrahydrofuran, and water (120 mL) (mixed in a ratio of 1:1:1) and the resulting solution reacted overnight at room temperature. The reaction mixture was concentrated under reduced pressure, and then ethyl acetate (2 × 80 mL) was used to extract impurities and remove the organic phase. Then concentrated hydrochloric acid was added to the aqueous phase to adjust a pH value to 1, and then extracted with ethyl acetate (3 × 80 mL), organic phases were combined, dried with anhydrous sodium sulfate and filtered, and the filtrate was concentrated under reduced pressure to obtain compound **PY-87** (3.8g, yield: 70%).

LCMS(ESI): m/z 270.1 $[M+H]^+$; 292.1 $[M+Na]^+$.

$^1H$ NMR (400 MHz, DMSO-*d*$^6$) δ 12.07 (s, 1H), 9.99 (s, 1H), 7.50 (dd, *J* = 13.6, 2.4 Hz, 1H), 7.09-7.03 (m, 1H), 3.77 (d, *J* = 1.2 Hz, 3H), 2.63-2.53 (m, 2H), 2.25 (t, *J* = 7.2 Hz, 2H), 2.02 (s, 3H), 1.75 (dq, *J* = 9.2, 7.2 Hz, 2H).

Step 7: Preparation of N-(3-fluoro-4-methoxy-8-oxo-5,6,7,8-tetrahydronaphthalene-1-yl)acetamide (**PY-88**)

A solution of compound 4-(5-acetamido-3-fluoro-2-methoxyphenyl)butyric acid (**PY-87**) (1g, 3.71 mmol) in PPA (polyphosphoric acid) was stirred for 3 hours at 95°C. The reaction mixture was added into ice water, washed with water, then extracted with ethyl acetate (3 × 30 mL), dried with anhydrous sodium sulfate, and then filtered and concentrated under reduced pressure. The residue was purified by high performance liquid chromatograph (Oriendo, BRIX-2860; column: Phenomenex Luna C18 250 × 50 mm × 10 μm; mobile phase: water (0.225% trifluoroacetic acid)-acetonitrile, eluted from 45% to 85%) and lyophilized, to obtain compound **PY-88** (110 mg, yield: 11%).

LCMS(ESI): m/z, 252.1 $[M+H]^+$; 274.0 $[M+Na]^+$.

$^1H$ NMR (400 MHz, DMSO-*d*$^6$) δ 12.05 (s, 1H), 8.37 (d, *J* = 14.8 Hz, 1H), 3.81 (d, *J* = 1.2 Hz, 3H), 2.95 (t, *J* = 6.0 Hz, 2H), 2.66 (dd, *J* = 7.2, 5.6 Hz, 2H), 2.16 (s, 3H), 1.99 (q, *J* = 6.4 Hz, 2H).

Step 8: Preparation of N-(3-fluoro-4-hydroxyl-8-oxy-5,6,7,8-tetrahydronaphthalene-1-yl)acetamide (**PY-89**)

The compound N-(3-fluoro-4-methoxy-8-oxo-5,6,7,8-tetrahydronaphthalene-1-yl)acetamide (**PY-88**) (1.3g) was dissolved in DCE (20 mL), 5 equivalents of $AlCl_3$ were added and the resulting solution was stirred at 60°C for 5 hours. 100 mL of water was added for dilution, and the resulting solution was extracted with ethyl acetate, dried with anhydrous sodium sulfate, filtered, and concentrated under reduced pressure to obtain 910 mg of yellow solid compound **PY-89.**

LCMS(ESI): m/z, 238.0 $[M+1]^+$.

Step 9: Preparation of 4-acetamido-2-fluoro-5-oxy-5,6,7,8-tetrahydronaphthalene-1-yltrifluoromethanesulfonate (**PY-810**)

The compound N-(3-fluoro-4-hydroxyl-8-oxy-5,6,7,8-tetrahydronaphthalene-1-yl)acetamide (**PY-89**) (0.9g) was dissolved in DCM (20 mL), 3 equivalents of triethylamine and 3 equivalents of trifluoromethanesulfonic anhydride were added, the resulting solution was stirred at room temperature for 3 hours and concentrated under reduced pressure, and the residue was isolated and purified by silica gel column chromatography (ethyl acetate/n-hexane = 1:5) to obtain 0.9g of yellow solid compound **PY-810.**

LCMS(ESI): m/z, 370.1 $[M+1]^+$.

Step 10: Preparation of N-(4-(3-(benzyloxy)propyl-1-alkynyl-1-yl)-3-fluoro-8-oxy-5,6,7,8-tetrahydronaphthalene-1-yl) acetamide (**PY-811**)

The compound 4-acetamido-2-fluoro-5-oxy-5,6,7,8-tetrahydronaphthalene-1-yltrifluoromethanesulfonate (**PY-810**) (420 mg) was dissolved in DMF (10 mL), and 2 equivalents of triethylamine, 3 equivalents of propargyl benzyl ether, 0.2 equivalents of $Pd(PPh_3)_2Cl_2$, and 0.1 equivalents of CuI were added. The resulting solution was stirred at 80°C for 12 hours under a nitrogen atmosphere. The resulting solution was diluted with 100 mL of water, extracted with ethyl acetate, dried with anhydrous sodium sulfate, and filtered and the filtrate was concentrated under reduced pressure. The residue was purified by preparative high performance liquid chromatograph (Oriendo, BRIX-2860; column: Phenomenex Luna C18 250 × 50 mm × 10 μm; mobile phase: water (0.225% trifluoroacetic acid)-acetonitrile, eluted from 45% to 85%), to obtain 250 mg of yellow solid compound **PY-811.**

LCMS(ESI): m/z, 366.1 $[M+1]^+$.

Step 11: Preparation of N-(3-fluoro-4-(3-hydroxypropyl)-8-oxy-5,6,7,8-tetrahydronaphthalene-1-yl)acetamide (**PY-812**)

The compound N-(4-(3-(benzyloxy)propyl-1-alkynyl-1-yl)-3-fluoro-8-oxy-5,6,7,8-tetrahydronaphthalene-1-yl) acetamide (**PY-811**) (250 mg) was dissolved in MeOH (10 mL), 0.1 equivalents of palladium on carbon was added, and the resulting solution was stirred at room temperature for 12 hours under a hydrogen atmosphere. The resulting solution was filtered and the filtrate was concentrated under reduced pressure to obtain 150 mg of yellow solid compound **PY-812.**

LCMS(ESI): m/z, 280.2 $[M+1]^+$.

Step 12: Preparation of 8-amino-6-fluoro-5-(3-hydroxypropyl)-3,4-dihydronaphthalene-1(2H)-one (**PY-813**)

The compound N-(3-fluoro-4-(3-hydroxypropyl)-8-oxy-5,6,7,8-tetrahydronaphthalene-1-yl)acetamide (**PY-812**) (150 mg) was dissolved in 6N HCl (2 mL) and EtOH (2 mL), and the resulting solution was stirred at 60°C for 3 hours, filtered, and concentrated under reduced pressure to obtain 125 mg of yellow solid compound **PY-813.**

LCMS(ESI): m/z, 238.1 $[M+1]^+$.

Step 13: Preparation of (S)-9-ethyl-5-fluoro-9-hydroxy-4-(3-hydroxypropyl)-1,2,3,9,12,15-hexahydro-10H,13H-benzo [de]pyrano[3',4:6,7]indolizino[1,2-b]quinolin-10,13-dione (**PY-8**)

The compound 8-amino-6-fluoro-5-(3-hydroxypropyl)-3,4-dihydronaphthalene-1(2H)-one (**PY-813**) (125 mg, 0.53 mmol) and 1.1 equivalents of (S)-4-ethyl-4-hydroxy-7,8-dihydro-1H-pyran[3,4-f]indolizino-3,6,10(4H)-trione (ChemExpress, 152 mg, 0.58 mmol) were dissolved in 10 mL of xylene, and 0.3 equivalents of PPTS (pyridinium p-toluenesulfonate) (40 mg, 0.16 mmol) were added. The resulting solution was stirred at 120°C for 12 hours under a nitrogen atmosphere. The resulting solution was concentrated under reduced pressure. The residue was isolated and purified by high performance liquid chromatograph (Oriendo, BRIX-2860; column: Phenomenex Luna C18 250 × 50 mm × 10 μm; mobile phase: water (0.225% trifluoroacetic acid)-acetonitrile, eluted from 45% to 85%) and lyophilized, to obtain

a yellow solid compound PY-8 (42 mg, yield: 17%).

LCMS(ESI): m/z, 456.2 [M+1]$^+$.

$^1$H NMR (400 MHz, DMSO-$d^6$) δ 7.72 (d, *J* = 11.2 Hz, 1H), 7.30 (s, 1H), 6.51 (s, 1H), 5.43 (s, 2H), 5.24 (s, 2H), 4.60 (t, *J* = 5.2 Hz, 1H), 3.49 (q, *J* = 6.0 Hz, 2H), 3.16 (t, *J* = 6.0 Hz, 4H), 2.87 (t, *J* = 8.0 Hz, 2H), 2.08 (t, *J* = 6.0 Hz, 2H), 1.88-1.87 (m, 2H), 1.69 (p, *J* = 6.4 Hz, 2H), 0.88 (t, *J* = 7.2 Hz, 3H).

Preparation Example 13: Preparation of (S)-9-ethyl-5-fluoro-9-hydroxy-4-propyl-1,2,3,9,12,15-hexahydro-10H,13H-benzo[de]pyrano[3',4':6,7]indolizino[1,2-b]quinolin-10,13-dione (**PY-8B**)

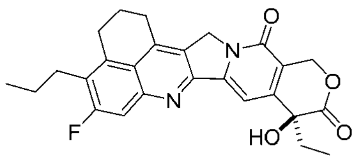

PY-8B

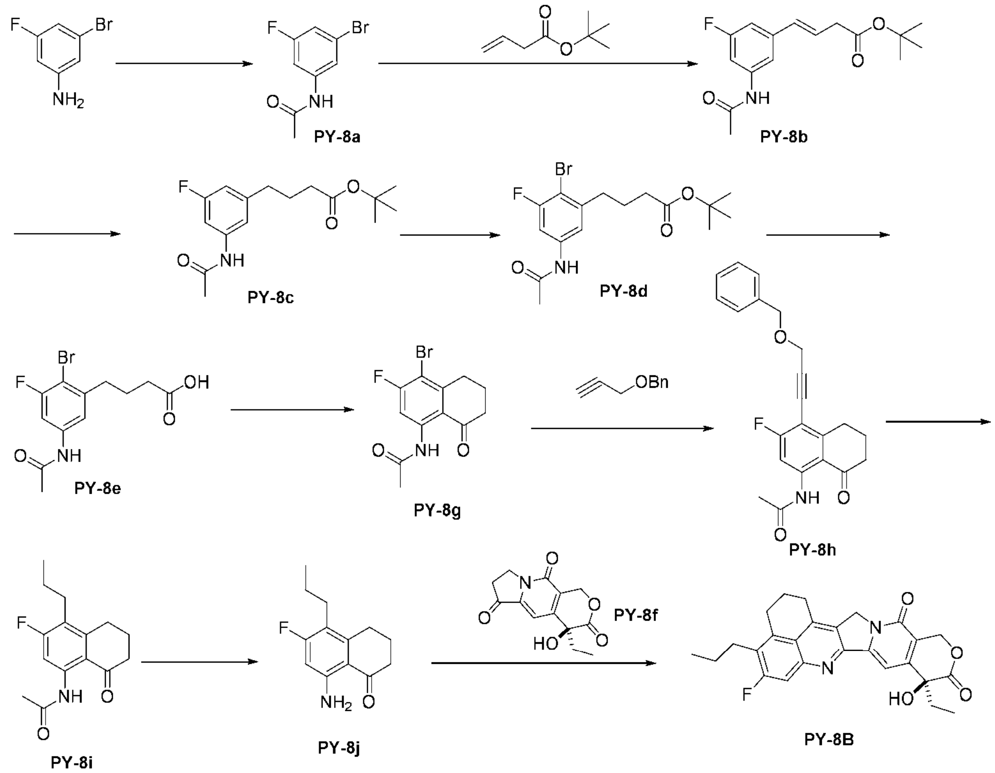


Step 1: Preparation of N-(3-bromo-5-fluorophenyl)acetamide (**PY-8a**)

3-bromo-5-fluoroaniline (50g, 0.263 mol) was dissolved in dichloromethane (700 mL), triethylamine (53.2g, 0.526 mol) was added at room temperature, acetic anhydride (40.3g, 0.395 mol) was slowly added dropwise under an ice water bath, and after addition, the resulting solution reacted at room temperature for 2 hours. Water (400 mL) was added, then the aqueous phase was extracted with dichloromethane (200 mL), the organic phases were combined, washed sequentially with the saturated sodium chloride solution (300 mL × 2), dried with the anhydrous sodium sulfate, and filtered, the filtrate was concentrated under reduced pressure, and the residue was pulped with n-heptane and filtered to obtain N-(3-bromo-5-fluorophenyl)acetamide (54g, yield: 88.4%).

Step 2: Preparation of tert-butyl (E)-4-(3-acetamido-5-fluorophenyl)but-3-enoate (**PY-8b**)

N-(3-bromo-5-fluorophenyl)acetamide (**PY-8a**) (54g, 0.2327 mol) was dissolved in N,N-dimethylformamide (700 mL), bis(tri-tert-butylphosphine)palladium (5.97g, 11.64 mmol), tris(o-methylphenyl)phosphorus (7.07g, 23.27 mmol), N-methyldicyclohexylamine (100g, 0.5119 mol) and tert-butyl 3-butenoate (66.1g, 0.4654 mol) were added sequentially, nitrogen displacement was performed three times, and then the resulting solution reacted at 100°C for 16 hours. Ethyl acetate (600 mL) and water (1 L) were added for extraction, then the aqueous phase was extracted with ethyl acetate (300 mL), the organic phases were combined, washed sequentially with the saturated sodium chloride solution (500 mL × 2), dried with the anhydrous sodium sulfate, and filtered, the filtrate was concentrated under reduced pressure, and the residue was purified by silica gel column chromatography with an eluent system of ethyl acetate/n-heptane (0%-30%) to obtain tert-butyl (E)-4-(3-acetamido-5-fluorophenyl)but-3-enoate (65g, yield: 95.3%).

Step 3: Preparation of tert-butyl 4-(3-acetamido-5-fluorophenyl)butyrate (**PY-8c**)

Tert-butyl (E)-4-(3-acetamido-5-fluorophenyl)but-3-enoate (**PY-8b**) (65g, 0.2218 mol) was dissolved in methanol (1.3 L), nitrogen displacement was performed once, then Pd/C (33g, 0.3101 mol) was added, and after addition, nitrogen displacement was performed again, hydrogen displacement was performed twice, and the resulting solution reacted at room temperature for 16 hours. The resulting solution was filtered, the filtrate was concentrated under reduced pressure, and the residue was purified by reversed-phase chromatography with an eluent system of acetonitrile/purified water (0%-90%) to obtain tert-butyl 4-(3-acetamido-5-fluorophenyl)butyrate (**PY-8c**) (55g, yield: 84%).

Step 4: Preparation of tert-butyl 4-(5-acetamido-2-bromo-3-fluorophenyl)butyrate (**PY-8d**)

Tert-butyl 4-(3-acetamido-5-fluorophenyl)butyrate (**PY-8c**) (55g, 0.1864 mol) was dissolved in N,N-dimethylformamide (600 mL), NBS (36.5g, 0.2051 mol) was added in different batches under an ice water bath, and after addition, the resulting solution was stirred at room temperature for 1 hour. Then ethyl acetate (300 mL) and water (400 mL) were added for extraction, then the aqueous phase was extracted with ethyl acetate (100 mL) once, the organic phases were combined, washed sequentially with the saturated sodium chloride solution (300 mL × 2), dried with the anhydrous sodium sulfate, and filtered, the filtrate was concentrated under reduced pressure, and the residue was purified by silica gel column chromatography with an eluent system of ethyl acetate/n-heptane (0%-30%) to obtain tert-butyl 4-(5-acetamido-2-bromo-3-fluorophenyl)butyrate (59g, yield: 84.6%).

Step 5: Preparation of 4-(5-acetamido-2-bromo-3-fluorophenyl)butyric acid (**PY-8e**)

Tert-butyl 4-(5-acetamido-2-bromo-3-fluorophenyl)butyrate (**PY-8d**) (59g, 0.1577 mol) was dissolved in dichloromethane (300 mL), HCl/dioxane (400 mL, 1.6 mol) was added dropwise under an ice water bath, and after addition, the resulting solution reacted at room temperature for 16 hours and filtered, and the filtrate was concentrated under reduced pressure to obtain 4-(5-acetamido-2-bromo-3-fluorophenyl)butyric acid (42g, yield: 83.7%).

Step 6: Preparation of N-(4-bromo-3-fluoro-8-oxo-5,6,7,8-tetrahydronaphthalene-1-yl)acetamide (**PY-8g**)

4-(5-acetamido-2-bromo-3-fluorophenyl)butyric acid (**PY-8e**) (42g, 0.1321 mol) and Eaton's reagent (210g) were added to a 1L three-necked flask, nitrogen displacement was performed 3 times, and the resulting solution reacted at 85°C for 1 hour. The reaction solution was slowly added dropwise to water (2 L) under an ice water bath and filtered, the filter cake was washed with water, and then the filtrate was concentrated and dried under reduced pressure, and then purified by silica gel column chromatography with an eluent system of ethyl acetate/dichloromethane (0%-30%) to obtain a crude product (30g). Then the crude product was pulped with a system of ethyl acetate (10 mL)/petroleum ether (100 mL), and filtrated, to obtain N-(4-bromo-3-fluoro-8-oxo-5,6,7,8-tetrahydronaphthalene-1-yl)acetamide (20g, yield: 50.4%).

LCMS(ESI): m/z, 300.0 $[M+H]^+$.

$^1$H NMR (400 MHz, $CDCl_3$) δ 12.35 (s, 1H), 8.61 (d, *J* = 11.6 Hz, 1H), 3.07 (t, *J* = 6.0 Hz, 2H), 2.69 (dd, *J* = 7.2 Hz, 2H), 2.24 (s, 3H), 2.11 (p, *J* = 6.4 Hz, 2H).

Step 7: Preparation of N-(4-(3-(benzyloxy)propyl-1-alkynyl-1-yl)-3-fluoro-8-oxo-5,6,7,8-tetrahydronaphthalene-1-yl) acetamide (**PY-8h**)

N-(4-bromo-3-fluoro-8-oxo-5,6,7,8-tetrahydronaphthalene-1-yl)acetamide (**PY-8g**) (1.8g, 6.0 mmol) was dissolved in anhydrous DMF (50 mL) in a nitrogen atmosphere, (prop-2-en-1-oxy)methyl)benzene (4.34 mL, 29.99 mmol), cuprous iodide (228.4 mg, 1.2 mmol), dichlorobis(triphenylphosphine)palladium (841.9 mg, 1.2 mmol) and triethylamine (3.33 mL, 23.99 mmol) were added sequentially, hydrogen displacement was performed three times, and then the resulting mixture was heated to 100°C and stirred for reaction for 16 hours. The reaction solution was concentrated to dryness, and the residue was purified by silica gel column chromatography (eluents: ethyl acetate/n-hexane = 1:20-1:5) to obtain compound PY-8h (1.0g, yield: 45.6%) as a yellow solid.

LCMS: m/z = 366.1 [M+1]$^+$; Rt = 1.108.

Step 8: Preparation of N-(3-fluoro-8-oxo-4-propyl-5,6,7,8-tetrahydronaphthalene-1-yl)acetamide (**PY-8i**)

The compound **PY-8h** (2.5g, 6.84 mmol) was dissolved in methanol (20 mL) and tetrahydrofuran (20 mL), palladium on carbon (4.0g) was added, hydrogen displacement was performed three times, and the resulting mixture was heated to 45°C and stirred for 4 hours. After being filtered, the reaction solution was directly concentrated, and the crude product was directly used for the next reaction.
LCMS: m/z = 264.1 [M+1]+; Rt = 1.056.

Step 9: Preparation of 8-amino-6-fluoro-5-propyl-3,4-dihydronaphthalene-1(2H)-one (**PY-8j**)

The compound **PY-8i** (1.91g, 6.84 mmol) was dissolved in 6M hydrochloric acid (20 mL) and ethanol (20 mL), and the resulting mixture was heated to 60°C and stirred for reaction for 1 hour. The reaction solution was concentrated to dryness, and the crude product was directly used for the next reaction.

Step 10: Preparation of (S)-9-ethyl-5-fluoro-9-hydroxy-4-propyl-1,2,3,9,12,15-hexahydro-10H,13H-benzo[de]pyrano [3',4':6,7]indolizino[1,2-b]quinolin-10,13-dione (**PY-8B**)

The compound **PY-8j** (960 mg, 1.0 eq) and (S)-4-ethyl-4-hydroxy-7,8-dihydro-1H-pyrano[3,4-flindolizino-3,6,10(4H)-trione (**PY-8f**) (ChemExpress, 1.17g, 1.1 eq) were dispersed in toluene (25 mL) in a nitrogen atmosphere, pyridinium p-toluenesulfonate (508 mg, 0.5 eq) was added, and the resulting mixture was heated to 120°C and stirred for reaction for 16 hours. The reaction solution was concentrated, and the residue was purified by silica gel column chromatography (eluents: dichloromethane/methanol = 10:1) to obtain compound **PY-8B** (340 mg, yield: 18%) as a brown solid.
LCMS: m/z =449.2 [M+H]$^+$; Rt = 3.313 min.
$^1$H NMR (400 MHz, DMSO-$d^{6)}$ δ 7.72 (d, *J* = 11.4 Hz, 1H), 7.30 (s, 1H), 6.52 (s, 1H), 5.43 (s, 2H), 5.23 (s, 2H), 3.15 (q, *J* = 5.6 Hz, 4H), 2.81 (t, *J* = 7.7 Hz, 2H), 2.08 (t, *J* = 5.9 Hz, 2H), 1.94-1.78 (m, *J* = 7.1 Hz, 2H), 1.58 (h, *J* = 7.4 Hz, 2H), 0.97 (t, *J* = 7.3 Hz, 3H), 0.88 (t, *J* = 7.3 Hz, 3H).

Preparation Example 14: Preparation of (S)-9-ethyl-5-fluoro-9-hydroxy-4-(2-hydroxyethoxy)-1,2,3,9,12,15-hexahydro-10H,13H-benzo[de]pyrano[3',4':6,7]indolizino[1,2-b]quinolin-10,13-dione (**PY-9**)

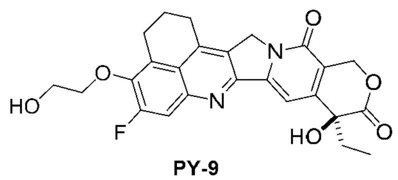

**PY-9**

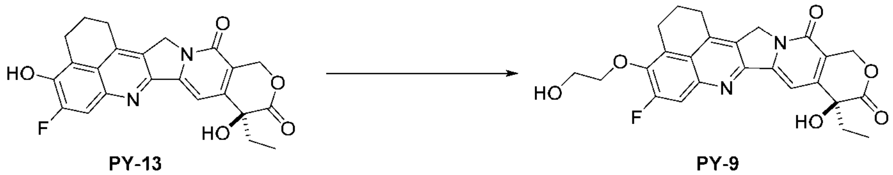

**PY-13** **PY-9**

1 equivalent of tetrabutylammonium bromide (TBAB) (15.3 mg), 0.2 equivalents of catalyst palladium chloride (1.7 mg), 250 mL of ethylene oxide, and 2.5 equivalents of potassium carbonate (16.4 mg) were separately added to 2 mL of aqueous solution of compound **PY-13** (20 mg). The reaction solution reacted at 25°C for 16 hours, and the reaction solution was subjected to preparative high performance liquid chromatograph (preparative chromatograph manufacturer: SHIMADZU, model: LC-20AP, column: Welch Xtimate C18 250 × 30 mm × 10 μm, mobile phase: water (0.225% HCOOH)-acetonitrile, an elution ratio of water: 12% to 42%) and lyophilized to obtain a crude product of a white solid compound **PY-9.** The white solid compound was further isolated and purified by thin layer chromatography to obtain 3.03 mg of compound **PY-9.**
LCMS(ESI): m/z, 467.2 [M+H]$^+$.
$^1$H NMR (400 MHz, DMSO-$d_6$) δ 7.83 (d, *J* = 12.5 Hz, 1H), 7.29 (s, 1H), 6.51 (s, 1H), 5.43 (s, 2H), 5.25 (s, 2H), 4.93

(t, *J* = 5.4 Hz, 1H), 4.14 (t, *J* = 4.9 Hz, 2H), 3.73 (q, *J* = 5.1 Hz, 2H), 3.16 (dt, *J* = 11.1, 6.1 Hz, 4H), 2.02 (dt, *J* = 12.6, 6.6 Hz, 2H), 1.86 (h, *J* = 7.0 Hz, 2H), 0.88 (t, *J* = 7.3 Hz, 3H); $^{19}$F NMR (377 MHz, DMSO-$d_6$) δ -123.40.

Preparation Example 15: Preparation of (S)-9-ethyl-5-fluoro-9-hydroxy-4-(hydroxymethyl)-1,2,3,9,12,15-hexahydro-10H,13H-benzo[de]pyrano[3',4':6,7]indolizino[1,2-b]quinolin-10,13-dione (**PY-10**)

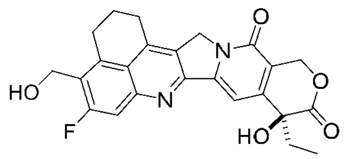

**PY-10**

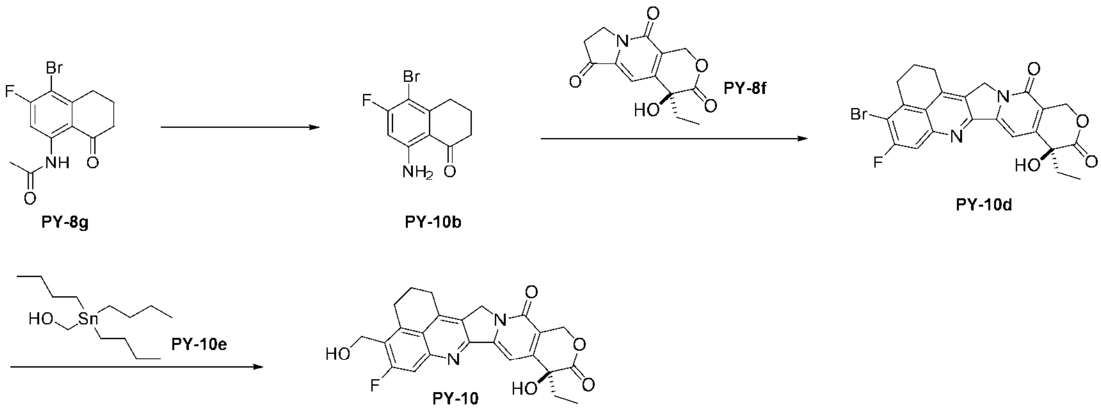


Step 1: Preparation of 8-amino-5-bromo-6-fluoro-3,4-dihydronaphthalene-1(2H)-one (**PY-10b**)

6N HCl (6 mL) was added to a solution of compound **PY-8g** (2g, 6.66 mmol, 1 eq) in ethanol (6 mL), the reaction solution was stirred at 80°C for 3 hours, and the reaction solution was cooled to room temperature, concentrated under reduced pressure, diluted with 20 mL of water and adjusted with a sodium bicarbonate solution until pH was neutral, then the mixture was extracted with dichloromethane (30 mL × 3), and the organic phases were combined, washed with saturated brine (30 mL), dried with anhydrous sodium sulfate, and concentrated under reduced pressure to obtain a crude compound 8-amino-5-bromo-6-fluoro-3,4-dihydronaphthalene-1(2H)-one (**PY-10b**) (1.52g, yield: 88%; purity: 91%) as a yellow solid.

LCMS(ESI): m/z, 258 $[M+1]^+$, 260 $[M+1]^+$.

Step 2: Preparation of (S)-4-bromo-9-ethyl-5-fluoro-9-hydroxy-1,2,3,9,12,15-hexahydro-10H,13H-benzo[de]pyrano[3',4':6,7]indolizino[1,2-b]quinolin-10,13-dione (**PY-10d**)

The compound **PY-8f** (1.55g, 5.89 mmol, 1 eq) and PPTS (1.48g, 5.89 mmol, 1 eq) were added to a solution of compound **PY-10b** (1.52g, 5.89 mmol, 1 eq) in toluene (20 mL), the reaction solution was stirred at 120°C for 16 hours in a nitrogen atmosphere, and the residue was purified by silica gel column chromatography (dichloromethane:methanol = 10:1), to obtain a yellow solid compound **PY-10d** (1.3g, yield: 45%; purity: 88%).

LCMS(ESI): m/z, 485 $[M+H]^+$, 487 $[M+H]^+$.

Step 3: Preparation of (S)-9-ethyl-5-fluoro-9-hydroxy-4-(hydroxymethyl)-1,2,3,9,12,15-hexahydro-10H,13H-benzo[de]pyrano[3',4':6,7]indolizino[1,2-b]quinolin-10,13-dione (**PY-10**)

(Tributylstannyl)methanol (2.84g, 8.84 mmol, 3.3 eq) and Xphos Pd G2 (210.77 mg, 267.87 μmol, 0.1 eq) were added to a solution of compound **PY-10d** (1.30g, 2.68 mmol, 1 eq) in dioxane (5 mL) respectively, and the reaction solution was stirred at 90°C for 16 hours in a nitrogen atmosphere, concentrated under reduced pressure, and diluted with water and dichloromethane (50 mL/50 mL). A solid precipitated, the reaction solution was filtered, a filter cake was collected separately, a filtrate was extracted with dichloromethane (50 mL × 3), the organic phases were combined, then washed

with saturated brine (50 mL), dried with anhydrous sodium sulfate, and concentrated under reduced pressure, and the residue and the filter cake were purified by silica gel column chromatography (dichloromethane: methanol = 10:1) to obtain the yellow solid compound **PY-10** (649 mg, yield: 55.5%, purity: 100%).

LCMS (ESI): m/z, 437 [M+H]+.

Preparation Example 16: Preparation of (S)-9-ethyl-5-fluoro-9-hydroxy-10,13-dioxo-2,3,9,10,13,15-hexahydro-1H,12H-benzo[de]pyrano[3',4':6,7]indolizino[1,2-b]quinolin-4-carboxylic acid (**PY-10A**) and (S)-9-ethyl-5-fluoro-9-hydroxy-1,2,3,9,12,15-hexahydro-10H,13H-benzo[de]pyrano[3',4': 6,7]indolizino[1,2-b]quinolin-10,13-dione (**PY-10B**)

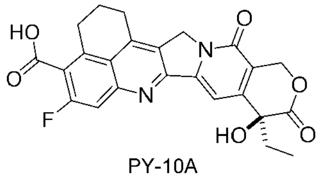

PY-10A

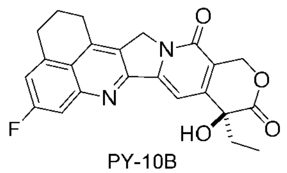

PY-10B

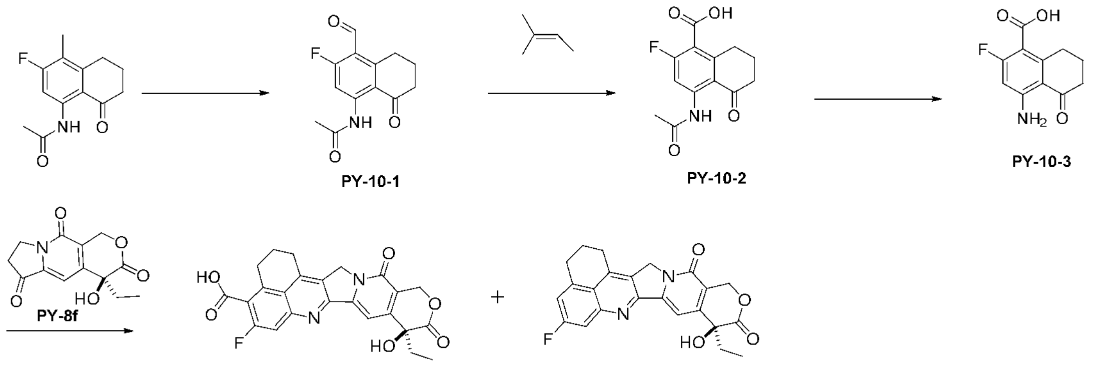


PY-10-1 PY-10-2 PY-10-3

PY-10A PY-10B

Step 1: Preparation of N-(3-fluoro-4-formyl-8-oxo-5,6,7,8-tetrahydronaphthalene-1-yl)acetamide (**PY-10-1**)

$H_2SO_4$ (4.34g, 44.21 mmol, 5.2 eq) was added to a solution of N-(3-fluoro-4-methyl-8-oxo-5,6,7,8-tetrahydronaphthalene-1-yl)acetamide (2g, 8.5 mmol, 1.0 eq) and $MnO_2$ (11.09g, 127.52 mmol, 15 eq) in chloroform (100 mL) at 0°C, and the reaction solution was gradually cooled to room temperature and further reacted for 16 hours. After being filtered, the reaction solution was adjusted with a saturated sodium bicarbonate solution until pH was neutral, extracted with dichloromethane (30 mL × 3), and concentrated to obtain a brownish-yellow solid crude product (2.1g) to be directly used for the next step without purification.

LCMS(ESI): m/z, 250.1 [M+1]+.

Step 2: Preparation of 4-acetamido-2-fluoro-5-oxo-5,6,7,8-tetrahydronaphthalene-1-carboxylic acid **(PY-10-2)**

Dimethylbutadiene (1.55g, 22.07 mmol, 10 eq) was added to a solution of the compound N-(3-fluoro-4-formyl-8-oxo-5,6,7,8-tetrahydronaphthalene-1-yl)acetamide (1.1g, 2.21 mmol, 1 eq), sodium chlorite (1.20g, 13.24 mmol, 6 eq), and sodium phosphate monobasic (534 mg, 4.41 mmol, 2 eq) in methanol/water = 1/1 (30 mL) at 25°C. The reaction solution was stirred at 50°C for 2 hours. The aqueous solution of saturated sodium bicarbonate was added to adjust pH to 8, the reaction solution was extracted with dichloromethane (30 mL × 3) to recover the raw materials, then the aqueous phase was adjusted with 2N HCl to adjust pH to 2, and the aqueous phase was extracted with ethyl acetate (30 mL × 3) and concentrated to obtain the compound PY-10-2 (324 mg, yield: 55.36%).

LCMS (ESI): m/z, 266.0[M+1]+.

Step 3: Preparation of 4-amido-2-fluoro-5-oxo-5,6,7,8-tetrahydronaphthalene-1-carboxylic acid **(PY-10-3)**

The compound 4-acetamido-2-fluoro-5-oxo-5,6,7,8-tetrahydronaphthalene-1-carboxylic acid (415 mg,1.67 mmol,1 eq) was added to a solution of NaOH (5 M, 15 mL), the reaction solution was stirred at 80°C for 2 hours, adjusted with 2N dilute hydrochloric acid to adjust pH to 2, and extracted with ethyl acetate (30 mL × 3), and the organic phase was

concentrated to obtain compound **PY-10-3** (320 mg, yield: 85.6%).

LCMS (ESI): m/z: 224.0[M+1]$^+$.

Step 4: Preparation of (S)-9-ethyl-5-fluoro-9-hydroxy-10,13-dioxo-2,3,9,10,13,15-hexahydro-1H,12H-benzo[de]pyrano [3',4':6,7]indolizino[1,2-b]quinolin-4-carboxylic acid **(PY-10A)** and (S)-9-ethyl-5-fluoro-9-hydroxy-1,2,3,9,12,15-hexahydro-10H, 13H-benzo[de]pyrano[3°,4': 6,7]indolizino[1,2-b]quinolin-10,13-dione **(PY-10B)**

The compound 4-amido-2-fluoro-5-oxo-5,6,7,8-tetrahydronaphthalene-1-carboxylic acid (364.64 mg,1.43 mmol,1 eq), the compound (S)-4-ethyl-4-hydroxy-7,8-dihydro-1H-pyran[3,4-f]indolizino-3,6,10(4H)-trione (377.42 mg, 1.43 mmol, 1 eq) and PPTS (360.29 mg, 1.43 mmol, 1 eq) were dissolved in a solution of toluene (15 mL), the reaction solution was stirred at 120°C for 16 hours, and after the solvent was removed under reduced pressure, the residue was purified by silica gel column chromatography (eluents: dichloromethane/methanol = 10:1-5:1) to obtain a crude product of a brown oily compound, and then the crude product was isolated by preparative high performance liquid chromatography (preparative chromatograph manufacturer: Oriendo, model: Lab311-DJ-R2, column: YMC-Triart Prep C18 250 × 30 mm × 10 μm, mobile phase: water (0.225% HCOOH)-acetonitrile, an elution ratio of water: 30% to 60%) and lyophilized, to obtain compound **PY-10A** (18.19g, yield: 2.82%), LCMS (ESI): m/z: 451.1[M+1]$^+$; and a compound **PY-10B** (46 mg, yield: 7.9%), LCMS (ESI): m/z: 407.1[M+1]$^+$.

Preparation Example 17: Preparation of (S)-(9-ethyl-5-fluoro-9-hydroxy-10,13-dioxy-2,3,9,10,13,15-hexahydro-1H,12H-benzo[d]pyrano[3',4':6,7]indolizino[1,2-b]quinolin-4-yl)methyl(2-hydroxyethyl)carbamate **(PY- 10Car)**

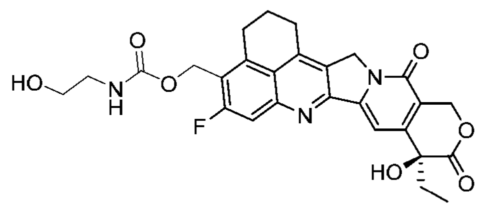

**PY-10 Car**

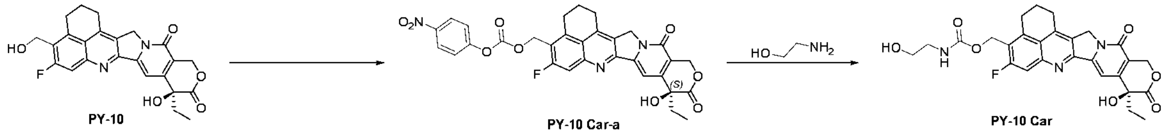

PY-10

PY-10 Car-a

PY-10 Car

Step 1: Preparation of (S)-(9-ethyl-5-fluoro-9-hydroxy-10,13-dioxo-2,3,9,10,13,15-hexahydro-1H,12H-benzo[de]pyrano[3',4':6,7]indolizino[1,2-b]quinolin-4-yl)methyl(4-nitrophenyl)carbonate (PY-10 Cara)

The compound PY-10 (8.0 mg, 0.018 mmol, 1.0 eq), bis(4-nitrophenyl)carbonate (44.6 mg, 0.147 mmol, 8.0 eq), and N,N-diisopropylethylamine (28.4 mg, 0.220 mmol, 12.0 eq) were added to a solution of N,N-dimethylformamide (2 mL) at room temperature and the reaction solution was stirred at 50°C for 16 hours. LCMS showed that the reaction ended. The reaction solution was concentrated under reduced pressure and purified with a developing agent system of dichloromethane/methanol (15/1) of a thin layer chromatography plate, to obtain a yellow oily compound **PY-10 Car-a** (18 mg, overweighted, no yield calculated).

LCMS (ESI): m/z, 602.3 [M+H]$^+$.

Step 2: Preparation of (S)-(9-ethyl-5-fluoro-9-hydroxy-10,13-dioxy-2,3,9,10,13,15-hexahydro-1H,12H-benzo[d]pyrano [3',4':6,7]indolizino[1,2-b]quinolin-4-yl)methyl(2-hydroxyethyl)carbamate (PY-10Car)

The compound PY-10 Car-a (18.0 mg, impure), aminoethanol (1.83 mg, 0.030 mmol), and N,N-diisopropylethylamine (11.6 mg, 0.090 mmol) were added to a solution of N,N-dimethylformamide (1 mL) at room temperature and the reaction solution was stirred at 25°C for 0.5 hours. LCMS showed that the reaction ended. The reaction solution was purified by high performance liquid chromatograph (preparative chromatograph manufacturer: Luna, model: Lab311-ISCO-R4, column: Phenomenex Luna C18 250 × 50 mm × 10 μm, mobile phase: water (0.225% HCOOH)-acetonitrile, an elution ratio of water: 12% to 42%) and the eluate was lyophilized, to obtain a white solid product **PY-10 Car** (4.14 mg, 7.56 μmol, two-step yield: 42%).

LCMS (ESI): m/z, 524.2 [M+H]+.

$^{1}$H NMR (400 MHz, DMSO-$d^6$) $\delta$ 7.79 (d, $J$ = 11.2 Hz, 1H), 7.32 (s, 1H), 7.19 (t, $J$ = 5.6 Hz, 1H), 6.54 (s, 1H), 5.44 (s, 2H), 5.27 (d, $J$ = 4.4 Hz, 3H), 4.63 (t, $J$ = 5.6 Hz, 1H), 3.30 - 3.23 (m, 5H), 3.18 (s, 2H), 3.04 (q, $J$ = 6.0 Hz, 2H), 2.08 (s, 2H), 1.86 (dt, $J$ = 15.2, 7.2 Hz, 2H), 0.87 (t, $J$ = 7.2 Hz, 3H).

Preparation Example 18: Preparation of (S)-9-ethyl-5-fluoro-9-hydroxy-4-methoxy-1,2,3,9,12,15-hexahydro-10H,13H-benzo[de]pyrano[3',4:6,7]indolizino[1,2-b]quinolin-10,13-dione **(PY-11)**

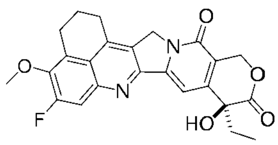

PY-11

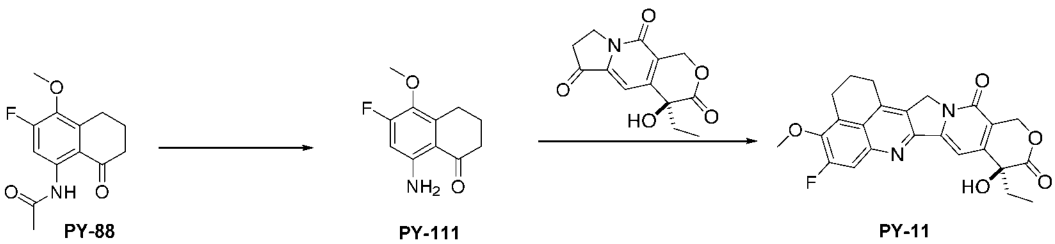

**PY-88** **PY-111** **PY-11**

Step 1: Preparation of 8-amino-6-fluoro-5-methoxy-3,4-dihydronaphthalene-1(2H)-one **(PY-111)**

A solution of the compound N-(3-fluoro-4-methoxy-8-oxo-5,6,7,8-tetrahydronaphthalene-1-yl)acetamide (PY-88) (100 mg) in HCl (7N, 10 mL) was stirred at 100°C for 2 hours. Saturated $NaHCO_3$ solution (10 mL) was slowly added to quench the reaction and adjust a pH value to neutral (pH 7), the reaction solution was extracted with ethyl acetate (3 × 30 mL), organic phases were combined, dried with anhydrous sodium sulfate and filtered, and the filtrate was concentrated under reduced pressure to obtain a yellow solid compound **PY-111** (66 mg, 79%).

LCMS (ESI): m/z, 210.1 [M+H]+.

Step 2: Preparation of (S)-9-ethyl-5-fluoro-9-hydroxy-4-methoxy-1,2,3,9,12,15-hexahydro-10H,13H-benzo[de]pyrano [3',4:6,7]indolizino[1,2-b]quinolin-10,13-dione **(PY-11)**

PPTS (0.67 eq, 16 mg) and (S)-4-ethyl-4-hydroxy-7,8-dihydro-1H-pyrano[3,4-f]indolizino-3,6,10(4H)-trione (1 eq, 25 mg) were added to a solution of the compound 8-amino-6-fluoro-5-methoxy-3,4-dihydronaphthalene-1(2H)-one **(PY-111)** (20 mg) in toluene (10 mL) and the reaction solution reacted at 140°C for 16 hours. The reaction solution was concentrated under reduced pressure to remove toluene, and 5 mL of DMF was added. The resulting mixture was purified by preparative high performance liquid chromatograph (Oriendo, BRIX-2860; column: Phenomenex Luna C18 250 × 50 mm × 10 μm; mobile phase: water (0.225% trifluoroacetic acid)-acetonitrile, eluted from 45% to 85%), to obtain a white solid compound **PY-11** (8.4 mg, yield: 20%).

LCMS (ESI): m/z, 437.2 [M+H]+.

$^{1}$H NMR (400 MHz, DMSO-$d^6$) $\delta$ 7.85 (d, $J$ = 12.5 Hz, 1H), 7.30 (s, 1H), 6.52 (s, 1H), 5.43 (s, 2H), 5.25 (s, 2H), 3.94 (s, 3H), 3.14 (dt, $J$ = 11.2, 6.0 Hz, 4H), 2.05 (q, $J$ = 6.4, 5.8 Hz, 2H), 1.88 (dq, $J$= 14.4, 7.2 Hz, 2H), 0.88 (t, $J$ = 7.2 Hz, 3H).

Preparation Example 19: Preparation of (1S,9S)-1-amino-9-ethyl-5-fluoro-9-hydroxy-4-methoxy-1,2,3,9,12,15-hexahydro-10H,13H-benzo[de]pyrano[3',4:6,7]indolizino[1,2-b]quinolin-10,13-dione and (1R,9S)-1-amino-9-ethyl-5-fluoro-9-hydroxy-4-methoxy-1,2,3,9,12,15-hexahydro-10H,13H-benzo[de]pyrano[3',4:6,7]indolizino[1,2-b]quinolin-10,13-dione **(PY-12A** and **PY-12B)**

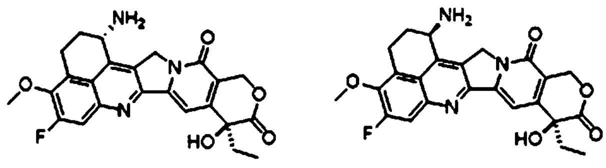

PY-12A and PY-12B

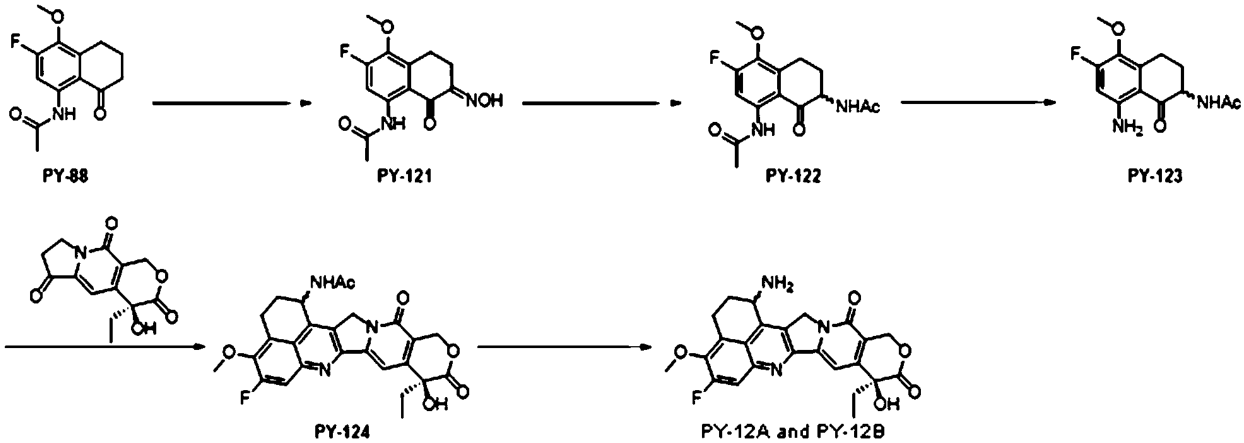

PY-88 PY-121 PY-122 PY-123

PY-124 PY-12A and PY-12B

Step 1: Preparation of (E)-N-(3-fluoro-7-(hydroxyimino)-4-methoxy-8-oxy-5,6,7,8-tetrahydronaphthalene-1-yl)acetamide **(PY-121)**

3 equivalents of tetrahydrofuran solution of potassium tert-butoxide and tert-butyl nitrite were separately added to 10 mL of tetrahydrofuran solution of compound N-(3-fluoro-4-methoxy-8-oxo-5,6,7,8-tetrahydronaphthalene-1-yl) acetamide **(PY-88)** (200 mg) at 0°C, the reaction solution was stirred at 0°C for 1 hour, diluted with 30 mL of water, and extracted with 10 mL of ethyl acetate 3 times. The organic phases were combined, dried with anhydrous sodium sulfate, filtered and concentrated under reduced pressure. The residue was purified by preparative high performance liquid chromatograph (Oriendo, BRIX-2860; column: Phenomenex Luna C18 250 × 50 mm × 10 μm; mobile phase: water (0.225% trifluoroacetic acid)-acetonitrile, eluted from 45% to 85%), to obtain 250 mg of yellow solid compound **PY-121.**

LCMS (ESI): m/z, 281.1 $[M+1]^+$.

Step 2: Preparation of N,N'-(3-fluoro-4-methoxy-8-oxy-5,6,7,8-tetrahydronaphthalene-1,7-diacyl)diacetamide **(PY-122)**

1 mL of acetic anhydride and 2 mL of acetic acid were added to compound (E)-N-(3-fluoro-7-(hydroxyimino)-4-methoxy-8-oxy-5,6,7,8-tetrahydronaphthalene-1-yl)acetamide **(PY-121)** (180 mg) at room temperature, zinc powder (35 mg) was added, and the reaction solution was stirred for 4 hours at room temperature. The reaction solution was concentrated under reduced pressure, diluted with 100 mL of water, extracted with ethyl acetate, dried with anhydrous sodium sulfate, and filtered and the filtrate was concentrated under reduced pressure. The residue was purified by preparative high performance liquid chromatograph (Oriendo, BRIX-2860; column: Phenomenex Luna C18 250 × 50 mm × 10 μm; mobile phase: water (0.225% trifluoroacetic acid)-acetonitrile, eluted from 45% to 85%), to obtain 250 mg of white solid compound **PY-122.**

LCMS (ESI): m/z, 309.1 $[M+1]^+$.

Step 3: Preparation of N-(8-amino-6-fluoro-5-methoxy-1-oxy-1,2,3,4-tetrahydronaphthalene-2-yl)acetamide **(PY-123)**

The compound N,N'-(3-fluoro-4-methoxy-8-oxy-5,6,7,8-tetrahydronaphthalene-1,7-diacyl)diacetamide **(PY-122)** (150 mg) was dissolved in 6N HCl (2 mL) and EtOH (2 mL), and the resulting solution was stirred at 60°C for 3 hours. The reaction solution was concentrated under reduced pressure to obtain 115 mg of crude product, and the crude product was directly used for the next reaction.

LCMS (ESI): m/z, 267.1 $[M+1]^+$.

Step 4: Preparation of N-(9S)-9-ethyl-5-fluoro-9-hydroxy-4-methoxy-10,13-dioxy-2,3,9,10,13,15-hexahydro-1H,12H-benzo[de]pyrano[3',4:6,7]indolizino[1,2-b]quinolin-1-yl)acetamide **(PY-124)**

1 equivalent of (S)-4-ethyl-4-hydroxy-7,8-dihydro-1H-pyrano[3,4-f]indolizino-3,6,10(4H)-trione (ChemExpress) (92 mg) and 0.5 equivalents of PPTS (24 mg) were added to a solution of the compound N-(8-amino-6-fluoro-5-methoxy-1-oxy-1,2,3,4-tetrahydronaphthalene-2-yl)acetamide (PY-123) (80 mg) in toluene and the reaction solution was stirred at 120°C for 12 hours. The reaction solution was concentrated under reduced pressure. The residue was purified by high performance liquid chromatograph (Oriendo, BRIX-2860; column: Phenomenex Luna C18 250 × 50 mm × 10 μm; mobile phase: water (0.225% trifluoroacetic acid)-acetonitrile, eluted from 45% to 85%), to obtain 55 mg of a yellow solid mixture PY-124.

LCMS (ESI): m/z, 494.2 $[M+1]^+$.

Step 5: Preparation of (1S,9S)-1-amino-9-ethyl-5-fluoro-9-hydroxy-4-methoxy-1,2,3,9,12,15-hexahydro-10H,13H-benzo[de]pyrano[3',4:6,7]indolizino[1,2-b]quinolin-10,13-dione and (1R,9S)-1-amino-9-ethyl-5-fluoro-9-hydroxy-4-methoxy-1,2,3,9,12,15-hexahydro-10H,13H-benzo[de]pyrano[3',4:6,7]indolizino[1,2-b]quinolin-10,13-dione **(PY-12A** and **PY-12B)**

N-(9S)-9-ethyl-5-fluoro-9-hydroxy-4-methoxy-10,13-dioxy-2,3,9,10,13,15-hexahydro-1H,12H-benzo[de]pyrano[3',4:6,7]indolizino[1,2-b]quinolin-1-yl)acetamide **(PY-124)** (55 mg) was dissolved in 3 mL of 6N HCl, and the resulting solution was stirred at 85°C for 5 hours. The reaction solution was concentrated under reduced pressure. The residue was purified by high performance liquid chromatograph (Oriendo, BRIX-2860; column: Phenomenex Luna C18 250 × 50 mm × 10 μm; mobile phase: water (0.225% HCOOH)-acetonitrile, eluted from 45% to 85%) and lyophilized, to separately obtain a compound **PY-12A** (4.55 mg) and a compound **PY-12B** (6.24 mg).

**PY-12A** (LCMS retention time: 0.651 min):
LCMS (ESI): m/z, 452.2 $[M+1]^+$, Rt=0.651.

$^1$H NMR (400 MHz, DMSO-$d^6$) $\delta$ 7.87 (d, $J$ = 12.4 Hz, 1H), 7.30 (s, 1H), 6.53 (s, 1H), 5.62 (d, $J$ = 19.2 Hz, 1H), 5.46 - 5.29 (m, 3H), 4.38 (s, 1H), 3.95 (s, 3H), 3.28 - 3.03 (m, 4H), 2.08 (s, 2H), 1.87 (dd, $J$ = 9.2, 7.2 Hz, 2H), 0.87 (t, $J$ = 7.2 Hz, 3H).

**PY-12B** (LCMS retention time: 0.677 min):
LCMS (ESI): m/z, 452.2 $[M+1]^+$, Rt=0.677.

$^1$H NMR (400 MHz, DMSO-$d^6$) $\delta$ 8.66 (s, 2H), 8.01 (d, $J$ = 12.4 Hz, 1H), 7.34 (s, 1H), 6.57 (s, 1H), 5.90 (d, $J$ = 19.2 Hz, 1H), 5.60-5.30 (m, 3H), 5.08 (s, 1H), 4.00 (d, $J$= 1.2 Hz, 3H), 3.15 (t, $J$ = 12.8 Hz, 3H), 2.12 (t, $J$ = 13.6 Hz, 1H), 1.97-1.76 (m, $J$ = 7.2 Hz, 2H), 0.87 (t, $J$ = 7.2 Hz, 3H).

Preparation Example 20: Preparation of (S)-9-ethyl-5-fluoro-4,9-dihydroxy-1,2,3,9,12,15-hexahydro-10H,13H-benzo [de]pyrano[3',4:6,7]indolizino[1,2-b]quinolin-10,13-dione (PY-13)

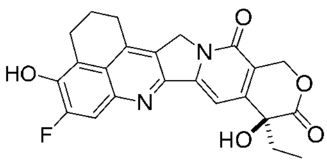

PY-13

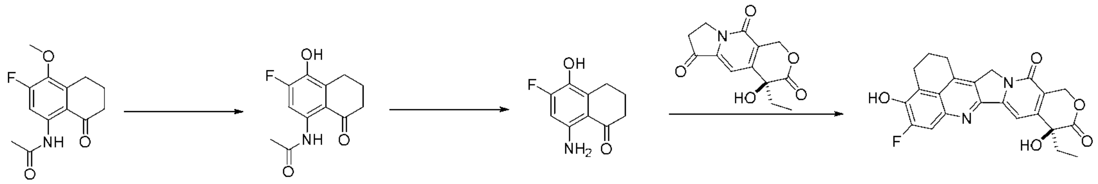

PY-88 PY-131 PY-132 PY-13

Step 1: Preparation of N-(3-fluoro-4-hydroxyl-8-oxy-5,6,7,8-tetrahydronaphthalene-1-yl)acetamide **(PY-131)**

3 equivalents of $AlCl_3$ was added to a solution of the compound N-(3-fluoro-4-methoxy-8-oxo-5,6,7,8-tetrahydronaphthalene-1-yl)acetamide **(PY-88)** (200 mg) in DCM, and the reaction solution reacted at 60°C for 2 hours.

Saturated $NaHCO_3$ solution (10 mL) was slowly added to quench the reaction and further adjust a pH value to 3 to 4, the reaction solution was extracted with ethyl acetate (3 × 30 mL), organic phases were combined, dried with anhydrous sodium sulfate and filtered, and the filtrate was concentrated under reduced pressure to obtain a yellow solid compound **PY-131** (130 mg, 68%).

LCMS (ESI): m/z, 238.0 $[M+H]^+$, 260.1 [M+Na].

Step 2: Preparation of 8-amino-6-fluoro-5 hydroxyl-3,4-dihydronaphthalene-1(2H)-one **(PY-132)**

A solution of the compound N-(3-fluoro-4-hydroxyl-8-oxy-5,6,7,8-tetrahydronaphthalene-1-yl)acetamide **(PY-131)** (70 mg) in HCl (12N, 3.5 mL) was stirred at 100°C for 16 hours. Saturated $NaHCO_3$ solution (10 mL) was slowly added to quench the reaction and further adjust a pH value to 3 to 4, and the reaction solution was extracted with ethyl acetate (3 × 30 mL). The organic phases were combined, dried with anhydrous sodium sulfate, and filtered, the filtrate was concentrated under reduced pressure, and 5 mL of MeCN was added. The resulting solution was isolated by preparative high performance liquid chromatograph (Oriendo, BRIX-2860; column: Phenomenex Luna C18 250 × 50 mm × 10 μm; mobile phase: water (0.225% trifluoroacetic acid)-acetonitrile, eluted from 45% to 85%), to obtain of a yellow solid compound **PY-132** (12 mg, 20%).

LCMS (ESI): m/z, 196.1 $[M+H]^+$.

Step 3: Preparation of (S)-9-ethyl-5-fluoro-4,9-dihydroxy-1,2,3,9,12,15-hexahydro-10H,13H-benzo[de]pyrano[3',4:6,7]indolizino[1,2-b]quinolin-10,13-dione **(PY-13)**

PPTS (0.67 eq, 12.9 mg) and (S)-4-ethyl-4-hydroxy-7,8-dihydro-1H-pyrano[3,4-f]indolizino-3,6,10(4H)-trione (1 eq, 20.2 mg) were added to a solution of 8-amino-6-fluoro-5-hydroxyl-3,4-dihydronaphthalene-1(2H)-one **(PY-132)** (15 mg) in toluene (10 mL) and the reaction solution reacted at 140°C for 16 hours. Toluene was removed under reduced pressure, and 5 mL of DMF was added. The resulting mixture was purified by preparative high performance liquid chromatograph (Oriendo, BRIX-2860; column: Phenomenex Luna C18 250 × 50 mm × 10 μm; mobile phase: water (0.225% HCOOH)-acetonitrile, eluted from 45% to 85%), to obtain a white solid compound **PY-13** (3.2 mg).

LCMS (ESI): m/z, 423.1 $[M+H]^+$.

$^1H$ NMR (400 MHz, DMSO-$d^6$) δ 7.77 (d, *J* = 11.9 Hz, 1H), 7.26 (s, 1H), 6.50 (s, 1H), 5.42 (s, 2H), 5.22 (s, 2H), 3.11 (t, *J* = 6.1 Hz, 2H), 3.03 (t, *J* = 6.2 Hz, 2H), 2.01 (q, *J* = 6.2 Hz, 2H), 1.87 (dq, *J* = 14.5, 7.1 Hz, 2H), 0.87 (t, *J* = 7.3 Hz, 3H).

Preparation Example 21: Preparation of (S)-4,11-diethyl-8,10-difluoro-4,9-dihydroxy-1,12-dihydro-14H-pyrano[3',4':6,7]indolizino[1,2-b]quinolin-3,14(4H)-dione **(PY-14)**

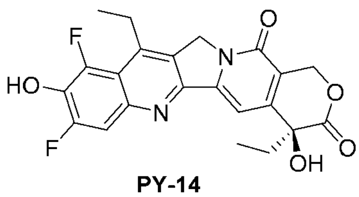

PY-14

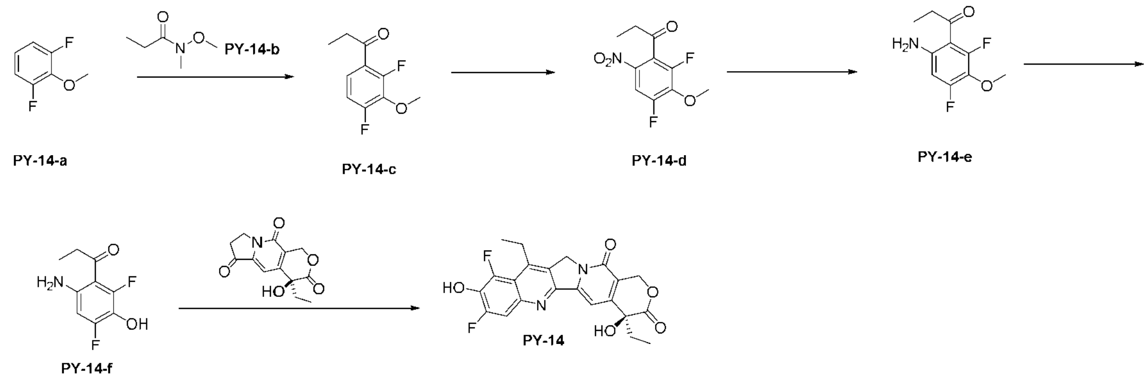


PY-14-a PY-14-c PY-14-d PY-14-e

PY-14-f PY-14

Step 1: Preparation of 1-(2,4-difluoro-3-methoxyphenyl)propyl-1-one (PY-14-c)

N-BuLi (1.07g, 10.41 mL, 16.65 mmol, 1.2 eq, 1.60 M) was slowly added dropwise to the compound 1,3-difluoro-2-methoxybenzene (PY-14-a) (2g, 13.88 mmol, 1 eq) in 20 mL of anhydrous THF at - 70°C, the reaction solution was stirred for half an hour at the same temperature, then compound N-methoxy-N-methylpropionamide (PY-14-b) (4.55g, 34.69 mmol, 2.5 eq) was added, the reaction solution was further stirred at -70°C for half an hour, the low temperature bath was removed, the temperature was raised to 25°C and the reaction solution was stirred for 16 hours. LCMS showed that a product was generated, and at room temperature, 5 mL of saturated ammonium chloride solution was used to quench the reaction, then 3 × 10 mL of ethyl acetate was used to extract the organic phase, the organic phase was washed with saturated brine, dried with anhydrous sodium sulfate, filtered, and concentrated under reduced pressure, and the residue was purified by silica gel column chromatography (eluent: petroleum ether: ethyl acetate = 10:1), to obtain a light yellow oily target compound (1.6g, yield: 54%).

LCMS (ESI): m/z, 214.9 $[M+H]^+$.

Step 2: Preparation of 1-(2,4-difluoro-3-methoxy-6-nitrophenyl)propyl-1-one (PY-14-d)

Fuming nitric acid (699.74 mg, 95%, 474.82 μL, 10.55 mmol, 0.96 eq) was slowly added dropwise to 22 mL of solution of 1-(2,4-difluoro-3-methoxyphenyl)propyl-1-one (2.20g, 10.99 mmol, 1 eq) in $H_2SO_4$ at -40°C and the reaction solution was stirred for 1 hour at the temperature. The reaction solution was added into ice water, the reaction flask was washed with 10 mL of absolute ethanol, 100 mL of DCM was added to extract the organic phase, the organic phase was washed with saturated brine, dried with anhydrous sodium sulfate, filtered, and concentrated under reduced pressure, and the residue was purified by silica gel column chromatography (eluent: petroleum ether: ethyl acetate = 10:1) and dried, to obtain a light yellow oily compound 1-(2,4-difluoro-3-methoxy-6-nitrophenyl)propyl-1-one (1.2g, yield: 34%).

LCMS (ESI): m/z, 246.1 $[M+H]^+$.

$^1$H NMR (400 MHz, DMSO-$d_6$) δ 8.24 - 8.13 (m, 1H), 4.16 (t, *J* = 2.1 Hz, 3H), 2.89 - 2.81 (m, 2H), 1.14 (t, *J* = 7.1 Hz, 3H).

Step 3: Preparation of 1-(6-amino-2,4-difluoro-3-methoxyphenyl)propyl-1-one (PY-14-e)

Iron powder (250.57 mg, 31.88 μL, 4.49 mmol, 5.5 eq), water (339.85 mg, 339.85 μL, 18.86 mmol, 23.12 eq) and concentrated hydrochloric acid (16.36 mg, 37.39 μL, 448.65 μmol, 0.55 eq, 12 M) were separately added to 10 mL of solution of 1-(2,4-difluoro-3-methoxy-6-nitrophenyl)propyl-1-one (Py-14-d) (200 mg, 815.73 μmol, 1 eq) in absolute ethanol, and the reaction solution was stirred at 80°C for 16 hours in a nitrogen atmosphere. The reaction solution was concentrated under reduced pressure, and the residue was purified by silica gel column chromatography (eluents: petroleum ether: ethyl acetate = 10:1), to obtain a white solid compound 1-(6-amino-2,4-difluoro-3-methoxyphenyl) propyl-1-one (141 mg, yield: 80%).

LCMS (ESI): m/z, 216.1 $[M+H]^+$.

Step 4: Preparation of 1-(6-amino-2,4-difluoro-3-hydroxyphenyl)propyl-1-one (PY-14-f)

3 equivalents of $AlCl_3$ (557.61 mg, 4.18 mmol, 3 eq) were added to 10 mL of solution of 1-(6-amino-2,4-difluoro-3-methoxyphenyl)propyl-1-one (Py-14-e) (300 mg, 1.39 mmol, 1 eq) in DCM, and the reaction solution was stirred at 70°C for 4 hours in a nitrogen atmosphere. The reaction was quenched with 100 mL of saturated ammonium chloride solution, and then the organic phase was extracted with 2 × 100 mL of DCM, washed with saturated brine, dried with anhydrous sodium sulfate, filtered, and concentrated under reduced pressure to obtain a yellow solid crude product of compound 1-(6-amino-2,4-difluoro-3-hydroxyphenyl)propyl-1-one (165 mg, no yield was calculated for the crude product).

LCMS (ESI): m/z, 202.0 $[M+H]^+$.

$^1$H NMR (400 MHz, DMSO-$d^6$) δ 9.06 (s, 1H), 6.42 (dd, *J* = 13.0, 2.1 Hz, 1H), 3.45 (s, 2H), 2.87-2.82 (m, 2H), 1.05 (t, *J* = 7.2 Hz, 3H).

Step 5: Preparation of (S)-4,11-diethyl-8,10-difluoro-4,9-dihydroxy-1,12-dihydro-14H-pyrano[3',4':6,7]indolizino[1,2-b] quinolin-3,14(4H)-dione **(PY-14)**

PPTS (25.11 mg, 99.92 μmol, 0.67 eq) and (S)-4-ethyl-4-hydroxy-7,8-dihydro-1H-pyrano[3,4-f]indolizino-3,6,10(4H)-trione (ChemExpress) (30 mg, 149.13 μmol, 1 eq) were separately added to 10 mL of solution of 1-(6-amino-2,4-difluoro-3-hydroxyphenyl)propyl-1-one (PY-14-f) (30 mg, 149.13 μmol, 1 eq) in toluene, and the reaction solution was stirred at 130°C for 16 hours in a nitrogen atmosphere. The reactant was purified by preparative high performance liquid chromatograph (preparative chromatograph manufacturer: SHIMADZU, model: LC-20AP, column:

YMC-Triart Prep C18 250 × 30 mm × 10 μm, mobile phase: water (0.225% HCOOH)-acetonitrile, an elution ratio of water: 12% to 42%) and lyophilized, to obtain a yellow solid (35.55 mg, 55.65%).

LCMS (ESI): m/z, 429.1 [M+H]+.

$^1$H NMR (400 MHz, DMSO-$d^6$) $\delta$ 7.84 (d, $J$ = 11.4 Hz, 1H), 7.26 (s, 1H), 6.52 (s, 1H), 5.43 (s, 2H), 5.31 (s, 2H), 3.22 - 3.15 (m, 2H), 1.86 (hept, $J$ = 7.1 Hz, 2H), 1.32 (t, $J$ = 7.4 Hz, 3H), 0.87 (t, $J$= 7.3 Hz, 3H).

Preparation Example 22: Preparation of (S)-4-(aminomethyl)-9-ethyl-5-fluoro-9-hydroxy-1,2,3,9,12,15-hexahydro-10H,13H-benzo[de]pyrano[3',4':6,7]indolizino[1,2-b]quinolin-10,13-dione **(PY-16)**

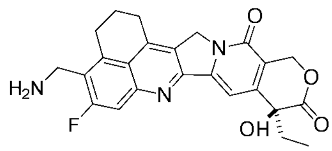

**PY-16**

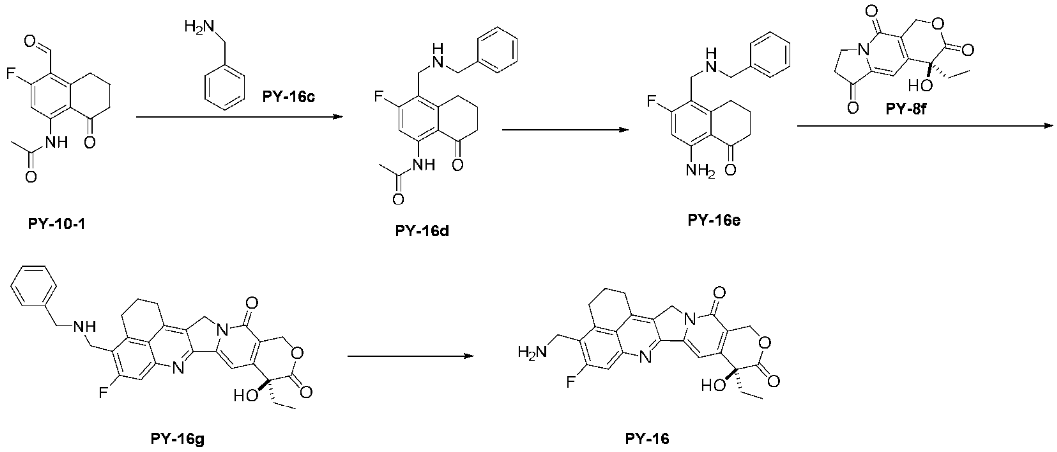


Step 1: Preparation of N-(4-((benzylamino)methyl)-3-fluoro-8-oxo-5,6,7,8-tetrahydronaphthalene-1-yl)acetamide (PY-16d)

NaBH(OAc)$_3$ (2.06g, 9.79 mmol, 2 eq) was added to a solution of compound N-(3-fluoro-4-formyl-8-oxo-5,6,7,8-tetrahydronaphthalene-1-yl)acetamide (1.22g, 8.5 mmol, 1 eq) and benzylamine (642.29 μL) in DCM (110 mL). The reaction solution was stirred for 12 hours at room temperature. Water (100 mL) was added to quench the reaction, the reaction solution was extracted with DCM (50 mL) three times, the organic phases were combined, washed with saturated brine (100 mL), dried with anhydrous sodium sulfate, filtered and concentrated. The residue was purified by silica gel column chromatography (eluents: ethyl acetate/petroleum ether = 1/5-1/2) to obtain a brown oily substance N-(4-((benzylamino)methyl)-3-fluoro-8-oxo-5,6,7,8-tetrahydronaphthalene-1-yl)acetamide (PY-16d) (499.5 mg, yield: 30%).

LCMS (ESI): m/z, 341[M+H]+.

Step 2: Preparation of 8-amino-5-((benzylamino)methyl)-6-fluoro-3,4-dihydronaphthalene-1(2H)-one (PY-16e)

6N HCl (3 mL) and EtOH (3 mL) were added to the compound N-(4-((benzylamino)methyl)-3-fluoro-8-oxo-5,6,7,8-tetrahydronaphthalene-1-yl)acetamide (499.5 mg,1.47 mmol,1 eq), the reaction solution was stirred at 60°C for 3 hours, and concentrated under reduced pressure, to obtain a brown oily crude product 8-amino-5-((benzylamino)methyl)-6-fluoro-3,4-dihydronaphthalene-1(2H)-one (PY-16e) (517.9 mg).

LCMS (ESI): m/z, 341[M+H]+.

Step 3: Preparation of (S)-4-((benzylamino)methyl)-9-ethyl-5-fluoro-9-hydroxy-1,2,3,9,12,15-hexahydro-10H,13H-benzo[de]pyrano[3',4':6,7]indolizino[1,2-b]quinolin-10,13-dione (PY-16g)

PPTS (305.35 mg, 1.22 mmol, 1 eq) was added to a solution of the compound 8-amino-5-((benzylamino)methyl)-6-fluoro-3,4-dihydronaphthalene-1(2H)-one (517.9 mg, 1.22 mmol, 1 eq) and compound (S)-4-ethyl-4-hydroxy-7,8-dihydro-1H-pyran[3,4-f]indolizino-3,6,10(4H)-trione (ChemExpress) (319.87 mg, 1.22 mmol, 1 eq) in toluene (15 mL) and the reaction solution was stirred at 120°C for 12 hours. Water (100 mL) was added to quench the reaction, then the reaction solution was extracted with DCM (50 mL) three times, the organic phases were combined, washed with saturated brine (100 mL), dried with anhydrous sodium sulfate, filtered and concentrated. The residue was purified by silica gel column chromatography (eluents: dichloromethane/methanol = 12/1-11/1) to obtain a brown oily substance (S)-4-((benzylamino)methyl)-9-ethyl-5-fluoro-9-hydroxy-1,2,3,9,12,15-hexahydro-10H,13H-benzo[de]pyrano[3',4':6,7]indolizino [1,2-b]quinolin-10,13-dione (PY-16g) (466 mg, yield: 73%).

LCMS (ESI): m/z, 526[M+H]$^+$.

Step 4: Preparation of (S)-4-(aminomethyl)-9-ethyl-5-fluoro-9-hydroxy-1,2,3,9,12,15-hexahydro-10H,13H-benzo[de] pyrano[3',4':6,7]indolizino[1,2-b]quinolin-10,13-dione (PY-16)

Pd/C (100 mg, 939.67 μmol, 2.27 eq) and $Pd(OH)_2$ (100 mg, 712.1 μmol, 1.72 eq) were added to a solution of compound (S)-4-((benzylamino)methyl)-9-ethyl-5-fluoro-9-hydroxy-1,2,3,9,12,15-hexahydro-10H,13H-benzo[de]pyrano[3',4':6,7]indolizino[1,2-b]quinolin-10,13-dione (218 mg, 414.78 μmol, 1 eq) in MeOH (10 mL) in a hydrogen atmosphere. The reaction solution was stirred at room temperature for 5 hours, filtered with diatomite to remove Pd/C and then concentrated to obtain the brown solid product (S)-4-(aminomethyl)-9-ethyl-5-fluoro-9-hydroxy-1,2,3,9,12,15-hexahydro-10H,13H-benzo[de]pyrano[3',4':6,7]indolizino[1,2-b]quinolin-10,13-dione (PY-16) (296.6 mg, yield: 82%).

LCMS (ESI): m/z, 436[M+H]$^+$.

$^1$H NMR (400 MHz, DMSO-$d^6$) δ 8.27 (s, 1H), 7.75 (d, *J*= 11.2 Hz, 1H), 7.31 (s, 1H), 6.54 (s, 1H), 5.44 (s, 2H), 5.24 (s, 2H), 4.00 (s, 2H), 3.25 (t, *J* = 5.8 Hz, 2H), 3.16 (t, *J* = 6.2 Hz, 2H), 2.09 (t, *J* = 6.2 Hz, 2H), 1.87 (hept, *J* = 7.0 Hz, 2H), 0.88 (t, *J* = 7.2 Hz, 3H);$^{19}$F NMR (377 MHz, DMSO-$d_6$) δ-113.60.

Preparation Example 23: Preparation of 2-hydroxyethyl (S)-((9-ethyl-5-fluoro-9-hydroxy-10,13-dioxo-2,3,9,10,13,15-hexahydro-1H,12H-benzo[de]pyrano[3',4':6,7]indolizino[1,2-b]quinolin-4-yl)methyl)carbamate **(PY- 16Car)**

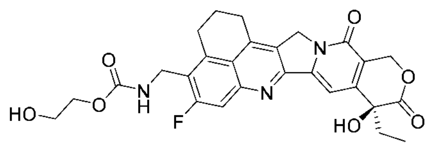

PY-16Car

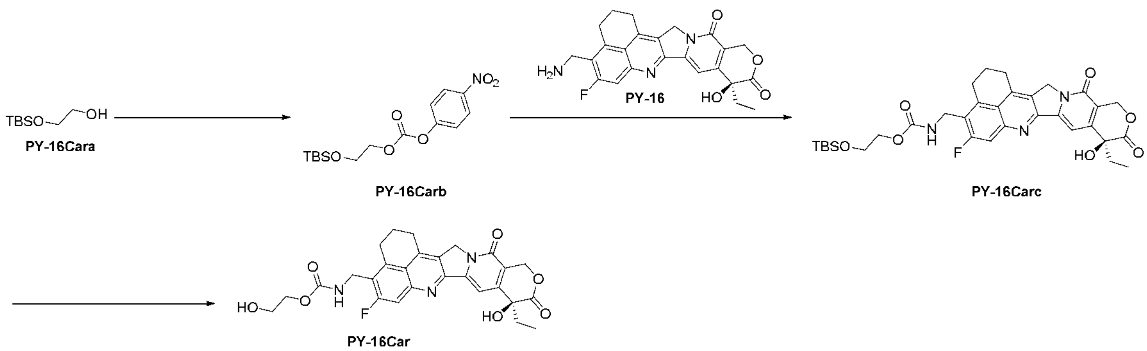

PY-16Cara

PY-16Carb

PY-16

PY-16Carc

PY-16Car

Step 1: Preparation of 2-((tert-butyldimethylsilyl)oxy)ethyl(4-nitrophenyl)carboxylate (PY-16Carb)

2-(tert-butyldimethylsilyloxy)ethanol (2.0g, 11.34 mmol, 1.0 eq) was added to a solution of bis(4-nitrophenyl) carbonate (NPC) (5.18g, 17.01 mmol, 1.5 eq) and N,N-diisopropylethylamine (DIPEA, 4.40g, 34.03 mmol, 3.0 eq) in tetrahydrofuran (120 mL) at 0°C, and the reaction solution was stirred at 25°C for 16 hours. The reaction solution was concentrated under reduced pressure and purified by silica gel column chromatography with an eluent system of ethyl acetate/petroleum ether (0%-10%), to obtain the yellow oily compound PY-16Carb (2.70g, 7.91 mmol, yield: 70%).

$^1$H NMR (400 MHz, $CHCl_3$-d) δ 8.28 (d, *J* = 9.2 Hz, 2H), 7.38 (d, *J* = 9.2 Hz, 2H), 4.36 (dd, *J* = 5.6, 4.1 Hz, 2H), 3.91 (dd, *J* = 5.6, 4.1 Hz, 2H), 0.91 (s, 9H), 0.10 (s, 6H).

Step 2: Preparation of 2-((tert-butyldimethylsilyl)oxy)ethyl(S)-((9-ethyl-5-fluoro-9-hydroxy-10,13-dioxo-2,3,9,10,13,15-hexahydro-1H,12H-benzo[de]pyrano[3',4':6,7]indolizino[1,2-b]quinolin-4-yl)methyl)carbamate(PY-16Carc)

Compound 2-((tert-butyldimethylsilyl)oxy)ethyl(4-nitrophenyl)carboxylate (35.75 mg, 0.105 mmol, 1.2 eq) and compound (S)-4-(aminomethyl)-9-ethyl-5-fluoro-9-hydroxy-1,2,3,9,12,15-hexahydro-10H,13H-benzo[de]pyrano [3',4':6,7]indolizino[1,2-b]quinolin-10,13-dione (38.0 mg, 0.087 mmol, 1.0 eq) and N,N-diisopropylethylamine (33.84 mg, 0.262 mmol, 3.0 eq) were added to a solution of N,N-dimethylformamide (2 mL) at room temperature, and the reaction solution was stirred at 25°C for 1 hour. The reaction solution was directly used for the next step.

LCMS (ESI): m/z, 638.3 $[M+H]^+$.

Step 3: Preparation of 2-hydroxyethyl (S)-((9-ethyl-5-fluoro-9-hydroxy-10,13-dioxo-2,3,9,10,13,15-hexahydro-1H,12H-benzo[de]pyrano[3',4':6,7]indolizino[1,2-b]quinolin-4-yl)methyl)carbamate (PY- 16Car)

MeOH (2 mL) and 4 N hydrochloric acid were added to the reaction solution in the previous step and the resulting solution was stirred at 25°C for 1 hour. The reaction solution was purified by high performance liquid chromatograph (preparative chromatograph manufacturer: Luna, model: Lab311-ISCO-R4, Phenomenex Luna C18 (250×50 mm×10 μm) was used as a chromatographic column, mobile phase: water (0.225% HCOOH)-acetonitrile, an elution ratio of water: 12% to 42%) and the eluate was lyophilized, to obtain a white solid product PY 16Car (20.4 mg, 0.039 mmol, two-step yield: 45%).

LCMS (ESI): m/z, 524.2 $[M+H]^+$.

$^1$H NMR (400 MHz, DMSO-$d_6$) δ 7.73 (d, *J* = 11.2 Hz, 1H), 7.65 (t, *J* = 5.6 Hz, 1H), 7.31 (s, 1H), 6.52 (s, 1H), 5.43 (s, 2H), 5.24 (s, 2H), 4.71 (t, *J* = 5.2 Hz, 1H), 4.43 (d, *J* = 5.2 Hz, 2H), 3.96 (t, *J* = 5.2 Hz, 2H), 3.52 (q, *J* = 5.2 Hz, 2H), 3.24 (t, *J* = 6.0 Hz, 2H), 3.15 (t, *J* = 6.4 Hz, 2H), 2.12 - 2.02 (m, 2H), 1.87 (hept, *J* = 7.2 Hz, 2H), 0.88 (t, *J* = 7.2 Hz, 3H).

Preparation Example 24: Preparation of (S)-4-(2-aminoethoxy)-9-ethyl-5-fluoro-9-hydroxy-1,2,3,9,12,15-hexahydro-10H,13H-benzo[d]pyrano[3',4':6,7]indolizino[1,2-b]quinolin-10,13-dione **(PY-17)**

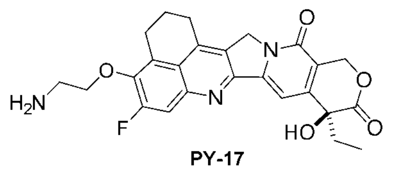

PY-17

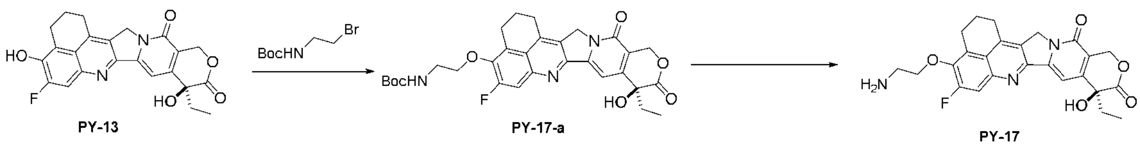

PY-13 PY-17-a PY-17

Step 1: Preparation of (S)-(2-((9-ethyl-5-fluoro-9-hydroxy-10,13-dioxy-2,3,9,10,13,15-hexahydro-1H,12H-benzo[d]pyrano[3',4':6,7]indolizino[1,2-b]quinolin-4-yl)oxy)ethyl)tert-butyl carbamate (PY-17-a)

Potassium carbonate (10.7 mg, 77.26 μmol, 2.0 eq) was added to a solution of compound PY-13 (41 mg, 38.63 μmol, 1.0 eq) in DMF and the resulting solution was stirred at 25°C for half an hour, then (2-bromoethyl)tert-butyl carbamate (21.6 mg, 91.6 μmol, 2.5 eq) was added at the same temperature and the reaction solution was stirred at 25°C for 16 hours. The reactant was purified by preparative high performance liquid chromatograph (preparative chromatograph manufacturer: SHIMADZU, model: LC-20AP, column: YMC-Triart Prep C18 250 × 30 mm × 10 μm, mobile phase: water (0.225% HCOOH)-acetonitrile, an elution ratio of water: 12% to 42%) and lyophilized, to obtain a gray solid compound **PY-17-a** (5 mg, yield: 23%).

LCMS (ESI): m/z, 566.2 $[M+H]^+$.

Step 2: Preparation of (S)-4-(2-aminoethoxy)-9-ethyl-5-fluoro-9-hydroxy-1,2,3,9,12,15-hexahydro-10H,13H-benzo[d]pyrano[3',4':6,7]indolizino[1,2-b]quinolin-10,13-dione **(PY-17)** 0.5 mL of TFA was slowly added dropwise to 5 mL of solution of compound PY-17-a (7 mg, 12.38 μmol, 1.0 eq) in DCM under an ice water bath condition, and the reaction solution was naturally warmed to 25°C under the ice water bath condition and stirred for 16 hours. The reactant was purified by preparative high performance liquid chromatograph (preparative chromatograph manufacturer: SHIMADZU, model: LC-20AP, column: YMC-Triart Prep C18 250 × 30 mm × 10 μm, mobile phase: water (0.225% HCOOH)-acetonitrile, an

elution ratio of water: 12% to 42%) and lyophilized, to obtain a white solid compound **PY-17** (1.97 mg, yield: 34%).

LCMS (ESI): m/z, 466.2 $[M+H]^+$.

$^1H$ NMR (400 MHz, DMSO-$d^6$) $\delta$ 8.06 (s, 3H), 7.90 (d, $J$ = 12.3 Hz, 1H), 7.30 (s, 1H), 6.52 (s, 1H), 5.44 (s, 2H), 5.27 (s, 2H), 4.27 (t, $J$ = 5.1 Hz, 2H), 3.17 (t, $J$ = 6.1 Hz, 5H), 2.06 (t, $J$ = 6.2 Hz, 2H), 1.87 (p, $J$ = 6.8 Hz, 2H), 0.88 (t, $J$ = 7.3 Hz, 3H).

Preparation Example 25: Preparation of (S)-9-ethyl-5-fluoro-9-hydroxy-4-(2-hydroxyethyl)-1,2,3,9,12,15-hexahydro-10H,13H-benzo[de]pyrano[3',4':6,7]indolizino[1,2-b]quinolin-10,13-dione **(PY-18)**

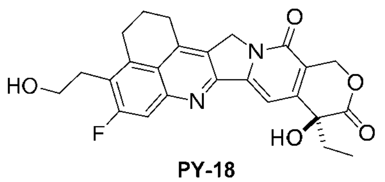

**PY-18**

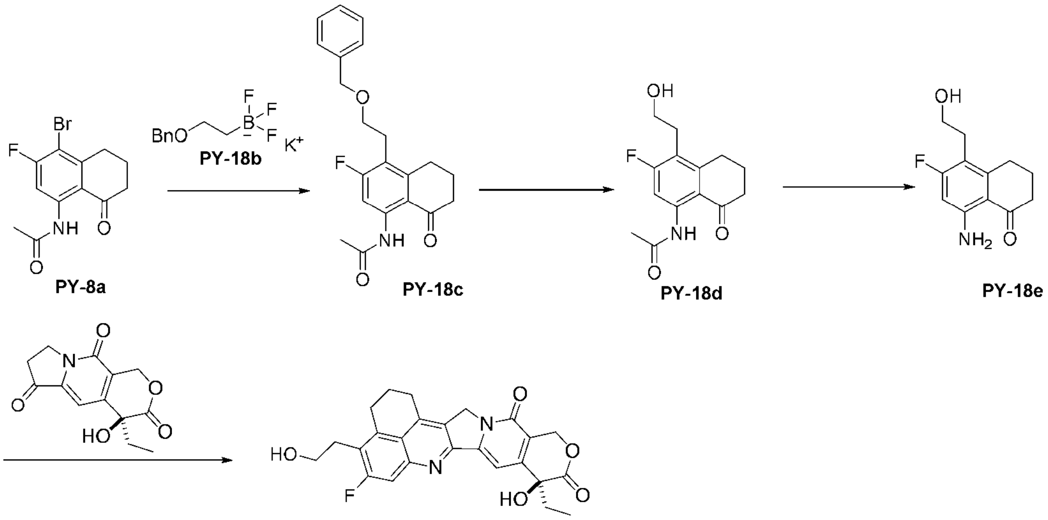
PY-18b
PY-8a
PY-18c
PY-18d
PY-18e

PY-18

Step 1: Preparation of N-(4-(2-(benzyloxy)ethyl)-3-fluoro-8-oxo-5,6,7,8-tetrahydronaphthalene-1-yl)acetamide (PY-18c)

3.3 equivalents of compound potassium (2-(benzyloxy)ethyl)trifluoroborate (532 mg), 3 equivalents of potassium carbonate, 30 mg of palladium acetate and 88 mg of S-Phos ligand were sequentially separately added to a mixed solution of compound N-(4-bromo-3-fluoro-8-oxo-5,6,7,8-tetrahydronaphthalene-1-yl)acetamide (200 mg) in 12.5 mL of toluene and water (volume ratio: 4:1) in a nitrogen atmosphere. The reaction solution was stirred at 100°C for 16 hours in a nitrogen atmosphere, and concentrated under reduced pressure, and then the residue was purified by silica gel column chromatography (eluents: ethyl acetate/n-hexane = 1:20-1:8) to obtain 140 mg of the white solid compound N-(4-(2-(benzyloxy)ethyl)-3-fluoro-8-oxo-5,6,7,8-tetrahydronaphthalene-1-yl)acetamide (PY-18c) (yield: 59%).

LCMS (ESI): m/z, 356 $[M+1]^+$.

$^1H$ NMR (400 MHz, DMSO-$d_6$) δ 12.26 (s, 1H), 8.29 (d, $J$ = 13.3 Hz, 1H), 5.10 (t, $J$ = 5.3 Hz, 1H), 4.50 (dd, $J$ = 5.4, $J$ = 2.2 Hz, 2H), 3.10 (t, $J$ = 6.2 Hz, 2H), 2.67 (dd, $J$ = 7.3, $J$ = 5.8 Hz, 2H), 2.16 (s, 3H), 1.99 (p, $J$ = 6.3 Hz, 2H).

Step 2: Preparation of N-(3-fluoro-4-(2-hydroxyethyl)-8-oxo-5,6,7,8-tetrahydronaphthalene-1-yl)acetamide (PY-18d)

0.25 equivalents of Pd/C catalyst and 0.25 equivalents of $Pd(OH)_2$ catalyst were separately added to a solution of the compound N-(4-(2-(benzyloxy)ethyl)-3-fluoro-8-oxo-5,6,7,8-tetrahydronaphthalene-1-yl)acetamide (140 mg) in 10 mL of methanol, and the reaction solution reacted at 25°C for 16 hours in a hydrogen atmosphere. After the catalysts were filtered out, the reaction solution was concentrated under reduced pressure and dried to obtain a pale solid crude product of a compound N-(3-fluoro-4-(2-hydroxyethyl)-8-oxo-5,6,7,8-tetrahydronaphthalene-1-yl)acetamide (PY-18d) (100 mg, no yield was calculated for the crude product).

LCMS (ESI): m/z, 266 $[M+H]^+$, 288 $[M+Na]^+$.

Step 3: Preparation of 8-amino-6-fluoro-5 (2-hydroxyethyl)-3,4-dihydronaphthalene-1(2H)-one (PY-18e)

4 mL of 6N HCl was added to the compound N-(3-fluoro-4-(2-hydroxyethyl)-8-oxo-5,6,7,8-tetrahydronaphthalene-1-yl)acetamide (80 mg) and the reaction solution reacted at 40°C for 3 hours. The reaction solution was cooled to room temperature, diluted with water, and adjusted with 2N dilute hydrochloric acid to adjust the pH value to 3-5, then ethyl acetate was used to extract the organic phase, the organic phase was then washed with saturated brine, then dried with anhydrous sodium sulfate, and concentrated under reduced pressure, to obtain 30 mg of yellow solid compound 8-amino-6-fluoro-5 (2-hydroxyethyl)-3,4-dihydronaphthalene-1(2H)-one (Py-18e) (yield: 37%).
LCMS (ESI): m/z, 224 $[M+H]^+$.

Step 4: Preparation of (S)-9-ethyl-5-fluoro-9-hydroxy-4-(2-hydroxyethyl)-1,2,3,9,12,15-hexahydro-10H,13H-benzo[de]pyrano[3',4':6,7]indolizino[1,2-b]quinolin-10,13-dione **(PY-18)**

0.67 equivalents of PPTS (18.8 mg) and 1 equivalent of compound (S)-4-ethyl-4-hydroxy-7,8-dihydro-1H-pyran[3,4-f]indolizino-3,6,10(4H)-trione (29.4 mg) (ChemExpress) were separately added to a solution of compound 8-amino-6-fluoro-5 (2-hydroxyethyl)-3,4-dihydronaphthalene-1(2H)-one in 5 mL of toluene in a nitrogen atmosphere, and the reaction solution was stirred at 130°C for 16 hours and isolated by preparative high performance liquid chromatograph (preparative chromatograph manufacturer: SHIMADZU, model: LC-20AP, column: Synergi Max-RP 280 × 30 mm × 10 μm, mobile phase: water (0.225% HCOOH)-acetonitrile, an elution ratio of water: 12% to 42%) and lyophilized, to obtain 6.0 mg of a yellow solid compound (S)-9-ethyl-5-fluoro-9-hydroxy-4-(2-hydroxyethyl)-1,2,3,9,12,15-hexahydro-10H,13H-benzo[de]pyrano[3',4':6,7]indolizino[1,2-b]quinolin-10,13-dione **(PY-18)** (yield: 12%).
LCMS (ESI): m/z, 451 $[M+H]^+$.
$^1$H NMR (400 MHz, DMSO-$d^6$) δ 7.71 (dd, $J$ = 11.3, 6.3 Hz, 1H), 7.33 - 7.28 (m, 1H), 6.54 (s, 1H), 5.43 (s, 2H), 5.23 (d, $J$ = 11.2 Hz, 2H), 4.88 (t, $J$ = 5.3 Hz, 1H), 3.61 (d, $J$ = 6.5 Hz, 2H), 3.20 - 3.12 (m, 4H), 3.01 (d, $J$ = 6.6 Hz, 2H), 2.07 (s, 2H), 1.87 (hept, $J$ = 7.0 Hz, 2H), 0.88 (t, $J$ = 7.3 Hz, 3H). $^{19}$F NMR (377 MHz, DMSO-$d_6$) δ -112.5.

Preparation Example 26: Preparation of (S)-4-(3-aminopropyl)-9-ethyl-5-fluoro-9-hydroxy-1,2,3,9,12,15-hexahydro-10H,13H-benzo[de]pyrano[3',4':6,7]indolizino[1,2-b]quinolin-10,13-dione **(PY-19)**

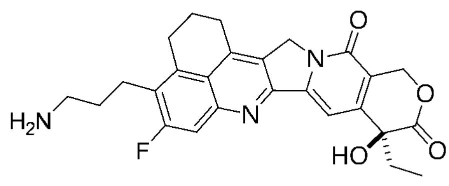

**PY-19**

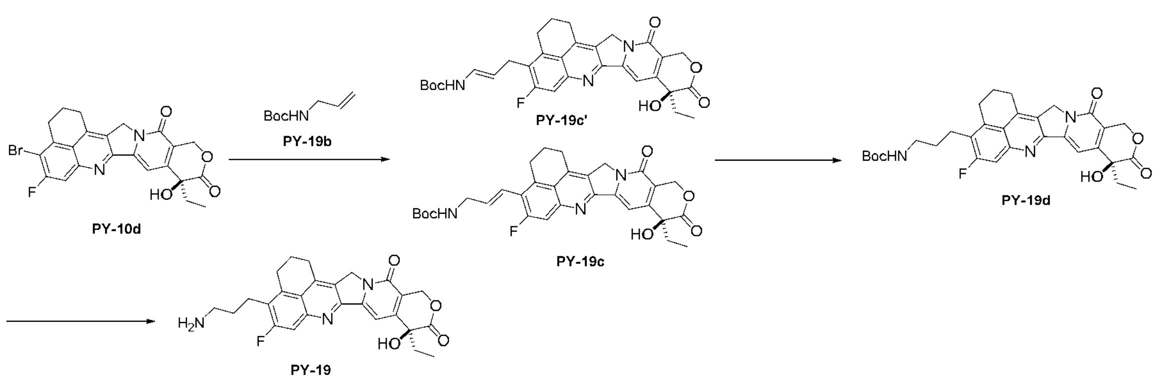

Step 1: Preparation of mixed intermediates of (S,E)-(3-(9-ethyl-5-fluoro-9-hydroxy-10,13-dioxy-2,3,9,10,13,15-hexahydro-1H,12H-benzo[de]pyrano[3',4':6,7]indolizino[1,2-b]quinolin-4-yl)allyl)tert-butyl carbamate (PY-19c) and (S,E)-(3-(9-ethyl-5-fluoro-9-hydroxy-10,13-dioxy-2,3,9,10,13,15-hexahydro-1H,12H-benzo[de]pyrano[3',4':6,7]indolizino[1,2-b]quinolin-4-yl)prop-1-en-1-yl)tert-butyl carbamate (PY-19c')

0.1 equivalents of bis(tert-butylphosphine) palladium (2.1 mg), 0.2 equivalents of tri(o-tolyl)-phosphine (2.5 mg), 6 equivalents of DIPEA (41 μL), and 2.5 equivalents of compound tert-butyl allylcarbamate (17.8 mg) were separately added to a solution of compound (S)-4-bromo-9-ethyl-5-fluoro-9-hydroxy-1,2,3,9,12,15-hexahydro-10H,13H-benzo[de]pyrano[3',4':6,7]indolizino[1,2-b]quinolin-10,13-dione (20 mg) in 5 mL of toluene. The reaction solution was stirred at 120°C for 16 hours, the reaction solution was concentrated, then purified by silica gel column chromatography (eluents: dichloromethane/methanol = 20:1-10:1), and concentrated under reduced pressure to obtain 10 mg of compounds (S,E)-(3-(9-ethyl-5-fluoro-9-hydroxy-10,13-dioxy-2,3,9,10,13,15-hexahydro-1H,12H-benzo[de]pyrano[3',4':6,7]indolizino[1,2-b]quinolin-4-yl)allyl)tert-butyl carbamate (PY-19c) and (S,E)-(3-(9-ethyl-5-fluoro-9-hydroxy-10,13-dioxy-2,3,9,10,13,15-hexahydro-1H,12H-benzo[de]pyrano[3',4':6,7]indolizino[1,2-b]quinolin-4-yl)prop-1-en-1-yl)tert-butyl carbamate (PY-19c') as a mixture (yield: 43%; purity: 77%).

Step 2: Preparation of (S)-(3-(9-ethyl-5-fluoro-9-hydroxy-10,13-dioxo-2,3,9,10,13,15-hexahydro-1H,12H-benzo[de]pyrano[3',4':6,7]indolizino[1,2-b]quinolin-4-yl)propyl)tert-butyl carbamate **(PY- 19d)**

0.25 equivalents of Pd/C (14.2 mg) and 0.25 equivalents of $Pd(OH)_2$ (9.4 mg) were separately added to a solution of the mixture (S,E)-(3-(9-ethyl-5-fluoro-9-hydroxy-10,13-dioxy-2,3,9,10,13,15-hexahydro-1H,12H-benzo[de]pyrano[3',4':6,7]indolizino[1,2-b]quinolin-4-yl)allyl)tert-butyl carbamate (PY-19c) and (S,E)-(3-(9-ethyl-5-fluoro-9-hydroxy-10,13-dioxy-2,3,9,10,13,15-hexahydro-1H,12H-benzo[de]pyrano[3',4':6,7]indolizino[1,2-b]quinolin-4-yl)prop-1-en-1-yl)tert-butyl carbamate (PY-19c') (30 mg) in 5 mL of methanol. The reaction solution was stirred at 25°C for 16 hours in a hydrogen atmosphere, and after filtration, the reaction solution was concentrated under reduced pressure, to obtain 19.6 mg of crude product of compound (S)-(3-(9-ethyl-5-fluoro-9-hydroxy-10,13-dioxo-2,3,9,10,13,15-hexahydro-1H,12H-benzo[de]pyrano[3',4':6,7]indolizino[1,2-b]quinolin-4-yl)propyl)tert-butyl carbamate (PY-19d) (yield: 65%), which was directly used for the next reaction.

LCMS (ESI): m/z, 564.3 $[M+1]^+$.

Step 3: Preparation of (S)-4-(3-aminopropyl)-9-ethyl-5-fluoro-9-hydroxy-1,2,3,9,12,15-hexahydro-10H,13H-benzo[de]pyrano[3',4':6,7]indolizino[1,2-b]quinolin-10,13-dione (PY-19)

0.8 mL of TFA was slowly added to a solution of compound (S)-(3-(9-ethyl-5-fluoro-9-hydroxy-10,13-dioxo-2,3,9,10,13,15-hexahydro-1H,12H-benzo[de]pyrano[3',4':6,7]indolizino[1,2-b]quinolin-4-yl)propyl)tert-butyl carbamate (16.3 mg, purity: 82%) in 2.4 mL of dichloromethane under an ice water bath condition, and the reaction solution was naturally warmed to 25°C and stirred for 16 hours. The reaction solution was concentrated under reduced pressure, and the residue was purified by silica gel column chromatography (eluents: dichloromethane/methanol = 20:1-8:1), to obtain a crude product, and then the crude product was purified by preparative high performance liquid chromatograph (preparative chromatograph manufacturer: SHIMADZU, model: LC-20AP, column: Welch Xtimate C18 250 × 50 mm × 10 μm, mobile phase: water (0.225% FA)-ACN, an elution ratio of water: 12% to 42%) and lyophilized, to obtain 2.53 mg of a compound (S)-4-(3-aminopropyl)-9-ethyl-5-fluoro-9-hydroxy-1,2,3,9,12,15-hexahydro-10H,13H-benzo[de]pyrano[3',4':6,7]indolizino[1,2-b]quinolin-10,13-dione **(PY-19)** (yield: 12%).

LCMS (ESI): m/z, 473.2 $[M+H]^+$.

$^1$H NMR (400 MHz, DMSO-$d_6$) δ $^1$H NMR (400 MHz, DMSO-$d_6$) δ 8.39 (s, 1H), 7.75 (d, *J* = 11.4 Hz, 1H), 7.30 (s, 1H), 6.53 (s, 1H), 5.43 (s, 2H), 5.25 (s, 2H), 3.16 (d, *J* = 6.5 Hz, 6H), 2.88 (s, 2H), 2.82 (s, 2H), 2.08 (t, *J* = 6.2 Hz, 2H), 1.87 (p, *J* = 7.0 Hz, 2H), 1.76 (s, 2H), 0.88 (t, *J* = 7.3 Hz, 3H);$^{19}$ F NMR (377 MHz, DMSO-$d_6$) δ -113.12.

Preparation Example 27: Preparation of (S)-4-(2-aminoethyl)-9-ethyl-5-fluoro-9-hydroxy-1,2,3,9,12,15-hexahydro-10H,13H-benzo[depyrano[3',4':6,7]indolizino[1,2-b]quinolin-10,13-dione **(PY-20)**

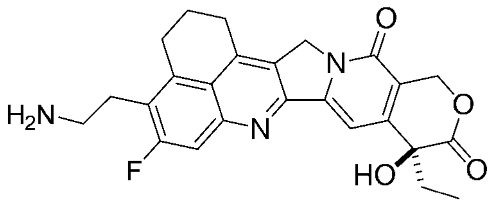

**PY-20**

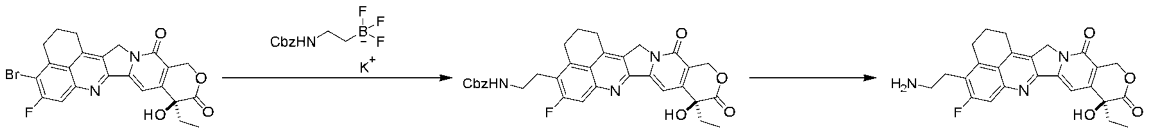


PY-10d PY-20a PY-20

Step 1: Preparation of (S)-(2-(9-ethyl-5-fluoro-9-hydroxy-10,13-dioxo-2,3,9,10,13,15-hexahydro-1H,12H-benzo[de] pyrano[3',4':6,7]indolizino[1,2-b]quinolin-4-yl)ethyl)carbamic acid benzyl ester **(PY -20a)**

The compound PY-10d (90.91 mg, 66%, 123.63 μmol, 1 eq) was dissolved in dioxane (4 mL)/water (1 mL), then potassium benzyl N-[2-(trifluoroboranuidyl)ethyl]carbamate (148.91 mg, 494.53 μmol, 4 eq), tri(o-tolyl)-phosphine (18.81 mg, 61.82 μmol, 0.5 eq), and potassium phosphate (78.73 mg, 30.71 μL, 370.90 μmol, 3 eq) were added, and the reaction solution was stirred at 90°C for 12 hours in a nitrogen atmosphere. The reaction solution was concentrated under reduced pressure and filtered, and the residue solution was purified by preparative high performance liquid chromatograph (preparative chromatograph manufacturer: Oriendo, model: BRIX-2860 (R1, 4,5,6), column: Phenomenex Luna C18 250 × 50 mm × 10 μm, mobile phase: water (0.225% HCOOH)-acetonitrile, an elution ratio of water: 30% to 60%), to obtain a white solid product **PY -20a** (10 mg, yield 13.86%).

LCMS (ESI): m/z, 584.2 $[M+H]^+$.

Step 2: Preparation of (S)-4-(2-aminoethyl)-9-ethyl-5-fluoro-9-hydroxy-1,2,3,9,12,15-hexahydro-10H,13H-benzo[depyrano[3',4':6,7]indolizino[1,2-b]quinolin-10,13-dione **(PY-20)**

The compound **PY-20a** (10 mg, 17.13 μmol, 1 eq) was dissolved in MeCN (0.5 mL), TMSI (56 mg, 40 μL, 279.87 μmol, 16.334 eq) was added to the solution, and the solution was stirred at 25°C for 12 hours. The reaction solution was purified by preparative high performance liquid chromatograph (preparative chromatograph manufacturer: SHIMADZU, model: LC-20AP, column: YMC-Triart Prep C18 250 × 30 mm × 10 μm, mobile phase: water (0.225% HCOOH)-acetonitrile, an elution ratio of water: 3% to 33%), to obtain a white solid product (5.16 mg, yield: 67%, purity: 97.64%).

LCMS (ESI): m/z, 450.2 $[M+H]^+$.

Preparation Example 28: Preparation of (S)-4-ethyl-8-fluoro-4,9-dihydroxy-11-propyl-1,12-dihydro-14H-pyrano [3',4':6,7]indolizino[1,2-b]quinolin-3,14(4H)-dione **(PY-21-A)**

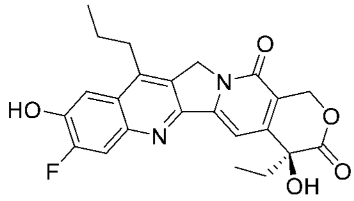

**PY-21-A**

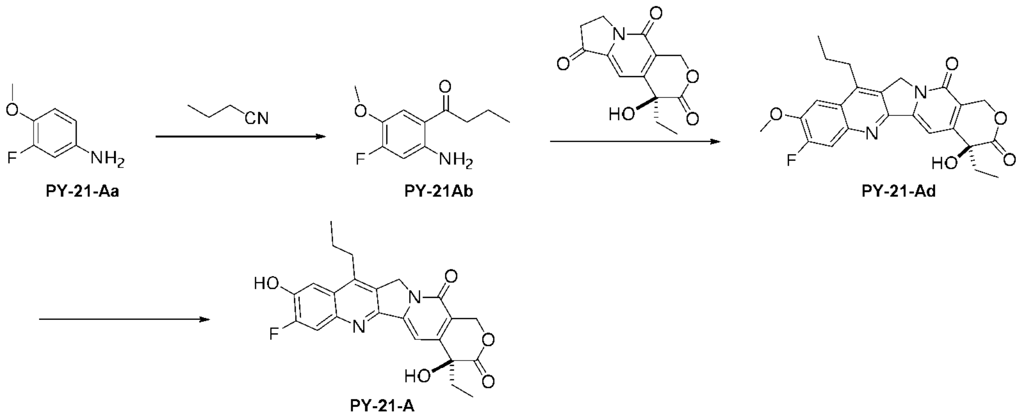

PY-21-Aa

PY-21Ab

PY-21-Ad

PY-21-A

Step 1: Preparation of 1-(2-amino-4-fluoro-5-methoxyphenyl)but-1-one **(PY-21-Ab)**

$BCl_3$ (1 eq, 830.04 mg, 7.08 mmol) was added to 10 mL of anhydrous benzene solvent under an ice water bath, then 3-fluoro-4-methoxyaniline (1 eq, 1g, 7.08 mmol) was added to a solution of 20 mL of anhydrous benzene solvent, and nitrile (2 eq, 979.24 mg, 14.17 mmol, 1.23 mL) and $AlCl_3$ (1.1053 eq, 1.04g, 1.04 mol) were sequentially added in a nitrogen atmosphere, and the reaction solution reacted at 100°C for 16 hours. After cooling in the ice water bath, 50 mL of 2M hydrochloric acid was slowly added and then the mixture solution was further stirred at 80°C for 1 hour. 100 mL of water was used to quench the reaction in the ice water bath, then 2 × 100 mL of ethyl acetate was used to extract the organic phase, the organic phase was washed with saturated brine, dried with anhydrous sodium sulfate, filtered, and concentrated under reduced pressure, and the residue was purified by silica gel column chromatography (eluents: petroleum ether: ethyl acetate = 10:1), to obtain a light yellow solid compound PY-21-Ab (440 mg, yield: 29%).
LCMS (ESI): m/z, 212.1 $[M+H]^+$.

Step 2: Preparation of (S)-4-ethyl-8-fluoro-4-hydroxy-9-methoxy-11-propyl-1,12-dihydro-14H-pyrano[3',4':6,7]indolizino[1,2-b]quinolin-3,14(4H)-dione **(PY-21-Ad)**

(S)-4-ethyl-4-hydroxy-7,8-dihydro-1H-pyran[3,4-f]indolizino-3,6,10(4H)-trione (1 eq, 62.31 mg, 236.70 μmol) and PPTS (1 eq, 59.48 mg, 236.70 μmol, 10 mL) were separately added to a solution of 1-(2-amino-4-fluoro-5-methoxyphenyl)but-1-one (PY-21-Ab) (1 eq, 62.31 mg, 236.70 μmol) in 10 mL of toluene,, and the reaction solution was stirred at 130°C for 16 hours in a nitrogen atmosphere. The reaction solution was concentrated under reduced pressure, and the residue was purified by silica gel column chromatography (eluents: petroleum ether: ethyl acetate = 5:1), to obtain a yellow solid compound PY-21-Ad (80 mg, yield: 77%).
LCMS (ESI): m/z, 439.1 $[M+H]^+$.

Step 3: Preparation of (S)-4-ethyl-8-fluoro-4,9-dihydroxy-11-propyl-1,12-dihydro-14H-pyrano[3',4':6,7]indolizino[1,2-b] quinolin-3,14(4H)-dione **(PY-21-A)**

$AlCl_3$ (18.25 mg, 136.84 μmol, 6 eq) was added to a solution of compound **PY-21-Ad** (10 mg, 22.81 μmol, 1 eq) in 4 mL of DCM. The reaction solution was stirred under a reaction condition of 70°C for 16 hours. 100 mL of saturated ammonium chloride solution was used to quench the reaction, then 2 × 100 mL of ethyl acetate was used to extract the organic phase, the organic phase was washed with saturated brine, dried with anhydrous sodium sulfate, filtered, and concentrated under reduced pressure, and the residue was purified by preparative high performance liquid chromatograph (preparative chromatograph manufacturer: SHIMADZU, model: LC-20AP, column: YMC-Triart Prep C18 250 × 30 mm × 10 μm, mobile phase: water (0.225% HCOOH)-acetonitrile, an elution ratio of water: 12% to 42%) and lyophilized, to obtain a white solid compound **PY-21-A** (4.3 mg, yield: 44%).

LCMS (ESI): m/z, 425.2 $[M+H]^+$.

$^1H$ NMR (400 MHz, DMSO-$d_6$) $\delta$ 10.90 (s, 1H), 7.91 (dd, $J$ = 11.9, 2.8 Hz, 1H), 7.64 - 7.57 (m, 1H), 7.26 (s, 1H), 6.49 (s, 1H), 5.43 (s, 2H), 5.27 (d, $J$ = 3.1 Hz, 2H), 3.06 (t, $J$ = 7.9 Hz, 2H), 1.86 (dq, $J$ = 14.1, 7.0 Hz, 2H), 1.73 (p, $J$ = 7.5 Hz, 2H), 1.05 (t, $J$ = 7.3 Hz, 3H), 0.88 (t, $J$ = 7.3 Hz, 3H).

Preparation Example 29: Preparation of (S)-4-(4-aminobutyl)-9-ethyl-5-fluoro-9-hydroxy-1,2,3,9,12,15-hexahydro-10H,13H-benzo[depyrano[3',4':6,7]indolizino[1,2-b]quinolin-10,13-dione **(PY-24)**

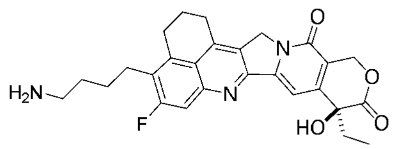

**PY-24**

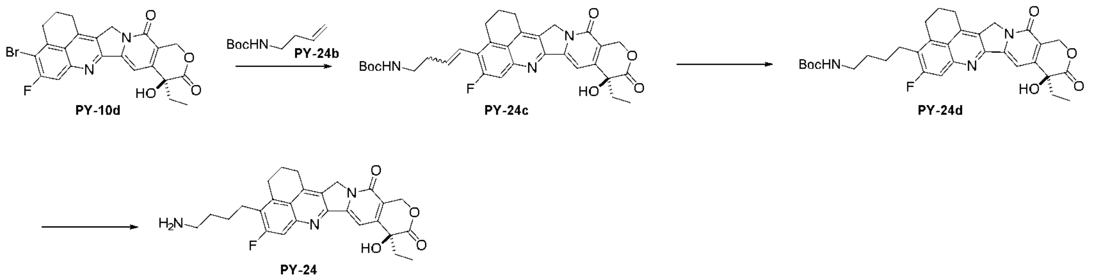

PY-10d

PY-24b

PY-24c

PY-24d

PY-24

Step 1: Preparation of (S)-(4-(9-ethyl-5-fluoro-9-hydroxy-10,13-dioxo-2,3,9,10,13,15-hexahydro-1H,12H-benzo[de]pyrano[3',4':6,7]indolizino[1,2-b]quinolin-4-yl)but-3-en-1-yl)tert-butyl carbamate **(PY-24c)**

0.1 equivalents of bis(tri-tert-butylphosphine)palladium (21 mg), 0.2 equivalents of tris(o-methylphenyl)phosphorus (25 mg), 6 equivalents of DIPEA (410 $\mu$L), and 2.5 equivalents of tert-butyl but-3-en-1-ylcarbamate (176 mg) were separately added to a solution of compound (S)-4-bromo-9-ethyl-5-fluoro-9-hydroxy-1,2,3,9,12,15-hexahydro-10H,13H-benzo[de]pyrano[3',4':6,7]indolizino[1,2-b]quinolin-10,13-dione (200 mg) in 15 mL of toluene, and the reaction solution was stirred at 120°C for 16 hours. The reaction solution was concentrated, then purified by silica gel column chromatography (eluents: dichloromethane/methanol = 20:1-10:1), and concentrated under reduced pressure, to obtain 129 mg of a crude product (yield: 54%), which was directly used for the next reaction.

LCMS (ESI): m/z, 576.3 $[M+1]^+$.

Step 2: Preparation of (S)-(4-(9-ethyl-5-fluoro-9-hydroxy-10,13-dioxo-2,3,9,10,13,15-hexahydro-1H,12H-benzo[de]pyrano[3',4':6,7]indolizino[1,2-b]quinolin-4-yl)butyl)tert-butyl carbamate (PY-24d)

0.25 equivalents of Pd/C (1.3 mg) and 0.25 equivalents of $Pd(OH)_2$ (1.7 mg) were separately added to a solution of 6.8 mg of crude product (S)-(4-(9-ethyl-5-fluoro-9-hydroxy-10,13-dioxy-2,3,9,10,13,15-hexahydro-1H,12H-benzo[de]pyrano[3',4':6,7]indolizino[1,2-b]quinolin-4-yl)but-3-en-1-yl)tert-butyl carbamate in 3 mL of methanol. The reaction solution was stirred at 25°C for 16 hours in a hydrogen atmosphere, and after filtration, the reaction solution was concentrated under reduced pressure, to obtain 19.6 mg of crude product of compound (S)-(4-(9-ethyl-5-fluoro-9-hydroxy-10,13-dioxo-2,3,9,10,13,15-hexahydro-1H,12H-benzo[de]pyrano[3',4':6,7]indolizino[1,2-b]quinolin-4-yl)butyl)tert-butyl carbamate (PY-24d) (yield: 55%, purity: 63%), which was directly used for the next reaction.

LCMS (ESI): m/z, 578.2 $[M+1]^+$.

Step 3: Preparation of (S)-4-(4-aminobutyl)-9-ethyl-5-fluoro-9-hydroxy-1,2,3,9,12,15-hexahydro-10H,13H-benzo[depyrano[3',4':6,7]indolizino[1,2-b]quinolin-10,13-dione **(PY-24)**

0.7 mL of TFA was slowly added to a solution of compound (S)-(4-(9-ethyl-5-fluoro-9-hydroxy-10,13-dioxo-2,3,9,10,13,15-hexahydro-1H,12H-benzo[de]pyrano[3',4':6,7]indolizino[1,2-b]quinolin-4-yl)butyl)tert-butyl carbamate (6.8 mg, purity: 63%) in 2.1 mL of dichloromethane under an ice water bath condition, and the reaction solution was naturally warmed to 25°C and stirred for 16 hours. The reaction solution was purified by preparative high performance

liquid chromatograph (preparative chromatograph manufacturer: SHIMADZU, model: LC-20AP, column: Welch Xtimate C18 250 × 50 mm × 10 μm, mobile phase: water (0.225% FA)-ACN, an elution ratio of water: 12% to 42%) and lyophilized, to obtain 0.35 mg of compound **PY-24** (yield: 5.4%).

LCMS (ESI): m/z, 473.2 $[M+H]^+$.

$^1H$ NMR (400 MHz, DMSO-d6) δ 1H NMR (400 MHz, DMSO-d6) δ 8.37 (s, 1H), 7.78-7.71 (m, 1H), 7.30 (s, 1H), 6.51 (s, 1H), 5.43 (s, 2H), 5.25 (s, 2H), 3.16 (d, J = 6.0 Hz, 6H), 2.86 (s, 2H), 2.76 (s, 2H), 2.08 (s, 2H), 1.88 (q, J = 7.0 Hz, 2H), 1.59 (s, 2H), 0.88 (t, J = 7.3 Hz, 3H); $^{19}F$ NMR (377 MHz, DMSO-$d^6$) δ -112.93.

Preparation Example 30: Preparation of 2-((9S)-9-ethyl-5-fluoro-9-hydroxy-4-methyl-10,13-dioxy-2,3,9,10,13,15-hexahydro-1H,12H-benzo[de]pyrano[3',4:6,7]indolizino[1,2-b]quinolin-1-yl)acetic acid **(PY-25)**

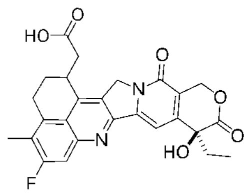

PY-25

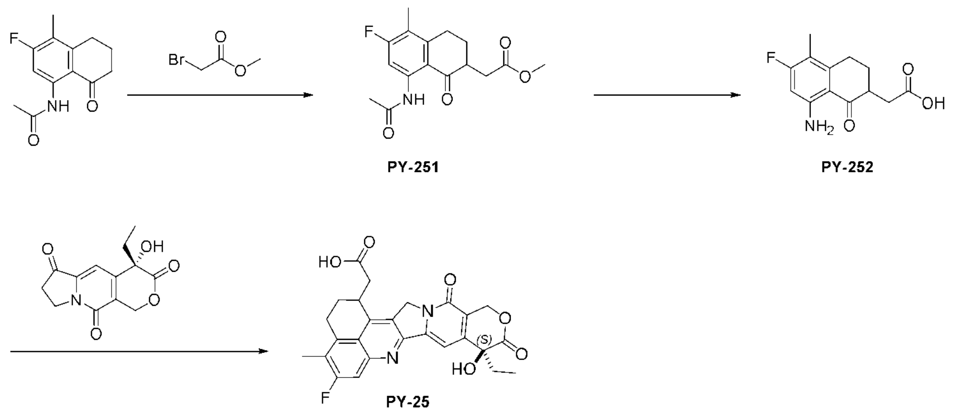

PY-251

PY-252

PY-25

Step 1: Preparation of 2-(8-acetamido-6-fluoro-5-methyl-1-oxo-1,2,3,4-tetrahydronaphthalene-2-yl)methyl acetate **(PY-251)**

N-(3-fluoro-4-methyl-8-oxo-5,6,7,8-tetrahydronaphthalene-1-yl)acetamide (2g, 8.5 mmol) was added into a 100 mL three-necked flask and dissolved with 30 mL of THF in a nitrogen atmosphere. The reaction solution was cooled to -78°C, LDA (10.6 mL, 2M THF) was slowly added to the foregoing reaction solution, and after addition, the reaction solution reacted at -78°C for 1 hour. Methyl bromoacetate (1.3g, 8.5 mmol) was then added to the reaction solution and the reaction solution was heated to room temperature and reacted for 16 hours. 150 mL of water was added to the reaction solution, the reaction solution was extracted with ethyl acetate (100 mL × 3), the organic phase was washed with saturated brine, dried with anhydrous sodium sulfate and filtered, the filtrate was concentrated under reduced pressure, and the residue was purified by silica gel column chromatography (80% PE/20% EA) to obtain 800 mg of yellowish solid compound 2-(8-acetamido-6-fluoro-5-methyl-1-oxo-1,2,3,4-tetrahydronaphthalene-2-yl)methyl acetate **(PY-251)** (yield: 30.6%).

Step 2: Preparation of 2-(8-amino-6-fluoro-5-methyl-1-oxo-1,2,3,4-tetrahydronaphthalene-2-yl)acetic acid **(PY-252)**

2 mL of aqueous solution of NaOH (2 mol/mL) was added to a solution of compound 2-(8-acetamido-6-fluoro-5-methyl-1-oxo-1,2,3,4-tetrahydronaphthalene-2-yl)methyl acetate **(PY-251)** (400 mg, 1.3 mol) in EtOH (8 mL) and the reaction solution reacted at 75°C for 16 hours. The reaction solution was adjusted with 2N hydrochloric acid to adjust pH to 7-8, concentrated under reduced pressure, and extracted with ethyl acetate (30 mL × 3), the organic phase was washed with saturated brine, dried with anhydrous sodium sulfate, and filtered, and the filtrate was concentrated under reduced pressure to obtain 200 mg of crude product, which was directly used for the next step.

Step 3: Preparation of 2-((9S)-9-ethyl-5-fluoro-9-hydroxy-4-methyl-10,13-dioxy-2,3,9,10,13,15-hexahydro-1H,12H-benzo[de]pyrano[3',4:6,7]indolizino[1,2-b]quinolin-1-yl)acetic acid **(PY-25)**

2-(8-amino-6-fluoro-5-methyl-1-oxo-1,2,3,4-tetrahydronaphthalene-2-yl)acetic acid **(PY-252)** (200 mg, 0.796 mmol) and (S)-4-ethyl-4-hydroxy-7,8-dihydro-1H-pyrano[3,4-f]indolizino-3,6,10(4H)-trione (251 mg, 0.954 mmol) were added into a 50 mL three-necked flask, toluene (10 mL, 50v) was added, then p-toluenesulfonic acid (27.3 mg, 0.159 mmol) and o-cresol (0.6 mL, 3v) were added, and a reaction solution reacted at 120°C-125°C for 16 hours. The reaction solution was concentrated under reduced pressure, and the residue was purified by preparative high performance liquid chromatograph (Oriendo, BRIX-2860; column: Phenomenex Luna C18 250 × 50 mm × 10 μm; mobile phase: water (0.225% HCOOH)-acetonitrile, eluted from 45% to 85%), to obtain 70 mg of compound **PY-25** as yellow powder, with yield of 18.38%.

$^1$H NMR (400 MHz, DMSO) δ 12.50 (s, 1H), 7.76 (d, *J* = 11.1 Hz, 1H), 7.31 (s, 1H), 6.51 (s, 1H), 5.44 (s, 2H), 5.36 (s, 2H), 3.83 - 3.74 (m, 1H), 3.19-3.05 (m, 2H), 2.70-2.64 (m, 1H), 2.58-2.53 (m, 1H), 2.38 (s, 3H), 2.23 (d, *J* = 13.6 Hz, 1H), 2.06-1.95 (m, 1H), 1.87 (tt, *J* = 14.1, 7.0 Hz, 2H), 0.87 (t, *J* = 7.3 Hz, 3H).

Preparation Example 31: Preparation of 2-((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)-N-(2-hydroxyethyl)acetamide and 2-((1R,9S)-9-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)-N-(2-hydroxyethyl)acetamide **(PY-25A and PY-25B)**

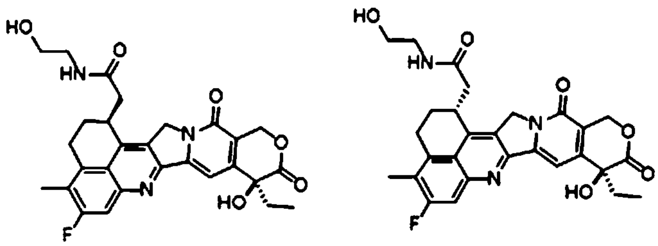

PY-25A and PY-25B

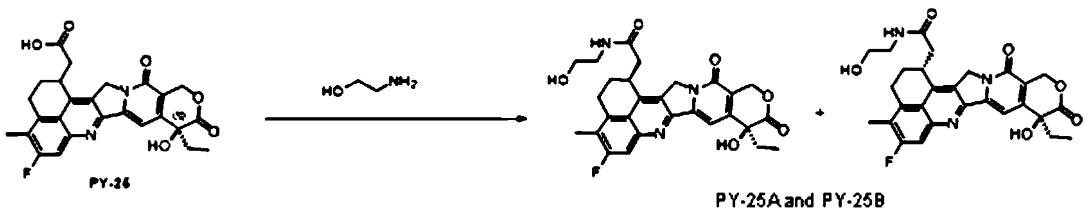

PY-25

PY-25A and PY-25B

HATU (17 mg, 45.14 μmol, 1.2 eq), DIEPA (20 mg, 150.5 μmol, 4 eq), and ethanolamine (2.3 mg, 37.62 μmol, 1.0 eq) were added to the solution of compound 2-((9S)-9-ethyl-5-fluoro-9-hydroxy-4-methyl-10,13-dioxy-2,3,9,10,13,15-hexahydro-1H,12H-benzo[de]pyrano[3',4]:6,7]indolizino[1,2-b]quinolin-1-yl)acetic acid **(PY-25)** (18 mg, 37.62 μmol, 1.0 eq) in DMF (3 mL) under an ice water bath, and after addition, the reaction solution was warmed to room temperature and stirred for 2 hours. The reaction solution was concentrated under reduced pressure, and the residue was isolated and purified by preparative high performance liquid chromatograph (Oriendo, BRIX-2860; column: Phenomenex Luna C18 250 × 50 mm × 10 μm; mobile phase: water (0.225% HCOOH)-acetonitrile, eluted from 45% to 85%), to obtain compound PY-25A (4.01 mg, yield: 18%) and PY-25B (6.62 mg, yield: 30%) as white solids.

**PY-25A** (LCMS retention time: 1.73 min):

LCMS (ESI): m/z, 522.3[M+1]$^+$.

$^1$H NMR (400 MHz, DMSO-*d*$^6$) δ 8.07 (t, *J* = 4.0 Hz, 1H), 7.76 (d, *J* = 12.0 Hz, 1H), 7.30 (s, 1H), 6.51 (s, 1H), 5.44 (s, 2H), 5.34 (s, 2H), 4.67 (t, *J* = 4.0 Hz, 1H), 3.78-3.77 (m, 1H), 3.44-3.36 (m, 3H), 3.18-3.14 (m, 4H), 2.45-2.44 (m, 1H), 2.39 (s, 3H), 2.13-2.11 (m, 1H), 1.97-1.80 (m, 3H), 0.87 (t, *J* = 8.0 Hz, 3H).

**PY-25B** (LCMS retention time: 1.83 min):

LCMS (ESI): m/z, 522.2[M+1]$^+$.

$^1$H NMR (400 MHz, DMSO-*d*$^6$) δ 8.46 (brs, 0.36H, HCOOH), 8.08-8.06 (m, 1H), 7.75 (d, *J* = 12.0 Hz, 1H), 7.30 (s, 1H), 6.51 (s, 1H), 5.44 (s, 2H), 5.34 (s, 2H), 4.66 (s, 1H), 3.78-3.76 (m, 1H), 3.38-3.36 (m, 2H), 3.15-3.12 (m, , 4H), 2.48 - 2.30 (m, 5H), 2.14 (s, 1H), 1.88-1.85 (m, 3H), 0.87 (t, *J* = 8.0 Hz, 3H).

Preparation Example 32: Preparation of (S)-N-((9-ethyl-5-fluoro-9-hydroxy-10,13-dioxo-2,3,9,10,13,15-hexahydro-1H,12H-benzo[de]pyrano[3',4':6,7]indolizino[1,2-b]quinolin-4-yl)methyl)-2-hydroxyacetamide **(PY-26)**

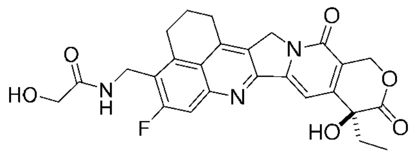

**PY-26**

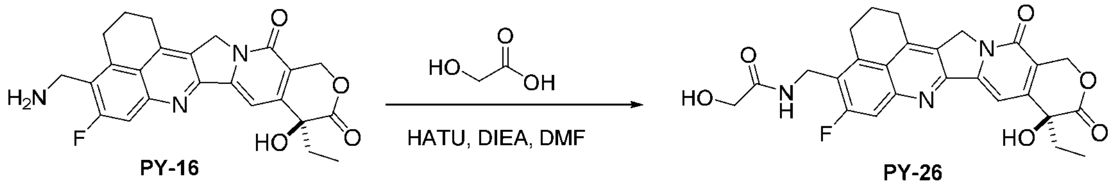


**PY-16** **PY-26**

N,N-diisopropylethylamine (26.71 mg, 206.68 μmol, 3 eq) and HATU (31.43 mg, 82.67 μmol, 1.2 eq) were added to a solution of compound **PY-16** (30 mg, 68.89 μmol, 1 eq) and 2-hydroxyacetic acid (6.29 mg, 82.67 μmol, 1.2 eq) in N,N-dimethylformamide (3 mL) at 0°C. After addition, the reaction solution reacted for 2 hours at 25°C. The reaction solution was purified by preparative high performance liquid chromatograph (preparative chromatograph manufacturer: ISCO, model: ISCO-R1, column: YMC-Triart Prep C18 250 × 50 mm × 7 μm, mobile phase: water (0.225% FA)-acetonitrile, an elution ratio of acetonitrile: 28% to 37%), to obtain a white solid compound **PY-26** (4.37 mg, yield = 12.8%).

LCMS (ESI): m/z, 494.2[M+H]$^+$.

$^1$H NMR (400 MHz, DMSO-$d_6$) δ 8.06-8.05 (m, 1H), 7.74 (d, *J* = 12 Hz, 1H), 7.31 (s, 1H), 6.54 (s, 1H), 5.43-5.42 (m, 3H), 5.25 (s, 2H), 4.56 (d, *J* = 8.0 Hz, 2H), 3.82 (d, *J* = 8.0 Hz, 2H), 3.26-3.23 (m, 2H), 3.15-3.15 (m, 2H), 2.08-2.05 (m, 2H), 1.90-1.83 (m, 2H), 0.87 (t, *J* = 7.2 Hz, 3H). $^{19}$F NMR (377 MHz, DMSO-$d_6$) δ -112.11.

Preparation Example 33: Preparation of (S)-(9-ethyl-5-fluoro-9-hydroxy-10,13-dioxo-2,3,9,10,13,15-hexahydro-1H,12H-benzo[de]pyrano[3',4':6,7]indolizino[1,2-b]quinolin-4-yl)methyl(2-hydroxyethyl)carbamate **(PY-27)**

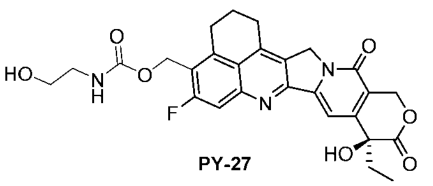

**PY-27**

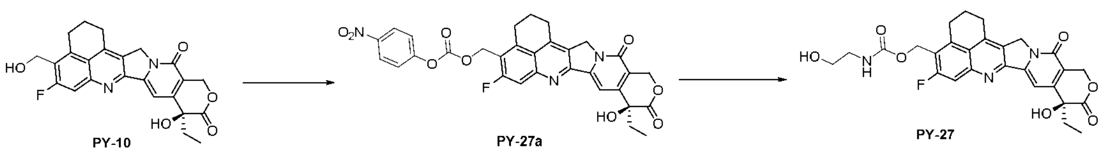

PY-10 PY-27a PY-27

Step 1: Preparation of (S)-(9-ethyl-5-fluoro-9-hydroxy-10,13-dioxo-2,3,9,10,13,15-hexahydro-1H,12H-benzo[de]pyrano[3',4':6,7]indolizino[1,2-b]quinolin-4-yl)methyl(4-nitrophenyl)carbonate **(PY -27a)**

The compound **PY-10** (8.0 mg, 0.018 mmol, 1.0 eq), bis(4-nitrophenyl)carbonate (44.6 mg, 0.147 mmol, 8.0 eq), and N,N-diisopropylethylamine (28.4 mg, 0.220 mmol, 12.0 eq) were added to N,N-dimethylformamide (2 mL) at room temperature and the reaction solution was stirred at 50°C for 16 hours. The reaction solution was concentrated under reduced pressure and the residue was purified by thin layer chromatography by using a developing agent system of dichloromethane/methanol (15/1), to obtain a yellow oily compound **PY -27a** (18 mg).

LCMS (ESI): m/z, 602.3 [M+H]$^+$.

Step 2: Preparation of (S)-(9-ethyl-5-fluoro-9-hydroxy-10,13-dioxo-2,3,9,10,13,15-hexahydro-1H,12H-benzo[de]pyrano[3',4':6,7]indolizino[1,2-b]quinolin-4-yl)methyl(2-hydroxyethyl)carbamate **(PY-27)**

The compound **PY-27a** (18.0 mg, impure), aminoethanol (1.83 mg, 0.030 mmol), and N,N-diisopropylethylamine (11.6 mg, 0.090 mmol) were added to N,N-dimethylformamide (1 mL) at room temperature and the reaction solution stirred at 25°C for 0.5 hours. The reaction solution was purified by high performance liquid chromatograph (preparative chromatograph manufacturer: Luna, model: Lab311-ISCO-R4, Phenomenex Luna C18 (250×50 mm×10 μm) was used as a chromatographic column, mobile phase: water (0.225% HCOOH)-acetonitrile, an elution ratio of water: 12% to 42%) and lyophilized, to obtain a white solid product **PY-27** (4.14 mg, 7.56 umol, two-step yield: 42%).

LCMS (ESI): m/z, 524.2 $[M+H]^+$.

$^1H$ NMR (400 MHz, DMSO-$d_6$) δ 7.79 (d, *J* = 11.2 Hz, 1H), 7.32 (s, 1H), 7.19 (t, *J* = 5.6 Hz, 1H), 6.54 (s, 1H), 5.44 (s, 2H), 5.27 (d, *J* = 4.4 Hz, 3H), 4.63 (t, *J* = 5.6 Hz, 1H), 3.30-3.23 (m, 5H), 3.18 (s, 2H), 3.04 (q, *J* = 6.0 Hz, 2H), 2.08 (s, 2H), 1.86 (dt, *J* = 15.2, 7.2 Hz, 2H), 0.87 (t, *J* = 7.2 Hz, 3H).

Preparation Example 34: Preparation of (S)-4-ethyl-8,10-difluoro-4,9-dihydroxy-11-(4-hydroxybutyl)-1,12-dihydro-14H-pyrano[3',4':6,7]indolizino[1,2-b]quinolin-3,14(4H)-dione **(PY-29)** and (S)-4-ethyl-8,10-difluoro-4-hydroxy-11-(4-hydroxybutyl)-9-methoxy-1,12-dihydro-14H-pyrano[3',4':6,7]indolizino[1,2-b]quinolin-3,14(4H)-dione **(PY-29B)**

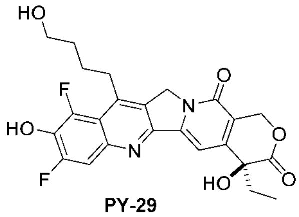

**PY-29**

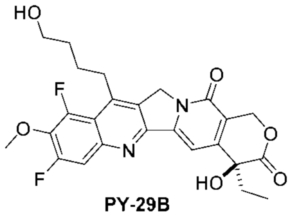

**PY-29B**

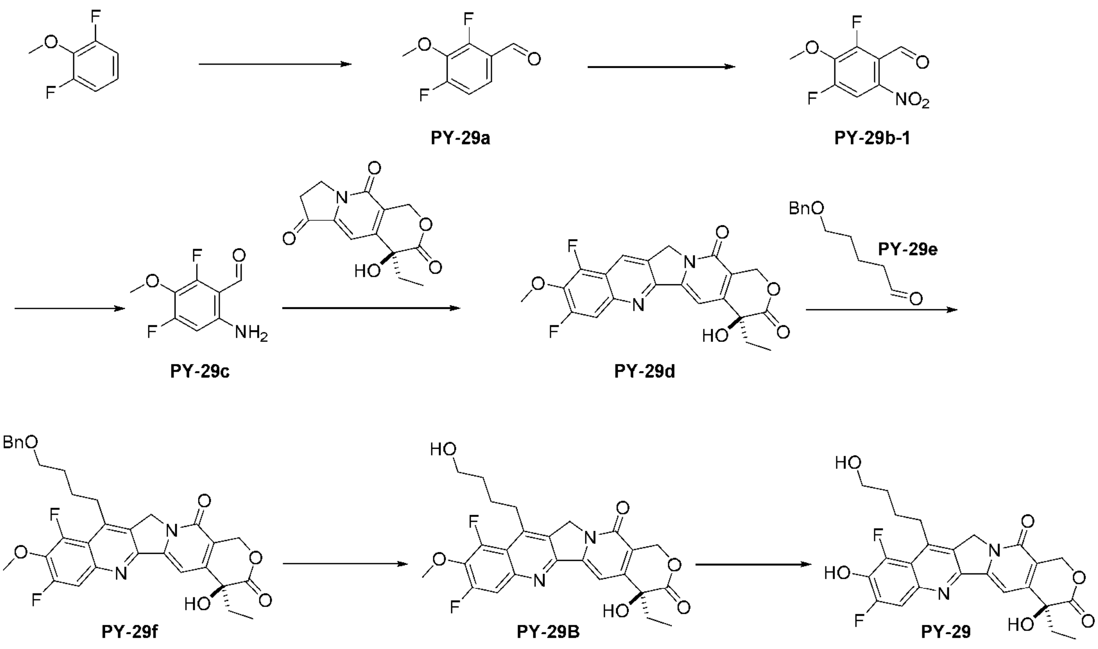

**PY-29a** **PY-29b-1** **PY-29c** **PY-29d** **PY-29e** **PY-29f** **PY-29B** **PY-29**

Step 1: Preparation of 2,4-difluoro-3-methoxybenzaldehyde **(PY -29a)**

1.2 equivalents of n-BuLi (9.3 mL, 2.5M) was added to a solution of 1,3-difluoro-2-methoxybenzene (2.8g) in 20 mL of THF at -78°C, the reaction solution was stirred at the temperature for half an hour, then 6 equivalents of DMF (9.15 mL) was added, the reaction solution was further stirred for half an hour, the low-temperature reaction bath was removed, and the reaction solution reacted at 15°C for 1 hour. The reaction was quenched with 4M HCl (6 mL), the reaction solution was extracted with ethyl acetate (3 × 50 mL), the organic layer was concentrated under reduced pressure, and the residue was purified by silica gel column chromatography (eluents: petroleum ether/ethyl acetate = 20:1-10:1), to obtain 1.8g of

yellow oily compound **PY-29a** (yield: 53%).

LCMS (ESI): m/z, 173 $[M+1]^+$.

$^1$H NMR (400 MHz, DMSO-$d_6$) δ 10.96-9.56 (m, 1H), 7.83-7.43 (m, 1H), 7.30 (b, 1H), 4.22-3.90 (m, 3H).

Step 2: Preparation of 2,4-difluoro-3-methoxy-6-nitrobenzaldehyde **(PY-29b-1)**

0.96 equivalents of fuming nitric acid (446 μL) was added to a solution of compound 2,4-difluoro-3-methoxybenzaldehyde (1.78g) in 20.3 mL of sulfuric acid and the reaction solution reacted at 25°C for 1 hour. The reaction solution was added into ice water, subjected to suction filtration under pressure, washed with water and dried, to obtain 925 mg of yellow solid compound 2,4-difluoro-3-methoxy-6-nitrobenzaldehyde **(PY-29b-1)** (yield: 40%).

$^1$H NMR (400 MHz, DMSO-$d_6$) δ 10.14 (s, 1H), 8.21 (dd, *J* = 11.1, 2.0 Hz, 1H), 4.13 (t, *J*= 1.9 Hz, 3H).

Step 3: Preparation of 6-amino-2,4-difluoro-3-methoxybenzaldehyde **(PY- 29c)**

Iron powder (5.5 eq, 1.31g), water (23.12 eq, 1.77 mL) and hydrochloric acid (0.55 eq, 71 μL) were separately added to a solution of compound 2,4-difluoro-3-methoxy-6-nitrobenzaldehyde (925 mg) in 30 mL of ethanol, and the reaction solution was stirred at 80°C for 16 hours. LCMS showed that a product was generated, the reaction solution was cooled to room temperature, then filtered under pressure, and concentrated to obtain a crude product, and the crude product was purified by silica gel column chromatography (eluents: petroleum ether/ethyl acetate = 10:1-5:1), and dried to obtain 488 mg of green solid 6-amino-2,4-difluoro-3-methoxybenzaldehyde **(PY-29d)** (yield: 61%).

LCMS (ESI): m/z, 187.9 $[M+H]^+$.

$^1$H NMR (400 MHz, DMSO-$d_6$) δ 10.04 (s, 1H), 7.48 (s, 2H), 6.44 (dd, *J* = 13.4, 2.0 Hz, 1H), 3.77 (s, 3H).

Step 4: Preparation of (S)-4-ethyl-8,10-difluoro-4-hydroxy-9-methoxy-1,12-dihydro-14H-pyrano[3',4':6,7]indolizino[1,2-b]quinolin-3,14(4H)-dione **(PY-29d)**

0.67 equivalents of PPTS (134.9 mg) and 1.2 equivalents of compound **PY-8f** (253.2 mg) were separately added to a solution of compound 6-amino-2,4-difluoro-3-methoxybenzaldehyde **(PY-29c)** (150 mg) in 10 mL of toluene, and the reaction solution was stirred at 130°C for 16 hours in a nitrogen atmosphere. The reaction solution was concentrated under reduced pressure, and the residue was purified by silica gel column chromatography (eluents: petroleum ether/ethyl acetate = 5:1-0:1) to obtain 233 mg of yellow solid compound **PY-29d** (yield: 67%; purity: 78%).

LCMS (ESI): m/z, 415 $[M+H]^+$.

Step 5: Preparation of (S)-11-(4-(benzyloxy)butyl)-4-ethyl-8,10-difluoro-4-hydroxy-9-methoxy-1,12-dihydro-14H-pyrano[3',4':6,7]indolizino[1,2-b]quinolin-3,14(4H)-dione) **(PY-29f)**

The compound **PY-29d** (193 mg, purity: 78%) was dissolved in 14.6 mL of glacial acetic acid solvent, and 3.6 mL of concentrated sulfuric acid was slowly added under an ice water bath condition. 1.2 equivalents of ferrous sulfate (66.2 mg) and 2.6 mL of deionized water were added to another reaction flask, the ferrous sulfate solution was added to the solution of the foregoing compound **PY-29d** at the same temperature, then 5 equivalents of 5-(benzyloxy)valeraldehyde **(PY-29e)** (349 mg) and 54.4 μL of hydrogen peroxide were added sequentially, and the reaction solution was stirred for 45 minutes under the ice water bath condition. The reaction solution was added into ice water, the organic phase was extracted with ethyl acetate (50 mL × 3), washed with saturated brine, dried with anhydrous sodium sulfate, filtered, and concentrated under reduced pressure to obtain a crude product, and the crude product was purified by silica gel column chromatography (eluents: dichloromethane/methanol = 20:1-4:1) to obtain 230 mg of compound **PY-29f** (purity: 64%).

LCMS (ESI): m/z, 577.3 $[M+H]^+$.

Step 6: Preparation of (S)-4-ethyl-8,10-difluoro-4-hydroxy-11-(4-hydroxybutyl)-9-methoxy-1,12-dihydro-14H-pyrano [3',4':6,7]indolizino[1,2-b]quinolin-3,14(4H)-dione **(PY-29B)**

0.25 equivalents of palladium on carbon (78 mg) and 0.25 equivalents of $Pd(OH)_2$ (51.4 mg) were separately added to a solution of compound **PY-29f** (497 mg) in 10 ml of methanol, and the reaction solution was stirred at 30°C for 16 hours. The reaction solution was filtered and then concentrated under reduced pressure, and the residue was purified by silica gel column chromatography (eluents: dichloromethane/methanol = 20:1-10:1) to obtain 55 mg of compound **PY-29B** (yield: 38%; purity: 78%).

LCMS (ESI): m/z, 487.1 $[M+H]^+$.

Step 7: Preparation of (S)-4-ethyl-8,10-difluoro-4,9-dihydroxy-11-(4-hydroxybutyl)-1,12-dihydro-14H-pyrano[3',4':6,7]indolizino[1,2-b]quinolin-3,14(4H)-dione **(PY-29)**

6 equivalents of compound $AlCl_3$ (79 mg) were added in batches to a solution of compound **PY-29B** (48 mg, purity: 78%) in 4 mL of DCM at intervals of 2 hours at 90°C, and the reaction solution was purified by preparative high performance liquid chromatograph (preparative chromatograph manufacturer: SHIMADZU, model: LC-20AP, column: YMC-Triart Prep C18 250 × 30 mm × 10 μm, mobile phase: water (0.225% HCOOH)-acetonitrile, an elution ratio of water: 12% to 42%) and lyophilized, to obtain 6.77 mg of white solid compound **PY-29** (yield: 14.5%).
LCMS (ESI): m/z, 473.2 $[M+H]^+$.
$^1H$ NMR (400 MHz, DMSO-$d_6$) δ 8.35 (d, *J* = 6.8 Hz, 1H), 7.89 (d, *J*= 7.5 Hz, 2H), 7.76 (d, *J* = 9.0 Hz, 1H), 7.71 (d, *J* = 7.5 Hz, 2H), 7.55 (t, *J* = 6.2 Hz, 1H), 7.42 (t, *J* = 7.5 Hz, 2H), 7.33 (t, *J* = 7.5 Hz, 2H), 4.33 - 4.20 (m, 4H), 4.11 (p, *J* = 7.0 Hz, 1H), 3.67 (d, *J* = 6.2 Hz, 2H), 1.95 (dt, *J* = 12.6, 6.3 Hz, 1H), 1.38 (d, *J* = 1.9 Hz, 9H), 1.24 (d, *J* = 7.2 Hz, 3H), 0.86 (dd, *J* = 19.2, 6.7 Hz, 6H); $^{19}F$ NMR (377 MHz, DMSO-$d_6$) δ -125.50 (d, *J* = 13.5 Hz).

Preparation Example 35: Preparation of (S)-4-ethyl-8,10-difluoro-4,9-dihydroxy-1,12-dihydro-14H-pyrano[3',4':6,7]indolizino[1,2-b]quinolin-3,14(4H)-dione **(PY-29A)**

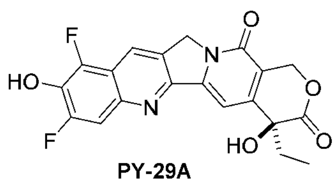

**PY-29A**

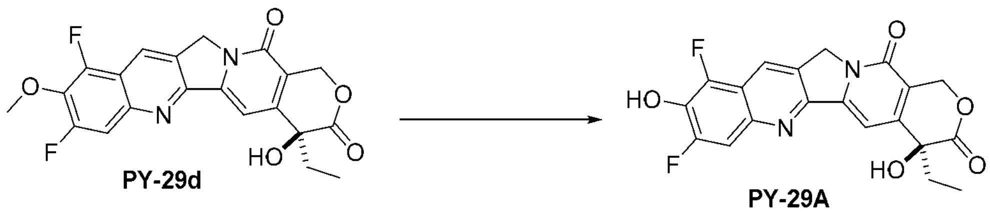

**PY-29d**

**PY-29A**

Step 1: Preparation of (S)-4-ethyl-8,10-difluoro-4,9-dihydroxy-1,12-dihydro-14H-pyrano[3',4':6,7]indolizino[1,2-b]quinolin-3,14(4H)-dione **(PY-29A)**

$AlCl_3$ (22.2 mg, 166.52 μmol, 3 eq) was added to a solution of compound PY-29d (29.5 mg, 55.51 μmol, 1 eq, purity: 78%) in 3 mL of DCM. The reaction solution reacted at 70°C for 2 hours. The reactant was purified by preparative high performance liquid chromatograph (preparative chromatograph manufacturer: SHIMADZU, model: LC-20AP, column: YMC-Triart Prep C18 250 × 30 mm × 10 μm, mobile phase: water (0.225% HCOOH)-acetonitrile, an elution ratio of water: 12% to 42%) and lyophilized, to obtain a white solid compound PY-29A (1.46 mg, yield: 4.9%).
LCMS (ESI): m/z, 401.1 $[M+H]^+$.
$^1H$ NMR (400 MHz, DMSO-$d_6$) δ 8.54 (s, 1H), 7.77 (d, *J* = 11.9 Hz, 1H), 7.25 (s, 1H), 6.52 (s, 1H), 5.42 (s, 2H), 5.23 (s, 2H), 1.86 (p, *J* = 7.0 Hz, 2H), 0.87 (t, *J* = 7.3 Hz, 3H).

Preparation Example 36: Preparation of (S)-4-(aminomethyl) 5-chloro-9-ethyl-9-hydroxy-1,2,3,9,12,15-hexahydro-10h,13h-benzo[de]pyrano[3',4':6,7]indolizino[1,2-b]quinolin-10,13-dione **(PY-36)**

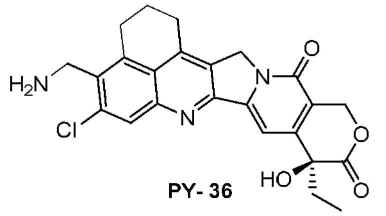

PY- 36

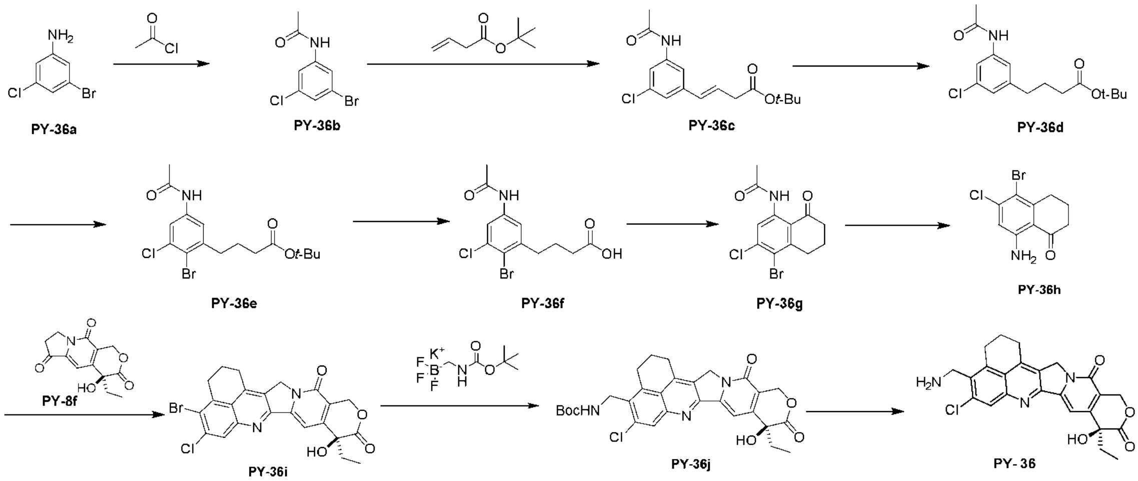

PY-36a PY-36b PY-36c PY-36d PY-36e PY-36f PY-36g PY-36h PY-8f PY-36i PY-36j PY- 36

Step 1: Preparation of N-(3-bromo-5-chlorophenyl)acetamide **(PY-36b)**

3-bromo-5-chloro-aniline **(PY-36a)** (5.00g, 24.2 mmol, 1.0 eq) and triethylamine (4.90g, 48.4 mmol, 6.74 mL, 2.0 eq) were added to dichloromethane (50.0 mL), nitrogen displacement was performed three times, acetyl chloride (2.85g, 36.3 mmol, 2.58 mL, 1.5 eq) was added dropwise at 0°C, and then the reaction solution was stirred at 25°C for 2 hours in a nitrogen atmosphere. 30.0 mL of water was added dropwise to the reaction solution at 0°C, the precipitate precipitated and was filtered, and the filtrate was concentrated under reduced pressure to obtain the compound N-(3-bromo-5-chlorophenyl)acetamide (5.90g, yield: 97%) as a white solid.

$^1$H NMR (400 MHz, DMSO-d$^6$) δ 10.26 (s, 1H), 7.77 (d, J = 1.2 Hz, 1H), 7.68 (d, J = 1.2 Hz, 1H), 7.36 (d, J = 2.0 Hz, 1H), 2.05 (s, 3H).

Step 2: Preparation of tert-butyl (E)-4-(3-acetamido-5-chlorophenyl)but-3-enoate **(PY-36c)**

N-(3-bromo-5-chlorophenyl)acetamide (5.00g, 20.1 mmol, 1.0 eq) was dissolved in N,N dimethylformamide (30 mL), and tert-butyl 3-butenoate (4.29g, 30.2 mmol, 4.89 mL, 1.5 eq), tris(o-methylphenyl)phosphorus (306.2 mg, 1.01 mmol, 0.05 eq), triethylamine (4.07g, 40.2 mmol, 5.60 mL, 2.0 eq), bis(tri-tert-butylphosphine)palladium (103 mg, 201.2 μmol, 0.01 eq), and N-methyldicyclohexylamine (7.86g, 40.2 mmol, 8.54 mL, 2.0 eq) were added. The reaction solution reacted at 100°C for 4 hours in a nitrogen atmosphere. The reaction solution was cooled to room temperature, water (50.0 mL) was added, and then the reaction solution was extracted with ethyl acetate twice (25 mL). The organic phase was washed three times with saturated brine (30 mL), then dried with anhydrous sodium sulfate, and filtered, and the filtrate was concentrated to obtain a crude product. The crude product was purified by silica gel column chromatography and eluted with petroleum ether/ethyl acetate = 15/1 - 3/2, to obtain tert-butyl (E)-4-(3-acetamido-5-chlorophenyl)but-3-enoate (5.10g, 16.5 mmol, yield: 82.09 %) as a white solid.

LCMS: RT = 0.634 min, MS (ESI) m/z = 254.0 [M+H]$^+$.

Step 3: Preparation of tert-butyl 4-(3-acetamido-5-chlorophenyl)butyrate **(PY-36d)**

Tert-butyl (E)-4-(3-acetamido-5-chlorophenyl)but-3-enoate (5.00g, 16.1 mmol, 1.0 eq) was added to methanol (50.0 mL), tris(triphenylphosphine)chlororhodium(I) (1.49g, 1.61 mmol, 0.1 eq) was added in an argon atmosphere, and then the reaction solution reacted at 25°C in a hydrogen (30 psi) condition for two hours. The reaction solution was filtered and the filtrate was concentrated, to obtain the crude product tert-butyl 4-(3-acetamido-5-chlorophenyl)butyrate (3.60g, 11.5 mmol, yield: 71.42%) as a white solid.

$^1$HNMR(400MHz, $CDCl_3$) δ ppm 7.41 (s, 1 H), 7.13-7.08 (m, 2 H), 6.85 (s, 1 H), 2.52 (t, J = 7.2 Hz, 2 H), 2.15 (t, J = 7.6 Hz, 1 H), 2.10 (s, 1 H), 1.83-1.81 (m, 2 H), 1.38 (s, 9 H).

Step 4: Preparation of tert-butyl 4-(5-acetamido-2-bromo-3-chlorophenyl)butyrate **(PY-36e)**

Tert-butyl 4-(3-acetamido-5-chlorophenyl)butyrate (100 mg, 320.7 μmol, 1.0 eq) was dissolved in N,N-dimethylformamide (5.00 mL), NBS (68.5 mg, 385 μmol, 1.2 eq) was slowly added and the reaction solution reacted at 25°C for 1 hour. Water (10.0 mL) was added dropwise to the reaction solution, then the reaction solution was extracted with ethyl acetate (10.0 mL) twice, the organic phase was washed with saturated brine (10.0 mL) three times, then dried with anhydrous sodium sulfate, and filtered, and the filtrate was concentrated to obtain the crude product tert-butyl 4-(5-acetamido-2-bromo-3-chlorophenyl)butyrate (120 mg, 96.0%) as a white solid.

LCMS: RT = 2.885 min, MS (ESI) m/z = 336.1 $[M+H]^+$.

Step 5: Preparation of 4-(5-acetamido-2-bromo-3-chlorophenyl)butyric acid **(PY-36f)**

Tert-butyl 4-(5-acetamido-2-bromo-3-chlorophenyl)butyrate (100 mg, 256 μmol, 1.0 eq) was dissolved in dichloromethane (2.00 mL), trifluoroacetic acid (2.00 mL) was slowly added dropwise at 0°C, and the reaction solution reacted at 25°C for 2 hours. The reaction solution was directly concentrated under reduced pressure to obtain a solid, and then dichloromethane (8.00 mL) was added, stirred and filtered to obtain the crude product 4-(5-acetamido-2-bromo-3-chlorophenyl)butyric acid (60.0 mg, 70.0%) as a white solid.

$^1$HNMR (400MHz, $CDCl_3$) δ ppm 12.09 (br, 1H), 10.19 (s, 1 H), 7.87 (d, J = 2.4 Hz, 1 H), 7.37 (d, J = 2.4 Hz, 1 H), 2.72 (t, J = 7.6 Hz, 2 H), 2.28 (t, J = 7.2 Hz, 2 H), 2.04 (s, 3 H), 1.78 (t, J = 7.6 Hz, 2 H).

Step 6: Preparation of N-(4-bromo-3-chloro-8-oxo-5,6,7,8-tetrahydronaphthalene-1-yl)acetamide **(PY-36g)**

4-(5-acetamido-2-bromo-3-chlorophenyl)butyric acid (1.80g, 5.38 mmol, 1.0 eq) was dissolved in Eaton's reagent (113.6g, 477.3 mmol, 75.00 mL, 88.73 eq) and the reaction solution reacted at 100°C for 1 hour in a nitrogen atmosphere. The reaction solution was cooled to room temperature, slowly added to cold water to fully quench the reaction, and filtered to obtain a solid. The solid was dissolved in dichloromethane (10.0 mL), then concentrated, pulped with mixed solvents (petroleum ether/ethyl acetate = 10/1), and filtered, to obtain N-(4-bromo-3-chloro-8-oxo-5,6,7,8-tetrahydronaphthalene-1-yl)acetamide (1.20g, 3.79 mmol, yield: 70.45%) as a yellow solid.

LCMS: RT = 0.961 min, MS (ESI) m/z = 317.9 $[M+H]^+$.

$^1$HNMR (400MHz, $CDCl_3$) δ ppm 12.11 (s, 1H), 8.86 (s, 1 H), 3.03 (t, J = 6.0 Hz, 2 H), 2.61 (t, J = 6.4 Hz, 2 H), 2.16 (s, 3 H), 2.04 (t, J = 6.4 Hz, 2 H).

Step 7: Preparation of 8-amino-5-bromo-6-fluoro-3,4-dihydronaphthalene-1(2H)-1-one **(PY-36h)**

N-(4-bromo-3-chloro-8-oxo-5,6,7,8-tetrahydronaphthalene-1-yl)acetamide (900 mg, 2.84 mmol, 1.0 eq) was dissolved in ethanol (15 mL), hydrochloric acid solution (5 mL, 6 mol/mL) was added at room temperature, and the reaction solution was stirred at 80°C for 3 hours. The reaction solution was cooled to room temperature and concentrated, the concentrate was adjusted with saturated sodium bicarbonate solution to adjust pH to 8-9, and then extracted with ethyl acetate (10 mL) twice, the organic phase was washed once with saturated brine, dried with anhydrous sodium sulfate, filtered, and concentrated to obtain the crude product 8-amino-5-bromo-6-fluoro-3,4-dihydronaphthalene-1(2H)-1-one (700 mg, 2.55 mmol, yield: 89.8%) as a yellow solid.

LCMS: RT = 0. 942 min, MS (ESI) m/z = 275.8 $[M+H]^+$.

Step 8: Preparation of (S)-4-bromo-5-chloro-9-ethyl-9-hydroxy-1,2,3,9,12,15-hexahydro-10h,13h-benzo[de]pyrano [3',4':6,7]indolizino[1,2-b]quinolin-10,13-dione **(PY-36i)**

8-amino-5-bromo-6-fluoro-3,4-dihydronaphthalene-1(2H)-1-one (574 mg, 2.09 mmol, 1.1 eq) and compound **PY-8f** (500 mg, 1.90 mmol, 1.0 eq) were dissolved in toluene (10 mL), p-toluenesulfonic acid (36.1 mg, 0.19 mmol, 0.1 eq) was added and the reaction solution reacted at 110°C for 16 hours in the nitrogen atmosphere. The reaction solution was cooled to room temperature, a solid precipitated, filtered, and then dried to obtain crude product **PY-36i** as a yellow solid (850 mg, 1.69 mmol, yield: 89.4%).

LCMS: RT = 2.233 min, MS (ESI) m/z = 503.3 $[M+H]^+$.

Step 9: Preparation of (S)-(5-fluoro-9-ethyl-9-hydroxy-10,13-dioxo-2,3,9,10,13,15-hexahydro-1h,12h-benzo[de]pyrano [3',4':6,7]indolizino[1,2-b]quinolin-4-yl)methyl)tert-butyl carbamate **(PY-36j)**

The compound **PY-36i** (400 mg, 0.80 mmol, 1.0 eq), potassium (N-Boc-aminomethyl)trifluoroborate (948 mg, 4.00 mmol, 5.0 eq), butyl di-1-adamantylphosphine (375 mg, 0.40 mmol, 0.5 eq), potassium carbonate (221 mg, 1.60

mmol, 2.0 eq) and palladium acetate (53.88 mg, 0.24 mmol, 0.3 eq) were dissolved in 1,4-dioxane (10 mL) and water (2 mL) and the reaction solution was stirred at 80°C for 2 hours in a nitrogen atmosphere. The reaction solution was cooled to room temperature and concentrated, water (10 mL) was added to the concentrate, then the concentrate was extracted with ethyl acetate (10 mL) twice, the organic phase was washed once with saturated brine, dried with anhydrous sodium sulfate, filtered, and concentrated to obtain a crude product, and the crude product was purified by reversed-phase chromatography (column: Xtimate C18 150 × 40 mm × 10 μm; mobile phase: [water (FA)-ACN]; gradient: 26%-66% B, 36 min), to obtain compound **PY-36j** (54.2 mg, 0.095 mmol, yield: 11.87%) as a white solid.

LCMS: RT = 2.127 min, MS (ESI) m/z = 552.5 $[M+H]^+$.

Step 10: Preparation of (S)-4-(aminomethyl) 5-chloro-9-ethyl-9-hydroxy-1,2,3,9,12,15-hexahydro-10h,13h-benzo[de]pyrano[3',4':6,7]indolizino[1,2-b]quinolin-10,13-dione **(PY-36)**

Compound **PY-36j** (52.4 mg, 0.095 mmol, 1.0 eq) was dissolved in dichloromethane (5 mL), trifluoroacetic acid (2 mL) was added at 0°C, and the reaction solution reacted at 25°C for 2 hours. The reaction solution was concentrated under reduced pressure, and the crude product was purified by reversed-phase chromatography (column: Xtimate C18 150 × 40 mm × 10 μm; mobile phase: [water (TFA)-ACN]; gradient: 0%-38% B, 30 min), to obtain compound **PY-36** (4.55 mg, 0.01 mmol, yield: 10.53%) as a white solid.

LCMS: RT = 1.566 min, MS (ESI) m/z = 452.2 $[M+H]^+$.

$^1$HNMR (400MHz, DMSO-$d^6$) δ ppm 8.23 (s, 1 H), 8.19 (s, 2 H), 7.34 (s, 1 H), 6.55 (s, 1 H), 5.45 (s, 2 H), 5.30 (s, 2 H), 4.41 (s, 2 H), 3.20-3.18 (m, 4 H), 2.12 (t, *J* = 4.8 Hz, 2 H), 1.90-1.86 (m, 2 H), 0.88 (t, *J* = 7.2 Hz, 3 H).

Preparation Example 37: Preparation of (S)-5-chloro-9-ethyl-9-hydroxy-4-(hydroxymethyl)-1,2,3,9,12,15-hexahydro-10h,13h-benzo[de]pyrano[3',4':6,7]indolizino[1,2-b]quinolin-10,13-dione **(PY-37)**

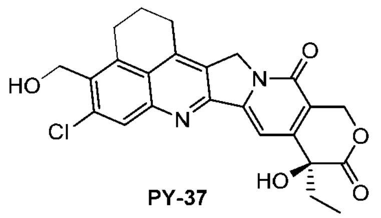

PY-37

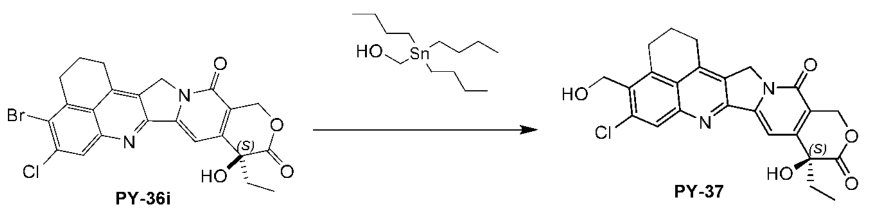

PY-36i

PY-37

The compound **PY-36i** (200 mg, 0.40 mmol, 1.0 eq) and (tributylstannyl)methanol (192 mg, 0.60 mmol, 1.5 eq) were dissolved in 1,4-dioxane (5 mL), and the catalyst chloro(2-dicyclohexylphosphino-2,4,6-triisoporpyl-1,1-biphenyl)[2-(2-amino-1,1-biphenyl)]palladium (II) (XPhos Pd G2) (31.4 mg, 0.04 mmol, 0.1 eq) were added, and the reaction solution reacted at 90°C for 4 hours in the nitrogen atmosphere. The reaction solution was cooled to room temperature and filtered, the filtrate was concentrated under reduced pressure, diluted with water, and then extracted twice with ethyl acetate (10 mL), the organic phase was washed with saturated brine (10 mL), dried with anhydrous sodium sulfate, filtered, and concentrated to obtain a crude product. The crude product was purified by reversed-phase chromatography (column: Welch Xtimate C18 150 × 30 mm × 5 μm; mobile phase: [water (FA)-ACN]; gradient: 6%-46% B, 25 min), to obtain compound **PY-37** (2.08 mg, 0.005 mmol, yield: 1.25%) as a white solid.

LCMS: RT = 1.475 min, MS (ESI) m/z = 453.3 $[M+H]^+$.

$^1$HNMR (400MHz, DMSO-$d^6$) δ ppm 8.10 (s, 1 H), 7.32 (s, 1 H), 6.53 (s, 1 H), 5.44 (s, 2 H), 5.28 (s, 2 H), 5.19 (t, *J* = 5.2 Hz, 1 H), 4.83 (d, *J* = 5.2 Hz , 2 H), 3.17 (t, *J* = 7.6 Hz , 2 H), 2.09 (t, *J* = 7.6 Hz, 2 H), 1.89-1.84 (m, 2 H), 1.15-1.14 (m, 2 H), 0.88 (t, *J* = 7.2 Hz, 3 H).

Preparation Example 38: Preparation of (S)-9-ethyl-9-hydroxy-4-(hydroxymethyl)-1,2,3,9,12,15-hexahydro-10h,13h-benzo[de]pyrano[3',4':6,7]indolizino[1,2-b]quinolin-10,13-dione (PY-37A)

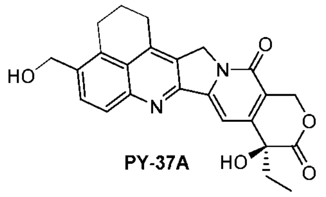

**PY-37A**

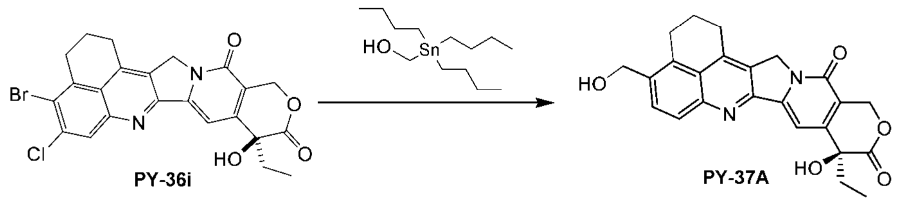

**PY-36i** **PY-37A**

The compound **PY-36i** (200 mg, 0.40 mmol, 1.0 eq) and (tributylstannyl)methanol (192 mg, 0.60 mmol, 1.5 eq) were dissolved in 1,4-dioxane (5 mL), and the catalyst chloro(2-dicyclohexylphosphino-2,4,6-triisoporpyl-1,1-biphenyl)[2-(2-amino-1,1-biphenyl)]palladium (II) (XPhos Pd G2) (31.4 mg, 0.04 mmol, 0.1 eq) were added, and the reaction solution reacted at 90°C for 4 hours in the nitrogen atmosphere. The reaction solution was cooled to room temperature and filtered, the filtrate was concentrated under reduced pressure, diluted with water, and then extracted twice with ethyl acetate (10 mL), the organic phase was washed with saturated brine (10 mL), dried with anhydrous sodium sulfate, filtered, and concentrated to obtain a crude product. The crude product was purified by reversed-phase chromatography (column: Welch Xtimate C18 150 × 30 mm × 5 μm; mobile phase: [water (FA)-ACN]; gradient: 6%-46% B, 25 min), to obtain compound **PY-37A** (2.23 mg, 0.005 mmol, yield: 1.25%) as a white solid.

LCMS: RT = 1.233 min, MS (ESI) m/z = 419.4 $[M+H]^+$.

$^1$HNMR (400MHz, DMSO-$d^6$) δ ppm 7.99 (d, $J$ = 8.8 Hz 1 H), 7.90 (d, $J$ = 8.8 Hz 1 H), 7.32 (s, 1 H), 6.51 (s, 1 H), 5.44 (s, 2 H), 5.33 (t, $J$ = 4.4 Hz , 1 H), 5.27 (s, 2 H), 4.73 (d, $J$ = 5.2 Hz , 2 H), 3.19-3.16 (m, 1 H), 3.10-3.07 (m, 1 H), 2.08 (t, $J$ = 3.2 Hz, 2 H), 1.90-1.86 (m, 2 H), 1.15-1.13 (m, 2 H), 0.89 (t, $J$ = 7.2 Hz, 3 H).

Preparation Example 39: Preparation of (S)-5-chloro-9-ethyl-4,9-dihydroxy-1,2,3,9,12,15-hexahydro-10h,13h-benzo[de]pyrano[3',4':6,7]indolizino[1,2-b]quinolin-10,13-dione **(PY-38)**

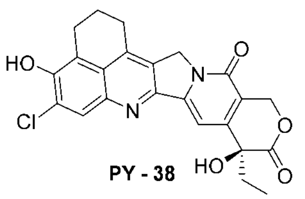

**PY - 38**

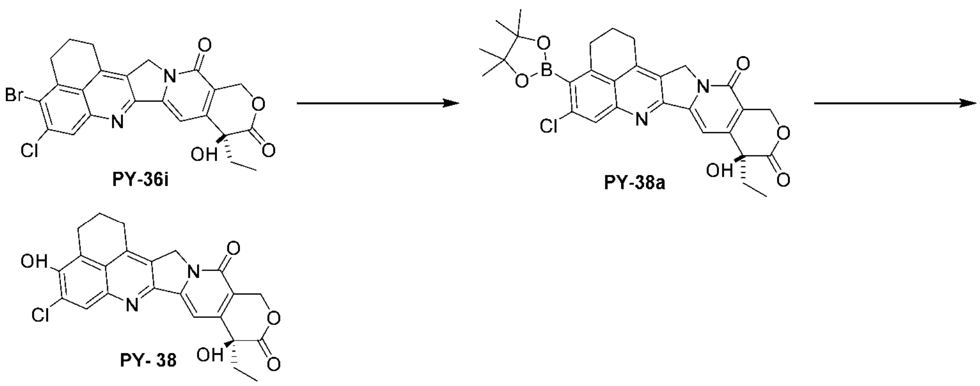

**PY-36i** **PY-38a** **PY- 38**

Step 1: Preparation of (S)-5-chloro-9-ethyl-9-hydroxy-4-(4,4,5,5-tetramethyl-1,3,2-dioxaborolan-2-yl)-1,2,3,9,12,15-hexahydro-10h,13h-benzo[de]pyrano[3',4':6,7]indolizino[1,2-b]quinolin-10,13-dione **(PY-38a)**

The compound **PY-36i** (400 mg, 0.80 mmol, 1.0 eq), bis(pinacolato)diboron (305 mg, 1.20 mmol, 1.5 eq), potassium acetate (235 mg, 2.40 mmol, 3.0 eq), and [1,1'-bis(diphenylphosphino)ferrocene]dichloropalladium (176 mg, 0.24 mmol, 0.3 eq) were dissolved in 1,4-dioxane (10 mL) and the reaction solution was stirred at 90°C for 5 hours in a nitrogen atmosphere. The reaction solution was cooled to room temperature and concentrated, water (10 mL) was added to the concentrate, then the concentrate was extracted with ethyl acetate (10 mL) twice, the organic phase was washed once with saturated brine, dried with anhydrous sodium sulfate, filtered, and concentrated to obtain a crude product, and the crude product was purified by preparative thin-layer chromatography (PE/EA=1/4), to obtain compound **PY-38a** (70.2 mg, 0.13 mmol, yield: 16.25%) as a yellow solid.

LCMS: RT = 2.391 min, MS (ESI) m/z = 549.3 $[M+H]^+$.

Step 2: Preparation of (S)-5-chloro-9-ethyl-4,9-dihydroxy-1,2,3,9,12,15-hexahydro-10h,13h-benzo[de]pyrano[3',4':6,7]indolizino[1,2-b]quinolin-10,13-dione **(PY-38)**

The compound **PY-38a** (70.2 mg, 0.13 mmol, 1.0 eq) was dissolved in tetrahydrofuran (0.2 mL) and water (0.2 mL), sodium perborate tetrahydrate (44.15 mg, 0.39 mmol, 3.0 eq) was added at 0°C, and the reaction solution reacted at 25°C for 1 hour. The reaction was quenched with saturated ammonium chloride solution (0.2 mL), and then the reaction solution was purified by reversed-phase chromatography (column: Xtimate C18 150 × 40 mm × 10 μm; mobile phase: [water (FA)-ACN]; gradient: 10%-50% B, 36 min), to obtain compound **PY-38** (0.82 mg, 0.002 mmol, yield: 1.53%) as a white solid.

LCMS: RT = 1.491 min, MS (ESI) m/z = 439.3 $[M+H]^+$.

$^1$HNMR (400MHz, DMSO-$d^6$) δ ppm 8.36 (s, 0.16 H), 8.03 (s, 1 H), 7.23 (s, 1 H), 6.49 (s, 1 H), 5.42 (s, 2 H), 5.21 (s, 2 H), 3.10 -3.07 (m, 2 H), 3.02 (t, *J* = 5.6 Hz, 2 H), 2.01 (t, *J* = 6.4 Hz, 2 H), 1.88-1.85 (m, 2 H), 1.24 (s, 1 H), 0.88 (t, *J* = 7.6 Hz, 3 H).

Preparation Example 40: Preparation of PY-41A and PY-41B

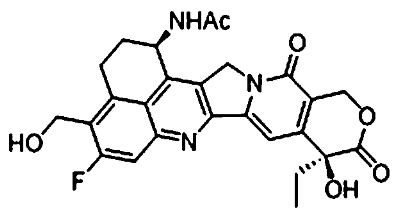

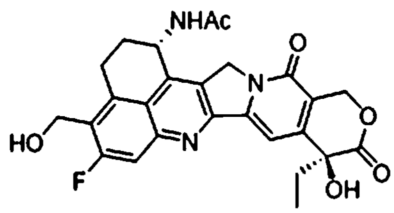

PY-41A and PY-41B

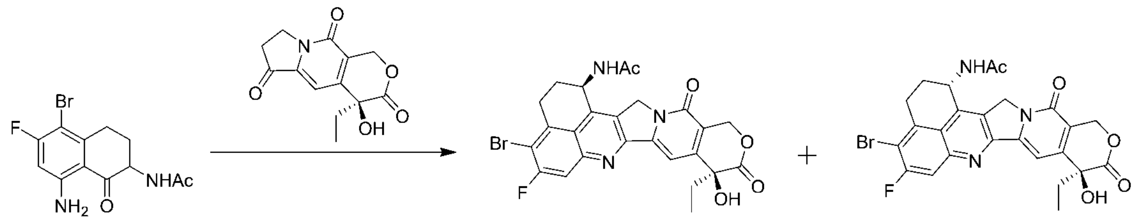

PY-U-c

PY-41A-a

PY-41A-b

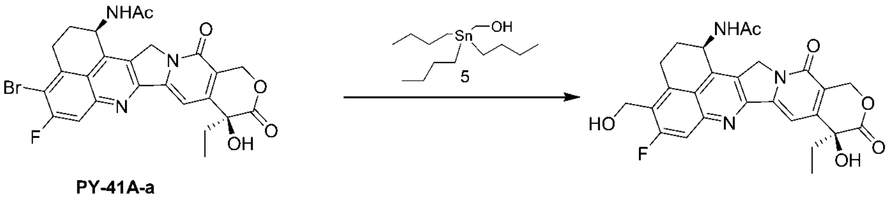

PY-41A-a

PY-41A

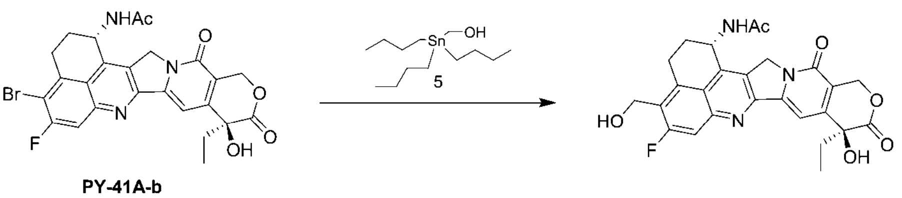

PY-41A-b

PY-41B

Step 1: Preparation of compounds PY-41A-a and PY-41A-b

The compound N-(8-amino-5-bromo-6-fluoro-1-oxo-1,2,3,4-tetrahydronaphthalene-2-yl)acetamide (PY-U-c) (for the preparation method, refer to Preparation Example 44) (140 mg, 444.25 μmol, 1 eq) was dissolved in toluene (10 mL), (S)-4-ethyl-4-hydroxy-7,8-dihydro-1H-pyrano[3,4-f]indolizino-3,6,10(4H)-trione **(PY-8f)** (116.95 mg, 444.25 μmol, 1 eq) and PPTS (111.64 mg, 444.25 μmol, 1 eq) were added, and the reaction solution was stirred at 125°C for 12 hours. The reaction solution was concentrated under reduced pressure, and the residue was purified by high performance liquid chromatograph (preparative chromatograph manufacturer: SHIMADZU, model: LC-20AP, column: YMC-Triart Prep C18 250 × 50 mm × 7 μm, mobile phase: water (0.225% HCOOH)-acetonitrile, an elution ratio of water: 21% to 51%), to obtain two brown isomer solids, namely, PY-41-a (54 mg, yield: 22.41%) and PY-41-b (54 mg, yield: 22.41%).
LCMS (ESI): m/z, 542.2 $[M+H]^+$.

Step 2: Preparation of compound **PY-41A**

The compound PY-41A-a (15 mg, 27.66 μmol, 1 eq) was dissolved in dioxane (2 mL), hydroxymethyl tributylstannane (26.64 mg, 82.97 μmol, 3 eq) and XPhos Pd G2 (4.35 mg, 5.53 μmol, 0.2 eq) were added, nitrogen displacement was performed on the reaction solution three times, and the reaction solution was stirred at 90°C for 12 hours in the nitrogen atmosphere. The reaction solution was filtered and concentrated under reduced pressure, and the residue was purified by preparative high performance liquid chromatograph (preparative chromatograph manufacturer: Oriendo, model: BRIX-2860 (R1, 4, 5, 6), Phenomenex Luna C18 (250×50 mm×10 μm) was used as a chromatographic column, mobile phase: water (0.225% HCOOH)-acetonitrile, an elution ratio of water: 14% to 24%), to obtain a white solid PY-41A (1.10 mg, yield: 8.06%).
LCMS (ESI): m/z, 494.2 $[M+H]^+$.

Step 3: Preparation of compound **PY-41B**

The compound PY-41A-b (15 mg, 27.66 μmol, 1 eq) was dissolved in dioxane (2 mL), hydroxymethyl tributylstannane (26.64 mg, 82.97 μmol, 3 eq) and XPhos Pd G2 (4.35 mg, 5.53 μmol, 0.2 eq) were added, nitrogen

displacement was performed on the reaction solution three times, and the reaction solution was stirred at 90°C for 12 hours in the nitrogen atmosphere. The reaction solution was filtered and concentrated under reduced pressure, and the residue was purified by high performance liquid chromatograph (preparative chromatograph manufacturer: Oriendo, model: BRIX-2860 (R1, 4, 5, 6), Phenomenex Luna C18 (250×50 mm×10 μm) was used as a chromatographic column, mobile phase: water (0.225% HCOOH)-acetonitrile, an elution ratio of water: 12% to 42%), to obtain a white solid product PY-41B (1.02 mg, yield: 7.47%).

LCMS (ESI): m/z, 494.1 $[M+H]^+$.

Preparation Example 41: Preparation of ((9S)-9-ethyl-5-fluoro-9-hydroxy-4-(hydroxymethyl)-10,13-dioxo-2,3,9,10,13,15-hexahydro-1H,12H-benzo[d]pyrano[3',4':6,7]indolizino[1,2-b]quinolin-1-yl)ethyl carbamate **(PY-42)**

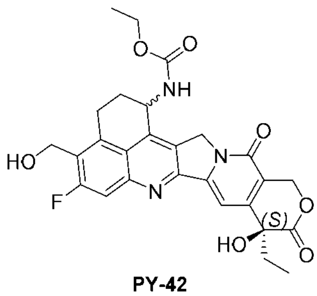

PY-42

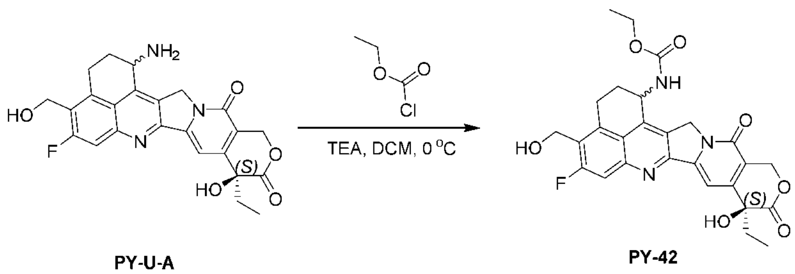


PY-U-A

PY-42

The compound PY-U-A (for the preparation method, refer to Preparation Example 44) (10.53 mg, 95%, 22.15 μmol, 1 eq) was dissolved in DCM (5 mL), TEA (4.48 mg, 6.16 μL, 44.30 μmol, 2 eq) was added, and ethyl chloroformate (4.81 mg, 4.24 μL, 44.30 μmol, 2 eq) was added at 0°C. The reaction solution was stirred at 0°C for 8 hours. The reaction solution was diluted with DCM (10 mL) and washed with water (10 mL), the organic phase was dried, then filtered, and purified by high performance liquid chromatograph (preparative chromatograph manufacturer: Oriendo, model: BRIX-2860 (R1, 4, 5, 6), column: Welch Xtimate C18 250 × 30 mm × 10 μm, mobile phase: water (0.225% HCOOH)-acetonitrile, an elution ratio of water: 20% to 50%), to obtain a white solid product **PY-42** (1.12 mg, yield: 9.66%, purity: 90%).

LCMS (ESI): m/z, 524.2 $[M+H]^+$.

Preparation Example 42: Preparation of N-((9S)-9-ethyl-5-fluoro-9-hydroxy-4-(hydroxymethyl)-10,13-dioxo-2,3,9,10,13,15-hexahydro-1H,12H-benzo[d]pyrano[3',4':6,7]indolizino[1,2-b]quinolin-1-yl)-2-hydroxyacetamide **(PY-43)**

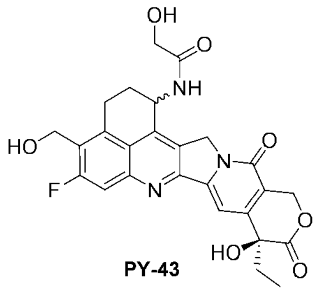

PY-43

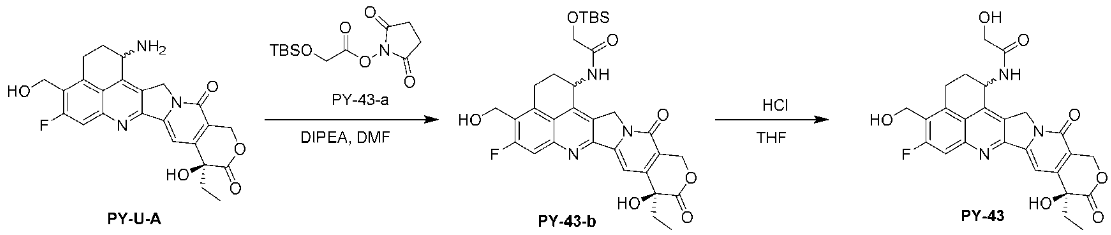


PY-U-A PY-43-b PY-43

Step 1: 2-((tert-butyldimethylsilyl)oxy)-N-((9S)-9-ethyl-5-fluoro-9-hydroxy-4-(hydroxymethyl)-10,13-dioxo-2,3,9,10,13,15-hexahydro-1H,12H-benzo[d]pyrano[3',4':6,7]indolizino[1,2-b]quinolin-1-yl)acetamide **(PY-43-b)**

The compound PY-U-A (5 mg, 100%, 11.08 μmol, 1 eq) was dissolved in DMF (1 mL), and DIPEA (2.86 mg, 3.66 μL, 22.15 μmol, 2 eq) and 2,5-dioxopyrrolidin-1-yl-2-((tertbutyldimethylsilyl)oxy)acetate (PY-43-a) (3.82 mg, 13.29 μmol, 1.2 eq) were added. The reaction solution was stirred at 20°C for 12 hours. The reaction solution was purified by preparative high performance liquid chromatograph (preparative chromatograph manufacturer: Oriendo, model: BRIX-2860 (R1, 4, 5, 6), column: GS-120-10-C18AP, mobile phase: water (0.225% HCOOH)-acetonitrile, an elution ratio of water: 30% to 60%), to obtain a white solid product (2 mg, yield: 28.95%).

LCMS (ESI): m/z, 624.2 $[M+H]^+$.

Step 2: Preparation of N-((9S)-9-ethyl-5-fluoro-9-hydroxy-4-(hydroxymethyl)-10,13-dioxo-2,3,9,10,13,15-hexahydro-1H,12H-benzo[d]pyrano[3',4':6,7]indolizino[1,2-b]quinolin-1-yl)-2-hydroxyacetamide **(PY-43)**

The compound **PY-43-b** (2 mg, 3.21 μmol, 1 eq) was dissolved in THF (1 mL), and HCl (116.91 μg, 500 μL, 3.21 μmol, 1 eq) was added. The reaction solution was stirred at 15°C for 2 hours. The reaction solution was purified by preparative high performance liquid chromatograph (preparative chromatograph manufacturer: Oriendo, model: BRIX-2860 (R1, 4, 5, 6), column: Welch Xtimate C18 250 × 30 mm × 10 μm, mobile phase: water (0.225% HCOOH)-acetonitrile, an elution ratio of water: 12% to 42%), to obtain a yellow solid product PY-43 (1.10 mg, yield: 67.33%, purity: 100%).

LCMS (ESI): m/z, 510.2 $[M+H]^+$.

Preparation Example 43: Preparation of (S)-4-amino-9-ethyl-5-fluoro-9-hydroxy-1,2,3,9,12,15-hexahydro-10H,13H-benzo[de]pyrano[3',4':6,7]indolizino[1,2-b]quinolin-10,13-dione **(PY-A)**

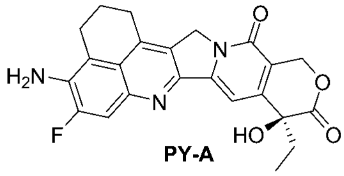

PY-A

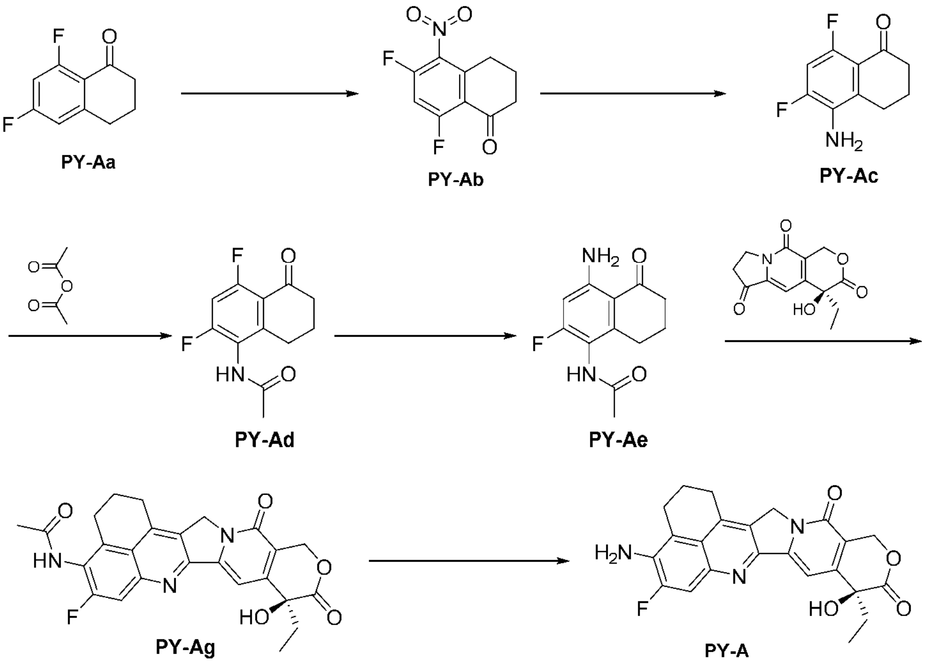

PY-Aa PY-Ab PY-Ac PY-Ad PY-Ae PY-Ag PY-A

Step 1: Preparation of 6,8-difluoro-5-nitro-1,2,3,4-tetrahydronaphthalene-1-one **(PY-Ab)**

0.96 equivalents of potassium nitrate (546 mg) was added to a solution of 6,8-difluoro-3,4-dihydronaphthalene-1(2H)-one (PY-Aa) (1.0g) in 6 mL of sulfuric acid and the reaction solution was stirred under an ice water bath for 2 hours. The reaction solution was added into ice water, the reaction was quenched with 50 mL of water, the reaction solution was extracted with ethyl acetate (60 mL × 3), the organic phase was washed with saturated brine, dried with anhydrous sodium sulfate, filtered, and concentrated under reduced pressure to obtain 800 mg of a crude product of compound PY-Ab (purity: 41%).

$^1$H NMR (400 MHz, DMSO-$d_6$) δ 7.20 - 7.07 (m, 1H), 2.96 (q, $J$ = 6.0 Hz, 2H), 2.61 (ddd, $J$ = 27.9, 7.3, 5.8 Hz, 2H), 2.12 - 2.00 (m, 2H).

Step 2: Preparation of 6,8-difluoro-5-amino-1,2,3,4-tetrahydronaphthalene-1-one **(PY-Ac)**

8 equivalents of iron powder (894 mg) and 3 equivalents of ammonium chloride (321 mg) were separately added to a mixed solution of 6,8-difluoro-5-nitro-1,2,3,4-tetrahydronaphthalene-1-one (800 mg) in 9 mL of ethanol and water (volume ratio = 8:1), and the reaction solution was stirred at 80°C for 2 hours. After iron powder was filtered out, the reaction solution was directly concentrated, and the residue was purified by silica gel column chromatography (eluents: ethyl acetate/petroleum ether = 1:1) to obtain 176 mg of 6,8-difluoro-5-amino-1,2,3,4-tetrahydronaphthalene-1-one (PY-Ac) (yield: 45%).

LCMS (ESI): m/z, 198.1 [M+H]$^+$.

$^1$H NMR (400 MHz, DMSO-$d_6$) δ 7.03 (t, $J$ = 11.4 Hz, 1H), 5.02 (s, 2H), 2.70 (t, $J$ = 6.2 Hz, 2H), 2.08 (s, 1H), 2.00 (p, $J$ = 6.4 Hz, 2H).

Step 3: Preparation of N-(2,4-difluoro-5-oxo-5,6,7,8-tetrahydronaphthalene-1-yl)acetamide **(PY-Ad)**

1.2 equivalents of acetic anhydride (343 μL) and 1.2 equivalents of triethylamine (508 μL) were separately added to a solution of 6,8-difluoro-5-amino-1,2,3,4-tetrahydronaphthalene-1-one (600 mg) in 25 mL of dichloromethane, and the reaction solution was stirred at 60°C for 36 hours. The reaction solution was concentrated, and the residue was purified by silica gel column chromatography (eluents: dichloromethane/methanol = 20:1) to obtain 483 mg of N-(2,4-difluoro-5-oxo-5,6,7,8-tetrahydronaphthalene-1-yl)acetamide (PY-Ad) (yield: 66%, purity: 95%).

LCMS (ESI): m/z, 240.1 $[M+H]^+$.

Step 4: Preparation of N-(4-amino-2-fluoro-5-oxo-5,6,7,8-tetrahydronaphthalene-1-yl)acetamide **(PY-Ae)**

35 mL of ammonia was added to a solution of N-(2,4-difluoro-5-oxo-5,6,7,8-tetrahydronaphthalene-1-yl)acetamide (PY-Ad) (1.43g) in 30 mL of DMSO in a sealed tube, and the reaction solution was stirred at 100°C for 16 hours. The reaction was quenched with 50 mL of water, the reaction solution was extracted with ethyl acetate, the organic phase was washed with saturated brine, dried with anhydrous sodium sulfate, filtered, and concentrated under reduced pressure, and the residue was purified by silica gel column chromatography (eluents: dichloromethane/methanol = 20:1) to obtain 1.01g of N-(4-amino-2-fluoro-5-oxo-5,6,7,8-tetrahydronaphthalene-1-yl)acetamide (PY-Ae) (yield: 72%, purity: 94%).

LCMS (ESI): m/z, 237.2 $[M+H]^+$.

Step 5: Preparation of (S)-N-(9-ethyl-5-fluoro-9-hydroxy-10,13-dioxo-2,3,9,10,13,15-hexahydro-1H,12H-benzo[de]pyrano[3',4':6,7]indolizino[1,2-b]quinolin-4-yl)acetamide (PY-Ag)

1.2 equivalents of compound PY-8f (1.3g) and 0.67 equivalents of PPTS (691 mg) were separately added to a solution of N-(4-amino-2-fluoro-5-oxo-5,6,7,8-tetrahydronaphthalene-1-yl)acetamide (1.01g) in 50 mL of toluene, and the reaction solution was stirred at 130°C for 16 hours. The reaction solution was concentrated, and the residue was purified by silica gel column chromatography (eluents: dichloromethane/methanol = 10:1) to obtain 584 mg of compound **PY-Ag** (yield: 30%; purity: >99%).

LCMS (ESI): m/z, 464.0 $[M+H]^+$.

$^1$H NMR (400 MHz, DMSO-$d_6$) δ 9.82 (s, 1H), 7.80 (d, $J$ = 11.0 Hz, 1H), 7.31 (s, 1H), 6.52 (s, 1H), 5.44 (s, 2H), 5.25 (s, 2H), 3.16 (t, $J$ = 6.1 Hz, 2H), 2.98 (t, $J$ = 6.0 Hz, 2H), 2.14 (s, 3H), 2.06 - 1.99 (m, 2H), 1.87 (p, $J$ = 7.1 Hz, 2H), 0.88 (t, $J$ = 7.3 Hz, 3H).

Step 6: Preparation of (S)-4-amino-9-ethyl-5-fluoro-9-hydroxy-1,2,3,9,12,15-hexahydro-10H,13H-benzo[de]pyrano[3',4':6,7]indolizino[1,2-b]quinolin-10,13-dione **(PY-A)**

12N hydrochloric acid (6 mL) was added to a solution of compound PY-Ag (78 mg) in 6 mL of ethanol and the reaction solution was stirred at 60°C for 16 hours. The reaction solution was purified by preparative high performance liquid chromatograph (preparative chromatograph manufacturer: SHIMADZU, model: LC-20AP, column: YMC-Triart Prep C18 250 × 30 mm × 10 μm, mobile phase: water (0.225% HCOOH)-acetonitrile, an elution ratio of water: 21% to 51%) and lyophilized, to obtain 43.9 mg of compound **PY-A** (yield: 61%, purity: 98%).

LCMS (ESI): m/z, 422.1 $[M+H]^+$.

$^1$H NMR (400 MHz, DMSO-$d_6$) δ 7.63 (d, $J$ = 12.5 Hz, 1H), 7.20 (s, 1H), 6.47 (s, 1H), 5.76 (s, 2H), 5.41 (s, 2H), 5.18 (s, 2H), 3.06 (t, $J$ = 6.1 Hz, 2H), 2.84 (t, $J$ = 6.1 Hz, 2H), 2.02 (t, $J$ = 6.2 Hz, 2H), 1.86 (dq, $J$ = 14.4, 7.0 Hz, 2H), 0.87 (t, $J$ = 7.3 Hz, 3H); $^{19}$F NMR (377 MHz, DMSO-$d_6$) δ -125.24.

Preparation Example 44: Preparation of PY-U-A and PY-U-B

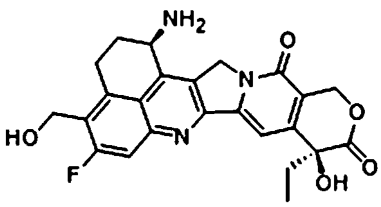

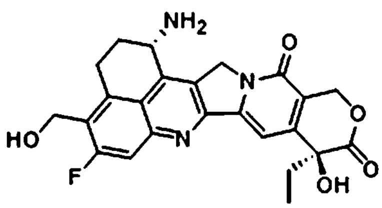

PY-U-A and PY-U-B

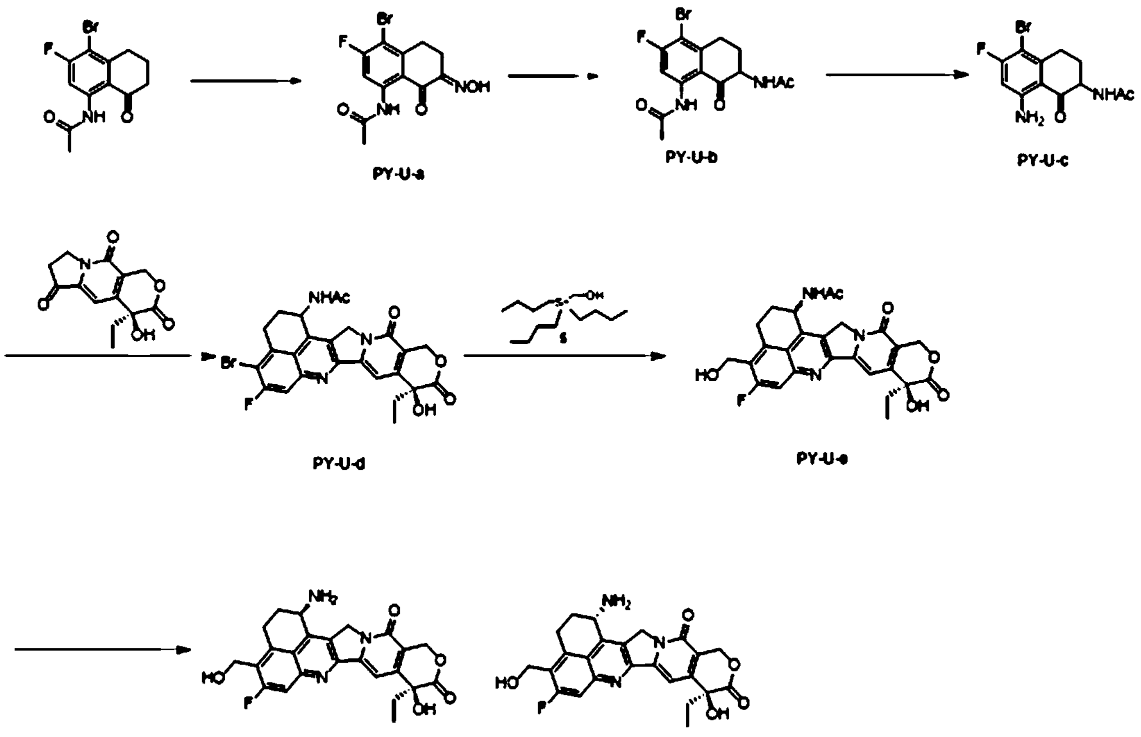

PY-U-a PY-U-b PY-U-c

PY-U-d PY-U-e

PY-U-A and PY-U-B

Step 1: Preparation of (E)-N-(4-bromo-3-fluoro-7-(hydroxyimino)-8-oxo-5,6,7,8-tetrahydronaphthalene-1-yl)acetamide **(PY-U-a)**

Potassium tert-butoxide (3.36g, 29.99 mL, 29.99 mmol, 3 eq, 1M in THF) and tert-butyl nitrite (3.09g, 3.60 mL, 29.99 mmol, 3 eq) were added to a solution of N-(4-bromo-3-fluoro-8-oxo-5,6,7,8-tetrahydronaphthalene-1-yl)acetamide (PY-8g) (3g, 10 mmol, 1 eq) in anhydrous THF (150 mL) at 0°C in a nitrogen atmosphere, and after addition, the reaction solution reacted at 0°C-10°C for 1.5 hours. 200 mL of water was added to the reaction solution at 0°C, the reaction solution was then extracted with ethyl acetate (200 mL × 3), the organic phases were combined and then washed with saturated brine (100 mL), dried with anhydrous sodium sulfate, filtered, and concentrated under reduced pressure to obtain a crude product of compound **PY-U-a** (3.20g, crude product, yield: 48.63%, purity: 50%) as a yellow solid.
LCMS (ESI): m/z, 329.0 $[M+1]^+$.

Step 2: Preparation of compound N,N'-(4-bromo-3-fluoro-8-oxo-5,6,7,8-tetrahydronaphthalene-1,7-diyl)diacetylamide **(PY-U-b)**

Zinc powder (4.77g, 668.47 μL, 72.92 mmol, 15 eq) was added to the solution of compound PY-U-a (3.20g, 50%, 4.86 mmol, 1 eq) in acetic acid (40 mL) and acetic anhydride (20 mL), and after addition, the reaction solution reacted at 15°C-20°C for 16 hours. The reaction solution was concentrated to remove acetic acid, the residue was added into a mixed solution of ethyl acetate (50 mL) and water (50 mL), the mixed solution was stirred for 10 minutes and filtered with diatomite, the filtrate was added into a separating funnel to separate liquids, the aqueous phase was extracted with ethyl acetate twice (50 mL × 2), the organic phases were combined, washed with brine, dried with anhydrous sodium sulfate, and filtered, and the filtrate was concentrated under reduced pressure. The residue was purified by silica gel column chromatography (dichloromethane: methanol = 10:1) to obtain the yellow solid compound **PY-U-b** (1.15g, yield: 55.63%, purity: 84%).
LCMS (ESI): m/z, 357 $[M+H]^+$, 359 $[M+H]^+$.

Step 3: Preparation of N-(8-amino-5-bromo-6-fluoro-1-oxo-1,2,3,4-tetrahydronaphthalene-2-yl)acetamide **(PY-U-c)**

The compound PY-U-b (1.15g, 84%, 2.70 mmol, 1 eq) was dissolved in ethanol (15 mL), then HCl (3.28g, 15 mL, 90 mmol, 33.2776 eq, 6 M) was added, and the reaction solution was stirred at 60°C for 2 hours. The reaction solution was concentrated to remove most of the solvent, neutralized with sodium bicarbonate (30 mL), and then extracted with DCM (20 mL × 2), and the organic phase was dried, filtered, and concentrated under reduced pressure to be directly used for the

next step. The resulting product was a brown solid (700 mg, yield: 68.99%).

LCMS (ESI): m/z, 315.0 $[M+H]^+$.

Step 4: Preparation of N-((9S)-4-bromo-9-ethyl-5-fluoro-9-hydroxy-10,13-dioxo-2,3,9,10,13,15-hexahydro-1H,12H-benzo[d]pyrano[3',4':6,7]indolizino[1,2-b]quinolin-1-yl)acetamide **(PY-U-d)**

The compound PY-U-c (700 mg, 2.22 mmol, 1 eq) was dissolved in toluene (10 mL), (S)-4-ethyl-4-hydroxy-7,8-dihydro-1H-pyrano[3,4-f]indolizino-3,6,10(4H)-trione (PY-8f) (877.11 mg, 3.33 mmol, 1.5 eq) and PPTS (558.20 mg, 2.22 µmol, 1 eq) were added to the solution, and the solution was stirred at 125°C for 12 hours. The reaction solution was concentrated under reduced pressure, and the residue was purified by preparative high performance liquid chromatography to obtain the brown solidproduct (1g, yield: 83.01%, purity: 75%).

LCMS (ESI): m/z, 542.1 $[M+H]^+$.

Step 5: Preparation of N-((9S)-9-ethyl-5-fluoro-9-hydroxy-4-(hydroxymethyl)-10,13-dioxo-2,3,9,10,13,15-hexahydro-1H,12H-benzo[d]pyrano[3',4':6,7]indolizino[1,2-b]quinolin-1-yl)-acetamide **(PY-U-e)**

The compound PY-U-d (300 mg, 75%, 414.85 µmol, 1 eq) was dissolved in dioxane (10 mL), and (tributylstannyl) methanol (399.62 mg, 1.24 µmol, 3 eq) and XPhos Pd G2 (65.28 mg, 82.97 µmol, 0.2 eq) were added. Nitrogen displacement was performed on the reaction solution, and the reaction solution was stirred at 90°C for 12 hours in the nitrogen atmosphere. The reaction solution was concentrated under reduced pressure, and the residue was purified by high performance liquid chromatography to obtain the white solid product (120 mg, yield: 58.6%).

LCMS (ESI): m/z, 494.1 $[M+H]^+$.

Step 6: Preparation of compounds PY-U-A and PY-U-B

The compound PY-U-e (50 mg, 101.32 µmol, 1 eq) was dissolved in HCl (6N, 6 mL), DIPEA was added, and the reaction solution was stirred at 85°C for 6 hours. The reaction solution was purified by preparative high performance liquid chromatograph (preparative chromatograph manufacturer: Oriendo, model: R-120g, Phenomenex Luna C18 (250×50 mm×10 µm) was used as a chromatographic column, mobile phase: water (0.225% HCOOH)-acetonitrile, an elution ratio of water: 10% to 20%), to obtain two white isomer solids, namely, PY-U-A (62 mg, no yield was calculated for the crude product) and PY-U-B (16 mg, yield: 34.98%).

LCMS (ESI): m/z, 452.2 $[M+H]^+$.

Preparation Example 45: Preparation of (S)-7-ethyl-7-hydroxy-14-(3-hydroxypropyl)-10,13-dihydro-11H-[1,3]dioxolo[4,5-g]pyrano[3',4':6,7]indolizino[1,2-b]quinolin-8,11(7H)-dione (PY-Y)

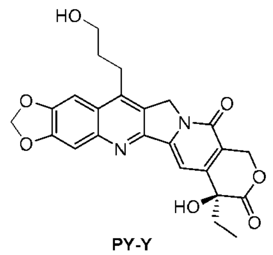

PY-Y

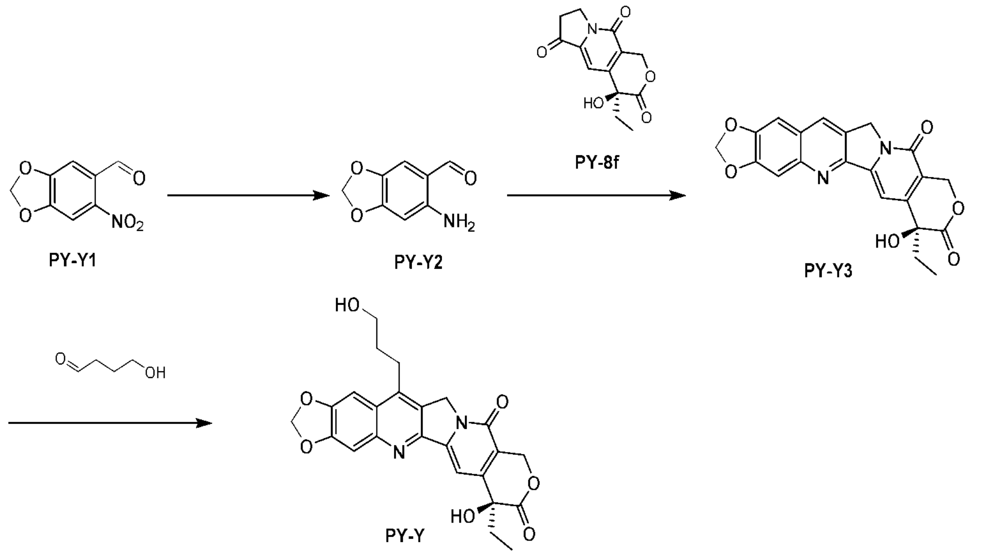

PY-8f

PY-Y1 PY-Y2 PY-Y3

PY-Y

Step 1: Preparation of 6-aminobenzo[d][1,3]dioxole-5-carbaldehyde (PY-Y2).

Iron powder (3.94g, 70.47 mmol, 5.5 eq) and ammonium chloride (3.77g, 70.47 mmol, 5.5 eq) were added to ethanol (30.0 mL) and water (3.00 mL), then 6-nitrobenzo[d][1,3]oxole-5-carbaldehyde (2.50g, 12.81 mmol, 1 eq), and the reaction solution reacted at 80°C for 4 hours. The reaction solution was filtered, the filtrate was concentrated under reduced pressure, the residue was dissolved in water and dichloromethane (100 mL), the organic phase was washed with saturated sodium bicarbonate (30 mL), dried with anhydrous sodium sulfate, and concentrated under reduced pressure, and the residue was purified by silica gel column chromatography ($SiO_2$, DCM: MeOH = 1:0, $Rf_{(P)}$ = 0.39), to obtain 6-aminobenzo[d][1,3]dioxole-5-carbaldehyde (1.27g, yield: 59.83%).

LCMS: RT = 0. 996 min, MS (ESI) m/z = 166.1 $[M+H]^+$.

Step 2: Preparation of (S)-7-ethyl-7-hydroxy-10,13-dihydro-11h-[1,3]dioxolo[4,5-g]pyrano[3',4':6,7]indolizino[1,2-b]quinolin-8,11(7H)-dione **(PY-Y3)**

The compound **PY-8f** (2.00g, 7.60 mmol, 1 eq) and 6-aminobenzo[d][1,3]dioxole-5-carbaldehyde (1.25g, 7.60 mmol, 1 eq) were dissolved in toluene (160 mL), then p-toluene sulfonic acid monohydrate (144.52 mg, 759.75 μmol, 0.1 eq) was added, the reaction solution was stirred at 125°C for 16 hours, cooled to 25°C and filtered, and the filter cake was washed with 10 mL of tetrahydrofuran and dried under reduced pressure to obtain compound PY-Y3 (1.51g, yield: 50.65%).

LCMS: RT = 1. 271 min, MS (ESI) m/z = 393.2 $[M+H]^+$.

Step 3: Preparation of (S)-7-ethyl-7-hydroxy-14-(3-hydroxypropyl)-10,13-dihydro-11H-[1,3]dioxolo[4,5-g]pyrano [3',4':6,7]indolizino[1,2-b]quinolin-8,11(7H)-dione **(PY-Y)**

The compound PY-Y3 (400 mg, 795.19 μmol, 1 eq) and ferrous sulfate heptahydrate (353.72 mg, 1.27 mmol, 1.6 eq) were dissolved in 10 mL of water, 98% sulfuric acid (4.59g, 46.83 mmol, 2.50 mL, 58.89 eq) was added under stirring, the reaction solution was stirred thoroughly for 10 minutes, then 4-hydroxybutyraldehyde (343.3 mg, 3.90 mmol, 5 eq) was added dropwise at 0°C, hydrogen peroxide (1.26g, 11.10 mmol, 1.07 mL, purity: 30%, 13.96 eq) was further added dropwise in 20 mL of aqueous solution, and the reaction solution was stirred at 0°C-5°C for 1 hour. The reaction solution was added into ice water, adjusted with saturated sodium bicarbonate solution until pH reached 8.0, extracted with ethyl

acetate (50 mL × 5), and concentrated under reduced pressure, and the residue was purified by preparative high performance liquid chromatography (Xtimate C18 150 × 40 mm × 10 μm; mobile phase: [water (0.1% FA)-acetonitrile]; gradient: 10%-44% acetonitrile, 36 min) to obtain compound PY-Y (99.5 mg, purity: 99.6%).

LCMS: RT = 1. 890 min, MS (ESI) m/z = 451.3 $[M+H]^+$.

$^1$HNMR (400MHz, DMSO-$d^6$) $\delta$ ppm 7.63 (s, 1 H), 7.50 (s, 1 H), 7.24 (s, 1 H), 6.50 (s, 1 H), 6.29 (s, 2 H), 5.42 (s, 2 H), 5.25 (s, 2 H), 4.68 (t, $J$ = 5.2 Hz, 1 H), 3.49 (t, $J$ = 5.6 Hz, 2 H), 3.15 (t, $J$ = 6.8 Hz, 2 H), 2.53 (t, $J$ = 1.6 Hz, 1 H), 2.33 (t, $J$ = 1.6 Hz, 1 H), 1.81 - 1.88 (m, 2 H), 0.88 (t, $J$ = 7.2 Hz, 3 H).

Preparation Example 46: Preparation of N2-(6-(2,5-dioxo-2,5-dihydro-1H-pyrrol-1-yl) hexanoyl)-N6-(2R,3S,4R,5R)-2,3,4,5,6-pentahydroxyhexanoyl)-L-lysine (A)

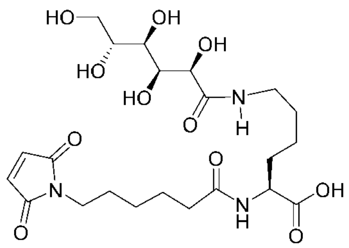

A

Route 1:

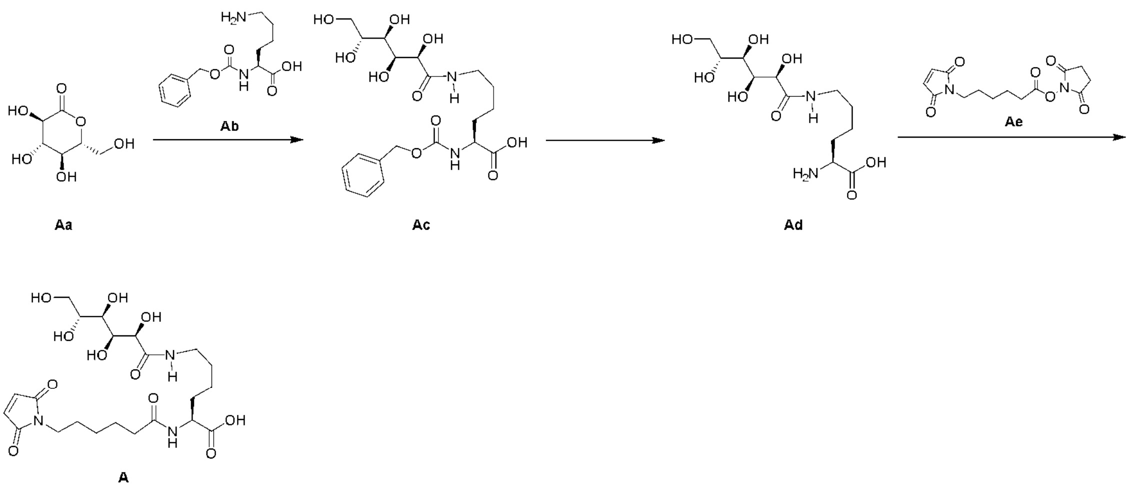


Aa Ac Ad A

Step 1: Preparation of N2-(benzyloxy)carbonyl)-N6-(2R,3S,4R,5R)-2,3,4,5,6-pentahydroxyhexanoyl)-L-lysine **(Ac)**

(3R,4S,5S,6R)-3,4,5-trihydroxy-6-(hydroxymethyl)tetrahydro-2H-pyrano-2-one **(Aa)** (1.27g, 7.13 mmoL) and ((benzyloxy)carbonyl)-L-lysine **(Ab)** (2g, 7.13 mmol) were dissolved in methanol (30 mL) at room temperature, and then triethylamine (1.44g, 14.27 mmoL) was added. The reaction solution was stirred at 70°C for 16 hours. The reaction solution was concentrated under reduced pressure. The resulting residue was isolated by high performance liquid chromatograph (Oriendo, BRIX-2860; column: Phenomenex Luna C18 250 × 50 mm × 10 μm; mobile phase: water (0.225% trifluoroacetic acid)-acetonitrile, eluted from 45% to 85%), to obtain a white solid compound **Ac** (2.5g, yield: 76.43%).

LCMS: $[M+H]^+$ = 459.1.

Step 2: Preparation of N6-(2R,3S,4R,5R)-2,3,4,5,6-pentahydroxyhexanoyl)-L-lysine **(Ad)**

The compound N2-(benzyloxy)carbonyl)-N6-(2R,3S,4R,5R)-2,3,4,5,6-pentahydroxyhexanoyl)-L-lysine (Ac) (2.5g, 5.45 mmoL) was dissolved in methanol (40 mL) at room temperature, and 10% wet palladium on carbon (250 mg) was further added to the reaction solution. The reaction solution was stirred for 6 hours at room temperature in the hydrogen atmosphere. The reaction solution was filtered with diatomite, the filter cake was washed with water, and the

filtrate was lyophilized to obtain a white solid compound **Ad** (1.6g, yield: 88.96%).

LCMS: $[M+H]^+$ = 325.1.

$^1H$ NMR (400 MHz, DMSO-$d^6$) δ 4.16-4.19 (d, J = 9.4 Hz, 1H), 3.93-3.97 (t, J = 9.4 Hz, 1H), 3.72 - 3.50 (m, 5H), 3.10-3.20 (m, 2H), 1.68-1.79 (m, 2H), 1.40-1.55 (m, 2H), 1.20-1.35 (m, 2H).

Step 3: Preparation of $N^2$-(6-(2,5-dioxo-2,5-dihydro-1H-pyrrol-1-yl)hexanoyl)-$N^6$-(2R,3S,4R,5R)-2,3,4,5,6-pentahydroxyhexanoyl)-L-lysine **(A)**

The compound $N^6$-(2R,3S,4R,5R)-2,3,4,5,6-pentahydroxyhexanoyl)-L-lysine **(Ad)** (1.6g,4.93 mmol) was dissolved in N,N-dimethylformamide (30 mL) at room temperature, and then 2,5-dioxopyrrolidin-1-yl-6-(2,5-dioxo-2,5-dihydro-1H-pyrrol-1-yl)hexanoate **(Ae)** (1.52g, 4.93 mmoL) and DIEA (1.27g, 9.87 mmoL) were separately added. The reaction solution was stirred at 70°C for 16 hours in the nitrogen atmosphere. The reaction solution was filtered and isolated by preparative high performance liquid chromatograph (Oriendo, BRIX-2860; column: Phenomenex Luna C18 250 × 50 mm × 10 μm; mobile phase: water (0.225% trifluoroacetic acid)-acetonitrile, eluted from 45% to 85%), to obtain a white solid linker A (400 mg, yield: 15.67%).

LCMS: $[M+H]^+$ = 517.9.

$^1H$ NMR (400 MHz, DMSO-$d_6$) δ 7.93 (d, *J* = 7.6 Hz, 1H), 7.60 (t, *J* = 7.6 Hz, 1H), 6.97 (s, 2H), 5.32 (m, 1H), 4.59 - 4.30 (m, 2H), 4.17 - 3.99 (m, 1H), 3.96-3.91 (m, 1H), 3.89-3.81 (m, 1H), 3.57-3.5 (m, 1H), 3.48-3.39 (m, 2H), 3.34 (s, 4H), 3.12 - 2.89 (m, 2H), 2.05 (t, *J* = 6.4Hz ,2H), 1.82 - 0.95 (m, 13H).

Route 2:

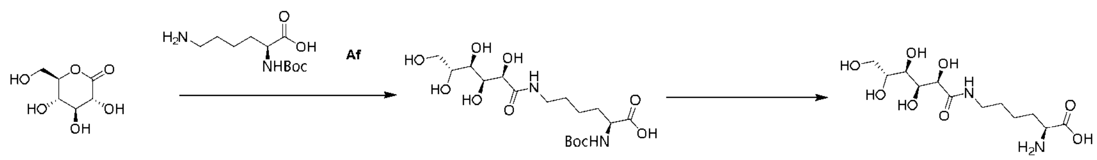


Aa Ag Ad

Step 1: Preparation of $N^2$-(tert-butoxycarbonyl)-$N^6$-(2R,3S,4R,5R)-2,3,4,5,6-pentahydroxyhexanoyl)-L-lysine **(Ag)**

(tert-butoxycarbonyl)-L-lysine **(Af)** (5g, 0.02 mol), (3R,4S,5S,6R)-3,4,5-trihydroxy-6-(hydroxymethyl)tetrahydro-2H-pyrano-2-one **(Aa)** (3.6g, 0.02 mol) were dissolved in MeOH (80 mL), TEA (4.1g, 0.04 mol) was added, and the reaction solution reacted at 70°C for 16 hours. The reaction solution was concentrated, MTBE (50 mL × 3) was added, the resulting solution was concentrated under reduced pressure and then pulped with petroleum ether to obtain 8g of a crude product, namely, a white foam crude product (the product was a salt formed by an acidic compound and TEA), wherein the yield was not calculated.

LCMS (ESI): m/z, 425.2$[M+H]^+$, 447.2$[M+Na]^+$.

$^1H$ NMR (400 MHz, DMSO) δ 7.50 (brs, 1H), 5.61 (d, *J* = 4.0 Hz, 1H), 4.27 (s, 1H), 4.13 (s, 1H), 4.10-3.85 (m, 1H), 3.85-3.66 (m, 4H), 3.28-3.15 (m, 2H), 1.80-1.55 (m, 2H),1.55-1.48 (m, 2H), 1.50-1.21 (m, 11H.

Step 2: Preparation of $N^6$-(2R,3S,4R,5R)-2,3,4,5,6-pentahydroxyhexanoyl)-L-lysine **(Ad)**

$N^2$-(tert-butoxycarbonyl)-$N^6$-(2R,3S,4R,5R)-2,3,4,5,6-pentahydroxyhexanoyl)-L-lysine **(Ag)** (7g) was dissolved in DCM (35 mL), TFA (35 mL) was added, and the reaction solution reacted at 10°C-15°C for 16 hours. The reaction solution was concentrated, DCM (50 mL × 3) was added, the resulting solution was concentrated under reduced pressure, then water was added for full dissolution, and the resulting solution was lyophilized to obtain 8g of a yellow viscous crude product, with yield of 100%.

$^1H$ NMR (400 MHz, DMSO) δ 9.26 (s, 1H), 8.26 (d, *J* = 25.1 Hz, 3H), 7.69 (dd, *J* = 37.2, 31.4 Hz, 1H), 3.99 (d, *J* = 3.7 Hz, 1H), 3.94 - 3.84 (m, 2H), 3.58 (dd, *J* = 13.3, 3.7 Hz, 1H), 3.51 - 3.45 (m, 2H), 3.40-3.34 (m, 1H), 3.09 (qt, *J* = 15.7, 7.9 Hz, 2H), 1.84 - 1.67 (m, 2H), 1.60 - 1.27 (m, 4H), 1.18 (t, *J* = 7.3 Hz, 5H).

Other steps are the same as those in Route 1 and a linker A was obtained.

Preparation Example 47: Preparation of antibody-tag 2-(2-aminoethoxy)-N-(3-azidopropyl)acetamide hydrochloride **(TS-1)**

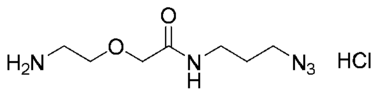

TS-1

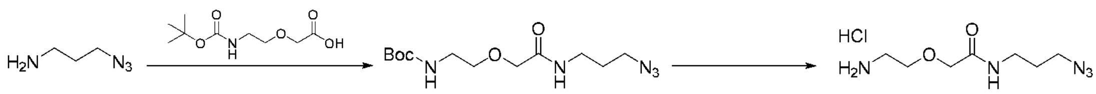


TS-11 TS-13 TS-1

Step 1: Preparation of (2-(2-(3-azidopropyl)amino)-2-oxoethoxy)tert-butyl carbamate **(TS-13)**

3-azidopropylamine (205.4 mg, 2.0 mmol, 1.0 eq), DIEA (662.6 mg, 5.1 mmol, 2.5 eq) and $T_3P$ (2.3g, 3.7 mmol, 1.8 eq) were sequentially added to the solution of 2-(2-tert-butoxycarbonyl)amino)ethoxy)acetic acid (TS-12) (ChemExpress, 450.0 mg, 2.0 mmol, 1.0 eq) in dichloromethane (4.5 mL). The reaction solution was stirred at room temperature for 16 hours and then diluted with 30 mL of dichloromethane, and the organic phase was washed with 20 mL of water. The aqueous phase was extracted once with 30 mL of dichloromethane, and the organic phases were combined and washed with 20 mL of saturated brine. The organic phases were dried, filtered, and concentrated. The residue was purified by high-speed chromatography (eluents: ethyl acetate/n-hexane = 1:20-1:1) via silica gel columns to obtain a colorless oily compound TS-13 (492 mg, yield: 79%).
LCMS (ESI): m/z, 302 $[M+H]^+$.
$^1H$ NMR (400 MHz, Chloroform-*d*) $\delta$ 6.84 (s, 1H), 4.88 (s, 1H), 3.95 (s, 2H), 3.56 (t, $J$ = 5.2 Hz, 2H), 3.41 - 3.31 (m, 6H), 1.81 (q, $J$ = 6.7 Hz, 2H), 1.44 (s, 9H).

Step 2: Preparation of 2-(2-aminoethoxy)-N-(3-azidopropyl)acetamide hydrochloride **(TS-1)**

The compound (2-(2-(3-azidopropyl)amino)-2-oxoethoxy)tert-butyl carbamate (TS-13) (482.0 mg, 1.6 mmol, 1.0 eq) and 5 mL of methanol were added to the three-necked flask, a solution of 1,4-dioxane (1.6 mL) in 6M HCl was slowly added dropwise at 0°C, and the temperature did not exceed 5°C. After the reaction solution was warmed to room temperature and stirred for 16 hours, the reaction system was concentrated. The residue was purified by high performance liquid chromatograph (Oriendo, BRIX-2860; column: Phenomenex Luna C18 250 $\times$ 50 mm $\times$ 10 $\mu$m; mobile phase: water (0.1% HCl)-acetonitrile, an elution ratio of water: 55% to 85%) and lyophilized, to obtain a colorless oily compound TS-1 (94.8 mg, yield: 24.7%).
LCMS (ESI): m/z, 202 $[M+H]^+$.
$^1H$ NMR (400 MHz, DMSO-$d_6$) $\delta$ ppm 1.67 - 1.72 (m, J=6.85 Hz, 2 H), 2.97 - 3.04 (m, $J$=5.30 Hz, 2 H), 3.15 - 3.20 (q, J=6.71 Hz, 2 H), 3.34- 3.37 (t, J=6.82 Hz, 2 H), 3.62 - 3.64 (t, $J$=5.00 Hz, 2 H), 3.91 (s, 2 H), 8.21 - 8.30 (m, 3 H).

Example 1: Preparation of compound LY-1

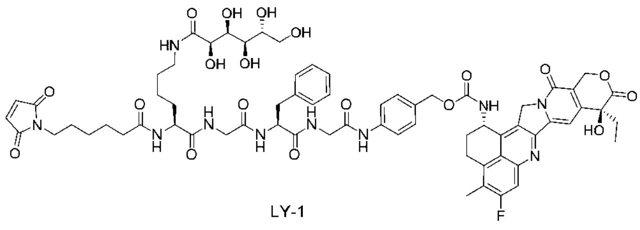

LY-1

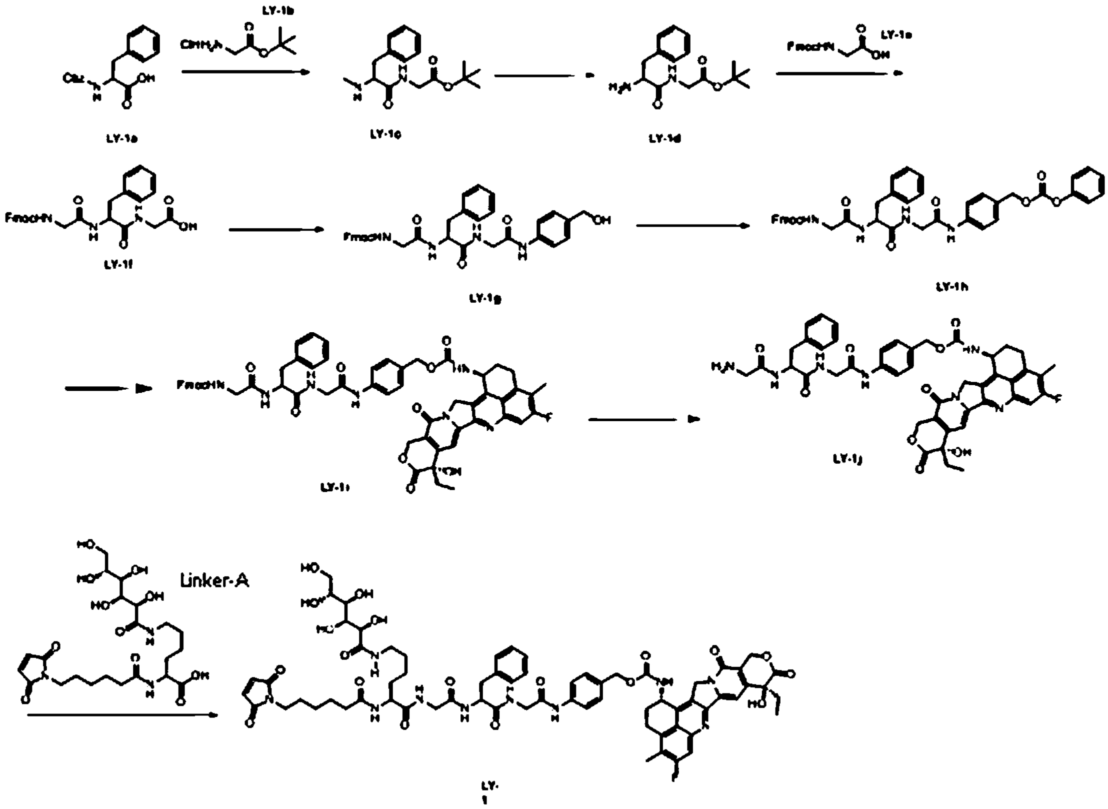


Step 1: Preparation of methyl-L-phenylalanylglycine tert-butyl ester **(LY-1c)**

((benzyloxy)carbonyl)-L-phenylalanine (LY-1a) (3.0g, 10.0 mmol) was dissolved in 50 mL of DMF, and glycine tert-butyl ester hydrochloride (1.9g, 10.1 mmol), HOBt (0.68g, 5.0 mmol), EDCI (2.3g, 12.0 mmol) and DIPEA (3.87g, 30.0 mmol) were added sequentially. The resulting solution was stirred at room temperature for 18 hours in the nitrogen atmosphere. The reaction solution was diluted with water (80 mL) and extracted twice with DCM (100 mL), and organic phases were combined, washed with saturated brine, and concentrated under reduced pressure. The residue was purified by silica gel column chromatography (eluents: ethyl acetate/n-hexane = 1:20-1:5) to obtain compound **LY-1c** (3.5g, yield: 85%) as a colorless oily compound.

Step 2: Preparation of L-phenylalanylglycine tert-butyl ester **(LY-1d)**

Methyl-L-phenylalanylglycine tert-butyl ester **(LY-1c)** (3.0g, 7.3 mmol) was dissolved in 30 mL of dichloromethane and 10 mL of trifluoroacetic acid was added at 0°C. The resulting solution was stirred at room temperature for 2 hours in the nitrogen atmosphere. The resulting solution was concentrated under reduced pressure. The product as a pale yellow solid was directly used for the next step without being purified.

Step 3: Preparation of ((9H-fluoren-9-yl)methoxy)carbonyl)glycyl-L-phenylalanylglycine **(LY-1f)**

L-phenylalanylglycine tert-butyl ester **(LY-1d)** (1.39g, 5 mmol) and **LY-1e** (1.49g, 5 mmol) were dissolved in 50 mL of DMF, and HOBt (0.68g, 5.0 mmol), EDCI (1.9g, 10.0 mmol) and DIPEA (3.87g, 30.0 mmol) were added sequentially. The resulting solution was stirred at room temperature for 18 hours in the nitrogen atmosphere. Dilute hydrochloric acid (30 mL) was added to adjust the pH to 1-2, DCM (100 mL) was used for extraction twice, and organic phases were combined,

washed with saturated brine, and concentrated under reduced pressure. The residue was isolated and purified by silica gel column chromatography (eluents: ethyl acetate/n-hexane = 1:20-1:5) to obtain compound **LY-1f** (3.1g, yield: 84%, two steps) as a white solid.

LCMS (ESI): m/z, 502.4 $[M+H]^+$.

Step 4: Preparation of (9H-fluoren-9-yl)methyl-(2-(1-(2-(4-(hydroxymethyl)phenyl)amino)-2-oxoethyl)amino)-1-oxo-3-phenylpropan-2-yl)amino)-2-oxoethyl)carbamate **(LY-1g)**

The compound ((9H-fluoren-9-yl)methoxy)carbonyl)glycyl-L-phenylalanylglycine (2.5g, 5 mmol) was dissolved in 20 mL of DMF, and HATU (2.3g, 6.0 mmol), DIPEA (1.9g, 15.0 mmol) and 4-aminobenzyl alcohol (0.74g, 6 mmol) were added sequentially. The reaction solution was stirred at room temperature for 10 hours in the nitrogen atmosphere. The reaction solution was diluted with water (50 mL), DCM (100 mL) was used for extraction twice, and organic phases were combined, washed with saturated brine, and concentrated under reduced pressure. The residue was isolated and purified by silica gel column chromatography (eluents: ethyl acetate/n-hexane = 1:20-1:5) to obtain compound **LY-1g** (2.8g, yield: 92%) as a white solid.

LCMS (ESI): m/z, 607.2 $[M+H]^+$.

Step 5: Preparation of (9H-fluoren-9-yl)methyl-(2-(1-(2-(4-(4-(nitrophenoxy)carbonyl)oxy)methyl)phenyl)amino)-2-oxoethyl)amino)-1-oxo-3-phenylpropan-2-yl)amino)-2-oxoethyl)carbamate **(LY-1h)**

(9H-fluoren-9-yl)methyl-(2-(1-(2-(4-(hydroxymethyl)phenyl)amino)-2-oxoethyl)amino)-1-oxo-3-phenylpropan-2-yl)amino)-2-oxoethyl)carbamate **(LY-1g)** (2.0g, 3.3 mmol) and bis(4-nitrophenyl)carbonate (2g, 6.6 mmol) were dissolved in DMF (20 mL), and DIPEA (0.43g, 3.3 mmol) was added. The resulting solution was stirred at room temperature for 2 hours in the nitrogen atmosphere. The reaction solution was diluted with water (30 mL), DCM was used for extraction twice, and organic phases were combined, washed with saturated brine, and concentrated under reduced pressure. The residue was isolated and purified by silica gel column chromatography (eluents: ethyl acetate/n-hexane = 1:20-1:5) to obtain compound **LY-1h** (0.92g, yield: 36%) as a white solid.

Step 6: Preparation of compounds (**LY-1i**) and **(LY-1j)**

(9H-fluoren-9-yl)methyl-(2-(1-(2-(4-(4-(nitrophenoxy)carbonyl)oxy)methyl)phenyl)amino)-2-oxoethyl)amino)-1-oxo-3-phenylpropan-2-yl)amino)-2-oxoethyl)carbamate **(LY-1h)** (397 mg, 0.54 mmol) and exatecan mesylate (300 mg, 0.56 mmol) were dissolved in DMF (2 mL), HOBt (73 mg, 0.54 mmol), pyridine (425 mg, 5.38 mmol) and DIPEA (208 mg, 1.61 mmol) were added, and the reaction solution was stirred overnight at room temperature to obtain **LY-1i.** NMM (109 mg, 1.08 mmol) was added, and the reaction solution was stirred at room temperature for 8 hours. The reaction solution was isolated by preparative HPLC (column: Phenomenex Luna C18 250 × 50 mm × 10 μm; mobile phase: water (0.225% HCOOH)-acetonitrile, eluted from 45% to 85%), to obtain compound **LY-1j** (123 mg, yield: 21%) as a white solid.

LCMS (ESI): m/z, 1068.5 $[M+H]^+$.

Step 7: Preparation of compound **(LY-1)**

HATU (10.3 mg, 0.027 mmol, 1.5 eq), DIPEA (7.0 mg, 0.054 mmol, 3.0 eq) and **LY-1j** (15 mg, 0.018 mmol, 1 eq) were added to the solution of linker A (11.2 mg, 0.022 mmol, 1.2 eq) in anhydrous tetrahydrofuran (7 mL). The reaction solution was stirred for 2 hours at 60°C. The reaction solution was isolated and purified by preparative high performance liquid chromatography (column: Phenomenex Luna C18 250 × 50 mm × 10 um; mobile phase: water (0.225% HCOOH)-acetonitrile, eluted from 45% to 85%) and lyophilized, to obtain a target compound **LY-1** (9.4 mg, yield: 38%) as a yellow solid.

LCMS (ESI): m/z, 1345.6 $[M+H]^+$, 673.5 $[1/2M+H]^+$.

$^1H$ NMR (400 MHz, DMSO-$d_6$) δ 9.87 (s, 1H), 8.40 (s, 1H), 8.10 (dd, *J* = 20.8, 8.0 Hz, 3H), 7.94 (d, *J* = 7.2 Hz, 1H), 7.78 (d, *J* = 10.8 Hz, 1H), 7.66 - 7.53 (m, 3H), 7.37 (d, *J* = 8.2 Hz, 2H), 7.31 (s, 1H), 7.27 - 7.22 (m, 3H), 7.18 (t, *J* = 4.4 Hz, 1H), 6.98 (d, *J* = 2.2 Hz, 2H), 6.53 (s, 1H), 5.44 (s, 2H), 5.35 (s, 1H), 5.29 (s, 3H), 5.08 (s, 2H), 4.50 (dd, *J* = 13.6, 8.4 Hz, 5H), 4.18 - 4.07 (m, 1H), 3.96 (s, 1H), 3.93 - 3.83 (m, 3H), 3.72 (s, 1H), 3.64 - 3.51 (m, 2H), 3.46 (s, 2H), 3.23 (s, 4H), 3.05 (dt, *J* = 15.2, 7.2 Hz, 5H), 2.90 - 2.77 (m, 2H), 2.38 (d, *J* = 1.9 Hz, 3H), 2.25 - 2.01 (m, 5H), 1.92 - 1.82 (m, 2H), 1.58 (s, 2H), 1.49 - 1.33 (m, 6H), 1.26 - 1.10 (m, 4H), 0.87 (t, *J* = 7.3 Hz, 3H).

Example 2: Preparation of compound **LY-2**

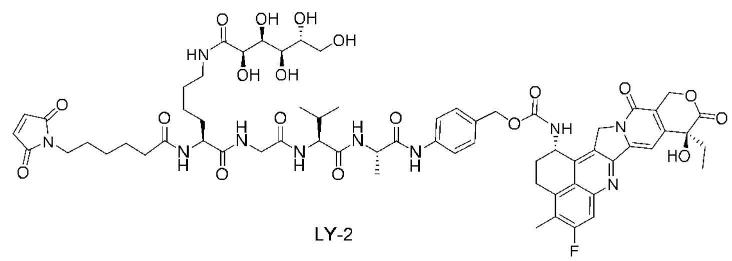

LY-2

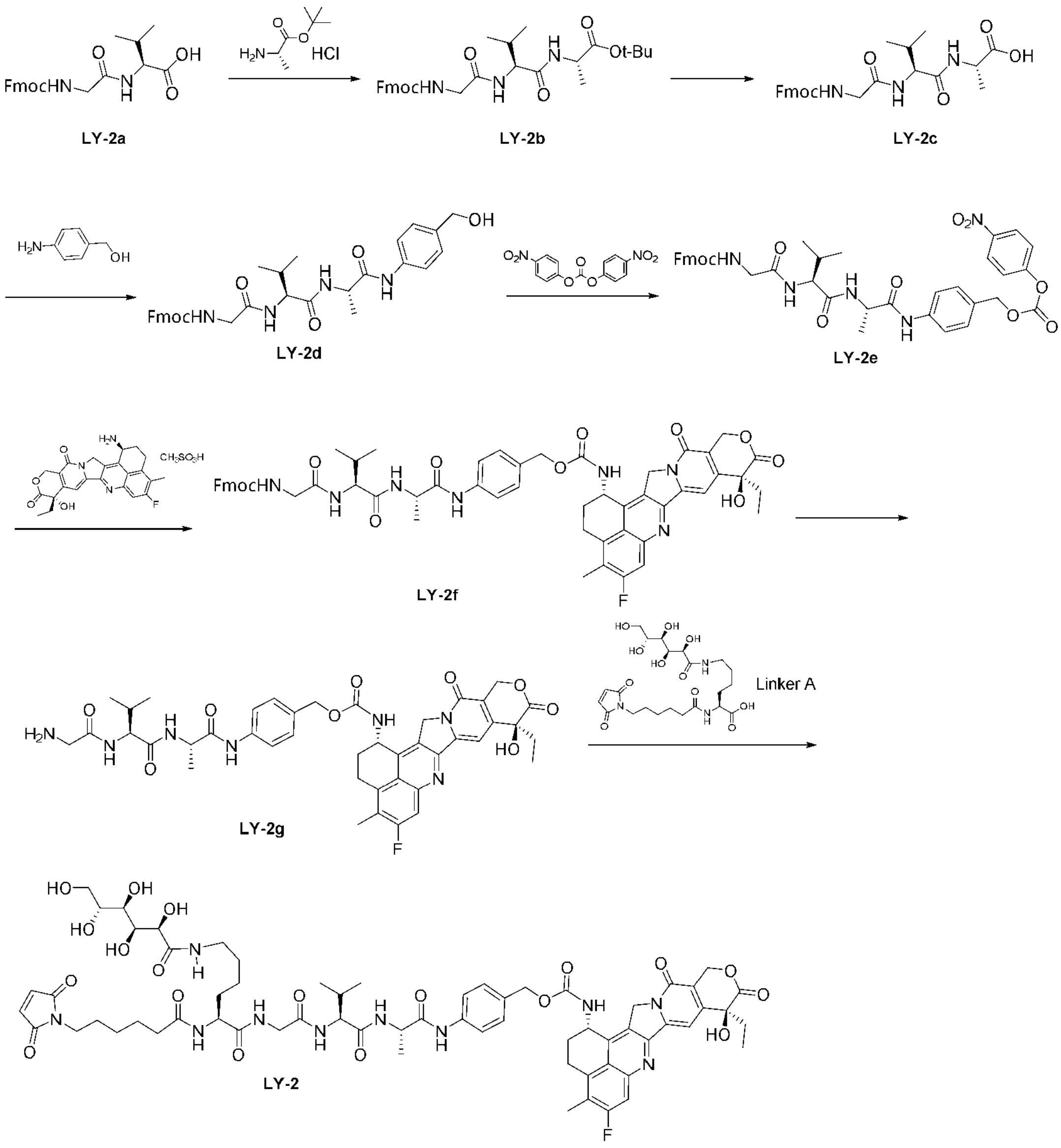


LY-2a LY-2b LY-2c LY-2d LY-2e LY-2f LY-2g LY-2

Step 1: Preparation of ((9H-fluoren-9-yl)methoxy)carbonyl)glycyl-L-valeryl-L-alanine tert-butyl ester **(LY-2b)**

(((9H-fluoren-9-yl)methoxy)carbonyl)glycyl-L-valine **(LY-2a)** (8g, 20.2 mmol) and tert-butyl 2-aminoacetate (3.66g, 20.2 mmol) were added to a 250 mL three-necked flask, DMF (80 mL) was added, DIEA (7.8g, 60.6 mmol) was added dropwise in a nitrogen atmosphere, the resulting solution was stirred for 5 minutes after addition, the solution of HATU (9.2g, 24.2 mmol) in DMF (30 mL) was added dropwise, and after addition, the reaction solution was stirred at room temperature for 1 hour. The reaction solution was slowly added into water (800 mL), the resulting solution was stirred for 30 minutes and filtered, the solid was dissolved with DCM (200 mL), the resulting solution was washed with water once, and the organic phase was dried with anhydrous sodium sulfate, filtered, and concentrated to obtain 9.5g of yellow oily substance with yield of 90%.

$^1$H NMR (400 MHz, DMSO) δ 8.32 (d, *J* = 6.7 Hz, 1H), 7.88 (d, *J* = 7.5 Hz, 2H), 7.70 (d, *J* = 7.3 Hz, 3H), 7.51 (t, *J* = 6.0 Hz, 1H), 7.41 (t, *J* = 7.4 Hz, 2H), 7.32 (t, *J* = 7.4 Hz, 2H), 4.19 (m , 5H), 3.66 (d, *J* = 5.1 Hz, 2H), 1.95 (m, 1H), 1.37 (s, 9H), 1.23 (d, *J* = 7.2 Hz, 3H), 0.85 (dd, *J* = 18.7, 6.8 Hz, 6H).

Step 2: Preparation of ((9H-fluoren-9-yl)methoxy)carbonyl)glycyl-L-valyl-L-alanine **(LY-2c)**

((9H-fluoren-9-yl)methoxy)carbonyl)glycyl-L-valeryl-L-alanine tert-butyl ester (**LY-2b**) (6g, 11.47 mmol) was dissolved in DCM (30 mL), TFA (20 mL) was added dropwise in a nitrogen atmosphere, and after addition, the reaction solution reacted at room temperature for 2 hours. The reaction solution was concentrated until the volume was small, saturated sodium bicarbonate solution was added to adjust pH to 7-8, DCM (100 mL) was added to extract impurities, pH was adjusted to 1-2 with 1N HCl, EA (200 mL × 2) was used for extraction, the organic phase was washed with water (100 mL × 3), the organic phase was dried with anhydrous sodium sulfate, filtered, and concentrated under reduced pressure to obtain 4.8g of white solid with yield of 89%.

$^1$H NMR (400 MHz, DMSO) δ 7.88 (d, *J* = 7.5 Hz, 2H), 7.70 (d, *J* = 7.4 Hz, 2H), 7.41 (t, *J* = 7.4 Hz, 2H), 7.32 (t, *J* = 7.3 Hz, 2H), 4.21 (m, 5H), 3.65 (s, 2H), 1.92 (m, 1H), 1.26 (d, *J* = 7.3 Hz, 3H), 0.84 (dd, *J* = 20.8, 6.7 Hz, 6H).

Step 3: Preparation of (9H-fluoren-9-yl)methyl(2-((S)-1-((S)-1-((4-(hydroxymethyl)phenyl)amino)-1-oxopropane-2-yl) amino)-3-methyl-1-oxobutane-2-yl)amino)-2-oxoethyl)carbamate **(LY-2d)**

((9H-fluoren-9-yl)methoxy)carbonyl)glycyl-L-valyl-L-alanine (**LY-2c**) (2.5g, 5.35 mmol) and aminobenzyl alcohol (645 mg, 5.24 mmol) were added to the reaction flask, DMF (25 mL) was added for dissolution, 2,6-dimethylpyridine (1.72g, 16 mmol) was added in a nitrogen atmosphere, the resulting solution was stirred for 5 minutes after addition, the solution of HATU (2.44g, 6.4 mmol) in DMF (8 mL) was added dropwise, and after addition, the reaction solution reacted at room temperature for 1 hour. The reaction solution was added to water (200 mL), EA (200 mL × 2) was used for extraction, the organic phase was washed with dilute hydrochloric acid and sodium bicarbonate, dried with anhydrous sodium sulfate, filtered, and concentrated under reduced pressure to obtain 2.4g of a near white solid with yield of 80%.

$^1$H NMR (400 MHz, DMSO) δ 7.90 (d, *J* = 7.5 Hz, 2H), 7.70 (d, *J* = 7.5 Hz, 2H), 7.55 (d, *J* = 8.3 Hz, 2H), 7.43 (t, *J* = 7.4 Hz, 2H), 7.33 (t, *J* = 7.4 Hz, 2H), 7.24 (d, *J* = 8.3 Hz, 2H), 4.48 - 4.35 (m, 3H), 4.25 (dd, *J* = 21.4, 6.6 Hz, 4H), 3.70 (d, *J* = 2.4 Hz, 2H), 2.00 - 1.95 (m, 1H), 1.33 (d, *J* = 7.1 Hz, 3H), 0.87 (dd, *J* = 19.4, 6.8 Hz, 6H).

Step 4: Preparation of (9H-fluoren-9-yl)methyl(2-((S)-3-methyl-1-((S)-1-((4-(nitrophenoxy)carbonyl)oxy)methyl)phenyl) amino)-1-oxopropane-2-yl)amino)-1-oxobutan-2-yl)amino)-2-oxoethyl)carbamate (**LY-2e**)

(9H-fluoren-9-yl)methyl(2-((S)-1-((S)-1-((4-(hydroxymethyl)phenyl)amino)-1-oxopropane-2-yl)amino)-3-methyl-1-oxobutane-2-yl)amino)-2-oxoethyl)carbamate **(LY-2d)** (1g, 1.74 mmol) and bis(4-nitrophenyl)carbonate (0.8g, 2.62 mmol) were dissolved in DMF (10 mL), DIEA (563 mg, 4.37 mmol) was added dropwise in the nitrogen atmosphere, and after addition, the reaction solution reacted at room temperature for 2 hours. The reaction solution was added to water (50 mL), EA (100 mL × 2) was used for extraction, the organic phase was dried with anhydrous sodium sulfate, filtered, and concentrated under reduced pressure, and the residue was purified by silica gel column chromatography (PE: EA = 10:1), to obtain 1g of near white solid with yield of 78%.

$^1$H NMR (400 MHz, DMSO) δ 9.96 (s, 1H), 8.35 - 8.23 (m, 3H), 7.87 (m, 3H), 7.62 (m, 7H), 7.41 (t, *J* = 6.8 Hz, 4H), 7.32 (t, *J* = 7.0 Hz, 2H), 5.23 (s, 2H), 4.45 - 4.33 (m, 1H), 4.22 (m, 4H), 3.70 (s, 2H), 1.99 (m, 1H), 1.33 (m, 3H), 0.86 (dd, *J* = 19.9, 6.8 Hz, 6H).

Step 5: Preparation of (9H-fluoren-9-yl)methyl(2-((S)-1-((S)-1-((4-(((((1S,9S)-9-ethyl-5-fluoro-9-hydroxy-4-methyl-10,13-dioxo-2,3,9,10,13,15-hexahydro-1H,12H-benzo[de]pyrano[3',4:6,7]indolizino[1,2-b]quinolin-1-yl)carbamoyl)oxy)methyl)phenyl)amino)-1-oxopropane-2-yl)amino)-3-methyl-1-oxobutan-2-yl)amino)-2-oxoethyl)carbamate **(LY-2f)**

(9H-fluoren-9-yl)methyl(2-((S)-3-methyl-1-((S)-1-((4-(nitrophenoxy)carbonyl)oxy)methyl)phenyl)amino)-1-oxopropane-2-yl)amino)-1-oxobutan-2-yl)amino)-2-oxoethyl)carbamate **(LY-2e)** (415 mg, 0.54 mmol) and exatecan mesylate (300 mg, 0.56 mmol) were dissolved in DMF (2 mL), HOBt (73 mg, 0.54 mmol), pyridine (425 mg, 5.38 mmol) and DIPEA (208 mg, 1.61 mmol) were added. The reaction solution was stirred overnight at room temperature to obtain **LY-2f.** The reaction solution was directly used for the next step.

Step 6: Preparation of compound **(LY-2g)**

Piperidine (92 mg, 1.08 mmol) was added to the reaction solution in step 5, and the resulting solution was stirred

at room temperature for 8 hours. The reaction solution was purified by preparative high performance liquid chromatograph (Oriendo, BRIX-2860; column: Phenomenex Luna C18 250 × 50 mm × 10 μm; mobile phase: water (0.225% HCOOH)-acetonitrile, eluted from 45% to 85%), to obtain compound **LY-2g** (81.0 mg, yield: 18.5%) as a white solid, which was directly used for the next step.

LCMS (ESI): m/z, 834.4 $[M+Na]^+$, 812.5 $[M+H]^+$.

Step 7: Preparation of compound **(LY-2)**

HATU (2.83 mg, 0.0074 mmol, 1.2 eq), DIPEA (2.4 mg, 0.019 mmol, 3.0 eq) and compound **LY-2g** (5 mg, 0.0062 mmol, 1 eq) were added to the solution of linker **A** (3.9 mg, 0.0074 mmol, 1.2 eq) in anhydrous tetrahydrofuran (2 mL). The reaction solution was stirred for 2 hours at 60°C. The reaction solution was isolated by preparative high performance liquid chromatograph (Oriendo, BRIX-2860; column: Phenomenex Luna C18 250 × 50 mm × 10 um; mobile phase: water (0.225% HCOOH)-acetonitrile, eluted from 45% to 85%) and lyophilized, to obtain compound **LY-2** (2.0 mg, yield: 24.6%) as a yellow solid.

LCMS (ESI): m/z, 1333.5 $[M+Na]^+$, 1311.6 $[M+H]^+$, 656.4 $[1/2M+H]^+$.

$^1H$ NMR (400 MHz, DMSO-$d^6$) δ 9.91 (s, 1H), 8.25 - 8.14 (m, 2H), 8.06 (d, *J* = 8.8 Hz, 1H), 7.93 (d, *J* = 7.6 Hz, 1H), 7.75 (dd, *J* = 21.2, 9.6 Hz, 2H), 7.65 - 7.55 (m, 3H), 7.36 (d, *J* = 8.2 Hz, 2H), 7.31 (s, 1H), 7.00 (s, 2H), 6.52 (s, 1H), 5.45 (s, 2H), 5.29 (s, 3H), 5.07 (s, 2H), 4.37 (t, *J* = 7.0 Hz, 2H), 4.23 - 4.10 (m, 3H), 3.97 (d, *J* = 3.6 Hz, 1H), 3.90 (s, 1H), 3.73 (d, *J* = 5.6 Hz, 2H), 3.59 - 3.53 (m, 1H), 3.46 (s, 2H), 3.22 (s, 1H), 3.04 (t, *J* = 7.6 Hz, 3H), 2.38 (s, 3H), 2.25 - 2.04 (m, 5H), 2.01 - 1.80 (m, 4H), 1.60 (s, 2H), 1.52 - 1.35 (m, 7H), 1.30 (d, *J* = 7.2 Hz, 3H), 1.19 (dt, *J* = 15.4, 9.0 Hz, 4H), 0.92 - 0.75 (m, 9H).

Example 3: Preparation of compound **LY-3**

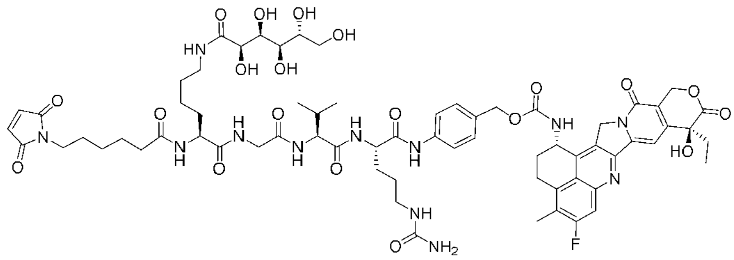

LY-3

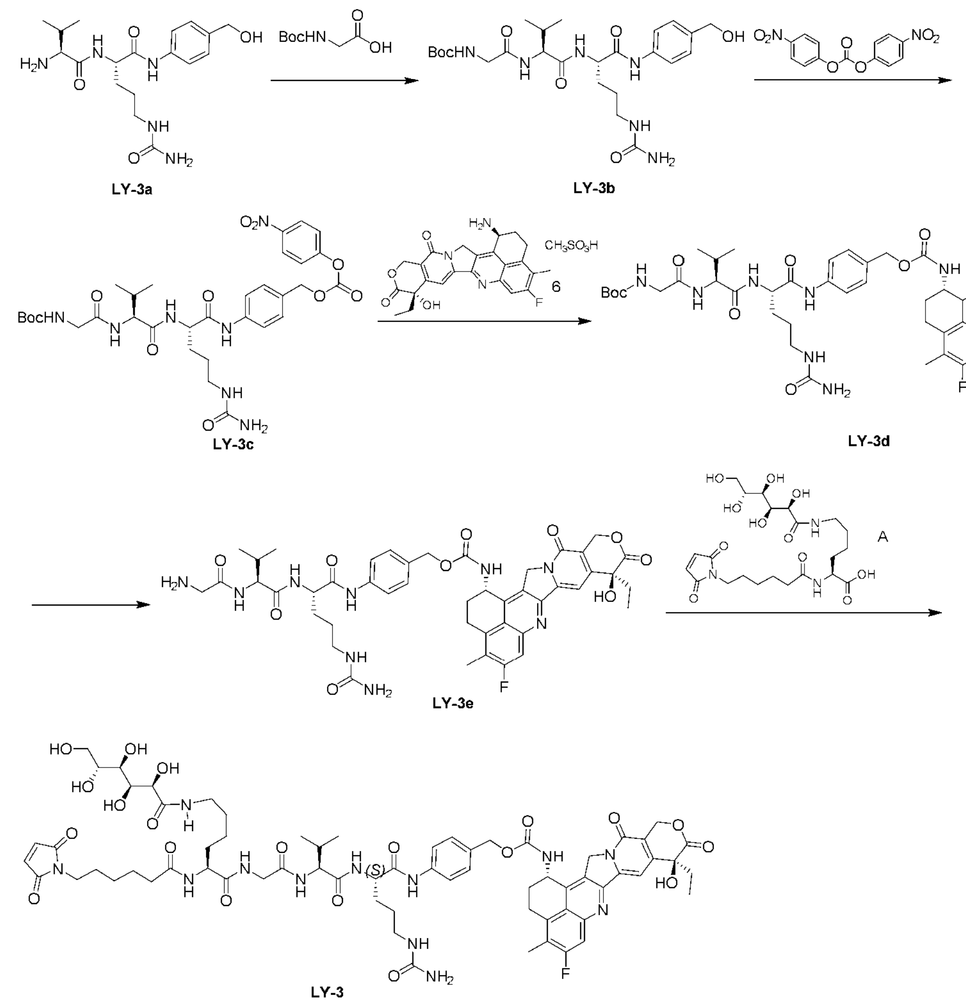

LY-3a LY-3b LY-3c LY-3d LY-3e LY-3

Step 1: Preparation of (2-(S)-1-(S)-1-(4-(hydroxymethyl)phenyl)amino)-1-oxo-5-ureidopentan-2-yl)amino)-3-methyl-1-oxobutane-2-yl)amino)-2-oxoethyl)tert-butyl carbamate **(LY-3b)**

(S)-2-((S)-2-amino-3-methylbutylamido)-N-(4-(hydroxymethyl)phenyl)-5-ureidopentanamide (ChemExpress, **LY-3a)** (300 mg, 0.79 mmol) was dissolved in N,N-dimethylformamide (10 mL) at room temperature, and then Boc-protected glycine (138.5 mg, 0.79 mmol), EDCI (227.7 mg, 1.19 mmoL), HOBT (53.4 mg, 0.40 mmoL) and triethylamine (240 mg, 2.37 mmoL) were added respectively. The reaction solution was stirred for 16 hours at room temperature. The reaction solution was filtered and isolated by preparative high performance liquid chromatograph (Oriendo, BRIX-2860; column: Phenomenex Luna C18 250 × 50 mm × 10 μm; mobile phase: water (0.225% HCOOH)-acetonitrile, eluted from 45% to 85%), to obtain compound **LY-3b** (300 mg, yield: 69.04%) as a white solid.

LCMS: $[M+H]^+$ = 537.3.

$^1H$ NMR (400 MHz, DMSO-d6) δ 9.84 (s, 1H), 8.32 - 8.04 (d, J = 7.6 Hz ,1H), 7.58-7.64 (d, J = 8.6 Hz, 1H), 7.49-7.54 (d, J = 8.8 Hz, 2H), 7.17-7.22 (d, J = 8.8 Hz, 2H), 7.00-7.08 (t, J = 7.6 Hz ,1H), 5.90-6.00 (t, J = 5.6 Hz ,1H), 5.37 (s, 2H), 5.06 (t, J = 6.0 Hz, 1H), 4.37-4.42 (d, J = 5.6 Hz, 2H), 4.30 - 4.36 (m, 1H), 4.19-4.26(m, 1H),3.52-3.58 (m, 2H), 2.85-3.05 (m, 3H),2.05-2.07 (m, 1H),1.85-2.05 (m, 1H), 1.74 - 1.44 (m, 2H), 1.33 (s, 9H), 0.75 - 0.90 (dd, J = 17.6, 6.8Hz,

6H).

Step 2: Preparation of (2-(((S)-3-methyl-1-((S)-1-((4-((4-nitrophenoxy)carbonyl)oxy)methyl)phenyl)amino)-1-oxo-5-ureidopentan-2-yl)amino)-1-oxobutane-2-yl)amino)-2-oxoethyl)tert-butyl carbamate **(LY-3c)**

(2-(S)-1-(S)-1-(4-(hydroxymethyl)phenyl)amino)-1-oxo-5-ureidopentan-2-yl)amino)-3-methyl-1-oxobutane-2-yl)amino)-2-oxoethyl)tert-butyl carbamate **(LY-3b)** (250 mg, 0.466 mmoL) was dissolved in N,N-dimethylformamide (10 mL) at room temperature, 4-nitrocarbonate (383.45 mg, 0.932 mmoL) and DIEA (180.3 mg, 1.4 mmoL) were added, and the reaction solution was stirred at room temperature for 16 hours. The reaction solution was filtered and isolated by preparative high performance liquid chromatograph (Oriendo, BRIX-2860; column: Phenomenex Luna C18 250 × 50 mm × 10 μm; mobile phase: water (0.225% HCOOH)-acetonitrile, eluted from 45% to 85%), to obtain compound **LY-3c** (200 mg, yield: 61.18%) as a yellow solid.
LCMS: $[M+H]^+$ = 702.3.

Step 3: Preparation of compound **(LY-3d)**

The compound **(LY-3c)** (200 mg, 0.285 mmoL) was dissolved in N,N-dimethylformamide (10 mL) at room temperature, then exatecan mesylate (148.9 mg, 0.342 mmoL), HOBT (19.26 mg, 0.143 mmoL) and DIEA (110.3 mg, 0.855 mmoL) were separately added, and the reaction solution was stirred at room temperature for 8 hours. The reaction solution was filtered and isolated by preparative high performance liquid chromatograph (Oriendo, BRIX-2860; column: Phenomenex Luna C18 250 × 50 mm × 10 μm; mobile phase: water (0.225% trifluoroacetic acid)-acetonitrile, eluted from 45% to 85%), to obtain compound **LY-3d** (200 mg, yield: 70.31%) as a white solid.
LCMS: $[M+H]^+$ = 998.60.

Step 4: Preparation of compound **(LY-3e)**

The compound **LY-3d** (200 mg, 0.2 mmoL) was added to 10% trifluoroacetic acid/dichloromethane solution (15 mL) at 0°C and the reaction solution was stirred at 0°C for 4 hours. The reaction solution was concentrated under reduced pressure, and the residue was isolated and purified by the preparative HPLC (mobile phase: acetonitrile/water; acidic system: 0.1% HCl), to obtain the compound **LY-3e** (120 mg, yield: 66.69%) as the yellow solid.
LCMS: $[M+H]^+$ = 898.3.

Step 5: Preparation of compound **(LY-3)**

The compound **LY-3e** (120 mg, 0.133 mmoL), the linker **A** (83 mg, 0.160 mmoL), and HATU (61 mg, 0.160 mmoL) were separately dissolved in N,N-dimethylformamide (6 mL) at room temperature, and then DIEA (51.72 mg, 0.4 mmoL) was added to the reaction solution. The reaction solution was stirred for 1 hour at 60°C. The reaction solution was filtered and isolated by preparative high performance liquid chromatograph (Oriendo, BRIX-2860; column: Phenomenex Luna C18 250 × 50 mm × 10 μm; mobile phase: water (1% formic acid)-acetonitrile, eluted from 45% to 85%), to obtain compound **LY-3** (74.4 mg, yield: 39.84%) as a yellow solid.
LCMS: $[M/2+2H]^+$ = 699.4.
$^1$H NMR (400 MHz, DMSO-$d_6$) δ 9.92 (s, 1H), 8.18-8.08 (m, 2H), 8.02 (d, *J* = 8.5 Hz, 1H), 7.90 (d, *J* = 6.0 Hz, 1H), 7.73 (d, *J* = 10.9 Hz, 1H), 7.68 (d, *J* = 9.0 Hz, 1H), 7.57 (m, 3H), 7.32 (d, *J* = 7.2 Hz, 2H), 7.27 (s, 1H), 6.96 (s, 2H), 5.97 (s, 1H), 5.41 (s, 2H), 5.24 (m, 3H), 5.04 (s, 2H), 4.33 (m, 1H), 4.19 (m, 1H), 4.13 - 4.06 (m, 1H), 3.94 (d, *J* = 4 Hz 1H), 3.86 (m, 1H), 3.70 (m, 3H), 3.31-3.17 (m, 9H), 3.13-2.83 (m, 7H), 2.33 (m, 3H), 2.22 - 1.02 (m, 25H), 0.97 - 0.67 (m, 10H).

Example 4: Preparation of compound **LY-5**

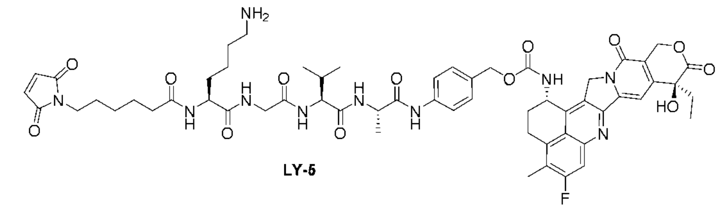

LY-5

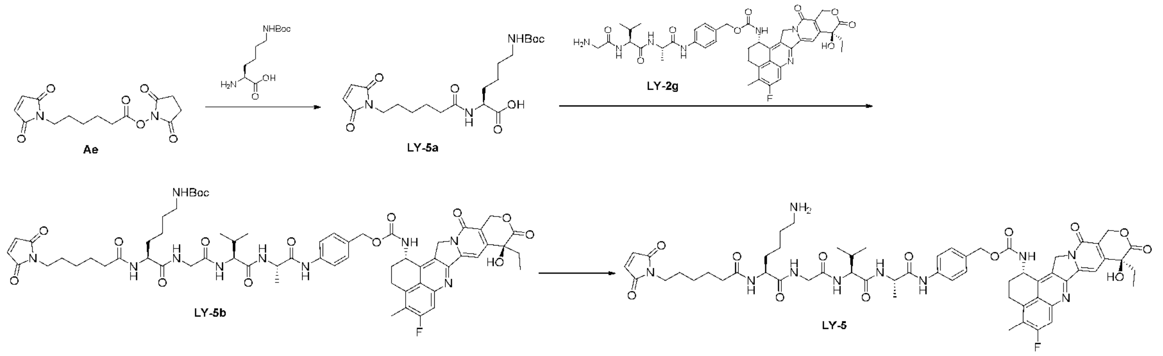

Ae, LY-5a, LY-2g, LY-5b, LY-5

Step 1: Preparation of $N^6$-(tert-butoxycarbonyl)-$N^2$-(6-(2,5-dioxo-2,5-dihydro-1H-pyrrol-1-yl)hexanoyl)-L-lysine **(LY -5a)**

$N^6$-(tert-butoxycarbonyl)-L-lysine (300 mg, 1.0 eq), 2,5-dioxopyrrolidin-1-yl-6-(2,5-dioxo-2,5-dihydro-1H-pyrrol-1-yl)hexanoate **(Ae)** (375 mg, 1.0 eq) and DIPEA (315 mg, 2 eq) were added to the reaction flask in the nitrogen atmosphere, then 10 mL of anhydrous DMF was added, nitrogen displacement was performed three times, and the reaction solution reacted at room temperature for 16 hours. The reaction solution was isolated by preparative high performance liquid chromatograph (Oriendo, BRIX-2860; column: Phenomenex Luna C18 250 × 50 mm × 10 μm; mobile phase: water (0.225% trifluoroacetic acid)-acetonitrile, eluted from 45% to 85%), to obtain 400 mg of a colorless colloidal compound **LY-5a.**

LCMS (ESI): m/z, 340.2 $[M-100+1]^+$, 462.3 $[M+Na]^+$.

Step 2: Preparation of compound **(LY-5b)**

The compound **LY-5a** (6.33 mg, 1.3 eq), HATU (5 mg, 1.2 eq), the compound **LY-2g** (9 mg, 1.0 eq), and DIPEA (4.3 mg, 3 eq) were added to the reaction flask in the nitrogen atmosphere, and then 3 mL of DMF was added. The resulting solution reacted at room temperature for 2 hours in the nitrogen atmosphere. The reaction solution was isolated by preparative high performance liquid chromatography (column: Phenomenex Luna C18 250 × 50 mm × 10 μm; mobile phase: water (0.225% HCOOH)-acetonitrile, eluted from 45% to 85%), to obtain 2 mg of compound **LY-5b** as a yellow solid.

LCMS (ESI): m/z, 1255.5 $[M+Na]^+$, 1233.8 $[M+H]^+$.

Step 3: Preparation of compound **(LY-5)**

The compound **LY-5b** (2 mg, 1.0 eq) was added to the reaction flask, then 3 mL of DCM and 0.3 mL of TFA was added, and the resulting solution reacted for 1 hour at 15°C. The reaction solution was concentrated under reduced pressure, 5 mL of DCM was added, the resulting solution was concentrated under reduced pressure, and the residue was isolated by preparative HPLC (Oriendo, BRIX-2860; column: Phenomenex Luna C18 250 × 50 mm × 10 μm; mobile phase: water (0.225% HCOOH)-acetonitrile, eluted from 45% to 85%), to obtain 0.61 mg of compound **LY-5** as a white solid.

LCMS (ESI): m/z, 1155.6 $[M+Na]^+$, 1133.6 $[M+H]^+$.

$^1H$ NMR (400 MHz, DMSO-$d^6$) δ $^1H$ NMR (400 MHz, DMSO-$d^6$) δ 9.92 (s, 1H), 8.22 (d, *J* = 6.8 Hz, 1H), 8.16 (s, 1H), 8.01 (dd, *J* = 39.6, 8.2 Hz, 3H), 7.79 (d, *J* = 10.4 Hz, 2H), 7.59 (d, *J* = 8.2 Hz, 4H), 7.36 (d, *J* = 8.2 Hz, 2H), 7.31 (s, 1H), 7.00 (s, 2H), 6.53 (s, 1H), 5.45 (s, 2H), 5.29 (s, 2H), 5.08 (s, 2H), 4.38 (t, *J* = 7.0 Hz, 2H), 4.20 (d, *J* = 7.7 Hz, 2H), 3.74 (d, *J* = 5.7 Hz, 2H), 2.78-2.72 (m, 3H), 2.68-2.65 (m, 2H), 2.38 (s, 3H), 2.34-2.32 (m, 3H), 2.20 (d, *J*= 10.4 Hz, 2H), 2.10 (t, *J* = 7.2 Hz, 2H), 1.98 (q, *J* = 6.8 Hz, 2H), 1.88 (dd, J = 15.6, 7.2 Hz, 3H), 1.52-1.48 (m, 4H), 1.31 (d, *J* = 7.2 Hz, 3H), 1.18 (t, *J* = 7.8 Hz, 2H), 0.89-0.86 (m, 4H), 0.82 (d, *J* = 6.8 Hz, 3H).

Example 5: Preparation of compound **LY-6**

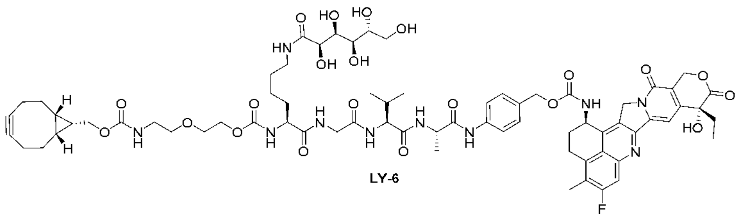

LY-6

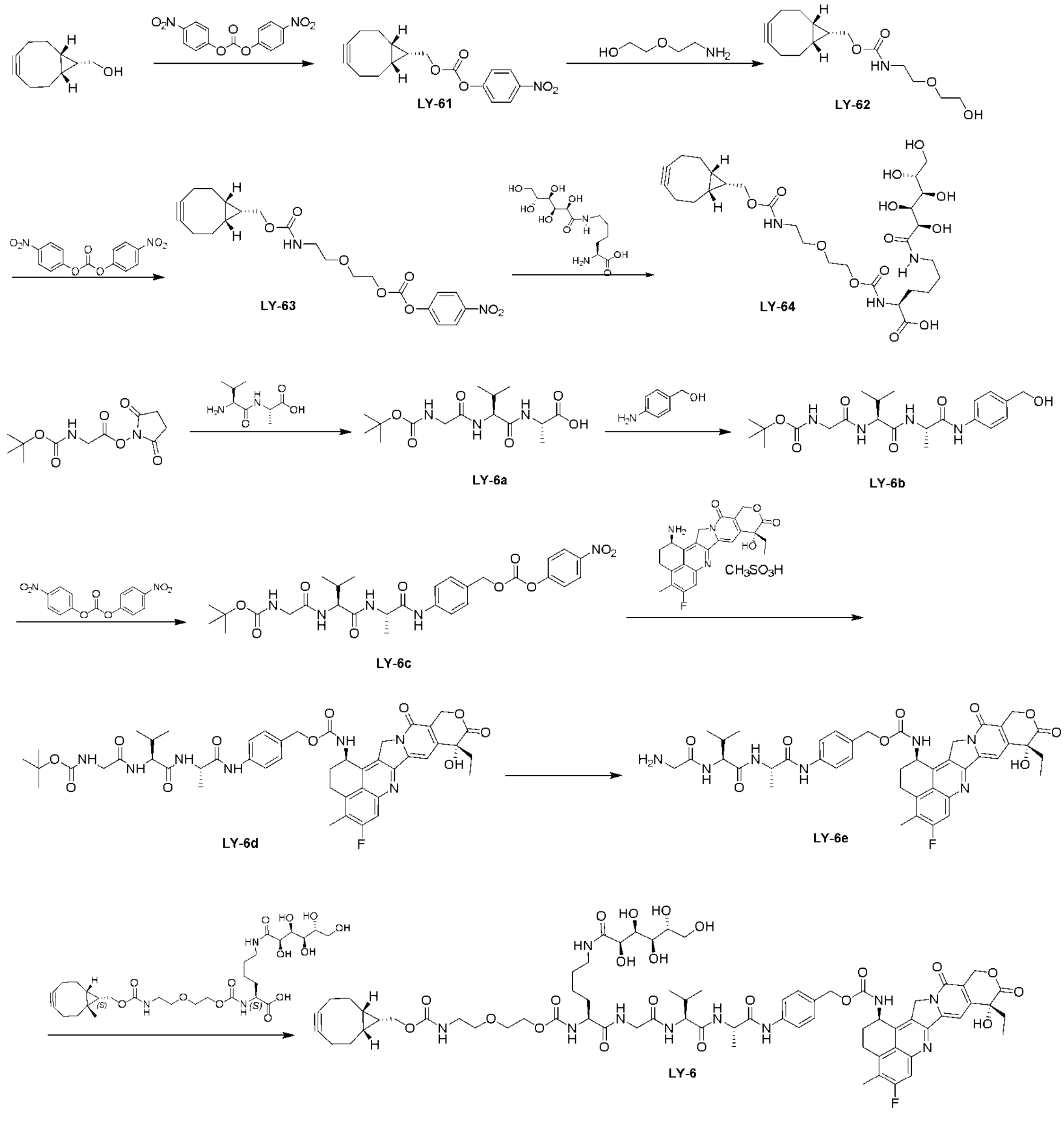


Step 1: Preparation of ((1R,8S,9s)bicyclo[6.1.0]non-4-yn-9-yl)methyl (4-nitrophenyl)carboxylate (**LY-61**)

((1R,8S,9s)bicyclo[6.1.0]non-4-yn-9-yl)methanol (BCN) (500 mg, 3.33 mmol) and DIEA (1.3g, 9.99 mmol) were dissolved in DMF (8 mL) and nitrophenyl carbonate (2.03g, 6.66 mmol) was slowly added at room temperature and the reaction solution was stirred overnight. The reaction solution was diluted with 100 mL of water, extracted with EA (30 mL × 3), and concentrated under reduced pressure, and the residue was purified by column chromatography (ethyl acetate: n-hexane = 1:10) to obtain 850 mg of white solid compound **LY-61.**

LCMS: m/z = 338.1 $[M+Na]^+$.

Step 2: Preparation of ((1R,8S,9s)bicyclo[6.1.0]non-4-yn-9-yl)methyl(2-(2-hydroxyethoxy)ethyl)carbamate **(LY-62)**

((1R,8S,9s)bicyclo[6.1.0]non-4-yn-9-yl)methyl (4-nitrophenyl)carboxylate **(LY-61)** (750 mg, 2.5 mmol) and $Et_3N$ (0.78g, 7.5 mmol) were dissolved in DMF (10 mL), ethylene glycolethylamine (0.54g, 5 mmol) was slowly dropwise added at room temperature and the reaction solution was stirred overnight. The reaction solution was diluted with 100 mL of water, extracted with EA (30 mL × 3), dried with anhydrous sodium sulfate, filtered, and concentrated under reduced pressure. The residue was purified by preparative high performance liquid chromatograph (Oriendo, BRIX-2860; column: Phenomenex Luna C18 250 × 50 mm × 10 um; mobile phase: water (0.225% HCOOH)-acetonitrile, eluted from 45% to 85%) and lyophilized, to obtain 265 mg of a compound **LY-62** as a white solid.
LCMS: m/z = 304.2 $[M+Na]^+$.

Step 3: Preparation of ((1R,8S,9s)bicyclo[6.1.0]non-4-yn-9-yl)methyl(2-(2-(4-nitrophenoxy)carbonyl)oxy)ethoxy)carbamate **(LY-63)**

((1R,8S,9s)bicyclo[6.1.0]non-4-yn-9-yl)methyl(2-(2-hydroxyethoxy)ethyl)carbamate **(LY-62)** (500 mg, 1.78 mmol) and DIEA (689 mg, 5.33 mmol) were dissolved in DMF (10 mL), nitrophenyl carbonate (1.08g, 3.55 mmol) was added at room temperature and the reaction solution was stirred overnight. The residue was purified by preparative high performance liquid chromatograph (Oriendo, BRIX-2860; column: Phenomenex Luna C18 250 × 50 mm × 10 um; mobile phase: water (0.225% HCOOH)-acetonitrile, eluted from 45% to 85%), to obtain 295 mg of a compound **LY-63** as a white solid.
LCMS: m/z = 447.2 $[M+1]^+$.

Step 4: Preparation of **LY-64**

((1R,8S,9s)bicyclo[6.1.0]non-4-yn-9-yl)methyl(2-(2-(4-nitrophenoxy)carbonyl)oxy)ethoxy)carbamate **(LY-63)** (446 mg, 1.0 mmol) and DIEA (689 mg, 5.33 mmol) were dissolved in DMF (10 mL), $N^6$-((2R,3S,4R,5R)-2,3,4,5,6-pentahydroxyhexanoyl)-L-lysine (1.15g, 3.55 mmol) was added at room temperature and the reaction solution was stirred overnight. The residue was purified by preparative high performance liquid chromatograph (Oriendo, BRIX-2860; column: Phenomenex Luna C18 250 × 50 mm × 10 um; mobile phase: water (0.225% HCOOH)-acetonitrile, eluted from 45% to 85%), to obtain 380 mg of a compound **LY-64** as a white solid.
LCMS: m/z = 632.2 $[M+1]^+$.

Step 5: Preparation of (tert-butoxycarbonyl)glycyl-L-valyl-L-alanine **(LY-6a)**

Sodium bicarbonate (26g, 79 mmol) and L-valyl-L-alanine (5g, 27 mmol) were added to a solution of 2,5-dioxopyrrolidin-1-yl(tert-butoxycarbonyl)glycinate (7.23g, 27 mmol) in THF (100 mL) and water (50 mL), and the reaction solution was stirred for 12 hours at room temperature. The reaction solution was concentrated under reduced pressure and extracted with EA (60 mL), the aqueous phase was adjusted with 2 mol/L dilute HCl to adjust pH to 3-5, and then extracted with EA (15 mL × 3), the organic phases were combined and dried with the anhydrous sodium sulfate, and the filtrate was concentrated under reduced pressure to obtain 9g of compound **LY-6a** as a white solid.
LCMS: m/z = 346.2 $[M+1]^+$.

Step 6: Preparation of (2-((S)-1-((S)-1-((4-(hydroxymethyl)phenyl)amino)-1-oxopropane-2-yl)amino)-3-methyl-1-oxobutane-2-yl)amino)-2-oxoethyl)tert-butyl carbamate **(LY-6b)**

$T_3P$ (14.9g, 46.9 mmol) and aminobenzyl alcohol (3.53g, 29 mmol) were added to a solution of (tert-butoxycarbonyl)glycyl-L-valyl-L-alanine **(LY-6a)** (9g, 26 mmol) in DMF (100 mL) and the reaction solution was stirred for 12 hours at room temperature. The reaction solution was purified by preparative high performance liquid chromatograph (Oriendo, BRIX-2860; column: Phenomenex Luna C18 250 × 50 mm × 10 um; mobile phase: water (0.225% HCOOH)-acetonitrile, eluted from 45% to 85%), to obtain 2g of a compound **LY-6b** as a yellow solid.

Step 7: Preparation of (2-((S)-3-methyl-1-((S)-1-((4-((4-nitrophenoxy)carbonyl)oxy)methyl)phenyl)amino)-1-oxopropane-2-yl)amino)-1-oxobutane-2-yl)amino)-2-oxoethyl)tert-butyl carbamate **(LY-6c)**

DIEA (430.3, 3.33 mmol) and nitrophenyl carbonate (675.3 mg, 2.22 mmol) were added to a solution of (2-((S)-1-((S)-1-((4-(hydroxymethyl)phenyl)amino)-1-oxopropane-2-yl)amino)-3-methyl-1-oxobutane-2-yl)amino)-2-oxoethyl)tert-butyl carbamate **(LY-6b)** in DMF (15 mL),and the reaction solution was stirred for 12 hours at room temperature. The reaction solution was purified by preparative high performance liquid chromatograph (Oriendo, BRIX-2860; column: Phenomenex Luna C18 250 × 50 mm × 10 um; mobile phase: water (0.225% HCOOH)-acetonitrile, eluted from 45% to 85%), to obtain 412 mg of a compound **LY-6c** as a yellow solid.

LCMS: m/z = 638.2 $[M+Na]^+$.

Step 8: Preparation of compound **(LY-6d)**

HOBt (11 mg, 0.5 eq), DIEA (63 mg, 3 eq), and exatecan mesylate (103.6 mg, 1.2 eq) were added to the solution of compound **LY-6c** (100 mg, 1 eq) in DMF (5 mL) and the reaction solution was stirred for 12 hours at room temperature. The reaction solution was purified by preparative high performance liquid chromatograph (Oriendo, BRIX-2860; column: Phenomenex Luna C18 250 × 50 mm × 10 um; mobile phase: water (0.225% HCOOH)-acetonitrile, eluted from 45% to 85%), to obtain 150 mg of a compound **LY-6d** as a yellow solid.

LCMS: m/z = 912.4 $[M+1]^+$.

Step 9: Preparation of compound **(LY-6e)**

TFA (0.1 mL) was added to the solution of compound **LY-6d** (10 mg, 1 eq) in DCM (1 mL) under an ice water bath, and the reaction solution was stirred for 1 hour and concentrated under reduced pressure to obtain 8.9 mg of crude product to be directly used for the next reaction.

LCMS: m/z = 812.4 $[M+1]^+$.

Step 10: Preparation of compound **(LY-6)**

The compound **LY-64** (7 mg, 0.01 mmol), HATU (6.3 mg, 0.015 mmol), and DIEA (4.5 mg, 0.03 mmol) were added to the solution of compound **LY-6e** (8.9 mg, 0.01 mmol) in DMF (2 mL) and the reaction solution was stirred for 1 hour at room temperature. The reaction solution was purified by preparative high performance liquid chromatograph (Oriendo, BRIX-2860; column: Phenomenex Luna C18 250 × 50 mm × 10 um; mobile phase: water (0.225% HCOOH)-acetonitrile, eluted from 45% to 85%), to obtain 4 mg of a compound **LY-6** as a yellow solid.

LCMS: m/z = 713.5 $[M/2+1]^+$.

$^1$H NMR (400 MHz, DMSO-$d^6$) δ 9.86 - 9.96 (m, 1 H), 7.99 - 8.27 (m, 3 H), 7.52 - 7.82 (m, 5 H), 7.27 - 7.45 (m, 4 H), 7.12 (d, *J* = 8.0 Hz, 1H), 6.45 - 6.56 (m, 1 H), 5.42 (d, *J* = 6.4 Hz, 2H), 5.20 - 5.36 (m, 4 H), 5.01 - 5.14 (s, 2H), 4.27 - 4.58 (m, 6 H), 3.81 - 4.09 (m, 6 H), 3.64 - 3.80 (d, *J* = 7.2 Hz, 2H), 3.38 - 3.62 (m, 5 H), 2.94 - 3.20 (m, 7 H), 2.64 - 2.70 (m, 1 H), 2.30 - 2.42 (m, 4 H), 2.05 - 2.29 (m, 8 H), 1.76 - 2.04 (m, 4 H), 1.16 - 1.68 (m, 12 H), 0.70 - 0.94 (m, 11 H).

Example 6: Preparation of compounds **LY-7-P1** and **LY-7-P2**

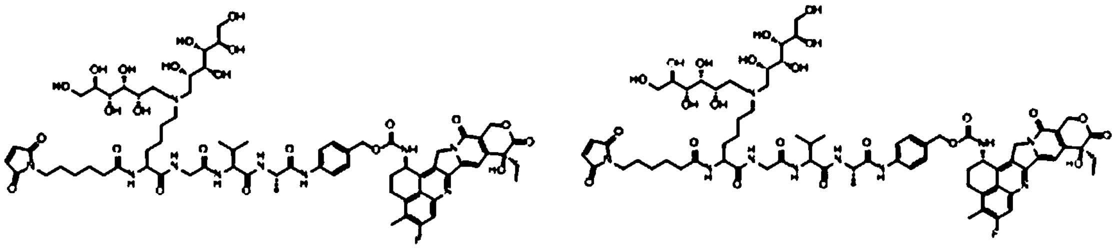

LY-7-P1 and LY-7-P2

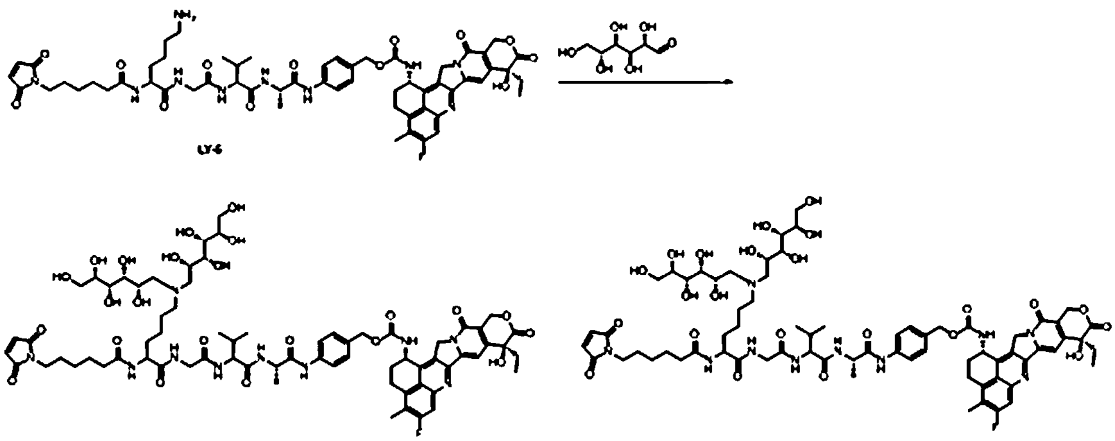

LY-6

LY-7-P1 and LY-7-P2

The compound LY-5 (25 mg, 20 µmol, 1.0 eq, TFA salt) were added to the reaction flask in a nitrogen atmosphere, then MeOH (8 mL)/HOAc (0.8 mL), (2R,3S,4R,5R)-2,3,4,5,6-pentahydroxyhexanal (D-glucose) (108 mg, 0.6 mmol, 30 eq) and $NaBH_3CN$ (19 mg, 0.3 mmol, 15 eq) were added. After addition, the reaction solution reacted at 65°C for 6 hours in a nitrogen atmosphere, compound D-glucose (108 mg, 0.6 mmol, 30 eq) and $NaBH_3CN$ (19 mg, 0.3 mmol, 15 eq) were added again, and then the reaction solution further reacted at 65°C for 16 hours. The reaction solution was concentrated under reduced pressure, and purified by preparative high performance liquid chromatograph (Oriendo, BRIX-2860; column: Phenomenex Luna C18 250 × 50 mm × 10 µm; mobile phase: water (0.225% HCOOH)-acetonitrile, eluted from 45% to 85%), to obtain compounds **LY-7-P1** (5.11 mg, yield: 17.5%) and **LY-7-P2** (3.81 mg, yield: 13%) as white solids (under alkaline conditions, chiral carbon racemization).

**LY-7-P1** (retention time: 2.013 min).

LCMS (ESI): m/z, 731.5 $[M+2]^+/2$, 1483.7 $[M+Na]^+$.

$^1H$ NMR (400 MHz, DMSO-*d*$^6$) δ 9.93 (s, 2H), 8.33 (d, *J* = 7.2 Hz, 1H), 8.23 (d, *J* = 6.8 Hz, 2H), 8.14 (s, 1H), 8.05 (s, 4H), 7.80 - 7.72 (m, 3H), 7.64 - 7.57 (m, 3H), 7.39 - 7.34 (m, 3H), 7.31 (d, *J* = 1.3 Hz, 1H), 6.98 (d, *J* = 3.2 Hz, 2H), 6.53 (s, 2H), 5.44 (s, 3H), 5.29 (d, *J*= 3.5 Hz, 5H), 5.07 (s, 3H), 4.52 (d, *J* = 5.6 Hz, 3H), 4.43 - 4.30 (m, 6H), 4.15 (d, *J* = 12.5 Hz, 4H), 3.74 (d, *J* = 6.8 Hz, 5H), 3.59 (d, *J* = 4.4 Hz, 5H), 2.37 (d, *J* = 2.0 Hz, 4H), 2.12 - 2.07 (m, 3H), 1.87 (dd, *J* = 10.0, 7.2 Hz, 2H), 1.47 (d, *J* = 10.8 Hz, 6H), 1.30 (dd, *J* = 7.2, 5.6 Hz, 4H), 1.23 - 1.16 (m, 4H), 0.90 - 0.81 (m, 12H).

**LY-7-P2** (retention time: 2.070 min).

LCMS (ESI): m/z, 731.4 $[M+2]^+/2$, 1483.5 $[M+Na]^+$.

$^1H$ NMR (400 MHz, DMSO-*d*$^6$) δ 9.92 (s, 2H), 8.33 (d, *J* = 6.8 Hz, 1H), 8.21 (s, 1H), 8.15 (s, 1H), 8.06 (s, 1H), 7.97 (dd, *J* = 15.6, 7.2 Hz, 2H), 7.81 - 7.71 (m, 3H), 7.65 - 7.56 (m, 3H), 7.39 - 7.30 (m, 4H), 6.99 (d, *J* = 3.0 Hz, 2H), 6.53 (s, 1H), 5.45 (s, 3H), 5.28 (s, 3H), 5.08 (s, 2H), 4.51 (d, *J* = 5.6 Hz, 3H), 4.46 - 4.31 (m, 7H), 4.17 (t, *J* = 8.8 Hz, 4H), 3.73 (s, 5H), 3.57 (d, *J* = 11.0 Hz, 5H), 2.38 (d, *J* = 2.0 Hz, 4H), 2.13 - 2.07 (m, 3H), 1.88 (dd, *J* = 15.6, 7.6 Hz, 4H), 1.46 (s, 9H), 1.32 - 1.27 (m, 4H), 1.22 - 1.13 (m, 4H), 0.89-0.80 (m, 12H).

Example 7: Preparation of compound **(LY-8)**

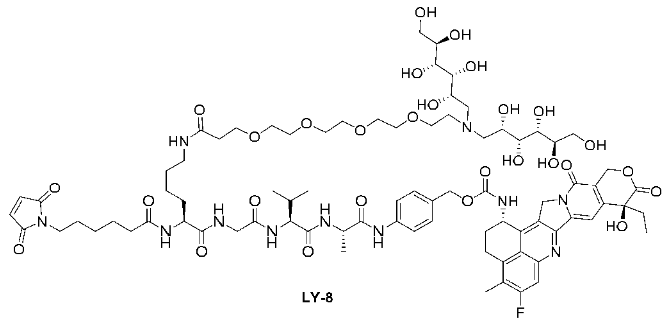

LY-8

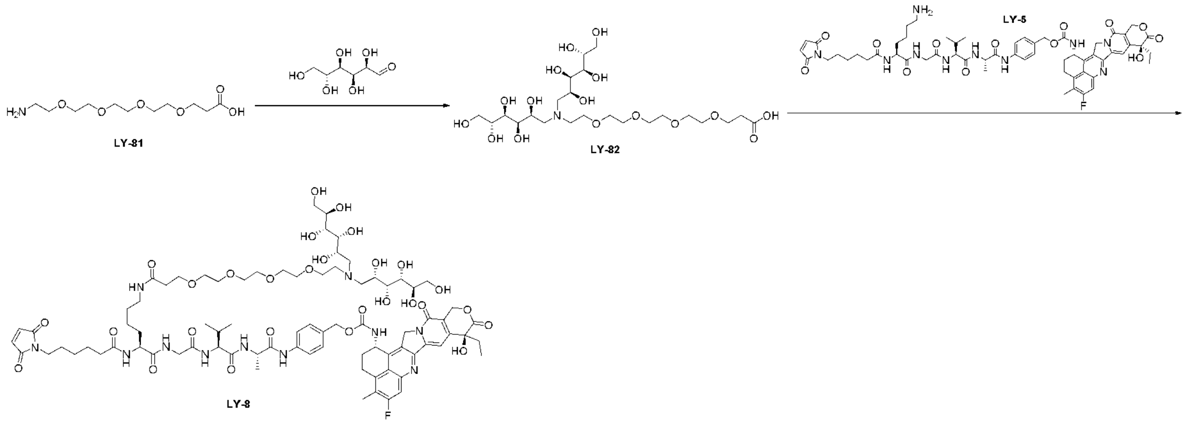

LY-81 LY-82 LY-5 LY-8

Step 1: Preparation of (18S,19R,20R,21R)-18,19,20,21,22-pentahydroxy-16-(2S,3R,4R,5R)-2,3,4,5,6-pentahydroxyhexyl)-4,7,10,13-tetraoxa-16-azadocosanoic acid **(LY-82)**

(2R,3S,4R,5R)-2,3,4,5,6-pentahydroxyhexanal (D-glucose) (1.36g, 7.54 mmol, 10 eq) and $NaBH_3CN$ (237 mg, 3.77 mmol, 5 eq) were added to a solution of 1-amino-3,6,9,12-tetraoxaicosan-15-acetic acid **(LY-81)** (200 mg, 0.75 mmol, 1.0 eq) in anhydrous MeOH (15 mL) and HOAc (1.5 mL), and after addition, the reaction solution reacted at 40°C-45°C for 24 hours in a nitrogen atmosphere. The reaction solution was concentrated under reduced pressure, approximately 8 mL of water and 2 mL of methanol were added to the residue, and the residue was purified by preparative high performance liquid chromatograph (Oriendo, BRIX-2860; column: Phenomenex Luna C18 250 × 50 mm × 10 μm; mobile phase: water (0.225% HCOOH)-acetonitrile, eluted from 45% to 85%) and lyophilized, to obtain the compound **LY-82** (384 mg, yield: 72%, TFA salt) (colorless/off-white gel-like substance).

LCMS (ESI): m/z, 594.5$[M+1]^+$.

$^1H$ NMR (400 MHz, $D_2O$) δ 4.22-4.20 (m, 2H), 3.90-3.88 (m, 2H), 3.87-3.59 (m, 25H), 3.49-3.44 (m, 5H), 2.63 (t, *J* = 8.0 Hz, 2H).

Step 2: Preparation of compound **(LY-8)**

HATU (3.2 mg, 1.2 eq) and DIPEA (3.7 mg, 4 eq) were added to the solution of compound **LY-82** (5 mg, 8.50 μmol, 1.2 eq, TFA salt) and compound **LY-5** (8 mg, 8.50 μmol, 1.0 eq, TFA salt) in anhydrous DMF (2 mL), and the reaction solution was further stirred for 2 hours at room temperature. The reaction solution was purified by preparative high performance liquid chromatograph (Oriendo, BRIX-2860; column: Phenomenex Luna C18 250 × 50 mm × 10 um; mobile phase: water (0.225% HCOOH)-acetonitrile, eluted from 45% to 85%) and lyophilized, to obtain the compound **LY-8** (3.06 mg, yield: 25%) as a white solid.

LCMS (ESI): m/z, 854.9$[M+2]^+/2$, 1730.8 $[M+Na]^+$.

$^1H$ NMR (400 MHz, DMSO-*d*$^6$) δ 9.92 (s, 1H), 8.34 (s, 2H), 8.25 - 8.15 (m, 2H), 8.07 (d, *J* = 8.8 Hz, 1H), 7.94 (d, *J* = 7.2 Hz, 1H), 7.82 - 7.71 (m, 3H), 7.60 (dd, *J* = 11.8, 8.4 Hz, 2H), 7.36 (d, *J* = 8.0 Hz, 2H), 7.31 (s, 1H), 6.98 (d, *J* = 3.4 Hz, 2H), 6.54 (s, 1H), 5.44 (s, 2H), 5.29 (s, 3H), 5.07 (s, 2H), 4.50 (d, *J* = 5.6 Hz, 2H), 4.42 - 4.30 (m, 4H), 4.16 (dq, *J* = 13.8, 7.2 Hz, 3H), 3.76 - 3.47 (m, 28H), 2.98 (d, *J* = 6.7 Hz, 4H), 2.67-2.66 (m, 2H), 2.58 (dd, *J* = 13.2, 4.4 Hz, 3H), 2.37 (d, *J* = 1.8 Hz, 3H), 2.34-2.32 (m, 2H), 2.27 (td, J = 6.8, 2.8 Hz, 3H), 2.21 - 2.04 (m, 5H), 2.00 - 1.81 (m, 5H), 1.60 (d, *J* = 7.8 Hz, 2H), 1.47 (d, *J* = 11.8 Hz, 5H), 1.36 - 1.14 (m, 10H), 0.91 - 0.77 (m, 8H).

Example 8: Preparation of compound **LY-10**

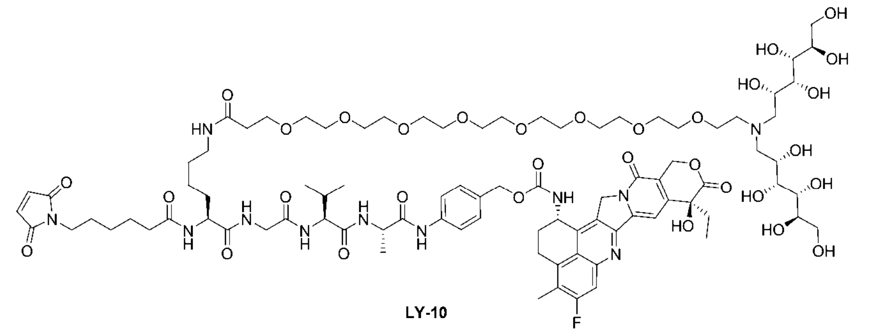

LY-10

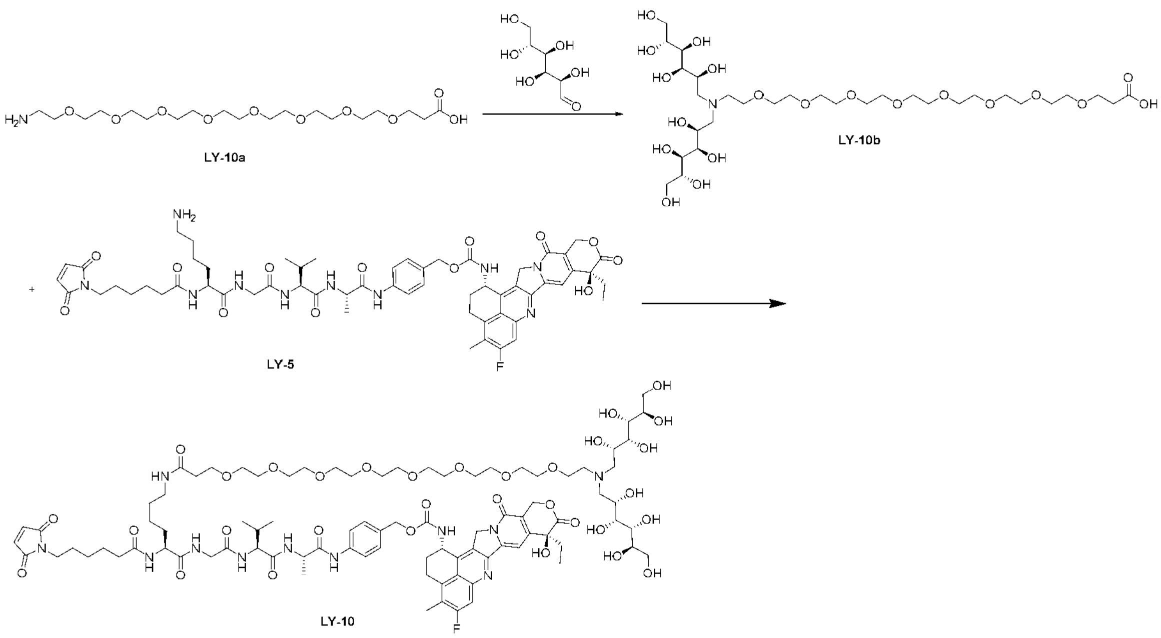

LY-10a

LY-10b

LY-5

LY-10

Step 1: Preparation of (30S,31R,32R,33R)-30,31,32,33,34-pentahydroxy-28-(2S,3R,4R,5R)-2,3,4,5,6-pentahydroxyhexyl)-4,7,10,13,16,19,22,25-octaoxa-28-azagheddic acid **(LY-10b)**

D-glucose (502 mg, 2.79 mmol, 10 eq) and $NaBH_3CN$ (294 mg, 1.39 mmol, 5 eq) were added to a solution of 1-amino-3,6,9,12,15,18,21,24-octaoxaheptane-27-oleic acid **(LY-10a)** (123 mg, 0.28 mmol, 1.0 eq) in methanol (10 mL)/acetic acid (1 mL), and the reaction solution reacted at 40°C for 40 hours. The reaction system was concentrated to approximately 5 mL, 5 mL of methanol and 3 mL of water were added, and the reaction system was purified by preparative high performance liquid chromatography (column: Phenomenex Luna C18 250× 50 mm× 10 μm; mobile phase: water (0.225% HCOOH)-acetonitrile, eluted from 45% to 85%) and lyophilized, to obtain a colorless oily compound **LY-10b** (124 mg, yield: 50.4%, TFA salt).

LCMS (ESI): m/z, 770.7$[M+1]^+$.

$^1H$ NMR (400 MHz, $D_2O$) δ 4 26 (m, 2H), 3.99-3.94 (m, 2H), 3.90-3.78 (m, 12H), 3.78-3.71 (m, 26H), 3.70-3.65 (m, 4H), 3.60-3.56 (m, 5H), 2.70 (t, *J* = 8.0 Hz, 2H).

Step 2: Preparation of compound **(LY-10)**

HATU (7 mg, 18.5 μmol, 2.0 eq), DIEPA (7.2 mg, 55.6 μmol, 6 eq), and compound **LY-5** (10.5 mg, 9.27 μmol, 1.0 eq, TFA salt) were added to the solution of compound **LY-10b** (14.3 mg, 18.53 μmol, 2.0 eq, TFA salt) in DMF (3 mL) under an ice water bath, and after addition, the reaction solution reacted at room temperature for 2 hours. The reaction solution was purified by preparative high performance liquid chromatography (column: Phenomenex Luna C18 250 × 50 mm × 10 um; mobile phase: water (0.225% HCOOH)-acetonitrile, eluted from 45% to 85%), to obtain compound **LY-10** (9.3 mg,

8.29 mg, yield: 40%) as a white solid.

LCMS (ESI): m/z, 943.1[M+2]$^+$/2, 1908.1 [M+Na]$^+$.

$^1$H NMR (400 MHz, DMSO-$d^6$) δ 9.89 (s, 1H), 8.41 - 8.29 (m, 2H), 8.23 - 8.12 (m, 2H), 8.08 - 7.90 (m, 3H), 7.81 - 7.67 (m, 4H), 7.61 (dd, *J* = 11.8, 8.4 Hz, 2H), 7.36 (d, *J* = 8.0 Hz, 2H), 7.31 (s, 1H), 6.99 (d, *J* = 3.2 Hz, 2H), 6.50 (s, 1H), 5.44 (s, 2H), 5.29 (d, *J* = 3.2 Hz, 3H), 5.08 (s, 2H), 4.60 - 4.25 (m, 12H), 4.16-4.13 (m, 4H), 3.73 (d, *J* = 5.6 Hz, 2H), 3.65 (dt, *J* = 8.4, 4.4 Hz, 3H), 3.59-3.57 (m, 7H), 3.50 (d, *J* = 4.2 Hz, 28H), 2.99 (q, *J* = 6.8 Hz, 3H), 2.73 - 2.63 (m, 3H), 2.61 - 2.53 (m, 4H), 2.38 (d, *J* = 1.8 Hz, 3H), 2.33 - 2.24 (m, 3H), 2.10 (td, *J* = 7.2, 4.4 Hz, 3H), 2.00 - 1.84 (m, 4H), 1.53 - 1.42 (m, 5H), 1.35 - 1.16 (m, 9H), 0.86-0.82 (m, 9H).

Example 9: Preparation of compound **LY-21**

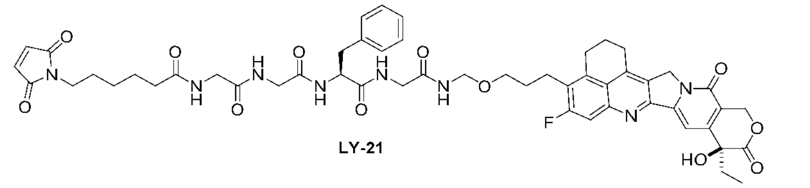

LY-21

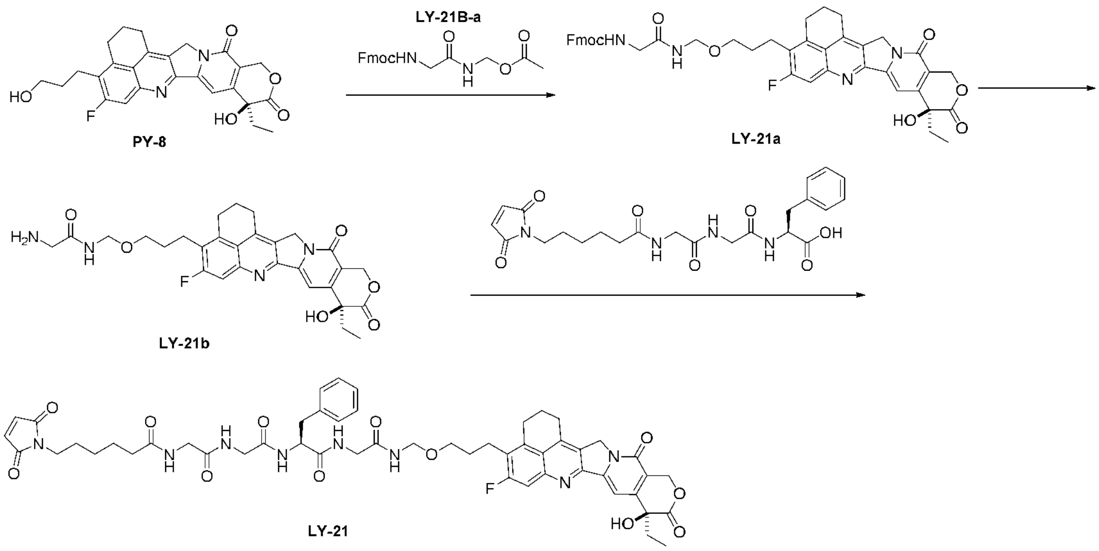


PY-8 LY-21a LY-21b LY-21

Step 1: Preparation of compound **LY-21a**

The compound **PY-8** (50 mg, 0.1 mmol) and (2-((((9H-fluoren-9-yl)methoxy)carbonyl)amino)acetamide)methyl acetate **(LY-21B-a)** (Chengdu Zen Sunda) (79 mg, 0.2 mmol) were dissolved in 3mL of tetrahydrofuran, PPTS (13 mg, 0.05 mmol) was added, and the reaction solution was stirred at 45°C for 12 hours. The reaction solution was subjected to prep-HPLC (preparative chromatograph manufacturer SHIMADZU, model: Lab311-DJ-R2), column: Saga 220 × 50 mm ×10 μm, mobile phase: water (0.225% HCOOH)-acetonitrile, an elution ratio: 21% to 51%), to obtain a yellow solid **LY-21a** (20 mg, yield: 25%).

LCMS: m/z = 733.3 [M+1]$^+$; Rt = 2.133.

Step 2: Preparation of compound **LY-21b**

The compound **LY-21a** (12 mg, 0.015 mmol) was dissolved in 1 mL of DMAc, diethylamine (5 mg, 0.075 mmol) was added, and the reaction solution was stirred at 25°C for two hours. Redundant ethylenediamine was pumped out by an oil pump for 10 minutes to obtain a crude product (10 mg, yield: 85%) as a yellow solid to be directly used for the next reaction.

LCMS: m/z = 551.1 [M+1]$^+$; Rt = 0.657.

Step 3: Preparation of compound **LY-21**

The compound **LY-21b** (10 mg, 0.018 mmol), (6-(2,5-dioxo-2,5-dihydro-1H-pyrrol-1-yl)hexanoyl)glycyl-L-phenylalanine (Chengdu Zen Sunda) (8 mg, 0.018 mmol), PPTS (9 mg, 0.036 mmol) and HOBt (10 mg, 0.074 mmol) were dissolved in DMAc (1 mL), EDCI (4 mg, 0.02 mmol) was added, and the reaction solution was stirred at 25°C for 12 hours. The reaction solution was purified by preparative high performance liquid chromatograph (preparative chromatograph manufacturer: SHIMADZU, model: R5, column: Welch Xtimate C18 250 × 30 mm × 10 μm, mobile phase: (0.225% HCOOH)-acetonitrile, an elution ratio: 21% to 51%), to obtain a yellow solid compound **LY-21** (2.71 mg, yield: 15%).
$^1$H NMR (400 MHz, DMSO-$d_6$) δ 8.53 (s, 2H), 8.31 (s, 1H), 8.16 - 8.02 (m, 3H), 7.72 (d, *J* = 11.2 Hz, 1H), 7.30 (s, 1H), 7.23 (d, *J* = 6.0 Hz, 4H), 7.17 (dd, *J* = 6.0, 2.5 Hz, 1H), 6.98 (s, 2H), 6.51 (s, 1H), 5.43 (s, 2H), 5.23 (s, 2H), 4.57 (d, *J* = 6.8 Hz, 2H), 4.48 (d, *J* = 4.8 Hz, 1H), 3.79 - 3.69 (m, 3H), 3.66 (d, *J* = 5.6 Hz, 2H), 3.58 (dd, *J* = 16.8, 5.4 Hz, 2H), 3.44 (d, *J* = 6.0 Hz, 2H), 3.14 (q, *J* = 5.6 Hz, 4H), 3.04 (d, *J* = 9.6 Hz, 1H), 2.84 (dt, *J* = 19.6, 6.0 Hz, 4H), 2.09 (q, *J* = 9.2, 8.3 Hz, 4H), 1.89 - 1.82 (m, 2H), 1.74 (d, *J* = 8.0 Hz, 2H), 1.46 (q, *J* = 7.6 Hz, 4H), 1.17 (t, *J*= 7.2 Hz, 2H), 0.87 *(t, J*= 7.2 Hz, 3H).

Example 10: Preparation of compound **LY-21B**

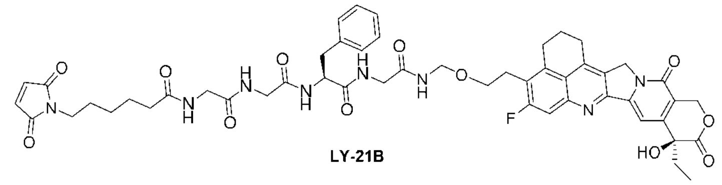

LY-21B

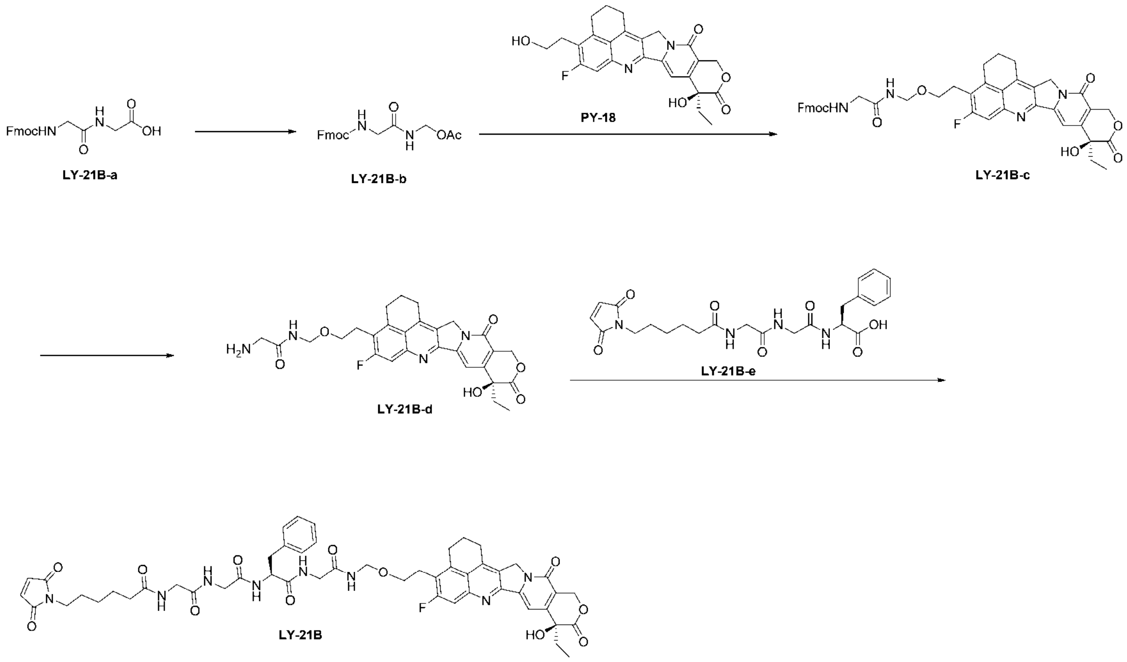


LY-21B-a LY-21B-b LY-21B-c LY-21B-d LY-21B-e LY-21B

Step 1: Preparation of (2-((((9H-fluoren-9-yl)methoxy)carbonyl)amino)acetamide)methyl acetate **(LY-21B-b)**

A solution of compound **LY-21B-a** (5g, 14.11 mmol, 1 eq) and LTA (8.76g, 19.75 mmol, 1.4 eq) in THF (100 mL) and toluene (33 mL) was stirred at 85°C for 2.5 hours in the nitrogen atmosphere. The reaction solution was filtered with diatomite, and after being concentrated, the filtrate was pulped (PE: EA = 8:1), the solution was oily, petroleum ether was added and the reaction solution was further stirred, and the solution became a white suspension. The reaction solution was filtered to obtain a filter cake, and was drained by using the oil pump to obtain the white solid product **LY-21B-b** (5.56g, yield: 99%).
LCMS (ESI): m/z, 391[M+Na]$^+$.
$^1$H NMR (400 MHz, $CHCl_3$-*d*) δ 7.77 (d, *J* = 7.6 Hz, 2H), 7.59 (d, *J* = 7.6 Hz, 2H), 7.44 - 7.38 (m, 2H), 7.32 (td, *J*= 7.4,

1.2 Hz, 2H), 7.11 (d, *J* = 8.0 Hz, 1H), 5.42 (d, *J* = 7.6 Hz, 1H), 5.25 (d, *J* = 7.2 Hz, 2H), 4.45 (d, *J* = 6.8 Hz, 2H), 4.23 (t, *J* = 6.8 Hz, 1H), 3.90 (d, *J* = 5.6 Hz, 2H), 2.06 (s, 3H).

Step 2: Preparation of compound **LY-21B-c**

HCl (59.94 μL, 239.75 μmol, 3 eq, 4M in dioxane) was added to a solution of compounds **LY-21B-b** (44.16 mg, 119.87 μmol, 1.5 eq) and **PY-18** (36 mg, 79.92 μmol, 1 eq) in DMF (3.5 mL) in the nitrogen atmosphere, the reaction solution was stirred at room temperature for 2 hours, then the compound (2-((((9H-fluoren-9-yl)methoxy)carbonyl)amino) acetamide)methyl acetate (50 mg, 135.8 μmol) was added again, and the reaction solution was further stirred overnight. The reaction solution was concentrated and purified by reversed-phase preparative chromatograph (preparative chromatograph manufacturer: Oriendo, model: BRIX-2860, column: YMC-Triart Prep C18 250 × 50 mm × 7 μm, mobile phase: water (0.225% HCOOH)-acetonitrile, an elution ratio of acetonitrile: 35% to 70%), to obtain the product **LY-21B-c** (20.4 mg, yield = 33.6%).

LCMS (ESI): m/z, 759$[M+H]^+$.

Step 3: Preparation of compound **LY-21B-d**

A solution of compound **LY-21B-c** (20.4 mg, 26.88 μmol, 1 eq) and diethylamine (16.6 μL, 161.3 μmol, 6 eq) in DMF was stirred at room temperature for 2 hours. The reaction solution was drained by using the oil pump to obtain the crude product of compound **LY-21B-d** (19 mg).

LCMS (ESI): m/z, 537$[M+H]^+$.

Step 4: Preparation of compound **LY-21B**

A solution of the compound **LY-21B-d** (19 mg, 35.41 μmol, 1 eq), (6-(2,5-dioxo-2,5-dihydro-1H-pyrrol-1-yl) hexanoyl)glycyl-L-phenylalanine **(LY-21B-e)** (Chengdu Zen Sunda) (16.73 mg, 35.41 μmol, 1 eq), PPTS (17.8 mg, 70.82 μmol, 2 eq), HOBt (19.14 mg, 141.64 μmol, 4 eq), and EDCI (7.47 mg, 38.95 μmol, 1.1 eq) in DMA (1.5 mL) were stirred overnight at room temperature in the nitrogen atmosphere, and the reaction solution was purified by preparative high performance liquid chromatograph (preparative chromatograph manufacturer: SHIMADZU, model: LC-20AP, column: YMC-Triart Prep C18 250 × 30 mm × 10 μm, mobile phase: water (0.225% HCOOH)-acetonitrile, an elution ratio of acetonitrile: 20% to 55%), to obtain a white solid **LY-21B** (3.23 mg, 9.2%).

LCMS (ESI): m/z, 991$[M+H]^+$.

$^1$H NMR (400 MHz, DMSO-$d_6$) δ 8.46 (t, *J* = 6.6 Hz, 1H), 8.25 (t, *J* = 5.8 Hz, 1H), 8.10 - 8.05 (m, 2H), 8.00 (t, *J* = 5.6 Hz, 1H), 7.73 (d, *J* = 11.2 Hz, 1H), 7.30 (s, 1H), 7.22 - 7.12 (m, 5H), 6.98 (s, 2H), 6.53 (d, *J* = 7.6 Hz, 1H), 5.43 (s, 2H), 5.21 (s, 2H), 4.55 (d, *J* = 6.6 Hz, 2H), 4.45 (q, *J* = 4.6 Hz, 1H), 3.74 - 3.53 (m, 10H), 3.15 (dd, *J* = 15.6, 6.2 Hz, 4H), 3.11 - 3.06 (m, 2H), 2.98 (dd, *J* = 13.8, 4.4 Hz, 1H), 2.74 - 2.69 (m, 1H), 2.08 (q, *J* = 7.6 Hz, 4H), 1.87 (p, *J* = 6.8 Hz, 2H), 1.46 (h, *J* = 7.2 Hz, 4H), 1.21 - 1.14 (m, 2H), 0.88 (t, *J* = 7.2 Hz, 3H); $^{19}$F NMR (377 MHz, DMSO-$d_6$) δ -112.37.

Example 11: Preparation of compound **LY-21C**

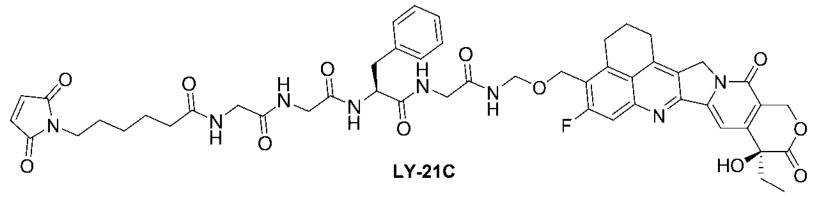

LY-21C

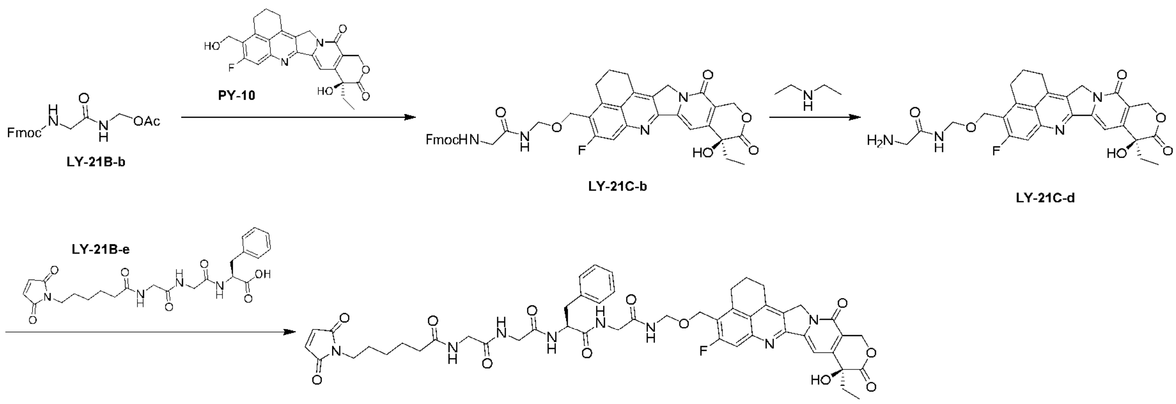


LY-21C

Step 1: Preparation of compound **LY-21C-b**

The compound **PY-10** (195 mg, 98.7%, 440.99 μmol, 1 eq) was added to the solution of compound **LY-21B-b** (243.68 mg, 100%, 661.48 μmol, 1.5 eq) in tetrahydrofuran (20 mL), then p-toluenesulfonic acid (TSOH) (75.94 mg, 70.97 μL, 440.99 μmol, 1 eq) was added. The reaction solution was stirred for 12 hours at 26°C. The reaction solution was concentrated under reduced pressure, and the residue was purified by column chromatography (eluents: dichloromethane: methanol = = 1:0-10:1), and then isolated and purified by preparative high performance liquid chromatograph (preparative chromatograph manufacturer: ISCO, model: ISCO-4, column: Welch Xtimate C18 250 × 50 mm × 10 μm, mobile phase: water (0.225% FA)-ACN, an elution ratio of water: 30% to 60%) and lyophilized, to obtain a white solid product **LY-21C-b** (95 mg, yield: 28.92%).

LCMS (ESI): m/z, 745.2 $[M+H]^+$.

Step 2: Preparation of compound **LY-21C-d**

The compound **LY-21C-b** (13 mg, 15.15 μmol, 1 eq) was dissolved in DMF (6 mL), diethylamine (33.93 mg, 47.79 μL, 463.88 μmol, 3.6367 eq) was added to the solution, and the solution was stirred at 10°C for 2 hours in the nitrogen atmosphere. The reaction solution was concentrated under reduced pressure to obtain the crude product **LY-21C-d** to be directly used for the next step.

LCMS (ESI): m/z, 523.2 $[M+H]^+$.

Step 3: Preparation of compound **LY-21C**

Compounds **LY-21C-d** (65 mg, 124.39 μmol, 1 eq) and **LY-21B-e** (70.53 mg, 149.27 μmol, 1.2 eq) were dissolved in DMF (5 mL), and 1-hydroxy-benzo-triazole (25.21 mg, 16.81 μL, 186.59 μmol, 1.5 eq) and 1-(3-dimethylaminopropyl)-3-ethylcarbodiimide hydrochloride (35.77 mg, 40.79 μL, 186.59 μmol, 1.5 eq) were added sequentially and the reaction solution was stirred for 2 hours. After being filtered, the reaction solution was isolated by reversed-phase preparative chromatograph (preparative chromatograph manufacturer: ISCO, model: ISCO-6, column: Welch Xtimate C18 250 × 30 mm × 10 μm, mobile phase: water (0.225% FA)-ACN, an elution ratio of water: 21% to 51%), to obtain a white solid product **LY-21C** (84.85 mg, yield: 69.81%).

$^1H$ NMR (400 MHz, DMSO-$d_6$) δ 8.73 (t, *J* = 6.7 Hz, 1H), 8.38 (t, *J* = 5.9 Hz, 1H), 8.18 (d, *J* = 8.1 Hz, 1H), 8.07 (dt, *J* = 19.9, 5.8 Hz, 2H), 7.76 (d, *J* = 11.2 Hz, 1H), 7.31 (s, 1H), 7.25 (d, *J* = 4.4 Hz, 4H), 7.19 - 7.15 (m, 1H), 6.99 (s, 2H), 6.55 (s, 1H), 5.44 (s, 2H), 5.26 (s, 2H), 4.69 (d, *J* = 6.5 Hz, 4H), 4.52 - 4.47 (m, 1H), 4.37 (t, *J* = 5.1 Hz, 1H), 3.81 - 3.75 (m, 2H), 3.73 (dd, *J* = 5.9, 2.0 Hz, 1H), 3.66 (d, *J* = 5.7 Hz, 2H), 3.59 (dd, *J* = 16.7, 5.4 Hz, 1H), 3.47 - 3.41 (m, 1H), 3.20 (dd, *J* = 17.1, 5.8 Hz, 3H), 3.10 - 3.03 (m, 1H), 2.82 (dd, *J* = 13.8, 9.7 Hz, 1H), 2.11 - 2.03 (m, 4H), 1.87 (dt, *J* = 15.0, 7.1 Hz, 2H), 1.45 (h, *J* = 7.6 Hz, 4H), 1.21 - 1.12 (m, 2H), 1.05 (t, *J* = 7.0 Hz, 2H), 0.87 (t, *J* = 7.3 Hz, 3H).

LCMS (ESI): m/z, 977.2$[M+H]^+$.

Example 12: Preparation of compound **LY-22**

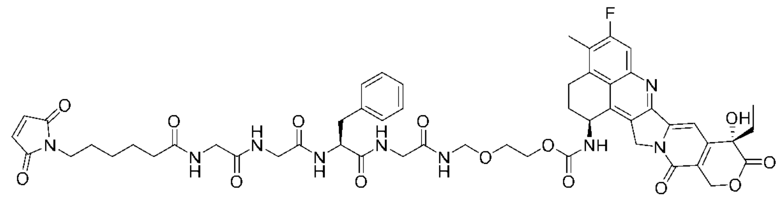

**LY-22**

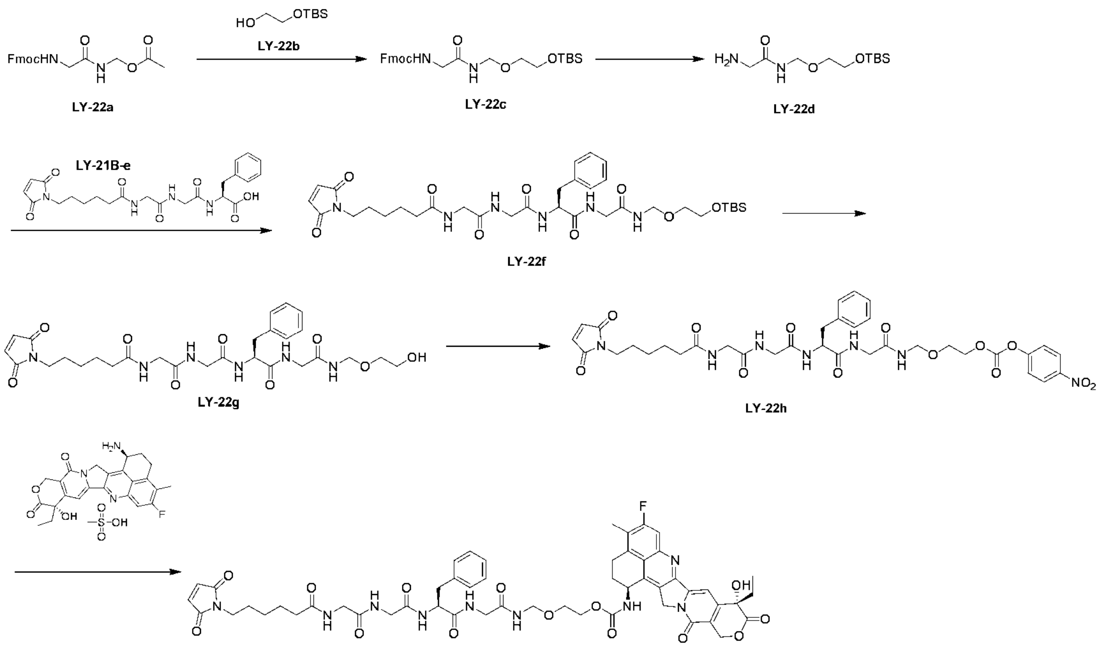


LY-22

Step 1: Preparation of compound **LY-22c**

(2-((((9H-fluoren-9-yl)methoxy)carbonyl)amino)acetamide) acetate (Chengdu Zen Sunda) (3g, 8.14 mmol, 1 eq) was dissolved in tetrahydrofuran (60 mL) in the nitrogen atmosphere, compound 2-((tertbutyldimethylsilyl)oxy) ethane-1-ol (2.87g, 16.29 mmol, 2 eq) was added at room temperature, and a catalytic amount of p-toluenesulfonic acid (140.23 mg, 131.06 μL, 814.35 μmol, 0.1 eq) was further added. The reaction solution was stirred at 20°C-35°C for 2.5 hours. The reaction solution was slowly added into ice water (60 mL), and extracted with ethyl acetate (60 mL × 3), the organic phase was dried with anhydrous sodium sulfate, filtered, and concentrated under reduced pressure, the resulting crude product was purified by silica gel column chromatography, the mobile phase was petroleum ether/ethyl acetate, and the gradient was 1:0-1:2, to obtain the semi-solid compound **LY-22c** (2.10g, yield: 53.21%, purity: 96%) as a white solid.
LCMS (ESI): m/z, 507.3 $[M+Na]^+$.

Step 2: Preparation of compound **LY-22d**

The compound **LY-22c** (2.10g, 96%, 4.16 mmol, 1 eq) was dissolved in DMA (20 mL), DBU (316.62 mg, 310.72 μL, 2.08 mmol, 0.5 eq) was added to the solution, and the solution was stirred at 15°C for 1.5 hours in the nitrogen atmosphere. The reaction solution was directly used for the next step without any treatment.

Step 3: Preparation of compound **LY-22f**

P-toluenesulfonic acid (374.02 mg, 349.55 μL, 2.17 mmol, 0.5 eq) was added to the solution of compound **LY-22d** (1.14g, 4.34 mmol, 1 eq) in DMA (20 mL) at 0°C, then compound **LY-21B-e** (1.85g, 3.91 mmol, 0.9 eq) and HOBt (586.99 mg, 4.34 mmol, 1 eq) were sequentially added, EDCI (832.75 mg, 4.34 mmol, 1 eq) was finally added, and after

addition, the reaction solution was stirred at 0°C-10°C for 2 hours in the nitrogen atmosphere. The reaction solution was slowly added into ice water (60 mL) and extracted with dimethyltetrahydrofuran (60 mL × 2), the organic phase was sequentially washed with saturated sodium bicarbonate (60 mL), saturated sodium chloride (60 mL) and water (60 mL), the organic phase was dried with anhydrous sodium sulfate, filtered, and concentrated under reduced pressure, and the resulting crude product was purified by silica gel column chromatography (Instrument Name: ISCO-4, mobile phases: DCM and MeOH, and gradient: 0:1-10:1, to obtain the compound LY-22f (2.80g, yield: 89.91%, purity: 96%) as a white solid.

LCMS (ESI): m/z, 512.3 (lcms value at a fragment peak).

Step 4: Preparation of compound **LY-22g**

The compound **LY-22f** (1.04g, 96%, 1.39 mmol, 1 eq) was dissolved in THF (10 mL) and reagent TEA.3HF (899.47 mg, 5.58 mmol, 4 eq) was added at 0°C. The reaction solution was stirred for 6 hours at 25°C in the nitrogen atmosphere. The reaction solution was isolated and purified by reversed-phase preparative chromatograph (preparative chromatograph manufacturer: ISCO, model: ISCO-4, column: YMC-Triart Prep C18 250 × 50 mm × 7 μm, mobile phase: water (0.05% $NH_3H_2O$)-acetonitrile, an elution ratio of water: 5% to 20%) and lyophilized, to obtain a white solid product **LY-22g** (600 mg, yield: 71.38%).

LCMS (ESI): m/z, 512.3 (lcms value at a fragment peak).

Step 5: Preparation of compound **LY-22h**

The compound **LY-22g** (300 mg, 497.80 μmol, 1 eq) was dissolved in DMA (20 mL), and NPC (181.72 mg, 597.36 μmol, 1.2 eq) and DIPEA (77.21 mg, 98.73 μL, 597.36 μmol, 1.2 eq) were added to the reaction solution. The reaction solution was stirred for 4 hours at 30°C in the nitrogen atmosphere. The reaction solution was directly used for the next step without treatment.

LCMS (ESI): m/z, 790.3$[M+Na]^+$.

Step 6: Preparation of compound **LY-22**

The compound exatecan mesylate (ChemExpress) (263.10 mg, 494.95 μmol, 1 eq) was dissolved in DMA (2 mL), DIPEA (63.97 mg, 81.81 μL, 494.95 μmol, 1 eq) was added, the reaction solution was stirred at 25°C for 10 minutes, then the reaction solution of compound **LY-22h** (380 mg, 494.95 μmol, 1 eq) was added, then HOBt (33.44 mg, 22.29 μL, 247.48 μmol, 0.5 eq) and pyridine (195.75 mg, 200.16 μL, 2.47 mmol, 5 eq) were added sequentially, and the reaction solution was stirred at 25°C for 3 hours in the nitrogen atmosphere. The reaction solution was directly purified by preparative high performance liquid chromatograph (preparative chromatograph manufacturer: Oriendo, model: BRIX-2860, Phenomenex Luna C18 (250×50 mm×10 μm) was used as a chromatographic column, mobile phase: water (0.225% HCOOH)-acetonitrile, an elution ratio of water: 30% to 60%) and lyophilized, to obtain a white solid product (220 mg, yield: 41.77%).

LCMS (ESI): m/z, 1064.5$[M+H]^+$.

$^1$H NMR (400 MHz, DMSO-$d_6$) δ = 8.52 (t, *J* = 6.7 Hz, 1H), 8.30 (t, *J* = 5.8 Hz, 1H), 8.15 - 7.97 (m, 4H), 7.78 (d, *J* = 10.9 Hz, 1H), 7.31 (s, 1H), 7.28 - 7.12 (m, 5H), 6.99 (s, 2H), 6.52 (s, 1H), 5.43 (s, 2H), 5.25 (s, 3H), 4.62 - 4.55 (m, 2H), 4.48 (s, 1H), 4.18 (s, 2H), 3.78 - 3.53 (m, 8H), 3.23 (s, 2H), 3.15 - 2.99 (m, 3H), 2.78 (dd, *J* = 13.8, 9.7 Hz, 1H), 2.38 (d, *J* = 1.8 Hz, 2H), 2.18 (s, 2H), 2.09 (t, *J* = 7.4 Hz, 2H), 1.86 (dt, *J* = 15.4, 7.0 Hz, 3H), 1.46 (h, *J* = 7.1 Hz, 4H), 1.19 (h, *J* = 7.7 Hz, 2H), 0.87 (t, *J* = 7.3 Hz, 3H).

Example 13: Preparation of compound **LY-22BM1**

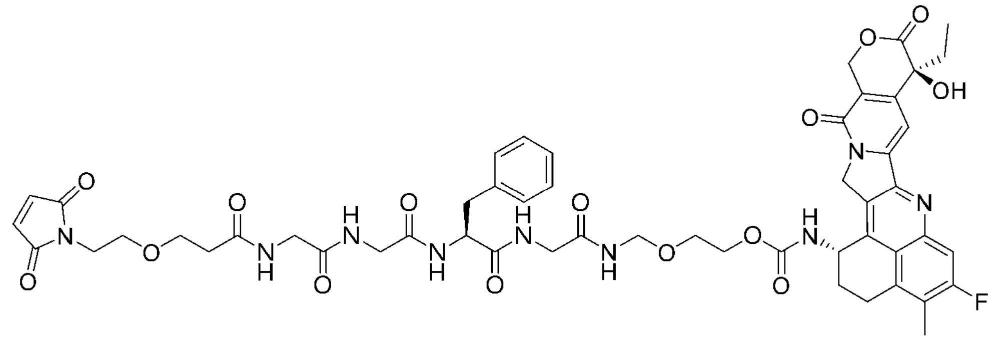

**LY-22BM1**

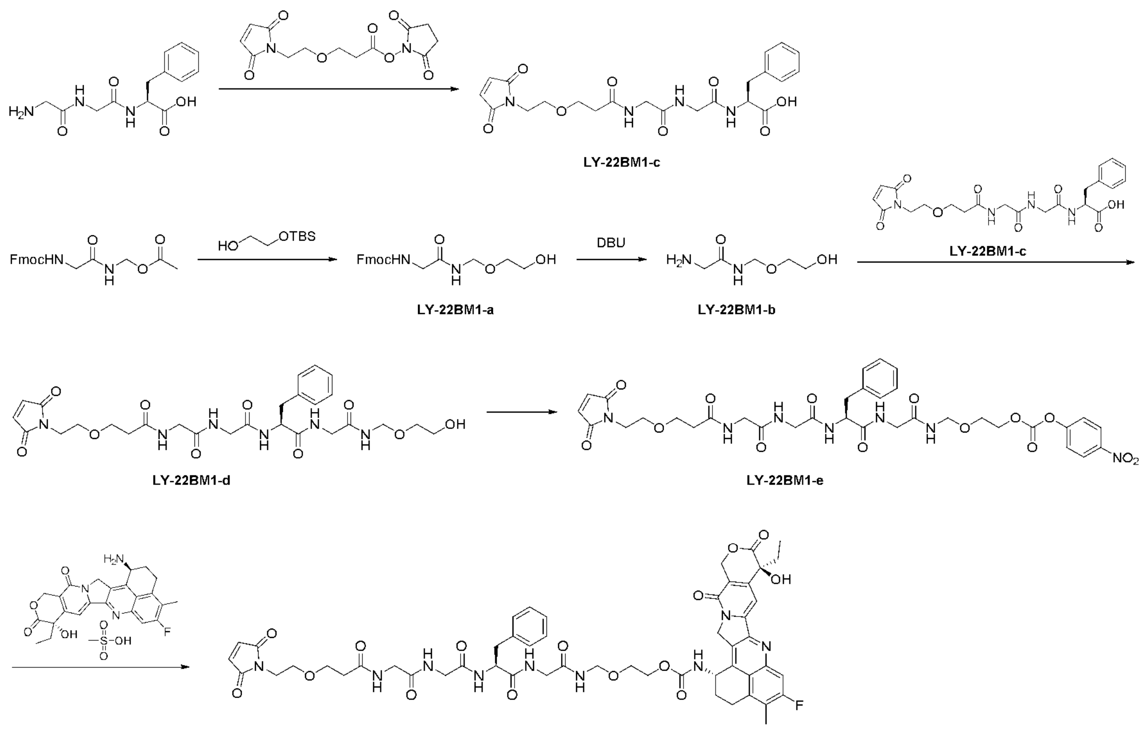


LY-22BM1-c

LY-22BM1-a

LY-22BM1-b

LY-22BM1-d

LY-22BM1-e

LY-22BM1

Step 1: Preparation of compound LY-22BM1-c

DIPEA (680.23 mg, 99%, 869.87 μL, 5.26 mmol, 3 eq) was added to the solution of the compound glycyl-L-phenylalanine (ChemExpress) (500 mg, 98%, 1.75 mmol, 1 eq) and the compound Mal-PEG1-NHS ester (544.34 mg, 98%, 1.75 mmol, 1 eq) in DMF (5 mL), the reaction solution reacted for 1 hour at room temperature, LCMS showed that the reaction ended, and the residue was purified by silica gel column chromatography (C18) (ISCO, R-220g SepaFlash Silica Flash Column, Eluent of 5-25% $H_2O/CH_3CN$, 80 mL/min), to obtain a white solid compound LY-22BM1-c (696.60 mg, yield: 83.68%, purity: 97%). LCMS (ESI): m/z, 475[M+H]$^+$.

Step 2: Preparation of compound LY-22BM1-a

TsOH (472.2 mg, 2.71 mmol, 1 eq) was added to a solution of compound [[2-(Fmoc-amino)acetamino]methyl] acetate (1g, 2.71 mmol, 1 eq) and 2-(tert-butyldimethylsiloxy)ethanol (957.3 mg, 5.43 mmol, 2 eq) in THF (15 mL), the reaction solution was stirred at 30°C for 2 hours, the LCMS showed that the raw material disappeared, and the reaction solution was purified (under medium pressure) by silica gel column chromatography (C18) (ISCO, R-330g SepaFlash Silica Flash Column, Eluent of 5%-35% $H_2O/CH_3CN$, 100 mL/min) to obtain a white solid LY-22BM1-a (316 mg, yield = 31.43%).

LCMS (ESI): m/z, 393 [M+Na]$^+$, Rt=1.537 min.

Step 3: Preparation of compound LY-22BM1-b

DBU (63.73 μL, 426.55 μmol, 0.5 eq) was added to the solution of compound LY-22BM1-a (316 mg, 853.11 μmol, 1 eq) in DMF (3 mL), the reaction solution was stirred at room temperature for 1 hour, and the LCMS showed that the raw materials completed reaction. TsOH (74.19 mg, 426.55 μmol, 0.5 eq) was added to the reaction solution. The reaction solution was directly used for the next step. A theoretical mass of LY-22BM1-b was 126.40 mg (no yield was calculated for the crude product).

Step 4: Preparation of compound LY-22BM1-d

LY-22BM1-b (63.20 mg, 426.57 μmol, 1 eq), HOBT (57.66 mg, 426.57 μmol, 1 eq), and EDCI (82.60 mg, 426.57 μmol, 1 eq) were added to the solution of compound LY-22BM1-c (208.65 mg, 426.57 μmol, 1 eq) in DMF (2 mL) under an ice water bath under protection of nitrogen, and the reaction solution was stirred at room temperature for 2 hours. The LCMS showed that the raw materials completed reaction. The reaction solution was purified (preparative chromatograph manufacturer: Oriendo, model: BRIX-2860, column: YMC-Triart Prep C18 150 × 30 mm × 10 μm, mobile phase: water (0.225% HCOOH)-acetonitrile, an elution ratio of acetonitrile: 20% to 50%), to obtain a pale yellow solid LY-22BM1-d (172.40 mg, yield = 66.8%).

LCMS (ESI): m/z, 627 $[M+Na]^+$, Rt=0.764 min.

Step 5: Preparation of compound LY-22BM1-e

DIEA (142.80 μL, 855.42 μmol, 3 eq) and bis(p-nitrobenzene)carbonate (178.85 mg, 570.28 μmol, 2 eq) were added to the solution of LY-22BM1-d (172.40 mg, 285.14 μmol, 1 eq) in DMF (5 mL), the reaction solution was stirred at 35°C for 3 hours, the solution was concentrated by the oil pump, and the residue was purified by silica gel column chromatography (eluents: ethyl acetate/petroleum ether = 0%-50%, methanol/methylene dichloride = 0%-8%), to obtain a white solid LY-22BM1-e (99.60 mg, yield = 45.38%).

LCMS (ESI): m/z, 792$[M+Na]^+$, Rt=1.563 min.

Step 6: Preparation of compound LY-22BM1

DIEA (85.54 μL, 517.59 μmol, 4 eq) and HOBT (8.75 mg, 64.70 μmol, 0.5 eq) were added to the solution of compound LY-22BM1-e (99.60 mg, 129.40 μmol, 1 eq) and exatecan mesylate (69.11 mg, 130 μmol, 1 eq) in DMF (6 mL), the reaction solution was stirred for 2 hours at room temperature, and the LCMS showed that the raw materials completed reaction. The reaction solution was subjected to high performance liquid chromatograph (preparative chromatograph manufacturer: Oriendo, model: BRIX-2860, column: YMC-Triart Prep C18 150 × 30 mm × 10 μm, mobile phase: water (0.225% HCOOH)-acetonitrile, an elution ratio of acetonitrile: 30% to 60%), to obtain pale yellow solid LY-22BM1 (66.50 mg, yield = 48.21%, purity = 94.38%).

LCMS (ESI): m/z, 1066$[M+H]^+$, Rt=1.675. $^1H$ NMR (400 MHz, DMSO-$d_6$) δ 8.53 (s, 1H), 8.30 (s, 1H), 8.12 (d, *J* = 7.2 Hz, 2H), 8.05 (d, *J* = 8.8 Hz, 1H), 8.00 (s, 1H), 7.78 (d, *J* = 10.8 Hz, 1H), 7.31 (s, 1H), 7.28 - 7.15 (m, 5H), 7.00 (s, 2H), 6.52 (s, 1H), 5.43 (s, 2H), 5.25 (s, 3H), 4.58 (d, *J* = 7.4 Hz, 2H), 4.48 (s, 1H), 4.18 (s, 2H), 3.78 - 3.64 (m, 5H), 3.61 (t, *J* = 4.8 Hz, 2H), 3.54 (dt, *J* = 11.4, 5.8 Hz, 4H), 3.45 (t, *J* = 5.6 Hz, 2H), 3.12 (s, 2H), 3.03 (dd, *J* = 13.8, 4.6 Hz, 1H), 2.87 - 2.70 (m, 2H), 2.38 (s, 3H), 2.33 (d, *J* = 6.0 Hz, 2H), 2.18 (s, 2H), 1.86 (dt, *J* = 15.4, 7.2 Hz, 2H), 0.87 *(t, J*= 7.4 Hz, 3H).

Example 14: Preparation of compound **LY-22BM2**

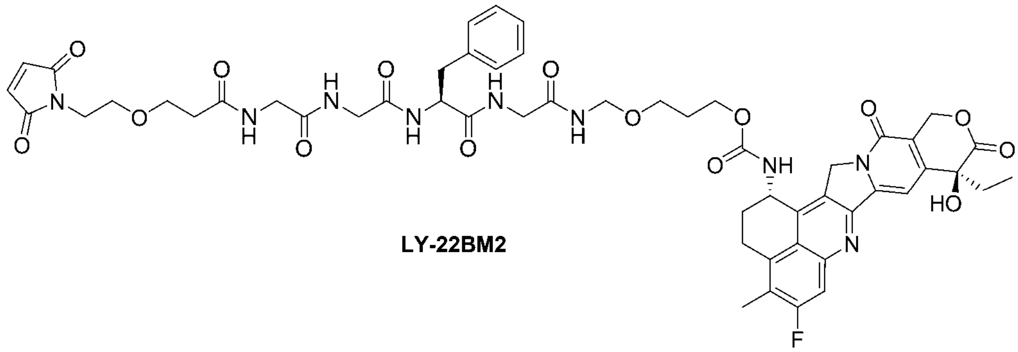

LY-22BM2

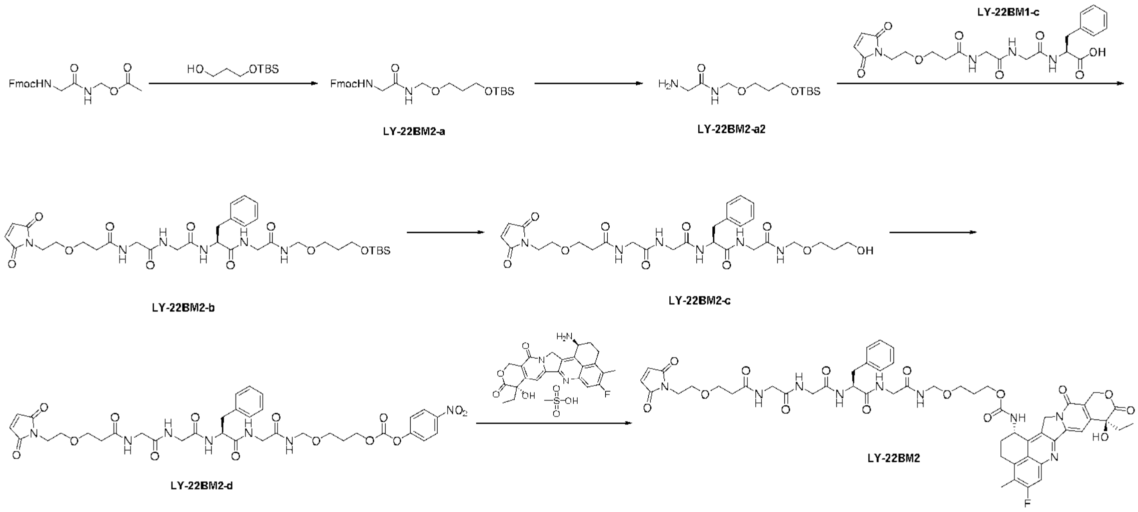

LY-22BM1-c

LY-22BM2-a

LY-22BM2-a2

LY-22BM2-b

LY-22BM2-c

LY-22BM2-d

LY-22BM2

Step 1: Preparation of compound ((9H-fluoren-9-yl)methyl(2,2,3,3-tetramethyl-11-oxo-4,8-dioxo-10-aza-3-siladodecane-12-yl)carbamate (LY-22BM2-a)

The compound (2-((((9H-fluoren-9-yl)methoxy)carbonyl)amino)acetamide)methyl acetate (Chengdu Zen Sunda) (1g, 2.71 mmol, 1 eq) was dissolved in THF (20 mL), 3-((tertbutyldimethylsilyl)oxy)propyl-1-ol (1.03g, 99%, 5.43 mmol, 2 eq) and TsOH (46.75 mg, 99%, 43.69 μL, 271.46 μmol, 0.1 eq) were added, and the reaction solution was stirred at 25°C for 1 hour. The LCMS showed the target product. The reaction solution was purified by silica gel column chromatography (C18) (ISCO, R-330g SepaFlash Silica Flash Column, Eluent of 5%-30% $H_2O/CH_3CN$, 100 mL/min) to obtain a brown solid product (600 mg, yield: 44.32%).

LCMS (ESI): m/z, 521.3[M+Na]$^+$.

Step 2: Preparation of compound LY-22BM2-a2

The compound LY-22BM2-a (600.01 mg, 1.20 mmol, 1 eq) was dissolved in DMF (20 mL), DBU (91.58 mg, 89.88 μL, 601.57 μmol, 0.5 eq) was added, and the reaction solution was stirred at 25°C for 2 hours. The LCMS showed the target product. The reaction solution was directly used for the next step without purification.

LCMS (ESI): m/z, 299.3[M+Na]$^+$.

Step 3: Preparation of compound LY-22BM2-b

The compound LY-22BM2-a2 (150 mg, 542.59 μmol, 1 eq) was dissolved in DMF (5 mL), TsOH (47.19 mg, 99%, 44.10 μL, 271.30 μmol, 0.5 eq) was added, then the compound LY-22BM1-c (for synthesis of the compound, refer to the synthesis example of LY-22BM1) (265.41 mg, 542.59 μmol, 1 eq) and HOBT (88.01 mg, 99.96%, 58.68 μL, 651.11 μmol, 1.2 eq) were added sequentially, finally EDCI (126.08 mg, 99%, 651.11 μmol, 1.2 eq) was added at 0°C, and the reaction solution was stirred at 25°C for 1 hour. The LCMS showed that the product was generated. A yellow solution was obtained (300 mg, yield: 75.44%), to be directly used for the next step.

LCMS (ESI) m/z, 755.4[M+Na]$^+$.

Step 4: Preparation of compound LY-2BM2-c

$Et_3N.3HF$ (68.03 mg, 97%, 66.72 μL, 409.33 μmol, 1 eq) was added to the solution of compound LY-22BM2-b (300 mg, 409.33 μmol, 1 eq) in DMF (5 mL), and the reaction solution was stirred at 25°C for 2 hours. LCMS showed that the reaction ended. The reaction solution was subjected to silica gel column chromatography to obtain a white solid product (150 mg, yield: 59.24%).

The preparation conditions of the silica gel column chromatography were as follows:
The residue was purified by silica gel column chromatography (C18) (ISCO, R-120g SepaFlash Silica Flash Column, Eluent of 25%-50% $H_2O$ (0.225% FA)-ACN, 100 mL/min).

LCMS (ESI): m/z, 641.3[M+Na]$^+$.

Step 5: Preparation of compound LY-22BM2-d

The compound LY-22BM2-c (150 mg, 242.47 μmol, 1 eq) was dissolved in DMF (5 mL) at room temperature, bis(p-nitrobenzene)carbonate (152.08 mg, 97%, 101.39 μL, 484.93 μmol, 2 eq) and DIPEA (94.96 mg, 99%, 121.43 μL, 727.40 μmol, 3 eq) were added, and the reaction solution was stirred at 33°C for 3 hours. The LCMS showed that the raw materials completed reaction. The reaction solution was concentrated to dryness, and the residue was purified by silica gel column chromatography (eluents: ethyl acetate/petroleum ether = 1:1, dichloromethane: methanol = 10:1), to obtain a white solid product (150 mg, yield: 78.93%).

LCMS (ESI): m/z, 806.3[M+Na]$^+$.

Step 6: Preparation of compound LY-22BM2

The compound LY-22BM2-d (60 mg, 76.56 μmol, 1 eq) was dissolved in DMF (3 mL), exatecan mesylate (41.11 mg, 99%, 76.56 μmol, 1 eq), DIPEA (39.98 mg, 99%, 51.12 μL, 306.22 μmol, 4 eq) and HOBT (5.17 mg, 99.96%, 3.45 μL, 38.28 μmol, 0.5 eq) were added, the reaction solution was stirred at 25°C for 2 hours, and the LCMS showed that the raw materials completed reaction. The reaction solution was subjected to preparative high performance liquid chromatography to obtain a yellow solid product (31.19 mg, yield: 37.72%, purity: 95%).

The conditions of the preparative high performance liquid chromatography were as follows:
Preparative chromatograph manufacturer: SHIMADZU, model: LC-20AP, column: YMC-Triart Prep C18 250 × 50 mm × 7 μm, mobile phase: water (0.225% HCOOH)-acetonitrile, an elution ratio of water: 30% to 60%.

LCMS (ESI): m/z,1078.5[M+H]$^+$.

$^1$H NMR (400 MHz, DMSO-*d*$^6$) δ 8.49 (t, *J* = 6.6 Hz, 1H), 8.30 (t, *J* = 5.9 Hz, 1H), 8.15 - 8.08 (m, 2H), 8.03 - 7.92 (m, 2H), 7.78 (d, *J* = 11.0 Hz, 1H), 7.31 (s, 1H), 7.28 - 7.13 (m, 5H), 7.00 (s, 2H), 6.52 (s, 1H), 5.43 (s, 2H), 5.23 (d, *J* = 7.4 Hz, 3H), 4.51 (dd, *J* = 23.3, 5.8 Hz, 3H), 4.21 - 4.04 (m, 2H), 3.81 - 3.63 (m, 5H), 3.56 (ddd, *J* = 17.9, 13.2, 5.4 Hz, 5H), 3.50 - 3.41 (m, 4H), 3.23 (s, 2H), 3.15 - 3.07 (m, 1H), 3.03 (dd, *J* = 14.0, 4.5 Hz, 1H), 2.77 (dd, *J* = 13.8, 9.6 Hz, 1H), 2.38 (d, *J* = 1.9 Hz, 3H), 2.32 (t, *J* = 6.6 Hz, 2H), 2.17 (d, *J* = 24.5 Hz, 2H), 1.86 (dp, *J* = 18.5, 6.8, 6.2 Hz, 4H), 0.87 (t, *J* = 7.3 Hz, 3H).

Example 15: Preparation of compound **LY-22BM3**

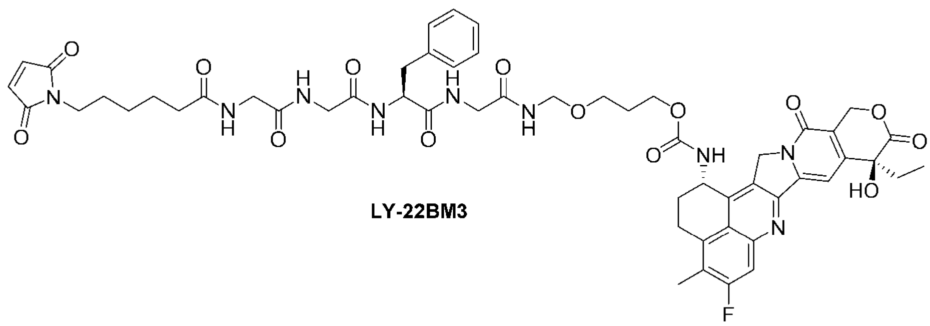

**LY-22BM3**

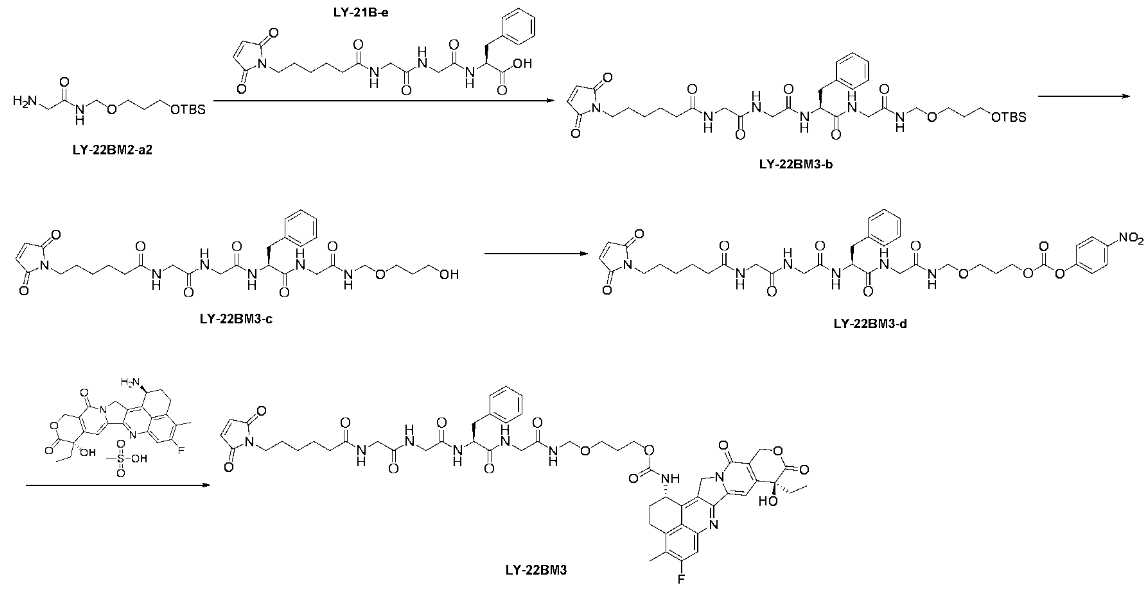

LY-21B-e

LY-22BM2-a2

LY-22BM3-b

LY-22BM3-c

LY-22BM3-d

LY-22BM3

Step 1: Preparation of compound LY-22BM3-b

The compound LY-22BM2-a2 (for synthesis of the compound, refer to the synthesis example of LY-22BM2) (80 mg, 289.38 μmol, 1 eq) was dissolved in DMF (3 mL), TsOH (24.92 mg, 99%, 23.29 μL, 144.69 μmol, 0.5 eq) was added, then LY-21B-e (Chengdu Zen Sunda) (136.73 mg, 289.38 μmol, 1 eq) and HOBT (46.92 mg, 99.96%, 31.28 μL, 347.26 μmol, 1.2 eq) were added sequentially, finally EDCI (66.57 mg, 99%, 347.26 μmol, 1.2 eq) was added at 0°C, and the reaction solution was stirred at 0°C-10°C for 2 hours. The LCMS showed that the product was generated. A yellow solution was obtained (150 mg, yield: 70.92%), to be directly used for the next step.

LCMS (ESI): m/z, 753.5[M+Na]$^+$.

Step 2: Preparation of compound LY-22BM3-c

$Et_3N.3HF$ (170.53 mg, 97%, 172.43 μL, 1.03 mmol, 5 eq) was added to the solution of compound LY-22BM3-b (150 mg, 205.22 μmol, 1 eq) in DMF (3 mL), and the reaction solution was stirred at 25°C for 1 hour. LCMS showed that the reaction ended. The reaction solution was subjected to preparative high performance liquid chromatography to obtain a white solid product LY-22BM3-c (80 mg, yield: 63.22%).

The preparation conditions of the reversed-phase chromatography were as follows:

The residue was purified by silica gel column chromatography (C18) (ISCO, R-330g SepaFlash Silica Flash Column, Eluent of 12%-42% $H_2O$ (0.225% FA)-ACN, 100 mL/min).

LCMS (ESI): m/z, 639.3[M+Na]$^+$.

Step 3: Preparation of compound LY-22BM3-d

The compound LY-22BM3-c (80 mg, 129.73 μmol, 1 eq) was dissolved in DMF (2 mL) at room temperature, bis(p-nitrobenzene)carbonate (81.37 mg, 97%, 54.25 μL, 259.46 μmol, 2 eq) and DIPEA (50.81 mg, 99%, 64.97 μL, 389.19 μmol, 3 eq) were added, and the reaction solution was stirred at 30°C for 1 hour. The LCMS showed that the raw materials completed reaction. The reaction solution was concentrated to dryness, and the residue was purified by silica gel column chromatography (eluents: ethyl acetate/petroleum ether = 1:1, dichloromethane: methanol = 10:1), to obtain a white solid product (90 mg, yield: 88.74%).

LCMS (ESI): m/z, 804.4[M+Na]$^+$.

Step 4: Preparation of compound LY-22BM3

The compound LY-22BM3-d (40 mg, 51.17 μmol, 1 eq) was dissolved in DMF (3 mL), exatecan mesylate (27.20 mg, 51.17 μmol, 1 eq), DIPEA (26.72 mg, 99%, 34.17 μL, 204.66 μmol, 4 eq) and HOBT (3.46 mg, 99.96%, 2.31 μL, 25.58 μmol, 0.5 eq) were added, the reaction solution was stirred at 25°C for 1 hour, and the LCMS showed that the raw materials completed reaction. The reaction solution was subjected to preparative high performance liquid chromatography to obtain a yellow solid product LY-22BM3 (32.19 mg, yield: 58.35%, purity: 98%).

The conditions of the preparative high performance liquid chromatography were as follows: Preparative chromatograph manufacturer: SHIMADZU, model: LC-20AP, column: YMC-Triart Prep C18 250 × 50 mm × 7 μm, mobile phase: water (0.225% HCOOH)-acetonitrile, an elution ratio of water: 30% to 60%.

LCMS (ESI): m/z, 1078.5[M+H]$^+$.

$^1$H NMR (400 MHz, DMSO-$d_6$) δ 8.49 (t, *J* = 6.6 Hz, 1H), 8.30 (t, *J* = 5.9 Hz, 1H), 8.12 (d, *J* = 8.0 Hz, 1H), 8.07 (t, *J* = 5.7 Hz, 1H), 8.01 (t, *J* = 5.7 Hz, 1H), 7.95 (d, *J* = 9.0 Hz, 1H), 7.77 (d, *J* = 10.9 Hz, 1H), 7.31 (s, 1H), 7.27-7.12 (m, 5H), 6.99 (s, 2H), 6.53 (s, 1H), 5.42 (s, 2H), 5.31-5.16 (m, 3H), 4.61-4.43 (m, 3H), 4.13 (ddt, *J* = 19.6, 12.9, 6.4 Hz, 2H), 3.80-3.53 (m, 6H), 3.47 (t, *J* = 6.2 Hz, 2H), 3.31-3.18 (m, 3H), 3.12 (d, *J* = 7.5 Hz, 1H), 3.03 (dd, *J* = 13.8, 4.4 Hz, 1H), 2.78 (dd, *J* = 13.8, 9.7 Hz, 1H), 2.41-2.33 (m, 3H), 2.24-2.05 (m, 4H), 1.86 (dp, *J* = 18.8, 6.8, 6.3 Hz, 4H), 1.46 (h, *J* = 7.2 Hz, 4H), 1.18 (p, *J* = 7.8, 7.4 Hz, 2H), 0.87 (t, *J* = 7.3 Hz, 3H).

Example 16: Preparation of compound **LY-22C**

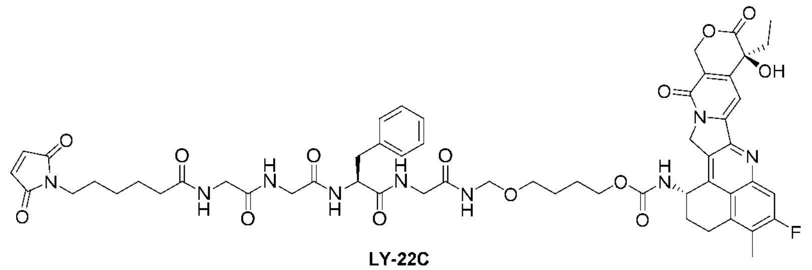

**LY-22C**

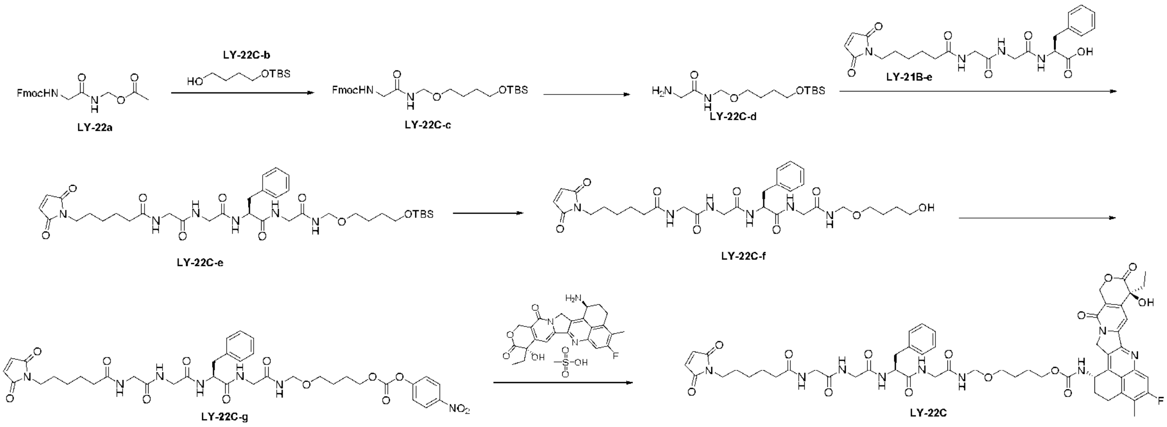


The preparation method is the same as that in Example 12, except that the compound **LY-22C** was prepared with 4-((tert-butyldimethylsilyl)oxy)but-1-ol instead of 2-((tertbutyldimethylsilyl)oxy)ethane-1-ol.

Example 17: Preparation of compound LY-22CDX

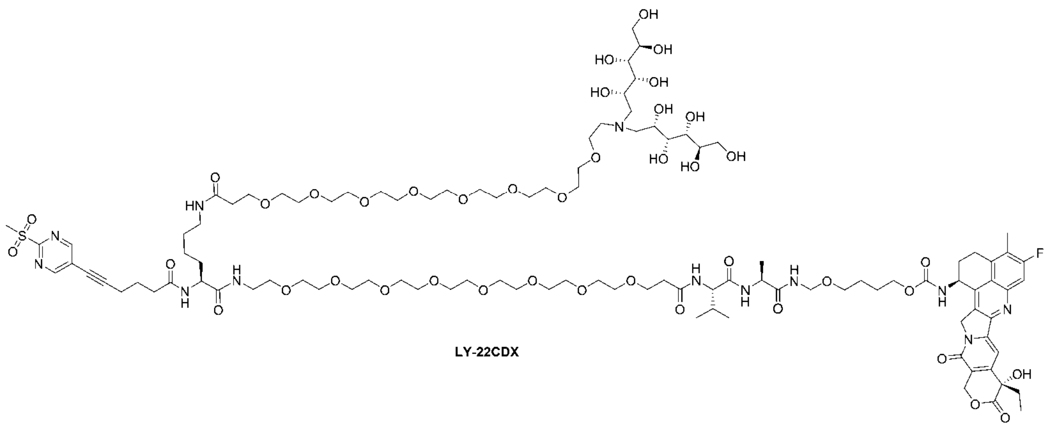

LY-22CDX

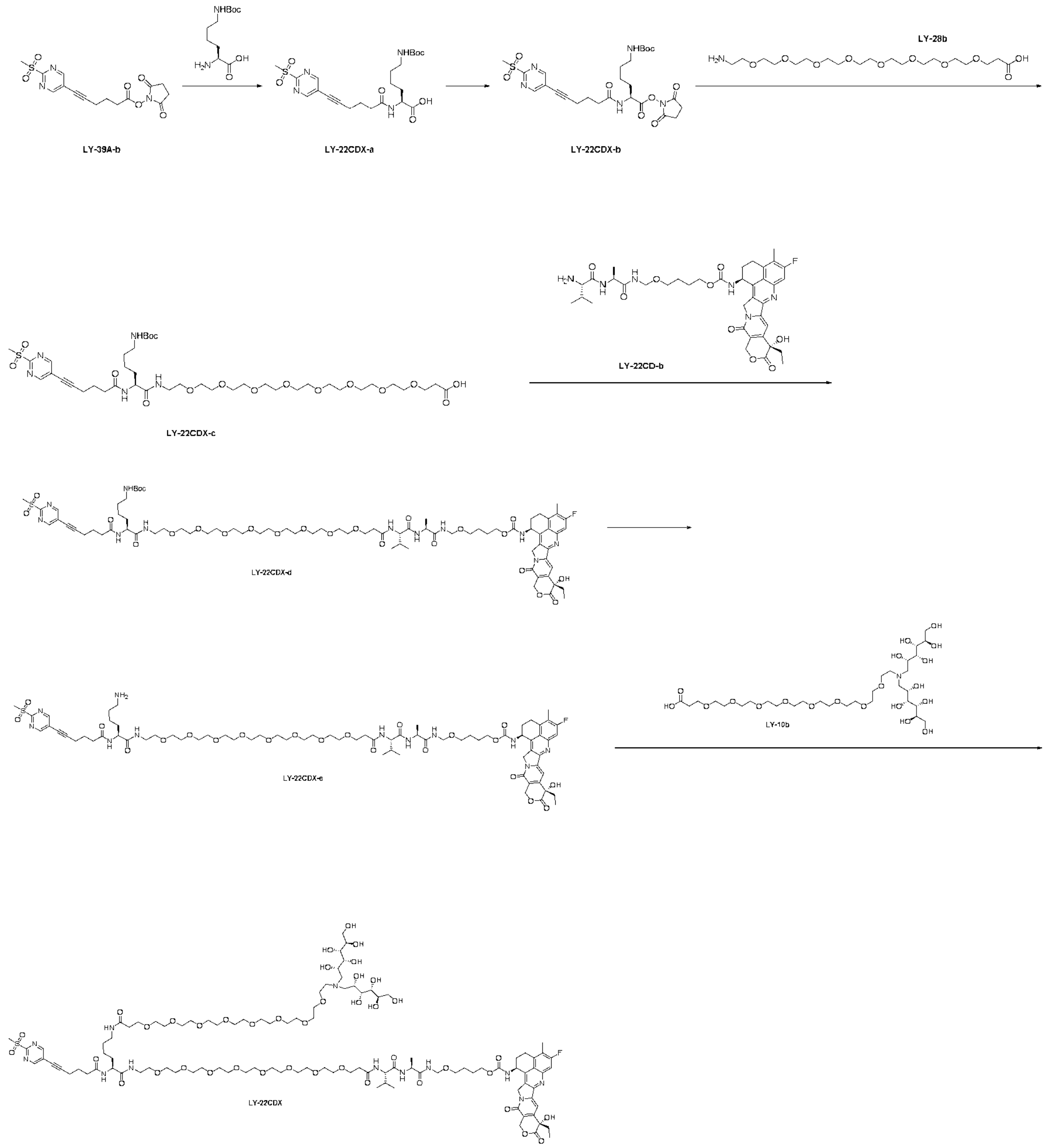


LY-39A-b

LY-22CDX-a

LY-22CDX-b

LY-22CDX-c

LY-22CDX-d

LY-22CDX-e

LY-22CDX

Step 1: Preparation of compound LY-22CDX-a

DIEA (318.36 mg, 407.11 μL, 2.46 mmol, 2 eq) was added to the solution of compound LY-39A-b (500 mg, 1.23 mmol, 1 eq, purity: 90%; for the synthesis of this compound, refer to synthesis processes of LY-39A and LY-44A) in DMF (3 mL) under an ice water bath, then (2S)-2-amino-6-(tert-butoxycarbonylamino)hexanoic acid (303.36 mg, 1.23 mmol, 1 eq) in DMF (2 mL) was added dropwise, the reaction solution was stirred at room temperature for 2 hours, and the LCMS showed that the raw materials basically completed reaction. The residue was purified (under medium pressure) by silica gel column chromatography (C18) (ISCO, R-330g SepaFlash Silica Flash Column, Eluent of 5%-40% $H_2O/CH_3CN$, 100 mL/min) to obtain a white solid LY-22CDX-a (484.60 mg, yield= 68.93%, purity: 87%).
LCMS (ESI): m/z, 519$[M+Na]^+$, Rt=1.478min.

Step 2: Preparation of compound LY-22CDX-b

DIC (264.55 mg, 1.7 mmol, 2 eq) was added to the solution of LY-22CDX-a (484.6 mg, 849.01 μmol, 1 eq) and N-hydroxysuccinimide (122.14 mg, 1.061 mmol, 1.25 eq) in DMF (10 mL) in the ice water bath, the reaction solution was stirred at room temperature for 3 hours, the LCMS showed that the raw materials completed reaction, EA (10 mL) and water (10 mL) were added to the reaction solution, and after liquid separation, the organic phases were washed with water (10 mL) three times and washed with saturated brine (10 mL) twice, and the organic phases were combined and dried with anhydrous sodium sulfate, filtered, concentrated and directly used for the next step to obtain the crude product LY-22CDX-b (579.50 mg, yield = 95.62%, purity = 80%).
LCMS (ESI): m/z, 616$[M+Na]^+$, Rt = 1.788 min.

Step 3: Preparation of compound LY-22CDX-c

DIEA (258.12 μL, 1.56 mmol, 2 eq) was added to the solution of compound LY-22CDX-b (579.5 mg, 780.93 μmol, 1 eq) and LY-28b (344.78 mg, 780.93 μmol, 1 eq, Bidepharm) in DMF (10 mL) under an ice water bath, the reaction solution was stirred for 1 hour at room temperature, and the LCMS showed that the raw materials completed reaction. The reaction solution was subjected to preparative high performance liquid chromatograph (preparative chromatograph manufacturer: Oriendo, model: BRIX-2860, column: Welch Xtimate C18 150 × 30 mm × 10 μm, mobile phase: water (0.225% HCOOH)-acetonitrile, an elution ratio of acetonitrile: 20%-50%), to obtain yellow oily LY-22CDX-c (408.90 mg, yield = 56.9%).
LCMS (ESI): m/z, 942$[M+Na]^+$, Rt = 1.563 min.

Step 4: Preparation of compound LY-22CDX-d

DIEA (17.21 mg, 22.01 μL, 133.19 μmol, 2.5 eq) and HATU (30.39 mg, 79.91 μmol, 1.5 eq) were added to the solution of compounds LY-22CDX-c (48.9 mg, 53.27 μmol, 1 eq) and LY-22CD-b (40 mg, 53.27 μmol, 1 eq; for synthesis of the compound, refer to the synthesis example of LY-22CD) in DMF (3 mL) under the ice water bath, the reaction solution was stirred for 2 hours at room temperature, and the LCMS showed that the raw materials completed reaction. The reaction solution was purified by preparative high performance liquid chromatograph (preparative chromatograph manufacturer: Oriendo, model: BRIX-2860, column: YMC-Triart Prep C18 150 × 30 mm × 10 μm, mobile phase: water (0.225% HCOOH)-acetonitrile, an elution ratio of acetonitrile: 30%-60%), to obtain a white solid LY-22CDX-d (54.6 mg, yield = 62%).
LCMS (ESI): m/z, 1675$[M+Na]^+$, Rt = 2.151 min.

Step 5: Preparation of compound LY-22CDX-e

TFA (0.5 mL) was added to the solution of LY-22CDX-d (54.6 mg, 33 μmol, 1 eq) in DCM (5 mL) under an ice water bath, the reaction solution was stirred for 10 minutes and then concentrated immediately, and then DMF (1 mL) was added. The LCMS showed that the raw materials basically completed reaction. The reaction solution was used for the next step. The theoretical mass of LY-22CDX-e was 34.19 mg (no yield was calculated for the crude product).
LCMS (ESI): m/z, 1553$[M+H]^+$, Rt = 1.653 min.

Step 6: Preparation of compound LY-22CDX

DIEA (18.2 mg, 110 μmol, 5 eq) and HATU (10 mg, 26.4 μmol, 1.2 eq) were added to the solution of compounds LY-22CDX-e (34.19 mg, 22 μmol, 1 eq) and LY-10b (18.6 mg, 24.2 μmol, 1 eq; for synthesis of the compound, refer to the synthesis example of LY-10) in DMF (3 mL) under the ice water bath, the reaction solution was stirred for 1 hour at room

temperature, and the LCMS showed that the raw materials completed reaction. The reaction solution was purified by preparative high performance liquid chromatograph (preparative chromatograph manufacturer: Oriendo, model: BRIX-2860, column: Synergi Max-RP 200 × 30 mm × 10 μm, mobile phase: water (0.225% HCOOH)-acetonitrile, an elution ratio of acetonitrile: 20%-50%), to obtain a white solid LY-22CDX (27.8 mg, yield = 55%).

LCMS (ESI): m/z, 1153[1/2M+H]$^+$, Rt=1.583 min. $^1$H NMR (400 MHz, DMSO-$d_6$) δ 9.12 (s, 2H), 8.55 (t, *J*= 6.6 Hz, 1H), 8.05 (d, *J* = 7.2 Hz, 1H), 7.99 - 7.89 (m, 3H), 7.85 (d, *J* = 8.8 Hz, 1H), 7.82 - 7.75 (m, 2H), 7.31 (s, 1H), 6.53 (s, 1H), 5.43 (s, 2H), 5.30 - 5.19 (m, 3H), 4.61 - 4.42 (m, 6H), 4.32 (t, *J* = 5.8 Hz, 3H), 4.19 (dt, *J* = 15.0, 8.0 Hz, 4H), 4.14 - 3.97 (m, 3H), 3.65 (dd, *J* = 8.0, 4.2 Hz, 2H), 3.57 (td, *J* = 6.8, 4.2 Hz, 7H), 3.53 - 3.44 (m, 50H), 3.40 (d, *J* = 8.6 Hz, 11H), 3.20 (q, *J* = 7.0, 5.8 Hz, 6H), 3.16 - 3.07 (m, 2H), 2.98 (d, *J* = 6.8 Hz, 2H), 2.77 - 2.60 (m, 4H), 2.59 - 2.54 (m, 3H), 2.44 (t, *J* = 6.8 Hz, 2H), 2.40 - 2.37 (m, 3H), 2.36 - 2.23 (m, 6H), 2.22 - 2.06 (m, 3H), 1.98 - 1.75 (m, 6H), 1.68 - 1.42 (m, 7H), 1.34 (s, 3H), 1.21 (d, *J* = 7.2 Hz, 4H), 0.94 - 0.74 (m, 9H).

Example 18: Preparation of compound LY-22CG

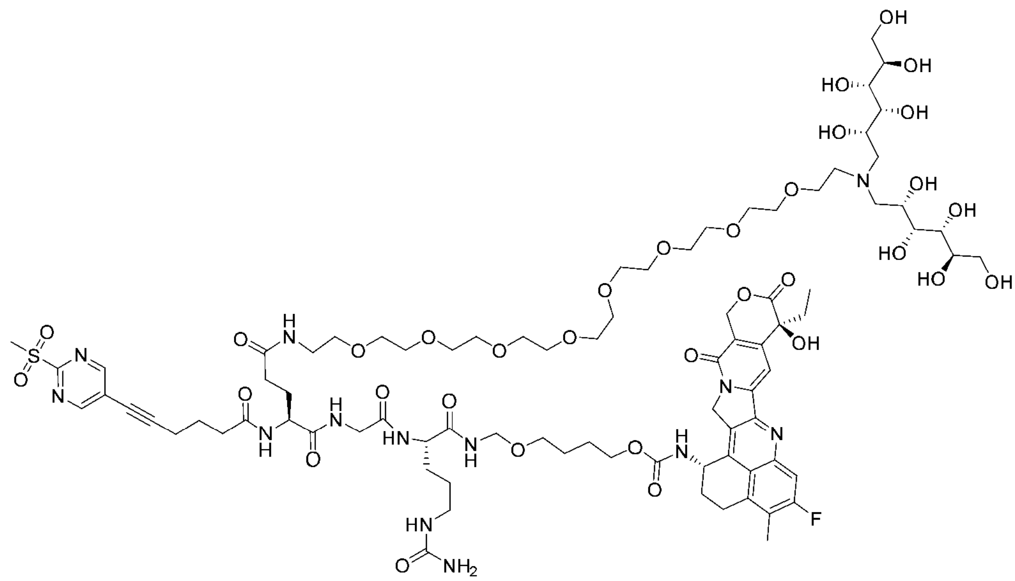

**LY22CG**

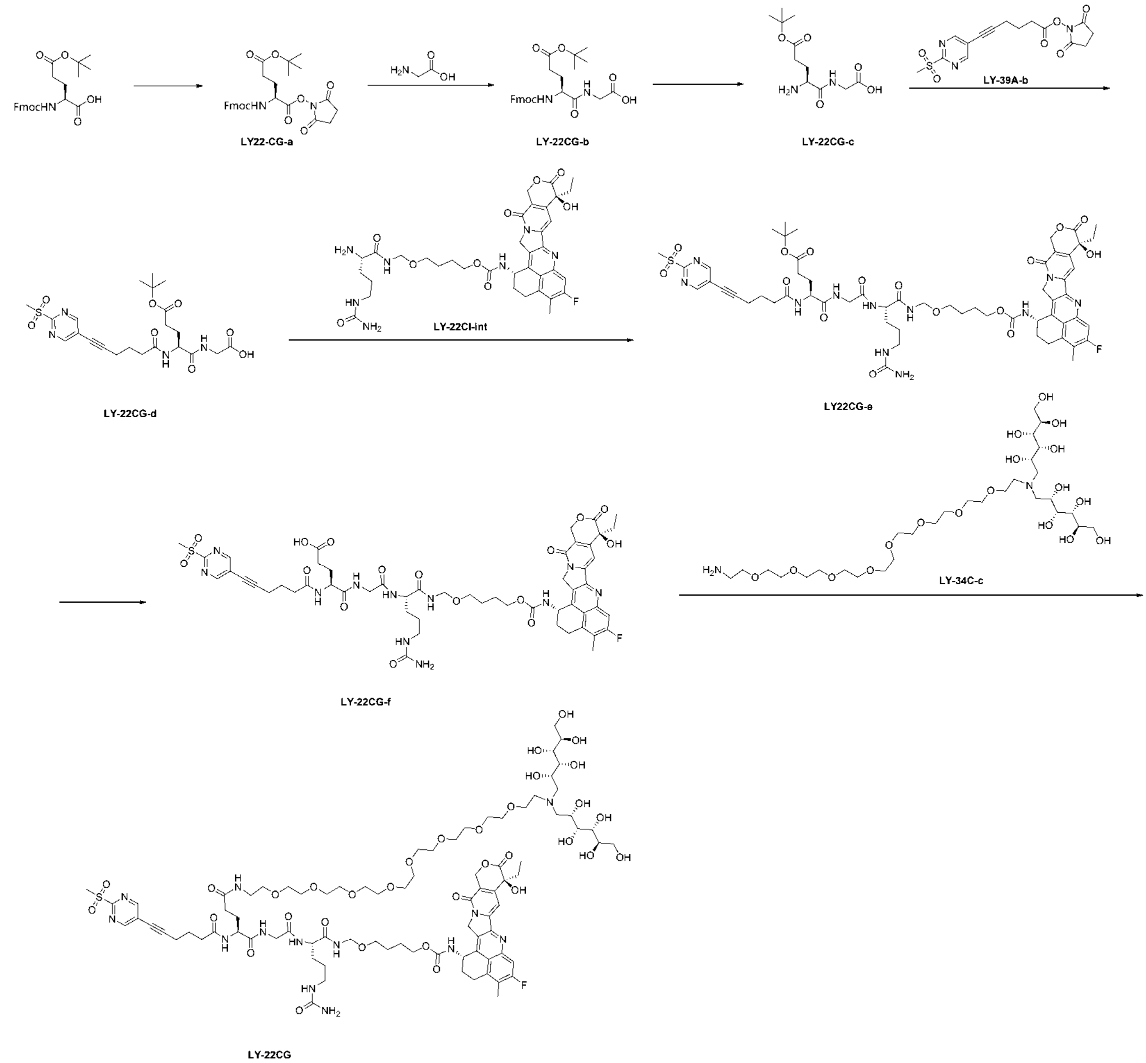


LY22-CG-a LY-22CG-b LY-22CG-c LY-22CG-d LY22CG-e LY-22CG-f LY-22CG

Step 1: Synthesis of compound LY-22CG-a

N-hydroxysuccinimide (649.20 mg, 5.64 mmol, 1.2 eq) and N,N'-dicyclohexylcarbodiimide (1.26g, 6.11 mmol, 1.3 eq) were added to a solution of Fmoc-L-glutamic acid-5-tert-butyl ester (2g, 4.70 mmol, 1 eq) in dichloromethane (40 mL) under the ice water bath, the reaction solution was stirred for 2 hours at room temperature, and the LCMS showed that the raw materials completed reaction. After the insoluble substance was filtered, the concentrated residue was purified by silica gel column chromatography (eluents: ethyl acetate: petroleum ether = 0:1-1:1, methanol: dichloromethane = 0:100-3:97) to obtain the white solid LY-22CG-a (1.13g, yield = 42.74%, purity = 93%).

LCMS (ESI): m/z, 545 $[M+Na]^+$.

Step 2: Preparation of compound LY-22CG-b

Glycine (40.08 mg, 533.92 μmol, 1 eq) and sodium bicarbonate (44.85 mg, 533.92 μmol, 1 eq) were dissolved in water (5 mL), the reaction solution was stirred at room temperature for 15 minutes, then a solution of LY22CG-a (300 mg, 533.92 μmol, 1 eq) in tetrahydrofuran (5 mL) was added to the solution, the reaction solution was stirred at room temperature for 24 hours, and the LCMS showed that the raw materials completed reaction. After concentration, the reaction solution was adjusted with 2N dilute hydrochloric acid to adjust pH to 3 at 0°C, water (5 mL) was added, the aqueous phase was extracted with ethyl acetate (5 mL) 3 times, and the organic phases were combined, washed with saturated brine (15 mL), dried with anhydrous sodium sulfate, filtered and concentrated, to obtain the crude product LY22CG-b (323.20 mg, no yield was calculated for the crude product).

LCMS (ESI): m/z, 505 $[M+Na]^+$.

Step 3: Preparation of compound LY-22CG-c

$Et_2NH$ (276 μL, 2.68 mmol, 5 eq) was added to the solution of LY22CG-b (323.20 mg, 535.84 μmol, 1 eq) in DMF (5 mL), the reaction solution was stirred at room temperature for 1 hour, and the LCMS showed that the raw materials completed reaction. The reaction solution was drained by an oil pump and used directly for the next step. A theoretical mass of LY22CG-c was 139.47 mg (no yield was calculated for the crude product).
LCMS (ESI): m/z, 261 $[M+H]^+$.

Step 4: Preparation of compound LY-22CG-d

DIEA (177.11 μL, 1.07 mmol, 2 eq) and LY-22CG-c (139.47 mg, 535.83 μmol, 1 eq) were added to the solution of compound LY-39A-b (217.52 mg, 535.83 μmol, 1 eq) in DMF (3 mL) under the ice water bath, the reaction solution was stirred for 1 hour at room temperature, and the LCMS showed that the raw materials completed reaction. The reaction solution was purified by preparative high performance liquid chromatograph (preparative chromatograph manufacturer: Oriendo, model: BRIX-2860, column: Welch Xtimate C18 250 × 30 mm × 10 μm, mobile phase: water (0.225% HCOOH)-acetonitrile, an elution ratio of acetonitrile: 20% to 50%) and lyophilized, to obtain a white solid compound LY-22CG-d (198.20 mg, yield: = 43.47%, purity = 60%).
LCMS (ESI): m/z, 533 $[M+Na]^+$.

Step 5: Preparation of compound LY-22CG-e

DIEA (8.96 μL, 54.22 μmol, 2.2 eq) and HATU (12.37 mg, 32.53 μmol, 1.2 eq) were added to the solution of compounds LY-22CG-d (15.22 mg, 29.82 μmol, 1.1 eq) and LY-22CI-int (for synthesis of the compound, refer to the synthesis process of LY-22CI) (20 mg, 27.11 μmol, 1 eq) in DMF (2 mL) at 0°C. After addition, the reaction solution reacted at 25°C for 1 hour. LCMS showed that the reaction ended. The reaction solution was purified by preparative high performance liquid chromatograph (preparative chromatograph manufacturer: Oriendo, model: BRIX-2860, column: Welch Xtimate C18 150 × 30 mm × 10 μm, mobile phase: water (0.225% HCOOH)-acetonitrile, an elution ratio of acetonitrile: 30% to 60%) and lyophilized, to obtain a white solid compound LY-22CG-e (25 mg, yield: = 74.96%, purity = 95%).
LCMS (ESI): m/z, 1230 $[M+H]^+$.

Step 6: Preparation of compound LY-22CG-f

TFA (1.6 mL) was added to the solution of compound LY-22CG-e (26.32 mg, 95%, 20.32 μmol, 1 eq) in DCM (4 mL) at 0°C. After addition, the reaction solution reacted at 0°C-5°C for 3 hours. LCMS showed that the reaction ended. The reaction solution was directly spun to dryness to obtain the white solid compound LY22CG-f (24 mg, crude product).
LCMS (ESI): m/z, 1196.5 $[M+Na]^+$.

Step 7: Preparation of compound LY-22CG

DIEA (13.21 mg, 16.89 μL, 102.19 μmol, 5 eq) and HATU (7.77 mg, 20.44 μmol, 1 eq) were added to the solution of compounds LY-22CG-f (24 mg, 20.44 μmol, 1 eq) and LY-34C-c (for synthesis of the compound, refer to the synthesis example of LY-34C) (15.14 mg, 20.44 μmol, 1 eq) in DMF (2 mL) at 0°C. After addition, the reaction solution reacted at 25°C for 2 hours. LCMS showed that the reaction ended. The reaction solution was purified by preparative high performance liquid chromatograph (preparative chromatograph manufacturer: Oriendo, model: BRIX-2860, column: Welch Xtimate C18 150 × 30 mm × 10 μm, mobile phase: water (0.225% HCOOH)-acetonitrile, an elution ratio of acetonitrile: 20% to 50%) and lyophilized, to obtain a white solid compound LY-22CG (11.51 mg, yield: 29.69%, purity: 95%).
LCMS (ESI): m/z, 1897.1 $[M+H]^+$, 949.1 $[1/2M+H]^+$.
$^1H$ NMR (400 MHz, DMSO-$d_6$) δ 9.10 (s, 2H), 8.62 (d, *J* = 6.7 Hz, 1H), 8.22 - 8.10 (m, 3H), 7.98 - 7.86 (m, 3H), 7.77 (d, *J* = 10.9 Hz, 1H), 7.31 (s, 1H), 6.52 (s, 1H), 5.92 (t, *J* = 5.7 Hz, 1H), 5.40 (d, *J* = 23.6 Hz, 4H), 5.24 (d, *J* = 3.2 Hz, 3H), 4.58 - 4.43 (m, 5H), 4.31 (s, 3H), 4.20 (dt, *J* = 14.4, 8.3 Hz, 3H), 4.07 (s, 2H), 3.77 - 3.63 (m, 4H), 3.61 - 3.54 (m, 4H), 3.49 (q, *J* = 2.2 Hz, 30H), 3.39 (d, *J* = 6.0 Hz, 9H), 3.16 (p, *J* = 6.7, 6.2 Hz, 5H), 2.93 (p, *J* = 6.8 Hz, 3H), 2.77 - 2.67 (m, 2H), 2.65 - 2.53 (m, 5H), 2.38 (d, *J* = 1.9 Hz, 3H), 2.32 (t, *J* = 6.6 Hz, 3H), 2.24 - 2.06 (m, 5H), 1.85 (ddd, *J* = 30.7, 14.4, 7.3 Hz, 7H), 1.68 - 1.42 (m, 7H), 1.33 (s, 3H), 0.87 (t, *J* = 7.3 Hz, 3H).

Example 19: Preparation of compound LY-22CH

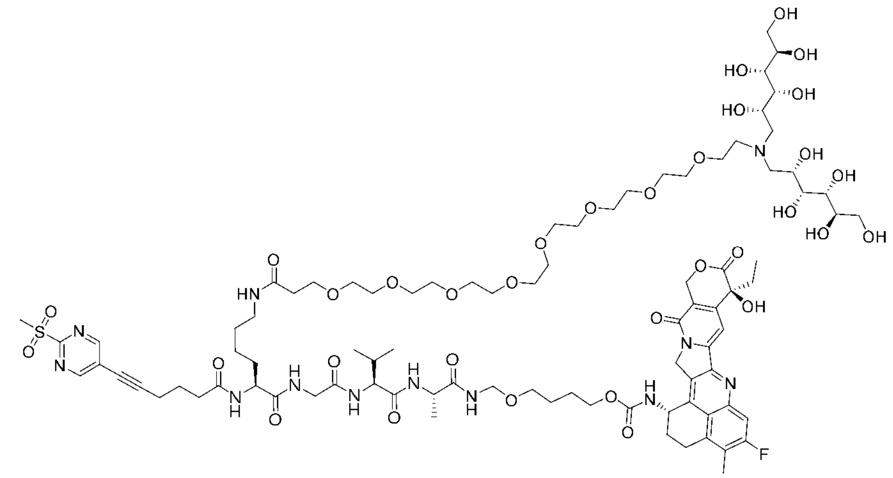

LY-22CH

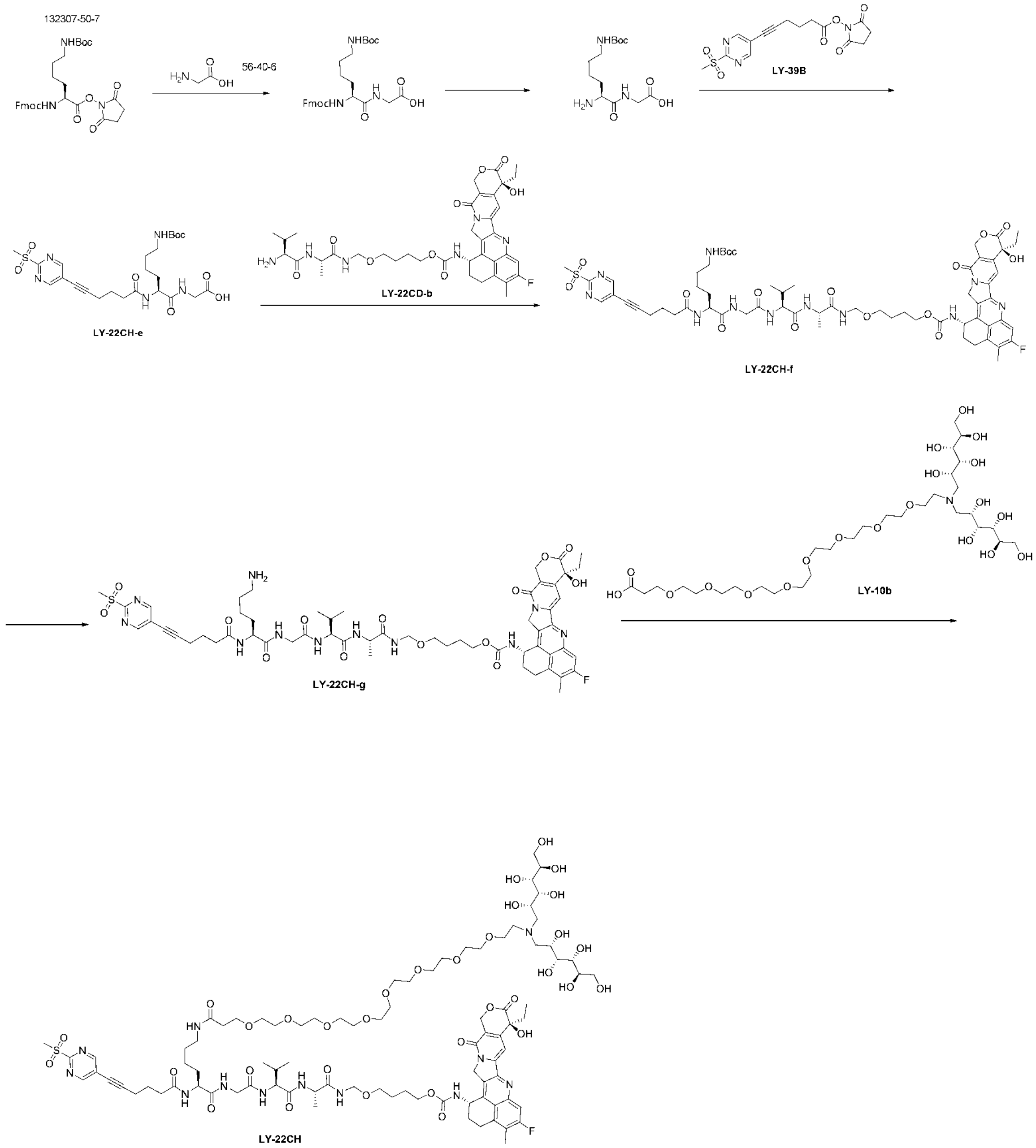


LY-22CH-e

LY-22CD-b

LY-22CH-f

LY-22CH-g

LY-10b

LY-22CH

Step 1: Preparation of compound N2-(((9H-fluoren-9-yl)methoxy)carbonyl)-N6-(tert-butoxycarbonyl)-L-lysylglycine

The compound glycine (66.36 mg, 41.61 μL, 883.99 μmol, 1 eq) and $NaHCO_3$ (74.26 mg, 40.36 μL, 883.99 μmol, 1 eq) were dissolved in $H_2O$ (5 mL), the mixture solution was stirred for 15 minutes at room temperature, and a mixture solution of compound 2,5-dioxopyrrolidin-1-ylN2-(((9H-fluoren-9-yl)methoxy)carbonyl)-N6-(tert-butoxycarbonyl)-L-lysine (500 mg, 883.99 μmol, 1 eq) in THF (5 mL) was added to the above mixture solution, the resulting solution reacted for 2 hours at room temperature, and the LCMS showed that the reaction ended. The resulting solution was spun to dryness, adjusted with 2N HCl to adjust pH to 2, and extracted with EA (20 mL × 3), the organic phases were combined and washed with water (20 mL × 3), washed with saturated brine (20 mL × 1), dried with anhydrous sodium sulfate, and spun to dryness under reduced pressure to obtain the yellow solid compound N2-(((9H-fluoren-9-yl)methoxy)carbonyl)-N6-(tert-butoxycarbonyl)-L-lysylglycine (427 mg, yield: 91.90%). LCMS (ESI): m/z, $548[M+Na]^+$.

Step 2: Preparation of compound N6-(tert-butoxycarbonyl)-L-lysylglycine

$Et_2NH$ (297.10 mg, 418.45 μL, 4.06 mmol, 5 eq) was added to a solution of compound N2-(((9H-fluoren-9-yl)methoxy)carbonyl)-N6-(tert-butoxycarbonyl)-L-lysylglycine (427 mg, 812.40 μmol, 1 eq) in DMF (10 mL), the resulting solution reacted for 1 hour at room temperature, and the LCMS showed that the reaction ended. The resulting solution was spun to dryness, to obtain the white solid compound N6-(tert-butoxycarbonyl)-L-lysylglycine (246.45 mg, no yield was calculated for the crude product) to be directly used for the next step. LCMS (ESI): m/z, $304[M+H]^+$.

Step 3: Preparation of compound LY-22CH-e

DIPEA (209.99 mg, 268.53 μL, 1.62 mmol, 2 eq) was added to the solution of compound N6-(tert-butoxycarbonyl)-L-lysylglycine (246.45 mg, 812.40 μmol, 1 eq) in DMF (3 mL), a mixture solution of compound LY-39B (329.80 mg, 90%, 812.40 μmol, 1 eq) (refer to the synthesis example of LY-39B) and DMF (1 mL) was added dropwise at 0°C, the resulting solution reacted for 1 hour at room temperature, and the LCMS showed that the reaction ended. The residue was purified by silica gel column chromatography (C18) (ISCO, R-120g SepaFlash Silica Flash Column, Eluent of 5%-45% water/$CH_3CN$, 60 mL/min) to obtain the white solid compound LY-22CH-e (386.30 mg, yield: 85.89%), LCMS (ESI): m/z, $576[M+Na]^+$.

Step 4: Preparation of compound LY-22CH-f

LY-22CD-b (30 mg, 39.96 μmol, 1 eq) (for the synthesis process, refer to the synthesis example of LY-22CD), DIPEA (10.33 mg, 13.21 μL, 79.91 μmol, 2 eq) and HATU (30.39 mg, 79.91 μmol, 2 eq) were added to the solution of compound LY-22CH-e (22.12 mg, 39.96 μmol, 1 eq) in DMF (2 mL) at 0°C, the reaction solution reacted for 1 hour at room temperature, and the LCMS showed that the reaction ended. The reaction solution was filtered and purified by preparative high performance liquid chromatograph (preparative chromatograph manufacturer: Oriendo, model: BRIX-2860, column: Welch Xtimate C18 250 × 30 mm × 10 μm, mobile phase: water (0.225% HCOOH)-acetonitrile, an elution ratio of water: 30% to 60%) and lyophilized, to obtain a white solid compound LY-22CH-f (26.60 mg, yield: 51.75%). LCMS (ESI): m/z, $1308[M+Na]^+$.

Step 5: Preparation of compound LY-22CH-g

TFA (921 mg, 600 μL) was added to a solution of compound LY-22CH-f (26.60 mg, 20.68 μmol, 1 eq) in DCM (3 mL) at 0°C, the resulting solution reacted for 25 minutes at 0°C, and the LCMS showed that the reaction ended. The resulting solution was spun to dryness, to obtain the white solid compound LY-22CH-g (24.53 mg, no yield was calculated for the crude product) to be directly used for the next step. LCMS (ESI): m/z, $1208[M+Na]^+$.

Step 6: Preparation of compound LY-22CH

The compound LY-10b (52.56 mg, 68.28 μmol, 5 eq) (for the synthesis of the compound, refer to the synthesis example of LY-10), DIPEA (17.65 mg, 22.57 μL, 136.56 μmol, 10 eq) and HATU (25.96 mg, 68.28 μmol, 5 eq) were added to the solution of compound LY-22CH-g (20 mg, 81%, 13.66 μmol, 1 eq) in DMF (2 mL) at 0°C, the reaction solution reacted for 16 hours at room temperature, and the LCMS showed that the reaction ended. The reaction solution was filtered and purified by preparative high performance liquid chromatograph (preparative chromatograph manufacturer: Oriendo, model: BRIX-2860, column: Synergi Max-RP 200 × 30 mm × 10 μm, mobile phase: water (0.225% HCOOH)-acetonitrile, an elution ratio of acetonitrile: 20% to 50%), to obtain a white solid compound LY-22CH (3.07 mg, yield: 9.67%). LCMS (ESI): m/z, $1939.1[M+Na]^+$. Rt = 1.524 min.

Example 20: Preparation of compound LY-22CI

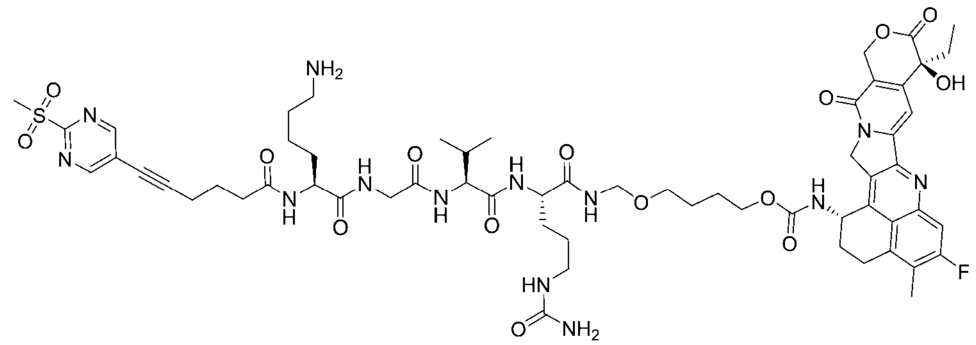

**LY-22CI**

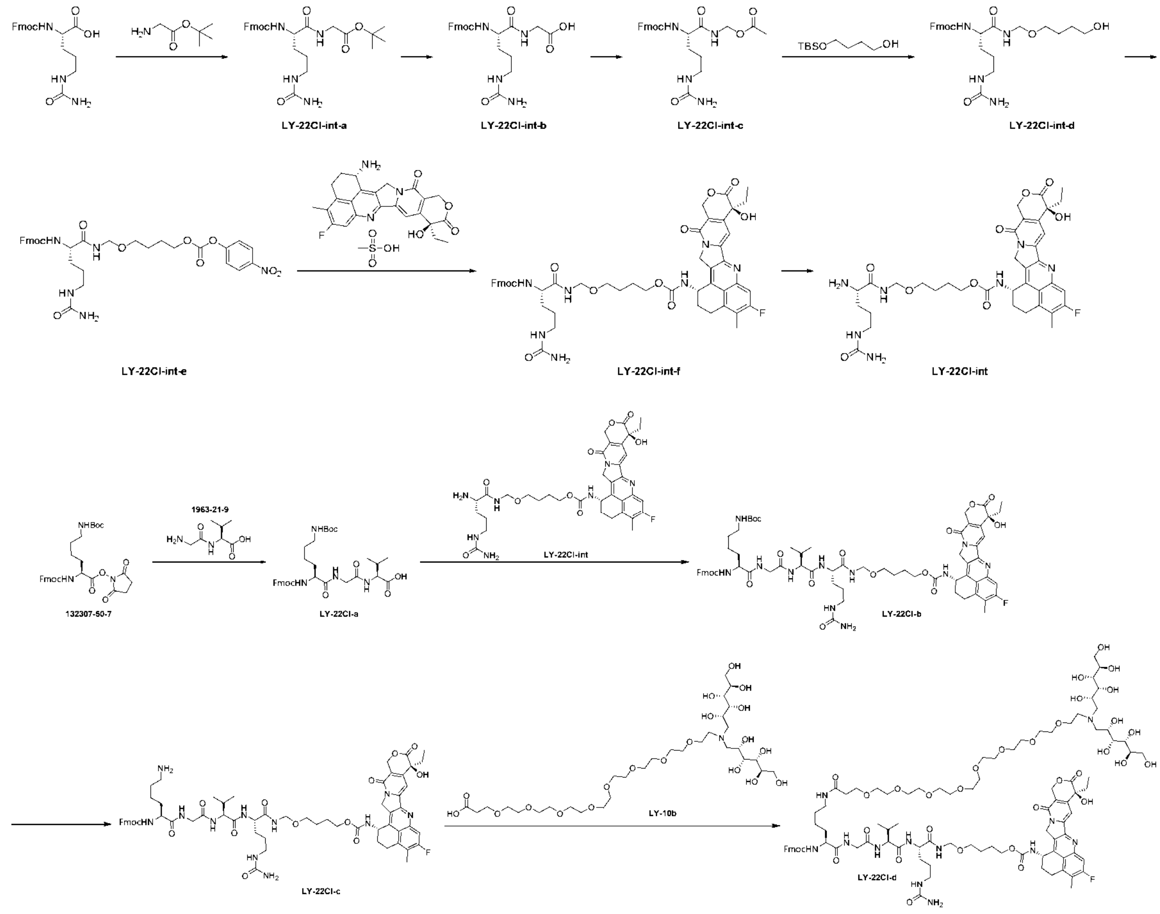

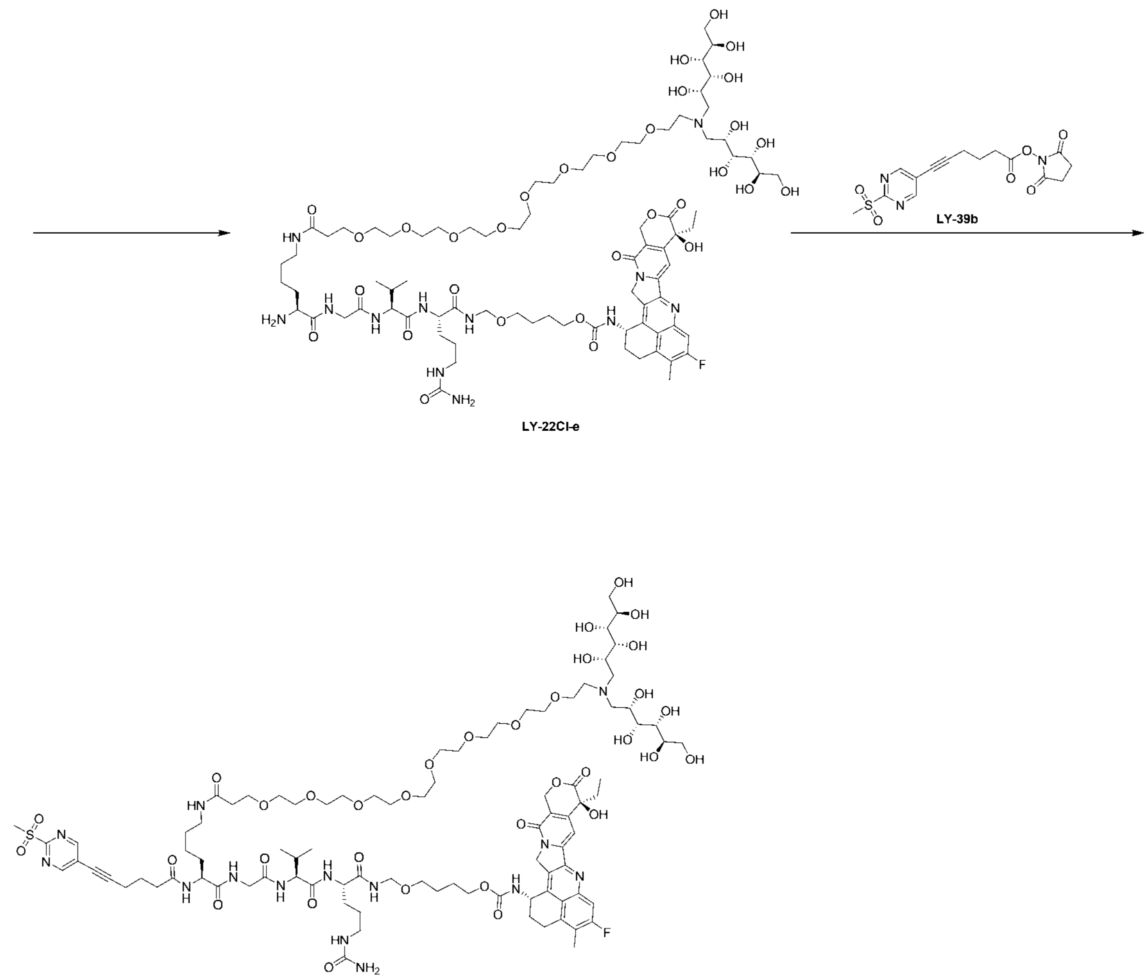

LY-22CI-e

LY-39b

LY-22CI

Step 1: Preparation of compound LY-22CI-int-a

Compounds DIPEA (9.76 g, 75.49 mmol, 3 eq) and HATU (11.48 g, 30.19 mmol, 1.2 eq) were added to the solution of compound Fmoc-L-citrulline (10g, 25.16 mmol, 1 eq) and glycine tert-butyl ester (4.95g, 37.74 mmol, 1.5 eq) in DCM (20 mL), and the reaction solution was stirred overnight at room temperature. LCMS showed that the reaction ended. Dichloromethane/methanol (10:1, 300 mL) and water (100 mL) were added to the reaction solution, the mixture was washed with dilute hydrochloric acid (0.5M) twice, 100 mL of dilute hydrochloric acid was used each time, the mixture was washed with saturated brine twice, 100 mL of saturated brine was used each time, the mixture was dried with anhydrous sodium sulfate, filtered, and then spun to dryness under reduced pressure, and the residue was purified by silica gel column chromatography (eluents: methanol/dichloromethane = 0:1-1:9) to obtain the white solid product LY-22CI-int-a (11.90g, yield: 92.62%). LCMS (ESI): m/z, 511.3$[M+H]^+$.

Step 2: Preparation of compound LY-22CI-int-b

TFA (4 mL) was added to the solution of compound LY-22CI-int-a (3.20g, 6.27 mmol, 1 eq) in DCM (20 mL) at 0°C and the reaction solution was stirred overnight at room temperature. LCMS showed that the reaction ended. The reaction solution was spun to dryness under reduced pressure. The residue was purified by silica gel column chromatography (C18) (ISCO, R-330g SepaFlash Silica Flash Column, Eluent of 10-45% water (0.225%FA)/$CH_3CN$, 100 mL/min) to obtain a white solid product LY-22CI-int-b (2.02g, yield: 70.92%). LCMS (ESI): m/z, 455.2$[M+H]^+$.

Step 3: Preparation of compound LY-22Cl-int-c

Acetic acid (800.70 mg, 764.03 μL, 13.33 mmol, 3 eq), copper acetate (242.18 mg, 226.76 μL, 1.33 mmol, 0.3 eq) and lead acetate (3.94g, 1.78 mL, 8.89 mmol, 2 eq) were added to the solution of compound LY-22Cl-int-b (2.02g, 4.44 mmol, 1 eq) in DMF (20 mL), and the reaction solution was stirred at 60°C for 1 hour. LCMS showed that the reaction ended. The reaction solution was spun to dryness under reduced pressure, saturated $NaHCO_3$ solution was added to the residue to adjust pH to 7, DCM/MeOH (10:1, 2000 mL) was added, the mixture solution was extracted with DCM/MeOH (10:1) 3 times, 1000 mL of DCM/MeOH was used each time, the organic phases were combined and washed with saturated brine (1000 mL), the organic phase was dried with anhydrous $Na_2SO_4$, and the mixture solution was filtered and then spun to dryness under reduced pressure. The residue was purified by silica gel column chromatography (eluents: methanol: dichloromethane = 0:1-10:1) to obtain the white solid product LY-22Cl-int-c (540 mg, yield: 25.93%). LCMS (ESI): m/z, 491.3[M+Na]$^+$.

Step 4: Preparation of compound LY-22Cl-int-d

Methanesulfonic acid (73.51 mg, 426.89 μmol, 1 eq) was added to the solution of compound LY-22Cl-int-c (200 mg, 426.89 μmol, 1 eq) and 4-(tert-butyldimethylsilyl)oxo-1-butanol (174.49 mg, 853.77 μmol, 2 eq) in THF (5 mL) and the reaction solution was stirred at 35°C for 2 hours. LCMS showed that the reaction ended. The reaction solution was spun to dryness under reduced pressure. The mixture was purified by preparative high performance liquid chromatograph (preparative chromatograph manufacturer: Oriendo, model: BRIX-2860, column: Welch Xtimate C18 150 × 30 mm × 10 μm, mobile phase: water (0.225% HCOOH)-acetonitrile, an elution ratio of water: 20% to 50%), to obtain a white solid product LY-22Cl-int-d (48.80 mg, yield: 22.93%). LCMS (ESI): m/z, 521.3[M+Na]$^+$.

Step 5: Preparation of compound LY-22Cl-int-e

Bis(p-nitrobenzene)carbonate (765.13 mg, 510.09 μL, 2.52 mmol, 6 eq) and DIPEA (325.06 mg, 415.67 μL, 2.52 mmol, 6 eq) were added to the solution of the compound LY-22Cl-int-d (209 mg, 419.19 μmol, 1 eq) in DMF (5 mL), and the reaction solution was stirred at 35°C for 2 hours. LCMS showed that the reaction ended. The reaction solution was spun to dryness under reduced pressure. The residue was purified by silica gel column chromatography (eluents: methanol: dichloromethane = 0:1-10:1) to obtain the white solid product LY-22Cl-int-e (223 mg, yield: 80.16%). LCMS (ESI): m/z, 686.4[M+Na]$^+$.

Step 6: Preparation of compound LY-22Cl-int-f

DIPEA (100.21 mg, 775.40 μL, 3 eq) and HOBT (34.92 mg, 258.47 μmol, 1 eq) were added to the solution of compound LY-22Cl-int-e (223 mg, 336.01 μmol, 1.3 eq) and exatecan mesylate (137.40 mg, 258.47 μmol, 1 eq) in DMF (5 mL), and the reaction solution was stirred for 2 hours at room temperature. LCMS showed that the reaction ended. The reaction solution was purified by silica gel column chromatography (C18) (ISCO, R-120g SepaFlash Silica Flash Column, Eluent of 10%-50% water/$CH_3CN$, 60 mL/min) to obtain a white solid product LY-22Cl-int-f (277 mg, no yield was calculated for the crude product). LCMS (ESI): m/z, 960.5[M+H]$^+$.

Step 7: Preparation of compound LY-22Cl-int

Diethylamine (211.03 mg, 2.89 mmol, 10 eq) was added to the solution of compound LY-22Cl-int-f (277 mg, crude product) in DMF (3 mL), the reaction solution was stirred at room temperature for 2 hours, and the LCMS showed that the raw materials completed reaction. The reaction solution was purified by silica gel column chromatography (C18) (ISCO, R-120g SepaFlash Silica Flash Column, Eluent of 5-35% water/$CH_3CN$, 60 mL/min) to obtain a white solid LY-22Cl-int (105 mg, yield: 49.32%). LCMS (ESI): m/z, 738.5[M+H]$^+$.

Step 8: Preparation of compound LY-22Cl-a

$NaHCO_3$ (29.71 mg, 353.59 μmol, 1 eq) was added to the solution of glycyl-L-valine (61.60 mg, 353.59 μmol, 1 eq) in $H_2O$ (5 mL) at room temperature, the reaction solution was stirred at 25°C for 30 minutes, and then the solution of N6-[(1,1-dimethylethoxy)carbonyl]-N2-[(9H-fluoren-9-ylmethoxy)carbonyl]-L-lysine 2,5-dioxo-1-pyrrolidinyl ester (200 mg, 353.59 μmol, 1 eq) in THF (5 mL) was added. After addition, the reaction solution was stirred for 90 minutes at 25°C. The LCMS showed that the reaction ended. The solvent was removed from the reaction solution under reduced pressure, pH was adjusted to 2 with 1N HCl, the reaction solution was then extracted with ethyl acetate (200 mL × 3), the organic phases were combined and washed with saturated brine (60 mL), dried with anhydrous sodium sulfate, and spun

to dryness under reduced pressure to obtain a white oily compound LY-22CI-a (240 mg, crude product).

LCMS (ESI): m/z, 647.5[M+Na]$^+$.

Step 9: Preparation of compound LY-22CI-b

DIEA (21.02 mg, 26.88 μL, 162.65 μmol, 3 eq) and HATU (30.92 mg, 81.32 μmol, 1.5 eq) were separately added to the solution of LY-22CI-a (38.63 mg, 54.22 μmol, 1 eq) and LY-22CI-int (40 mg, 54.22 μmol, 1 eq) in DMF (1 mL) at 0°C. After addition, the reaction solution was stirred for 1 hour at 25°C. The LCMS showed that the reaction ended. The reaction solution was subjected to preparative high performance liquid chromatograph (preparative chromatograph manufacturer: Oriendo, model: BRIX-2860, Phenomenex Luna C18 (250×30 mm×10 μm) was used as a chromatographic column, mobile phase: water (0.225% HCOOH)-acetonitrile, an elution ratio of water: 50% to 80%) and lyophilized, to obtain a white solid compound LY-22CI-b (23 mg, yield: 31.55%).

LCMS (ESI): m/z, 1367.7[M+Na]$^+$.

Step 10: Preparation of compound LY-22CI-c

Trifluoroacetic acid (0.5 mL) was added to the solution of compound LY-22CI-b (23 mg, 17.11 μmol, 1 eq) in DCM (2.5 mL) at 0°C, and after addition, the reaction solution was stirred at 0°C for 30 minutes. The LCMS showed that the reaction ended. The reaction solution was directly spun to dryness to obtain the yellow oily compound LY-22CI-c (21 mg, crude product).

LCMS (ESI): m/z, 1245.7[M+H]$^+$.

Step 11: Preparation of compound LY-22CI-d

DIEA (13.09 mg, 16.73 μL, 101.25 μmol, 6 eq) and HATU (19.25 mg, 50.63 μmol, 3 eq) were separately added to the solution of compounds LY-22CI-c (21 mg, 16.88 μmol, 1 eq) and LY-10b (for synthesis of the compound, refer to the synthesis example of LY-10) (38.97 mg, 50.63 μmol, 3 eq) in DMF (1 mL) at 0°C. After addition, the reaction solution was stirred for 1 hour at 25°C. The LCMS showed that the reaction ended. The reaction solution was purified by preparative high performance liquid chromatograph (preparative chromatograph manufacturer: Oriendo, model: BRIX-2860, Phenomenex Luna C18 (250×30 mm×10 μm) was used as a chromatographic column, mobile phase: water (0.225% HCOOH)-acetonitrile, an elution ratio of water: 30% to 60%) and lyophilized, to obtain a white solid compound LY-22CI-d (5 mg, yield: 14.84%).

LCMS (ESI): m/z, 998.4[(M+2)/2]$^+$.

Step 12: Preparation of compound LY-22CI-e

Diethylamine (915.99 μg, 1.29 μL, 12.52 μmol, 5 eq) was added to the solution of compound LY-22CI-d (5 mg, 2.50 μmol, 1 eq) in DMF (0.5 mL) at 0°C, and after addition, the reaction solution was stirred at 0°C for 30 minutes. The LCMS showed that the reaction ended. The reaction solution was directly spun to dryness to obtain the light yellow oily compound LY-22CI-e (4.44 mg, crude product).

LCMS (ESI): m/z, 887.7[(M+2)/2]$^+$.

Step 13: Preparation of compound LY-22CI

LY-39b (for synthesis of the compound, refer to the synthesis example of LY-39) (2.03 mg, 90%, 5.01 μmol, 2 eq) and DIEA (970.41 μg, 1.24 μL, 7.51 μmol, 3 eq) were sequentially added to the solution of LY-22CI-e (4.44 mg, 2.50 μmol, 1 eq) in DMF (0.5 mL), and after addition, the reaction solution was stirred at 25°C for 1 hour. LCMS showed that the reaction ended. The reaction solution was purified by high performance liquid chromatograph (preparative chromatograph manufacturer: SHIMADZU, model: LC-20AP, column: Welch Xtimate C18 150 × 21.2 mm × 5 μm, mobile phase: water (0.225% FA)-acetonitrile, an elution ratio of water: 30% to 60%) and lyophilized, to obtain a white solid compound LY-22CI (1.92 mg, yield: 37.90%).

LCMS (ESI): m/z, 1012.8[(M+2)/2]$^+$.

Example 21: Preparation of compound LY-22CJ

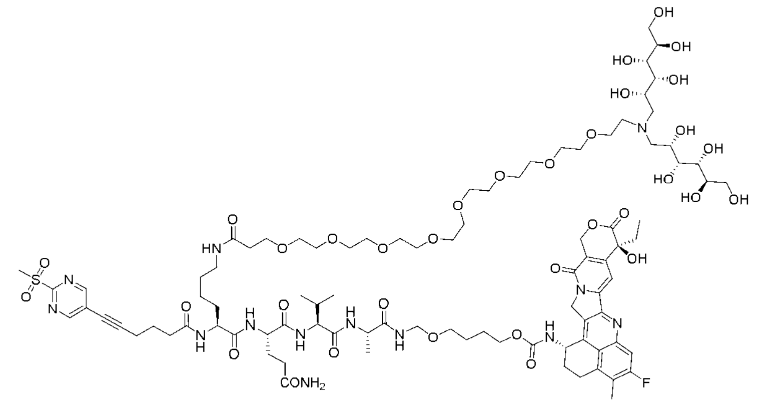

LY-22CJ

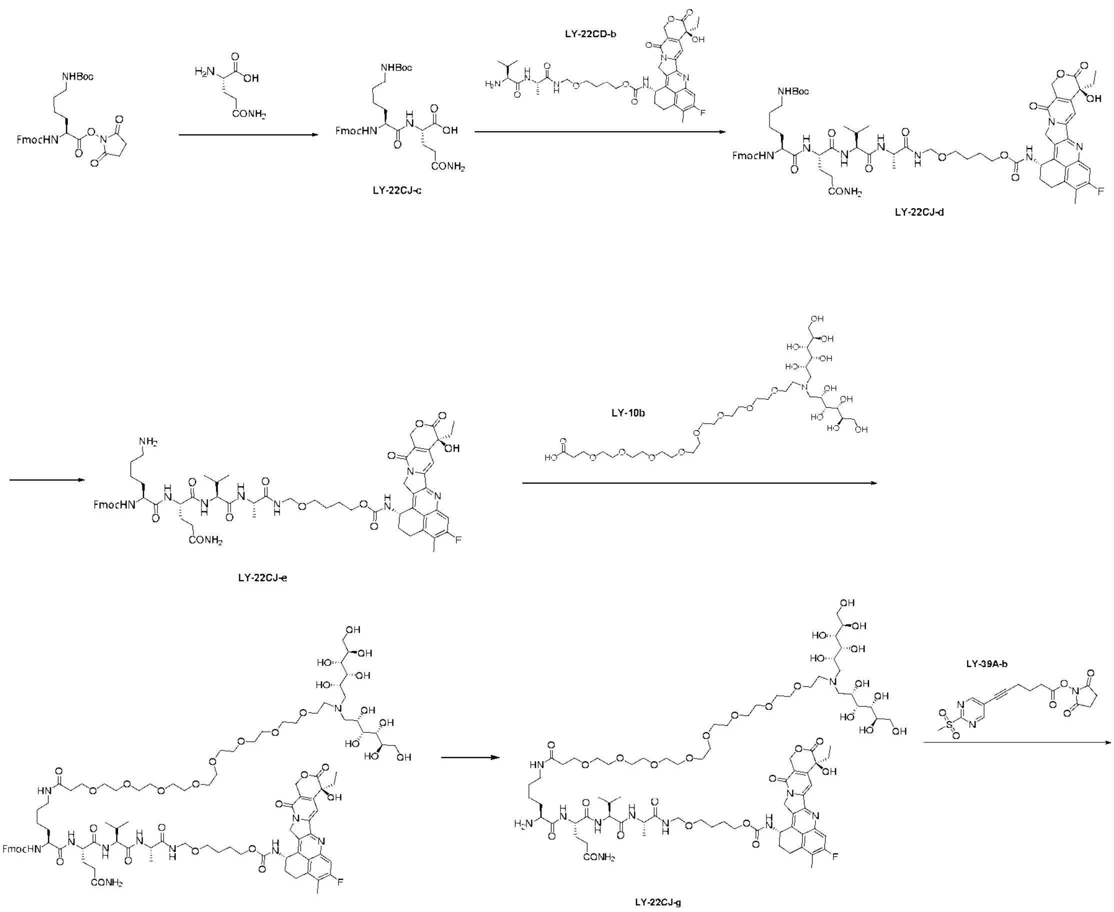

LY-22CD-b

LY-22CJ-c

LY-22CJ-d

LY-10b

LY-22CJ-e

LY-22CJ-f

LY-22CJ-g

LY-39A-b

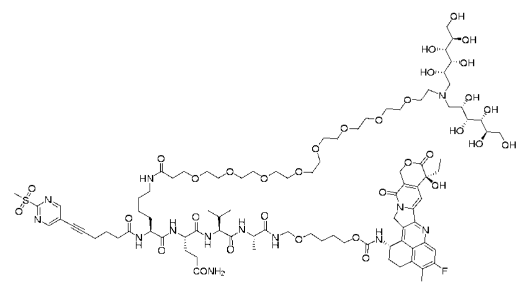

LY-22CJ

Step 1: Preparation of compound N2-(((9H-fluoren-9-yl)methoxy)carbonyl)-N6-(tert-butoxycarbonyl)-L-lysyl-L-glutamine

L-glutamine ethyl ester (194.65 mg, 1.87 mmol, 3 eq) was dissolved in water (15 mL), $NaHCO_3$ (96.54 mg, 52.47 μL, 1.15 mmol, 1.3 eq) was added, the solution of 2,5-dioxopyrrolidin-1-ylN2-(((9H-fluoren-9-yl)methoxy)carbonyl)-N6-(tert-butoxycarbonyl)-L-lysine salt (500 mg, 883.98 μmol, 1 eq) in THF (15 mL) was then added at 0°C, and the reaction solution was stirred at 25°C for 12 hours. The LCMS showed that the reaction ended, the reaction solution was adjusted with an aqueous solution of 1N hydrochloric acid to adjust pH to 5, and extracted with ethyl acetate (50 mL × 2), and the organic phase was dried with anhydrous sodium sulfate, filtered, and concentrated. The residue was purified by silica gel column chromatography (C18) (ISCO, R-220g SepaFlash Silica Flash Column, Eluent of 10%-50% water/$CH_3CN$, 100 mL/min), to obtain a white solid (500 mg, yield: 94.80%).
LCMS (ESI): m/z, 619.4[M+Na]$^+$.

Step 2: Preparation of compound LY-22CJ-d

LY-22CD-b (for synthesis of the compound, refer to the synthesis example of LY-22CD) (37.75 mg, 50.28 μmol, 1 eq) and DIPEA (13 mg, 16.62 μL, 100.56 μmol, 2 eq) were added to a solution of compound N2-(((9H-fluoren-9-yl)methoxy)carbonyl)-N6-(tert-butoxycarbonyl)-L-lysyl-L-glutamine (30 mg, 50.28 μmol, 1 eq) in DMF (1 mL), HATU (28.68 mg, 75.42 μmol, 1.5 eq) was added at 0°C, the reaction solution was stirred at 25°C for 1 hour, and the LCMS showed that the target product was generated. The reaction solution was purified by high performance liquid chromatography to obtain the white solid product LY-22CJ-d (30 mg, yield: 44.88%).
The conditions of the high performance liquid chromatography were as follows:
preparative chromatograph manufacturer: Oriendo, model: BRIX-2860, column: Synergi Max-RP 200 × 30 mm × 10 μm, mobile phase: water (0.225% HCOOH)-acetonitrile, an elution ratio of water: 30% to 60%.
LCMS (ESI): m/z, 1329.9[M+H]$^+$.

Step 3: Preparation of compound LY-22CJ-e

TFA (3.84g, 2.50 mL, 33.66 mmol, 1789.834 eq) was added to the solution of LY-22CJ-d (25 mg, 18.80 μmol, 1 eq) in DCM (3 mL) at 0°C, the reaction solution was stirred at 10°C for 0.5 hours, and the LCMS showed that the raw materials completed reaction. The reaction solution was directly spun to dryness to obtain a yellow oily product (20 mg, yield: 86.52%), to be directly used for the next step.
LCMS (ESI): m/z, 1229.7 [M+H]$^+$.

Step 4: Preparation of compound LY-22CJ-f

LY-10b (25.05 mg, 32.54 μmol, 2 eq) and DIPEA (2.10 mg, 2.69 μL, 16.27 μmol, 1 eq) were added to the solution of compound LY-22CJ-e (20 mg, 16.27 μmol, 1 eq) in DMF (1 mL), HATU (9.28 mg, 24.40 μmol, 1.5 eq) was added at 0°C, and the reaction solution was stirred at 25°C for 1 hour. The LCMS showed that the raw materials completed reaction. The reaction solution was purified by high performance liquid chromatography to obtain the white solid product LY-22CJ-f (20 mg, yield: 62.05%).
The conditions of the preparative high performance liquid chromatography were as follows:
preparative chromatograph manufacturer: Oriendo, model: BRIX-2860, column: Welch Xtimate C18 150 × 30 mm × 10 μm, mobile phase: water (0.225% HCOOH)-acetonitrile, an elution ratio of water: 20% to 50%.
LCMS (ESI): m/z, 1982.0[M+H]$^+$.

Step 5: Preparation of compound LY-22CJ-g

$Et_2NH$ (1.85 mg 2.60 μL, 25.24 μmol, 5 eq) was added to the solution of LY-22CJ-f (10 mg, 5.05 μmol, 1 eq) in DMF (1 mL), the reaction solution was stirred at room temperature for 1 hour, and the LCMS showed that the raw materials completed reaction. The reaction solution was directly spun to dryness to obtain a yellow oily product (8 mg, yield: 90.11%) to be directly used for the next step.
LCMS (ESI): m/z, 880.1[(M+2H)/2]$^+$.

Step 6: Preparation of compound LY-22CJ

2,5-dioxopyrrolidin-1-yl-6-(2-(methylsulfonyl)pyrimidin-5-yl)hex-5-ynoate (1.76 mg 2.26 μL, 13.64 μmol, 3 eq) and DIPEA (1.76 mg, 2.26 μL, 13.64 μmol, 3 eq) were added to the solution of compound LY-22CJ-g (8 mg, 4.55 μmol, 1

eq) in DMF (1 mL), the reaction solution was stirred at room temperature for 1 hour, and the LCMS showed that the raw materials completed reaction. The reaction solution was subjected to preparative high performance liquid chromatography to obtain a white solid product LY-22CJ (1.23 mg, yield: 13.46%, purity: 96%).

The conditions of the high performance liquid chromatography were as follows: preparative chromatograph manufacturer: Oriendo, model: BRIX-2860, column: Welch Xtimate C18 250 × 30 mm × 10 μm, mobile phase: water (0.225% HCOOH)-acetonitrile, an elution ratio of water: 20% to 50%.

$^1$H NMR (400 MHz, DMSO-d6) δ 9.11 (s, 2H), 8.59 (t, *J* = 6.6 Hz, 1H), 8.11 (d, *J* = 7.6 Hz, 1H), 8.04 (dd, *J* = 7.5, 4.4 Hz, 2H), 7.92 (d, *J* = 8.9 Hz, 1H), 7.82-7.75 (m, 2H), 7.69 (d, *J* = 8.7 Hz, 1H), 7.31 (s, 1H), 7.25 (s, 1H), 6.82-6.74 (m, 1H), 6.53 (s, 1H), 5.43 (s, 2H), 5.24 (s, 3H), 4.66-4.39 (m, 6H), 4.33 (q, *J* = 5.7 Hz, 2H), 4.28 - 4.02 (m, 7H), 3.75 (t, *J* = 7.9 Hz, 1H), 3.56 (t, *J* = 6.5 Hz, 5H), 3.54-3.43 (m, 23H), 3.41 (s, 3H), 3.23 (s, 8H), 3.16-3.03 (m, 4H), 2.98 (d, *J* = 6.8 Hz, 3H), 2.59-2.52 (m, 3H), 2.38 (d, *J* = 1.9 Hz, 3H), 2.34-2.23 (m, 4H), 2.22-2.05 (m, 5H), 1.89-1.87 (m, 9H), 1.67-1.42 (m, 7H), 1.40-1.14 (m, 8H), 0.92 - 0.73 (m, 8H).

Example 22: Preparation of compound LY-22F

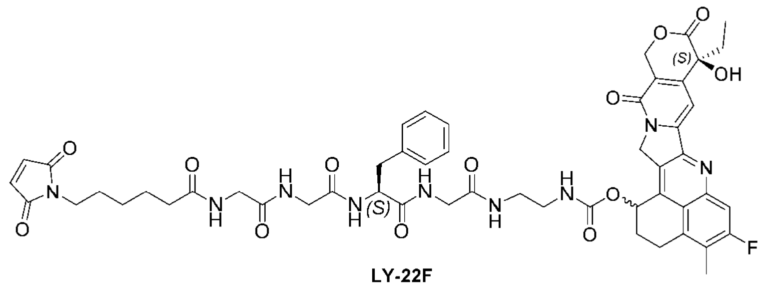

**LY-22F**

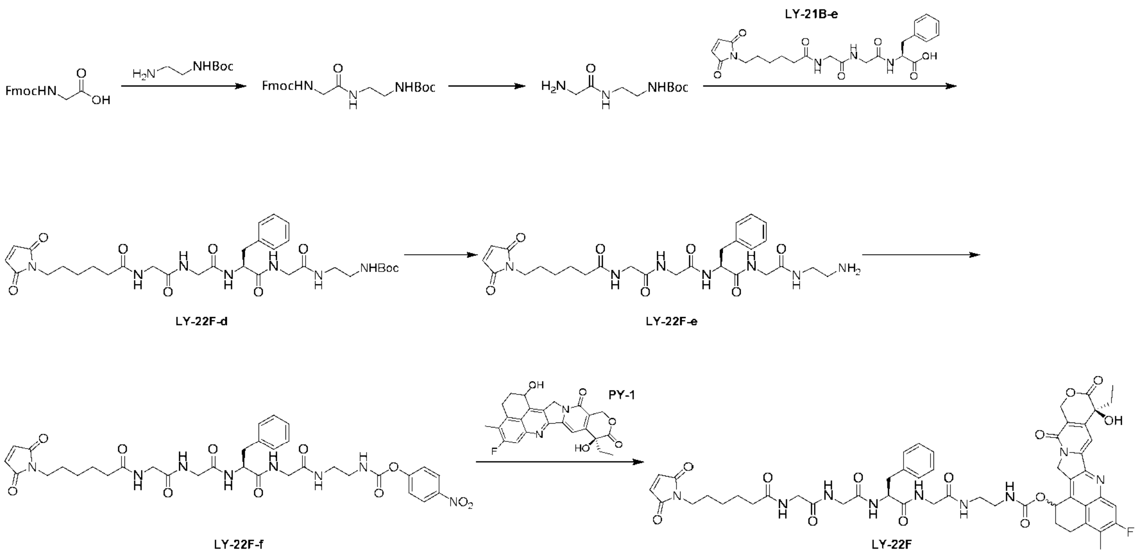


Step 1: Preparation of N-{2-[2-({[(9H-fluoren-9-yl)methoxy]carbonyl}amino)acetamide]ethyl}tert-butyl carbamate

((9H-fluoren-9-yl)methoxy)carbonyl)glycine (300 mg, 1.01 mmol, 1 eq) was dissolved in DMF (10 mL) in the nitrogen atmosphere. The compound N-tert-butoxycarbonylethylenediamine (193.99 mg, 190.94 μL, 1.21 mmol, 1.2 eq) and DIPEA (195.61 mg, 250.15 μL, 1.51 mmol, 1.5 eq) were added at room temperature, and then the HATU (575.52 mg, 1.51 mmol, 1.5 eq) was added. The reaction solution was stirred for 2 hours at 15°C. The reaction solution was directly used for the next reaction. The mass of the product was calculated according to the theoretical yield (440 mg, yield: 99.21%).

LCMS (ESI): m/z, 462.2 [M+Na]$^+$.

Step 2: Preparation of N-[2-[(2-aminoacetyl)amino]ethyl]-carbamate 1,1-dimethylethyl ester N-{2-[2-({[(9H-fluoren-9-yl)methoxy]carbonyl}amino)acetamide]ethyl}tert-butyl carbamate (440 mg, 1 mmol, 1 eq) was dissolved in DMF (5 mL), DBU (76.20 mg, 74.78 μL, 500.56 μmol, 0.5 eq) was added to the solution, and the solution was stirred at

20°C for 2 hours. The reaction solution was directly used for the next step without any treatment, and the mass of the product was calculated according to the theoretical amount 200 mg (yield: 91.95%).

LCMS (ESI): m/z, 259.3 $[M+H+MeCN]^+$.

Step 3: Preparation of compound LY-22F-d

LY-21B-e (217.47 mg, 460.26 μmol, 1 eq) was added to the solution of compound (2-(2-aminoacetylamino)ethyl) tert-butyl carbamate (100 mg, 460.26 μmol, 1 eq) in DMF (3 mL) at 0°C, then TsOH (39.63 mg, 37.04 μL, 230.13 μmol, 0.5 eq) and HOBt (62.19 mg, 41.46 μL, 460.26 μmol, 1 eq) were sequentially added, finally EDCI (88.23 mg, 100.61 μL, 460.26 μmol, 1 eq) was added, and the reaction solution was stirred at 20°C for 2 hours. The reaction solution was purified by preparative high performance liquid chromatograph (preparative chromatograph manufacturer: Oriendo, BRIX-2860 (R1, 4, 5, 6), column: Synergi Max-RP 250 × 40 mm × 10 μm, mobile phase: water (0.225% HCOOH)-acetonitrile, an elution ratio of water: 21% to 51%), to obtain a white solid product (120 mg, yield: 38.81%).

LCMS (ESI): m/z, 694.3 $[M+Na]^+$.

Step 4: Preparation of compound LY-22F-e

The compound LY-22F-d (120 mg, 178.64 μmol, 1 eq) was dissolved in DCM (3 mL)/TFA (1 mL) and the reaction solution was stirred at 15°C for 2 hours. The reaction solution was concentrated under reduced pressure, to obtain a yellow oily product (100 mg, yield: 97.93%).

LCMS (ESI): m/z, 572.3 $[M+H]^+$.

Step 5: Preparation of compound LY-22F-f

The compound LY-22F-e (100 mg, 174.94 μmol, 1 eq) was dissolved in DMF (3 mL), DIPEA (67.83 mg, 86.73 μL, 524.81 μmol, 3 eq) was added to the reaction solution, and then bis(4-nitrophenyl)carbonate (106.43 mg, 70.96 μL, 349.87 μmol, 2 eq) was added. The reaction solution was stirred for 12 hours at 30°C. The reaction solution was concentrated under reduced pressure, and the residue was purified by silica gel column chromatography (eluents: dichloromethane/methanol = 1:0-1:10) to obtain a colorless oily product (120 mg, yield: 93.11%).

LCMS (ESI): m/z, 737.3$[M+H]^+$.

Step 6: Preparation of compound LY-22F

The compound LY-22F-f (25.32 mg, 34.37 μmol, 1.5 eq) was dissolved in DMF (1 mL), and PY-1 (10 mg, 22.91 μmol, 1 eq) and DIPEA (8.88 mg, 11.36 μL, 68.74 μmol, 3 eq) were added. The reaction solution was purified by preparative high performance liquid chromatograph (preparative chromatograph manufacturer: Oriendo, BRIX-2860 (R1, 4, 5, 6), column: Synergi Max-RP 250 × 40 mm × 10 μm, mobile phase: water (0.225% HCOOH)-acetonitrile, an elution ratio of water: 21% to 51%), to obtain two batches of white solid products (with different retention times, where 2 isomers were probably obtained), and the masses of products were separately 1.67 mg (yield: 6.77%, purity: 96%) and 2.46 mg (yield: 9.97%, purity: 96%).

LCMS (ESI): m/z, 1056.4 $[M+Na]^+$.

Example 23: Preparation of compound LY-22AD

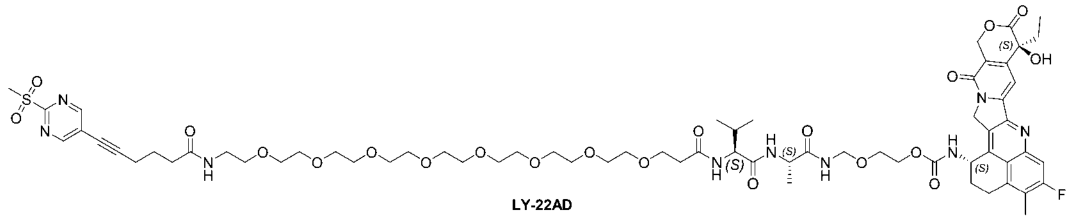

LY-22AD

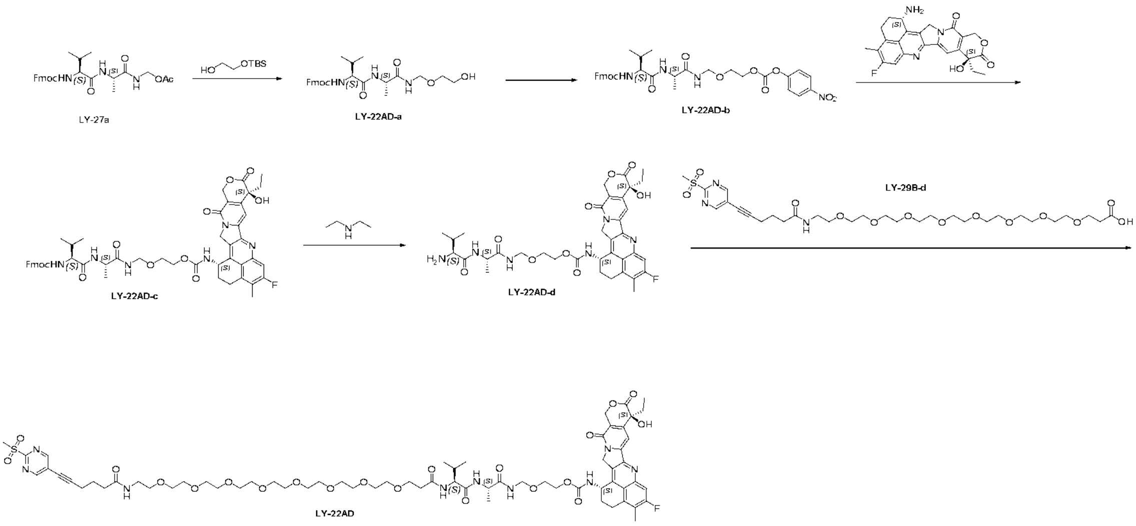


Step 1: Preparation of compound LY-22AD-a

2-((tert-butyldimethylsilyl)oxy)ethane-1-ol (585.87 mg, 3.32 mmol, 2 eq) and TsOH (85.82 mg, 80.21 μL, 498.39 μmol, 0.3 eq) were added to the solution of compound LY-27a (800 mg, 1.66 mmol, 1 eq) in THF (10 mL) at 0°C, and after addition, the reaction solution reacted at 30°C (ambient temperature) for 3 hours. The reaction solution was cooled to room temperature and concentrated under reduced pressure, and the residue was purified by conventional reversed-phase Flash and lyophilized to obtain the white solid compound LY-22AD-a (342 mg, yield: 42.57%).

LCMS (ESI): m/z, 506.3$[M+H]^+$, Rt=1.817 min.

Step 2: Preparation of compound LY-22AD-b

Bis(p-nitrobenzene)carbonate (50.33 mg, 33.55 μL, 165.44 μmol, 2 eq) and DIEA (32.07 mg, 41.01 μL, 248.15 μmol, 3 eq) were added to the solution of compound LY-22AD-a (43.01 mg, 93%, 82.72 μmol, 1 eq) in DMF (4 mL) at 0°C. After addition, the reaction solution was stirred for 2 hours at 30°C (ambient temperature). The reaction solution was concentrated under reduced pressure to obtain a crude product. The crude product was purified by silica gel column chromatography (eluents: dichloromethane/methanol = 20:1-10:1) to obtain compound LY-22AD-b (61 mg, yield: 80.72%, purity: 71%).

LCMS (ESI): m/z, 671.3$[M+H]^+$. Rt = 2.645 min.

Step 3: Preparation of compound LY-22AD-c

Exatecan mesylate (35.49 mg, 66.77 μmol, 1 eq) was dissolved in DMA (3 mL), DIPEA (8.63 mg, 11.03 μL, 66.77 μmol, 1 eq) was added, the reaction solution was stirred at 25°C for 10 minutes, then LY-22AD-b (61 mg, 71%, 66.77 μmol, 1 eq), HOBt (4.51 mg, 3.01 μL, 33.38 μmol, 0.5 eq) and pyridine (26.41 mg, 27 μL, 333.84 μmol, 5 eq) were added. The reaction solution was stirred for 3 hours at 25°C in the nitrogen atmosphere. LCMS showed that 7% of the raw materials remained, DIPEA (17 mg, 2.0 eq) was added again, and the reaction solution was further stirred at 25°C for 2 hours. The reaction solution was purified by preparative high performance liquid chromatograph (preparative chromatograph manufacturer: Oriendo, model: BRIX-2860, column: Welch Xtimate C18 250 × 30 mm × 10 μm, mobile phase: water (0.225% FA)-acetonitrile, an elution ratio of water: 50% to 80%) and lyophilized, to obtain a white solid product LY-22AD-c (43 mg, crude product, yield: 61.34%, purity: 90%).

LCMS (ESI): m/z, 945.5$[M+H]^+$. Rt = 2.606 min.

Step 4: Preparation of compound LY-22AD-d

Diethylamine (14.98 mg, 21.09 μL, 204.76 μmol, 5 eq) was added to the solution of compound LY-22AD-c (43 mg, 90%, 40.95 μmol, 1 eq) in DMF (3 mL) at 0°C, and after addition, the reaction solution was stirred at 25°C for 2 hours. The reaction solution was concentrated under reduced pressure, to obtain a yellow solid compound LY-22AD-d (29 mg, no yield was calculated for the crude product).

LCMS (ESI): m/z, 745.4$[M+Na]^+$, Rt = 1.323 min.

Step 5: Preparation of compound LY-22AD

DIEA (8.05 mg, 10.29 μL, 62.26 μmol, 3 eq) and HATU (9.47 mg, 24.90 μmol, 1.2 eq) were added to the solution of compounds LY-29B-d (16.89 mg, 85%, 20.75 μmol, 1 eq) and LY-22AD-d (15 mg, 20.75 μmol, 1 eq) in DMF (3 mL) at 0°C. After addition, the reaction solution reacted at 25°C for 2 hours. The reaction solution was purified by preparative high performance liquid chromatograph (preparative chromatograph manufacturer: Oriendo, model: BRIX-2860, column: YMC-Triart Prep C18 150 $\times$ 30 mm $\times$ 10 μm, mobile phase: water (0.225% FA)-acetonitrile, an elution ratio of water: 30% to 60%) and lyophilized, to obtain a white solid compound LY-22AD (6.18 mg, yield: 21.32%, purity: 97.98%).

$^1H$ NMR (400 MHz, DMSO-$d^6$) $\delta$ 9.11 (s, 2H), 8.59 (s, 1H), 8.04 (d, *J* = 6.9 Hz, 2H), 7.93 (s, 1H), 7.85 (d, *J* = 8.7 Hz, 1H), 7.78 (d, *J* = 10.9 Hz, 1H), 7.31 (s, 1H), 6.52 (s, 1H), 5.43 (s, 2H), 5.25 (s, 3H), 4.57 (dd, *J* = 16.9, 6.6 Hz, 2H), 4.26 - 4.10 (m, 4H), 3.59 (d, *J* = 6.3 Hz, 3H), 3.53 - 3.44 (m, 22H), 3.40 (d, *J* = 2.8 Hz, 10H), 3.20 (q, *J* = 5.8 Hz, 4H), 2.57 - 2.53 (m, 2H), 2.43 (t, *J* = 6.9 Hz, 2H), 2.40 - 2.36 (m, 3H), 2.26 (t, *J* = 7.3 Hz, 2H), 2.17 (d, *J* = 9.7 Hz, 2H), 1.94 - 1.76 (m, 5H), 1.20 (d, *J* = 7.1 Hz, 3H), 0.91 - 0.75 (m, 8H).

LCMS (ESI): m/z, 1418.8$[M+Na]^+$, Rt=1.803 min. HPLC:Rt = 4.812 min.

Example 24: Preparation of compound LY-22AF

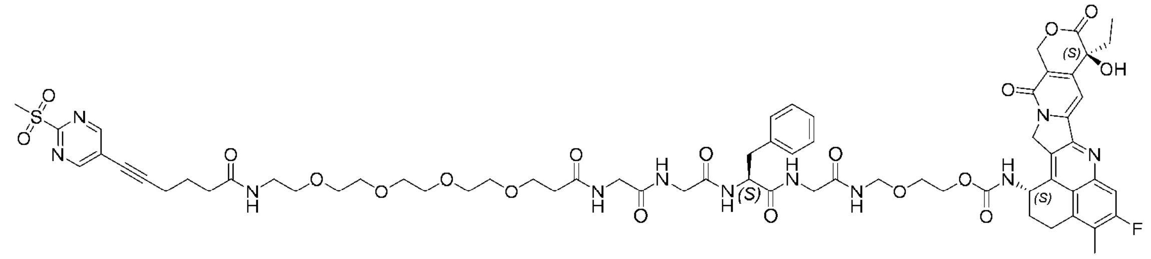

LY-22AF

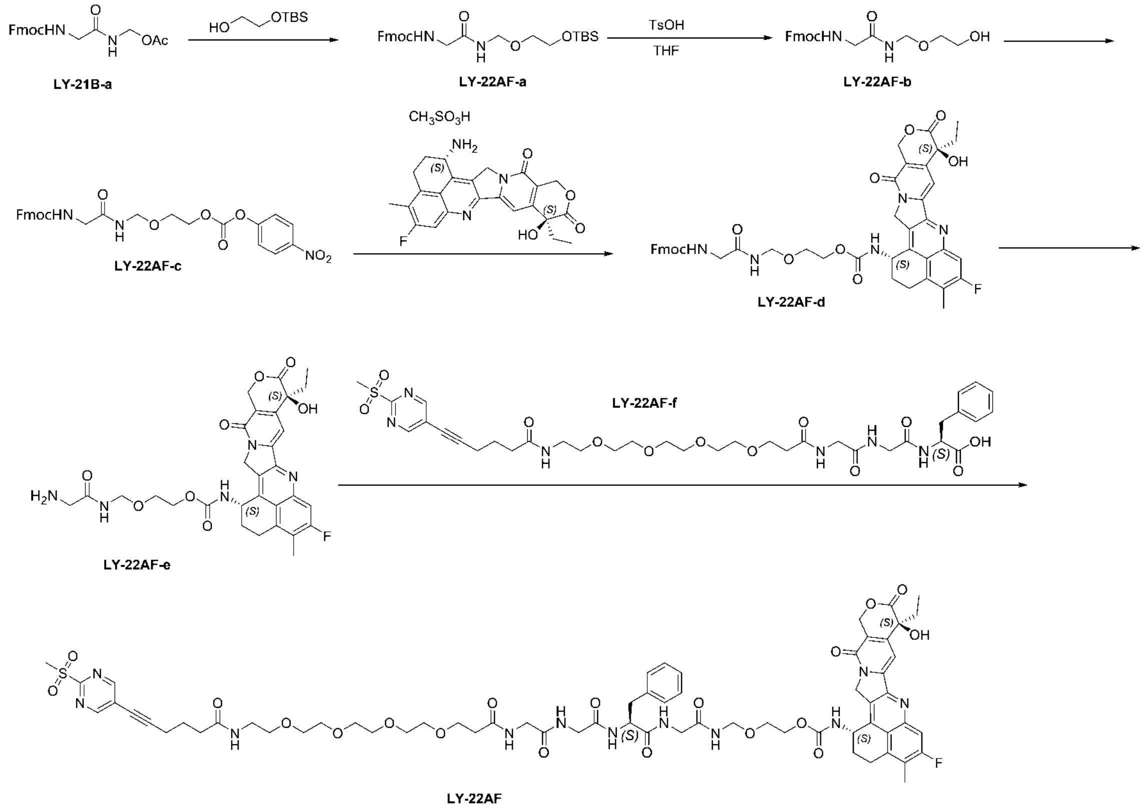


Step 1: Preparation of compound LY-22AF-a

2-((Tert-butyldimethylsilyl)oxy)ethane-1-ol (311.1 mg, 2.0 eq) and compound (2-((((9H-fluoren-9-yl)methoxy) carbonyl)amino)acetamide)methyl acetate (Chengdu Zen Sunda) (325 mg, 1.0 eq) were dissolved in anhydrous tetrahydrofuran (15 mL), p-toluenesulfonic acid (45.58 mg, 0.3 eq) was added, and then the reaction solution was stirred for reaction at 25°C for 1 hour. The reaction solution was directly used for the next step.

LCMS: m/z = 507.3 $[M+Na]^+$; Rt = 3.030 min.

Step 2: Preparation of compound LY-22AF-b

P-toluenesulfonic acid (45.51 mg, 0.3 eq) was added to the reaction solution of compound LY-22AF-a (427 mg) and then the resulting solution was stirred at 25°C for 1 hour. The reaction solution was concentrated under reduced pressure, the residue was dissolved in a small amount of DMF and then filtered, and the filtrate was purified by preparative high performance liquid chromatograph (preparative chromatograph manufacturer: SHIMADZU, model: LC-20AP, column: Welch Xtimate C18 250 × 50 mm × 10 μm, mobile phase: water (0.225% HCOOH)-acetonitrile, an elution ratio of water: 20% to 50%), to obtain compound LY-22AF-b (190 mg, yield: 58.2%).

LCMS: m/z = 393.2 $[M+Na]^+$; Rt = 1.496 min.

Step 3: Preparation of compound LY-22AF-c

The compound LY-22AF-b (370 mg, 1.0 eq) was dissolved in anhydrous DMF (5 mL), DIPEA (471 μL, 3.0 eq) and

bis(p-nitrobenzene)carbonate (577.4 mg, 2.0 eq) were added at 0°C, and the reaction solution was heated to 25°C and stirred for reaction for 2 hours. The reaction solution was filtered, and the filtrate was purified by preparative high performance liquid chromatograph (preparative chromatograph manufacturer: Oriendo, model: BRIX-2860, column: Phenomenex Luna C18 250 × 50 mm × 10 μm, mobile phase: water (0.225% HCOOH)-acetonitrile, an elution ratio of water: 30% to 60%), to obtain compound LY-22AF-c (235 mg, yield: 32.4 %, purity: 70.4%).

LCMS: m/z = 558.3 $[M+Na]^+$; Rt = 2.433 min.

Step 4: Preparation of compound LY-22AF-d

Exatecan mesylate (76.3 mg, 1.0 eq) was dissolved in anhydrous DMF (5 mL), DIPEA (23.7 μL, 1.0 eq) was added, and the reaction solution was stirred at 25°C for ten minutes. Then compound LY-22AF-c (142.8 mg, 1.3 eq), pyridine (58.1 μL, 5.0 eq), and HOBT (9.7 mg, 0.5 eq) were added sequentially, DIPEA (71.2 μL, 3.0 eq) was added again, and the reaction solution was stirred at 25°C for reaction for 2 hours. The reaction solution was filtered, and the filtrate was purified by preparative high performance liquid chromatograph (preparative chromatograph manufacturer: Oriendo, model: BRIX-2860, column: Welch Xtimate C18 250 × 30 mm × 10 μm, mobile phase: water (0.225% HCOOH)-acetonitrile, an elution ratio of water: 30% to 60%), to obtain compound LY-22AF-d (130 mg, yield: 80%, purity: 85.8%).

LCMS: m/z = 854.3 $[M+Na]^+$; Rt = 2.303 min.

Step 5: Preparation of compound LY-22AF-e

The compound LY-22AF-d (110 mg, 1.0 eq) was dissolved in anhydrous DMF (2 mL), diethylamine (117.1 μL, 10 eq) was added, and the reaction solution was stirred at 25°C for reaction for 2 hours. The reaction solution was concentrated under reduced pressure, and the crude product was directly used for the next reaction.

LCMS: m/z = 610.3 $[M+H]^+$; Rt = 1.169 min.

Step 6: Preparation of compound LY-22AF

The compounds LY-22AF-e (40 mg, 1.0 eq) and LY-22CE-f (63.7 mg, 1.0 eq) were dissolved in anhydrous DMF (2 mL), DIPEA (32.5 μL, 3.0 eq) and HATU (37.4 mg, 1.5 eq) were added at 0°C, and then the reaction solution was heated to 25°C and stirred for reaction for 2 hours. The reaction solution was filtered, and the filtrate was purified by preparative high performance liquid chromatograph (preparative chromatograph manufacturer: SHIMADZU, model: LC-20AP, column: Welch Xtimate C18 250 × 30 mm × 10 μm, mobile phase: water (0.225% HCOOH)-acetonitrile, an elution ratio of water: 30% to 60%), to obtain the product LY-22AF (8.10 mg, yield: 8.67%).

LCMS: m/z = 1391.5 $[M+Na]^+$; Rt = 1.730 min.

Example 25: Preparation of compound LY-22CA

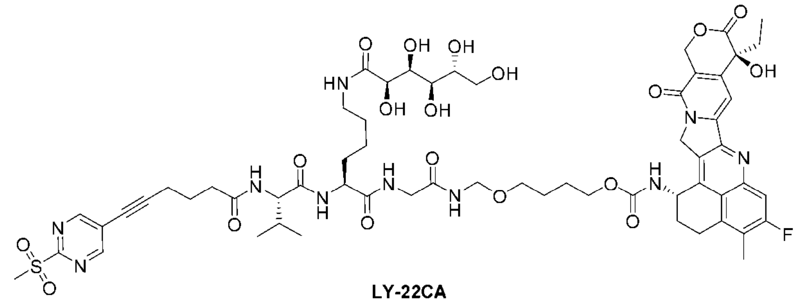

LY-22CA

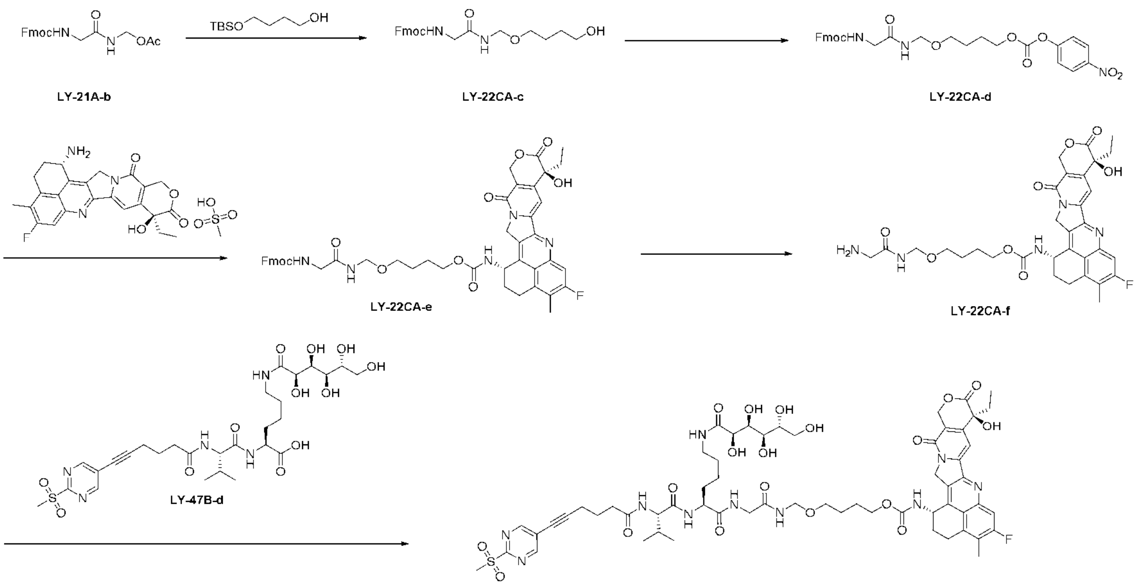

LY-21A-b LY-22CA-c LY-22CA-d

LY-22CA-e LY-22CA-f

LY-47B-d

LY-22CA

Step 1: Preparation of compound LY-22CA-c

P-toluenesulfonic acid (122 mg, 708.51 μmol, 0.3 eq) was added to a solution of compound LY-21A-b (1g, 2.36 mmol, 1 eq) and 4-((tert-butyldimethylsilyl)oxy)but-1-ol (965.3 mg, 4.72 mmol, 2 eq) in tetrahydrofuran (20 mL). The reaction solution was stirred at 30°C for 3 hours. The reaction solution was quenched with water (20 mL), the aqueous phase was extracted with ethyl acetate (20 mL) 3 times, the organic phases were combined, washed with saturated brine (30 mL) once, dried with anhydrous sodium sulfate, filtered and concentrated, and the residue was purified by silica gel column chromatography (eluents: ethyl acetate/petroleum ether = 1:2-1:1) to obtain compound LY-22CA-c (519 mg, yield = 55.1%).

LCMS (ESI): m/z, 421[M+Na]$^+$.

Step 2: Preparation of compound LY-22CA-d

N,N-diisopropylethylamine (0.64 mL, 3.9 mmol, 3 eq) was added to the solution of compound LY-22CA-c (519 mg, 1.3 mmol, 1 eq) and bis(p-nitrobenzene)carbonate (790.5 mg, 2.6 mmol, 2 eq) in N,N-dimethylformamide (15 mL). The reaction solution was stirred at room temperature for 2 hours. The reaction solution was quenched with water (10 mL), the aqueous phase was extracted with ethyl acetate (10 mL) 3 times, the organic phases were combined, washed with saturated brine (20 mL) once, dried with anhydrous sodium sulfate, filtered and concentrated, and the residue was purified by silica gel column chromatography (eluents: ethyl acetate/petroleum ether = 1:3-1:1) to obtain compound LY-22CA-d (425.1 mg, yield = 58.0%).

LCMS (ESI): m/z, 586[M+Na]$^+$.

Other steps are the same as those in Example 16, except that compound LY-22AF-c was replaced with LY-22CA-d and LY-22CE-f was replaced with LY-47B-d to prepare compound LY-22CA.

LCMS (ESI): m/z, 1293[M+H]$^+$.

$^1$H NMR (400 MHz, DMSO-$d_6$) δ 9.11 (s, 2H), 8.51 (t, *J* = 6.6 Hz, 1H), 8.17 (t, *J*= 5.8 Hz, 1H), 8.04 *(d, J= 7.2* Hz, 1H), 7.93 (dd, *J* = 8.6, 4.6 Hz, 2H), 7.77 (d, *J* = 10.8 Hz, 1H), 7.63 (t, *J* = 6.0 Hz, 1H), 7.31 (s, 1H), 6.52 (s, 1H), 5.43 (s, 2H), 5.37 (d, *J* = 5.0 Hz, 1H), 5.23 (d, *J* = 3.6 Hz, 2H), 4.51 (ddd, *J*= 17.6, 9.8, 5.6 Hz, 4H), 4.40 *(d, J=* 7.2 Hz, 1H), 4.34 (s, 1H), 4.21- 4.13

(m, 2H), 4.07 *(d, J*= 6.0 Hz, 2H), 3.97 (d, *J* = 4.2 Hz, 1H), 3.90 (s, 1H), 3.70 (qd, *J* = 16.8, 5.6 Hz, 2H), 3.57 (d, *J* = 11.2 Hz, 1H), 3.47 (s, 2H), 3.41 (s, 7H), 3.06 (ddt, *J* = 27.2, 13.2, 6.6 Hz, 4H), 2.55 (d, *J* = 7.2 Hz, 2H), 2.38 (d, *J* = 2.0 Hz, 4H), 2.24-2.11 (m, 2H), 2.02-1.88 (m, 2H), 1.89-1.77 (m, 4H), 1.62 (q, *J* = 6.8, 6.2 Hz, 3H), 1.58-1.49 (m, 3H), 1.39 (d, *J* = 7.2 Hz, 2H), 1.25 (d, *J* = 12.8 Hz, 2H), 0.91-0.78 (m, 9H). $^{19}F$ NMR (377 MHz, DMSO-$d_6$) δ -111.35.

Example 26: Preparation of compound LY-22CB

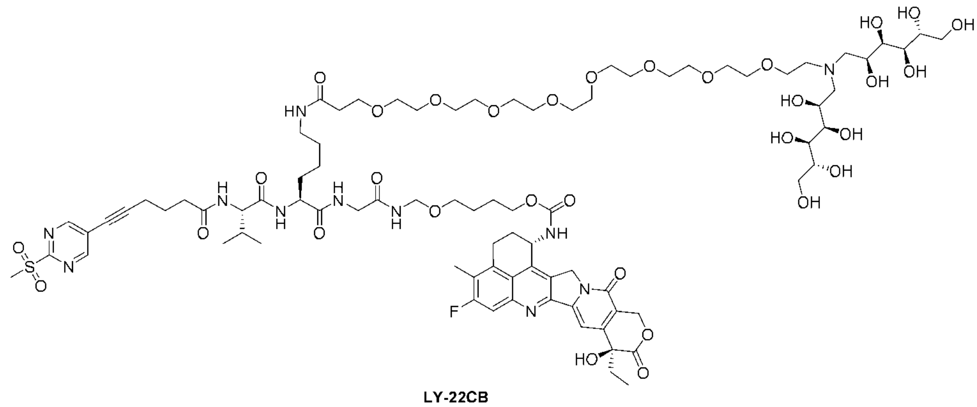

LY-22CB

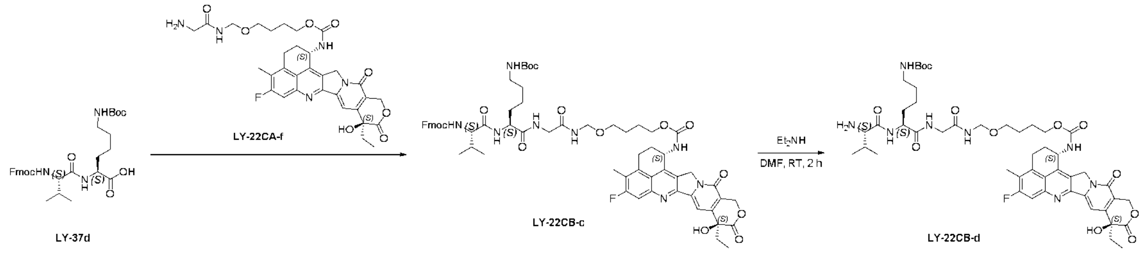

LY-37d, LY-22CA-f, LY-22CB-c, LY-22CB-d

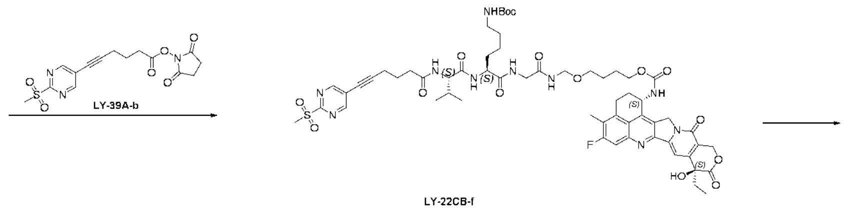
LY-39A-b, LY-22CB-f

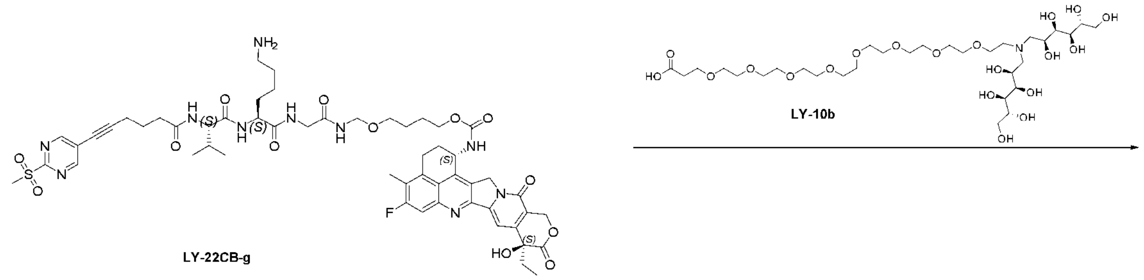
LY-22CB-g, LY-10b

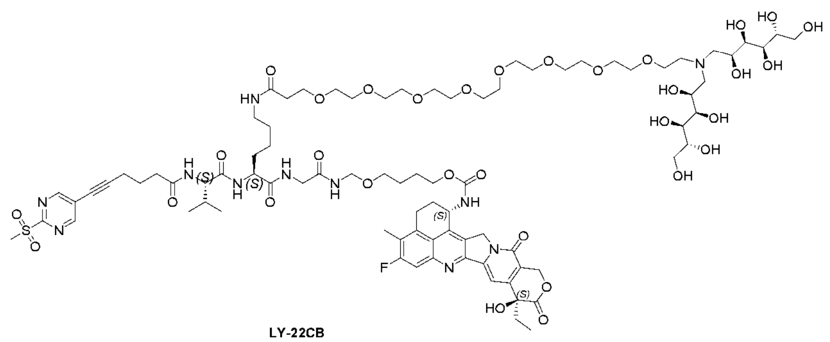
LY-22CB

Step 1: Preparation of compound LY-22CB-c

N,N-diisopropylethylamine (5.18 μL, 31.36 μmol, 2 eq) and N,N,N',N'-tetramethyl-O-(7-azabenzotriazole-1-yl) hexafluorophosphate (7.75 mg, 20.39 μmol, 1.3 eq) were added to the solution of compounds LY-37d (10.68 mg, 18.82 μmol, 1.2 eq) and LY-22CA-f (10 mg, 15.68 μmol, 1.2 eq) in N,N-dimethylformamide (1 mL) under an ice water bath, and then the reaction solution was warmed to room temperature again and stirred for 2 hours. The reaction solution was subjected to preparative high performance liquid chromatograph (preparative chromatograph manufacturer: SHIMADZU, model: LC-20AP, column: Welch Xtimate C18 250 × 30 mm × 10 μm, mobile phase: water (0.225% HCOOH)-acetonitrile, an elution ratio of acetonitrile: 48% to 78%), to obtain the product LY-22CB-c (14.4 mg, yield = 40.7%).

LCMS (ESI): m/z, 1187[M+H]$^+$.

Step 2: Preparation of compound LY-22CB-d

Diethylamine (6.25 µL, 60.64 µmol, 5 eq) was added to the solution of compound LY-22CB-c (14.4 mg, 12.13 µmol, 1 eq) in N,N-dimethylformamide (1 mL), and the reaction solution was stirred at room temperature for 2 hours. The reaction solution was concentrated under reduced pressure to obtain the crude product LY-22CB-d (14.7 mg).
LCMS (ESI): m/z, 965$[M+H]^+$.

Step 3: Preparation of compound LY-22CB-f

N,N-diisopropylethylamine (6 µL, 36.33 µmol, 3 eq) and LY-22CB-d (14.7 mg, 12.11 µmol, 1 eq, purity: 79.5%) were added to a solution of compound LY-39A-b (8.85 mg, 24.22 µmol, 2 eq) in dichloromethane (1 mL), and the reaction solution was stirred at room temperature overnight. The reaction solution was purified by high performance liquid chromatograph (preparative chromatograph manufacturer: SHIMADZU, model: LC-20AP, column: YMC-Triart Prep C18 250 × 30 mm × 10 µm, mobile phase: water (0.225% HCOOH)-acetonitrile, an elution ratio of acetonitrile: 39% to 69%), to obtain the product LY-22CB-f (10.1 mg, yield = 68.63%).
LCMS (ESI): m/z, 1215$[M+H]^+$.

Step 4: Preparation of compound LY-22CB-g

Trifluoroacetic acid (0.2 mL) was added to a solution of compound LY-22CB-f (10.1 mg, 8.31 µmol, 1 eq) in dichloromethane (2 mL) under an ice water bath and the reaction solution was stirred for 2 hours in the ice water bath. The reaction solution was concentrated under reduced pressure to obtain the crude product LY-22CB-g (16.4 mg).
LCMS (ESI): m/z, 1115$[M+H]^+$.

Step 5: Preparation of LY-22CB

N,N-diisopropylethylamine (8.24 µL, 49.85 µmol, 6 eq) and N,N,N',N'-tetramethyl-O-(7-azabenzotriazole-1-yl) hexafluorophosphate (6.32 mg, 16.62 µmol, 2 eq) were added to the solution of compounds LY-22CB-g (16.4 mg, 8.31 µmol, 1 eq, purity: 56.5%) and LY-10b (19.2 mg, 24.93 µmol, 3 eq) in N,N-dimethylformamide (3 mL), and then the reaction solution was stirred for 2 hours at room temperature. The reaction solution was purified by preparative high performance liquid chromatograph (preparative chromatograph manufacturer: Oriendo, model: BRIX-2860, column: Synergi Max-RP 250 × 40 mm × 10 µm, mobile phase: water (0.225% HCOOH)-acetonitrile, an elution ratio of acetonitrile: 21% to 51%), to obtain the product LY-22CB (1.83 mg, yield = 11.8%).
LCMS (ESI): m/z, 1866$[M+H]^+$.

Example 27: Preparation of compound LY-22CC

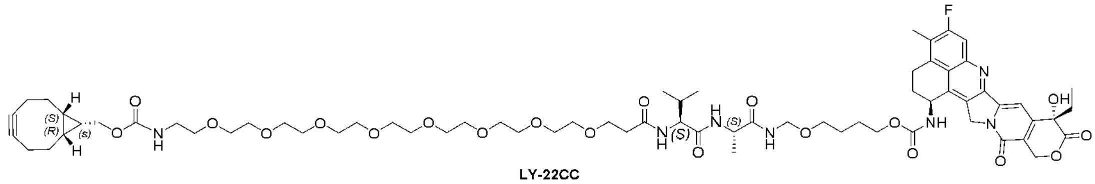

LY-22CC

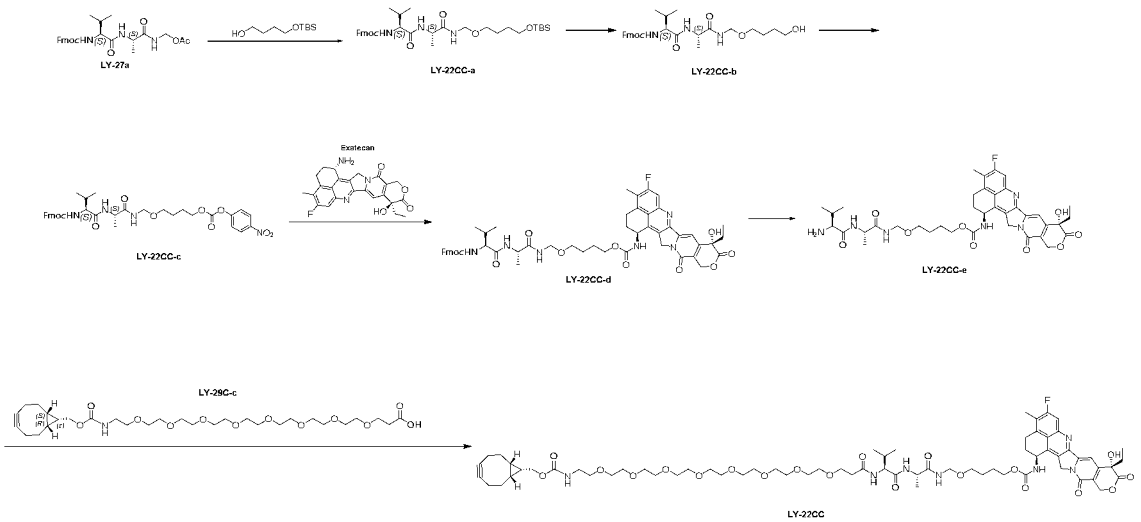


Step 1: Preparation of compound LY-22CC-a

The compound LY-27a (500 mg, 1.04 mmol, 1 eq) was dissolved in THF (10 mL), then compound 4-((tert-butyldimethylsilyl)oxy)but-1-ol (424.42 mg, 2.08 mmol, 2 eq) and TsOH (16.46 mg, 15.38 μL, 95.59 μmol, 1 eq) were added, and the reaction solution was stirred at 30°C for 4 hours. The reaction solution was directly used for the next step.
LCMS (ESI): m/z, 648.4 $[M+Na]^+$.

Step 2: Preparation of compound LY-22CC-b

The compound LY-22CC-a (500 mg, 798.88 μmol, 1 eq) was dissolved in THF (10 mL), and 4M hydrogen chloride 1,4 dioxane solution (29.13 mg, 199.72 μL, 798.88 μmol, 1 eq) was added to the solution, and the solution was stirred at 15°C for 3 hours. The reaction solution was concentrated under reduced pressure, and the residue was purified by high performance liquid chromatograph (preparative chromatograph manufacturer: SHIMADZU, model: LC-20AP, column: XT-15*30-10, mobile phase: water (0.225% HCOOH)-acetonitrile, an elution ratio of water: 40% to 70%, to obtain a white solid compound LY-22CC-b (70 mg, yield: 17.13%).
LCMS (ESI): m/z, 534.3 $[M+Na]^+$.

Step 3: Preparation of compound LY-22CC-c

The compound LY-22CC-b (70 mg, 136.82 μmol, 1 eq) was dissolved in a solution of DMF (5 mL), and bis(4-nitrophenyl)carbonate (83.24 mg, 55.50 μL, 273.64 μmol, 2 eq) and DIPEA (53.05 mg, 67.84 μL, 410.46 μmol, 3 eq) were added. The reaction solution was stirred at 15°C for 12 hours. The reaction solution was concentrated to remove most of DMF, DCM (30 mL) was added, the reaction solution was washed with water (30 mL), the organic phase was dried, filtered, and concentrated under reduced pressure, and the residue was purified by silica gel column chromatography (eluents: DCM: MeOH = 0:1-5:1) to obtain the yellow solid product LY-22CC-c (70 mg, yield: 75.60%).
LCMS (ESI): m/z, 699.3 $[M+Na]^+$.
Other steps are the same as those in Example 16, except that LY-22AF-c was replaced with LY-22CC-c and LY-22CE-f was replaced with LY-29C-c to prepare compound LY-22CC.
LCMS (ESI): m/z, 1350.7 $[M+H]^+$.

Example 28: Preparation of compound LY-22CD

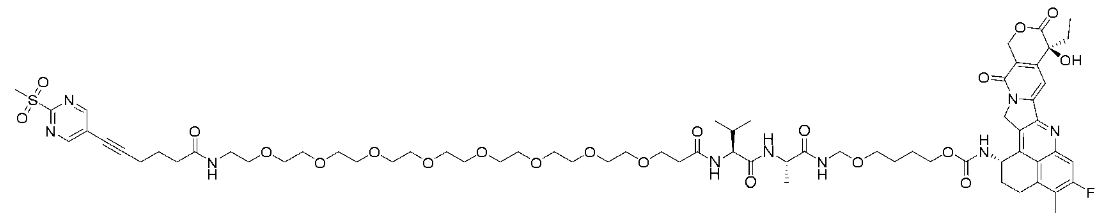

LY-22CD

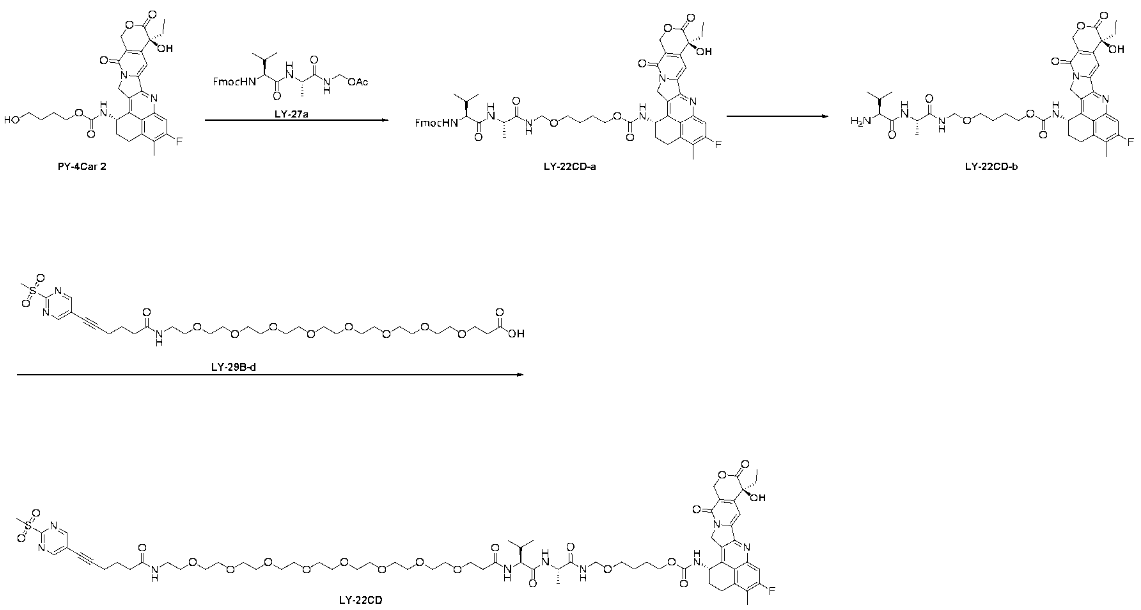


PY-4Car 2

LY-22CD-a

LY-22CD-b

LY-22CD

Step 1: Preparation of LY-22CD-a

P-toluenesulfonic acid (7.86 mg, 45.66 μmol, 1 eq) was added to a solution of PY-4Car2 (25.25 mg, 45.66 μmol, 1 eq) and compound LY-27a (219.87 mg, 456.59 μmol, 10 eq) in tetrahydrofuran (5 mL) and the reaction solution was stirred at 30°C for 20 hours. After being concentrated, the reaction solution was dissolved in N,N-dimethylformamide and filtered, and the filtrate was purified by preparative high performance liquid chromatograph (preparative chromatograph manufacturer: SHIMADZU, model: LC-20AP, column: XT-15*30-10, mobile phase: water (0.225% HCOOH)-acetonitrile, an elution ratio of acetonitrile: 60% to 90%), to obtain the product LY-22CD-a (12.8 mg, yield = 28.81%).

LCMS (ESI): m/z, 995[M+Na]$^+$.

Step 2: Preparation of LY-22CD-b

Diethylamine (6.78 μL, 65.77 μmol, 5 eq) was added to the solution of compound LY-22CD-a (12.8 mg, 12.15 μmol, 1 eq) in N,N-dimethylformamide (2 mL), and the reaction solution was stirred at room temperature for 2 hours. The reaction solution was concentrated under reduced pressure to obtain the crude product LY-22CD-b (15 mg).

LCMS (ESI): m/z, 773[M+Na]$^+$.

Step 3: Preparation of LY-22CD

N,N-diisopropylethylamine (4.35 μL, 26.32 μmol, 2 eq) and N,N,N',N'-tetramethyl-O-(7-azabenzotriazole-1-yl) hexafluorophosphate (6 mg, 15.79 μmol, 1.2 eq) were added to the solution of compounds LY-22CD-b (9.88 mg, 13.16 μmol, 1 eq) and LY-29B-d (11.78 mg, 14.47 μmol, 1.1 eq) in N,N-dimethylformamide (2 mL), and then the reaction solution was stirred at room temperature for 2 hours. The reaction solution was purified by preparative high performance liquid chromatograph (preparative chromatograph manufacturer: SHIMADZU, model: LC-20AP, column: Global 30 mm × 250 mm, mobile phase: water (0.225% HCOOH)-acetonitrile, an elution ratio of acetonitrile: 30% to 60%), to obtain the product LY-22CD (4.05 mg, yield = 21.6%).

LCMS (ESI): m/z, 1446[M+Na]$^+$.

Example 29: Preparation of compound LY-22CD-2

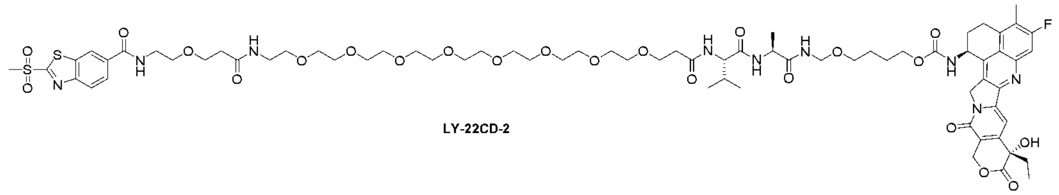

LY-22CD-2

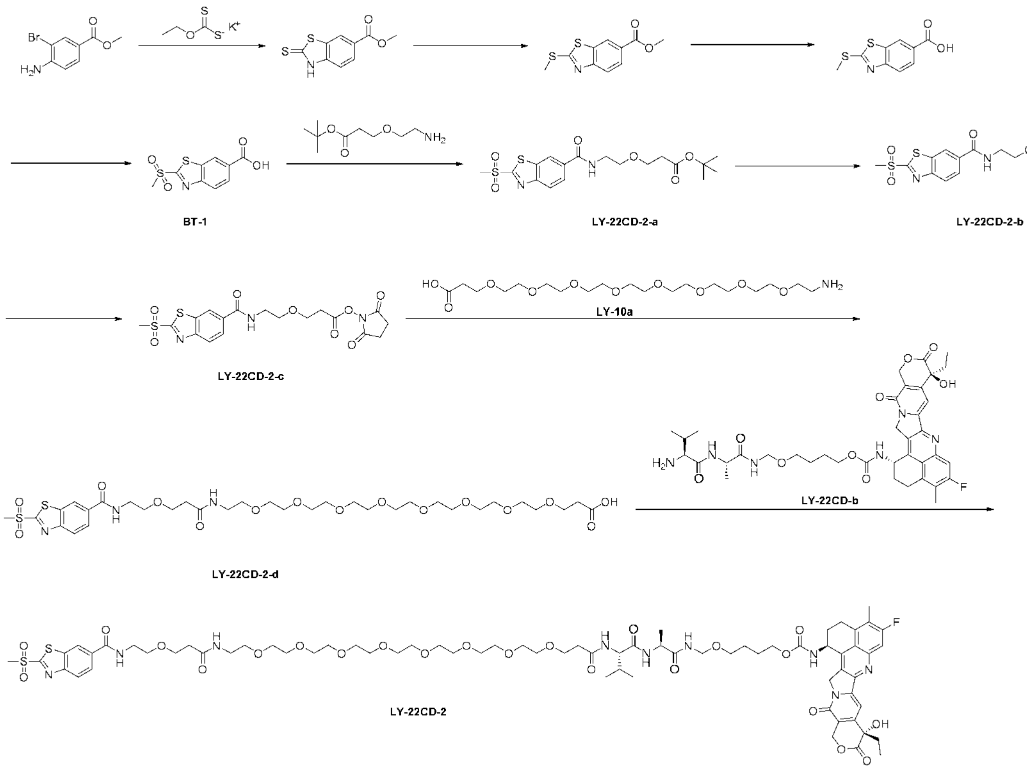


Step 1: Preparation of methyl 2-thio-2,3-dihydro-1,3-benzothiazole-6-carboxylate

A solution of the compound 2-bromo-4-methoxycarbonylaniline (7g, 30.43 mmol, 1 eq) and potassium (ethoxymethionyl) sulfonamide (9.75g, 60.85 mmol, 2 eq) in N,N-dimethylformamide (30 mL) was stirred at 130°C for 16 hours. The solution was cooled to room temperature again, slowly dropwise added into ice water (50 mL), and adjusted with concentrated hydrochloric acid to adjust pH to 1, the solid precipitated, and was filtered and washed with ice water (10 mL), the filter cake was collected and dissolved in methanol, and isolated by C18 reversed-phase column, to obtain the white solid methyl 2-thio-2,3-dihydro-1,3-benzothiazole-6-carboxylate (454.4 mg, yield = 6.63%).

LCMS (ESI): m/z, 226[M+H]$^+$.

Step 2: Preparation of methyl 2-(methylthioalkyl)-1,3-benzothiazole-6-carboxylate

Triethylamine (336 μL, 2.42 mmol, 1.2 eq) was dropwise added into the solution of methyl 2-thio-2,3-dihydro-1,3-benzothiazole-6-carboxylate (454.4 mg, 2.017 mmol, 1 eq) and methyl iodide (150.7 μL, 2.42 mmol, 1.2 eq) in ethanol (8 mL) at room temperature, and then the reaction solution was stirred at 90°C for 1 hour. After the reaction solution was concentrated, the residue was purified by silica gel column chromatography (eluents: ethyl acetate/petroleum ether = 10%-15%), to obtain the product methyl 2-(methylthioalkyl)-1,3-benzothiazole-6-carboxylate (434.9 mg, yield = 90%).

LCMS (ESI): m/z, 240[M+H]$^+$.

Step 3: Preparation of 2-(methylthioalkyl)-1,3-benzothiazole-6-carboxylic acid

Lithium hydroxide monohydrate (164.8 mg, 3.92 mmol, 4 eq) was added to the solution of the compound methyl 2-(methylthioalkyl)-1,3-benzothiazole-6-carboxylate (234.9 mg, 0.981 mmol, 1 eq) in tetrahydrofuran/water (4 mL/0.6 mL) under the ice water bath, the reaction solution was stirred at room temperature for 10 hours, and the LCMS showed that the reaction did not end. After the reaction solution was heated to 80°C and stirred for 5 hours, the LCMS showed that the raw materials completed reaction. The reaction solution was diluted with dichloromethane (5 mL) and water (5 mL), and then adjusted with 1N dilute hydrochloric acid to adjust pH to 3, the aqueous phase was reversely extracted with dichloromethane (5 mL) three times, the organic phases were combined, washed with saturated brine (15 mL) once, dried with anhydrous sodium sulfate, filtered and concentrated, to obtain the product 2-(methylthioalkyl)-1,3-benzothiazole-6-carboxylic acid (209.1 mg, yield = 94.6%).

LCMS (ESI): m/z, 226$[M+H]^+$.

Step 4: Preparation of 2-methylsulfonyl-1,3-benzothiazole-6-carboxylic acid

A solution of compound 2-(methylthioalkyl)-1,3-benzothiazole-6-carboxylic acid (209.1 mg, 0.928 mmol, 1 eq) and meta-chloroperoxybenzoic acid (800.9 mg, 4.64 mmol, 5 eq) in dichloromethane (5 mL) was stirred at room temperature for 16 hours. The reaction was quenched with saturated sodium thiosulfate solution (3 mL), the pH was adjusted to neutral with saturated sodium bicarbonate solution, the aqueous phase was extracted with dichloromethane (10 mL) three times, the organic phases were combined, washed with saturated brine (10 mL) once, dried with anhydrous sodium sulfate, filtered and concentrated, and the residue was purified by preparative high performance liquid chromatograph (preparative chromatograph manufacturer: Oriendo, model: BRIX-2860-R1, column: Welch Xtimate C18 250 × 30 mm × 10 μm, mobile phase: water (0.225% HCOOH)-acetonitrile, an elution ratio of water: 20% to 50%), to obtain the product 2-methylsulfonyl-1,3-benzothiazole-6-carboxylic acid (150.4 mg, yield = 63%).

LCMS (ESI): m/z, 258$[M+H]^+$.

Step 5: Preparation of tert-butyl 3-(2-((2-methylsulfonyl-1,3-benzothiazole-6-yl)formamido)ethoxy)propionate (LY-22CD-2-a)

N,N-diisopropylethylamine (193 mL, 1.17 μmol, 2 eq) and N,N,N',N'-tetramethyl-O-(7-azabenzotriazole-1-yl) hexafluorophosphate (266.9 mg, 0.702 μmol, 1.2 eq) were added to the solution of compounds 2-methylsulfonyl-1,3-benzothiazole-6-carboxylic acid (150.4 mg, 0.585 mmol, 1 eq) and tert-butyl 3-(2-aminoethoxy)propionate (132.7 mg, 0.702 μmol, 1.2 eq) in N,N-dimethylformamide (4 mL), and the reaction solution was stirred at room temperature for 1 hour. The reaction solution was filtered and then purified by preparative high performance liquid chromatograph (preparative chromatograph manufacturer: Oriendo, model: BRIX-2860-R1, column: Welch Xtimate C18 250 × 30 mm × 10 μm, mobile phase: water (0.225% HCOOH)-acetonitrile, an elution ratio of water: 30% to 60%), to obtain the product LY-22CD-2-a (169.9 mg, yield = 67.8%).

LCMS (ESI): m/z, 451$[M+Na]^+$.

Step 6: Preparation of 3-{2-((2-methylsulfonyl-1,3-benzothiazole-6-yl)formamido)ethoxy)propionic acid (LY-22CD-2-b)

Trifluoroacetic acid (1 mL) was added to a solution of compound LY-22CD-2-a (169.9 mg, 0.396 mmol, 1 eq) in dichloromethane (2 mL) in ice water bath and then the reaction solution was stirred for 2 hours at room temperature. The reaction solution was concentrated under reduced pressure to obtain a crude product (200 mg).

LCMS (ESI): m/z, 373$[M+H]^+$.

Step 7: Preparation of 2,5-dioxopyrrolidin-1-yl3-{2-((2-methylsulfonyl-1,3-benzothiazole-6-yl)formamido)ethoxy)propionate (LY-22CD-2-c)

N-hydroxysuccinimide (91.15 mg, 0.792 mmol, 2 eq) and N,N'-diisopropylcarbodiimide (123 mL, 0.792 mmol, 2 eq) were added to a solution of compound LY-22CD-2-b (147.7 mg, 0.396 mmol, 1 eq) in N,N-dimethylformamide (4 mL) in the ice water bath, the mixture was stirred for 18 hours at room temperature, and the LCMS showed that there was basically no reaction. N-hydroxysuccinimide (182.3 mg, 1.584 mmol, 4 eq) and N,N'-diisopropylcarbodiimide (246 mL, 1.584 mmol, 4 eq) were added again, the mixture was stirred for 6 hours at 50°C, and then the LCMS showed that the raw materials completed reaction. The reaction solution was filtered and then subjected to preparative high performance liquid chromatograph (preparative chromatograph manufacturer: Oriendo, model: BRIX-2860-R1, column: Welch Xtimate C18 250 × 30 mm × 10 μm, mobile phase: water (0.225% HCOOH)-acetonitrile, an elution ratio of water: 20% to 50%), to obtain the product 2,5-dioxopyrrolidin-1-yl3-{2-((2-methylsulfonyl-1,3-benzothiazole-6-yl)formamido)ethoxy)propionate

(124.3 mg, yield = 67.0%).

LCMS (ESI): m/z, 470[M+H]+.

Step 8: Preparation of 1-(3-(2-((2-methylsulfonyl-1,3-benzothiazole-6-yl)formamido)ethoxy)propionamido)-3,6,9,12,15,18,21,24-octaoxaheptacosan-27-acid (LY-22CD-2-d)

N,N-diisopropylethylamine (42.24 mL, 255.6 μmol, 2 eq) was added to a solution of compound LY-22CD-2-c (60 mg, 127.8 μmol, 1 eq) and 27-amino-4,7,10,13,16,19,22,25-octaoxaheptacosanoic acid (LY-10a) (67.71 mg, 153.36 μmol, 1.2 eq) in N,N-dimethylformamide (3 mL). The reaction solution was stirred at room temperature for 1 hour. The reaction solution was filtered and then subjected to preparative high performance liquid chromatograph (preparative chromatograph manufacturer: SHIMADZU, model: LC-20AP, column: Welch Xtimate C18 250 × 30 mm × 10 μm, mobile phase: water (0.225% HCOOH)-acetonitrile, an elution ratio of water: 10% to 40%), to obtain compound LY-22CD-2-d 81 mg, yield = 79.6%).

LCMS (ESI): m/z, 796[M+H]+.

Step 9: Preparation of compound LY-22CD-2

N,N-diisopropylethylamine (6.92 mL, 41.88 μmol, 2 eq) and N,N,N',N'-tetramethyl-O-(7-azabenzotriazole-1-yl) hexafluorophosphate (9.55 mg, 25.13 μmol, 1.2 eq) were added to the solution of compounds LY-22CD-b (19.03 mg, purity: 82.6%; 20.94 μmol, 1 eq) and LY-22CD-2-d (20 mg, 25.13 μmol, 1.2 eq) in N,N-dimethylformamide (2 mL), and then the reaction solution was stirred at room temperature for 1 hour. The reaction solution was filtered and then subjected to preparative high performance liquid chromatograph (preparative chromatograph manufacturer: Oriendo, model: BRIX-2860-R1, column: Welch Xtimate C18 250 × 30 mm × 10 μm, mobile phase: water (0.225% HCOOH)-acetonitrile, an elution ratio of water: 30% to 60%), to obtain the product LY-22CD-2 (13.48 mg, yield = 42.11%).

LCMS (ESI): m/z, 1550[M+Na]+.

$^1$H NMR (400 MHz, DMSO-$d_6$) δ 8.81 (d, *J* = 1.6 Hz, 1H), 8.73 (t, *J* = 5.6 Hz, 1H), 8.54 (t, *J* = 6.8 Hz, 1H), 8.32 (d, *J* = 8.6 Hz, 1H), 8.14 (dd, *J* = 8.6, 1.8 Hz, 1H), 8.03 (d, *J* = 7.2 Hz, 1H), 7.91 (d, *J* = 7.2 Hz, 1H), 7.84 (d, *J*= 8.8 Hz, 1H), 7.77 (d, *J* = 10.8 Hz, 1H), 7.31 (s, 1H), 6.51 (s, 1H), 5.42 (s, 2H), 5.23 (s, 3H), 4.51 (ddd, *J* = 29.6, 10.0, 6.4 Hz, 2H), 4.19 (dt, *J* = 15.6, 7.4 Hz, 2H), 4.06 (d, *J* = 6.4 Hz, 2H), 3.67-3.51 (m, 9H), 3.51-3.43 (m, 26H), 3.37 (t, *J* = 5.8 Hz, 6H), 3.23 (s, 2H), 3.18 (q, *J* = 5.8 Hz, 4H), 2.48 - 2.33 (m, 7H), 2.28-2.05 (m, 3H), 1.97-1.81 (m, 3H), 1.66-1.50 (m, 4H), 1.21 (d, *J* = 7.2 Hz, 3H), 0.96-0.70 (m, 9H).

Example 30: Preparation of compound LY-22CD-3

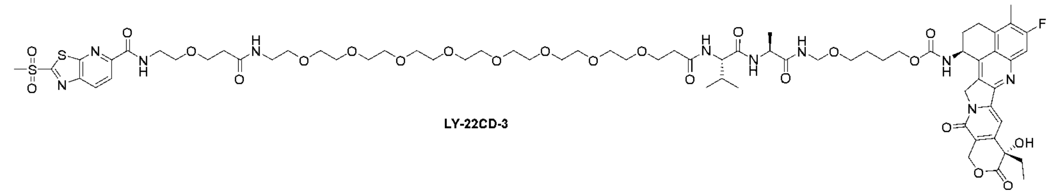

LY-22CD-3

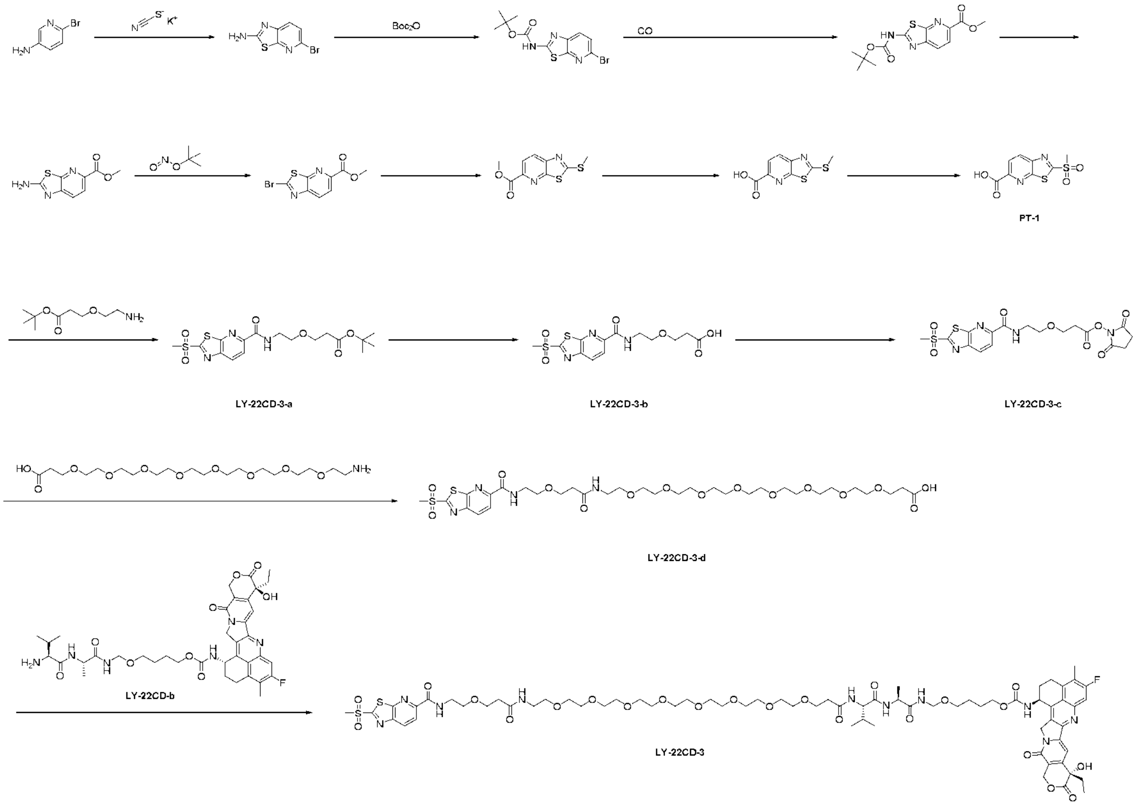


PT-1

LY-22CD-3-a

LY-22CD-3-b

LY-22CD-3-c

LY-22CD-3-d

LY-22CD-b

LY-22CD-3

Step 1: Preparation of 5-bromothiazolo[5,4-b]pyridine-2-amine

A solution (20 mL) of liquid bromine in acetic acid was added to a solution (100 mL) of 5-amino-2-bromopyridine (10g, 57.80 mmol, 1 eq) and potassium thiocyanate (22.47g, 11.91 mL, 231.20 mmol, 4 eq) in acetic acid at 0°C and the reaction solution was stirred for 3 hours at 20°C. The reaction solution was quenched with water (50 mL) and filtered to obtain a filtrate, and the filtrate was concentrated under reduced pressure to obtain a crude product. The crude product was alkalized with the aqueous solution of sodium bicarbonate until pH reached 8, the resulting solution was extracted with ethyl acetate (50 mL × 4), and organic phases were combined, dried with anhydrous sodium sulfate, and concentrated under reduced pressure, to obtain 5-bromothiazolo[5,4-b]pyridine-2-amine (9.80g) as a yellow solid.

LCMS (ESI): m/z, 230.0[M+H]$^+$.

$^1$H NMR (400 MHz, DMSO-*d*$^6$) δ 7.96 (s, 2H), 7.57 (d, *J* = 8.4 Hz, 1H), 7.43 (d, *J*= 8.4 Hz, 1H).

Step 2: Preparation of (5-bromothiazolo[5,4-b]pyridin-2-yl)tert-butyl carbamate

5-bromothiazolo[5,4-b]pyridine-2-amine (7.80g, 33.90 mmol, 1 eq)/triethylamine (4.46g, 6.13 mL, 44.07 mmol, 1.3 eq)/4-dimethylaminopyridine (414.15 mg, 414.15 μL, 3.39 mmol, 0.1 eq) and di-t-butyl dicarbonate (9.62g, 9.43 mL, 44.07 mmol, 1.3 eq) were added to a solution of tetrahydrofuran (320 mL) at room temperature and the solution was stirred at 25°C for 16 hours. After the reaction solution was concentrated, the residue was purified by silica gel column chromatography (eluents: ethyl acetate/petroleum ether = 40%-50%), to obtain the product (5-bromothiazolo[5,4-b]pyridin-2-yl)tert-butyl carbamate (7.0g, 21.20 mmol, two-step yield = 26%).

LCMS (ESI): m/z, 330.0[M+H]$^+$.

$^1$H NMR (400 MHz, DMSO-*d*$_6$) δ 12.12 (s, 1H), 7.99 (d, *J* = 8.4 Hz, 1H), 7.64 (d, *J* = 8.4 Hz, 1H), 1.52 (s, 9H).

Step 3: Preparation of methyl 2-((tert-butoxycarbonyl)amino)thiazolo[5,4-b]pyridin-5-carboxylate

(5-bromothiazolo[5,4-b]pyridin-2-yl)tert-butyl carbamate (6g, 18.17 mmol, 1 eq), potassium carbonate (7.53g, 3.10 mL, 54.51 mmol, 3 eq), palladium acetate (1.22g, 5.45 mmol, 0.3 eq), and 1,3-bis(diphenylphosphino)propane (1.80g, 4.36 mmol, 0.24 eq) were sequentially added to a solution of methanol (120 mL) and N, N-dimethylformamide (60 mL) at room temperature. The reaction solution was stirred at 60°C for 16 hours in a carbon monoxide atmosphere. After the reaction solution was concentrated, the residue was purified by silica gel column chromatography (eluents: ethyl acetate/petroleum ether = 40%-80%), to obtain the product methyl 2-((tert-butoxycarbonyl)amino)thiazolo[5,4-b]pyridin-5-carboxylate (2.20g, 7.11 mmol, yield = 39%).
LCMS (ESI): m/z, 310.1[M+H]$^+$.
$^1$H NMR (400 MHz, DMSO-$d_6$) δ 12.26 (s, 1H), 8.25 - 8.04 (m, 2H), 3.90 (s, 3H), 1.53 (s, 9H).

Step 4: Preparation of methyl 2-aminothiazolo[5,4-b]pyridin-5-carboxylate

Trifluoroacetic acid (40 mL) was added to a solution of methyl 2-((tert-butoxycarbonyl)amino)thiazolo[5,4-b]pyridin-5-carboxylate (2.20g, 7.11 mmol, 1 eq) in dichloromethane (80 mL) at 0°C and the reaction solution was stirred for 2 hours at 20°C. After the reaction solution was concentrated, the aqueous solution of saturated sodium bicarbonate was added to the residue, then the reaction solution was filtered, and the filter cake was concentrated under reduced pressure to obtain methyl 2-aminothiazolo[5,4-b]pyridin-5-carboxylate (1.38g, 6.60 mmol, yield: 93%).
LCMS (ESI): m/z, 210.1[M+H]$^+$.
$^1$H NMR (400 MHz, DMSO-$d_6$) δ 8.27 (s, 2H), 7.95 (d, *J* = 8.4 Hz, 1H), 7.68 (d, *J* = 8.4 Hz, 1H), 3.85 (s, 3H).

Step 5: Preparation of methyl 2-bromothiazolo[5,4-b]pyridin-5-carboxylate

Tert-butyl nitrite (1.22g, 1.42 mL, 11.87 mmol, 1.8 eq) and methyl 2-aminothiazolo[5,4-b]pyridin-5-carboxylate (1.38g, 6.60 mmol, 1 eq) were added to the solution of copper bromide (1.84g, 386.06 μL, 8.24 mmol, 1.25 eq) in acetonitrile (60 mL) at 0°C, and the reaction solution was stirred at 25°C for 16 hours. After the reaction solution was concentrated, the residue was purified by silica gel column chromatography (eluents: ethyl acetate/petroleum ether = 20%-40%), to obtain the product methyl 2-bromothiazolo[5,4-b]pyridin-5-carboxylate (1.45g, 5.31 mmol, yield = 81%).
LCMS (ESI): m/z, 273.1[M+H]$^+$.
$^1$H NMR (400 MHz, Chloroform-*d*) δ 8.37 - 8.27 (m, 2H), 4.07 (s, 3H).

Step 6: Preparation of methyl 2-(methylthio)thiazolo[5,4-b]pyridin-5-carboxylate

A solution of methyl 2-bromo-[1,3]thiazolo[5,4-b]pyridin-5-carboxylate (375 mg, 1.37 mmol, 1 eq) and sodium methyl mercaptan (240.61 mg, 3.43 mmol, 2.5 eq) in ethylene glycol dimethyl ether (10 mL) was stirred for 2 hours at -15°C. The aqueous solution of saturated ammonium chloride (5 mL) was added to the reactant, the resulting solution was concentrated, tetrahydrofuran (30 mL) was added, the resulting solution was filtered, and the filter cake was collected and purified by preparative high performance liquid chromatograph (preparative chromatograph manufacturer: Oriendo, model: BRIX-2860-R1, column: Welch Xtimate C18 150 × 30 mm × 10 μm, mobile phase: water (0.225% HCOOH)-acetonitrile, an elution ratio of water: 30% to 60%), to obtain white solids of methyl 2-(methylthio)thiazolo[5,4-b]pyridin-5-carboxylate (180 mg, yield = 25.89%) and methyl 2-(methylthio)thiazolo[5,4-b]pyridin-5-carboxylate (100 mg, yield = 15.28%).
LCMS (ESI): m/z, 241.1[M+H]$^+$.

Step 7: Preparation of 2-(methylthioalkyl)-1,3-benzothiazole-6-carboxylic acid

A solution of compound methyl 2-(methylthio)thiazolo[5,4-b]pyridin-5-carboxylate (180 mg, 749.06 μmol, 1 eq) and lithium hydroxide monohydrate (62.92 mg, 41.67 μL, 1.50 mmol, 2 eq) in tetrahydrofuran (2 mL) was mixed with water (2 mL) at room temperature, the solution was stirred at 20°C for 12 hours, the reaction solution was concentrated, water (50 mL) and hydrochloric acid (2M, aq) were added until pH was below 5, the reaction solution was filtered and washed with water (3 mL × 2), and the filter cake was collected to obtain the product 2-(methylthioalkyl)-1,3-benzothiazole-6-carboxylic acid (169 mg, crude product).
LCMS (ESI): m/z, 227.1[M+H]$^+$.

Step 8: Preparation of 2-methylsulfonyl-1,3-benzothiazole-6-carboxylic acid

Potassium peroxymonosulfate sulfate (3.29g, 9.51 mmol, 8 eq) was added to the solution of 2-(methylthioalkyl)-1,3-benzothiazole-6-carboxylic acid (269 mg, 1.19 mmol, 1 eq) in tetrahydrofuran (10 mL) and water (10 mL) and the mixture was stirred at 20°C for 2 hours. Ethyl acetate (30 mL) and $H_2O$ (20 mL) were added to the mixture, and the aqueous phase was extracted with ethyl acetate (20 mL × 3). The combined organic layers were washed with saturated brine (10 mL), dried with anhydrous sodium sulfate, filtered and concentrated, and the residue was purified by preparative high performance liquid chromatograph (preparative chromatograph manufacturer: Oriendo, model: BRIX-2860, column: YMC-Triart Prep C18 150 × 30 mm × 10 μm, mobile phase: water (0.225% HCOOH)-acetonitrile, an elution ratio of water: 10% to 40%), to obtain the product 2-methylsulfonyl-1,3-benzothiazole-6-carboxylic acid (220 mg, yield = 71.65%).

LCMS (ESI): m/z, 259.1 $[M+H]^+$.

Step 9: Preparation of compound (LY-22CD-3-a)

N,N-diisopropylethylamine (80.07 mg, 102.38 μL, 619.51 μmol, 2 eq) and N,N,N',N'-tetramethyl-O-(7-azabenzotriazole-1-yl)hexafluorophosphate (141.34 mg, 371.70 μmol, 1.2 eq) were added to the solution of compounds tert-butyl 3-(2-aminoethoxy)propionate (70.34 mg, 371.70 μmol, 1.2 eq) and 2-methylsulfonyl-1,3-benzothiazole-6-carboxylic acid (80 mg, 309.75 μmol, 1 eq) in N,N-dimethylformamide (4 mL), and the reaction solution was stirred at room temperature for 1 hour. The reaction solution was filtered and then subjected to preparative high performance liquid chromatograph (preparative chromatograph manufacturer: Oriendo, model: BRIX-2860-R6, column: Synergi Max-RP 250 × 40 mm × 10 μm, mobile phase: water (0.225% HCOOH)-acetonitrile, an elution ratio of water: 30% to 60%), to obtain compound LY-22CD-3-a (114.3 mg, yield = 85.91%).

LCMS (ESI): m/z, 452$[M+Na]^+$.

Step 10: Preparation of compound (LY-22CD-3-b)

Trifluoroacetic acid (1 mL) was added to a solution of compound (LY-22CD-3-a) (114.30 mg, 266.12 μmol, 1 eq) in dichloromethane (2 mL) in the ice water bath and then the reaction solution was stirred for 2 hours at room temperature. The reaction solution was concentrated under reduced pressure to obtain a crude product (150 mg).

LCMS (ESI): m/z, 396$[M+Na]^+$.

Step 11: Preparation of compound (LY-22CD-3-c)

N-hydroxysuccinimide (183.77 mg, 1.60 mmol, 6 eq) and N,N'-diisopropylcarbodiimide (201.51 mg, 248.77 μL, 1.60 mmol, 6 eq) were added to the solution of compound LY-22CD-3-b (99.37 mg, 266.12 μmol, 1 eq) in N,N-dimethylformamide (4 mL) in the ice water bath and then the reaction solution was stirred at 50°C for 2 hours. The reaction solution was filtered and then subjected to preparative high performance liquid chromatograph (preparative chromatograph manufacturer: Oriendo, model: BRIX-2860, column: YMC-Triart Prep C18 150 × 30 mm × 10 μm, mobile phase: water (0.225% HCOOH)-acetonitrile, an elution ratio of water: 10% to 40%), to obtain compound LY-22CD-3-c (100 mg, yield: 79.87%).

LCMS (ESI): m/z, 493$[M+Na]^+$.

Step 12: Preparation of compound LY-22CD-3-d

N,N-diisopropylethylamine (54.94 mg, 70.26 μL, 425.11 μmol, 2 eq) was added to a solution of compound LY-22CD-3-c (100 mg, 212.55 μmol, 1 eq) and 27-amino-4,7,10,13,16,19,22,25-octaoxaheptacosanoic acid (112.61 mg, 102.37 μL, 255.06 μmol, 1.2 eq) in N,N-dimethylformamide (3 mL). The reaction solution was stirred at room temperature for 1 hour. The reaction solution was filtered and then purified by preparative high performance liquid chromatograph (preparative chromatograph manufacturer: SHIMADZU, model: LC-20AP, column: Welch Xtimate C18 150 × 30 mm × 10 μm, mobile phase: water (0.225% HCOOH)-acetonitrile, an elution ratio of water: 10% to 40%), to obtain compound LY-22CD-3-d 48.4 mg, yield = 28.57%).

LCMS (ESI): m/z, 797$[M+H]^+$.

Step 13: Preparation of compound LY-22CD-3

N,N-diisopropylethylamine (7.60 mg, 9.72 μL, 58.79 μmol, 2 eq) and N,N,N',N'-tetramethyl-O-(7-azabenzotriazole-1-yl)hexafluorophosphate (13.41 mg, 35.27 μmol, 1.2 eq) were added to the solution of compounds LY-22CD-b (22.07 mg, 29.39 μmol) and LY-22CD-3-d (28.11 mg, 35.27 μmol, 1.2 eq) in N,N-dimethylformamide (2 mL), and then the reaction solution was stirred at room temperature for 1 hour. The reaction solution was filtered and then purified by preparative high performance liquid chromatograph (preparative chromatograph manufacturer: Oriendo, model: BRIX-2860-R7, column: Synergi Max-RP 250 × 40 mm × 10 μm, mobile phase: water (0.225% HCOOH)-acetonitrile,

an elution ratio of water: 30% to 60%), to obtain the product LY-22CD-3 (17.03 mg, yield: = 37.87%).

LCMS (ESI): m/z, 1551[M+Na]+.

$^1$H NMR (400 MHz, DMSO-$d_6$) δ 9.05 (s, 1H), 8.86 (d, *J* = 8.6 Hz, 1H), 8.54 (t, *J* = 6.6 Hz, 1H), 8.37 (d, *J* = 8.6 Hz, 1H), 8.03 (d, *J* = 7.2 Hz, 1H), 7.90 (s, 2H), 7.84 (d, *J* = 8.8 Hz, 1H), 7.77 (d, *J* = 10.8 Hz, 1H), 7.31 (s, 1H), 6.51 (s, 1H), 5.42 (s, 2H), 5.23 (s, 3H), 4.58-4.44 (m, 3H), 4.19 (dt, *J* = 15.8, 7.4 Hz, 3H), 4.06 (d, *J* = 6.4 Hz, 2H), 3.66-3.52 (m, 10H), 3.48 (d, *J* = 3.2 Hz, 28H), 3.18 (q, *J*= 5.8 Hz, 3H), 2.43 (t, *J* = 6.8 Hz, 2H), 2.38 (s, 3H), 2.33 (d, *J* = 6.2 Hz, 3H), 2.24-2.11 (m, 3H), 1.97-1.81 (m, 4H), 1.67-1.49 (m, 5H), 1.21 (d, *J* = 7.2 Hz, 3H), 0.90-0.77 (m, 9H).

Example 31: Preparation of compound LY-22CE

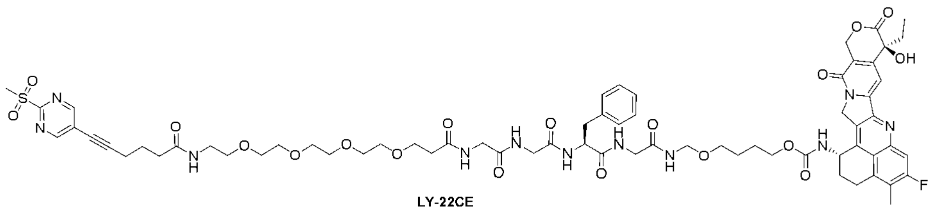

LY-22CE

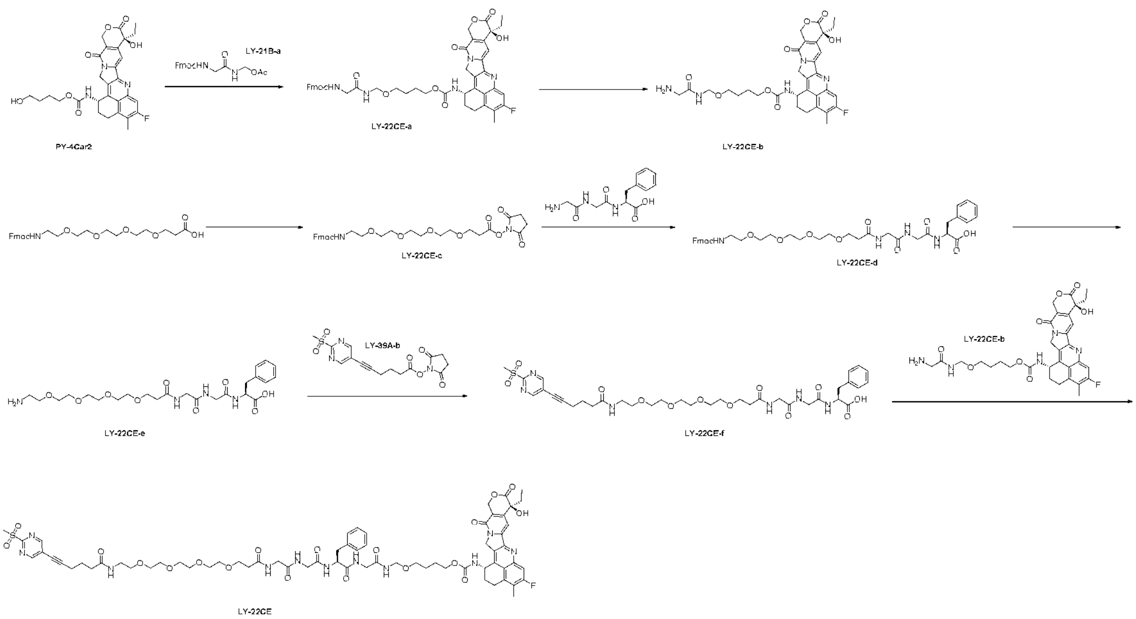


Step 1: Preparation of compound LY-22CE-a

P-toluenesulfonic acid (9.37 mg, 54.39 μmol, 1 eq) was added to a solution of PY-4Car2 (30 mg, 54.39 μmol, 1 eq) and (2-((((9H-fluoren-9-yl)methoxy)carbonyl)amino)acetamide)methyl acetate (200.36 mg, 543.9 μmol, 10 eq) in tetrahydrofuran (5 mL) and the reaction solution was stirred at 30°C for 21 hours. After the reaction solution was concentrated, the residue was dissolved in N,N-dimethylformamide, the resulting solution was filtered, and the filtrate was purified by high performance liquid chromatograph (preparative chromatograph manufacturer: SHIMADZU, model: LC-20AP, column: XT-15*30-10, mobile phase: water (0.225% HCOOH)-acetonitrile, an elution ratio of acetonitrile: 60% to 90%), to obtain the product LY-22CE-a (7.7 mg, purity: 80%; yield = 13.17%).

LCMS (ESI): m/z, 882[M+Na]+.

Step 2: Preparation of compound LY-22CE-b

Diethylamine (3.69 μL, 35.82 μmol, 5 eq) was added to the solution of compound LY-22CE-a (7.7 mg, 7.16 μmol, 1 eq, purity: 80%) in N,N-dimethylformamide (1 mL), and the reaction solution was stirred at room temperature for 2 hours. The reaction solution was concentrated under reduced pressure to obtain the crude product LY-22CE-b (10 mg).

LCMS (ESI): m/z, 638[M+H]+.

Step 3: Preparation of compound LY-22CE-c

The compound 5,8,11,14-tetraoxa-2-azanonadecanedioic acid (1g, 2.05 mmol, 1 eq), N-hydroxysuccinimide (259.67 mg, 162.29 μL, 2.26 mmol, 1.1 eq), and dicyclohexylcarbodiimide (465.53 mg, 574.72 μL, 2.26 mmol, 1.1 eq) were sequentially added to dichloromethane (20 mL) at room temperature. The reaction solution was stirred for 60 hours at 20°C. After the reaction solution was filtered, the filtrate was concentrated under reduced pressure to obtain the crude product of compound LY-22CE-c (1.1g).
LCMS (ESI): m/z, 585.2$[M+H]^+$.

Step 4: Preparation of compound LY-22CE-d

The compound LY-22CE-c (1.65g), glycyl-glycyl-L-phenylalanine (788.28 mg, 2.82 mmol, 1 eq), and N,N-diisopropylethylamine (1.09g, 1.40 mL, 8.47 mmol, 3 eq) were sequentially added to N,N-dimethylformamide (16 mL) and water (4 mL) at room temperature. The reaction solution was stirred for 1.5 hours at 20°C. The reaction solution was purified by preparative high performance liquid chromatograph (preparative chromatograph manufacturer: ISCO, model: ISCO-R5, column: YMC-Triart Prep C18 250 × 50 mm × 7 μm, mobile phase: water (0.225% HCOOH)-acetonitrile, an elution ratio of water: 30% to 60%) and lyophilized, to obtain a white solid compound LY-22CE-d (1.84g, two-step yield: 82%).
LCMS (ESI): m/z, 749.4$[M+H]^+$.

Step 5: Preparation of compound LY-22CE-e

Diethylamine (1.80g, 2.53 mL, 24.57 mmol, 10 eq) was added to the solution of compound LY-22CE-d (1.84g, 2.46 mmol, 1 eq) in N,N-dimethylformamide (20 mL) and the reaction solution was stirred at 20°C for 2 hours. The reaction solution was concentrated under reduced pressure to obtain the yellow oily crude product compound LY-22CE-e (2.43g).
LCMS (ESI): m/z, 527.3$[M+H]^+$.

Step 6: Preparation of compound LY-22CE-f

The compound LY-39A-b (982.93 mg, 2.69 mmol, 1.1 eq), the compound LY-22CE-e (2.43g, purity of the crude product: 53%; 2.45 mmol, 1 eq), and N,N-diisopropylethylamine (948.26mg, 1.21 mL, 7.34 mmol, 3 eq) were sequentially added to N,N-dimethylformamide (30 mL) at room temperature. The reaction solution was stirred for 16 hours at 20°C. The reaction solution was purified by preparative high performance liquid chromatograph (preparative chromatograph manufacturer: ISCO, model: ISCO-R5, column: YMC-Triart Prep C18 250 × 50 mm × 7 μm, mobile phase: water (0.225% HCOOH)-acetonitrile, an elution ratio of water: 10% to 30%) and lyophilized, to obtain a white solid product LY-22CE-f.
LCMS (ESI): m/z, 777.3$[M+H]^+$.

Step 7: Preparation of LY-22CE

N,N-diisopropylethylamine (1.18 μL, 7.17 μmol, 2 eq) and N,N,N',N'-tetramethyl-O-(7-azabenzotriazole-1-yl) hexafluorophosphate (3.27 mg, 8.6 μmol, 1.2 eq) were added to the solution of compounds LY-22CE-f (12.25 mg, 7.88 μmol, 1.1 eq, purity: 50%) and LY-22CE-b (4.57 mg, 7.17 μmol, 1 eq) in N,N-dimethylformamide (1 mL), and then the reaction solution was stirred at room temperature for 2 hours. The reaction solution was subjected to preparative high performance liquid chromatograph (preparative chromatograph manufacturer: SHIMADZU, model: LC-20AP, column: P Synergi Max-RP 250 × 40 mm × 10 μm, mobile phase: water (0.225% HCOOH)-acetonitrile, an elution ratio of acetonitrile: 40% to 60%) to obtain the product LY-22CE (0.86 mg, yield = 8.59%).
LCMS (ESI): m/z, 1418$[M+Na]^+$.

Example 32: Preparation of compound LY-22CF

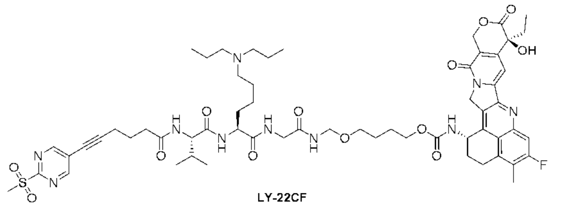

LY-22CF

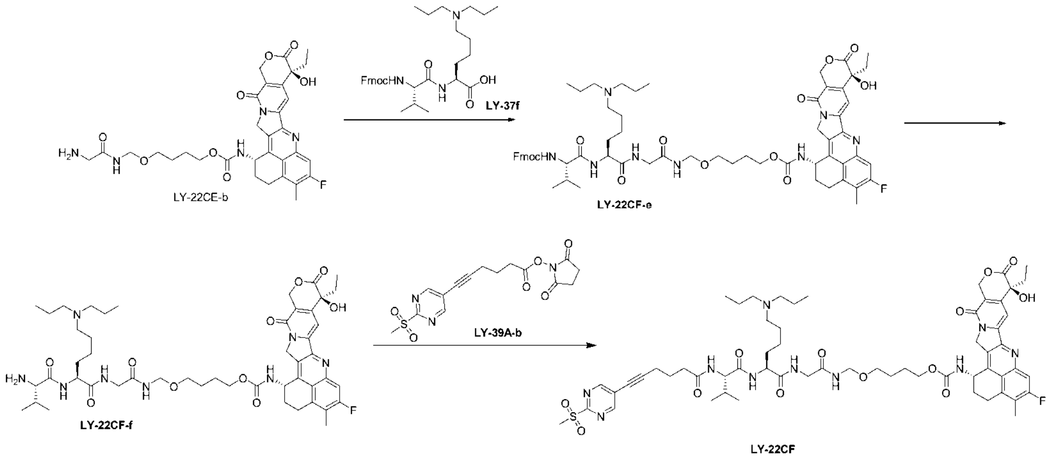


Step 1: Preparation of compound LY-22CF-e

DIPEA (7.09 mg, 9.07 μL, 54.89 μmol, 2 eq) and HATU (12.52 mg, 32.93 μmol, 1.2 eq) were added to a solution of compound **LY-22CE-b** (17.50 mg, 27.44 μmol, 1.1 eq) and compound **LY-37f** (16.66 mg, 30.19 μmol, 1.1 eq) in DMF (2 mL) in the ice water bath, and then the reaction solution was warmed to room temperature again and further stirred for 2 hours. The reactant was purified by preparative high performance liquid chromatograph (preparative chromatograph manufacturer: Oriendo, model: BRIX-2860 R5, column: Welch Xtimate C18 150 × 30 mm × 10 μm, mobile phase: water (0.225% HCOOH)-acetonitrile, an elution ratio of acetonitrile: 40% to 70%) and lyophilized, to obtain a white solid compound LY-22CF-e (5.60 mg, yield: 17.42%, purity: 100%).

LCMS (ESI): m/z, 1193[M+Na]$^+$.

Step 2: Preparation of compound LY-22CF-f

Diethylamine (1.75 mg, 2.46 μL, 23.90 μmol, 5 eq) was added to the solution of compound LY-22CF-e (5.60 mg, 100%, 4.78 μmol, 1 eq) in DMF (1 mL), and the reaction solution was stirred at room temperature for 2 hours. The reaction solution was concentrated under reduced pressure, to obtain a yellow oily liquid crude product (4.50 mg).

LCMS (ESI): m/z, 949[M+H]$^+$.

Step 3: Preparation of compound LY-22CF

DIPEA (1.23 mg, 1.57 μL, 9.48 μmol, 2 eq) was added to a solution of compound LY-22CF-f (4.50 mg, 4.74 μmol, 1 eq) and compound 2,5-dioxopyrrolidin-1-yl-6-(2-methylsulfonylpyrimidin-5-yl)hex-5-ynoate (LY-39A-b) (2.08 mg, 5.69 μmol, 1.2 eq) in DMF (1 mL); and the reaction solution was stirred for 4 hours. The reactant was subjected to preparative high performance liquid chromatograph (preparative chromatograph manufacturer: Oriendo, model: Lab311-DJ-R2, column: Welch Xtimate C18 150 × 30 mm × 10 μm, mobile phase: water (0.225% HCOOH)-acetonitrile, an elution ratio of acetonitrile: 30% to 60%) and lyophilized, to obtain a white solid LY-22CF (0.61 mg, yield: 10.7%, purity: 95%).

LCMS (ESI): m/z, 1199[M+H]$^+$.

Example 33: Preparation of compound **LY-27**

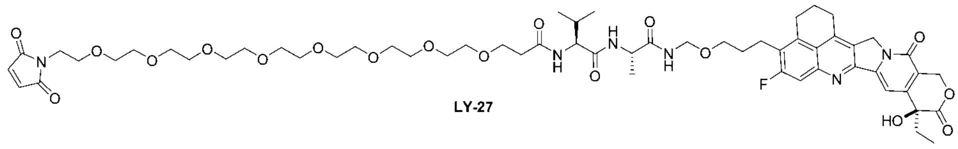

LY-27

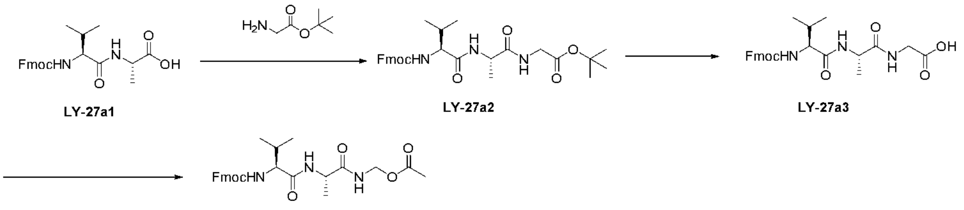


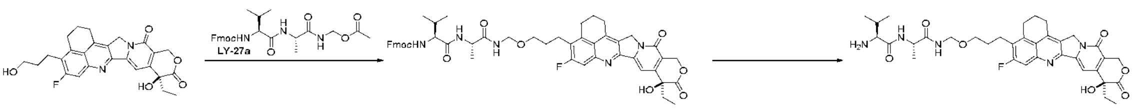


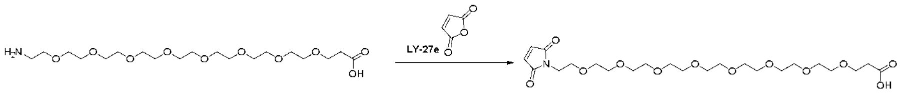


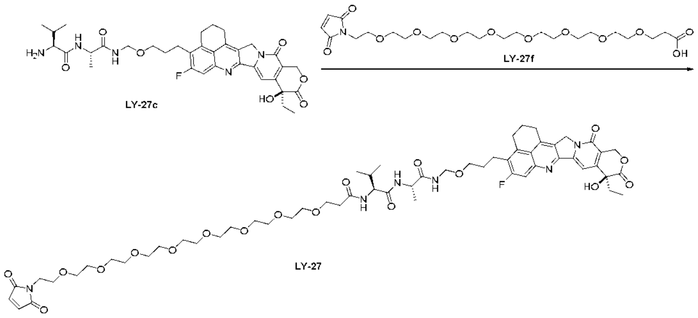


Step 1: Preparation of compound **LY-27a2**

2-ethoxy-1-ethoxycarbonyl-1,2-dihydroquinoline (7.68g, 31.06 mmol, 1.5 eq) was added to a solution of (((9H-fluoren-9-yl)methoxy)carbonyl)-L-valine-L-alanine (ChemExpress, **LY-27a1**) (8.5g, 20.71 mmol, 1 eq) and tert-butyl glycinate (3.26g, 24.85 mmol, 1.2 eq) in methanol/dichloromethane (50 mL/100 mL) in the ice water bath, and the reaction solution was stirred for 10 minutes and then stirred at 40°C overnight. After concentration, the residue was pulped with petroleum ether/ethyl acetate (100 mL/50 mL), and the resulting solution was stirred for 20 minutes and then filtered to obtain a white solid (10.6g, yield = 97.76%).
LCMS (ESI): m/z, 546[M+Na]$^+$.

Step 2: Preparation of compound **LY-27a3**

Trifluoroacetic acid (20 mL) was added to the solution of compound **LY-27a2** (10.6g, 20.24 mmol, 1.0 eq) in dichloromethane (60 mL) in the ice water bath, and the reaction solution was naturally warmed to room temperature and stirred overnight. The reaction solution was concentrated with dichloromethane (10 mL) 3 times to obtain a light yellow sticky crude product (15.24g).
LCMS (ESI): m/z, 468[M+H]$^+$.

Step 3: Preparation of compound **LY-27a**

Lead tetraacetate (23.3g, 52.52 mmol, 2.6 eq), acetic acid (4.6 mL, 80.8 mmol, 4 eq), and copper acetate (1.46g, 8.08 mmol, 0.4 eq) were added to a solution of compound **LY-27a3** (15.24g, 20.2 mmol, 1.0 eq, purity: 62.1%) in N,N-dimethylformamide (100 mL) under a nitrogen atmosphere, and the reaction solution was stirred at 60°C for 1 hour and warmed to room temperature. The reaction solution was concentrated under reduced pressure, water was added to the

residue, a large amount of insoluble substances were generated and dissolved in water (100 mL) and acetonitrile (100 mL), the resulting solution was extracted with ethyl acetate (100 mL), washed with brine (50 mL × 2), dried with anhydrous sodium sulfate, filtered, and concentrated to obtain compound **LY-27a** (8g, yield = 82.2%, purity = 80%).

LCMS (ESI): m/z, 504[M+Na]$^+$.

$^1$H NMR (400 MHz, Methanol-$d_4$) δ 7.81 (d, *J* = 7.6 Hz, 2H), 7.69 (t, *J*= 7.0 Hz, 2H), 7.40 (t, *J* = 7.6 Hz, 2H), 7.32 (t, *J* = 7.4 Hz, 2H), 5.20 (d, *J* = 6.2 Hz, 2H), 4.48-4.30 (m, 3H), 4.24 (t, *J* = 6.8 Hz, 1H), 3.94 (d, *J* = 6.8 Hz, 1H), 2.01 (s, 3H), 1.36 (d, *J* = 7.2 Hz, 3H), 0.97 (dd, *J* = 9.6, 6.6 Hz, 6H).

Step 4: Preparation of compound **LY-27b**

0.39 equivalents of PPTS (10.55 mg, 41.98 μmol) was added to a solution of compound **PY-8** (50 mg, 107.64 μmol, 1 eq, purity: 80%) and compound **LY-27a** (51.84 mg, 107.64 μmol, 1 eq) in 10 mL of THF solvent and the reaction solution was stirred at 45°C for 16 hours. The reaction solution was filtered and then purified by preparative high performance liquid chromatograph (preparative chromatograph manufacturer: SHIMADZU, model: LC-20AP, column: Welch Xtimate C18 250 × 30 mm × 10 μm, mobile phase: water (0.225% HCOOH)-acetonitrile, an elution ratio of water: 12% to 42%) and lyophilized, to obtain a yellow solid compound LY- **27b** (17 mg, yield: 14%, purity: 78%).

LCMS (ESI): m/z, 886.4 [M+H]$^+$.

Step 5: Preparation of compound **LY-27c**

10 equivalents of diethylamine (16 μL, 123.3 μmol, 10 eq) was added to a mixture solution of dichloromethane and a solution of compound **LY-27b** (17 mg, purity: 78%; 12.33 μmol, 1 eq) in 4 mL of tetrahydrofuran (volume ratio: 1:1) and the reaction solution was stirred at 25°C for 16 hours. The reaction solution was filtered and then purified by preparative high performance liquid chromatograph (preparative chromatograph manufacturer: SHIMADZU, model: LC-20AP, column: Welch Xtimate C18 250 × 30 mm × 10 μm, mobile phase: water (0.225% HCOOH)-acetonitrile, an elution ratio of water: 12% to 42%) and lyophilized, to obtain 14 mg of a yellow solid **LY-27c.**

LCMS (ESI): m/z, 664.1 [M+H]$^+$.

Step 6: Preparation of compound **LY-27f**

H2N-PEG8-COOH (ChemExpress, 200 mg, 452.98 μmol, 1 eq) and maleic anhydride (44.42 mg, 30.01 μL, 452.98 μmol, 1 eq) were added to the solution of acetic acid (10 mL) and the reaction solution was stirred at 160°C for 16 hours. The reaction solution was concentrated under reduced pressure and purified by prep-HPLC (preparative chromatograph manufacturer: Luna, model: Lab311-ISCO-R4, Phenomenex Luna C18 (250×50 mm×10 μm) is used as a chromatographic column. mobile phase: water (0.225% HCOOH)-acetonitrile, an elution ratio of water: 12% to 42%), to obtain a white solid **LY-27f** (90 mg, 173 μmol, yield: 38%).

LCMS (ESI): m/z, 522.3 [M+H]$^+$.

Step 7: Preparation of compound **LY-27**

Chloro-N,N,N',N'-tetramethylformamidinium hexafluorophosphate (2.5 mg, 9.0 μmol, 1.2 eq) and N-methylimidazole (1.9 mg, 23 μmol, 3.0 eq) were added to the solution of compounds **LY-27c** (5.0 mg, 7.5 μmol, 1.0 eq) and **LY-27f** (3.9 mg, 7.5 μmol, 1.0 eq) in N,N-dimethylformamide (0.5 mL), and the reaction solution was stirred at 25°C for 2 hours. The reaction solution was purified by high performance liquid chromatograph (preparative chromatograph manufacturer: Luna, model: Lab311-ISCO-R4, Phenomenex Luna C18 (250×50 mm×10 μm) is used as a chromatographic column. mobile phase: water (0.225% HCOOH)-acetonitrile, an elution ratio of water: 30% to 60%), to obtain a white solid **LY-27** (0.64 mg, 0.5 FA salt, yield: 5%).

LCMS (ESI): m/z, 1189.6 [M+Na]$^+$.

$^1$H NMR (400 MHz, DMSO-d6) δ 8.58 (t, *J* = 6.7 Hz, 1H), 8.05 (d, *J*= 7.2 Hz, 1H), 7.85 (d, *J* = 8.7 Hz, 1H), 7.72 (d, *J* = 11.3 Hz, 1H), 7.30 (s, 1H), 7.02 (s, 2H), 6.52 (s, 1H), 5.43 (s, 3H), 5.24 (s, 2H), 4.56 (qd, *J* = 10.2, 6.4 Hz, 3H), 4.28 - 4.13 (m, 3H), 3.56 (dd, *J* = 8.8, 5.6 Hz, 4H), 3.51 (s, 2H), 3.46 (s, 12H), 3.45 - 3.43 (m, 5H), 3.11-3.14 (m, 5H), 2.85 (t, *J* = 7.8 Hz, 2H), 2.47 - 2.32 (m, 4H), 2.10-2.08 (m, 3H), 1.90-1.85 (m, 4H), 1.74 (t, *J* = 7.6 Hz, 3H), 1.20 (d, *J* = 7.2 Hz, 4H), 0.93 - 0.76 (m, 12H).

Example 34: Preparation of compound **LY-28**

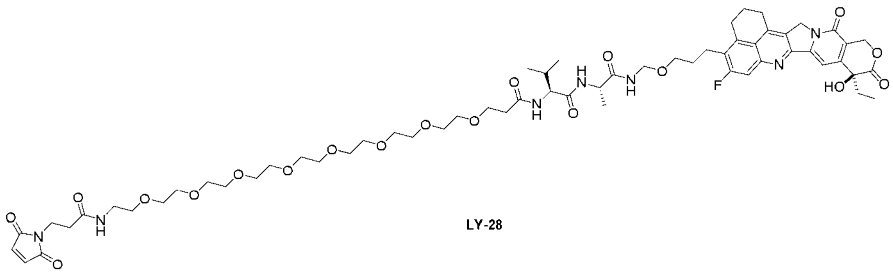

LY-28

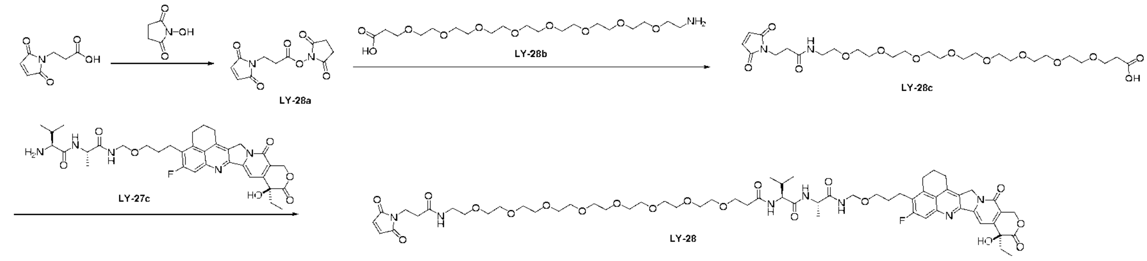


Step 1: Preparation of compound **LY-28a**

DIC (747.1 mg, 5.92 mmol, 2 eq) was added to a low-temperature solution of 3-(2,5-dioxo-2,5-dihydro-1H-pyrrol-1-yl)propionic acid (500 mg, 2.96 mmol, 1 eq) and N-hydroxysuccinimide (425.6 mg, 3.7 mmol, 1.25 eq) in DMF (15 mL) in the nitrogen atmosphere, and the reaction solution was stirred for 10 minutes under an ice water bath, and then warmed to room temperature and stirred overnight. The reaction solution was concentrated under reduced pressure, washed with water (15 mL) 3 times, washed with saturated brine (15 mL) 2 times, and dried with anhydrous sodium sulfate, and the reaction solution was filtered and concentrated to obtain a crude product of a white solid compound (859.1 mg).

LCMS (ESI): m/z, 289 $[M+Na]^+$.

Step 2: Preparation of compound **LY-28c**

Triethylamine (348 mg, 3.44 mmol, 4 eq) and compound **LY-28b** (ChemExpress) (459.1 mg, 1.72 mmol, 1.5 eq) were added to the solution of compound **LY-28a** (507 mg, 1.15 mmol, 1 eq) in DCM (5mL) under nitrogen atmosphere, and the reaction solution was stirred at room temperature for 1 hour. The reaction solution was concentrated under reduced pressure, and the residue was purified by preparative high performance liquid chromatograph (preparative chromatograph manufacturer: ISCO, model: ISCO-3, column: YMC-Triart Prep C18 250 × 30 mm × 10 μm, mobile phase: water (0.225% FA)-ACN, an elution ratio of water: 21% to 51%), to obtain a light yellow oily compound **LY-28c** (500 mg).

LCMS (ESI): m/z, 593$[M+H]^+$.

$^1H$ NMR (400 MHz, $CHCl_3$-*d*) δ 6.96 (s, 1H), 6.70 (s, 2H), 3.84 (t, *J* = 7.0 Hz, 2H), 3.77 (t, *J* = 6.0 Hz, 2H), 3.68 - 3.62 (m, 28H), 3.54 (t, *J* = 4.8 Hz, 2H), 3.42 (q, *J* = 5.2 Hz, 2H), 2.60 (t, *J* = 6.0 Hz, 2H), 2.53 (t, *J* = 7.2 Hz, 2H).

Step 3: Preparation of compound **LY-28**

TCFH (3.55 mg, 12.7 μmol, 1.2 eq) and NMI (2.6 mg, 31.8 μmol, 3 eq) were added to a solution of compounds **LY-28c** (6.9 mg, 11.6 μmol, 1.1 eq) and **LY-27c** (7 mg, 10.6 μmol, 1 eq) in acetonitrile (1 mL) and DMF (0.3 mL) under the nitrogen atmosphere, and the reaction solution was stirred at room temperature for 1 hour. The reaction solution was concentrated, and the residue was purified by preparative high performance liquid chromatograph (preparative chromatograph manufacturer: ISCO, model: ISCO-3, column: YMC-Triart Prep C18 250 × 30 mm × 10 μm, mobile phase: water (0.225% FA)-ACN, an elution ratio of water: 31% to 61%), to obtain compound **LY-28** (1.03 mg, white solid).

LCMS (ESI): m/z, 1260$[M+Na]^+$.

$^1H$ NMR (400 MHz, $CHCl_3$-*d*) δ 7.68-7.60 (m, 2H), 7.41 (s, 1H), 7.20 (s, 1H), 6.92 (d, *J* = 7.6 Hz, 1H), 6.80 (s, 1H), 6.70 (s, 2H), 5.75 (d, *J* = 16.2 Hz, 1H), 5.30 (s, 6H), 5.18 (s, 2H), 4.79 - 4.69 (m, 2H), 4.49 (q, *J* = 6.8, 6.4 Hz, 1H), 4.16 (t, *J* = 6.4 Hz, 1H), 3.87 - 3.76 (m, 4H), 3.64 (dq, *J* = 8.4, 3.2 Hz, 26H), 3.58 - 3.51 (m, 4H), 3.41 (q, *J* = 5.2 Hz, 2H), 3.14 (dt, *J* = 18.8, 6.2 Hz, 4H), 2.93 (t, *J* = 7.8 Hz, 2H), 2.71 - 2.63 (m, 1H), 2.52 (t, *J* = 7.2 Hz, 2H), 2.20 (q, *J* = 7.4, 6.4 Hz, 3H), 1.93-1.83 (m, 4H), 1.37 (d, *J* = 7.2 Hz, 2H), 1.07-0.93 (m, 9H).

Example 35: Preparation of compound **LY-28B**

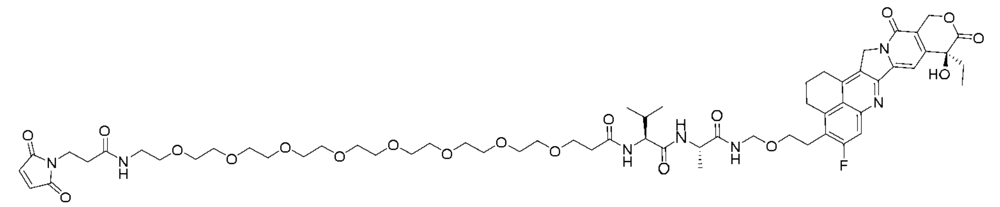

**LY-28B**

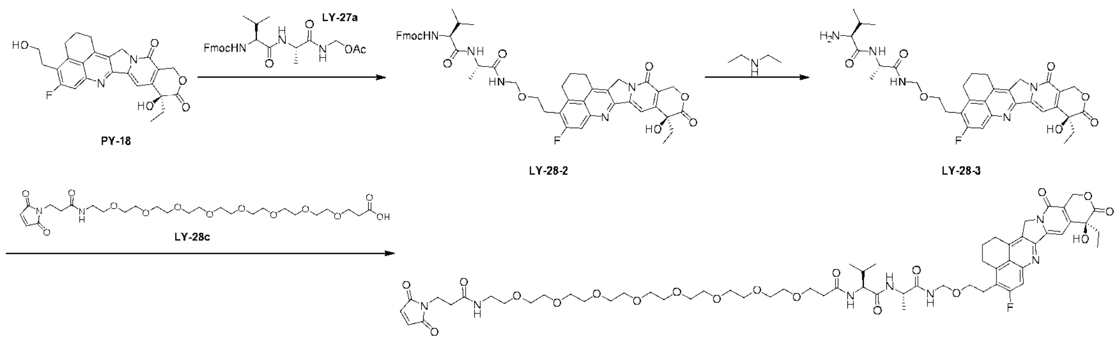


Step 1: Preparation of compound **LY-28-2**

The compound **LY-27a** (21.22 mg, 44.07 μmol, 1.5 eq) was added to the solution of compound **PY-18** (14.20 mg, 93.2%, 29.38 μmol, 1 eq) in tetrahydrofuran (1 mL), and then a catalytic amount of p-toluenesulfonic acid (1.52 mg, 1.42 μL, 8.81 μmol, 0.3 eq) was added. The reaction solution was stirred for 12 hours at 20°C. The reaction solution was isolated and purified by reversed-phase preparative chromatograph (preparative chromatograph manufacturer: ISCO, model: ISCO-4, column: YMC-Triart Prep C18 250 × 30 mm × 10 μm, mobile phase: water (0.225% FA)-ACN, an elution ratio of water: 39% to 69%) and lyophilized, to obtain a white solid product LY-28-2 (12 mg, yield: 43.66%).
LCMS (ESI): m/z, 872.4[M+H]$^+$.

Step 2: Preparation of compound **LY-28-3**

The compound LY-28-2 (12 mg, 13.76 μmol, 1 eq) was dissolved in DMF (1 mL), diethylamine (6.04 mg, 8.51 μL, 82.57 μmol, 6 eq) was added to the solution, and the solution was stirred at 20°C for 2 hours in the nitrogen atmosphere. The reaction solution was concentrated under reduced pressure to obtain the crude product LY-28-3 (8.50 mg, yield: 95.06%) to be directly used for the next step.
LCMS (ESI): m/z, 650.4 [M+H]$^+$.

Step 3: Preparation of compound **LY-28B**

The compound **LY-28c** (4.38 mg, 7.39 μmol, 1.2 eq) and DIPEA (1.59 mg, 2.04 μL, 12.31 μmol, 2 eq) were added to the solution of compound **LY-28-3** (4 mg, 6.16 μmol, 1 eq) in DMF (0.5 mL), HATU (3.51 mg, 9.23 μmol, 1.5 eq) was further added, and the reaction solution was stirred at 20°C for 1 hour. The reaction solution was purified by preparative high performance liquid chromatograph (preparative chromatograph manufacturer: ISCO, model: ISCO-6, column: YMC-Triart Prep C18 250 × 50 mm × 7 μm, mobile phase: water (0.225% FA)-ACN, an elution ratio of water: 30% to 60%), to obtain a white solid compound **LY-28B** (2.84 mg, yield: 37.68%, purity: 98.02%).
LCMS (ESI): m/z, 1246.2[M+H]$^+$.

Example 36: Preparation of compound LY-28C

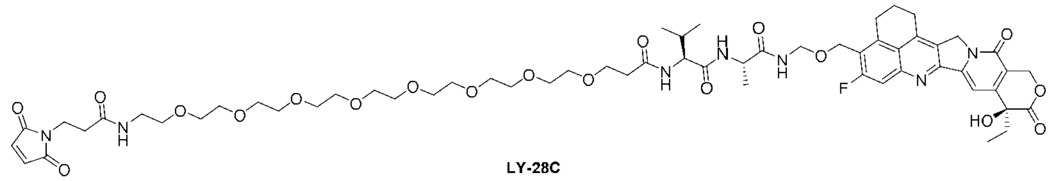

LY-28C

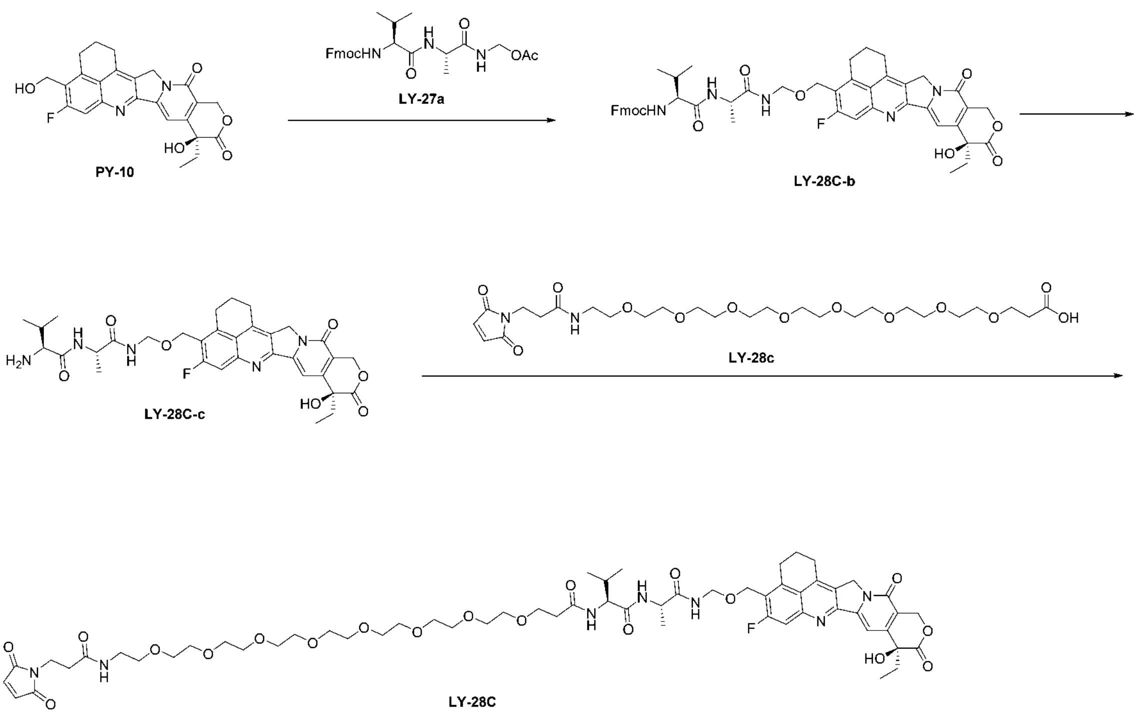

PY-10

LY-27a

LY-28C-b

LY-28C-c

LY-28c

LY-28C

The preparation method is the same as that in Example 10, except that the compound **PY-18** is replaced with the compound **PY-10** to prepare the compound **LY-28C.**

LCMS (ESI): m/z, 1210[M+H]$^+$.

$^1$H NMR (400 MHz, DMSO-d$_6$) δ 8.83 (t, *J* = 6.6 Hz, 1H), 8.11 (d, *J* = 7.2 Hz, 1H), 8.02 (t, *J* = 5.6 Hz, 1H), 7.88 (d, *J* = 8.8 Hz, 1H), 7.75 (d, *J* = 11.2 Hz, 1H), 7.32 (s, 1H), 7.00 (s, 2H), 6.53 (s, 1H), 5.44 (s, 2H), 5.26 (s, 2H), 4.74 (dd, *J* = 10.4, 7.0 Hz, 1H), 4.68-4.61 (m, 3H), 4.30 (p, *J* = 7.2 Hz, 1H), 4.21 (dd, *J* = 8.8, 6.6 Hz, 1H), 3.59 (ddd, *J* = 8.0, 6.4, 2.4 Hz, 4H), 3.50-3.48 (m, 26H), 3.16 (dq, *J* = 17.2, 6.2, 5.8 Hz, 8H), 2.47-2.34 (m, 4H), 2.30 (s, 2H), 2.10-2.02 (m, 2H), 1.96 (dt, *J* = 15.2, 7.6 Hz, 1H), 1.87 (dt, *J* = 14.8, 7.2 Hz, 2H), 1.29 (d, *J* = 7.2 Hz, 3H), 0.90 - 0.86 (m, 6H), 0.83 (d, *J* = 6.8 Hz, 3H). $^{19}$F NMR (377 MHz, DMSO-*d*$_6$) δ-114.09.

Example 37: Preparation of compound **LY-29**

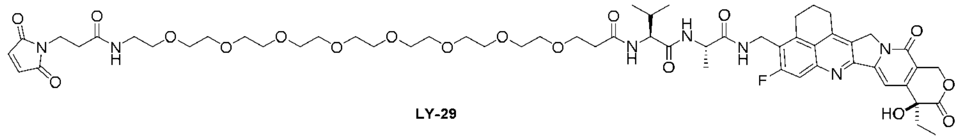

LY-29

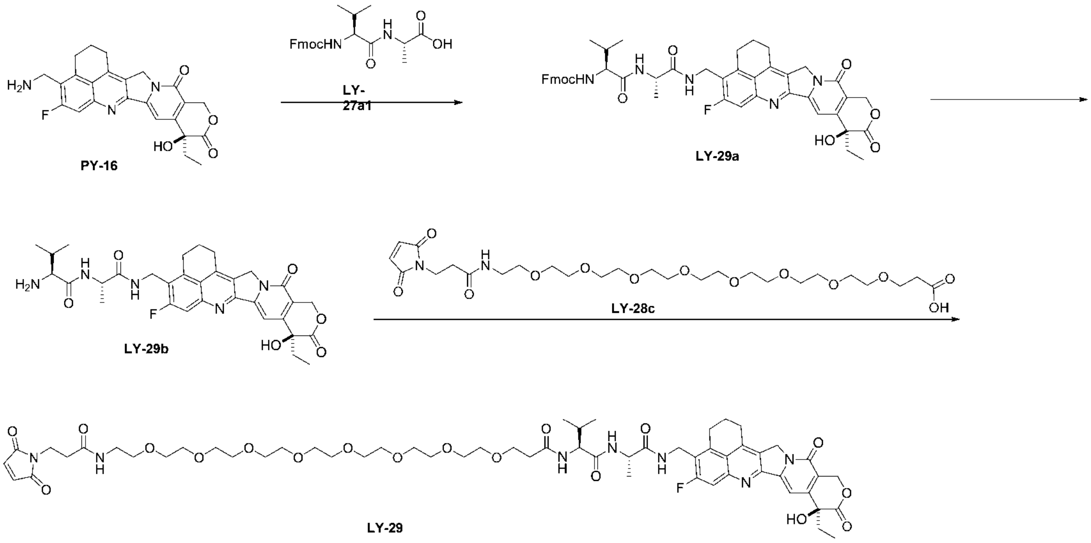

PY-16 LY-27a1 LY-29a

LY-29b LY-28c

LY-29

Step 1: Preparation of compound **LY-29a**

N,N-diisopropylethylamine (0.2 mL, 1.2 mmol, 2 eq) and N,N,N',N'-tetramethyl-O-(7-azabenzotriazole-1-yl) hexafluorophosphate (274.45 mg, 721.77 μmol, 1.2 eq) were added to the solution of compounds **LY-27a1** (246.89 mg, 601.48 μmol, 1 eq) and **PY-16** (327.4 mg, 601.48 μmol, 1 eq) in N,N-dimethylformamide (8 mL), and the reaction solution was stirred at room temperature for 2 hours. The reaction solution was added dropwise to ice water (20 mL), the filter cake was collected and dissolved with methanol, and the resulting solution was purified by column chromatography to obtain the compound **LY-29a** (223.3 mg, yield = 44.84%).

LCMS (ESI): m/z, 828 $[M+H]^+$.

Step 2: Preparation of compound **LY-29b**

Diethylamine (0.15 mL, 1.49 mmol, 6 eq) was added to the solution of compound **LY-29a** (223.3 mg, 248.14 μmol, 1 eq) in N,N-dimethylformamide (8 mL), and the reaction solution was stirred at room temperature for 1 hour. The reaction solution was concentrated under reduced pressure, small amounts of acetonitrile and water were added, and the reaction solution was lyophilized to obtain the light yellow solid compound **LY-29b** (215.9 mg, crude product).

LCMS (ESI): m/z, 606 $[M+H]^+$.

Step 3: Preparation of compound **LY-29**

N,N-diisopropylethylamine (60.36 μL, 365.22 μmol, 2 eq) and N,N,N',N'-tetramethyl-O-(7-azabenzotriazole-1-yl)hexafluorophosphate (76.38 mg, 200.87 μmol, 1.1 eq) were added to the solution of compounds **LY-29b** (158 mg, 182.61 μmol, 1 eq, purity: 70%) and **LY-28c** (151.51 mg, 255.65 μmol, 1.4 eq) in N,N-dimethylformamide (10 mL), and then the reaction solution was stirred at room temperature for 2 hours. The reaction solution was purified by preparative high performance liquid chromatograph (preparative chromatograph manufacturer: Oriendo, model: BRIX-2860, column: Synergi Max-RP 250 × 40 mm × 10 μm, mobile phase: water (0.225% HCOOH)-acetonitrile, an elution ratio of acetonitrile: 20% to 55%), to obtain the compound **LY-29** (96.83 mg, yield = 44.93%).

LCMS (ESI): m/z, 1080 $[M+H]^+$.

$^1H$ NMR (400 MHz, DMSO-$d_6$) δ 8.16 (t, *J* = 5.2 Hz, 1H), 8.02 (d, *J* = 7.0 Hz, 2H), 7.84 (d, *J* = 8.6 Hz, 1H), 7.74 (d, *J* = 11.2 Hz, 1H), 7.31 (s, 1H), 7.00 (s, 2H), 6.53 (s, 1H), 5.44 (s, 2H), 5.23 (s, 2H), 4.51 (s, 2H), 4.25 (t, *J* = 7.2 Hz, 1H), 4.13 (dd, *J* = 8.6, 6.6 Hz, 1H), 3.59 (t, *J* = 7.2 Hz, 2H), 3.56-3.40 (m, 34H), 3.15 (p, *J* = 5.2 Hz, 6H), 2.47-2.34 (m, 2H), 2.10-2.01 (m, 2H), 1.89 (dq, *J* = 17.0, 6.6 Hz, 3H), 1.19 (d, *J* = 6.8 Hz, 3H), 0.88 (t, *J* = 7.2 Hz, 3H), 0.79 (q, *J* = 7.2, 6.4 Hz, 6H). $^{19}F$ NMR (377 MHz, DMSO-$d_6$) δ -112.70.

Example 38: Preparation of compound **LY-29B**

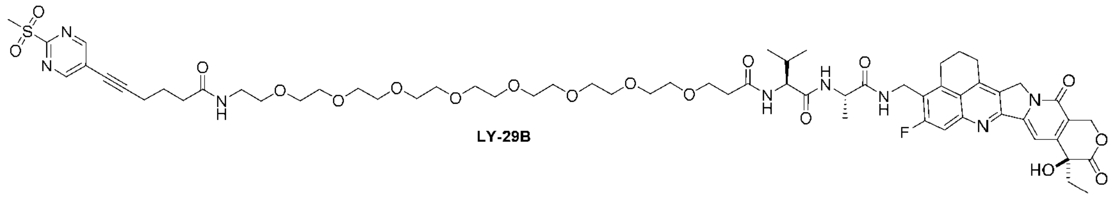

LY-29B

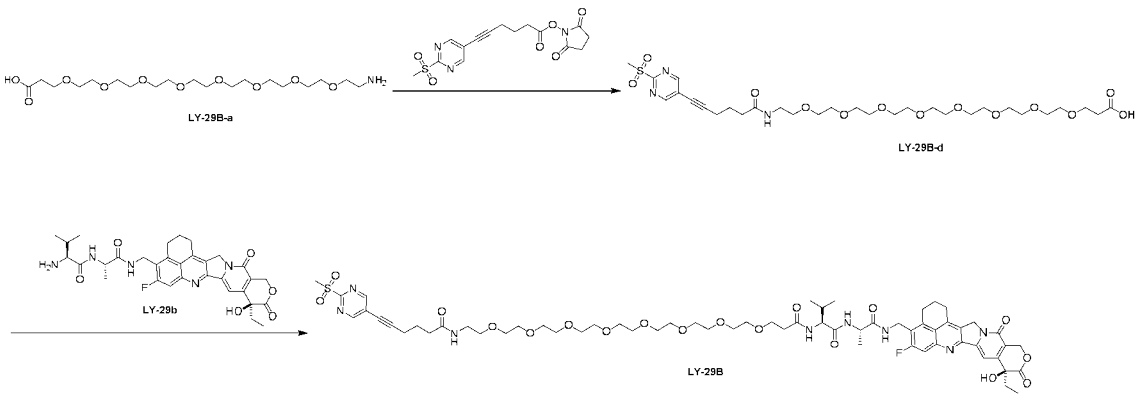

LY-29B-a LY-29B-d LY-29b LY-29B

Step 1: Preparation of compound **LY-29B-d**

The compound **LY-29B-a** (Bidepharm, 60 mg, 135.89 μmol, 1 eq) and 2,5-dioxopyrrolidin-1-yl6-(2-(methylsulfonyl)pyrimidin-5-yl)hex-5-ynoate (refer to the synthesis example LY-44A and Example 56; 59.11 mg, 84%, 135.89 μmol, 1 eq) were dissolved in N,N-dimethylformamide (3 mL), then N,N-diisopropylethylamine (35.13 mg, 44.92 μL, 271.79 μmol, 2 eq) was added, and the reaction solution was stirred at 20°C for 1 hour. The reaction solution was purified by preparative high performance liquid chromatograph (preparative chromatograph manufacturer: ISCO, model: ISCO-6, column: Welch Xtimate C18 250 × 50 mm × 10 μm, mobile phase: water (0.225% FA)-ACN, an elution ratio of water: 12% to 42%), to obtain a yellow product (23 mg, crude product, yield: 24.47%).

LCMS (ESI): m/z, 692.3 $[M+H]^+$.

Step 2: Preparation of compound **LY-29B**

The compound **LY-29B-d** (18 mg, 26.02 μmol, 1 eq) was dissolved in N,N-dimethylformamide (2 mL), the compound **LY-29b** (15.76 mg, 26.02 μmol, 1 eq) and N,N-diisopropylethylamine (6.73 mg, 8.60 μL, 52.04 μmol, 2 eq) were added to the solution, then HATU (14.84 mg, 39.03 μmol, 1.5 eq) was added, and the solution was stirred at 20°C for 12 hours. The reaction solution was purified by preparative high performance liquid chromatograph (preparative chromatograph manufacturer: ISCO, model: ISCO-6, column: Welch Xtimate C18 250 × 50 mm × 10 μm, mobile phase: water (0.225% FA)-ACN, an elution ratio of water: 21% to 51%), to obtain the product **LY-29B** (9.71 mg, yield: 28.73%, purity: 98.51%).

LCMS (ESI): m/z, 1279.6$[M+H]^+$.

$^1$H NMR (400 MHz, DMSO-*d*$^6$) δ 9.11 (s, 2H), 8.16 (t, *J* = 5.2 Hz, 1H), 8.02 *(d, J*= 7.4 Hz, 1H), 7.93 (t, *J* = 5.7 Hz, 1H), 7.83 (d, *J* = 8.7 Hz, 1H), 7.75 (d, *J* = 11.2 Hz, 1H), 7.32 (s, 1H), 6.52 (s, 1H), 5.44 (s, 2H), 5.26 (s, 2H), 4.51 (s, 2H), 4.25 (t, *J* = 7.2 Hz, 1H), 4.13 (dd, *J* = 8.6, 6.7 Hz, 1H), 3.57-3.46 (m, 26H), 3.45-3.41 (m, 4H), 3.18 (dq, *J* = 15.6, 5.9 Hz, 7H), 2.58-2.52 (m, 3H), 2.42 (dt, J = 13.8, 6.7 Hz, 2H), 2.33-2.24 (m, 3H), 2.05 (t, *J* = 6.4 Hz, 3H), 1.95-1.75 (m, 6H), 1.18 (d, *J* = 7.1 Hz, 3H), 0.88 (t, *J* = 7.3 Hz, 3H), 0.80-0.73 (m, 6H).

Example 39: Preparation of compound **LY-29C**

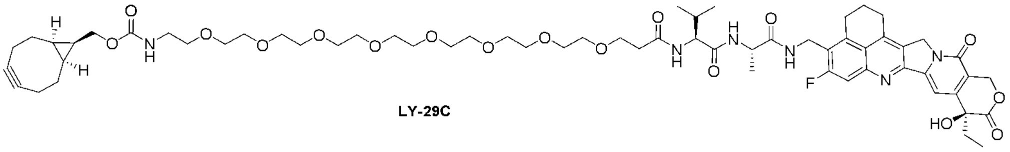

LY-29C

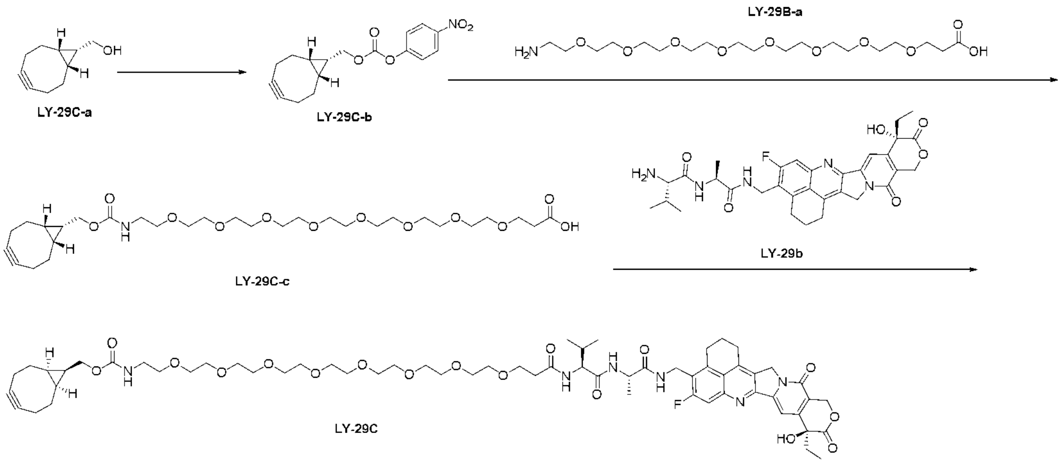

LY-29B-a

LY-29C-a

LY-29C-b

LY-29C-c

LY-29b

LY-29C

Step 1: Preparation of ((1R,8S,9s)-bicyclo[6.1.0]non-4-yn-9-yl)methyl (4-nitrophenyl)carbonate **(LY-29C-b)**

Pyridine (105.31 mg, 107.68 μL, 1.33 mmol, 2.5 eq) and 4-nitrophenyl chloroformate (134.18 mg, 665.69 μmol, 1.25 eq) were added to the solution of compound (1R, 8S, 9S)-bicyclo[6.1.0]non-4-yn-9-ylmethanol (**LY-29C-a**) (80 mg, 532.55 μmol, 1 eq) (Shandong Xingzhi Biopharmaceutical) in anhydrous DCM (5 mL) at 0°C in the nitrogen atmosphere, and after addition, the reaction solution reacted at room temperature for half an hour. The reaction solution was quenched with saturated ammonium chloride (10 mL) at 0°C and extracted with DCM (40 mL × 3) three times. The organic phases were combined, washed with brine, dried with anhydrous sodium sulfate, and filtered, the filtrate was concentrated under reduced pressure, and the residue was purified by silica gel column chromatography (eluents: ethyl acetate/n-hexane = 1:20-1:9) to obtain anhydrous oily compound **LY-29C-b** (75 mg, yield: 44.66%).

Step 2: Preparation of compound **LY-29C-c**

**LY-29C-b** (112 mg, 355.18 μmol, 1 eq) and DIPEA (114.77 mg, 146.76 μL, 887.96 μmol, 2.5 eq) were added to the solution of compound **LY-29B-a** (156.82 mg, 355.18 μmol, 1 eq) in anhydrous DMF (5 mL) at 0°C in the nitrogen atmosphere. After addition, the reaction solution reacted at room temperature for 16 hours, and concentrated under reduced pressure at no more than 40°C, and the residue was purified by preparative high performance liquid chromatograph (preparative chromatograph manufacturer: ISCO, model: ISCO-R1, column: Synergi Max-RP 250 × 40 mm × 10 μm, mobile phase: water (0.225% HCOOH)-acetonitrile, an elution ratio of water: 21% to 51%, to obtain anhydrous oily compound **LY-29C-c** (148 mg, yield: 67.45%).

Step 3: Preparation of compound **LY-29C**

DIEA (5.35 μL, 32.4 μmol, 2 eq) and HATU (7.4 mg, 19.4 μmol, 1.2 eq) were added to the solution of compounds **LY-29C-c** (10 mg, 16.2 μmol, 1 eq) and **LY-29b** (23.3 mg, 16.2 μmol, 1 eq) in DMF (3 mL) in the nitrogen atmosphere, and the reaction solution was naturally warmed to room temperature in the ice water bath and reacted for two hours. The reaction solution was purified by preparative high performance liquid chromatograph (preparative chromatograph manufacturer: SHIMADZU, model: LC-20AP, column: Synergi Max-RP 280 × 30 mm × 10 μm, mobile phase: water (0.225% HCOOH)-acetonitrile, an elution ratio of water: 30% to 60%), to obtain the product **LY-29C** (7.77 mg, yield: = 39.8%).

LCMS (ESI): m/z, 1205[M+H]$^+$.

$^1$H NMR (400 MHz, DMSO-$d_6$) δ 8.16 (t, *J* = 5.2 Hz, 1H), 8.02 (d, *J*= 7.2 Hz, 1H), 7.83 *(d, J*= 8.6 Hz, 1H), 7.74 (t, *J* = 11.6 Hz, 1H), 7.32 (s, 1H), 7.08 (t, *J* = 5.8 Hz, 1H), 6.53 (s, 1H), 5.44 (s, 2H), 5.26 (s, 2H), 4.51 (s, 2H), 4.25 (t, *J*= 7.2 Hz, 1H), 4.13 (dd, *J* = 8.6, 6.6 Hz, 1H), 4.02 (d, *J*= 8.0 Hz, 1H), 3.56-3.40 (m, 36H), 3.13 (dq, *J* = 21.6, 6.0 Hz, 6H), 2.41 (q, *J* = 7.2, 6.8 Hz, 2H), 2.32-2.10 (m, 4H), 2.09-2.00 (m, 2H), 1.96-1.81 (m, 3H), 1.51 (d, *J*= 11.2 Hz, 1H), 1.31-1.22 (m, 1H), 1.20-1.14 (m, 4H), 0.88 (t, *J* = 7.2 Hz, 3H), 0.83 (s, 1H), 0.78 (t, *J* = 7.2 Hz, 6H). $^{19}$F NMR (377 MHz, DMSO-*$d_6$)* δ -112.71.

Example 40: Preparation of compound **LY-29D**

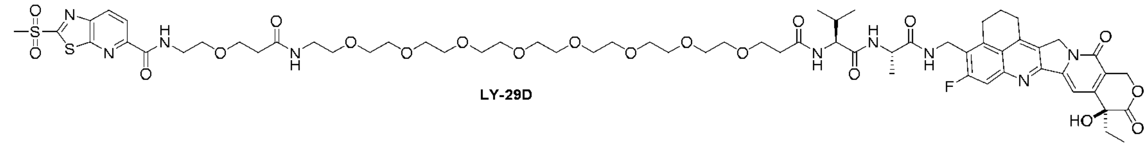

LY-29D

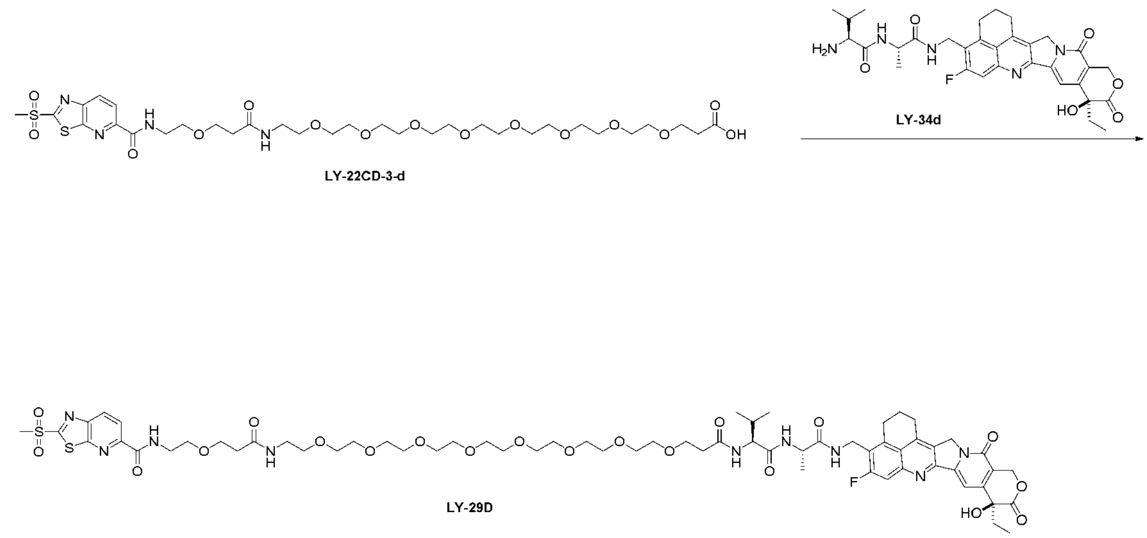

LY-34d

LY-22CD-3-d

LY-29D

Step 1: Preparation of compound LY-29D

DIEA (12.16 mg, 15.55 μL, 94.11 μmol, 3 eq) and HATU (11.93 mg, 31.37 μmol, 1 eq) were added to the solution of compounds LY-22CD-3-d (25 mg, 31.37 μmol, 1 eq, for synthesis of LY-22CD-3-d, refer to the synthesis example of LY-22CD-3) and LY-34d (40.98 mg, 51%, 34.51 μmol, 1.1 eq, for synthesis of LY-34d, refer to the synthesis example of LY-34B) in DMF (2 mL), the reaction solution was stirred for 1 hour at room temperature, and the LCMS showed that the raw materials completed reaction. The reaction solution was subjected to preparative high performance liquid chromatograph (preparative chromatograph manufacturer: Oriendo, model: BRIX-2860 (R5), column: Welch Xtimate C18 150 × 30 mm × 10 μm, mobile phase: water (0.225% HCOOH)-acetonitrile, an elution ratio of acetonitrile: 20% to 50%), to obtain the product LY-29D (15.29 mg, yield = 35.20%).

LCMS (ESI): m/z, 1384[M+H]$^+$. $^1$H NMR (400 MHz, DMSO-d$_6$) δ 9.20 (t, *J* = 5.6 Hz, 1H), 9.00 (d, *J= 8.6* Hz, 1H), 8.51 (d, *J* = 8.6 Hz, 1H), 8.30 (d, *J* = 5.4 Hz, 1H), 8.16 (d, *J* = 7.4 Hz, 1H), 8.04 (t, *J= 5.8* Hz, 1H), 7.98 (d, *J* = 8.6 Hz, 1H), 7.89 (d, *J* = 11.2 Hz, 1H), 7.46 (s, 1H), 6.67 (s, 1H), 5.58 (s, 2H), 5.40 (s, 2H), 4.65 (s, 2H), 4.39 (t, *J* = 7.2 Hz, 1H), 4.31-4.24 (m, 1H), 3.78 (d, *J* = 10.6 Hz, 5H), 3.71-3.54 (m, 34H), 3.32 (q, *J* = 6.2, 6.0 Hz, 6H), 2.60-2.38 (m, 5H), 2.20 (d, *J* = 7.8 Hz, 2H), 2.01 (tt, *J* = 14.2, 6.8 Hz, 3H), 1.32 (d, *J* = 7.0 Hz, 3H), 1.02 (t, *J* = 7.2 Hz, 3H), 0.92 (t, *J* = 7.2 Hz, 6H).

Example 41: Preparation of compound **LY-33**

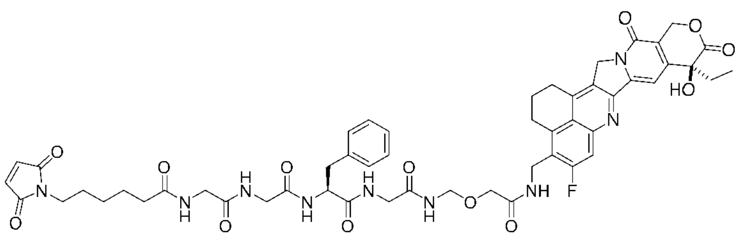

LY-33

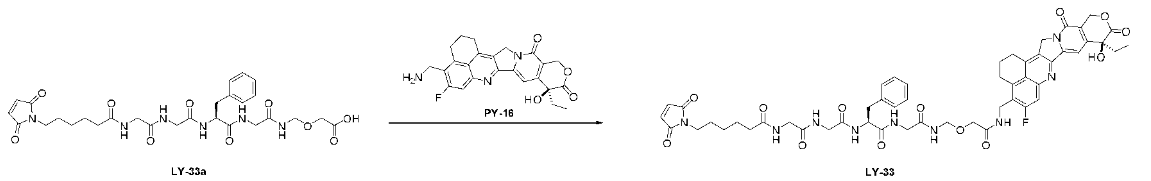


TCFH (16.4 mg, 58.38 μmol, 1.2 eq) and NMI (12.0 mg, 145.95 μmol, 3 eq) were added to the solution of compounds **LY-33a** (Chengdu Zen Sunda) (30 mg, 48.65 μmol, 1 eq) and **PY-16** (27.5 mg, 63.25 μmol, 1.3 eq) in DMF (2 mL) under the nitrogen atmosphere, and the reaction solution was stirred at room temperature for 1 hour. The reaction solution was purified by preparative high performance liquid chromatograph (preparative chromatograph manufacturer: SHIMADZU, model: LC-20AP, column: Synergi Max-RP 280 × 30 mm × 10 μm, mobile phase: water (0.225% FA)-ACN, an elution ratio of acetonitrile: 30% to 50%), to obtain light yellow solid compound **LY-33** (6.62 mg, yield = 13.2%).

LCMS (ESI): m/z, 1034[M+H]$^+$.

$^1$H NMR (400 MHz, DMSO-$d_6$) δ 8.58 (t, *J* = 6.6 Hz, 1H), 8.29 (t, *J* = 5.8 Hz, 1H), 8.17-8.10 (m, 2H), 8.06 (t, *J* = 5.6 Hz, 1H), 7.99 (t, *J* = 5.6 Hz, 1H), 7.73 (d, *J* = 11.2 Hz, 1H), 7.30 (d, *J* = 7.6 Hz, 1H), 7.24-7.15 (m, 5H), 6.98 (s, 2H), 6.51 (s, 1H), 5.43 (s, 1H), 5.23 (s, 1H), 4.57 *(d, J=* 6.2 Hz, 4H), 4.47-4.43 (m, 1H), 3.88 (s, 2H), 3.78 - 3.52 (m, 8H), 3.23 (d, *J* = 5.6 Hz, 2H), 3.16 - 3.13 (m, 2H), 3.01 (dd, *J* = 13.6, 4.6 Hz, 2H), 2.81-2.74 (m, 2H), 2.08 (d, *J* = 8.0 Hz, 4H), 1.90-1.84 (m, 2H), 1.46 (q, *J=* 7.4 Hz, 4H), 1.17 (t, *J* = 7.6 Hz, 2H), 0.88 (t, *J* = 7.2 Hz, 3H).

Example 42: Preparation of compound **LY-33A**

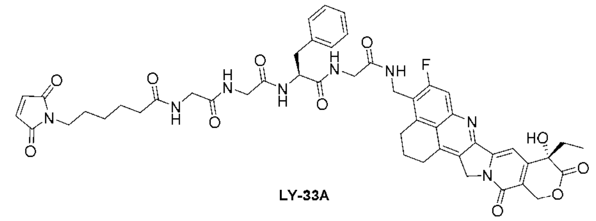

LY-33A

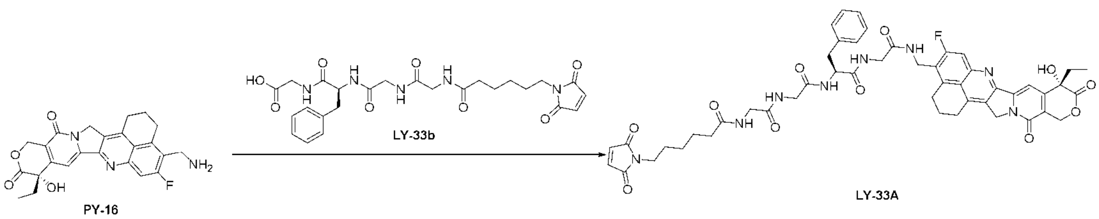


HOBt (11.5 mg, 85.0 μmol, 3.0 eq) and EDCI (9.8 mg, 60 μmol, 1.8 eq) were added to the solution of compounds **PY-16** (16 mg, 37 μmol, 1.3 eq) and **LY-33b** (15 mg, 28 μmol, 1.0 eq) (Chengdu Zen Sunda) in DMA (0.5 mL), and the reaction solution was stirred at 25°C for 2 hours. The reaction solution was purified by high performance liquid chromatograph (preparative chromatograph manufacturer: Luna, model: Lab311-ISCO-R4, column: Phenomenex Luna C18 250 × 50 mm × 10 μm, mobile phase: water (0.225% HCOOH)-acetonitrile, an elution ratio of water: 21% to 51%) and the eluate was lyophilized, to obtain a white solid compound **LY-33A** (3.63 mg, yield: 14%).

LCMS (ESI): m/z, 947.4 [M+H]$^+$.

$^1$H NMR (400 MHz, DMSO-$d^6$) δ 8.24 (t, *J* = 5.6 Hz, 1H), 8.18 (t, *J* = 5.2 Hz, 1H), 8.07 (dd, *J* = 7.2, 4.0 Hz, 2H), 7.99 (t, *J* = 5.6 Hz, 1H), 7.76 (d, *J* = 11.2 Hz, 1H), 7.31 (s, 1H), 7.26-7.18 (m, 4H), 7.15 (td, *J* = 6.0, 2.4 Hz, 1H), 6.98 (s, 2H), 6.53 (s, 1H), 5.43 (s, 2H), 5.25 (s, 2H), 4.53 (d, *J* = 5.2 Hz, 2H), 4.48-4.43 (m, 1H), 3.72-3.62 (m, 5H), 3.55 (d, *J* = 5.6 Hz, 1H), 3.52-3.50 (m, 1H), 3.22 (t, *J* = 6.0 Hz, 2H), 3.17-3.12 (m, 2H), 3.02-2.97 (m, 1H), 2.78-2.72 (m, 1H), 2.09 (dd, *J* = 10.4, 4.4 Hz, 4H), 1.86 (dq, *J* = 14.0, 7.2 Hz, 2H), 1.45 (h, *J* = 7.2 Hz, 4H), 1.27-1.09 (m, 3H), 0.87 (t, *J* = 7.2 Hz, 3H).

Example 43: Preparation of compound **LY-34**

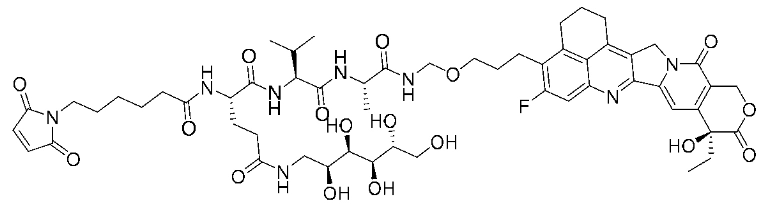

LY-34

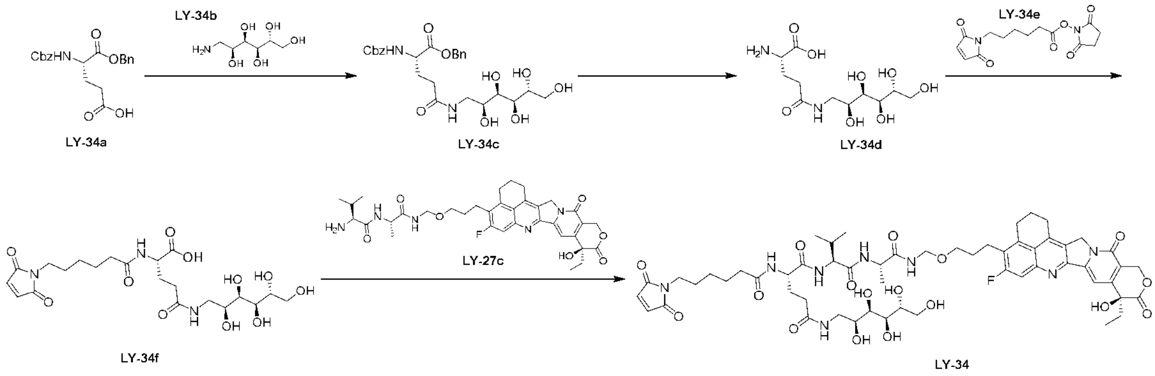


Step 1: Preparation of compound **LY-34c**

The compound N-benzyloxycarbonyl-L-glutamic acid 1-benzyl ester (**LY-34a**) (1.50g, 4.04 mmol, 1 eq) was dissolved in N,N-dimethylformamide (60 mL), and compound 4-(4,6-dimethoxytriazine)-4-methylmorpholinyl hydrochloride (Bidepharm, 2.24g, 8.08 mmol, 2 eq) and triethylamine (613.04 mg, 842.09 μL, 6.06 mmol, 1.5 eq) were added at room temperature. The reaction solution was stirred at 25°C for 0.5 hours, and then D-glucosamine (731.80 mg, 4.04 mmol, 1 eq) was added. The reaction solution was stirred for 11.5 hours at 25°C. The reaction solution was purified by preparative high performance liquid chromatograph (preparative chromatograph manufacturer: ISCO, model: ISCO-R4, column: Phenomenex Luna C18 250 × 50 mm × 10 μm, mobile phase: water (0.225% HCOOH)-acetonitrile, an elution ratio of water: 11% to 31%), to obtain a white solid product (2g, yield: 78.74%, purity: 85%).

LCMS (ESI): m/z, 535.3 $[M+H]^+$.

Step 2: Preparation of compound **LY-34d**

The compound **LY-34c** (300 mg, 561.21 μmol, 1 eq) was dissolved in methanol (10 mL)/water (10 mL), palladium carbon (59.72 mg, 4.97 μL, 561.21 μmol, 1 eq) was added to the solution, hydrogen displacement was performed three times, and the reaction solution was stirred at 25°C for 12 hours in the hydrogen atmosphere. The reaction solution was filtered and lyophilized to obtain a white solid product (180 mg, yield: 103.36%).

LCMS (ESI): m/z, 333.1$[M+Na^+]$.

Step 3: Preparation of compound **LY-34f**

6-(maleimino)succinimidyl ester (142.07 mg, 460.84 μmol, 1.1 eq) and triethylamine (169.57 mg, 232.93 μL, 1.68 mmol, 4 eq) were added to the solution of compound **LY-34d** (130 mg, 418.95 μmol, 1 eq) in N,N-dimethylformamide (6 mL), and the reaction solution was stirred at 25°C for 12 hours. The reaction solution was purified by preparative high performance liquid chromatograph (preparative chromatograph manufacturer: Oriendo, model: BRIX-2860, column: YMC-Triart Prep C18 250 × 50 mm × 7 μm, mobile phase: water (0.225% formic acid)-acetonitrile, an elution ratio of water: 1% to 24%), to obtain a white solid product (70 mg, yield: 33.18%).

LCMS (ESI): m/z, 504.2$[M+H^+]$.

Step 4: Preparation of compound **LY-34**

Compound **LY-27c** (6.59 mg, 9.93 μmol, 1 eq), p-toluenesulfonic acid (4.99 mg, 19.86 μmol, 2 eq), and 4-hydroxybenzotriazole (5.37 mg, 39.72 μmol, 4eq) were added to the solution of compound **LY-34f** (5 mg, 9.93 μmol, 1 eq) in N,N-dimethylacetamide, and finally 1-ethyl-3-(3-dimethylaminopropyl)carbodiimide hydrochloride (2.28 mg, 2.60 μL, 11.92 μmol, 1.2 eq) was added. The reaction solution was stirred at 25°C for 12 hours. The reaction solution was purified by

preparative high performance liquid chromatograph (preparative chromatograph manufacturer: Oriendo, model: BRIX-2860, column: Synergi Max-RP 250 × 40 mm × 10 μm, mobile phase: water (0.225% formic acid)-acetonitrile, an elution ratio of water: 21% to 51%), to obtain white solid product **LY-34** (1.13 mg, yield: 9.90%).

LCMS (ESI): m/z, 1149.5[M+H]$^+$.

$^1$H NMR (400 MHz, DMSO-d6) δ 8.61 (t, *J* = 6.7 Hz, 2H), 8.11 (d, *J* = 7.0 Hz, 2H), 8.00 (s, 1H), 7.72 (d, *J* = 11.0 Hz, 4H), 7.66 (d, *J* = 8.7 Hz, 2H), 7.30 (d, *J* = 8.0 Hz, 2H), 6.99 (s, 2H), 6.54 (d, *J* = 10.6 Hz, 2H), 5.43 (s, 2H), 5.26 (s, 2H), 4.75 (d, *J* = 4.6 Hz, 1H), 4.60-4.52 (m, 3H), 4.48 (s, 1H), 4.37 (dd, *J* = 13.5, 5.9 Hz, 3H), 4.27 - 4.18 (m, 5H), 3.62-3.56 (m, 4H), 3.15 (d, *J* = 6.8 Hz, 4H), 3.03-2.99 (m, 2H), 2.86 (s, 2H), 2.09 (s, 5H), 1.88 (d, *J* = 7.3 Hz, 2H), 1.46 (dd, *J* = 12.7, 5.7 Hz, 6H), 1.19 (t, *J* = 6.5 Hz, 6H), 0.82 (dt, *J* = 14.7, 7.4 Hz, 9H).

Example 44: Preparation of compound **LY-34B**

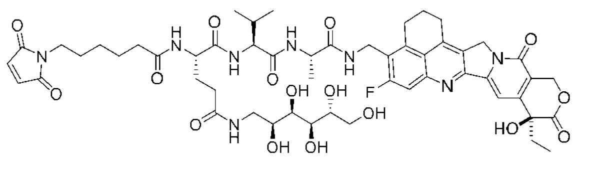

LY-34B

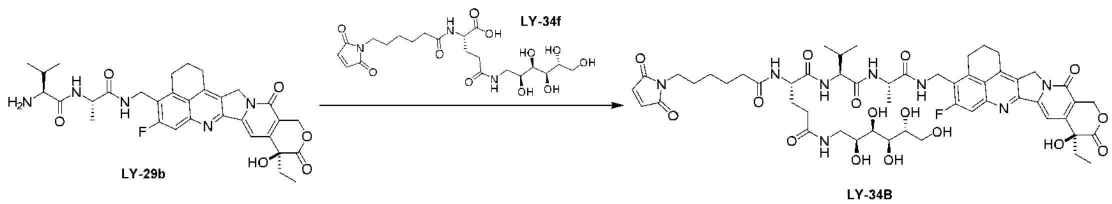

LY-34f

LY-29b

LY-34B

Compound **LY-34f** (6.23 mg, 12.38 μmol, 1 eq) and HOBt (2.51 mg, 1.67 μL, 18.57 μmol, 1.5 eq) were added to the solution of compound **LY-29b** (7.50 mg, 12.38 μmol, 1 eq) in DMF (0.5 mL), then EDCI (3.56 mg, 4.06 μL, 18.57 μmol, 1.5 eq) was added, and after addition, the reaction solution was stirred at 25°C for 2 hours in the nitrogen atmosphere. The reaction solution was purified by preparative high performance liquid chromatograph (preparative chromatograph manufacturer: ISCO, model: ISCO-4, column: YMC-Triart Prep C18 250 × 50 mm × 7 μm, mobile phase: water (0.225% FA)-acetonitrile, an elution ratio of water: 21% to 51%), to obtain a white solid compound **LY-34B** (450 μg, yield: 3.18%, purity: 99.6%).

LCMS (ESI): m/z, 1091.5.

Example 45: Preparation of compound **LY-34C**

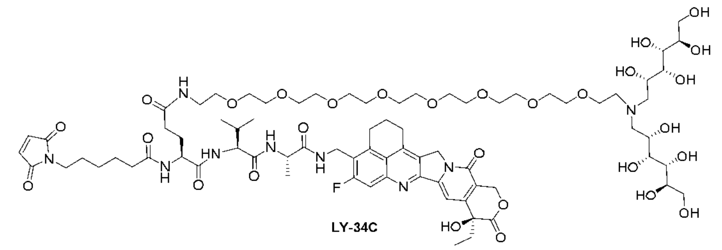

LY-34C

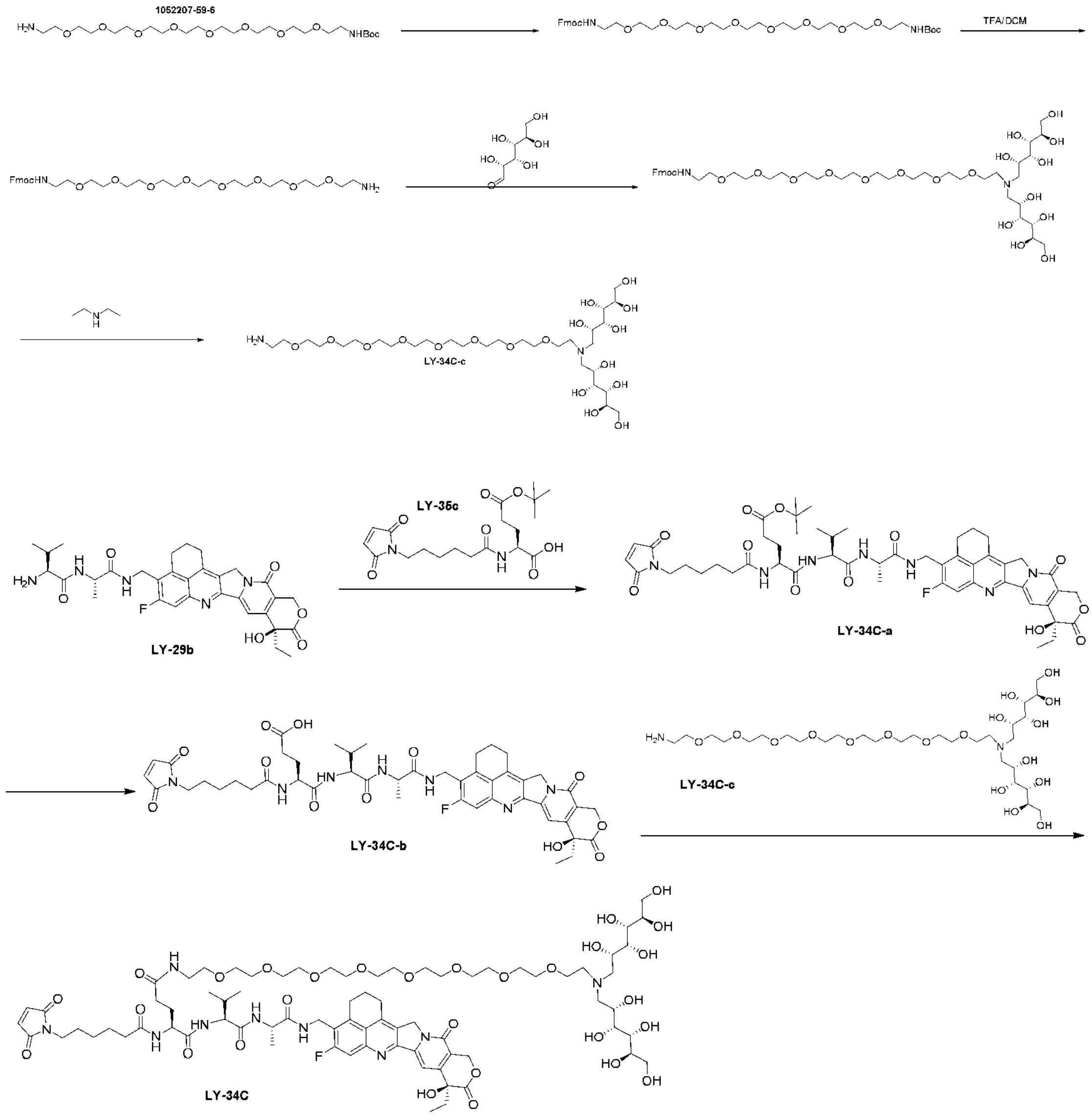


LY-34C-c

LY-29b

LY-34C-a

LY-34C-b

LY-34C-c

LY-34C

Step 1: Preparation of (9H-fluoren-9-yl)methyl tert-butyl(3,6,9,12,15,18,21,24-octoxahexane-1,26-yl)tert-butyl dicarboxylate

(9H-fluoren-9-yl)methyl(2,5-dioxopyrrolidin-1-yl)carbonate (411.86 mg, 1.22 mmol, 1.1 eq) and sodium carbonate (235.29 mg, 92.63 μL, 2.22 mmol, 2 eq, dissolved in 5 mL of water) were added to a solution of (26-amino-3,6,9,12,15,18,21,24-octaoxahexacosyl)carbamate (Bidepharm) (569 mg, 1.11 mmol, 1 eq) in tetrahydrofuran (20 mL) at 0°C. After addition, the reaction solution reacted at 25°C for 2 hours, the LCMS showed that the reaction ended, the reaction solution was first concentrated to remove most of tetrahydrofuran, the residue was diluted with 30 mL of water, then extracted with ethyl acetate (30 mL × 3) 3 times, dried with anhydrous sodium sulfate and filtered, and the filtrate was concentrated to obtain yellow oily (9H-fluoren-9-yl)methyl tert-butyl(3,6,9,12,15,18,21,24-octoxahexane-1,26-yl)tert-butyl dicarboxylate (890 mg).

LCMS (ESI): m/z, 757.4 $[M+H]^+$.

Step 2: Preparation of (9H-fluoren-9-yl)methyl(26-amino-3,6,9,12,15,18,21,24-octaoxahexacosyl)carbamate

Trifluoroacetic acid (1.5 mL) was added to (9H-fluoren-9-yl)methyl tert-butyl(3,6,9,12,15,18,21,24-octoxahexane-1,26-yl)tert-butyl dicarboxylate (890 mg, 1.21 mmol, 1 eq) in dichloromethane (1.5 mL) at 0°C, and after addition, the reaction solution reacted at 20°C for 2 hours. The reaction solution was concentrated to obtain a yellow oily compound (70 mg).

LCMS (ESI): m/z, 635.4 $[M+H]^+$.

Step 3: Preparation of (9H-fluoren-9-yl)methyl((29S,30R,31R,32R)-29,30,31,32,33-pentahydroxy-27-((2S,3R,4R,5R)-2,3,4,5,6-pentahydroxyhexyl)-3,6,9,12,15,18,21,24-octaoxa-27-azatripentyl)carbamate

Sodium cyanoborohydride (381.14 mg, 6.07 mmol, 5 eq) was added to the solution of (9H-fluoren-9-yl) methyl(26-amino-3,6,9,12,15,18,21,24-octaoxahexacosyl)carbamate (770 mg,1.21 mmol,1 eq) and D-glucose (2.19g, 12.13 mmol, 10 eq) in methanol (20 mL) and acetic acid (2 mL), and after addition, the reaction solution was heated to 55°C for reaction for 40 hours. The reaction solution was purified by preparative high performance liquid chromatograph (preparative chromatograph manufacturer: ISCO, model: ISCO, column: YMC-Triart Prep C18 250 × 50 mm × 7 μm, mobile phase: water (0.225% HCOOH)-acetonitrile, an elution ratio of water: 30% to 60%) and lyophilized, to obtain a yellow oily compound (676 mg, yield 54.97%, purity: 95%).

LCMS (ESI): m/z, 963.6$[M+H]^+$.

Step 4: Preparation of compound **LY-34C-c**

Diethylamine (243.86 mg, 343.46 μL, 3.33 mmol, 5 eq) was added to the solution of (9H-fluoren-9-yl) methyl((29S,30R,31R,32R)-29,30,31,32,33-pentahydroxy-27-((2S,3R,4R,5R)-2,3,4,5,6-pentahydroxyhexyl)-3,6,9,12,15,18,21,24-octaoxa-27-azatripentyl)carbamate (676 mg, 95%, 666.82 μmol, 1 eq) in DMF (5 mL), and after addition, the reaction solution reacted at 25°C for 16 hours. The reaction solution was purified by preparative high performance liquid chromatograph (preparative chromatograph manufacturer: ISCO, model: ISCO-R5, column: Welch Xtimate C18 250 × 30 mm × 10 μm, mobile phase: water (0.225% HCOOH)-acetonitrile, an elution ratio of water: 1% to 24%) and lyophilized, to obtain a colorless oily compound (490 mg, yield: 99.19%).

LCMS (ESI): m/z, 741.5$[M+H]^+$.

$^1$H NMR (400 MHz, deuterated water) δ 3.90-3.53 (m, 47H), 3.22 (t, *J* = 5.0 Hz, 6H).

Step 5: Preparation of compound **LY-34C-a**

Compound **LY-35c** (7.85 mg, 19.81 μmol, 1.2 eq) was added to the solution of compound **LY-29b** (10 mg, 16.51 μmol, 1 eq) in N,N-dimethylformamide (0.5 mL), then 4-hydroxybenzotriazole (3.35 mg, 2.23 μL, 24.77 μmol, 1.5 eq) and N-(3-dimethylaminopropyl)-N'-ethylcarbodiimide hydrochloride (4.75 mg, 24.77 μmol, 1.5 eq) were added sequentially, and after addition, the reaction solution was stirred at 25°C for 2 hours. The reaction solution was purified by preparative high performance liquid chromatograph (preparative chromatograph manufacturer: ISCO, model: ISCO-R1, column: Phenomenex Luna C18 250 × 50 mm × 10 μm, mobile phase: water (0.225% HCOOH)-acetonitrile, an elution ratio of water: 32% to 62%), to obtain a white solid product (8 mg, yield: 49.24%).

LCMS (ESI): m/z, 984.5.

Step 6: Preparation of compound **LY-34C-b**

Compound **LY-34C-a** (6 mg, 6.10 μmol, 1 eq) was stirred in dichloromethane/trifluoroacetic acid (volume ratio: 5:1, total volume: 1 mL) for 1 hour at 25°C. The reaction solution was directly concentrated to obtain a crude product of a yellow solid (5 mg, yield: 88.37%).

LCMS (ESI): m/z, 928.4$[M+H^+]$.

Step 7: Preparation of compound **LY-34C**

Compound **LY-34C-c** (3.19 mg, 4.31 μmol, 1 eq) and N,N-diisopropylethylamine (1.11 mg, 1.42 μL, 8.62 μmol, 2 eq) were added to the solution of compound **LY-34C-b** (4 mg, 4.31 μmol, 1 eq) in N,N-dimethylformamide (500 μL), then 2-(7-azabenzotriazole)-N,N,N',N'-tetramethyluronium hexafluorophosphate (1.97 mg, 5.17 μmol, 1.2 eq) was added, and the reaction solution was stirred at 0°C for 2 hours. The reaction solution was purified by preparative high performance liquid chromatograph (preparative chromatograph manufacturer: Oriendo, model: BRIX-2860, column: YMC-Triart Prep C18 250 × 50 mm × 7 μm, mobile phase: water (0.225% formic acid)-acetonitrile, an elution ratio of water: 30% to 60%), to

obtain a white solid product (3.31 mg, yield: 45.93%, purity: 98.73%).

LCMS (ESI): m/z, 1651.7[M+H]+.

$^1$H NMR (400 MHz, DMSO-$d_6$) δ 8.20 (d, *J*= 5.5 Hz, 1H), 8.05 (d, *J* = 7.2 Hz, 1H), 7.97 (d, *J* = 7.8 Hz, 1H), 7.84 (t, *J* = 5.7 Hz, 1H), 7.75 (d, *J*= 11.2 Hz, 1H), 7.61 (d, *J* = 8.7 Hz, 1H), 7.32 (s, 1H), 6.98 (s, 2H), 6.52 (s, 1H), 5.43 (s, 2H), 5.26 (s, 2H), 4.56-4.45 (m, 6H), 4.32 (t, *J* = 5.6 Hz, 4H), 4.26-4.07 (m, 6H), 3.65 (dt, *J* = 8.6, 4.4 Hz, 3H), 3.60-3.54 (m, 5H), 3.52-3.47 (m, 30H), 3.25-3.01 (m, 14H), 2.74-2.68 (m, 2H), 2.59 (dd, *J* = 13.2, 4.3 Hz, 3H), 2.06 (d, *J* = 7.5 Hz, 6H), 1.87 (tq, *J* = 14.8, 7.5, 7.1 Hz, 5H), 1.65 (dd, *J* = 13.5, 6.7 Hz, 2H), 1.44 (d, *J* = 7.6 Hz, 4H), 1.18 (d, *J* = 7.1 Hz, 4H), 0.89 (d, *J* = 7.2 Hz, 3H), 0.77 (dd, *J* = 14.3, 6.7 Hz, 5H).

Example 46: Preparation of compound **LY-35**

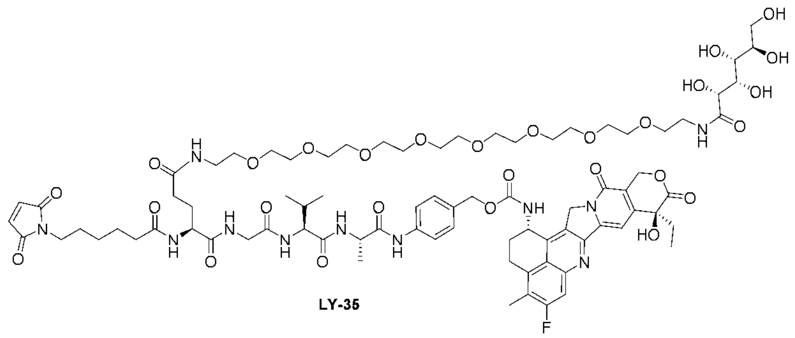

**LY-35**

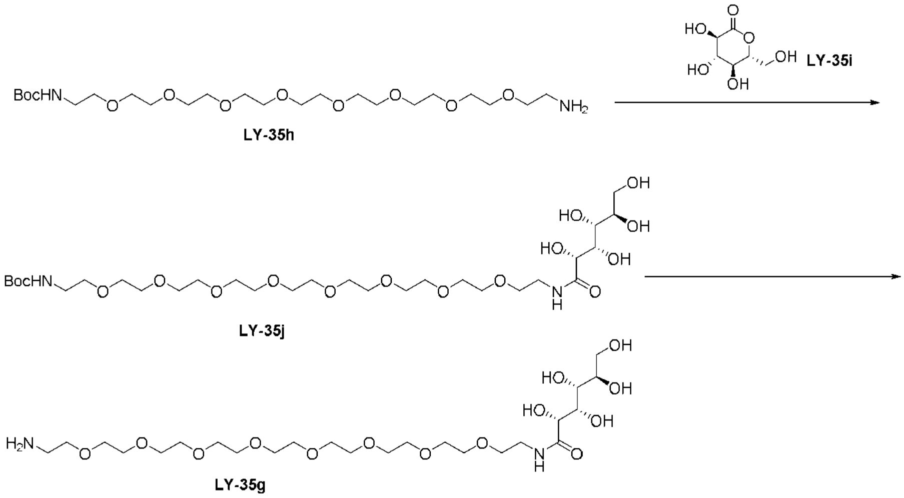

**LY-35h**

**LY-35i**

**LY-35j**

**LY-35g**

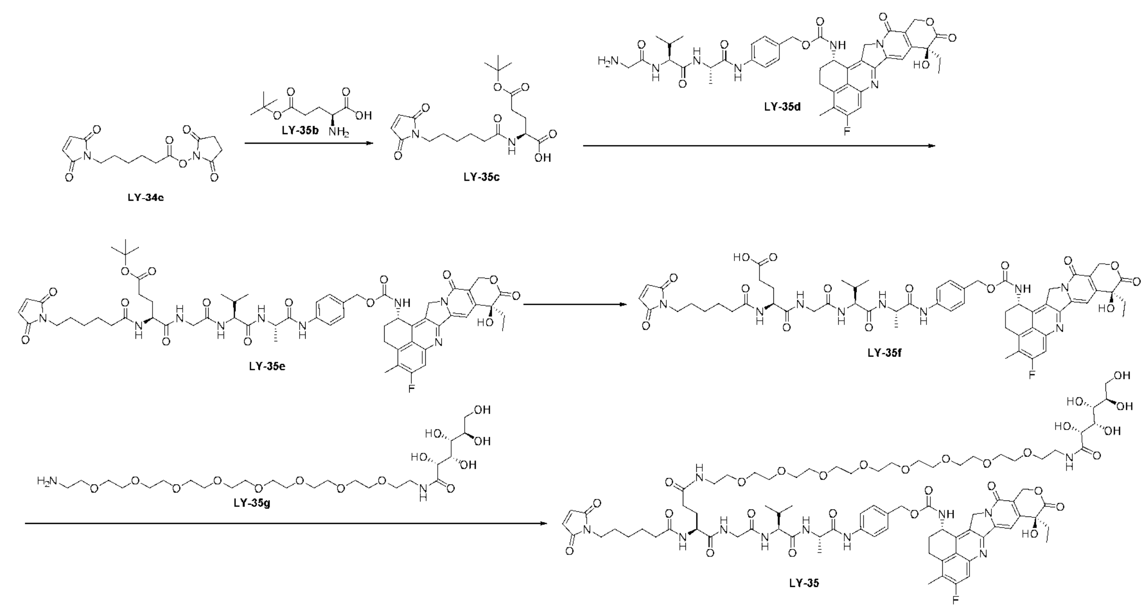

LY-34c
LY-35c
LY-35e
LY-35f
LY-35

Step 1: Preparation of compound **LY-35j**

(3R,4S,5S,6R)-3,4,5-trihydroxy-6-(hydroxymethyl)tetrahydro-2H-pyrano-2-one (1.04g, 5.85 mmol, 10 eq) and triethylamine (296 mg, 2.93 mmol, 5 eq) were added to a solution of compound LY-35h (Xi'an Jixue) (300 mg, 0.58 mmol, 1 eq) in methanol (15 mL) in the nitrogen atmosphere at room temperature. The reaction solution reacted at 70°C for 14 hours and cooled to room temperature, and the reaction solution was concentrated under reduced pressure to obtain the crude product of compound **LY-35j** (410 mg).

LCMS (ESI): m/z, 691.4[M+1]$^+$/713.3 [M+Na]$^+$.

Step 2: Preparation of compound **LY-35g**

TFA (4 mL) was added to the solution of compound **LY-35j** (410 mg, 0.593 mmol, 1.0 eq) in DCM (15 mL) in the ice water bath, the reaction solution reacted for 2 hours at room temperature after addition, the reaction solution was concentrated under reduced pressure to obtain a crude product, and the crude product was purified by preparative high performance liquid chromatograph (preparative chromatograph manufacturer: ISCO, model: ISCO-R1, column: YMC-Triart Prep C18 250 × 50 mm × 7 μm, mobile phase: water (0.225% HCOOH)-acetonitrile, an elution ratio of water: 1% to 24%), to obtain a yellow oily compound **LY-35g** (181 mg, yield: 51%).

LCMS (ESI): m/z, 591.4[M+1]$^+$/613.3 [M+Na]$^+$.

$^1$H NMR (400 MHz, DMSO-*d*$^6$)$\delta$ 4.29 (d, *J* = 3.7 Hz, 1H), 4.06 (d, *J* = 3.1 Hz, 1H), 3.87 - 3.54 (m, 36H), 3.43 (d, *J*= 5.9 Hz, 2H), 3.21 - 3.15 (m, 2H).

Step 3: Preparation of compound **LY-35c**

(S)-2-amino-5-(tert-butoxy)-5-oxovaleric acid (660 mg, 3.24 mmol, 1.0 eq) and DIEA (839 mg, 6.49 mmol, 2.0 eq) were added to the solution of 2,5-dioxopyrrolidin-1-yl-6-(2,5-dioxo-2,5-dihydro-1H-pyrrol-1-yl)hexanoate (1g, 3.24 mmol, 1.0 eq) in DMF (15 mL), the reaction solution reacted at room temperature for 6 hours after addition, and the reaction solution was concentrated to remove half of DMF, and purified by preparative high performance liquid chromatograph (preparative chromatograph manufacturer: ISCO, model: ISCO-R4, column: Phenomenex Luna C18 250 × 50 mm × 10 μm, mobile phase: water (0.225% HCOOH)-acetonitrile, an elution ratio of water: 10% to 40%, to obtain anhydrous oily compound **LY-35c** (1.18g, yield: 91%).

LCMS (ESI): m/z, 419.2 [M+Na]$^+$.

Step 4: Preparation of compound **LY-35e**

The compound LY-35d (Chengdu Zen Sunda) (50 mg, 61.6 μmol, 1 eq), HATU (30.4 mg, 80 μmol, 1.3 eq), and DIPEA (48 mg, 370 μmol, 6.0 eq) were added to the solution of compound **LY-35c** (61 mg, 154 μmol, 2.5 eq) in DMF (5 mL) at 0°C in the nitrogen atmosphere, and the reaction solution reacted at room temperature for 2 hours. The reaction solution

was purified by preparative high performance liquid chromatograph (preparative chromatograph manufacturer: ISCO, model: ISCO-R1, column: Synergi Max-RP 250 × 40 mm × 10 μm, mobile phase: water (0.225% HCOOH)-acetonitrile, an elution ratio of water: 39% to 69%) and lyophilized, to obtain white solid compound **LY-35e** (38.4 mg, yield: 37.4%).

LCMS (ESI): m/z, 1190.6[M+H]$^+$ / 1212.5[M+Na]$^+$.

Step 5: Preparation of compound **LY-35f**

TFA (0.8 mL) was added dropwise to the solution of compound **LY-35e** (10 mg, 8.4 μmol, 1.0 eq) in DCM (4 mL) at 0°C, and the reaction solution reacted at 0°C-10°C for 4 hours after addition, and was concentrated at room temperature to obtain compound **LY-35f** (10 mg, yield: 75.56%, purity: 72%).

LCMS (ESI): m/z, 1134.5[M+H]$^+$ / 567.8[1/2M+H].

Step 6: Preparation of compound **LY-35**

The compound **LY-35g** (4.9 mg, 8.25 μmol, 1.3 eq), HATU (3.14 mg, 8.25 μmol, 1.3 eq), and DIPEA (5 mg, 38.09 μmol, 6.0 eq) were added to the solution of compound **LY-35f** (10 mg, 6.35 μmol, purity: 72%, 1 eq) in DMF (2 mL) at 0°C in the nitrogen atmosphere, and the reaction solution reacted at room temperature for 2 hours after addition. The reaction solution was purified by preparative high performance liquid chromatograph (preparative chromatograph manufacturer: ISCO, model: ISCO-R1, column: YMC-Triart Prep C18 250 × 50 mm × 7 μm, mobile phase: water (0.225% HCOOH)-acetonitrile, an elution ratio of water: 30% to 60%) and lyophilized, to obtain white solid **LY-35** (4.34 mg, yield: 29.45%).

$^1$H NMR (400 MHz, DMSO-$d^6$) δ 9.93 (s, 2H), 8.47 (s, 4H), 8.36 (d, *J* = 7.2 Hz, 1H), 8.25 (d, *J* = 6.8 Hz, 1H), 8.20-8.11 (m, 2H), 8.09-8.00 (m, 3H), 7.85 (t, *J* = 5.8 Hz, 2H), 7.81-7.73 (m, 3H), 7.64-7.57 (m, 4H), 7.36 (d, *J* = 8.0 Hz, 2H), 7.31 (s, 1H), 6.98 (d, *J* = 3.3 Hz, 2H), 6.53 (s, 1H), 5.44 (s, 4H), 5.29 (s, 4H), 5.07 (s, 2H), 4.54 (d, *J* = 22.8 Hz, 4H), 4.45-4.33 (m, 5H), 4.18-4.16 (m, 4H), 3.99 (s, 2H), 3.90 (s, 2H), 3.76-3.71 (m, 3H), 3.57 (d, *J* = 11.2 Hz, 11H), 3.18 (d, *J* = 7.2 Hz, 4H), 2.38 (d, *J* = 2.0 Hz, 3H), 2.09 (d, *J* = 7.2 Hz, 9H), 1.97-1.77 (m, 9H), 1.46 (d, *J* = 7.8 Hz, 5H), 1.35-1.27 (m, 5H), 1.17 (t, *J* = 8.1 Hz, 3H), 0.91 - 0.80 (m, 13H).

LCMS (ESI): m/z, 1728.8[M+Na]$^+$ / 854[1/2M+H]$^+$.

Example 47: Preparation of compound **LY-37A**

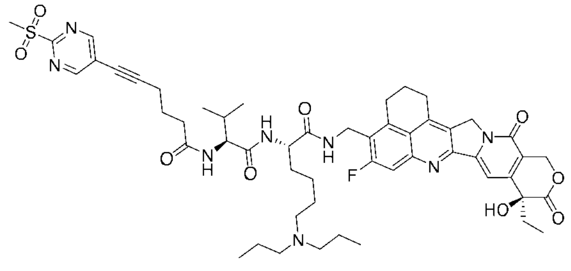

LY-37A

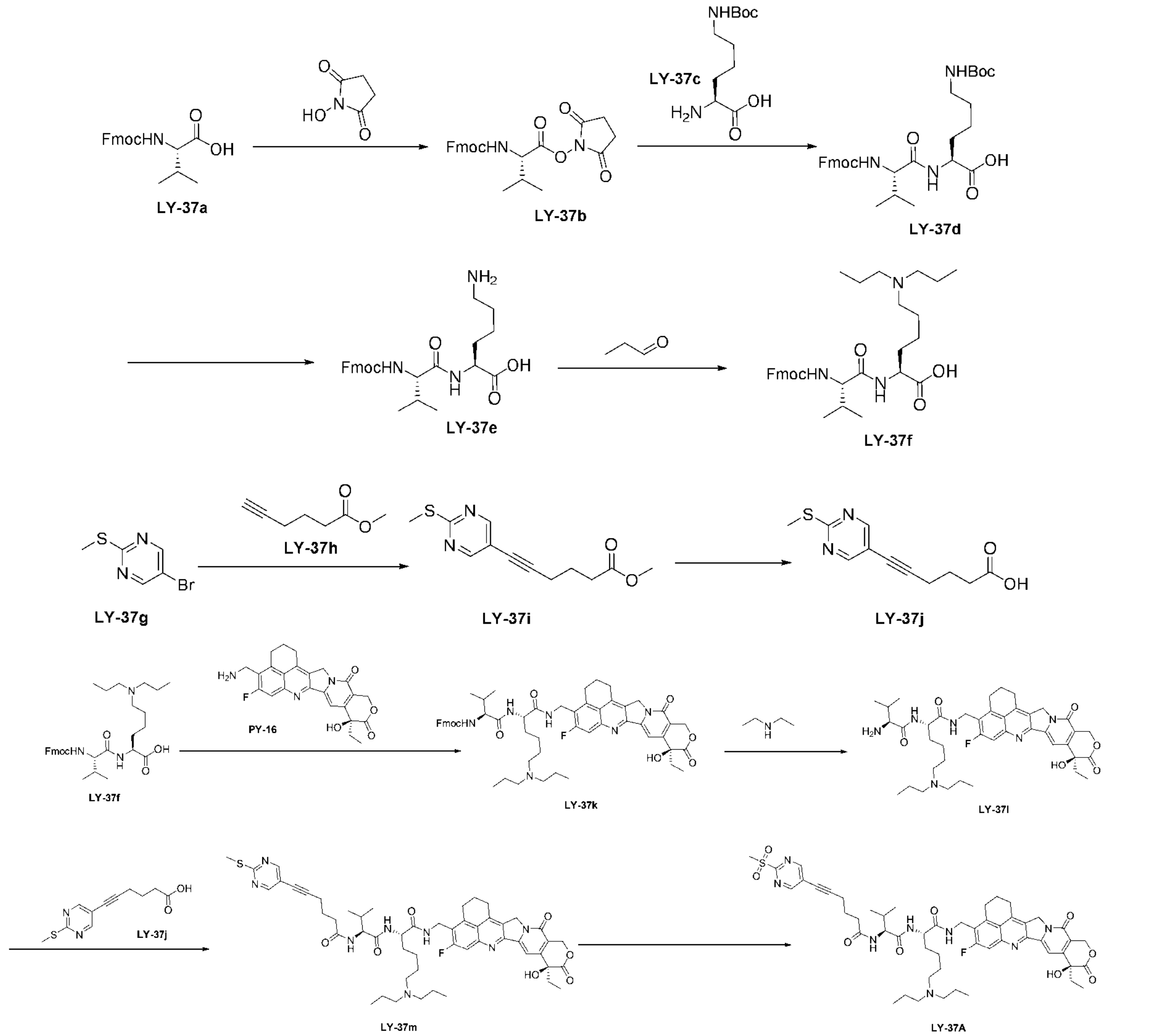


Step 1: Preparation of compound **LY-37b**

The compound (((9H-fluoren-9-yl)methoxy)carbonyl)-L-valine (5g, 1.0 eq) and NHS (2.03g, 1.2 eq) were dissolved in anhydrous tetrahydrofuran (40 mL) in the nitrogen atmosphere, DCC (3.65g, 4.50 mL, 1.2 eq) was added, and the reaction solution was stirred at room temperature for reaction for 16 hours. The reaction solution was diluted with anhydrous tetrahydrofuran (20 mL) and filtered, the filter cake was washed with tetrahydrofuran (20 mL), and the filtrates were combined and concentrated, to obtain compound 2,5-dioxopyrrolidin-1-yl (((9H-fluoren-9-yl)methoxy)carbonyl)-L-valine (7.3g, yield: 113%) to be directly used for the next reaction.

LCMS: m/z =459.2 $[M+Na]^+$; Rt = 1.034.

Step 2: Preparation of compound **LY-37d**

The compounds **LY-37b** (3.0g, 1.0 eq) and **LY-37c** (Bidepharm) (2.03g, 1.2 eq) were dissolved in tetrahydrofuran (37.5 mL) in the nitrogen atmosphere, the aqueous solution of sodium bicarbonate (2.31g of sodium bicarbonate, 4.0 eq, 37.5 mL of water) was added dropwise, and after the dropwise addition, the reaction solution was stirred at room temperature for reaction for 16 hours. The reaction solution was washed with petroleum ether (30 mL) once, the eluate was neutralized with dilute hydrochloric acid until pH reached about 5, and extracted with ethyl acetate (30 mL) twice, the organic phases were combined, dried and concentrated, to obtain the compound LY-37d (3.6g, yield: 92%) as the white solid to be directly used for the next reaction.

LCMS: m/z = 468.3 $[M+H]^+$; Rt = 0.971 min.

Step 3: Preparation of compound **LY-37e**

The compound **LY-37d** (245 mg, 1.0 eq) was dissolved in anhydrous methylene chloride (15 mL), the reaction solution was cooled to 0°C, trifluoroacetic acid (1.5 mL, 46.5 eq) was added dropwise, and the reaction solution was heated to room temperature and stirred for 2 hours. The reaction solution was concentrated to dryness to obtain the crude product of compound **LY-37e** (201 mg, yield: 99%) to be directly used for the next reaction.

LCMS: m/z = 468.3 $[M+H]^+$; Rt = 0.771 min.

Step 4: Preparation of compound **LY-37f**

The compound **LY-37e** (202 mg, 1.0 eq) and propionaldehyde (312 μL, 10.0 eq) were dissolved in tetrahydrofuran (10 mL) and methanol (2.5 mL) in the nitrogen atmosphere, the reaction solution was cooled to 0°C, sodium cyanoborohydride (136 mg, 5.0 eq) was added, and the reaction solution was warmed to room temperature and stirred for reaction for 16 hours. The reaction solution was concentrated to dryness, and the residue was purified by silica gel column chromatography (eluents: dichloromethane/methanol = 20:1) to obtain compound LY-37f (190 mg, yield: 80%).

LCMS: m/z = 552.4 $[M+H]^+$; Rt = 0.830 min.

Step 5: Preparation of compound **LY-37i**

5-bromo-2-(methylthio)pyrimidine (5g, 1.0 eq) and methyl hex-5-ynoate (3.26 mL, 1.0 eq) were dissolved in anhydrous DMF (80 mL) under the nitrogen atmosphere, cuprous iodide (465 mg, 0.1 eq), triethylamine (1.5 mL, 1.0 eq) and catalyst dichlorobis(triphenylphosphine)palladium (1.8g, 0.1 eq) were added, and the reaction solution was heated to 95°C and stirred for reaction for 12 hours. The reaction solution was concentrated, the residue was purified by silica gel column chromatography (eluents: petroleum ether/ethyl acetate = 5:1), to obtain the product methyl 6-(2-(methylthio)pyrimidine-5-yl)hex-5-ynoate (**LY-37i**) (3.3g, yield: 54%) as a yellow oily substance.

LCMS: m/z = 251.1 $[M+H]^+$; Rt = 2.072 min.

Step 6: Preparation of compound **LY-37j**

The compound **LY-37i** (1.0g, 1.0 eq) was dissolved in tetrahydrofuran (20 mL) and water (20 mL), lithium hydroxide monohydrate (1.2g, 7.0 eq) was added, and the reaction solution was stirred at 20°C for reaction for 12 hours. The reaction solution was diluted with pure water (40 mL), neutralized with 1M hydrochloric acid until pH reached about 3, and extracted with dichloromethane (60 mL) three times, organic phases were combined, and dried and concentrated to obtain a light yellow solid compound 6-(2-(methylthio)pyrimidin-5-yl)hex-5-ynoic acid (**LY-37j**) (930 mg, yield: 98%).

LCMS: m/z = 237.1 $[M+H]^+$; Rt = 1.455 min.

Step 7: Preparation of compound **LY-37k**

The compound $N^2$-(((9H-fluoren-9-yl)methoxy)carbonyl)-L-valeryl)-$N^6$, $N^6$-dipropyl-L-lysine (**LY-37f**) (19.01 mg, 1.0 eq) and compound **PY-16** (15 mg, 1.0 eq) were dissolved in anhydrous DMF (2 mL), HATU (19.65 mg, 1.5 eq) and DIPEA (17.1 μL, 3.0 eq) were added at 0°C, the reaction solution was warmed to room temperature and stirred for reaction for 16 hours. The reaction solution was filtered, and the filtrate was purified by preparative high performance liquid chromatograph (preparative chromatograph manufacturer: ISCO, model: ISCO-R1, column: Synergi Max-RP 280 × 30 mm × 10 μm, mobile phase: water (0.225% HCOOH)-acetonitrile, an elution ratio of water: 30% to 60%), to obtain compound LY-37k (12 mg, yield: 36%) as white solid powder.

LCMS: m/z = 969.5 $[M+H]^+$; Rt = 2.026 min.

Step 8: Preparation of compound **LY-37l**

The compound **LY-37k** (12 mg, 1.0 eq) was dissolved in DMF (1 mL), diethylamine (12.7 μL, 10.0 eq) was added, and the reaction solution was stirred for reaction for 3 hours at room temperature. The reaction solution was concentrated to dryness, to obtain the crude product **LY-37l** (10 mg, no yield was calculated for the crude product).
LCMS: m/z = 747.5 $[M+H]^+$; Rt = 0.633 min.

Step 9: Preparation of compound **LY-37m**

The compounds **LY-37l** (10 mg, 1.0 eq) and **LY-37j** (3.16 mg, 1.0 eq) were dissolved in anhydrous DMF (1 mL), HATU (6.11 mg, 1.2 eq) and DIPEA (6.6 μL, 3.0 eq) were added at 0°C, and the reaction solution was warmed to room temperature and stirred for reaction for 2 hours. The reaction solution was filtered, and the filtrate was purified by preparative high performance liquid chromatograph (preparative chromatograph manufacturer: ISCO, model: ISCO-R1, column: Synergi Max-RP 280 × 30 mm × 10 μm, mobile phase: water (0.225% HCOOH)-acetonitrile, an elution ratio of water: 21% to 51%), to obtain a white solid product LY-37m (5 mg, yield: 38%).
LCMS: m/z = 965.6 $[M+H]^+$; Rt = 1.795 min.

Step 10: Preparation of compound **LY-37A**

The compound **LY-37m** (4 mg, 1.0 eq) was dissolved in tetrahydrofuran (2 mL) and water (2 mL), Oxone (23 mg, 10 eq) was added, and the reaction solution was stirred at room temperature for reaction for 16 hours. The reaction solution was quenched with sodium sulfite solution, and concentrated under reduced pressure to remove tetrahydrofuran, the residue was diluted with a small amount of pure water, the resulting solution was filtered, and the filtrate was purified by preparative high performance liquid chromatograph (preparative chromatograph manufacturer: ISCO, model: ISCO-R1, column: Synergi Max-RP 280 × 30 mm × 10 μm, mobile phase: water (0.225% HCOOH),-acetonitrile, an elution ratio of water: 12% to 42%, to obtain a white solid compound **LY-37A** (0.24 mg, yield: 5.8%).
LCMS: m/z = 997.5 $[M+H]^+$; Rt = 1.544 min.

Example 48: Preparation of compound **LY-37B**

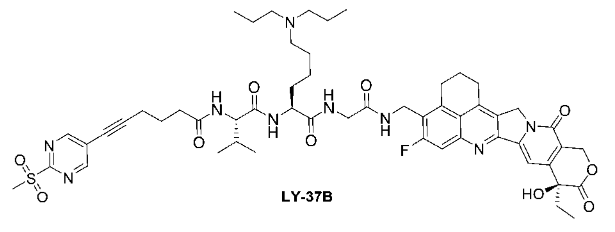

LY-37B

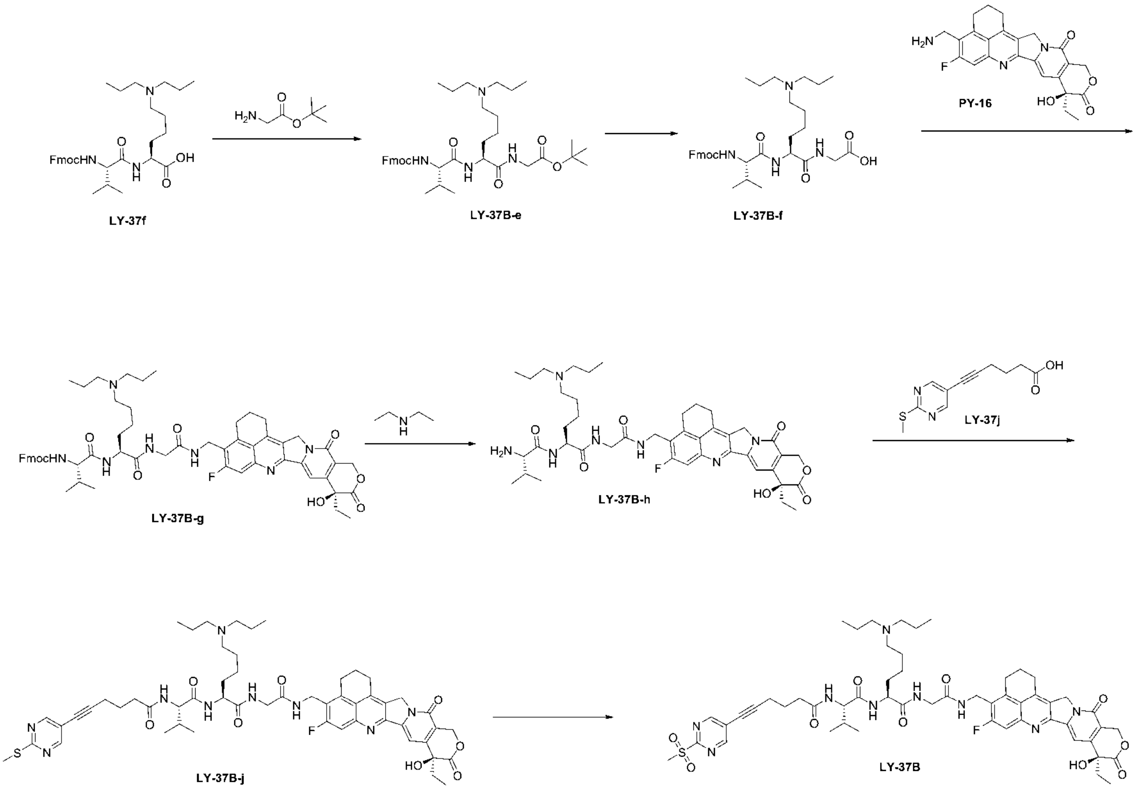

LY-37f, LY-37B-e, LY-37B-f, PY-16, LY-37B-g, LY-37B-h, LY-37j, LY-37B-j, LY-37B

Step 1: Preparation of compound **LY-37B-e**

HATU (141 mg, 370 μmol, 1.2 eq) and DIEA (119 mg, 924 μmol, 3.0 eq) were added to the solution of compound LY-37f (170 mg, 308 μmol, 1.0 eq) and tert-butyl glycine (48.5 mg, 370 μmol, 1.2 eq) in DMF (5 mL) at 0°C. After addition, the reaction solution reacted at room temperature for 4 hours. The reaction solution was diluted with ethyl acetate and added into cold water, the resulting solution was stirred for 3 minutes, the aqueous phase was extracted with ethyl acetate twice, the organic phases were combined, washed with water twice, then washed with brine once, dried with anhydrous sodium sulfate, and filtered, and the filtrate was concentrated under reduced pressure to obtain yellow solid compound LY-37B-e (206 mg, no yield was calculated for the crude product).

LCMS: m/z = 665.5 $[M+H]^+$; Rt = 2.246 min.

Step 2: Preparation of compound **LY-37B-f**

TFA (1 mL) was added to the solution of compound LY-37B-e (206 mg, 310 μmol, 1.0 eq) in DCM (10 mL); and after addition, the reaction solution reacted at room temperature for 3 hours, the reaction solution was concentrated under reduced pressure to obtain a crude product, and the crude product was purified by silica gel column chromatography (eluents: dichloromethane/methanol = 1:20-1:5) to obtain compound LY-37B-f (174 mg, yield: 85%, purity: 93%).

LCMS: m/z = 609.4 $[M+H]^+$; Rt = 1.803 min.

Step 3: Preparation of compound **LY-37B-g**

HATU (42 mg, 110 μmol, 1.2 eq) and DIEA (36 mg, 275 μmol, 3.0 eq) were added to the solution of compounds LY-37B-f (60 mg, 91.66 μmol, 1.0 eq) and PY-16 (40 mg, 91.66 μmol, 1.0 eq) in DMF (2 mL) at 0°C. After addition, the reaction solution reacted at room temperature for 3 hours. The reaction solution was added into water and extracted with

ethyl acetate 3 times, the organic phases were washed with water twice, then washed with brine once, dried with anhydrous sodium sulfate, and filtered, and the filtrate was concentrated under reduced pressure to obtain yellow solid compound LY-37B-g (62 mg, crude product).

LCMS: m/z = 1026.5 $[M+H]^+$/513.8$[1/2M+1]^+$; Rt = 1.020 min.

Step 4: Preparation of compound **LY-37B-h**

Diethylamine (18 mg, 242 μmol, 5.0 eq) was added to the solution of compound LY-37B-g (62 mg, 48.33 μmol, 1.0 eq, crude product) in DMF (2 mL); and after addition, the reaction solution reacted at room temperature for 2 hours, and concentrated under reduced pressure to obtain the yellow solid compound LY-37B-h (38 mg, crude product).

LCMS: m/z =804.5 $[M+H]^+$; Rt = 1.083 min.

Step 5: Preparation of compound **LY-37B-j**

HATU (20.9 mg, 55 μmol, 1.3 eq) and DIPEA (27 mg, 211 μmol, 5.0 eq) were added to the solution of compounds LY-37j (10 mg, 42.3 μmol, 1.0 eq) and LY-37B-h (34 mg, 42.3 μmol, 1.0 eq) in anhydrous DMF (4 mL) at 0°C. After addition, the reaction solution was warmed to room temperature and stirred for reaction for 12 hours. The reaction solution was concentrated and purified by preparative high performance liquid chromatograph (preparative chromatograph manufacturer: ISCO, model: ISCO-R1, column: YMC-Triart Prep C18 250 × 50 mm × 7 μm, mobile phase: water (0.225% HCOOH)-acetonitrile, an elution ratio of water: 21% to 51%, to obtain a white solid compound LY-37B-j (13 mg, yield: 27%).

LCMS: m/z = 1022.4 $[M+H]^+$/511.8$[1/2+1]^+$; Rt = 1.71 min.

Step 6: Preparation of compound **LY-37B**

Oxone (12 mg, 19.56 μmol, 10.0 eq) was added to a solution of compound LY-37B-j (2 mg, 1.96 μmol, 1 eq) in THF (1 mL)/$H_2O$ (1 mL). After addition, the reaction solution reacted at 25°C for 16 hours. After the reaction solution was filtered, the filtrate was purified by preparative high performance liquid chromatograph (preparative chromatograph manufacturer: ISCO, model: ISCO-R4, column: Welch Xtimate C18 150 × 21.2 mm × 5 μm, mobile phase: water (10 mM $NH_4HCO_3$)-acetonitrile, an elution ratio of water: 21% to 51%) and lyophilized, to obtain white solid compound LY-37B (1 mg, yield: 48%).

LCMS: m/z = 1054.4 $[M+H]^+$; Rt = 1.47 min.

Example 49: Preparation of compound **LY-39A**

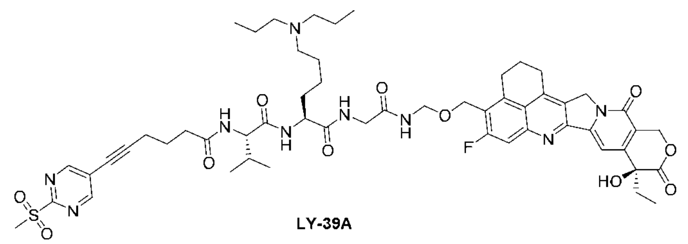

LY-39A

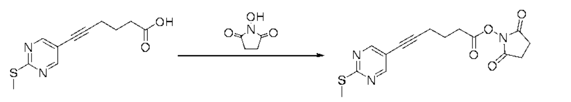

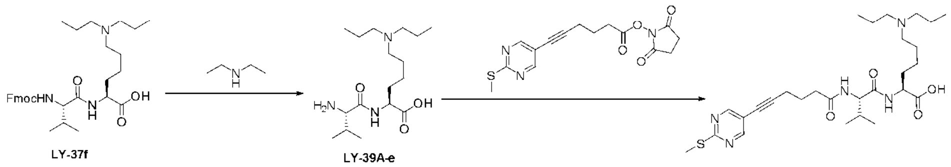

LY-37f LY-39A-e LY-39A-f

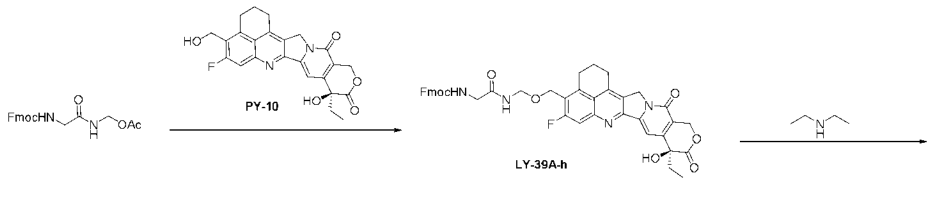

PY-10 LY-39A-h

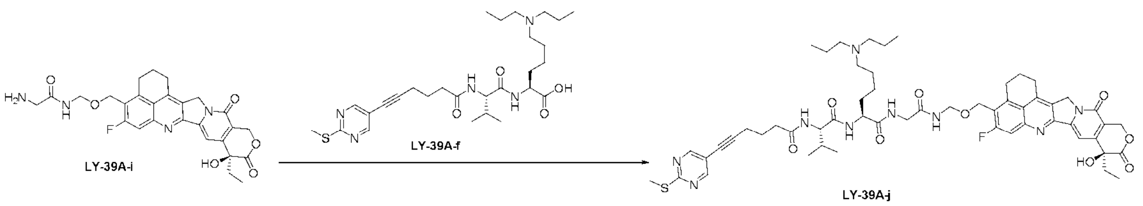

LY-39A-i LY-39A-f LY-39A-j

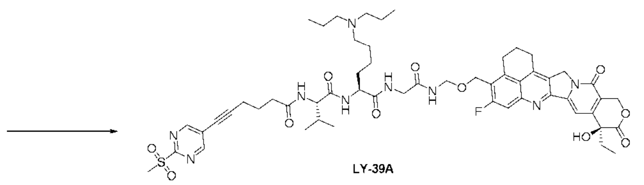

LY-39A

Step 1: Preparation of compound 2,5-dioxopyrrolidin-1-yl6-(2-(methylthio)pyrimidin-5-yl)hex-5-ynoate

The compound 6-(2-(methylthio)pyrimidin-5-yl)hex-5-ynoic acid (300 mg, 1.0 eq) and NHS (183 mg, 1.2 eq) were dissolved in DMF (10 mL) in the nitrogen atmosphere, the resulting solution was cooled to 0°C, and after DIC (401 μL, 2.0 eq) was added, the reaction solution was stirred at room temperature for reaction for 16 hours. The reaction solution was diluted with dichloromethane (25 mL) and water (20 mL), stirred and then allowed to stand to stratify, the organic phases were collected, the aqueous phase was extracted twice with dichloromethane (20 mL), the organic phases were combined, washed with saturated brine, dried and then concentrated, to obtain a crude product of compound 2,5-dioxopyrrolidin-1-yl6-(2-(methylthio)pyrimidin-5-yl)hex-5-ynoate (520 mg) as a pale yellow solid to be directly used for the next reaction.

LCMS: m/z = 334.0 $[M+H]^+$; Rt = 0.659 min.

Step 2: Preparation of compound $N^2$-(L-valine)-$N^6$,$N^6$-dipropyl-L-lysine (**LY-39A-e**)

The compound **LY-37f** (250 mg, 1.0 eq) was dissolved in anhydrous DMF (5 mL), diethylamine (467 μL, 10.0 eq) was added, and the reaction solution was stirred at room temperature for reaction for 3 hours. The reaction solution was concentrated to obtain the crude product of compound LY-39A-e (150 mg) to be directly used for the next reaction without purification.

LCMS: m/z = 330.2 $[M+H]^+$; Rt = 0.473 min.

Step 3: Preparation of compound **LY-39A-f**

Compound **LY-39A-e** (135 mg, 1.0 eq) and compound 2,5-dioxopyrrolidin-1-yl6-(2-(methylthio)pyrimidin-5-yl) hex-5-ynoate (164 mg, 1.2 eq) were mixed in anhydrous DMF (2 mL), DIPEA (237 μL, 3.5 eq) was added at room temperature and the reaction solution was stirred for reaction for 12 hours. After the reaction solution was filtered, the filtrate was purified by preparative high performance liquid chromatograph (preparative chromatograph manufacturer: ISCO, model: ISCO-R1, column: Synergi Max-RP 280 × 30 mm × 10 μm, mobile phase: water (0.225% HCOOH)-acetonitrile, an elution ratio of water: 12% to 42%), to obtain compound **LY-39A-f** (37 mg, yield: 16.5%) as a white solid.
LCMS: m/z = 548.4 $[M+H]^+$; Rt = 1.497 min.

Step 4: Preparation of compound **LY-39A-h**

Compound (2-((((9H-fluoren-9-yl)methoxy)carbonyl)amino)acetamide)methyl acetate (Chengdu Zen Sunda) (92.85 mg, 5.5 eq) and compound **PY-10** (20 mg, 1.0 eq) were mixed in anhydrous tetrahydrofuran (3 mL) in the nitrogen atmosphere, p-toluenesulfonic acid (2.37 mg, 0.3 eq) was added, and the reaction solution was stirred at room temperature for reaction for 16 hours. Compound (2-((((9H-fluoren-9-yl)methoxy)carbonyl)amino)acetamide)methyl acetate (92.85 mg, 5.5 eq) was added again, and the reaction solution was further stirred for reaction for 16 hours. The reaction solution was filtered and then concentrated, and the residue was purified by preparative high performance liquid chromatograph (preparative chromatograph manufacturer: ISCO, model: ISCO-R1, column: YMC-Triart Prep C18 250 × 50 mm × 7 μm, mobile phase: water (0.225% HCOOH)-acetonitrile, an elution ratio of water: 39% to 69%), to obtain compound LY-39A-h (22 mg, yield: 64.5%).
LCMS: m/z = 745.3 $[M+H]^+$; Rt = 2.111 min.

Step 5: Preparation of compound **LY-39A-i**

The compound **LY-39A-h** (22 mg, 1.0 eq) was dissolved in anhydrous DMF (1 mL), diethylamine (30.43 μL, 10.0 eq) was added, and the reaction solution was stirred at room temperature for reaction for 3 hours. The reaction solution was concentrated to obtain the crude product of compound LY-39A-i (15 mg) to be directly used for the next reaction without purification.
LCMS: m/z = 523.2 $[M+H]^+$; Rt = 0.598 min.

Step 6: Preparation of compound **LY-39A-j**

Compounds **LY-39A-i** (10 mg, 1.0 eq) and **LY-39A-f** (10.48 mg, 1.0 eq) were mixed in anhydrous DMF (2 mL), HATU (9.5 mg, 1.3 eq) and DIEA (8.7 mg, 3.5 eq) were added, and after addition, the reaction solution was stirred at room temperature for reaction for 1 hour. The reaction solution was directly used for the next step.
LCMS: m/z = 1052.5 $[M+H]^+$; Rt = 1.776 min.

Step 7: Preparation of compound **LY-39A**

Tetrahydrofuran (1 mL), water (1 mL) and DMF (4 mL) were added to the reaction solution containing compound **LY-39A-j** (20.14 mg, 1.0 eq), Oxone (106 mg, 33 eq) was added at room temperature, and the reaction solution was stirred at room temperature for reaction for 16 hours. The reaction solution was filtered and concentrated, and the residue was purified by preparative high performance liquid chromatograph (preparative chromatograph manufacturer: ISCO, model: ISCO-R1, column: Synergi Max-RP 280 × 30 mm × 10 μm, mobile phase: water (0.225% HCOOH)-acetonitrile, an elution ratio of water: 12% to 42%), to obtain compound **LY-39A** (3.13 mg, yield: 15.08%).
LCMS: m/z = 1084.5 $[M+H]^+$; Rt = 4.199 min.
$^1$H NMR (400 MHz, DMSO-$d_6$) δ 9.08 (s, 1H), 8.76 (t, *J* = 6.6 Hz, 1H), 8.33 (s, 1H), 8.23 (t, *J* = 5.8 Hz, 1H), 8.04 (d, *J* = 7.2 Hz, 2H), 7.95 (d, *J* = 8.5 Hz, 2H), 7.74 (d, *J* = 11.1 Hz, 2H), 7.32 (s, 2H), 6.54 (s, 2H), 5.44 (s, 2H), 5.26 (s, 2H), 4.69 (d, *J* = 5.6 Hz, 5H), 4.19 (dt, *J* = 27.8, 7.5 Hz, 5H), 3.85-3.68 (m, 6H), 3.19 (dd, *J* = 13.8, 6.6 Hz, 12H), 2.40-2.22 (m, 15H), 2.07 (t, *J* = 6.3 Hz, 4H), 2.00-1.73 (m, 12H), 1.68 (s, 2H), 1.56 (dd, *J* = 9.1, 4.9 Hz, 3H), 1.33 (h, *J* = 7.3 Hz, 10H), 1.24 (s, 2H), 0.91-0.75 (m, 9H).

Example 50: Preparation of compound **LY-39B**

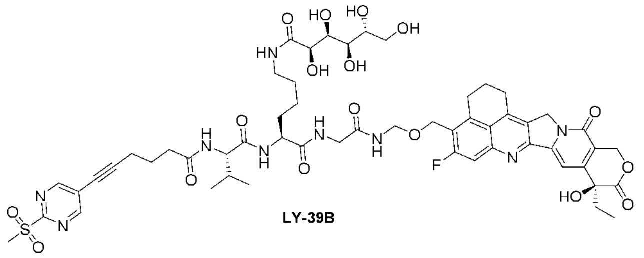

LY-39B

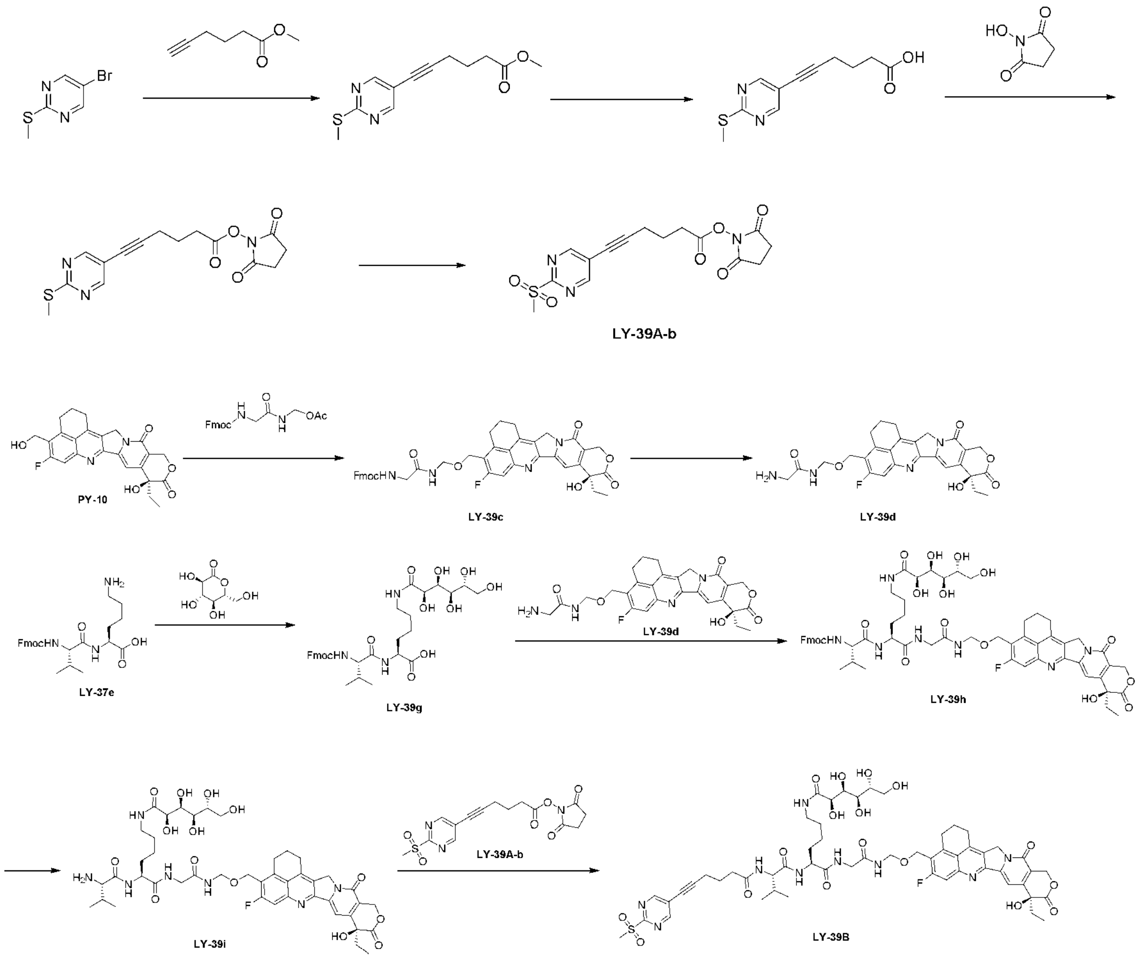

LY-39A-b

PY-10

LY-39c

LY-39d

LY-37e

LY-39g

LY-39d

LY-39h

LY-39i

LY-39A-b

LY-39B

Step 1: Preparation of methyl 6-(2-(methylthio)pyrimidin-5-yl)hex-5-ynoate

Methyl5-hexynote (3.91 mL, 29.26 mmol, 1.2 eq), triethylamine (3.39 mL, 24.38 mmol, 1 eq), cuprous iodide (664.35 mg, 2.44 mmol, 0.1 eq), and bis(triphenylphosphine)dichloropalladium (2.6g, 2.44 mmol, 0.1 eq) were added to the solution of 5-bromo-2-(methylthio)pyrimidine (5g, 24.38 mmol, 1 eq) in N,N-dimethylformamide (60 mL) in the nitrogen atmosphere, the reaction solution reacted at 95°C for 12 hours, and the reaction was monitored by the LCMS until the raw materials completed reaction. Ethyl acetate of an equal volume was added for dissolution, the mixture was filtered, diluted with water (100 mL), and extracted with ethyl acetate (100 mL × 3), the organic phases were combined, washed with water (100 mL × 2) and saturated brine (100 mL × 1), then dried with anhydrous sodium sulfate, filtered, and concentrated, and the residue was purified by silica gel column chromatography (eluents: petroleum ether/dichloromethane = 5:1) to obtain yellow oily methyl 6-(2-(methylthio)pyrimidin-5-yl)hex-5-ynoate (3.43g, yield = 56.20%).
LCMS (ESI): m/z, 251[M+H]$^+$.

Step 2: Preparation 6-(2-(methylthio)pyrimidin-5-yl)hex-5-ynoic acid

Lithium hydroxide monohydrate (4.02g, 95.64 mmol, 7 eq) was added to a solution of methyl 6-(2-(methylthio)pyrimidin-5-yl)hex-5-ynoate (3.4g, 24.38 mmol, 1 eq) in tetrahydrofuran (60 mL) and water (60 mL) and the reaction

solution reacted at 25°C for 12 hours. Water of an equal volume was added for dissolution, the mixture was filtered, adjusted with 1N hydrochloric acid until pH reached 3, and extracted with dichloromethane (200 mL × 3), and the organic phases were combined, washed with water (200 mL × 2) and saturated brine (100 mL × 1), then dried with anhydrous sodium sulfate, filtered, and concentrated under reduced pressure to obtain yellow oily 6-(2-(methylthio)pyrimidin-5-yl) hex-5-ynoic acid (2.76g, yield = 85.49%).

LCMS (ESI): m/z, 237[M+H]$^+$.

Step 3: Preparation of 2,5-dioxopyrrolidin-1-yl6-(2-(methylthio)pyrimidin-5-yl)hex-5-ynoate

1-hydroxypyrrolidine-2,5-dione (1.98g, 7.36 mmol, 1 eq) was added to a solution of 6-(2-(methylthio)pyrimidin-5-yl)hex-5-ynoic acid (1.69g, 14.72 mmol, 2 eq) in N,N-dimethylformamide (20 mL), N,N-diisopropylcarbodiimide (2.32 mL) was added at 0°C, and the reaction solution reacted at 25°C for 12 hours. Ethyl acetate of an equal volume was added for dissolution, the mixture was filtered, then diluted with 100 mL of water, and extracted with ethyl acetate (100 mL × 3), the organic phases were combined, washed with water (100 mL × 2) and saturated brine (100 mL × 1), then dried with anhydrous sodium sulfate, filtered, and concentrated, to obtain yellow oily 2,5-dioxopyrrolidin-1-yl6-(2-(methylthio) pyrimidin-5-yl)hex-5-ynoate (4.4g, yield = 163.59%).

LCMS (ESI): m/z, 366[M+H]$^+$.

Step 4: Preparation of 2,5-dioxopyrrolidin-1-yl6-(2-(methylsulfonyl)pyrimidin-5-yl)hex-5-ynoate

Potassium peroxymonosulfate sulfate (22.20g, 131.99 mmol, 10 eq) was added to a solution of 2,5-dioxopyrrolidin-1-yl6-(2-(methylthio)pyrimidin-5-yl)hex-5-ynoate (4.4g, 13.2 mmol, 2 eq) in tetrahydrofuran (70 mL) and water (70 mL) and the reaction solution reacted at 25°C for 12 hours. Ice water of an equal volume was added, the mixture was stirred for 2 minutes and extracted with dichloromethane (200 mL × 3), and the organic phases were combined, washed with water (200 mL × 2) and saturated brine (200 mL), then dried with anhydrous sodium sulfate, filtered, and concentrated to obtain yellow oily 2,5-dioxopyrrolidin-1-yl6-(2-(methylsulfonyl)pyrimidin-5-yl)hex-5-ynoate (2.58g, yield = 51.78%).

LCMS (ESI): m/z, 366[M+H]$^+$.

$^1$H NMR (400 MHz, DMSO-d6) δ 9.14 (s, 2H), 3.41 (s, 3H), 2.90 (t, *J* = 7.2 Hz, 2H), 2.83 (s, 4H), 2.70 (t, *J* = 7.2 Hz, 2H), 1.96 (p, *J* = 7.2 Hz, 2H).

Step 5: Preparation of compound **LY-39c**

The compound (2-((((9H-fluoren-9-yl)methoxy)carbonyl)amino)acetamide)methyl acetate (50.64 mg, 137.48 μmol, 2 eq) was added to the solution of compound **PY-10** (30 mg, 68.74 μmol, 1 eq) in tetrahydrofuran (2 mL), and then a catalytic amount of p-toluenesulfonic acid (3.55 mg, 3.32 μL, 20.62 μmol, 0.3 eq) was added. The reaction solution was stirred for 12 hours at 20°C. The reaction solution was isolated and purified by preparative high performance liquid chromatograph (preparative chromatograph manufacturer: ISCO, model: ISCO-4, column: YMC-Triart Prep C18 250 × 30 mm × 10 μm, mobile phase: water (0.225% FA)-ACN, an elution ratio of water: 39% to 69%) and lyophilized, to obtain a white solid product **LY-39c** (13 mg, yield: 25.39%).

LCMS (ESI): m/z, 745.3 [M+H]$^+$.

Step 6: Preparation of compound **LY-39d**

The compound **LY-39c** (13 mg, 15.15 μmol, 1 eq) was dissolved in DMF (1 mL), diethylamine (7.66 mg, 10.79 μL, 104.73 μmol, 6 eq) was added to the solution, and the solution was stirred at 20°C for 3 hours in the nitrogen atmosphere. The reaction solution was concentrated under reduced pressure to obtain the product **LY-39d** (8 mg, yield: 87.71%) to be directly used for the next step.

LCMS (ESI): m/z, 1045.3 [2M+H]$^+$.

Step 7: Preparation of compound **LY-39g**

Compound **LY-37e** (400 mg, 855.49 μmol, 1 eq) was dissolved in MeOH (10 mL), TEA (432.83 mg, 4.28 mmol, 5 eq) and (3R,4S,5S,6R)-3,4,5-trihydroxy-6-(hydroxymethyl)tetrahydro-2H-pyrano-2-one (304.79 mg, 507.99 μL, 1.71 mmol, 2 eq) were added and the reaction solution was stirred at 70°C for 12 hours. The reaction solution was purified by preparative high performance liquid chromatograph (preparative chromatograph manufacturer: ISCO, model: ISCO-6, column: Welch Xtimate C18 250 × 50 mm × 10 μm, mobile phase: water (0.225% FA)-ACN, an elution ratio of water: 21% to 41%) and lyophilized, to obtain a white solid product **LY-39g** (18 mg, yield: 3.26%).

LCMS (ESI): m/z, 646.3.

Step 8: Preparation of compound **LY-39h**

HOBt (3.77 mg, 2.51 μL, 27.88 μmol, 1.5 eq) and compound **LY-39d** (9.71 mg, 18.58 μmol, 1 eq) were added to the solution of compound **LY-39g** (12 mg, 18.58 μmol, 1 eq) in DMF (1 mL), and then EDCI (5.34 mg, 27.88 μmol, 1.5 eq) was further added. The reaction solution was stirred for 1 hour at 20°C. The reaction solution was directly purified by preparative high performance liquid chromatograph (preparative chromatograph manufacturer: ISCO, model: ISCO-6, column: YMC-Triart Prep C18 250 × 30 mm × 10 μm, mobile phase: water (0.225% FA)-ACN, an elution ratio of water: 21% to 51%) and lyophilized, to obtain a white solid **LY-39h** (10 mg, yield: 46.78%).

LCMS (ESI): m/z, 1150.6[M+H]$^+$.

Step 9: Preparation of compound **LY-39i**

$Et_2NH$ (3.18 mg, 4.48 μL, 43.47 μmol, 5 eq) was added to the solution of compound **LY-39h** (10 mg, 8.69 μmol, 1 eq) in DMF (2 mL) and the reaction solution was stirred at 20°C for 1 hour. The reaction solution was concentrated under reduced pressure to obtain the product LY-39i (8 mg, yield: 99.16%).

LCMS (ESI): m/z, 928.4[M+H]$^+$.

Step 10: Preparation of compound **LY-39B**

Compounds **LY-39A-b** (5 mg, 5.39 μmol, 1 eq) and DIPEA (696.41 μg, 5.39 μmol, 1 eq) were added to the solution of compound **LY-39i** (5 mg, 5.39 μmol, 1 eq) in DMF (1 mL), and the reaction solution was stirred at 20°C for 2 hours. The reaction solution was filtered and then purified by preparative high performance liquid chromatograph (preparative chromatograph manufacturer: Oriendo, model: BRIX-2860, column: Synergi Max-RP 280 × 30 mm × 10 μm, mobile phase: water (0.225% HCOOH)-acetonitrile, an elution ratio of water: 12% to 42%) and lyophilized, to obtain a white solid compound **LY-39B** (840 μg, yield: 13.23%).

LCMS (ESI): m/z, 1178.5[M+H]$^+$.

Example 51: Preparation of compound **LY-41**

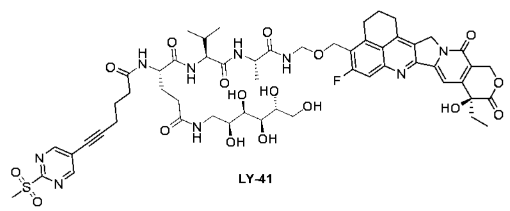

LY-41

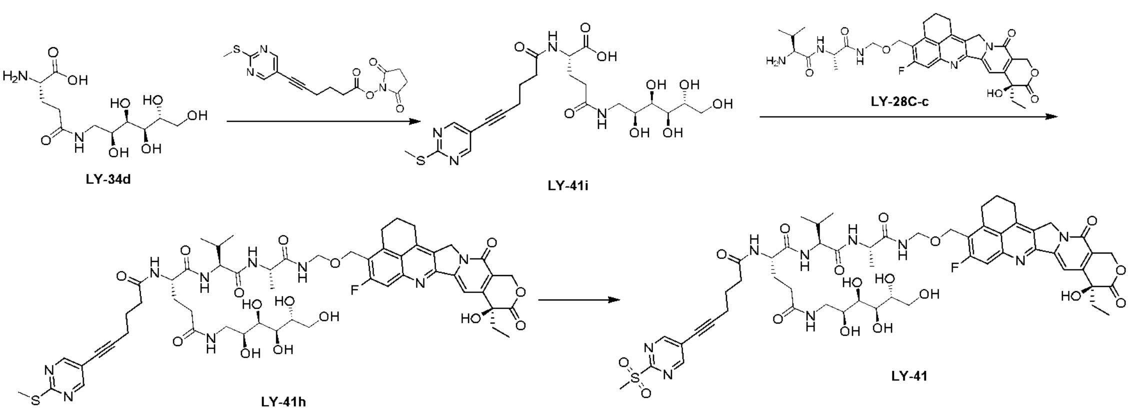


Step 1: Preparation of compound **LY-41i**

Compound **LY-34d** (30 mg, 96.68 μmol, 1 eq) was dissolved in DMF (0.5 mL), and DIPEA (24.99 mg, 31.96 μL, 193.36 μmol, 2 eq) and compound 2,5-dioxopyrrolidin-1-yl6-(2-(methylthio)pyrimidin-5-yl)hex-5-ynoate (32.23 mg, 96.68 μmol, 1 eq) were added to the resulting solution. The reaction solution was stirred for 2 hours at 20°C. The reaction solution

was purified by preparative high performance liquid chromatograph (preparative chromatograph manufacturer: ISCO, model: ISCO-6, column: YMC-Triart Prep C18 250 × 50 mm × 7 μm, mobile phase: water (10 mM $NH_4HCO_3$)-ACN, an elution ratio of water: 1% to 24%) and lyophilized, to obtain a white solid LY-41i (30 mg, yield: 58.70%).

LCMS (ESI): m/z, 529.2[M+H]$^+$.

Step 2: Preparation of compound **LY-41h**

Compound **LY-28C-c** (6.01 mg, 9.46 μmol, 1 eq) was added to the solution of compound **LY-41i** (5 mg, 9.46 μmol, 1 eq) in DMF (0.5 mL), then HOBt (1.92 mg, 1.28 μL, 14.19 μmol, 1.5 eq) and EDCI (2.72 mg, 14.19 μmol, 1.5 eq) were sequentially added, and the reaction solution was stirred at 25°C for 1 hour in the nitrogen atmosphere. The reaction solution was purified by preparative high performance liquid chromatograph (preparative chromatograph manufacturer: Oriendo, model: BRIX-2860, column: Synergi Max-RP 280 × 30 mm × 10 μm, mobile phase: water (0.225% HCOOH)-acetonitrile, an elution ratio of water: 21% to 51%) and lyophilized, to obtain a white solid product LY-41h (3 mg, yield: 27.67%).

LCMS (ESI): m/z, 1146.3[M+H]$^+$.

Step 3: Preparation of compound **LY-41**

Oxone (5.87 mg, 34.90 μmol, 10 eq) was added to a solution of compound **LY-41h** (4 mg, 3.49 μmol, 1 eq) in THF (0.5 mL)/$H_2O$ (0.5 mL), and the reaction solution was stirred at 20°C for 12 hours. The reaction solution was filtered and then purified by preparative high performance liquid chromatograph (preparative chromatograph manufacturer: Oriendo, model: BRIX-2860, column: Synergi Max-RP 280 × 30 mm × 10 μm, mobile phase: water (0.225% HCOOH)-acetonitrile, an elution ratio of water: 12% to 42%) and lyophilized, to obtain a white solid compound **LY-41** (230 μg, yield: 5.59%, purity: 100%).

LCMS (ESI): m/z, 1200.4[M+Na]$^+$.

Example 52: Preparation of compound **LY-41B**

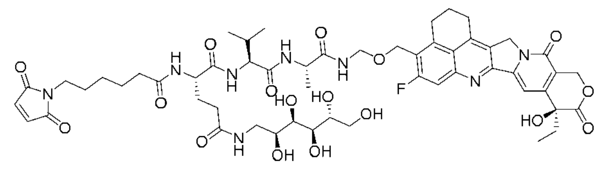

LY-41B

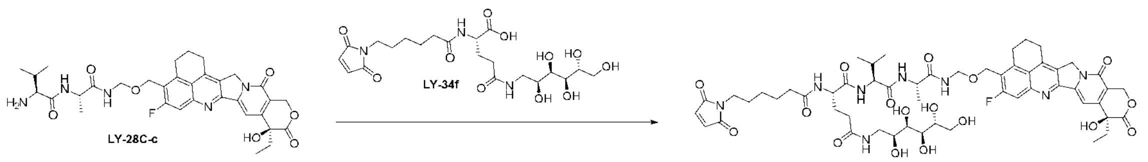

LY-28C-c

LY-34f

LY-41B

LY-34f (14.26 mg, 28.32 μmol, 1.5 eq) was added to the solution of compound LY-28C-c (12 mg, 18.88 μmol, 1 eq) in DMF (60 mL), then HOBT (3.83 mg, 2.55 μL, 28.32 μmol, 1.5 eq) and EDCI.HCl (5.43 mg, 6.19 μL, 28.32 μmol, 1.5 eq) were added, and the reaction solution was stirred at 10°C for 12 hours. The reaction solution was purified by reversed-phase preparative chromatograph (preparative chromatograph manufacturer: SHIMADZU, model: LC-20AP, column: Welch Xtimate C18 150 × 21.2 mm × 5 μm, mobile phase: water (0.225% HCOOH)-acetonitrile, an elution ratio of water: 14% to 42%) and lyophilized, to obtain a white solid product LY-41B (1.20g, yield: 55.58%).

LCMS (ESI): m/z, 1121.2[M+H]$^+$.

Example 53: Preparation of compound **LY-43C**

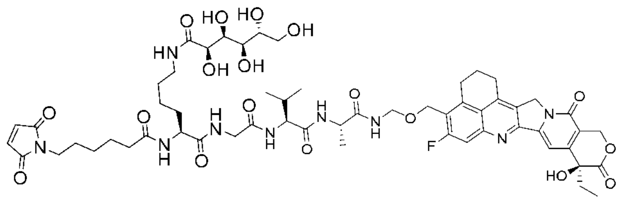

**LY-43C**

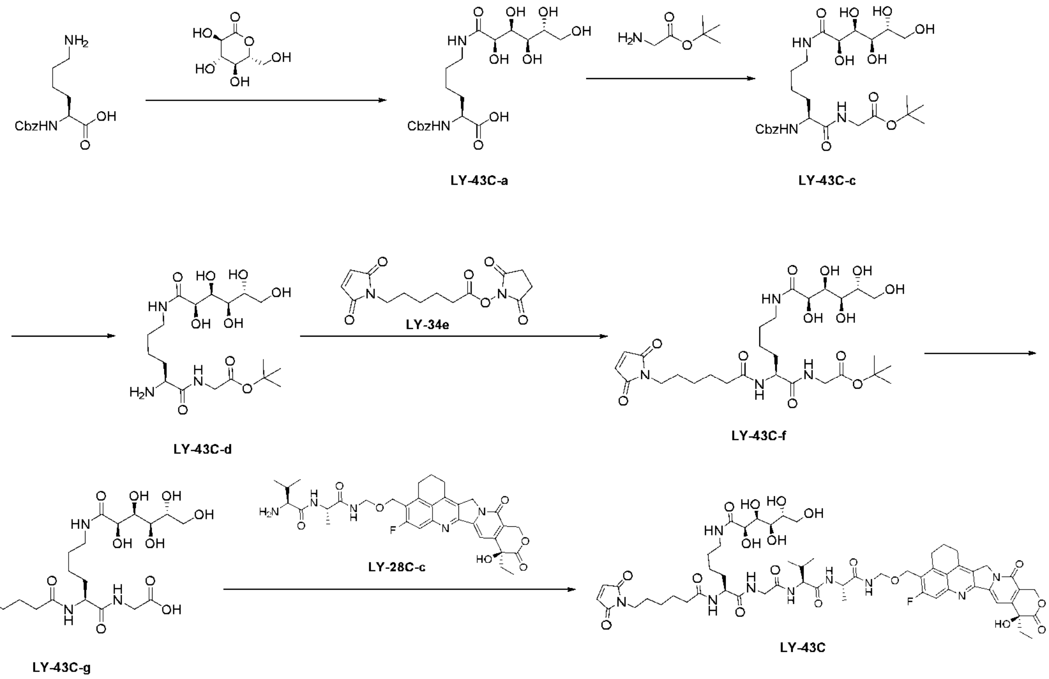


Step 1: Preparation of compound **LY-43C-a**

Gluconolactone (3.81g, 6.35 mL, 21.40 mmol, 2 eq) and triethylamine (5.41g, 7.44 mL, 53.51 mmol, 5 eq) were added to a solution of ((benzyloxy)carbonyl)-L-lysine (3g, 10.70 mmol, 1 eq) in methanol (100 mL), and after addition, the reaction solution was stirred at 70°C for 16 hours. The reaction solution was purified by reversed-phase C18 column chromatography (ISCO, 80g SepaFlash Silica Flash Column, elution gradient: 0%-45% water/acetonitrile, 50 mL/min) to obtain white solid compound **LY-43C-a** (3.84g, yield: 78%).

LCMS: m/z = 459.1 $[M+H]^+$; Rt = 1.43 min.

Step 2: Preparation of compound **LY-43C-c**

Tert-butyl glycinate (154.51 mg, 1.18 mmol, 1.2 eq), 1-hydroxybenzotriazole (198.94 mg, 1.47 mmol, 1.5 eq) and 1-(3-dimethylaminopropyl)-3-ethylcarbodiimide (282.24 mg, 1.47 mmol, 1.5 eq) were added to a solution of **LY-43C-a** (450 mg, 981.55 μmol, 1 eq) in N,N-dimethylformamide (10 mL) in the nitrogen atmosphere, and the reaction solution was stirred at room temperature for 2 hours. The reaction solution was purified by preparative high performance liquid chromatograph (preparative chromatograph manufacturer: SHIMADZU, model: LC-20AP, column: YMC-Triart Prep C18 250 × 30 mm × 10 μm, mobile phase: water (0.225% HCOOH)-acetonitrile, an elution ratio of acetonitrile: 12% to 45%), to obtain compound **LY-43C-c** (523 mg, yield = 93.2%).

LCMS (ESI): m/z, $572[M+H]^+$.

Step 3: Preparation of compound **LY-43C-d**

Palladium on carbon (100 mg) was added to the solution of compound **LY-43C-c** (260 mg, 454.85 μmol) in methanol (8 mL) in the hydrogen atmosphere, and the reaction solution was stirred at room temperature for 2 hours, filtered with diatomite, washed with water and methanol (5 mL/5 mL) and concentrated to obtain the compound **LY-43C-d** (232 mg).

LCMS (ESI): m/z, $438[M+H]^+$.

Step 4: Preparation of compound **LY-43C-f**

N,N-diisopropylethylamine (262.92 μL, 1.59 mmol, 3 eq) was added to the solution of compounds **LY-43C-d** (232 mg, 530.3 μmol, 1 eq) and **LY-34e** (196.18 mg, 636.36 μmol, 1.2 eq) in N,N-dimethylformamide (6 mL). The reaction solution was stirred at room temperature for 2 hours. The reaction solution was purified by preparative high performance liquid chromatograph (preparative chromatograph manufacturer: Oriendo and SHIMADZU, model: LC-20 AP, column: GS-120-10-C18AP, mobile phase: water (0.225% HCOOH)-acetonitrile, an elution ratio of acetonitrile: 12% to 42%), to obtain compound LY-**43C-f** (165 mg, yield = 49.3%).
LCMS (ESI): m/z, 631[M+H]$^+$.

Step 5: Preparation of compound **LY-43C-g**

Trifluoroacetic acid (1 mL) was added to the solution of compound **LY-43C-f** (165 mg, 261.62 μmol, 1 eq) in dichloromethane (5 mL) under the ice water bath, the reaction solution was stirred for 10 minutes and then warmed to room temperature and stirred for 2 hours, then trifluoroacetic acid (1 mL) was added, and the reaction solution was stirred for 3 hours at room temperature. The reaction solution was concentrated under reduced pressure to obtain the compound **LY-43C-g** (311.2 mg).
LCMS (ESI): m/z, 575[M+H]$^+$.

Step 6: Preparation of compound **LY-43C**

N,N-diisopropylethylamine (33.8 mL, 204.5 μmol, 10 eq) was added to the solution of compounds **LY-43C-g** (22 mg, 1.5 μmol) and **LY-28C-c** (13 mg, 20.45 μmol, 1 eq) in N,N-dimethylformamide (6 mL), then N,N,N',N'-tetramethyl-O-(7-azabenzotriazole-1-yl)hexafluorophosphate (9.33 mg, 24.54 μmol, 1.2 eq) was added, and then the reaction solution was stirred in the ice water bath for 5 minutes, then warmed to room temperature, and stirred for 2 hours. The reaction solution was purified by preparative high performance liquid chromatograph (preparative chromatograph manufacturer: Oriendo and SHIMADZU, model: LC-20 AP, column: YMC-Triart Prep C18 250 × 30 mm × 10 μm, mobile phase: water (0.225% HCOOH)-acetonitrile, an elution ratio of acetonitrile: 12% to 42%), to obtain compound **LY-43C** (0.56 mg, yield = 2.94%).
LCMS (ESI): m/z, 1192[M+H]$^+$.

Example 54: Preparation of compound **LY-43D**

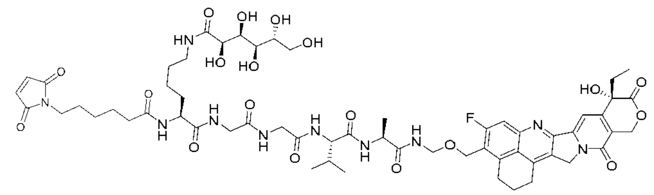

LY-43D

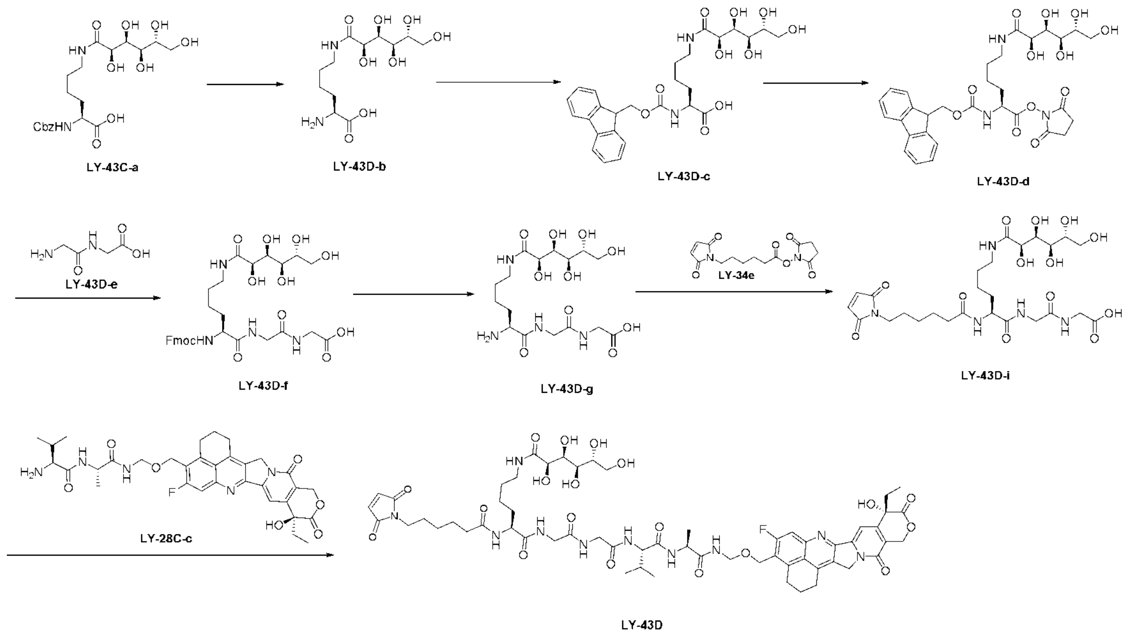


Step 1: Preparation of compound **LY-43D-b**

Pd/C (purity: 10%, 286 mg) was added to a solution of compound **LY-43C-a** (1.43g, 3.12 mmol, 1 eq) in methanol (25 mL) at room temperature, and the reaction solution was stirred at 20°C for 16 hours in the hydrogen atmosphere (15 Psi). After the reaction solution was filtered, the filtrate was concentrated under reduced pressure to obtain 320 mg of product. The filter cake was added to water (20 mL), the resulting solution was stirred for 0.5 hours at room temperature and then filtered, and the filtrate was lyophilized to obtain white solid product **LY-43D-b** (1.12g).

Step 2: Preparation of compound **LY-43D-c**

Compound **LY-43D-b** (1.44g, crude product) and 9-fluorenylmethyl-N-succinimidyl carbonate (1.65g, 4.88 mmol, 1.1 eq) were added to tetrahydrofuran (30 mL) at room temperature. Sodium bicarbonate (1.12g, 518.05 μL, 13.32 mmol, 3 eq) was dissolved in water (10 mL), the solution was added to the above solution at room temperature, and the resulting solution was stirred at 15°C for 16 hours. The reaction solution was concentrated under reduced pressure, and the residue was purified by preparative high performance liquid chromatograph (preparative chromatograph manufacturer: Luna, model: Lab311-ISCO-R4, column: Phenomenex Luna C18 250 × 50 mm × 10 μm, mobile phase: water (0.225% HCOOH)-acetonitrile, an elution ratio of water: 21% to 51%) and lyophilized, to obtain compound **LY-43D-c** (1.60g, 2.93 mmol, two-step yield: 94%).

LCMS (ESI): m/z, 547.2 $[M+H]^+$.

Step 3: Preparation of compound **LY-43D-d**

Compound **LY-43D-c** (470 mg, 860 μmol, 1 eq), diisopropylcarbodiimide (130.22 mg, 160.77 μL, 1.03 mmol, 1.2 eq), and N-hydroxysuccinimide (118.76 mg, 74.23 μL, 1.03 mmol, 1.2 eq) were added to tetrahydrofuran (4 mL) and N,N-dimethylformamide (4 mL) at room temperature, and the resulting solution was stirred at 25°C for 3 hours. The reaction solution was directly used for the next step.

LCMS (ESI): m/z, 644.3 $[M+H]^+$.

Step 4: Preparation of compound **LY-43D-f**

Glycylglycine (113.61 mg, 860 μmol, 1 eq), triethylamine (174.03 mg, 239 μL, 1.72 mmol, 2 eq), and water (3 mL) were added to the reaction solution of **LY-43D-d** at room temperature. The reaction solution was stirred at 20°C for 16 hours. The reaction solution was concentrated under reduced pressure, and the residue was purified by preparative high performance liquid chromatograph (preparative chromatograph manufacturer: Luna, model: Lab311-ISCO-R4, column: Phenomenex Luna C18 250 × 50 mm × 10 μm, mobile phase: water (0.225% HCOOH)-acetonitrile, an elution ratio of water: 21% to 51%) and lyophilized, to obtain compound **LY-43D-f** (232 mg, 351 μmol, two-step yield: 41%).

LCMS (ESI): m/z, 661.2 [M+H]$^+$.

Step 5: Preparation of compound **LY-43D-g**

Compound **LY-43D-f** (40 mg, 60.54 μmol, 1 eq) and diethylamine (22.14 mg, 31.18 μL, 302.72 μmol, 5 eq) were added to N,N-dimethylformamide (1 mL) at room temperature. The reaction solution was stirred at 20°C for 2 hours. The reaction solution was concentrated under reduced pressure to obtain the compound **LY-43D-g** (33 mg).

LCMS (ESI): m/z, 439.2 [M+H]$^+$.

Step 6: Preparation of compound **LY-43D-i**

Compound **LY-43D-g** (33 mg), 6-(maleimino)succinimidyl ester **(LY-34e)** (23.20 mg, 75.27 μmol, 1 eq), and N,N-diisopropylethylamine (29.18 mg, 37.32 μL, 225.80 μmol, 3 eq) were added to N,N-dimethylformamide (1 mL) at room temperature. The reaction solution was stirred at 20°C for 16 hours. The reaction solution was purified by high performance liquid chromatograph (preparative chromatograph manufacturer: Luna, model: Lab311-ISCO-R4, column: Phenomenex Luna C18 250 × 50 mm × 10 μm, mobile phase: water (0.225% HCOOH)-acetonitrile, an elution ratio of water: 3% to 33%) and lyophilized, to obtain compound **LY-43D-i** (20 mg, 31.7 μmol, two-step yield: 52.3%).

LCMS (ESI): m/z, 632.2 [M+H]$^+$.

Step 7: Preparation of compound **LY-43D**

Compound **LY-43D-i** (6.9 mg, 10.9 μmol, 1.3 eq), compound **LY-28C-c** (8 mg, purity: 66.8%; 8.41 μmol, 1 eq), 2-(7-azabenzotriazole)-N,N,N',N'-tetramethyluronium hexafluorophosphate (4.48 mg, 11.77 μmol, 1.4 eq), and N,N-diisopropylethylamine (3.26 mg, 4.17 μL, 25.22 μmol, 3 eq) was added to N,N-dimethylformamide (1 mL) at room temperature and the reaction solution was stirred at 15°C for 2 hours. The reaction solution was purified by high performance liquid chromatograph (preparative chromatograph manufacturer: Luna, model: Lab311-ISCO-R4, column: Phenomenex Luna C18 250 × 50 mm × 10 μm, mobile phase: water (0.225% HCOOH)-acetonitrile, an elution ratio of water: 12% to 42%) and lyophilized, to obtain compound **LY-43D** (3.26 mg, 2.91 μmol, yield: 51%).

LCMS (ESI): m/z, 1271.6[M+Na]$^+$.

Example 55: Preparation of compound **LY-43E**

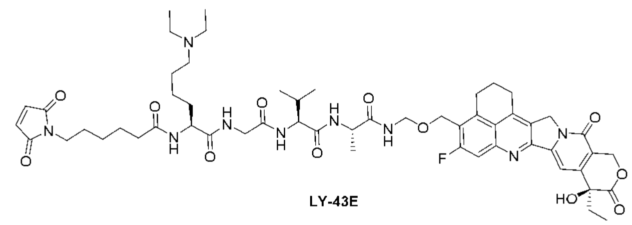

LY-43E

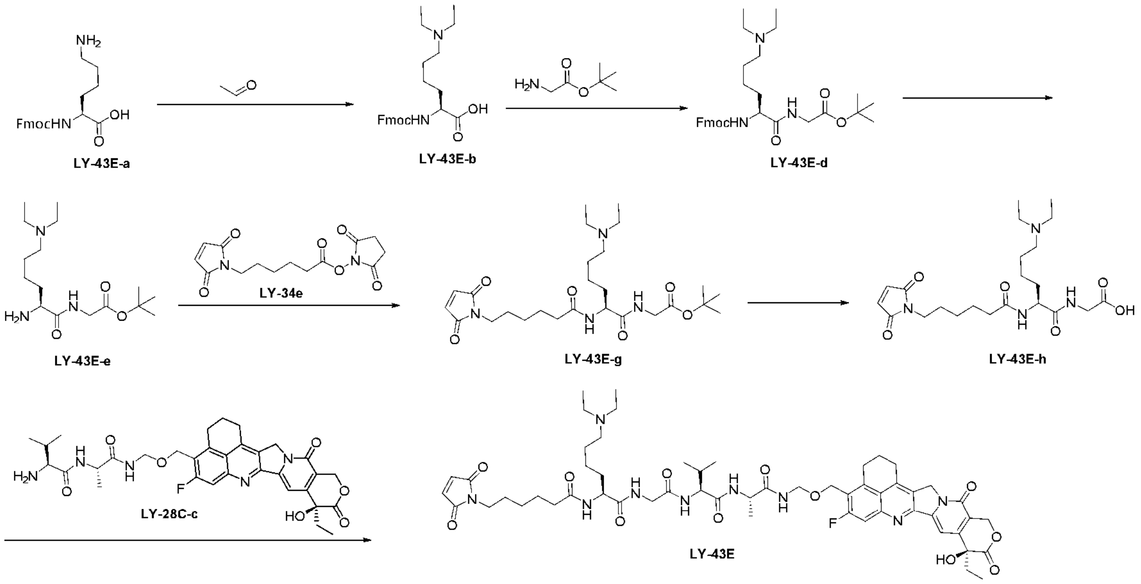

LY-43E-a LY-43E-b LY-43E-d LY-43E-e LY-34e LY-43E-g LY-43E-h LY-28C-c LY-43E

Step 1: Preparation of compound **LY-43E-b**

The compound **LY-43E-a** (4.0g, 1.0 eq) and acetaldehyde (6.09 mL, 10.0 eq) were dissolved in tetrahydrofuran (30 mL) and methanol (7.5 mL), sodium cyanoborohydride (3.41g, 5.0 eq) was added in the ice water bath, and then the reaction solution was naturally warmed to room temperature and stirred for reaction for 16 hours. The reaction solution was concentrated, water (30 mL) was added to the residue, the resulting solution was extracted twice with dichloromethane/methanol (10:1, 30 mL), the organic phases were combined and concentrated, and the residue was purified by silica gel column chromatography (eluents: dichloromethane/methanol = 5:1) to obtain compound **LY-43E-b** (1.9g, yield: 41.2%).
LCMS (ESI): m/z, 425.3 $[M+H]^+$.

Step 2: Preparation of compound **LY-43E-d**

Compound **LY-43E-b** (1.0g, 1.0 eq) and tert-butyl glycine (370.8 mg, 1.2 eq) were dissolved in anhydrous DMF (10 mL), HATU (1.07g, 1.2 eq) and DIPEA (1.17 mL, 3.0 eq) were added, and the reaction solution was stirred at room temperature for reaction for 1 hour. The reaction solution was concentrated, and the residue was purified by silica gel column chromatography (eluents: dichloromethane/methanol = 8:1) to obtain compound **LY-43E-d** (900 mg, yield: 71%).
LCMS (ESI): m/z, 538.3$[M+H]^+$.

Step 3: Preparation of compound **LY-43E-e**

Diethylamine (1.72 mL, 16.74 mmol, 10 eq) was added to the solution of compound **LY-43E-d** (900 mg, 1.67 mmol, 1 eq) in N,N-dimethylformamide (20 mL), and the reaction solution was stirred at room temperature for 2 hours. The reaction solution was concentrated under reduced pressure to obtain the crude product (840.8 mg).
LCMS (ESI): m/z, 316$[M+H]^+$.

Step 4: Preparation of compound **LY-43E-g**

N,N-diisopropylethylamine (829.89 μL, 5.02 mmol, 3 eq) was added to the solution of compounds **LY-43E-e** (840.8 mg, purity: 62.8%; 1.67 mmol, 1 eq) and **LY-34e** (619.23 mg, 2.01 mmol, 1.2 eq) in N,N-dimethylformamide (15 mL). The reaction solution was stirred at room temperature for 2 hours. The reaction solution was purified by preparative high

performance liquid chromatograph (preparative chromatograph manufacturer: SHIMADZU, model: LC-20AP, column: Synergi Max-RP 250 × 40 mm × 10 μm, mobile phase: water (0.225% HCOOH)-acetonitrile, an elution ratio of acetonitrile: 10% to 45%), to obtain compound **LY-43E-g** (234 mg, yield = 27.5%).

LCMS (ESI): m/z, 509[M+H]$^+$.

Step 5: Preparation of compound **LY-43E-h**

Trifluoroacetic acid (3 mL) was added to the solution of compound **LY-43E-g** (234 mg, 460.03 μmol, 1 eq) in dichloromethane (15 mL) in the ice water bath, the reaction solution was stirred for 5 minutes, then warmed to room temperature, and stirred for 4 hours, and the reaction solution was concentrated, and then concentrated with dichloromethane to obtain crude product **LY-43E-h** (308 mg).

LCMS (ESI): m/z, 453[M+H]$^+$.

Step 6: Preparation of compound **LY-43E**

1-hydroxybenzotriazole (5.06 mg, 37.46 μmol, 1.6 eq) and 1-(3-dimethylaminopropyl)-3-ethylcarbodiimide (6.73 mg, 35.12 μmol, 1.5 eq) were added to the solution of compounds **LY-28C-c** (21.2 mg, purity: 70.2%; 23.41 μmol, 1 eq) and **LY-43E-h** (18.7 mg, purity: 67.6%; 27.96 μmol, 1.2 eq) in N,N-dimethylformamide (2 mL), and the reaction solution was stirred at room temperature overnight. The reaction solution was purified by preparative high performance liquid chromatograph (preparative chromatograph manufacturer: Oriendo, model: BRIX-2860, column: Synergi Max-RP 250 × 40 mm × 10 μm, mobile phase: water (0.225% HCOOH)-acetonitrile, an elution ratio of acetonitrile: 10% to 45%), to obtain compound **LY-43E** (7.01 mg, yield = 28.11%).

LCMS (ESI): m/z, 1070[M+H]$^+$.

$^1$H NMR (400 MHz, DMSO-$d^6$) δ 8.80 (d, *J* = 7.2 Hz, 1H), 8.20 (s, 1H), 8.16 (d, *J* = 7.2 Hz, 1H), 7.99 (d, *J* = 7.8 Hz, 1H), 7.78-7.72 (m, 2H), 7.33 (s, 1H), 7.01 (s, 2H), 6.53 (s, 1H), 5.44 (s, 2H), 5.27 (s, 2H), 4.70 (ddt, *J* = 32.2, 16.6, 8.8 Hz, 4H), 4.30 (q, *J* = 7.2 Hz, 1H), 4.27-4.18 (m, 2H), 3.75 (d, *J* = 5.6 Hz, 2H), 3.24-2.89 (m, 7H), 2.09 (dq, *J* = 12.8, 6.4, 5.8 Hz, 4H), 2.00 (q, *J* = 6.6 Hz, 1H), 1.93-1.82 (m, 2H), 1.67 (s, 1H), 1.62-1.40 (m, 8H), 1.30 (d, *J* = 7.2 Hz, 6H), 1.18 (d, *J* = 16.8 Hz, 9H), 0.90-0.86 (m, 6H), 0.82 (d, *J* = 6.8 Hz, 3H). $^{19}$F NMR (377 MHz, DMSO-$d^6$) δ -114.09.

Example 56: Preparation of compound **LY-44A**

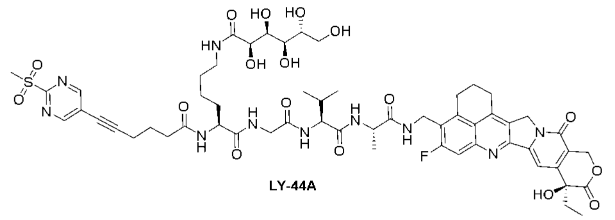

LY-44A

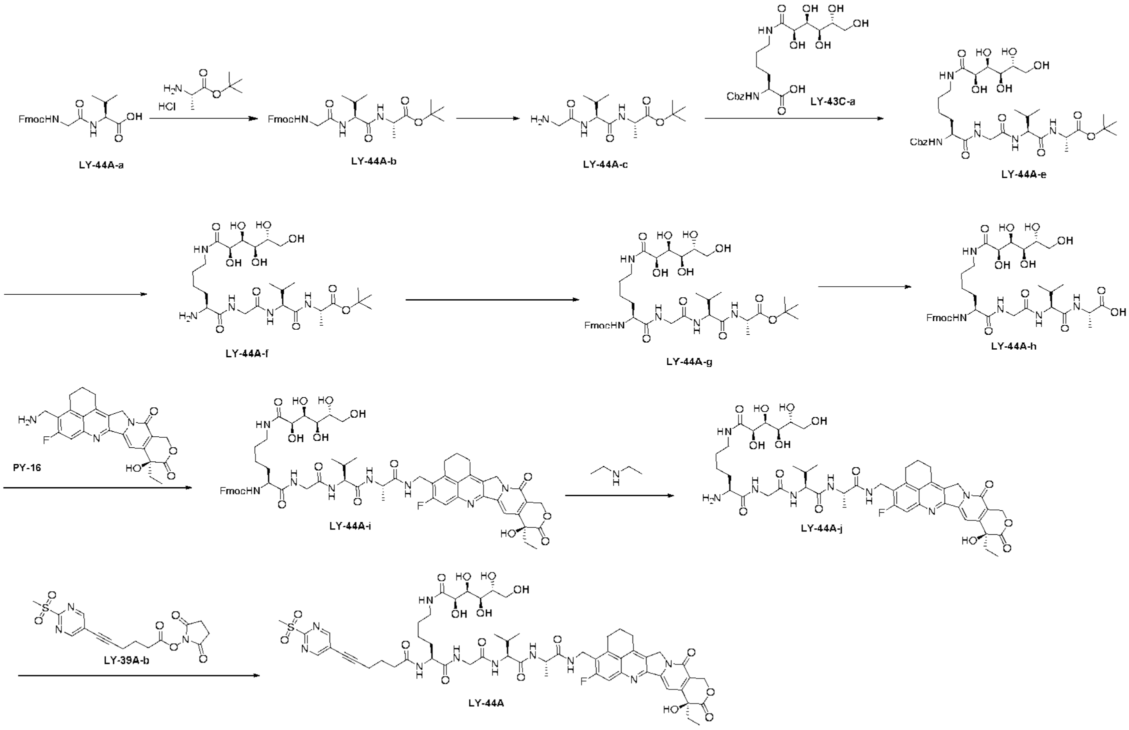


Step 1: Preparation of compound **LY-44A-b**

1.2 equivalents of tert-butyl L-alaninate hydrochloride (275 mg), 1.2 equivalents of O-(7-azabenzotriazol-1-yl)-N,N,N',N'-tetramethyluronium hexafluorophosphate (576 mg, 1.2 eq) and 3.0 equivalents of N,N-diisopropylethylamine (489 mg) were added to the solution of the compound **LY-44A-a** (500 mg, 1.26 mmol, 1.0 eq) in N,N-dimethylformamide (10 mL), and after addition, the reaction solution was stirred at room temperature for 16 hours. The reaction solution was quenched with 20 mL of water and extracted with ethyl acetate (10 mL × 3), organic phases were combined, washed with saturated brine once, dried with anhydrous sodium sulfate, and filtered, the filtrate was concentrated under reduced pressure, and the residue was purified by silica gel column chromatography (eluents: ethyl acetate/petroleum ether = 1: 20-1:5), to obtain white solid compound **LY-44A-b** (567 mg, yield: 80%; purity: 93%) (colorless oily substance).

LCMS: m/z = 546.3 [M+Na]$^+$; Rt = 2.584 min.

$^1$H NMR (400 MHz, DMSO-$d^6$) δ 8.35 (d, *J* = 6.8 Hz, 1H), 7.89 (d, *J* = 7.5 Hz, 2H), 7.76 (d, *J* = 9.0 Hz, 1H), 7.71 (d, *J* = 7.5 Hz, 2H), 7.55 (t, *J* = 6.2 Hz, 1H), 7.42 (t, *J* = 7.5 Hz, 2H), 7.33 (t, *J* = 7.5 Hz, 2H), 4.33 - 4.20 (m, 4H), 4.11 (p, *J* = 7.0 Hz, 1H), 3.67 (d, *J* = 6.2 Hz, 2H), 1.95 (dt, *J* = 12.6, 6.3 Hz, 1H), 1.38 (d, *J* = 2.0 Hz, 9H), 1.24 (d, J = 7.2 Hz, 3H), 0.86 (dd, *J* = 19.2, 6.7 Hz, 6H).

Step 2: Preparation of compound **LY-44A-c**

10 equivalents of diethylamine (1.0 mL) were added to the solution of compound **LY-44A-b** (567 mg, 1.01 mmol, 1.0 eq) in N, N-dimethylformamide (10 mL). The reaction solution was stirred for 16 hours at room temperature. The reaction solution was concentrated under reduced pressure, to obtain a yellow oily substance (300 mg, crude product).

LCMS: m/z = 302.2 [M+H]$^+$; Rt = 0.852 min.

Step 3: Preparation of compound **LY-44A-e**

N,N-diisopropylethylamine (144 mg, 1.11 mmol, 3 eq) and O-(7-azabenzotriazol-1-yl)-N,N,N',N'-tetramethyluronium hexafluorophosphate (141.30 mg, 371.61 μmol, 1 eq) were added to the solution of compounds **LY-44A-c** (170.37 mg, 371.61 μmol, 1 eq) and **LY-43C-a** (112 mg, 371.61 μmol, 1 eq) in DMF (3 mL) at 0°C, and after addition, the reaction solution was stirred at room temperature for 2 hours. The reaction solution was concentrated under reduced pressure, and the residue was purified by preparative high performance liquid chromatograph (preparative chromatograph manufacturer: ISCO, model: ISCO-R2, column: YMC-Triart Prep C18 250 × 30 mm × 10 μm, mobile phase: water (0.225% FA)-acetonitrile, an elution ratio of water: 21% to 51%), to obtain white solid compound **LY-44A-e** (198 mg, yield: 64.8%).

LCMS: m/z = 741.1 $[M+H]^+$/764.4[M+Na]; Rt = 1.552 min.

Step 4: Preparation of compound **LY-44A-f**

Palladium on carbon (12 mg) was added to the solution of compound **LY-44A-e** (20 mg, 26.96 μmol, 1 eq) in MeOH (3 mL), hydrogen displacement was conducted 3 times via hydrogen balloons after addition, and then the reaction solution reacted in the hydrogen atmosphere for 2 hours. 2 mL of water was added to the reaction solution, then the reaction solution was filtered through diatomite, the filter cake was washed with methanol, and the filtrate was concentrated under reduced pressure to obtain the compound **LY-44A-f** (16 mg).

LCMS: m/z = 608.3 $[M+H]^+$; Rt = 0.883 min.

Step 5: Preparation of compound **LY-44A-g**

Sodium bicarbonate (22.81 mg, 10.56 μL, 271.52 μmol, 3 eq) (dissolved in 2 mL of water) was added to a solution of compound **LY-44A-f** (55 mg, 90.51 μmol, 1 eq) and N-(9-fluorophenylmethoxycarbonyloxy) succinimide (30.53 mg, 90.51 μmol, 1 eq) in tetrahydrofuran (6 mL) at 0°C. The reaction solution reacted at 25°C for 16 hours and concentrated under reduced pressure, and the crude product was dissolved in water and methanol and purified by preparative high performance liquid chromatograph (preparative chromatograph manufacturer: ISCO, model: ISCO-R2, column: Synergi Max-RP 250 × 40 mm × 10 μm, mobile phase: water (0.225% FA)-acetonitrile, an elution ratio of water: 30% to 60%), to obtain white solid compound **LY-44A-g** (70 mg, yield: 72%).

LCMS: m/z = 830.5 $[M+H]^+$/852.5$[M+Na]^+$; Rt =2.3 min.

Step 6: Preparation of compound **LY-44A-h**

Trifluoroacetic acid (2 mL) was added to the solution of compound **LY-44A-g** (70 mg, 84.34 μmol, 1 eq) in dichloromethane (10 mL) at 0°C, the reaction solution reacted at 25°C for 2 hours and concentrated under reduced pressure to obtain the colorless oily compound **LY-44A-h** (60 mg, crude product).

LCMS: m/z = 774.3 $[M+H]^+$; Rt = 1.793 min.

Step 7: Preparation of compound **LY-44A-i**

N,N-diisopropylethylamine (40 mg, 310.14 μmol, 4 eq) and O-(7-azabenzotriazol-1-yl)-N,N,N',N'-tetramethyluronium hexafluorophosphate (35.38 mg, 93.04 μmol, 1.2 eq) were added to the solution of compounds **LY-44A-h** (60 mg, 77.54 μmol, 1 eq) and **PY-16** (33.76 mg, 77.54 μmol, 1 eq) in DMF (3 mL) at 0°C, and after addition, the reaction solution was heated to 20°C and stirred for reaction for 2 hours. The reaction solution was purified by preparative high performance liquid chromatograph (preparative chromatograph manufacturer: ISCO, model: ISCO-R5, column: Phenomenex Luna C18 250 × 30 mm × 10 μm, mobile phase: water (0.225% FA)-acetonitrile, an elution ratio of water: 30% to 60%), to obtain white solid compound **LY-44A-i** (17 mg, yield: 18.4%).

LCMS: m/z = 1191.3 $[M+H]^+$/596.3 $[1/2M+1]^+$; Rt = 1.64 min.

Step 8: Preparation of compound **LY-44A-j**

Diethylamine (2.46 mg, 33.58 μmol, 5 eq) was added to the solution of compound **LY-44A-i** (8 mg, 6.72 μmol, 1 eq) in N,N-dimethylformamide (2 mL) at 0°C, and after addition, the reaction solution reacted for 2 hours at 20°C. The reaction solution was concentrated, to obtain white solid compound **LY-44-j** (6.5 mg, crude product).

LCMS: m/z = 969.4 $[M+H]^+$; Rt = 1.090 min.

Step 9: Preparation of compound **LY-44A**

Compound **LY-39A-b** (6.09 mg, 60%, 10 μmol, 1 eq) and N,N-diisopropylethylamine (3.47 mg, 4.43 μL, 26.83 μmol, 4 eq) were added to the solution of **LY-44A-j** (6.50 mg, 6.71 μmol, 1 eq) in N,N-dimethylformamide (1 mL) at 0°C, and after addition, the reaction solution reacted for 2 hours at 20°C. The reaction solution was purified by preparative high

performance liquid chromatograph (preparative chromatograph manufacturer: ISCO, model: ISCO-R2, column: YMC-Triart Prep C18 250 × 30 mm × 10 μm, mobile phase: water (0.225% FA)-acetonitrile, an elution ratio of water: 21% to 51%), to obtain white solid compound **LY-44A** (2.71 mg, yield: 33.13%).

LCMS: m/z = 1219.2 [M+H]$^+$/610.2[1/2M+1]$^+$; Rt = 1.318 min.

Example 57: Preparation of compound **LY-44C**

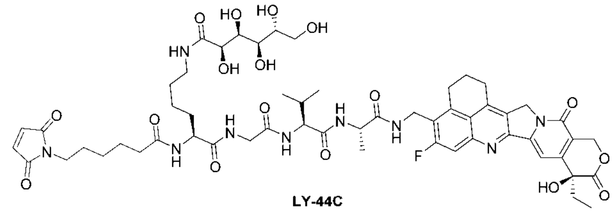

LY-44C

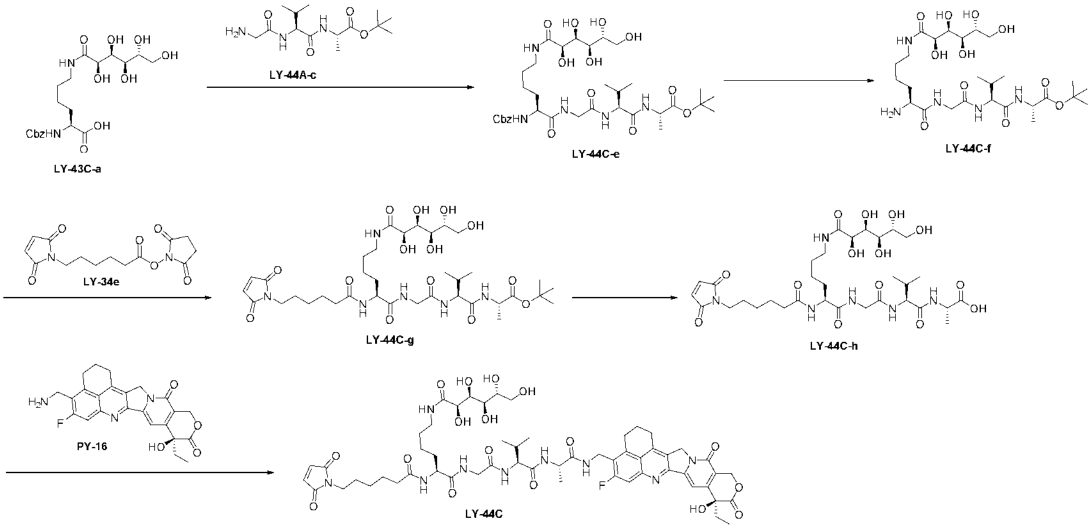


Step 1: Preparation of compound **LY-44C-e**

N,N-diisopropylethylamine (385.96 mg, 2.99 mmol, 3 eq) and O-(7-azabenzotriazol-1-yl)-N,N,N',N'-tetramethyluronium hexafluorophosphate (454.18 mg, 1.19 mmol, 1.2 eq) were added to the solution of compounds **LY-43C-a** (456.35 mg, 0.99 mmol, 1 eq) and **LY-44A-c** (300 mg, 0.99 mmol, 1 eq) in N,N-dimethylformamide (10 mL) at 0°C. After addition, the reaction solution reacted for 16 hours at room temperature. The reaction solution was purified by preparative high performance liquid chromatograph (preparative chromatograph manufacturer: ISCO, model: ISCO-R6, column: Welch Xtimate C18 250 × 30 mm × 10 μm, mobile phase: water (0.225% FA)-acetonitrile, an elution ratio of water: 21% to 51%), to obtain white solid compound **LY-44C-e** (417 mg, yield: 56%, purity: 82%).

LCMS: m/z = 742.2 [M+H]$^+$; Rt = 1.551 min.

Step 2: Preparation of compound **LY-44C-f**

Palladium on carbon (36 mg) was added to a solution of compound **LY-44C-e** (110 mg, 82%, 121.32 μmol, 1 eq) in methanol (10 mL) in the nitrogen atmosphere. Hydrogen displacement was conducted 3 times after addition, and the reaction solution reacted in the hydrogen atmosphere for 2 hours. 3 mL of water was added to the reaction solution, then the reaction solution was filtered through diatomite, and the filtrate was concentrated under reduced pressure to obtain anhydrous oily compound **LY-44C-f** (75 mg, crude product).

LCMS: m/z = 608.3 [M+H]$^+$; Rt = 1.103 min.

Step 3: Preparation of compound **LY-44C-g**

Compound **LY-34e** (46 mg, 148.10 μmol, 1.2 eq) and N,N-diisopropylethylamine (47.85 mg, 61.19 μL, 370.25

µmol, 3 eq) were added to the solution of compound **LY-44C-f** (75 mg, 123.42 µmol, 1 eq) in N,N-dimethylformamide (3 mL), and after addition, the reaction solution reacted for 16 hours at 20°C. The reaction solution was concentrated under reduced pressure, and the residue was purified by preparative high performance liquid chromatograph (preparative chromatograph manufacturer: ISCO, model: ISCO-R3, column: YMC-Triart Prep C18 250 × 50 mm × 7 µm, mobile phase: water (0.225% FA)-acetonitrile, an elution ratio of water: 12% to 42%), to obtain compound **LY-44C-g** (70 mg, yield: 70.8%).

LCMS: m/z = 801.4 $[M+H]^+$; Rt = 1.345 min.

Step 4: Preparation of compound **LY-44C-h**

Trifluoroacetic acid (0.8 mL) was added to a solution of compound **LY-44C-g** (70 mg, 87.40 µmol, 1 eq) in dichloromethane (4 mL) at 0°C and after addition, the reaction solution reacted for 2 hours at 20°C. The reaction solution was concentrated under reduced pressure to obtain anhydrous oily compound **LY-44C-h** (65 mg, crude product).

LCMS: m/z = 745.4 $[M+H]^+$; Rt = 0.770 min.

Step 5: Preparation of compound **LY-44C**

N,N-diisopropylethylamine (20.82 mg, 26.63 µL, 161.12 µmol, 4 eq) and O-(7-azabenzotriazol-1-yl)-N,N,N',N'-tetramethyluronium hexafluorophosphate (18.38 mg, 48.34 µmol, 1.2 eq) were added to the solution of compounds **LY-44C-h** (30 mg, 40.28 µmol, 1 eq) and **PY-16** (15.79 mg, 36.25 µmol, 0.9 eq) in N,N-dimethylformamide (2 mL) at 0°C and after addition, the reaction solution reacted for 2 hours at 25°C. The reaction solution was purified by preparative high performance liquid chromatograph (preparative chromatograph manufacturer: ISCO, model: ISCO-R6, column: Welch Xtimate C18 250 × 30 mm × 10 µm, mobile phase: water (0.01% TFA)-acetonitrile, an elution ratio of water: 12% to 42%), to obtain white solid compound **LY-44C** (1.49 mg, yield: 3.18 %).

LCMS: m/z = 1162.4 $[M+H]^+$; Rt = 1.332 min.

Example 58: Preparation of compound **LY-44D**

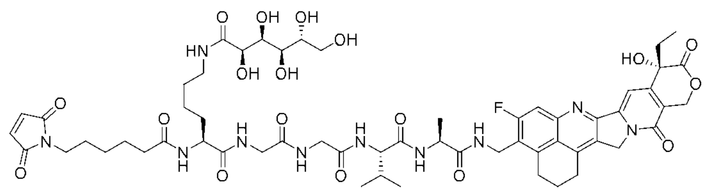

LY-44D

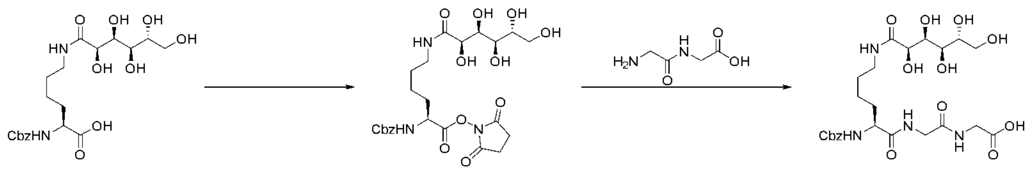

LY-43C-a

LY-44a

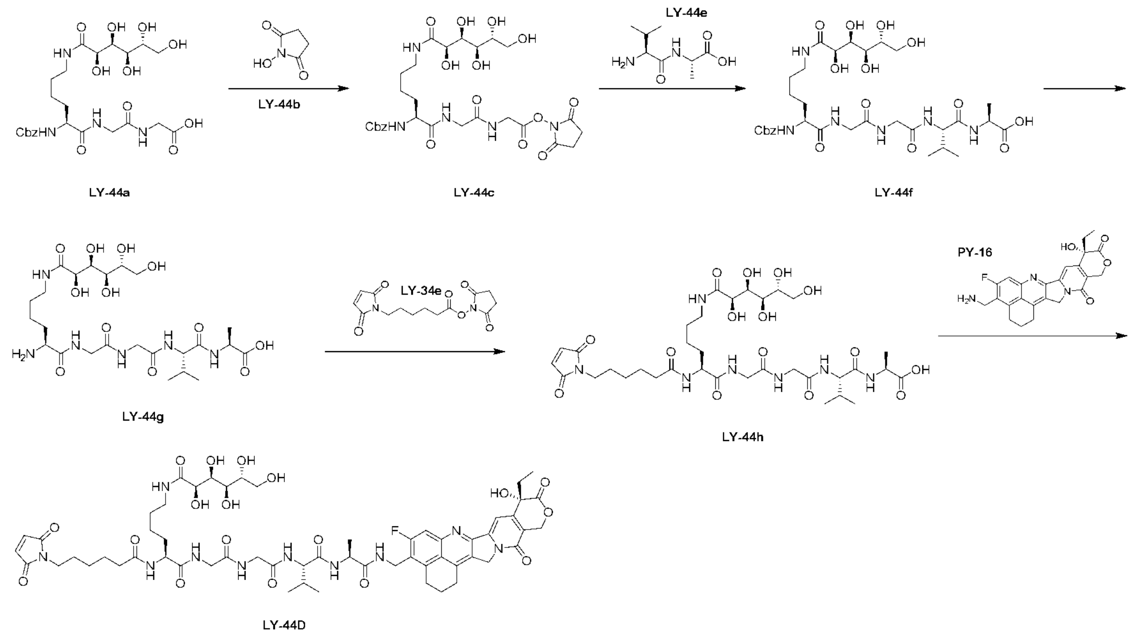


Step 1: Preparation of 2,5-dioxopyrrolidin-1-yl$N^2$-((benzyloxy)carbonyl)-$N^6$-((2R,3S,4R,5R)-2,3,4,5,6-pentahydroxyhexanoyl)-L-lysine

DIC (264.26 mg, 330.32 μL, 2.09 mmol, 1.2 eq) and NHS (240.99 mg, 2.09 mmol, 1.2 eq) were added to the solution of compound LY-43C-a (800 mg, 1.74 mmol, 1 eq) in THF (8 mL) and DMF (4 mL). After addition, the reaction solution reacted for 4 hours at 25°C, and the reaction solution was directly used for the next step without treatment.
LCMS (ESI): m/z, 556.2 $[M+H]^+$.

Step 2: Preparation of $N^2$-((benzyloxy)carbonyl)-$N^6$-((2R,3S,4R,5R)-2,3,4,5,6-pentahydroxyhexanoyl)-L-lysyl-glycylglycine **(LY-44a)**

Glycylglycine (230.45 mg, 1.74 mmol, 1 eq), triethylamine (353 mg, 484.89 μL, 3.49 mmol, 2 eq) and 5 mL of water were added to the solution of 2,5-dioxopyrrolidin-1-yl$N^2$-((benzyloxy)carbonyl)-$N^6$-((2R,3S,4R,5R)-2,3,4,5,6-pentahydroxyhexanoyl)-L-lysine (969 mg, 1.74 mmol, 1 eq) in THF (8 mL) and DMF (4 mL). After addition, the reaction solution reacted for 16 hours at 25°C, the reaction solution was concentrated under reduced pressure, and the residue was purified by preparative high performance liquid chromatograph (preparative chromatograph manufacturer: ISCO, model: R3, column: GS-120-10-C18AP, mobile phase: water (0.225% FA)-acetonitrile, an elution ratio of water: 3% to 33%) and lyophilized, to obtain a white solid compound **LY-44a** (680 mg, yield: 68.09%).
LCMS (ESI): m/z, 595.3 $[M+Na]^+$.

Step 3: Preparation of compound **LY-44c**

The compound **LY-44a** (200 mg, 349.30 μmol, 1 eq) was dissolved in N,N-dimethylformamide (5 mL), then the compound 1-hydroxypyrrolidine-2,5-dione **(LY-44b)** (48.24 mg, 419.16 μmol, 1.2 eq) was added, and 1,3-diisopropylcarbodiimide (52.90 mg, 66.12 μL, 419.16 μmol, 1.2 eq) was further added. The reaction solution was stirred at 15°C for 5 hours. The reaction solution was directly used for the next step without treatment.
LCMS (ESI): m/z, 670.3 $[M+H]^+$.

Step 4: Preparation of compound **LY-44f**

The compound **LY-44c** (200 mg, 298.67 μmol, 1 eq) was dissolved in N,N-dimethylformamide (2 mL), and L-valinamide-L-alanine **(LY-44e)** (7.66 mg, 10.79 μL, 104.73 μmol, 6 eq) and N,N-diisopropylethylamine (77.21 mg, 98.73 μL, 597.34 μmol, 2 eq) were added to the solution. The solution was stirred for 12 hours at 15°C in the nitrogen atmosphere. The reaction solution was purified by preparative high performance liquid chromatograph (preparative chromatograph manufacturer: ISCO, model: ISCO-6, column: Welch Xtimate C18 250 × 50 mm × 10 μm, mobile phase: water (0.225% FA)-ACN, an elution ratio of water: 12% to 42%), to obtain compound **LY-44f** (55 mg, yield: 24.79%).

LCMS (ESI): m/z, 743.3 $[M+H]^+$.

Step 5: Preparation of compound **LY-44g**

The compound **LY-44f** (35 mg, 47.12 μmol, 1 eq) was dissolved in methanol (3 mL), palladium on carbon (15 mg, 140.95 μL, 2.9913 eq) was added, hydrogen displacement was performed three times, and the reaction solution was stirred for 2 hours in the hydrogen atmosphere. The reaction solution was filtered and then concentrated under reduced pressure to obtain the white solid product **LY-44g** (25 mg, yield: 87.17%).

LCMS (ESI): m/z, 609.3 $[M+H]^+$.

Step 6: Preparation of compound **LY-44h**

Compound **LY-44g** (20 mg, 32.86 μmol, 1 eq) was dissolved in N,N-dimethylformamide (1 mL), then N,N-diisopropylethylamine (12.74 mg, 16.29 μL, 98.58 μmol, 3 eq) and compound **LY-34e** (304.79 mg, 507.99 μL, 1.71 mmol, 2 eq) were added, and the reaction solution was stirred at 15°C for 12 hours. The reaction solution was purified by preparative high performance liquid chromatograph (preparative chromatograph manufacturer: ISCO, model: ISCO-6, column: Welch Xtimate C18 250 × 50 mm × 10 μm, mobile phase: water (0.225% FA)-ACN, an elution ratio of water: 3% to 33%) and lyophilized, to obtain a white solid product **LY-44h** (12 mg, yield: 45.54%).

LCMS (ESI): m/z, 802.3$[M+H]^+$.

Step 7: Preparation of compound **LY-44D**

Compound **PY-16** (7.82 mg, 17.96 μmol, 1.2 eq) and N,N-diisopropylethylamine (2.90 mg, 3.71 μL, 22.45 μmol, 1.5 eq) were added to the solution of compound **LY-44h** (12 mg, 14.97 μmol, 1 eq) in N,N-dimethylformamide (0.5 mL), and then 2-(7-azabenzotriazole)-N,N,N',N'-tetramethyluronium hexafluorophosphate (8.54 mg, 22.45 μmol, 1.5 eq) was added. The reaction solution was stirred for 2 hours at 15°C. The reaction solution was purified by preparative high performance liquid chromatograph (preparative chromatograph manufacturer: ISCO, model: ISCO-6, column: YMC-Triart Prep C18 250 × 30 mm × 10 μm, mobile phase: water (0.225% FA)-ACN, an elution ratio of water: 12% to 42%) and lyophilized, to obtain a white solid **LY-44D** (5.57 mg, yield: 30.53%, purity: 97.4%).

LCMS (ESI): m/z, 1219.5$[M+H]^+$.

$^1$H NMR (400 MHz, DMSO-$d_6$) δ 8.20 - 8.13 (m, 2H), 8.09 (d, *J* = 7.4 Hz, 1H), 7.99 (s, 1H), 7.93 (d, *J* = 7.4 Hz, 1H), 7.76 (dd, *J* = 10.0, 5.7 Hz, 2H), 7.61 (t, *J* = 5.8 Hz, 1H), 7.32 (s, 1H), 6.99 (s, 2H), 6.52 (s, 1H), 5.44 (s, 2H), 5.35 (d, *J* = 5.0 Hz, 1H), 5.26 (s, 2H), 4.52 (s, 3H), 4.47 (d, *J* = 5.0 Hz, 1H), 4.39 (d, *J* = 7.2 Hz, 1H), 4.32 (t, *J* = 5.7 Hz, 1H), 4.24 (t, *J* = 7.1 Hz, 1H), 4.16 (t, *J* = 7.8 Hz, 2H), 3.97 (t, *J* = 4.4 Hz, 1H), 3.90 (s, 1H), 3.71 (dd, *J* = 12.6, 5.7 Hz, 4H), 3.55 (d, *J* = 7.3 Hz, 1H), 3.47 (s, 2H), 3.21-3.13 (m, 5H), 3.07-3.02 (m, 2H), 2.07 (dd, *J* = 14.0, 5.3 Hz, 4H), 1.89 (dt, *J* = 14.8, 7.1 Hz, 4H), 1.62 (s, 1H), 1.52-1.35 (m, 8H), 1.18 (d, *J* = 7.1 Hz, 8H), 0.88 (t, *J* = 7.3 Hz, 3H), 0.77 (dd, *J* = 9.9, 6.8 Hz, 6H).

Example 59: Preparation of compound **LY-46D**

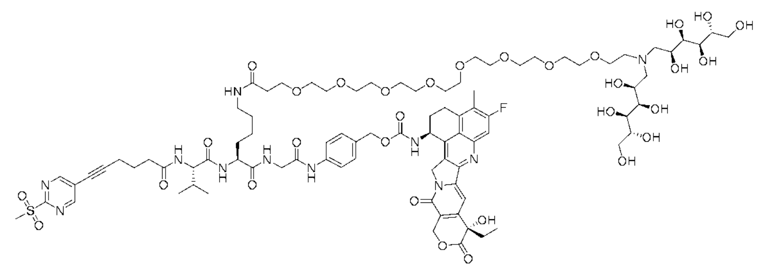

LY-46D

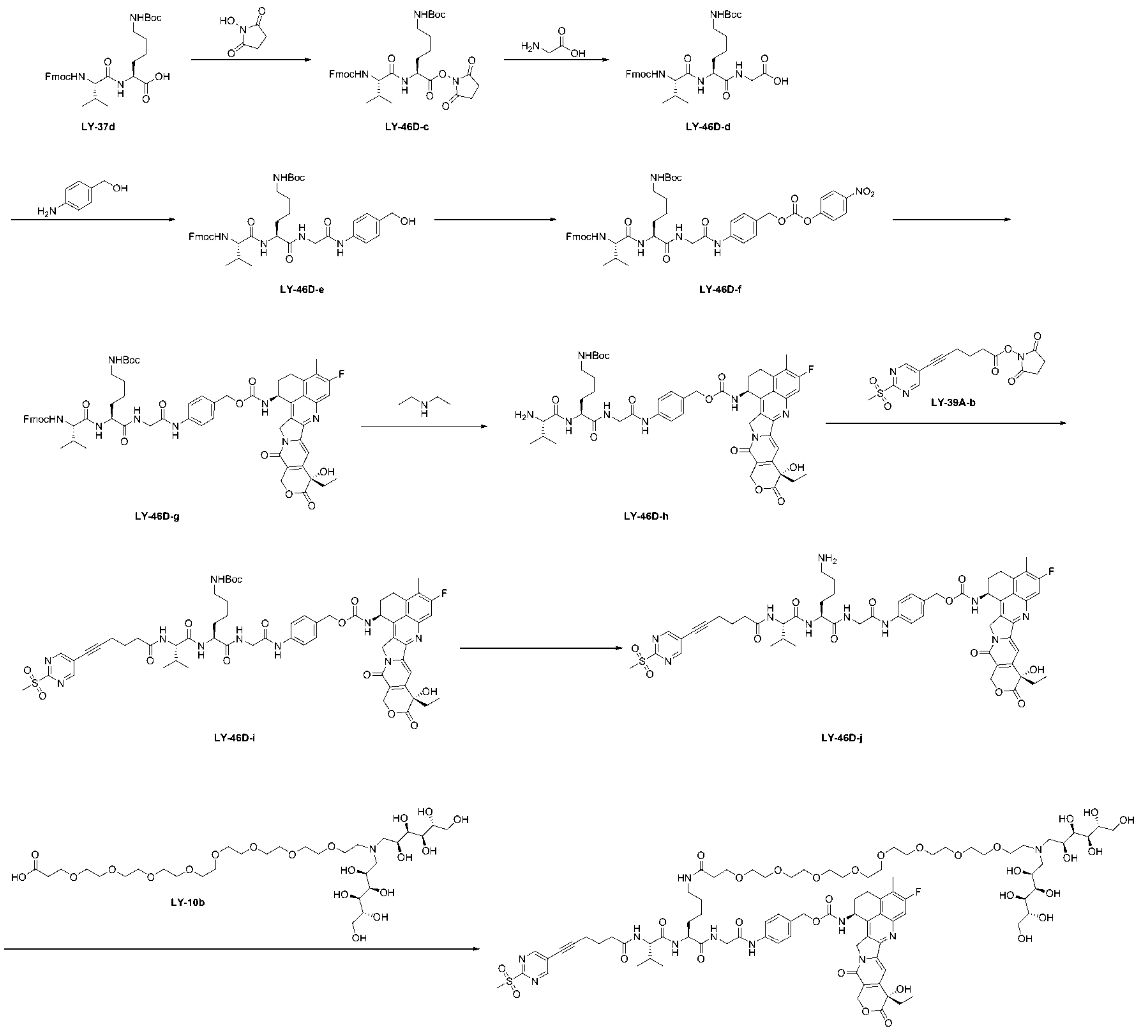

LY-37d, LY-46D-c, LY-46D-d, LY-46D-e, LY-46D-f, LY-46D-g, LY-46D-h, LY-39A-b, LY-46D-i, LY-46D-j, LY-10b, LY-46D

Step 1: Preparation of compound **LY-46D-c**

Dicyclohexylcarbodiimide (1.82g, 2.24 mL, 8.81 mmol, 1 eq) was added to the solution of N-hydroxysuccinimide (1.22g, 760.27 μL, 10.57 mmol, 1.2 eq) and compound **LY-37d** (5g, 8.81 mmol, 1 eq) in dichloromethane (100 mL) at room temperature and the reaction solution was stirred at 20°C for 16 hours. The reaction solution was filtered and then the filtrate was concentrated to obtain the white solid product LY-46D-c (4.40g, yield: 75.15%, purity: 90%).

LCMS (ESI): m/z, 687.4[M+Na]$^+$.

Step 2: Preparation of compound **LY-46D-d**

Sodium bicarbonate (250.23 mg, 135.99 μL, 2.98 mmol, 1 eq) and glycine (223.60 mg, 140.19 μL, 2.98 mmol, 1 eq) were dissolved in water (22 mL) and the reaction solution was stirred at 20°C for 15 minutes. LY-46D-c (2.20g, 90%, 2.98 mmol, 1 eq) was dissolved in tetrahydrofuran (22 mL), the resulting solution was then added to the above solution, and the reaction solution was stirred at 20°C for 16 hours. The reaction solution was distilled under pressure to remove tetrahydrofuran, and the remaining aqueous phase was acidified with 2M hydrochloric acid until pH reached 2, and extracted with ethyl acetate (20 mL) three times. The organic phases were combined, dried with anhydrous sodium sulfate, and concentrated under reduced pressure to obtain crude product **LY-46D-d** (3.3g, no yield was calculated for the crude product).

LCMS (ESI): m/z, 647.4 [M+Na]$^+$.

Step 3: Preparation of compound **LY-46D-e**

Compound **LY-46D-d** (3.30g, 63%, 3.33 mmol, 1 eq), 4-aminobenzyl alcohol (614.72 mg, 4.99 mmol, 1.5 eq), and

2-ethoxy-1-ethoxycarbonyl-1,2-dihydroquinoline (2.47g, 2.06 mL, 9.98 mmol, 3 eq) were sequentially added to dichloromethane (50 mL) and methanol (25 mL) at room temperature. The reaction solution was stirred for 60 hours at 25°C. The reaction solution was filtered to obtain a filter cake and after the filter cake was dried under reduced pressure, the white solid product **LY-46D-e** (1g, 1.37 mmol, yield: 41.17%) was obtained. The filtrate was concentrated under reduced pressure to obtain a crude product, where the crude product was pulped with petroleum ether (10 mL) and ethyl acetate (10 mL). The reaction solution was filtered to obtain a filter cake and after the filter cake was dried under reduced pressure, milk white solid product **LY-46D-e** (290 mg, no yield was calculated for the crude product) was obtained.

LCMS (ESI): m/z, 752.5$[M+Na]^+$.

Step 4: Preparation of compound **LY-46D-f**

The compound **LY-46D-e** (1g, 100%, 1.37 mmol, 1 eq), 4-nitrophenyl carbonate (833.59 mg, 555.73 μL, 2.74 mmol, 2 eq), and N,N-diisopropylethylamine (531.21 mg, 679.30 μL, 4.11 mmol, 3 eq) were sequentially added to N,N-dimethylformamide (10 mL) at room temperature. The reaction solution was stirred for 16 hours at 20°C. The reaction solution was purified by preparative high performance liquid chromatograph (preparative chromatograph manufacturer: Oriendo, model: BRIX-2860, column: Phenomenex Luna C18 250 × 50 mm × 10 μm, mobile phase: water (0.225% HCOOH)-acetonitrile, an elution ratio of water: 10% to 70%) and lyophilized, to obtain white solid product **LY-46D-f** (290 mg, yield: 23.65%, purity: 80%) and impure white solid product **LY-46D-f** (94 mg, purity: 60%).

LCMS (ESI): m/z, 917.5$[M+Na]^+$.

Step 5: Preparation of compound **LY-46D-g**

Exatecan mesylate (137.80 mg, 259.22 μmol, 1 eq) and N,N-diisopropylethylamine (33.50 mg, 42.84 μL, 259.22 μmol, 1 eq) were added to N,N-dimethylformamide (15 mL) at room temperature and the reaction solution was stirred at 20°C for 20 minutes. Then compound **LY-46D-f** (290 mg, 80%, 259.22 μmol, 1 eq), 1-hydroxybenzotriazole (17.51 mg, 11.68 μL, 129.61 μmol, 0.5 eq) and pyridine (102.52 mg, 104.83 μL, 1.30 mmol, 5 eq) were sequentially added to the above reaction solution at room temperature, and the reaction solution was stirred at 20°C for 16 hours. The reaction solution was directly used for the next step.

LCMS (ESI): m/z, 1191.4$[M+H]^+$.

Step 6: Preparation of compound **LY-46D-h**

Diethylamine (189.60 mg, 267.04 μL, 2.59 mmol, 10 eq) was added to the reaction solution in the previous step at room temperature and the resulting solution was stirred at 20°C for 3 hours. The reaction solution was concentrated under reduced pressure to obtain the brown oily crude product **LY-46D-h** (theoretical output: 251 mg).

LCMS (ESI): m/z, 991.5$[M+Na]^+$.

Step 7: Preparation of compound **LY-46D-i**

N,N-diisopropylethylamine (100.42 mg, 128.42 μL, 777.03 μmol, 3 eq) was added to the solution of compounds **LY-39A-b** (113.56 mg, 310.81 μmol, 1.2 eq) and **LY-46D-h** (251 mg, 259.01 μmol, 1 eq) in N,N-dimethylformamide (6 mL) at room temperature. The reaction solution was stirred at 20°C for 2 hours. The reaction solution was purified by preparative high performance liquid chromatograph (preparative chromatograph manufacturer: Oriendo, model: BRIX-2860, column: Phenomenex Luna C18 250 × 50 mm × 10 μm, mobile phase: water (0.225% HCOOH)-acetonitrile, an elution ratio of water: 39% to 69%) and lyophilized, to obtain a white solid product **LY-46D-i** (255 mg, 209.13 μmol, four-step yield: 15%).

LCMS (ESI): m/z, 1241.6$[M+Na]^+$.

Step 8: Preparation of compound **LY-46D-j**

Compound **LY-46D-i** (135 mg, 110.71 μmol, 1 eq) was added to trifluoroacetic acid (0.2 mL) and dichloromethane (2 mL) at 0°C. The reaction solution was stirred at 0°C for 2 hours, then heated to 20°C, and stirred for 14 hours. The reaction solution was concentrated under reduced pressure to obtain yellow solid product **LY-46D-j** (124 mg, 110.8 μmol, theoretical output).

LCMS (ESI): m/z, 1119.6$[M+H]^+$.

Step 9: Preparation of compound **LY-46-D**

N,N,N',N'-tetramethyl-O-(7-azabenzotriazole-1-yl)hexafluorophosphate (105.32 mg, 276.97 μmol, 2.5 eq) was added to the solution of compounds **LY-46D-j** (124 mg, 110.79 μmol, 1 eq) and **LY-10b** (255.87 mg, 332.37 μmol, 3 eq), and N,N-diisopropylethylamine (114.55 mg, 146.48 μL, 886.32 μmol, 8 eq) in N,N-dimethylformamide (6 mL) at room temperature, and then the reaction solution was stirred at 20°C for 2 hours. The reaction solution was purified by preparative high performance liquid chromatograph (preparative chromatograph manufacturer: Oriendo, model: BRIX-2860, column: Phenomenex Luna C18 250 × 50 mm × 10 μm, mobile phase: water (0.1% TFA)-acetonitrile, an elution ratio of water: 20% to 50%), to obtain product **LY-46-D** (72.67 mg, 38.84 μmol, second-step yield: 35%, purity: 98.01%).

LCMS (ESI): m/z, 936.4[1/2M+H]$^+$.

$^1$H NMR (400 MHz, DMSO-$d_6$) δ 9.85 (s, 1H), 9.10 (s, 2H), 8.25 (t, *J* = 5.6 Hz, 1H), 8.18-8.03 (m, 3H), 7.95 (d, *J* = 8.4 Hz, 1H), 7.82-7.75 (m, 2H), 7.62 (d, *J* = 8.4 Hz, 2H), 7.37 (d, *J* = 8.0 Hz, 2H), 7.31 (s, 1H), 6.53 (s, 1H), 5.44 (d, *J* = 7.2 Hz, 4H), 5.28 (s, 3H), 5.07 (s, 2H), 4.79 (s, 2H), 4.54 (s, 5H), 4.20 (dd, *J* = 16.4, 7.6 Hz, 2H), 3.99 (s, 2H), 3.90-3.84 (m, 2H), 3.78 (t, *J* = 5.2 Hz, 2H), 3.68 (s, 2H), 3.61 (d, *J* = 3.2 Hz, 1H), 3.59-3.54 (m, 6H), 3.53-3.46 (m, 25H), 3.43 (s, 2H), 3.40 (s, 4H), 3.25 (d, *J* = 14.4 Hz, 3H), 3.13 (s, 2H), 3.01 (q, *J* = 6.4 Hz, 2H), 2.55 (d, *J* = 7.2 Hz, 2H), 2.39-2.33 (m, 4H), 2.29 (t, *J* = 6.8 Hz, 2H), 2.18 (d, *J* = 14.0 Hz, 2H), 2.02-1.95 (m, 1H), 1.92-1.76 (m, 4H), 1.68 (d, *J* = 7.2 Hz, 1H), 1.60-1.53 (m, 1H), 1.42-1.22 (m, 6H), 1.16-1.08 (m, 1H), 0.91-0.80 (m, 13H).

Example 60: Preparation of compound **LY-46E**

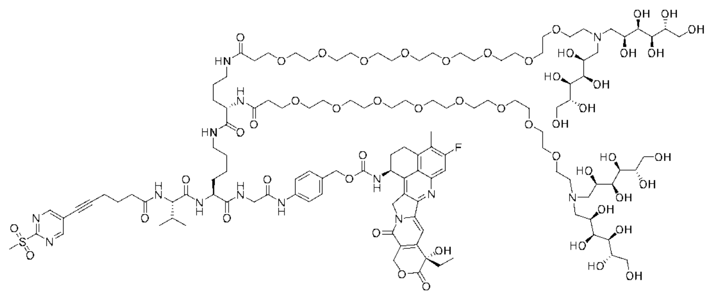

LY-46E

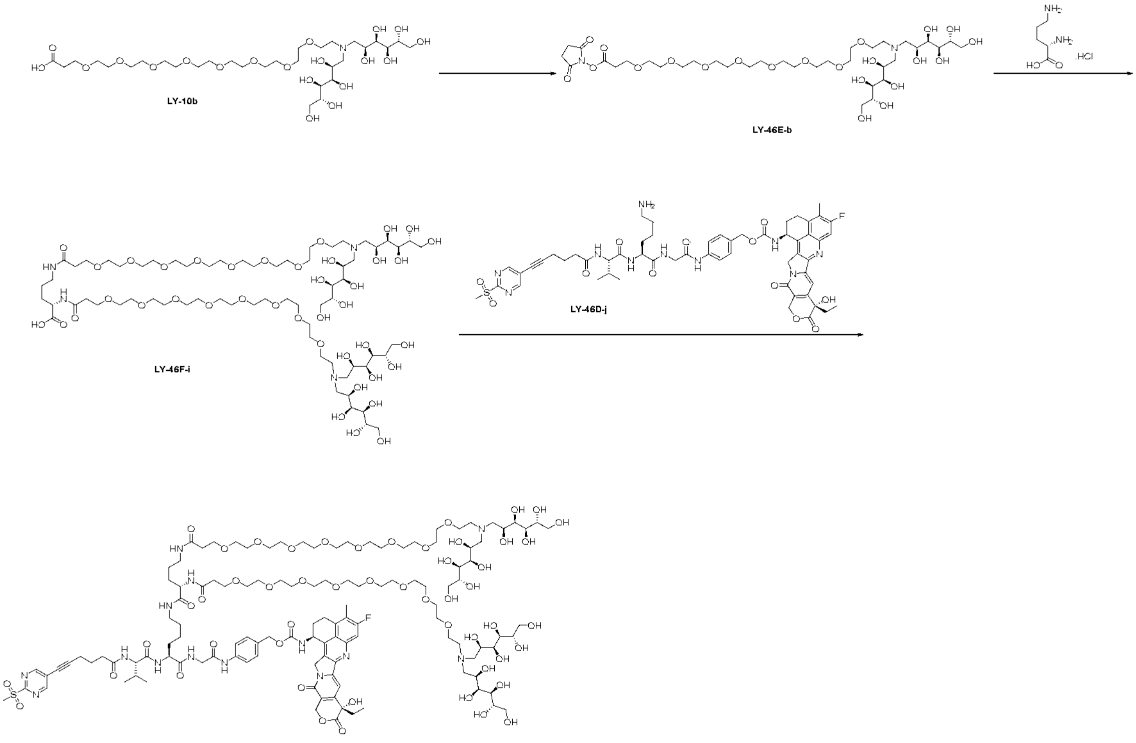


LY-10b

LY-46E-b

LY-46F-i

LY-46D-j

LY-46E

Step 1: Preparation of compound **LY-46E-b**

Compound **LY-10b** (100 mg, 129.90 μmol, 1 eq), N-hydroxysuccinimide (14.95 mg, 9.34 μL, 129.90 μmol, 1 eq), and 1-(3-dimethylaminopropyl)-3-ethylcarbodiimide hydrochloride (32.37 mg, 36.91 μL, 168.87 μmol, 1.3 eq) were added to N,N-dimethylformamide (2.5 mL) at room temperature, and the resulting solution was stirred at 20°C for 16 hours. The reaction solution was directly used for the next step.

LCMS (ESI): m/z, 867.4[M+H]$^+$.

Step 2: Preparation of compound **LY-46F-i**

L-omithine hydrochloride (12 mg, 71.17 μmol, 1 eq) and N,N-diisopropylethylamine (45.99 mg, 58.81 μL, 355.83 μmol, 5 eq) were sequentially added to the reaction solution in the previous step at room temperature, and the reaction solution was stirred at 20°C for 16 hours. The reaction solution was purified by preparative high performance liquid chromatograph (preparative chromatograph manufacturer: Oriendo, model: BRIX-2860 (R1, 4, 5, 6, 7), column: Phenomenex Luna C18 250 × 50 mm × 10 μm, mobile phase: water (0.225% HCOOH)-acetonitrile, an elution ratio of water: 1% to 25%) and lyophilized, to obtain a white solid product **LY-46F-i** (54 mg, 33.01 μmol, two-step yield: 24%).

LCMS (ESI): m/z, 818.7[1/2M+H]$^+$.

Step 3: Preparation of compound **LY-46E**

N,N-diisopropylethylamine (5.94 mg, 7.60 μL, 45.96 μmol, 8 eq) was added to the solution of compound **LY-46D-**

**j** (6.43 mg, 5.74 μmol, 1 eq), compound **LY-46F-i** (28.19 mg, 17.23 μmol, 3 eq), and N,N,N',N'-tetramethyl-O-(7-azabenzotriazole-1-yl)hexafluorophosphate (5.46 mg, 14.36 μmol, 2.5 eq) in N,N-dimethylformamide (1 mL) at room temperature, and then the reaction solution was stirred at 20°C for 2 hours. The reaction solution was purified by preparative high performance liquid chromatograph (preparative chromatograph manufacturer: Oriendo, model: BRIX-2860 (R1, 4, 5, 6, 7), column: Phenomenex Luna C18 250 × 50 mm × 10 μm, mobile phase: water (0.1% TFA)-acetonitrile, an elution ratio of water: 21% to 51%) and lyophilized, to obtain product **LY-46-E** (2.56 mg, 935.32 nmol, yield: 16.28%, purity: 96.98%).

LCMS (ESI): m/z, 913.5[1/3M+2]$^+$.

Example 61: Preparation of compound **LY-46F**

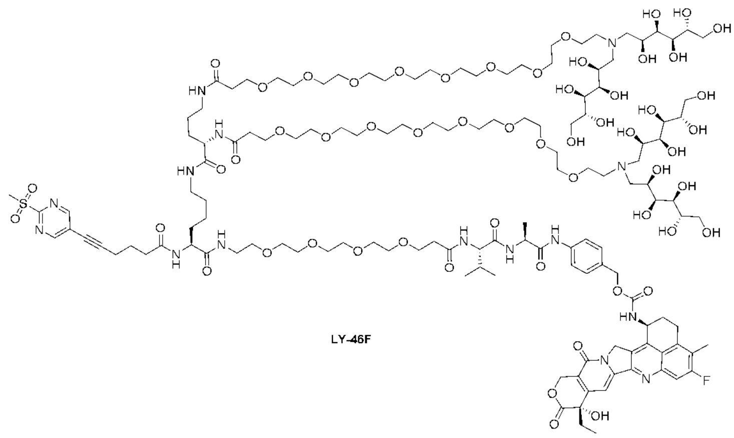

LY-46F

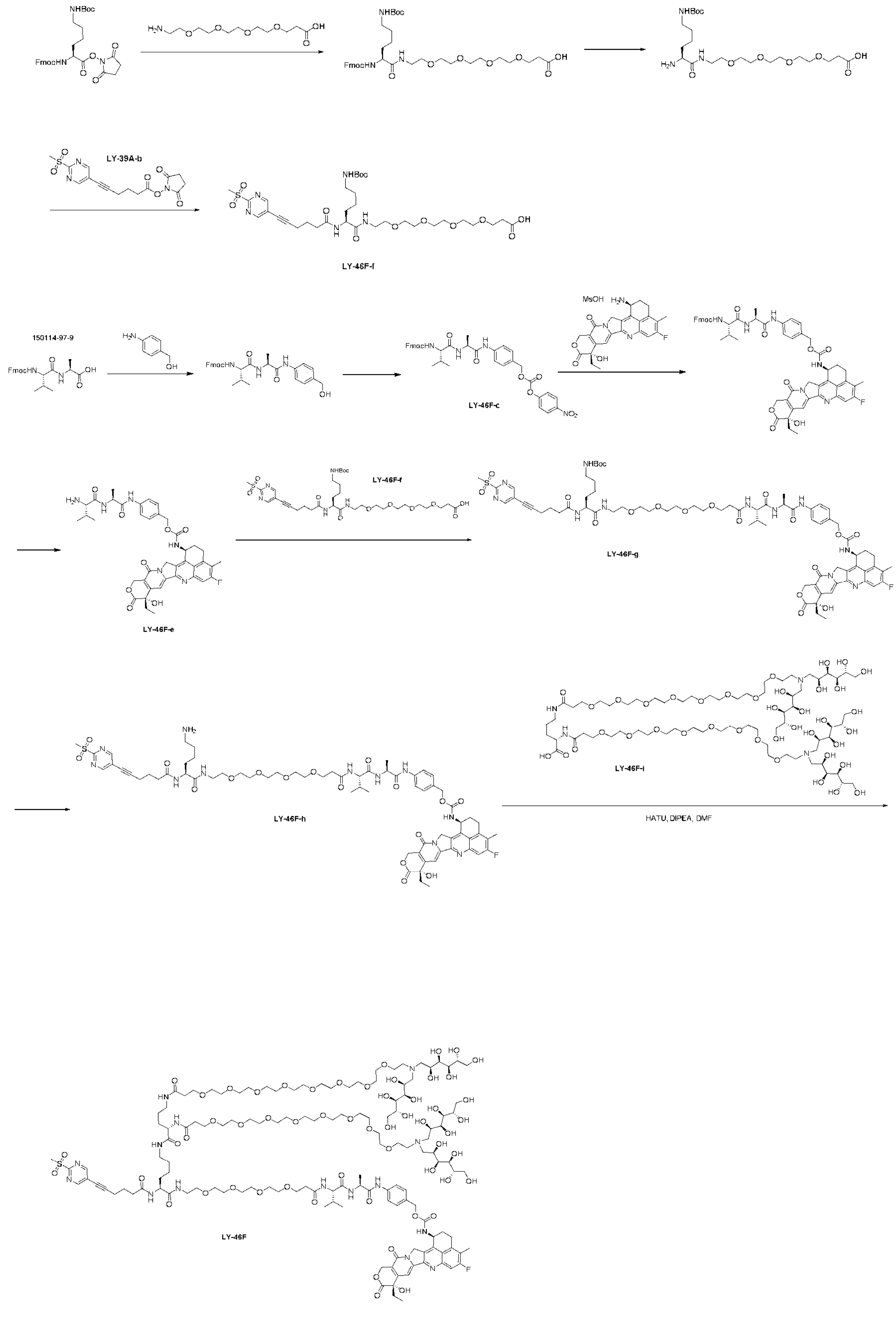
LY-39A-b
LY-46F-f
150114-97-9
LY-46F-c
MsOH
LY-46F-f
LY-46F-g
LY-46F-e
LY-46F-i
LY-46F-h
HATU, DIPEA, DMF
LY-46F

Step 1: Preparation of (S)-10-(((9H-fluoren-9-yl)methoxy)carbonyl)amino)-2,2-dimethyl-4,11-dioxo-3,15,18,24-pentaoxa-5,12-diazaheptan-27-acid

N,N-diisopropylethylamine (342.74 mg, 438.29 μL, 2.65 mmol, 3 eq) was added to a solution of 1-amino-3,6,9,12-tetraoxopentadecan-15-acid (234.52 mg, 883.99 μmol, 1 eq) and $N^6$-[(1,1-dimethylethoxy)carbonyl)-$N^2$-[(9H-fluoren-9-ylmethoxy)carbonyl)-L-lysine 2,5-dioxo-1-pyrrolidinyl ester (500 mg, 883.99 μmol, 1 eq) in N,N-dimethylformamide (10 mL) at room temperature. The reaction solution was stirred for 16 hours at 20°C. The reaction solution was purified by preparative high performance liquid chromatograph (preparative chromatograph manufacturer: Oriendo, BRIX-2860, column: Phenomenex Luna C18 150 × 25 mm × 10 μm, mobile phase: water (0.225% HCOOH)-acetonitrile, an elution ratio of water: 10% to 55%) and lyophilized, to obtain a white solid product (S)-10-(((9H-fluoren-9-yl)methoxy)carbonyl)amino)-2,2-dimethyl-4,11-dioxo-3,15,18,24-pentaoxa-5,12-diazaheptan-27-acid (300 mg, yield: 47.41%).

LCMS (ESI): m/z, 738.5 $[M+Na]^+$.

Step 2: Preparation of (S)-10-amino-2,2-dimethyl-4-11-dioxo-3,15,18,24-pentaoxa-5,12-diazaheptan-27-acid

Ethylenediamine (51.09 mg, 71.95 μL, 698.48 μmol, 10 eq) was added to a solution of (S)-10-(((9H-fluoren-9-yl)methoxy)carbonyl)amino)-2,2-dimethyl-4,11-dioxo-3,15,18,24-pentaoxa-5,12-diazaheptan-27-acid (50 mg, 69.85 μmol, 1 eq) in N,N-dimethylformamide (1 mL) at room temperature. The reaction solution was stirred for 2 hours at 20°C. The reaction solution was concentrated under reduced pressure, to obtain a yellow oily product (34 mg, no yield was calculated for the crude product).

LCMS (ESI): m/z, 494.3 $[M+Na]^+$.

Step 3: Preparation of compound **LY-46F-f**

(S)-10-amino-2,2-dimethyl-4-11-dioxo-3,15,18,24-pentaoxa-5,12-diazaheptan-27-acid (25.17 mg, 68.88 μmol, 1 eq), compound **LY-39A-b** (25.17 mg, 68.88 μmol, 1 eq), and N,N-diisopropylethylamine (26.71 mg, 34.15 μL, 206.65 μmol, 3 eq) were sequentially added to a solution of N,N-dimethylformamide (1 mL). The reaction solution was stirred for 2 hours at 20°C. The reaction solution was purified by preparative high performance liquid chromatograph (preparative chromatograph manufacturer: SHIMADZU, model: LC-20AP, column: YMC-Triart Prep C18 250 × 50 mm × 7 μm, mobile phase: water (0.225% HCOOH)-acetonitrile, an elution ratio of water: 20% to 50%) and lyophilized, to obtain a white solid product **LY-46F-f** (15 mg, three-step yield: 14%).

LCMS (ESI): m/z, 766.4 $[M+Na]^+$.

Step 4: Preparation of (9H-fluoren-9-yl)methyl((S)-1-(((S)-1-(4-(hydroxymethyl)phenyl)amino)-1-oxopropane-2-yl)amino)-3-methyl-1-oxobutane-2-yl)carbamate

The compound (((9H-fluoren-9-yl)methoxy)carbonyl)-L-valine-L-alanine (1g, 2.44 mmol, 1 eq) was dissolved in DCM (20 mL)/MeOH (10 mL), and (4-aminophenyl)methanol (360.04 mg, 2.92 mmol, 1.2 eq) and EEDQ (903.71 mg, 753.09 μL, 3.65 mmol, 1.5 eq) were added. The reaction solution was stirred for 12 hours at 25°C. The reaction solution was concentrated under reduced pressure, the obtained solid was pulped with petroleum ether/ethyl acetate (ratio: 3:1, total volume: 30 mL), and the filter cake was washed with petroleum ether/ethyl acetate (ratio: 3:1, total volume: 50 mL), to obtain the yellow solid product (9H-fluoren-9-yl)methyl((S)-1-(((S)-1-(4-(hydroxymethyl)phenyl)amino)-1-oxopropane-2-yl)amino)-3-methyl-1-oxobutane-2-yl)carbamate (1.10g, yield: 87.57%).

LCMS (ESI): m/z, 538.2 $[M+Na]^+$.

Step 5: Preparation of compound **LY-46F-c**

(9H-fluoren-9-yl)methyl((S)-1-(((S)-1-(4-(hydroxymethyl)phenyl)amino)-1-oxopropane-2-yl)amino)-3-methyl-1-oxobutane-2-yl)carbamate (500 mg, 969.74 μmol, 1 eq) was dissolved in DMF (6 mL), and bis(4-nitrophenyl) carbonate (590.01 mg, 393.34 μL, 1.94 mmol, 2 eq) and DIPEA (375.99 mg, 480.80 μL, 2.91 mmol, 3 eq) were added to the solution. The reaction solution reacted for 16 hours at 25°C. The reaction solution was concentrated under reduced pressure, and the residue was purified by silica gel column chromatography (eluents: ethyl acetate/petroleum ether = 1:30-1:1), to obtain a yellow solid compound **LY-46F-c** (380 mg, yield: 57.57%).

LCMS (ESI): m/z, 703.3 $[M+Na]^+$.

Step 6: Preparation of compound **LY-46F-d**

The compound exatecan mesylate (78.10 mg, 146.91 μmol, 1 eq) was dissolved in DMF (3 mL), DIPEA (18.99 mg, 24.28 μL, 146.91 μmol, 1 eq) was added, the reaction solution was stirred for 10 minutes, then the compound LY-46F-c (100 mg, 146.91 μmol, 1 eq), HOBT (9.93 mg, 6.62 μL, 73.45 μmol, 0.5 eq) and pyridine (58.10 mg, 59.41 μL, 734.54 μmol, 5 eq) were added, and the reaction solution was stirred at 25°C for 16 hours. The reaction solution was concentrated under reduced pressure, and purified by silica gel column chromatography (eluents: DCM: MeOH = 1:0-10:1), to obtain the yellow solid product **LY-46F-d** (150 mg, yield: 104.50%).
LCMS (ESI): m/z, 977.4 $[M+H]^+$.

Step 7: Preparation of compound **LY-46F-e**

Compound **LY-46F-d** (150 mg, 153.52 μmol, 1 eq) was dissolved in DMF (5 mL), $Et_2NH$ (56.14 mg, 79.07 μL, 767.61 μmol, 5 eq) was added, and the reaction solution was stirred at 25°C for 2 hours. The reaction solution was concentrated under reduced pressure, to obtain brown oily product **LY-46F-e** (100 mg, yield: 86.30%).
LCMS (ESI): m/z, 755.4 $[M+H]^+$.

Step 8: Preparation of compound **LY-46F-g**

Compound **LY-46F-f** was dissolved in DMF (2 mL), compound **LY-46F-e** (10.15 mg, 13.44 μmol, 1 eq) and DIPEA (3.47 mg, 4.44 μL, 26.89) were added to the reaction solution, then HATU (7.67 mg, 20.16 μmol, 1.5 eq) was added at 0°C, and the reaction solution was stirred at 5°C for 2 hours. LCMS showed that the reaction ended. The reaction solution was purified by preparative high performance liquid chromatograph (preparative chromatograph manufacturer: Oriendo, BRIX-2860 (R1, 4, 5, 6), column: Phenomenex Luna C18 150 × 25 mm × 10 μm, mobile phase: water (0.225% HCOOH)-acetonitrile, an elution ratio of water: 40% to 70%) and lyophilized, to obtain a white solid product **LY-46F-g** (9 mg, yield: 45.21%).
LCMS (ESI): m/z, 1502.7 $[(M+Na)]^+$.

Step 9: Preparation of compound **LY-46F-h**

The compound **LY-46F-g** (9 mg, 6.08 μmol, 1 eq) was dissolved in DCM (5 mL), TFA (693.05 μg, 465.1 μL, 6.08 μmol, 1 eq) was added to the reaction solution, and the reaction solution was stirred at 5°C for 2 hours. The reaction solution was directly spun to dryness to obtain a yellow solid product (8 mg, yield: 95.34%).
LCMS (ESI): m/z, 1380.5 $[(M+H)]^+$.

Step 10: Preparation of compound **LY-46F**

The compound **LY-46F-h** (8 mg, 5.79 μmol, 1 eq) was dissolved in DMF (1 mL), compound **LY-46E-i** (9.48 mg, 5.79 μmol, 1 eq) and DIPEA (1.50 mg, 1.92 μL, 11.59 μmol, 2 eq) were added to the reaction solution, the reaction solution was cooled to 0°C, and HATU (3.31 mg, 8.69 μmol, 1.5 eq) was added. The reaction solution was stirred at 25°C for 1 hour. The reaction solution was subjected to preparative high performance liquid chromatography to obtain the white solid product (620 μg, yield: 3.57%, purity: 98%).
LCMS (ESI): m/z, 1000.13 $[(M+2H)/2]^+$.

Example 62: Preparation of compound **LY-46G**

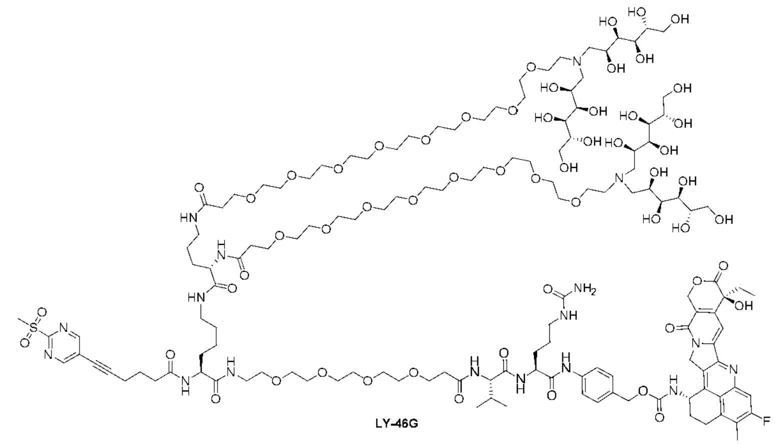

LY-46G

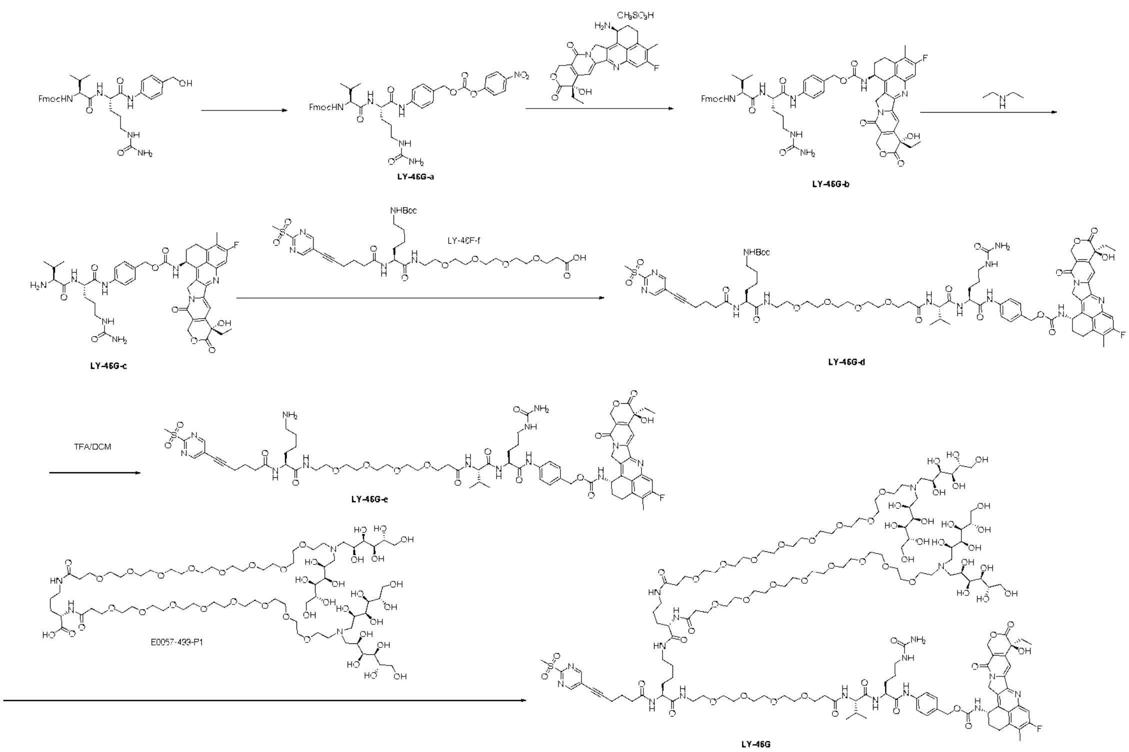


LY-46G-a

LY-46G-b

LY-46G-c

LY-46G-d

LY-46G-e

LY-46G

Step 1: Preparation of compound **LY-46G-a**

The compound (9H-fluoren-9-yl)methyl((S)-1-(((S)-1-(4-(hydroxymethyl)phenyl)amino)-1-oxo-5-ureidopentan-2-yl)amino)-3-methyl-1-oxobutan-2-yl)carbamate (300 mg, 1.0 eq, Bidepharm) was dissolved in anhydrous DMF (6 mL), DIEA (164.8 μL, 2.0 eq) and bis(p-nitrobenzene)carbonate (303.35 mg, 2.0 eq) were added in the ice water bath, and then the reaction solution was stirred at 25°C for reaction for1 hour. The reaction solution was cooled to 0°C, ether (120 mL) was slowly added dropwise to the reaction solution, and after the solid precipitated, the reaction solution was further stirred at 0°C for 1 hour, filtered, and dried to obtain compound **LY-46G-a** as a yellow solid (400 mg, crude yield: 104.6%)

LCMS: m/z = 767.4 $[M+H]^+$; Rt = 2.664 min.

Step 2: Preparation of compound **LY-46G-b**

The compound exatecan mesylate (197.58 mg, 1.0 eq, MCE) was dissolved in anhydrous DMF (5 mL), DIPEA (61.43 μL, 1.0 eq) was added at 25°C, and then the reaction solution was stirred for ten minutes. The compound **LY-46G-a** (300 mg, 1.0 eq) was then added, then HOBT (25.11 mg, 0.5 eq) and pyridine (150.3 μL, 5.0 eq) were further added, and then the reaction solution was stirred at 25°C for reaction for 16 hours. The reaction solution was filtered, and the filtrate was purified by reversed-phase C18 (mobile phase: water (0.225% HCOOH)-acetonitrile, an elution ratio of water: 10% to

65%), to obtain compound **LY-46G-b** (270 mg, yield: 68.33%).

LCMS: m/z = 1063.5 $[M+H]^+$; Rt = 2.525 min.

Step 3: Preparation of compound **LY-46G-c**

The compound **LY-46G-b** (250 mg, 1.0 eq) was dissolved in anhydrous DMF (2 mL), diethylamine (238 μL, 10.0 eq) was added, and the reaction solution was stirred at 25°C for reaction for 2 hours. The reaction solution was concentrated to dryness to obtain the crude product of compound **LY-46G-**c (240 mg, yield: 92.89%) to be directly used for the next reaction without purification.

LCMS: m/z = 841.5 $[M+H]^+$; Rt = 1.423 min.

Step 4: Preparation of compound **LY-46G-d**

Compound **LY-46G-c** (30 mg, 1.2 eq) and compound **LY-46F-f** (25.52 mg, 1.0 eq) were dissolved in anhydrous DMF (2 mL), DIEA (11.06 μL, 3.0 eq) and HATU (11.02 mg, 1.3 eq) were added at 0°C, and then the reaction solution was stirred at 25°C for reaction for 2 hours. The reaction solution was filtered, and the filtrate was purified by preparative high performance liquid chromatograph (preparative chromatograph manufacturer: SHIMADZU, model: LC-20AP, column: GS-120-10-C18AP, mobile phase: water (0.225% HCOOH)-acetonitrile, an elution ratio of water: 30% to 60%), to obtain compound **LY-46G-d** (56 mg, yield: 56.93%).

LCMS: m/z = 1589.8$[M+Na]^+$; Rt = 2.088 min.

Step 5: Preparation of compound **LY-46G-e**

The compound **LY-46G-d** (51.07 mg, 1.0 eq) was dispersed in anhydrous DCM (2 mL), the reaction solution was cooled to 0°C, trifluoroacetic acid (200 μL, 84.0 eq) was added dropwise, and the reaction solution was stirred at 0°C for reaction for 1 hour. The reaction solution was concentrated to remove dichloromethane and trifluoroacetic acid, to obtain the crude product of compound **LY-46G-**e (54 mg, yield: 100%) to be directly used for the next reaction without purification.

LCMS: m/z = 1489.7 $[M+Na]^+$; Rt = 1.598 min.

Step 6: Preparation of compound **LY-46G**

Compound **LY-46G-e** (54 mg, 1.0 eq) and compound **E0057-499-P1** were dissolved in anhydrous DMF (2 mL), the reaction solution was cooled to 0°C, DIEA (21.78 μL, 4.0 eq) and HATU (16.29 mg, 1.3 eq) were added, and then the reaction solution was stirred at 0°C for reaction for 1 hour. The reaction solution was filtered, and the filtrate was subjected to preparative high performance liquid chromatograph (preparative chromatograph manufacturer: Oriendo, model: BRIX-2860, column: Synergi Max-RP 250 × 40 mm × 10 μm, mobile phase: water (0.225% HCOOH),-acetonitrile, an elution ratio of water: 20% to 50%), to obtain a white solid compound LY-46G (7.02 mg, yield: 6.91%).

LCMS: m/z = 1542.8 $[1/2M+H]^+$; Rt = 1.421 min.

Example 63: Preparation of compound **LY-47A**

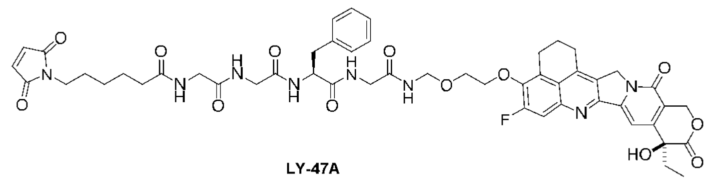

**LY-47A**

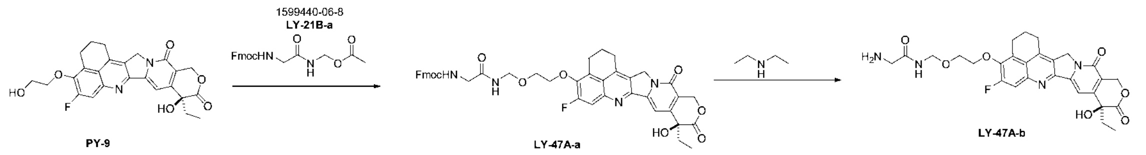


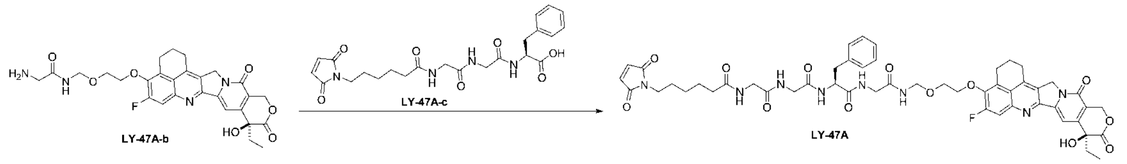


Step 1: Preparation of compound **LY-47A-a**

Compound **PY-9** (150 mg, 1.0 eq) and compound **LY-21B-a** (213.2 mg, 1.8 eq) were dissolved in anhydrous tetrahydrofuran (8 mL), p-toluenesulfonic acid (55 mg, 1.0 eq) was added, and then the reaction solution was stirred at 30°C for reaction for 3 hours. After the reaction solution was filtered, the filtrate was purified by reversed-phase preparative chromatograph (preparative chromatograph manufacturer: ISCO, model: ISCO-R1, column: Phenomenex Luna C18 250 × 30 mm × 10 μm, mobile phase: water (0.225% HCOOH)-acetonitrile, an elution ratio of water: 30% to 60%), to obtain compound **LY-47A-a** (57 mg, yield: 23%).
LCMS: m/z = 775.3 $[M+H]^+$; Rt = 2.118 min.

Step 2: Preparation of compound **LY-47A-b**

The compound **LY-47A-a** (57 mg, 1.0 eq) was dissolved in anhydrous DMF (2 mL), diethylamine (76 μL, 10.0 eq) was added, and the reaction solution was stirred at 25°C for reaction for 4 hours. The reaction solution was directly concentrated to dryness, to obtain compound **LY-47A-b** (42 mg, crude product) to be directly used for the next reaction.
LCMS: m/z = 553.3 $[M+H]^+$; Rt = 1.025 min.

Step 3: Preparation of compound **LY-47A**

The compound **LY-47A-b** (21 mg, 1.0 eq) and (6-(2,5-dioxo-2,5-dihydro-1H-pyrrole-1-yl)hexanoyl)glycyl-L-phenylalanine (Chengdu Zen Sunda) (18 mg, 1.0 eq) were dissolved in anhydrous DMF (3 mL), EDCI (11 mg, 1.5 eq) and HOBT (8.22 mg, 1.6 eq) were added, and the reaction solution was stirred at 25°C for reaction for 5 hours. The reaction solution was filtered, and the filtrate was purified by preparative high performance liquid chromatograph (preparative chromatograph manufacturer: ISCO, model: ISCO-R1, column: Phenomenex Luna C18 250 × 50 mm × 10 μm, mobile phase: water (0.225% HCOOH),-acetonitrile, an elution ratio of water: 21% to 51%), to obtain a white solid compound **LY-47A** (7.97 mg, yield: 20.8%).
LCMS: m/z = 1007.5 $[M+H]^+$; Rt = 1.652 min.
$^1$H NMR (400 MHz, DMSO-*d*$^6$) δ 8.57 (t, *J* = 6.7 Hz, 1H), 8.31 (t, *J* = 5.9 Hz, 1H), 8.13 (d, *J* = 8.0 Hz, 1H), 8.07 (t, *J* = 5.8 Hz, 1H), 8.02 (t, *J* = 5.7 Hz, 1H), 7.83 (d, *J* = 12.5 Hz, 1H), 7.29 (s, 1H), 7.22 (s, 3H), 7.28-7.12 (m, 2H), 6.99 (s, 2H), 6.51 (s, 1H), 5.43 (s, 2H), 5.24 (s, 2H), 4.62 (d, *J* = 6.7 Hz, 2H), 4.53-4.43 (m, 1H), 4.26-4.20 (m, 2H), 3.81-3.63 (m, 8H), 3.58 (dd, *J* = 16.7, 5.5 Hz, 1H), 3.14 (t, *J* = 6.1 Hz, 4H), 3.03 (dd, *J* = 13.8, 4.5 Hz, 1H), 2.78 (dd, *J* = 13.8, 9.7 Hz, 1H), 2.06 (dt, *J* = 24.4, 6.8 Hz, 4H), 1.96-1.77 (m, *J* = 7.1 Hz, 3H), 1.46 (h, *J* = 7.5 Hz, 4H), 1.18 (qd, *J* = 9.5, 9.0, 6.0 Hz, 2H), 0.88 (t, *J* = 7.3 Hz, 3H).

Example 64: Preparation of compound **LY-47B**

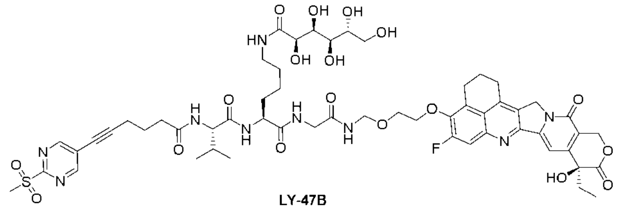

LY-47B

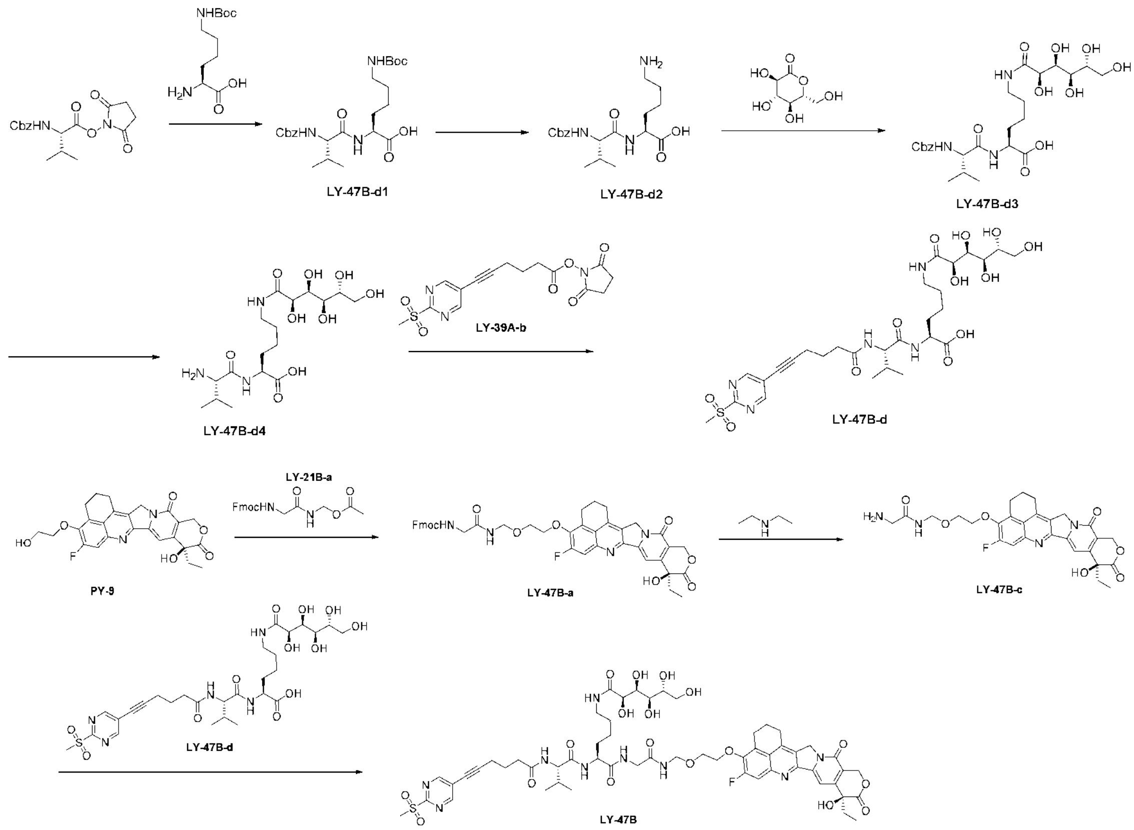

LY-47B-d1 LY-47B-d2 LY-47B-d3 LY-39A-b LY-47B-d4 LY-47B-d LY-21B-a PY-9 LY-47B-a LY-47B-c LY-47B-d LY-47B

Step 1: Preparation of compound **LY-47B-d1**

DIPEA (11.13g, 14.24 mL, 86.13 mmol, 3 eq) was added to a solution of 2,5-dioxopyrrolidin-1-yl((benzyloxy) carbonyl)-L-valine ester (10g, 28.71 mmol, 1 eq) and $N^6$-(tert-butoxycarbonyl)-L-lysine (7.07g, 28.71 mmol, 1 eq) in anhydrous DMF (100 mL) at 0°C, and after addition, the reaction solution was heated to 20°C for reaction for 16 hours. The reaction was concentrated at no more than 40°C to remove most of DMF, and 200 mL of water was added to the residue. The pH of the aqueous solution of the residue was adjusted to 4 with 2N hydrochloric acid at 0°C, then the resulting solution was extracted with ethyl acetate (100 mL × 3), and the organic phases were combined, separately washed with water (300 mL, twice) and saturated brine (100 mL) once, dried with anhydrous sodium sulfate, and filtered, and the filtrate was concentrated under reduced pressure to obtain compound **LY-47B-d1** (15.9g, crude product).

LCMS (ESI): m/z, 502.2[M+Na]$^+$.

Step 2: Preparation of compound **LY-47B-d2**

HCl/dioxane (4M/L, 50 mL) was added to the solution of compound **LY-47B-d1** (8.30g, 17.31 mmol, 1 eq) in anhydrous DCM (60 mL) at 0°C, and after addition, the reaction solution was heated to 20°C for reaction for 2 hours. The reaction solution was concentrated under reduced pressure to obtain compound **LY-47B-d2** (6g, crude product).

LCMS (ESI): m/z, 380.2[M+H]$^+$.

Step 3: Preparation of compound **LY-47B-d3**

(3R,4S,5S,6R)-3,4,5-trihydroxy-6-(hydroxymethyl)tetrahydro-2H-pyrano-2-one (1.26g, 2.10 mL, 7.08 mmol, 2 eq) and triethylamine (1.79g, 2.46 mL, 17.70 mmol, 5 eq) were added to a solution of compound **LY-47B-d2** (3.95g, 34%,

3.54 mmol, 1 eq) in anhydrous MeOH (40 mL) at room temperature, and after addition, the reaction solution was heated to 70°C for reaction for 16 hours. The reaction solution was concentrated to remove most of methanol, 5 mL of water was added, and the reaction solution was purified by preparative high performance liquid chromatograph (preparative chromatograph manufacturer: Oriendo, model: BRIX-2860, column: Phenomenex Luna C18 250 × 50 mm × 10 μm, mobile phase: water (0.225% HCOOH)-acetonitrile, an elution ratio of water: 12% to 42%) and lyophilized, to obtain a white solid compound **LY-47B-d3** (866 mg, yield: 43.88%).

LCMS (ESI): m/z, 558.3[M+H]$^+$.

Step 4: Preparation of compound **LY-47B-d4**

Palladium on carbon (200 mg, 16.64 μL, 1.88 mmol, 1.2101 eq) was added to the solution of compound **LY-47B-d3** (866 mg, 1.55 mmol, 1 eq) in methanol (30 mL) in the hydrogen atmosphere, and the reaction solution was stirred at room temperature for 16 hours. Water (3 mL) was added to the reaction solution, the reaction solution was filtered and concentrated under reduced pressure to obtain solid product **LY-47B-d4** (566 mg, yield: 86%).

LCMS (ESI): m/z, 424[M+H]$^+$.

Step 5: Preparation of compound **LY-47B-d**

N,N-diisopropylethylamine (883.60 μL, 5.35 mmol, 4 eq) was added to a solution of compounds **LY-47B-d4** (566 mg, 1.34 mmol, 1 eq) and **LY-39A-b** (634.85 mg, 1.74 mmol, 1.3 eq) in N,N-dimethylformamide (30 mL). The reaction solution was stirred at room temperature for 2 hours. The reaction solution was purified by preparative high performance liquid chromatograph (preparative chromatograph manufacturer: Oriendo, model: BRIX-2860, column: Phenomenex Luna C18 250 × 30 mm × 10 μm, mobile phase: water (0.225% HCOOH)-acetonitrile, an elution ratio of acetonitrile: 3% to 33%) and lyophilized, to obtain a white solid product **LY-47B-d** (411 mg, yield: 45.6%).

LCMS (ESI): m/z, 674[M+H]$^+$.

Step 6: Preparation of compound **LY-47B-a**

Compound **PY-9** (100 mg, 1.0 eq) and (2-((((9H-fluoren-9-yl)methoxy)carbonyl)amino)acetamide)methyl acetate **(LY-21B-a)** (157.9 mg, 2.0 eq) were dissolved in anhydrous tetrahydrofuran (3 mL), p-toluenesulfonic acid (36.9 mg, 1.0 eq) was added, and then the reaction solution was stirred at 30°C for reaction for 3 hours. After the reaction solution was filtered, the filtrate was purified by preparative high performance liquid chromatograph (preparative chromatograph manufacturer: ISCO, model: ISCO-R1, column: Phenomenex Luna C18 250 × 30 mm × 10 μm, mobile phase: water (0.225% HCOOH)-acetonitrile, an elution ratio of water: 30% to 60% to obtain compound **LY-47B-a** (20 mg, yield: 12.1%).

LCMS: m/z = 775.3 [M+H]$^+$; Rt = 2.118 min.

Step 7: Preparation of compound **LY-47B-c**

The compound **LY-47B-a** (20 mg, 1.0 eq) was dissolved in anhydrous DMF (1 mL), diethylamine (26.6 μL, 10.0 eq) was added, and the reaction solution was stirred at 25°C for reaction for 2 hours. The reaction solution was concentrated under reduced pressure to obtain compound **LY-47B-c** (14.26 mg, crude product) to be directly used for the next reaction.

LCMS: m/z = 553.3 [M+H]$^+$; Rt = 1.104 min.

Step 8: Preparation of compound **LY-47B**

Compound **LY-47B-c** (14.26 mg, 1.0 eq) and compound **LY-47B-d** (20.87 mg, 1.2 eq) were dissolved in anhydrous DMF (5 mL), EDCI (7.42 mg, 1.5 eq) and HOBT (5.58 mg, 1.6 eq) were added, and then the reaction solution was stirred at 25°C for reaction for 3 hours. After the reaction solution was filtered, the filtrate was purified by preparative high performance liquid chromatograph (preparative chromatograph manufacturer: ISCO, model: ISCO-R1, column: Phenomenex Luna C18 250 × 50 mm × 10 μm, mobile phase: water (0.1% TFA),-acetonitrile, an elution ratio of water: 21% to 51%), to obtain a white solid compound **LY-47B** (6.08 mg, yield: 19.5%).

LCMS: m/z = 1208.6 [M+H]$^+$; Rt = 1.363 min.

Example 65: Preparation of compound **LY-47C**

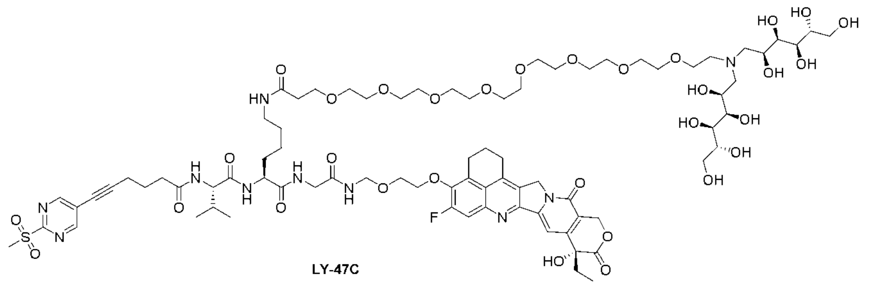

LY-47C

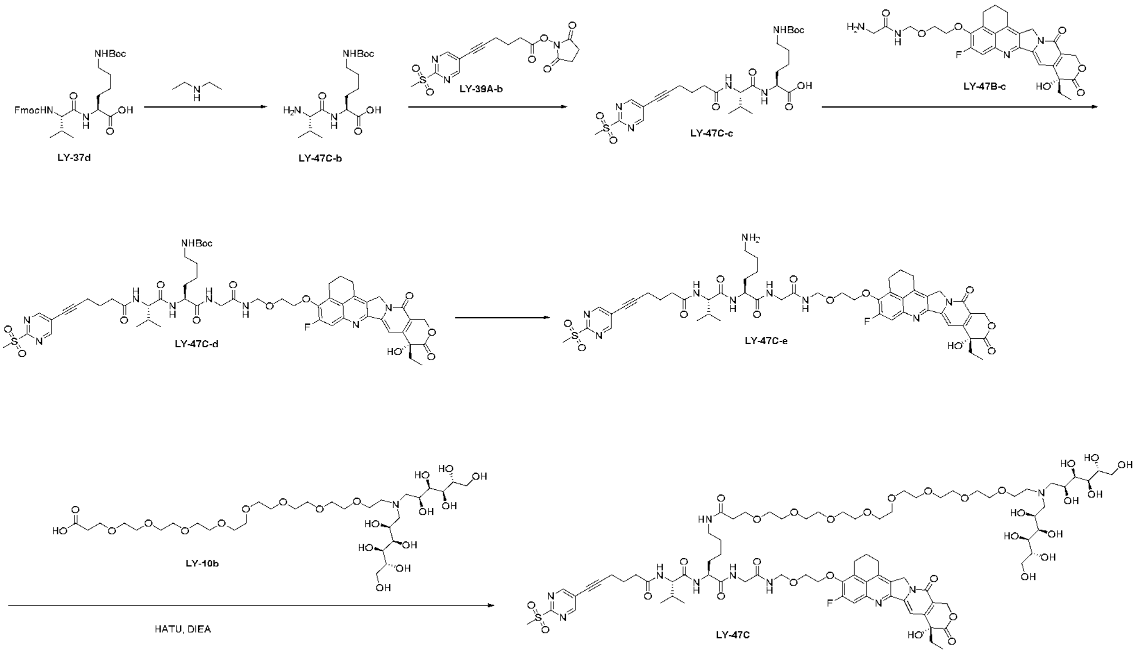


Step 1: Preparation of compound **LY-47C-b**

Diethylamine (25.77 mg, 36.29 μL, 352.31 μmol, 10 eq) was added to the solution of compound **LY-37d** (20 mg, 35.23 μmol, 1 eq) in DMF (2 mL). After addition, the reaction solution reacted at 25°C for 6 hours. The reaction solution was concentrated under reduced pressure, to obtain off-white compound **LY-47C-b** (12 mg, no yield was calculated for the crude product).

LCMS (ESI): m/z, 346.3[M+H]$^+$.

Step 2: Preparation of compound **LY-47C-c**

DIEA (112.24 mg, 143.53 μL, 868.46 μmol, 3 eq) was added to the solution of compound **LY-47C-b** (100 mg, 289.49 μmol, 1 eq) and compound **LY-39A-b** in DMF (2 mL) at 0°C. After addition, the reaction solution was stirred at 25°C for 16 hours. The reaction solution was purified by preparative high performance liquid chromatograph (preparative chromatograph manufacturer: Oriendo, model: BRIX-2860, column: Welch Xtimate C18 250 × 30 mm × 10 μm, mobile phase: water (0.225% FA)-acetonitrile, an elution ratio of water: 21% to 51%) and lyophilized, to obtain a white solid compound **LY-47C-c** (100 mg, yield: 57.99%).

LCMS (ESI): m/z, 618.2[M+H]$^+$.

Step 3: Preparation of compound **LY-47C-d**

HOBt (8.31 mg, 5.54 μL, 61.53 μmol, 1.7 eq) and EDCI (10.41 mg, 11.87 μL, 54.29 μmol, 1.5 eq) were added to the solution of compounds **LY-47C-c** (21.56 mg, 36.20 μmol, 1 eq) and **LY-47B-c** (20 mg, 36.20 μmol, 1 eq) in DMF (2 mL) at 0°C. After addition, the reaction solution was stirred at 25°C for 2 hours. The reaction solution was purified by preparative high performance liquid chromatograph (preparative chromatograph manufacturer: Oriendo, model: BRIX-2860, column: Synergi Max-RP 250 × 40 mm × 10 μm, mobile phase: water (0.225% FA)-acetonitrile, an elution ratio of water: 30% to

60%) and lyophilized, to obtain a white solid compound **LY-47C-d** (22 mg, yield: 53.78%).
LCMS (ESI): m/z, 1152.3[M+Na]$^+$.

Step 4: Preparation of compound **LY-47C-e**

TFA (0.9 mL) was added to the solution of compound **LY-47C-d** (22 mg, 19.46 μmol, 1 eq) in DCM (9 mL) at 0°C. After addition, the reaction solution was stirred at 0°C-10°C for 3 hours. The reaction solution was concentrated under reduced pressure, then water and acetonitrile were added, and the reaction solution was lyophilized to obtain the white solid compound **LY-47C-e** (23 mg, yield: 82.62%, purity: 80%).
LCMS (ESI): m/z, 1052.3[M+H]$^+$.

Step 5: Preparation of compound **LY-47C**

DIPEA (12.04 mg, 15.40 μL, 93.19 μmol, 6 eq) and HATU (11.81 mg, 31.06 μmol, 2 eq) were added to the solution of compound **LY-10** (35.87 mg, 46.60 μmol, 3 eq) and compound **LY-47C-e** (20 mg, 80%, 15.53 μmol, 1 eq) in DMF (10 mL). After addition, the reaction solution reacted at 20°C for 2 hours. The reaction solution was purified by preparative high performance liquid chromatograph (preparative chromatograph manufacturer: Oriendo, model: BRIX-2860, column: Phenomenex Luna C18 250 × 30 mm × 10 μm, mobile phase: water (0.225% FA)-acetonitrile, an elution ratio of water: 21% to 51%) and lyophilized, to obtain a white solid compound **LY-47C** (10.88 mg, yield: 34.18%).
LCMS (ESI): m/z, 1803.6 [M+Na]$^+$, 891.4[1/2M+1]$^+$. HPLC: Rt = 3.924 min.

Example 66: Preparation of compound **LY-47D**

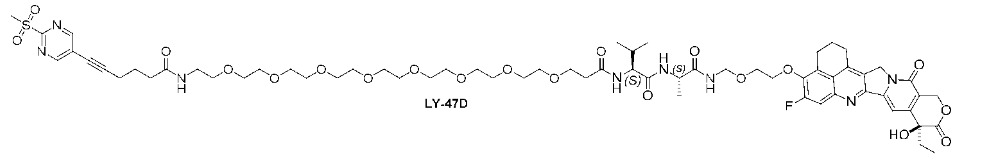

LY-47D

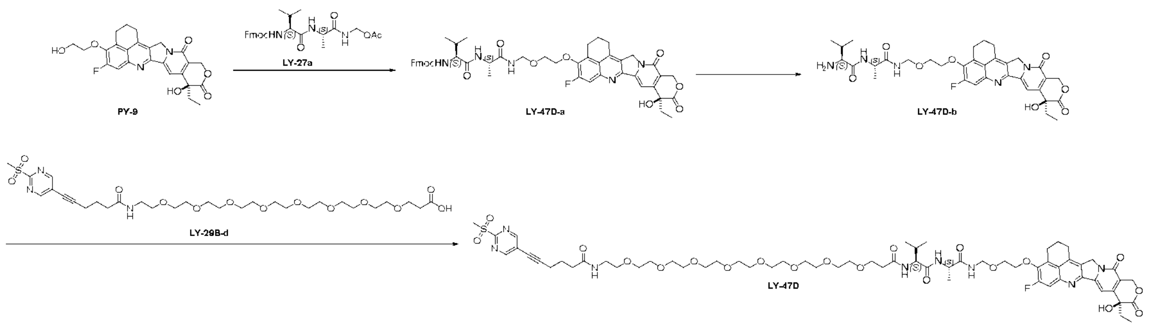


Step 1: Preparation of compound **LY-47D-a**

P-toluenesulfonic acid (18.09 mg, 105.04 μmol, 1 eq) was added to a solution of compound **PY-9** (50 mg, 105.04 μmol, 1 eq) and compound **LY-27a** (505.84 mg, 1.05 μmol, 10 eq) in tetrahydrofuran (10 mL) and the reaction solution was stirred at room temperature for 15 hours. After being concentrated, the reaction solution was dissolved in N,N-dimethylformamide and filtered, and the filtrate was purified by high performance liquid chromatograph (preparative chromatograph manufacturer: SHIMADZU, model: LC-20AP, column: PWelch Xtimate C18 250 × 30 mm × 10 μm, mobile phase: water (0.225% HCOOH)-acetonitrile, an elution ratio of acetonitrile: 39% to 69%), to obtain a product **LY-47D-a** (43.7 mg, yield = 46.8%).
LCMS (ESI): m/z, 888[M+H]$^+$.

Step 2: Preparation of compound **LY-47D-b**

Diethylamine (25.35 μL, 246.07 μmol, 5 eq) was added to the solution of compound **LY-47D-a** (43.7 mg, 49.21 μmol, 1 eq) in N,N-dimethylformamide (5 mL), and the reaction solution was stirred at room temperature for 2 hours. The reaction solution was concentrated under reduced pressure to obtain the crude product (41 mg, no yield was calculated for

the crude product).

LCMS (ESI) m/z, 666[M+H]$^+$.

Step 3: Preparation of compound **LY-47D**

N,N-diisopropylethylamine (19.08 mg, 147.6 μmol, 3 eq) and N,N,N',N'-tetramethyl-O-(7-azabenzotriazole-1-yl) hexafluorophosphate (22.5 mg, 59.04 μmol, 1.2 eq) were added to the solution of compounds **LY-47D-b** (41 mg, 49.2 μmol, 1 eq, purity: 79.9%) and **LY-29B-d** (40.8 mg, 59.04 μmol, 1.2 eq) in N,N-dimethylformamide (3 mL), and the reaction solution was stirred at room temperature for 2 hours. The reaction solution was purified by preparative high performance liquid chromatograph (preparative chromatograph manufacturer: SHIMADZU, model: LC-20AP, column: Global 30 mm × 250 mm, mobile phase: water (0.225% HCOOH)-acetonitrile, an elution ratio of acetonitrile: 30% to 60%), to obtain a white solid compound **LY-47D** (11.17 mg, yield = 19.3%).

LCMS (ESI): m/z, 1361[M+Na]$^+$.

Example 67: Preparation of compound **LY-47E**

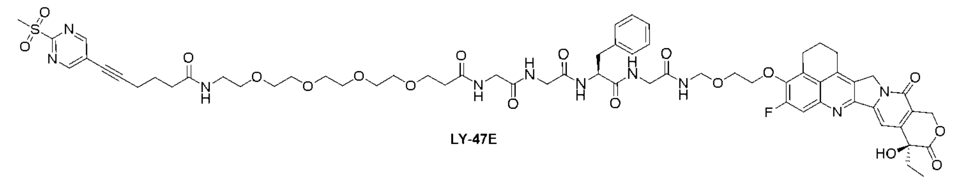

LY-47E

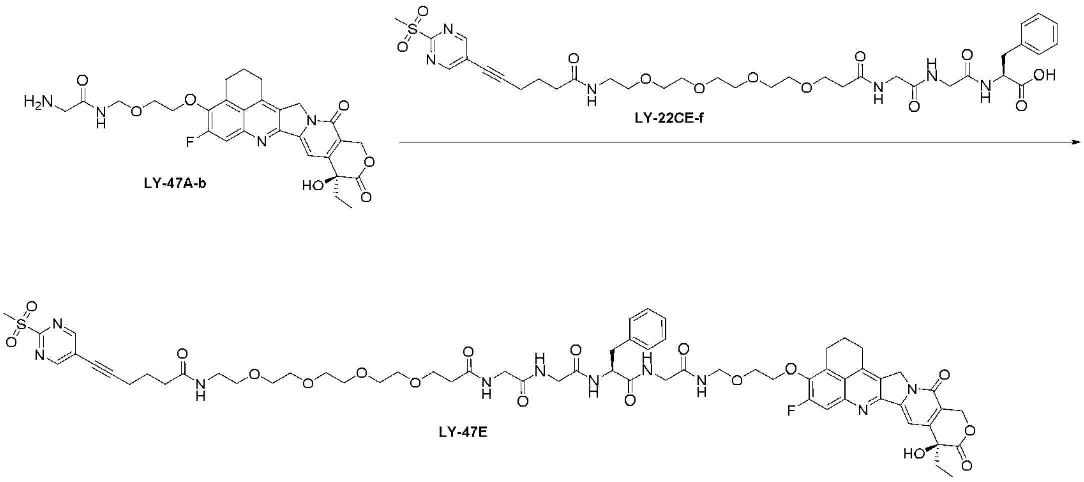

LY-47A-b

LY-22CE-f

LY-47E

The compounds **LY-47A-b** (20 mg, 1.0 eq) and **LY-22CE-f** (53.43 mg, purity: 50%) were dissolved in anhydrous DMF (2 mL), the reaction solution was cooled to 0°C in the ice water bath, DIPEA (17 μL, 3.0 eq) and HATU (17 mg, 1.3 eq) were added, and then the reaction solution was naturally warmed to room temperature and stirred for reaction for 4 hours. The reaction solution was filtered, and the filtrate was purified by preparative high performance liquid chromatograph (preparative chromatograph manufacturer: SHIMADZU, model: LC-20AP, column: GS-120-10-C18AP, mobile phase: water (0.225% HCOOH),-acetonitrile, an elution ratio of water: 21% to 51%), to obtain a white solid compound **LY-47E** (4.73 mg, yield: 10.5%).

LCMS: m/z = 1333.5 [M+Na]$^+$; Rt = 1.583 min.

Example 68: Preparation of compound **LY-47F**

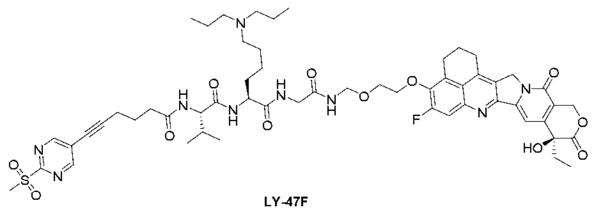

LY-47F

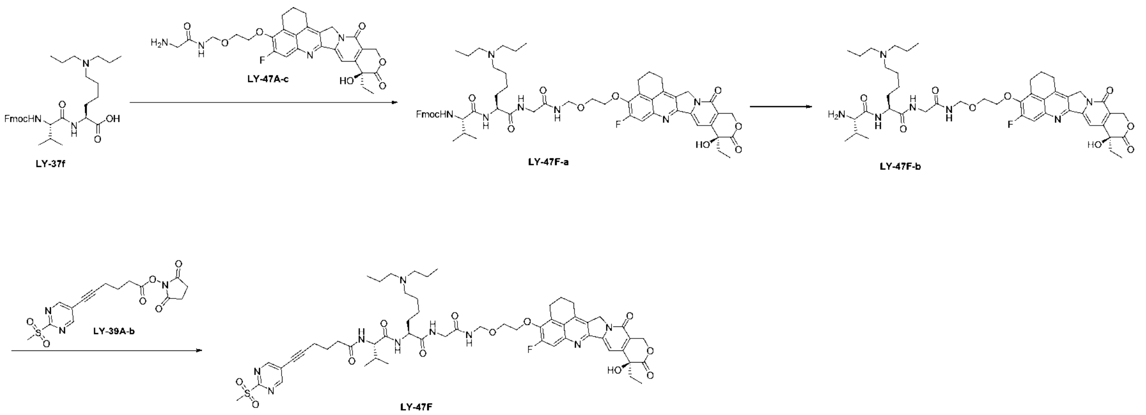

LY-37f

LY-47A-c

LY-47F-a

LY-47F-b

LY-39A-b

LY-47F

Step 1: Preparation of compound **LY-47F-a**

The compounds **LY-37f** (35.57 mg, 1.5 eq) and **LY-47A-c** (25 mg, 1.0 eq) were dissolved in anhydrous DMF (2 mL), the reaction solution was cooled to 0°C, HATU (21.25 mg, 1.3 eq) and DIPEA (21.3 μL, 3.0 eq) were added, and then the reaction solution was heated to 25°C and stirred for reaction for 16 hours. After the reaction solution was filtered, the filtrate was subjected to conventional reversed-phase preparative chromatography (mobile phase: water (0.225% HCOOH)-acetonitrile, an elution ratio of water: 10% to 50%), to obtain compound **LY-47F-a** (30 mg, yield: 64.3%).

LCMS: m/z = 1086.6 $[M+H]^+$; Rt = 2.033 min.

Step 2: Preparation of compound **LY-47F-b**

The compound **LY-47F-a** (30 mg, 1.0 eq) was dissolved in anhydrous DMF (2 mL), diethylamine (28.45 μL, 10.0 eq) was added at room temperature, namely, 25°C, and then the reaction solution was stirred for reaction for 2 hours. The reaction solution was directly concentrated to dryness to obtain the crude product (20 mg, yield: 83.8%) to be directly used for the next reaction.

LCMS: m/z = 864.5 $[M+H]^+$; Rt = 1.144 min.

Step 3: Preparation of compound **LY-47F**

The compounds **LY-47F-b** (20 mg, 1.0 eq) and **LY-39A-b** (13.19 mg, 1.3 eq) were dissolved in anhydrous DMF (5 mL), the reaction solution was cooled to 0°C, DIPEA (13.77 μL, 3.0 eq) was added dropwise, and then the reaction solution was heated to 25°C and stirred for reaction for 2 hours. The reaction solution was filtered, and the filtrate was subjected to preparative high performance liquid chromatograph (preparative chromatograph manufacturer: Oriendo, model: BRIX-2860, column: Phenomenex Luna C18 150 × 25 mm × 10 μm, mobile phase: water (0.225% HCOOH),-acetonitrile, an elution ratio of water: 20% to 50%), to obtain a white solid compound **LY-47F** (2.66 mg, yield: 8.6%).

LCMS: m/z = 1114.5 $[M+Na]^+$; Rt = 1.559 min.

Example 69: Preparation of compound **LY-48A**

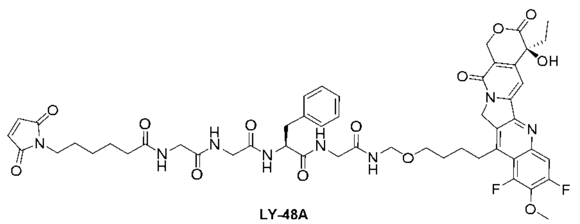

LY-48A

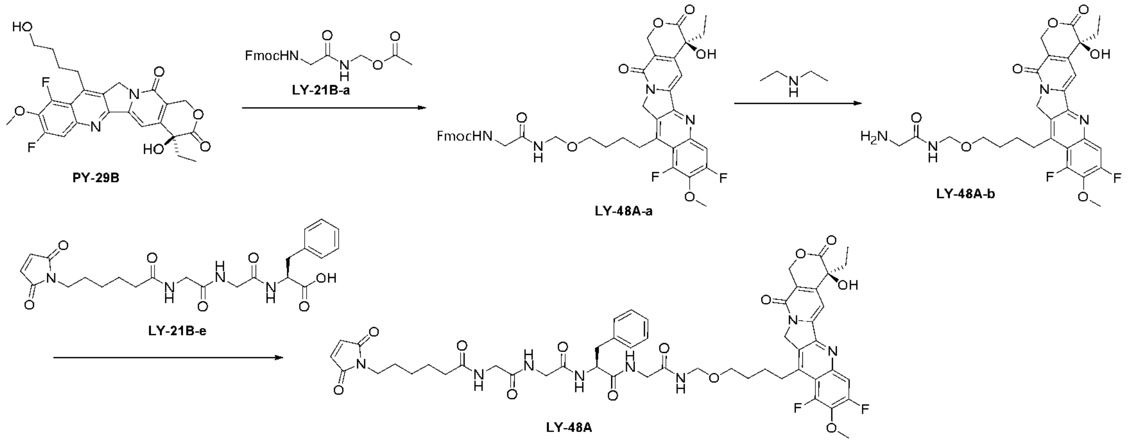


**Step** 1: Preparation of compound **LY-48A-a**

Compound **LY-21B-a** (34.83 mg, 94.56 μmol, 2 eq) and p-toluenesulfonic acid (8.14 mg, 7.61 μL, 47.28 μmol, 1 eq) were added to the solution of compound **PY-29B** (23 mg, purity: 95%; 47.28 μmol, 1 eq) in 3 mL of tetrahydrofuran respectively, and the reaction solution was stirred at 25°C for 16 hours. The reaction solution was concentrated to obtain 37.5 mg of crude product of compound **LY-48A-a** (purity: 48%) to be directly used for the next reaction.
LCMS (ESI): m/z, 795.3 $[M+H]^+$.

Step 2: Preparation of compound **LY-48A-b**

Diethylamine (16.56 mg, 23.33 μL, 226.47 μmol, 10 eq) was added to the solution of crude product of compound **LY-48A-a** (37.50 mg, purity: 48%, 22.65 μmol, 1 eq) in 3 mL of DMF solvent, and after addition, the reaction solution was stirred at 25°C for 16 hours. The reaction solution was purified by preparative high performance liquid chromatograph (preparative chromatograph manufacturer: SHIMADZU, model: LC-20AP, column: YMC-Triart Prep C18 250 × 30 mm × 10 μm, mobile phase: water (0.225% HCOOH)-acetonitrile, an elution ratio of water: 12% to 42%) and lyophilized, to obtain a light yellow solid compound **LY-48A-b** (1.8 mg, yield: 14%).
LCMS (ESI): m/z, 573.2 $[M+H]^+$.

Step 3: Preparation of compound **LY-48A**

Compound **LY-21B-e** (Chengdu Zen Sunda) (9.61 mg, 20.33 μmol, 1.2 eq), EDCI (5.55 μL, 25.41 μmol, 1.5 eq) and HOBt (3.66 mg, 2.44 μL, 27.11 μmol, 1.6 eq) were added to the solution of compound **LY-48A-b** (9.70 mg, 16.94 μmol, 1 eq) in 2 mL of DMF solvent respectively, and the reaction solution was stirred at 25°C for 16 hours. The reaction solution was purified by preparative high performance liquid chromatograph (preparative chromatograph manufacturer: SHIMADZU, model: LC-20AP, column: YMC-Triart Prep C18 250 × 30 mm × 10 μm, mobile phase: water (0.225% HCOOH)-acetonitrile, an elution ratio of water: 12% to 42%) and lyophilized, to obtain 1.21 mg of white solid compound **LY-48A** (yield: 7%).
LCMS: m/z = 1027.3 $[M+H]^+$; Rt = 1.929 min.

Example 70: Preparation of compound **LY-48B**

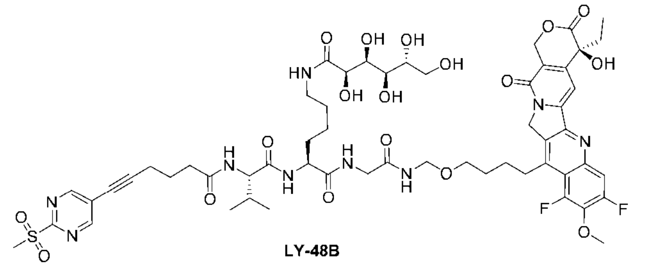

LY-48B

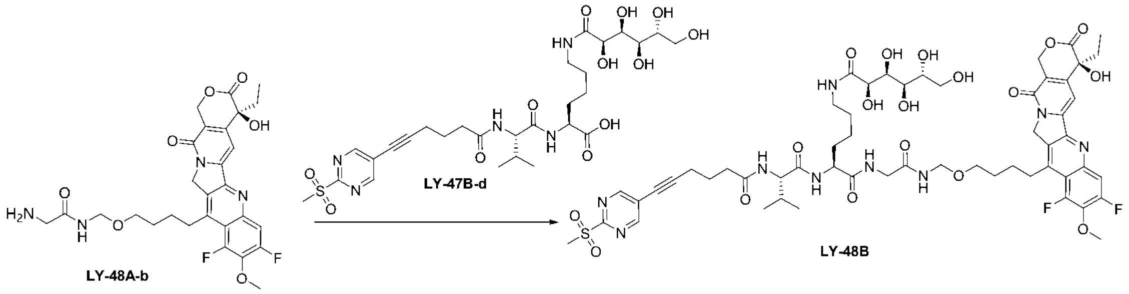

LY-48A-b

LY-47B-d

LY-48B

**LY-47B-d** (2.54 mg, 3.77 μmol, 1.2 eq), EDCI (903.98 μg, 4.72 μmol, 1.5 eq) and HOBt (679.65 μg, 5.03 μmol, 1.6 eq) were added to the solution of compound **LY-48A-b** (1.80 mg, 3.14 μmol, 1 eq) in 1 mL of DMF solvent respectively at 0°C. After addition, the reaction solution was stirred at 25°C for 16 hours. The reaction solution was purified by preparative high performance liquid chromatograph (preparative chromatograph manufacturer: SHIMADZU, model: LC-20AP, column: YMC-Triart Prep C18 250 × 30 mm × 10 μm, mobile phase: water (0.225% HCOOH)-acetonitrile, an elution ratio of water: 12% to 42%) and lyophilized, to obtain a white solid compound **LY-48B** (1.50 mg, yield: 39%).

LCMS (ESI): m/z, 1228.3 $[M+H]^+$; Rt= 1.625 min.

Example 71: Preparation of compound **LY-48C**

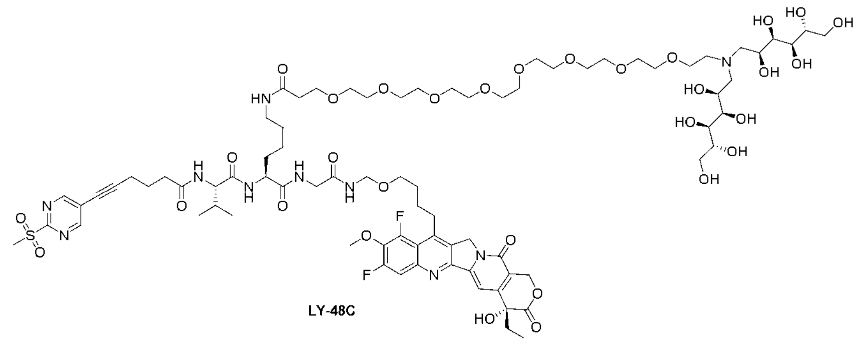

LY-48C

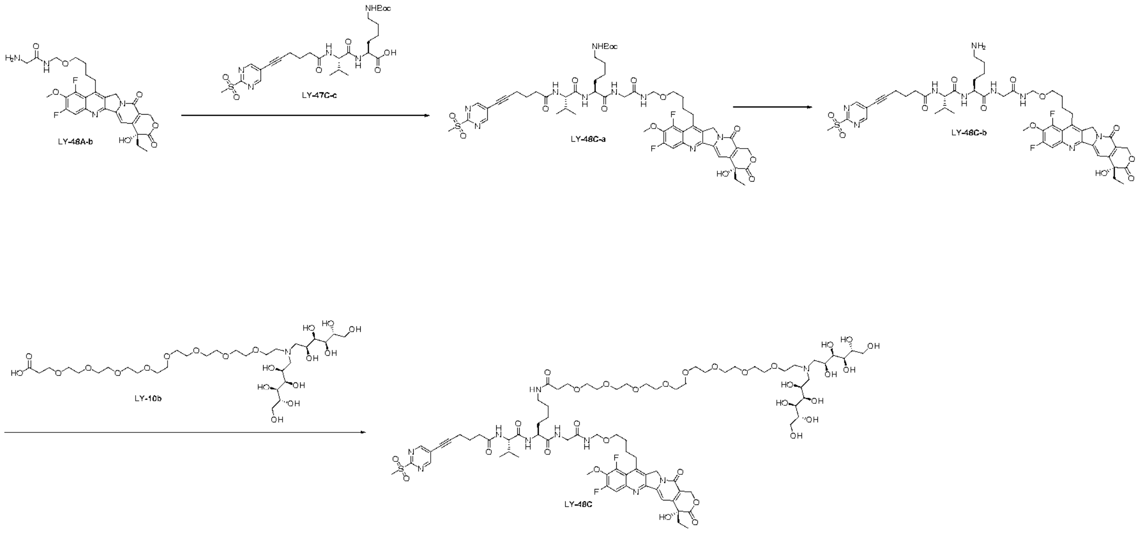

LY-48A-b LY-47C-c LY-48C-a LY-48C-b LY-10b LY-48C

Step 1: Preparation of compound **LY-48C-a**

HOBt (1.89 mg, 1.26 μL, 13.97 μmol, 1.6 eq) and EDCI (2.51 mg, 2.86 μL, 13.10 μmol, 1.5 eq) were added to the solution of compounds **LY-47C-c** (6.24 mg, 10.48 μmol, 1.2 eq) and **LY-48A-b** (5 mg, 8.73 μmol, 1 eq) in 2mL of DMF solvent. After addition, the reaction solution was stirred 25°C for 3 hours. The reaction solution was purified by preparative high performance liquid chromatograph (preparative chromatograph manufacturer: Oriendo, model: BRIX-2860, column: Welch Xtimate C18 250 × 30 mm × 10 μm, mobile phase: water (0.225% HCOOH)-acetonitrile, an elution ratio of water: 39% to 69%) and lyophilized, to obtain a white solid compound **LY-48C-a** (6 mg, yield: 59.73%).

LCMS (ESI): m/z, 1172.4[M+Na]$^+$.

Step 2: Preparation of compound **LY-48C-b**

TFA (0.4 mL) was added to the solution of compound **LY-48C-a** (6 mg, 5.22 μmol, 1 eq) in DCM (4 mL) at 0°C. After addition, the reaction solution was stirred at 0°C-10°C for 2 hours. The reaction solution was concentrated at no more than 35°C, then water and acetonitrile were added, and the reaction solution was lyophilized to obtain light yellow solid compound **LY-48C-b** (6 mg, yield of crude product: 98.81%).

LCMS (ESI): m/z, 1050.4[M+H]$^+$.

Step 3: Preparation of compound **LY-48C**

DIPEA (3.57 mg, 4.57 μL, 27.66 μmol, 7 eq) and HATU (3.76 mg, 9.88 μmol, 2.5 eq) were added to the solution of compound **LY-10b** (9.13 mg, 11.85 μmol, 3 eq) and compound **LY-48C-b** (4.60 mg, 3.95 μmol, 1 eq) in DMF (2 mL). After addition, the reaction solution reacted at 20°C for 2 hours. The reaction solution was purified by preparative high performance liquid chromatograph (preparative chromatograph manufacturer: Oriendo, model: BRIX-2860, column: Welch Xtimate C18 250 × 30 mm × 10 μm, mobile phase: water (0.1% TFA)-acetonitrile, an elution ratio of water: 21% to 51%) and lyophilized, to obtain a white solid compound **LY-48C** (4.98 mg, yield: 53.80%).

LCMS (ESI): m/z, 1801.7 [M+Na]$^+$, 902[1/2M+1]$^+$. HPLC: Rt = 4.298 min.

Example 72: Preparation of compound **LY-48D**

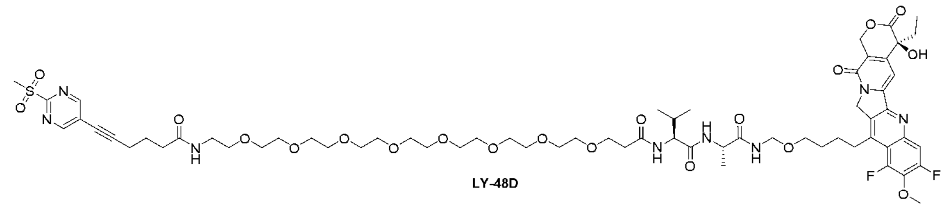

LY-48D

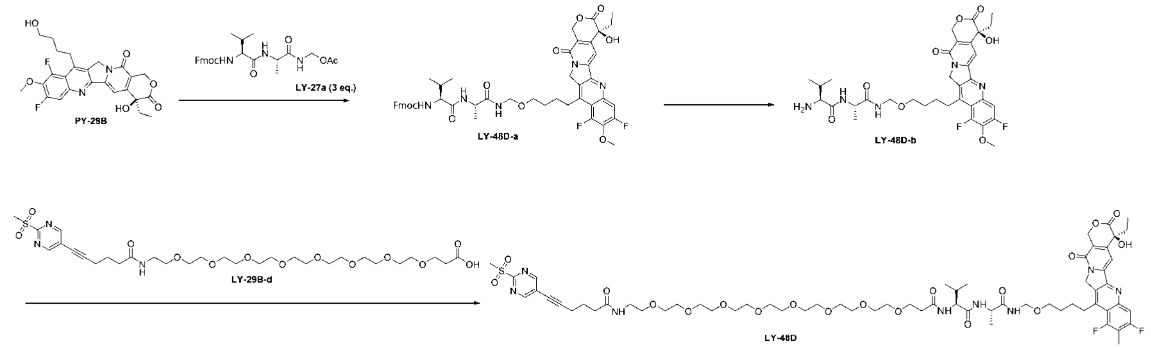


Step 1: Preparation of compound **LY-48D-a**

TsOH (159 mg, 326.84 μmol, 1 eq) was added to a solution of compound **PY-29B** (159 mg, 326.84 μmol, 1 eq) and compound LY-27a (524.64 mg, 90%, 980.53 μmol, 3 eq) in THF (30 mL) and the mixture was stirred at 25°C for 2 hours. The mixture was concentrated under reduced pressure to remove the solvent, dissolved in DMF, filtered, and purified by preparative high performance liquid chromatograph (preparative chromatograph manufacturer: Oriendo, model: BRIX-2860, column: Welch Xtimate C18 250 × 30 mm × 10 μm, mobile phase: water (0.225% HCOOH)-acetonitrile, an elution ratio of acetonitrile: 50% to 80%) and lyophilized, to obtain a white solid compound **LY-48D-**a (33.80 mg, yield: 38.12%).

LCMS (ESI): m/z, 930[M+Na]$^+$.

Step 2: Preparation of compound **LY-48D-b**

$Et_2NH$ (13.61 mg, 19.17 μL, 186.13 μmol, 5 eq) was added to a solution of compound **LY-48D-a** (33.80 mg, 37.23 μmol, 1 eq) in DMF (2 mL), and the mixture was stirred at 25°C for 2 hours. The reaction solution was concentrated under reduced pressure, to obtain a white solid product (36.6 mg, no yield was calculated for the crude product).

LCMS (ESI): m/z, 686[M+H]$^+$.

Step 3: Preparation of compound **LY-48D**

DIPEA (33.92 mg, 43.38 μL, 262.49 μmol, 2 eq) and HATU (74.86 mg, 196.87 μmol, 1.5 eq) were added to a solution of compound **LY-48D-b** (90 mg, 131.25 μmol, 1 eq) and compound **LY-29B-d** (213.64 mg, 85%, 262.49 μmol, 2 eq) in DMF (5 mL) in the ice water bath, and then the mixture was stirred at 25°C for 2 hours. The reactant was purified by preparative high performance liquid chromatograph (preparative chromatograph manufacturer: Oriendo, model: Lab311-DJ-R2, column: GS-120-10-C18AP, mobile phase: water (0.225% HCOOH)-acetonitrile, an elution ratio of acetonitrile: 30% to 60%) and lyophilized, to obtain a white solid product **LY-48D** (3.42 mg, yield: 24.75%, purity: 98%).

LCMS (ESI): m/z, 1381[M+Na]$^+$.

$^1$H NMR (400 MHz, DMSO-*d*$^6$) δ 9.11 (s, 2H), 8.55 (s, 1H), 8.04 (d, *J* = 7.2 Hz, 1H), 7.95 (d, *J* = 12.2 Hz, 2H), 7.82 (d, *J* = 8.7 Hz, 1H), 7.31 (s, 1H), 6.55 (s, 1H), 5.44 (s, 2H), 5.33 (s, 2H), 4.54 (dd, *J* = 12.8, 6.7 Hz, 2H), 4.22 (t, *J* = 7.1 Hz, 1H), 4.18 - 4.13 (m, 1H), 4.09 (s, 3H), 3.56 (t, *J* = 6.5 Hz, 2H), 3.49 (d, *J* = 5.9 Hz, 28H), 3.40 (d, *J* = 2.9 Hz, 10H), 3.20 (d, *J* = 6.3 Hz, 6H), 2.42 (s, 2H), 2.36 (s, 2H), 2.26 (t, *J* = 7.4 Hz, 2H), 1.89-1.84 (m, 2H), 1.80 (t, *J* = 7.3 Hz, 2H), 1.19 (d, *J* = 7.1 Hz, 3H), 0.87 (t, *J* = 7.3 Hz, 3H), 0.79 (dd, *J* = 11.0, 6.7 Hz, 6H).

Example 73: Preparation of compound **LY-48E**

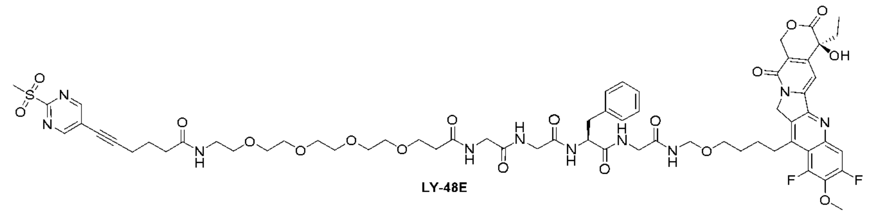

LY-48E

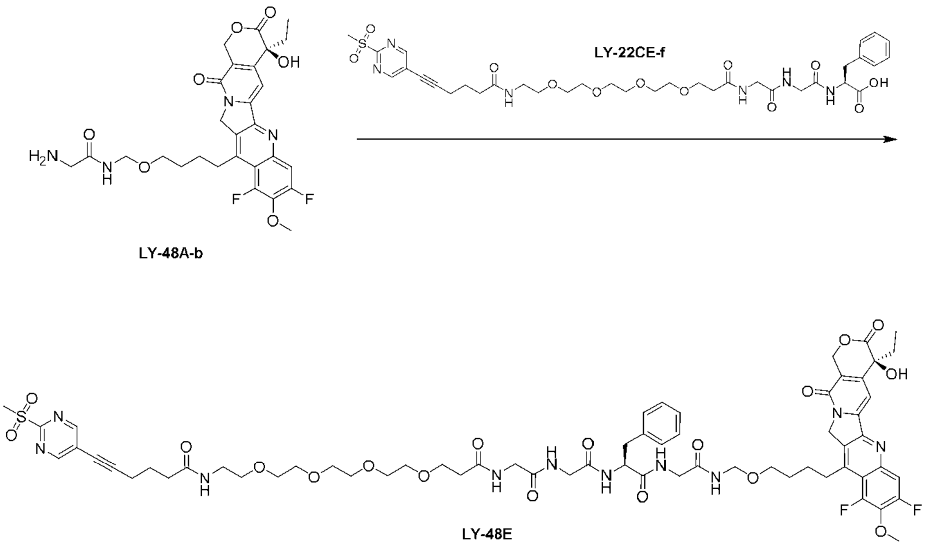

LY-22CE-f

LY-48A-b

LY-48E

Step 1: Preparation of compound LY-48E

The compound **LY-48A-b** (4 mg, 6.99 μmol, 1 eq) was dissolved in DMF (2 mL), **LY-22CE-f** (9.71 mg, 80%, 10 μmol, 1 eq) and DIPEA (1.81 mg, 2.31 μL, 13.97 μmol, 2 eq) were added, finally HATU (4.52 mg, 11.89 μmol, 1.5 eq) was added, and the reaction solution was stirred at 25°C for 1 hour. The reaction solution was purified by preparative high performance liquid chromatograph (preparative chromatograph manufacturer: SHIMADZU, model: LC-20AP, column: Welch Xtimate C18 150 × 21.2 mm × 5 μm, mobile phase: water (0.225% HCOOH)-acetonitrile, an elution ratio of water: 30% to 60%), to obtain a white solid product (1.47 mg, yield: 15.80%, purity: 96%).

LCMS (ESI): m/z, 1353.5 $[M+Na]^+$.

Example 74: Preparation of compound **LY-48F**

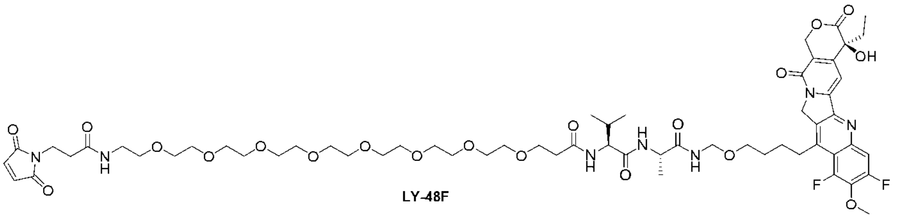

LY-48F

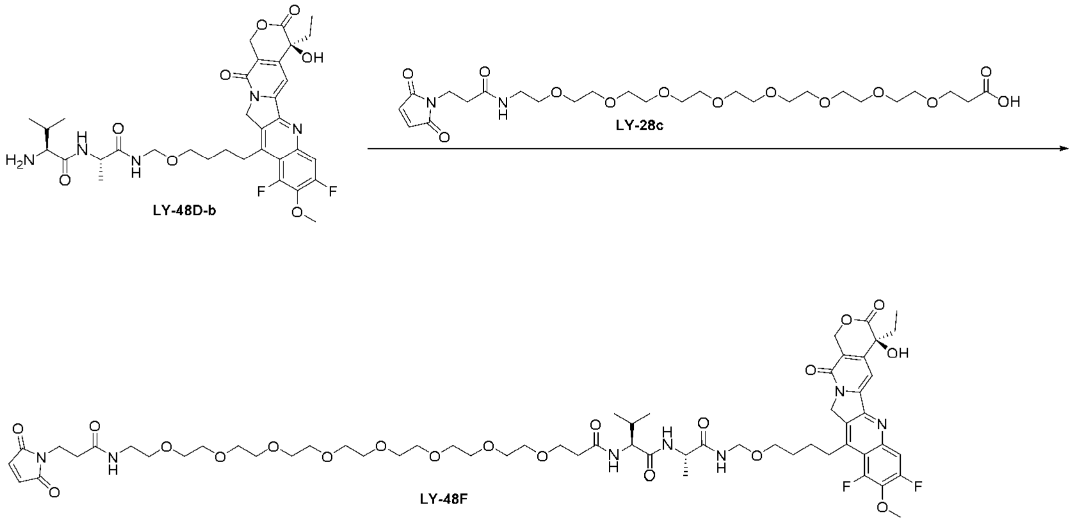

LY-48D-b

LY-28c

LY-48F

HATU (136.06 mg, 357.82 μmol, 1.5 equivalents) was added to compound **LY-48D-b** (163.58 mg, 238.55 μmol, 1 equivalent) and compound **LY-28c** (282.73 mg, 477.10 μmol, 2 equivalents), and the mixture was stirred at 25°C for 2 hours. The reactant was purified by preparative high performance liquid chromatograph (preparative chromatograph manufacturer: Oriendo, model: Lab311-DJ-R2, column: GS-120-10-C18AP, mobile phase: water (0.225% HCOOH)-acetonitrile, an elution ratio of acetonitrile: 30% to 60%) and lyophilized, to obtain a white solid product **LY-48F** (240.60 mg, yield: 80.03%, purity: 88%).

LCMS (ESI): m/z, 1260[M+Na]$^+$.

$^1$H NMR (400 MHz, DMSO-$d_6$) δ 8.56 (t, *J* = 6.6 Hz, 1H), 8.10-7.99 (m, 2H), 7.95 (d, *J* = 11.5 Hz, 1H), 7.83 (d, *J* = 8.7 Hz, 1H), 7.31 (s, 1H), 7.00 (s, 1H), 6.55 (s, 1H), 5.44 (s, 2H), 5.32 (s, 2H), 4.61-4.47 (m, 2H), 4.22 (t, *J* = 7.2 Hz, 1H), 4.15 (dd, *J* = 8.7, 6.6 Hz, 1H), 4.09 (s, 3H), 3.62-3.53 (m, 4H), 3.52-3.38 (m, 34H), 3.16 (dd, *J* = 14.3, 8.5 Hz, 5H), 2.42 (s, 2H), 2.33 (td, *J* = 8.3, 6.6 Hz, 3H), 1.87 (d, *J* = 7.1 Hz, 4H), 1.19 (d, *J* = 7.1 Hz, 3H), 0.87 (t, *J* = 7.3 Hz, 3H), 0.79 (dd, *J* = 11.0, 6.8 Hz, 6H).

Example 75: Preparation of compound **LY-49B**

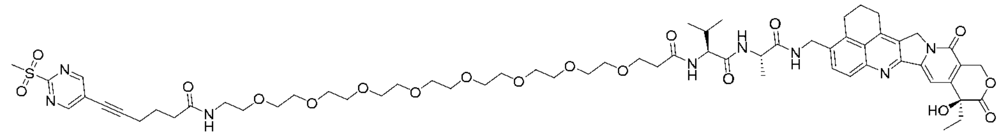

LY-49B

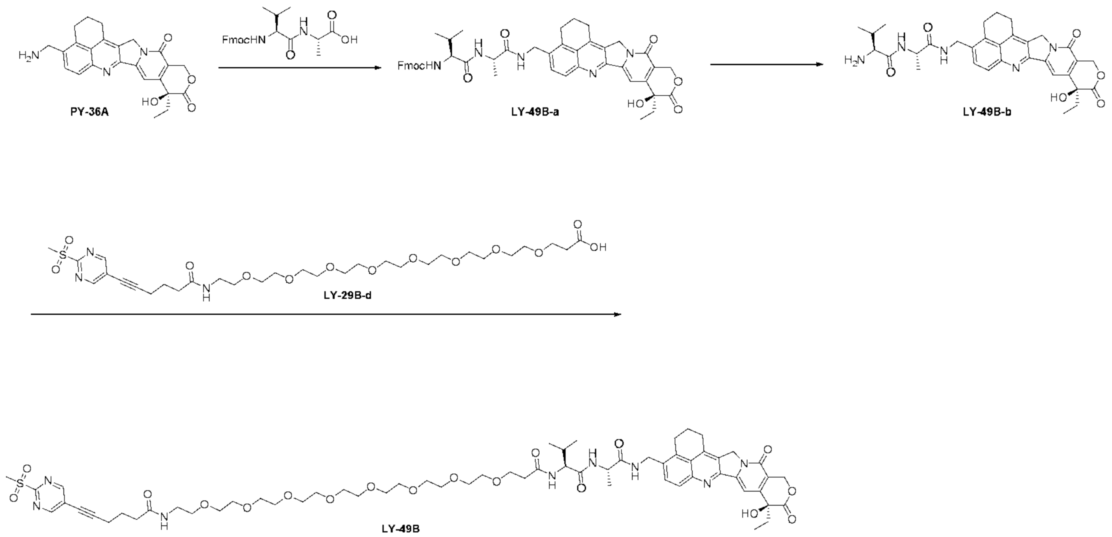


PY-36A LY-49B-a LY-49B-b

LY-49B

Step 1: Preparation of compound **LY-49B-a**

N,N-diisopropylethylamine (3.96 μL, 23.95 μmol, 2 eq) and N,N,N',N'-tetramethyl-O-(7-azabenzotriazole-1-yl) hexafluorophosphate (5.46 mg, 14.37 μmol, 1.2 eq) were added to the solution of compound **PY-36A** (5 mg, 11.98 μmol, 1 eq) and (((9H-fluoren-9-yl)methoxy)carbonyl)-L-valine-L-alanine (4.92 mg, 11.98 μmol, 1 eq) in N,N-dimethylformamide (1.5 mL), and the reaction solution was stirred at room temperature for 2 hours. The reaction solution was purified by preparative high performance liquid chromatograph (preparative chromatograph manufacturer: SHIMADZU, model: LC-20AP, column: XT-15*30-10, mobile phase: water (0.225% HCOOH)-acetonitrile, an elution ratio of acetonitrile: 30% to 60%), to obtain a product **LY-49B-a** (6.1 mg, yield = 62.88%).

LCMS (ESI): m/z, 810[M+H]$^+$.

Step 2: Preparation of compound **LY-49B-b**

Diethylamine (3.88 μL, 37.66 μmol, 5 eq) was added to the solution of compound **LY-49B-a** (6.1 mg, 7.53 μmol, 1 eq) in N,N-dimethylformamide (1 mL), and the reaction solution was stirred at room temperature for 2 hours. The reaction solution was concentrated under reduced pressure to obtain the crude product of compound **LY-49B-b** (7 mg).

LCMS (ESI): m/z, 588[M+H]$^+$.

Step 3: Preparation of compound **LY-49B**

N,N-diisopropylethylamine (2.49 μL, 15.08 μmol, 2 eq) and N,N,N',N'-tetramethyl-O-(7-azabenzotriazole-1-yl) hexafluorophosphate (3.44 mg, 9.05 μmol, 1.2 eq) were added to the solution of compounds **LY-49B-b** (4.43 mg, 7.54 μmol, 1 eq) and **LY-29B-d** (11.78 mg, 14.47 μmol, 1.1 eq) in N,N-dimethylformamide (1 mL), and then the reaction solution was stirred at room temperature for 2 hours. The reaction solution was purified by preparative high performance liquid chromatograph (preparative chromatograph manufacturer: SHIMADZU, model: LC-20AP, column: YMC-Triart Prep C18 150 × 30 mm × 10 μm, mobile phase: water (0.225% HCOOH)-acetonitrile, an elution ratio of acetonitrile: 20% to 50%), to obtain a product **LY-49B** (2.93 mg, yield = 30.3%).

LCMS (ESI): m/z, 1261[M+H]$^+$.

Example 76: Preparation of compound **LY-50A**

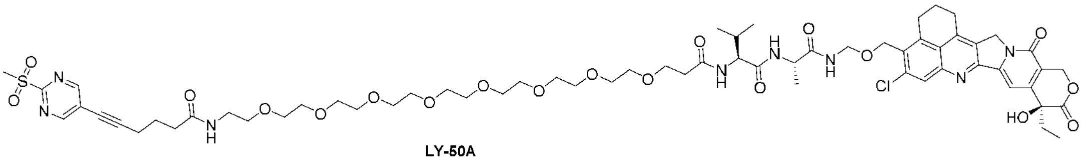

LY-50A

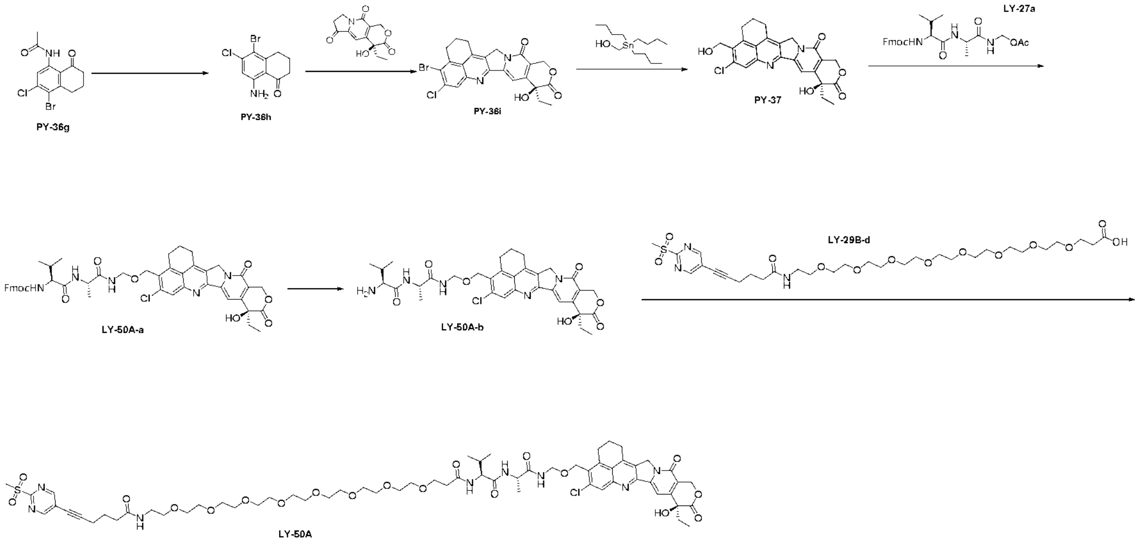


PY-36g PY-36h PY-36i PY-37 LY-50A-a LY-50A-b LY-50A

The preparation method was the same as that in Example 75, except that the compound PY-36A was replaced with the compound PY-37 and the compound (((9H-fluoren-9-yl)methoxy)carbonyl)-L-valine-L-alanine was replaced with the compound LY-27a to prepare the compound **LY-50A.**

LCMS (ESI): m/z, 1347.5[M+Na]$^+$, Rt = 1.687 min; HPLC: Rt = 5.52 min.

Example 77: Preparation of compound **LY-50B**

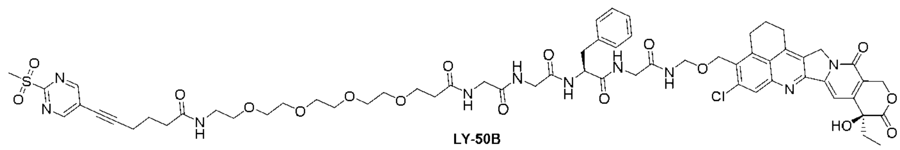

LY-50B

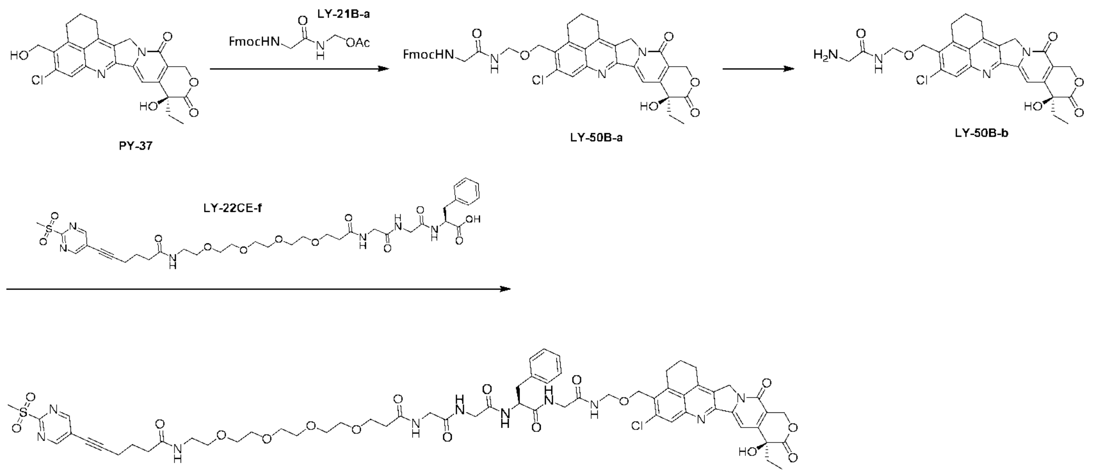


PY-37 LY-50B-a LY-50B-b

LY-50B

The preparation method was the same as that in Example 76, except that the compound LY-27a was replaced with the compound LY-21B-a and the compound LY-29B-d was replaced with the compound LY-22CE-f to prepare the

compound **LY-50B.**

LCMS (ESI): m/z, 1297.4 [M+H]+.

Example 78: Preparation of compound **LY-51A**

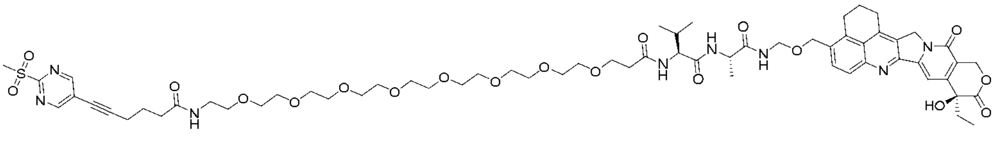

LY-51A

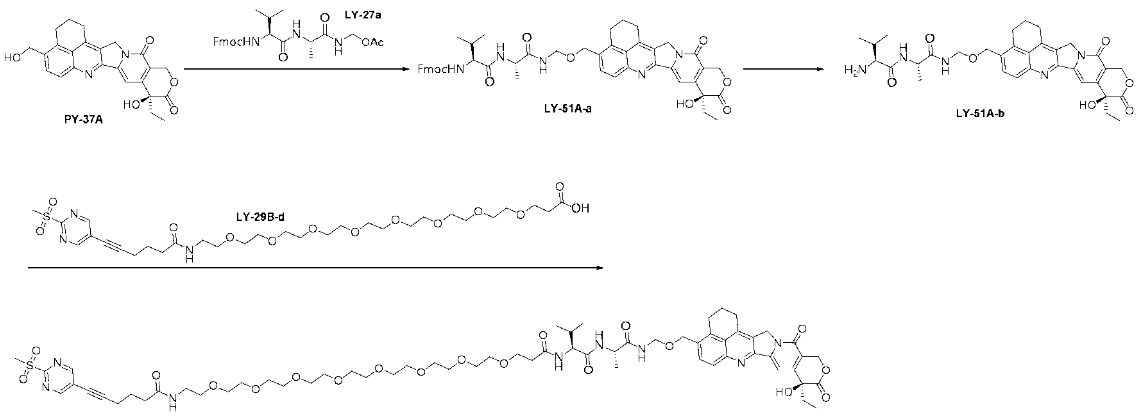


LY-51A

The preparation method was the same as that in Example 76, except that the compound PY-37 was replaced with the compound PY-37A to prepare the compound **LY-51A.**

LCMS: m/z = 1291.6 [M+H]+; Rt = 1.538 min.

Example 79: Preparation of compound **LY-51B**

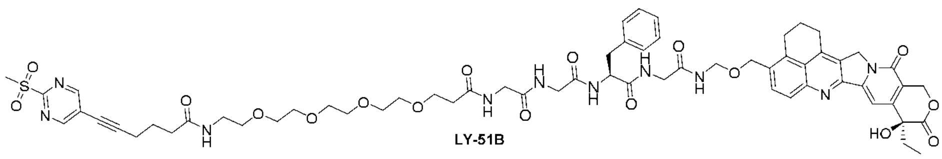

LY-51B

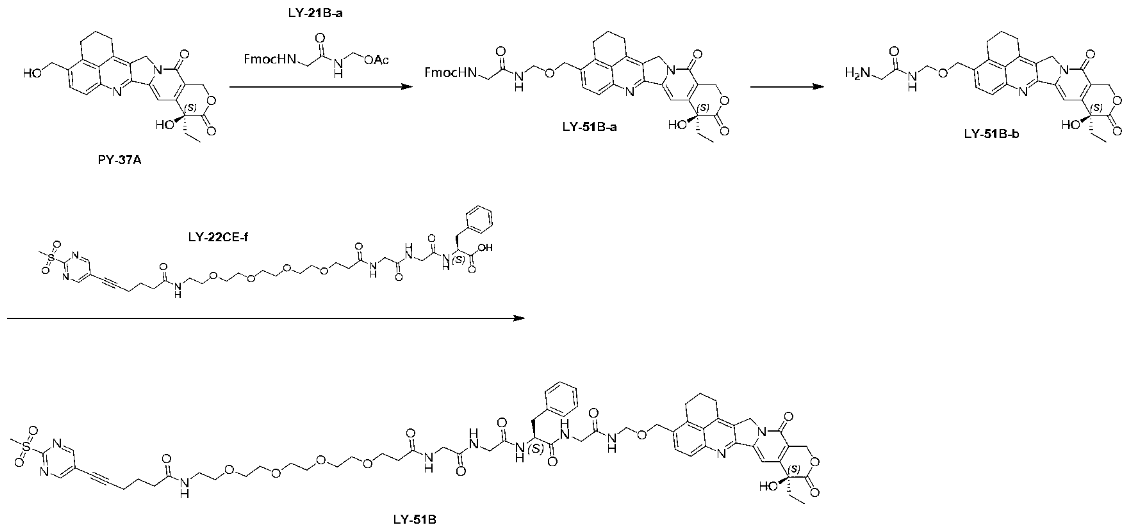


The preparation method was the same as that in Example 77, except that the compound PY-37 was replaced with the compound PY-37A to prepare the compound **LY-51B.**

LCMS (ESI): m/z, 1285.6 [M+Na]$^+$.

Example 80: Preparation of compound **BY-1B**

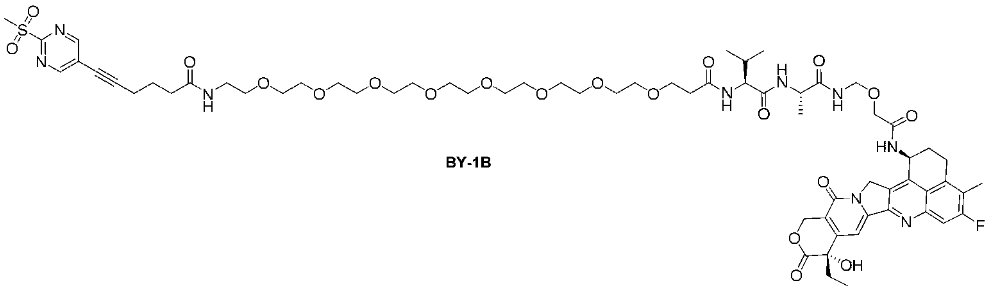

BY-1B

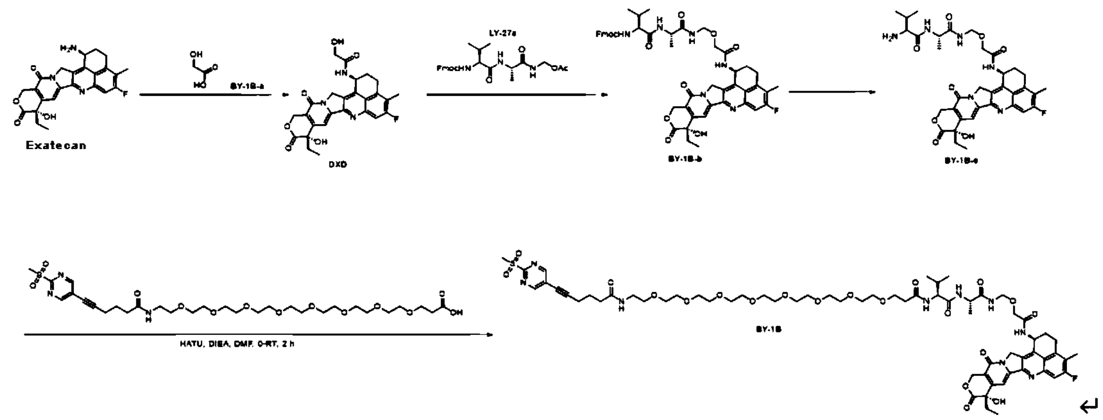


Step 1: Synthesis of compound **DXD**

DIEA (18.66 μL, 0.113 mmol, 3 eq) was added to the solution of the compound exatecan mesylate (20 mg, 37.63 μmol, 1 eq) and glycolic acid (2.48 μL, 41.39 μmol, 1.1 eq) in DMF (2 mL) under nitrogen protection, and after the reaction solution was stirred at room temperature for 3 minutes, HATU (17.17 mg, 45.15 μmol, 1.2 eq) was added to the reaction solution under the ice water bath. The reaction solution was warmed to room temperature again and further stirred for 2 hours. The reaction solution was purified by reversed-phase preparative chromatograph (preparative chromatograph

manufacturer: SHIMADZU, model: LC-20AP, column: YMC-Triart Prep C18 250 × 30 mm × 10 μm, mobile phase: water (0.225% HCOOH)-acetonitrile, an elution ratio of acetonitrile: 20% to 55%), to obtain **DXD** (7.22 mg, 38.9%).

LCMS (ESI): m/z, 494[M+H]$^+$.

$^1$H NMR (400 MHz, DMSO-$d_6$) δ 8.42 (d, *J* = 8.8 Hz, 1H), 7.77 (dd, *J* = 10.8, 3.2 Hz, 1H), 7.30 (s, 1H), 6.53 (s, 1H), 5.62-5.55 (m, 1H), 5.51 (t, *J* = 5.8 Hz, 1H), 5.42 (s, 2H), 5.26-5.11 (m, 2H), 3.96 (d, *J* = 3.8 Hz, 2H), 3.23-3.10 (m, 2H), 2.39 (d, *J* = 2.0 Hz, 3H), 2.18 (ddd, *J* = 13.2, 10.4, 6.0 Hz, 2H), 1.93-1.80 (m, 2H), 0.87 (t, *J* = 7.2 Hz, 3H). $^{19}$F NMR (377 MHz, DMSO-$d_6$) δ -111.33.

Step 2: Preparation compound **BY-1B-b**

TsOH (10.15 mg, 9.49 μL, 58.97 μmol, 1 eq) was added to a solution of compound DXD (30 mg, 97%, 58.97 μmol, 1 eq) and compound **LY-27a** (85.19 mg, 176.90 μmol, 3 eq) in THF (2 mL) in the ice water bath and the reaction solution was warmed to room temperature again and further stirred for 4 hours. The reactant was purified by preparative high performance liquid chromatograph (preparative chromatograph model: BRIX-2860R7, column: YMC-Triart Prep C18 150 × 30 mm × 10 μm, mobile phase: water-(0.225% HCOOH)-acetonitrile, an elution ratio of acetonitrile: 30% to 60%) and lyophilized, to obtain a white solid product **BY-1B-b** (14.10 mg, yield: 26.13%).

LCMS (ESI): m/z, 914[M+H]$^+$, 937 [M+Na]$^+$.

Step 2: Preparation of compound **BY-1B-c**

Diethylamine (5.64 mg, 7.94 μL, 77.05 μmol, 5 eq) was added to the solution of compound **BY-1B-b** (14.10 mg, 15.41 μmol, 1 eq) in DMF (1 mL), and the reaction solution was stirred at room temperature for 2 hours. The reaction solution was concentrated under reduced pressure, to obtain a brown solid crude product (14.30 mg, no yield was calculated for the crude product).

LCMS (ESI): m/z, 639[M+H]$^+$.

Step 3: Preparation of compound **BY-1B**

DIPEA (5.34 mg, 6.82 μL, 41.28 μmol, 2 eq) and HATU (9.42 mg, 24.77 μmol, 1.2 eq) were added to a solution of compound **BY-1B-c** (14.30 mg, 20.64 μmol, 1 eq) and compound **LY-29B-d** (18.48 mg, 85%, 22.71 μmol, 1.1 eq) in DMF (2 mL) in the ice water bath, and the reaction solution was warmed to room temperature again and further stirred for 4 hours. The reaction solution was purified by preparative high performance liquid chromatograph (preparative chromatograph model: R6, column: Welch Xtimate C18 250 × 30 mm × 10 μm, mobile phase: water (0.225% HCOOH)-acetonitrile, an elution ratio of acetonitrile: 30% to 60%) and lyophilized, to obtain a white solid **BY-1B** (4.40 mg, yield: 15.60%, purity: 96%).

LCMS (ESI): m/z, 1388[M+Na]$^+$.

Example 81: Preparation of compound **BY-1C**

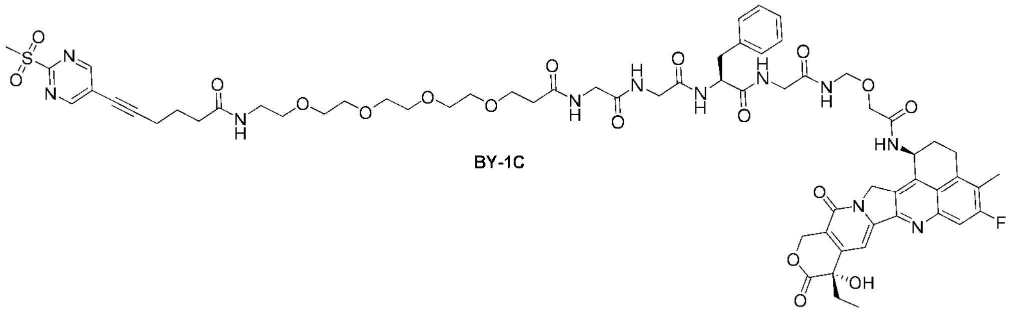

BY-1C

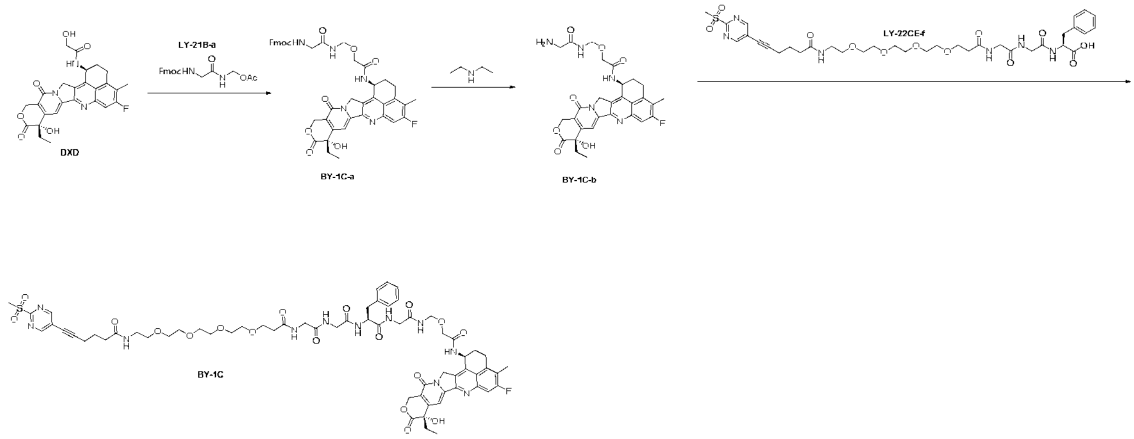


DXD BY-1C-a BY-1C-b BY-1C

The preparation method was the same as that in Example 80, except that the compound **LY-27a** was replaced with the compound **LY-21B-a** and the compound **LY-29B-d** was replaced with the compound **LY-22CF-f** to obtain the compound **BY-1C.**

LCMS (ESI): m/z, 1338.5 $[M+H]^+$/1360.5$[M+Na]^+$; HPLC: Rt = 4.508 min.

Example 82: Preparation of compound **BY-2**

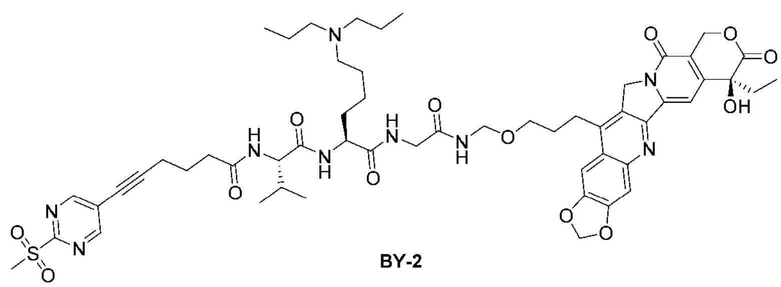

BY-2

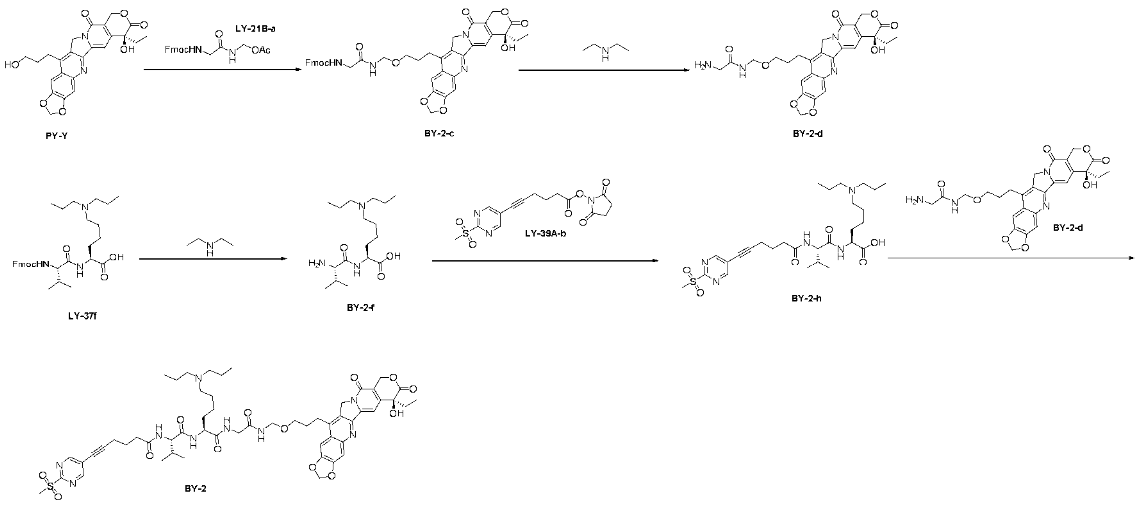


Step 1: Preparation of compound **BY-2-c**

Compound **PY-Y** (20 mg, 1.0 eq) and compound **LY-21B-a** (40.89 mg, 2.5 eq) were dissolved in anhydrous tetrahydrofuran (2 mL), p-toluenesulfonic acid (7.65 mg, 1.0 eq) was added, and then the reaction solution reacted at room temperature for 16 hours. After the reaction solution was concentrated, the residue was dissolved in N,N-dimethylformamide (4 mL) and filtered, and then purified by preparative high performance liquid chromatograph (preparative chromatograph manufacturer: ISCO, model: Lab311-DJ-R2, column: Synergi Max-RP 250 × 40 mm × 10 μm, mobile phase: water (0.225% HCOOH)-acetonitrile, an elution ratio of water: 30% to 60%), to obtain compound **BY-2-c** (16 mg, yield: 47.5%).

LCMS: m/z =759.1 $[M+H]^+$; Rt = 2.010 min.

Step 2: Preparation of compound **BY-2-d**

The compound **BY-2-c** (16 mg, 1.0 eq) was dissolved in DMF (1 mL), diethylamine (21.72 μL, 10.0 eq) was added, and then the reaction solution was stirred at room temperature for reaction for 2 hours. The reaction solution was concentrated to dryness, to obtain the crude product (12 mg) to be directly used for the next reaction.

LCMS: m/z =537.1 $[M+H]^+$; Rt = 1.383 min.

Step 3: Preparation of compound **BY-2-f**

The compound **LY-37f** (66 mg, 1.0 eq) was dissolved in anhydrous DMF (1 mL), diethylamine (123.2 μL, 10 eq) was added, and the reaction solution was stirred at room temperature for reaction for 2 hours. The reaction solution was concentrated to dryness, to obtain the crude product of compound **BY-2-f** (12 mg) to be directly used for the next reaction.

LCMS: m/z =330.2 $[M+H]^+$; Rt = 1.337 min.

Step 4: Preparation of compound **BY-2-h**

The compounds **BY-2-f** (29.86 mg, 1.0 eq) and **LY-39A-b** (61.13 mg, 1.2 eq) were dissolved in anhydrous DMF, DIPEA (44.93 μL, 3.0 eq) was added, and then the reaction solution was stirred at room temperature for reaction for 2 hours. After being filtered, the reaction solution was purified by preparative high performance liquid chromatograph (preparative chromatograph manufacturer: ISCO, model: Lab311-DJ-R3, column: GS-120-10-C18AP, mobile phase:

water (0.225% HCOOH)-acetonitrile, an elution ratio of water: 12% to 42%), to obtain compound **BY-2-h** (15 mg, yield: 28.6%).

LCMS: m/z =580.3 [M+H]+; Rt = 1.118 min.

Step 5: Preparation of compound **BY-2**

The compounds **BY-2-h** (12.21 mg, 1.0 eq) and **BY-2-d** (11.3 mg, 1.0 eq) were dissolved in anhydrous DMF (1 mL), HATU (10.41 mg, 1.3 eq) and DIPEA (10.44 μL, 3.0 eq) were added, and then the reaction solution was stirred at room temperature for reaction for 1 hour. After being filtered, the reaction solution was purified by preparative high performance liquid chromatograph (preparative chromatograph manufacturer: ISCO, model: Lab311-DJ-R2, column: YMC-Triart Prep C18 250 × 30 mm × 10 μm, mobile phase: water (0.225% HCOOH),-acetonitrile, an elution ratio of water: 21% to 51%), to obtain a white solid compound **BY-2** (3.65 mg, yield: 15.8%).

LCMS: m/z =1098.4 $[M+H]^+$; Rt = 1.703 min.

$^1H$ NMR (400 MHz, DMSO-*d*$^6$) δ 9.10 (s, 2H), 8.62 (s, 1H), 8.19 (s, 1H), 8.06 (*d,* J = 7.4 Hz, 1H), 7.96 (dd, *J* = 21.1, 8.5 Hz, 1H), 7.59 (s, 1H), 7.51 (s, 1H), 7.24 (s, 1H), 6.49 (d, *J* = 2.4 Hz, 1H), 6.28 (s, 2H), 5.42 (s, 2H), 5.25 (s, 2H), 4.65-4.52 (m, 2H), 4.23 (d, *J* = 7.1 Hz, 1H), 4.15 (t, *J* = 7.8 Hz, 1H), 3.73 (t, *J* = 4.6 Hz, 2H), 3.50 (t, *J* = 6.0 Hz, 3H), 3.40 (s, 3H), 3.15-3.03 (m, 4H), 2.93 (s, 1H), 2.33 (dd, *J* = 23.2, 7.7 Hz, 7H), 2.03-1.73 (m, 12H), 1.28 (s, 2H), 1.23 (s, 2H), 0.84 (dt, *J* = 18.7, 6.8 Hz, 15H).

Example 83: Preparation of compound **BY-3**

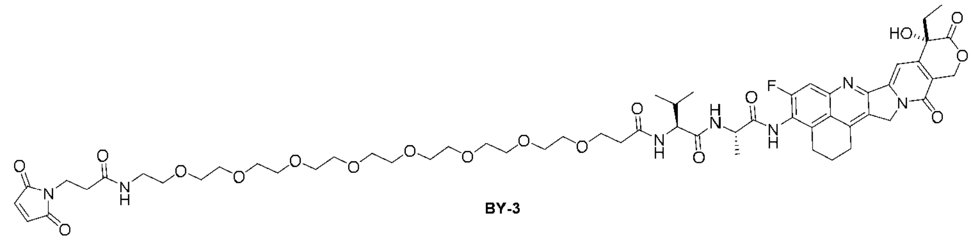

BY-3

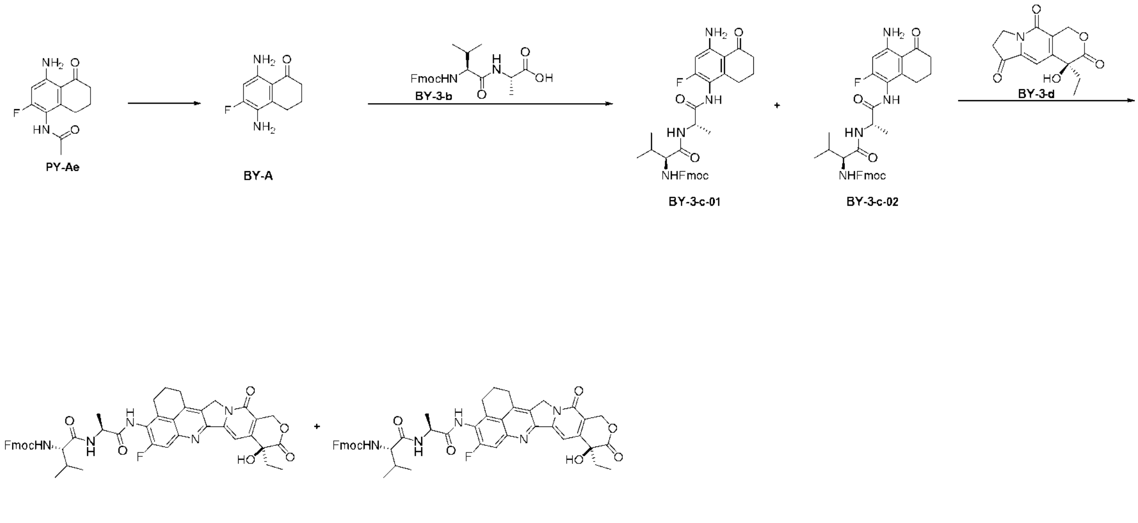


PY-Ae BY-A BY-3-c-01 + BY-3-c-02

BY-3-e-01 + BY-3-e-02

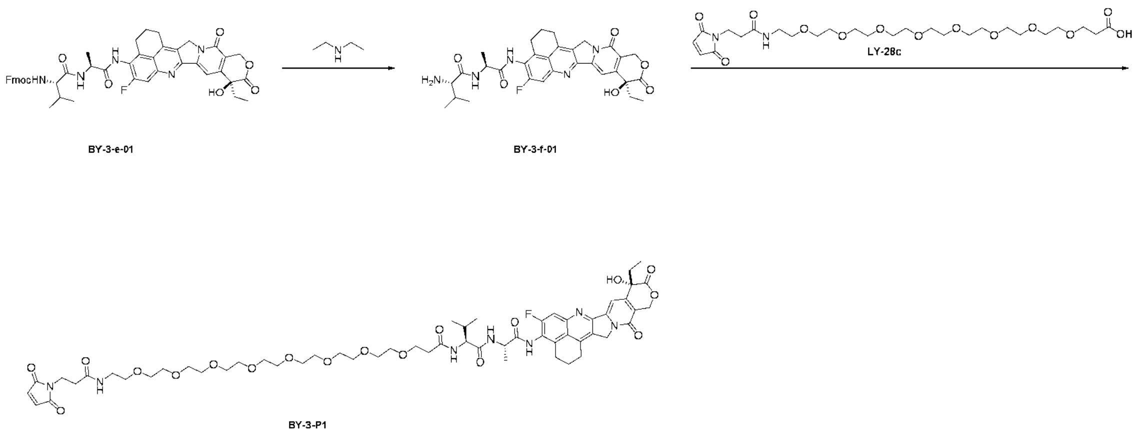


BY-3-e-01 BY-3-f-01

BY-3-P1

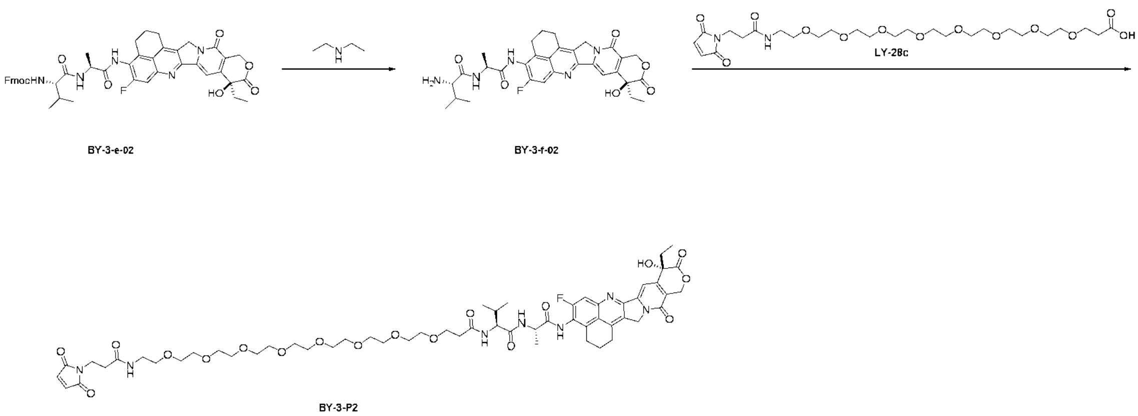


BY-3-e-02 BY-3-f-02

BY-3-P2

Step 1: Preparation of compound BY-A

The compound PY-Ae (for the synthesis of the compound, refer to the preparation example of PY-A) (240 mg, 1.02 mmol, 1 eq) was added to 25 mL of concentrated hydrochloric acid solution at 0°C, the reaction solution was stirred at 80°C for 16 hours after addition, the LCMS showed that the reaction ended, and the reaction solution was directly spun to dryness to obtain the yellow solid compound BY-A (206 mg, yield: 75.91%).

Step 2: Preparation of compounds BY-3-c-01 and BY-3-c-02

N,N-diisopropylethylamine (548.34 mg, 701.21 μL, 4.24 mmol, 4 eq) and 2-(7-azabenzotriazole)-N,N,N',N'-tetramethyluronium hexafluorophosphate (604.99 mg, 1.59 mmol, 1.5 eq) were added to the solution of 5,8-diamino-6-fluoro-1,2,3,4-tetrahydronaphthalene-1-one (206 mg,1.06 mmol,1 eq) and Fmoc-L-valyl-L-alanine (522.47 mg, 1.27 mmol, 1.2 eq) in N,N-dimethylformamide (10 mL) at room temperature, and the reaction solution was stirred at 50°C for 16 hours. The reaction solution was purified by preparative high performance liquid chromatograph (preparative chromatograph manufacturer: ISCO, model: ISCO-R6, column: Welch Xtimate C18 250 × 30 mm × 10 μm, mobile phase: water (0.225% FA)-acetonitrile, an elution ratio of water: 39% to 69%) and lyophilized, to obtain white solid isomer products By-3-c-01 (2.21 min, 121 mg) and By-3-c-02 (2.25 min, 75 mg) (yield: 32%).

LCMS (ESI): m/z, 587.4 $[M+H]^+$.

Step 3: Preparation of compounds **By-3-e-01** and**BY-3-e-01**

Compounds **BY-3-c-01** and **BY-3-c-02** (mixture, 75 mg, 127.84 μmol, 1 eq), (S)-4-ethyl-4-hydroxy-7,8-dihydro-1H-pyran[3,4-f]indolizino-3,6,10(4H)-trione **(BY-3-d)** (ChemExpress) (33.65 mg, 127.84 μmol, 1 eq) and toluene-4-sulfonic acid pyridine salt (32.13 mg, 127.84 μmol, 1 eq) were added to the solution of toluene (7 mL) at room temperature, and nitrogen displacement was performed 3 times. The reaction solution was stirred at 120°C for 16 hours. The reaction solution was concentrated under reduced pressure to obtain a crude product, and the crude product was purified by preparative high performance liquid chromatograph (preparative chromatograph manufacturer: Luna, model: Lab311-ISCO-R4, column: Phenomenex Luna C18 250 × 50 mm × 10 μm, mobile phase: water (0.225% formic acid)-acetonitrile, an elution ratio of water: 30% to 60%), and an eluate was lyophilized, to obtain white solid isomer products **BY-3-e-01** (2.14 min, 49 mg) and **BY-3-e-02** (2.31 min, 46 mg) (yield: 53%).

LCMS (ESI): m/z, 814.3 $[M+H]^+$.

Step 3: Preparation of compound **BY-3-f-01**

**BY-3-e-01** (49 mg, 60.21 μmol, 1 eq) and diethylamine (22.02 mg, 31.01 μL, 301.03 μmol, 5 eq) were sequentially added to N,N-dimethylformamide (5 mL) at room temperature. The reaction solution was stirred at 25°C for 16 hours. The reaction solution was concentrated under reduced pressure, to obtain the crude product **BY-3-f-01** (0.981 min, 49 mg) (no yield was calculated for the crude product).

LCMS (ESI): m/z, 592.2 $[M+H]^+$.

Step 4: Preparation of compound **BY-3-P1**

DIPEA (23.59 mg, 30.17 μL, 182.54 μmol, 3 eq) was added to the solution of compound **BY-3-f-01** (36 mg, 60.85 μmol, 1 eq), compound **LY-28c** (43.27 mg, 73.02 μmol, 1.2 eq), and 2-(7-azabenzotriazole)-N,N,N',N'-tetramethyluronium hexafluorophosphate (27.76 mg, 73.02 μmol, 1.2 eq) in N,N-dimethylformamide (3 mL) at room temperature and the reaction solution was stirred at 25°C for 16 hours. The reaction solution was purified by preparative high performance liquid chromatograph (preparative chromatograph manufacturer: ISCO, model: ISCO-R1, column: YMC-Triart Prep C18 250 × 50 mm × 7 μm, mobile phase: water (0.225% HCOOH)-acetonitrile, an elution ratio of water: 21% to 51%) and the eluate was lyophilized, to obtain a white solid product **BY-3-P1** (12.14 mg, second-step yield: 19%).

LCMS (ESI): m/z, 1166.7 $[M+H]^+$.

$^1$H NMR (400 MHz, DMSO-$d^6$) δ 9.75 (s, 1H), 8.46 (d, *J* = 7.2 Hz, 1H), 8.02 (d, *J* = 7.6 Hz, 2H), 7.81 (d, *J* = 11.2 Hz, 1H), 7.32 (s, 1H), 7.00 (s, 2H), 6.52 (s, 1H), 5.44 (s, 2H), 5.27 (s, 2H), 4.56 (t, *J*= 7.2 Hz, 1H), 4.19 (t, *J*= 7.6 Hz, 1H), 3.59 (t, *J*= 7.2 Hz, 2H), 3.54 (d, *J* = 2.4 Hz, 1H), 3.51-3.46 (m, 22H), 3.45 (s, 5H), 3.44-3.40 (m, 4H), 3.15 (dq, *J* = 11.6, 6.0 Hz, 4H), 2.96 (t, *J* = 6.0 Hz, 2H), 2.46 (t, *J* = 7.2 Hz, 1H), 2.34 (dt, *J* = 14.4, 6.8 Hz, 3H), 2.01 (dp, *J* = 27.6, 7.2 Hz, 3H), 1.87 (q, *J* = 7.2 Hz, 2H), 1.41 (d, *J* = 7.2 Hz, 3H), 0.93-0.84 (m, 10H).

Step 5: Preparation of compound **BY-3-f-02**

Compound **BY-3-e-02** (46 mg, 56.52 μmol, 1 eq) and diethylamine (20.67 mg, 29.11 μL, 282.60 μmol, 5 eq) were sequentially added to N,N-dimethylformamide (5 mL) at room temperature. The reaction solution was stirred at 25°C for 16 hours. The reaction solution was concentrated under reduced pressure, to obtain the crude product **BY-3-f-02** (0.888 min, 46 mg) (no yield was calculated for the crude product).

LCMS (ESI): m/z, 592.2 $[M+H]^+$.

Step 6: Preparation of compound **BY-3-P2**

DIPEA (22.28 mg, 28.49 μL, 172.40 μmol, 3 eq) was added to the solution of compound **BY-3-f-02** (34 mg, 57.47 μmol, 1 eq), compound **LY-28c** (40.87 mg, 68.96 μmol, 1.2 eq), and 2-(7-azabenzotriazole)-N,N,N',N'-tetramethyluronium hexafluorophosphate (26.22 mg, 68.96 μmol, 1.2 eq) in N,N-dimethylformamide (3 mL) at room temperature and the reaction solution was stirred at 25°C for 16 hours. The reaction solution was purified by preparative high performance liquid chromatograph (preparative chromatograph manufacturer: Oriendo, model: BRIX-2860, column: Phenomenex Luna C18 250 × 50 mm × 10 μm, mobile phase: water (0.225% HCOOH)-acetonitrile, an elution ratio of water: 12% to 42%) and the eluate was lyophilized, to obtain a white solid product **BY-3-P2** (5.78 mg, second-step yield: 12%).

LCMS (ESI): m/z, 1166.7 $[M+H]^+$.

$^1H$ NMR (400 MHz, DMSO-$d^6$) δ 9.86 (s, 1H), 8.27 (d, *J* = 6.8 Hz, 1H), 8.01 (t, *J*= 5.6 Hz, 1H), 7.92 (d, *J* = 8.8 Hz, 1H), 7.81 (d, *J* = 10.8 Hz, 1H), 7.32 (s, 1H), 7.00 (s, 2H), 6.52 (s, 1H), 5.44 (s, 2H), 5.26 (s, 2H), 4.54 (t, *J* = 7.2 Hz, 1H), 4.26 (dd, *J* = 8.8, 6.4 Hz, 1H), 3.59 (td, *J* = 7.2, 3.2 Hz, 4H), 3.50 (d, *J* = 3.6 Hz, 28H), 3.36 (d, *J* = 6.0 Hz, 2H), 3.18-3.11 (m, 4H), 2.96 (d, *J*= 7.2 Hz, 2H), 2.46-2.40 (m, 1H), 2.40 - 2.29 (m, 3H), 1.99 (dq, *J* = 13.2, 8.0, 6.8 Hz, 3H), 1.87 (p, *J* = 7.2 Hz, 2H), 1.41 (d, *J* = 7.2 Hz, 3H), 0.94-0.79 (m, 10H).

Preparation Example 1 of ADC: Preparation of Trastuzumab Deruxtecan (Enhertu) (T-BY-1) (Method 1A, DAR 8)

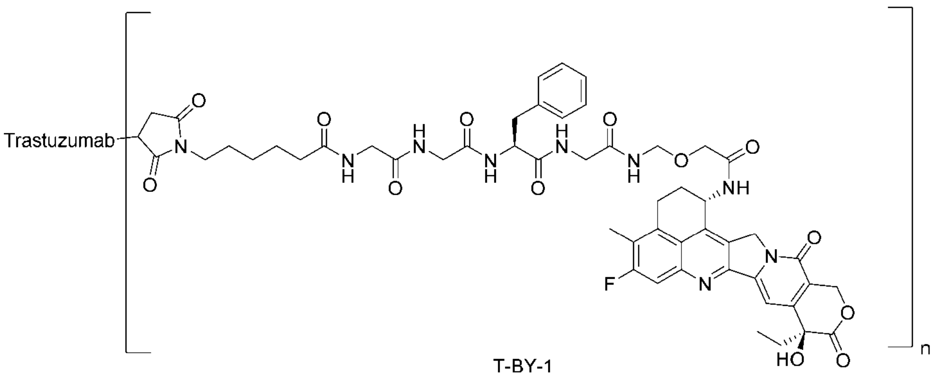

T-BY-1

An aqueous solution (40 mM, 23.3 μL, 931 nmol) of tris(2-carboxyethyl)phosphine (TCEP) was added to the solution of trastuzumab (Trastuzumab) (Herceptin, Genentech, 10.0 mg/mL, 2 mL, 133 nmol) in the aqueous solution of PBS buffer (0.04M aqueous solution of PBS buffer, pH = 7.4) at 37°C, and the resulting solution was placed in a water bath and reacted at 37°C for 2 hours before stopping the reaction. The reaction solution was cooled to 25°C in a water bath.

The Compound BY-1 (ChemExpress, 1.38 mg, 1330 nmol) was dissolved in 83.3 μL of DMSO, the resulting solution was added to the above reaction solution, the reaction solution was placed in a water bath and reacted at 25°C for 20 minutes, and then 1% (mass ratio) N-ethylmaleimide (NEM) was added to end the reaction. The reaction solution was purified by dialysis with a dialysis card (Slide-A-Lyzer™ dialysis kit, 10K MWCO) to obtain PBS buffer (4.353 mg/mL, 3.30 mL) of Trastuzumab Deruxtecan (Enhertu), and the PBS buffer was stored at 4°C and stored at -80°C for a long time.

Average drug loading calculated by LC-MS: n = 8.0 (for LC-MS, refer to Preparation Example 3).

Preparation Example 2 of ADC: Preparation of Trastuzumab Deruxtecan (Enhertu) (T-BY-1-4) (Method 1B, DAR 4)

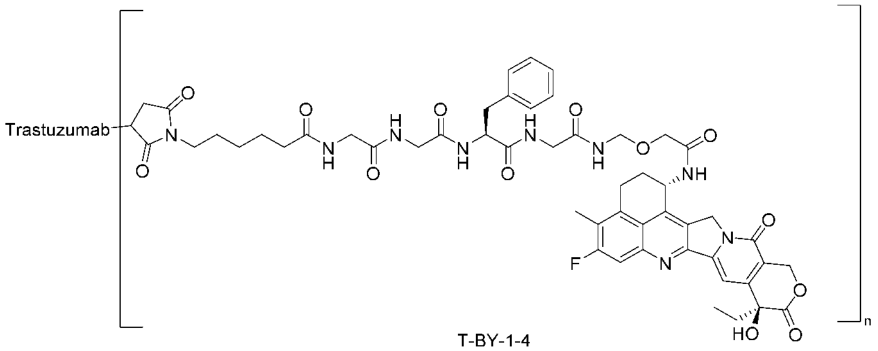

T-BY-1-4

An aqueous solution (5 mM, 4.8 μL, 24 nmol) of tris(2-carboxyethyl)phosphine (TCEP) was added to the solution of trastuzumab (Trastuzumab) in the aqueous solution of sodium borate buffer (25 mM aqueous solution of sodium borate buffer, pH = 7.4; 5.0 mg/mL, 0.3 mL, 10 nmol) at 25°C, and the resulting solution was placed in a water bath and reacted at 25°C for 2 hours before stopping the reaction.

The Compound BY-1 was dissolved in DMSO (10 mM BY-1), the resulting solution was added to the above reaction solution (10 μL, 100 nmol), and the reaction solution was placed in a water bath and reacted at 25°C for 2 hours. The reaction solution was purified by dialysis with a dialysis kit (brand: Thermo Fisher; model: Slide-A-Lyzer™) to obtain PBS buffer (3.275 mg/mL, 0.3 mL) of Trastuzumab Deruxtecan (Enhertu), and the PBS buffer was stored at 4°C and stored at -80°C for a long time.

Average drug loading calculated by RP-HPLC: n = 4.2 (refer to Preparation Example 3).

Preparation Example 3 of ADC: Preparation of T-LY-1 (Method 1A)

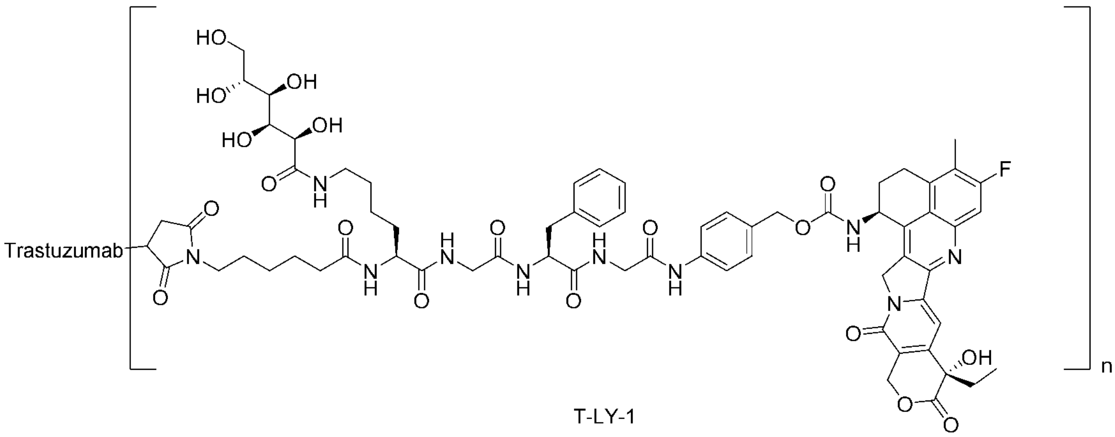

T-LY-1

An aqueous solution (40 mM, 23.3 μL, 931 nmol) of tris(2-carboxyethyl)phosphine (TCEP) was added to the solution of trastuzumab (Trastuzumab) (Herceptin, Genentech, 10.0 mg/mL, 2 mL, 133 nmol) in the aqueous solution of PBS buffer (0.04M aqueous solution of PBS buffer, pH = 7.4) at 37°C, and the resulting solution was placed in a water bath and reacted at 37°C for 2 hours before stopping the reaction. The reaction solution was cooled to 25°C in a water bath.

The compound **LY-1** (1.94 mg, 1330 nmol) was dissolved in 83.3 μL of DMSO, the resulting solution was added to the above reaction solution, the reaction solution was placed in a water bath and reacted at 25°C for 20 minutes, and then 1% (mass ratio) N-ethylmaleimide (NEM) was added to end the reaction. The reaction solution was purified by dialysis with a dialysis card (Slide-A-Lyzer™ dialysis kit, 10K MWCO) to obtain PBS buffer (4.364 mg/mL, 3.85 mL) of T-LY-1, and the PBS buffer was stored at 4°C and stored at -80°C for a long time.

Average drug loading calculated by LC-MS: n = 8.0 (LC-MS experimental instrument: UPLC-MS, Thermo Fisher Scientific, DionexUltiMate 3000 UPLC-Q Exactive MS; High resolution mass spectrometer, Thermo Q EXACTIVE HF-X; column: Waters XBridge BEH C4, 300Å, 3.5 μm, 4.6 × 100 mm; Mobile phase: water (0.1% trifluoroacetic acid)-acetonitrile (0.1% trifluoroacetic acid), elution from 20% to 80%; and data analysis was performed by using Protein Deconvolution software).

Preparation Example 4 of ADC: Preparation of T-LY-2 (Method 1A)

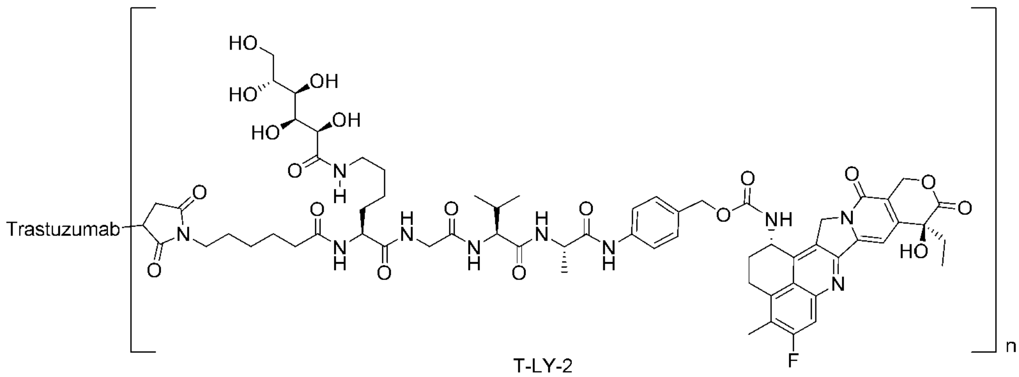

T-LY-2

An aqueous solution (40 mM, 23.3 μL, 931 nmol) of tris(2-carboxyethyl)phosphine (TCEP) was added to the solution of trastuzumab (Trastuzumab) (Herceptin, Genentech, 10.0 mg/mL, 2 mL, 133 nmol) in the aqueous solution of PBS buffer (0.04M aqueous solution of PBS buffer, pH = 7.4) at 37°C, and the resulting solution was placed in a water bath and reacted at 37°C for 2 hours before stopping the reaction. The reaction solution was cooled to 25°C in a water bath.

The compound **LY-2** (1.90 mg, 1330 nmol) was dissolved in 83.3 μL of DMSO, the resulting solution was added to the above reaction solution, the reaction solution was placed in a water bath and reacted at 25°C for 20 minutes, and then 1% (mass ratio) N-ethylmaleimide (NEM) was added to end the reaction. The reaction solution was purified by dialysis with a dialysis card (Slide-A-Lyzer™ dialysis kit, 10K MWCO) to obtain PBS buffer (4.353 mg/mL, 3.70 mL) of T-LY-2, and the PBS buffer was stored at 4°C and stored at -80°C for a long time.

Average drug loading calculated by LC-MS: n = 8.0 (the method of LC-MS was the same as the above method of LC-MS).

Preparation Example 5 of ADC: Preparation of T-LY-6 (T-LY-6, Site-specific DAR4) (Method 2)

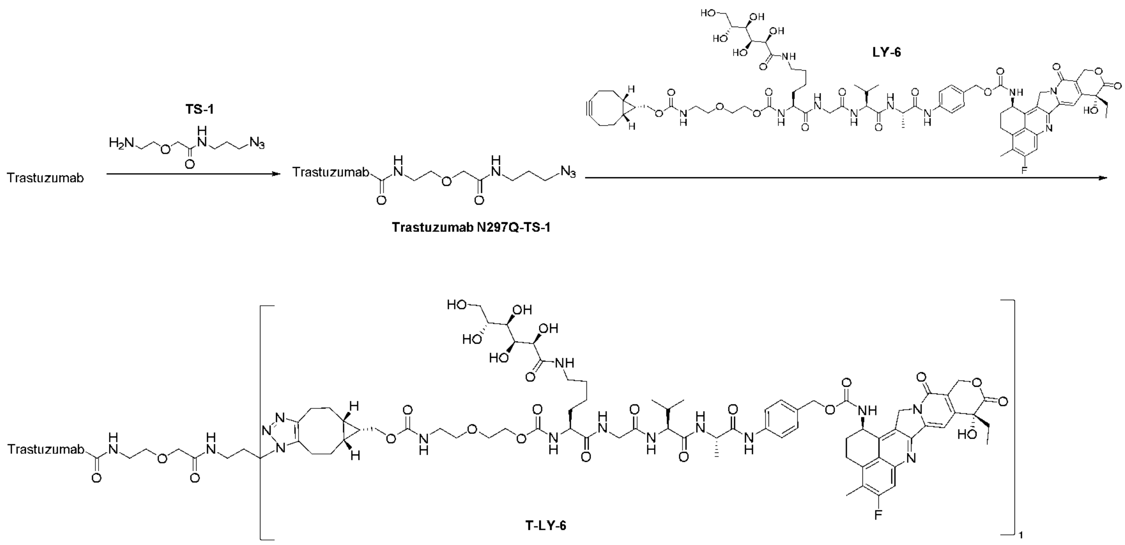


Azide-modified trastuzumab mutant (Trastuzumab N297Q-TS-1) (1.0 mg/mL, 1 mL, 6.9 μM) was prepared according to the method described in the literature (Angew. Chem. Int. Ed. 2010, 49, 9995-9997).

Compound LY-6 was dissolved in DMSO (2 mM), the resulting solution was added to the solution of trastuzumab mutant antibody in the aqueous solution of PBS (69 μM), and the reaction solution was placed in a water bath and reacted at 25°C for 6 hours. The reaction solution was purified by dialysis with a dialysis kit (brand: Thermo Fisher; model: Slide-A-Lyzer™) to obtain PBS buffer of T-LY-6, and the PBS buffer was stored at 4°C and stored at -80°C for a long time.

Average drug loading calculated by RP-HPLC: n = 4.0 (refer to Preparation Example 3).

Preparation Example 6 of ADC: Preparation of T-LY-10 (Method 3)

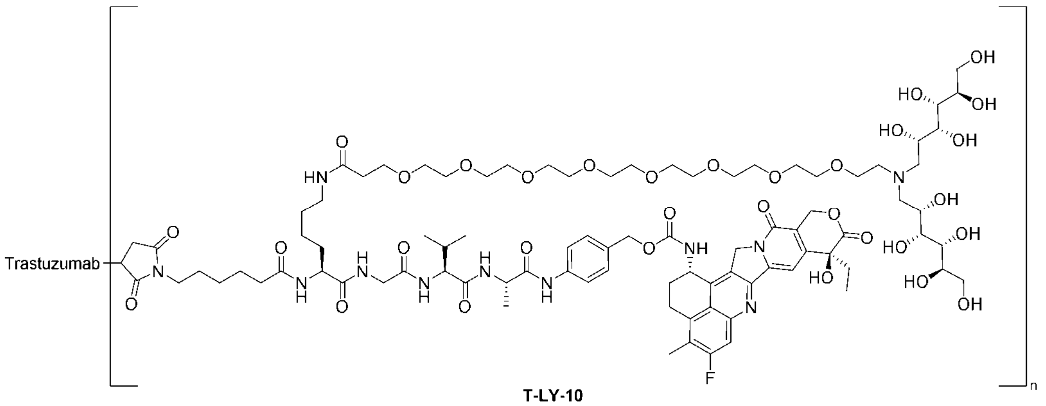

T-LY-10

A prepared aqueous solution (40 mM, 23.3 μL, 931 nmol) of tris(2-carboxyethyl)phosphine (TCEP) was added to the solution of antibody trastuzumab (Herceptin, Genentech, 10.0 mg/mL, 2 mL, 133 nmol) in the aqueous solution of PBS buffer (0.04M aqueous solution of PBS buffer, pH = 7.4) at 37°C, and the resulting solution was placed in a water bath and reacted at 37°C for 2 hours before stopping the reaction. The reaction solution was cooled to 25°C in a water bath.

The compound LY-10 (2.51 mg, 1330 nmol) was dissolved in 83.3 μL of DMSO, the resulting solution was added to the above reaction solution, the reaction solution was placed in a water bath and reacted at 25°C for 2 hours, and then 1% (mass ratio) N-ethylmaleimide (NEM) was added to end the reaction. The reaction solution was purified by dialysis with a dialysis card (Slide-A-Lyzer™ dialysis kit, 10K MWCO) to obtain PBS buffer (2.88 mg/mL) of exemplary product T-LY-10, and the PBS buffer was stored at 4°C after sub-packaging and stored at -80°C for a long time.

Average drug loading calculated by LC-MS: n = 8.0.

Preparation Example 7 of ADC: Preparation of T-LY-22C (Method 4)

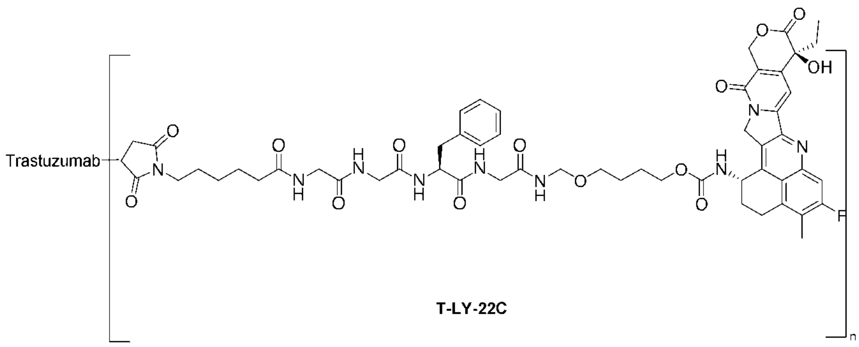

T-LY-22C

A prepared aqueous solution (40 mM, 23.3 μL, 931 nmol) of tris(2-carboxyethyl)phosphine (TCEP) was added to the solution of antibody trastuzumab (Herceptin, Genentech, 10.0 mg/mL, 2 mL, 133 nmol) in the aqueous solution of PBS buffer (0.04M aqueous solution of PBS buffer, pH = 7.4) at 37°C, and the resulting solution was placed in a water bath and reacted at 37°C for 2 hours before stopping the reaction. The reaction solution was cooled to 25°C in a water bath.

The compound **LY-22C** (2.12 mg, 1995 nmol) was dissolved in 125 μL of DMSO, the resulting solution was added to the above reaction solution, the reaction solution was placed in a water bath and reacted at 25°C for 30 minutes, and then 1% (mass ratio) N-ethylmaleimide (NEM) was added to end the reaction. The reaction solution was purified by dialysis with a dialysis card (Slide-A-Lyzer™ dialysis kit, 10K MWCO) to obtain PBS buffer of T-LY-22C, and the PBS buffer was stored at 4°C and stored at -80°C for a long time.

Average drug loading calculated by RP: n = 7.13.

Preparation Example 8 of ADC: Preparation of T-LY-39B (Method 5)

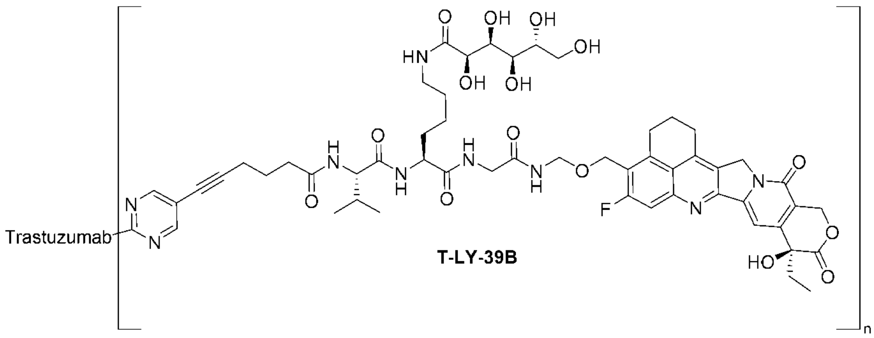

T-LY-39B

A prepared aqueous solution (40 mM, 23.3 μL, 931 nmol) of tris(2-carboxyethyl)phosphine (TCEP) was added to the solution of antibody trastuzumab (Herceptin, Genentech, 10.0 mg/mL, 2 mL, 133 nmol) in the aqueous solution of PBS buffer (0.04M aqueous solution of PBS buffer, pH = 7.4) at 37°C, and the resulting solution was placed in a water bath and reacted at 37°C for 2 hours before stopping the reaction. The reaction solution was cooled to 25°C in a water bath.

The compound **LY-39B** (1.57 mg, 1330 nmol) was dissolved in 83.3 μL of DMSO, the resulting solution was added to the above reaction solution, the reaction solution was placed in a water bath and reacted at 25°C for 30 minutes, and then 1% (mass ratio) N-ethylmaleimide (NEM) was added to end the reaction. The reaction solution was purified by dialysis with a dialysis card (Slide-A-Lyzer™ dialysis kit, 10K MWCO) to obtain PBS buffer of T-LY-39B, and the PBS buffer was stored at 4°C and stored at -80°C for a long time.

Average drug loading calculated by RP: n = 7.25.

ADCs prepared in the present invention are shown in the following table:

| No. | Structure | DAR | Method |
|---|---|---|---|
| T-LY1 | 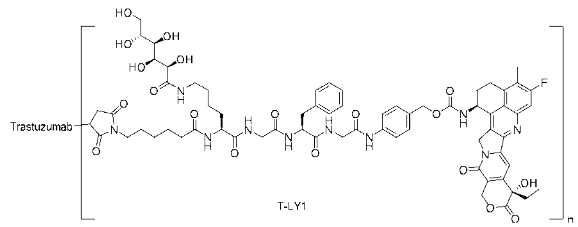 T-LY1 | 8.0 | 1A |
| T-LY2 | 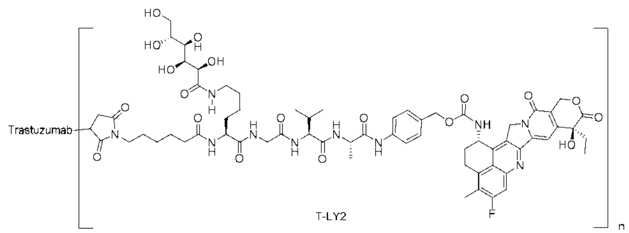 T-LY2 | 8.0 | 1A |
| T-BY1 | 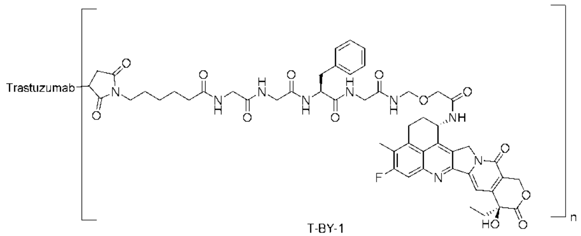 T-BY-1 | 8.0 | 1A |
| T-BY1B | 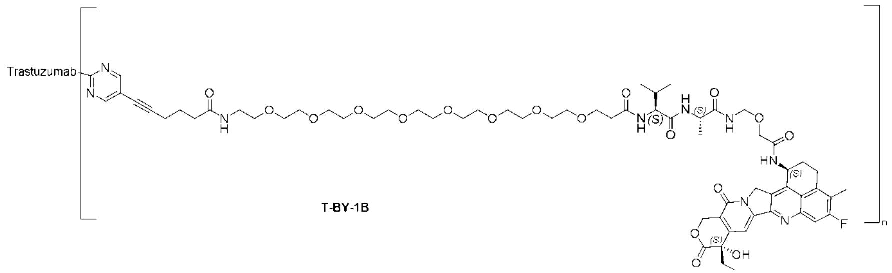 T-BY-1B | 7.66 | 5 |

(continued)

| No. | Structure | DAR | Method |
|---|---|---|---|
| T-BY1C | 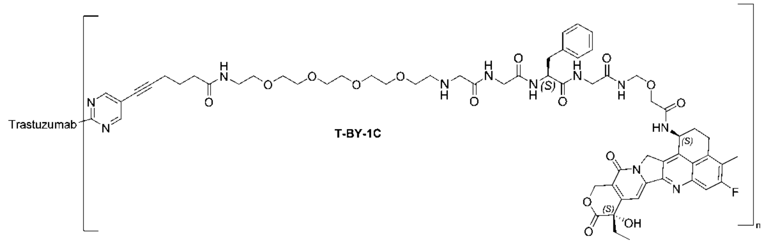 T-BY-1C | 7.57 | 5 |
| T-BY2 | 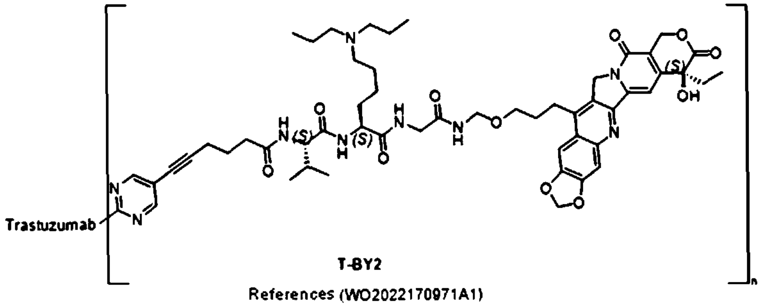 T-BY2 References (WO2022170971A1) | 6.81 | 5 |
| T-BY3P1 | 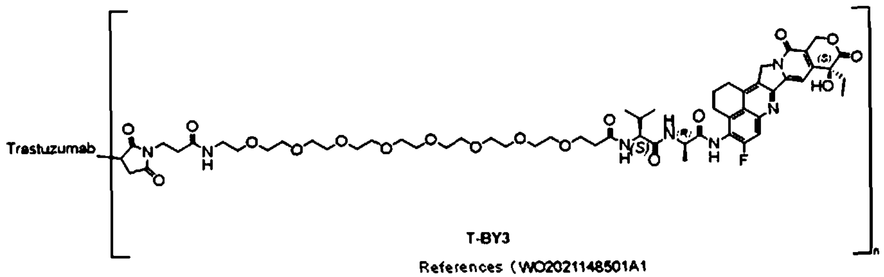 T-BY3 References (WO2021148501A1) | 7.90 | 1A |

(continued)

| No. | Structure | DAR | Method |
|---|---|---|---|
| T-BY3P2 | 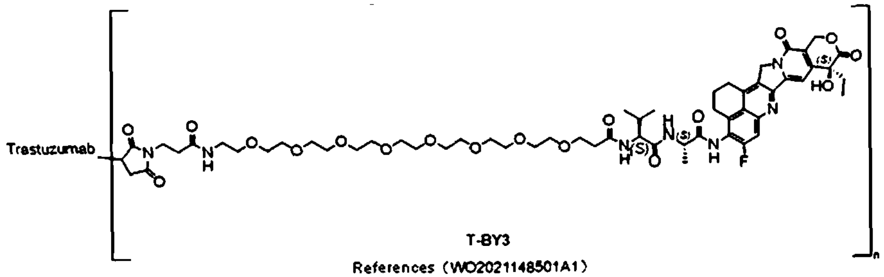 T-BY3 References（WO2021148501A1） | 7.82 | 1A |
| T-LY6 | 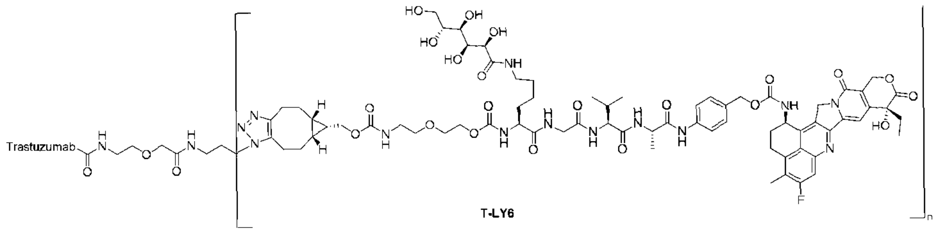 T-LY6 | 4.0 | 2 |
| T-LY8 | 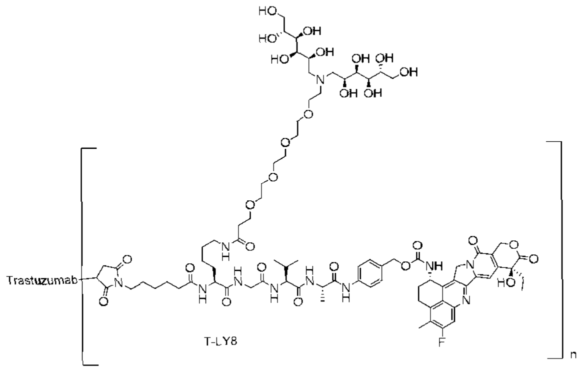 T-LY8 | 6.92 | 1A |

(continued)

| No. | Structure | DAR | Method |
|---|---|---|---|
| T-LY10 | 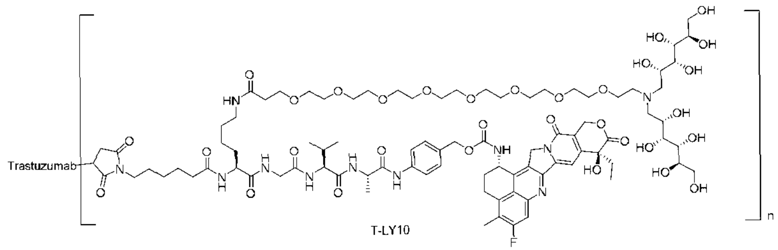 T-LY10 | 8.0 | 3 |
| T-LY21 | 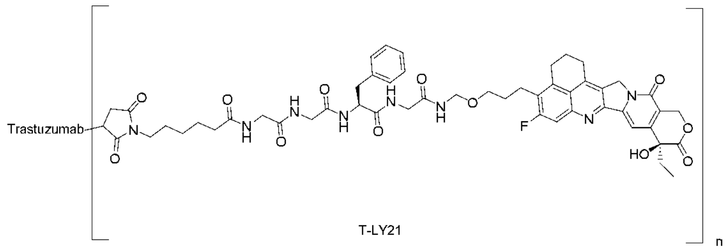 T-LY21 | 5.4 | 1A |
| T-LY21B | 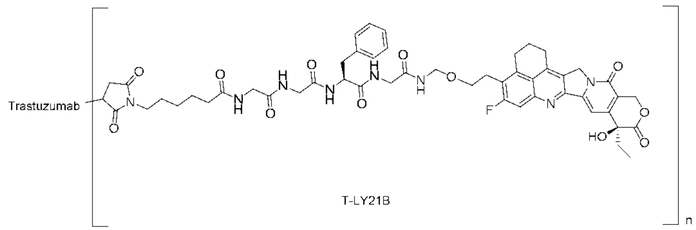 T-LY21B | 6.25 | 1A |
| T-LY21C | 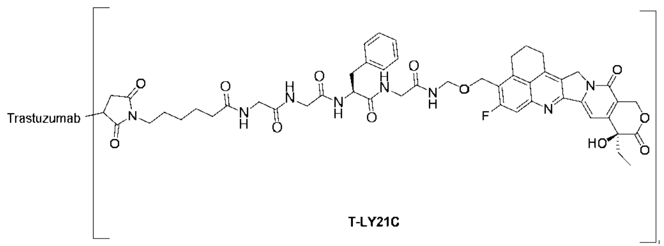 T-LY21C | 7.95 | 1A |

(continued)

| No. | Structure | DAR | Method |
|---|---|---|---|
| T-LY22 | 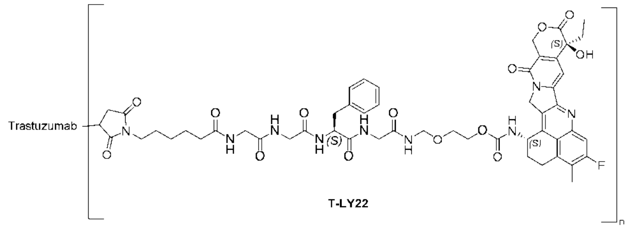 T-LY22 | 7.09 | 1A |
| T-LY22AD | 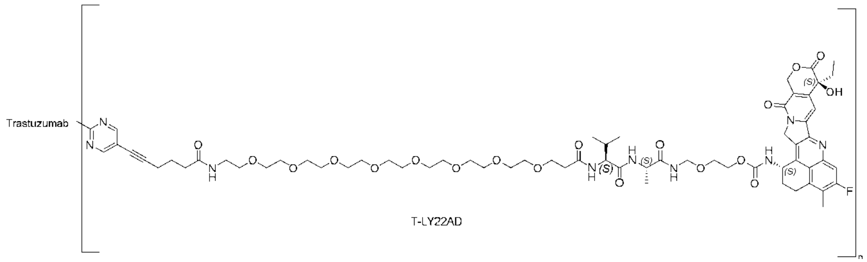 T-LY22AD | 7.76 | 5 |
| T-LY22AF | 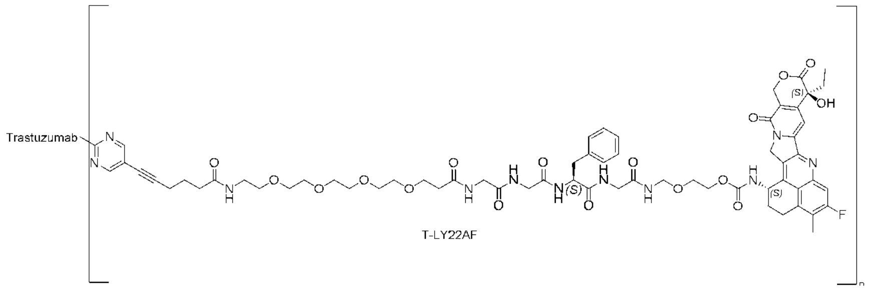 T-LY22AF | 7.62 | 5 |
| T-LY22C | 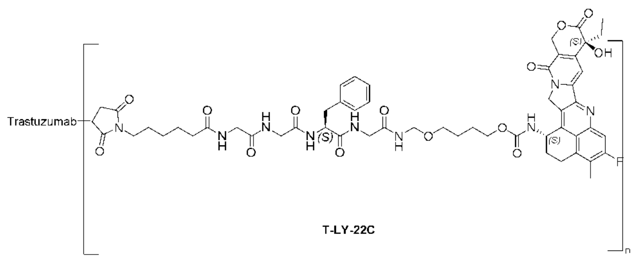 T-LY-22C | 7.81 | 4 |

(continued)

| No. | Structure | DAR | Method |
|---|---|---|---|
| T-LY22CA | 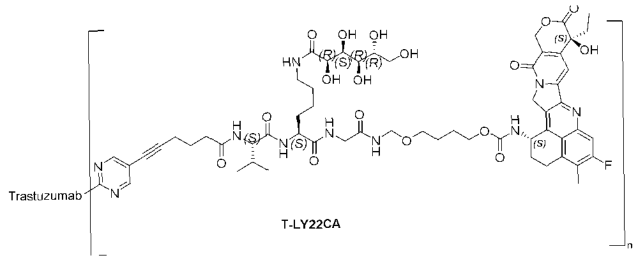 T-LY22CA | 7.92 | 5 |
| T-LY22CB | 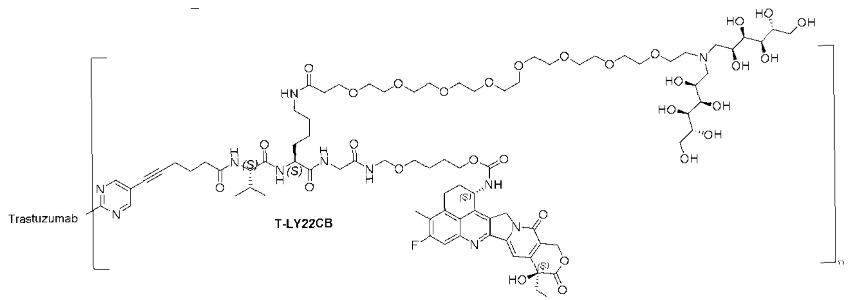 T-LY22CB | 7.80 | 5 |
| T-LY22CC | 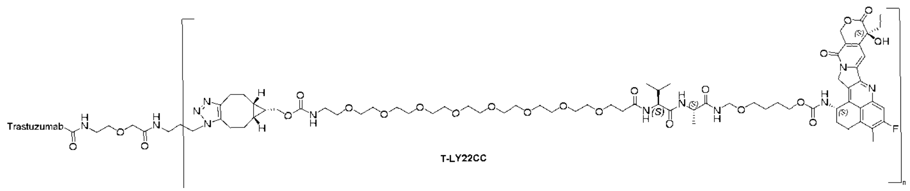 T-LY22CC | 4.0 | 2 |
| T-LY22CD | 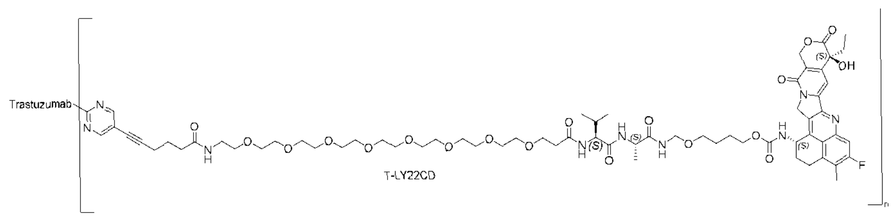 T-LY22CD | 7.87 | 5 |

(continued)

| No. | Structure | DAR | Method |
|---|---|---|---|
| T-LY22CD-2 | 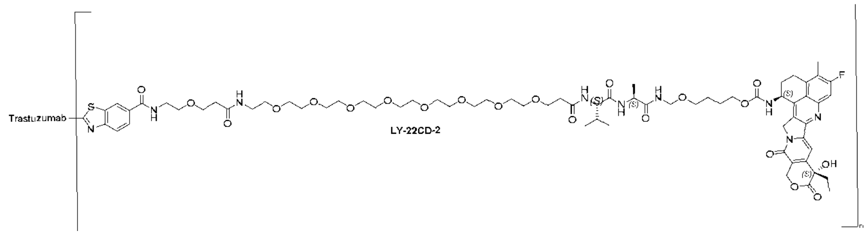 LY-22CD-2 | 7.85 | 5 |
| T-LY22CD-3 | 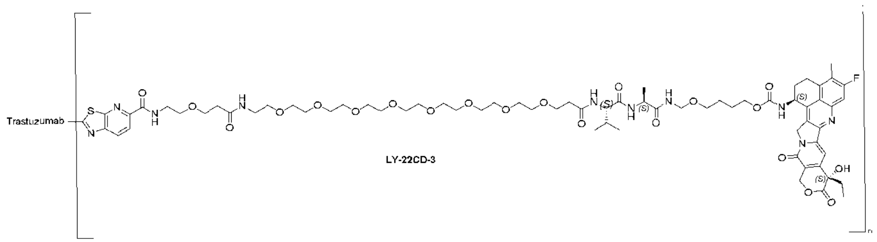 LY-22CD-3 | 7.82 | 5 |
| T-LY22CE | 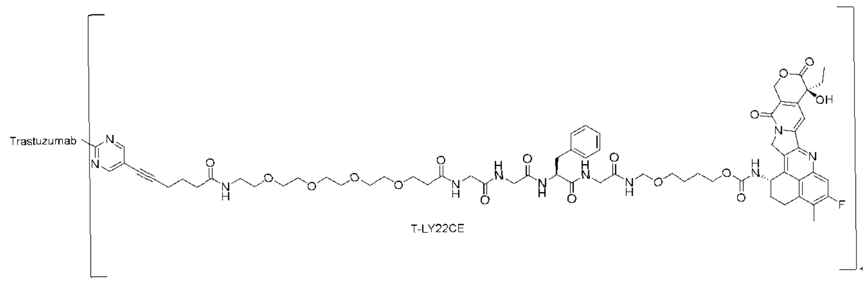 T-LY22CE | 7.42 | 5 |
| T-LY22CF | 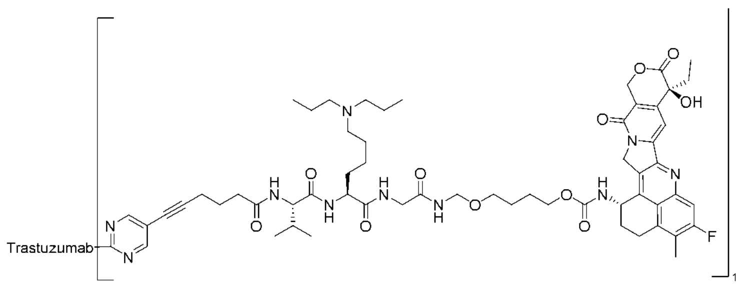 T-LY22CF | 7.5 | 5 |

(continued)

| No. | Structure | DAR | Method |
|---|---|---|---|
| T-LY22CG | 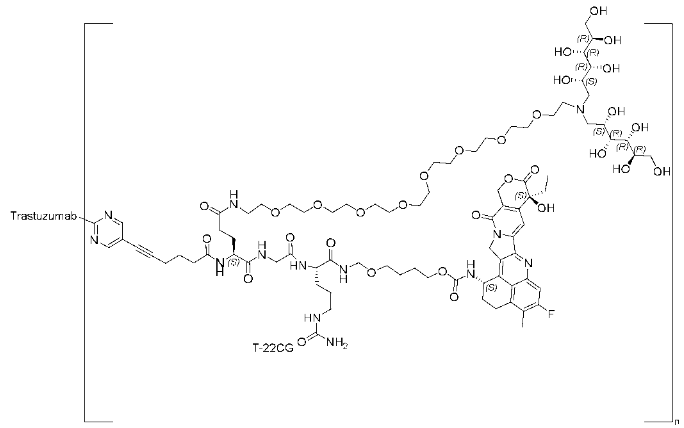 T-22CG | 7.90 | 5 |
| T-LY22CH | 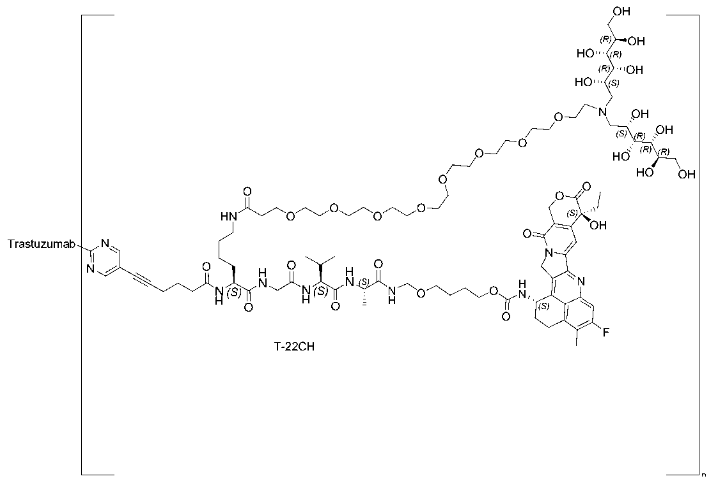 T-22CH | 7.51 | 5 |

(continued)

| No. | Structure | DAR | Method |
|---|---|---|---|
| T-LY22CI | 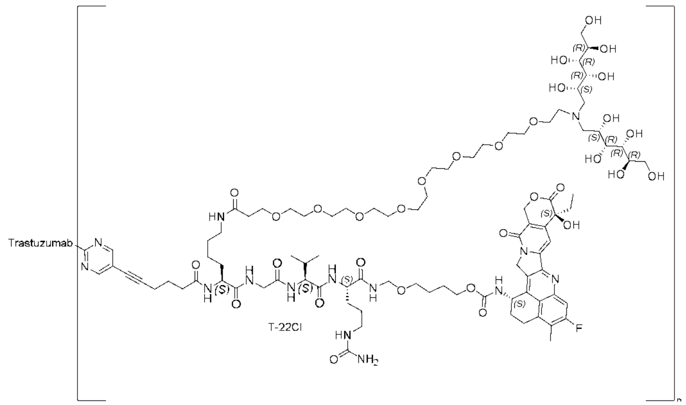 T-22CI | 7.92 | 5 |
| T-LY22CJ | 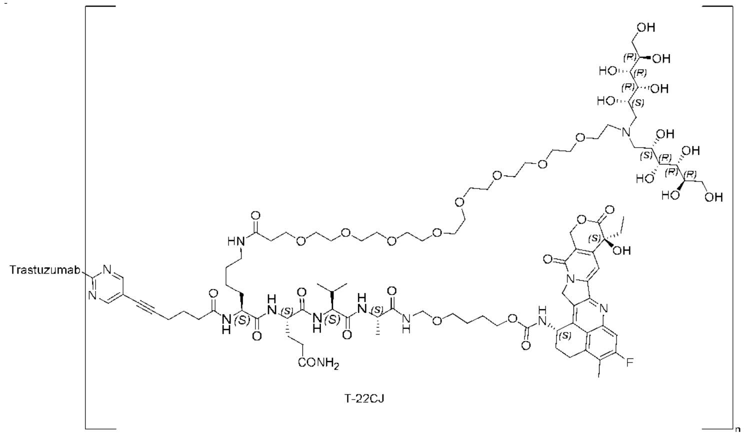 T-22CJ | 6.94 | 5 |

(continued)

| No. | Structure | DAR | Method |
|---|---|---|---|
| T-LY22CDX | 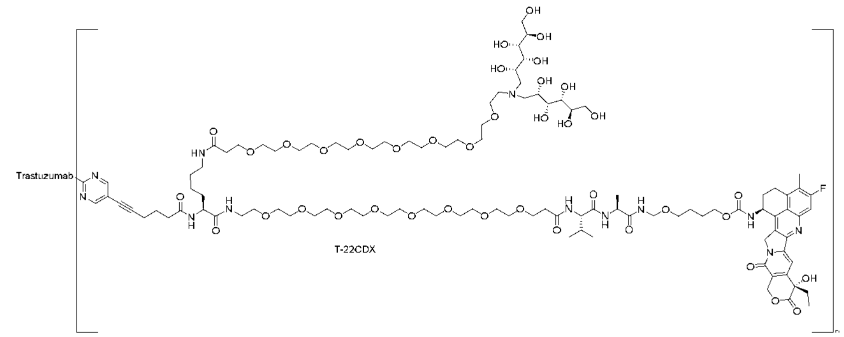 T-22CDX | 7.86 | 5 |
| T-LY22CDC | 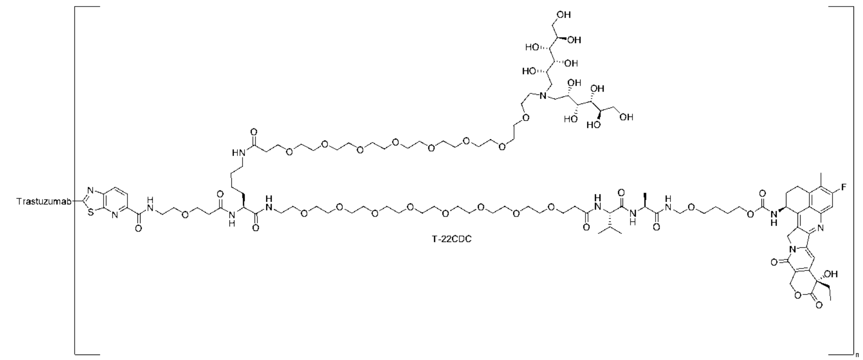 T-22CDC | 7.74 | 5 |
| T-LY22BM1 | 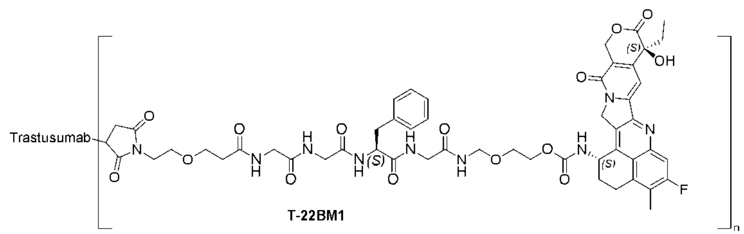 T-22BM1 | 7.92 | 3 |

(continued)

| No. | Structure | DAR | Method |
|---|---|---|---|
| T-LY22BM2 | 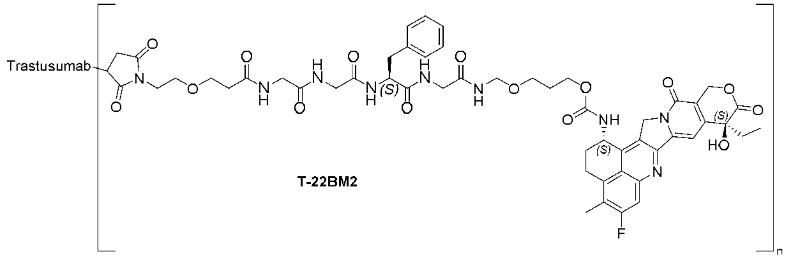 T-22BM2 | 7.89 | 3 |
| T-LY22BM3 | 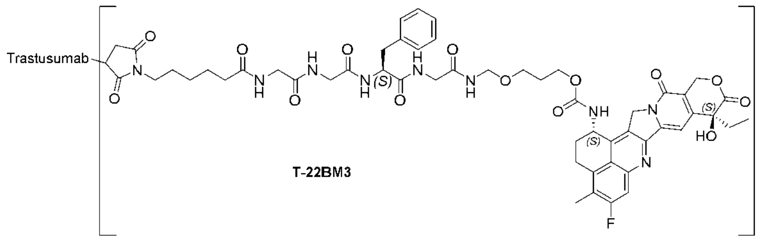 T-22BM3 | 7.94 | 3 |
| T-LY27 | 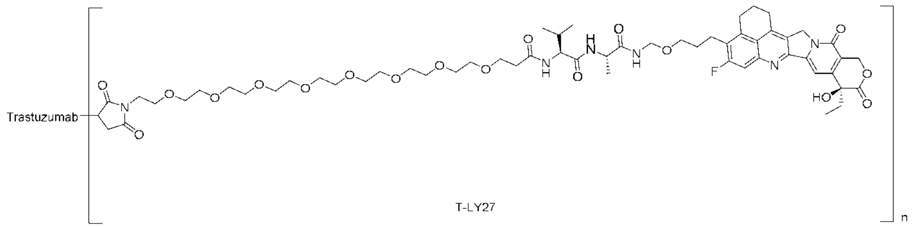 T-LY27 | 6.30 | 3 |
| T-LY28 | 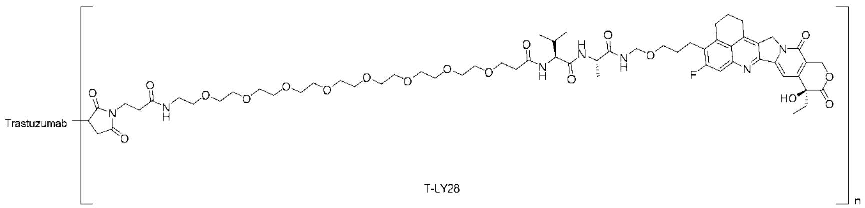 T-LY28 | 5.80 | 3 |

(continued)

| No. | Structure | DAR | Method |
|---|---|---|---|
| T-LY28B | 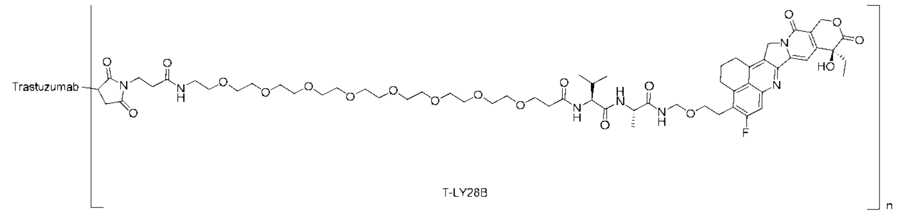 T-LY28B | 6.95 | 3 |
| T-LY28C | 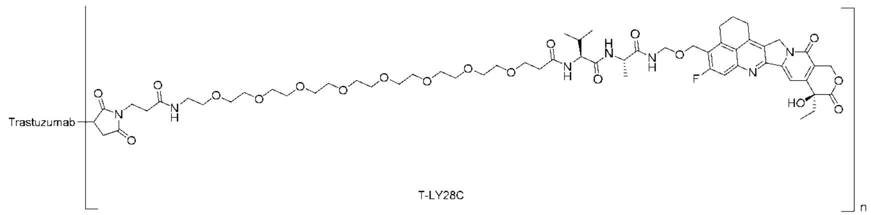 T-LY28C | 7.07 | 1A |
| T-LY29 | 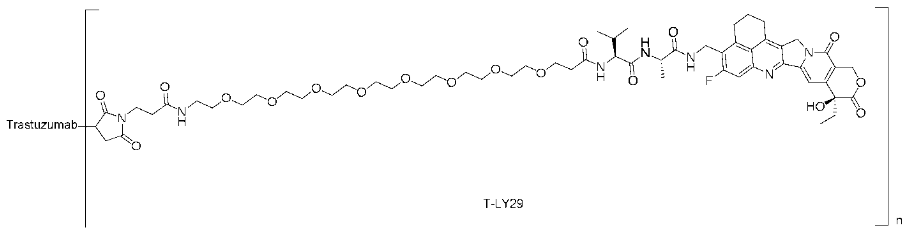 T-LY29 | 7.59 | 1A |
| T-LY29B | 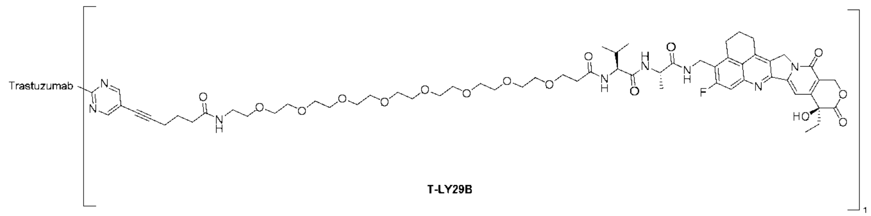 T-LY29B | 5.53 | 5 |
| T-LY29C | 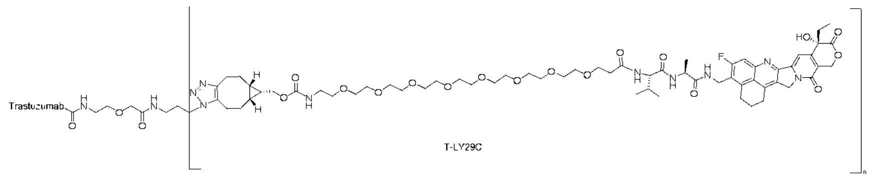 T-LY29C | 3.87 | 2 |

(continued)

| No. | Structure | DAR | Method |
|---|---|---|---|
| T-LY29D | 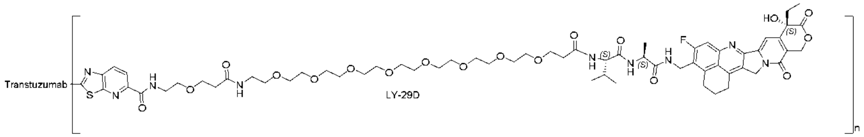 LY-29D | 4.01 | 2 |
| T-LY33 | 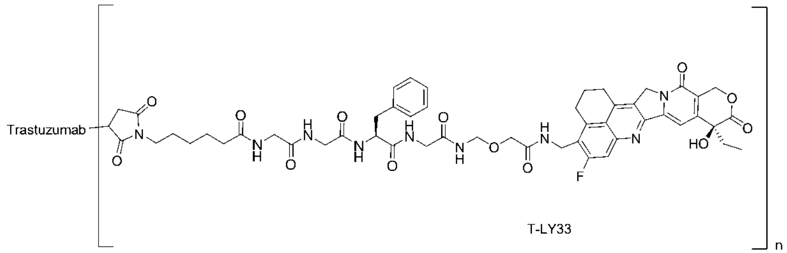 T-LY33 | 7.6 | 3 |
| T-LY33A | 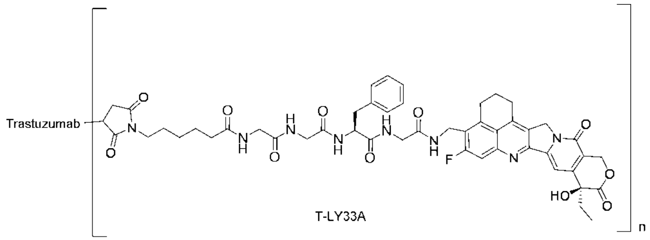 T-LY33A | 7.96 | 3 |
| T-LY34 | 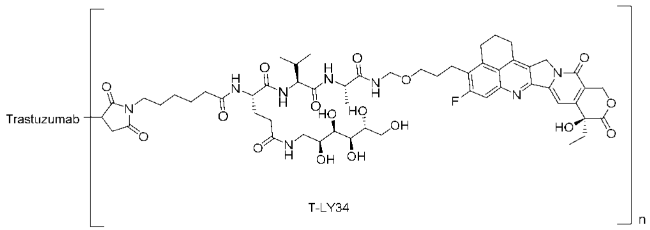 T-LY34 | 7.50 | 3 |

(continued)

| No. | Structure | DAR | Method |
|---|---|---|---|
| T-LY34B | 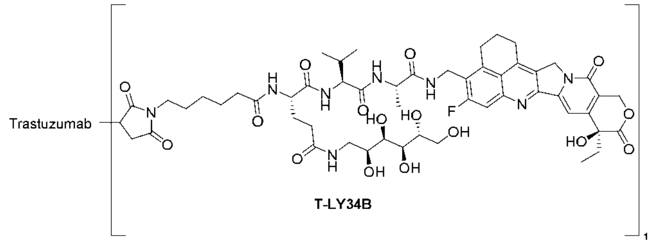 T-LY34B | 6.85 | 3 |
| T-LY34C | 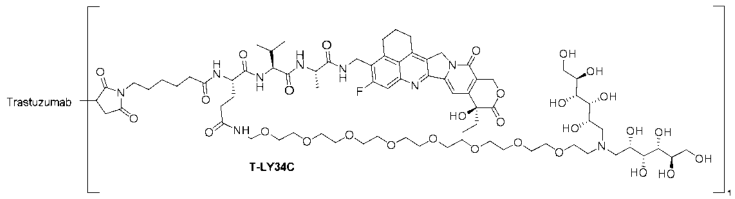 T-LY34C | 8.0 | 1A |
| T-LY35 | 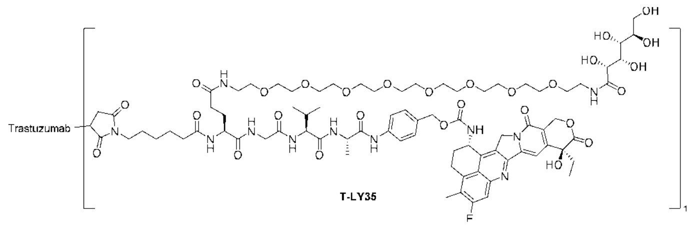 T-LY35 | 6.30 | 1A |
| T-LY37A | 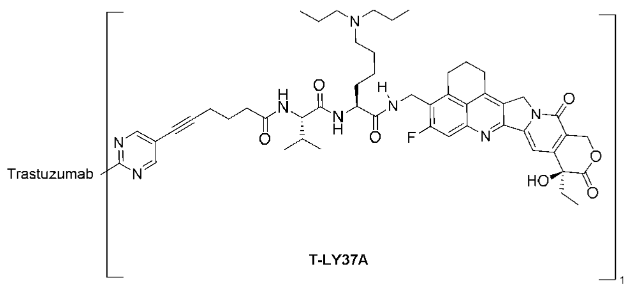 T-LY37A | 2.60 | 5 |

(continued)

| No. | Structure | DAR | Method |
|---|---|---|---|
| T-LY37B | 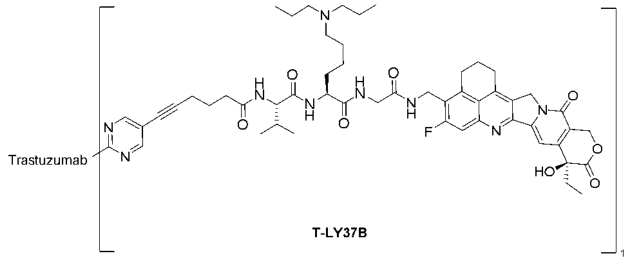 T-LY37B | 7.28 | 5 |
| T-LY39A | 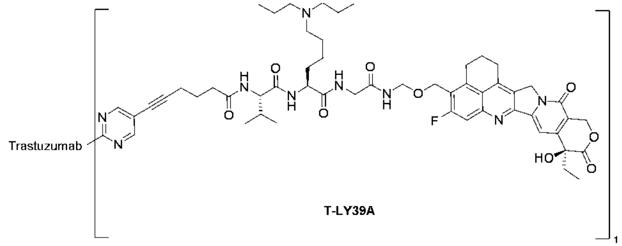 T-LY39A | 7.42 | 5 |
| T-LY39B | 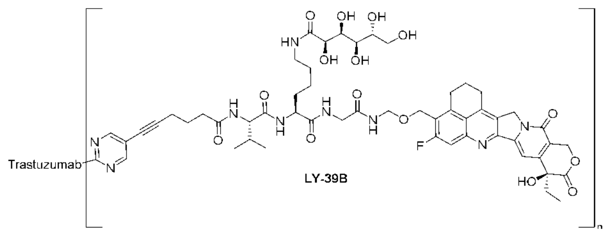 LY-39B | 7.25 | 5 |
| T-LY41 | 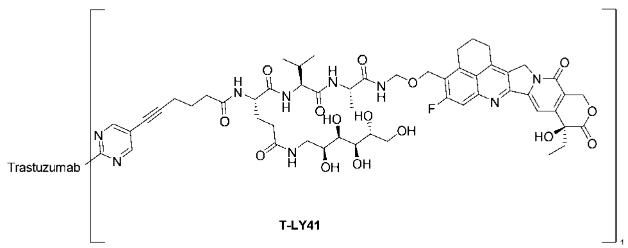 T-LY41 | 6.80 | 5 |

(continued)

| No. | Structure | DAR | Method |
|---|---|---|---|
| T-LY41B | 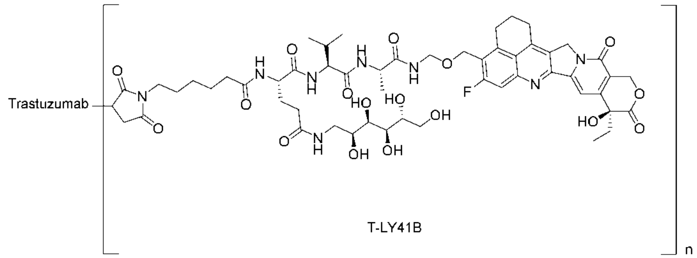 T-LY41B | 7.90 | 1A |
| T-LY43C | 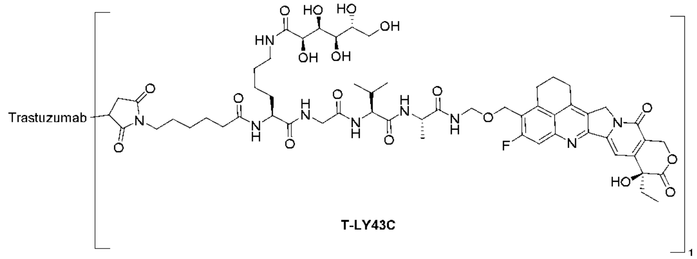 T-LY43C | 4.71 | 1B |
| T-LY43D | 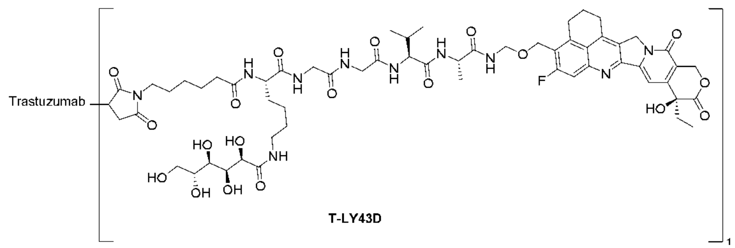 T-LY43D | 8.0 | 1A |
| T-LY43E | 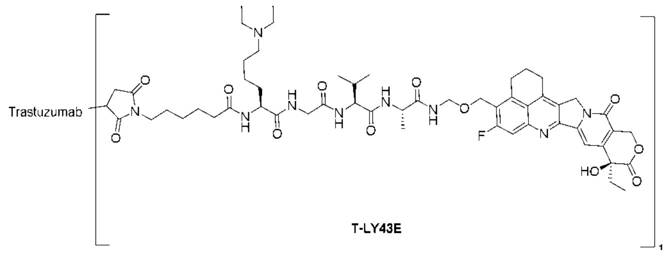 T-LY43E | 4.65 | 1B |

(continued)

| No. | Structure | DAR | Method |
|---|---|---|---|
| T-LY44A | 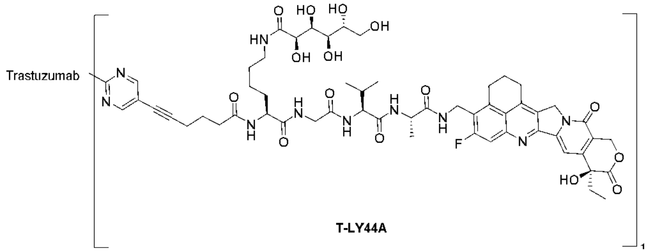 T-LY44A | 7.1 | 1A |
| T-LY44C | 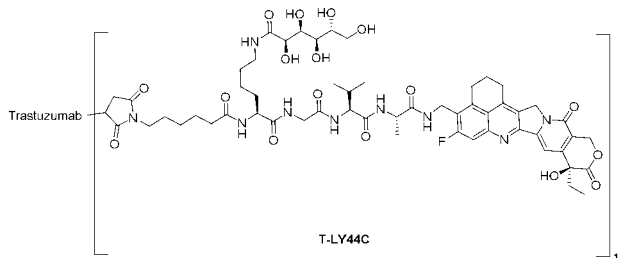 T-LY44C | 5.58 | 1A |
| T-LY44D | 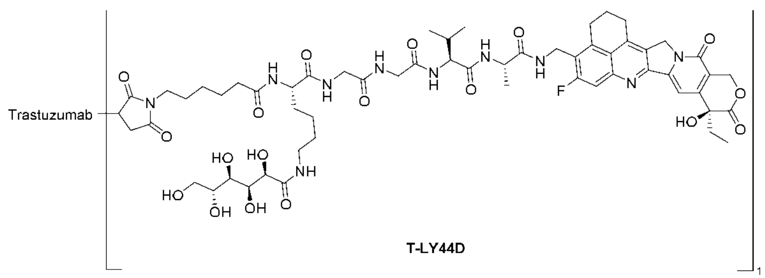 T-LY44D | 7.95 | 1A |
| T-LY46D | 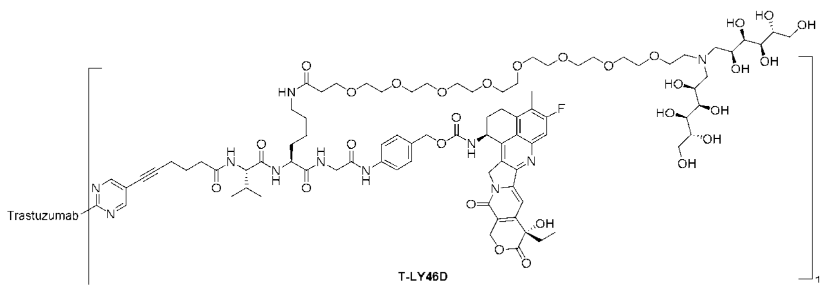 T-LY46D | 7.19 | 5 |

(continued)

| No. | Structure | DAR | Method |
|---|---|---|---|
| T-LY46E | 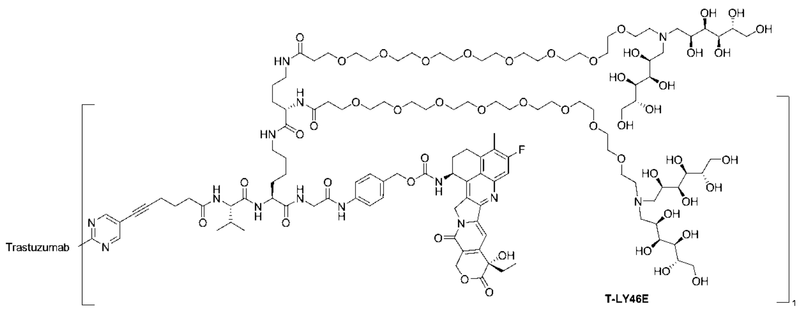 T-LY46E | 7.63 | 5 |
| T-LY46F | 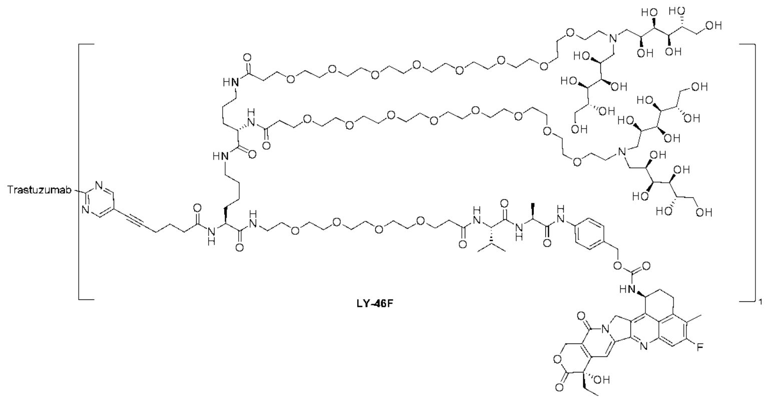 LY-46F | 7.65 | 5 |

(continued)

| No. | Structure | DAR | Method |
|---|---|---|---|
| T-LY46G | 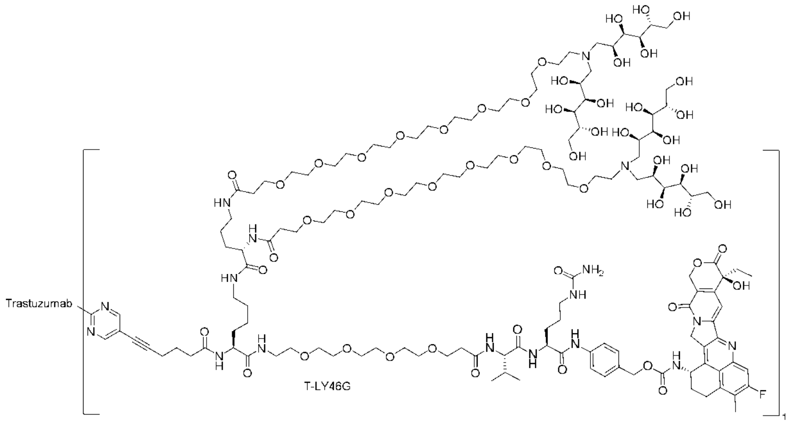 T-LY46G | 7.81 | 5 |
| T-LY47A | 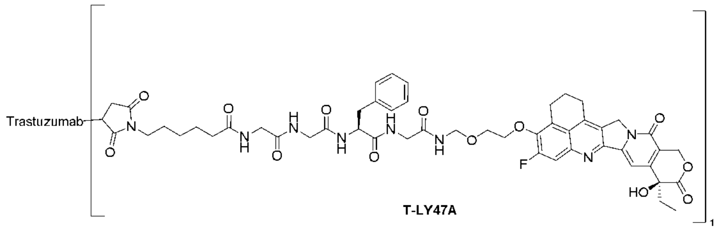 T-LY47A | 7.92 | 4 |
| T-LY47B | 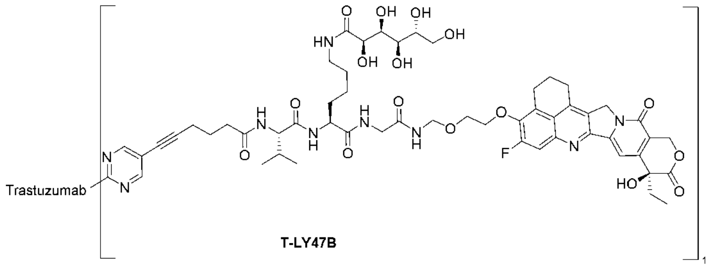 T-LY47B | 7.41 | 5 |

(continued)

| No. | Structure | DAR | Method |
|---|---|---|---|
| T-LY47C | 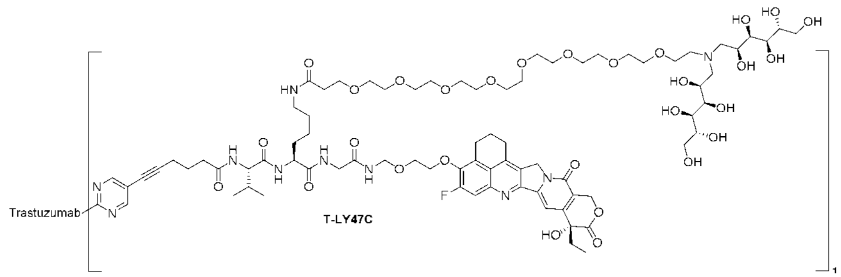 T-LY47C | 8.00 | 5 |
| T-LY47D | 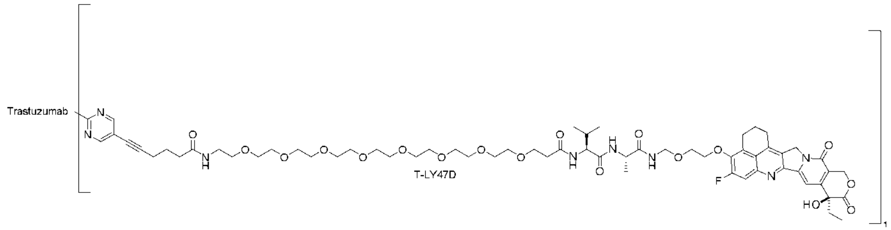 T-LY47D | 7.84 | 5 |
| T-LY47E | 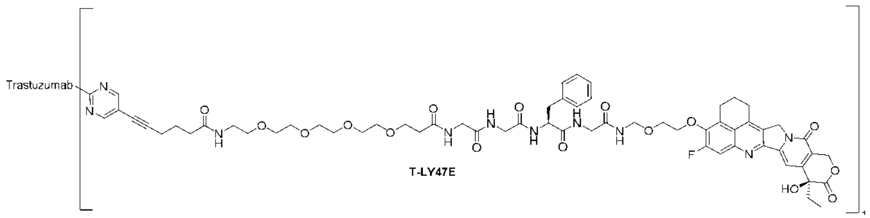 T-LY47E | 7.76 | 5 |
| T-LY47F | 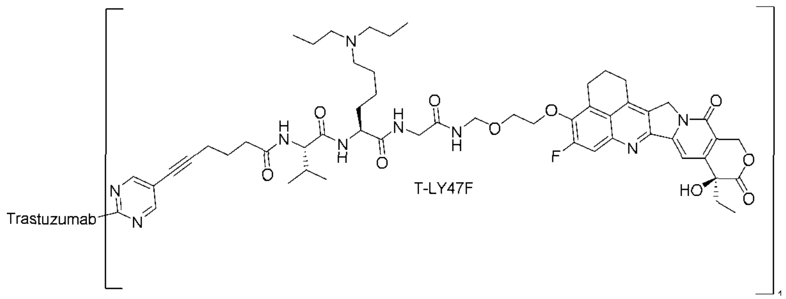 T-LY47F | 7.92 | 5 |

(continued)

| No. | Structure | DAR | Method |
|---|---|---|---|
| T-LY48A | 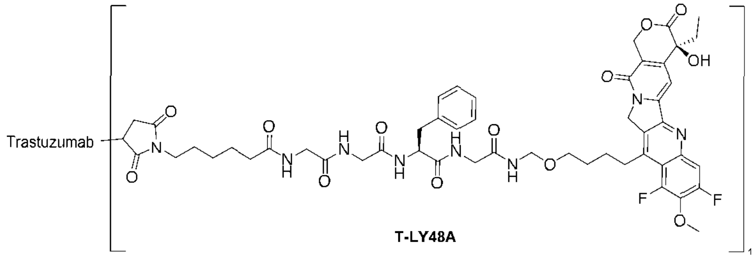 T-LY48A | 7.89 | 5 |
| T-LY48B | 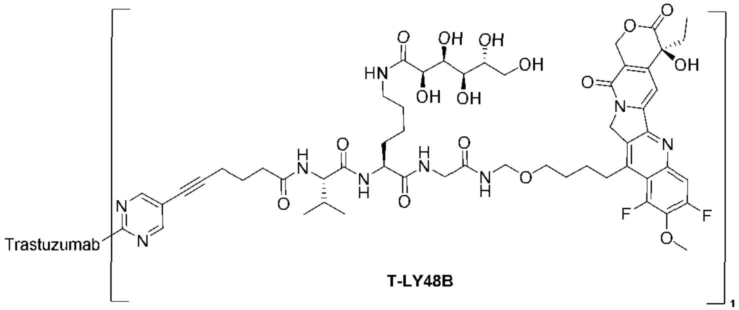 T-LY48B | 7.35 | 5 |
| T-LY48C | 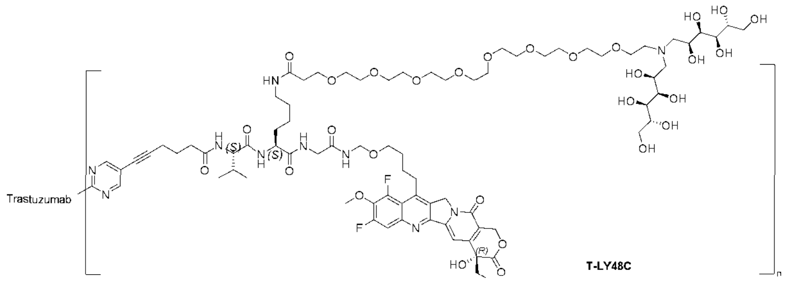 T-LY48C | 7.80 | 5 |
| T-LY48D | 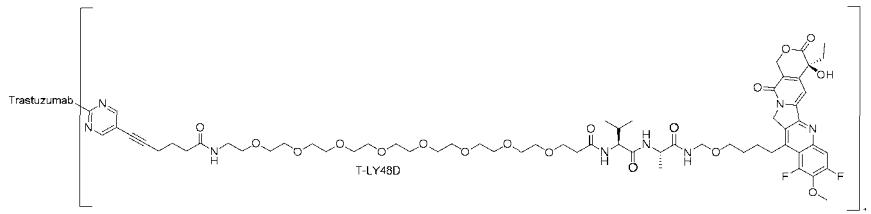 T-LY48D | 7.86 | 5 |

(continued)

| No. | Structure | DAR | Method |
|---|---|---|---|
| T-LY48E | 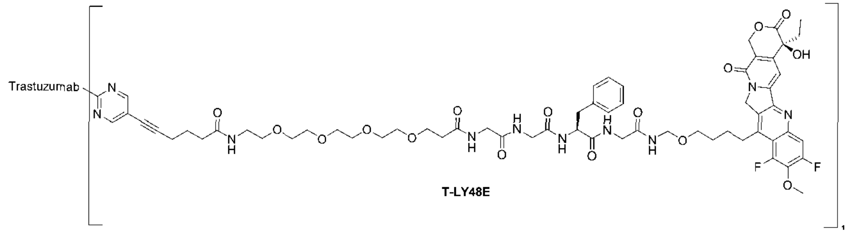 T-LY48E | 7.37 | 5 |
| T-LY48F | 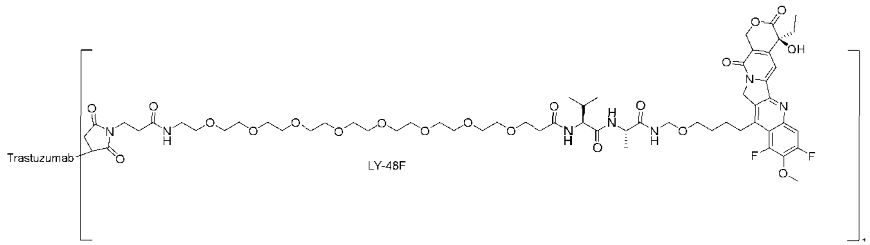 LY-48F | 7.83 | 1A |
| T-LY49B | 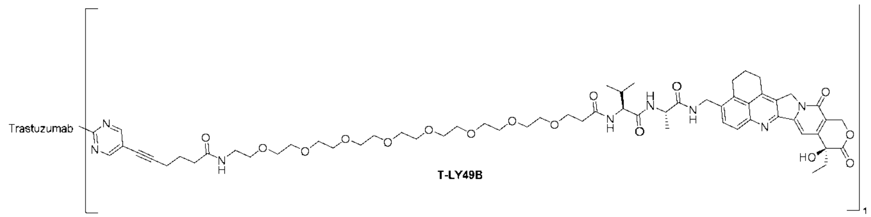 T-LY49B | 7.92 | 5 |
| T-LY50A | 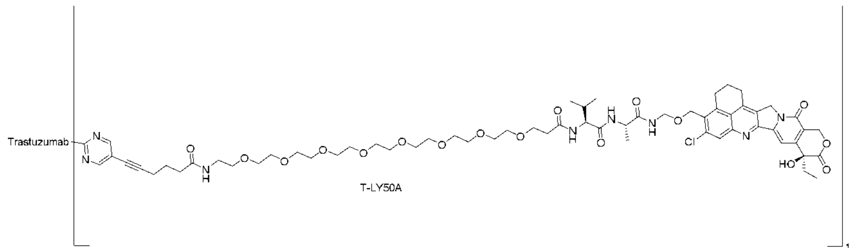 T-LY50A | 7.92 | 5 |

(continued)

| No. | Structure | DAR | Method |
|---|---|---|---|
| T-LY50B | 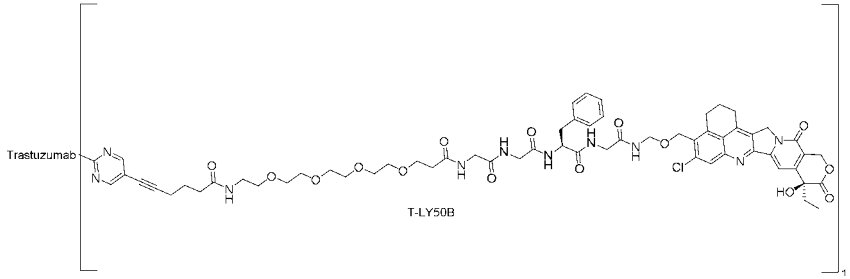 T-LY50B | 7.92 | 5 |
| T-LY51A | 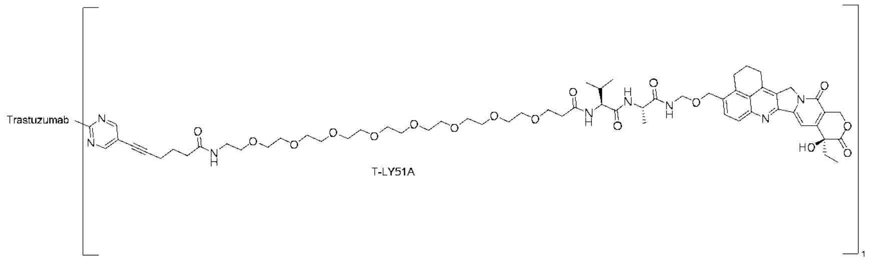 T-LY51A | 7.88 | 5 |
| T-LY51B | 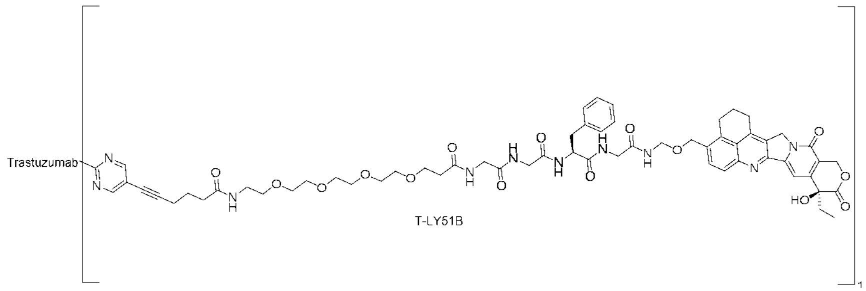 T-LY51B | 8.00 | 5 |

Bioassay

Test Example 1: Inhibitory Activity of Compounds of the Present Invention on Cancer Cells

N87 gastric cancer cells (Shanghai Cell Bank of Chinese Academy of Sciences, SCSP-534) were cultured in RPMI640 medium (Gibco, 22400-105) containing 10% FBS (Gibco, 10099-141). On the day of the experiment, the cells were digested with 0.25% trypsin (Gibco, 25200-072), filtered through a 40 μm sieve, diluted with the corresponding medium to a cell density of $1 \times 10^4$ cells/mL, and inoculated into a 96-well white cell culture plate (Beyotime, FCP968), with 100 μL of cells added per well. The culture plate was placed in a 37°C incubator (Thermo 311) containing 5% $CO_2$ and incubated for 24 hours. On the second day, the samples to be tested were prepared as a stock solution at a final concentration of 1M in DMSO (Sigma, D1650), then diluted with the corresponding medium to a final concentration of 200 nM, and subjected to four-fold serial dilutions, resulting in a total of 9 concentrations. 100 μL of the diluted sample was added to each well of the 96-well plate. The plate was placed in a 37°C incubator containing 5% $CO_2$ and incubated for 144 hours. The cell culture plate was then removed, and 100 μL of the cell viability assay reagent (CellTiter-Glo® Luminescent Cell Viability Assay, Promega, G7571) was added to each well. The reaction was carried out in the dark at room temperature for 5 minutes. Luminescence values were read using a microplate reader (TECAN SPARK), and data were processed using Graphpad Prism 7 software.

The $IC_{50}$ values for the inhibition of N87 gastric cancer cells and MDA-MB-468 breast cancer cells (China Academy of Sciences Shanghai Cell Bank, TCHu136) by the compounds of the present invention are shown in Table 1 below. The $IC_{50}$ value of DXD (PY-X) was set to 1, and $IC_{50}$ values of other compounds were multiples of DXD. As shown in Table 2, the inhibitory $IC_{50}$ effects of different toxins PY-10, PY-13, and PY-16 on different cell lines are also shown. "Multiple of the inhibitory $IC_{50}$ value compared to PY-X" refers to a ratio of the $IC_{50}$ value of another toxin to the $IC_{50}$ value of PY-X, and a greater value indicates that the cytotoxicity is stronger compared to PY-X. PY-E was exatecan mesylate, and PY-X was exatecan DXD (ChemExpress). PY-Y is referenced in the document (WO2022170971A1); and PY-A is referenced in the document (WO2021148501A1).

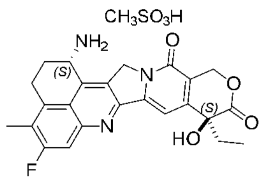

PY-E

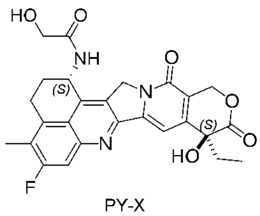

PY-X

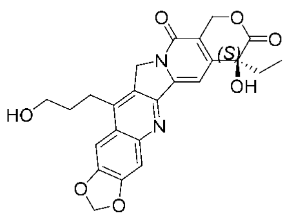

PY-Y

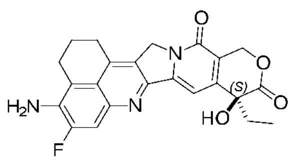

PY-A

Table 1 : $IC_{50}$ Values of Compounds in the Present Invention on N87 Gastric Cancer Cells and MDA-MB-468 Breast Cancer Cells

| No. | PY-E | PY-X (DXD)[a] | PY-1 | PY-1AB | PY-4 | PY-4Car | PY-4Car2 | PY-5 |
|---|---|---|---|---|---|---|---|---|
| N87 cells | 0.4782 | 1.0000 | 0.1589 | 0.2668 | 1.1725 | 0.3427 | 0.5294 | 2.3591 |
| 468' cells | 0.3546 | 1.0000 | 0.0931 | 0.3750 | 2.2546 | 0.3552 | 0.5959 | 3.4395 |
| No. | PY-8 | PY-8B | PY-9 | PY-10 | PY-10d | PY-27(10Car) | PY-10B | PY-11 |
| N87 cells | 0.1486 | 0.3360 | 0.4712 | 0.0904 | 0.0602 | 3.3500 | 0.1623 | 0.0606 |
| 468' cells | 0.4035 | 2.3133 | 1.0622 | 0.2283 | 0.3129 | 3.7610 | 0.4362 | 0.9642 |
| No. | PY-12A | PY-12B | PY-13 | PY-14 | PY-16 | PY-16Car | PY-17 | PY-18 |
| N87 cells | 1.6441 | 5.3044 | 0.0852 | 4.3321 | 0.2446 | 2.9600 | 3.7859 | 0.0938 |

(continued)

| No. | PY-12A | PY-12B | PY-13 | PY-14 | PY-16 | PY-16Car | PY-17 | PY-18 |
|---|---|---|---|---|---|---|---|---|
| 468' cells | 3.3035 | 8.4257 | 0.0798 | 10.5958 | 0.3458 | 2.7200 | 8.0396 | 0.2154 |
| No. | PY-19 | PY-20 | PY-21A | PY-24 | PY-25A | PY-25B | PY-26 | PY-29 |
| N87 cells | 0.7040 | 1.0070 | 0.7361 | 1.5288 | 9.0899 | 2.9428 | 6.0638 | 8.4714 |
| 468' cells | 0.7356 | 1.2806 | 1.4821 | 2.2057 | 8.4260 | 1.3101 | 5.8345 | 176.8753 |
| No. | PY-29B | PY-36 | PY-37 | PY-37A | PY-37B | PY-38 | PY-Y | PY-A |
| N87 cells | 0.5572 | 0.8205 | 0.6030 | 0.4267 | 6.728 | 0.3502 | 0.2989 | 0.0662 |
| 468' cells | 1.1096 | 1.0551 | 0.8515 | 0.5446 | 17.58 | 0.8774 | 0.4878 | 0.0828 |
| No. | PY-4Car[b] | PY-4Car3C[b] | PY-4Car2[b] | | | | | |
| N87 cells | 0.3112 | 0.3593 | 0.5046 | | | | | |
| 468' cells | 0.5193 | 0.5740 | 0.8170 | | | | | |
| a: $IC_{50}$ value of PY-X (DXD) is set to 1, and $IC_{50}$ values of toxins in the table are ratios of their measured values to DXD; b: samples are retested together | | | | | | | | |

The inhibitory $IC_{50}$ values of the compounds in the present invention obtained using different cells according to the above similar methods are shown in Table 2-1 and Table 2-2 below.

Table 2-1: IC50 Values of Compounds in the Present Invention in Different Cells

| Cell names | Cell sources | IC50[1] values | | | |
|---|---|---|---|---|---|
| | | **PY-4CAR** | **PY-16** | **PY-10** | **PY-13** |
| H358 | NSCLC | 0.3935 | 0.2592 | 0.1289 | 0.1062 |
| H82 | SCLC | 0.4523 | 0.3391 | 0.1525 | 0.1364 |
| H1650 | NSCLC | 0.4491 | 0.1421 | 0.0456 | 0.0415 |
| H1975 | NSCLC | 0.3814 | 0.0716 | 0.0655 | 0.0426 |
| H1437 | NSCLC | 0.6516 | 0.2768 | 0.1209 | 0.0969 |
| H23 | NSCLC | 0.7911 | 0.1634 | 0.0895 | 0.0819 |
| SW1573 | NSCLC | 0.3187 | 0.2256 | 0.1540 | 0.1570 |
| PANC-1 | Pancreatic | 0.4763 | 0.1325 | 0.0915 | 0.0810 |
| Colo-205 | Colon | 0.2923 | 0.3082 | 0.1546 | 0.1643 |
| HT-29 | Colon | 0.3312 | 0.1933 | 0.0874 | 0.1434 |
| A549 | NSCLC | 0.4832 | 0.2053 | 0.0792 | 0.0899 |
| FADU | HNSCC | 0.4574 | 0.4215 | 0.1913 | 0.1648 |
| OVCAR3 | Ovarian | 0.2626 | 0.2646 | 0.0681 | 0.0560 |
| PA-1 | Ovarian | 1.0605 | 0.4925 | 0.1746 | 0.1600 |
| SW1990 | Pancreatic | 0.4822 | 0.4346 | 0.2133 | 0.1255 |
| DMS53 | SCLC | 0.5151 | 0.5137 | 0.1968 | 0.1624 |
| a: $IC_{50}$ value or PY-X (DXD) is set to 1, and $IC_{50}$ values of toxins in the table are ratios of their measured values to DXD. | | | | | |

Table 2-2: $IC_{50}$ Values of Compounds in the Present Invention in Different Cells

| Cell names | Cell sources | IC50 values | | | | | | |
|---|---|---|---|---|---|---|---|---|
| | | **PY-E** | **PY-4Car2** | **PY-37** | **PY-37A** | **PY-36** | **PY-29B** | **PY-09** |
| H358 | NSCLC | 0.2694 | 0.6362 | 0.7007 | 0.3022 | 1.1695 | 0.7611 | 0.3899 |
| H82 | SCLC | 0.5103 | 0.5803 | 1.1563 | 0.4153 | 1.5585 | | |
| H1650 | NSCLC | 0.1570 | 0.7384 | 0.7310 | 0.25611 | 0.8758 | | |
| H1975 | NSCLC | 0.0963 | 0.7358 | 0.6276 | 0.1711 | 0.5909 | 0.3232 | 0.1922 |
| H1437 | NSCLC | 0.2303 | 0.9590 | 0.9440 | 0.2619 | 1.1187 | | |
| H23 | NSCLC | 0.1245 | 1.1508 | 0.6637 | 0.1785 | 0.5677 | 0.4416 | 0.2937 |
| SW1573 | NSCLC | 0.3310 | 0.4766 | 0.6712 | 0.2884 | 0.7619 | | |
| PANC-1 | Pancreatic | 0.1086 | 0.4865 | 0.3514 | 0.1992 | 0.6317 | | |
| Colo-205 | Colon | 0.2938 | 0.4478 | 0.6449 | 0.2763 | 0.9857 | | |
| HT-29 | Colon | 0.1376 | 0.4854 | 0.4700 | 0.2181 | 0.4909 | | |
| A549 | NSCLC | 0.1645 | 0.5433 | 0.4179 | 0.2044 | 0.4500 | | |
| FADU | HNSCC | 0.4464 | 0.8139 | 0.9803 | 2.9002 | 1.7031 | 1.6627 | 0.6274 |
| OVCAR3 | Ovarian | 0.2269 | 0.3491 | 0.4392 | 0.0793 | 0.7701 | 0.5623 | 0.2965 |
| PA-1 | Ovarian | 0.3572 | 1.3950 | 0.8566 | 0.4153 | 1.1505 | 0.8427 | 0.5130 |
| SWL990 | Pancreatic | 0.3581 | 0.3573 | 0.7777 | 0.2986 | 1.3305 | | |
| a $IC_{50}$ value of PY-X (DXT) is set to 1 and $IC_{50}$ values of toxins in the table are ratios of their measured values to DXD. | | | | | | | | |

Test Example 2: Inhibitory Activity of ADC of the Present Invention on Cancer Cells

1) Test Steps

Day 0: Cells in good growth condition were digested and filtered, then counted. 2000 cells/100μL medium (N87 cells) were added to each well of a 96-well plate. 1000 cells/100μL medium were added to each well of a 96-well plate (468 cells, N87 or HCC1954). 200μL sterile water or PBS was added to the outer wells and incubated for 24 hours.

Day 1: Samples in the medium were gradient diluted (9 gradients + 1 negative control). For N87, Payload or ADC was plated: Plate 1 (T-LY21, T-LY29, T-LY33), Plate 2 (T-LY33A, T-LY22, T-LY22C), Plate 3 (T-LY34, T-LY34B, T-LY35), Plate 4 (T-LY21C, T-LY28B, T-LY28C), Plate 5 (T-LY43C, T-LY43D, T-LY43E). Each sample had two parallel controls. The initial concentration was 100 nM, followed by 8 subsequent wells with 4-fold serial dilutions. 100 μL of the corresponding sample was added to each well of the Day 0 plate, with the 11th column as the control group with culture medium. Each sample had two parallel controls with an initial concentration of 1000 nM, followed by 8 subsequent wells with 4-fold serial dilutions. 100 μL of the corresponding sample was added to each well of the Day 0 plate, with the 11th column as the control group with culture medium.

Day 7: After 144 hours of cell treatment, the reagent CellTiter-Glo® was dissolved 30 minutes in advance. 100μL of the reagent was added to each well, protected from light for 2-5 minutes, and the results were read twice to obtain the average.

Table 3: The inhibitory $IC_{50}$ values of ADC on N87 gastric cancer cells and MDA-MB-468 as well as HCC1954 breast cancer cells

| ADC No. | DAR | Inhibitory $IC_{50}$ Values (nM) | | |
|---|---|---|---|---|
| | | N87 | 468 | HCC1954 |
| T-BY1 | 7.99 | 0.126 | 49.44 | |
| T-BY1B | 7.66 | 0.237 | 32.2 | |
| T-BY1C | 7.54 | 0.252 | 35.83 | |

(continued)

| ADC No. | DAR | Inhibitory $IC_{50}$ Values (nM) | | |
|---|---|---|---|---|
| | | N87 | 468 | HCC1954 |
| T-BY2 | 6.81 | 0.1382 | 157 | |
| T-BY3P1 | 7.9 | >100 | 301.7 | |
| T-BY3P2 | 7.82 | 0.1545 | 128.5 | |
| T-LY1 | / | 1.793 | 8.242 | |
| T-LY2 | / | 6.579 | 57.05 | |
| T-LY8 | 6.92 | 0.06801 | 55.31 | |
| T-LY10 | 8 | 8.088 | 603.7 | |
| T-LY21 | 5.4 | 8.088 | / | 603.7 |
| T-LY21B | 6.25 | 0.13 | 25.3 | 0.1764 |
| T-LY21C | 7.94 | 0.0622 | 19.97 | |
| T-LY22 | 7.7 | 0.0606 | 31.73 | |
| T-LY22AD | 7.76 | 0.1446 | 412.1 | |
| T-LY22AF | 7.62 | 0.09849 | 126.2 | |
| T-LY22C | 7.69 | 0.5088 | 17.89 | |
| T-LY22CA | 7.08 | 0.1971 | 39.4 | |
| T-LY22CB | 7.8 | 0.3026 | 345.8 | |
| T-LY22CD | 7.43 | 0.4744 | 184.6 | |
| T-LY22CD-2 | 7.8 | 0.3559 | 163.1 | |
| T-LY22CD-3 | 7.6 | 0.3336 | 200.3 | |
| T-LY22CE | 7.85 | 0.3156 | 252.9 | |
| T-LY22CF | 7.7 | 0.4258 | 185.2 | |
| T-LY22CG | 7.90 | 0.4286 | 187.3 | |
| T-LY22CH | 7.51 | 0.4998 | 116.3 | |
| T-LY22CI | 7.92 | 0.4209 | 90.6 | |
| T-LY22CJ | 6.94 | 1.668 | 56.51 | |
| T-LY22CDX | 7.86 | 0.4163 | 139.9 | |
| T-LY22CDC | 7.74 | 0.4791 | 83.11 | |
| T-LY22BM1 | 7.92 | 0.2072 | 58.95 | |
| T-LY22BM2 | 7.89 | 0.2942 | 89.57 | |
| T-LY22BM3 | 7.94 | 0.3884 | 77.47 | |
| T-LY28B | 6.95 | 0.0924 | 44.07 | |
| T-LY28C | 7.07 | 0.0681 | 55.31 | |
| T-LY29 | 7.59 | 0.0537 | 165.4 | |
| T-LY29B | 7.62 | 0.0754 | 104.9 | |
| T-LY29C | 7.75 | 0.0945 | 45.68 | |
| T-LY29D | 4.01 | 0.0577 | 174.1 | |
| T-LY33 | 7.6 | 0.5647 | / | -0.09825 |
| T-LY33A | 7.54 | 0.0547 | 49.62 | 0.02866 |

(continued)

| ADC No. | DAR | Inhibitory $IC_{50}$ Values (nM) | | |
|---|---|---|---|---|
| | | N87 | 468 | HCC1954 |
| T-LY34 | 7.5 | 0.0442 | 73.57 | |
| T-LY34B | 6.85 | 0.07023 | 59.5 | 0.3278 |
| T-LY34C | 7.5 | 0.06872 | 15.61 | |
| T-LY35 | 6.3 | 0.1216 | / | 0.3505 |
| T-LY37B | 7.26 | 0.5173 | 186.1 | |
| T-LY39A | 7.42 | 0.0880 | 40.9 | |
| T-LY39B | 7.25 | 0.0841 | 114.8 | |
| T-LY43C | 8 | 0.1078 | 3.899 | |
| T-LY43D | 8 | 0.0924 | 15.96 | |
| T-LY43E | 8 | 0.0811 | 2.826 | |
| T-LY44C | 5.58 | 0.08413 | 114.8 | |
| T-LY44D | 7.95 | 0.08243 | 14.45 | |
| T-LY46D | 7.15 | 0.4295 | 110.7 | |
| T-LY46E | 7.63 | 0.8186 | 94.67 | |
| T-LY46E | 7.65 | 0.6967 | 333 | |
| T-LY46G | 7.81 | 0.3705 | 131 | |
| T-LY47A | 7.62 | 0.10 | 28.22 | |
| T-LY47B | 7.53 | 12.71 | 21.19 | |
| T-LY47C | 7.24 | 0.877 | 73.57 | |
| T-LY47D | 7.84 | 0.6118 | 396.14 | |
| T-LY47E | 7.76 | 0.4615 | \ | |
| T-LY47F | 7.92 | 0.2761 | 201.6 | |
| T-LY48A | 7.68 | 0.16 | 79.54 | |
| T-LY48B | 7.74 | 15.76 | 48.75 | |
| T-LY48C | 7.65 | 0.877 | 73.57 | |
| T-LY48D | 7.68 | 0.9574 | 419.35 | |
| T-LY48E | 7.58 | 1.2150 | 382.52 | |
| T-LY48F | 7.83 | 0.3716 | 254 | |
| T-LY50A | 7.92 | 0.1972 | 161.4 | |
| T-LY50B | 7.92 | 0.2441 | 290.5 | |
| T-LY51A | 7.88 | 0.1755 | 69.63 | |
| T-LY51B | 8 | 0.1054 | 67.87 | |
| Note: DAR values were adjusted to DAR8 during cell inhibition experiments. | | | | |

Test Example 3: Efficacy Experiment of Antibody-Drug Conjugates in Human Gastric Cancer NCI-N87 Xenograft Model

The human gastric cancer NCI-N87 cell line (ATCC, CTL-5822) was cultured in RPMI-1640 medium (Gibco, catalog number: 22400-089, containing 10% FBS (Excell Bio, catalog number: FCS500)) in a 37°C incubator with 5% $CO_2$. Cells were digested with 0.25% trypsin-EDTA, washed with PBS twice, counted by a cell counter, and diluted with PBS to a

cell concentration of 1x$10^8$ cells/mL before ten consecutive generations of cells were cultured.

CB17 SCID mice (female, 4-6 weeks of age, Beijing Vital River Laboratory Animal Technology Co., Ltd.) were used. CB17 SCID mice were acclimated to the laboratory environment for 2-5 days, NCI-N87 cells were inoculated subcutaneously into the right flank of each mouse at a dose of 5x$10^6$ cells/mouse in a volume of 0.1 mL (containing 50% Matrigel). When the tumor grew to approximately 200 $mm^3$, drug administration was performed according to the scheme shown in Table 4, and DAR values were all adjusted to 8.

Table 4

| No. | Group | Number of animals | Dosage | Route of administration and administration period |
|---|---|---|---|---|
| 1 | PBS | 6 | - | i.v. Once |
| 2 | T-BY1 | 6 | 1 mg/kg | i.v. Once |
| 3 | T-LY10 | 6 | 1 mg/kg | i.v. Once |
| 4 | T-LY46D | 6 | 1 mg/kg | i.v. Once |
| 5 | T-LY22 | 6 | 1 mg/kg | i.v. Once |
| 6 | T-LY22CD | 6 | 1 mg/kg | i.v. Once |
| 7 | T-LY44D | 6 | 1 mg/kg | i.v. Once |
| 8 | T-LY43D | 6 | 1 mg/kg | i.v. Once |
| 9 | T-LY47D | 6 | 1 mg/kg | i.v. Once |
| 10 | T-LY48D | 6 | 1 mg/kg | i.v. Once |

Tumor volumes and body weight were measured twice a week, and the tumor proliferation rate and relative tumor inhibition rate were calculated. The calculation formulas are as follows:

$$\text{Relative tumor proliferation rate T/C(\%)} = T_{RTV} / C_{RTV} \times 100\%$$

($T_{RTV}$: average RTV of the treatment group; $C_{RTV}$: average RTV of the control group; RTV = $V_t/V_O$, where $V_O$ is the tumor volume of the animal at the time of grouping, and Vt is the tumor volume of the animal after treatment);

$$\text{Relative tumor inhibition rate TGI\%} = (1\text{-T/C}) \times 100\%$$

(T and C are relative tumor volumes (RTV) of the treatment and control groups at a specific time point, respectively);

The formula for calculating the tumor volume (V): $V = 1/2 \times L_L \times L_S^2$

where $L_L$ and $L_S$ represent the long and short diameters of the tumor, respectively. At the end of the experiment, the mice were sacrificed and the tumors were cut off and weighed.

The experimental results are shown in Table 5 and FIG. 1.

Table 5: Tumor Volume Data of NCI-N87 Gastric Cancer Mouse Model

| | Tumor Volume $mm^1$ | | | | | | | | T.C(%) | TGI% |
|---|---|---|---|---|---|---|---|---|---|---|
| Samples | 0 days after dosing | 7 days after dosing | 11 days after dosing | 14 days after dosing | 18 days after dosing | 21 days after dosing | 24 days after dosing | 27 days after dosing | 27 days after dosing | 27 days after dosing |
| PBS | 203.91 | 269.21 | 314.34 | 394.15 | 438.09 | 525.40 | 614.61 | 705.18 | - | - |
| T-BY1 | 204.14 | 83.77 | 123.0 | 108.05 | 108.00 | 149.44 | 148.70 | 149.73 | 18.65 | 81.35 |
| T-LY10 | 205.09 | 90.05 | 78.10 | 66.55 | 74.38 | 91.34 | 83.88 | 90.73 | 10.16 | 89.84 |
| T-LY46D | 205.01 | 61.90 | 62.17 | 34.34 | 29.87 | 35.75 | 32.03 | 45.86 | 5.92 | 94.18 |
| T-LY22 | 204.33 | 42.28 | 30.30 | 20.06 | 17.19 | 15.61 | 11.05 | 15.22 | 1.93 | 98.07 |
| T-LY22CD | 204.22 | 20.06 | 16.91 | 5.45 | 4.26 | 4.23 | 4.74 | 6.50 | I.03 | 98.97 |
| T-LY44D | 204.05 | 41.32 | 42.76 | 23.23 | 11.53 | 16.56 | 16.03 | 17.77 | 2.21 | 97.79 |

(continued)

| | Tumor Volume mm1 | | | | | | | | T.C(%) | TGI% |
|---|---|---|---|---|---|---|---|---|---|---|
| Samples | 0 days after dosing | 7 days after dosing | 11 days after dosing | 14 days after dosing | 18 days after dosing | 21 days after dosing | 24 days after dosing | 27 days after dosing | 27 days after dosing | 27 days after dosing |
| T-LY43D | 204.51 | 30.90 | 23.85 | 14.51 | 8.53 | 5.29 | 5.56 | 9.55 | 1.19 | 98.81 |
| T-LY47D | 204.76 | 28.86 | 22.29 | 29.92 | 19.59 | 18.49 | 16.02 | 26.39 | 3.21 | 96.79 |
| T-LY48D | 203.76 | 55.11 | 51.90 | 62.47 | 68.01 | 100.37 | 95.19 | 105.25 | 13.03 | 86.97 |

Results: On Day 27, the average tumor volume of the vehicle group (PBS) reached 705 $mm^3$, and the average tumor volume of each treated group was less than 200 $mm^3$. In addition, the relative tumor inhibition rates of T-LY10, T-L46D, T-LY22, T-LY22CD, T-LY44D, T-LY43D, T-LY47D, and T-LY48D were better than that of the T-BY1 control group. During the treatment period, no significant body weight loss or notable drug toxicity was observed in any group.

Conclusion: The compound of the present invention has good tumor inhibition activity.

Test Example 4: Efficacy Experiment of Antibody-Drug Conjugates in the Human Breast Cancer JIMT-1 Xenograft Model

The human breast cancer JIMT-1 cell line (Cobioer, CBP60378) was cultured in DMEM medium (Gibco, catalog number: 11995-065) and 10% FBS (Excell Bio, catalog number: FCS500) in a 37°C incubator with 5% $CO_2$. Cells were digested with 0.25% trypsin-EDTA, washed with PBS twice, counted by a cell counter, and diluted with PBS to a cell concentration of $1x10^8$ cells/mL.

CB17 SCID mice (female, 4-6 weeks of age, Beijing Vital River Laboratory Animal Technology Co., Ltd.) were used. CB17 SCID mice were acclimated to the laboratory environment for 2-5 days, JIMT-1 cells were inoculated subcutaneously into the right flank of each mouse at a dose of $5x10^6$ cells/mouse in a volume of 0.1 mL (containing 50% Matrigel). When the tumor grew to 150-200 $mm^3$, drug administration was performed according to the scheme shown in Table 6. Tumor volumes and body weight were measured twice a week, and the tumor proliferation rate and relative tumor inhibition rate were calculated.

Table 6

| No. | Group | Number of animals | Dosage | Route of administration and administration period |
|---|---|---|---|---|
| 1 | PBS | 6 | - | i.v. Twice |
| 2 | T-BY1 | 6 | 3 mg/kg | i.v. Twice |
| 3 | T-LY22 | 6 | 3 mg/kg | i.v. Twice |
| 4 | T-LY22BM1 | 6 | 3 mg/kg | i.v. Twice |
| 5 | T-LY22BM2 | 6 | 3 mg/kg | i.v. Twice |
| 6 | T-LY22BM3 | 6 | 3 mg/kg | i.v. Twice |
| 7 | T-LY22C | 6 | 3 mg/kg | i.v. Twice |
| 8 | T-LY22CD | 6 | 3 mg/kg | i.v. Twice |
| 9 | T-LY22CD-3 | 6 | 3 mg/kg | i.v. Twice |
| 10 | T-LY22CDC | 6 | 3 mg/kg | i.v. Twice |

The experimental results are shown in Table 7 and FIG. 2.

Table 7: Tumor Volume Data of JIMT-1 Breast Cancer Mouse Model

| Samples | Tumor volume ($mm^2$) | | | | | | | | TC(%) | TGI(%) |
|---|---|---|---|---|---|---|---|---|---|---|
| | 0 days after dosing | 4 days after dosing | 8 days after dosing | 13 days after dosing | 15 days after dosing | 19 days after dosing | 22 days after dosing | 25 days after dosing | 25 days after dosing | 25 days after dosing |
| PBS | 163.47 | 219.48 | 290.36 | 352.15 | 385.55 | 442.62 | 476.97 | 587.87 | | |
| T-BY1 | 164.38 | 159.07 | 168.66 | 130.09 | 135.92 | 142.86 | 149.41 | 203.64 | 34.45 | 65.55 |
| T-LY22 | 162.87 | 201.05 | 162.31 | 152.50 | 135.29 | 117.52 | 158.23 | 221.09 | 37.75 | 62.25 |
| T-LY22BM1 | 163.53 | 171.76 | 175.77 | 143.63 | 141.19 | 137.48 | 138.70 | 197.89 | 33.65 | 66.35 |
| T-LY22BM2 | 163.01 | 179.39 | 141.33 | 136.68 | 141.02 | 120.77 | 119.14 | 161.00 | 27.46 | 72.54 |
| T-LY22BM3 | 163.03 | 197.76 | 145.53 | 133.48 | 119.87 | 92.57 | 96.78 | 154.02 | 26.27 | 73.73 |
| T-LY22C | 164.10 | 163.16 | 154.58 | 115.43 | 104.12 | 88.90 | 83.70 | 111.12 | 18.83 | 81.17 |
| T-LY22CD | 162.51 | 184.26 | 128.73 | 120.65 | 91.08 | 85.47 | 98.34 | 110.29 | 18.87 | 81.13 |
| T-LY22CD-3 | 164.09 | 222.89 | 207.28 | 156.43 | 120.12 | 92.06 | 100.26 | 105.99 | 17.96 | 82.04 |
| T-LY22CDC | 163.93 | 196.96 | 126.39 | 123.55 | 111.60 | 95.83 | 114.50 | 127.51 | 21.6-1 | 78.36 |

Results: On Day 25, the average tumor volume of the vehicle group (PBS) reached 588 $mm^3$, and the average tumor volume of each treated group was less than 250 $mm^3$. In addition, the relative tumor inhibition rates of T-LY22 and T-LY22BM1 were similar to that of T-BY1 control group. The relative tumor inhibition rates of T-LY22BM2 and T-LY22BM3 were higher than that of T-BY1 control group. The relative tumor inhibition rates of T-LY22C, T-LY22CD, T-LY22CD-3, and T-LY22CDC were the highest. During the treatment period, no significant body weight loss or notable drug toxicity was observed in any group.

Conclusion: The compound of the present invention has good tumor inhibition activity.

Test Example 5: Efficacy Experiment of Antibody-Drug Conjugates in the Human Gastric Cancer NCI-N87 Xenograft Model

The human gastric cancer NCI-N87 cell line (ATCC, CTL-5822) was cultured in RPMI-1640 medium (Gibco, catalog number: 22400-089, containing 10% FBS (Excell Bio, catalog number: FCS500)) in a 37°C incubator with 5% $CO_2$. Cells were digested with 0.25% trypsin-EDTA, washed with PBS twice, counted by a cell counter, and diluted with PBS to a cell concentration of $1x10^8$ cells/mL before ten consecutive generations of cells were cultured.

CB17 SCID mice (female, 4-6 weeks of age, Beijing Vital River Laboratory Animal Technology Co., Ltd.) were used. CB17 SCID mice were acclimated to the laboratory environment for 2-5 days, NCI-N87 cells were inoculated subcutaneously into the right flank of each mouse at a dose of $5x10^6$ cells/mouse in a volume of 0.1 mL (containing 50% Matrigel). When the tumor grew to approximately 100 $mm^3$, drug administration was performed according to the scheme shown in Table 8, and DAR values were all adjusted to 8.

Table 8

| No. | Group | Number of animals | Dosage | Route of administration and administration period |
|---|---|---|---|---|
| 1 | PBS | 6 | - | i.v. Once |
| 2 | T-BY1 | 6 | 1 mg/kg | i.v. Once |
| 3 | T-LY22 | 6 | 1 mg/kg | i.v. Once |
| 4 | T-LY22BM1 | 6 | 1 mg/kg | i.v. Once |
| 5 | T-LY22BM2 | 6 | 1 mg/kg | i.v. Once |
| 6 | T-LY22BM3 | 6 | 1 mg/kg | i.v. Once |
| 7 | T-LY22C | 6 | 1 mg/kg | i.v. Once |
| 8 | T-LY22CD | 6 | 1 mg/kg | i.v. Once |

(continued)

| No. | Group | Number of animals | Dosage | Route of administration and administration period |
|---|---|---|---|---|
| 9 | T-LY22CD-3 | 6 | 1 mg/kg | i.v. Once |
| 10 | T-LY22CDC | 6 | 1 mg/kg | i.v. Once |

Tumor volumes and body weight were measured twice a week, and the tumor proliferation rate and relative tumor inhibition rate were calculated. The calculation formulas are as follows:

$$\text{Relative tumor proliferation rate T/C(\%)} = T_{RTV} / C_{RTV} \times 100\%$$

($T_{RTV}$: average RTV of the treatment group; $C_{RTV}$: average RTV of the control group; RTV = Vt/$V_O$, where $V_O$ is the tumor volume of the animal at the time of grouping, and Vt is the tumor volume of the animal after treatment).

$$\text{Relative tumor inhibition rate TGI\%} = (1\text{-T/C}) \times 100\%$$

(T and C are relative tumor volumes (RTV) of the treatment and control groups at a specific time point, respectively);

The formula for calculating the tumor volume (V): $V = 1/2 \times L_L \times L_S^2$

where $L_L$ and $L_S$ represent the long and short diameters of the tumor, respectively. At the end of the experiment, the mice were sacrificed and the tumors were cut off and weighed.

The experimental results are shown in Table 9 and FIG. 6A and FIG. 6B.

Table 9: Tumor Volume Data of NCI-N87 Gastric Cancer Mouse Model

| Samples | Tumor volume ($mm^2$) | | | | | | | | TC(%) | TGI(%) |
|---|---|---|---|---|---|---|---|---|---|---|
| | 0 days after dosing | 7 days after dosing | 10 days after dosing | 13 days after dosing | 16 days after dosing | 19 days after dosing | 24 days after dosing | 28 days after dosing | 28 days after dosing | 28 days after dosing |
| PBS | 98.78 | 221.61 | 304.07 | 360.23 | 415.38 | 465.79 | 564.23 | 804.54 | | |
| T-BY1 | 98.08 | 147.97 | 187.85 | 220.18 | 224.36 | 230.25 | 199.35 | 210.98 | 26.41 | 73.59 |
| T-LY22 | 98.13 | 94.76 | 79.17 | 55.16 | 53.06 | 48.56 | 30.70 | 32.70 | 4.09 | 95.91 |
| T-LY22BM1 | 98.20 | 78.56 | 43.11 | 34.07 | 39.27 | 42.24 | 29.22 | 33.23 | 4.15 | 95.85 |
| T-LY22BM2 | 97.56 | 73.27 | 79.91 | 64.36 | 40.52 | 47.25 | 26.34 | 27.97 | 3.52 | 9648 |
| T-LY22BM3 | 97.11 | 77.91 | 49.72 | 41.44 | 35.82 | 10.42 | 21.30 | 20.81 | 2.63 | 97.37 |
| T-LY22C | 97.76 | 53.06 | 37.42 | 36.79 | 39.60 | 34.71 | 10.49 | 13.92 | 1.75 | 98.25 |
| T-LY22CD | 97.16 | 58.37 | 35.03 | 30.53 | 22.61 | 25.49 | 3.49 | 8.13 | 1.03 | 98.97 |
| T-LY22CD-3 | 96.90 | 57.67 | 31.65 | 31.66 | 27.54 | 21.40 | 5.97 | 7.71 | 0.98 | 99.02 |
| T-LY22CDC | 96.93 | 50.47 | 39.54 | 35.47 | 25.42 | 17.38 | 7.70 | 9.81 | 1.24 | 98.76 |

Results: On Day 28, the average tumor volume of the vehicle group (PBS) reached 804 $mm^3$, and the average tumor volume of each treated group was less than 210 $mm^3$. In addition, the relative tumor inhibition rates of T-LY22, T-LYBM1, T-LYBM2, T-LYBM3, T-LY22C, T-LY22CD, T-LY22CD-3, and T-LY22CDC were better than that of T-BY1 control group. The tumor inhibition rates of T-LYBM2 and T-LYBM3 were slightly better than those of T-LY22 and T-LYBM1 under the same cleavable amino acid sequence, but slightly worse than T-LY22C. With the same toxin, the tumor inhibition rates of T-LY22CD and T-LY22CD-3, which contained more stable linkers, were better than T-LY22C. In the case of increased linker hydrophilicity alone, the tumor inhibition rate of LY-T-LY22CDC was not better than T-LY22CD and T-LY22CD-3. During the administration period, each group of animals showed no significant weight loss and drug toxicity.

Conclusion: ADCs containing toxins with better lipid solubility and more stable linkers have better tumor inhibition activity.

Test Example 6: Bystander Killing Test

Test Method:

NCI-N87 cells (Shanghai Cell Bank of Chinese Academy of Sciences, SCSP-534) were cultured in RPMI1640 medium (Gibco, 22400-089) and 10% fetal bovine serum (BOVOGEN). MDA-MB-468-luc cells (Shanghai Cell Bank of Chinese Academy of Sciences, TCHul36) with stably transfected firefly luciferase gene (self-constructed) were cultured in DMEM medium (Gibco, 11995-065) and 10% fetal bovine serum (BOVOGEN). The cells were digested with trypsin, and three black transparent-bottom cell culture plates (Corning, 3063) were used: plate 1 was added with 1000 N87 cells/well, plate 2 was added with 1000 468-luc cells/well, plate 3 was added with 2000 N87 and 1000 468-luc mixed cells/well. and the cells were incubated overnight at 37°C in the incubator with 5% $CO_2$. ADC samples were diluted with medium to final concentrations of 10 nM and 1 nM, 100 μL of ADC samples was added to each well, blank media were set as a control group, and the cells were further cultured for 144 hours. 100 μL of CELL Counting-Lite 2.0 Test Reagent (Vazyme, DD1101-03) was added to each well in plate 1 and was kept in the dark for 5 minutes for coloration. 100 μL of One-LumiTMII Test Reagent (Beyotime, RG056M) was added to each well in plate 2 and plate 3 and was kept in the dark for 10 minutes for coloration. The fluorescence value was measured by a multifunctional microplate reader (Tecan Spark) with a reading time of 200 ms. Bystander killing activity was calculated using the formula: (Control group reading - ADC group reading) / (Control group reading) × 100%.

Test Results:

The results of the bystander killing tests are shown in FIG. 3A, FIG. 3B, and FIG. 3C. Both T-BY1 and T-LY22C at concentrations of 1 nM and 10 nM effectively killed N87 cells with high expression of HER2, but basically could not kill 468-luc cells with low expression of HER2. When N87 and 468-luc cells were co-cultured, both T-BY1 and T-LY22C could effectively kill 468-luc cells. At concentrations of 1nM and 10nM, T-BY1 killed 468-luc cells at proportions of 13.9% and 34.0%, respectively, while T-LY22C killed 468-luc cells at proportions of 39.3% and 48.5%. These results indicate that the bystander killing activity of T-LY22C was significantly higher than that of T-BY1.

Conclusion: The compound of the present invention has good bystander killing effects.

Test Example 7: Acute Toxicity Study in Rats

1. Test method: SD rats were purchased from Beijing Vital River Laboratory Animal Technology Co., Ltd. and kept in SPF animal facility. Rats weighing 210g-250g were randomly divided into 3 groups, with 5 rats in each group, and the rats were administered with ADCs or vehicle control at a dose of 200 mg/kg via tail vein injection, with the injection day considered as day 1. Rats' body weight and food intake were measured daily, and their activity, fur condition, and any signs of diarrhea or soft stools were observed. Blood samples were collected on days 5 and 21 post-administration to test blood biochemistry and hematology parameters. The study ended on day 22 post-administration, and the rats were euthanized. Major organs (heart, liver, spleen, lungs, kidneys, etc.) were dissected and observed for gross pathological changes.

2. Test Results

First Acute Toxicity Study in Rats

From day 1 to day 10 post-administration, the body weight of rats in the LY-22CD group was similar to that of the vehicle control, while the body weight of rats in the T-BY1 group was significantly lower than that of the vehicle control, as shown in FIG. 4.

Second Acute Toxicity Study in Rats

From day 1 to day 10 post-administration, the body weight of rats in the LY-22CD and LY-22CD-3 groups was similar to that of the vehicle control, while the body weight of rats in the T-BY1, T-LY22, T-LY22C, and T-LY22BM3 groups was significantly lower than that of the vehicle control. On day 5 post-administration, three rats in the T-LY22 group and one rat in the T-LY22BM3 group died. No deaths were observed in the other groups, but the body weight of rats in the LY22C group was approximately 5% lower than that of the vehicle control. On day 7 post-administration, the remaining rats in the T-LY22 group had a body weight reduction of about 18%, and those in the T-LY22BM3 group had a reduction of about 9%. The body weight of the LY22C group had mostly recovered to the level of the vehicle control. From day 2 to day 21 post-administration, the body weight growth of rats in the T-LY22CD and T-LY22CD-3 groups was similar to that of the vehicle

control, as shown in FIG. 5.

Conclusion: With the same linker, the order of toxin tolerance is T-LY22C > T-LY22BM3 > T-LY22. With different linkers but the same toxin, the order of tolerance is T-LY22CD ~ T-LY22CD-3 > T-LY22C. This indicates that both the toxin structure and linker significantly impact the overall ADC tolerance, and ADC T-LY22CD and T-LY22CD-3 have a larger therapeutic window compared to T-BY1.

Test Example 8: Property Study of Small Molecule LogD

1. Preparation of buffer solution:

(1) Preparation of 100 mM phosphate buffer (PB) solution at pH 2.5 and pH 7.4 respectively:

a. Preparation of a solution of 100 mM $H_3PO_4$ in water: 14.7 M $H_3PO_4$ was diluted with water to a concentration of 100 mM $H_3PO_4$, and the pH was measured to be approximately 1.8.

b. Preparation of 100 mM $NaH_2PO_4$: 5.999g of $NaH_2PO_4$ was dissolved in 500 mL of water and the pH was measured to be approximately 4.5.

c. Preparation of 100 mM $Na_2HPO_4$: 7.098g of $Na_2HPO_4$ was dissolved in water to a volume of 500 mL and the pH was measured to be approximately 9.4.

d. Preparation of 100 mM PB (pH 2.5): 15 mL of 100 mM $H_3PO_4$ was added to a 50 mL tube, and then pH was adjusted to 2.5 $\pm$ 0.05 with 100 mM $NaH_2PO_4$.

d. Preparation of 100 mM PB (pH 7.4): 15 mL of 100 mM $Na_2HPO_4$ was added to a 50 mL tube, and then pH was adjusted to 7.4 $\pm$ 0.05 with 100 mM $NaH_2PO_4$.

(2) Saturation of PBs (pH 2.5 and pH 7.4) with 1-octanol: 10 mL of 1-octanol was added to 100 mL of 100 mM PBs (pH 2.5 and pH 7.4), and the mixture was shaken vigorously, and then allowed to stand overnight at room temperature for later use.

(3) Saturation of 1-octanol with PBs (pH 2.5 and pH 7.4): 10 mL of 100 mM PBs (pH 2.5 and pH 7.4) was added to 100 mL of 1-octanol, and the mixture was shaken vigorously, and then allowed to stand overnight at room temperature for later use.

2. Operation Procedure

2.1) 2μL of 10 mM DMSO stock solution of the test compound were dispensed into test tubes, in duplicate.

2.2) 149μL of PB saturated with 1-octanol (pH 2.5 and 7.4) were added to the tubes.

2.3) 149μL of PB (pH 2.5 and 7.4) saturated with 1-octanol were added to the corresponding tubes.

2.4) Each tube was vigorously mixed for 2 minutes, then shaken at 800rpm for 1 hour at room temperature.

2.5) The tubes were centrifuged at 4000rpm for 5 minutes at room temperature.

2.6) Appropriate volumes of the buffer phase and 1-octanol phase samples were aliquoted.

2.7) These samples were diluted and analyzed using LC-MS/MS without running a calibration curve.

2.8) Data analysis: The log D value of each compound was calculated using the following formula:

$$Log\ D_{octanol/buffer} = \log 10\left(\frac{Mean\ Octanol\ Layer\ Peak\ Area\ Ratio\ *\ Octanol\ Layer\ Dilution\ Factor}{Mean\ Buffer\ Layer\ Peak\ Area\ Ratio\ *\ Buffer\ Layer\ Dilution\ Factor}\right)$$

3.

3. LC-MS Analysis Conditions

| Names of analytes | Column model | Mobile phase A | Mobile phase B |
|---|---|---|---|
| Chlorpromazine hydrochloride | ACQUITY UPLC BEH C18 1.7 µm 2.1 × 50 mm Column, Part No. 186002350 | A: Solution of 0.1% formic acid in water | B: Solution of 0.1% formic acid in acetonitrile |
| Nadolol | ACQUITY UPLC BEH C18 1.7 µm 2.1 × 50 mm Column, Part No. 186002350 | A: Solution of 0.1% formic acid in water | B: Solution of 0.1% formic acid in acetonitrile |
| Propranolol hydrochloride | ACQUITY UPLC BEH C18 1.7 µm 2.1 × 50 mm Column, Part No. 186002350 | A: Solution of 0.1% formic acid in water | B: Solution of 0.1% formic acid in acetonitrile |
| PY-4Car 2 | ACQUITY UPLC Protein BEH C4 300 µm 2.1 × 50 mm Part No. 186004495 | A: Solution of 2% formic acid in water | B: Solution of 2% formic acid in acetonitrile |
| PY-4Car | ACQUITY UPLC Protein BEH C4 300 µm 2.1 × 50 mm Part No. 186004495 | A: Solution of 2% formic acid in water | B: Solution of 2% formic acid in acetonitrile |
| PY-X (DXD) | ACQUITY UPLC Protein BEH C4 300 µm 2.1 × 50 mm Part No. 186004495 | A: Solution of 2% formic acid in water | B: Solution of 2% formic acid in acetonitrile |
| PY-4Car 3C (pH 7.4) | ACQUITY UPLC Protein BEH C4 300 µm 2.1 × 50 mm Part No. 186004495 | A: Solution of 2% formic acid in water | B: Solution of 2% formic acid in acetonitrile |
| PY-4Car 3C | ACQUITY UPLC Protein BEH C4 300 µm | A: Solution of | B: Solution of |
| (pH 2.5) | 2.1 × 50 mm Part No. 186004495 | 2% formic acid in water | 2% formic acid in acetonitrile |

4. Analysis Results:

| Names of compounds | Batch No. | Log D2.5 (Oct/buff) Result | Log D7.4 (Oct/buff) Result |
|---|---|---|---|
| PY-4Car | E0006-1145-P1 | 2.10 | 2.09 |
| PY-4Car3C | E0600-039-P1 | 2.20 | 2.29 |
| PY-4Car2 | E0010-367-P1 | 2.44 | 2.43 |
| PY-X (DXD) | E0006-794-1 | 1.68 | 1.67 |

Conclusion of the test: PY-4Car2 demonstrated better solubility in n-octanol at different pH values compared to the corresponding PY-4Car and PY-4Car3C, as well as the control DXD. This indicates that PY-4Car2 has better lipid solubility, predicting better bystander effects *in vivo,* which is consistent with the *in vivo* efficacy results.

## Claims

**1.** A compound represented by general formula (A) or a pharmaceutically acceptable salt thereof,

$$L\text{-}L_2\text{-}L_1\text{-}Dr \qquad (A)$$

wherein:

Dr is selected from the following structures:

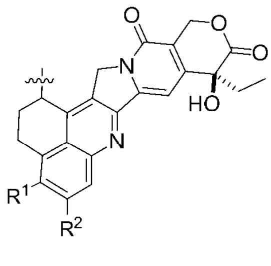 , 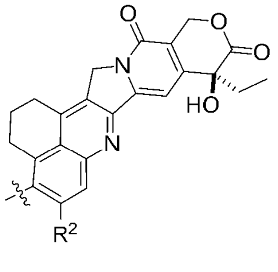 , 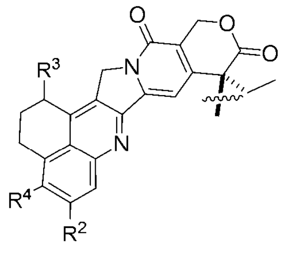 ,

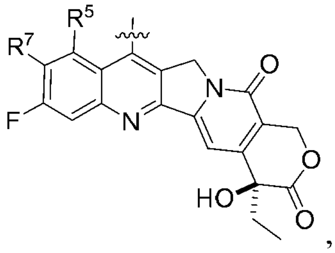 , and 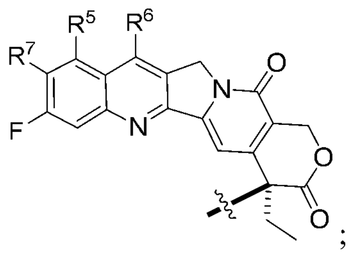 ;

$R^1$ is selected from hydrogen, halogen, hydroxyl, amino, cyano, alkyl, alkoxy, alkenyl, alkynyl, cycloalkyl, $-NR^dR^e$, $-(CH_2)_m-OH$, $-(CH_2)_m-NR^dR^e$, $-OC(=O)NR^f-(CH_2)_m-OH$, $-OC(=O)NR^f-(CH_2)_m-NR^dR^e$, $-(CH_2)_m-C(=O)OH$, $-(CH_2)_m-C(=O)-NR^dR^e$, $-(CH_2)_m-C(=O)NR^f-(CH_2)_n-OH$, $-(CH_2)_m-C(=O)NR^f-(CH_2)_n-NR^dR^e$, $-NR^f-(CH_2)_m-OH$, $-NR^f-(CH_2)_m-NR^dR^e$, $-O-(CH_2)_m-OH$, $-O-(CH_2)_m-NR^dR^e$, $-NR^fC(=O)O-(CH_2)_m-OH$, $-NR^bC(=O)O-(CH_2)_m-NR^dR^e$, $-(CH_2)_m-NR^fC(=O)O-(CH_2)_n-OH-$, $-(CH_2)_m-NR^fC(=O)O-(CH_2)_n-NR^dR^e$, $-(CH_2)_m-OC(=O)NR^f-(CH_2)_n-NR^dR^e$, $-(CH_2)_m-OC(=O)NR^f-(CH_2)_n-OH$, $-(CH_2)_m-NR^fC(=O)-(CH_2)_n-NR^dR^e$, $-(CH_2)_m-NR^fC(=O)-(CH_2)_n-OH$, and $-(CH_2)_m-NR^fC(=O)-G-(CH_2)_n-OH$, wherein the alkyl, alkoxy, alkenyl, alkynyl, and cycloalkyl are optionally further substituted by one or more groups selected from halogen, deuterium, amino, alkyl, and hydroxyl; the $-(CH_2)_m-$ is optionally substituted by one or more deuterium or halogen groups; and G is selected from cycloalkylene, heterocyclylene, heteroarylene, and arylene;

$R^2$ is selected from hydrogen, halogen, hydroxyl, amino, cyano, alkyl, alkoxy, alkenyl, and alkynyl, wherein the alkyl, alkoxy, alkenyl, and alkynyl are optionally further substituted by one or more groups selected from halogen;

$R^3$ is selected from hydrogen, halogen, hydroxyl, amino, cyano, alkyl, alkoxy, alkenyl, alkynyl, $CH_2=$, $-NR^dR^e$, $-(CH_2)_m-OH$, $-(CH_2)_m-NR^dR^e$, $-OC(=O)NR^f-(CH_2)_m-OH$, $-OC(=O)NR^f-(CH_2)_m-NR^dR^e$, $-(CH_2)_m-C(=O)OH$, $-(CH_2)_m-C(=O)-NR^dR^e$, $-(CH_2)_m-C(=O)NR^f-(CH_2)_n-OH$, $-(CH_2)_m-C(=O)NR^f-(CH_2)_n-NR^dR^e$, $-NR^f-(CH_2)_m-OH$, $-NR^f-(CH_2)_m-NR^dR^e$, $-NR^f-C(=O)R^d$, $-NR^fC(=O)-(CH_2)_m-R^d$, $-NR^fC(=O)O-(CH_2)_m-R^d$, $-O-(CH_2)_m-OH$, $-O-(CH_2)_m-NR^dR^e$, $-NR^fC(=O)O-(CH_2)_m-OH$, $-NR^fC(=O)NR^d-(CH_2)_m-OH$, $-NR^fC(=O)NR^d-(CH_2)_m-O-(CH_2)_n-OH$, $-NR^fC(=O)O-(CH_2)_m-O-(CH_2)_n-OH$, $-NR^fC(=O)NR^d-(CH_2)_m-O-(CH_2)_n-NR^dR^e$, $-NR^fC(=O)O-(CH_2)_m-O-(CH_2)_n-NR^dR^e$, $-NR^fC(=O)NR^d-(CH_2)_m-NR^dR^e$, $-NR^fC(=O)O-(CH_2)_m-NR^dR^e$, $-(CH_2)_m-NR^fC(=O)O-(CH_2)_n-OH$, $-(CH_2)_m-NR^fC(=O)O-(CH_2)_n-NR^dR^e$, $-(CH_2)_m-OC(=O)NR^f-(CH_2)_n-NR^dR^e$, $-(CH_2)_m-OC(=O)NR^f-(CH_2)_n-OH$, $-(CH_2)_m-NR^fC(=O)-(CH_2)_n-NR^dR^e$, and $-(CH_2)_m-NR^fC(=O)-(CH_2)_n-OH$, wherein the alkyl, alkoxy, alkenyl, and alkynyl are optionally further substituted by one or more groups selected from halogen, deuterium, amino, alkyl, and hydroxyl;

$R^4$ is selected from hydrogen, halogen, hydroxyl, carboxyl, amino, cyano, alkyl, alkoxy, alkenyl, alkynyl, cycloalkyl, $-NR^dR^e$, $-(CH_2)_m-OH$, $-(CH_2)_m-NR^dR^e$, $-OC(=O)NR^f-(CH_2)_m-OH$, $-OC(=O)NR^f-(CH_2)_m-NR^dR^e$, $-(CH_2)_m-C(=O)OH$, $-(CH_2)_m-C(=O)-NR^dR^e$, $-(CH_2)_m-C(=O)NR^f-(CH_2)_n-OH$, $-(CH_2)_m-C(=O)NR^f-(CH_2)_n-NR^dR^e$, $-NR^f-(CH_2)_m-OH$, $-NR^f-(CH_2)_m-NR^dR^e$, $-O-(CH_2)_m-OH$, $-O-(CH_2)_m-NR^dR^e$, $-NR^fC(=O)O-(CH_2)_m-OH$, $-NR^bC(=O)O-(CH_2)_m-NR^dR^e$, $-(CH_2)_m-NR^fC(=O)O-(CH_2)_n-OH-$, $-(CH_2)_m-NR^fC(=O)O-(CH_2)_n-NR^dR^e$, $-(CH_2)_m-OC(=O)NR^f-(CH_2)_n-NR^dR^e$, $-(CH_2)_m-OC(=O)NR^f-(CH_2)_n-OH$, $-(CH_2)_m-NR^fC(=O)-(CH_2)_n-NR^dR^e$, $-(CH_2)_m-NR^fC(=O)-(CH_2)_n-OH$, and $-(CH_2)_m-NR^fC(=O)-G-(CH_2)_n-OH$, wherein the alkyl, alkoxy, alkenyl, alkynyl, and cycloalkyl are optionally further substituted by one or more groups selected from halogen, deuterium, amino, alkyl, and hydroxyl; the $-(CH_2)_m-$ is optionally substituted by one or more deuterium or halogen groups; and G is selected from cycloalkylene, heterocyclylene, heteroarylene, and arylene;

$R^5$ is selected from hydrogen, halogen, hydroxyl, amino, cyano, alkyl, alkoxy, alkenyl, and alkynyl, wherein the alkyl, alkoxy, alkenyl, and alkynyl are optionally further substituted by one or more groups selected from halogen;

$R^6$ is selected from hydrogen, halogen, hydroxyl, amino, cyano, alkyl, alkoxy, alkenyl, and alkynyl, wherein the alkyl, alkoxy, alkenyl, and alkynyl are optionally further substituted by one or more groups selected from halogen;

$R^7$ is selected from hydrogen, halogen, hydroxyl, amino, cyano, alkyl, alkoxy, alkenyl, and alkynyl, wherein the alkyl, alkoxy, alkenyl, and alkynyl are optionally further substituted by one or more groups selected from halogen;

$R^d$ and $R^e$ are each independently selected from hydrogen and alkyl;

$R^f$ is selected from hydrogen, alkyl, $-C(O)R^c$, $-S(O)R^c$, and $-S(O)_2R^c$, wherein the alkyl is optionally further substituted by cycloalkyl; and $R^c$ is selected from hydrogen, hydroxyl, and alkyl;

$L_1$ is selected from a bond, $-(CH_2)_m-*$, -O-*, $-NR^a-*$, $-(CH_2)_m-O-*$, $-(CH_2)_m-NR^a-*$, $-OC(=O)NR^b-(CH_2)_m-O-*$, $-OC(=O)NR^b-(CH_2)_m-NR^a-*$, $-(CH_2)_m-C(=O)O-*$, $-(CH_2)_m-C(=O)NR^a-*$, $-(CH_2)_m-C(=O)NR^b-(CH_2)_n-O-*$, $-(CH_2)_m-C(=O)NR^b-(CH_2)_n-NR^a-*$, $-NR^b-(CH_2)_m-O-*$, $-NR^b-(CH_2)_m-NR^a-*$, $-O-(CH_2)_m-O-*$, $-O-(CH_2)_m-NR^a-*$, $-NR^bC(=O)O-(CH_2)_m-O-*$, $-NR^bC(=O)O-(CH_2)_m-NR^a-*$, - $(CH_2)_m-NR^bC(=O)O-(CH_2)_n-O-*$, $-(CH_2)_m-NR^bC(=O)O-(CH_2)_n-NR^a-*$, $-(CH_2)_m-OC(=O)NR^b-(CH_2)_n-NR^a-*$, $-(CH_2)_m-OC(=O)NR^b-(CH_2)_n-O-*$, $-(CH_2)_m-NR^bC(=O)-(CH_2)_n-NR^a-*$, and $-(CH_2)_m-NR^bC(=O)-(CH_2)_n-O-*$, wherein * is a connection site with $L_2$;

$R^a$ and $R^b$ are each independently selected from hydrogen, alkyl, $-C(O)R^c$, $-S(O)R^c$, and $-S(O)_2R^c$, wherein the alkyl is optionally further substituted by cycloalkyl; and $R^c$ is selected from hydrogen, hydroxyl, and alkyl;

$L_2$ is selected from a bond,

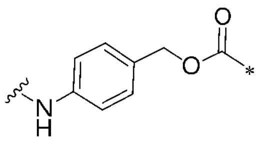 , 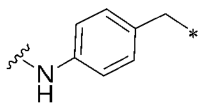 , 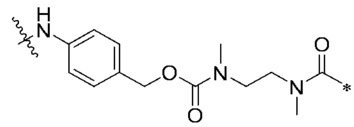 ,

and

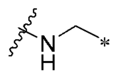 ,

wherein * is a connection site with $L_1$;

L is

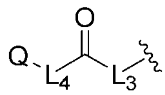 or 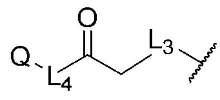 ;

$L_3$ is an amino acid residue formed by two or more amino acids, $L_3$ optionally comprises one or more of the following structures, and $L_6$ is selected from one or more of the following structures:

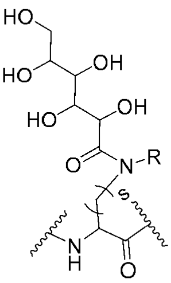 , 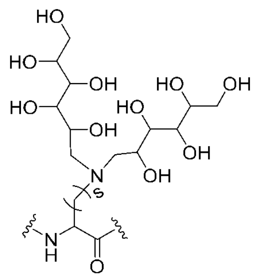 , 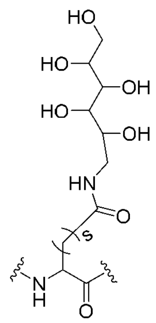 , 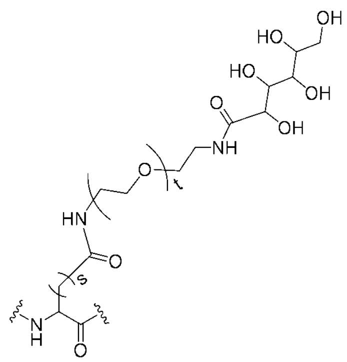 ,

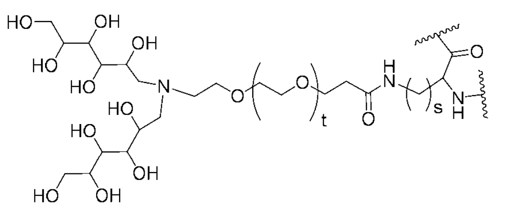 , 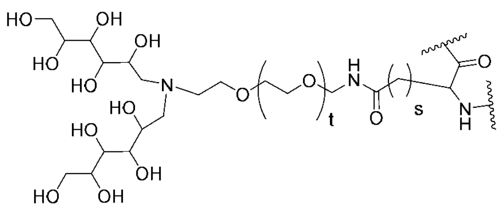 ,

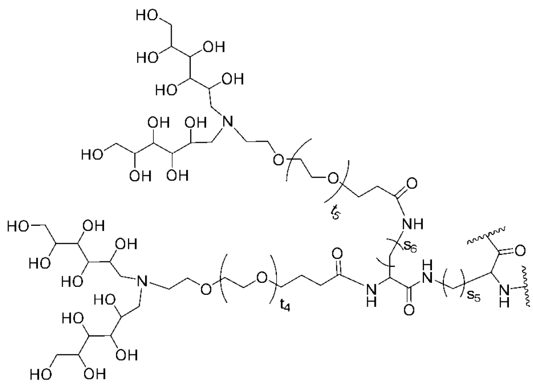, 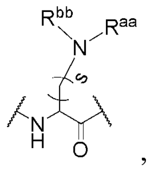, or 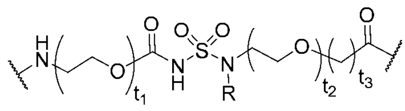,

wherein R, $R^{aa}$, and $R^{bb}$ are each independently selected from hydrogen and alkyl;

$L_4$ is

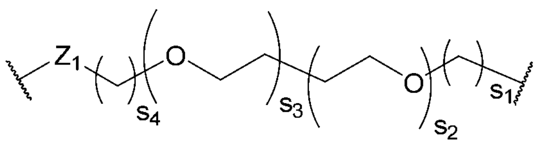;

$Z_1$ is selected from a bond, $-(CH_2)_p-$, $-(C_2H_4O)_q-$, $-(CH_2)_p-C(O)NH-$, $-(CH_2)_p-O-(CH_2)_p-C(O)NH-$, $-(CH_2)_p-C(O)-L_6-NH-$, $-(CH_2)_p-O-(CH_2)_p-C(O)-L_6-NH-$, -C(O)NH-, -C(O)O-, -C(O)-, -OC(O)-, -NH-, -O-, and -OC(O)NH-;

m is an integer from 1 to 6;

n is an integer from 1 to 6;

s is an integer from 1 to 6;

t is an integer from 0 to 10;

$s_1$, $s_2$, $s_3$, and $s_4$ are each independently an integer from 0 to 10;

$s_5$ and $s_6$ are each independently an integer from 1 to 6;

$t_1$ is an integer from 1 to 6;

$t_2$ is an integer from 0 to 6;

$t_3$ is an integer from 1 to 6;

$t_4$ is an integer from 0 to 10;

$t_5$ is an integer from 0 to 10;

p is an integer from 1 to 10;

q is an integer from 1 to 10; and

Q is a linker unit.

**2.** The compound represented by the general formula (A) or the pharmaceutically acceptable salt thereof according to claim 1, wherein

Dr is selected from the following structures:

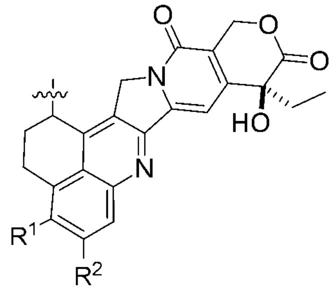;

$L_1$ is selected from -O-*, $-NR^a-*$, $-(CH_2)_m-O-*$, $-(CH_2)_m-NR^a-*$, $-OC(=O)NR^b-(CH_2)_m-O-*$, $-OC(=O)NR^b-(CH_2)_m-NR^a-*$, $-(CH_2)_m-C(=O)O-*$, $-(CH_2)_m-C(=O)NR^a-*$, $-NR^b-(CH_2)_m-O-*$, $-NR^b-(CH_2)_m-NR^a-*$,

$-O-(CH_2)_m-O-*$, $-O-(CH_2)_m-NR^a-*$, $-NR^bC(=O)O-(CH_2)_m-O-*$, $-NR^bC(=O)O-(CH_2)_m-NR^a-*$, $-(CH_2)_m-NR^bC(=O)-(CH_2)_n-NR^a-*$, and $-(CH_2)_m-NR^bC(=O)-(CH_2)_n-O-*$, wherein * is a connection site with $L_2$;
$R^a$ is selected from hydrogen and $C_1$-$C_6$ alkyl;
$R^b$ is selected from hydrogen, alkyl, $-C(O)R^c$, $-S(O)R^c$, and $-S(O)_2R^c$, wherein the alkyl is optionally further substituted by cycloalkyl; and $R^c$ is selected from hydrogen, hydroxyl, and $C_1$-$C_6$ alkyl;
m is an integer from 1 to 6; preferably, an integer from 1 to 4; more preferably, an integer from 1 to 2;
n is an integer from 1 to 6; preferably, an integer from 1 to 4; more preferably, an integer from 1 to 2;
$R^1$ is selected from hydrogen, halogen, hydroxyl, amino, cyano, $C_1$-$C_6$ alkyl, $C_1$-$C_6$ alkoxy, $C_2$-$C_6$ alkenyl, $C_2$-$C_6$ alkynyl, $C_3$-$C_6$ cycloalkyl, $-NR^dR^e$, $-(CH_2)_m-OH$, $-(CH_2)_m-NR^dR^e$, $-OC(=O)NR^f-(CH_2)_m-OH$, $-OC(=O)NR^f-(CH_2)_m-NR^dR^e$, $-(CH_2)_m-C(=O)OH$, $-(CH_2)_m-C(=O)-NR^dR^e$, $-(CH_2)_m-C(=O)NR^f-(CH_2)_n-OH$, $-(CH_2)_m-C(=O)NR^f-(CH_2)_n-NR^dR^e$, $-NR^f-(CH_2)_m-OH$, $-NR^f(CH_2)_m-NR^dR^e$, $-O-(CH_2)_m-OH$, $-O-(CH_2)_m-NR^dR^e$, $-NR^fC(=O)O-(CH_2)_m-OH$, $-NR^bC(=O)O-(CH_2)_m-NR^dR^e$, $-(CH_2)_m-NR^fC(=O)O-(CH_2)_n-OH-$, $-(CH_2)_m-NR^fC(=O)O-(CH_2)_n-NR^dR^e$, $-(CH_2)_m-OC(=O)NR^f-(CH_2)_n-NR^dR^e$, $-(CH_2)_m-OC(=O)NR^f-(CH_2)_n-OH$, $-(CH_2)_m-NR^fC(=O)-(CH_2)_n-NR^dR^e$, $-(CH_2)_m-NR^fC(=O)-(CH_2)_n-OH$, and $-(CH_2)_m-NR^fC(=O)-G-(CH_2)_n-OH$, wherein the $C_1$-$C_6$ alkyl, $C_1$-$C_6$ alkoxy, $C_2$-$C_6$ alkenyl, $C_2$-$C_6$ alkynyl, and $C_3$-$C_6$ cycloalkyl are optionally further substituted by one or more groups selected from halogen, deuterium, amino, $C_1$-$C_6$ alkyl, and hydroxyl; the $C_1$-$C_6$ alkyl and $C_1$-$C_6$ alkoxy are preferred; the $-(CH_2)_m-$ is optionally substituted by one or more deuterium or halogen groups; and G is selected from cycloalkylene, heterocyclylene, heteroarylene, and arylene;
$R^2$ is selected from hydrogen, halogen, hydroxyl, amino, cyano, $C_1$-$C_6$ alkyl, $C_1$-$C_6$ alkoxy, $C_2$-$C_6$ alkenyl, and $C_2$-$C_6$ alkynyl, wherein the $C_1$-$C_6$ alkyl, $C_1$-$C_6$ alkoxy, $C_2$-$C_6$ alkenyl, and $C_2$-$C_6$ alkynyl are optionally further substituted by one or more groups selected from halogen; wherein halogen is preferred; and
$R^d$, $R^e$, $R^f$, m, and n are defined as those in claim 1.

**3.** The compound represented by the general formula (A) or the pharmaceutically acceptable salt thereof according to claim 1, wherein

Dr is selected from the following structures:

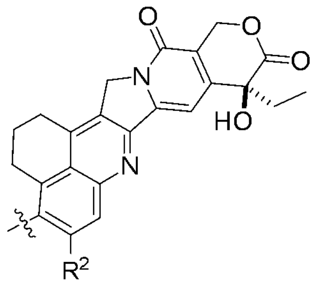

;

$L_1$ is selected from a bond, $-(CH_2)_m-*$, $-O-*$, $-(CH_2)_m-O-*$, $-NR^a-*$, $-(CH_2)_m-NR^a-*$, $-NR^b-(CH_2)_m-O-*$, $-NR^b-(CH_2)_m-NR^a-*$, $-O-(CH_2)_m-O-*$, $-O-(CH_2)_m-NR^a-*$, $-(CH_2)_m-OC(=O)NR^b-(CH_2)_n-NR^a-*$, $-(CH_2)_m-OC(=O)NR^b-(CH_2)_n-O-*$, $-(CH_2)_m-NR^bC(=O)-(CH_2)_n-NR^a-*$, and $-(CH_2)_m-NR^bC(=O)-(CH_2)_n-O-*$, wherein * is a connection site with $L_2$;
$R^a$ is selected from hydrogen and $C_1$-$C_6$ alkyl;
$R^b$ is selected from hydrogen, alkyl, $-C(O)R^c$, $-S(O)R^c$, and $-S(O)_2R^c$, wherein the alkyl is optionally further substituted by cycloalkyl; and $R^c$ is selected from hydrogen, hydroxyl, and $C_1$-$C_6$ alkyl;
m is an integer from 1 to 6; preferably, an integer from 1 to 4;
n is an integer from 1 to 6; preferably, an integer from 1 to 4; and
$R^2$ is selected from hydrogen, halogen, hydroxyl, amino, cyano, $C_1$-$C_6$ alkyl, $C_1$-$C_6$ alkoxy, $C_2$-$C_6$ alkenyl, and $C_2$-$C_6$ alkynyl, wherein the $C_1$-$C_6$ alkyl, $C_1$-$C_6$ alkoxy, $C_2$-$C_6$ alkenyl, and $C_2$-$C_6$ alkynyl are optionally further substituted by one or more groups selected from halogen; wherein halogen is preferred.

**4.** The compound represented by the general formula (A) or the pharmaceutically acceptable salt thereof according to claim 1, wherein

Dr is selected from the following structures:

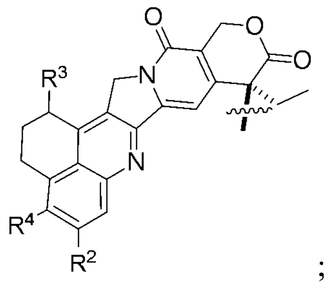

;

$L_1$ is selected from -O-*, wherein * is a connection site with $L_2$;
$R^2$ is selected from hydrogen, halogen, hydroxyl, amino, cyano, $C_1$-$C_6$ alkyl, $C_1$-$C_6$ alkoxy, $C_2$-$C_6$ alkenyl, and $C_2$-$C_6$ alkynyl, wherein the $C_1$-$C_6$ alkyl, $C_1$-$C_6$ alkoxy, $C_2$-$C_6$ alkenyl, and $C_2$-$C_6$ alkynyl are optionally further substituted by one or more groups selected from halogen; wherein halogen is preferred;
$R^3$ is selected from hydrogen, halogen, hydroxyl, amino, cyano, $C_1$-$C_6$ alkyl, $C_1$-$C_6$ alkoxy, $C_2$-$C_6$ alkenyl, and $C_2$-$C_6$ alkynyl, $CH_2$=, $-NR^dR^e$, $-(CH_2)_m-OH$, $-(CH_2)_m-NR^dR^e$, $-OC(=O)NR^f-(CH_2)_m-OH$, $-OC(=O)NR^f-(CH_2)_m-NR^dR^e$, $-(CH_2)_m-C(=O)OH$, $-(CH_2)_m-C(=O)-NR^dR^e$, $-(CH_2)_m-C(=O)NR^f-(CH_2)_n-OH$, $-(CH_2)_m-C(=O)NR^f-(CH_2)_n-NR^dR^e$, $-NR^f-(CH_2)_m-OH$, $-NR^f-(CH_2)_m-NR^dR^e$, $-NR^f-C(=O)R^d$, $-NR^f-C(=O)-(CH_2)_m-R^d$, $-NR^fC(=O)O-(CH_2)_m-R^d$, $-O-(CH_2)_m-OH$, $-O-(CH_2)_m-NR^dR^e$, $-NR^fC(=O)O-(CH_2)_m-OH$, $-NR^fC(=O)NR^d-(CH_2)_m-OH$, $-NR^fC(=O)NR^d-(CH_2)_m-O-(CH_2)_n-OH$, $-NR^fC(=O)O-(CH_2)_m-O-(CH_2)_n-OH$, $-NR^fC(=O)NR^d-(CH_2)_m-O-(CH_2)_n-NR^dR^e$, $-NR^fC(=O)O-(CH_2)_m-O-(CH_2)_n-NR^dR^e$, $-NR^fC(=O)NR^d-(CH_2)_m-NR^dR^e$, $-NR^fC(=O)O-(CH_2)_m-NR^dR^e$, $-(CH_2)_m-NR^fC(=O)O-(CH_2)_n-OH$, $-(CH_2)_m-NR^fC(=O)O-(CH_2)_n-NR^dR^e$, $-(CH_2)_m-OC(=O)NR^f-(CH_2)_n-NR^dR^e$, $-(CH_2)_m-OC(=O)NR^f-(CH_2)_n-OH$, $-(CH_2)_m-NR^fC(=O)-(CH_2)_n-NR^dR^e$, and $-(CH_2)_m-NR^fC(=O)-(CH_2)_n-OH$, wherein the $C_1$-$C_6$ alkyl, $C_1$-$C_6$ alkoxy, $C_2$-$C_6$ alkenyl, and $C_2$-$C_6$ alkynyl are optionally further substituted by one or more groups selected from halogen, deuterium, amino, $C_1$-$C_6$ alkyl, and hydroxyl; and hydrogen or hydroxyl is preferred;
$R^4$ is selected from hydrogen, halogen, hydroxyl, carboxyl, amino, cyano, $C_1$-$C_6$ alkyl, $C_1$-$C_6$ alkoxy, $C_2$-$C_6$ alkenyl, $C_2$-$C_6$ alkynyl, $C_3$-$C_6$ cycloalkyl, $-NR^dR^e$, $-(CH_2)_m-OH$, $-(CH_2)_m-NR^dR^e$, $-OC(=O)NR^f-(CH_2)_m-OH$, $-OC(=O)NR^f-(CH_2)_m-NR^dR^e$, $-(CH_2)_m-C(=O)OH$, $-(CH_2)_m-C(=O)-NR^dR^e$, $-(CH_2)_m-C(=O)NR^f-(CH_2)_n-OH$, $-(CH_2)_m-C(=O)NR^f-(CH_2)_n-NR^dR^e$, $-NR^f-(CH_2)_m-OH$, $-NR^f-(CH_2)_m-NR^dR^e$, $-O-(CH_2)_m-OH$, $-O-(CH_2)_m-NR^dR^e$, $-NR^fC(=O)O-(CH_2)_m-OH$, $-NR^bC(=O)O-(CH_2)_m-NR^dR^e$, $-(CH_2)_m-NR^fC(=O)O-(CH_2)_n-OH-$, $-(CH_2)_m-NR^fC(=O)O-(CH_2)_n-NR^dR^e$, $-(CH_2)_m-OC(=O)NR^f-(CH_2)_n-NR^dR^e$, $-(CH_2)_m-OC(=O)NR^f-(CH_2)_n-OH$, $-(CH_2)_m-NR^fC(=O)-(CH_2)_n-NR^dR^e$, $-(CH_2)_m-NR^fC(=O)-(CH_2)_n-OH$, and $-(CH_2)_m-NR^fC(=O)-G-(CH_2)_n-OH$, wherein the $C_1$-$C_6$ alkyl, $C_1$-$C_6$ alkoxy, $C_2$-$C_6$ alkenyl, $C_2$-$C_6$ alkynyl, and $C_3$-$C_6$ cycloalkyl are optionally further substituted by one or more groups selected from halogen, deuterium, amino, $C_1$-$C_6$ alkyl, and hydroxyl; hydroxyl, $C_1$-$C_6$ alkyl, and $C_1$-$C_6$ alkoxy are preferred; the $-(CH_2)_m-$ is optionally substituted by one or more deuterium or halogen groups; and G is selected from cycloalkylene, heterocyclylene, heteroarylene, and arylene; and
$R^d$, $R^e$, $R^f$, m, and n are defined as those in claim 1.

**5.** The compound represented by the general formula (A) or the pharmaceutically acceptable salt thereof according to claim 1, wherein

Dr is selected from the following structures:

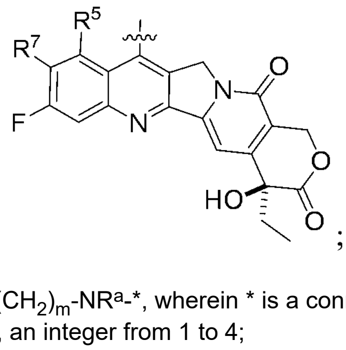

;

$L_1$ is selected from $-(CH_2)_m-O-*$ and $-(CH_2)_m-NR^a-*$, wherein * is a connection site with $L_2$;
m is an integer from 1 to 6; preferably, an integer from 1 to 4;
$R^a$ is selected from hydrogen and $C_1$-$C_6$ alkyl;
$R^5$ is selected from hydrogen, halogen, hydroxyl, amino, cyano, $C_1$-$C_6$ alkyl, $C_1$-$C_6$ alkoxy, $C_2$-$C_6$ alkenyl, and $C_2$-$C_6$ alkynyl, wherein the $C_1$-$C_6$ alkyl, $C_1$-$C_6$ alkoxy, $C_2$-$C_6$ alkenyl, and $C_2$-$C_6$ alkynyl are optionally further

substituted by one or more groups selected from halogen; wherein halogen is preferred; and
$R^7$ is selected from hydrogen, halogen, hydroxyl, amino, cyano, $C_1$-$C_6$ alkyl, $C_1$-$C_6$ alkoxy, $C_2$-$C_6$ alkenyl, and $C_2$-$C_6$ alkynyl, wherein the $C_1$-$C_6$ alkyl, $C_1$-$C_6$ alkoxy, $C_2$-$C_6$ alkenyl, and $C_2$-$C_6$ alkynyl are optionally further substituted by one or more groups selected from halogen; wherein hydroxyl and amino are preferred.

**6.** The compound represented by the general formula (A) or the pharmaceutically acceptable salt thereof according to claim 1, wherein

Dr is selected from the following structures:

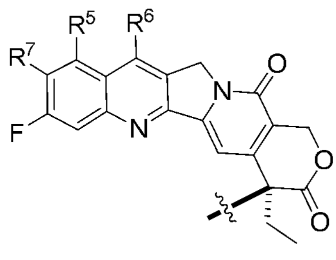
;

$L_1$ is selected from -O-*, wherein * is a connection site with $L_2$;
$R^5$ is selected from hydrogen, halogen, hydroxyl, amino, cyano, $C_1$-$C_6$ alkyl, $C_1$-$C_6$ alkoxy, $C_2$-$C_6$ alkenyl, and $C_2$-$C_6$ alkynyl, wherein the $C_1$-$C_6$ alkyl, $C_1$-$C_6$ alkoxy, $C_2$-$C_6$ alkenyl, and $C_2$-$C_6$ alkynyl are optionally further substituted by one or more groups selected from halogen; wherein halogen is preferred;
$R^6$ is selected from hydrogen, halogen, hydroxyl, amino, cyano, $C_1$-$C_6$ alkyl, $C_1$-$C_6$ alkoxy, $C_2$-$C_6$ alkenyl, and $C_2$-$C_6$ alkynyl, wherein the $C_1$-$C_6$ alkyl, $C_1$-$C_6$ alkoxy, $C_2$-$C_6$ alkenyl, and $C_2$-$C_6$ alkynyl are optionally further substituted by one or more groups selected from halogen; wherein hydroxyl and amino are preferred; and
$R^7$ is selected from hydrogen, halogen, hydroxyl, amino, cyano, $C_1$-$C_6$ alkyl, $C_1$-$C_6$ alkoxy, $C_2$-$C_6$ alkenyl, and $C_2$-$C_6$ alkynyl, wherein the $C_1$-$C_6$ alkyl, $C_1$-$C_6$ alkoxy, $C_2$-$C_6$ alkenyl, and $C_2$-$C_6$ alkynyl are optionally further substituted by one or more groups selected from halogen; wherein hydroxyl and amino are preferred.

**7.** The compound represented by the general formula (A) or the pharmaceutically acceptable salt thereof according to any one of claims 1 to 6, wherein Dr is selected from:

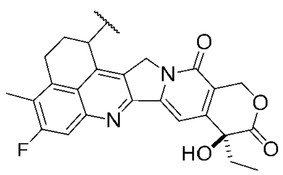
,
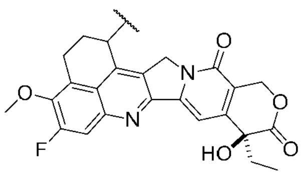
,
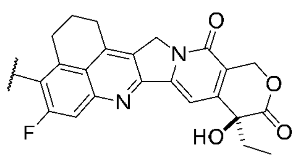
,
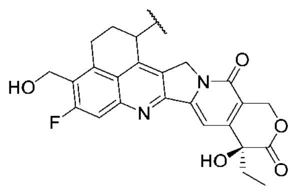
,
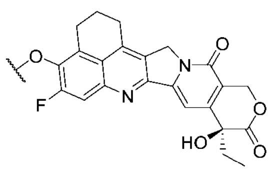
,
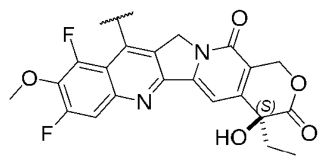
,
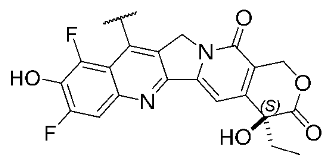
,
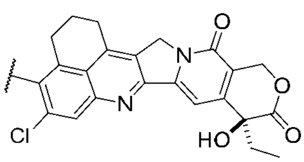
, and
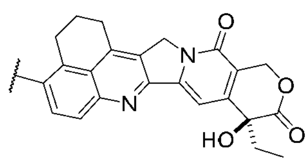
.

**8.** The compound represented by the general formula (A) or the pharmaceutically acceptable salt thereof according to any one of claims 1 to 7, wherein

when $L_1$ is selected from $-NR^a$-*, $-(CH_2)_m-NR^a$-*, $-OC(=O)NR^b-(CH_2)_m-NR^a$-*, $-(CH_2)_m-C(=O)NR^a$-*,

$-(CH_2)_m-C(=O)NR^b-(CH_2)_n-NR^a-*$, $-NR^b-(CH_2)_m-NR^a-*$, $-O-(CH_2)_m-NR^a-*$, $-NR^bC(=O)O-(CH_2)_m-NR^a-*$, $-(CH_2)_m-NR^bC(=O)O-(CH_2)_n-NR^a-*$, $-(CH_2)_m-OC(=O)NR^b-(CH_2)_n-NR^a-*$, and $-(CH_2)_m-NR^bC(=O)-(CH_2)_n-N-R^a-*$, wherein * is a connection site with $L_2$,
$L_2$ is selected from a bond and

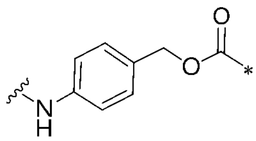,

wherein * is a connection site with $L_1$; and $R^a$, $R^b$, m, and n are defined as those in any one of claims 1 to 7.

**9.** The compound represented by the general formula (A) or the pharmaceutically acceptable salt thereof according to any one of claims 1 to 7, wherein

when $L_1$ is selected from a bond, -O-*, $-(CH_2)_m-O-*$, $-NR^a-*$, $-OC(=O)NR^b-(CH_2)_m-O-*$, $-(CH_2)_m-C(=O)O-*$, $-(CH_2)_m-C(=O)NR^b-(CH_2)_n-O-*$, $-NR^b-(CH_2)_m-O-*$, $-O-(CH_2)_m-O-*$, $-NR^bC(=O)O-(CH_2)_m-O-*$, $-(CH_2)_m-NR^bC(=O)O-(CH_2)_n-O-*$, $-(CH_2)_m-OC(=O)NR^b-(CH_2)_n-O-*$, and $-(CH_2)_m-NR^bC(=O)-(CH_2)_n-O-*$, wherein * is a connection site with $L_2$,
$L_2$ is selected from a bond,

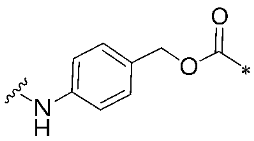, 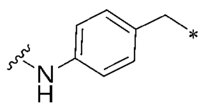, 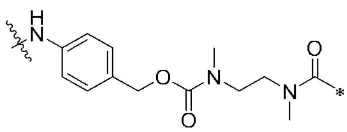,

and

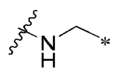,

wherein * is a connection site with $L_1$; and
$R^a$, $R^b$, m, and n are defined as those in any one of claims 1 to 7.

**10.** The compound represented by the general formula (A) or the pharmaceutically acceptable salt thereof according to any one of claims 1 to 9, wherein

$L_3$ is an amino acid residue formed by two or more amino acids selected from phenylalanine, alanine, glycine, valine, leucine, isoleucine, tryptophan, tyrosine, histidine, lysine, citrulline, serine, threonine, cysteine, glutamic acid, glutamine, aspartic acid, asparagine, methionine, and arginine, and $L_3$ optionally comprises one or more of the following structures:

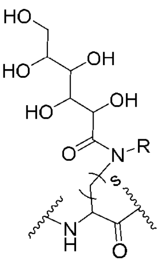, 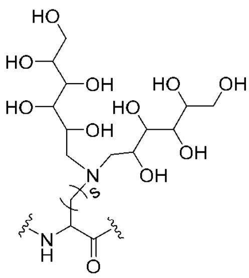, 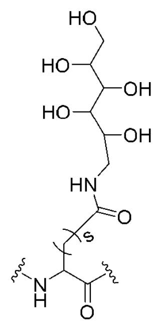, 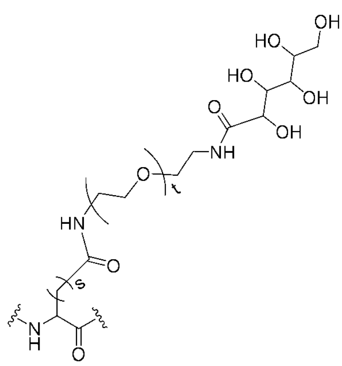,

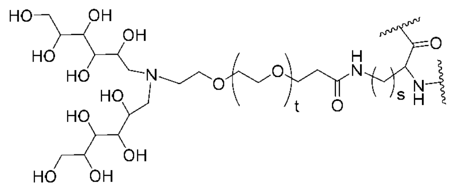, 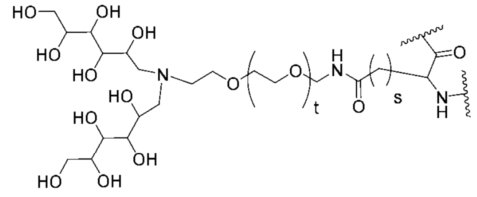,

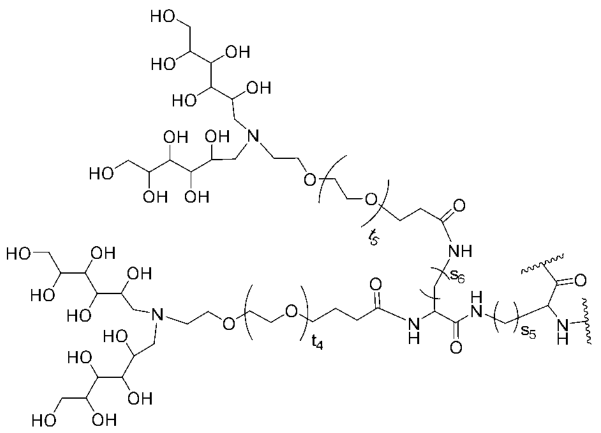, 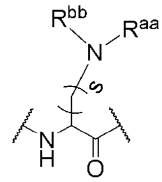,

or

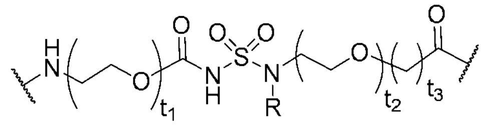;

preferably,

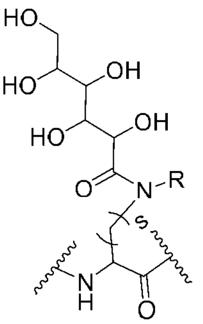, 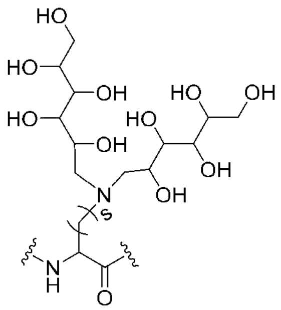, 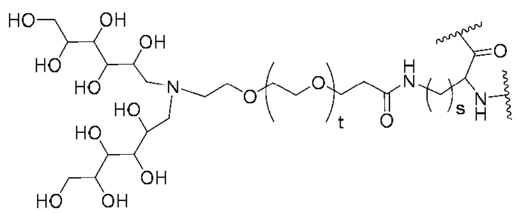,

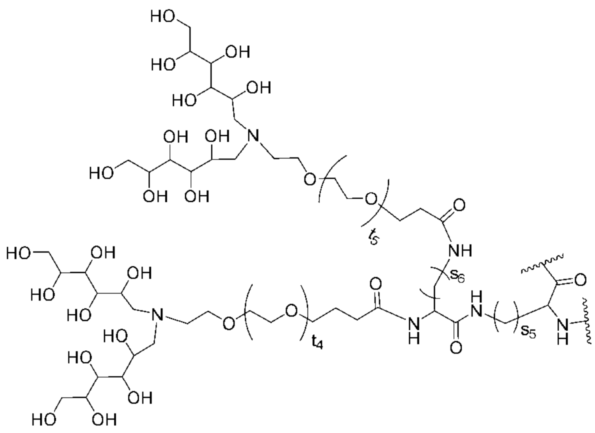

,

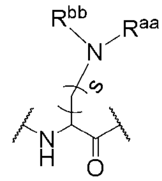

,

or

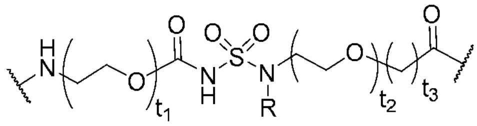

,

wherein R, $R^{aa}$, and $R^{bb}$ are each independently selected from hydrogen and $C_1$-$C_6$ alkyl;
s is an integer from 1 to 6; preferably, an integer from 2 to 6; more preferably, an integer from 2 to 4;
$s_5$ and $s_6$ are each independently an integer from 1 to 6; preferably, an integer from 2 to 6; more preferably, an integer from 2 to 4;
t is an integer from 0 to 10;
$t_1$ is an integer from 1 to 6; preferably, an integer from 1 to 4; more preferably, an integer from 2 to 4; more preferably 1 or 2;
$t_2$ is an integer from 0 to 6; preferably, an integer from 1 to 4; more preferably 1 or 2;
$t_3$ is an integer from 1 to 6; preferably, an integer from 1 to 4; more preferably 1 or 2;
$t_4$ is an integer from 0 to 10; and
$t_5$ is an integer from 0 to 10.

**11.** The compound represented by the general formula (A) or the pharmaceutically acceptable salt thereof according to any one of claims 1 to 10, wherein $L_3$ is

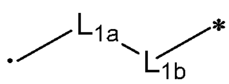

,

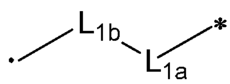

,

or

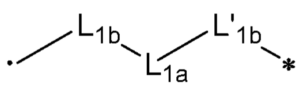

,

* is a connection site with $L_2$, and ·is a connection site with carbonyl or methylene;

$L_{1b}$ and $L'_{1b}$ are each independently an amino acid residue formed by one or more amino acids selected from phenylalanine, alanine, glycine, valine, leucine, isoleucine, tryptophan, tyrosine, histidine, lysine, citrulline, serine, threonine, cysteine, glutamic acid, glutamine, aspartic acid, asparagine, methionine, and arginine;
$L_{1a}$ is a bond or selected from one or more of the following structures:

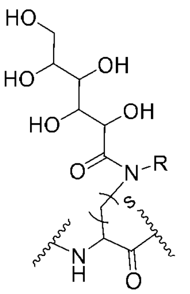 , 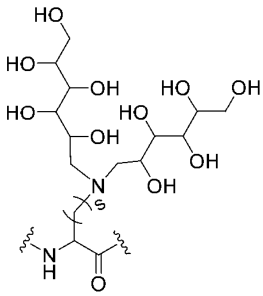 , 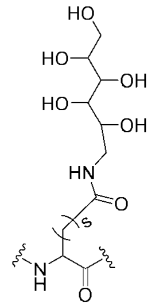 , 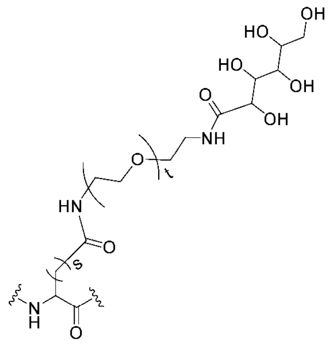 ,

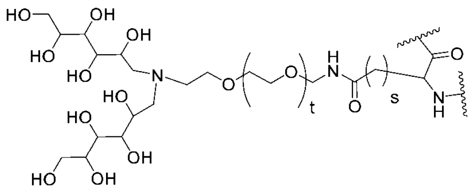 , 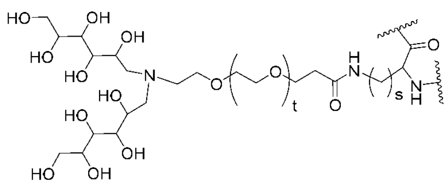 ,

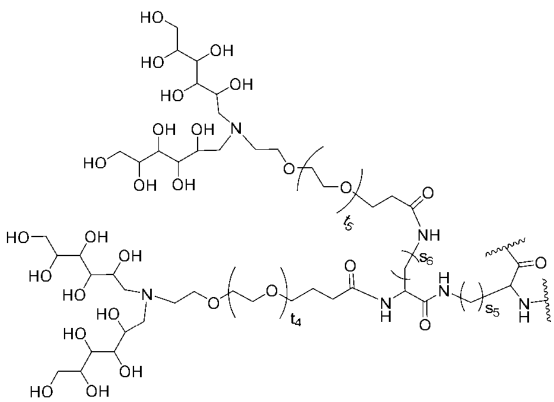 , 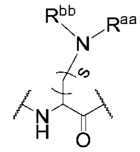 , or 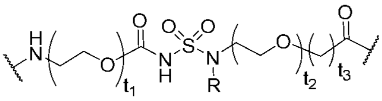 ;

preferably,

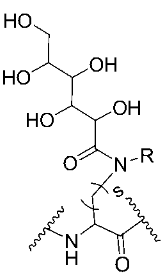 , 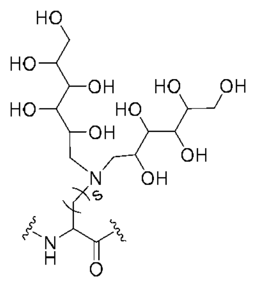 , 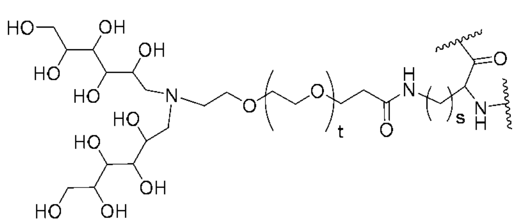 ,

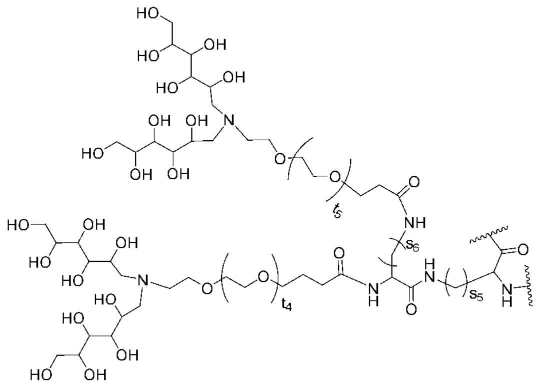, 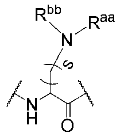, or 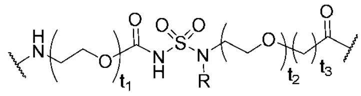,

wherein R is selected from hydrogen and $C_1$-$C_6$ alkyl, and is preferably hydrogen;
$R^{aa}$ and $R^{bb}$ are each independently selected from $C_1$-$C_6$ alkyl;
s is an integer from 1 to 6; preferably, an integer from 2 to 6; more preferably, an integer from 2 to 4;
$s_5$ and $s_6$ are each independently an integer from 1 to 6; preferably, an integer from 2 to 6; more preferably, an integer from 2 to 4;
t is an integer from 0 to 10;
$t_1$ is an integer from 1 to 6; preferably, an integer from 1 to 4; more preferably, an integer from 2 to 4; more preferably 1 or 2;
$t_2$ is an integer from 0 to 6; preferably, an integer from 1 to 4; more preferably 1 or 2;
$t_3$ is an integer from 1 to 6; preferably, an integer from 1 to 4; more preferably 1 or 2;
$t_4$ is an integer from 0 to 10; and
$t_5$ is an integer from 0 to 10.

**12.** The compound represented by the general formula (A) or the pharmaceutically acceptable salt thereof according to any one of claims 1 to 11, wherein $L_3$ is selected from:

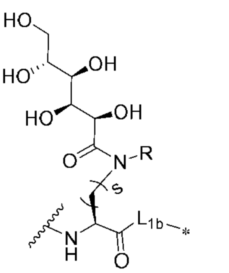, 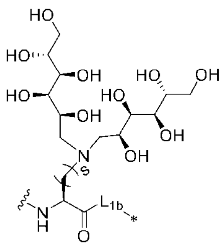, 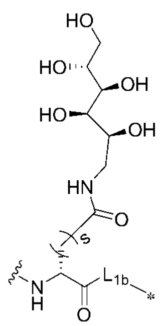, 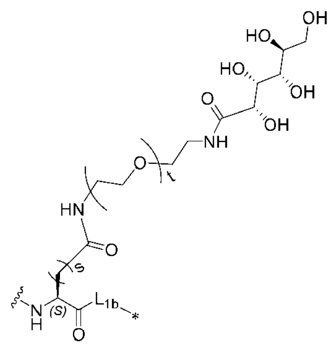,

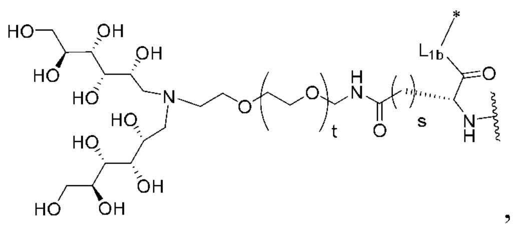, 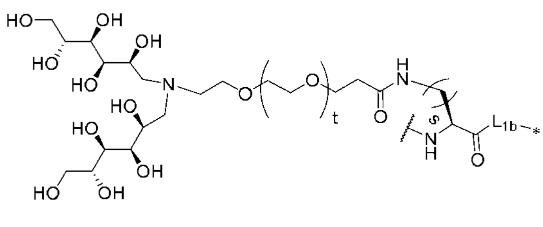,

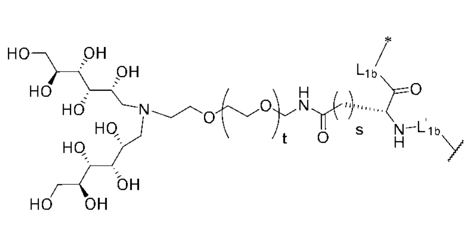, 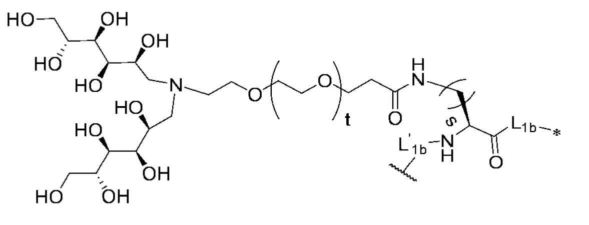,

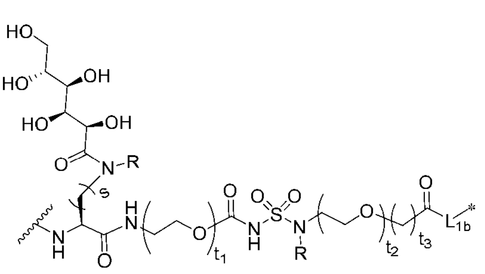 , 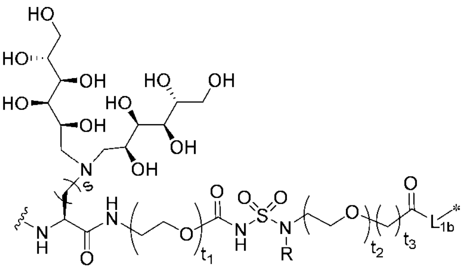 ,

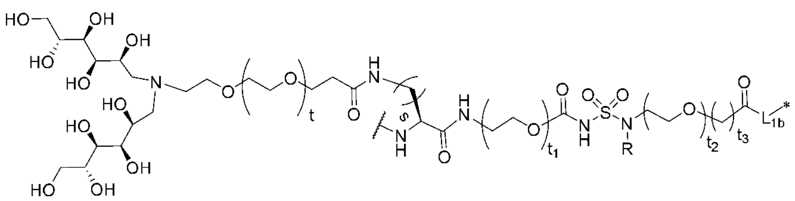 , 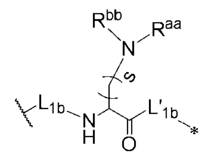 ,

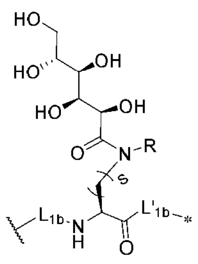 , and 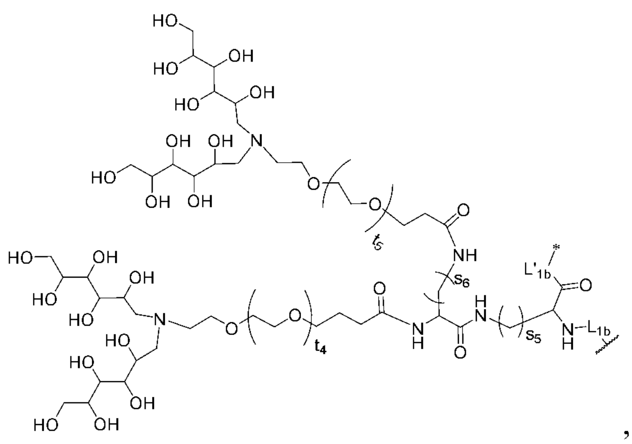 ,

wherein:

$L_{1b}$ and $L'_{1b}$ are each independently an amino acid residue formed by one or more amino acids selected from phenylalanine, alanine, glycine, valine, leucine, isoleucine, tryptophan, tyrosine, histidine, lysine, citrulline, serine, threonine, cysteine, glutamic acid, glutamine, aspartic acid, asparagine, methionine, and arginine;
R is selected from hydrogen and $C_1$-$C_6$ alkyl, and is preferably hydrogen;
$R^{aa}$ and $R^{bb}$ are each independently selected from $C_1$-$C_6$ alkyl;
s is an integer from 1 to 6; preferably, an integer from 2 to 6; more preferably, an integer from 2 to 4;
$s_5$ and $s_6$ are each independently an integer from 1 to 6; preferably, an integer from 2 to 6; more preferably, an integer from 2 to 4;
t is an integer from 0 to 10;
$t_1$ is an integer from 1 to 6; preferably, an integer from 1 to 4; more preferably, an integer from 2 to 4; even more preferably 1 or 2;
$t_2$ is an integer from 0 to 6; preferably, an integer from 1 to 4; more preferably 1 or 2;
$t_3$ is an integer from 1 to 6; preferably, an integer from 1 to 4; more preferably 1 or 2;
$t_4$ is an integer from 0 to 10;
$t_5$ is an integer from 0 to 10;
* is a connection site with $L_2$; and
~ is a connection site with carbonyl or methylene.

**13.** The compound represented by the general formula (A) or the pharmaceutically acceptable salt thereof according to claim 11 or 12, wherein $L_{1b}$ and $L'_{1b}$ are each independently an amino acid residue formed by one or more amino acids selected from glycine, phenylalanine, citrulline, leucine, isoleucine, alanine, valine, asparagine, glutamine, arginine, glutamic acid, and lysine; preferably an amino acid residue formed by two or more amino acids selected from glycine,

phenylalanine, citrulline, valine, lysine, glutamine, glutamic acid, aspartic acid, leucine, and alanine.

**14.** The compound represented by the general formula (A) or the pharmaceutically acceptable salt thereof according to any one of claims 11 to 13, wherein $L_{1b}$ and $L'_{1b}$ are each independently selected from -Gly-*, -Val-*, -Gly-Phe-Gly-*, -Phe-Gly-*, -Gly-Val-Cit-*, -Val-Cit-*, -Gly-Val-Arg-*, -Val-Arg-*, -Gly-Val-Ala-*, -Val-Ala-*, -Gly-Phe-*, -Phe-Gly-*, -Gly-Gly-Gly-*, Gly-Gly-*, -Gly-Val-Gly-*, -Gly-Ala-Gly-*, -Gly-Phe-Cit-*, -Gly-Phe-Val-*, -Gly-Phe-Ala-*, -Gly-Phe-Lys-*, -Phe-Lys-*, -Gly-Val-*, -Gly-Cit-*, -Gly-Ala-*, -Gly-Gly-Lys-*, Gly-Lys'-*, -Ala-Ala-Ala-*, -Gln-Val-Ala-*, -Gln-Val-Cit-*, -Asp-Val-Ala-*, -Asp-Val-Cit-*, -Lys-Gly-Val-Ala-*, -Lys-Gly-Val-Cit-*, -Lys-Gly-Gly-Val-Ala-*, -Lys-Gly-Gly-Val-Cit-*, Gly-Gly-Phe-Gly-*, -Lys-Gln-Val-Cit-*, -Lys-Gln-Val-Ala-*, -Lys-Glu-Val-Cit-*, -Lys-Glu-Val-ALa-*, -Lys-Asp-Val-Cit-*, -Lys-Asp-Val-Ala-*, Glu-Val-Cit-*, Glu-Val-Ala-*, -Lys-Val-Ala-*, -Lys-Val-Cit-*, -Val-Lys-Gly-*, and -Val-Lys-*; preferably, -Gly-Phe-Gly-*, -Gly-Val-Cit-*, -Val-Cit-*, -Gly-Val-Ala-*, -Val-Ala-*, -Gly-Phe-Gly-*, -Phe-Gly-*, - Gly-Phe-Lys-, -Phe-Lys- *, -Gln-Val-Ala-*, -Gln-Val-Cit-*, -Asp-Val-Ala-*, -Lys-Gly-Val-Ala-*, - Lys-Gly-Val-Cit-*, -Lys-Gly-Gly-Val-Ala-*, -Lys-Gly-Gly-Val-Cit-*, Gly-Gly-Phe-Gly-*, -Lys-Gln-Val-Cit-*, -Lys-Gln-Val-Ala-*, -Lys-Glu-Val-Cit-*, -Lys-Glu-Val-ALa-*, -Lys-Asp-Val-Cit-*, -Lys-Asp-Val-Ala-*, Glu-Val-Cit-*, Glu-Val-Ala-*, -Lys-Val-Ala-*, -Lys-Val-Cit-*, -Val-Lys-Gly-*, -Val-Lys-*, and -Asp-Val-Cit-*, wherein * is a connection site with $L_2$.

**15.** The compound represented by the general formula (A) or the pharmaceutically acceptable salt thereof according to any one of claims 1 to 14, wherein $L_3$ is selected from:

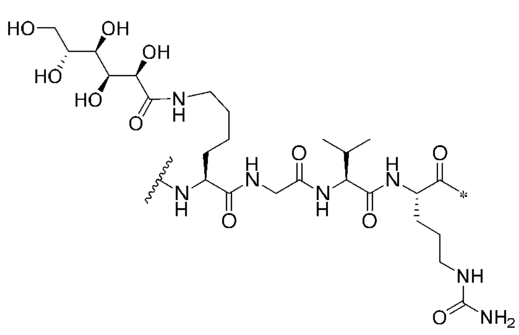
,
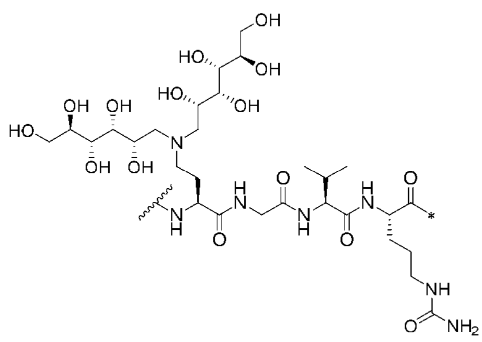
,
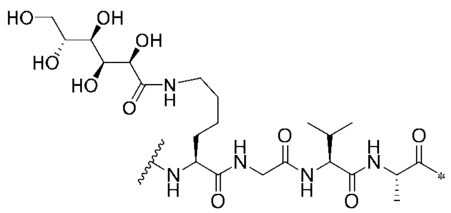
,
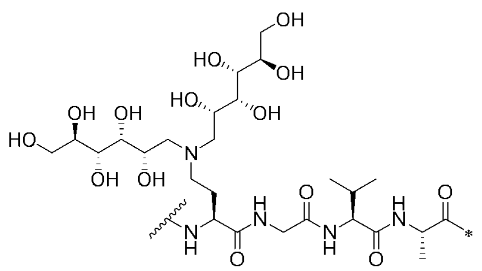
,
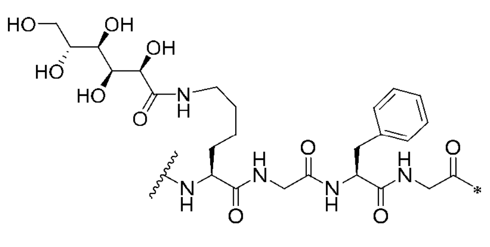
,
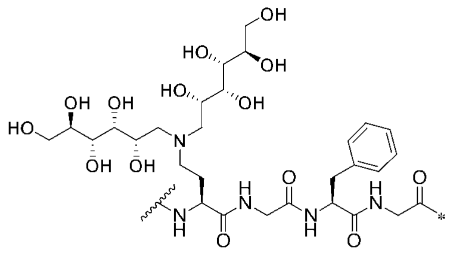
,

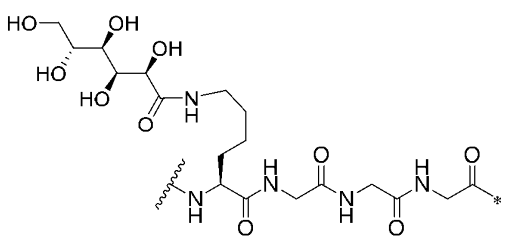

,

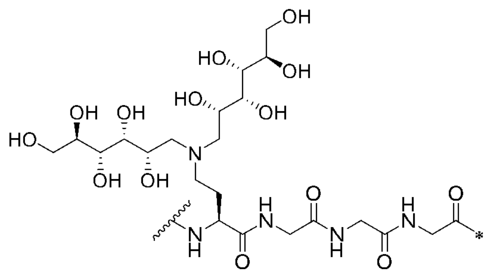

,

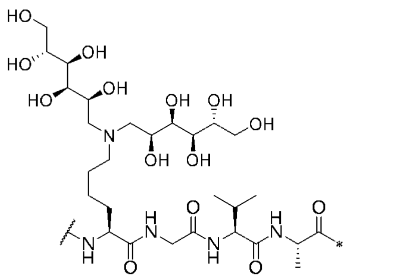

;

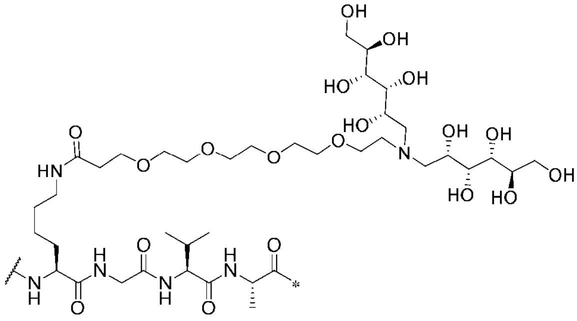

,

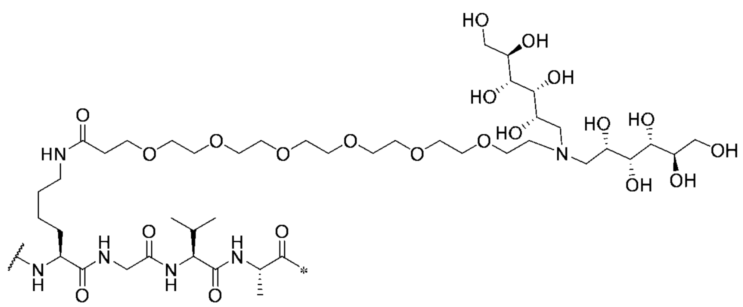

,

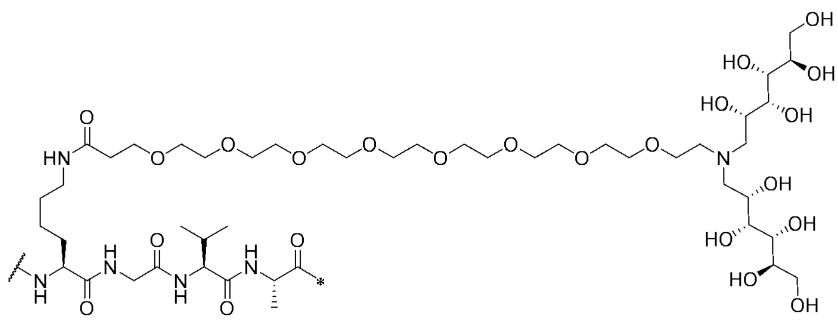

,

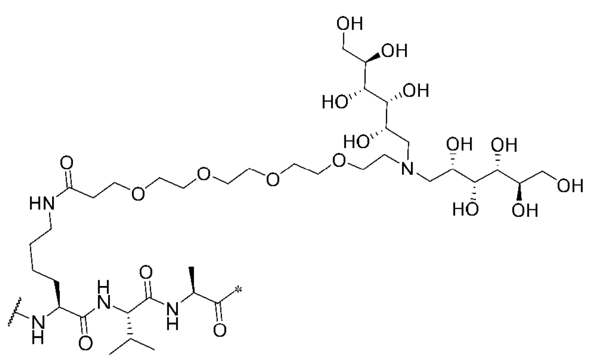

,

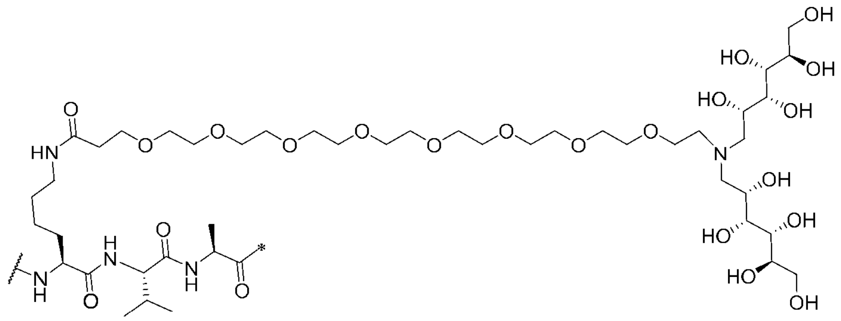

,

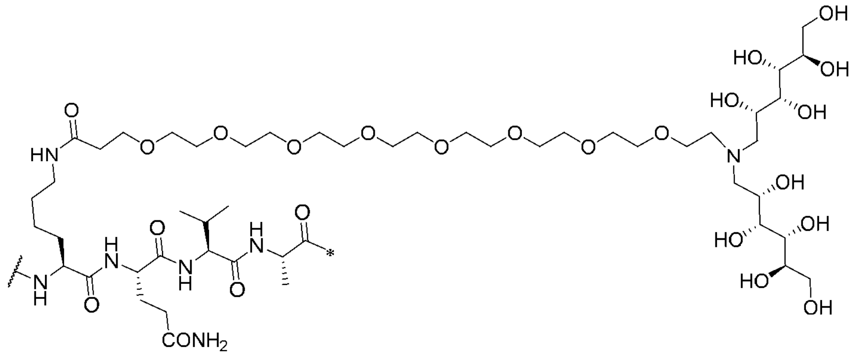

,

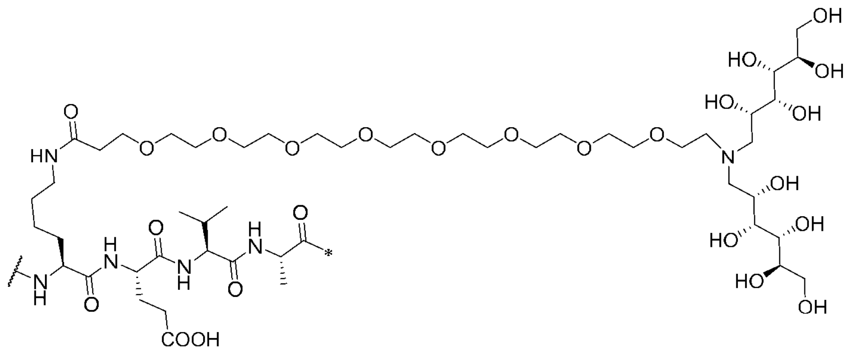

,

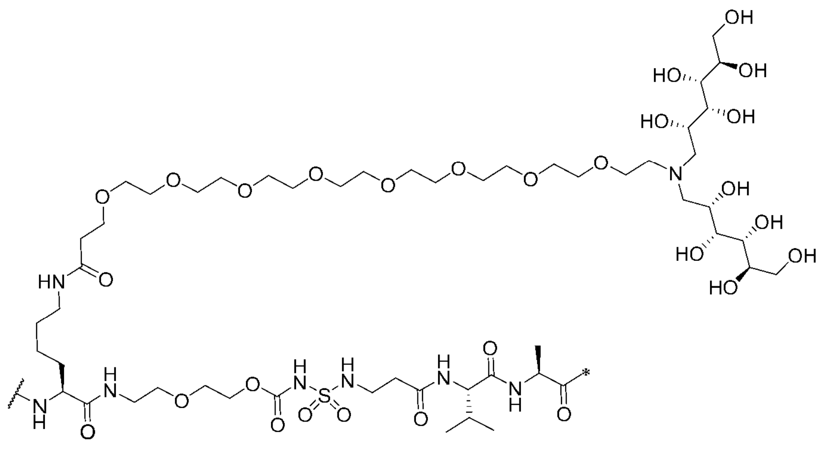

,

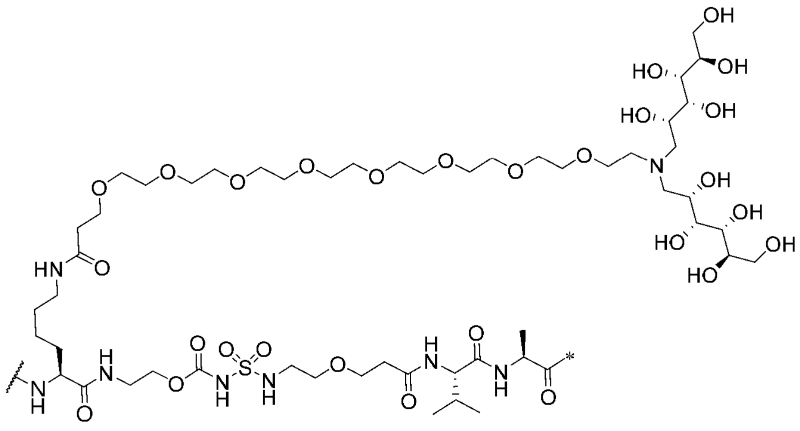
,
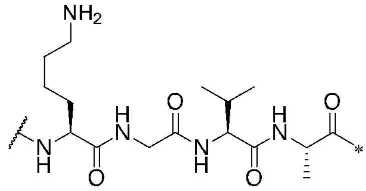
,

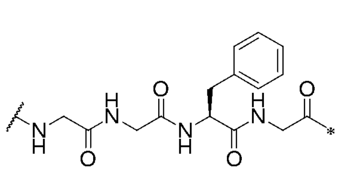
,
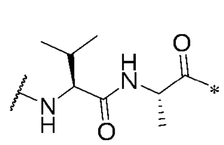
,
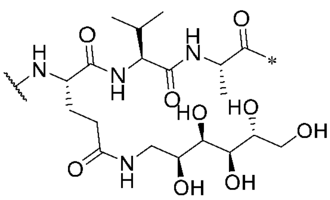
,
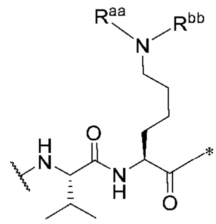
,

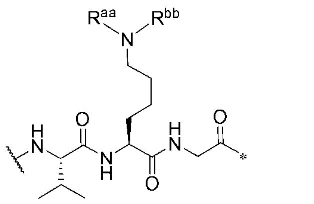
,
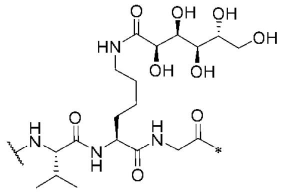
,
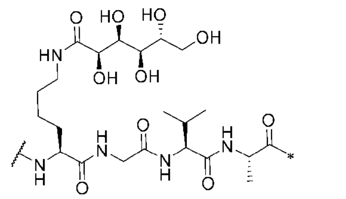
,

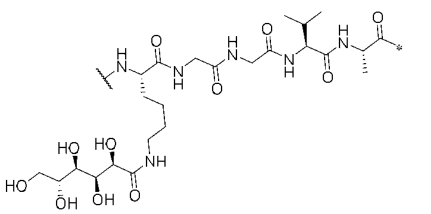
,

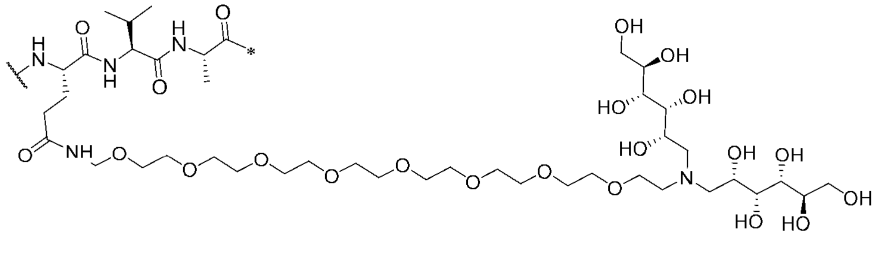
,

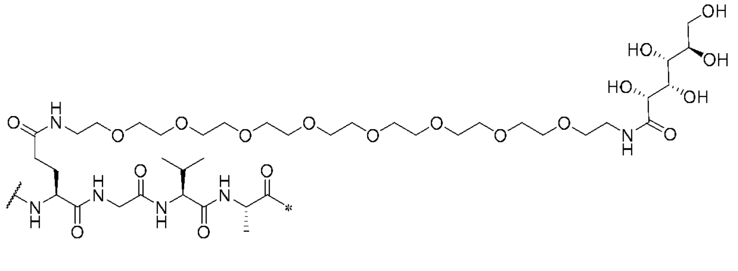
,

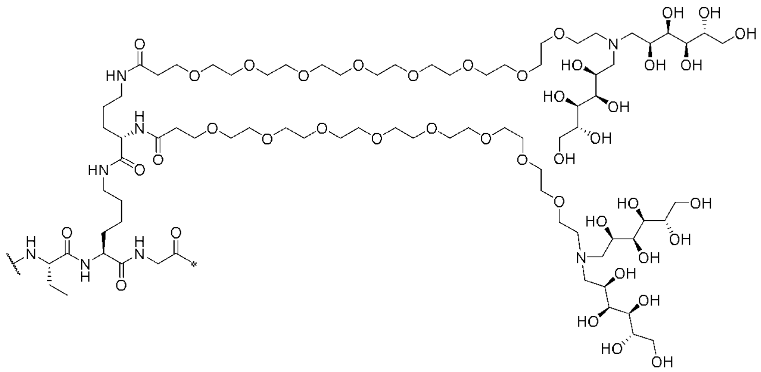

,

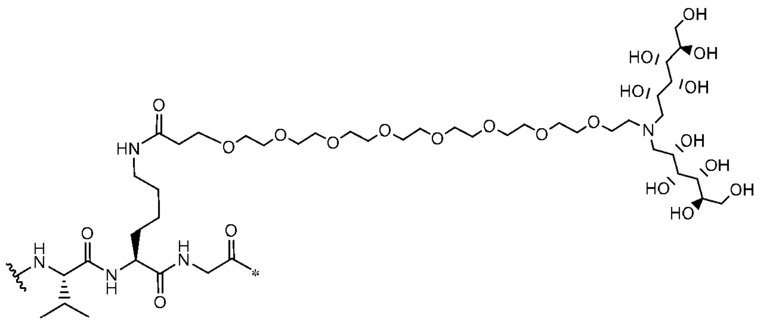

,

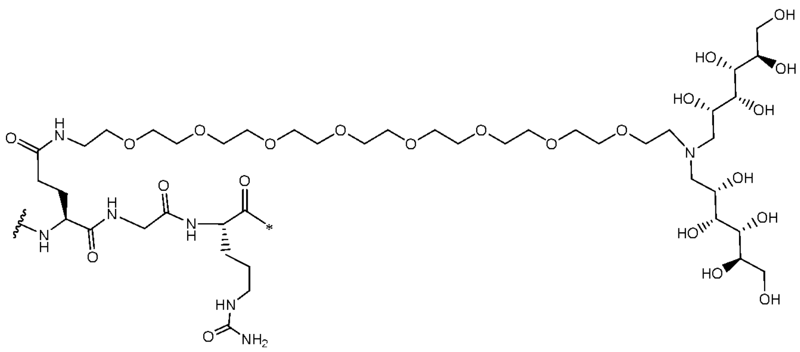

,

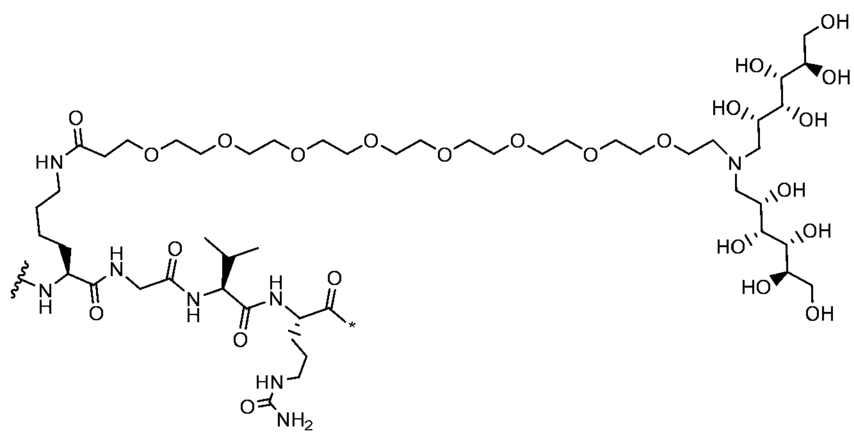

,

$R^{aa}$ and $R^{bb}$ are each independently selected from $C_1$-$C_6$ alkyl;
* is a connection site with L2; and

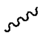 is a connection site with carbonyl or methylene.

**16.** The compound represented by the general formula (A) or the pharmaceutically acceptable salt thereof according to any one of claims 1 to 15, wherein Q is selected from

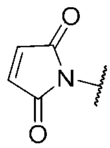, 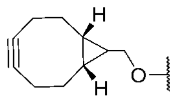,

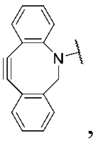, 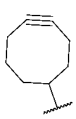, 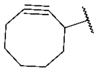, 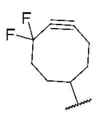, 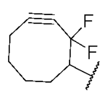, 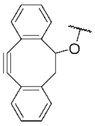, 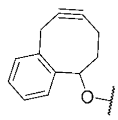, 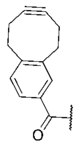, 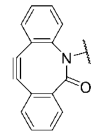, 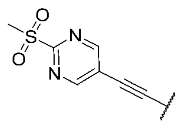,

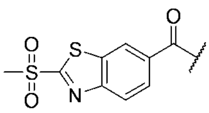, 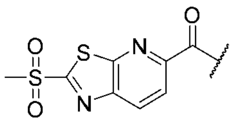, 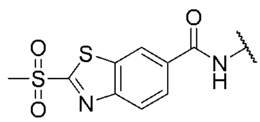, and 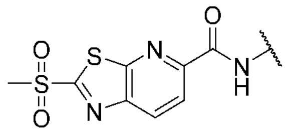;

preferably,

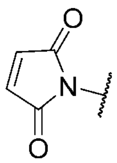, 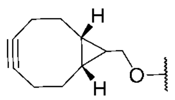, 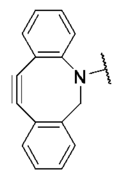, 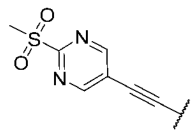, 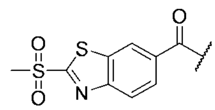,

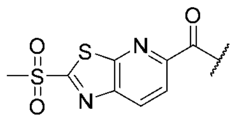, 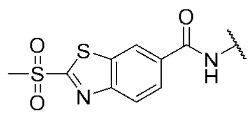, and 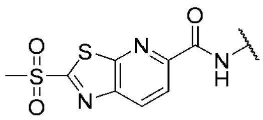.

**17.** The compound represented by the general formula (A) or the pharmaceutically acceptable salt thereof according to any one of claims 1 to 16, wherein $Z_1$ is selected from a bond, $-(CH_2)_p-$, $-(CH_2)_p-C(O)NH-$, $-(CH_2)_p-O-(CH_2)_p-C(O)NH-$, $-(CH_2)_p-C(O)-L_6-NH-$, $-(CH_2)_p-O-(CH_2)_p-C(O)-L_6-NH-$,$-C(O)NH-$, $-C(O)O-$, $-C(O)-$, $-OC(O)-$, and $-OC(O)NH-$, wherein p is an integer from 1 to 10; preferably, an integer from 1 to 6;

$s_1$, $s_2$, $s_3$, and $s_4$ are each independently an integer from 0 to 10; preferably, an integer from 0 to 6; more preferably, an integer from 0 to 4; even more preferably, an integer from 0 to 2;
$L_6$ is selected from

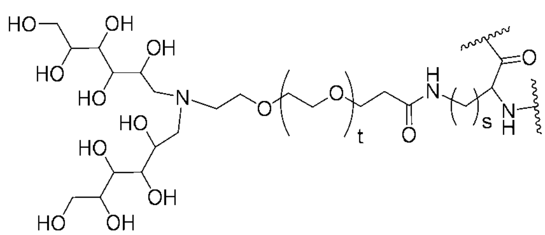

and

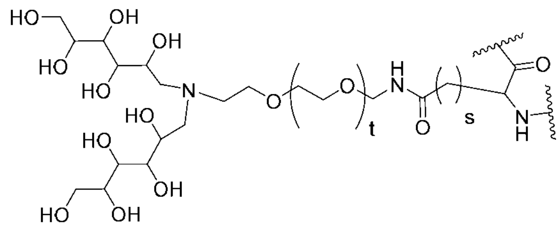

;

s is an integer from 1 to 6; preferably, an integer from 2 to 6; more preferably, an integer from 2 to 4;
t is an integer from 0 to 10; and
$L_6$ is preferably

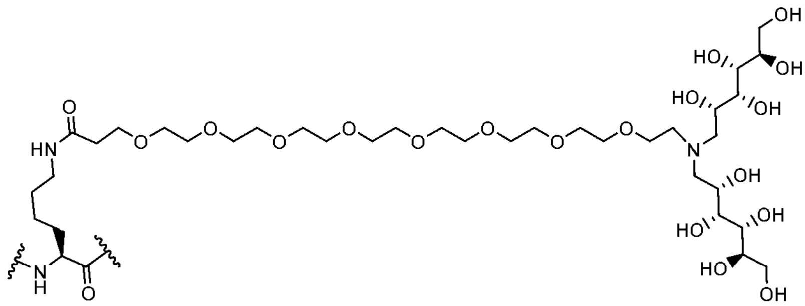

.

**18.** The compound represented by the general formula (A) or the pharmaceutically acceptable salt thereof according to any one of claims 1 to 17, wherein

$Z_1$ is selected from a bond, $-(CH_2)_p-$, $-(CH_2)_p-C(O)NH-$, $-(CH_2)_p-O-(CH_2)_p-C(O)NH-$, -C(O)NH-,-C(O)O-, -C(O)-, -OC(O)-, and -OC(O)NH-;
$s_1$ is an integer from 0 to 6; preferably, an integer from 0 to 2;
$s_2$ is an integer from 0 to 6; preferably, an integer from 0 to 2;
$s_3$ is 0;
$s_4$ is 0; and
p is an integer from 1 to 10; preferably, an integer from 1 to 6.

**19.** The compound represented by the general formula (A) or the pharmaceutically acceptable salt thereof according to any one of claims 1 to 17, wherein

$Z_1$ is selected from $-(CH_2)_p-C(O)NH-$, $-(CH_2)_p-O-(CH_2)_p-C(O)NH-$, and -C(O)NH-;
$s_1$ is an integer from 1 to 6; preferably, an integer from 2 to 6;
$s_2$ is an integer from 1 to 10; preferably, an integer from 2 to 10;
$s_3$ is 0;
$s_4$ is 0; and
p is an integer from 1 to 10; preferably, an integer from 1 to 6.

**20.** The compound represented by the general formula (A) or the pharmaceutically acceptable salt thereof according to any one of claims 1 to 19, wherein Q-$L_4$- is selected from:

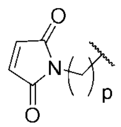

,

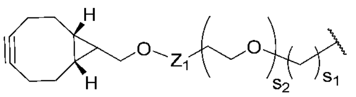

,

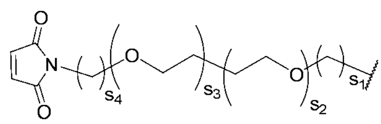

,

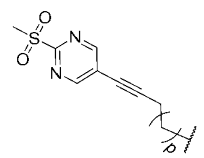

,

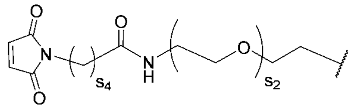

,

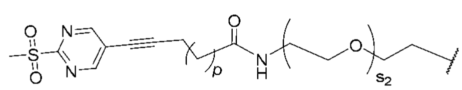

,

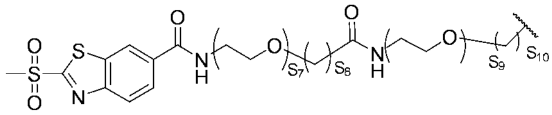, and 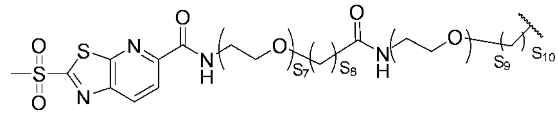,

wherein:

$Z_1$ is selected from -C(O)NH-, -C(O)O-, -C(O)-, -OC(O)-, and -OC(O)NH-, preferably -C(O)NH-;
p is an integer from 1 to 10; preferably, an integer from 1 to 6;
$s_1$ is an integer from 0 to 6; preferably, an integer from 0 to 2;
$s_2$ is an integer from 1 to 10; preferably, an integer from 1 to 8;
$s_3$ is an integer from 0 to 6; preferably, an integer from 0 to 2;
$s_4$ is an integer from 1 to 6; preferably, an integer from 1 to 2;
$s_7$ is an integer from 0 to 6; preferably, an integer from 1 to 2;
$s_8$ is an integer from 1 to 4; preferably, an integer from 1 to 2;
$s_9$ is an integer from 1 to 10; preferably, an integer from 1 to 8; and
$s_{10}$ is an integer from 1 to 4; preferably, an integer from 1 to 2.

**21.** The compound represented by the general formula (A) or the pharmaceutically acceptable salt thereof according to any one of claims 1 to 20, wherein the compound is selected from:

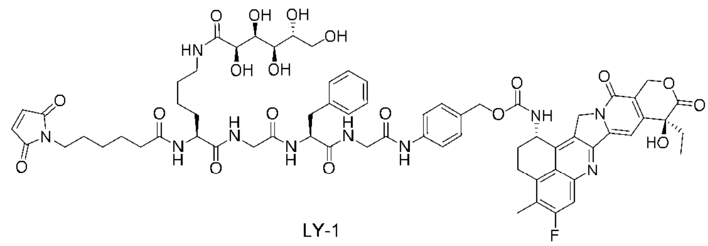

LY-1

,

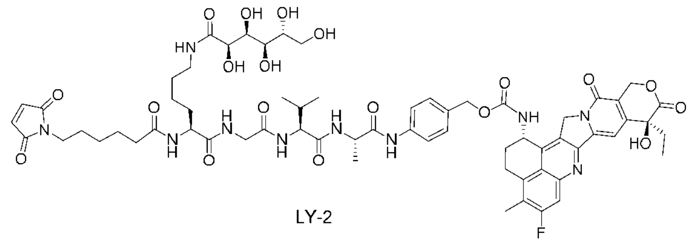

LY-2

,

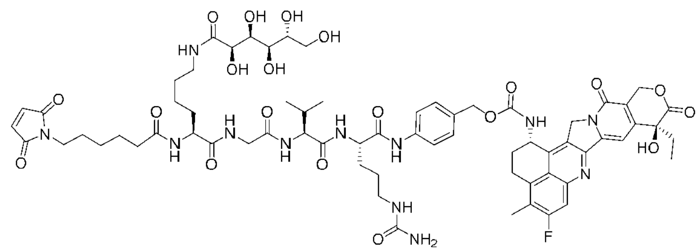

LY-3

,

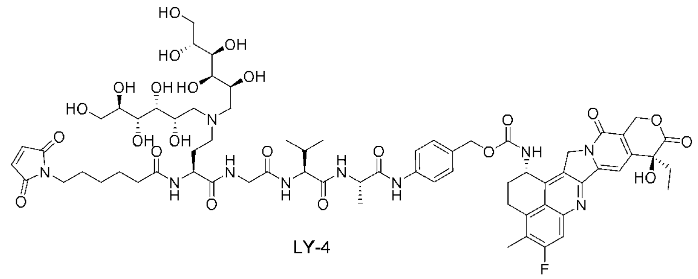

LY-4

,

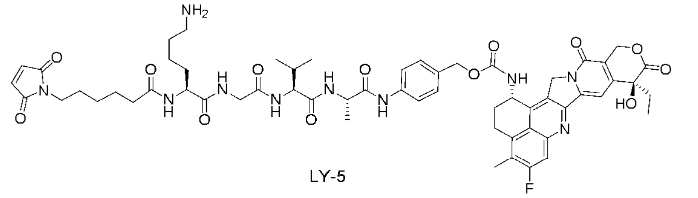

LY-5

,

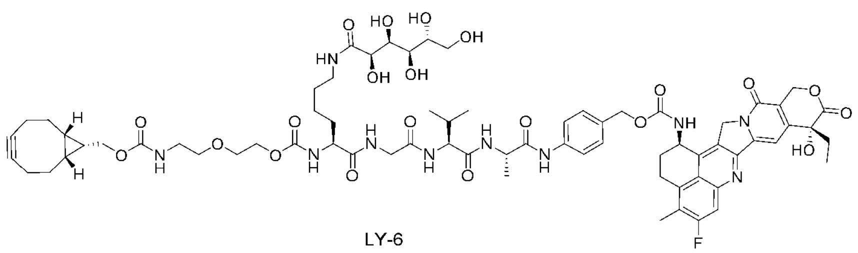

LY-6

,

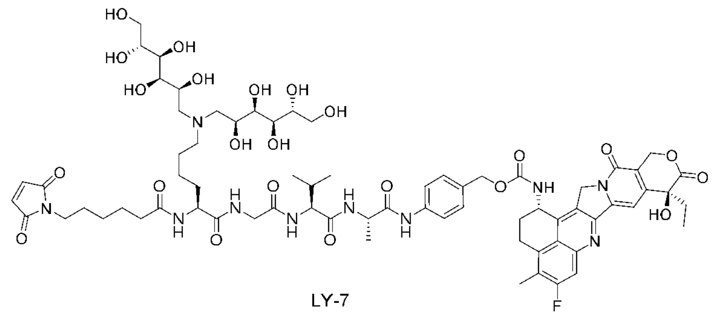

LY-7

,

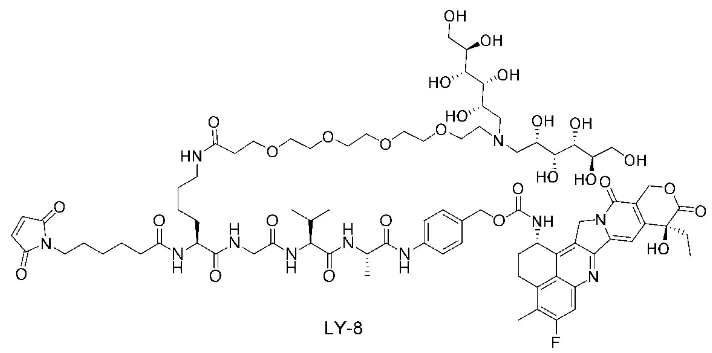

LY-8

,

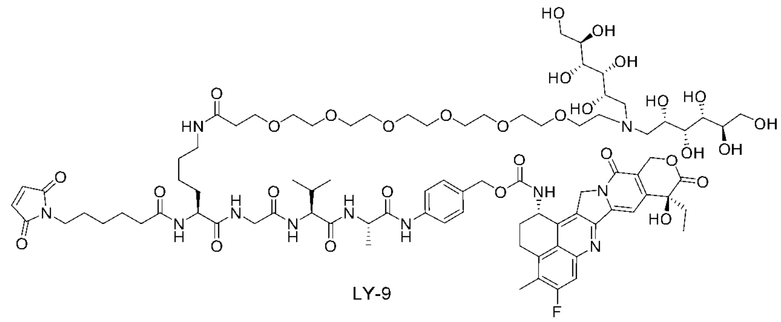

LY-9

,

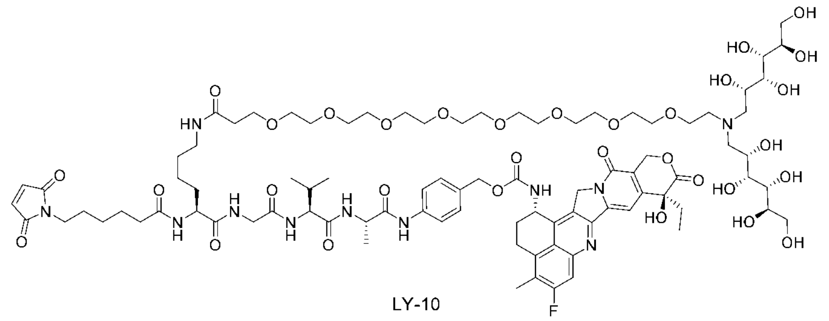

LY-10

,

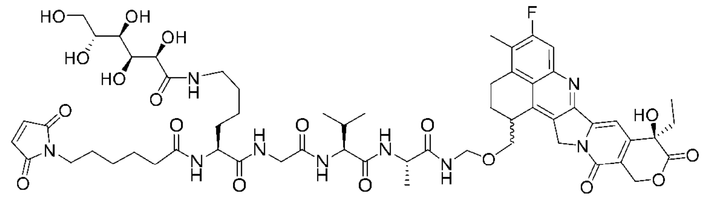

LY-11

,

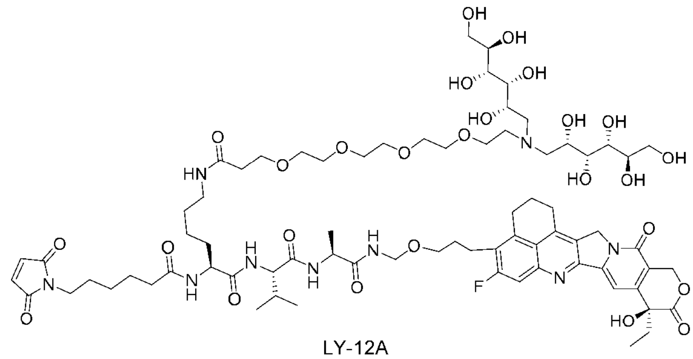

LY-12A

,

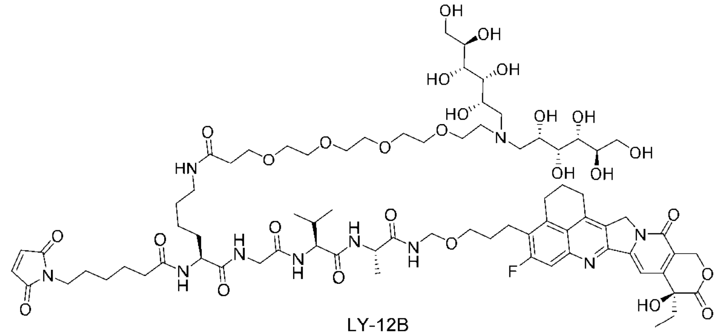

LY-12B

,

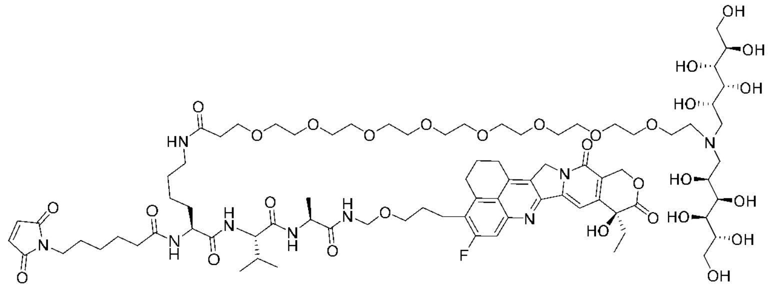

LY-18A

,

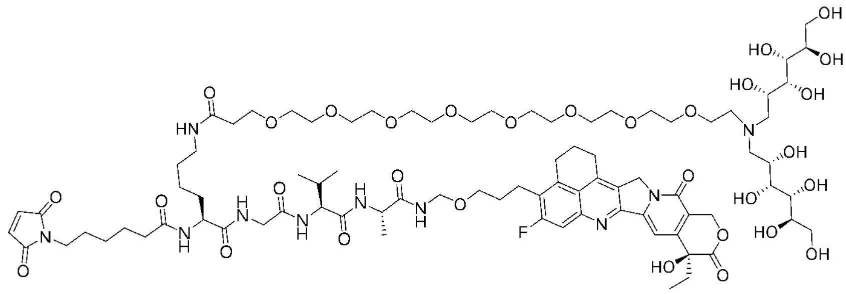

LY-18B

,

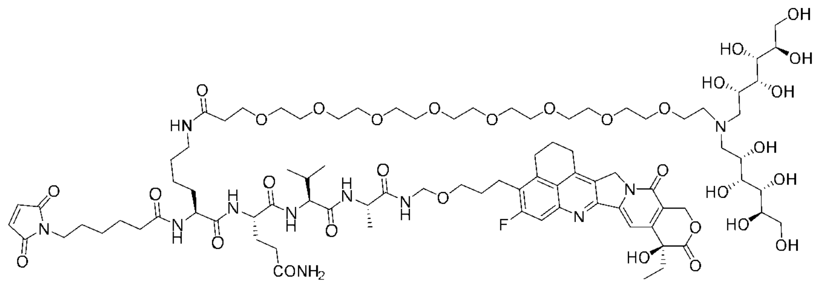

LY-18C

,

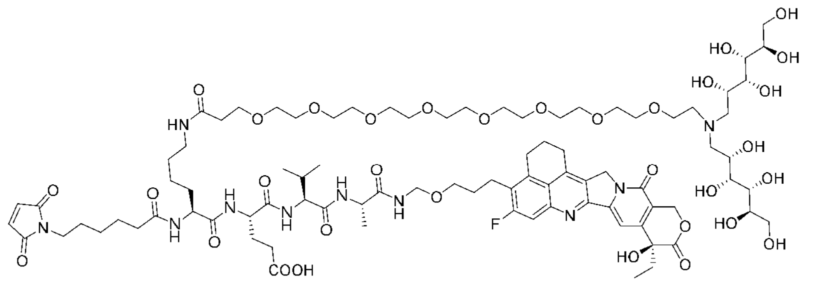

LY-18D

,

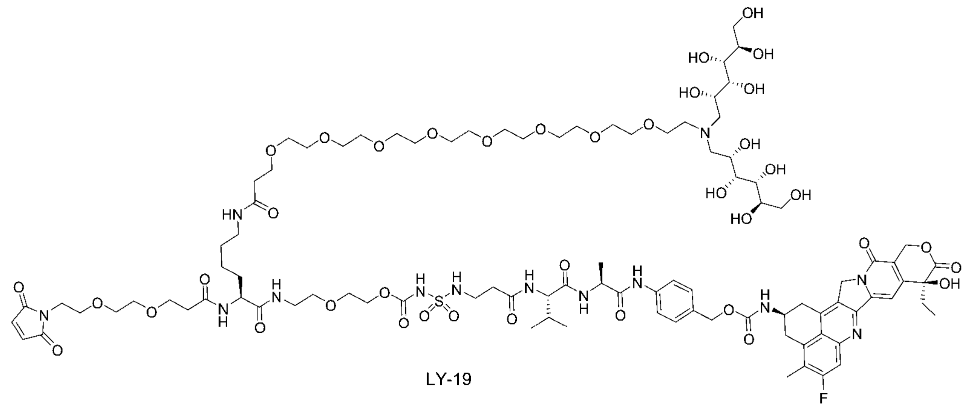

LY-19

,

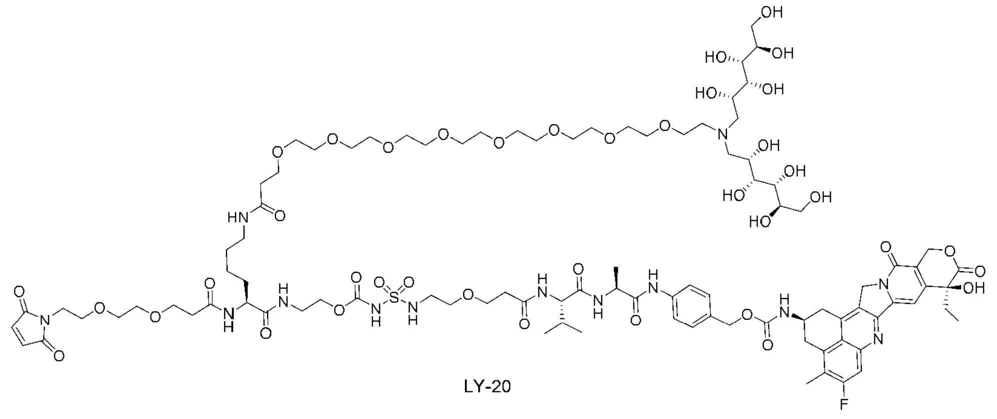

LY-20

,

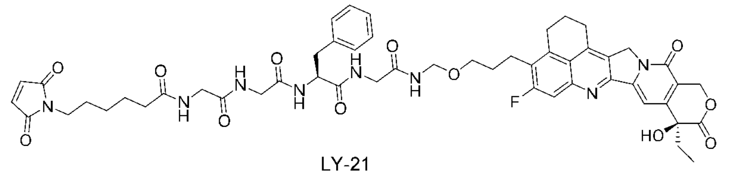

LY-21

,

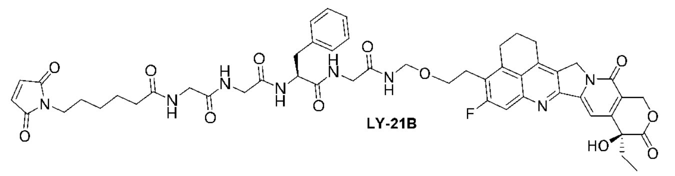

LY-21B

,

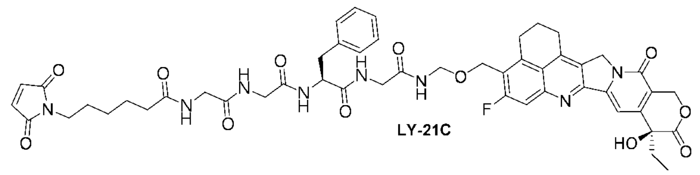

LY-21C

,

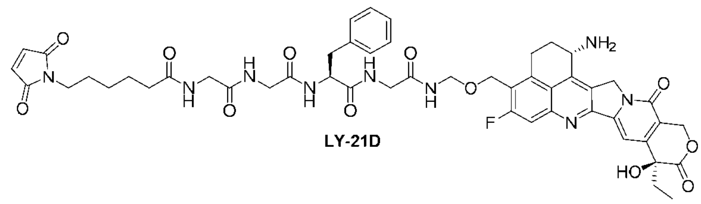

LY-21D

,

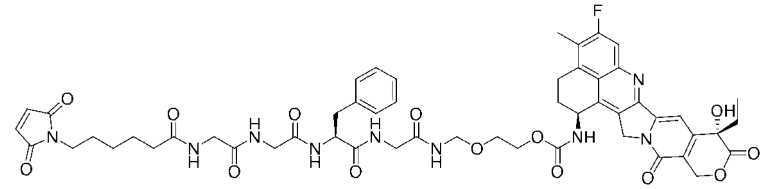

**LY-22**

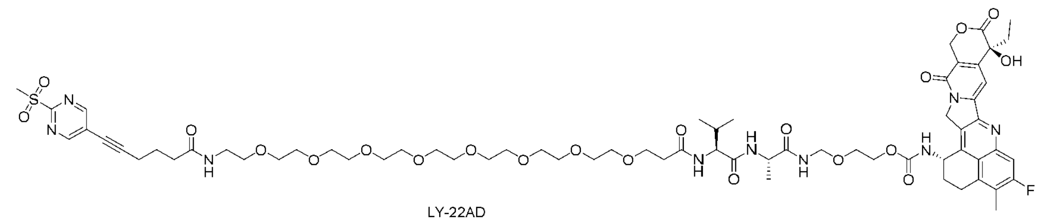

LY-22AD

,

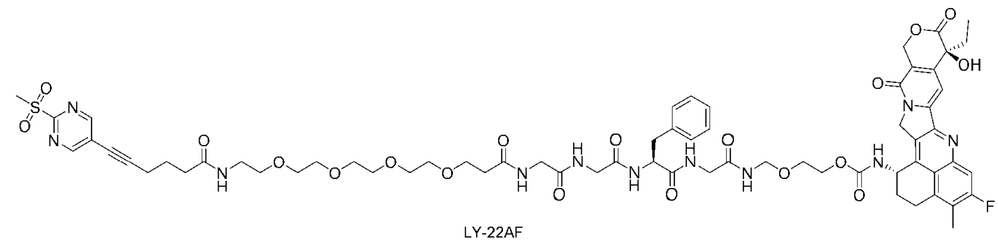

LY-22AF

,

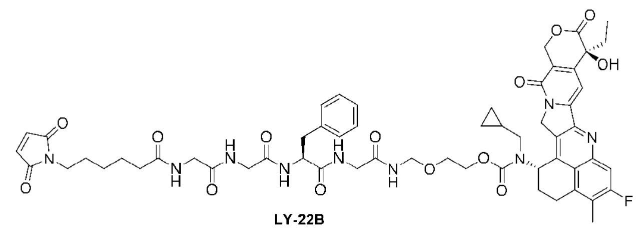

**LY-22B**

,

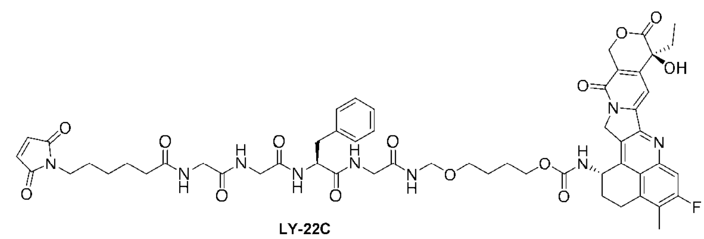

**LY-22C**

,

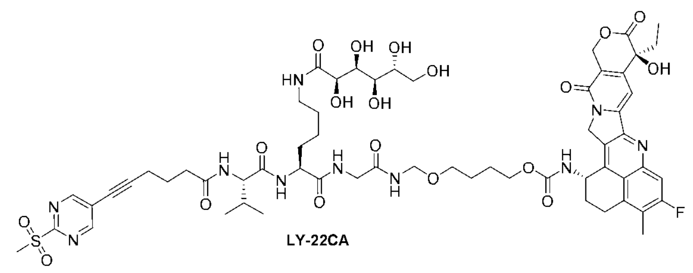

**LY-22CA**

,

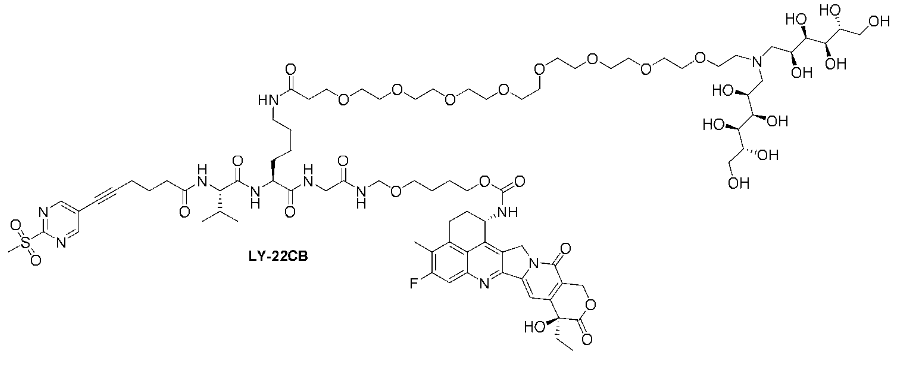

LY-22CB

,

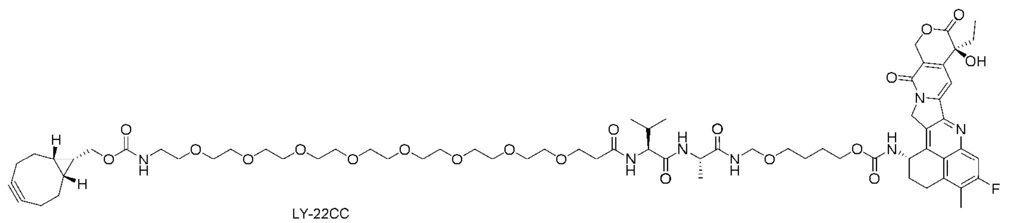

LY-22CC

,

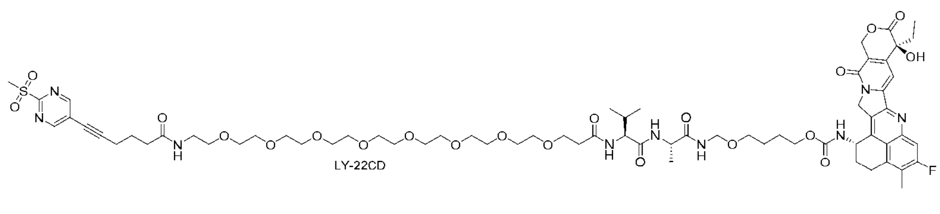

LY-22CD

,

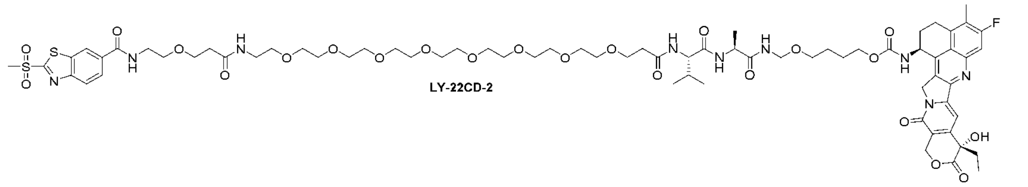

LY-22CD-2

,

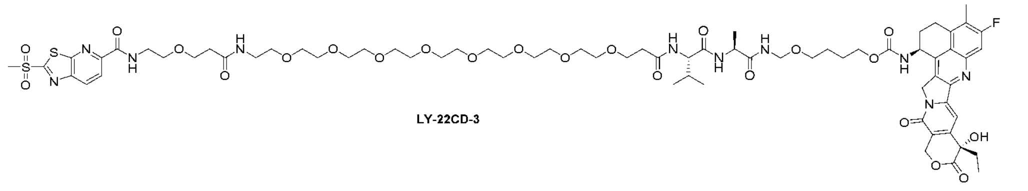

LY-22CD-3

,

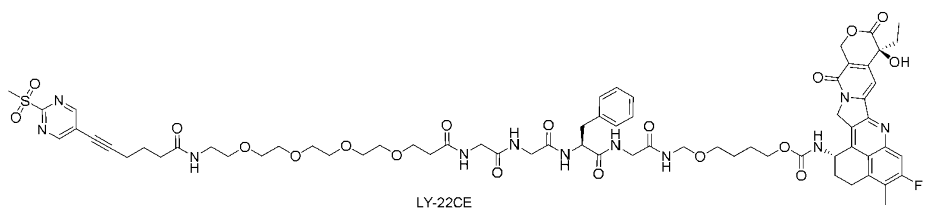

LY-22CE

,

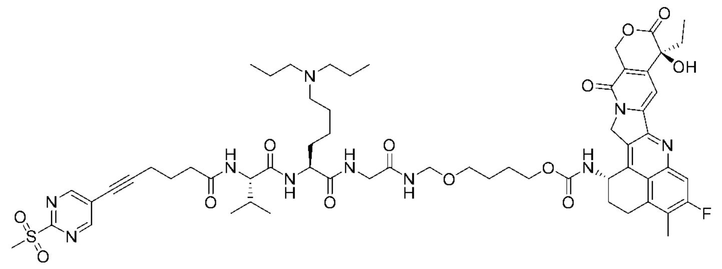

**LY-22CF**

,

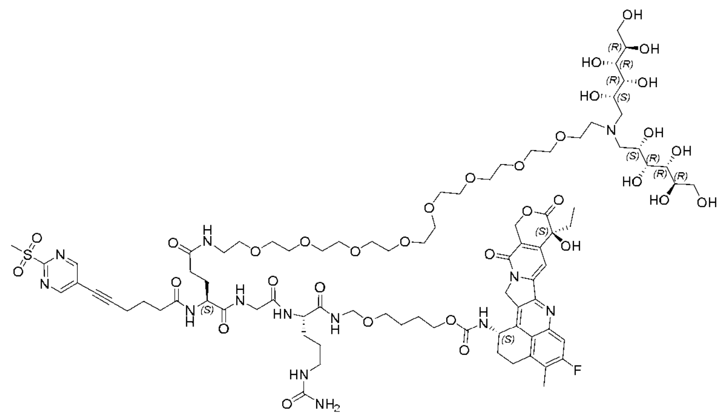

LY-22CG

,

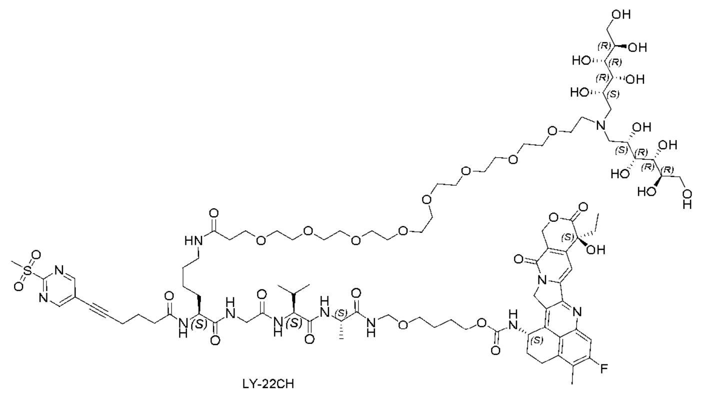

LY-22CH

,

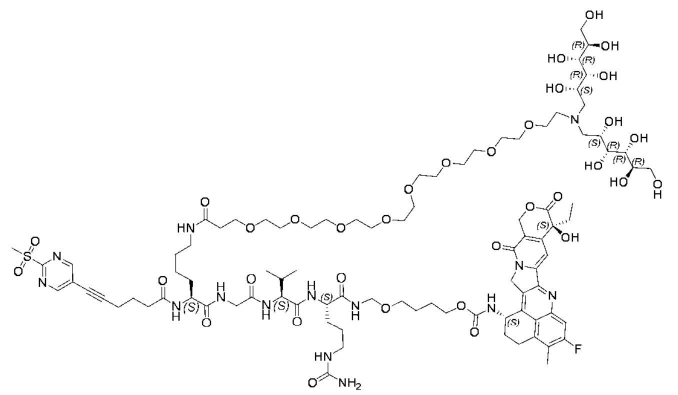

LY-22CI

,

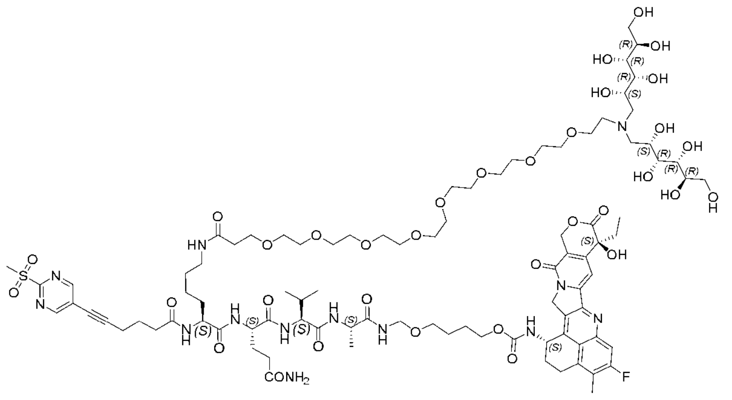

LY-22CJ

,

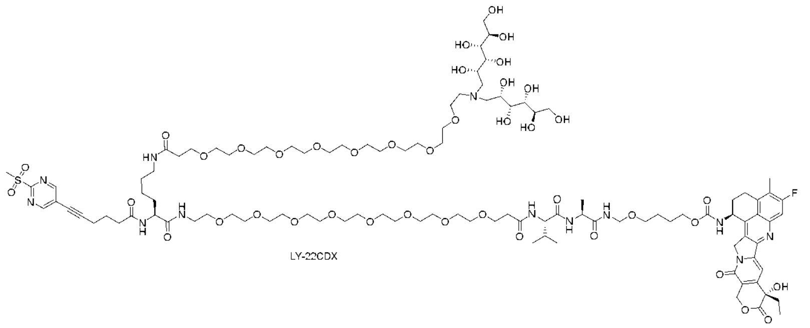

LY-22CDX

,

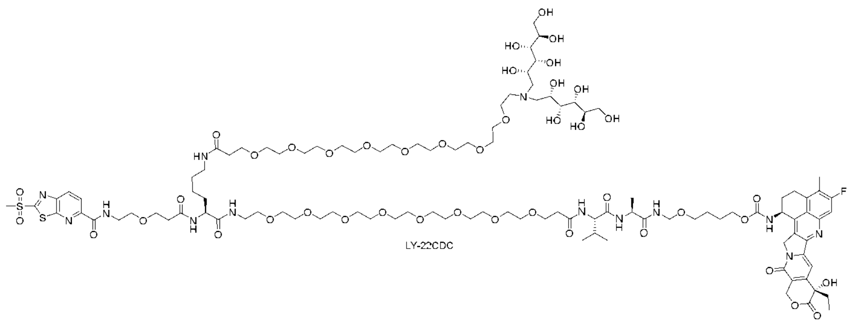

LY-22CDC

,

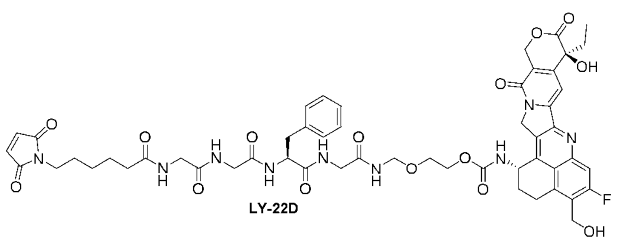

**LY-22D**

,

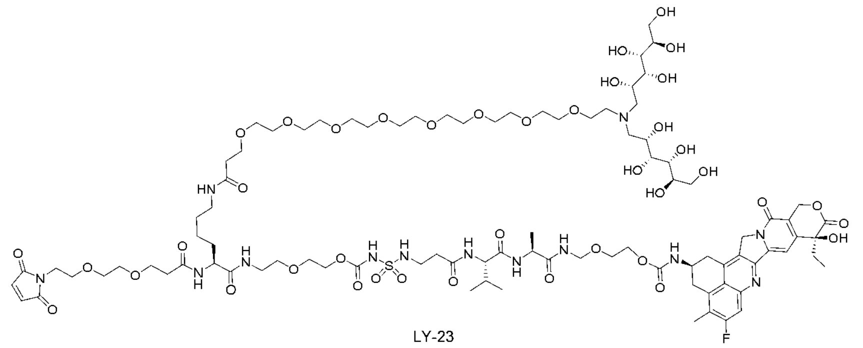

LY-23

,

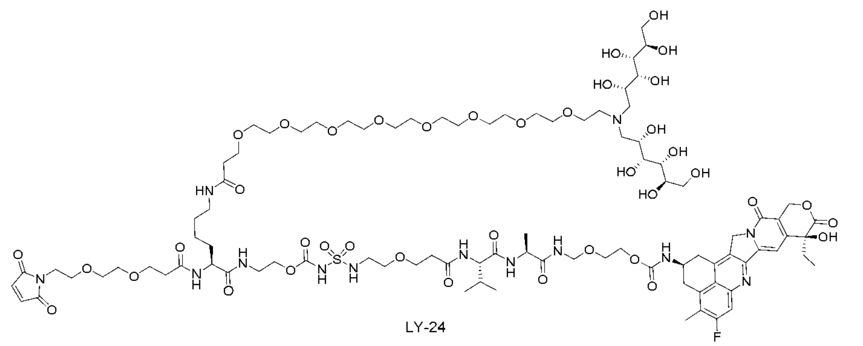

LY-24

,

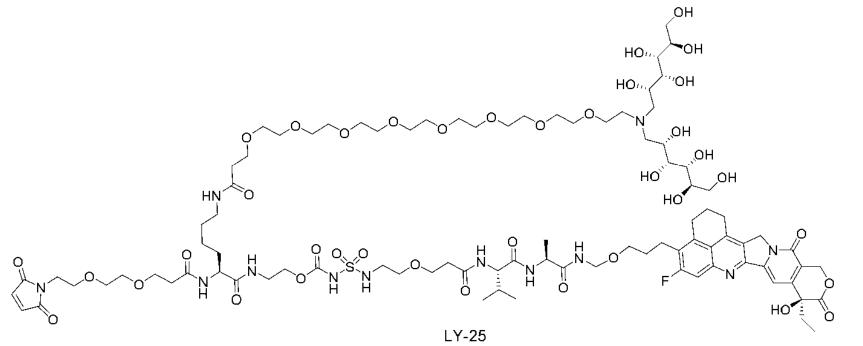

LY-25

,

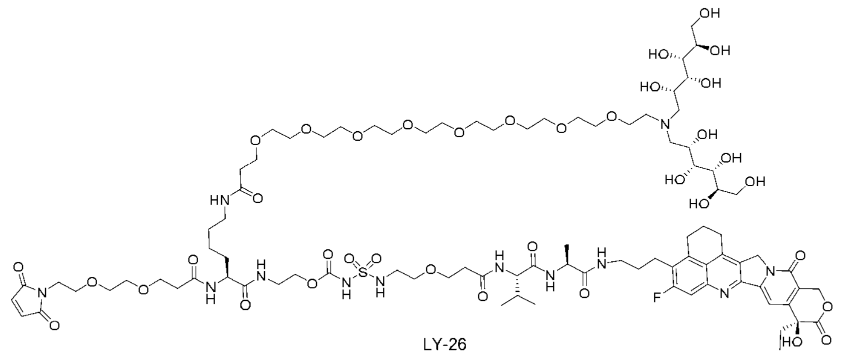

LY-26

,

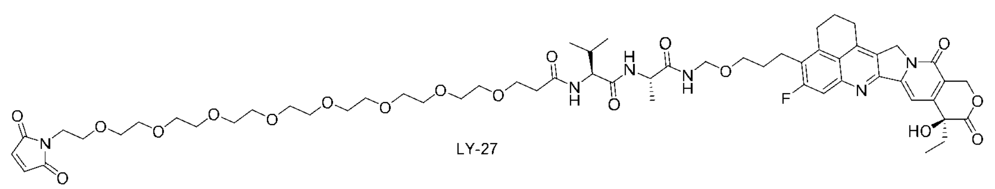

LY-27

,

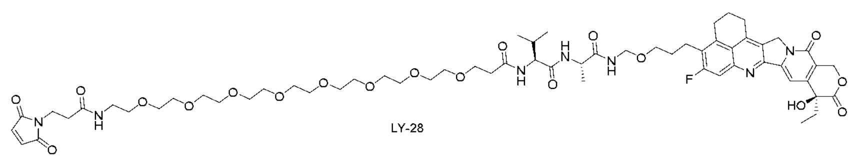
LY-28

,

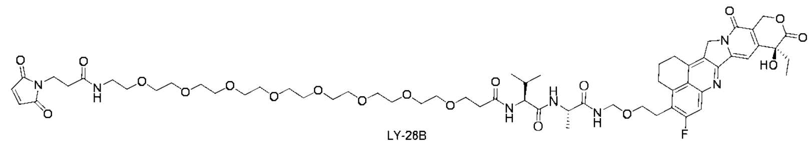
LY-28B

,

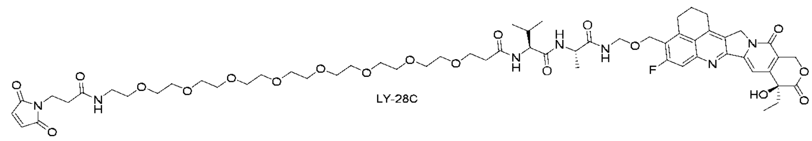
LY-28C

,

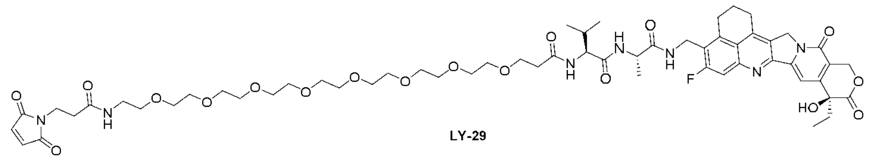
**LY-29**

,

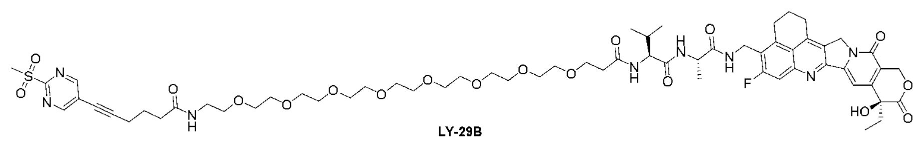
**LY-29B**

,

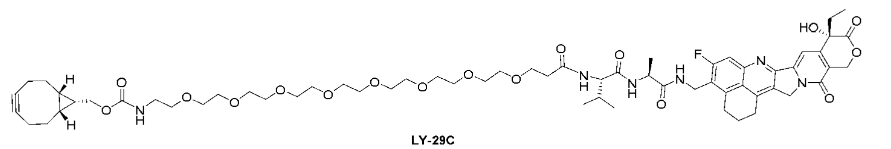
**LY-29C**

,

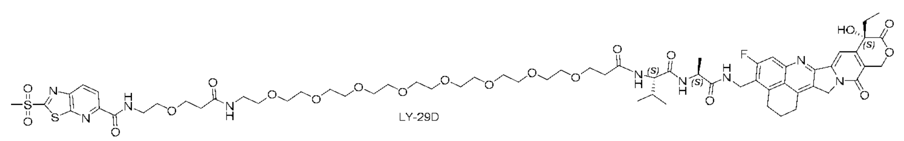
LY-29D

,

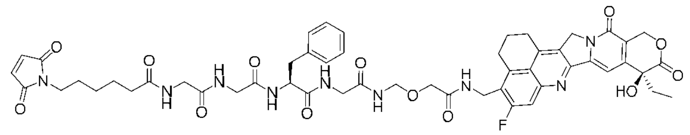
LY-33

,

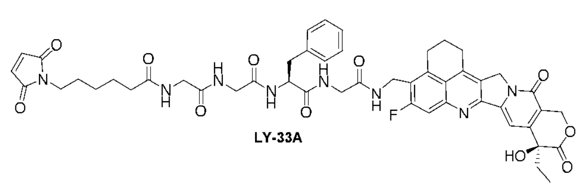
**LY-33A**

,

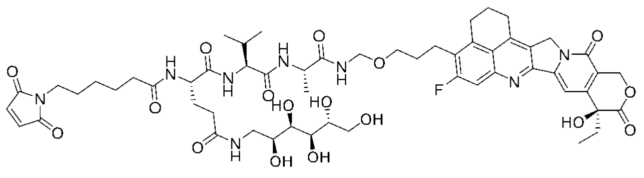

LY-34

,

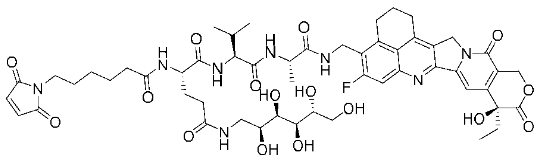

LY-34B

,

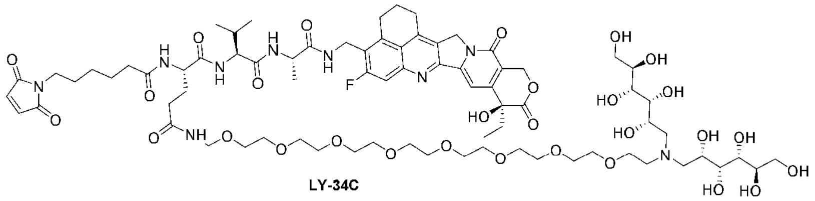

LY-34C

,

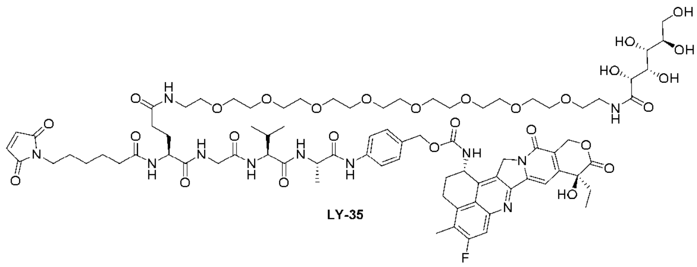

LY-35

,

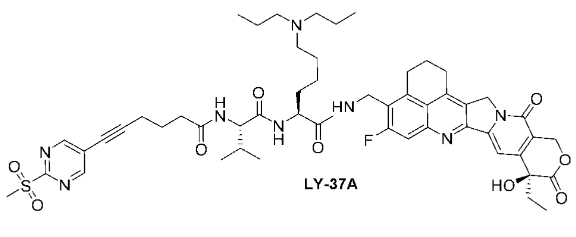

LY-37A

,

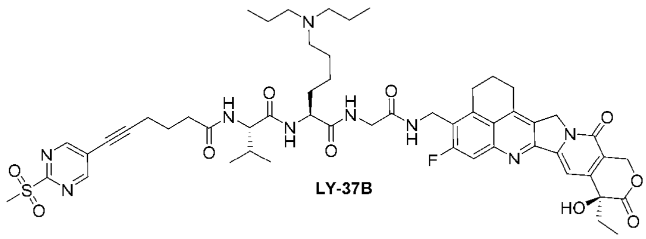

LY-37B

,

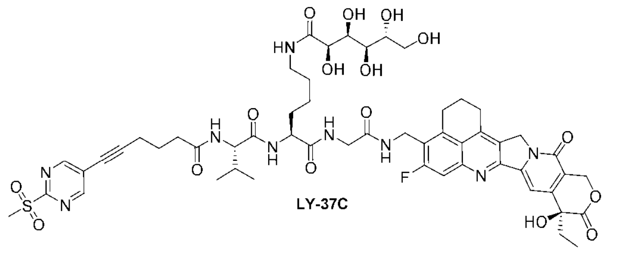

LY-37C

,

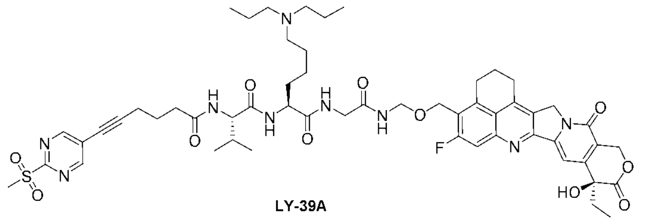

LY-39A

,

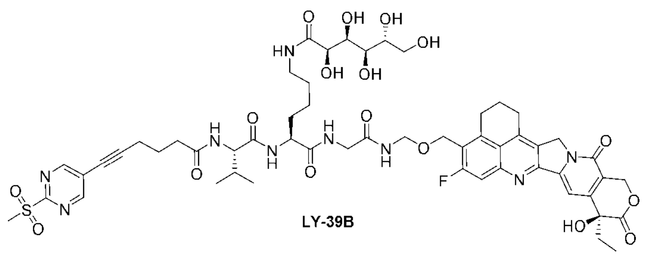

LY-39B

,

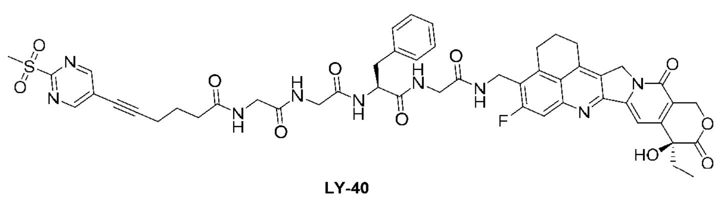

LY-40

,

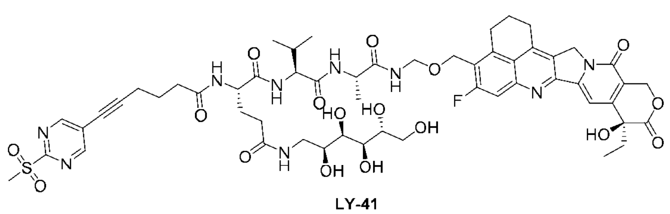

LY-41

,

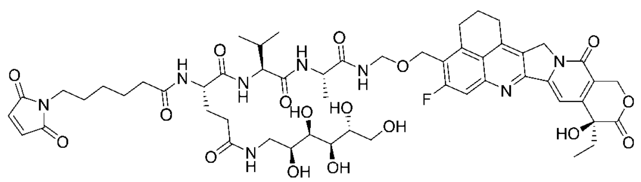

LY-41B

,

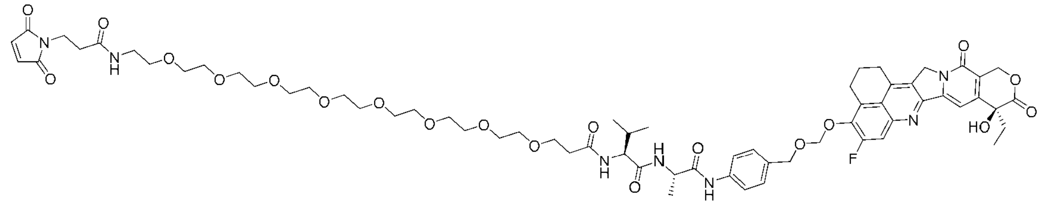

LY-42A

,

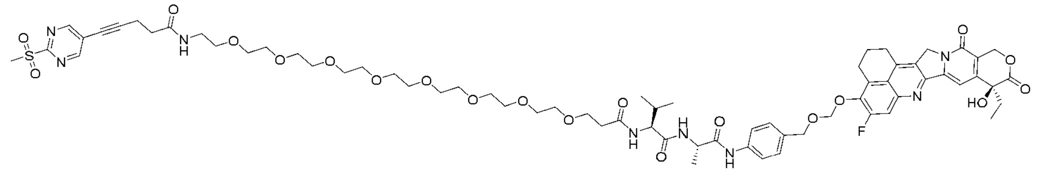

LY-42B

,

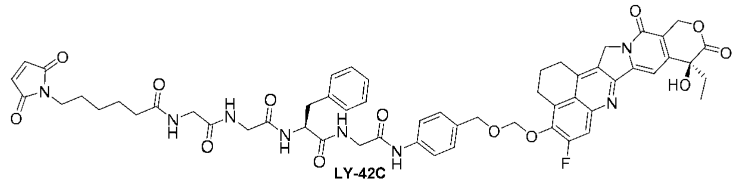

LY-42C

,

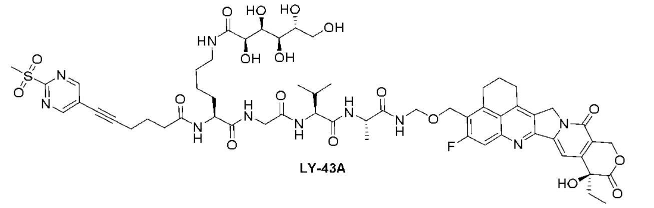

LY-43A

,

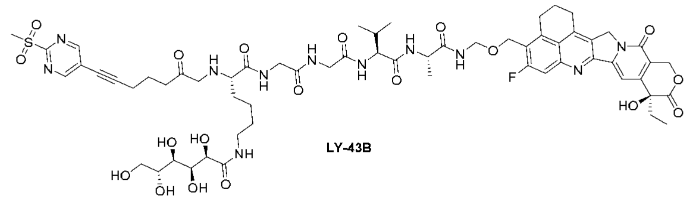

LY-43B

,

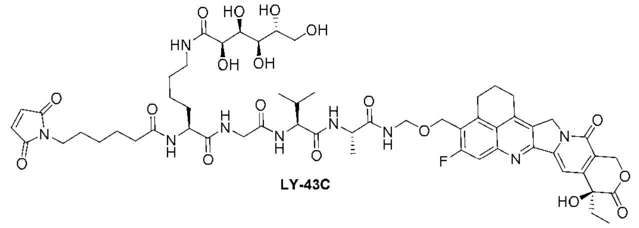

LY-43C

,

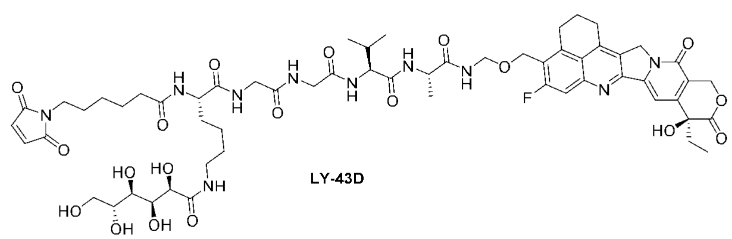

LY-43D

,

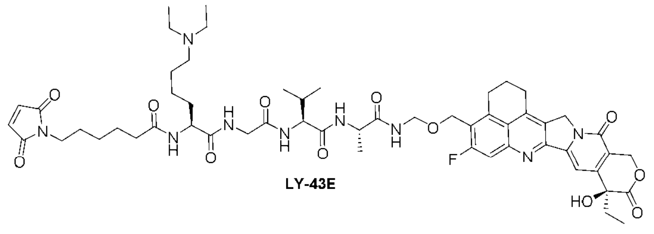

LY-43E

,

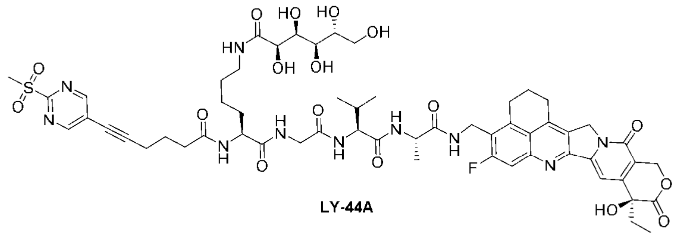

LY-44A

,

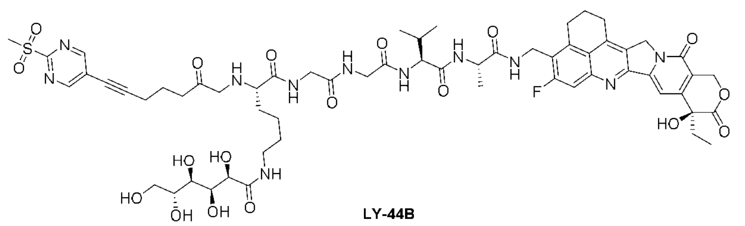

LY-44B

,

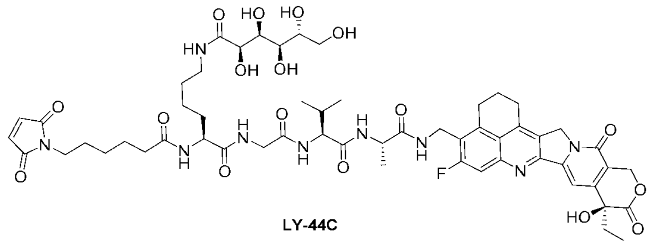

LY-44C

,

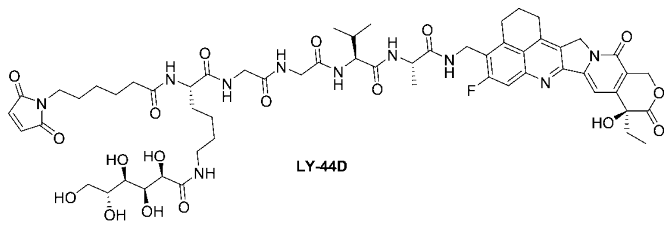

LY-44D

,

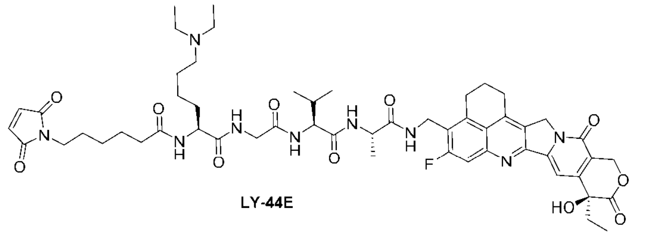

LY-44E

,

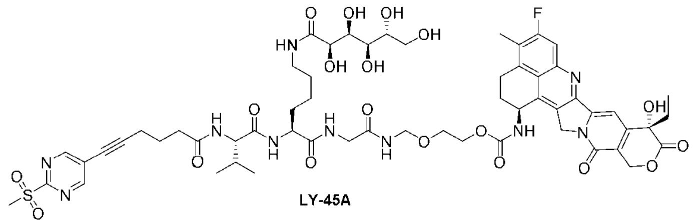

LY-45A

,

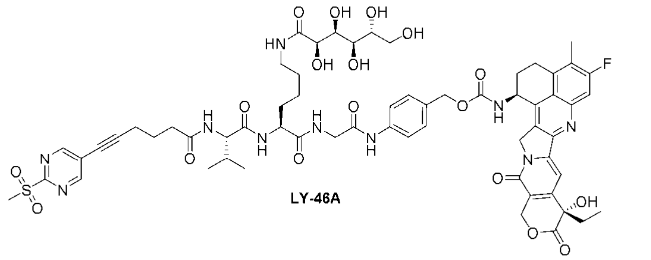

LY-46A

,

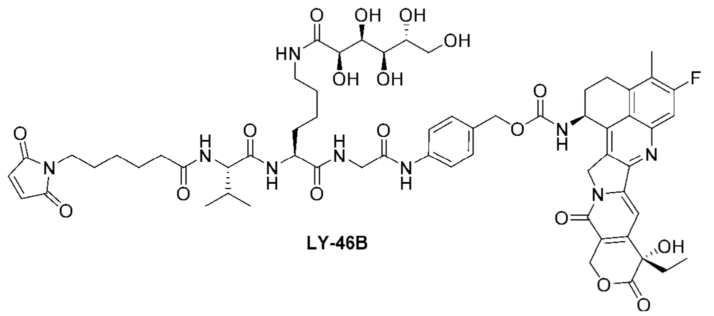

LY-46B

,

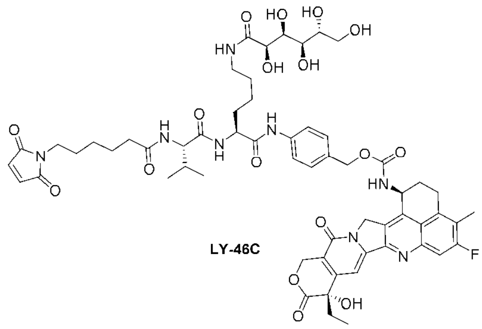

LY-46C

,

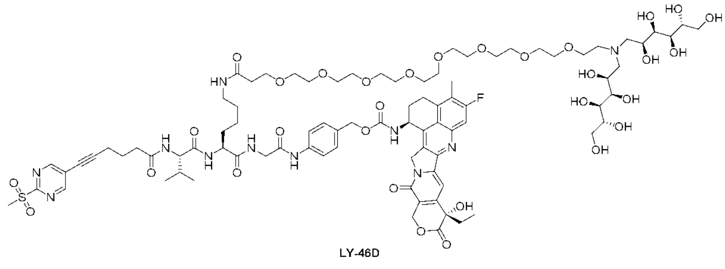

LY-46D

,

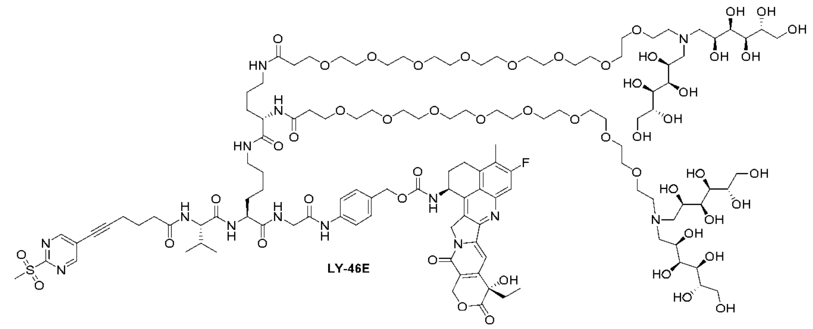

LY-46E

,

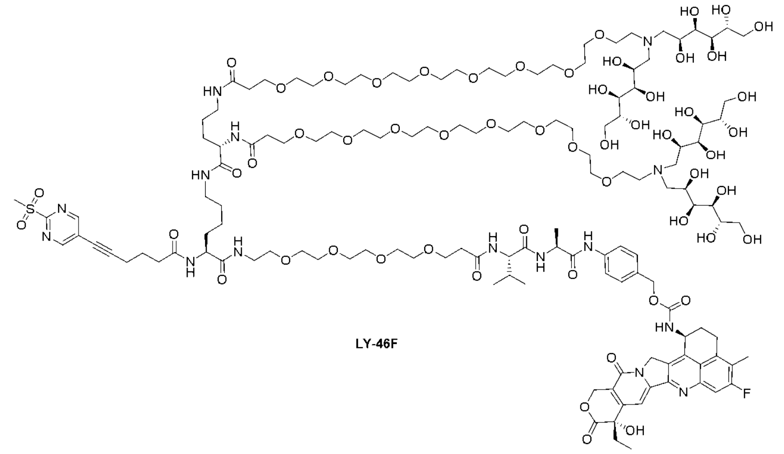

LY-46F

,

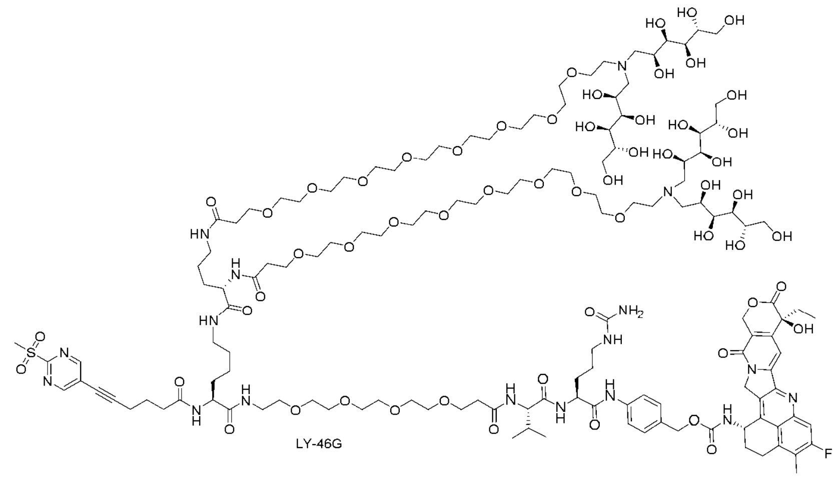

LY-46G

,

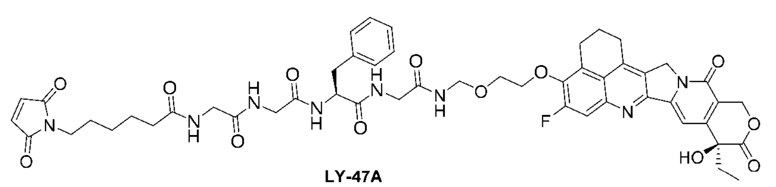

LY-47A

,

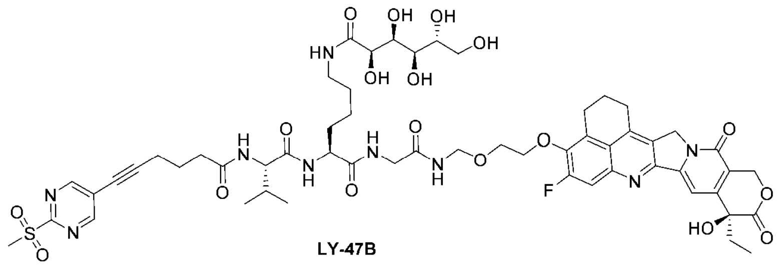

LY-47B

,

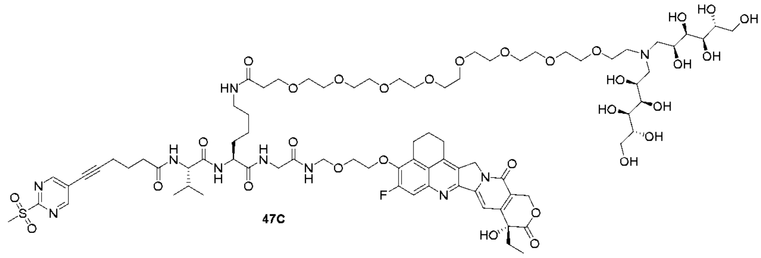

47C

,

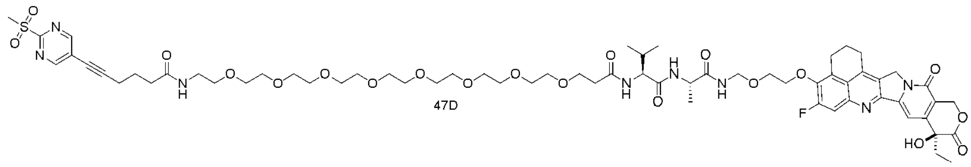

47D

,

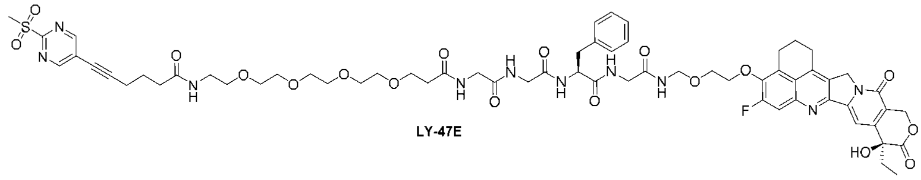

LY-47E

,

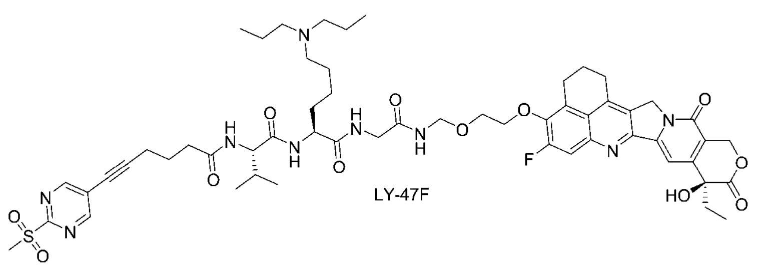

LY-47F

,

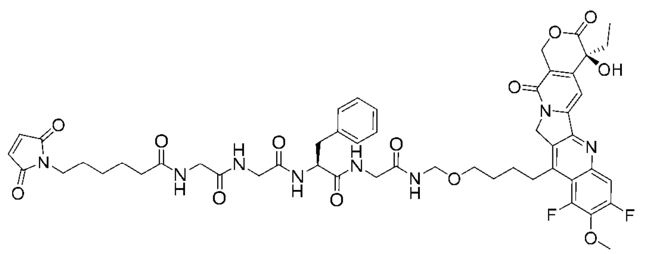

LY-48A

,

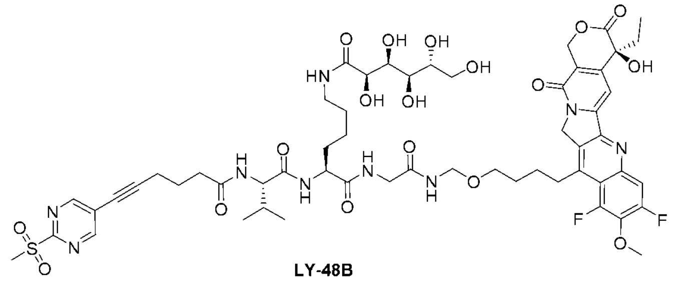

LY-48B

,

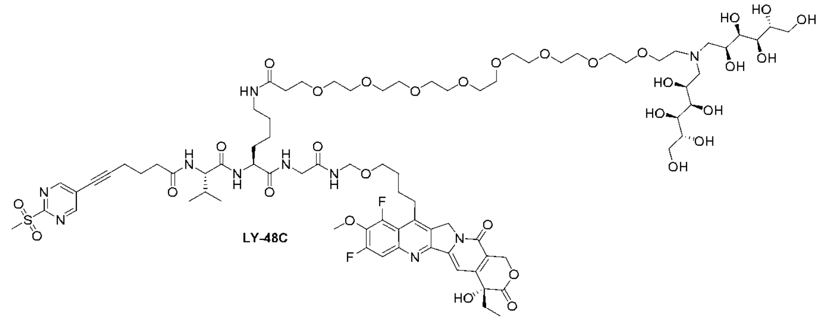

LY-48C

,

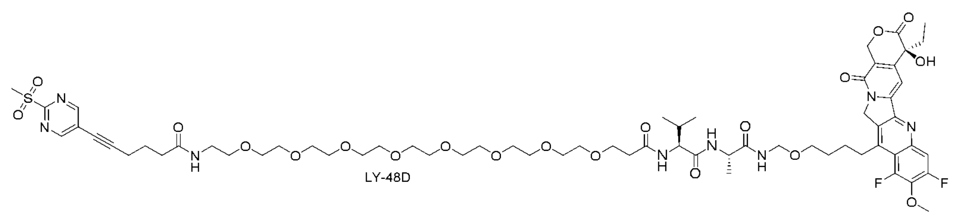

LY-48D

,

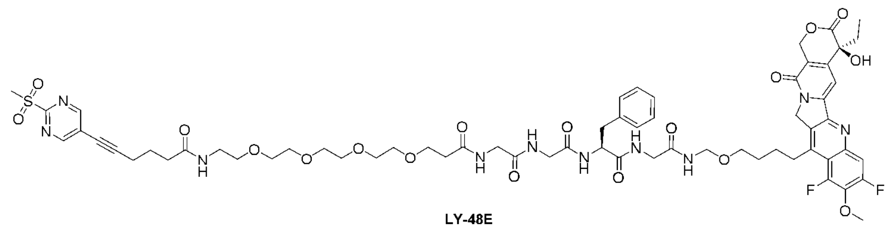

LY-48E

,

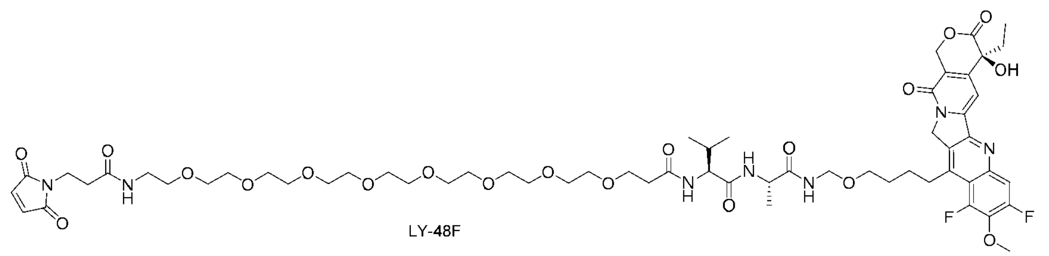

LY-48F

,

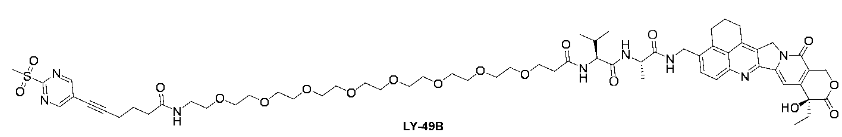

LY-49B

,

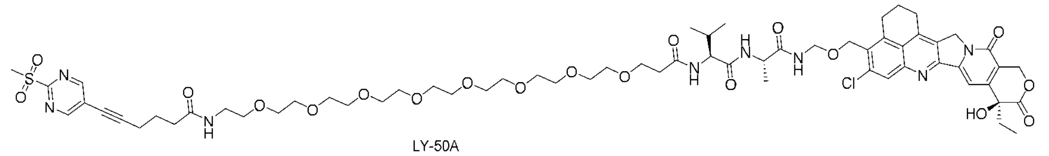

LY-50A

,

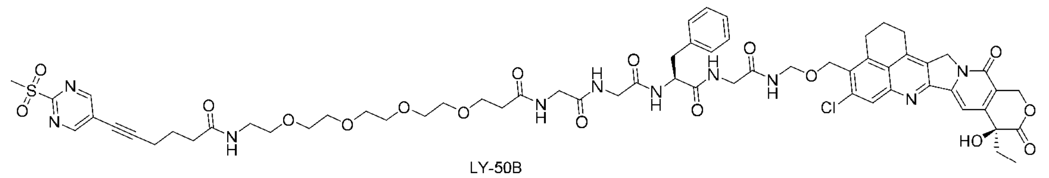

LY-50B

,

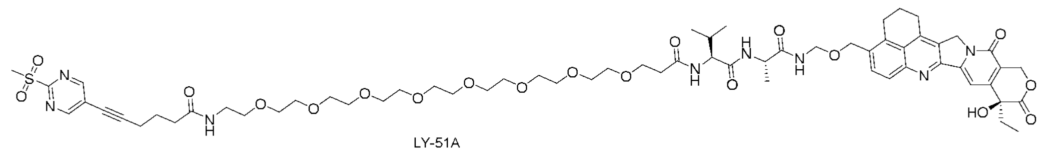

LY-51A

,

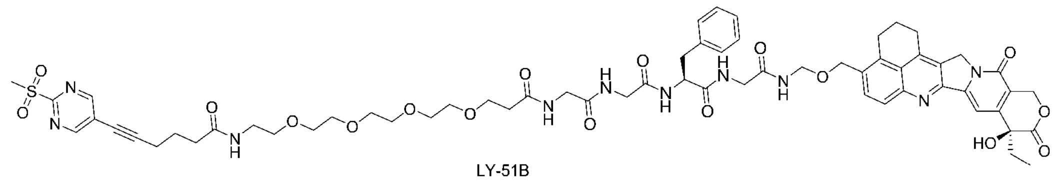

LY-51B

,

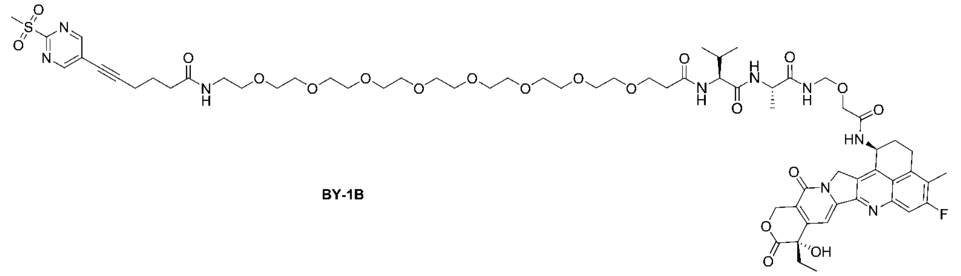

BY-1B

,

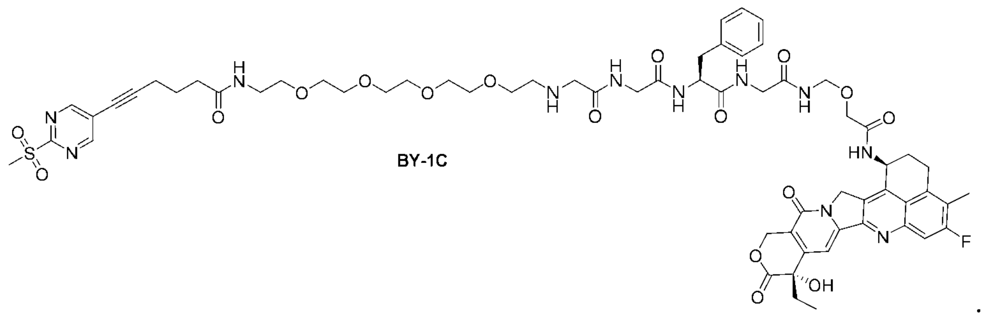

BY-1C

.

**22.** A compound represented by general formula (I) or a stereoisomer, tautomer, mesomer, racemate, enantiomer, or diastereomer thereof, or a mixture thereof, or a pharmaceutically acceptable salt thereof;

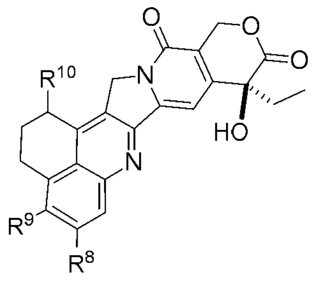

(I)

wherein,

$R^8$ is selected from hydrogen, halogen, hydroxyl, amino, cyano, alkyl, alkoxy, alkenyl, and alkynyl, wherein the alkyl, alkoxy, alkenyl, and alkynyl are optionally further substituted by one or more groups selected from halogen; wherein halogen is preferred;

$R^9$ is selected from hydrogen, halogen, hydroxyl, carboxyl, cyano, alkyl, alkoxy, alkenyl, alkynyl, cycloalkyl, $-NR^dR^e$, $-(CH_2)_m-OH$, $-(CH_2)_m-NR^dR^e$, $-OC(=O)NR^f-(CH_2)_m-OH$, $-OC(=O)NR^f-(CH_2)_m-NR^dR^e$, $-(CH_2)_m-C(=O)OH$, $-(CH_2)_m-C(=O)-NR^dR^e$, $-(CH_2)_m-C(=O)NR^f-(CH_2)_n-OH$, $-(CH_2)_m-C(=O)NR^f-(CH_2)_n-NR^dR^e$, $-NR^f-(CH_2)_m-OH$, $-NR^f-(CH_2)_m-NR^dR^e$, $-O-(CH_2)_m-OH$, $-O-(CH_2)_m-NR^dR^e$, $-NR^fC(=O)O-(CH_2)_m-OH$, $-NR^bC(=O)O-(CH_2)_m-NR^dR^e$, $-(CH_2)_m-NR^fC(=O)O-(CH_2)_n-OH$, $-(CH_2)_m-NR^fC(=O)O-(CH_2)_n-NR^dR^e$, $-(CH_2)_m-OC(=O)NR^f-(CH_2)_n-NR^dR^e$, $-(CH_2)_m-OC(=O)NR^f-(CH_2)_n-OH$, $-(CH_2)_m-NR^fC(=O)-(CH_2)_n-NR^dR^e$, $-(CH_2)_m-NR^fC(=O)-(CH_2)_n-OH$, and $-(CH2)_m-NR^fC(=O)-G-(CH_2)_n-OH$, wherein the alkyl, alkoxy, alkenyl, alkynyl, and cycloalkyl are optionally further substituted by one or more groups selected from deuterium, halogen, amino, alkyl, and hydroxyl; wherein hydroxyl, $C_1$-$C_6$ alkyl, $C_1$-$C_6$ alkoxy, $C_3$-$C_6$ cycloalkyl, $-NR^dR^e$, $-(CH_2)_m-OH$, $-(CH_2)_m-NR^dR^e$, $-(CH_2)_m-NR^fC(=O)O-(CH_2)_n-OH$, $-(CH_2)_m-OC(=O)NR^f-(CH_2)_n-OH$, $-(CH_2)_m-NR^fC(=O)-(CH_2)_n-OH$, $-O-(CH_2)_m-NR^dR^e$, $-O-(CH_2)_m-OH$, and $-(CH_2)_m-NR^fC(=O)-G-(CH_2)_n-OH$ are preferred; and the $-(CH_2)_m-$ is optionally substituted by one or more deuterium or halogen groups;

G is selected from cycloalkylene, heterocyclylene, heteroarylene, and arylene;

$R^{10}$ is selected from hydrogen, halogen, hydroxyl, cyano, alkyl, alkoxy, alkenyl, alkynyl, $CH_2=$, $-NR^dR^e$, $-(CH_2)_m-OH$, $-(CH_2)_m-NR^dR^e$, $-OC(=O)NR^f-(CH_2)_m-OH$, $-OC(=O)NR^f-(CH_2)_m-NR^dR^e$, $-(CH_2)_m-C(=O)OH$, $-(CH_2)_m-C(=O)-NR^dR^e$, $-(CH_2)_m-C(=O)NR^f-(CH_2)_n-OH$, $-(CH_2)_m-C(=O)NR^f-(CH_2)_n-NR^dR^e$, $-NR^f-(CH_2)_m-OH$, $-NR^f-(CH_2)_m-NR^dR^e$, $-NR^f-C(=O)R^d$, $-NR^fC(=O)-(CH_2)_m-R^d$, $-NR^fC(=O)O-(CH_2)_m-R^d$, $-O-(CH_2)_m-OH$, $-O-(CH_2)_m-NR^dR^e$, $-NR^fC(=O)O-(CH_2)_m-OH$, $-NR^fC(=O)NR^d-(CH_2)_m-OH$, $-NR^fC(=O)NR^d-(CH_2)_m-O-(CH_2)_n-OH$, $-NR^fC(=O)O-(CH_2)_m-O-(CH_2)_n-OH$, $-NR^fC(=O)NR^d-(CH_2)_m-O-(CH_2)_n-NR^dR^e$, $-NR^fC(=O)O-(CH_2)_m-O-(CH_2)_n-NR^dR^e$, $-NR^fC(=O)NR^d-(CH_2)_m-NR^dR^e$, $-NR^fC(=O)O-(CH_2)_m-NR^dR^e$, $-(CH_2)_m-NR^fC(=O)O-(CH_2)_n-OH$, $-(CH_2)_m-NR^fC(=O)O-(CH_2)_n-NR^dR^e$, $-(CH_2)_m-OC(=O)NR^f-(CH_2)_n-NR^dR^e$, $-(CH_2)_m-OC(=O)NR^f-(CH_2)_n-OH$, $-(CH_2)_m-NR^fC(=O)-(CH_2)_n-NR^dR^e$, and $-(CH_2)_m-NR^fC(=O)-(CH_2)_n-OH$, wherein the alkyl, alkoxy, alkenyl, and alkynyl are optionally further substituted by one or more groups selected from halogen; wherein hydrogen, hydroxyl, amino, $-(CH_2)_m-OH$, $-OC(=O)NR^f-(CH_2)_m-OH$, $-(CH_2)_m-C(=O)OH$, $-(CH_2)_m-C(=O)NR^f-(CH_2)_n-OH$, $-NR^f-(CH_2)_m-OH$, $-NR^f-C(=O)R^d$, $-NR^f-C(=O)-(CH_2)_m-R^d$, $-NR^fC(=O)O-(CH_2)_m-R^d$, $-NR^fC(=O)O-(CH_2)_m-OH$, $-NR^fC(=O)NR^d-(CH_2)_m-OH$, $-NR^fC(=O)NR^d-(CH_2)_m-O-(CH_2)_n-OH$, $-NR^fC(=O)O-(CH_2)_m-O-(CH_2)_n-OH$, $-NR^fC(=O)NR^d-(CH_2)_m-O-(CH_2)_n-NR^dR^e$, $-NR^fC(=O)O-(CH_2)_m-O-(CH_2)_n-NR^dR^e$, $-NR^fC(=O)NR^d-(CH_2)_m-NR^dR^e$, and $-NR^fC(=O)O-(CH_2)_m-NR^dR^e$ are preferred;

$R^d$ and $R^e$ are each independently selected from hydrogen and $C_1$-$C_6$ alkyl;

$R^f$ is selected from hydrogen, $C_1$-$C_6$ alkyl, $-C(O)R^c$, $-S(O)R^c$, and $-S(O)_2R^c$, wherein the $C_1$-$C_6$ alkyl is optionally further substituted by $C_3$-$C_6$ cycloalkyl, and $R^c$ is selected from hydrogen, hydroxyl, and $C_1$-$C_6$ alkyl;

m is an integer from 1 to 6; and

n is an integer from 1 to 6.

**23.** The compound represented by the general formula (I) or the stereoisomer, tautomer, mesomer, racemate, enantiomer, or diastereomer thereof, or the mixture thereof, or the pharmaceutically acceptable salt thereof according to claim 22,

wherein, $R^9$ is selected from hydrogen, $C_1$-$C_6$ alkyl, $C_1$-$C_6$ alkoxy, hydroxyl, carboxyl, $-NR^dR^e$, $-(CH_2)_m-OH$, $-(CH_2)_m-NR^dR^e$, $-(CH_2)_m-NR^fC(=O)O-(CH_2)_n-OH$, $-(CH_2)_m-OC(=O)NR^f-(CH_2)_n-OH$, $-(CH_2)_m-NR^fC(=O)-(CH_2)_n-OH$, $-O-(CH_2)_m-NR^dR^e$, and $-O-(CH_2)_m-OH$;

$R^d$ and $R^e$ are each independently selected from hydrogen and $C_1$-$C_6$ alkyl;
$R^f$ is selected from hydrogen and $C_1$-$C_6$ alkyl;
m is an integer from 1 to 6; and
n is an integer from 1 to 4, preferably 1 or 2.

**24.** The compound represented by the general formula (I) or the stereoisomer, tautomer, mesomer, racemate, enantiomer, or diastereomer thereof, or the mixture thereof, or the pharmaceutically acceptable salt thereof according to claim 22 or 23,

wherein, $R^{10}$ is selected from hydrogen, hydroxyl, amino, $CH_2$=, $-(CH_2)_m-OH$, $-OC(=O)NR^f-(CH_2)_m-OH$, $-(CH_2)_m-C(=O)OH$, $-(CH_2)_m-C(=O)NR^f-(CH_2)_n-OH$, $-NR^f-(CH_2)_m-OH$, $-NR^f-C(=O)R^d$, $-NR^f-C(=O)-(CH_2)_m-R^d$, $-NR^fC(=O)O-(CH_2)_m-R^d$, $-NR^f-C(=O)-(CH_2)_m-OH$, and $-NR^fC(=O)O-(CH_2)_m-OH$;
$R^d$ and $R^e$ are each independently selected from hydrogen and $C_1$-$C_6$ alkyl;
$R^f$ is selected from hydrogen, $C_1$-$C_6$ alkyl, $-C(O)R^c$, $-S(O)R^c$, and $-S(O)_2R^c$, wherein the $C_1$-$C_6$ alkyl is optionally further substituted by $C_3$-$C_6$ cycloalkyl, and $R^c$ is selected from hydrogen, hydroxyl, and $C_1$-$C_6$ alkyl;
m is an integer from 1 to 6; preferably, an integer from 1 to 4 or an integer from 4 to 6; and
n is an integer from 1 to 4, preferably 1 or 2.

**25.** The compound represented by the general formula (I) or the stereoisomer, tautomer, mesomer, racemate, enantiomer, or diastereomer thereof, or the mixture thereof, or the pharmaceutically acceptable salt thereof according to any one of claims 22 to 24,
wherein, $R^8$ is selected from halogen.

**26.** The compound represented by the general formula (I) or the stereoisomer, tautomer, mesomer, racemate, enantiomer, or diastereomer thereof, or the mixture thereof, or the pharmaceutically acceptable salt thereof according to any one of claims 22 to 25, which is selected from:

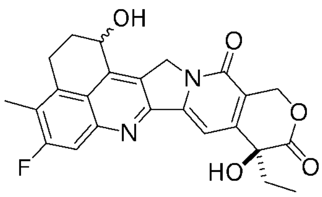 , 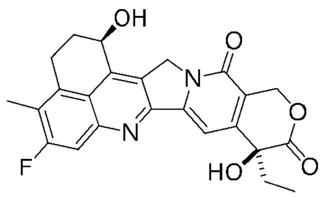 , 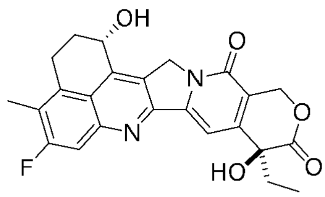 ,

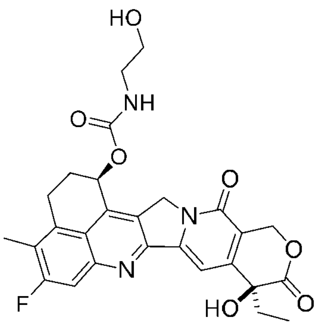 , 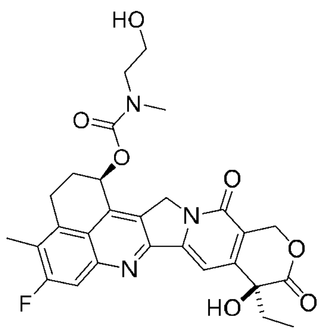 , 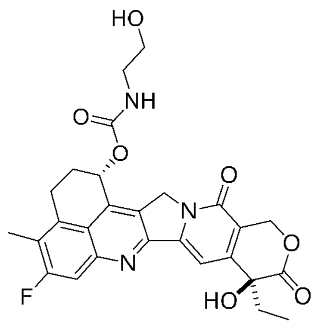 ,

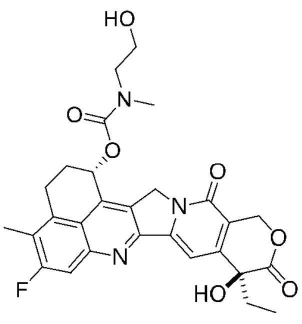 , 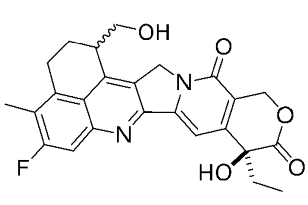 , 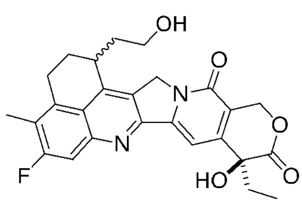 ,

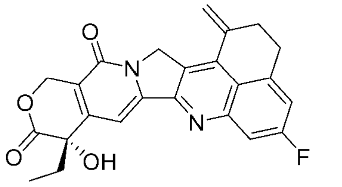 , 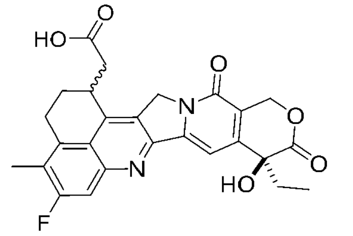 , 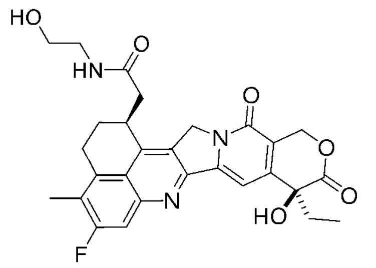 ,

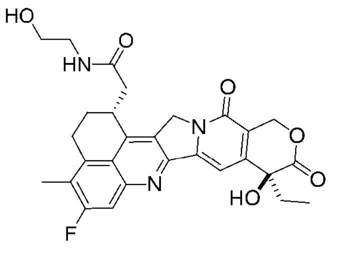 , 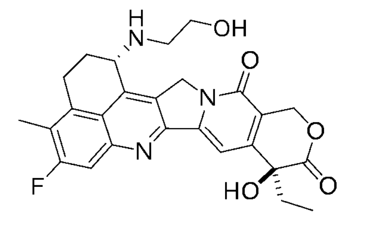 , 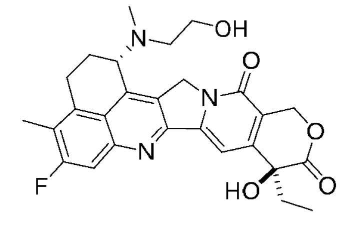 ,

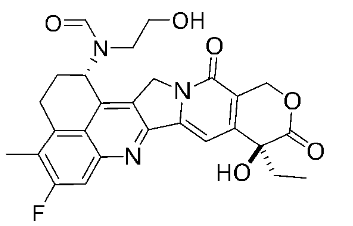 , 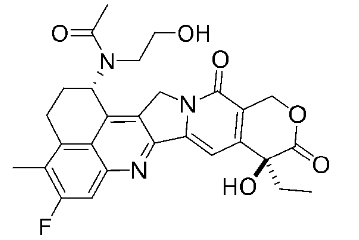 , 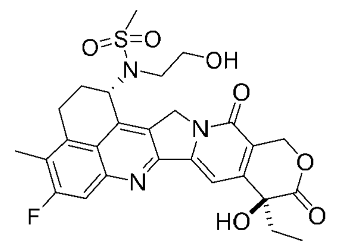 ,

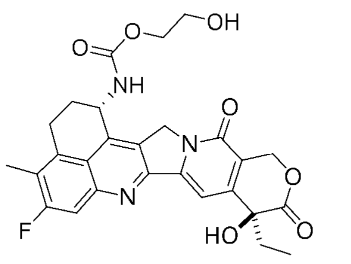 , 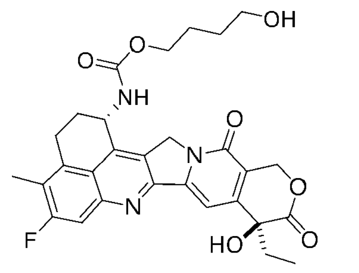 , 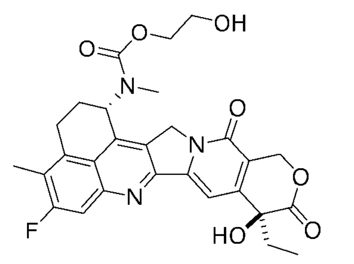 ,

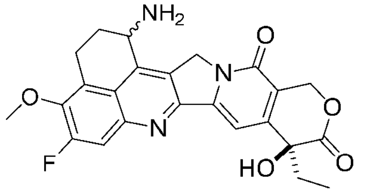 , 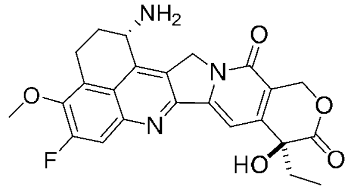 , 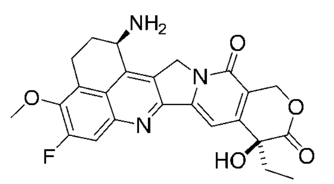 ,

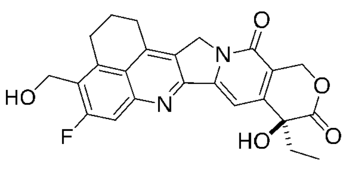 , 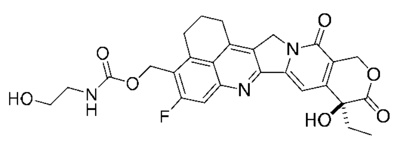 , 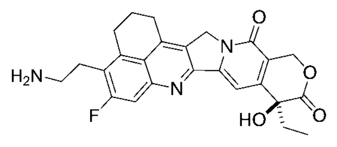 ,

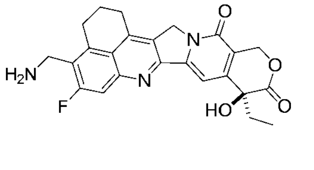 , 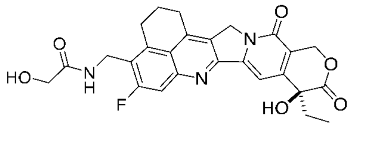 , 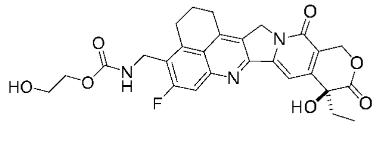 ,

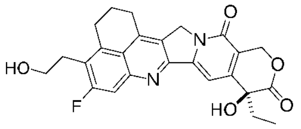 , 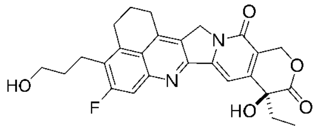 , 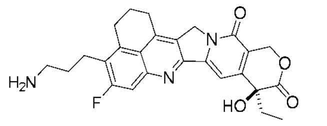 ,

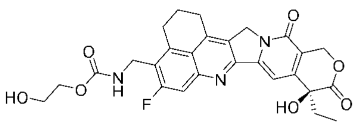 , 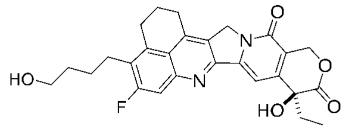 , 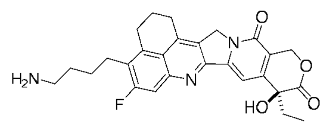 ,

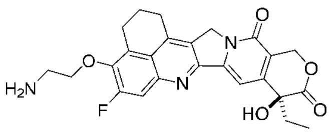 , 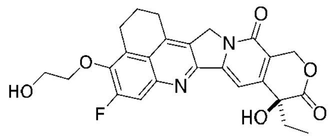 , 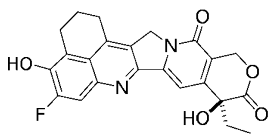 ,

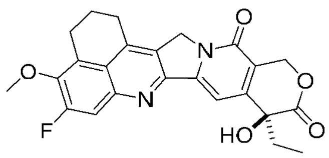 , 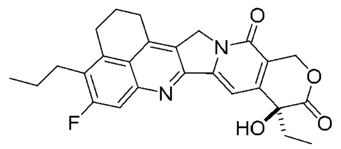 , 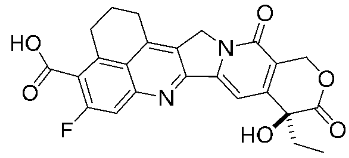 ,

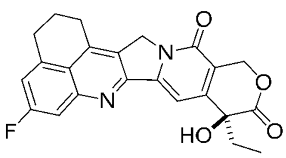 , 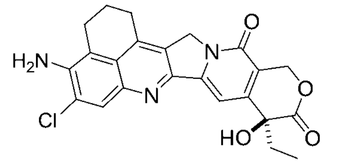 , 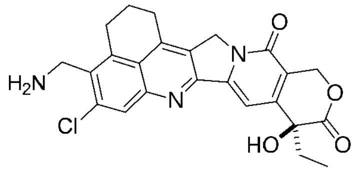 ,

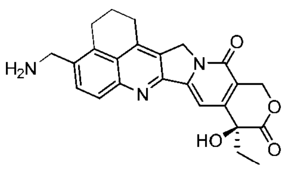 , 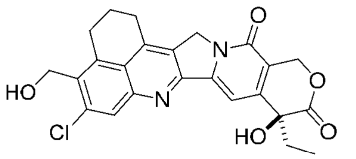 , 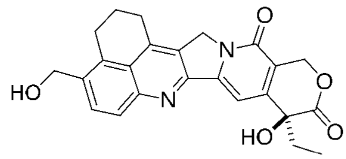 ,

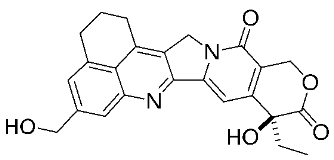 , 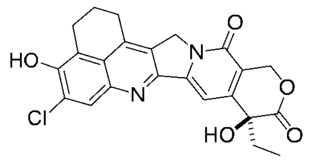 , 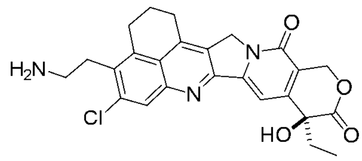 ,

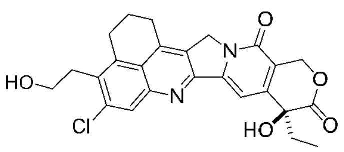 , 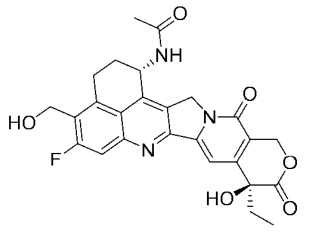 , 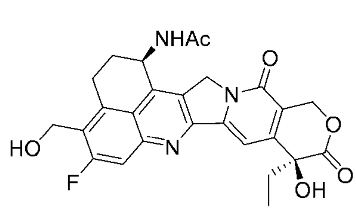 ,

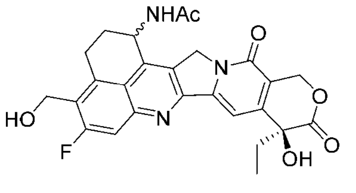
,
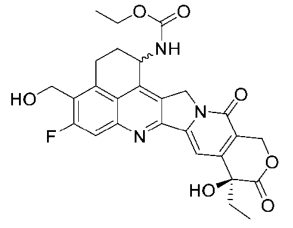
,
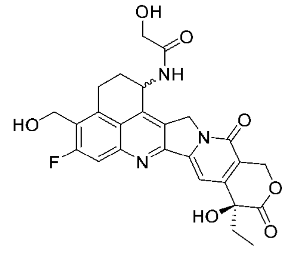
,

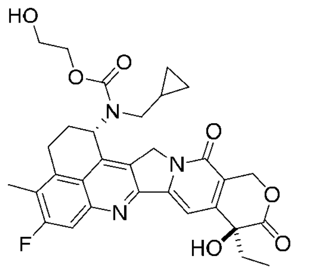
,
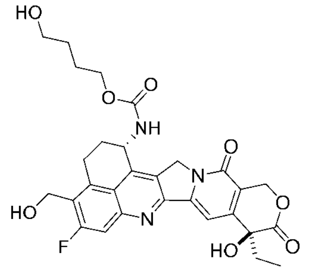
,
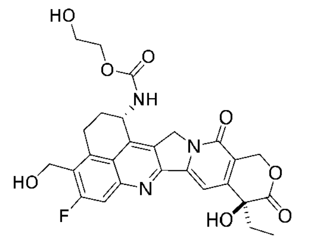
,

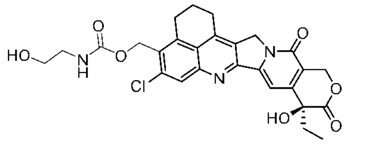
,
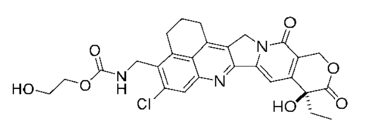
,
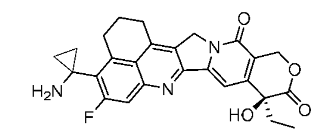
,

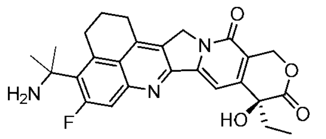
,
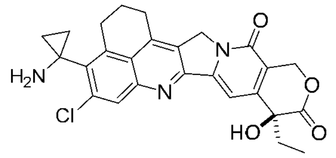
,
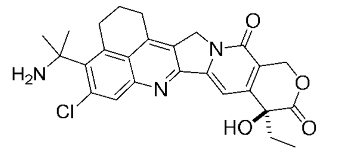
,

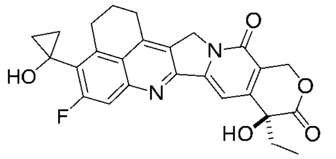
,
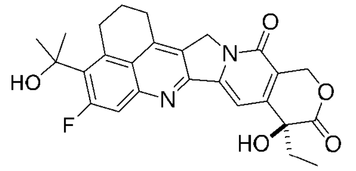
,
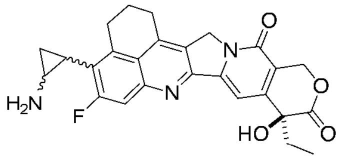
,

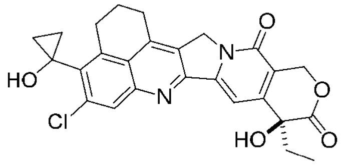
,
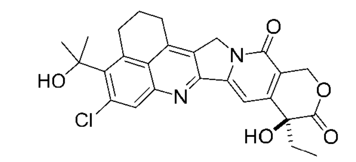
,
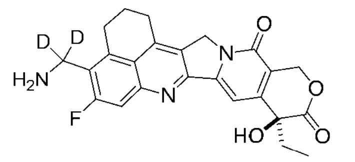
,

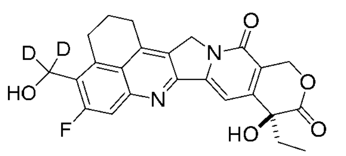
,
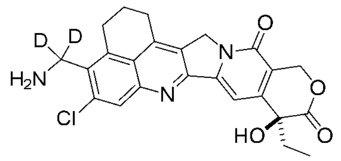
,
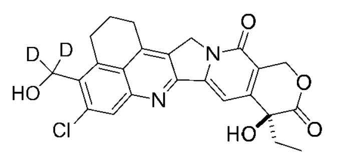
,

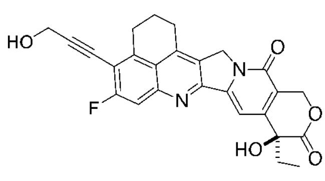 , 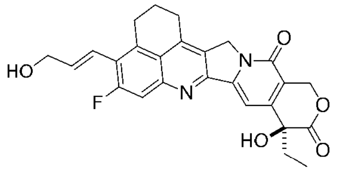 , 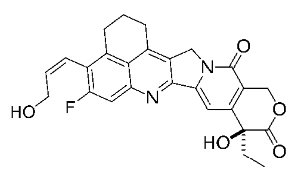 ,

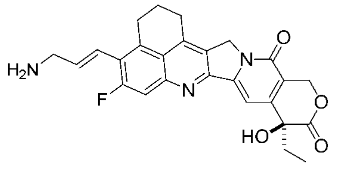 , 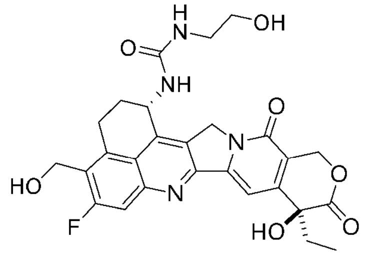 , 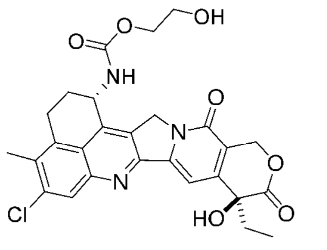 ,

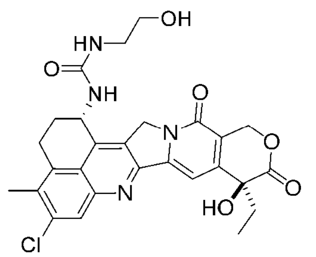 , 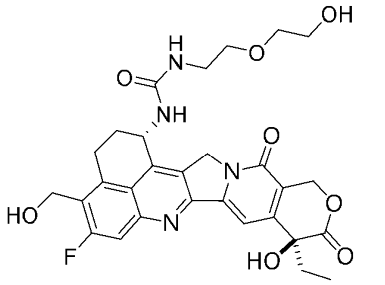 , 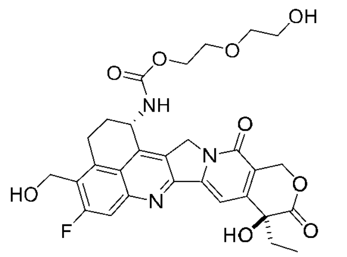 ,

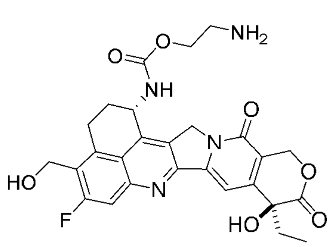 , 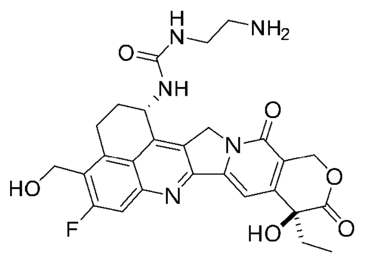 , 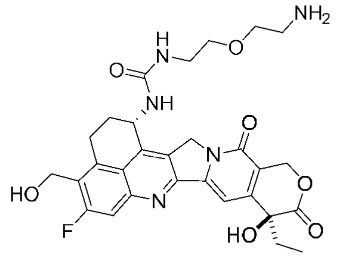 ,

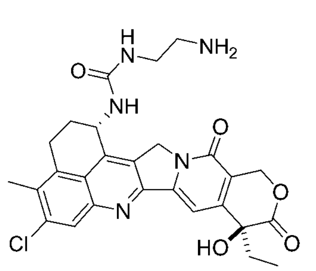 , 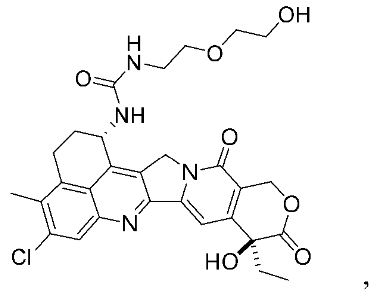 , 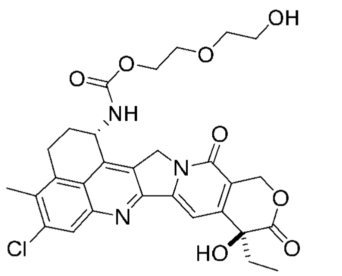 ,

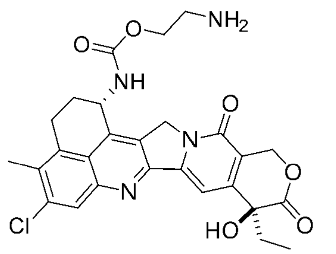 , 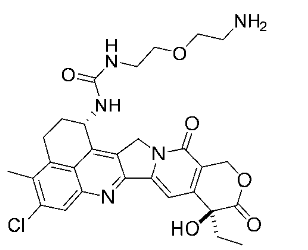 , 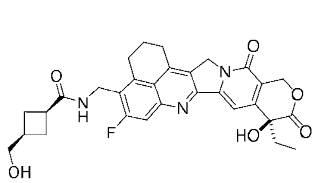 ,

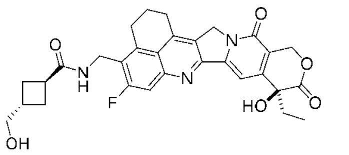, 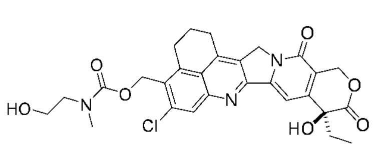, 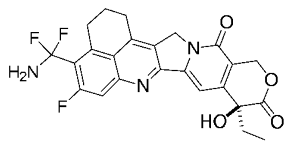,

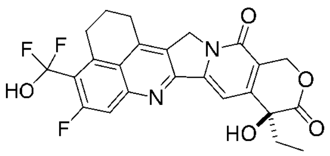, 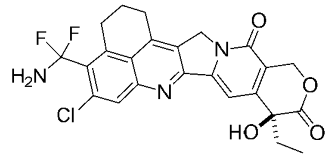, 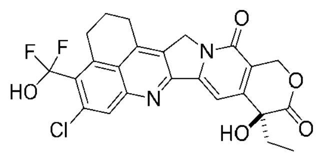,

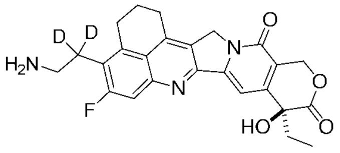, 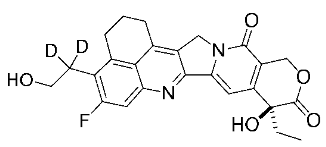, 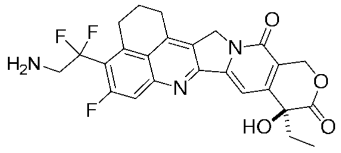,

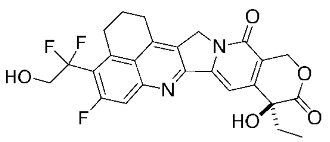, 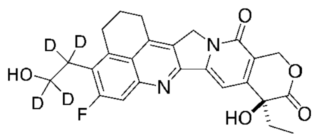, 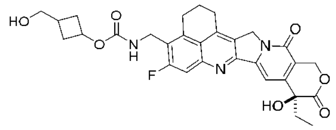,

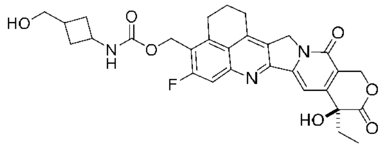, 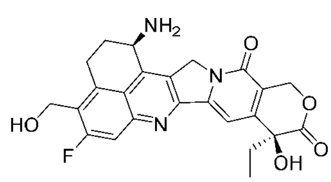, and 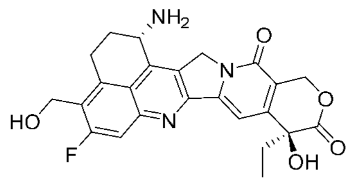.

**27.** A compound represented by general formula (II) or a stereoisomer, tautomer, mesomer, racemate, enantiomer, or diastereomer thereof, or a mixture thereof, or a pharmaceutically acceptable salt thereof;

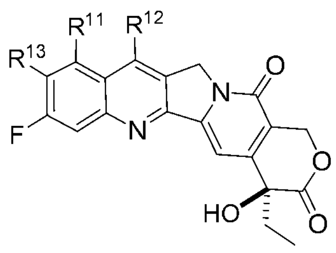

(II)

wherein,

$R^{11}$ is selected from hydrogen, halogen, hydroxyl, amino, cyano, alkyl, alkoxy, alkenyl, and alkynyl, wherein the alkyl, alkoxy, alkenyl, and alkynyl are optionally further substituted by one or more groups selected from halogen; wherein halogen and cyano are preferred;

$R^{12}$ is selected from hydrogen, halogen, hydroxyl, amino, cyano, alkyl, alkoxy, alkenyl, alkynyl, $-NR^dR^e$, $-(CH_2)_m-OH$, $-(CH_2)_m-NR^dR^e$, $-OC(=O)NR^f-(CH_2)_m-OH$, $-OC(=O)NR^f-(CH_2)_m-NR^dR^e$, $-(CH_2)_m-C(=O)OH$, $-(CH_2)_m-C(=O)-NR^dR^e$, $-(CH_2)_m-C(=O)NR^f-(CH_2)_n-OH$, $-(CH_2)_m-C(=O)NR^f-(CH_2)_n-NR^dR^e$, $-NR^f-(CH_2)_m-OH$, $-NR^f-(CH_2)_m-NR^dR^e$, $-O-(CH_2)_m-OH$, $-O-(CH_2)_m-NR^dR^e$, $-NR^fC(=O)O-(CH_2)_m-OH$, $-NR^bC(=O)O-(CH_2)_m-NR^a-*$, $-(CH_2)_m-NR^bC(=O)O-(CH_2)_n-O-*$, $-(CH_2)_m-NR^fC(=O)O-(CH_2)_n-NR^dR^e$, $-(CH_2)_m-OC(=O)NR^f-(CH_2)_n-NR^dR^e$, $-(CH_2)_m-OC(=O)NR^f-(CH_2)_n-OH$, $-(CH_2)_m-NR^fC(=O)-(CH_2)_n-NR^dR^e$, and

$-(CH_2)_m-NR^fC(=O)-(CH_2)_n-OH$, wherein the alkyl, alkoxy, alkenyl, and alkynyl are optionally further substituted by one or more groups selected from halogen; wherein hydroxyl, amino, $C_1-C_6$ alkyl, $-(CH_2)_m-OH$, and $-(CH_2)_m-NR^dR^e$ are preferred;

$R^{13}$ is selected from hydrogen, halogen, hydroxyl, amino, cyano, alkyl, alkoxy, alkenyl, alkynyl, $-NR^dR^e$, $-(CH_2)_m-OH$, $-(CH_2)_m-NR^dR^e$, $-OC(=O)NR^f-(CH_2)_m-OH$, $-OC(=O)NR^f-(CH_2)_m-NR^dR^e$, $-(CH_2)_m-C(=O)OH$, $-(CH_2)_m-C(=O)-NR^dR^e$, $-(CH_2)_m-C(=O)NR^f-(CH_2)_n-OH$, $-(CH_2)_m-C(=O)NR^f-(CH_2)_n-NR^dR^e$, $-NR^f-(CH_2)_m-OH$, $-NR^f-(CH_2)_m-NR^dR^e$, $-O-(CH_2)_m-OH$, $-O-(CH_2)_m-NR^dR^e$, $-NR^fC(=O)O-(CH_2)_m-OH$, $-NR^bC(=O)O-(CH_2)_m-NR^a-*$, $-(CH_2)_m-NR^bC(=O)O-(CH_2)_n-O-*$, $-(CH_2)_m-NR^fC(=O)O-(CH_2)_n-NR^dR^e$, $-(CH_2)_m-OC(=O)NR^f-(CH_2)_n-NR^dR^e$, $-(CH_2)_m-OC(=O)NR^f-(CH_2)_n-OH$, $-(CH_2)_m-NR^fC(=O)-(CH_2)_n-NR^dR^e$, and $-(CH_2)_m-NR^fC(=O)-(CH_2)_n-OH$, wherein the alkyl, alkoxy, alkenyl, and alkynyl are optionally further substituted by one or more groups selected from halogen;

wherein hydroxyl and amino are preferred;

$R^d$ and $R^e$ are each independently selected from hydrogen and $C_1-C_6$ alkyl;

$R^f$ is selected from hydrogen, $C_1-C_6$ alkyl, $-C(O)R^c$, $-S(O)R^c$, and $-S(O)_2R^c$, wherein $R^c$ is selected from hydrogen, hydroxyl, and $C_1-C_6$ alkyl;

m is an integer from 1 to 6; and

n is an integer from 1 to 6.

**28.** The compound represented by the general formula (II) or the stereoisomer, tautomer, mesomer, racemate, enantiomer, or diastereomer thereof, or the mixture thereof, or the pharmaceutically acceptable salt thereof according to claim 27, wherein $R^{11}$ is selected from halogen and cyano.

**29.** The compound represented by the general formula (II) or the stereoisomer, tautomer, mesomer, racemate, enantiomer, or diastereomer thereof, or the mixture thereof, or the pharmaceutically acceptable salt thereof according to claim 27, wherein $R^{12}$ is selected from $C_1-C_6$ alkyl, $-(CH_2)_m-OH$, and $-(CH_2)_m-NR^dR^e$;

$R^d$ and $R^e$ are each independently selected from hydrogen and $C_1-C_6$ alkyl; and

m is an integer from 1 to 6.

**30.** The compound represented by the general formula (II) or the stereoisomer, tautomer, mesomer, racemate, enantiomer, or diastereomer thereof, or the mixture thereof, or the pharmaceutically acceptable salt thereof according to claim 27, wherein $R^{13}$ is selected from hydroxyl and $C_1-C_6$ alkoxy.

**31.** The compound represented by the general formula (II) or the stereoisomer, tautomer, mesomer, racemate, enantiomer, or diastereomer thereof, or the mixture thereof, or the pharmaceutically acceptable salt thereof according to any one of claims 27 to 30, which is selected from:

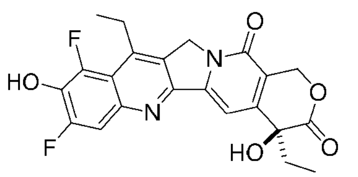,
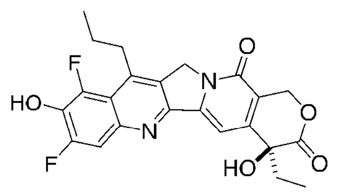,
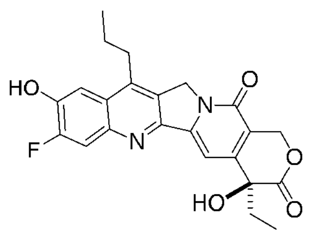,

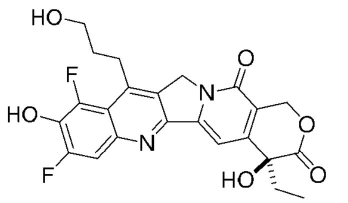,
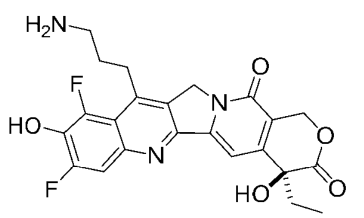,
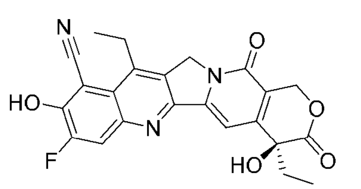,

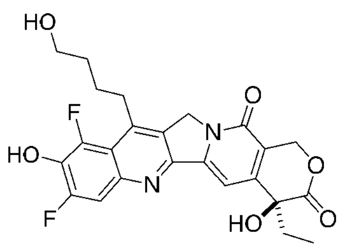,
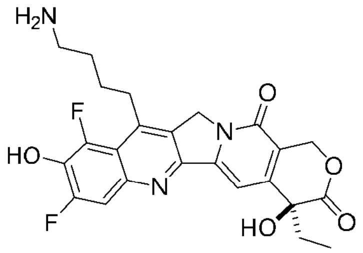,
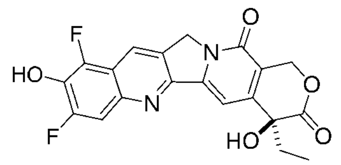,

and

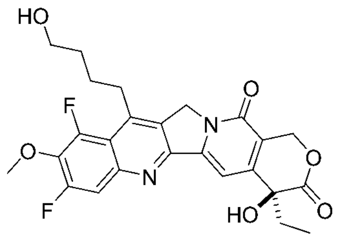.

**32.** A ligand-drug conjugate represented by general formula (B) or a pharmaceutically acceptable salt thereof,

$$Pc-\left[L'-L_2-L_1-Dr\right]_v$$

(B)

wherein:

Dr is selected from the following structures:

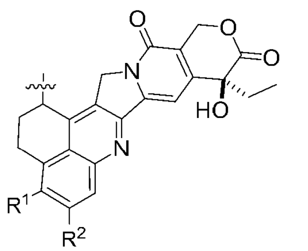,
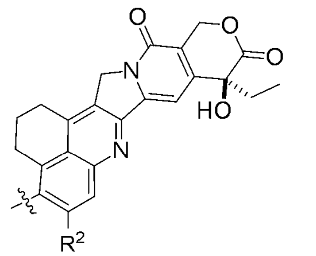,
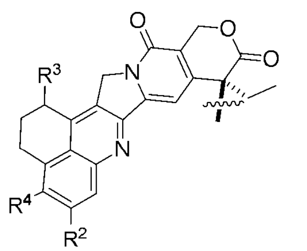,

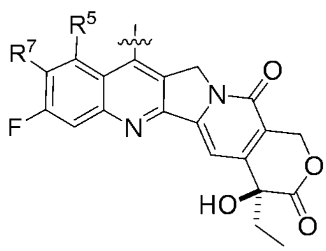, and
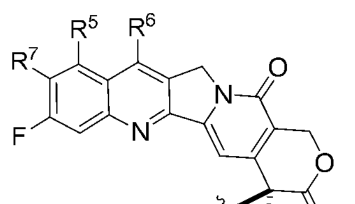;

$R^1$ is selected from hydrogen, halogen, hydroxyl, amino, cyano, alkyl, alkoxy, alkenyl, alkynyl, cycloalkyl, $-NR^dR^e$, $-(CH_2)_m-OH$, $-(CH_2)_m-NR^dR^e$, $-OC(=O)NR^f-(CH_2)_m-OH$, $-OC(=O)NR^f-(CH_2)_m-NR^dR^e$, $-(CH_2)_m-C(=O)OH$, $-(CH_2)_m-C(=O)-NR^dR^e$, $-(CH_2)_m-C(=O)NR^f-(CH_2)_n-OH$, $-(CH_2)_m-C(=O)NR^f-(CH_2)_n-NR^dR^e$, $-NR^f-(CH_2)_m-OH$, $-NR^f-(CH_2)_m-NR^dR^e$, $-O-(CH_2)_m-OH$, $-O-(CH_2)_m-NR^dR^e$, $-NR^fC(=O)O-(CH_2)_m-OH$, $-NR^bC(=O)O-(CH_2)_m-NR^dR^e$, $-(CH_2)_m-NR^fC(=O)O-(CH_2)_n-OH-$, $-(CH_2)_m-NR^fC(=O)O-(CH_2)_n-NR^dR^e$, $-(CH_2)_m-OC(=O)$

$NR^f-(CH_2)_n-NR^dR^e$, $-(CH_2)_m-OC(=O)NR^f-(CH_2)_n-OH$, $-(CH_2)_m-NR^fC(=O)-(CH_2)_n-NR^dR^e$, $-(CH_2)_m-NR^fC(=O)-(CH_2)_n-OH$, and $-(CH_2)_m-NR^fC(=O)-G-(CH_2)_n-OH$, wherein the alkyl, alkoxy, alkenyl, alkynyl, and cycloalkyl are optionally further substituted by one or more groups selected from halogen, deuterium, amino, alkyl, and hydroxyl; the $-(CH_2)_m-$ is optionally substituted by one or more deuterium or halogen groups; and G is selected from cycloalkylene, heterocyclylene, heteroarylene, and arylene;

$R^2$ is selected from hydrogen, halogen, hydroxyl, amino, cyano, alkyl, alkoxy, alkenyl, and alkynyl, wherein the alkyl, alkoxy, alkenyl, and alkynyl are optionally further substituted by one or more groups selected from halogen;

$R^3$ is selected from hydrogen, halogen, hydroxyl, amino, cyano, alkyl, alkoxy, alkenyl, alkynyl, $CH_2=$, $-NR^dR^e$, $-(CH_2)_m-OH$, $-(CH_2)_m-NR^dR^e$, $-OC(=O)NR^f-(CH_2)_m-OH$, $-OC(=O)NR^f-(CH_2)_m-NR^dR^e$, $-(CH_2)_m-C(=O)OH$, $-(CH_2)_m-C(=O)-NR^dR^e$, $-(CH_2)_m-C(=O)NR^f-(CH_2)_n-OH$, $-(CH_2)_m-C(=O)NR^f-(CH_2)_n-NR^dR^e$, $-NR^f-(CH_2)_m-OH$, $-NR^f-(CH_2)_m-NR^dR^e$, $-NR^f-C(=O)R^d$, $-NR^fC(=O)-(CH_2)_m-R^d$, $-NR^fC(=O)O-(CH_2)_m-R^d$, $-O-(CH_2)_m-OH$, $-O-(CH_2)_m-NR^dR^e$, $-NR^fC(=O)O-(CH_2)_m-OH$, $-NR^fC(=O)NR^d-(CH_2)_m-OH$, $-NR^fC(=O)NR^d-(CH_2)_m-O-(CH_2)_n-OH$, $-NR^fC(=O)O-(CH_2)_m-O-(CH_2)_n-OH$, $-NR^fC(=O)NR^d-(CH_2)_m-O-(CH_2)_n-NR^dR^e$, $-NR^fC(=O)O-(CH_2)_m-O-(CH_2)_n-NR^dR^e$, $-NR^fC(=O)NR^d-(CH_2)_m-NR^dR^e$, $-NR^fC(=O)O-(CH_2)_m-NR^dR^e$, $-(CH_2)_m-NR^fC(=O)O-(CH_2)_n-OH$, $-(CH_2)_m-NR^fC(=O)O-(CH_2)_n-NR^dR^e$, $-(CH_2)_m-OC(=O)NR^f-(CH_2)_n-NR^dR^e$, $-(CH_2)_m-OC(=O)NR^f-(CH_2)_n-OH$, $-(CH_2)_m-NR^fC(=O)-(CH_2)_n-NR^dR^e$, and $-(CH_2)_m-NR^fC(=O)-(CH_2)_n-OH$, wherein the alkyl, alkoxy, alkenyl, and alkynyl are optionally further substituted by one or more groups selected from halogen, deuterium, amino, alkyl, and hydroxyl;

$R^4$ is selected from hydrogen, halogen, hydroxyl, carboxyl, amino, cyano, alkyl, alkoxy, alkenyl, alkynyl, cycloalkyl, $-NR^dR^e$, $-(CH_2)_m-OH$, $-(CH_2)_m-NR^dR^e$, $-OC(=O)NR^f-(CH_2)_m-OH$, $-OC(=O)NR^f-(CH_2)_m-NR^dR^e$, $-(CH_2)_m-C(=O)OH$, $-(CH_2)_m-C(=O)-NR^dR^e$, $-(CH_2)_m-C(=O)NR^f-(CH_2)_n-OH$, $-(CH_2)_m-C(=O)NR^f-(CH_2)_n-NR^dR^e$, $-NR^f-(CH_2)_m-OH$, $-NR^f-(CH_2)_m-NR^dR^e$, $-O-(CH_2)_m-OH$, $-O-(CH_2)_m-NR^dR^e$, $-NR^fC(=O)O-(CH_2)_m-OH$, $-NR^bC(=O)O-(CH_2)_m-NR^dR^e$, $-(CH_2)_m-NR^fC(=O)O-(CH_2)_n-OH-$, $-(CH_2)_m-NR^fC(=O)O-(CH_2)_n-NR^dR^e$, $-(CH_2)_m-OC(=O)NR^f-(CH_2)_n-NR^dR^e$, $-(CH_2)_m-OC(=O)NR^f-(CH_2)_n-OH$, $-(CH_2)_m-NR^fC(=O)-(CH_2)_n-NR^dR^e$, $-(CH_2)_m-NR^fC(=O)-(CH_2)_n-OH$, and $-(CH_2)_m-NR^fC(=O)-G-(CH_2)_n-OH$, wherein the alkyl, alkoxy, alkenyl, alkynyl, and cycloalkyl are optionally further substituted by one or more groups selected from halogen, deuterium, amino, alkyl, and hydroxyl; the $-(CH_2)_m-$ is optionally substituted by one or more deuterium or halogen groups; and G is selected from cycloalkylene, heterocyclylene, heteroarylene, and arylene;

$R^5$ is selected from hydrogen, halogen, hydroxyl, amino, cyano, alkyl, alkoxy, alkenyl, and alkynyl, wherein the alkyl, alkoxy, alkenyl, and alkynyl are optionally further substituted by one or more groups selected from halogen;

$R^6$ is selected from hydrogen, halogen, hydroxyl, amino, cyano, alkyl, alkoxy, alkenyl, and alkynyl, wherein the alkyl, alkoxy, alkenyl, and alkynyl are optionally further substituted by one or more groups selected from halogen;

$R^7$ is selected from hydrogen, halogen, hydroxyl, amino, cyano, alkyl, alkoxy, alkenyl, and alkynyl, wherein the alkyl, alkoxy, alkenyl, and alkynyl are optionally further substituted by one or more groups selected from halogen;

$R^d$ and $R^e$ are each independently selected from hydrogen and alkyl;

$R^f$ is selected from hydrogen, alkyl, $-C(O)R^c$, $-S(O)R^c$, and $-S(O)_2R^c$, wherein the alkyl is optionally further substituted by $C_3$-$C_6$ cycloalkyl; and $R^c$ is selected from hydrogen, hydroxyl, and alkyl;

$L_1$ is selected from a bond, $-(CH_2)_m-*$, $-O-*$, $-NR^a-*$, $-(CH_2)_m-O-*$, $-(CH_2)_m-NR^a-*$, $-OC(=O)NR^b-(CH_2)_m-O-*$, $-OC(=O)NR^b-(CH_2)_m-NR^a-*$, $-(CH_2)_m-C(=O)O-*$, $-(CH_2)_m-C(=O)NR^a-*$, $-(CH_2)_m-C(=O)NR^b-(CH_2)_n-O-*$, $-(CH_2)_m-C(=O)NR^b-(CH_2)_n-NR^a-*$, $-NR^b-(CH_2)_m-O-*$, $-NR^b-(CH_2)_m-NR^a-*$, $-O-(CH_2)_m-O-*$, $-O-(CH_2)_m-NR^a-*$, $-NR^bC(=O)O-(CH_2)_m-O-*$, $-NR^bC(=O)O-(CH_2)_m-NR^a-*$, $-(CH_2)_m-NR^bC(=O)O-(CH_2)_n-O-*$, $-(CH_2)_m-NR^bC(=O)O-(CH_2)_n-NR^a-*$, $-(CH_2)_m-OC(=O)NR^b-(CH_2)_n-NR^a-*$, $-(CH_2)_m-OC(=O)NR^b-(CH_2)_n-O-*$, $-(CH_2)_m-NR^bC(=O)-(CH_2)_n-NR^a-*$, and $-(CH_2)_m-NR^bC(=O)-(CH_2)_n-O-*$, wherein * is a connection site with $L_2$;

$R^a$ and $R^d$ are each independently selected from hydrogen, alkyl, $-C(O)R^c$, $-S(O)R^c$, and $-S(O)_2R^c$, wherein the alkyl is optionally further substituted by cycloalkyl; and $R^c$ is selected from hydrogen, hydroxyl, and alkyl;

$L_2$ is selected from a bond,

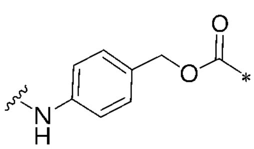
,
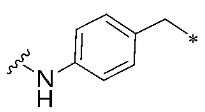
,
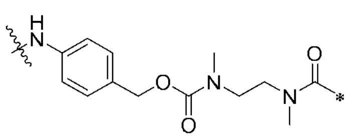
,

and

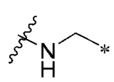
,

wherein * is a connection site with $L_1$;
L' is

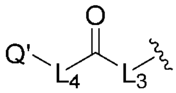 or 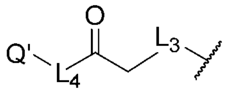 ;

Q' is selected from

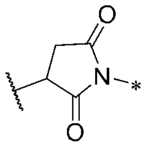 , 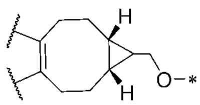 , 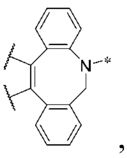 , 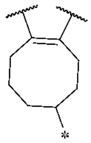 , 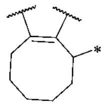 , 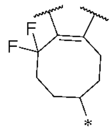 , 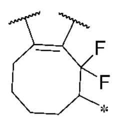 ,

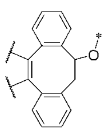 , 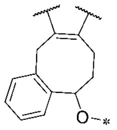 , 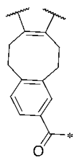 , 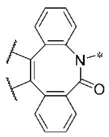 , 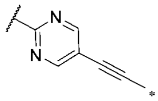 , 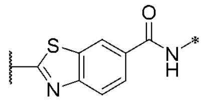 , 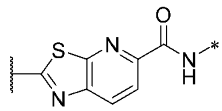 ,

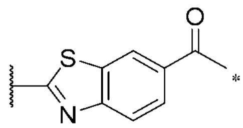 , and 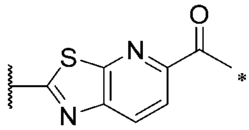 ,

wherein, * is a connection site with $L_4$, and ∿ is a connection site with Pc;
$L_3$ is an amino acid residue formed by two or more amino acids, $L_3$ optionally comprises one or more of the following structures, and $L_6$ is selected from one or more of the following structures:

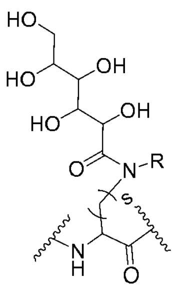 , 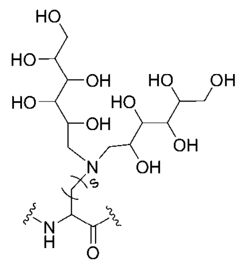 , 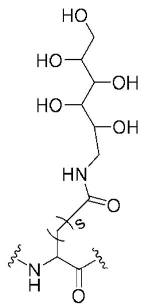 , 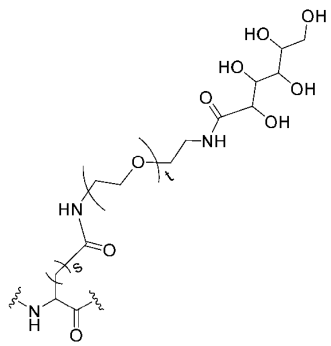 ,

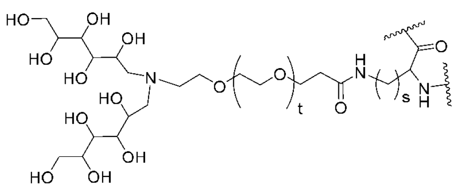 , 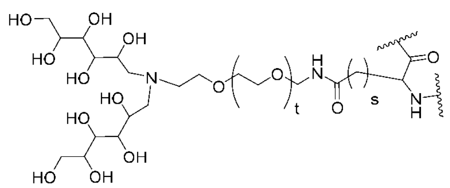 ,

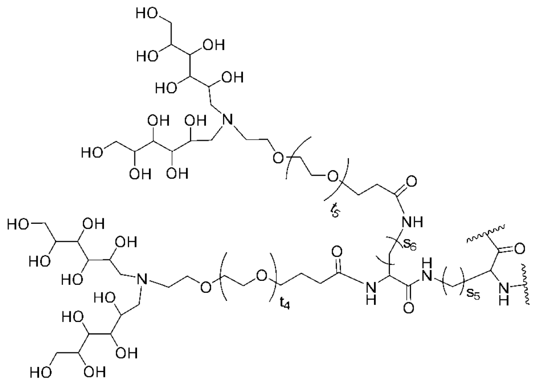, 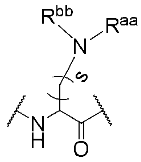, or 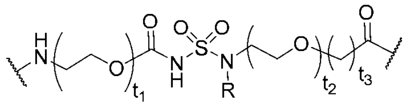;

wherein R, $R^{aa}$, and $R^{bb}$ are each independently selected from hydrogen and alkyl;

$L_4$ is

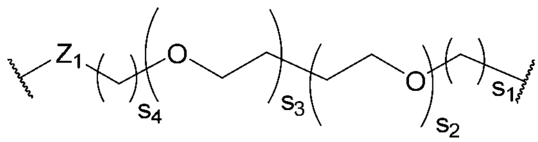;

$Z_1$ is selected from a bond, $-(CH_2)_p-$, $-(C_2H_4O)_q-$, $-(CH_2)_p-C(O)NH-$, $-(CH_2)_p-O-(CH_2)_p-C(O)NH-$, $-(CH_2)_p-C(O)-L_6-NH-$, $-(CH_2)_p-O-(CH_2)_p-C(O)-L_6-NH-$, -C(O)NH-, -C(O)O-, -C(O)-, -OC(O)-, -NH-, -O-, and -OC(O)NH-;

m is an integer from 1 to 6;

n is an integer from 1 to 6;

s is an integer from 1 to 6;

t is an integer from 0 to 10;

$s_1$, $s_2$, $s_3$, and $s_4$ are each independently an integer from 0 to 10;

$s_5$ and $s_6$ are each independently an integer from 1 to 6;

$t_1$ is an integer from 1 to 6;

$t_2$ is an integer from 0 to 6;

$t_3$ is an integer from 1 to 6;

$t_4$ is an integer from 0 to 10;

$t_5$ is an integer from 0 to 10;

p is an integer from 1 to 10;

q is an integer from 1 to 10;

v is from 1 to 10, and v is a decimal or an integer;

Pc is an antibody or an antigen-binding fragment thereof, or a modified antibody;

the modified antibody has $Pc'-((L_5)_w-F)_x$ structure, wherein:

Pc' is an antibody;

$L_5$ is a linker;

w is 0 or 1;

F is a clickable probe or sulfhydryl or a precursor thereof that can be connected to Q' after a reaction such as a metal-free click reaction, and preferably, F represents an azido group; and

x is an integer from 1 to 8.

**33.** The ligand-drug conjugate represented by the general formula (B) or the pharmaceutically acceptable salt thereof according to claim 32, wherein:

$L_3$ is selected from:

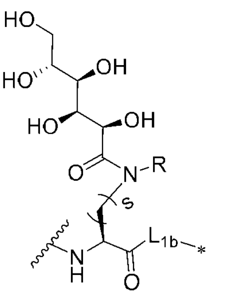,
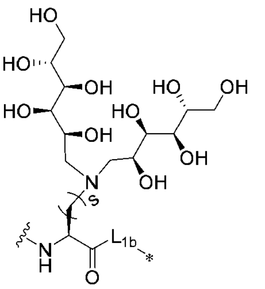,
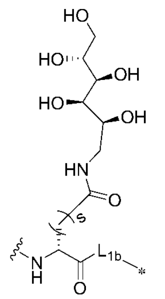,
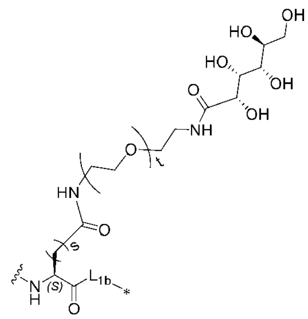,

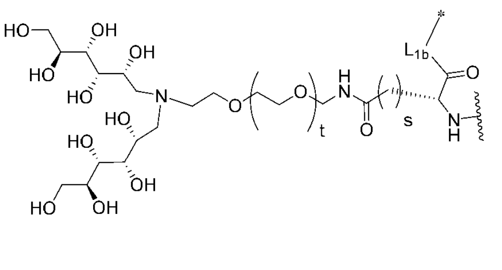,
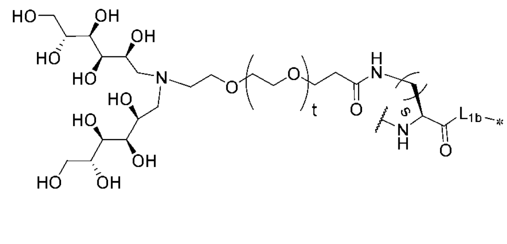,

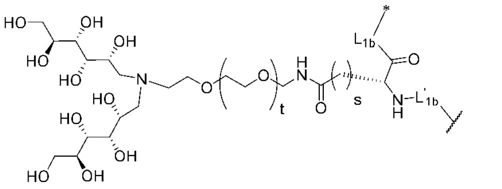,
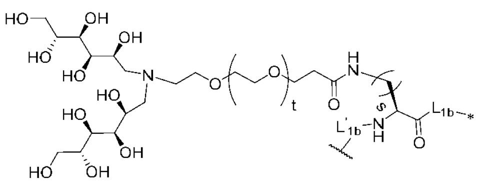,

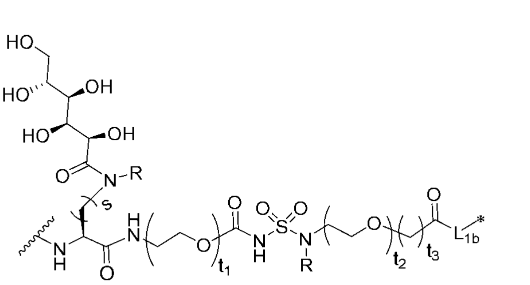,
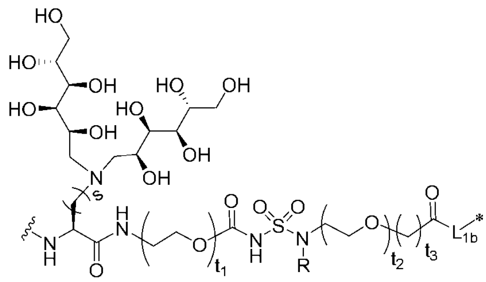,

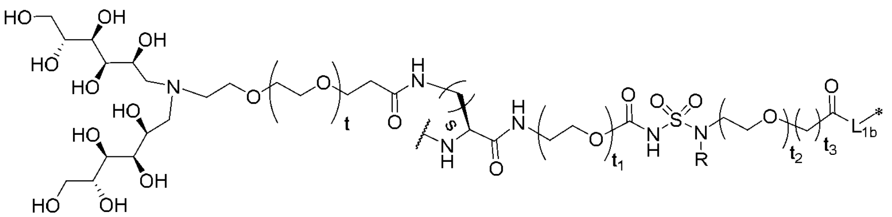,
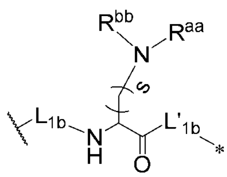,

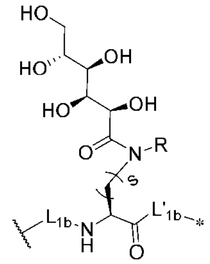, and 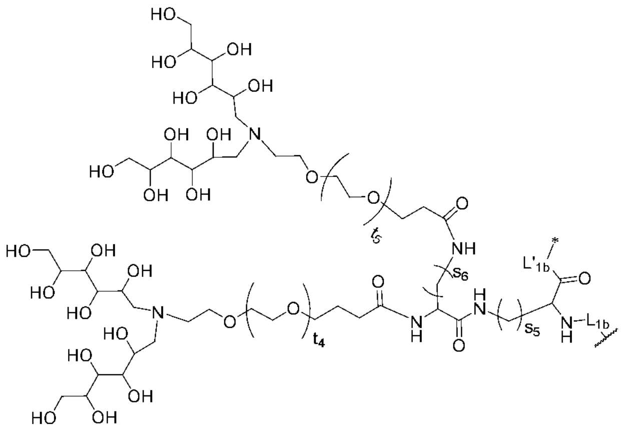,

R is selected from hydrogen and $C_1$-$C_6$ alkyl, and is preferably hydrogen;
$R^{aa}$ and $R^{bb}$ are each independently selected from $C_1$-$C_6$ alkyl;
s is an integer from 1 to 6; preferably, an integer from 2 to 6; more preferably, an integer from 2 to 4;
$s_5$ and $s_6$ are each independently an integer from 1 to 6; preferably, an integer from 2 to 6; more preferably, an integer from 2 to 4;
t is an integer from 0 to 10;
$t_1$ is an integer from 1 to 6; preferably, an integer from 1 to 4; more preferably, an integer from 2 to 4; even more preferably 1 or 2;
$t_2$ is an integer from 0 to 6; preferably, an integer from 1 to 4; more preferably 1 or 2;
$t_3$ is an integer from 1 to 6; preferably, an integer from 1 to 4; more preferably 1 or 2;
$t_4$ is an integer from 0 to 10;
$t_5$ is an integer from 0 to 10;
* is a connection site with $L_2$;
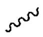 is a connection site with carbonyl or methylene;
$L_{1b}$ and $L'_{1b}$ are each independently an amino acid residue formed by one or more amino acids selected from glycine, phenylalanine, citrulline, leucine, isoleucine, alanine, valine, asparagine, glutamine, arginine, glutamic acid, and lysine; preferably an amino acid residue formed by two or more amino acids selected from glycine, phenylalanine, citrulline, valine, lysine, glutamine, glutamic acid, leucine, and alanine; and
preferably, $L_{1b}$ and $L'_{1b}$ are each independently selected from -Gly-*, -Val-*, -Gly-Phe-Gly-*, -Phe-Gly-*, -Gly-Val-Cit-*, -Val-Cit-*, -Gly-Val-Arg-*, -Val-Arg-*, -Gly-Val-Ala-*, -Val-Ala-*, -Gly-Phe-*, -Phe-Gly-*, -Gly-Gly-Gly-*, Gly-Gly-*, -Gly-Val-Gly-*, -Gly-Ala-Gly-*, -Gly-Phe-Cit-*, - Gly-Phe-Val-*, -Gly-Phe-Ala-*, -Gly-Phe-Lys-*, -Phe-Lys-*, -Gly-Val-*, -Gly-Cit-*, -Gly-Ala-*, - Gly-Gly-Lys-*, Gly-Lys'-*, -Ala-Ala-Ala-*, -Gln-Val-Ala-*, -Gln-Val-Cit-*, -Asp-Val-Ala-*, -Asp-Val-Cit-*, -Lys-Gly-Val-Ala-*, -Lys-Gly-Val-Cit-*, -Lys-Gly-Gly-Val-Ala-*, -Lys-Gly-Gly-Val-Cit-*, Gly-Gly-Phe-Gly-*, -Lys-Gln-Val-Cit-*, -Lys-Gln-Val-Ala-*, -Lys-Glu-Val-Cit-*, -Lys-Glu-Val-ALa-*, -Lys-Asp-Val-Cit-*, -Lys-Asp-Val-Ala-*, Glu-Val-Cit-*, Glu-Val-Ala-*, -Lys-Val-Ala-*, - Lys-Val-Cit-*, -Val-Lys-Gly-*, and -Val-Lys-*; preferably, -Gly-Phe-Gly-*, -Gly-Val-Cit-*, -Val-Cit-*, -Gly-Val-Ala-*, -Val-Ala-*, -Gly-Phe-Gly-*, -Phe-Gly-*, -Gly-Phe-Lys-, -Phe-Lys- *, -Gln-Val-Ala-*, -Gln-Val-Cit-*, -Asp-Val-Ala-*, -Lys-Gly-Val-Ala-*, -Lys-Gly-Val-Cit-*, -Lys-Gly-Gly-Val-Ala-*, -Lys-Gly-Gly-Val-Cit-*, Gly-Gly-Phe-Gly-*, -Lys-Gln-Val-Cit-*, -Lys-Gln-Val-Ala-*, -Lys-Glu-Val-Cit-*, -Lys-Glu-Val-ALa-*, -Lys-Asp-Val-Cit-*, -Lys-Asp-Val-Ala-*, Glu-Val-Cit-*, Glu-Val-Ala-*, -Lys-Val-Ala-*, -Lys-Val-Cit-*, -Val-Lys-Gly-*, -Val-Lys-*, and -Asp-Val-Cit-*, wherein * is a connection site with $L_2$.

**34.** The ligand-drug conjugate represented by the general formula (B) or the pharmaceutically acceptable salt thereof according to claim 32, wherein:

L' is

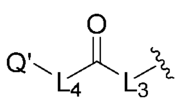 or 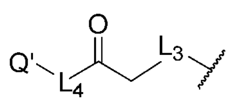;

Q' is selected from

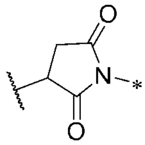, 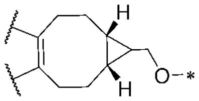, 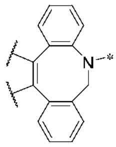, 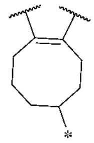, 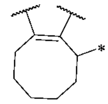, 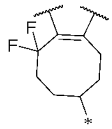, 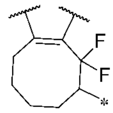,

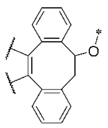, 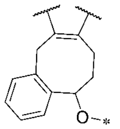, 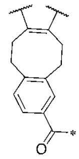, 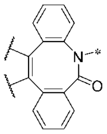, 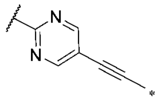, 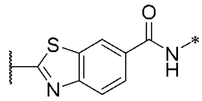, 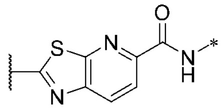,

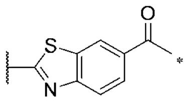, and 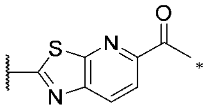,

wherein, * is a connection site with $L_4$, and ∿ is a connection site with Pc;

$L_3$ is an amino acid residue formed by two or more amino acids selected from glycine, phenylalanine, citrulline, leucine, isoleucine, alanine, valine, asparagine, glutamine, arginine, glutamic acid, and lysine, preferably an amino acid residue formed by two or more amino acids selected from glycine, phenylalanine, citrulline, valine, lysine, glutamine, glutamic acid, leucine, and alanine;

$L_4$ is

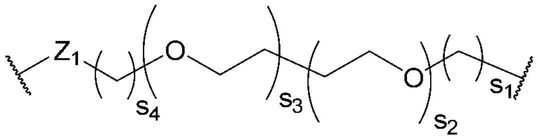;

$Z_1$ is selected from a bond, $-(CH_2)_p-$, $-(CH_2)_p-C(O)NH-$, $-(CH_2)_p-O-(CH_2)_p-C(O)NH-$, $-(CH_2)_p-C(O)-L_6-NH-$, $-(CH_2)_p-O-(CH_2)_p-C(O)-L_6-NH-$, -C(O)NH-, -C(O)O-, -C(O)-, -OC(O)-, -NH-, -O-, and - OC(O)NH-;

$s_1$ is an integer from 1 to 6; preferably, an integer from 2 to 6;

$s_2$ is an integer from 1 to 10; preferably, an integer from 2 to 10;

$s_3$ is 0;

$s_4$ is 0;

p is an integer from 1 to 10; preferably, an integer from 1 to 6;

$L_6$ is selected from

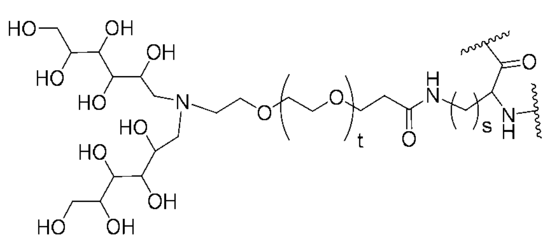

and

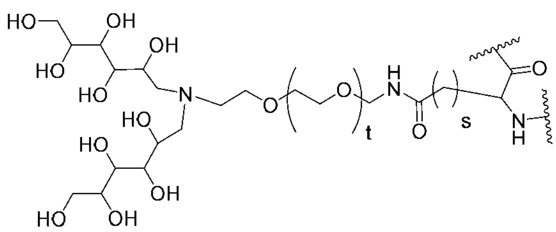;

s is an integer from 1 to 6; preferably, an integer from 2 to 6; more preferably, an integer from 2 to 4;
t is an integer from 0 to 10; and
$L_6$ is preferably

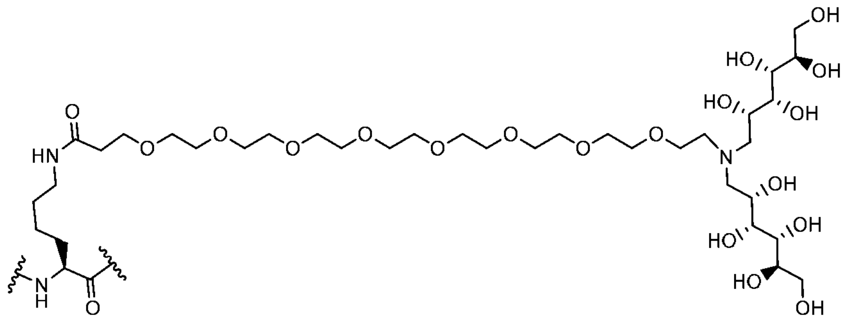

.

**35.** The ligand-drug conjugate represented by the general formula (B) or the pharmaceutically acceptable salt thereof according to any one of claims 32 to 34, wherein $L_5$ is

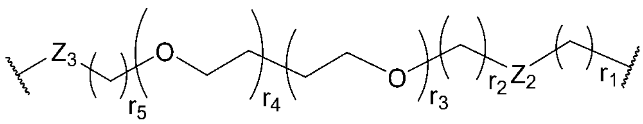

;

$Z_2$ and $Z_3$ are each independently selected from -C(O)NH-, -C(O)O-, -C(O)-, -OC(O)-, -NH-, -O-, and -OC(O)NH-, preferably -C(O)NH-;
$r_1$ is an integer from 1 to 8; preferably, an integer from 1 to 6; more preferably, an integer from 1 to 3;
$r_2$ is an integer from 1 to 6; preferably, an integer from 1 to 4; more preferably, an integer from 1 to 2;
$r_3$ is an integer from 0 to 6; preferably, an integer from 0 to 4; more preferably, an integer from 0 to 2;
$r_4$ is an integer from 0 to 6; preferably, an integer from 0 to 4; more preferably, an integer from 0 to 2; and
$r_5$ is an integer from 1 to 6; preferably, an integer from 1 to 4; more preferably, an integer from 1 to 2.

**36.** The ligand-drug conjugate represented by the general formula (B) or the pharmaceutically acceptable salt thereof according to any one of claims 32 to 35, wherein when w is 0, F is sulfhydryl; when w is 1, F is a clickable probe that can be connected to Q' after a reaction such as a metal-free click reaction; and F is preferably an azido group.

**37.** The ligand-drug conjugate represented by the general formula (B) or the pharmaceutically acceptable salt thereof according to any one of claims 32 to 36, wherein

Pc is a modified antibody and the modified antibody has a structure of:

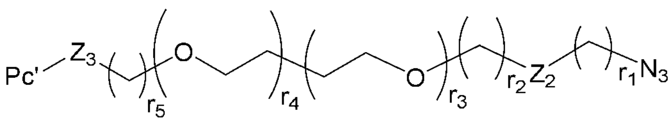

wherein:
Pc' is an antibody;
$Z_2$ and $Z_3$ are each independently selected from -C(O)NH-, -C(O)O-, -C(O)-, -OC(O)-, -NH-, -O-, and -OC(O)NH-, preferably -C(O)NH-;
$r_1$ is an integer from 1 to 8; preferably, an integer from 1 to 6; more preferably, an integer from 1 to 3;
$r_2$ is an integer from 1 to 6; preferably, an integer from 1 to 4; more preferably, an integer from 1 to 2;
$r_3$ is an integer from 0 to 6; preferably, an integer from 0 to 4; more preferably, an integer from 0 to 2;
$r_4$ is an integer from 0 to 6; preferably, an integer from 0 to 4; more preferably, an integer from 0 to 2; and
$r_5$ is an integer from 1 to 6; preferably, an integer from 1 to 4; more preferably, an integer from 1 to 2.

**38.** The ligand-drug conjugate represented by the general formula (B) or the pharmaceutically acceptable salt thereof according to any one of claims 32 to 37, wherein Pc is a modified antibody and Pc-Q' is selected from:

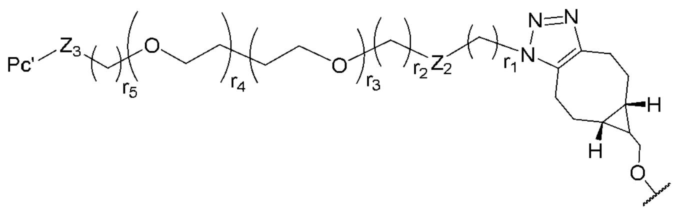

,

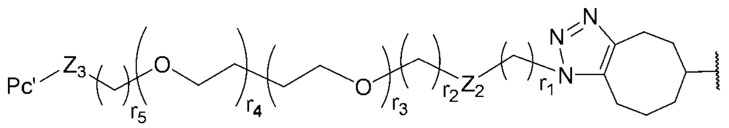

,

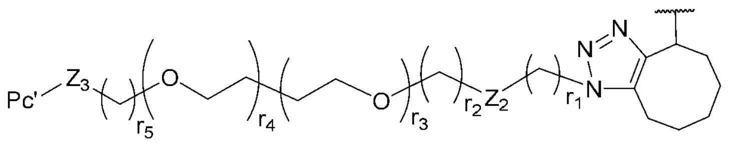

,

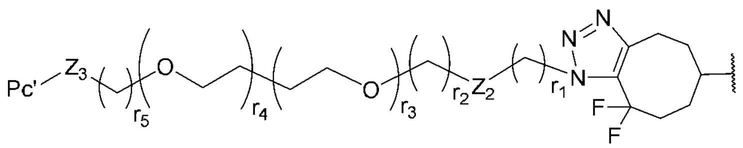

,

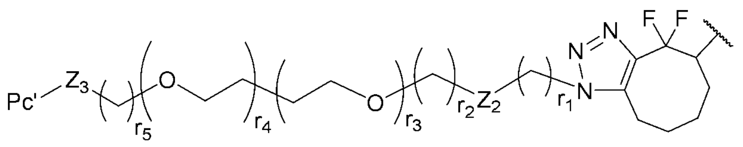

,

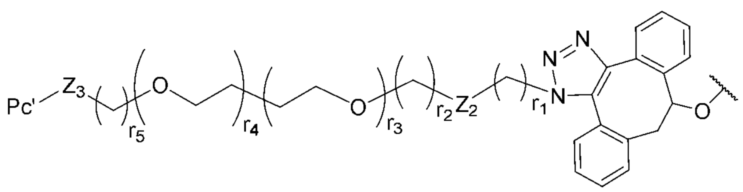

,

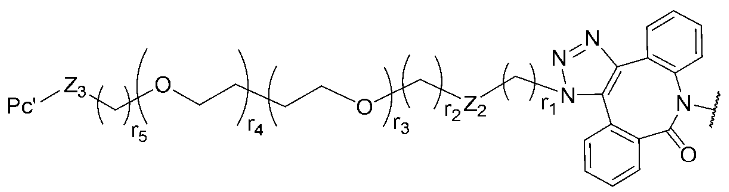

,

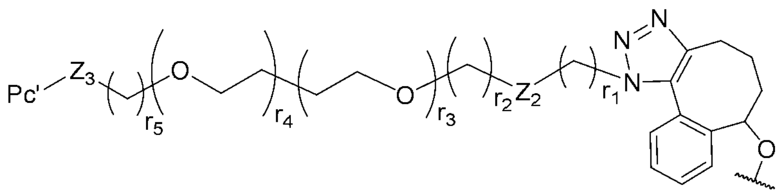

,

and

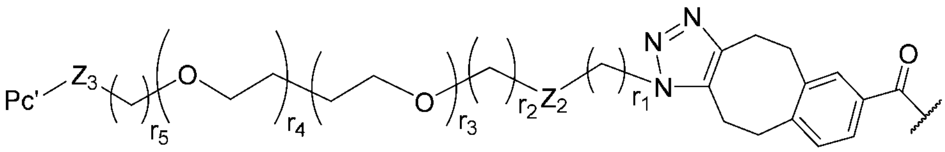

;

Pc' is an antibody;

$Z_2$ and $Z_3$ are each independently selected from -C(O)NH-, -C(O)O-, -C(O)-, -OC(O)-, -NH-, -O-, and -OC(O)NH-, preferably -C(O)NH-;

$r_1$ is an integer from 1 to 8; preferably, an integer from 1 to 6; more preferably, an integer from 1 to 3;

$r_2$ is an integer from 1 to 6; preferably, an integer from 1 to 4; more preferably, an integer from 1 to 2;

$r_3$ is an integer from 0 to 6; preferably, an integer from 0 to 4; more preferably, an integer from 0 to 2;

$r_4$ is an integer from 0 to 6; preferably, an integer from 0 to 4; more preferably, an integer from 0 to 2; and

$r_5$ is an integer from 1 to 6; preferably, an integer from 1 to 4; more preferably, an integer from 1 to 2.

**39.** The ligand-drug conjugate represented by the general formula (B) or the pharmaceutically acceptable salt thereof according to any one of claims 32 to 38, which is selected from:

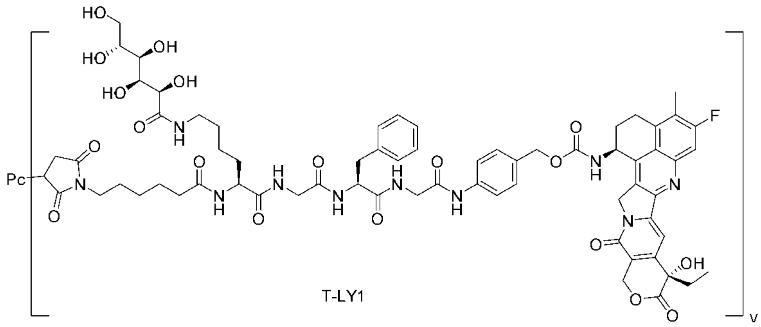

T-LY1

,

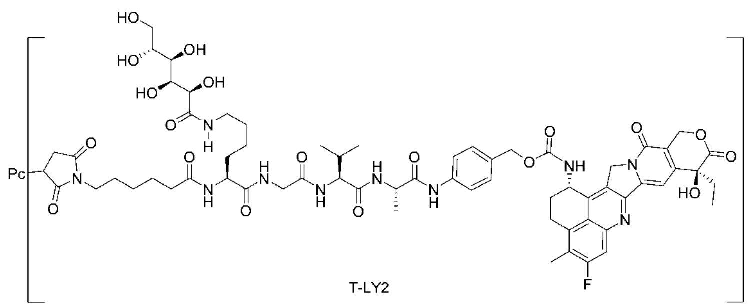

T-LY2

,

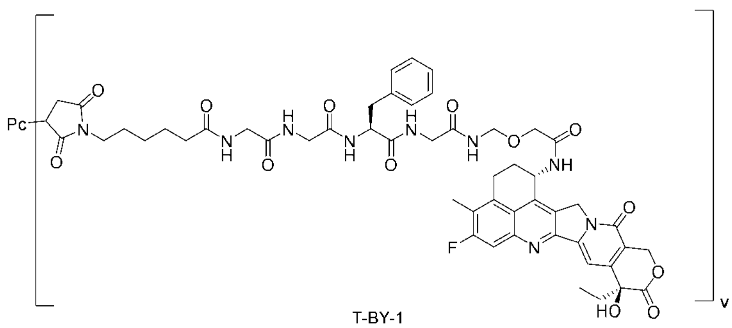

T-BY-1

,

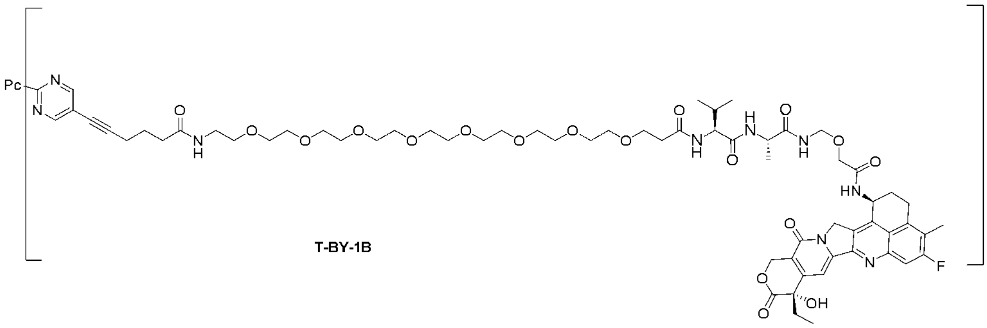

T-BY-1B

,

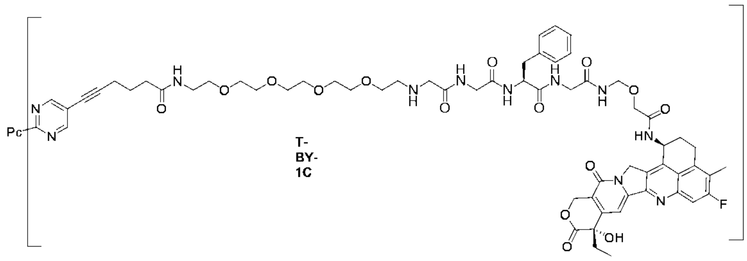

T-BY-1C

,

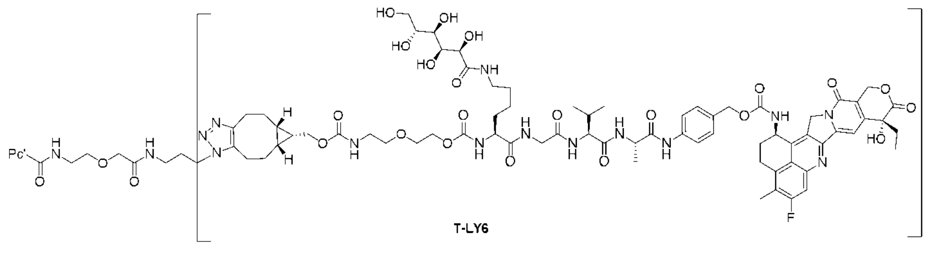

T-LY6

,

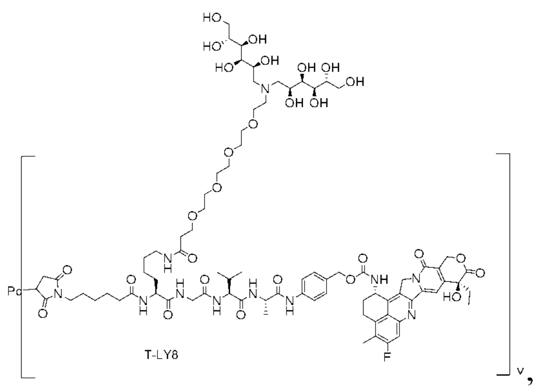

T-LY8

,

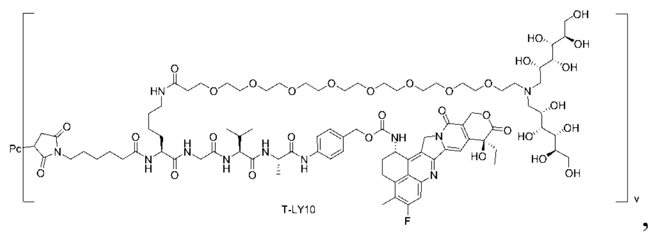

T-LY10

,

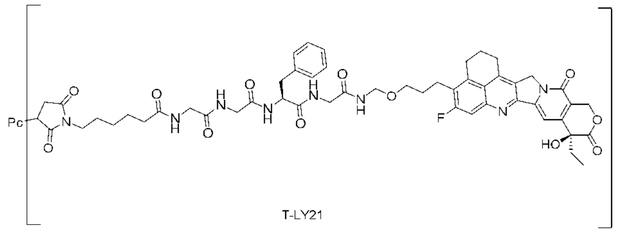

T-LY21

v,

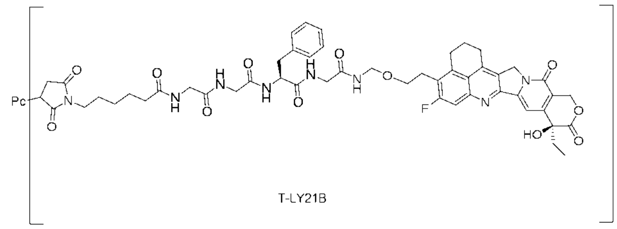

T-LY21B

v,

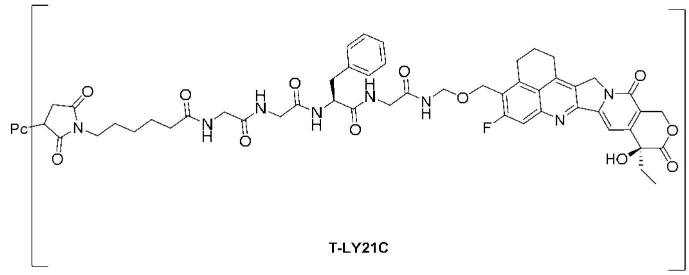

**T-LY21C**

v,

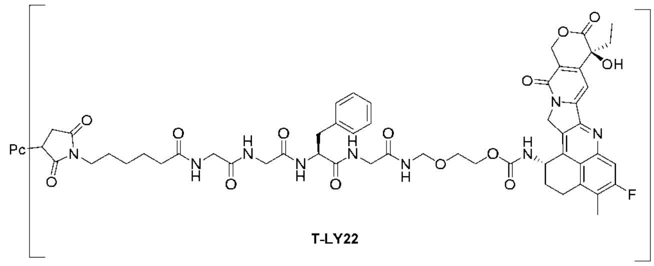

**T-LY22**

v,

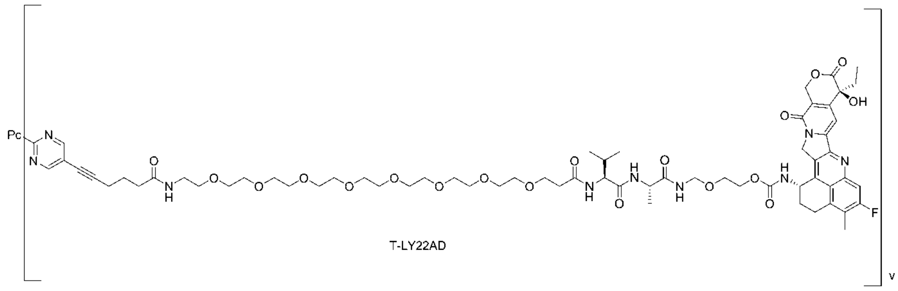

T-LY22AD

v,

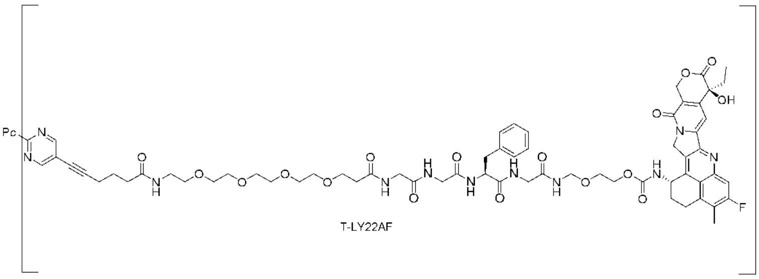

T-LY22AF

,

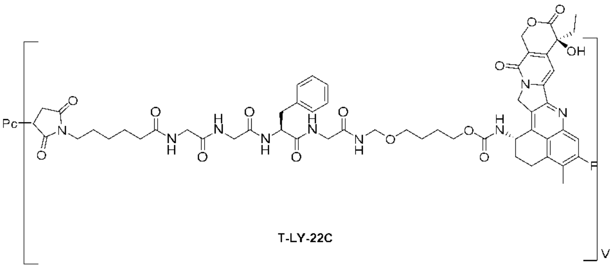

T-LY-22C

,

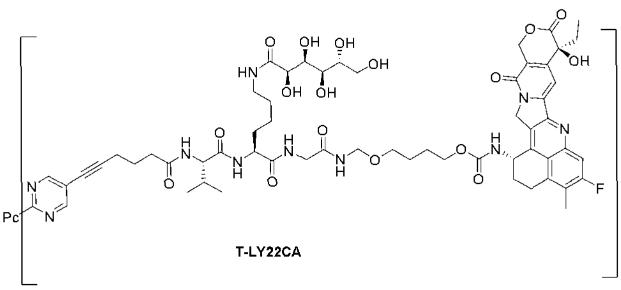

T-LY22CA

,

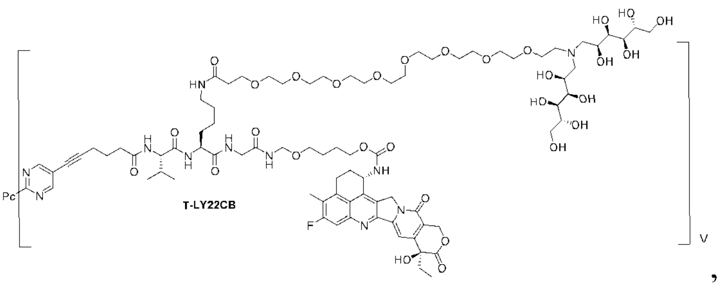

T-LY22CB

,

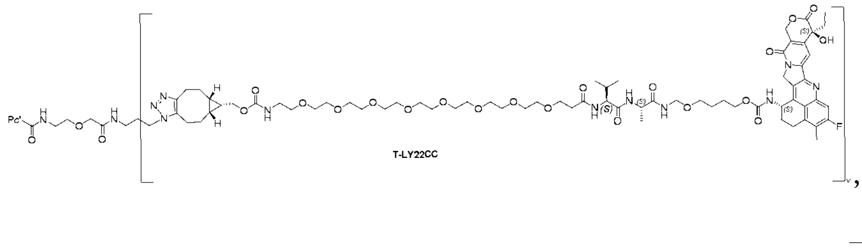

T-LY22CC

,

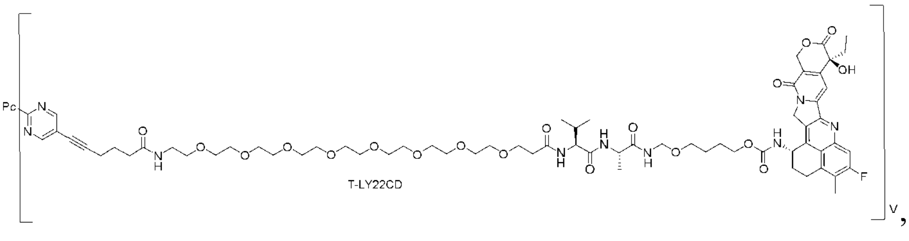

T-LY22CD

,

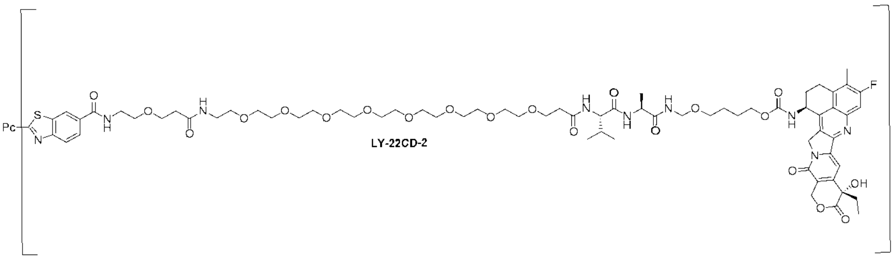

LY-22CD-2

,

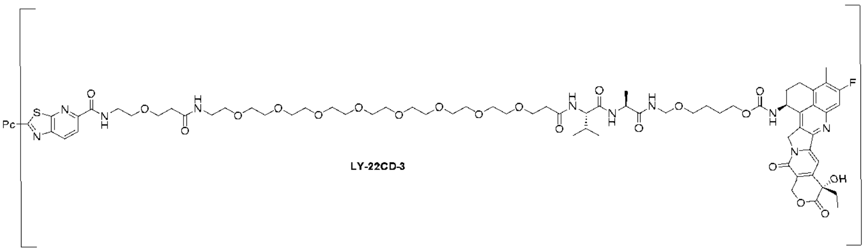

LY-22CD-3

,

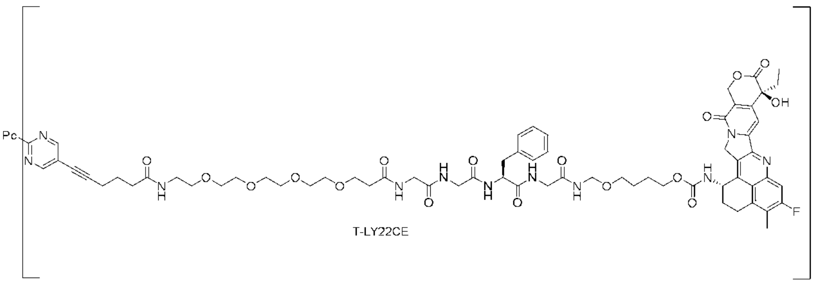

T-LY22CE

,

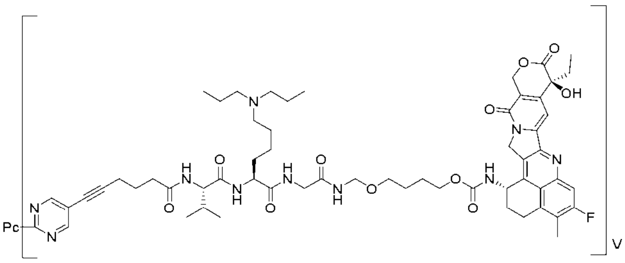

T-LY22CF

,

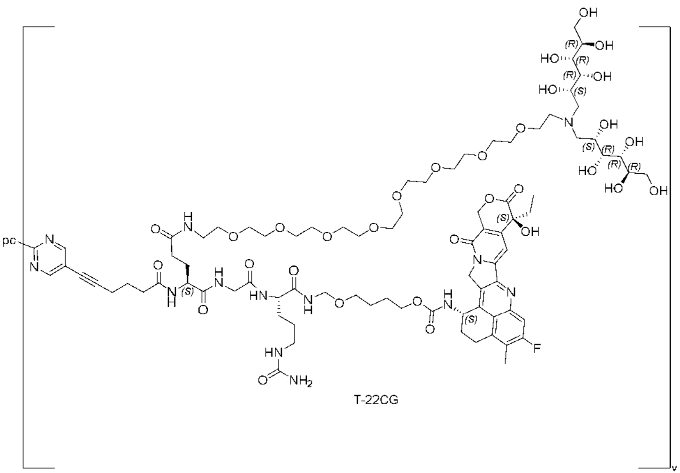

T-22CG

,

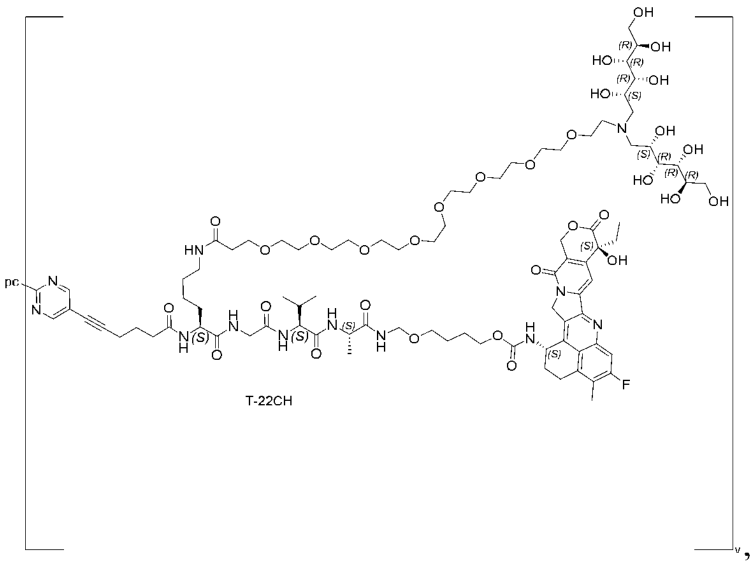

T-22CH

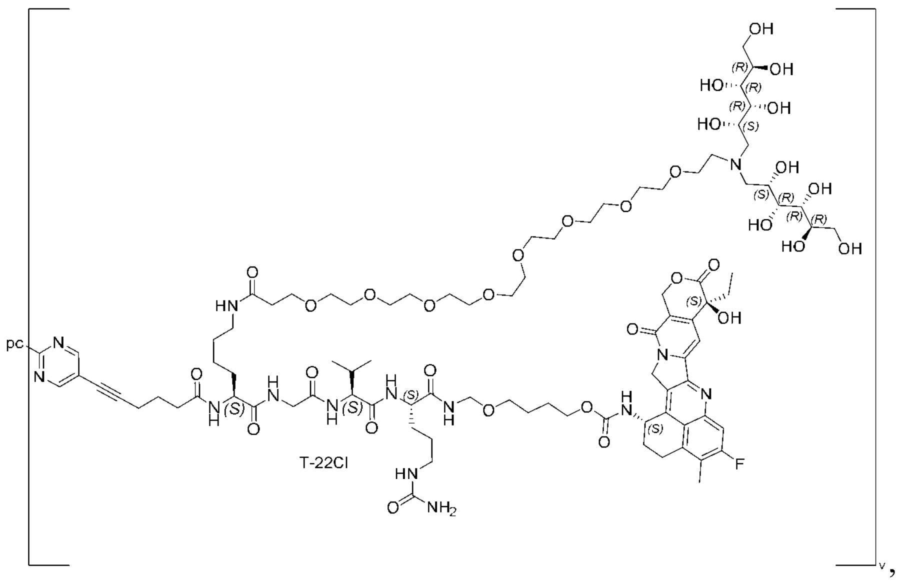

T-22CI

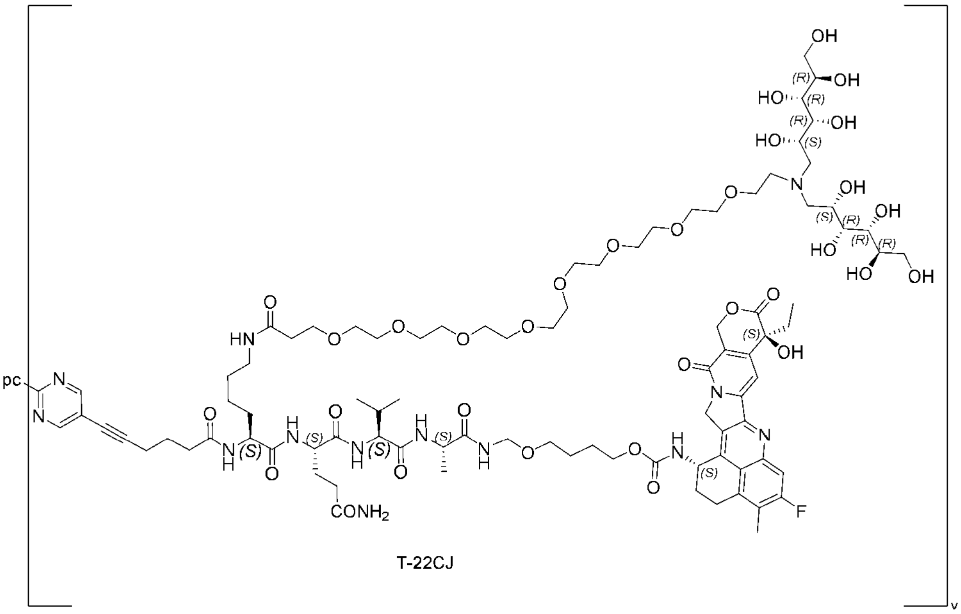

T-22CJ

,

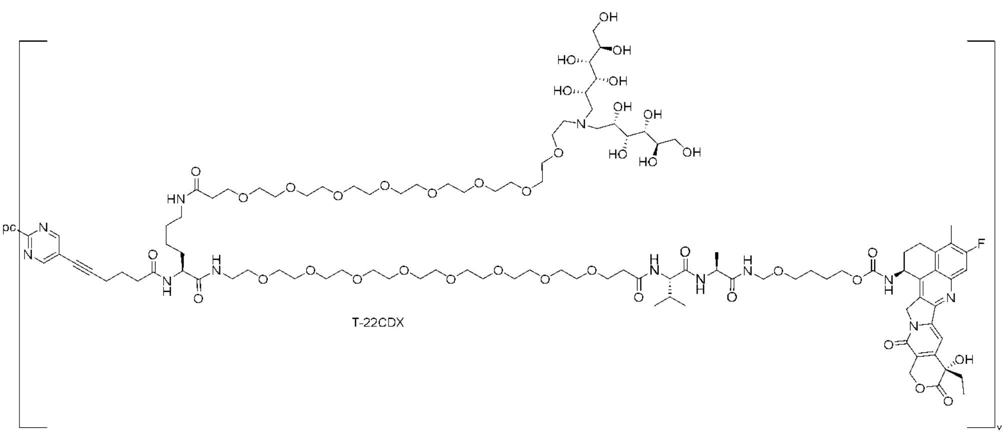

T-22CDX

,

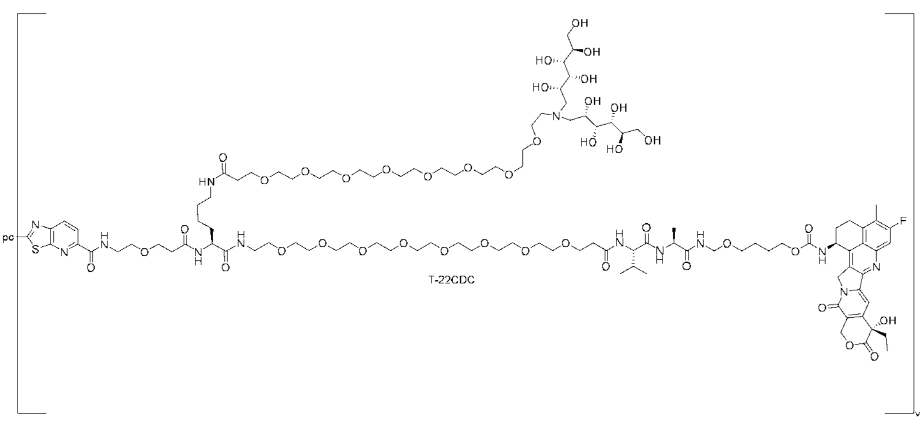

T-22CDC

,

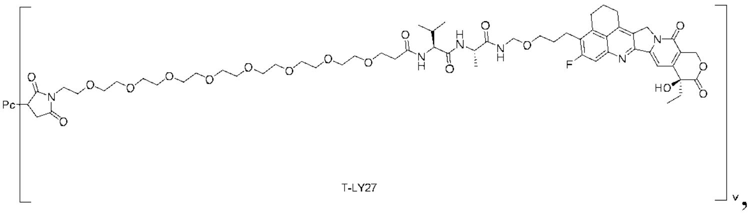

T-LY27

,

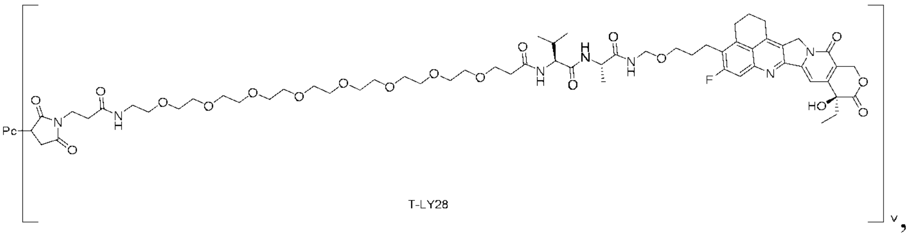

T-LY28

,

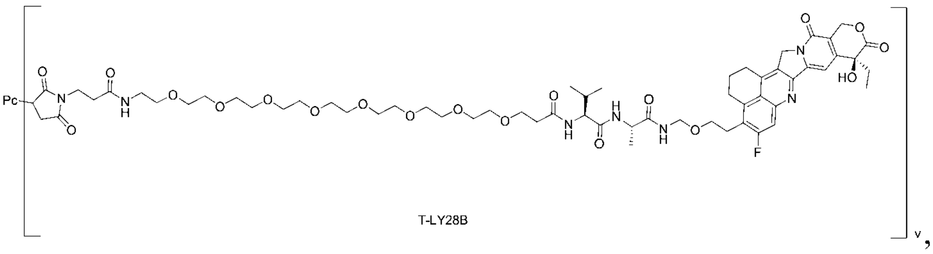

T-LY28B

,

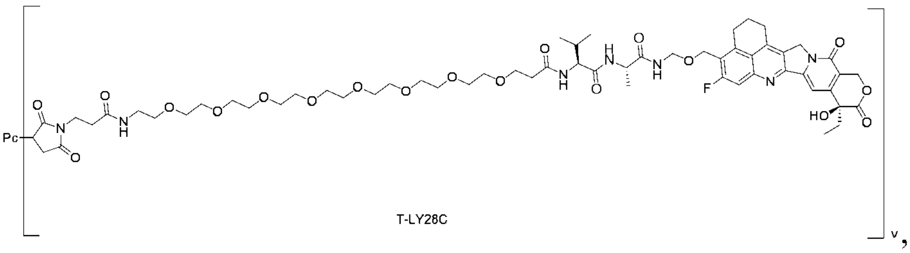

T-LY28C

,

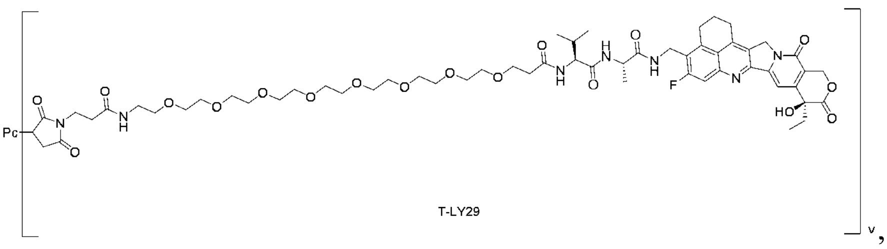

T-LY29

,

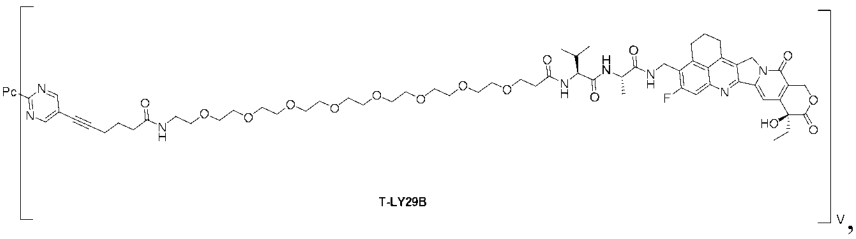

T-LY29B

,

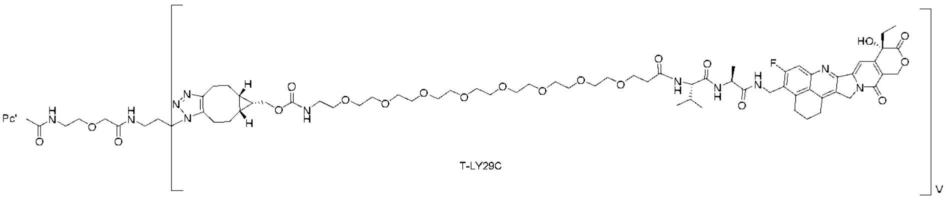

T-LY29C,

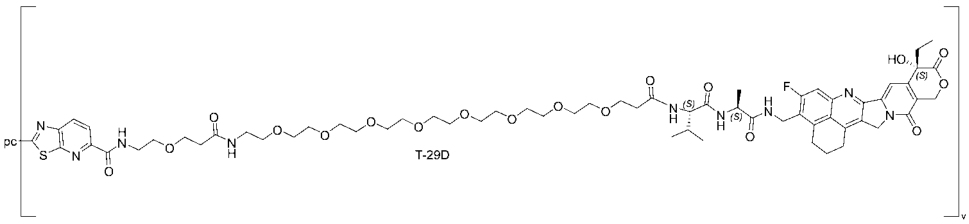

T-29D,

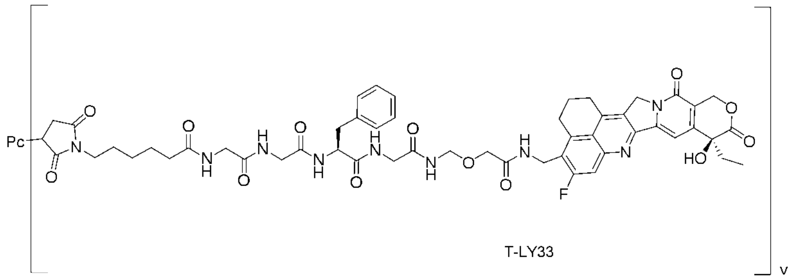

T-LY33,

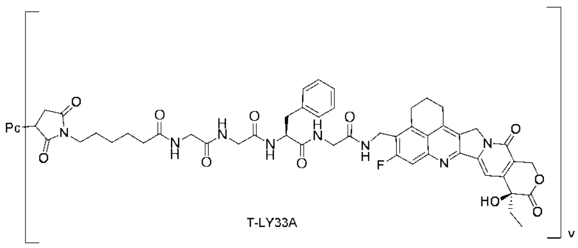

T-LY33A,

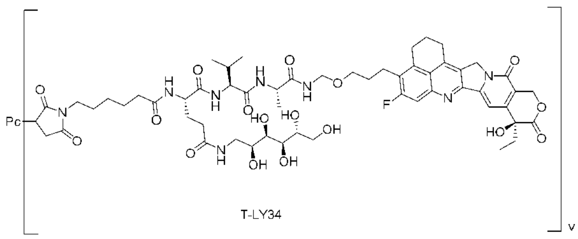

T-LY34,

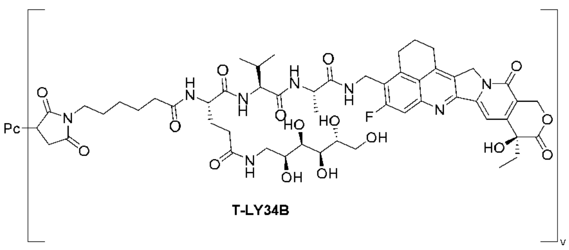

T-LY34B,

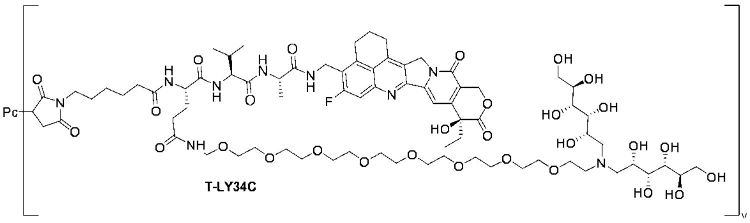

T-LY34C ,

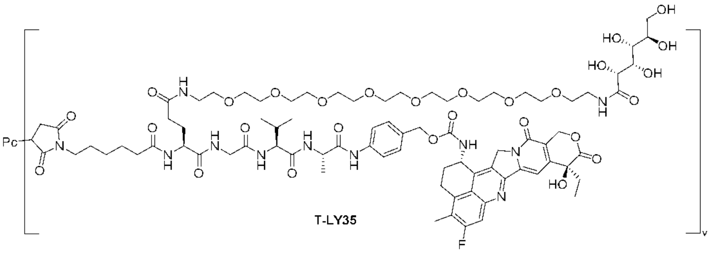

T-LY35 ,

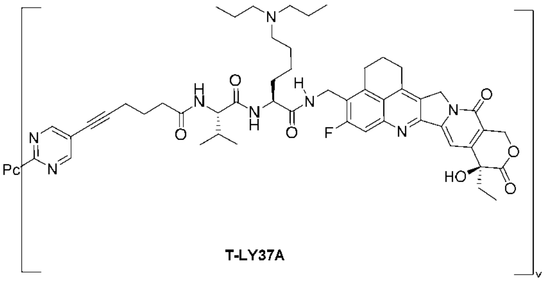

T-LY37A ,

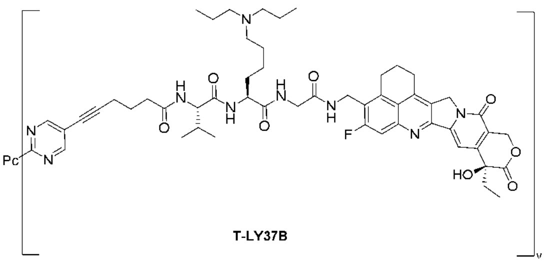

T-LY37B ,

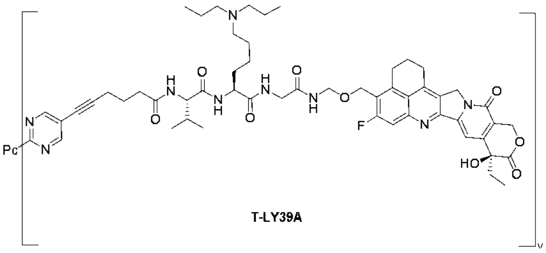

T-LY39A ,

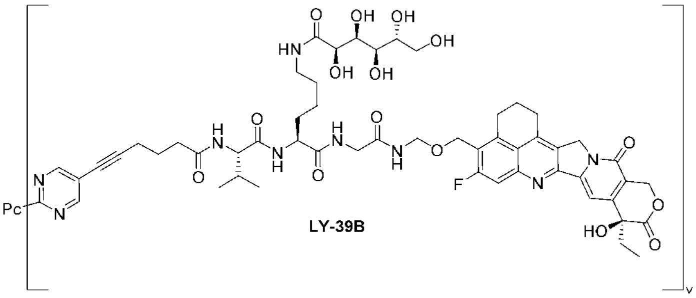
LY-39B
,

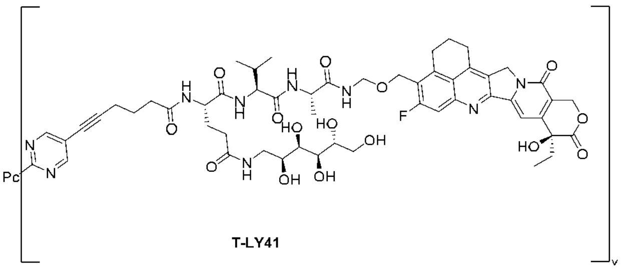
T-LY41
,

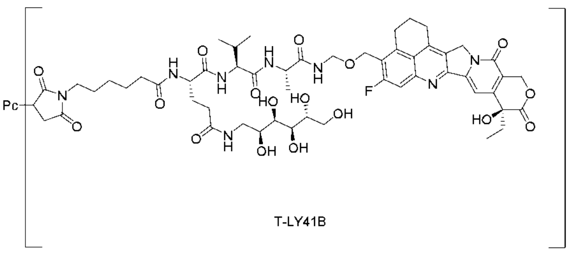
T-LY41B
,

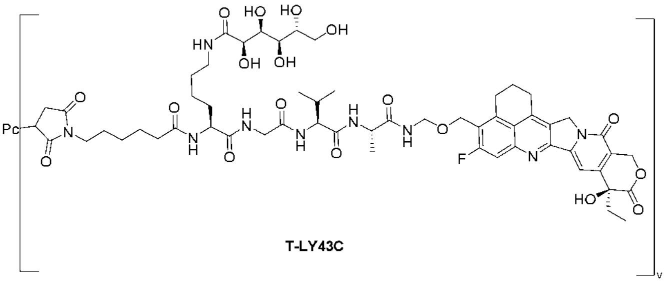
T-LY43C
,

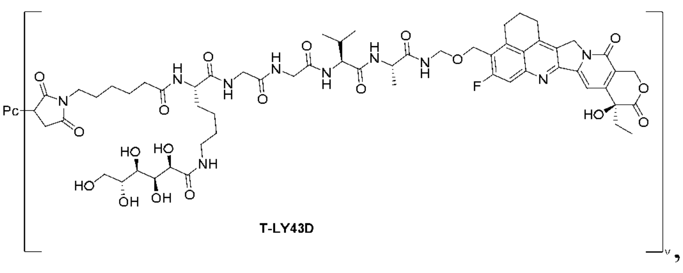
T-LY43D
,

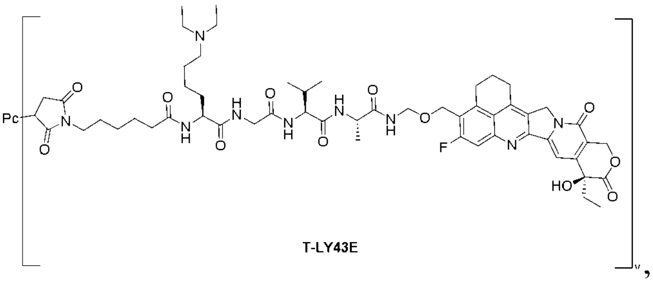

T-LY43E ,

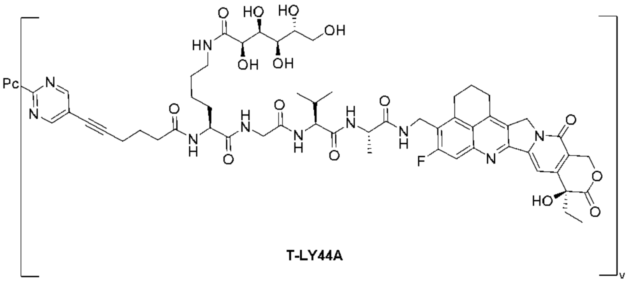

T-LY44A ,

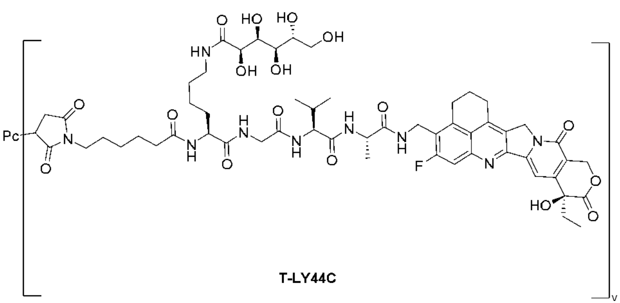

T-LY44C ,

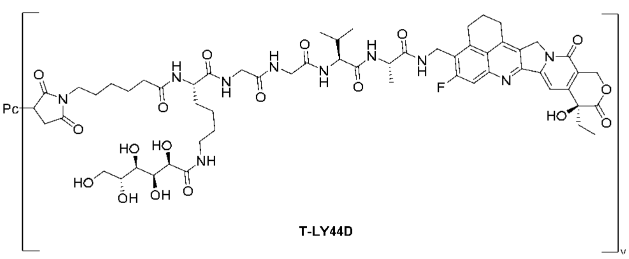

T-LY44D ,

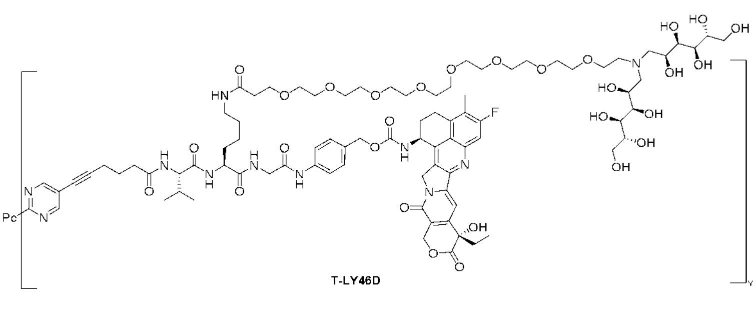

T-LY46D ,

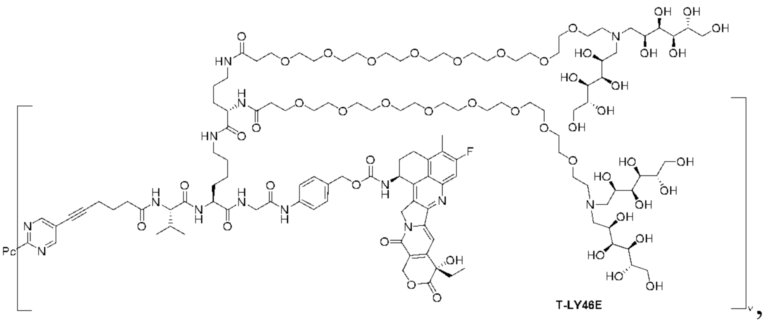

T-LY46E

,

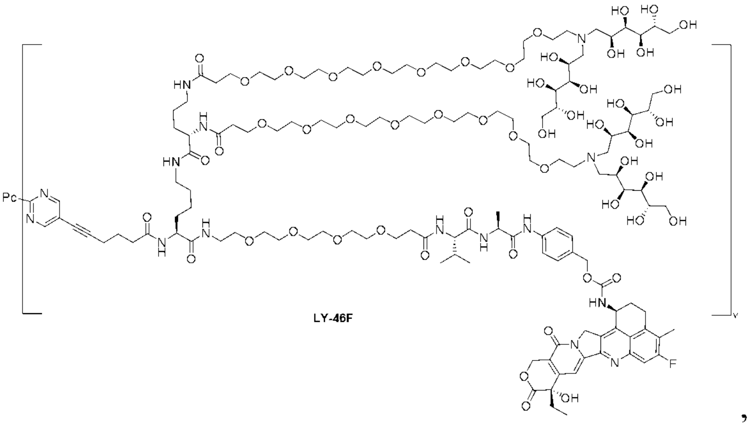

LY-46F

,

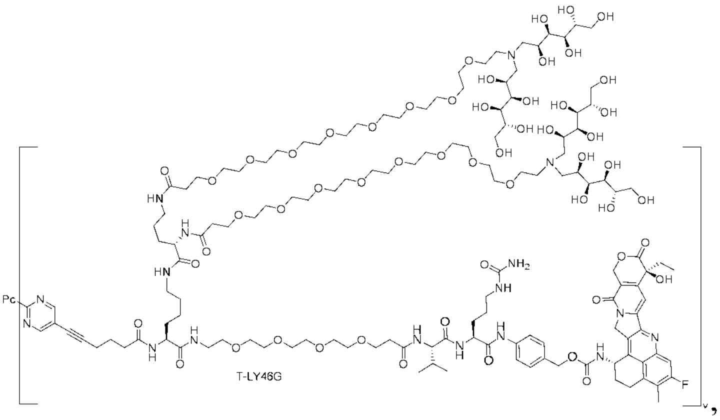

T-LY46G

,

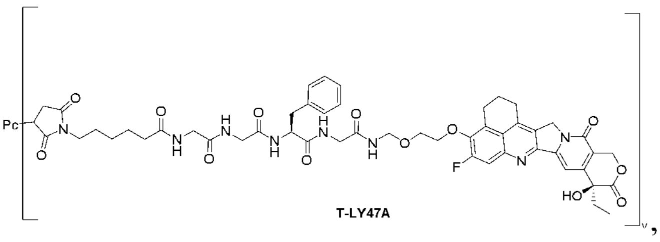

T-LY47A

,

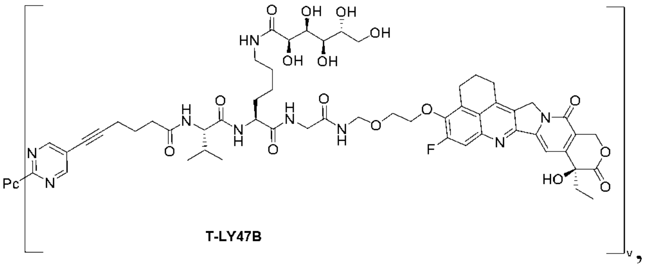

T-LY47B

,

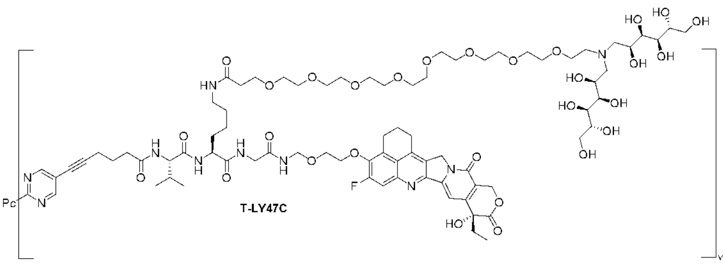

T-LY47C

,

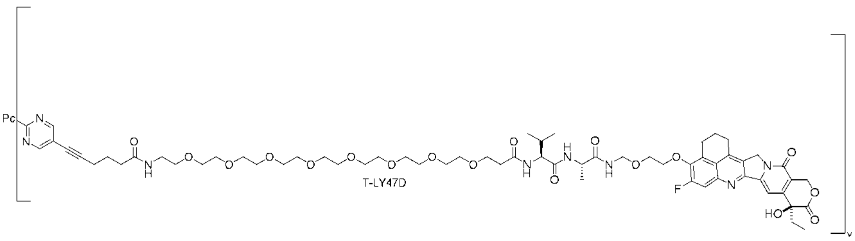

T-LY47D

,

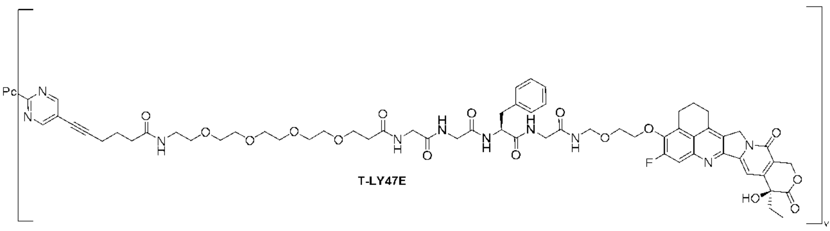

T-LY47E

,

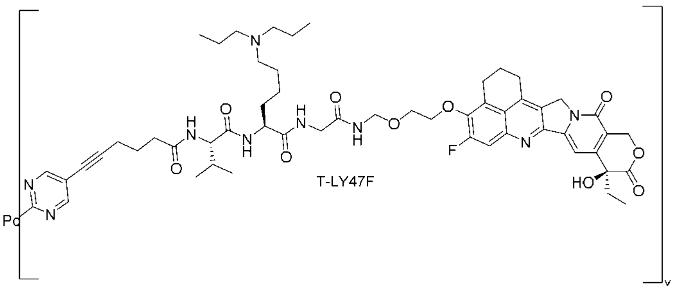

T-LY47F

,

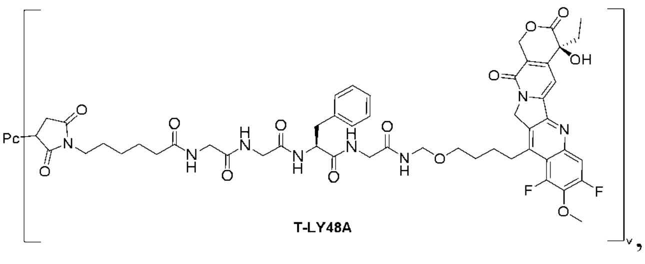

T-LY48A

,

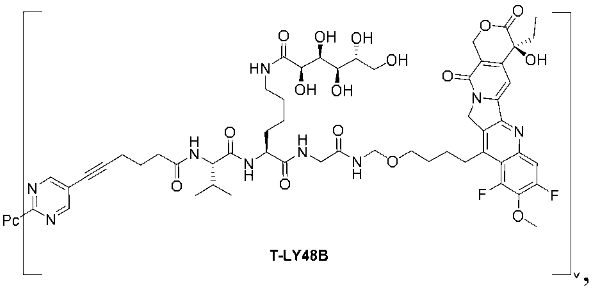

T-LY48B

,

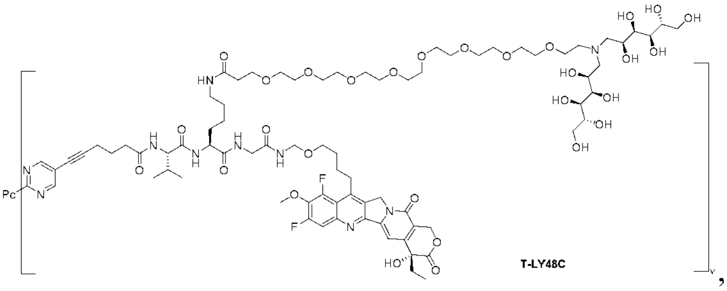

T-LY48C

,

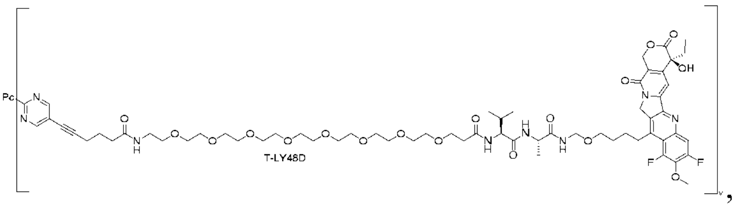

T-LY48D

,

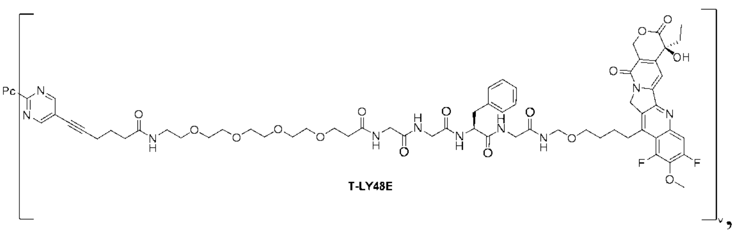

T-LY48E

,

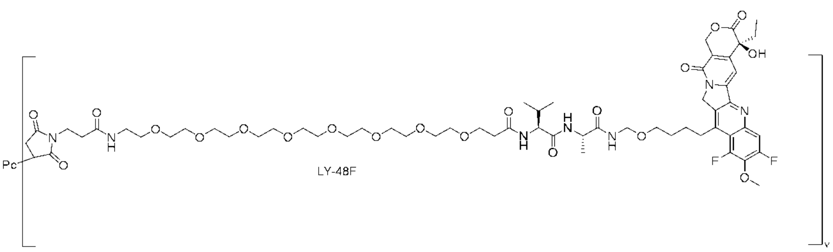

LY-48F

,

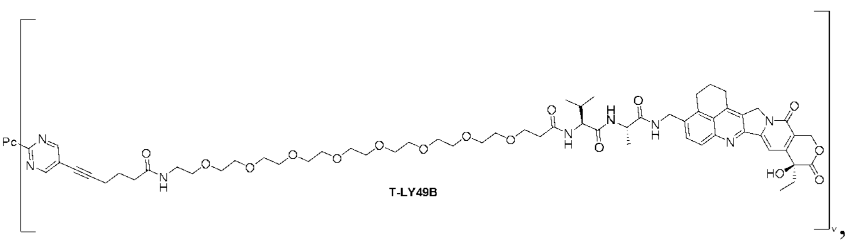

T-LY49B

,

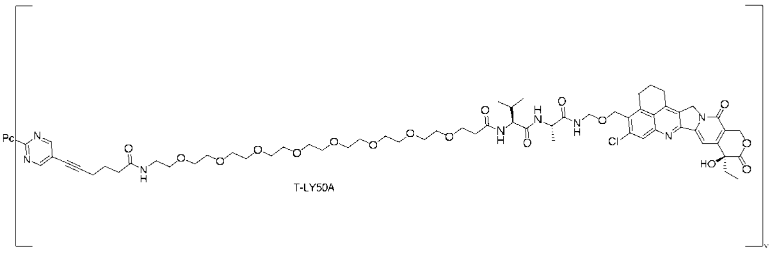

T-LY50A

,

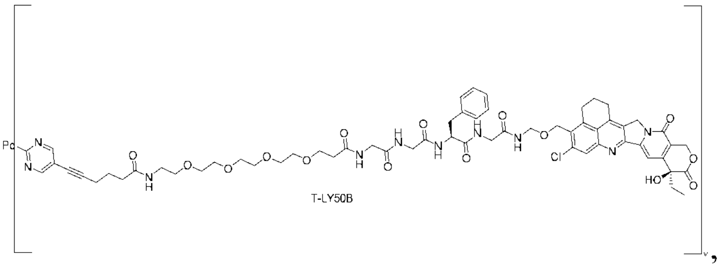

T-LY50B

,

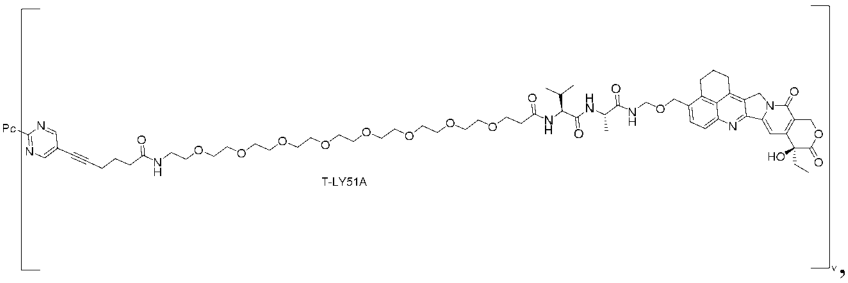

T-LY51A

,

and

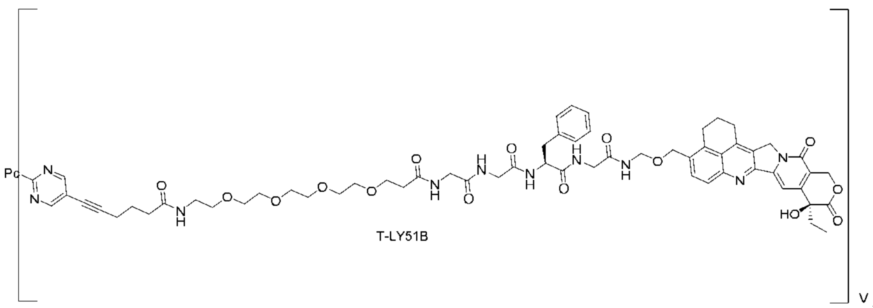

T-LY51B

;

wherein:

v is from 1 to 10, and v is a decimal or an integer;
Pc is an antibody or an antigen-binding fragment thereof; and
Pc' is an antibody.

**40.** The ligand-drug conjugate represented by the general formula (B) or the pharmaceutically acceptable salt thereof according to any one of claims 32 to 39, wherein the antibody is selected from a murine antibody, a chimeric antibody, a humanized antibody, and a fully human antibody; and preferably, the antibody or an antigen-binding fragment thereof is selected from anti-HER2 (ErbB2) antibody, anti-EGFR antibody, anti-B7-H3 antibody, anti-c-Met antibody, anti-HER3 (ErbB3) antibody, anti-HER4 (ErbB4) antibody, anti-ROR1 antibody, anti-CLDN6 antibody, anti-CLDN9 antibody, anti-CLDN18.2 antibody, anti-NaPi-2b antibody, anti-TNF-α antibody, anti-ENPP3 antibody, anti-DLL3 antibody, anti-CD20 antibody, anti-CD22 antibody, anti-CD28 antibody, anti-CD30 antibody, anti-CD33 antibody, anti-CD37 antibody, anti-CD38 antibody, anti-CD44 antibody, anti-CD45 antibody, anti-CD47 antibody, anti-CD48 antibody, anti-CD56 antibody, anti-CD70 antibody, anti-CD73 antibody, anti-CD98 antibody, anti-CD105 antibody, anti-CEA antibody, anti-EphA2 antibody, anti-MUCI antibody, anti-Lewis Y antibody, anti-VEGFR antibody, anti-GPNMB antibody, anti-Integrin antibody, anti-PSMA antibody, anti-Tenascin-C antibody, anti-SLC44A4 antibody, anti-CD79 antibody, anti-TROP-2 antibody, anti-CD79B antibody, anti-Mesothelin antibody, anti-Nectin-4 antibody, anti-TPBG antibody, or an antigen-binding fragment thereof.

**41.** The ligand-drug conjugate represented by the general formula (B) or the pharmaceutically acceptable salt thereof according to any one of claims 32 to 40, wherein the antibody or the antigen-binding fragment thereof is selected from Trastuzumab, Cetuximab, Pertuzumab, Nimotuzumab, Enoblituzumab, Emibetuzumab, Inotuzumab, Pinatuzumab, Brentuximab, Gemtuzumab, Bivatuzumab, Lorvotuzumab, or an antigen-binding fragment thereof.

**42.** The ligand-drug conjugate represented by the general formula (B) or the pharmaceutically acceptable salt thereof according to any one of claims 32 to 41, which is selected from:

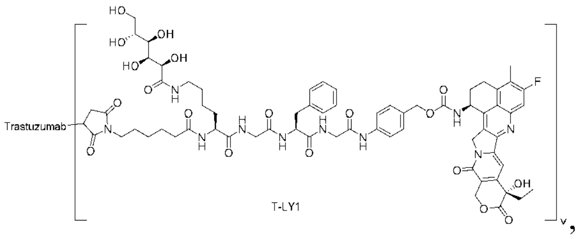
T-LY1
,

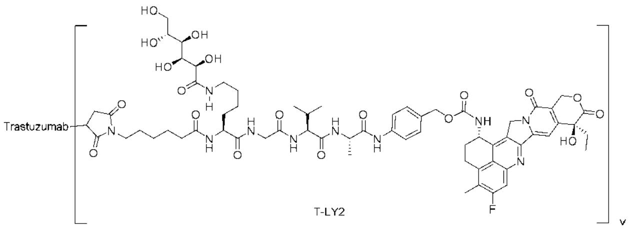
T-LY2
,

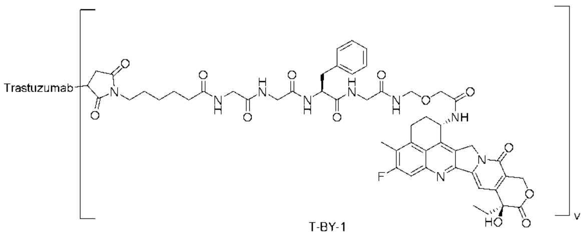
T-BY-1
,

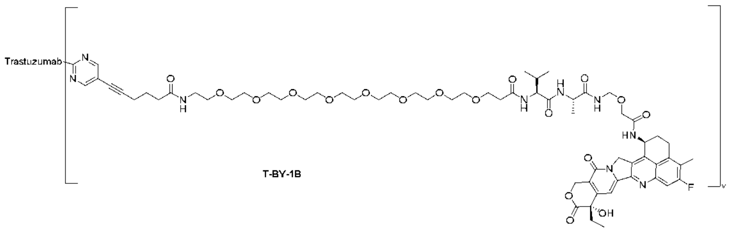
T-BY-1B
,

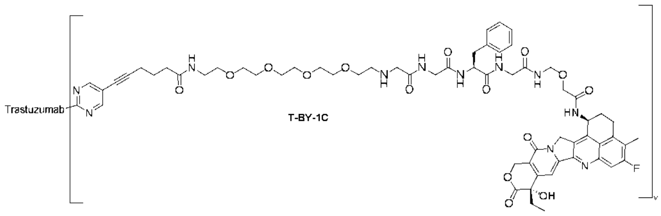
T-BY-1C
,

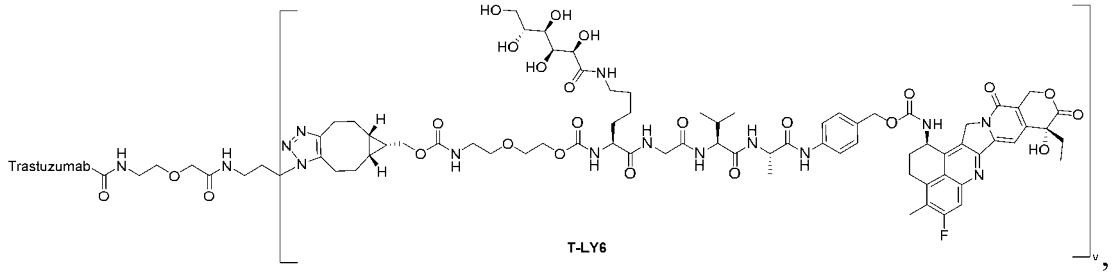

T-LY6 ,

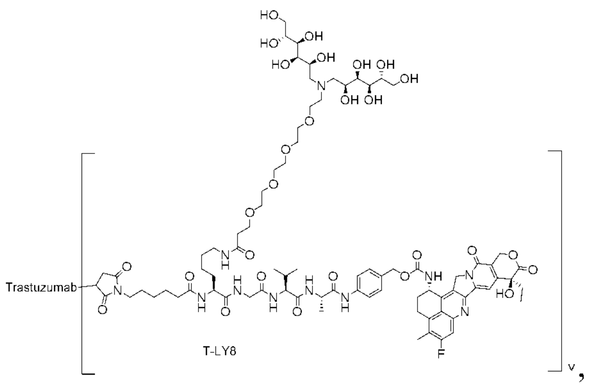

T-LY8 ,

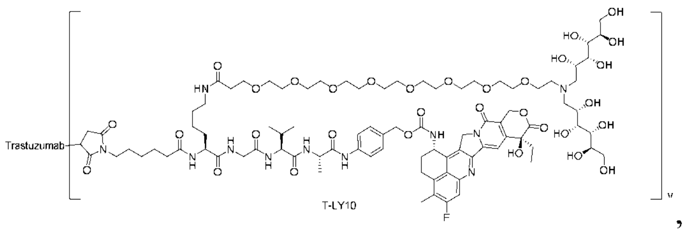

T-LY10 ,

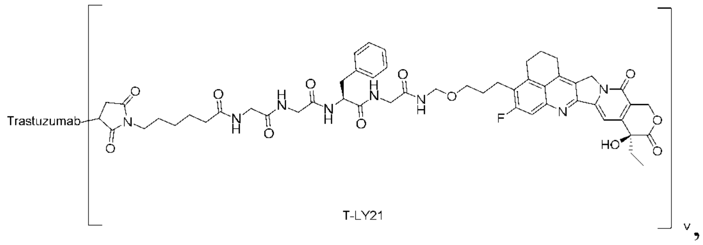

T-LY21 ,

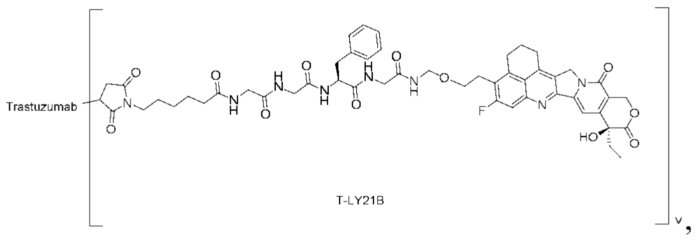

T-LY21B ,

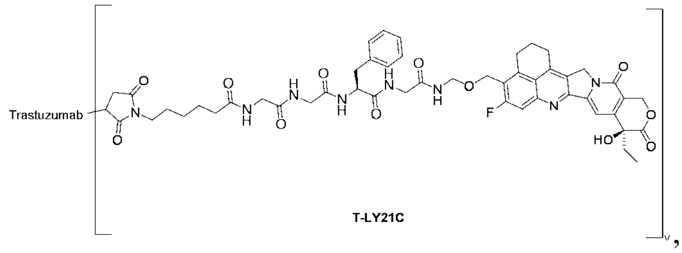

T-LY21C

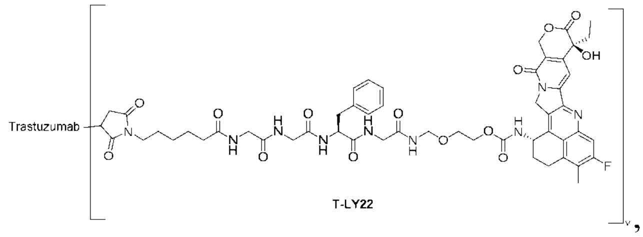

T-LY22

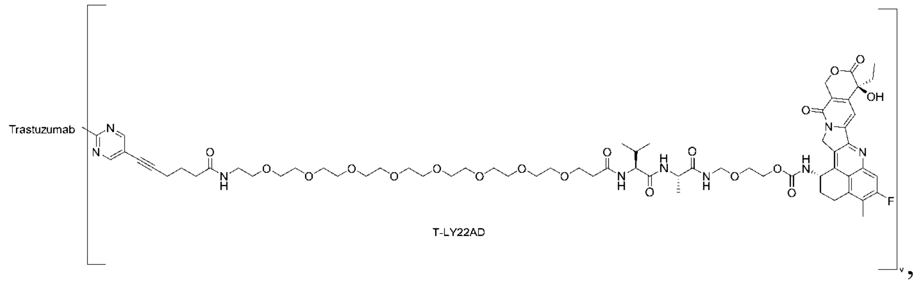

T-LY22AD

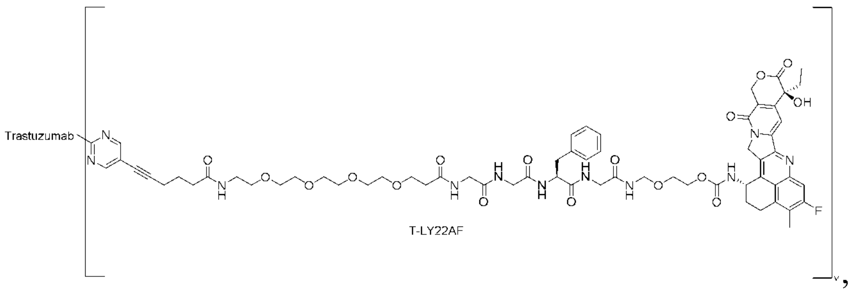

T-LY22AF

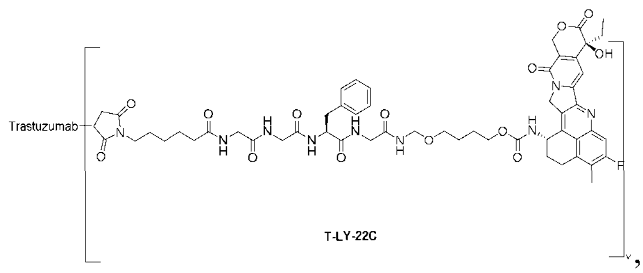

T-LY-22C

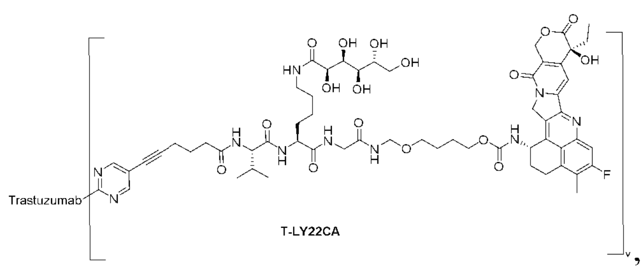

T-LY22CA

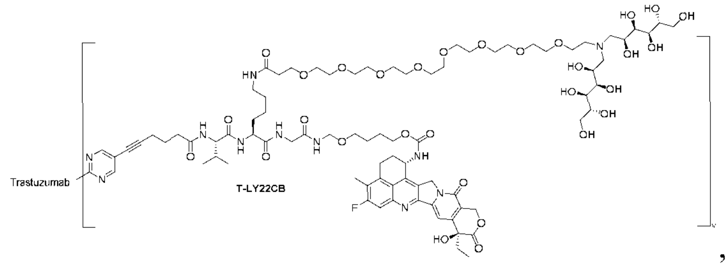

T-LY22CB

,

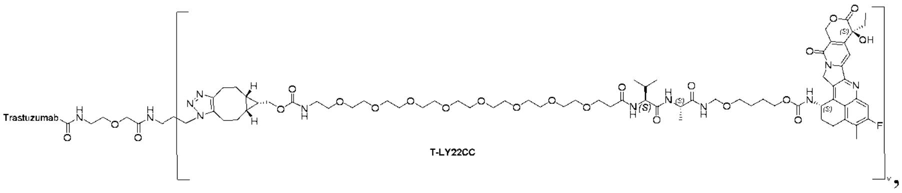

T-LY22CC

,

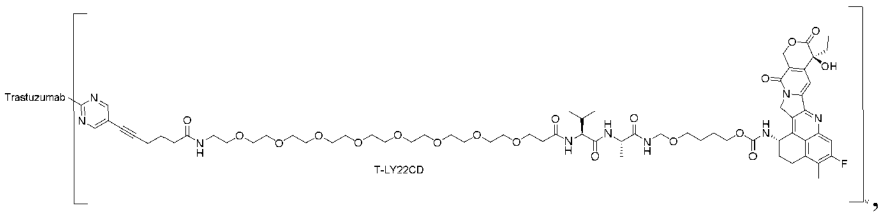

T-LY22CD

,

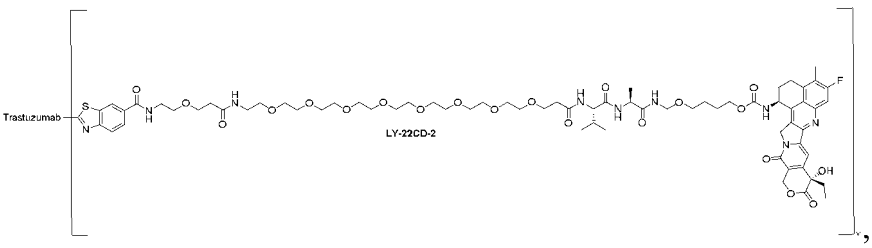

LY-22CD-2

,

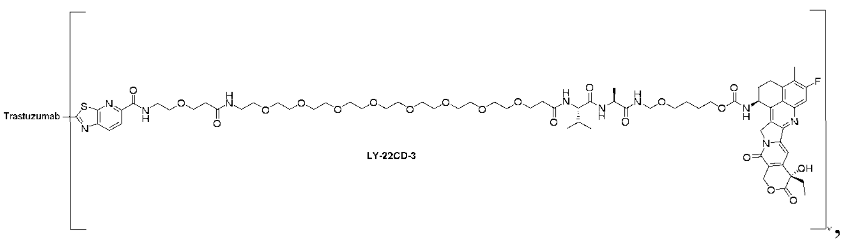

LY-22CD-3

,

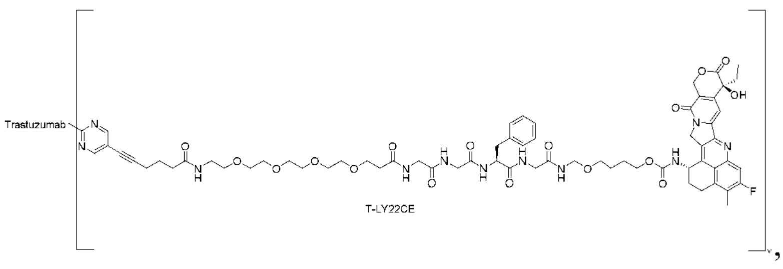

T-LY22CE

,

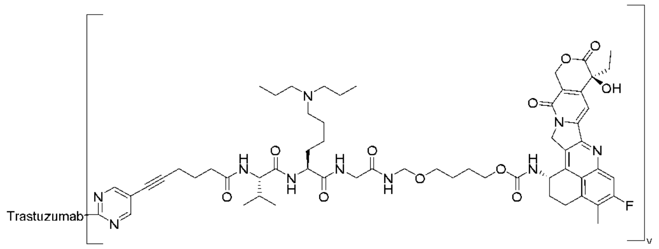

T-LY22CF

,

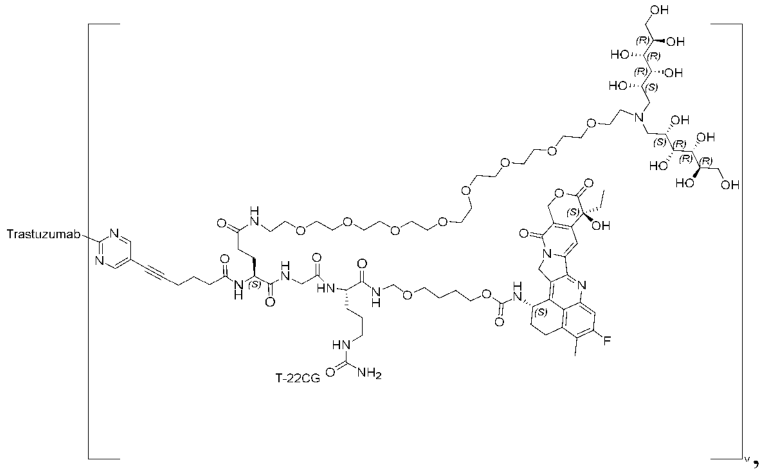

T-22CG

,

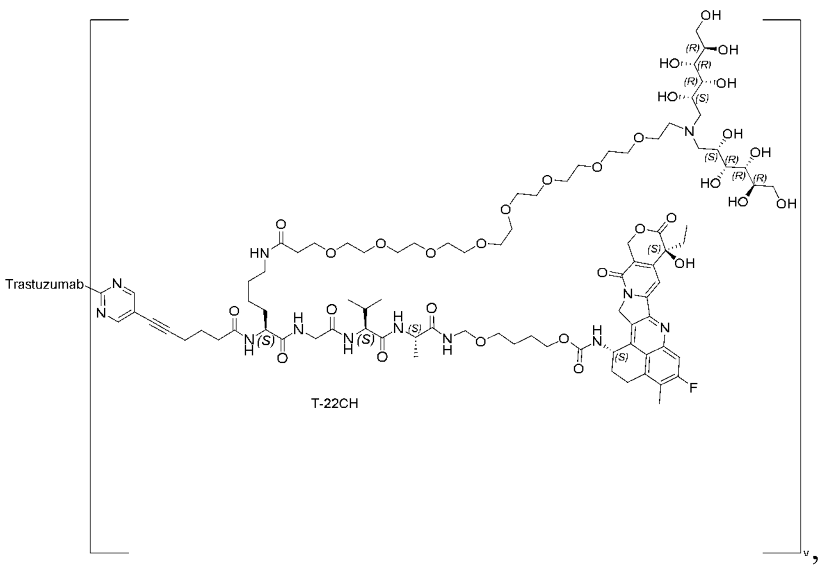

T-22CH

,

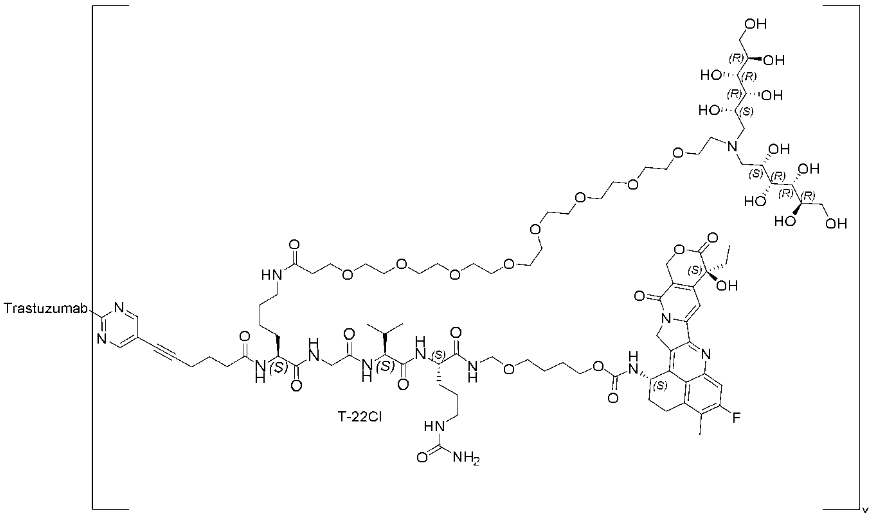

T-22CI

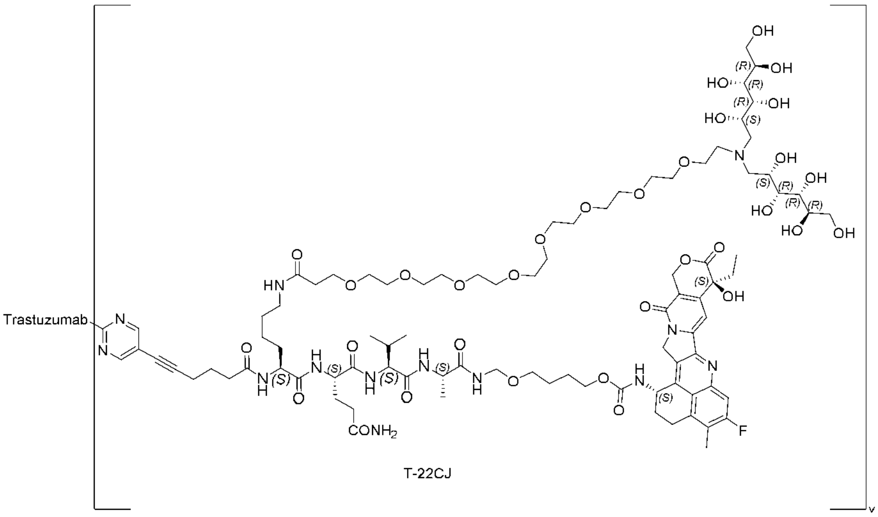

T-22CJ

,

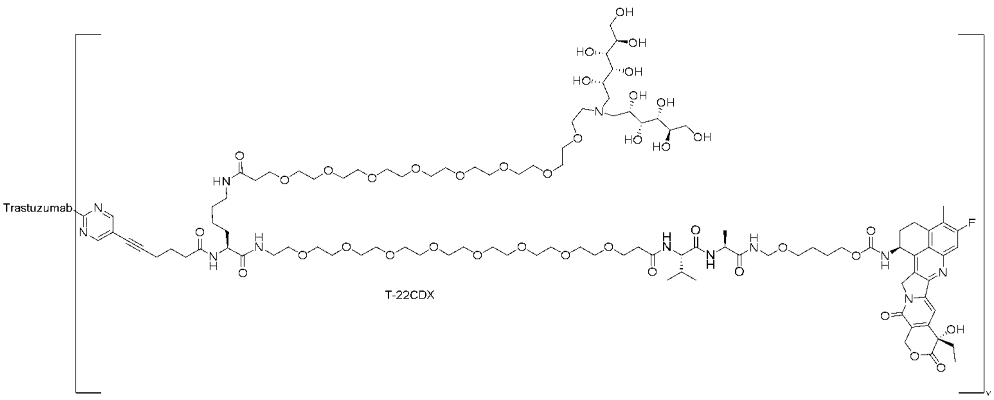

T-22CDX

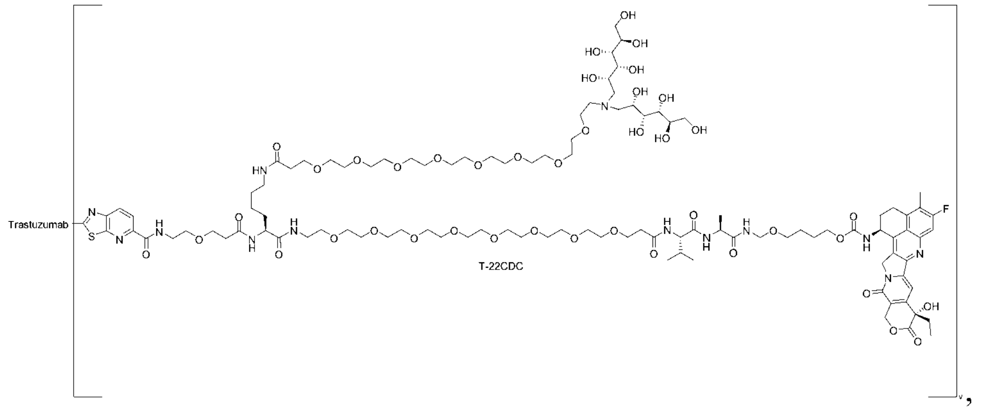

T-22CDC,

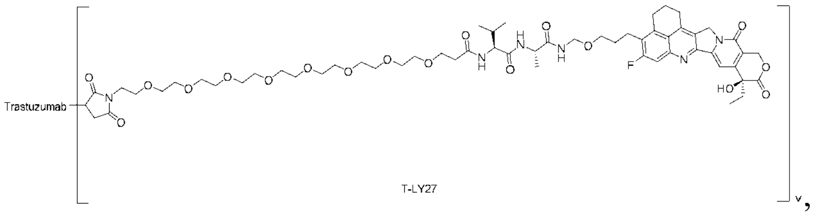

T-LY27,

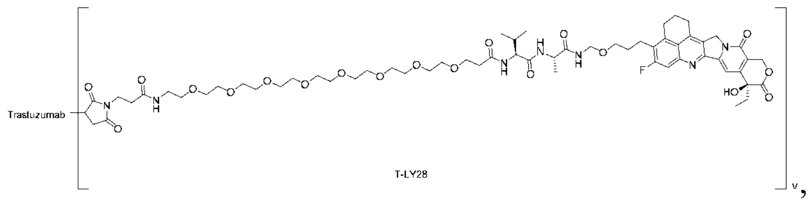

T-LY28,

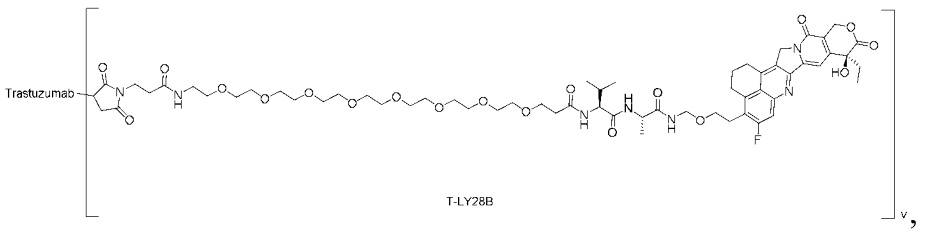

T-LY28B,

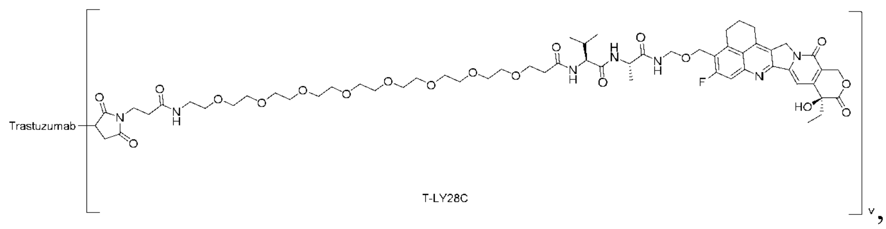

T-LY28C,

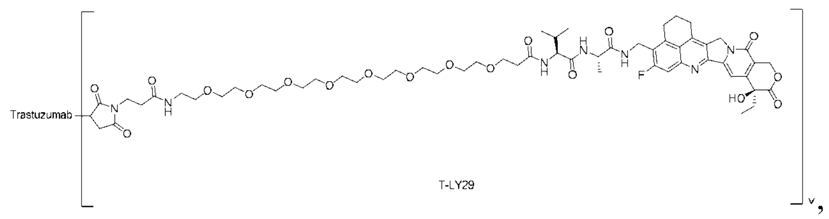

T-LY29,

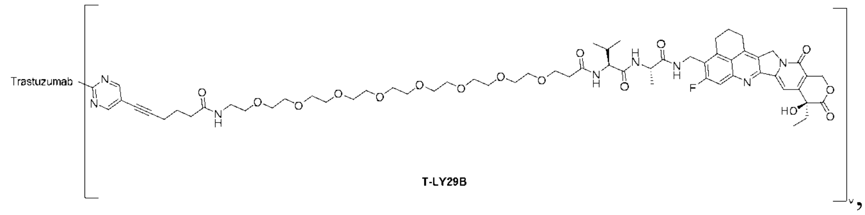

T-LY29B

,

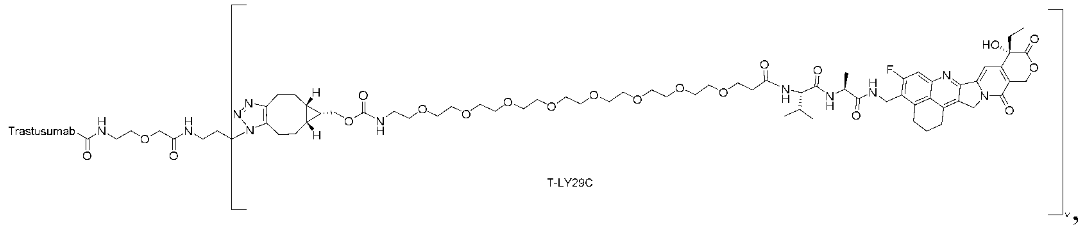

T-LY29C

,

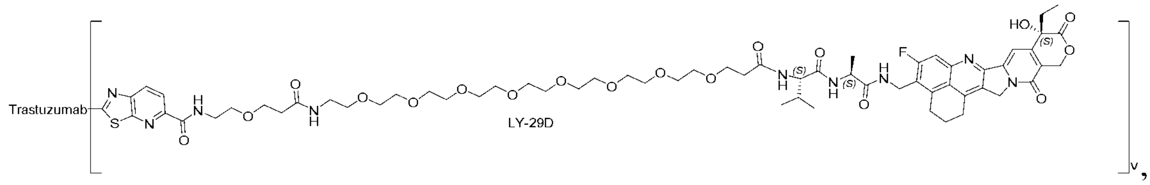

LY-29D

,

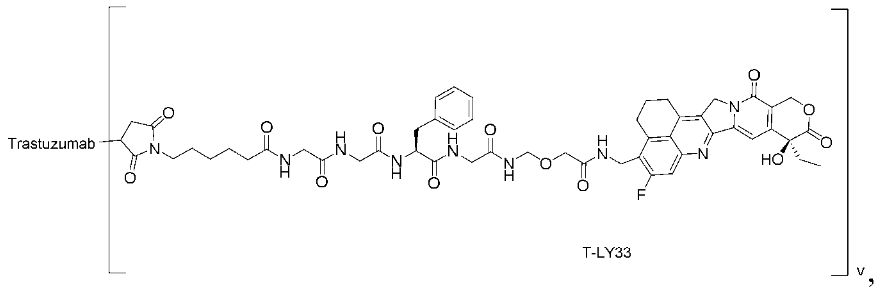

T-LY33

,

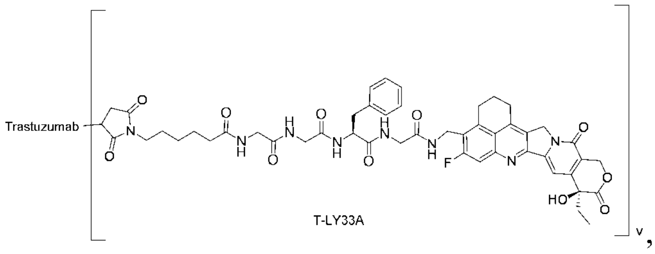

T-LY33A

,

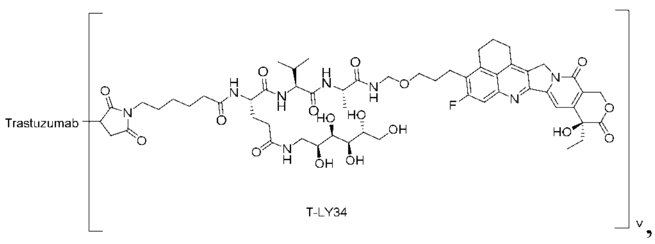

T-LY34

,

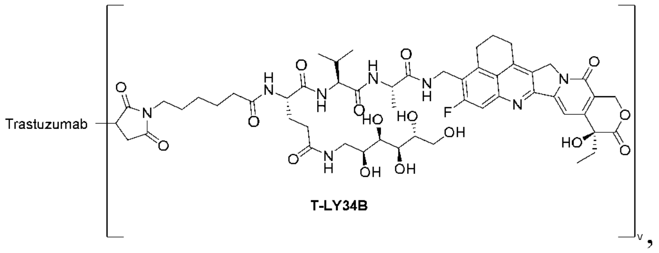

T-LY34B

,

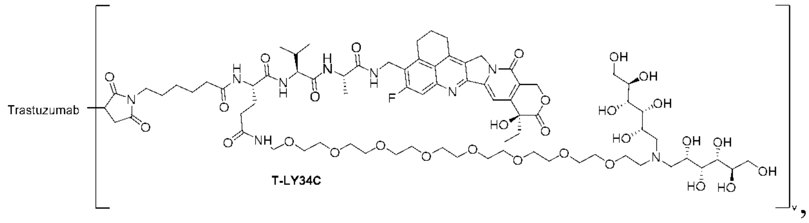

T-LY34C

,

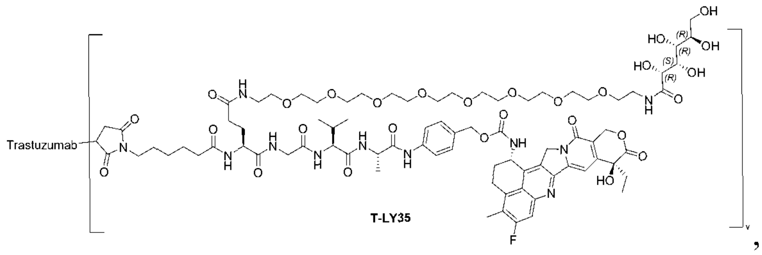

T-LY35

,

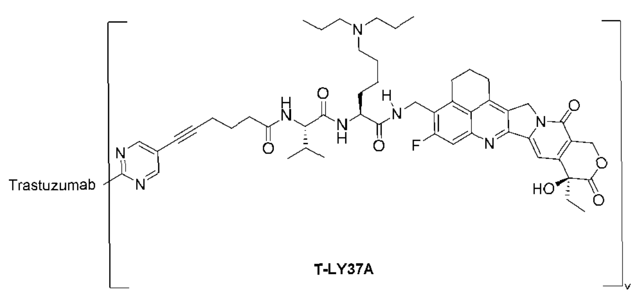

T-LY37A

,

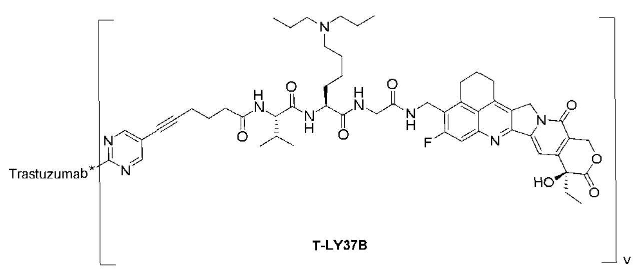

T-LY37B

,

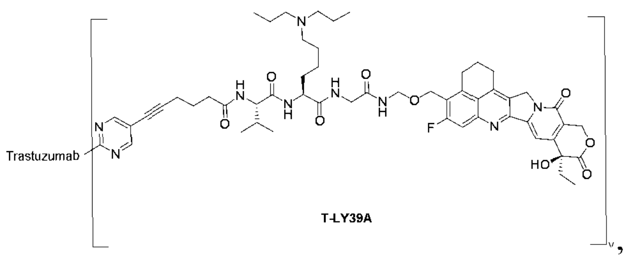

T-LY39A

,

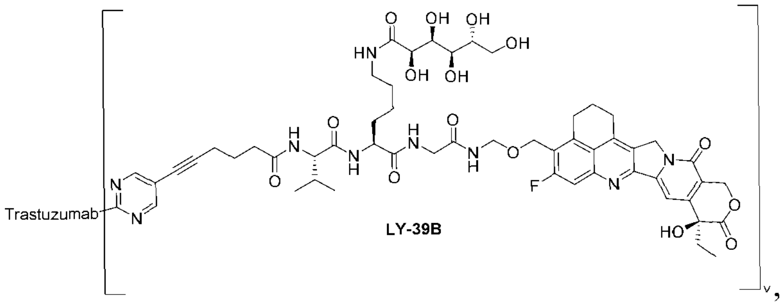

LY-39B

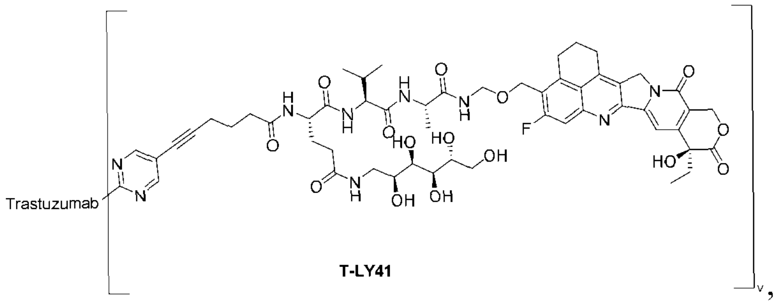

T-LY41

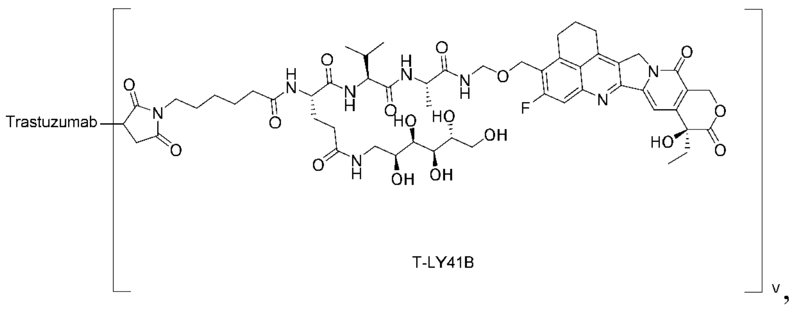

T-LY41B

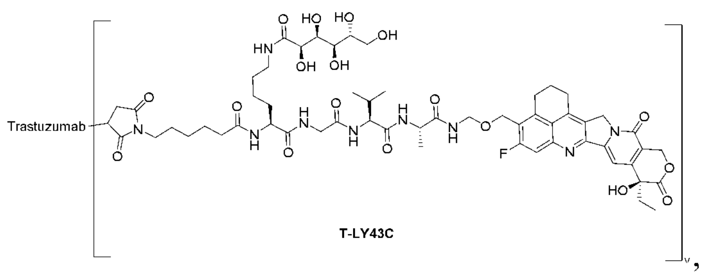

T-LY43C

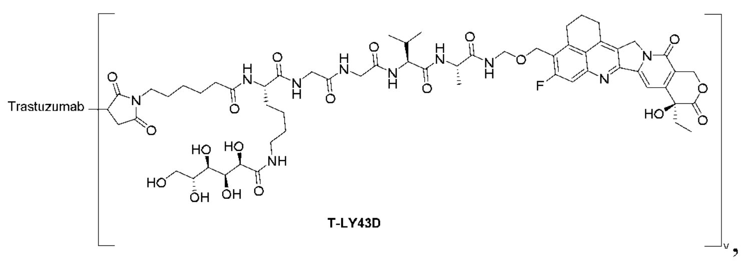

T-LY43D

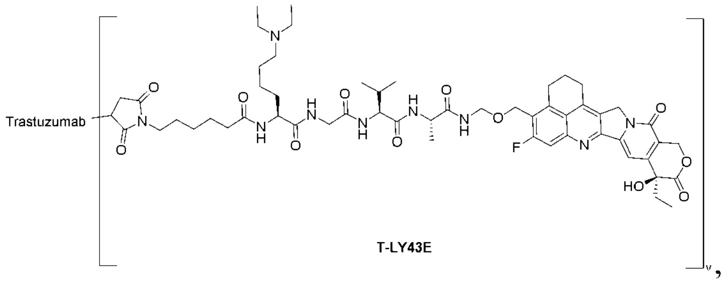

T-LY43E

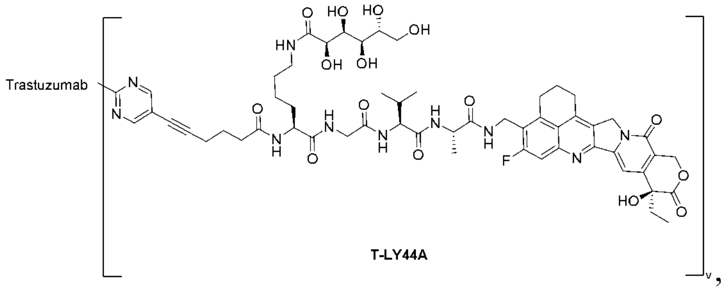

T-LY44A

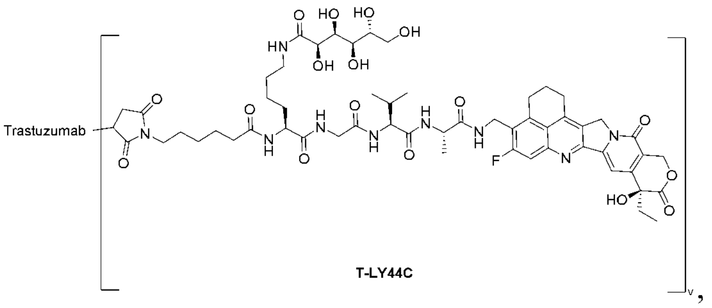

T-LY44C

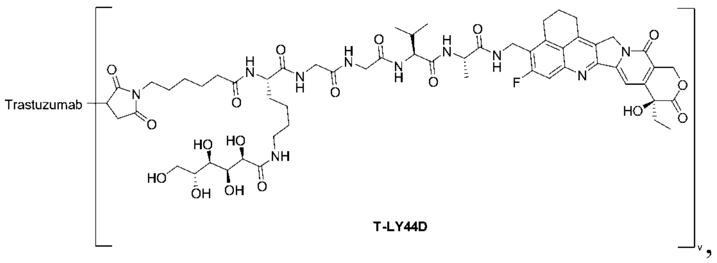

T-LY44D

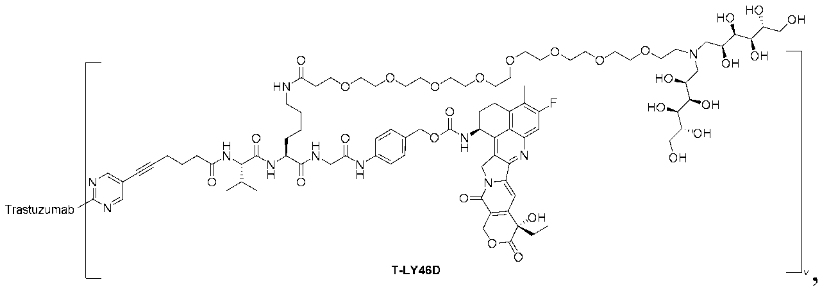

T-LY46D

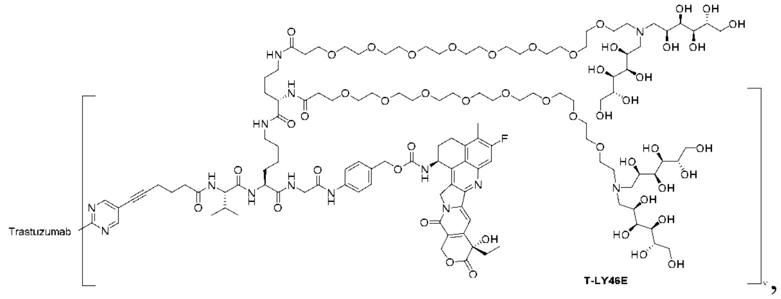

T-LY46E ,

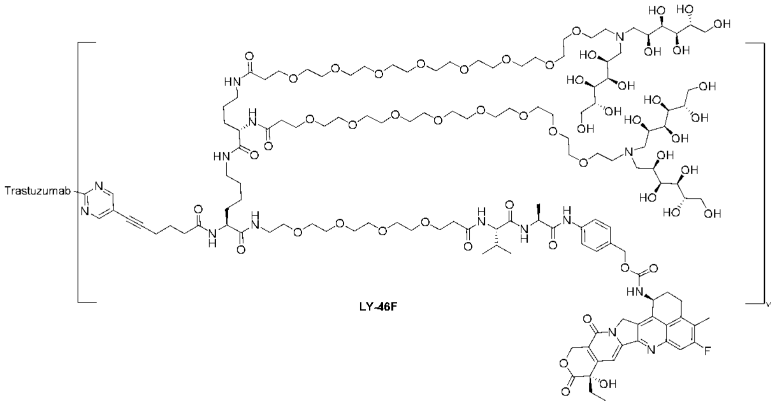

LY-46F ,

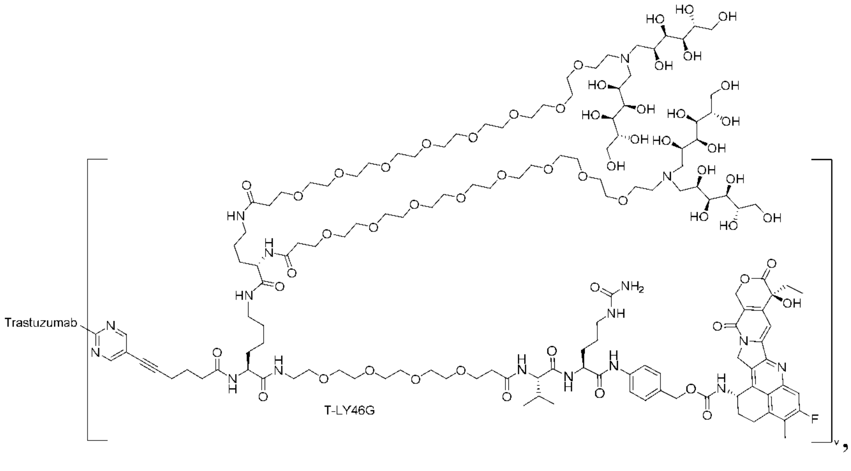

T-LY46G ,

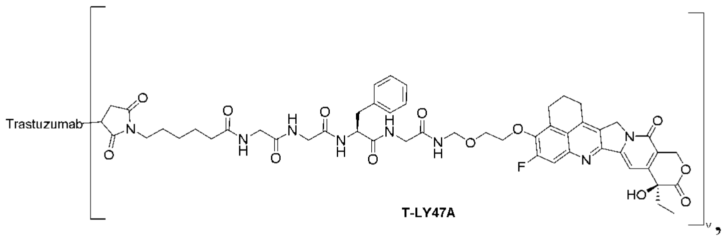

T-LY47A ,

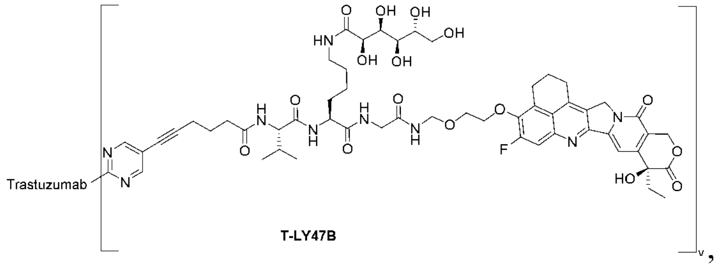

T-LY47B

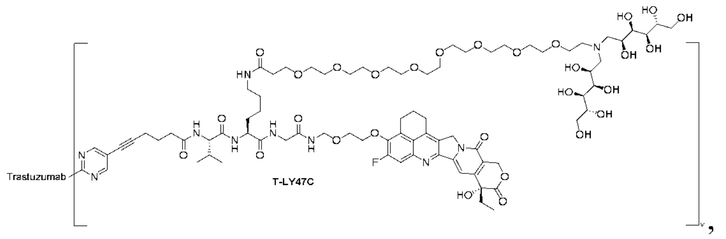

T-LY47C

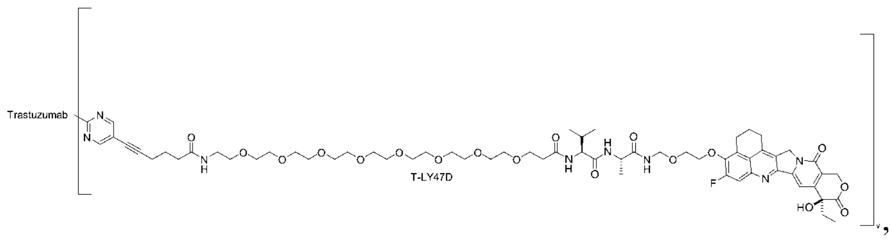

T-LY47D

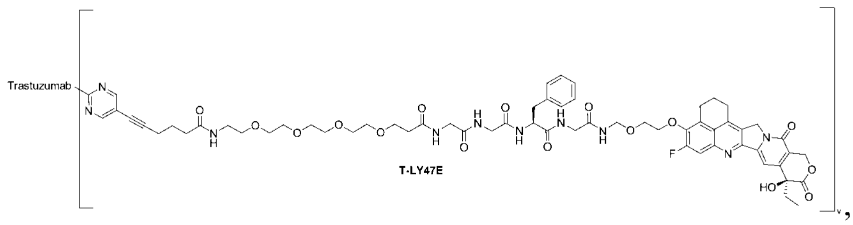

T-LY47E

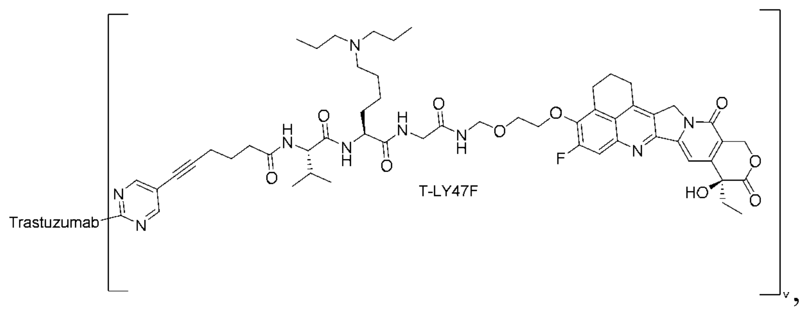

T-LY47F

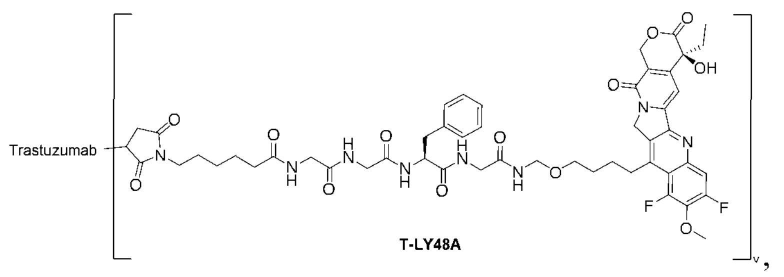

T-LY48A

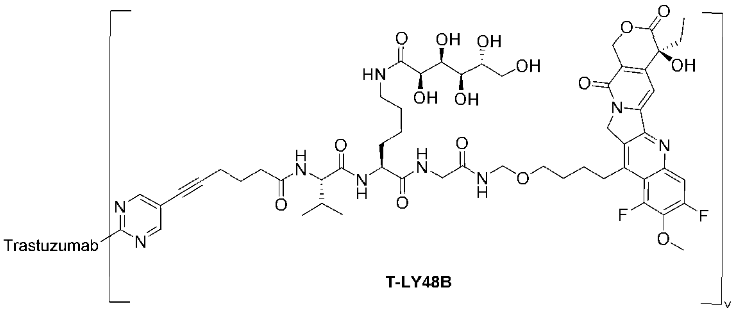

T-LY48B ,

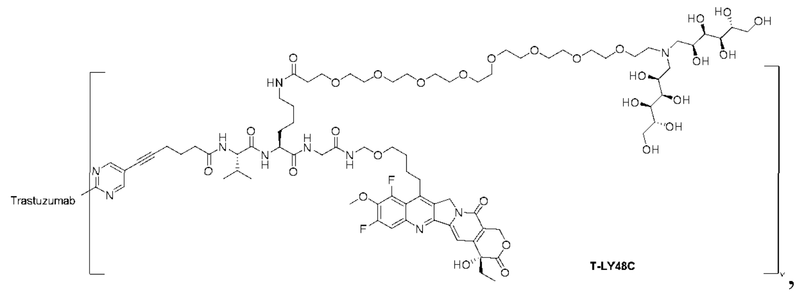

T-LY48C ,

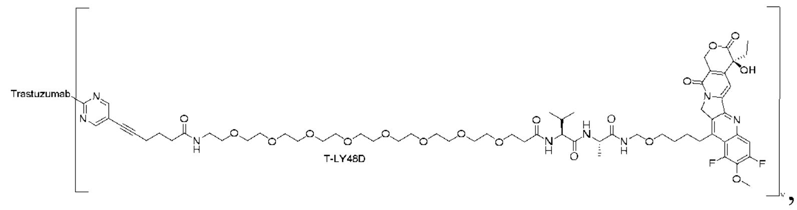

T-LY48D ,

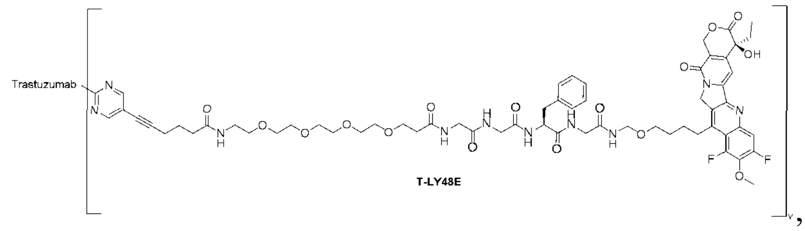

T-LY48E ,

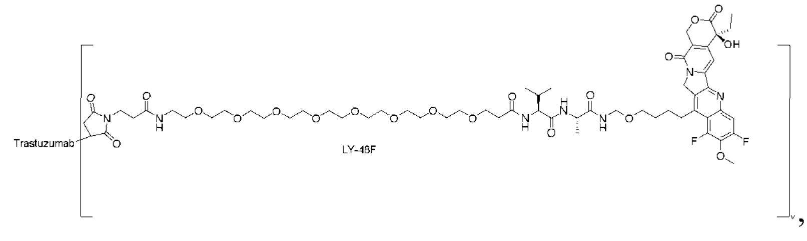

LY-48F ,

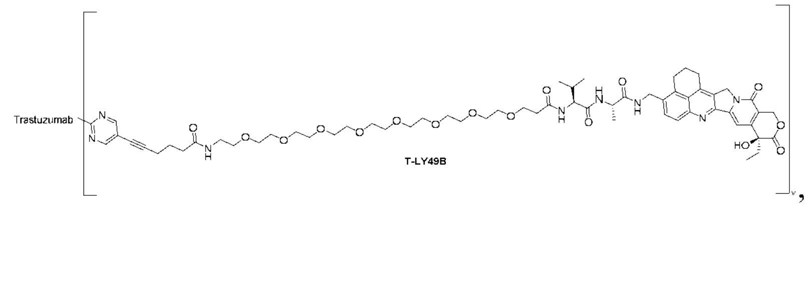

T-LY49B ,

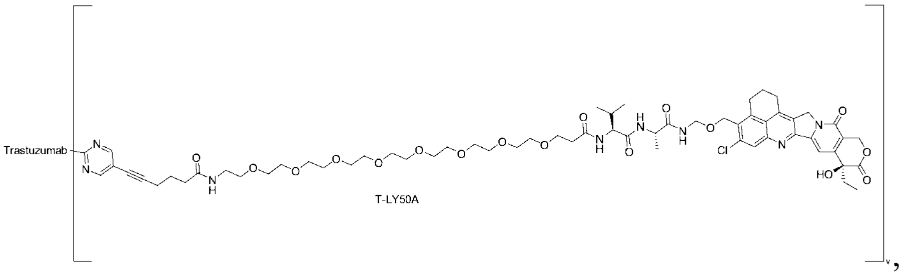

T-LY50A

,

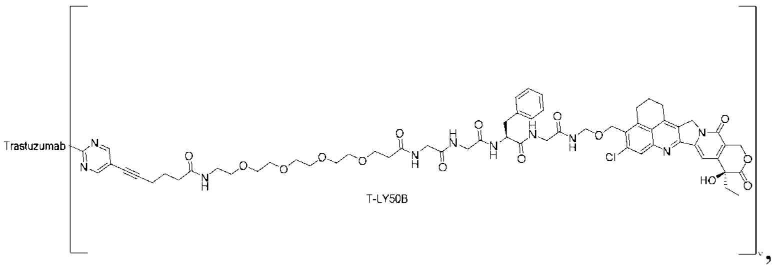

T-LY50B

,

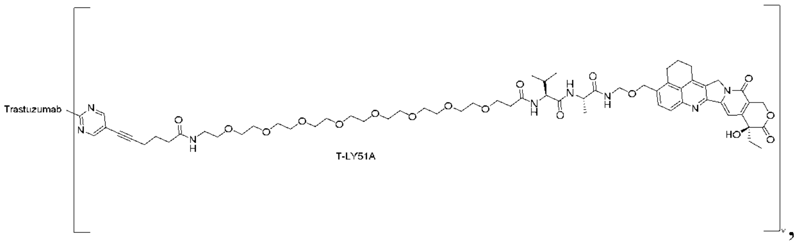

T-LY51A

,

and

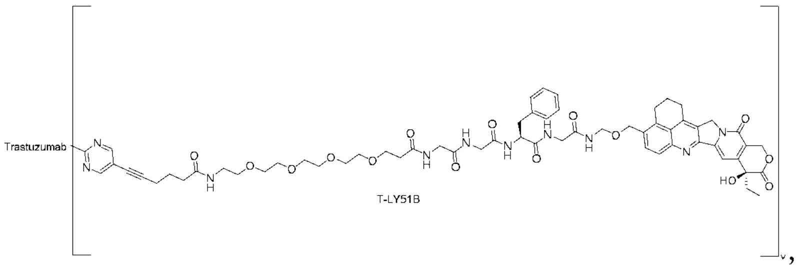

T-LY51B

,

wherein v is an integer or a decimal from 1 to 10; preferably, an integer or a decimal from 2 to 8.

**43.** A pharmaceutical composition, comprising the ligand-drug conjugate represented by the general formula (B) or the pharmaceutically acceptable salt thereof according to any one of claims 32 to 42, and one or more pharmaceutically acceptable carriers or excipients.

**44.** Use of the compound represented by the general formula (A) or the pharmaceutically acceptable salt thereof according to any one of claims 1 to 21, or the compound represented by the general formula (I) or the stereoisomer, tautomer, mesomer, racemate, enantiomer, or diastereomer thereof, or the mixture thereof, or the pharmaceutically acceptable salt thereof according to any one of claims 22 to 26, or the compound represented by the general formula (II) or the stereoisomer, tautomer, mesomer, racemate, enantiomer, or diastereomer thereof, or the mixture thereof, or the pharmaceutically acceptable salt thereof according to any one of claims 27 to 31 in preparation of a ligand-drug conjugate.

**45.** Use of the ligand-drug conjugate represented by the general formula (B) or the pharmaceutically acceptable salt thereof according to any one of claims 32 to 42, or the pharmaceutical composition according to claim 43 in preparation of a medication for treating a tumor or cancer, wherein the cancer is preferably breast cancer, ovarian cancer, soft

tissue sarcoma, liposarcoma, lung cancer, non-small cell lung cancer, gastric cancer, melanoma, head and neck cancer, cervical cancer, or prostate cancer.

**46.** Use of the compound represented by the general formula (I) or the stereoisomer, tautomer, mesomer, racemate, enantiomer, or diastereomer thereof, or the mixture thereof, or the pharmaceutically acceptable salt thereof according to any one of claims 22 to 26, or the compound represented by the general formula (II) or the stereoisomer, tautomer, mesomer, racemate, enantiomer, or diastereomer thereof, or the mixture thereof, or the pharmaceutically acceptable salt thereof according to any one of claims 27 to 31 in preparation of a medication for treating a tumor or cancer, wherein the cancer is preferably breast cancer, ovarian cancer, soft tissue sarcoma, liposarcoma, lung cancer, non-small cell lung cancer, gastric cancer, melanoma, head and neck cancer, cervical cancer, or prostate cancer.

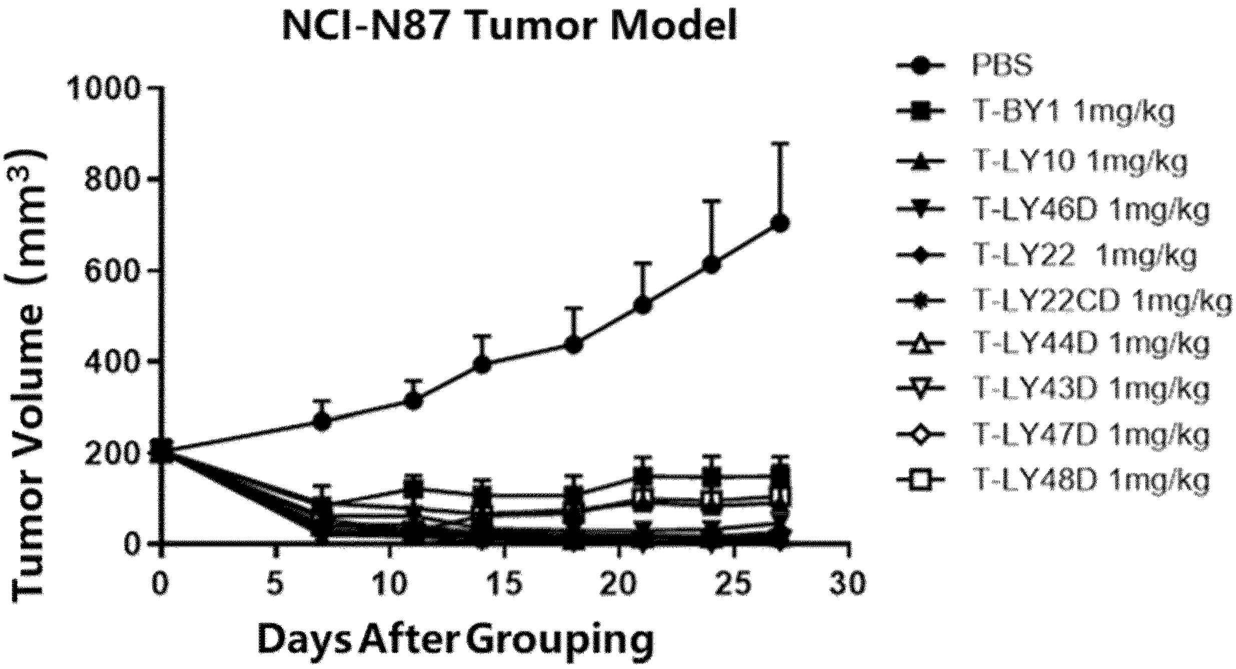
NCI-N87 Tumor Model
Tumor Volume (mm3)
1000
800
600
400
200
0
0
5
10
15
20
25
30
Days After Grouping
PBS
T-BY1 1mg/kg
T-LY10 1mg/kg
T-LY46D 1mg/kg
T-LY22 1mg/kg
T-LY22CD 1mg/kg
T-LY44D 1mg/kg
T-LY43D 1mg/kg
T-LY47D 1mg/kg
T-LY48D 1mg/kg

FIG. 1

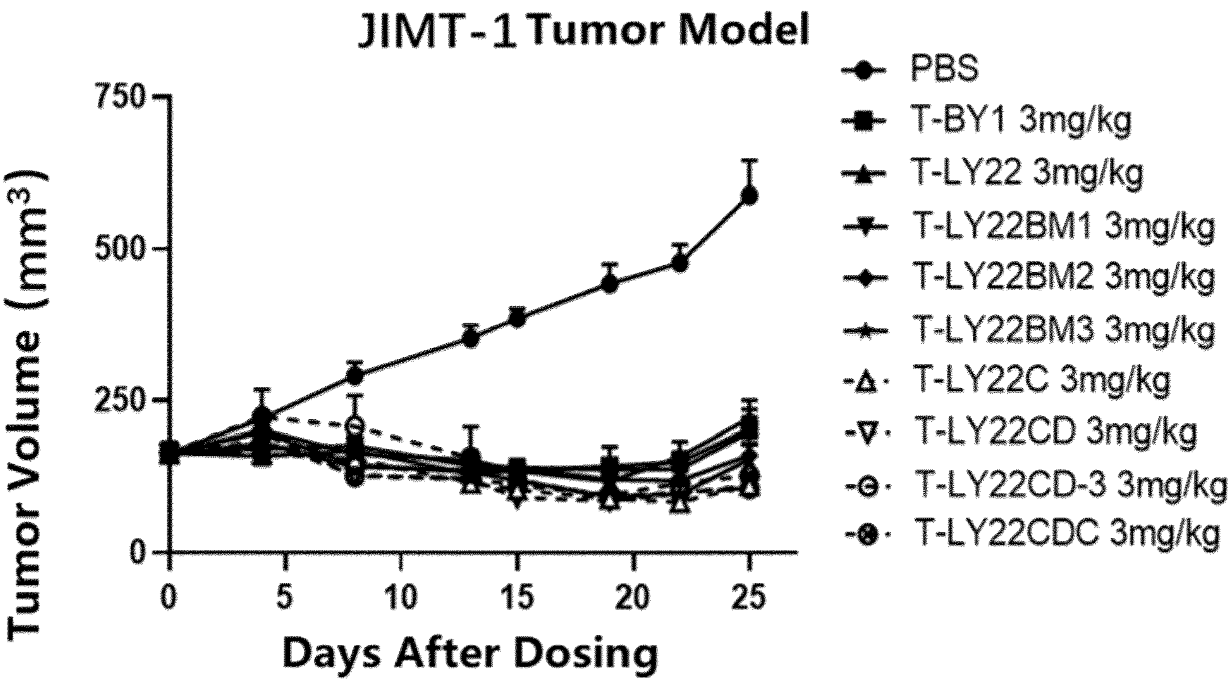
JIMT-1 Tumor Model
Tumor Volume (mm3)
750
500
250
0
0
5
10
15
20
25
Days After Dosing
PBS
T-BY1 3mg/kg
T-LY22 3mg/kg
T-LY22BM1 3mg/kg
T-LY22BM2 3mg/kg
T-LY22BM3 3mg/kg
T-LY22C 3mg/kg
T-LY22CD 3mg/kg
T-LY22CD-3 3mg/kg
T-LY22CDC 3mg/kg

FIG. 2

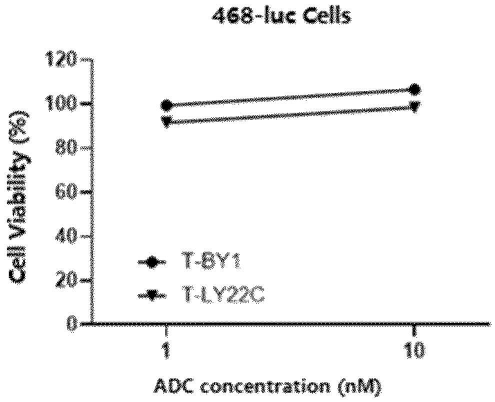


FIG. 3A

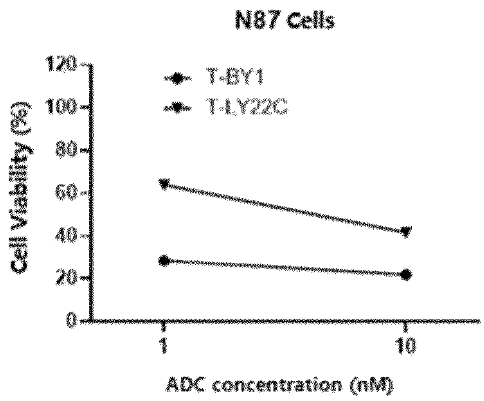


FIG. 3B

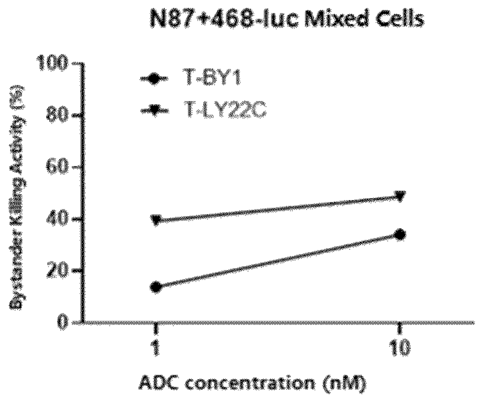


FIG. 3C

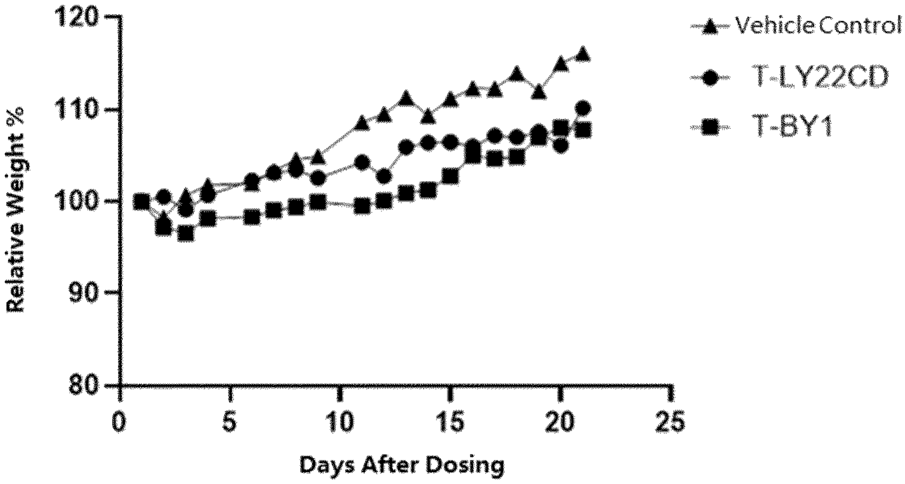


FIG. 4

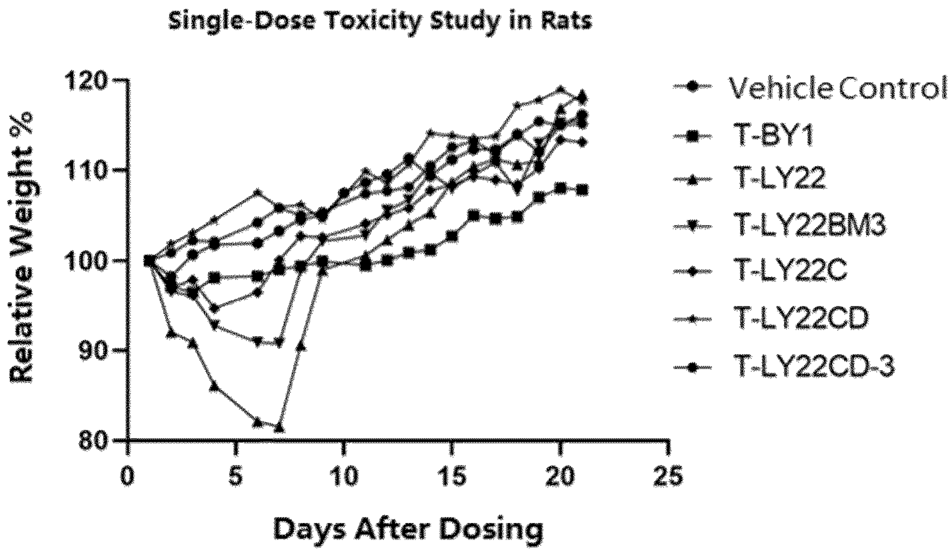


FIG. 5

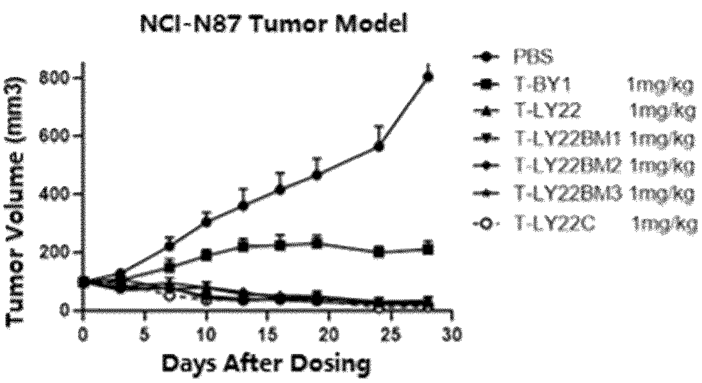


FIG. 6A

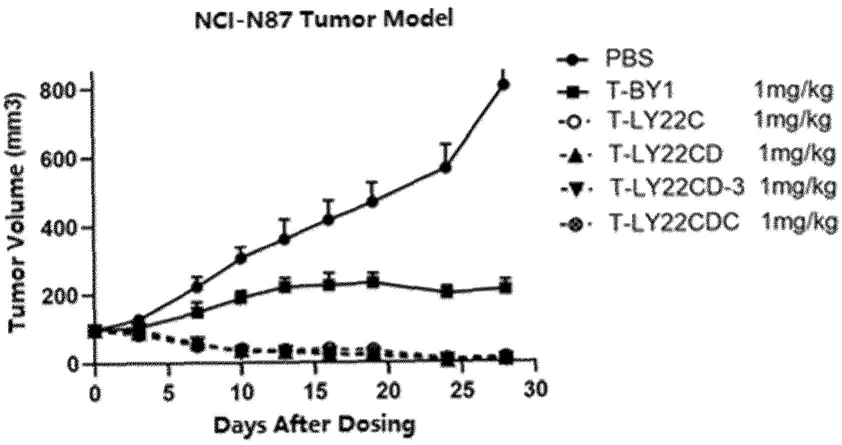
NCI-N87 Tumor Model
Tumor Volume (mm3)
800
600
400
200
0
0
5
10
15
20
25
30
Days After Dosing
PBS
T-BY1 1mg/kg
T-LY22C 1mg/kg
T-LY22CD 1mg/kg
T-LY22CD-3 1mg/kg
T-LY22CDC 1mg/kg

FIG. 6B

**INTERNATIONAL SEARCH REPORT**

International application No.
**PCT/CN2024/078189**

**A. CLASSIFICATION OF SUBJECT MATTER**

C07D491/22(2006.01)i; C07D491/147(2006.01)i; A61K47/68(2017.01)i; A61K47/54(2017.01)i; A61K47/65(2017.01)i; A61K31/4745(2006.01)i; A61K31/437(2006.01)i; A61K39/395(2006.01)i; A61P35/00(2006.01)i; C07K5/062(2006.01)i; C07K5/103(2006.01)i; C07K16/28(2006.01)i

According to International Patent Classification (IPC) or to both national classification and IPC

**B. FIELDS SEARCHED**

Minimum documentation searched (classification system followed by classification symbols)

IPC:C07D,A61K,A61P,C07K

Documentation searched other than minimum documentation to the extent that such documents are included in the fields searched

Electronic data base consulted during the international search (name of data base and, where practicable, search terms used)

CNTXT, VEN, DWPI, WPABS, WPABSC, ENTXT, ENTXTC, WOTXT, USTXT, EPTXT, JPTXT, CNKI, 百度, BAIDU, 百度学术, BAIDU SCHOLAR, ISI-WEB OF SCIENCE, 必应, BING, STN: 拓济医药, 一线医药, phrontline, 陈兆远, 毛彦利, 喜树碱, 连接子, 抗体, 偶联, 缀合, 曲妥珠单抗, 肿瘤, 癌, camptothecin, linker, ligand, antibody, conjugat+, trastuzumab, Her2, ADC, cancer, tumor, 权利要求1-6, 21-22, 26-27, 31, 39, 42中的结构式, HERCEPTIN®

**C. DOCUMENTS CONSIDERED TO BE RELEVANT**

| Category* | Citation of document, with indication, where appropriate, of the relevant passages | Relevant to claim No. |
|---|---|---|
| PX | WO 2023020605 A1 (JIANGSU SIMCERE PHARMACEUTICAL CO., LTD.) 23 February 2023 (2023-02-23)<br>description, embodiments 2-6, 8, 12, and 21-25 | 27-31 |
| PX | WO 2023178289 A2 (SEAGEN INC.) 21 September 2023 (2023-09-21)<br>description, paragraph [0560], and tables 7, 10-11, and 17-18 | 27-31 |
| X | US 2017035906 A1 (DAIICHI SANKYO CO., LTD.) 09 February 2017 (2017-02-09)<br>description, paragraph [0003], and embodiments 29-32, and claims 1-32 | 1-46 |
| X | CN 105829346 A (DAIICHI SANKYO COMPANY, LIMITED) 03 August 2016 (2016-08-03)<br>claims 1-27, and description, embodiments 1-42 | 1-46 |
| X | CN 113766954 A (IMMUNOGEN, INC.) 07 December 2021 (2021-12-07)<br>claims 1-119, and description, tables 1A, 1b, and 2, and embodiments 36 and 46 | 1-46 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **29 April 2024** | **09 May 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/078189**

**C. DOCUMENTS CONSIDERED TO BE RELEVANT**

| Category* | Citation of document, with indication, where appropriate, of the relevant passages | Relevant to claim No. |
|---|---|---|
| X | CN 112351999 A (DAIICHI SANKYO COMPANY, LIMITED) 09 February 2021 (2021-02-09) abstract, claims 1-55, and description, paragraphs [0220] and [0241] | 1-46 |
| X | CN 115052633 A (MEDIMMUNE LIMITED) 13 September 2022 (2022-09-13) description, examples 1-7, and claims 1-26 | 1-46 |
| X | WO 2023004266 A1 (RECURIUM IP HOLDINGS, LLC) 26 January 2023 (2023-01-26) description, embodiments 6, 21, and 23, and claims 1-113 | 1-46 |
| X | WO 2022236136 A1 (ALX ONCOLOGY INC.) 10 November 2022 (2022-11-10) description, tables 1-2, and claims 1-92 | 1-46 |
| X | WO 2022166762 A1 (SICHUAN KELUN-BIOTECH BIOPHARMACEUTICAL CO., LTD.) 11 August 2022 (2022-08-11) description, pages 12-17, and page 127, second-to-last paragraph, and claims 1-14 | 1-46 |
| X | WO 2023280227 A2 (PROFOUNDBIO US CO.) 12 January 2023 (2023-01-12) description, pages 136 and 140-141, and page 338, paragraph [0533], and claims 1-142 | 1-46 |
| X | CN 112533958 A (DAIICHI SANKYO COMPANY, LIMITED) 19 March 2021 (2021-03-19) description, paragraphs [0126] and [0333], and claims 1-51 | 1-46 |
| X | CN 115551552 A (YOUFANG CO., LTD.) 30 December 2022 (2022-12-30) claims 1-132, and description, paragraphs [1034], [1155], and [1171] | 1-46 |
| X | ATSUMI R. et al. "Urinary Metabolites of DX-8951, a Novel Camptothecin Analog, in Rats and Humans" *Arzneimittelforschung Drug Research,* Vol. 51, No. 1, 31 December 2001 (2001-12-31), pages 253-257 | 22-26 |
| X | OGUMA T. et al. "Validation study of assay method for DX-8951 and its metabolite in human plasma and urine by high-performance liquid chromatography/atmospheric pressure chemical ionization tandem mass spectrometry" *BIOMEDICAL CHROMATOGRAPHY.* Vol. 15, No. 2, 20 March 2001 (2001-03-20), pages 108-115 | 22-26 |

**INTERNATIONAL SEARCH REPORT**
**Information on patent family members**

International application No.
**PCT/CN2024/078189**

| Patent document cited in search report | Publication date (day/month/year) | Patent family member(s) | Publication date (day/month/year) |
|---|---|---|---|
| WO 2023020605 A1 | 23 February 2023 | CA 3228345 A1 | 23 February 2023 |
| | | AU 2022330892 A1 | 21 March 2024 |
| | | CN 117769555 A | 26 March 2024 |
| WO 2023178289 A2 | 21 September 2023 | US 2023381321 A1 | 30 November 2023 |
| | | TW 202400137 A | 01 January 2024 |
| | | WO 2023178289 A3 | 14 December 2023 |
| US 2017035906 A1 | 09 February 2017 | ES 2754348 T3 | 17 April 2020 |
| | | US 11185594 B2 | 30 November 2021 |
| | | WO 2015155976 A1 | 15 October 2015 |
| | | JP 2020079237 A | 28 May 2020 |
| | | JP 6861777 B2 | 21 April 2021 |
| | | JPWO 2015155976 A1 | 13 April 2017 |
| | | JP 6612738 B2 | 27 November 2019 |
| | | TW 201620554 A | 16 June 2016 |
| | | EP 3130608 A1 | 15 February 2017 |
| | | EP 3130608 A4 | 13 September 2017 |
| | | EP 3130608 B1 | 04 September 2019 |
| CN 105829346 A | 03 August 2016 | BR 112016013482 A2 | 03 October 2017 |
| | | BR 112016013482 B1 | 19 April 2022 |
| | | KR 20220025176 A | 03 March 2022 |
| | | KR 102417312 B1 | 05 July 2022 |
| | | JP 2022180504 A | 06 December 2022 |
| | | KR 20210088745 A | 14 July 2021 |
| | | KR 102314913 B1 | 19 October 2021 |
| | | IL 278891 A | 31 January 2021 |
| | | IL 278891 B1 | 01 February 2024 |
| | | KR 20230162159 A | 28 November 2023 |
| | | AU 2022203818 A1 | 23 June 2022 |
| | | PH 12016500904 A1 | 11 July 2016 |
| | | TW 201834692 A | 01 October 2018 |
| | | TWI 661839 B | 11 June 2019 |
| | | AU 2020200596 A1 | 20 February 2020 |
| | | AU 2020200596 B2 | 03 March 2022 |
| | | HRP 20190431 T1 | 19 April 2019 |
| | | SG 10201800210 TA | 27 February 2018 |
| | | EP 3466976 A1 | 10 April 2019 |
| | | EP 3466976 B1 | 08 September 2021 |
| | | LTPA 2021515 I1 | 10 August 2021 |
| | | LTC 3101032 I2 | 25 October 2022 |
| | | RU 2016123597 A | 02 March 2018 |
| | | RU 2016123597 A3 | 12 October 2018 |
| | | RU 2683780 C2 | 02 April 2019 |
| | | IL 310627 A | 01 April 2024 |
| | | ES 2895254 T3 | 18 February 2022 |
| | | CY 1121573 T1 | 29 May 2020 |
| | | JP 2019112403 A | 11 July 2019 |
| | | JP 6665325 B2 | 13 March 2020 |
| | | NL 301117 I1 | 19 July 2021 |
| | | NL 301117 I2 | 29 July 2021 |
| | | LT 3101032 T | 25 March 2019 |

Form PCT/ISA/210 (patent family annex) (July 2022)

**INTERNATIONAL SEARCH REPORT**
**Information on patent family members**

International application No.
**PCT/CN2024/078189**

| Patent document cited in search report | Publication date (day/month/year) | Patent family member(s) | | | Publication date (day/month/year) |
|---|---|---|---|---|---|
| | | EP | 3101032 | A1 | 07 December 2016 |
| | | EP | 3101032 | A4 | 06 September 2017 |
| | | EP | 3101032 | B1 | 16 January 2019 |
| | | TW | 202237113 | A | 01 October 2022 |
| | | TWI | 796243 | B | 11 March 2023 |
| | | US | 2024026030 | A1 | 25 January 2024 |
| | | TW | 202322817 | A | 16 June 2023 |
| | | TW | 202237191 | A | 01 October 2022 |
| | | TWI | 796245 | B | 11 March 2023 |
| | | US | 2020385486 | A1 | 10 December 2020 |
| | | US | 11584800 | B2 | 21 February 2023 |
| | | KR | 20210127803 | A | 22 October 2021 |
| | | KR | 102362920 | B1 | 14 February 2022 |
| | | HUE | 057464 | T2 | 28 May 2022 |
| | | MX | 2016006498 | A | 04 August 2016 |
| | | RS | 58415 | B1 | 30 April 2019 |
| | | JP | 2020097617 | A | 25 June 2020 |
| | | JP | 6914380 | B2 | 04 August 2021 |
| | | JP | 2021169493 | A | 28 October 2021 |
| | | JP | 7146031 | B2 | 03 October 2022 |
| | | EP | 4212552 | A1 | 19 July 2023 |
| | | EP | 3973995 | A1 | 30 March 2022 |
| | | RU | 2019107788 | A | 17 May 2019 |
| | | SI | 3101032 | T1 | 28 February 2019 |
| | | FR | 2C1030 | I1 | 27 August 2021 |
| | | FR | 2C1030 | I2 | 08 April 2022 |
| | | HUE | 044389 | T2 | 28 October 2019 |
| | | RS | 62618 | B1 | 31 December 2021 |
| | | LT | 3466976 | T | 10 November 2021 |
| | | TW | 202120088 | A | 01 June 2021 |
| | | TWI | 789700 | B | 11 January 2023 |
| | | WO | 2015115091 | A1 | 06 August 2015 |
| | | US | 2016333112 | A1 | 17 November 2016 |
| | | US | 10155821 | B2 | 18 December 2018 |
| | | PT | 3466976 | T | 27 October 2021 |
| | | ES | 2727351 | T3 | 15 October 2019 |
| | | KR | 20210040181 | A | 12 April 2021 |
| | | KR | 102275925 | B1 | 12 July 2021 |
| | | TW | 201934145 | A | 01 September 2019 |
| | | TWI | 722422 | B | 21 March 2021 |
| | | MX | 2020010682 | A | 06 November 2020 |
| | | KR | 20220154251 | A | 21 November 2022 |
| | | KR | 102510128 | B1 | 14 March 2023 |
| | | JP | 6105183 | B1 | 29 March 2017 |
| | | JP | 2017105789 | A | 15 June 2017 |
| | | SI | 3466976 | T1 | 31 December 2021 |
| | | PT | 3101032 | T | 03 April 2019 |
| | | JP | 2017095457 | A | 01 June 2017 |
| | | JP | 6466895 | B2 | 06 February 2019 |
| | | NO | 2021027 | I1 | 06 July 2021 |

Form PCT/ISA/210 (patent family annex) (July 2022)

**INTERNATIONAL SEARCH REPORT**
**Information on patent family members**

International application No.
**PCT/CN2024/078189**

| Patent document cited in search report | Publication date (day/month/year) | Patent family member(s) | Publication date (day/month/year) |
|---|---|---|---|
| | | KR 20180122038 A | 09 November 2018 |
| | | KR 102190548 B1 | 14 December 2020 |
| | | HUS 2100025 I1 | 28 July 2021 |
| | | ZA 201602802 B | 27 September 2017 |
| | | HRP 20211848 T1 | 04 March 2022 |
| | | KR 20230113822 A | 01 August 2023 |
| | | KR 102606930 B1 | 29 November 2023 |
| | | TW 201613649 A | 16 April 2016 |
| | | TWI 627967 B | 01 July 2018 |
| | | KR 20230042126 A | 27 March 2023 |
| | | KR 102557062 B1 | 18 July 2023 |
| | | IL 245252 A0 | 30 June 2016 |
| | | IL 245252 B | 30 June 2019 |
| | | KR 20220100994 A | 18 July 2022 |
| | | KR 102465042 B1 | 09 November 2022 |
| | | CY 1124774 T1 | 25 November 2022 |
| | | NZ 719202 A | 25 February 2022 |
| | | JP 6046301 B1 | 14 December 2016 |
| | | JP 2017036285 A | 16 February 2017 |
| | | KR 20200140932 A | 16 December 2020 |
| | | KR 102238848 B1 | 09 April 2021 |
| | | IL 266790 A | 31 July 2019 |
| | | DK 3101032 T3 | 29 April 2019 |
| | | TW 202237114 A | 01 October 2022 |
| | | TWI 796244 B | 11 March 2023 |
| | | JP 5998289 B2 | 28 September 2016 |
| | | JPWO 2015115091 A1 | 23 March 2017 |
| | | PL 3101032 T3 | 31 July 2019 |
| | | CA 2928794 A1 | 06 August 2015 |
| | | CA 2928794 C | 13 August 2019 |
| | | KR 20160113099 A | 28 September 2016 |
| | | KR 101916553 B1 | 07 November 2018 |
| | | TW 202237115 A | 01 October 2022 |
| | | TWI 796246 B | 11 March 2023 |
| | | US 2019077880 A1 | 14 March 2019 |
| | | US 11795236 B2 | 24 October 2023 |
| | | AU 2015212265 B2 | 02 January 2020 |
| | | SG 11201603960 XA | 28 July 2016 |
| | | DK 3466976 T3 | 13 December 2021 |
| | | PL 3466976 T3 | 20 December 2021 |
| CN 113766954 A | 07 December 2021 | EP 4316524 A2 | 07 February 2024 |
| | | TW 202106691 A | 16 February 2021 |
| | | DK 3958977 T3 | 11 December 2023 |
| | | PL 3958977 T3 | 11 March 2024 |
| | | AU 2020263231 A1 | 18 November 2021 |
| | | JP 2022529854 A | 24 June 2022 |
| | | PT 3958977 T | 15 December 2023 |
| | | FI 3958977 T3 | 12 December 2023 |
| | | KR 20220027828 A | 08 March 2022 |
| | | US 2021077482 A1 | 18 March 2021 |

Form PCT/ISA/210 (patent family annex) (July 2022)

**INTERNATIONAL SEARCH REPORT**
**Information on patent family members**

International application No.
**PCT/CN2024/078189**

| Patent document cited in search report | Publication date (day/month/year) | Patent family member(s) | Publication date (day/month/year) |
|---|---|---|---|
| | | US 11229639 B2 | 25 January 2022 |
| | | CA 3137125 A1 | 29 October 2020 |
| | | LT 3958977 T | 27 December 2023 |
| | | SG 11202110922 QA | 28 October 2021 |
| | | RS 64961 B1 | 31 January 2024 |
| | | WO 2020219287 A1 | 29 October 2020 |
| | | US 2022133711 A1 | 05 May 2022 |
| | | EP 3958977 A1 | 02 March 2022 |
| | | EP 3958977 B1 | 13 September 2023 |
| | | IL 286846 A | 31 October 2021 |
| CN 112351999 A | 09 February 2021 | PH 12020551969 A1 | 20 September 2021 |
| | | US 2023139769 A1 | 04 May 2023 |
| | | US 11872289 B2 | 16 January 2024 |
| | | WO 2019219889 A1 | 21 November 2019 |
| | | TW 202003583 A | 16 January 2020 |
| | | TWI 825098 B | 11 December 2023 |
| | | JP 2023027259 A | 01 March 2023 |
| | | EP 4257611 A2 | 11 October 2023 |
| | | EP 4257611 A3 | 06 March 2024 |
| | | SG 11202010493 XA | 27 November 2020 |
| | | PL 3794041 T3 | 28 August 2023 |
| | | HRP 20230787 T1 | 27 October 2023 |
| | | KR 20210010565 A | 27 January 2021 |
| | | US 2021221910 A1 | 22 July 2021 |
| | | CA 3100745 A1 | 21 November 2019 |
| | | EP 3794042 A1 | 24 March 2021 |
| | | EP 3794042 B1 | 27 March 2024 |
| | | MX 2020011997 A | 25 March 2021 |
| | | BR 112020023373 A2 | 09 February 2021 |
| | | EP 4249002 A2 | 27 September 2023 |
| | | EP 4249002 A3 | 22 November 2023 |
| | | SG 11202010496 WA | 30 December 2020 |
| | | PH 12020551970 A1 | 13 September 2021 |
| | | HUE 063066 T2 | 28 January 2024 |
| | | JP 2021524740 A | 16 September 2021 |
| | | US 2023372520 A1 | 23 November 2023 |
| | | BR 112020023346 A2 | 09 February 2021 |
| | | TW 202402806 A | 16 January 2024 |
| | | ZA 202006684 B | 29 September 2021 |
| | | CA 3100608 A1 | 21 November 2019 |
| | | AU 2019270459 A1 | 03 December 2020 |
| | | AU 2019270457 A1 | 03 December 2020 |
| | | ES 2951674 T3 | 24 October 2023 |
| | | TW 202003579 A | 16 January 2020 |
| | | CO 2020013664 A2 | 21 December 2020 |
| | | EP 3794041 A1 | 24 March 2021 |
| | | EP 3794041 C0 | 12 July 2023 |
| | | RS 64379 B1 | 31 August 2023 |
| | | CO 2020014435 A2 | 21 December 2020 |
| | | US 2021187118 A1 | 24 June 2021 |

Form PCT/ISA/210 (patent family annex) (July 2022)

**INTERNATIONAL SEARCH REPORT**
**Information on patent family members**

International application No.
**PCT/CN2024/078189**

| Patent document cited in search report | Publication date (day/month/year) | Patent family member(s) | Publication date (day/month/year) |
|---|---|---|---|
| | | MX 2020011996 A | 29 January 2021 |
| | | KR 20210013107 A | 03 February 2021 |
| | | WO 2019219891 A1 | 21 November 2019 |
| | | JP 2021524852 A | 16 September 2021 |
| | | JP 7257422 B2 | 13 April 2023 |
| CN 115052633 A | 13 September 2022 | TW 202140076 A | 01 November 2021 |
| | | KR 20220130191 A | 26 September 2022 |
| | | IL 294645 A | 01 September 2022 |
| | | BR 112022013966 A2 | 11 October 2022 |
| | | CO 2022011178 A2 | 30 August 2022 |
| | | WO 2021148501 A1 | 29 July 2021 |
| | | MX 2022008997 A | 15 August 2022 |
| | | AU 2021211892 A1 | 08 September 2022 |
| | | US 2023111996 A1 | 13 April 2023 |
| | | JP 2023512501 A | 27 March 2023 |
| | | CA 3167373 A1 | 29 July 2021 |
| | | ECSP 22064855 A | 30 September 2022 |
| | | CR 20220393 A | 16 September 2022 |
| | | EP 4093438 A1 | 30 November 2022 |
| WO 2023004266 A1 | 26 January 2023 | CA 3224492 A1 | 26 January 2023 |
| | | US 2023123041 A1 | 20 April 2023 |
| | | TW 202320858 A | 01 June 2023 |
| | | AU 2022315277 A1 | 18 January 2024 |
| | | CN 117715913 A | 15 March 2024 |
| WO 2022236136 A1 | 10 November 2022 | CA 3219236 A1 | 10 November 2022 |
| | | TW 202309042 A | 01 March 2023 |
| | | AU 2022269073 A1 | 23 November 2023 |
| | | EP 4334322 A1 | 13 March 2024 |
| WO 2022166762 A1 | 11 August 2022 | CA 3209426 A1 | 11 August 2022 |
| | | AU 2022216696 A1 | 17 August 2023 |
| | | BR 112023014914 A2 | 29 August 2023 |
| | | CN 116829561 A | 29 September 2023 |
| | | KR 20230142710 A | 11 October 2023 |
| | | IN 202317049950 A | 08 December 2023 |
| | | EP 4289851 A1 | 13 December 2023 |
| | | JP 2024506819 W | 15 February 2024 |
| | | CN 117567478 A | 20 February 2024 |
| | | VN 100933 A | 26 February 2024 |
| WO 2023280227 A2 | 12 January 2023 | CA 3225120 A1 | 12 January 2023 |
| | | WO 2023280227 A3 | 16 February 2023 |
| | | TW 202320857 A | 01 June 2023 |
| | | AU 2022306065 A1 | 01 February 2024 |
| CN 112533958 A | 19 March 2021 | CA 3107732 A1 | 30 January 2020 |
| | | WO 2020022475 A1 | 30 January 2020 |
| | | TW 202019973 A | 01 June 2020 |
| | | AU 2019311596 A1 | 28 January 2021 |
| | | IL 280296 A | 25 March 2021 |
| | | KR 20210040059 A | 12 April 2021 |
| | | EP 3831853 A1 | 09 June 2021 |

Form PCT/ISA/210 (patent family annex) (July 2022)

**INTERNATIONAL SEARCH REPORT**
**Information on patent family members**

International application No.
**PCT/CN2024/078189**

| Patent document cited in search report | | | Publication date (day/month/year) | Patent family member(s) | | | Publication date (day/month/year) |
|---|---|---|---|---|---|---|---|
| | | | | US | 2021169852 | A1 | 10 June 2021 |
| | | | | EP | 3831853 | A4 | 01 June 2022 |
| | | | | JP | 7406488 | B2 | 27 December 2023 |
| CN | 115551552 | A | 30 December 2022 | US | 2021283125 | A1 | 16 September 2021 |
| | | | | WO | 2021173773 | A1 | 02 September 2021 |
| | | | | EP | 4110402 | A1 | 04 January 2023 |
| | | | | CA | 3168882 | A1 | 02 September 2021 |
| | | | | AU | 2021226341 | A1 | 29 September 2022 |
| | | | | JP | 2023520605 | A | 17 May 2023 |
| | | | | KR | 20230004453 | A | 06 January 2023 |
| | | | | MX | 2022010457 | A | 16 November 2022 |
| | | | | BR | 112022017064 | A2 | 16 November 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7999083 B2
- US 20210169852 A
- US 20210077482 A1
- WO 2021173773 A
- WO 2022170971 A1
- WO 2021148501 A1
- DD 110103

### Non-patent literature cited in the description

- **T. W. GREENE** ; **G. M. WUTS**. Protective groups in Organic Synthesis. Wiley, 1999
- *Angew. Chem. Int. Ed.*, 2010, vol. 49, 9995-9997